# EUROPEAN PATENT APPLICATION

(11) **EP 4 328 219 A1**
(43) Date of publication of application: **28.02.2024**
(21) Application number: 22791138.5
(22) Date of filing: 22.04.2022
(51) Int. Cl.: C07D 239/94, C07D 401/14, A61K 31/517, A61P 35/00

(54) **SOS1 DEGRADING AGENT AND PREPARATION METHOD THEREFOR AND APPLICATION THEREOF**

(30) Priority: 23.04.2021 CN 202110443693; 02.03.2022 CN 202210201700
(71) Applicant: Shanghai Leadingtac Pharmaceutical Co. Ltd., Shanghai 201203 (CN)
(72) Inventor: YE, Zhengqing, Shanghai 201203 (CN); FENG, Yan, Shanghai 201203 (CN); LI, Shiqiang, Shanghai 201203 (CN); DING, Chenli, Shanghai 201203 (CN); WANG, Qiao, Shanghai 201203 (CN); WEI, Hong, Shanghai 201203 (CN); XIE, Shuchen, Shanghai 201203 (CN)
(74) Representative: Maiwald GmbH
(86) International application number: PCT/CN2022/088560
(87) International publication number: WO 2022/223033

(57) **Abstract**

Disclosed are an SOS1 degrading agent and a preparation method therefor and use thereof. Specifically, disclosed is a compound of formula I: S-L-E (I), wherein L is a linking chain, which links S and E by means of covalent bonds; E is a small molecule ligand of an E3 ubiquitin ligase complex. The compound of formula I of the present invention can degrade and/or inhibit SOS1 protein in cells, and can be used for the treatment and/or prevention of an SOS1-mediated disease or disorder, or a disease or disorder caused by the interaction of SOS1 with Ras or SOS 1 with Rac.

## Description

### TECHNICAL FIELD

The present invention belongs to the technical field of pharmaceuticals, and particularly relates to an SOS1 degrader, a preparation method therefor and use thereof as a therapeutic agent preventing and/or treating cancer.

### BACKGROUND

SOS1 (son of sevenless homolog 1) protein is a regulatory protein widely expressed in cells, and as a key protein in signal pathways, SOS1 plays an important role in the regulation in many signal transduction pathways in cells, such as being involved in regulation of Ras and Rac signal pathways. SOS1 consists of 1333 amino acids and comprises a proline-rich domain (PxxP) at its C-terminus. The domain can bind to growth factor receptor-bound protein 2 (Grb2) in the Ras pathway to form a complex of Grb2 and SOS1, thereby bringing SOS1 near the Ras protein on the cell membrane. SOS1 catalyzes the binding of Ras to GTP and promotes the activation of Ras, thereby activating multiple downstream signal pathways, such as Ras-Raf-Mek-Erk and Ras-PI3K-AKT-mTOR. PxxP can also bind to an SH3 (Src homology 3) domain of a protein such as E3B1 in a Rac pathway to form an EPS8-E3B1-SOS1 complex, and the EPS8-E3B1-SOS1 complex is linked to actin filaments through EPS8 to cause the conversion of GTP, so that Rac is activated, and then a signal pathway such as JNK and MAPK is activated.

A mutant ras gene is considered to be the major oncogene with high incidence in human cancer. Studies have shown that Ras mutations occur in 20-30% of tumor patients, where 85% are KRas mutations, 12% are NRas mutations, and 3% are HRas mutations. However, due to the picomolar affinity of GTP for its binding site and the smooth surface of Ras proteins, which means other suitable binding pockets are absent, direct acting on Ras to inhibit its activity is considered to be extremely challenging.

Abnormal expression or mutation of SOS1 is also closely related to the occurrence of clinical diseases. Studies have shown that SOS1 mutations are present in NS patients and CFC patients. HGF1 is a rare autosomal dominant genetic disease whose cause is also associated with mutations in the PxxP domain of SOS1. In addition, abnormal expression or mutation of SOS1 is also related to the occurrence of cancer.

WO2018172250A1, WO2020173935A1, WO2019201848A1, WO2020180768A1, WO2020180770A1, WO2019122129A1 and EP3558979A1 disclose several classes of SOS1 inhibitors, but so far there are no reports on SOS1 degraders. Proteolysis targeting chimera (PROTAC) is a technology different from the traditional small molecule inhibitors. The traditional small molecule inhibitors usually need to act on the active site of a target protein to inhibit the activity of the target protein, while the PROTAC is a heterogeneous bifunctional molecule, one end of which is a small molecule inhibitor capable of recognizing the target protein, and the other end of which is an E3 ubiquitin ligase ligand capable of recognizing E3 ubiquitin ligase via a linking chain. Such a bifunctional molecule recognizes the target protein and the E3 ubiquitin ligase *in vivo,* draws the target protein and the E3 ubiquitin ligase closer to form a ternary complex, and degrades the target protein *in vivo* through a ubiquitin-proteasome pathway after ubiquitinating the target protein. Compared with the traditional small molecule inhibitors, in one aspect, the PROTAC only needs to draw the target protein and the E3 ubiquitin ligase closer to degrade the substrate, and the action mode enables the technology to be applied to some non-druggable targets; in other aspect, the PROTAC molecules can be released to continue to participate in the degradation process of the next protein after the target protein is degraded, so that the degradation effect with a catalytic effect can realize efficient degradation with less dosage of the PROTAC medicament; in yet another aspect, the traditional small molecule inhibitors easily generate drug resistance often because a point mutation occurs, so that the small molecule inhibitors lose the inhibition effect on the target point, while the PROTAC can directly degrade the target protein, so that the drug resistance generated by the point mutation can be avoided to a certain extent. Therefore, compared with the traditional small molecule inhibitors, the PROTAC technology has high advantages and feasibility in research and development of small molecule new drugs.

### SUMMARY

The present invention provides a compound of formula I, and/or a stereoisomer, an enantiomer, a diastereomer, a deuteride, a hydrate, a solvate, a prodrug and/or a pharmaceutically acceptable salt thereof

S-L-E I

wherein:
S is a small molecule compound capable of inhibiting the activity of SOS1 protein or binding to SOS1 protein;
L is a linking chain, which links S and E by means of covalent bonds;
E is a small molecule ligand of an E3 ubiquitin ligase complex.

The present invention provides a compound of formula I, and/or a stereoisomer, an enantiomer, a diastereomer, a deuteride, a hydrate, a solvate, a prodrug and/or a pharmaceutically acceptable salt thereof

S-L-E I

S, L and E are as defined below.

Preferably, in certain embodiments of the present invention, S is S 1: 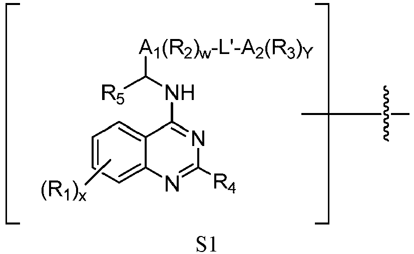 wherein in S1,
R₁ is selected from hydrogen, halogen, -OH, -CN, -NO₂, C₁-C₆ alkyl sulfhydryl and -NRₐR_{b}, wherein Rₐ and R_{b} are each independently hydrogen, C₁-C₆ alkyl, C₃-C₈ heterocycloalkyl or C₃-C₈ cycloalkyl;
R₁ is selected from C₁-C₆ alkyl, C₁-C₆ alkoxy, C₃-C₆ cycloalkoxy, C₃-C₆ heterocycloalkoxy, C₂-C₆ alkenyl, C₂-C₆ alkynyl, C₃-C₈ cycloalkyl, C₄-C₈ cycloalkenyl, 4- to 7-membered heterocycloalkyl, 5- to 10-membered heterocycloalkenyl, spiroheterocycloalkyl, fused heterocycloalkyl, bridged heterocycloalkyl, phenyl, heteroaryl, C₁-C₆ haloalkyl, -COOH and -COOR_{c}; R_{c} is -C₁-C₆ alkyl, C₃-C₆ alkenyl, C₃-C₆ alkynyl, C₃-C₈ cycloalkyl or -C₄-C₈ cycloalkenyl;
R₁ is -NS(O)(R_{d})(Rₑ), wherein R_{d} and Rₑ are each independently C₁-C₆ alkyl, C₂-C₆ alkenyl, C₂-C₆ alkynyl, C₃-C₈ cycloalkyl, C₄-C₈ cycloalkenyl, 6- to 10-membered aryl or 5- to 10-membered heteroaryl;
R₁ is -NHC(O)-(C₁-C₆ alkyl) or -NHC(O)-NRₐR_{b}, wherein Rₐ and R_{b} are each independently hydrogen, -C₁-C₆ alkyl, C₃-C₈ heterocycloalkyl or C₃-C₈ cycloalkyl;
R₁ is -NH-(CH₂)ₖ-NH-C(O)-Rₐₐ, wherein Rₐₐ is C₁-C₆ alkyl, C₃-C₈ heterocycloalkyl or C₃-C₈ cycloalkyl (e.g., Rₐₐ is C₁-C₆ alkyl), and k is 1 or 2;
R₁ is -NH-(CH₂)ᵢ-R_{f}, wherein i is 0, 1 or 2 and R_{f} is 4- to 7-membered heterocycloalkyl, heteroaryl or C₁-C₆ alkylsulfonyl;
furthermore, C₁-C₆ alkyl, C₁-C₆ alkoxy, 4- to 7-membered heterocycloalkyl and heteroaryl described above are optionally substituted with 1, 2 or 3 groups selected from halogen, -OH, oxo, -CN, -NO₂, C₁-C₆ alkyl, C₂-C₆ alkenyl, C₂-C₆ alkynyl, C₃-C₈ cycloalkyl, 4- to 7-membered heterocycloalkyl, C₁-C₆ alkoxy, C₁-C₆ haloalkyl, C₁-C₆ haloalkoxy, C₁-C₆ alkylsulfonyl, phenyl, benzyl, heteroaryl, -(CH₂)-heteroaryl, -NHC(O)(C₁-C₆ alkyl), C₃-C₈ cycloalkoxy, phenoxy, heteroaryloxy and -NRₐR_{b}; Rₐ and R_{b} are each independently selected from hydrogen, C₁-C₆ alkyl, C₃-C₈ heterocycloalkyl and C₃-C₈ cycloalkyl;
R₁ is -O-(CH₂)_{z}-phenyl, -O-(CH₂)_{z}-(4- to 7-membered heterocycloalkyl) or -O-(CH₂)_{z}-heteroaryl, wherein z is 0, 1 or 2, and the phenyl, the heterocycloalkyl and the heteroaryl are optionally substituted with a group selected from - OH, heterocycloalkyl and heterocycloalkenyl and can be substituted with methyl or oxo;
or R₁ is 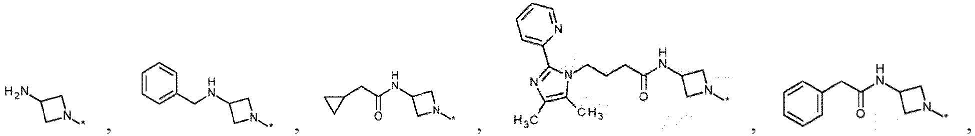 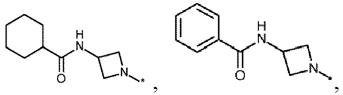 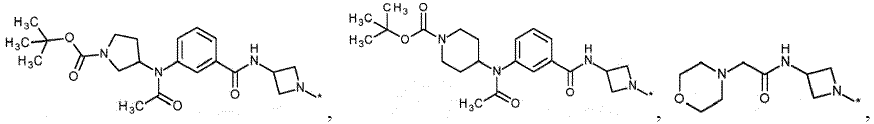 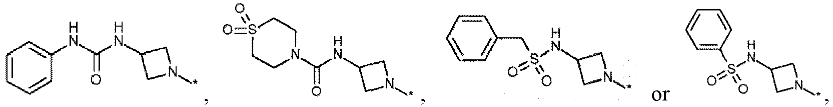
or 2 adjacent R₁, together with the carbon atom linked thereto, form 5- to 7-membered cycloalkyl or 5- to 7-membered heterocycloalkyl;
and x is 1, 2 or 3;
A₁ is C₄-C₁₂ cycloalkyl, heterocycloalkyl, aryl (e.g., phenyl) or heteroaryl (e.g., thienyl);
R₂ is hydrogen, -OH, oxo, halogen, -CN, C₁-C₆ alkyl, C₁-C₆ alkoxy, C₂-C₆ alkenyl, -C₂-C₆ alkynyl, -C₃-C₈ cycloalkyl, -C₃-C₈ cycloalkoxy, -C₃-C₈ halocycloalkoxy, -C₄-C₈ cycloalkenyl, 4- to 7-membered heterocycloalkyl, -O-CH₂-4- to 7-membered heterocycloalkyl, 5- to 10-membered heterocycloalkenyl, spiroheterocycloalkyl, fused heterocycloalkyl, bridged heterocycloalkyl, phenyl, heteroaryl, -C₁-C₆ haloalkyl, -C₁-C₆ haloalkoxy or -C₁-C₆ alkylsulfonyl;
R₂ is -NRₐR_{b}, wherein Rₐ and R_{b} are each independently hydrogen, C₁-C₆ alkyl or -C(O)C₁-C₃ alkyl;
R₂ is -C(O)NRₐR_{b}, wherein Rₐ and R_{b} are each independently hydrogen, C₁-C₆ alkyl, C₃-C₈ heterocycloalkyl or C₃-C₈ cycloalkyl;
R₂ is -C(O)OR_{g}, wherein R_{g} is hydrogen or C₁-C₆ alkyl;
R₂ is -ORₕ; Rₕ is C₁-C₆ alkyl;
R₂ is -(CH₂)NRₐR_{b}, wherein Rₐ and R_{b} are each independently hydrogen, C₁-C₆ alkyl, C₃-C₈ heterocycloalkyl or C₃-C₈ cycloalkyl;
and w is 1, 2, 3 or 4 (e.g., w is 1, 2 or 3);
A₂(R₃)_{Y} is hydrogen;
or A₂ is C₄-C₁₂ cycloalkyl, heterocycloalkyl, aryl (e.g., phenyl) or heteroaryl, and
R₃ is hydrogen, halogen, -OH, oxo, -CN, -NO₂, C₁-C₆ alkyl, C₂-C₆ alkenyl, C₂-C₆ alkynyl, C₃-C₈ cycloalkyl, C₄-C₈ cycloalkenyl, C₇-C₈ cycloalkynyl, 4- to 7-membered heterocycloalkyl, 5- to 10-membered heterocycloalkenyl, phenyl, heteroaryl or C₁-C₆ haloalkyl;
the alkyl, the alkenyl, the alkynyl, the cycloalkyl, the heterocycloalkyl, the aryl, the heteroaryl, the cycloalkenyl, the cycloalkynyl, and the heterocycloalkenyl are optionally substituted with 1, 2 or 3 substituents selected from halogen, -OH, oxo, -CN, -C₁-C₆ alkyl, -C₁-C₆ alkoxy, -C₁-C₆ haloalkyl, phenyl, heteroaryl and -C(O)NRᵢRⱼ, wherein Rᵢ and Rⱼ are each independently hydrogen or C₁-C₆ alkyl;
or the alkyl, the alkenyl, the alkynyl, the cycloalkyl, the heterocycloalkyl, the aryl, the heteroaryl, the cycloalkenyl, the cycloalkynyl and the heterocycloalkenyl are optionally substituted with -NRₖRₗ, Rₖ and Rₗ are each independently hydrogen, C₁-C₆ alkyl, C₂-C₆ alkenyl, C₂-C₆ alkynyl, C₃-C₈ cycloalkyl, C₁-C₆ alkylsulfonyl, phenyl, heteroaryl, -CH₂-C(O)-Rₘ, -C(O)Rₚ or 4- to 7-membered heterocycloalkyl, and the alkyl, the alkynyl, the alkenyl, the cycloalkyl, the phenyl, the heteroaryl and the heterocycloalkyl are each independently optionally substituted with 1, 2 or 3 substituents selected from C₁-C₆ haloalkyl, -OH, oxo, phenyl, -CN, C₁-C₆ alkoxy and heteroaryl, and the heteroaryl is optionally substituted with methyl; and Rₘ is bicyclic heteroaryl, C₁-C₆ alkoxy or -NRₙRₒ, Rₙ and Rₒ are each independently hydrogen, C₁-C₆ alkyl or phenyl, the alkyl is optionally substituted with C₁-C₆ alkoxy or phenyl, or -NRₙRₒ is a 4- to 7-membered azacycloalkyl, and the azacycloalkyl is linked to the rest of the molecule through an N atom and further contains 1 or more heteroatoms selected from N and O; Rₚ is selected from C₁-C₆ alkoxy, C₁-C₆ alkyl optionally substituted with 1, 2 or 3 groups selected from -OH and C₁-C₆ alkoxy, monocyclic or bicyclic heteroaryl and 4- to 7-membered heterocycloalkyl, or Rₚ is -CH₂-NR_{q}Rᵣ, and R_{q} and Rᵣ are each independently selected from hydrogen, phenyl and C₁-C₆ alkyl optionally substituted with F;
or the alkyl, the alkenyl, the alkynyl, the cycloalkyl, the heterocycloalkyl, the aryl, the heteroaryl, the cycloalkenyl, the cycloalkynyl and the heterocycloalkenyl are optionally substituted with -NRₛRₜ, the -NRₛRₜ is 4- to 7-membered azacycloalkyl linked to the rest of the molecule through a nitrogen atom or 6- to 10-membered spiroazacycloalkyl linked to the rest of the molecule through a nitrogen atom and further contains up to two heteroatoms selected from N and O and is optionally substituted with 1, 2 or 3 substituents selected from -OH, oxo, C₁-C₆ alkyl, C₁-C₆ hydroxyalkyl and -C(O)OR₂, and R_{z} is C₁-C₆ alkyl, halogen, -N(C₁-C₆ alkyl)₂, -CH₂N(C₁-C₆ alkyl)₂ or -C(O)NRₐR_{b}, and Rₐ and R_{b} are each independently hydrogen, C₁-C₆ alkyl, C₃-C₈ heterocycloalkyl or C₃-C₈ cycloalkyl;
or R₃ is C(O)Rᵥ, -C(O)NH₂, -C(O)NHRᵥ, -C(O)NRᵥR_{w}, or -C(O)ORᵥ, wherein Rᵥ and R_{w} are each independently hydrogen, C₁-C₄ alkyl, C₁-C₄ haloalkyl, phenyl or -(CH₂)₂NRₓR_{y}, wherein Rₓ and R_{y} are each independently hydrogen, C₁-C₄ alkyl or -(CH₂)₂N(CH₃)₂;
or R₃ is -NH₂, -NHR_{z}, -NR_{z}R_{za}, -NHC(O)R_{z}, -NHC(O)OR_{z}, -NHS(O)₂R_{z}, 4- to 7-membered heterocycloalkyl, heteroaryl, spiroheterocycloalkyl, fused heterocycloalkyl or bridged heterocycloalkyl, and R_{z} and R_{za} are each independently C₁-C₄ alkyl, C₁-C₄ haloalkyl or phenyl;
or R₃ is C₁-C₆ alkoxy, C₁-C₆ haloalkoxy, -O(CH₂)ₛ-C₃-C₈ cycloalkyl, -O(CH₂)ₛ-phenyl, -O(CH₂)ₛ-heterocycloalkyl, or -O(CH₂)ₛ-heteroaryl, wherein s is 0, 1, 2 or 3;
or R₃ is -S(O)₂R_{z}, -S(O)₂NH₂, -S(O)₂NHR_{z}, or -S(O)₂NR_{z}R_{za}, wherein R_{z} and R_{za} are each independently C₁-C₄ alkyl, C₁-C₄ haloalkyl or phenyl;
Y is 0, 1, 2, 3, 4 or 5 (e.g., Y is 0, 1, 2 or 3);
L' is a bond, -(CH₂)ₖ-, -O(CH₂)ₖ-, -(CH₂)ₖ-O- or -O-(CH₂)ₖ-O- (e.g., L' is a bond, -(CH₂)ₖ or -O(CH₂)ₖ-), wherein k is 0, 1, 2 or 3, or L' is -CH=CH-(CH₂)ₙ-, wherein n is 0, 1 or 2;
R₄ and R₅ are each independently C₁-C₆ alkyl, C₁-C₆ alkoxy, C₃-C₆ cycloalkyl or C₃-C₆ cycloalkoxy, wherein the alkyl, the alkoxy, the cycloalkyl and the cycloalkoxy are optionally substituted with cyano, hydroxy, C₁-C₆ alkoxy, C₃-C₆ cycloalkyl, C₃-C₆ cycloalkoxy or halogen.

Preferably, in certain embodiments of the present invention, in S 1, R₄ and R₅ are each independently methyl.

In some embodiments, in the definition of each group in S1, Rₐ and R_{b}, when occurring at any position, are each independently hydrogen or C₁-C₆ alkyl.

In some embodiments, in the definition of each group in S1, R_{d} and Rₑ, when occurring at any position, are each independently hydrogen or C₁-C₆ alkyl.

In certain embodiments of the present invention, in S1, R₅ is C₁-C₆ alkyl, e.g., methyl.

In certain embodiments of the present invention, in S1, R₄ is C₁-C₆ alkyl, e.g., methyl.

In certain embodiments of the present invention, in S1, R₁ is hydrogen or C₁-C₆ alkoxy.

In certain embodiments of the present invention, in S 1, A₁ is aryl or heteroaryl.

In certain embodiments of the present invention, in S1, A₁ is aryl, e.g., phenyl.

In certain embodiments of the present invention, in S1, A₁ is heteroaryl, e.g., thienyl.

In certain embodiments of the present invention, in S1, R₂ is hydrogen, halogen, C₁-C₆ alkyl or -C₁-C₆ haloalkyl.

In certain embodiments of the present invention, in S 1, L' is a bond.

In certain embodiments of the present invention, in S1, A₂(R₃)_{Y} is hydrogen.

In certain embodiments of the present invention, in S1, A₂ is aryl, e.g., phenyl.

In certain embodiments of the present invention, in S1, R₃ is hydrogen, halogen or C₁-C₆ alkyl, and the alkyl is optionally substituted with -NRₖRₗ; Rₖ and R₁ are each independently hydrogen or C₁-C₆ alkyl. In certain embodiments of the present invention, in S7, R₃ is hydrogen, F, Cl, methyl or ethyl, and the methyl and the ethyl are substituted with -NRₖRₗ; Rₖ is methyl or ethyl, and Rₗ is hydrogen, methyl or ethyl.

In certain embodiments of the present invention, in S1, R₃ is hydrogen, Cl, -CH₂NHCH₃ or -CH₂N(CH₃)₂.

In certain embodiments of the present invention, in S1, A₁ is aryl, L' is a bond, and A₂(R₃)_{Y} is hydrogen.

In certain embodiments of the present invention, in S1, A₁ is heteroaryl, L' is a bond, A₂ is aryl, R₃ is hydrogen, halogen or C₁-C₆ alkyl, and the alkyl is optionally substituted with -NRₖRₗ; Rₖ and Rₗ are each independently hydrogen or C₁-C₆ alkyl.

In certain embodiments of the present invention, S is S 1': 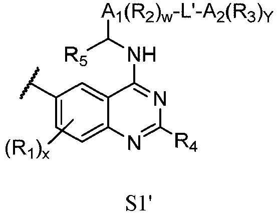 wherein in S1', R₁, R₂, R₃, R₄, R₅, L', A₁, A₂, w, x and Y are as defined in S1.

In certain embodiments of the present invention, S is S 1": 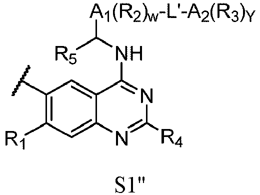 wherein in S1", R₁, R₂, R₃, R₄, R₅, L', A₁, A₂, w and Y are as defined in S1.

In certain embodiments of the present invention, in S1' or S1", R₁ is hydrogen, C₁-C₆ alkyl or C₁-C₆ alkoxy, and the C₁-C₆ alkyl and the C₁-C₆ alkoxy are optionally substituted with C₁-C₆ alkoxy (e.g. methoxy).

In certain embodiments of the present invention, in S1' or S1", R₁ is hydrogen, methyl, methoxy or

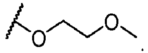

In certain embodiments of the present invention, in S1 and S1', the fragment 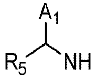 is

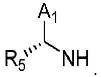

In certain embodiments of the present invention, S is -X-S1', and the structure of S1' is as described above; X is O, NH or S, preferably O.

In certain embodiments of the present invention, S is -X-S1", and the structure of S1" is as described above; X is O, NH or S, preferably O.

Preferably, in certain embodiments of the present invention, S is S1a 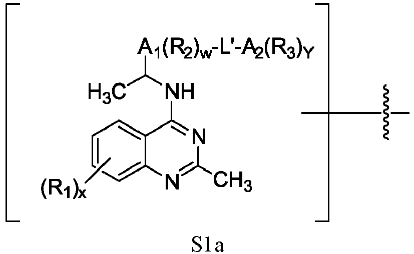 wherein in S1a,
R₁ is selected from hydrogen, -OCH₃, -OCH₂CH₃, 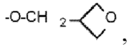 -CH₂OH, -C(O)OH, -C(O)OCH₃, -Br, - OCH(CH₃)₂, -O(CH₂)₂CH(CH₃)₂, -O(CH₂)₃CH₃, -O(CH₂)₂OCH₃, 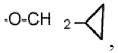 -O(CH₂)-phenyl, -N=S(O)(CH₃)₂, - CH₃, cyclopropyl, -N(CH₃)₂, -NHCH₃, -NH₂, 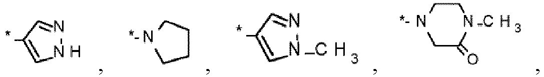 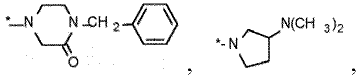 -C(CH₃)₂-OH, 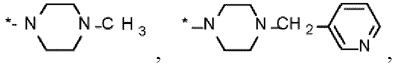 -NH(CH₂)-NH-C(O)CH₃, -NH(CH₂)-morpholine, -NH-C(O)CH₃, -NH-C(O)NHCH₃, -NH-C(O)-N(CH₃)₂, nitro, -NH-S(O)₂CH₃, -N=S(O)(CH₃)₂, hydroxy, -O-(CH₂)₂-S(O)₂CH₃, -F, 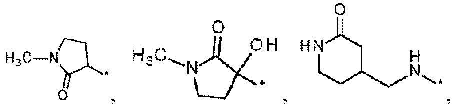 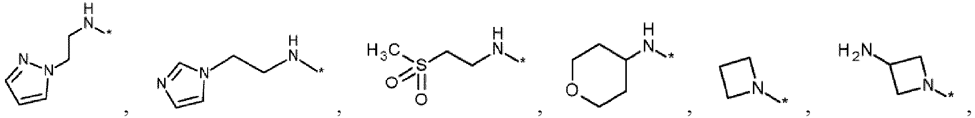 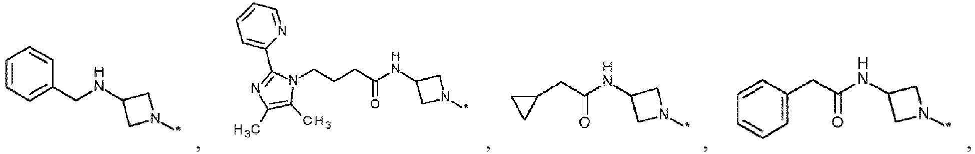 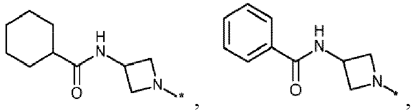 cyclopropyloxy, cyclobutyloxy, cyclopentyloxy, cyclohexyloxy, azetidinyl, azacyclopentyl, piperidinyl, piperazinyl, oxetanyl, oxolanyl, oxanyl, thietanyl, thiolanyl, thianyl, azetidinyloxy, azacyclopentyloxy, piperidinyloxy, piperazinyloxy, oxetanyloxy, oxolanyloxy, oxanyloxy, thietanyloxy, thiolanyloxy, thianyloxy, 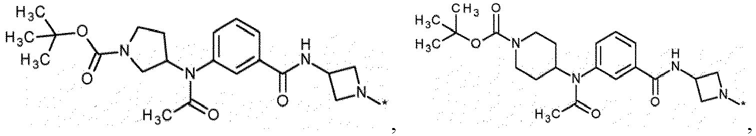 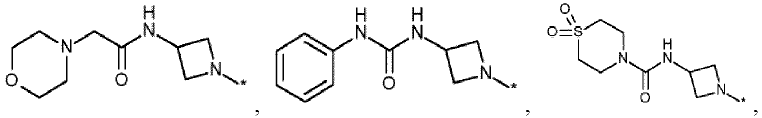 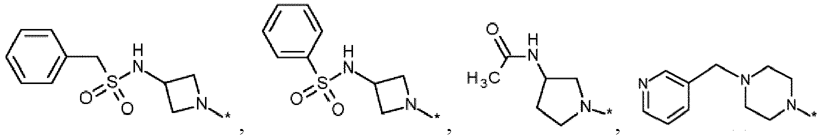 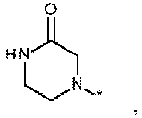 -OCH₂CH₂CH₃, -OCH₂CH₂CH₂N(CH₃)₂, -OCH₂CH₂CH₂OH, 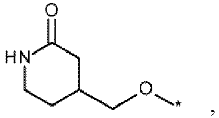 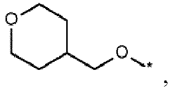 -OCH₂CH₂NC(O)CH₃, -OCH₂CH₂N(CH₃)₂, -OCH₂CH₂OH, 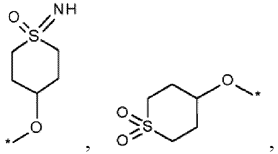 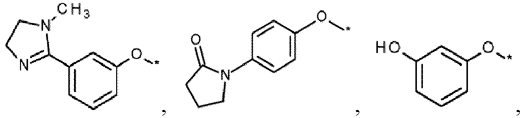 -SCH₃, -N(CH₃)₂, 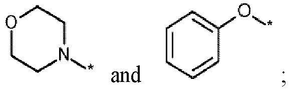
x is 1 or 2;
A₁ is selected from 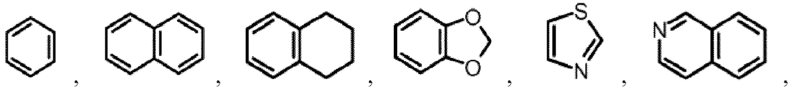 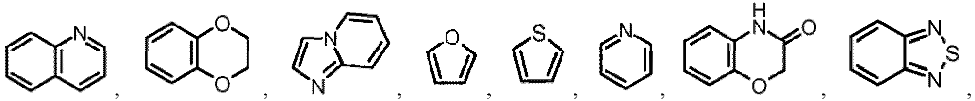 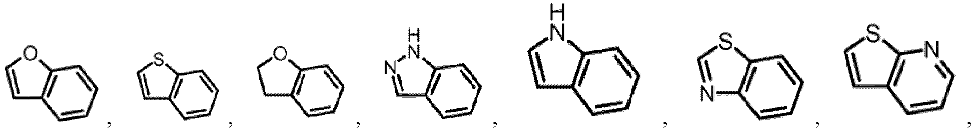 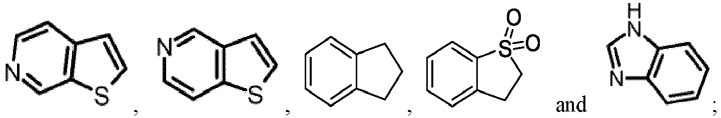
R₂ is selected from hydrogen, hydroxy, oxo, cyano, cyclopropyl, 1,1-dimethylcyclopropyl, -C(=CH₂)CH₃, - C(CH₃)(=CH₂)CH₃, -CH=CH(CH₂)₂CH₃, -CH=CHCH₃, -CH=CH-cyclopropyl, -C(O)NH₂, -C(O)OCH₃, - S(O)₂CH₃, -OCH₃, -CH₂NH₂, trifluoromethyl, methyl, trifluoromethoxy, halogen (F, Cl or Br), -NH₂, -NHC(O)CH₃, -NHCH₂CH₃ and -NHCH(CH₃)₂; w is 1, 2 or 3;
A₂ is selected from 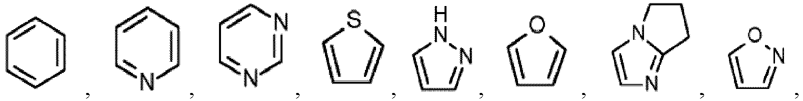 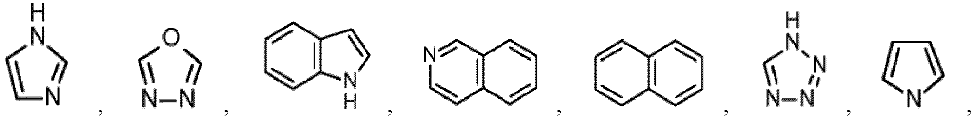 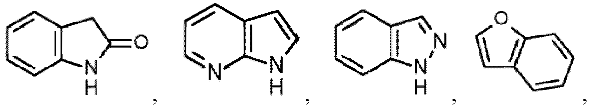 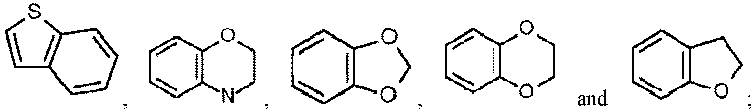
R₃ is selected from -C(O)NH(CH₂)₂CH₃, -C(O)N(CH₃)₂, -C(O)NH₂, -C(O)NH(CH₂)₂N(CH₃)₂, -CH₂C(O)NH₂, hydrogen, -F, -Cl, -Br, cyano, -CF₃, -CH₃, -CH₂CH₃, -CH=CH₂, -CH₂CN, -CH(CH₃)-NH₂, -CH=CH-CN, -C(O)-OH, -C(O)OCH₃, -C(O)CH₃, -C(CH₃)₂-C(O)-OCH₃, -C(CH₃)₂-CN, oxo, hydroxy, cyclopropyl, cyclobutyl, cyclopentyl, -NH₂, -NH-C(O)CH₃, -NH-S(O)₂CH₃, -NH-C(O)OC(CH₃)₃, 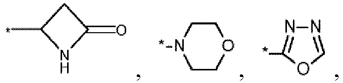 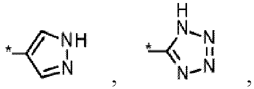 -S(O)₂CH₃, -S(O)₂NCH₃, -S(O)₂NH₂, -OCH₂CH₃, -O(CH₂)₂CH₃, -OCF₃, 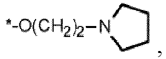 -OCH₂cyclopropyl, -OCH₃, -O(CH₂)₃CH₃, -OCH₂phenyl, -O-phenyl, -(CH₂)-OH, -(CH₂)₂-OH, -(CH₂)-OCH₃, -(CH₂)-OCH₂CH₃, -CH(OH)-CH₂-phenyl, -CH(OH)-CH₂CH₃, -CH(OH)-CH₂CH₂CH₃, -CH(OH)-CH₂CH₂CH₂CH₃, -CH(OH)-CH(CH₃)₂, -CH(OH)-phenyl, -CH(OH)-CN, -CH(OH)-CH₂-OH, -CH(OH)-CF₃, - CH(OH)-(CH₂)₂-phenyl, *-CH(OH)-C≡CH , -CH(NH₂)-CH₂-C(O)OH, -CH₂-NH-S(O)₂-CH₃, -CH₂-NH-(CH₂)₃CH₃, - CH₂-NH-CH₃, -CH₂-N(CH₃)₂, -CH₂-NH-CH₂CH₃, -CH(CH₃)-NH₂, -CH₂-NH₂, -CH₂CH₂-NH₂, -CH₂NH-CH₂-phenyl, -CH₂N(CH₂CH₃)₂, -CH₂NH-cyclopropyl, -CH₂NH-cyclobutyl, -CH₂NH-cyclopentyl, -CH₂NH-pyridyl, - CH₂NH-phenyl, -CH₂NH-(CH₂)₂-OH, -CH₂N(CH₃)-(CH₂)₂-OH, -CH₂NH-CH₂-CN, -CH₂N(CH₃)-CH₂-CN, - CH₂N(CH₃)-CH₂-CF₃, -CH₂N(CH₃)-CH₂-CF₂H, -CH₂NH-CH₂-CF₂H, -CH₂NH-(CH₂)₂-OCH₃, 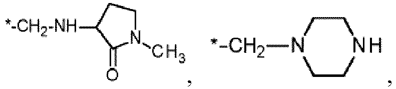 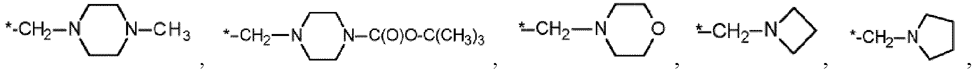 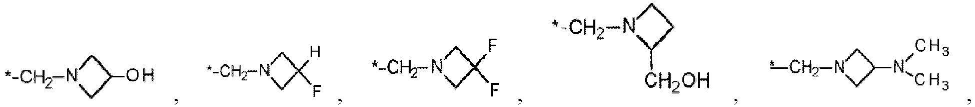 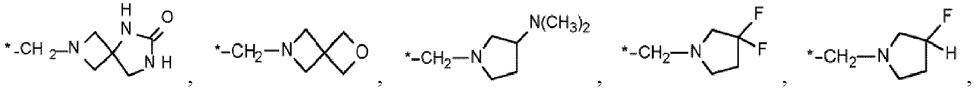 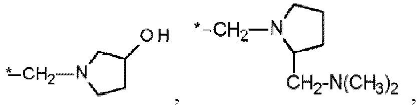 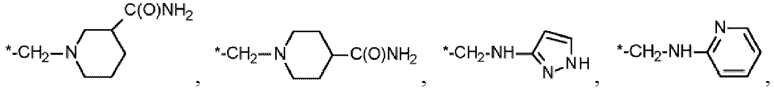 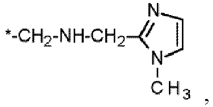 -CH₂NH-C(O)-OC(CH₃)₃, -CH₂CH₂NH-C(O)-OC(CH₃)₃, -CH₂NH-C(O)-CH₂NOH, - CH₂NH-C(O)-CH₂OCH₃, -CH(CH₃)NH-C(O)-OC(CH₃)₃, -CH₂NH-C(O)-CH₃, 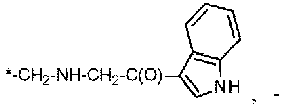 CH₂NHCH₂-C(O)-NH₂, -CH₂NHCH₂-C(O)-(CH₃)₂, -CH₂NHCH₂-C(O)-OCH₃, -CH₂NHCH₂-C(O)-NHCH₃, - CH₂NHCH₂-C(O)-NH(CH₂)₂-OCH₃, -CH₂NHCH₂-C(O)-NHCH₂-phenyl, 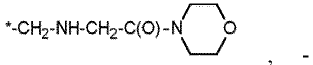 CH₂NHCH₂-C(O)-NH-phenyl, 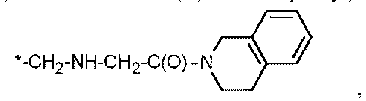 -CH₂NH-C(O)-CH₂NH-phenyl, 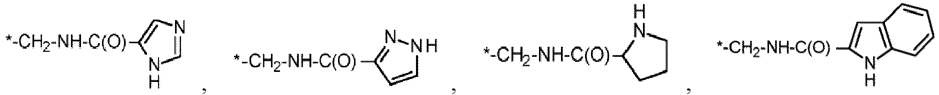 and - CH₂NH-C(O)-CH₂NH-CH₂CF₃;
Y is 1 or 2;
L' is a bond, -(CH₂)ₖ- or -O(CH₂)ₖ-, wherein k is 1 or 2, or L' is -CH=CH-(CH₂)ₙ-, wherein n is 0, 1 or 2.

In some embodiments, in S1a, R₂ is selected from hydrogen, hydroxy, oxo, cyano, cyclopropyl, 1,1-dimethylcyclopropyl, -C(=CH₂)CH₃, -C(CH₃)(=CH₂)CH₃, -CH=CH(CH₂)₂CH₃, -CH=CHCH₃, -CH=CH-cyclopropyl, -C(O)NH₂, -C(O)OCH₃, -S(O)₂CH₃, -OCH₃, -CH₂NH₂, trifluoromethyl, methyl, trifluoromethoxy and halogen (F, Cl or Br).

In certain embodiments of the present invention, in S1a, w is 1 or 2.

In certain embodiments of the present invention, in S1a, R₁ is hydrogen, -CH₃, -OCH₃, -OCH₂CH₃, -OCH(CH₃)₂, - O(CH₂)₂CH(CH₃)₂ or -O(CH₂)₃CH₃, e.g., -OCH₃.

In certain embodiments of the present invention, in S1a, A₁ is

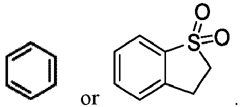

In certain embodiments of the present invention, in S1a, A₁ is

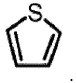

In certain embodiments of the present invention, in S1a, R₂ is hydrogen, halogen, -NH₂, C₁-C₆ alkyl or -C₁-C₆ haloalkyl.

In certain embodiments of the present invention, in S1a, L' is a bond.

In certain embodiments of the present invention, in S1a, A₂(R₃)_{Y} is hydrogen.

In certain embodiments of the present invention, in S1a, A₂ is

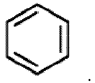

In certain embodiments of the present invention, in S1a, R₃ is hydrogen, -F, -Cl, -Br, -CH₂-NH-(CH₂)₃CH₃, -CH₂-NH-CH₃, -CH₂-N(CH₃)₂ or -CH₂-NH-CH₂CH₃.

In certain embodiments of the present invention, in S1a, R₃ is hydrogen, Cl, -CH₂NHCH₃ or -CH₂N(CH₃)₂.

In certain embodiments of the present invention, in S1a, A₁ is 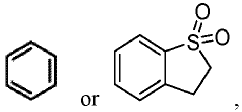 L' is a bond, and A₂(R₃)_{Y} is hydrogen.

In certain embodiments of the present invention, in S1a, A₁ is 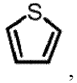 L' is a bond, A₂ is 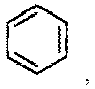 , and R₃ is hydrogen, -F, -Cl, -Br, -CH₂-NH-(CH₂)₃CH₃, -CH₂-NH-CH₃, -CH₂-N(CH₃)₂ or -CH₂-NH-CH₂CH₃. In certain embodiments of the present invention, S is S1a': 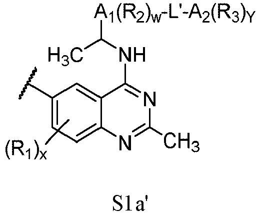 wherein in S1a', R₁, R₂, R₃, L', A₁, A₂, w, x and Y are as defined in S1 or S1a.

In certain embodiments of the present invention, S is S1a": 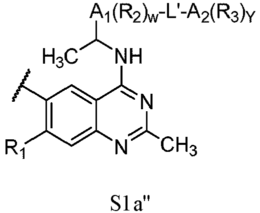 wherein in S1a", R₁, R₂, R₃, L', A₁, A₂, w and Y are as defined in S1 or S1a.

In certain embodiments of the present invention, in S1a' and S1a", R₁ is hydrogen, C₁-C₆ alkyl or C₁-C₆ alkoxy, and the C₁-C₆ alkyl and the C₁-C₆ alkoxy are optionally substituted with C₁-C₆ alkoxy (e.g. methoxy).

In certain embodiments of the present invention, in S1a' and S1a", Rₗ is hydrogen, methyl, methoxy or

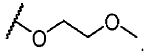

In certain embodiments of the present invention, in S1a, S1a' and S1a", the fragment 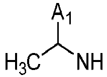 is

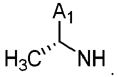

In certain embodiments of the present invention, S is -X-S1a', and the structure of S1a' is as described above; X is O, NH or S, preferably O.

In certain embodiments of the present invention, S is -X-S1a", and the structure of S1a" is as described above; X is O, NH or S, preferably O.

More preferably, in certain embodiments of the present invention, S1 is the specific compound according to Example 1 to Example 458 in WO2018172250A1 (incorporated herein by reference in its entirety).

Preferably, in certain embodiments of the present invention, S is S2: 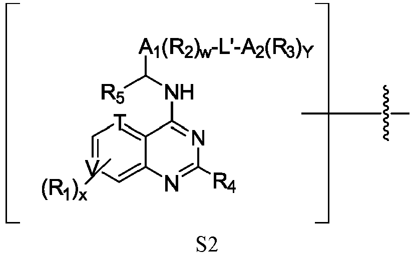 wherein in S2,
R₁ is hydrogen, halogen, -OH, -CN, -NO₂, C₁-C₆ alkyl sulfhydryl or -NRₐR_{b}, wherein Rₐ and R_{b} are each independently hydrogen, C₁-C₆ alkyl, C₃-C₈ heterocycloalkyl or C₃-C₈ cycloalkyl;
R₁ is selected from C₁-C₆ alkyl, C₁-C₆ alkoxy, C₃-C₆ cycloalkoxy, C₃-C₆ heterocycloalkoxy, C₂-C₆ alkenyl, C₂-C₆ alkynyl, C₃-C₈ cycloalkyl, C₄-C₈ cycloalkenyl, 4- to 7-membered heterocycloalkyl, 5- to 10-membered heterocycloalkenyl, spiroheterocycloalkyl, fused heterocycloalkyl, bridged heterocycloalkyl, phenyl, heteroaryl, C₁-C₆ haloalkyl, -COOH and -COOR_{c}; R_{c} is -C₁-C₆ alkyl, C₃-C₆ alkenyl, C₃-C₆ alkynyl, C₃-C₈ cycloalkyl or -C₄-C₈ cycloalkenyl;
R₁ is -NS(O)(R_{d})(Rₑ), wherein R_{d} and Rₑ are each independently C₁-C₆ alkyl, C₂-C₆ alkenyl, C₂-C₆ alkynyl, C₃-C₈ cycloalkyl or C₄-C₈ cycloalkenyl;
R₁ is -NHC(O)-(C₁-C₆ alkyl) or -NHC(O)-NRₐR_{b}, wherein Rₐ and R_{b} are each independently hydrogen, C₁-C₆ alkyl, C₃-C₈ heterocycloalkyl or C₃-C₈ cycloalkyl;
Rₗ is -NH-(CH₂)ₖ-NH-C(O)-(C₁-C₆ alkyl), wherein k is 1 or 2;
Rₗ is -NH-(CH₂)ᵢ-R_{f}, wherein i is 0, 1 or 2 and R_{f} is 4- to 7-membered heterocycloalkyl, heteroaryl or C₁-C₆ alkylsulfonyl;
furthermore, C₁-C₆ alkyl, C₁-C₆ alkoxy, 4- to 7-membered heterocycloalkyl and heteroaryl described above are optionally substituted with 1, 2 or 3 groups selected from halogen, -OH, oxo, -CN, -NO₂, C₁-C₆ alkyl, C₂-C₆ alkenyl, C₂-C₆ alkynyl, C₃-C₈ cycloalkyl, 4- to 7-membered heterocycloalkyl, C₁-C₆ alkoxy, C₁-C₆ haloalkyl, C₁-C₆ haloalkoxy, C₁-C₆ alkylsulfonyl, phenyl, benzyl, heteroaryl, -(CH₂)-heteroaryl, -NHC(O)(C₁-C₆ alkyl), C₃-C₈ cycloalkoxy, phenoxy, heteroaryloxy and -NRₐR_{b}; Rₐ and R_{b} are each independently selected from hydrogen, C₁-C₆ alkyl, C₃-C₈ heterocycloalkyl and C₃-C₈ cycloalkyl;
R₁ is -O-(CH₂)_{z}-phenyl, -O-(CH₂)_{z}-(4- to 7-membered heterocycloalkyl) or -O-(CH₂)_{z}-heteroaryl, wherein z is 0, 1 or 2, and the phenyl, the heterocycloalkyl and the heteroaryl are optionally substituted with a group selected from - OH, heterocycloalkyl and heterocycloalkenyl and can be substituted with methyl or oxo;
or R₁ is 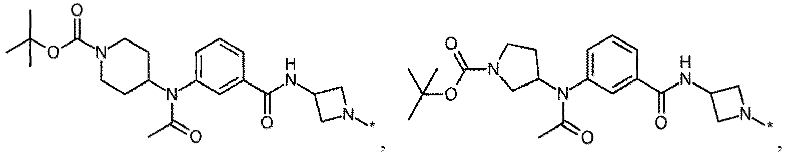 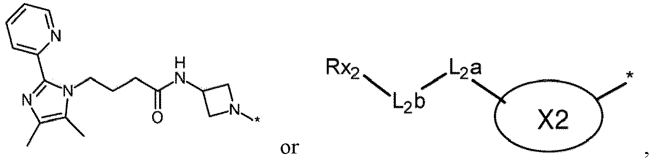 L₂a is C(O), L₂b is a bond or C₁-C₆ alkylene, X₂ is 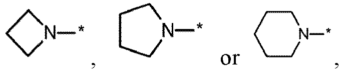 Rx₂ is 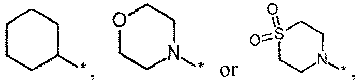
or R₁ is 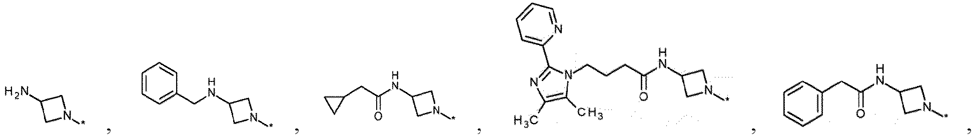 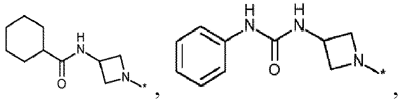 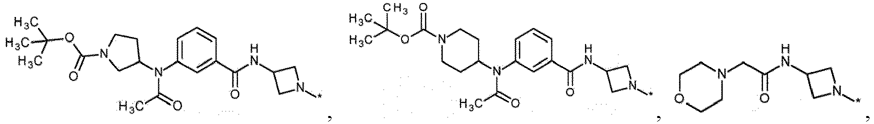 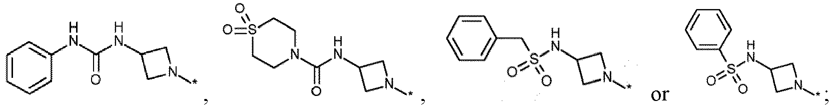
or 2 adjacent R₁, together with the carbon atom linked thereto, form 5- to 7-membered cycloalkyl or 5- to 7-membered heterocycloalkyl;
and x is 1, 2, 3 or 4 (e.g., x is 1, 2 or 3);
A₁ is C₄-C₁₂ cycloalkyl, heterocycloalkyl, aryl or heteroaryl;
R₂ is hydrogen, -OH, oxo, halogen, -CN, C₁-C₆ alkyl, C₁-C₆ alkoxy, C₂-C₆ alkenyl, -C₂-C₆ alkynyl, -C₃-C₈ cycloalkyl, -C₃-C₈ cycloalkoxy, -C₃-C₈ halocycloalkoxy, -C₄-C₈ cycloalkenyl, 4- to 7-membered heterocycloalkyl, -O-CH₂-4- to 7-membered heterocycloalkyl, 5- to 10-membered heterocycloalkenyl, spiroheterocycloalkyl, fused heterocycloalkyl, bridged heterocycloalkyl, phenyl, heteroaryl, -C₁-C₆ haloalkyl, -C₁-C₆ haloalkoxy or -C₁-C₆ alkylsulfonyl;
R₂ is -NRₐR_{b}, wherein Rₐ and R_{b} are each independently hydrogen, C₁-C₆ alkyl, C₃-C₈ heterocycloalkyl or C₃-C₈ cycloalkyl;
R₂ is -C(O)NRₐR_{b}, wherein Rₐ and R_{b} are each independently hydrogen, C₁-C₆ alkyl, C₃-C₈ heterocycloalkyl or C₃-C₈ cycloalkyl;
R₂ is -C(O)OR_{g}, wherein R_{g} is hydrogen or C₁-C₆ alkyl;
R₂ is -ORₕ; Rₕ is C₁-C₆ alkyl;
R₂ is -(CH₂)NRₐR_{b}, wherein Rₐ and R_{b} are each independently hydrogen, C₁-C₆ alkyl, C₃-C₈ heterocycloalkyl or C₃-C₈ cycloalkyl;
and w is 1, 2, 3 or 4 (e.g., w is 1 or 2);
A₂(R₃)_{Y} is hydrogen;
or A₂ is C₄-C₁₂ cycloalkyl, heterocycloalkyl, aryl or heteroaryl, and
R₃ is hydrogen, halogen, -OH, oxo, -CN, -NO₂, C₁-C₆ alkyl, C₂-C₆ alkenyl, C₂-C₆ alkynyl, C₃-C₈ cycloalkyl, C₄-C₈ cycloalkenyl, C₂-C₈ cycloalkynyl, 4- to 7-membered heterocycloalkyl, 5- to 10-membered heterocycloalkenyl, phenyl, heteroaryl or C₁-C₆ haloalkyl;
the alkyl, the alkenyl, the alkynyl, the cycloalkyl, the heterocycloalkyl, the aryl, the heteroaryl, the cycloalkenyl, the cycloalkynyl, and the heterocycloalkenyl are optionally substituted with 1, 2 or 3 substituents selected from halogen, -OH, oxo, -CN, -C₁-C₆ alkyl, -C₁-C₆ alkoxy, -C₁-C₆ haloalkyl, phenyl, heteroaryl and -C(O)NRᵢRⱼ, wherein Rᵢ and Rⱼ are each independently hydrogen or C₁-C₆ alkyl;
or the alkyl, the alkenyl, the alkynyl, the cycloalkyl, the heterocycloalkyl, the aryl, the heteroaryl, the cycloalkenyl, the cycloalkynyl and the heterocycloalkenyl are optionally substituted with -NRₖRₗ, Rₖ and Rₗ are each independently hydrogen, C₁-C₆ alkyl, C₂-C₆ alkenyl, C₂-C₆ alkynyl, C₃-C₈ cycloalkyl, C₁-C₆ alkylsulfonyl, phenyl, heteroaryl, -CH₂-C(O)-Rₘ, -C(O)Rₚ or 4- to 7-membered heterocycloalkyl, and the alkyl, the alkynyl, the alkenyl, the cycloalkyl, the phenyl, the heteroaryl and the heterocycloalkyl are each independently optionally substituted with 1, 2 or 3 substituents selected from C₁-C₆ haloalkyl, -OH, oxo, phenyl, -CN, C₁-C₆ alkoxy and heteroaryl, and the heteroaryl is optionally substituted with methyl; and Rₘ is bicyclic heteroaryl, C₁-C₆ alkoxy or -NRₙRₒ, Rₙ and Rₒ are each independently hydrogen, C₁-C₆ alkyl or phenyl, the alkyl is optionally substituted with C₁-C₆ alkoxy or phenyl, or -NRₙRₒ is a 4- to 7-membered azacycloalkyl, and the azacycloalkyl is linked to the rest of the molecule through an N atom and further contains 1 or more heteroatoms selected from N and O; Rₚ is selected from C₁-C₆ alkoxy, C₁-C₆ alkyl optionally substituted with 1, 2 or 3 groups selected from -OH and C₁-C₆ alkoxy, monocyclic or bicyclic heteroaryl and 4- to 7-membered heterocycloalkyl, or Rₚ is -CH₂-NR_{q}Rᵣ, and R_{q} and Rᵣ are each independently selected from hydrogen, phenyl and C₁-C₆ alkyl optionally substituted with F;
or the alkyl, the alkenyl, the alkynyl, the cycloalkyl, the heterocycloalkyl, the aryl, the heteroaryl, the cycloalkenyl, the cycloalkynyl and the heterocycloalkenyl are optionally substituted with -NRₛRₜ, the -NRₛRₜ is 4- to 7-membered azacycloalkyl linked to the rest of the molecule through a nitrogen atom or 6- to 10-membered spiroazacycloalkyl linked to the rest of the molecule through a nitrogen atom and further contains up to two heteroatoms selected from N and O and is optionally substituted with 1, 2 or 3 substituents selected from -OH, oxo, C₁-C₆ alkyl, C₁-C₆ hydroxyalkyl and -C(O)OR₂, and R_{z} is C₁-C₆ alkyl, halogen, -N(C₁-C₆ alkyl)₂, -CH₂N(C₁-C₆ alkyl)₂ or -C(O)NRₐR_{b}, and Rₐ and R_{b} are each independently hydrogen, C₁-C₆ alkyl, C₃-C₈ heterocycloalkyl or C₃-C₈ cycloalkyl;
or R₃ is C(O)Rᵥ, -C(O)NH₂, -C(O)NHRᵥ, -C(O)NRᵥR_{w}, or -C(O)ORᵥ, wherein Rᵥ and R_{w} are each independently hydrogen, C₁-C₄ alkyl, C₁-C₄ haloalkyl, phenyl or -(CH₂)₂NRₓR_{y}, wherein Rₓ and R_{y} are each independently hydrogen, C₁-C₄ alkyl or -(CH₂)₂NH(CH₃)₂;
or R₃ is -NH₂, -NHR_{z}, -NR_{z}R_{za}, -NHC(O)R_{z}, -NHC(O)OR_{z}, -NHS(O)₂R_{z}, 4- to 7-membered heterocycloalkyl, heteroaryl, spiroheterocycloalkyl, fused heterocycloalkyl or bridged heterocycloalkyl, and R_{z} and R_{za} are each independently C₁-C₄ alkyl, C₁-C₄ haloalkyl or phenyl;
or R₃ is C₁-C₆ alkoxy, C₁-C₆ haloalkoxy, -O(CH₂)ₛ-C₃-C₈ cycloalkyl, -O(CH₂)ₛ-phenyl, -O(CH₂)ₛ-heterocycloalkyl, or -O(CH₂)ₛ-heteroaryl, wherein s is 0, 1, 2 or 3;
or R₃ is -S(O)₂R_{z}, -S(O)₂NH₂, -S(O)₂NHR_{z}, or -S(O)₂NR_{z}R_{za}, wherein R_{z} and R_{za} are each independently C₁-C₄ alkyl, C₁-C₄ haloalkyl or phenyl;
Y is 0, 1, 2, 3, 4 or 5 (e.g., Y is 0, 1, 2 or 3);
L' is a bond, -(CH₂)ₖ-, -O(CH₂)ₖ-, -(CH₂)ₖ-O- or -O-(CH₂)ₖ-O- (e.g., L' is a bond, -(CH₂)ₖ or -O(CH₂)ₖ-), wherein k is 0, 1, 2 or 3, or L' is -CH=CH-(CH₂)ₙ-, wherein n is 0, 1 or 2;
R₄ and R₅ are each independently C₁-C₆ alkyl, C₁-C₆ alkoxy, C₃-C₆ cycloalkyl or C₃-C₆ cycloalkoxy, wherein the alkyl, the alkoxy, the cycloalkyl and the cycloalkoxy are optionally substituted with cyano, hydroxy, C₁-C₆ alkoxy, C₃-C₆ cycloalkyl, C₃-C₆ cycloalkoxy or halogen;
T and V are both N, or T is C and V is N, or T is N and V is C.

Preferably, in certain embodiments of the present invention, in S2, R₄ and R₅ are each independently methyl.

In some embodiments, in the definition of each group in S2, Rₐ and R_{b}, when occurring at any position, are each independently hydrogen or C₁-C₆ alkyl.

In certain embodiments of the present invention, in S2, T and V are both N.

In certain embodiments of the present invention, in S2, T is C and V is N.

In certain embodiments of the present invention, in S2, T is N and V is C.

In certain embodiments of the present invention, in S2, R₅ is C₁-C₆ alkyl, e.g., methyl.

In certain embodiments of the present invention, in S2, R₄ is C₁-C₆ alkyl, e.g., methyl.

In certain embodiments of the present invention, in S2, R₁ is hydrogen or C₁-C₆ alkoxy.

In certain embodiments of the present invention, in S2, A₁ is aryl or heteroaryl.

In certain embodiments of the present invention, in S2, A₁ is aryl, e.g., phenyl.

In certain embodiments of the present invention, in S2, A₁ is heteroaryl, e.g., thienyl.

In certain embodiments of the present invention, in S2, R₂ is hydrogen, halogen, C₁-C₆ alkyl or -C₁-C₆ haloalkyl. In certain embodiments of the present invention, in S2, L' is a bond.

In certain embodiments of the present invention, in S2, A₂(R₃)_{Y} is hydrogen.

In certain embodiments of the present invention, in S2, A₂ is aryl, e.g., phenyl.

In certain embodiments of the present invention, in S2, R₃ is hydrogen, halogen or C₁-C₆ alkyl, and the alkyl is optionally substituted with -NRₖRₗ; Rₖ and Rₗ are each independently hydrogen or C₁-C₆ alkyl. In certain embodiments of the present invention, in S7, R₃ is hydrogen, F, Cl, methyl or ethyl, and the methyl and the ethyl are substituted with -NRₖRₗ; Rₖ is methyl or ethyl, and Rₗ is hydrogen, methyl or ethyl.

In certain embodiments of the present invention, in S2, R₃ is hydrogen, Cl, -CH₂NHCH₃ or -CH₂N(CH₃)₂.

In certain embodiments of the present invention, in S2, A₁ is aryl, L' is a bond, and A₂(R₃)_{Y} is hydrogen.

In certain embodiments of the present invention, in S2, A₁ is heteroaryl, L' is a bond, A₂ is aryl, R₃ is hydrogen, halogen or C₁-C₆ alkyl, and the alkyl is optionally substituted with -NRₖRₗ; Rₖ and Rₗ are each independently hydrogen or C₁-C₆ alkyl.

In certain embodiments of the present invention, S is S2': 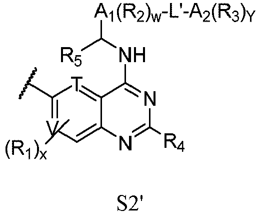 wherein in S2', T, V, R₁, R₂, R₃, R₄, R₅, L', A₁, A₂, w, x and Y are as defined in S2.

In certain embodiments of the present invention, S is S2": 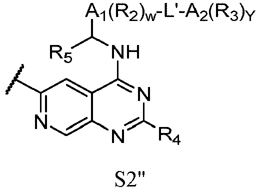 wherein in S2", R₂, R₃, R₄, R₅, L', A₁, A₂, w and Y are as defined in S2.

In certain embodiments of the present invention, in S2, S2' and S2", R₁ is hydrogen, C₁-C₆ alkyl or C₁-C₆ alkoxy, and the C₁-C₆ alkyl and the C₁-C₆ alkoxy are optionally substituted with C₁-C₆ alkoxy (e.g. methoxy).

In certain embodiments of the present invention, in S2, S2' and S2", R₁ is hydrogen, methyl, methoxy or

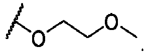

In certain embodiments of the present invention, in S2 and S2', the fragment 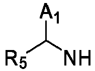 is

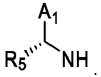

In certain embodiments of the present invention, S is -X-S2', and the structure of S2' is as described above; X is O, NH or S, preferably O.

In certain embodiments of the present invention, S is -X-S2", and the structure of S2" is as described above; X is O, NH or S, preferably O.

Preferably, in certain embodiments of the present invention, S is S2a, 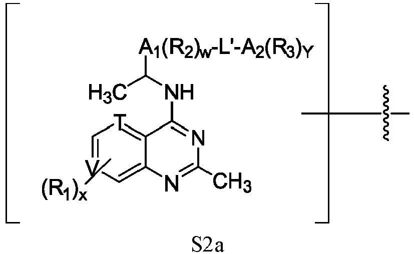 wherein in S2a,
T and V are both N, or T is C and V is N, or T is N and V is C;
R₁ is selected from hydrogen, -OCH₃, -OCH₂CH₃, 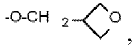 -CH₂OH, -C(O)OH, -C(O)OCH₃, -Br, - OCH(CH₃)₂, -O(CH₂)₂CH(CH₃)₂, -O(CH₂)₃CH₃, -O(CH₂)₂OCH₃, 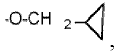 -O(CH₂),-phenyl, -N=S(O)(CH₃)₂, -CH₃, cyclopropyl, -N(CH₃)₂, -NHCH₃, -NH₂, 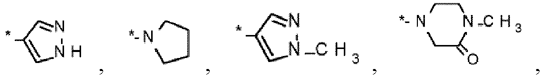 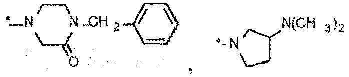 , -C(CH₃)₂-OH, 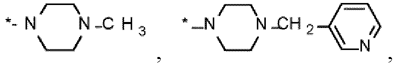 -NH(CH₂)-NH-C(O)CH₃, -NH(CH₂)-morpholine, -NH-C(O)CH₃, -NH-C(O)NHCH₃, -NH-C(O)-N(CH₃)₂, nitro, -NH-S(O)₂CH₃, -N=S(O)(CH₃)₂, hydroxy, -O-(CH₂)₂-S(O)₂CH₃, -F, 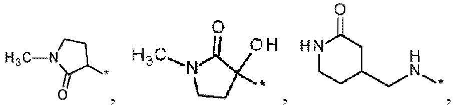 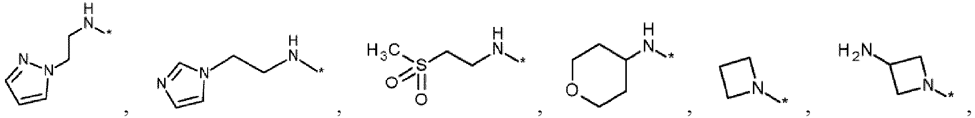 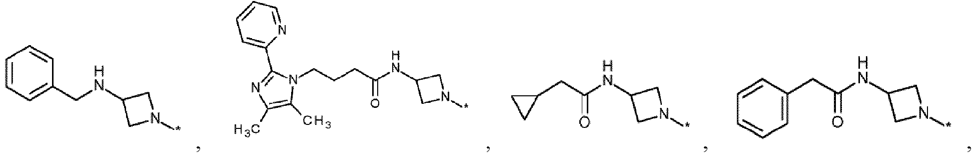 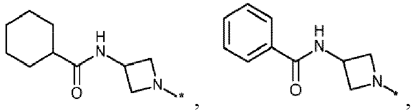 cyclopropyloxy, cyclobutyloxy, cyclopentyloxy, cyclohexyloxy, azetidinyl, azacyclopentyl, piperidinyl, piperazinyl, oxetanyl, oxolanyl, oxanyl, thietanyl, thiolanyl, thianyl, azetidinyloxy, azacyclopentyloxy, piperidinyloxy, piperazinyloxy, oxetanyloxy, oxolanyloxy, oxanyloxy, thietanyloxy, thiolanyloxy, thianyloxy, 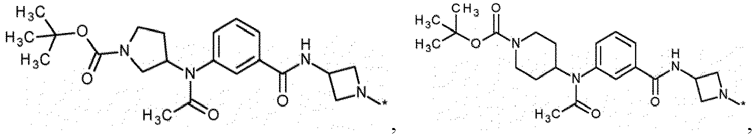 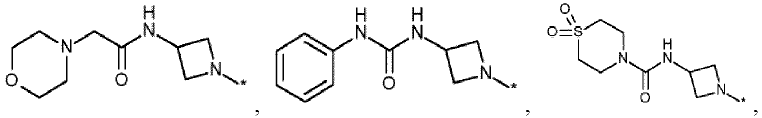 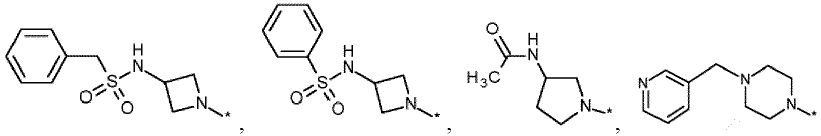 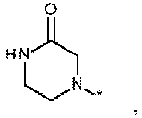 -OCH₂CH₂CH₃, -OCH₂CH₂CH₂N(CH₃)₂, -OCH₂CH₂CH₂OH, 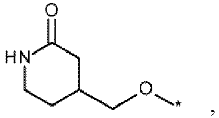 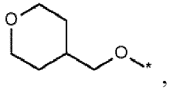 , -OCH₂CH₂NC(O)CH₃, -OCH₂CH₂N(CH₃)₂, -OCH₂CH₂OH, 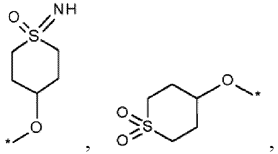 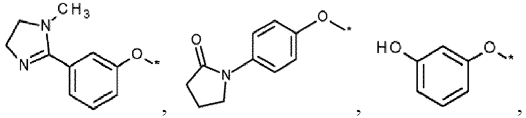 , -SCH₃, -N(CH₃)₂, 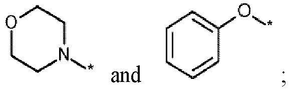
z is 1 or 2;
x is 1 or 2;
A₁ is selected from 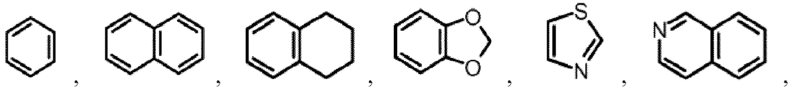 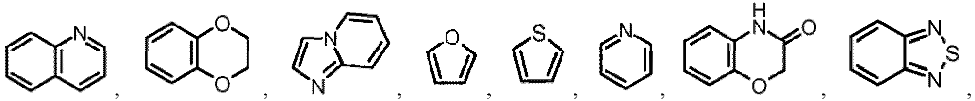 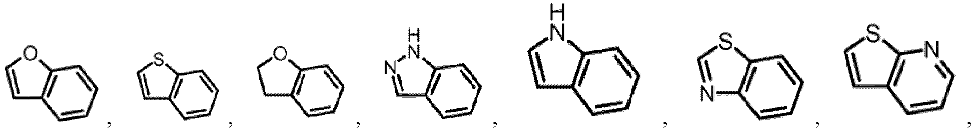 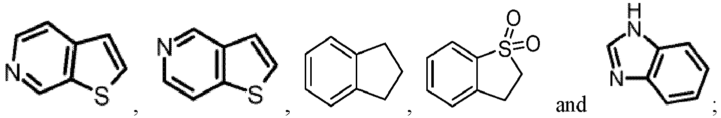
R₂ is selected from hydrogen, hydroxy, oxo, cyano, cyclopropyl, 1,1-dimethylcyclopropyl, -C(=CH₂)CH₃, - C(CH₃)(=CH₂)CH₃, -CH=CH(CH₂)₂CH₃, -CH=CHCH₃, -CH=CH-cyclopropyl, -C(O)NH₂, trifluoromethyl, methyl, trifluoromethoxy, -C(O)OCH₃, -S(O)₂CH₃, -OCH₃, -CH₂NH₂ and halogen (F, Cl or Br); w is 1, 2 or 3;
A₂ is selected from 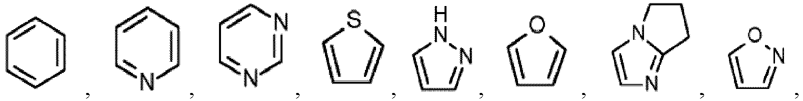 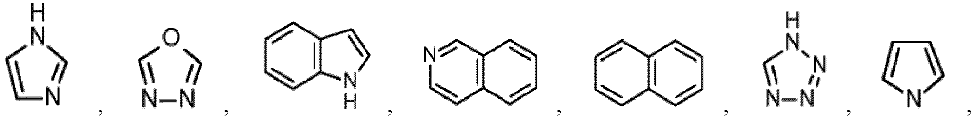 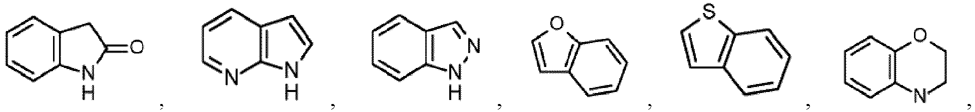 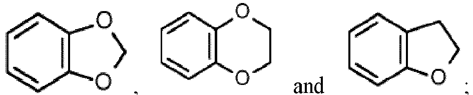
R₃ is selected from -C(O)NH(CH₂)₂CH₃, -C(O)N(CH₃)₂, -C(O)NH₂, -C(O)NH(CH₂)₂N(CH₃)₂, -CH₂C(O)NH₂, hydrogen, -F, -Cl, -Br, cyano, -CF₃, -CH₃, -CH₂CH₃, -CH=CH₂, -CH₂CN, -CH(CH₃)-NH₂, -CH=CH-CN, -C(O)-OH, -C(O)OCH₃, -C(O)CH₃, -C(CH₃)₂-C(O)-OCH₃, -C(CH₃)₂-CN, oxo, hydroxy, cyclopropyl, cyclobutyl, cyclopentyl, -NH₂, -NH-C(O)CH₃, -NH-S(O)₂CH₃, -NH-C(O)OC(CH₃)₃, 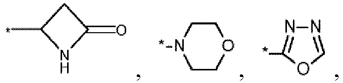 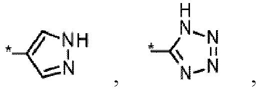 -S(O)₂CH₃, -S(O)₂NCH₃, -S(O)₂NH₂, -OCH₂CH₃, -O(CH₂)₂CH₃, -OCF₃, 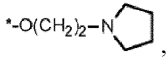 -OCH₂cyclopropyl, -OCH₃, -O(CH₂)₃CH₃, -OCH₂phenyl, -O-phenyl, -(CH₂)-OH, -(CH₂)₂-OH, -(CH₂)-OCH₃, -(CH₂)-OCH₂CH₃, -CH(OH)-CH₂-phenyl, -CH(OH)-CH₂CH₃, -CH(OH)-CH₂CH₂CH₃, -CH(OH)-CH₂CH₂CH₂CH₃, -CH(OH)-CH(CH₃)₂, -CH(OH)-phenyl, -CH(OH)-CN, -CH(OH)-CH₂-OH, -CH(OH)-CF₃, - CH(OH)-(CH₂)₂-phenyl, *-CH(OH)-C≡CH , -CH(NH₂)-CH₂-C(O)OH, -CH₂-NH-S(O)₂-CH₃, -CH₂-NH-(CH₂)₃CH₃, - CH₂-NH-CH₃, -CH₂-N(CH₃)₂, -CH₂-NH-CH₂CH₃, -CH(CH₃)-NH₂, -CH₂-NH₂, -CH₂CH₂-NH₂, -CH₂NH-CH₂-phenyl, -CH₂N(CH₂CH₃)₂, -CH₂NH-cyclopropyl, -CH₂NH-cyclobutyl, -CH₂NH-cyclopentyl, -CH₂NH-pyridyl, - CH₂NH-phenyl, -CH₂NH-(CH₂)₂-OH, -CH₂N(CH₃)-(CH₂)₂-OH, -CH₂NH-CH₂-CN, -CH₂N(CH₃)-CH₂-CN, - CH₂N(CH₃)-CH₂-CF₃, -CH₂N(CH₃)-CH₂-CF₂H, -CH₂NH-CH₂-CF₂H, -CH₂NH-(CH₂)₂-OCH₃, 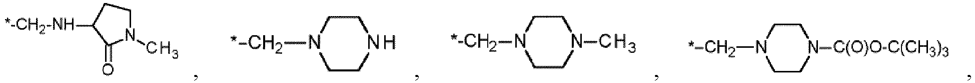 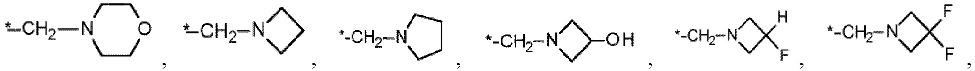 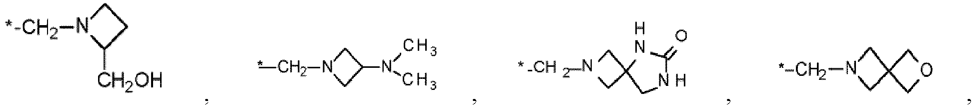 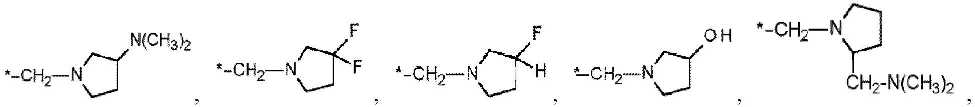 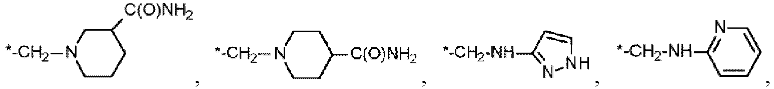 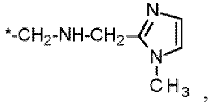 , -CH₂NH-C(O)-OC(CH₃)₃, -CH₂CH₂NH-C(O)-OC(CH₃)₃, -CH₂NH-C(O)-CH₂NOH, - CH₂NH-C(O)-CH₂OCH₃, -CH(CH₃)NH-C(O)-OC(CH₃)₃, -CH₂NH-C(O)-CH₃, 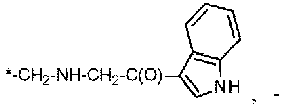 CH₂NHCH₂-C(O)-NH₂, -CH₂NHCH₂-C(O)-(CH₃)₂, -CH₂NHCH₂-C(O)-OCH₃, -CH₂NHCH₂-C(O)-NHCH₃, - CH₂NHCH₂-C(O)-NH(CH₂)₂-OCH₃, -CH₂NHCH₂-C(O)-NHCH₂-phenyl, 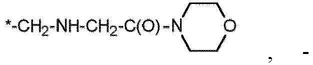 CH₂NHCH₂-C(O)-NH-phenyl, 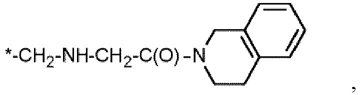 -CH₂NH-C(O)-CH₂NH-phenyl, 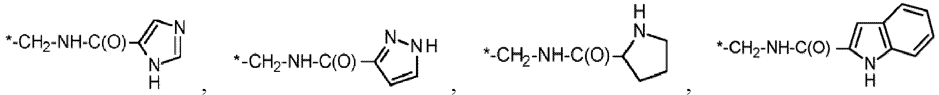 and - CH₂NH-C(O)-CH₂NH-CH₂CF₃;
Y is 1 or 2;
L' is a bond, -(CH₂)ₖ- or -O(CH₂)ₖ-, wherein k is 1 or 2, or L' is -CH=CH-(CH₂)ₙ, wherein n is 0 or 1.

More preferably, in certain embodiments of the present invention, S2 is the specific compound according to Example 1 to Example 100 in WO2019201848A1 (incorporated herein by reference in its entirety).

In certain embodiments of the present invention, in S2a, w is 1 or 2.

In certain embodiments of the present invention, in S2a, R₁ is hydrogen, methyl, -OCH₃, -OCH₂CH₃, -OCH(CH₃)₂, -O(CH₂)₂CH(CH₃)₂ or -O(CH₂)₃CH₃, e.g., -OCH₃.

In certain embodiments of the present invention, in S2a, A₁ is

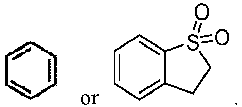

In certain embodiments of the present invention, in S2a, A₁ is

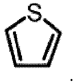

In certain embodiments of the present invention, in S2a, R₂ is hydrogen, halogen, -NH₂, C₁-C₆ alkyl or -C₁-C₆ haloalkyl.

In certain embodiments of the present invention, in S2a, L' is a bond.

In certain embodiments of the present invention, in S2a, A₂(R₃)_{Y} is hydrogen.

In certain embodiments of the present invention, in S2a, A₂ is

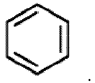

In certain embodiments of the present invention, in S2a, R₃ is hydrogen, -F, -Cl, -Br, -CH₂-NH-(CH₂)₃CH₃, -CH₂-NH-CH₃, -CH₂-N(CH₃)₂ or -CH₂-NH-CH₂CH₃.

In certain embodiments of the present invention, in S2a, R₃ is hydrogen, Cl, -CH₂NHCH₃ or -CH₂N(CH₃)₂.

In certain embodiments of the present invention, in S2a, A₁ is 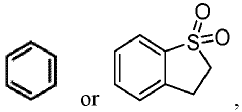 L' is a bond, and A₂(R₃)_{Y} is hydrogen.

In certain embodiments of the present invention, in S2a, A₁ is 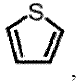 L' is a bond, A₂ is 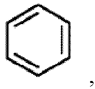 and R₃ is hydrogen, -F, -Cl, -Br, -CH₂-NH-(CH₂)₃CH₃, -CH₂-NH-CH₃, -CH₂-N(CH₃)₂ or -CH₂-NH-CH₂CH₃. In certain embodiments of the present invention, S is S2a', 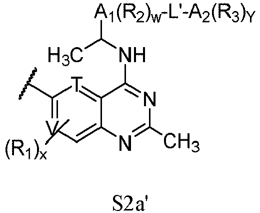 wherein in S2a', T, V, R₁, R₂, R₃, L', A₁, A₂, w, x and Y are as defined in S2 or S2a.

In certain embodiments of the present invention, S is S2a", 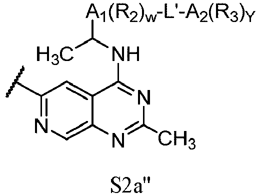 wherein in S2a", T, V, R₂, R₃, L', A₁, A₂, w and Y are as defined in S2 or S2a.

In certain embodiments of the present invention, in S2a' and S2a", R₁ is hydrogen, C₁-C₆ alkyl or C₁-C₆ alkoxy, and the C₁-C₆ alkyl and the C₁-C₆ alkoxy are optionally substituted with C₁-C₆ alkoxy (e.g. methoxy).

In certain embodiments of the present invention, in S2a' and S2a", R₁ is hydrogen, methyl, methoxy or

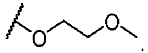

In certain embodiments of the present invention, in S2a, S2a' and S2a", the fragment 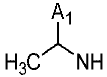 is

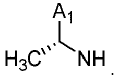

In certain embodiments of the present invention, S is -X-S2a', and the structure of S2a' is as described above; X is O, NH or S, preferably O.

In certain embodiments of the present invention, S is -X-S2a", and the structure of S2a" is as described above; X is O, NH or S, preferably O.

Preferably, in certain embodiments of the present invention, S is S3: 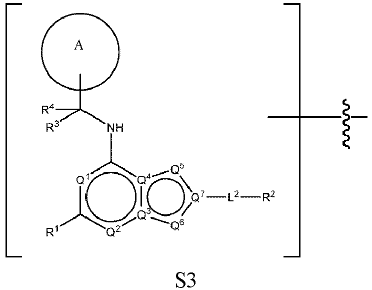 wherein in S3,
Q¹ and Q² are each independently CH or N;
Q³, Q⁴ and Q⁷ are each independently C or N, at least one of Q³ and Q⁴ is C, and not all of Q³, Q⁴ and Q⁷ are N;
Q⁵ is CH, N, NH, O or S;
Q⁶ is CH, N, NH, N(C₁-C₆ alkyl), N(C₁-C₆ heteroalkyl), N(3- to 7-membered cycloalkyl), N(3- to 7-membered heterocycle), O or S;
at least one of Q¹, Q², Q³, Q⁴, Q⁵, Q⁶ and Q⁷ is N, NH, O or S;
R¹ is selected from hydrogen, C₁-C₆ alkyl, C₁-C₆ alkoxy, halogen, -NHR₁ₐ, -OR₁ₐ, C₃-C₆ cycloalkyl, C₃-C₆ cycloalkoxy and -CN; the C₁-C₆ alkyl, the C₁-C₆ alkoxy, the C₃-C₆ cycloalkyl and the C₃-C₆ cycloalkoxy are optionally substituted with a substituent selected from halogen, -NHR^{1a} and -OR^{1a}; R^{1a} is hydrogen, C₁-C₆ alkyl, 3-to 6-membered heterocycle or C₁-C₆ haloalkyl;
L² is selected from a bond, -C(O)-, -C(O)O-, -C(O)NH(CH₂)ₒ-, -S(O)₂-, -S(O)-, -S(=O)(=NH)-, -S(=O)[=N(C₁-C₆ alkyl)]-, -N(C₁-C₆ alkyl)-, -C(O)(CH₂)ₚ-, -(CH₂)ₚ- and O; o is 0, 1 or 2; p is an integer of 1 to 6;
R² is selected from hydrogen, halogen, -CN, -NO₂, C₁-C₆ alkyl sulfhydryl, C₂-C₆ alkynyl, C₁-C₆ alkyl, C₂-C₆ alkenyl, -NR^{2b}R^{2c}, -OR^{2a}, 3- to 14-membered cycloalkyl, 3- to 14-membered cycloalkenyl, 3- to 14-membered heterocycloalkyl, 6- to 10-membered aryl and 5- to 10-membered heteroaryl; the C₁-C₆ alkyl, the C₂-C₆ alkenyl, the 3- to 14-membered cycloalkyl, the 3- to 14-membered cycloalkenyl, the 3- to 14-membered heterocycloalkyl, the 6- to 10-membered aryl, and the 5- to 10-membered heteroaryl are each independently optionally substituted with a substituent selected from C₁-C₆ alkyl, C₁-C₆ haloalkyl, -OH, -OR^{2a}, oxo, halogen, -C(O)R^{2a}, -C(O)OR^{2a}, - C(O)NR^{2b}R^{2c}, -CN, -NR^{2b}R^{2c}, 3- to 6-membered cycloalkyl, 3- to 7-membered heterocycloalkyl, 6- to 10-membered aryl and 5- to 10-membered heteroaryl; R^{2a} is hydrogen, C₁-C₆ alkyl, C₁-C₆ haloalkyl, 3- to 7-membered heterocycloalkyl, or -(CH₂)ᵣOCH₃, and r is 1, 2 or 3;
R^{2b} is hydrogen or C₁-C₆ alkyl;
R^{2c} is hydrogen or C₁-C₆ alkyl;
R² is -NHC(O)-(C₁-C₆ alkyl) or -NHC(O)-NRₐR_{b}, wherein Rₐ and R_{b} are each independently hydrogen, C₁-C₆ alkyl, C₃-C₈ heterocycloalkyl or C₃-C₈ cycloalkyl (e.g., Rₐ and R_{b} are each independently hydrogen or C₁-C₆ alkyl);
R² is -NH-(CH₂)ₖ-NH-C(O)-(C₁-C₆ alkyl), wherein k is 1 or 2;
R² is -NH-(CH₂)ᵢ-R_{f}, wherein i is 0, 1 or 2 and R_{f} is 4- to 7-membered heterocycloalkyl, heteroaryl or C₁-C₆ alkylsulfonyl;
R³ and R⁴ are each independently hydrogen, C₁-C₆ alkyl, C₁-C₆ alkoxy or C₃-C₆ cycloalkoxy; at least one of R³ and R⁴ is not hydrogen, or R³ and R⁴, together with the atom linked thereto, form 3- to 6-membered cycloalkyl; the alkyl, the alkoxy, the cycloalkyl and the cycloalkoxy are optionally substituted with cyano, hydroxy, C₁-C₆ alkoxy, C₃-C₆ cycloalkyl, C₃-C₆ cycloalkoxy or halogen;
A is optionally substituted (e.g., optionally substituted with 1 or more R₂, and R₂ is as defined in S1) 6-membered aryl or optionally substituted (e.g., optionally substituted with 1 or more R₂, and R₂ is as defined in S1) 5- to 6-membered heteroaryl.

Preferably, in certain embodiments of the present invention, S is S3a, 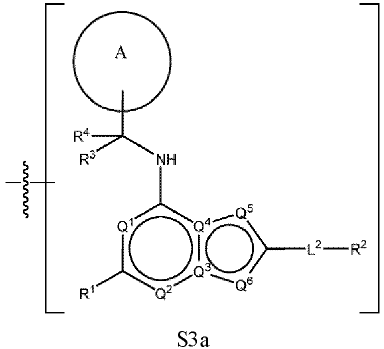 wherein in S3a,
Q¹ and Q² are each independently CH or N;
Q³ and Q⁴ are each independently C or N, and at least one of Q³ and Q⁴ is C;
Q⁶ is CH, N, NH, O or S;
Q⁵ is CH, N, NH, O or S;
at least one of Q¹, Q², Q³, Q⁴, Q⁵ and Q⁶ is N, NH, O or S;
R¹ is selected from hydrogen, C₁-C₆ alkyl, halogen, -OR^{1a}, cyclopropyl and -CN; R^{1a} is hydrogen or C₁-C₆ alkyl;
L² is selected from a bond, -C(O)-, -C(O)O-, -C(O)NH(CH₂)ₒ-, -S(O)₂-, -C(O)(CH₂)ₚ-, -(CH₂)ₚ- and O; o is 0, 1 or 2; p is an integer of 1 to 6;
R² is selected from hydrogen, -(CH₂)_{q}CH₃, 3- to 14-membered cycloalkyl, 3- to 14-membered cycloalkenyl, 3- to 14-membered heterocycloalkyl, 6- to 10-membered aryl and 5- to 10-membered heteroaryl; q is an integer of 1 to 5; the 3- to 14-membered cycloalkyl, the 3- to 14-membered cycloalkenyl, the 3- to 14-membered heterocycloalkyl, the 6- to 10-membered aryl and the 5- to 10-membered heteroaryl are each independently optionally substituted with a substituent selected from C₁-C₆ alkyl, -OH, halogen, -C(O)R^{2a} and -C(O)NR^{2b}R^{2c}; R^{2a} is C₁-C₆ alkyl or - (CH₂)ᵣOCH₃, and r is 1, 2 or 3; R^{2b} is hydrogen or C₁-C₆ alkyl; R^{2c} is hydrogen or C₁-C₆ alkyl;
R₃ and R⁴ are each independently hydrogen or C₁-C₆ alkyl, and at least one of R³ and R⁴ is not hydrogen, or R³ and R⁴, together with the atom linked thereto, form 3- to 6-membered cycloalkyl;
A is optionally substituted (e.g., optionally substituted with 1 or more R₂, and R₂ is as defined in S1) phenyl or optionally substituted (e.g., optionally substituted with 1 or more R₂, and R₂ is as defined in S1) 5- to 6-membered heteroaryl.

More preferably, in certain embodiments of the present invention, S3 is the specific compound according to Example 1 to Example 103 in WO2020180768A1 (incorporated herein by reference in its entirety).

Preferably, in certain embodiments of the present invention, S is S4: 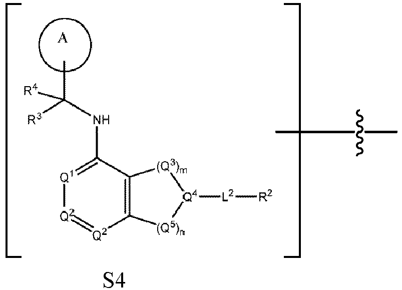 wherein in S4,
Q¹ is CH or N;
Q⁴ is CH, C or N;
each Q² is independently C-R¹ or N, and one of Q² is N and the other Q² is C-R¹;
each Q³ and Q⁵ molecule is independently C(R^{QC})₂, NR^{QN}, C(O), O, S or SO₂, R^{QC} is each independently hydrogen, F, Cl, Br or 6- to 10-membered aryl, and R^{QN} is each independently hydrogen, C₁-C₆ alkyl or 6- to 10-membered aryl;
at least one of Q¹, Q², Q³, Q⁴ and Q⁵ is N, NR^{QN}, O or SO₂;
m is 0, 1, 2 or 3;
n is 0, 1, 2 or 3;
and when m is 0, n is not 0;
R¹ is selected from hydrogen, C₁-C₆ alkyl, halogen, -C(O)NHR^{1a}, -NHR^{1a}, -OR^{1a}, cyclopropyl, azetidine and -CN; the C₁-C₆ alkyl and the azetidine are optionally substituted with a substituent selected from halogen, R^{1a}, -NHR^{1a} and -OR^{1a}; R^{1a} is hydrogen, C₁-C₆ alkyl, cyclopropyl, 3- to 6-membered heterocycle or C₁-C₆ haloalkyl;
L² is selected from a bond, -C(O)-, -C(O)O-, -C(O)NH(CH₂)ₒ-, -S(O)₂-, -S(O)-, -S(=O)(=NH)-, -S(=O)[=N(C₁-C₆ alkyl)]-, -N(C₁-C₆ alkyl)-, -C(O)(CH₂)ₚ-, -(CH₂)ₚ- and O; o is 0, 1 or 2; p is an integer of 1 to 6;
R² is selected from hydrogen, C₁-C₆ alkyl, -NR^{2b}R^{2c}, -OR^{2a}, 3- to 14-membered cycloalkyl, 3- to 14-membered cycloalkenyl, 3- to 14-membered heterocycloalkyl, 6- to 10-membered aryl and 5- to 10-membered heteroaryl; the C₁-C₆ alkyl, the 3- to 14-membered cycloalkyl, the 3- to 14-membered cycloalkenyl, the 3- to 14-membered heterocycloalkyl, the 6- to 10-membered aryl and the 5- to 10-membered heteroaryl are each independently optionally substituted with a substituent selected from C₁-C₆ alkyl, C₁-C₆ haloalkyl, C₁-C₆ hydroxyalkyl, C₁-C₆ methoxyalkyl, -OH, -OR^{2a}, oxo, halogen, =N, -C(O)R^{2a}, -C(O)OR^{2a}, -C(O)NR^{2b}R^{2c}, -S(O)₂R^{2a}, -CN, -NR^{2b}R^{2c}, 3-to 6-membered cycloalkyl, 3- to 7-membered heterocycloalkyl, 6- to 10-membered aryl and 5- to 10-membered heteroaryl; R^{2a} is hydrogen, C₁-C₆ alkyl, C₁-C₆ haloalkyl, 3- to 7-membered heterocycloalkyl, or -(CH₂)ᵣOCH₃, and R is 1, 2 or 3;
R^{2b} is hydrogen or C₁-C₆ alkyl;
R^{2c} is hydrogen or C₁-C₆ alkyl;
R³ and R⁴ are each independently hydrogen or C₁-C₆ alkyl optionally substituted with halogen or -OH, and at least one of R³ and R⁴ is hydrogen, or R³ and R⁴, together with the atom linked thereto, form 3- to 6-membered cycloalkyl; A is optionally substituted (e.g., optionally substituted with 1 or more R₂, and R₂ is as defined in S1) 6-membered aryl or optionally substituted (e.g., optionally substituted with 1 or more R₂, and R₂ is as defined in S1) 5- to 6-membered heteroaryl;
provided that when 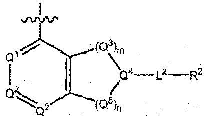 is 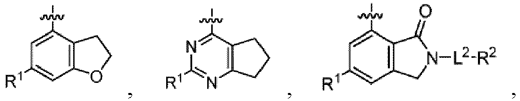 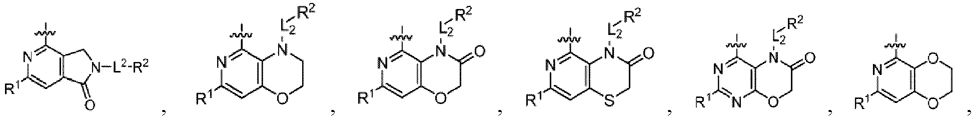 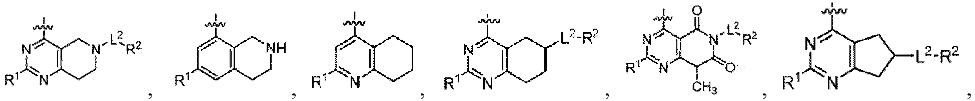 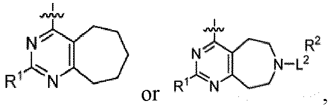 R¹ is not hydrogen.

Preferably, in certain embodiments of the present invention, S is S4a 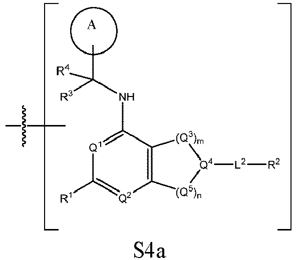 wherein in S4a,
Q¹ is CH or N;
Q⁴ is CH, C or N;
each Q² is independently CR¹ or N;
each Q³ and Q⁵ molecule is independently C(R^{QC})₂, NR^{QN}, C(O), O, S or SO₂, R^{QC} is each independently hydrogen, F, Cl, Br or 6- to 10-membered aryl, and R^{QN} is each independently hydrogen, C₁-C₆ alkyl or 6- to 10-membered aryl;
at least one of Q¹, Q², Q³, Q⁴ and Q⁵ is N, NR^{QN}, O or SO₂;
R¹ is selected from hydrogen, C₁-C₆ alkyl, halogen, cyclopropyl, cyano and -OR^{1a}, and R^{1a} is hydrogen or C₁-C₆ alkyl;
L² is selected from a bond, -C(O)-, -C(O)O-, -C(O)NH(CH₂)ₒ-, -S(O)₂-, -C(O)(CH₂)ₚ-, -(CH₂)ₚ- and O; o is 0, 1 or 2; p is an integer of 1 to 6;
R² is selected from hydrogen, -(CH₂)_{q}CH₃, 3- to 14-membered cycloalkyl, 3- to 14-membered cycloalkenyl, 3- to 14-membered heterocycloalkyl, 6- to 10-membered aryl and 5- to 10-membered heteroaryl; q is a multiple of 1 to 5; the 3- to 14-membered cycloalkyl, the 3- to 14-membered cycloalkenyl, the 3- to 14-membered heterocycloalkyl, the 6- to 10-membered aryl and the 5- to 10-membered heteroaryl are each independently optionally substituted with a group selected from C₁-C₆ alkyl, hydroxy, halogen, -C(O)R^{2a} and -C(O)NR^{2b}R^{2c}; R^{2a} is selected from C₁-C₆ alkyl and -(CH₂)ᵣOCH₃, and r is 1, 2 or 3; R^{2b} and R^{2c} are each independently hydrogen or C₁-C₆ alkyl;
R³ and R⁴ are each independently hydrogen or C₁-C₆ alkyl, and at least one of R³ and R⁴ is hydrogen, or R³ and R⁴, together with the atom linked thereto, form 3- to 6-membered cycloalkyl;
A is optionally substituted (e.g., optionally substituted with 1 or more R₂, and R₂ is as defined in S1) 6-membered aryl or optionally substituted (e.g., optionally substituted with 1 or more R₂, and R₂ is as defined in S1) 5- to 6-membered heteroaryl.

Preferably, in certain embodiments of the present invention, in S4a, R³ and R⁴ are each independently hydrogen or methyl, and at least one of R³ and R⁴ is hydrogen.

More preferably, in certain embodiments of the present invention, S4 is the specific compound according to Example 1 to Example 540 in WO2020180770A1 (incorporated herein by reference in its entirety).

Preferably, in certain embodiments of the present invention, S is S5: 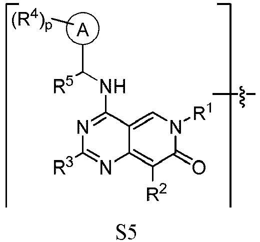 wherein in S5,
R¹ is hydrogen or R^{a1};
R^{a1} is selected from C₁-C₆ alkyl, C₁-C₆ haloalkyl, C₂-C₆ alkenyl, C₁-C₆ alkynyl, C₃-C₁₀ cycloalkyl, C₄-C₁₀ cycloalkenyl, 3- to 10-membered heterocycloalkyl, C₆-C₁₀ aryl and 5- to 10-membered heteroaryl, and the C₁-C₆ alkyl, the C₁-C₆ haloalkyl, the C₂-C₆ alkenyl, the C₁-C₆ alkynyl, the C₃-C₁₀ cycloalkyl, the C₄-C₁₀ cycloalkenyl, the 3- to 10-membered heterocycloalkyl, the C₆-C₁₀ aryl and the 5- to 10-membered heteroaryl are optionally substituted with 1 or more identical or different R^{b1} and/or R^{c1};
each R^{b1} is independently -OR^{c1}, -NR^{c1}R^{c1}, halogen, -CN, -C(O)R^{c1}, -C(O)OR^{c1}, -C(O)NR^{c1}R^{c1}, -S(O)₂R^{c1}, - S(O)₂NR^{c1}R^{c1}, -NHC(O)R^{c1}, -N(C₁-C₄ alkyl)C(O)R^{c1}, -NHC(O)OR^{c1} or -N(C₁-C₄ alkyl)C(O)OR^{c1};
each R^{c1} is independently C₁-C₆ alkyl, C₁-C₆ haloalkyl, C₂-C₆ alkenyl, C₂-C₆ alkynyl, C₃-C₁₀ cycloalkyl, C₄-C₁₀ cycloalkenyl, 3- to 10-membered heterocycloalkyl, C₆-C₁₀ aryl and 5- to 10-membered heteroaryl, and the C₁-C₆ alkyl, the C₁-C₆ haloalkyl, the C₂-C₆ alkenyl, the C₂-C₆ alkynyl, the C₃-C₁₀ cycloalkyl, the C₄-C₁₀ cycloalkenyl, the 3- to 10-membered heterocycloalkyl, the C₆-C₁₀ aryl and the 5- to 10-membered heteroaryl are each independently optionally substituted with 1 or more identical or different R^{d1} and/or R^{e1};
each R^{d1} is independently -OR^{e1}, -NR^{e1}R^{e1}, halogen, -CN, -C(O)R^{e1}, -C(O)OR^{e1}, -C(O)NR^{e1}R^{e1}, -S(O)₂R^{e1}, - S(O)₂NR^{e1}R^{e1}, -NHC(O)R^{e1}, -N(C₁-C₄ alkyl)C(O)R^{e1}, -NHC(O)OR^{e1} or -N(C₁-C₄ alkyl)C(O)OR^{e1},
each R^{e1} is independently hydrogen, C₁-C₆ alkyl, C₁-C₆ haloalkyl, C₂-C₆ alkenyl, C₂-C₆ alkynyl, C₃-C₁₀ cycloalkyl, C₄-C₁₀ cycloalkenyl, 3- to 10-membered heterocycloalkyl, C₆-C₁₀ aryl or 5- to 10-membered heteroaryl;
R² is selected from hydrogen, C₁-C₄ alkyl, C₃-C₆ cycloalkyl, 3- to 6-membered heterocycloalkyl and halogen; ring A is selected from C₆-C₁₀ aryl, 5- to 10-membered heteroaryl and 9- to 10-membered biheterocycloalkyl;
p is 1, 2 or 3;
each R⁴ is independently hydrogen, hydroxy, oxo, halogen, cyano, C₁-C₄ alkyl, -NH₂, C₁-C₄ haloalkyl, C₂-C₄ alkenyl, C₂-C₄ alkynyl, C₁-C₄ hydroxyalkyl, hydroxy-C₁-C₄ haloalkyl, C₃-C₆ cycloalkyl, C₃-C₆ cycloalkoxy, 3- to 6-membered heterocycloalkyl, C₃-C₆ hydroxycycloalkyl, C₁-C₄ haloalkyl substituted with 3- to 6-membered heterocycloalkyl or 3- to 6-membered heterocycloalkyl substituted with hydroxy, halogen, -NH₂, -S(O)₂-(C₁-C₄ alkyl) or oxo, and the substitution with the oxo is performed only on a non-aromatic ring;
R⁴ is -NRₐR_{b}, wherein Rₐ and R_{b} are each independently hydrogen, C₁-C₆ alkyl, C₃-C₈ heterocycloalkyl or C₃-C₈ cycloalkyl;
R⁴ is -C(O)NRₐR_{b}, wherein Rₐ and R_{b} are each independently hydrogen, C₁-C₆ alkyl, C₃-C₈ heterocycloalkyl or C₃-C₈ cycloalkyl;
R⁴ is -C(O)OR_{g}, wherein R_{g} is hydrogen or C₁-C₆ alkyl;
R⁴ is -ORₕ; Rₕ is C₁-C₆ alkyl;
or R⁴ is -(CH₂)NRₐR_{b}, wherein Rₐ and R_{b} are each independently hydrogen, C₁-C₆ alkyl, C₃-C₈ heterocycloalkyl or C₃-C₈ cycloalkyl;
R³ and R⁵ are each independently hydrogen, C₁-C₆ alkyl, C₁-C₆ alkoxy, C₃-C₆ cycloalkyl or C₃-C₆ cycloalkoxy, wherein the alkyl, the alkoxy, the cycloalkyl and the cycloalkoxy are optionally substituted with cyano, hydroxy, C₁-C₆ alkoxy, C₃-C₆ cycloalkyl, C₃-C₆ cycloalkoxy or halogen.

Preferably, in certain embodiments of the present invention, in S5, R⁵ is methyl.

In some embodiments, in the definition of each group in S5, Rₐ and R_{b}, when occurring at any position, are each independently hydrogen or C₁-C₆ alkyl.

In certain embodiments of the present invention, S is S5': 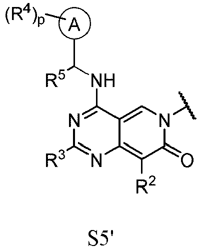 wherein in S5', R², R³, R⁴, R⁵, ring A and p are as defined and described in S5.

In certain embodiments of the present invention, S is -X-S5', and the structure of S5' is as described above; X is O, NH or S, preferably O.

Preferably, in certain embodiments of the present invention, S is S5a: 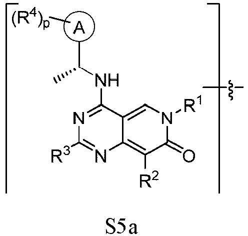 wherein R¹, R², R³, R⁴, ring A and p are as defined and described in S5.

In certain embodiments of the present invention, S is S5a': 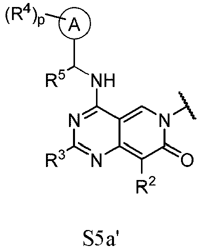 wherein in S5a', R², R³, R⁴, R⁵, ring A and p are as defined and described in S5a.

In certain embodiments of the present invention, S is -X-S5a', and the structure of S5a' is as described above; X is O, NH or S, preferably O.

More preferably, in certain embodiments of the present invention, S5 is the specific compound according to I-1 to I-179 in WO2019122129A1 (incorporated herein by reference in its entirety).

Preferably, in certain embodiments of the present invention, S is S6: 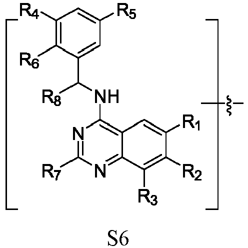 wherein in S6,
R₂ is selected from hydrogen, halogen, -OH, -CN, -NO₂, C₁-C₆ alkyl sulfhydryl and -NRₐR_{b}, wherein Rₐ and R_{b} are each independently hydrogen, C₁-C₆ alkyl, C₃-C₈ heterocycloalkyl or C₃-C₈ cycloalkyl;
R₂ is selected from C₁-C₆ alkyl, C₁-C₆ alkoxy, C₃-C₆ cycloalkoxy, C₃-C₆ heterocycloalkoxy (e.g., 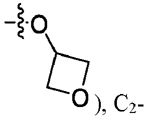 C₆ alkenyl, C₂-C₆ alkynyl, C₃-C₈ cycloalkyl, C₄-C₈ cycloalkenyl, 4- to 7-membered heterocycloalkyl (e.g., 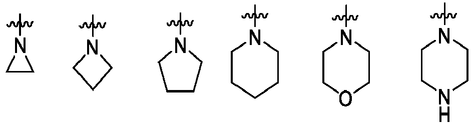 ), 5- to 10-membered heterocycloalkenyl, spiroheterocycloalkyl, fused heterocycloalkyl, bridged heterocycloalkyl, phenyl, heteroaryl, C₁-C₆ haloalkyl, - COOH and -COOR_{c}; R_{c} is -C₁-C₆ alkyl, C₃-C₆ alkenyl, C₃-C₆ alkynyl, C₃-C₈ cycloalkyl or -C₄-C₈ cycloalkenyl;
R₂ is -NS(O)(R_{d})(Rₑ), wherein R_{d} and Rₑ are each independently C₁-C₆ alkyl, C₂-C₆ alkenyl, C₂-C₆ alkynyl, C₃-C₈ cycloalkyl, C₄-C₈ cycloalkenyl, 6- to 10-membered aryl or 5- to 10-membered heteroaryl;
R₂ is -NHC(O)-(C₁-C₆ alkyl) or -NHC(O)-NRₐR_{b}, wherein Rₐ and R_{b} are each independently hydrogen, -C₁-C₆ alkyl, C₃-C₈ heterocycloalkyl or C₃-C₈ cycloalkyl;
R₂ is -NH-(CH₂)ₖ-NH-C(O)-Rₐₐ, wherein Rₐₐ is C₁-C₆ alkyl, C₃-C₈ heterocycloalkyl or C₃-C₈ cycloalkyl, wherein k is 1 or 2;
R² is -NH-(CH₂)ᵢ-R_{f}, wherein i is 0, 1 or 2 and R_{f} is 4- to 7-membered heterocycloalkyl, heteroaryl or C₁-C₆ alkylsulfonyl;
furthermore, C₁-C₆ alkyl, C₁-C₆ alkoxy, 4- to 7-membered heterocycloalkyl and heteroaryl described above are optionally substituted with 1, 2 or 3 groups selected from halogen, -OH, oxo, -CN, -NO₂, C₁-C₆ alkyl, C₂-C₆ alkenyl, C₂-C₆ alkynyl, C₃-C₈ cycloalkyl, 4- to 7-membered heterocycloalkyl, C₁-C₆ alkoxy, C₁-C₆ haloalkyl, C₁-C₆ haloalkoxy, C₁-C₆ alkylsulfonyl, phenyl, benzyl, heteroaryl, -(CH₂)-heteroaryl, -NHC(O)(C₁-C₆ alkyl), C₃-C₈ cycloalkoxy, phenoxy, heteroaryloxy and -NRₐR_{b}; Rₐ and R_{b} are each independently selected from hydrogen, C₁-C₆ alkyl, C₃-C₈ heterocycloalkyl and C₃-C₈ cycloalkyl;
R₂ is -O-(CH₂)_{z}-phenyl, -O-(CH₂)_{z}-(4- to 7-membered heterocycloalkyl) or -O-(CH₂)_{z}-heteroaryl, wherein z is 0, 1 or 2, and the phenyl, the heterocycloalkyl and the heteroaryl are optionally substituted with a group selected from - OH, heterocycloalkyl and heterocycloalkenyl and can be substituted with methyl or oxo;
or R₂ is 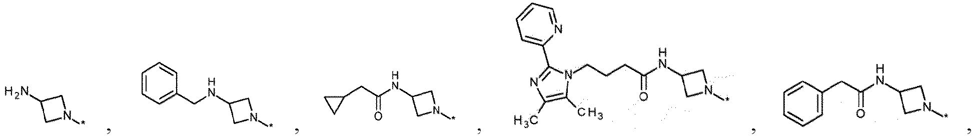 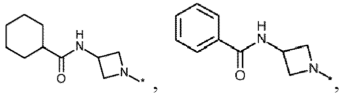 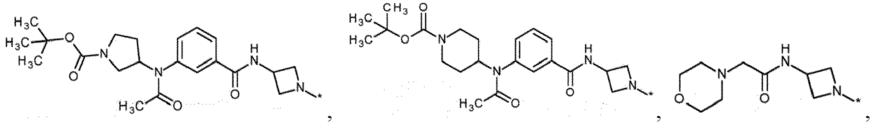 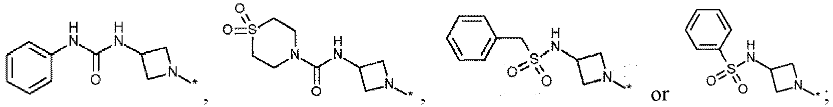
R₁ is hydrogen or -OR^{A};
R^{A} is hydrogen, C₃-C₁₀ cycloalkyl or 3- to 10-membered heterocycloalkyl, and the C₃-C₁₀ cycloalkyl and the 3- to 10-membered heterocycloalkyl are optionally substituted with 1 or more identical or different R^{a1} and/or R^{c1};
each R^{a1} is independently C₁-C₆ alkyl, C₂-C₆ alkenyl, C₂-C₆ alkynyl, C₃-C₁₀ cycloalkyl, C₆-C₁₀ aryl, 3- to 10-membered heterocycloalkyl or 5- to 10-membered heteroaryl, and the C₁-C₆ alkyl, the C₂-C₆ alkenyl, the C₂-C₆ alkynyl, the C₃-C₁₀ cycloalkyl, the C₆-C₁₀ aryl, the 3- to 10-membered heterocycloalkyl or the 5- to 10-membered heteroaryl is each independently optionally substituted with 1 or more identical or different R^{b1} and/or R^{c1},
each R^{b1} is independently substituted with -OR^{c1}, -NR^{c1}R^{c1}, halogen, -CN, -C(O)R^{c1}, -C(O)OR^{c1}, -C(O)NR^{c1}R^{c1}, - S(O)₂R^{c1}, -S(O)₂NR^{c1}R^{c1}, -NHC(O)R^{c1}, -N(C₁-C₄ alkyl)C(O)R^{c1} or oxo, and the substitution with the oxo is performed only on a non-aromatic ring;
each R^{c1} is independently hydrogen, C₁-C₆ alkyl, C₂-C₆ alkenyl, C₂-C₆ alkynyl, C₃-C₁₀ cycloalkyl, C₆-C₁₀ aryl, 3-to 10-membered heterocycloalkyl or 5- to 10-membered heteroaryl;
or R₁ is selected from C₃-C₁₀ cycloalkyl, C₃-C₁₀ cycloalkenyl, C₆-C₁₀ aryl, 3- to 10-membered heterocycloalkyl and 5- to 10-membered heteroaryl, and the C₃-C₁₀ cycloalkyl, the C₃-C₁₀ cycloalkenyl, the C₆-C₁₀ aryl, the 3- to 10-membered heterocycloalkyl or the 5- to 10-membered heteroaryl is optionally substituted with 1 or more R^{a2} and/or R^{b2};
each R^{a2} is independently selected from C₁-C₆ alkyl, C₂-C₆ alkenyl, C₂-C₆ alkynyl, C₃-C₁₀ cycloalkyl, C₆-C₁₀ aryl, 3- to 10-membered heterocycloalkyl and 5- to 10-membered heteroaryl, and the C₁-C₆ alkyl, the C₂-C₆ alkenyl, the C₂-C₆ alkynyl, the C₃-C₁₀ cycloalkyl, the C₆-C₁₀ aryl, the 3- to 10-membered heterocycloalkyl or the 5- to 10-membered heteroaryl is optionally substituted with 1 or more R^{c2} and/or R^{b2},
each R^{b2} is independently selected from -OR^{c2}, -NR^{c2}R^{c2}, halogen, -CN, -C(O)R^{c2}, -C(O)OR^{c2}, -C(O)NR^{c2}R^{c2}, - OC(O)R^{c2}, -S(O)₂R^{c2}, -S(O)₂NR^{c2}R^{c2}, -NHC(O)R^{c2}, -N(C₁-C₄ alkyl)C(O)R^{c2}, -NHC(O)OR^{c2}, oxo and =NH, and the substitution with the oxo or the =NH is performed only on a non-aromatic ring;
each R^{c2} is independently selected from hydrogen, C₁-C₆ alkyl, C₂-C₆ alkenyl, C₂-C₆ alkynyl, C₃-C₁₀ cycloalkyl, C₆-C₁₀ aryl, 3- to 10-membered heterocycloalkyl and 5- to 10-membered heteroaryl, and the C₁-C₆ alkyl, the C₂-C₆ alkenyl, the C₂-C₆ alkynyl, the C₃-C₁₀ cycloalkyl, the C₆-C₁₀ aryl, the 3- to 10-membered heterocycloalkyl or the 5-to 10-membered heteroaryl is optionally substituted with 1 or more R^{d2} and/or R^{e2};
each R^{d2} is independently -OR^{e2}, -NR^{e2}R^{e2}, halogen, -CN, -C(O)R^{e2}, -C(O)OR^{e2}, -C(O)NR^{e2}R^{e2}, -S(O)₂R^{e2}, - S(O)₂NR^{e2}R^{e2}, -NHC(O)R^{e2}, -N(C₁-C₄ alkyl)C(O)R^{e2} or oxo, and the substitution with the oxo is performed only on a non-aromatic ring;
each R^{e2} is independently selected from hydrogen, C₁-C₆ alkyl, C₂-C₆ alkenyl, C₂-C₆ alkynyl, C₃-C₁₀ cycloalkyl, C₆-C₁₀ aryl, 3- to 10-membered heterocycloalkyl and 5- to 10-membered heteroaryl, and the C₁-C₆ alkyl, the C₂-C₆ alkenyl, the C₂-C₆ alkynyl, the C₃-C₁₀ cycloalkyl, the C₆-C₁₀ aryl, the 3- to 10-membered heterocycloalkyl or the 5-to 10-membered heteroaryl is optionally substituted with 1 or more R^{f2} and/or R^{g2};
each R^{f2} is independently selected from -OR^{g2}, -NR^{g2}R^{g2}, halogen, -CN, -C(O)R^{g2}, -C(O)OR^{g2}, -C(O)NR^{g2}R^{g2}, - S(O)₂R^{g2}, -S(O)₂NR^{g2}R^{g2}, -NHC(O)R^{g2}, -N(C₁-C₄ alkyl)C(O)R^{g2} and oxo, and the substitution with the oxo is performed only on a non-aromatic ring;
each R^{g2} is independently selected from hydrogen, C₁-C₆ alkyl, C₂-C₆ alkenyl, C₂-C₆ alkynyl, C₃-C₁₀ cycloalkyl, C₆-C₁₀ aryl, 3- to 10-membered heterocycloalkyl and 5- to 10-membered heteroaryl;
or R₁ is selected from C₂-C₄ alkyl and C₂-C₄ alkenyl, and the C₂-C₄ alkyl and the C₂-C₄ alkenyl are optionally substituted with R^{b3};
R^{b3} is selected from -C(O)R^{c3}, -C(O)OR^{c3}, -C(O)NR^{c3}R^{c3}, -C(O)NHOR^{c3} and -C(O)N(C₁-C₄ alkyl)OR^{c3};
each R^{c3} is independently selected from hydrogen, C₁-C₆ alkyl, C₂-C₆ alkenyl, C₂-C₆ alkynyl, C₃-C₁₀ cycloalkyl, C₆-C₁₀ aryl, 3- to 10-membered heterocycloalkyl and 5- to 10-membered heteroaryl, and the C₁-C₆ alkyl, the C₂-C₆ alkenyl, the C₂-C₆ alkynyl, the C₃-C₁₀ cycloalkyl, the C₆-C₁₀ aryl, the 3- to 10-membered heterocycloalkyl or the 5-to 10-membered heteroaryl is optionally substituted with 1 or more R^{d3} and/or R^{e3};
each R^{d3} is independently selected from -OR^{e3}, -NR^{e3}R^{e3}, halogen, -CN, -C(O)R^{e3}, -C(O)OR^{e3}, -C(O)NR^{e3}R^{e3}, - S(O)₂R^{e3}, -S(O)₂NR^{e3}R^{e3}, -NHC(O)R^{e3}, -N(C₁-C₄ alkyl)C(O)R^{e3} and oxo, and the substitution with the oxo is performed only on a non-aromatic ring;
each R^{e3} is independently selected from hydrogen, C₁-C₆ alkyl, C₂-C₆ alkenyl, C₂-C₆ alkynyl, C₃-C₁₀ cycloalkyl, C₆-C₁₀ aryl, 3- to 10-membered heterocycloalkyl and 5- to 10-membered heteroaryl;
R₃ is selected from hydrogen, C₁-C₄ alkyl, -O(C₁-C₄ alkyl), -NH₂, -NH(C₁-C₄ alkyl), -N(C₁-C₄ alkyl)₂ and halogen; R₅ is selected from hydrogen, hydroxy and -NHR';
R' is selected from hydrogen, C₁-C₃ alkyl and -C(O)C₁-C₃ alkyl;
R₄ is selected from C₁-C₄ alkyl, hydroxy, oxo, cyano, C₁-C₄ alkoxy, C₁-C₄ haloalkyl, C₁-C₄ hydroxyalkyl, hydroxy-C₁-C₄ haloalkyl, C₂-C₆ alkenyl, -C₂-C₆ alkynyl, C₃-C₆ cycloalkoxy, C₃-C₆ cycloalkyl, 3- to 6-membered heterocycloalkyl, 3- to 6-membered hydroxyheterocycloalkyl, halogen and -SO₂-C₁-C₄ alkyl;
R₆ is selected from hydrogen, C₁-C₄ alkyl and halogen;
or R₄ and R₆, together with the carbon atom linked thereto, form optionally substituted (e.g., unsubstituted or oxo) C₅-C₆ cycloalkyl (e.g., forming 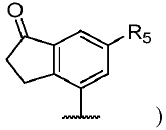 or 5- to 6-membered heterocycloalkyl, wherein the heteroatom in the 5- to 6-membered heterocycloalkyl is selected from a heteroatom and a heteroatom group comprising 1, 2, 3 or 4 heteroatoms or heteroatom groups independently selected from -NH, -O-, -S- and N;
R₇ and R₈ are each independently C₁-C₆ alkyl, C₁-C₆ alkoxy, C₃-C₆ cycloalkyl or C₃-C₆ cycloalkoxy, wherein the alkyl, the alkoxy, the cycloalkyl and the cycloalkoxy are optionally substituted with cyano, hydroxy, C₁-C₆ alkoxy, C₃-C₆ cycloalkyl, C₃-C₆ cycloalkoxy or halogen.

In some embodiments, in S6, when R₄ and R₆, together with the carbon atom linked thereto, form optionally substituted C₅-C₆ cycloalkyl and 5- to 6-membered heterocycloalkyl, the substitution refers to a substitution with 1, 2, 3 or 4 =O.

In some embodiments, in S6, when R₄ and R₆, together with the carbon atom linked thereto, form optionally substituted 5- to 6-membered heterocycloalkyl, the fragment 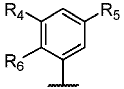 in S6 is 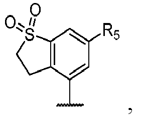 , e.g.,

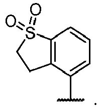

In some embodiments, in S6, R₁ is hydrogen or -OR^{A}, and R^{A} is hydrogen.

In some embodiments, in S6, R₁ is hydrogen.

In some embodiments, in S6, R² is hydrogen, C₁-C₄ alkyl, -O(C₁-C₄ alkyl), -NH₂, -NH(C₁-C₄ alkyl), -N(C₁-C₄ alkyl)₂ or halogen, and the C₁-C₄ alkyl and the -O(C₁-C₄ alkyl) are each independently optionally substituted with -O(C₁-C₄ alkyl).

In some embodiments, in S6, R₂ is hydrogen, C₁-C₄ alkyl, -O(C₁-C₄ alkyl), -NH₂, -NH(C₁-C₄ alkyl), -N(C₁-C₄ alkyl)₂ or halogen.

In some embodiments, in S6, R₂ is hydrogen, C₁-C₄ alkyl or -O(C₁-C₄ alkyl), wherein the -O(C₁-C₄ alkyl) is optionally substituted with -O(C₁-C₄ alkyl).

In other embodiments, in S6, R₂ is

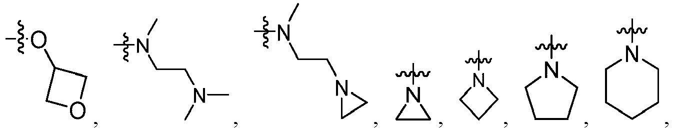

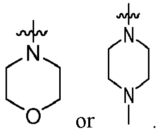

In some embodiments, in S6, R₃ is hydrogen.

In some embodiments, in S6, R₅ is hydrogen or -NHR'.

In some embodiments, in S6, R₄ is C₁-C₄ haloalkyl; R₆ is hydrogen or halogen; or R₄ and R₆, together with the carbon atom linked thereto, form a ring such that the fragment 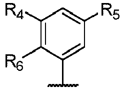 in S6 is

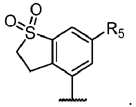

In some embodiments, in S6, R₇ is C₁-C₆ alkyl.

In some embodiments, in S6, R₈ is C₁-C₆ alkyl.

In some embodiments, in S6, R₁ is hydrogen or -OR^{A};
R^{A} is hydrogen;
R₂ is hydrogen, C₁-C₄ alkyl or -O(C₁-C₄ alkyl) (e.g., methoxy), wherein the -O(C₁-C₄ alkyl) is optionally substituted with -O(C₁-C₄ alkyl) (e.g., methoxy);
R₃ is hydrogen;
R₅ is hydrogen or -NHR';
R' is selected from hydrogen, C₁-C₃ alkyl and -C(O)C₁-C₃ alkyl;
R₄ is C₁-C₄ haloalkyl;
R₆ is hydrogen or halogen;
or R₄ and R₆, together with the carbon atom linked thereto, form a ring such that the fragment 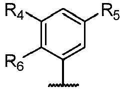 in S6 is 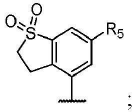
R₇ is C₁-C₆ alkyl;
R₈ is C₁-C₆ alkyl.

In some embodiments, in S6, when R₂ is C₁-C₄ alkyl, the C₁-C₄ alkyl may be methyl.

In some embodiments, in S6, when R₂ is -O(C₁-C₄ alkyl), the -O(C₁-C₄ alkyl) may be -OCH₃.

In some embodiments, in S6, R₂ is hydrogen, methyl, -OCH₃ or

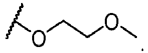

In some embodiments, in S6, R₂ is hydrogen, methyl or -OCH₃.

In some embodiments, in S6, R₃ is hydrogen.

In some embodiments, in S6, when R' is C₁-C₃ alkyl, the C₁-C₃ alkyl may be ethyl or isopropyl.

In some embodiments, in S6, when R' is -C(O)C₁-C₃ alkyl, the -C(O)C₁-C₃ alkyl may be -C(O)CH₃.

In some embodiments, in S6, R₅ is hydrogen or -NHR', wherein R' is hydrogen, ethyl, isopropyl or -C(O)CH₃.

In some preferred embodiments, in S6, R₅ is -NHR', wherein R' is hydrogen.

In some embodiments, in S6, R₄ is C₁-C₄ haloalkyl;
R₆ is hydrogen or halogen;
or R₄ and R₆, together with the carbon atom linked thereto, form a ring such that the fragment 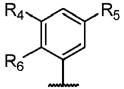 in S6 is

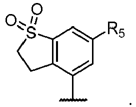

In some embodiments, in S6, when R₄ is C₁-C₄ haloalkyl, the C₁-C₄ haloalkyl may be C₁-C₄ fluoroalkyl, e.g., trifluoromethyl.

In some embodiments, in S6, R₇ is C₁-C₆ alkyl.

In some embodiments, in S6, when R₇ is C₁-C₆ alkyl, the C₁-C₆ alkyl may be methyl.

In some embodiments, in S6, R₈ is C₁-C₆ alkyl.

In some embodiments, in S6, when R₈ is C₁-C₆ alkyl, the C₁-C₆ alkyl may be methyl.

In some embodiments, the fragment 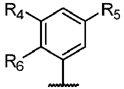 in S6 may be

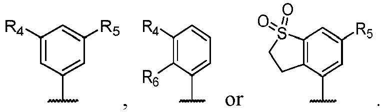

In some embodiments, the fragment 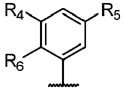 in S6 is preferably

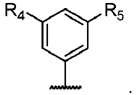

In some embodiments, the fragment 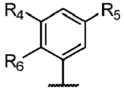 in S6 is preferably

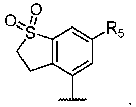

In some embodiments, the fragment 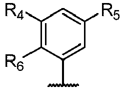 in S6 may be 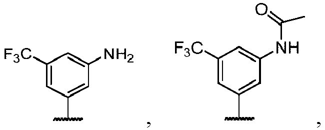 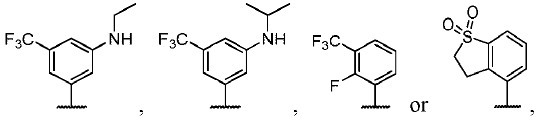 preferably

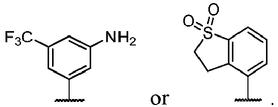

In some preferred embodiments, the fragment 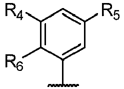 in S6 is

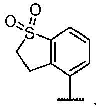

In some more preferred embodiments, the fragment 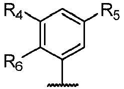 in S6 is

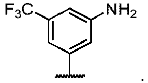

Preferably, in certain embodiments of the present invention, in S6, R₈ is methyl.

In certain embodiments of the present invention, S is S6': 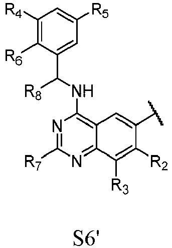 wherein in S6', R₂, R₃, R₄, R₅, R₆ and R₇ are as defined and described in S6.

In certain embodiments of the present invention, in S6 and S6', the fragment 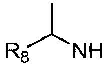 is

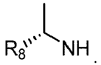

In certain embodiments of the present invention, S is -X-S6', and the structure of S6' is as described above; X is O, NH or S, preferably O.

Preferably, in certain embodiments of the present invention, S is S6a: 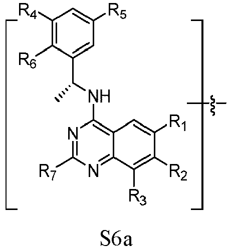 wherein in S6a, R₁, R₂, R₃, R₄, R₅, R₆ and R₇ are as defined and described in S6.

Preferably, in certain embodiments of the present invention, in S6 or S6a, R¹ is hydrogen; R² is selected from -O(C₁-C₄ alkyl); R³ is hydrogen; R⁴ is C₁-C₄ haloalkyl; R₅ is selected from -NH₂; R⁷ is selected from C₁-C₄ alkyl and C₁-C₄ haloalkyl.

Preferably, in certain embodiments of the present invention, S is S6b: 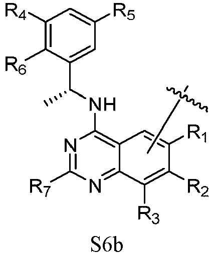 wherein in S6b, R₁, R₂, R₃, R₄, R₅, R₆ and R₇ are as defined and described in S6.

Preferably, in certain embodiments of the present invention, S is S6c: 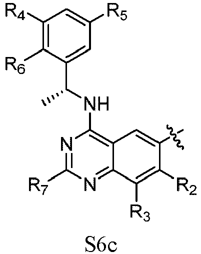 wherein in S6c, R₂, R₃, R₄, R₅, R₆ and R₇ are as defined and described in S6.

In certain embodiments of the present invention, S is -X-S6c, and the structure and the definition of S6c are as described above; wherein X is O, NH or S, preferably O.

Preferably, in certain embodiments of the present invention, S is S6d: 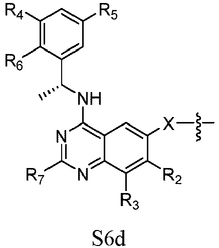 wherein in S6d, X is O, NH or S, preferably O; R₂, R₃, R₄, R₅, R₆ and R₇ are as defined and described in S6. Preferably, in certain embodiments of the present invention, S6 is the specific compound according to I-1 to I-383 in WO2018115380A1 (incorporated herein by reference in its entirety). Preferably, in certain embodiments of the present invention, S is S6e: 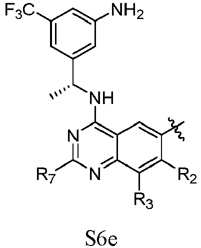 wherein in S6e, R₂, R₃ and R₇ are as defined and described in any one of the embodiments of S6.

Preferably, in certain embodiments of the present invention, S is -X-S6e, and the structure and the definition of S6e are as described above; wherein X is O, NH or S, preferably O.

Preferably, in certain embodiments of the present invention, in S6e, R₂ is -O(C₁-C₄ alkyl); R₃ is hydrogen; R₇ is C₁-C₆ alkyl.

Preferably, in certain embodiments of the present invention, in S6e, R₂ is -OCH₃; R₃ is hydrogen; R₇ is methyl.

In certain embodiments of the present invention, S6, S6', S6a or S6c is any one of the following structures:

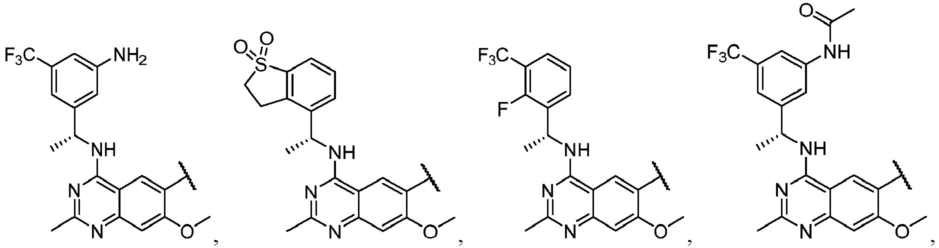

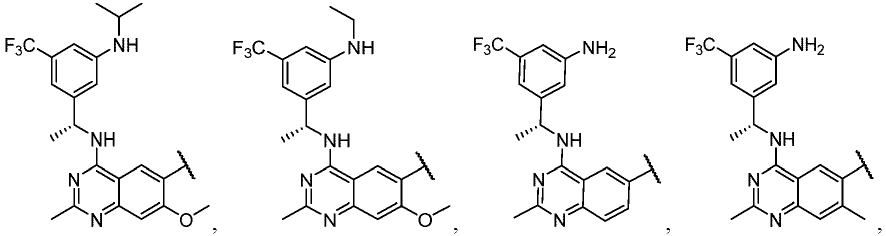

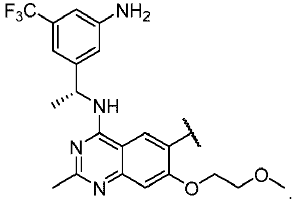

Preferably, in certain embodiments of the present invention, S6, S6', S6a or S6c is

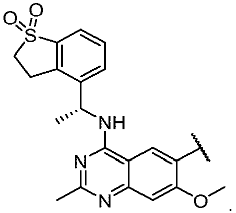

Preferably, in certain embodiments of the present invention, S6, S6', S6a, S6c or S6e is

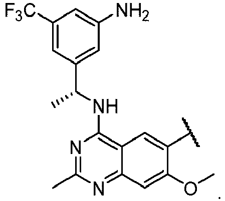

In certain embodiments of the present invention, S is

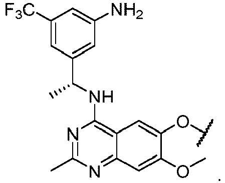

In certain embodiments of the present invention, S is S6": 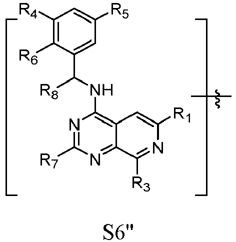 wherein in S6", R₁, R₃, R₄, R₅, R₆, R₇ and R₈ are as defined and described in S6.

In certain embodiments of the present invention, S is S6‴: 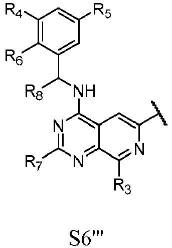 wherein in S6‴, R₃, R₄, R₅, R₆, R₇ and R₈ are as defined and described in S6.

In certain embodiments of the present invention, in S6" and S6‴, the fragment 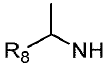 is

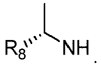

In certain embodiments of the present invention, S is -X-S6‴, and the structure of S6‴ is as described above; wherein X is O, NH or S, preferably O.

Preferably, in certain embodiments of the present invention, S6" is

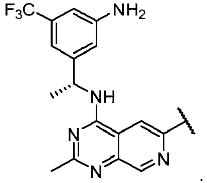

Preferably, in certain embodiments of the present invention, S is S6a": 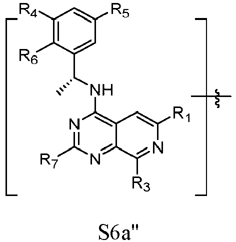 wherein in S6a", R₁, R₃, R₄, R₅, R₆ and R₇ are as defined and described in S6.

Preferably, in certain embodiments of the present invention, S is S6b": 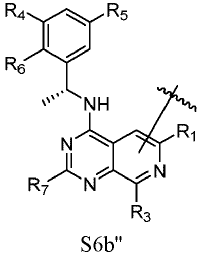 wherein in S6b", R₁, R₃, R₄, R₅, R₆ and R₇ are as defined and described in S6.

Preferably, in certain embodiments of the present invention, S is S6c": 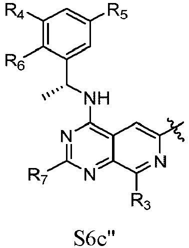 wherein in S6c", R₃, R₄, R₅, R₆ and R₇ are as defined and described in S6.

Preferably, in certain embodiments of the present invention, S is -X-S6c", and the structure and the definition of S6c" are as described above; wherein X is O, NH or S, preferably O.

Preferably, in certain embodiments of the present invention, S is S6d": 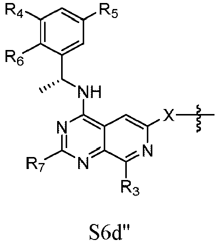 wherein in S6d", X is O, NH or S, preferably O; R₃, R₄, R₅, R₆ and R₇ are as defined and described in S6. Preferably, in certain embodiments of the present invention, S is S6e": 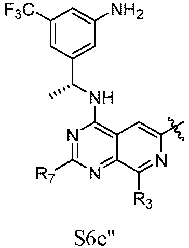 wherein in S6e", R₃ and R₇ are as defined and described in S6.

Preferably, in certain embodiments of the present invention, S is -X-S6e", and the structure and the definition of S6e" are as described above; wherein X is O, NH or S, preferably O.

Preferably, in certain embodiments of the present invention, in S6e", R₃ is hydrogen; R₇ is C₁-C₆ alkyl. Preferably, in certain embodiments of the present invention, in S6e", R₃ is hydrogen; R₇ is methyl.

Preferably, in certain embodiments of the present invention, S6", S6‴, S6a", S6c" or S6e" is

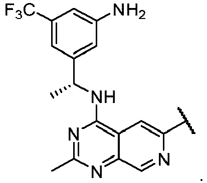

Preferably, in certain embodiments of the present invention, S is

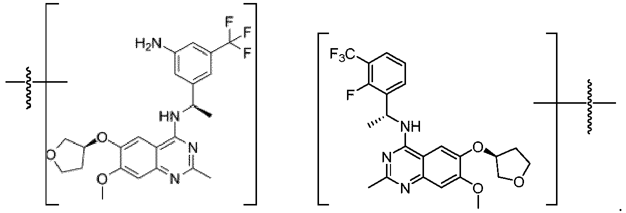

More preferably, in certain embodiments of the present invention, S is

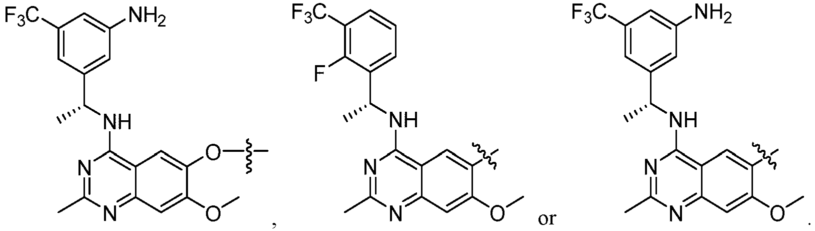

More preferably, in certain embodiments of the present invention, S is

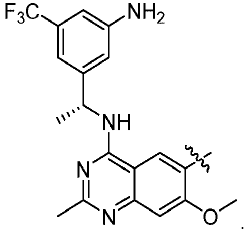

In certain embodiments of the present invention, L is a bond.

In certain embodiments of the present invention, L is -(CH₂)ⱼ-, wherein 1 or more of methylene groups in the - (CH₂)ⱼ- are optionally replaced by a group selected from -NR^{3'}-, -O-, -S-, -S(O)-, -S(O)NR^{3'}-, -NR^{3'}S(O)-, -S(O)₂-, -S(O)₂NR^{3'}-, -NR^{3'}S(O)₂-, -NR^{4'}S(O)₂NR^{3'}-, -CR^{1'}R^{2'}-, -C(O)-, -C(O)O-, -OC(O)-, -NR^{3'}C(O)O-, -OC(O)NR^{3'}-, - C(O)NR^{3'}-, -NR^{3'}C(O)-, -NR^{4'}C(O)NR^{3'}-, -P(O)-, -P(O)O-, -OP(O)-, -OP(O)O-, ethenylene, ethynylene, 3- to 12-membered cycloalkylene, 3- to 12-membered heterocycloalkylene containing 1 or more heteroatoms selected from N, O and S, 6- to 10-membered arylene and 5- to 10-membered heteroarylene, wherein the ethenylene, the cycloalkylene, the heterocycloalkylene, the arylene and the heteroarylene are each independently optionally substituted with 1 or more substituents selected from halogen, -OR^{3'}, -NR^{3'}R^{4'}, oxo, nitro, cyano, C₁-C₆ alkyl, -S(C₁-C₆ alkyl), C₃-C₁₀ cycloalkyl, C₃-C₁₀ heterocycloalkyl, C₆-C₁₀ aryl, 5- to 10-membered heteroaryl, -C(O)R^{1'}, - C(O)OR^{3'}, -OC(O)R^{1'}, -C(O)NR^{3'}, -NR^{3'}C(O)R^{1'}, -S(O)R^{1'}, -S(O)NR^{3'}, -S(O)₂R^{1'}, -S(O)₂NR^{3'}, -NR^{3'}S(O)₂R^{1'}, - NR^{4'}S(O)₂NR^{3'}, -OC(O)NR^{3'} and -NR^{4'}C(O)NR^{3'}, wherein the alkyl, the cycloalkyl, the heterocycloalkyl, the aryl and the heteroaryl are each independently optionally substituted with 1 or more substituents selected from halogen, -OH, -NR^{3'}R^{4'}, oxo, nitro, cyano, C₁-C₆ alkyl, C₃-C₁₀ cycloalkyl, C₃-C₁₀ heterocycloalkyl, 6- to 10-membered aryl and 5- to 10-membered heteroaryl; R^{1'} and R^{2'} are each independently halogen, -OH, -NR^{3'}R^{4'}, C₁-C₆ alkyl, C₁-C₆ chloroalkyl, hydroxy C₁-C₆ alkyl, -O(C₁-C₆ alkyl), -NH(C₁-C₆ alkyl), -NH(C₁-C₆ alkyl)₂, C₃-C₁₀ cycloalkyl, -O(C₃-C₁₀ cycloalkyl), -NH(C₃-C₁₀ cycloalkyl), C₃-C₁₀ heterocycloalkyl, -O(C₃-C₁₀ heterocycloalkyl), -NH(C₃-C₁₀ heterocycloalkyl), 6- to 10-membered aryl, -O(6- to 10-membered aryl), -NH(6- to 10-membered aryl), 5- to 10-membered heteroaryl, -O(5- to 10-membered heteroaryl) or -NH(5- to 10-membered heteroaryl), R^{3'} and R^{4'} are each independently hydrogen, deuterium, C₁-C₆ alkyl, C₃-C₁₀ cycloalkyl, C₃-C₁₀ heterocycloalkyl, 6- to 10-membered aryl or 5- to 10-membered heteroaryl; j is 1, 2, 3, 4, 5, 6, 7, 8, 9, 10, 11, 12, 13, 14, 15, 16, 17, 18, 19, 20, 21, 22, 23, 24 or 25.

Preferably, in certain embodiments of the present invention, L is -(CH₂)ⱼ-, wherein 1 or more of methylene groups in the -(CH₂)ⱼ- are optionally replaced by a group selected from -NR^{3'}-, -O-, -CR^{1'}R^{2'}-, -C(O)-, -S(O)-, -S(O)₂-, - C(O)O-, -OC(O)-, -C(O)NR^{3'}-, -NR^{3'}C(O)-, -S(O)₂NR^{3'}-, -NR^{3'}S(O)₂-, ethenylene, ethynylene, phenyl, 8- to 10-membered bicyclic arylene, 3- to 7-membered saturated or partially unsaturated cycloalkylene, 5- to 11-membered saturated or partially unsaturated spirocycloalkylene, 5- to 11-membered saturated or partially unsaturated fused cycloalkylene, 8- to 10-membered bicyclic saturated or partially unsaturated cycloalkylene, 4- to 7-membered saturated or partially unsaturated heterocycloalkylene having 1-2 heteroatoms independently selected from nitrogen, oxygen and sulfur, 5- to 11-membered saturated or partially unsaturated spiroheterocycloalkylene having 1-2 heteroatoms independently selected from nitrogen, oxygen and sulfur, 5- to 11-membered saturated or partially unsaturated fused heterocycloalkylene having 1-2 heteroatoms independently selected from nitrogen, oxygen and sulfur, 8- to 10-membered bicyclic saturated or partially unsaturated heterocycloalkylene having 1-2 heteroatoms independently selected from nitrogen, oxygen and sulfur, 5- to 6-membered heteroarylene having 1-4 heteroatoms independently selected from nitrogen, oxygen and sulfur, and 8- to 10-membered bicyclic heteroaryl having 1-5 heteroatoms selected from nitrogen, oxygen and sulfur, wherein the ethenylene, the ethynylene, the cycloalkylene, the heterocycloalkylene, the phenyl, the spiroheterocycloalkylene, the fused heterocycloalkylene, the spirocycloalkylene, the fused cycloalkylene and the heteroarylene are each independently optionally substituted with 1 or more substituents selected from halogen, oxo, -NR^{3'}R^{4'}, -OR^{3'}, nitro, -CN, C₁-C₆ alkyl, C₃-C₁₀ cycloalkyl and C₃-C₁₀ heterocycloalkyl, wherein the alkyl, the cycloalkyl and the heterocycloalkyl are optionally substituted with 1 or more substituents selected from halogen, -OH, -NH₂, -CN, C₁-C₄ alkyl and C₃-C₆ cycloalkyl; R^{1'} and R^{2'} are each independently halogen, -OH, -NH₂, C₁-C₄ alkyl, C₁-C₄ chloroalkyl, C₁-C₄ hydroxyalkyl, -O(C₁-C₄ alkyl), -NH(C₁-C₄ alkyl), C₃-C₆ cycloalkyl, -O(C₃-C₆ cycloalkyl), -NH(C₃-C₆ cycloalkyl), C₃-C₆ heterocycloalkyl, -O(C₃-C₆ heterocycloalkyl) or -NH(C₃-C₆ cycloalkyl); R^{3'} and R^{4'} are each independently hydrogen, deuterium, C₁-C₄ alkyl, C₃-C₆ cycloalkyl, or C₃-C₆ heterocycloalkyl, and j is 2, 3, 4, 5, 6, 7, 8, 9, 10, 11 or 12.

Preferably, in certain embodiments of the present invention, L is -(CH₂)ⱼ-, wherein 1 or more of methylene groups in the -(CH₂)ⱼ- are optionally replaced by a group selected from -NR^{3'}-, -O-, -CR^{1'}R^{2'}-, -C(O)-, -S(O)-, -S(O)₂-, - C(O)O-, -OC(O)-, -C(O)NR³'-, -NR^{3'}C(O)-, -S(O)₂NR^{3'}-, -NR^{3'}S(O)₂-, ethenylene, ethynylene, phenyl, 8- to 10-membered bicyclic arylene, 3- to 7-membered saturated or partially unsaturated monocyclic cycloalkylene, 5- to 11-membered saturated or partially unsaturated spirocycloalkylene, 5- to 11-membered saturated or partially unsaturated fused cycloalkylene, 8- to 10-membered bicyclic saturated or partially unsaturated cycloalkylene, 5- to 7-membered saturated or partially unsaturated heterocycloalkylene having 1-2 heteroatoms independently selected from nitrogen and oxygen, 5- to 11-membered saturated or partially unsaturated spiroheterocycloalkylene having 1-2 heteroatoms independently selected from nitrogen and oxygen, 5- to 11-membered saturated or partially unsaturated fused heterocycloalkylene having 1-2 heteroatoms independently selected from nitrogen and oxygen, 8- to 10-membered bicyclic saturated or partially unsaturated heterocycloalkylene having 1-2 heteroatoms independently selected from nitrogen and oxygen, 5- to 6-membered heteroarylene having 1-4 heteroatoms independently selected from nitrogen and oxygen, and 8- to 10-membered bicyclic heteroaryl having 1-5 heteroatoms selected from nitrogen and oxygen, wherein the ethenylene, the ethynylene, the cycloalkylene, the heterocycloalkylene, the phenyl, the spiroheterocycloalkylene, the fused heterocycloalkylene, the fused cycloalkylene, the spirocycloalkylene and the heteroarylene are each independently optionally substituted with 1 or more substituents selected from halogen, oxo, -NR^{3'}R^{4'}, -OR^{3'}, nitro, -CN, C₁-C₆ alkyl, C₃-C₆ cycloalkyl and C₃-C₆ heterocycloalkyl, wherein the alkyl, the cycloalkyl and the heterocycloalkyl are optionally substituted with 1 or more substituents selected from halogen, -OH, -NH2, -CN, C1-C4 alkyl and C3-C6 cycloalkyl; R^{1'} and R^{2'} are each independently halogen, -OH, -NH₂, C₁-C₄ alkyl, C₁-C₄ chloroalkyl, C₁-C₄ hydroxyalkyl, -O(C₁-C₄ alkyl), -NH(C₁-C₄ alkyl), -NH(C₁-C₄ alkyl), C₃-C₆ cycloalkyl, -O(C₃-C₆ cycloalkyl), -NH(C₃-C₆ cycloalkyl), C₃-C₆ heterocycloalkyl, -O(C₃-C₆ heterocycloalkyl) or -NH(C₃-C₆ cycloalkyl); R^{3'} and R^{4'} are each independently hydrogen, deuterium, C₁-C₄ alkyl, C₃-C₆ cycloalkyl, or C₃-C₆ heterocycloalkyl, and j is 2, 3, 4, 5, 6, 7, 8, 9 or 10. Preferably, in certain embodiments of the present invention, L is -(CH₂)ⱼ-, wherein 1, 2, 3, 4 or 5 methylene groups in the -(CH₂)ⱼ- are optionally replaced by a group selected from -NH-, -NCH₃-, -O-, -C(CH₃)₂-, -CHF-, -CHCF₃-, - C(O)-, -C(O)O-, -OC(O)-, -C(O)NH-, -C(O)NCH₃-, -NHC(O)-, -NCH₃C(O)-, ethenylene, ethynylene, cyclopropylene, cyclobutylene, cyclopentylene, cyclohexylene, oxiranylidene, oxetanylidene, oxolanylidene, oxanylidene, aziridinylidene, azetidinylidene, azolidinylidene, piperidinylidene, piperazinylidene, morpholinylidene, homomorpholinylidene, phenylene, pyrrolylidene, thienylidene, furylidene, imidazolylidene, pyrazolylidene, triazolylidene, tetrazolylidene, oxazolylidene, isoxazolylidene, thiazolylidene, isothiazolylidene, pyridinylidene, pyrimidinylidene, pyridazinylidene, pyrazinylidene, 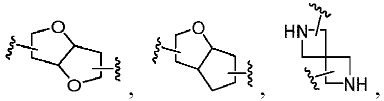 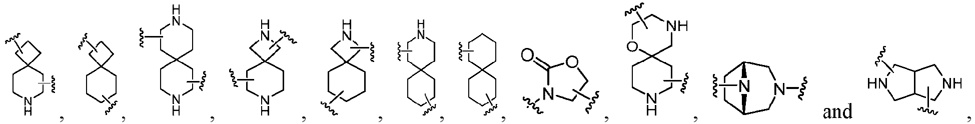 wherein the group for the replacement is optionally substituted with 1 or more substituents selected from halogen, oxo, -NR^{3'}R^{4'}, -OR^{3'} and C1-C4 alkyl, wherein the alkyl is optionally substituted with 1 or more substituents selected from halogen, -OH and -NH₂, R^{3'} and R^{4'} are each independently hydrogen, deuterium or C1-C4 alkyl, and j is 2, 3, 4, 5, 6, 7, 8, 9 or 10.

Preferably, in certain embodiments of the present invention, L is -(CH₂)ⱼ-, wherein 1, 2, 3, 4 or 5 methylene groups in the -(CH₂)ⱼ- are optionally replaced by a group selected from -NH-, -NCH₃-, -O-, -C(CH₃)₂-, -CHF-, -CHCF₃-, - C(O)-, -C(O)O-, -OC(O)-, -C(O)NH-, -C(O)NCH₃-, -NHC(O)-, -NCH₃C(O)-, cyclopropylene, cyclobutylene, cyclopentylene, cyclohexylene, oxiranylidene, oxetanylidene, oxolanylidene, oxanylidene, aziridinylidene, azetidinylidene, azolidinylidene, piperidinylidene, piperazinylidene, morpholinylidene, homomorpholinylidene, 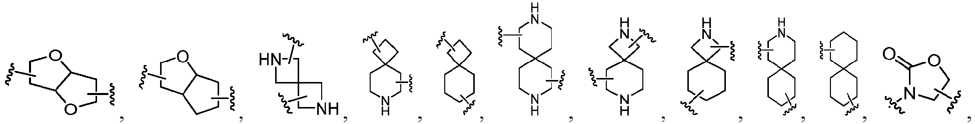 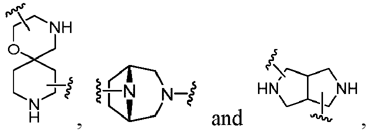 wherein the group for the replacement is optionally substituted with 1 or more substituents selected from F, Cl, oxo, -NR^{3'}R^{4'}, -OR^{3'} and C₁-C₄ alkyl, wherein the alkyl is optionally substituted with 1 or more substituents selected from halogen, -OH and -NH₂, R^{3'} and R^{4'} are each independently hydrogen, deuterium, methyl, ethyl or propyl, and j is 3, 4, 5, 6, 7, 8, 9 or 10.

Preferably, in certain embodiments of the present invention, L is -(CH₂)ⱼ-, wherein 1, 2, 3, 4 or 5 methylene groups in the -(CH₂)ⱼ- are optionally replaced by a group selected from -O-, -NH-, -NCH₃-, -C(O)-, -C(O)NH-, -NHC(O)-, -NCH₃C(O)-, -C(O)NCH₃-, cyclohexylene, aziridinylidene, azetidinylidene, azolidinylidene, piperidinylidene, piperazinylidene, 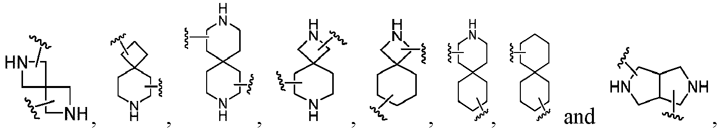 and j is 4, 5, 6, 7, 8, 9 or 10.

Preferably, in certain embodiments of the present invention, L is selected from -(CH₂)ⱼ₋ᵢ-C(O)-, methylene in the - (CH₂)ⱼ₋₁-C(O)- is as defined in L described above, and is optionally replaced by 1 or more groups, and j is as defined in L described above.

Preferably, in certain embodiments of the present invention, L is selected from

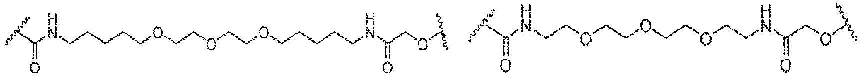

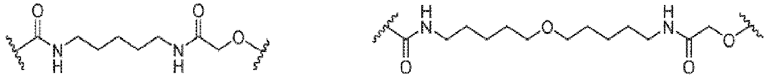

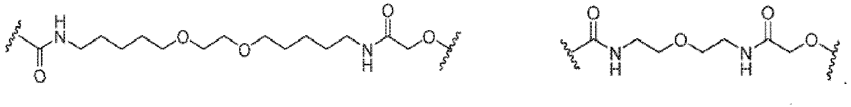

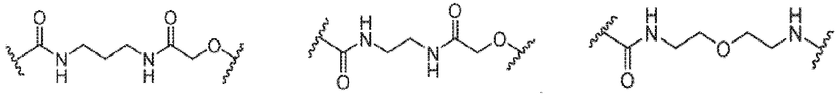

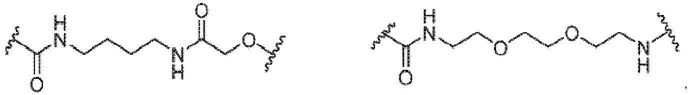

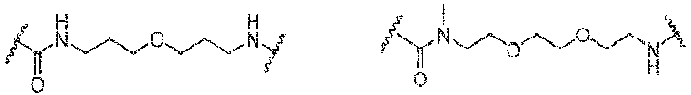

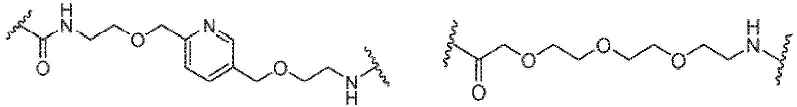

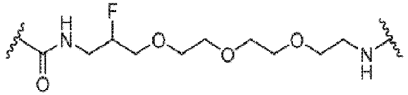

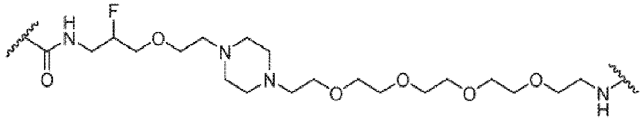

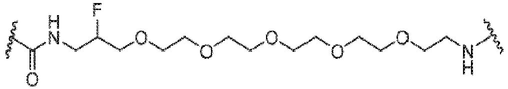

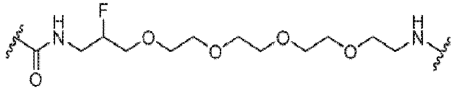

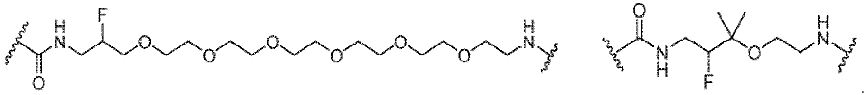

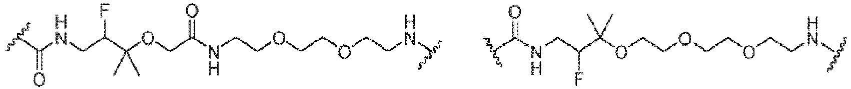

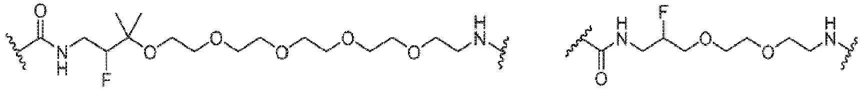

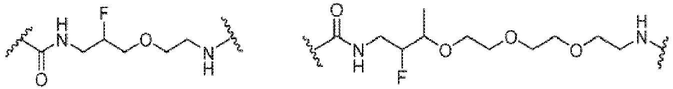

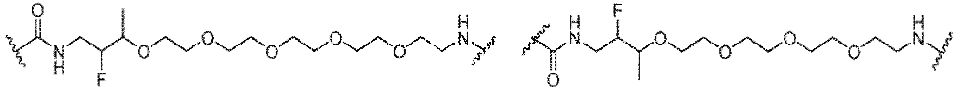

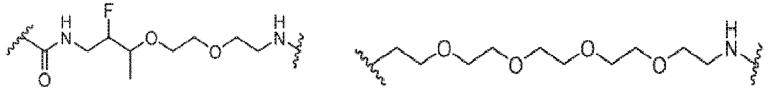

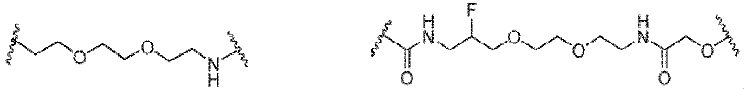

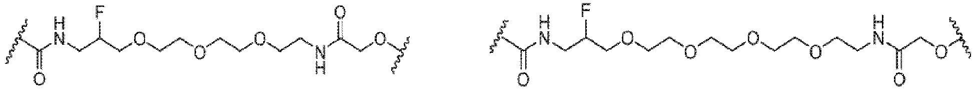

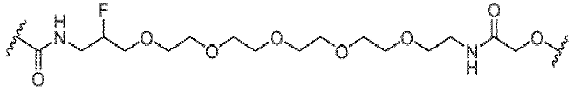

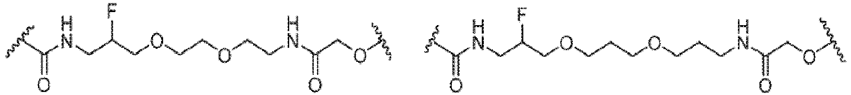

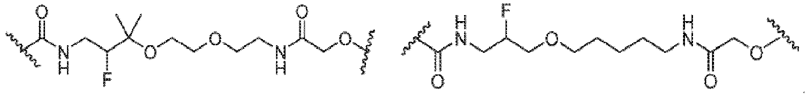

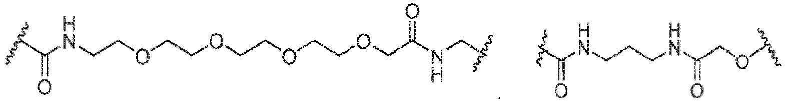

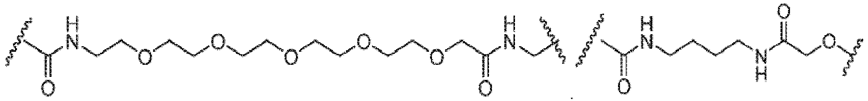

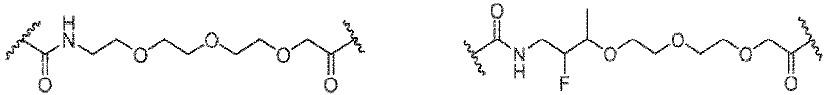

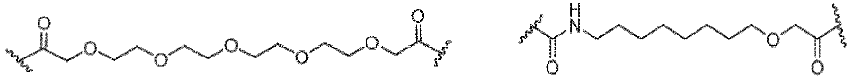

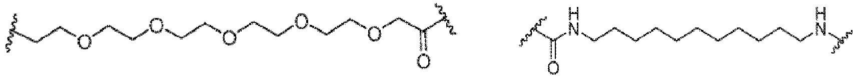

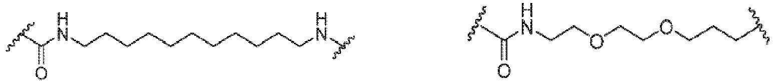

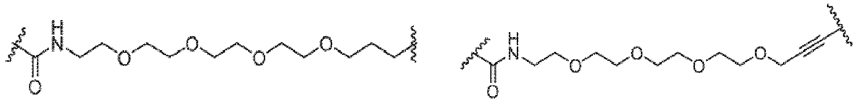

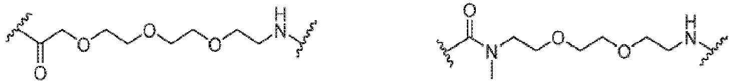

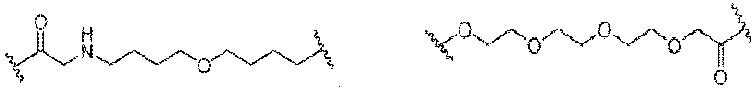

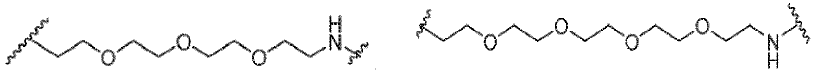

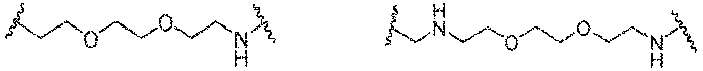

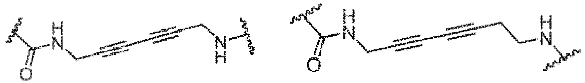

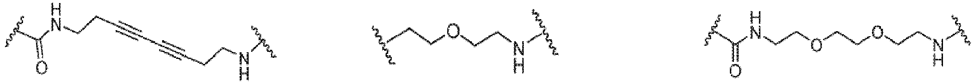

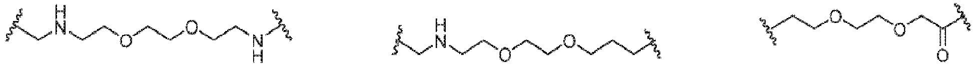

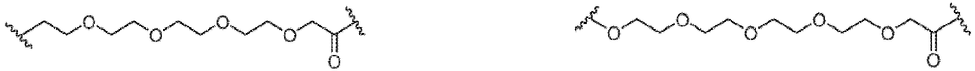

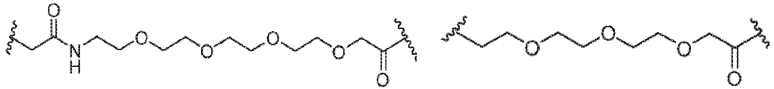

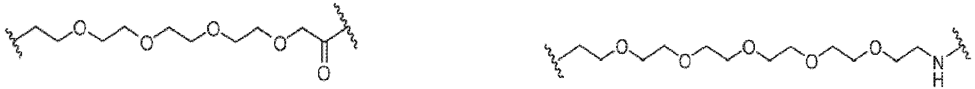

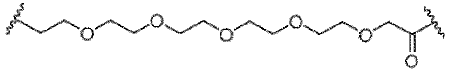

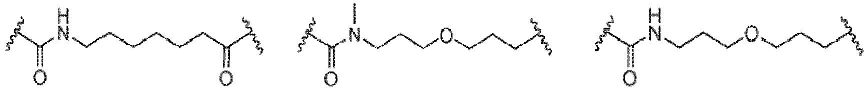

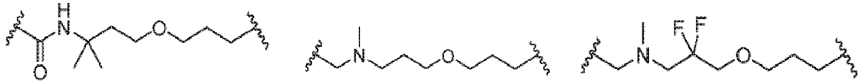

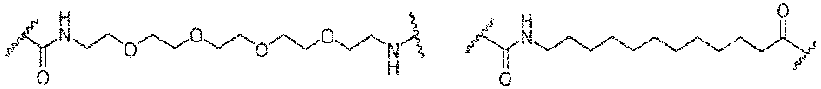

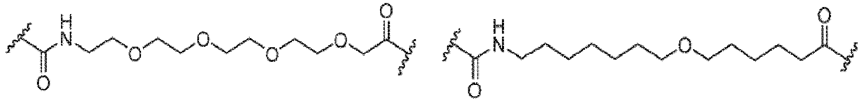

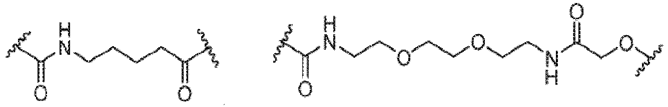

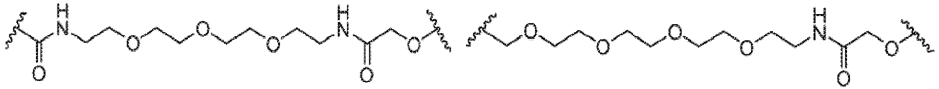

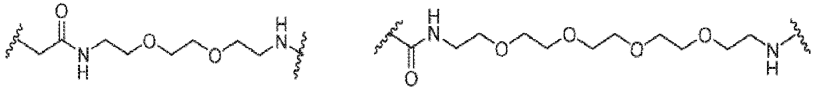

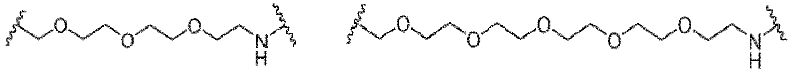

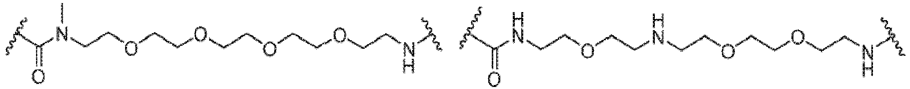

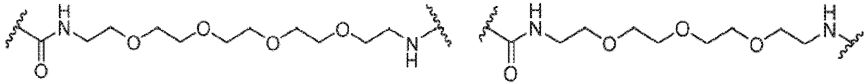

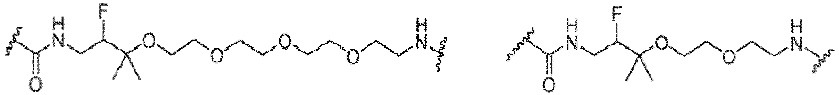

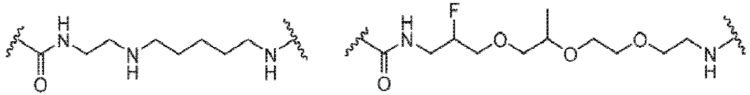

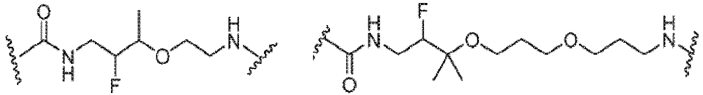

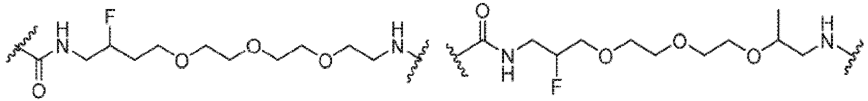

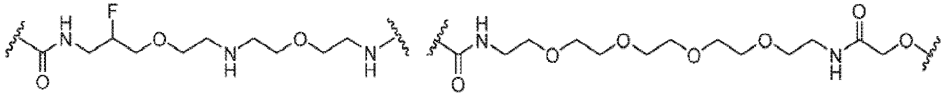

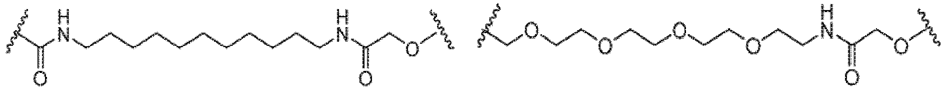

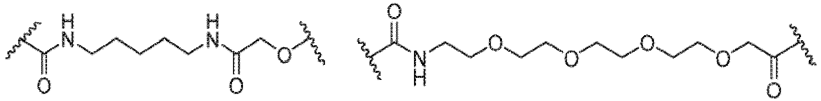

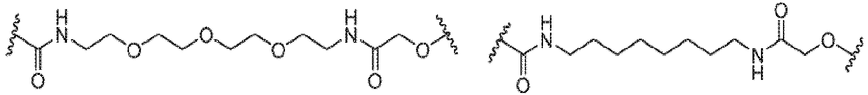

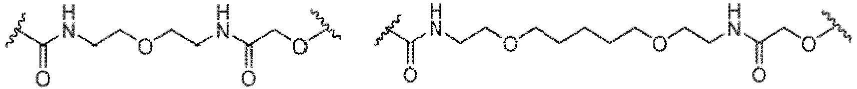

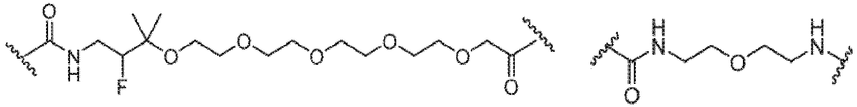

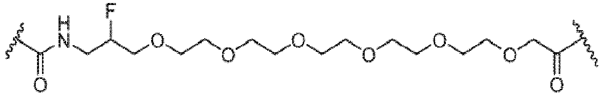

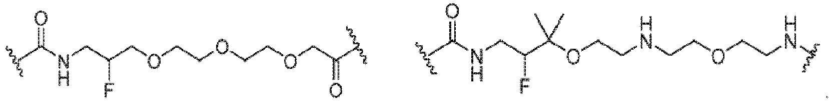

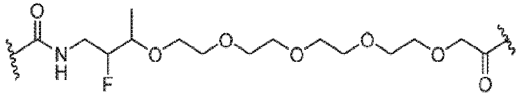

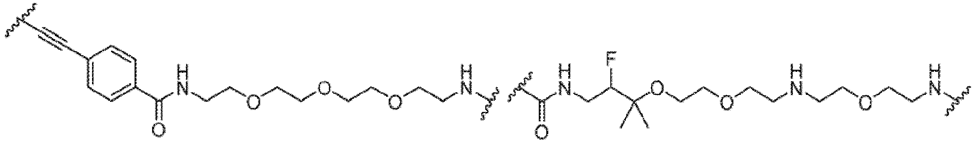

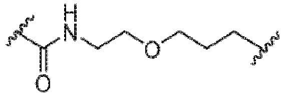

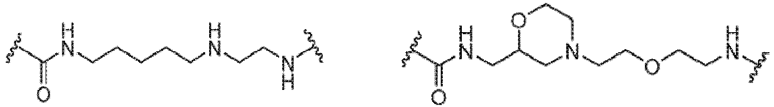

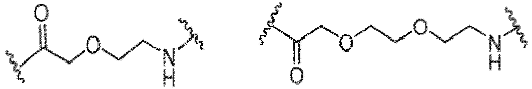

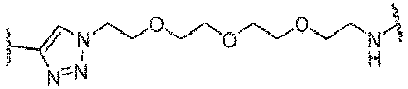

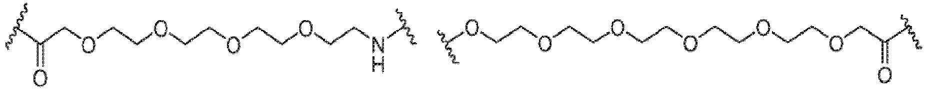

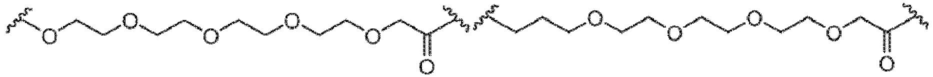

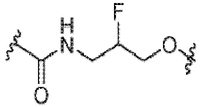

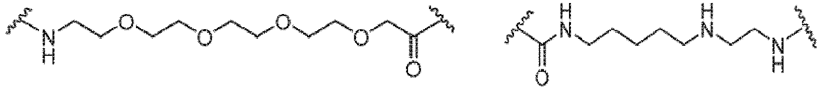

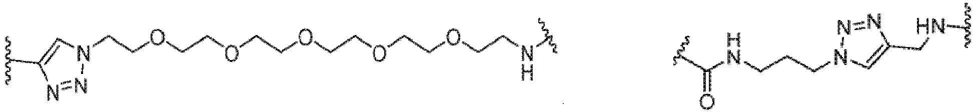

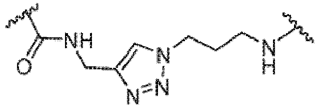

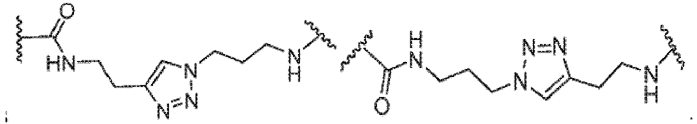

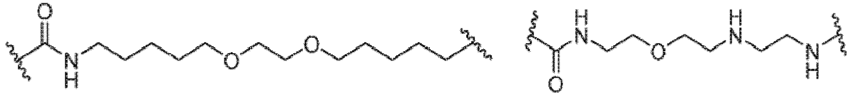

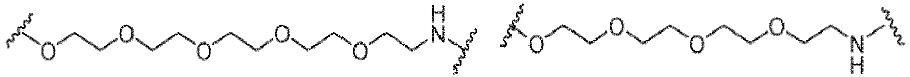

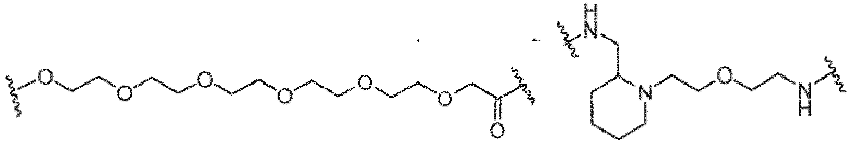

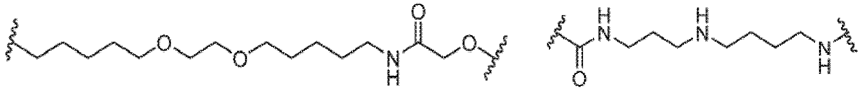

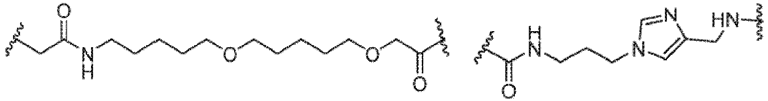

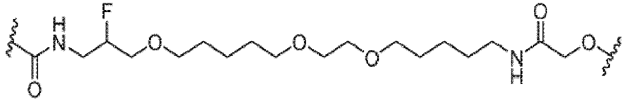

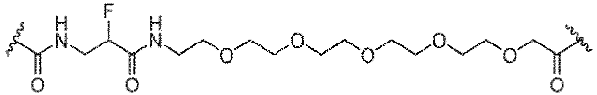

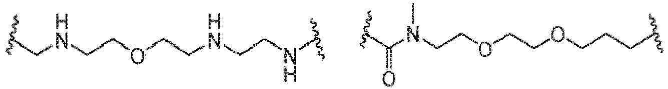

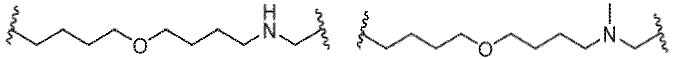

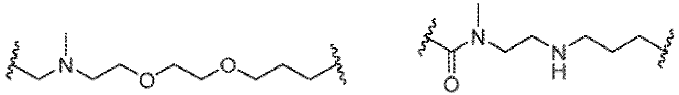

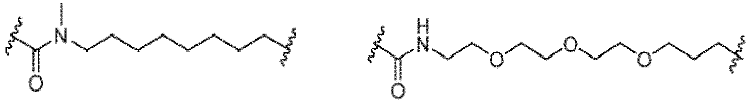

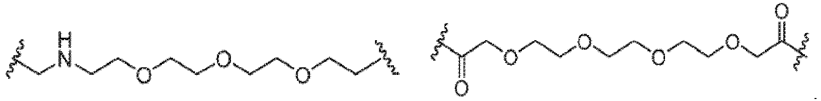

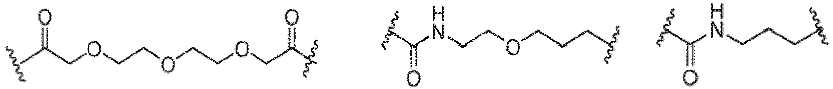

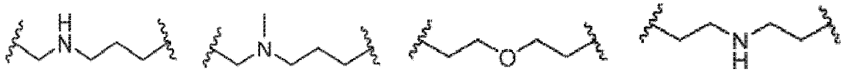

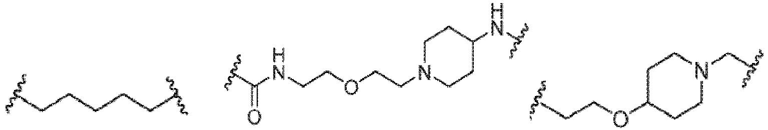

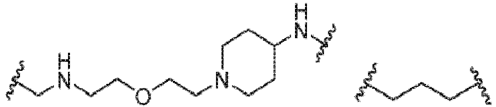

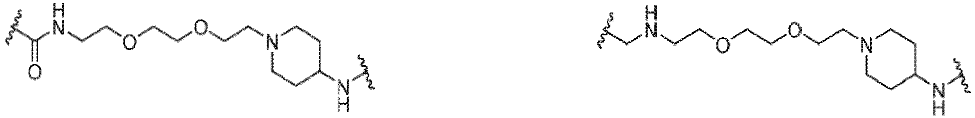

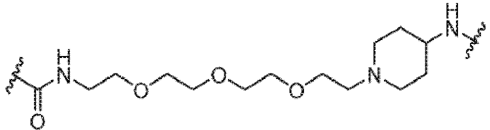

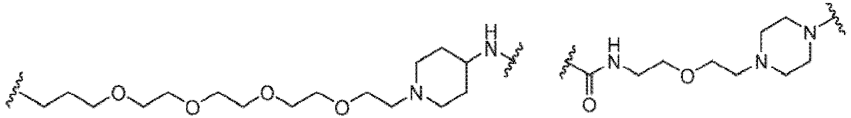

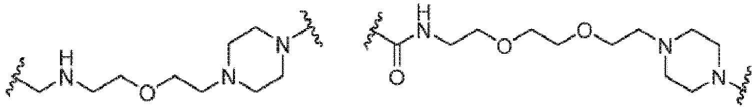

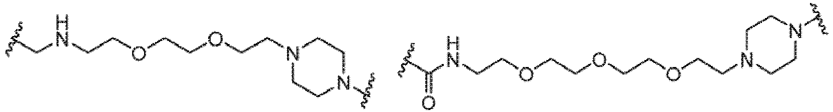

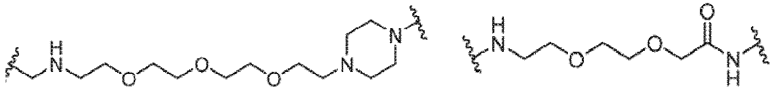

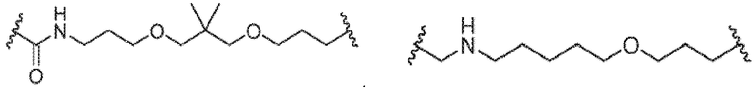

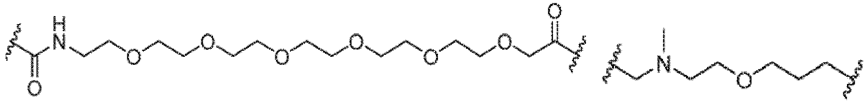

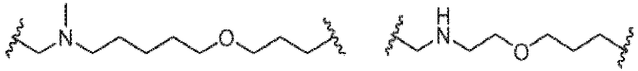

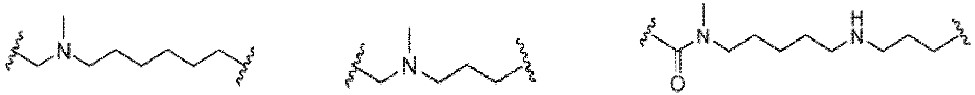

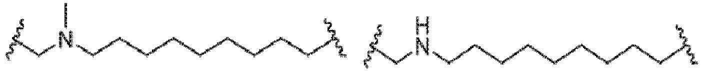

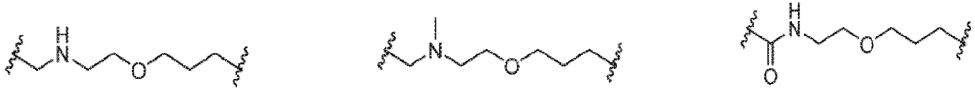

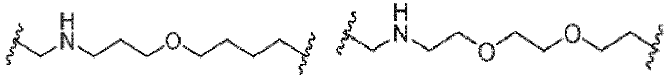

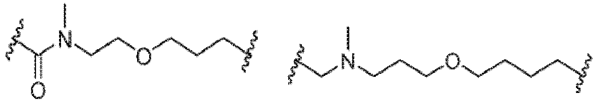

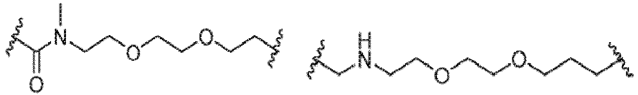

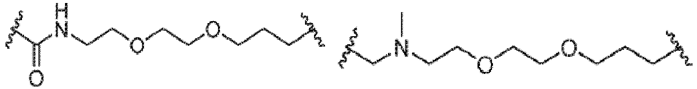

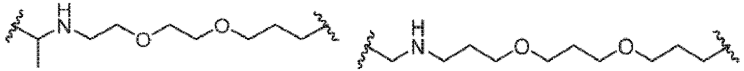

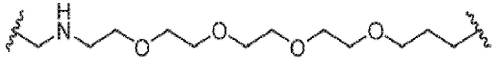

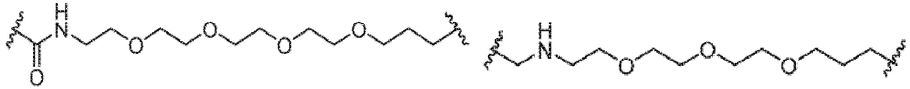

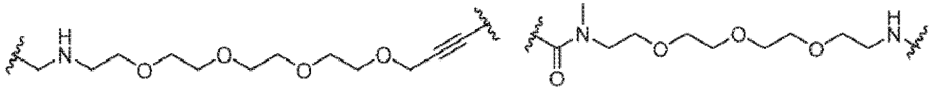

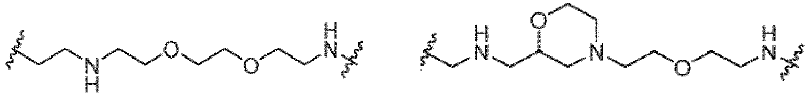

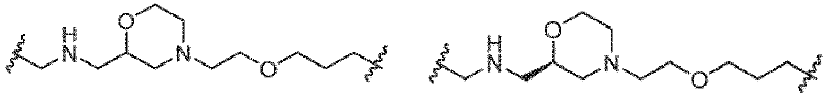

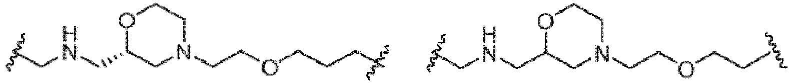

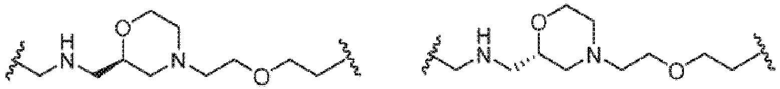

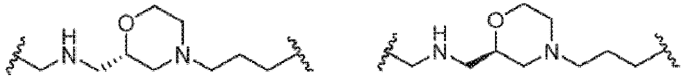

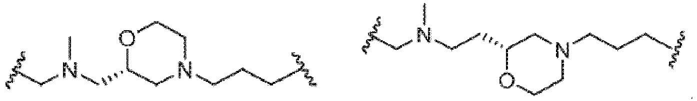

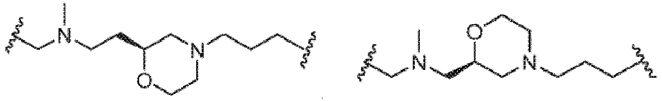

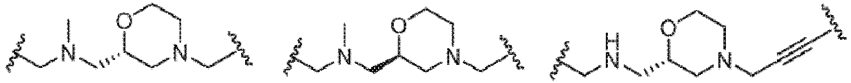

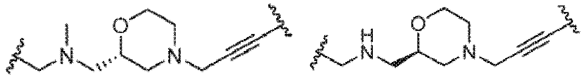

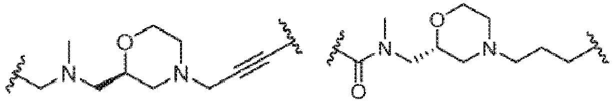

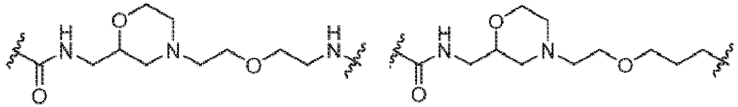

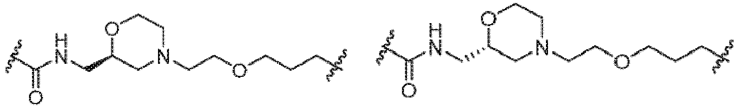

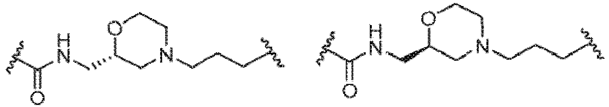

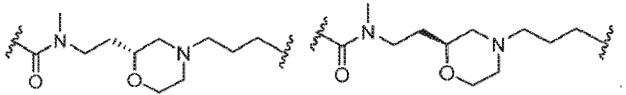

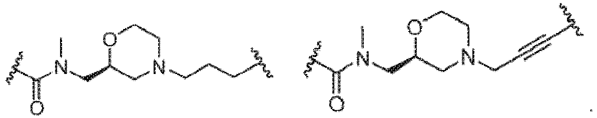

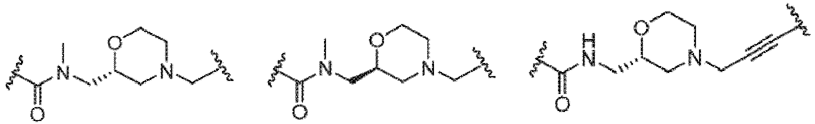

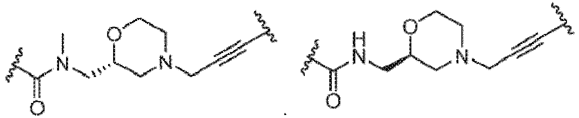

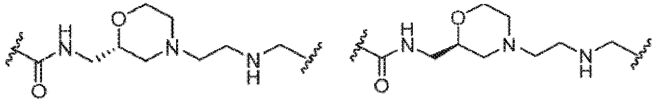

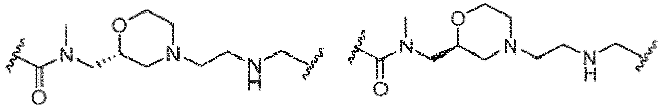

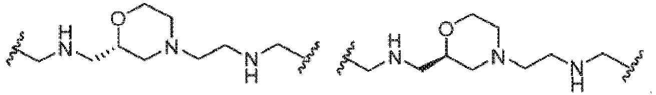

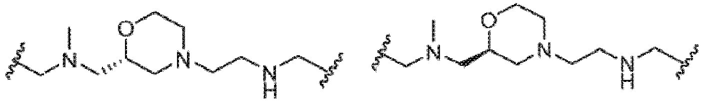

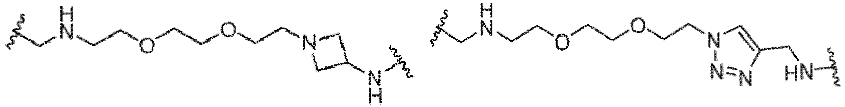

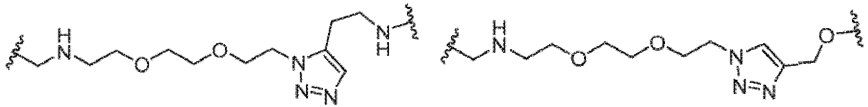

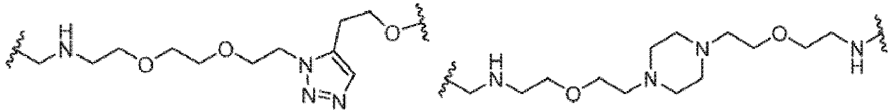

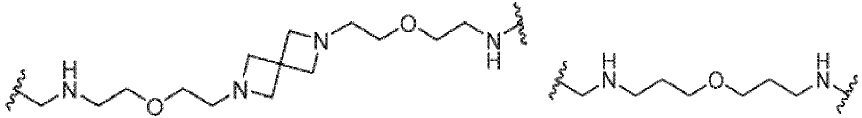

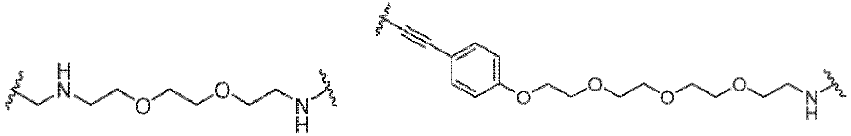

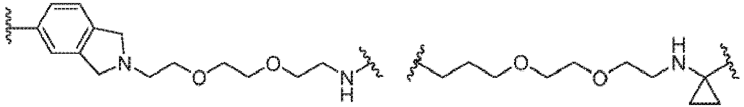

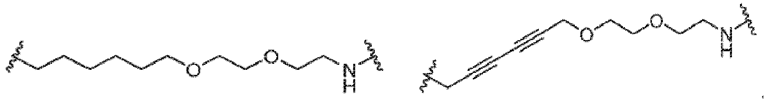

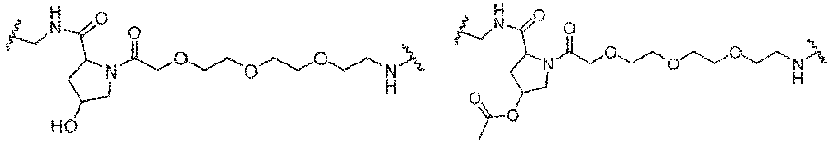

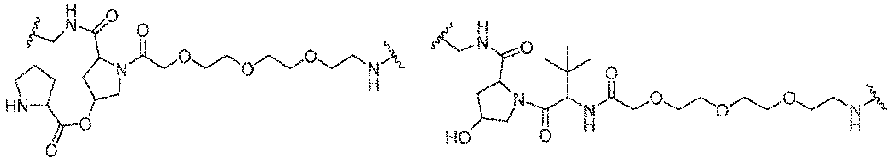

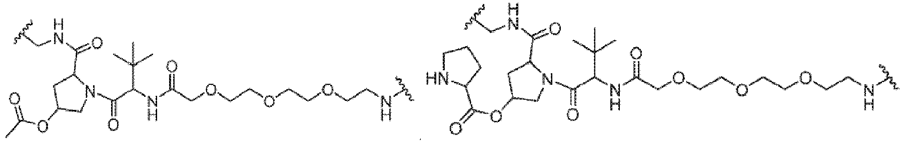

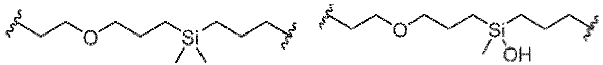

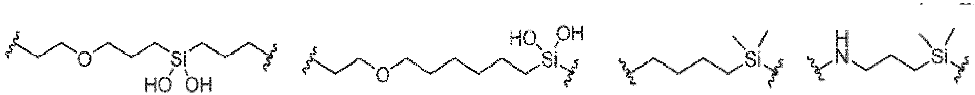

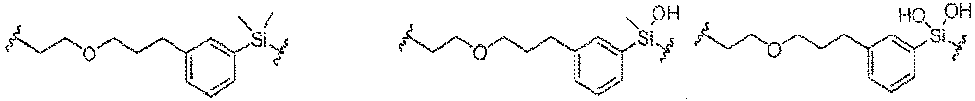

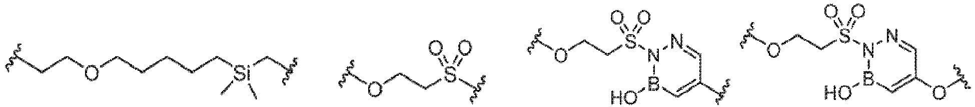

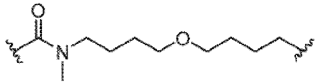

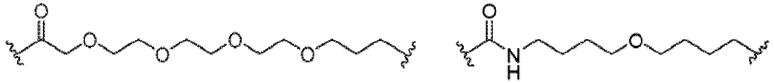

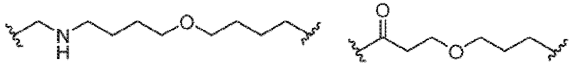

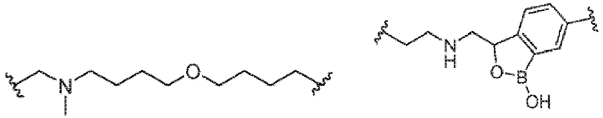

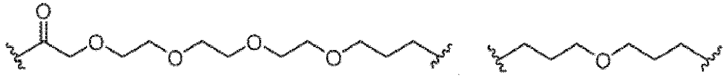

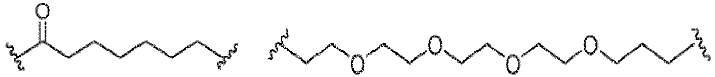

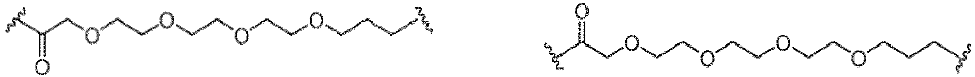

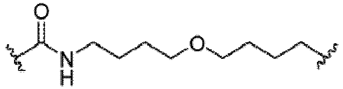

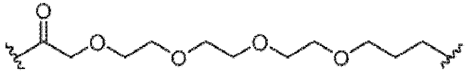

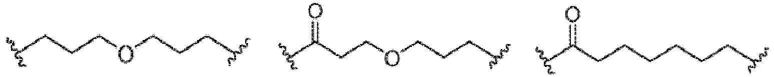

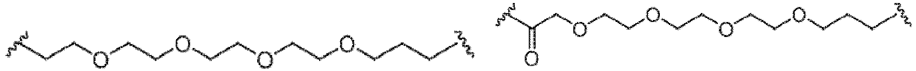

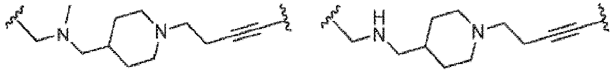

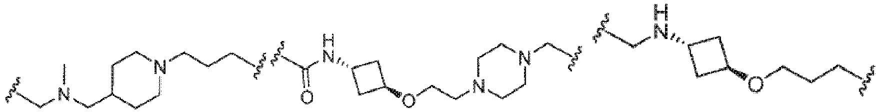

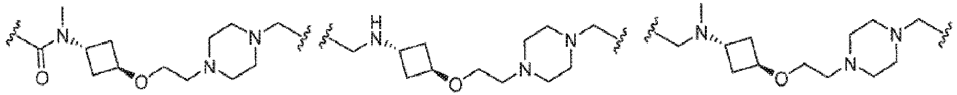

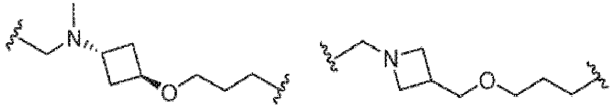

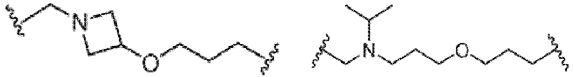

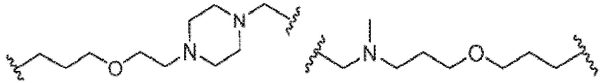

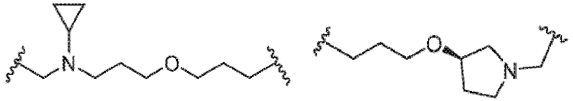

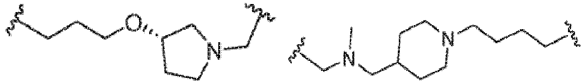

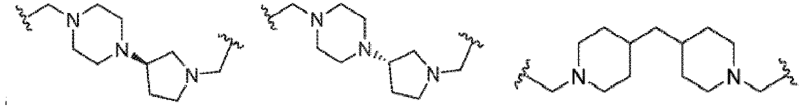

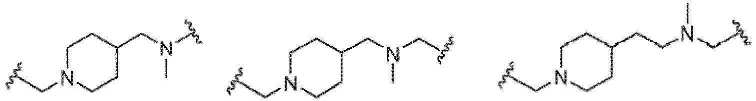

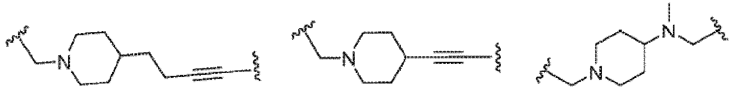

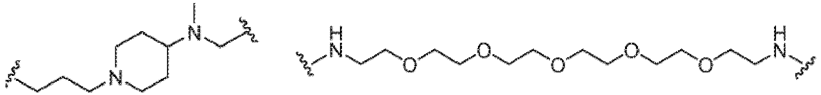

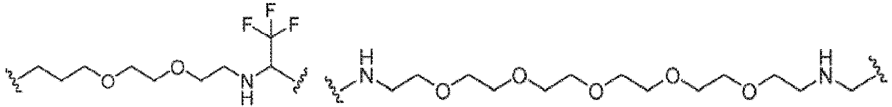

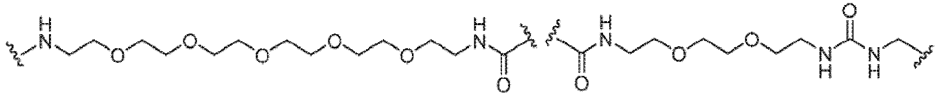

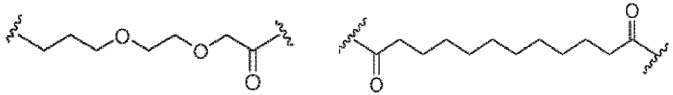

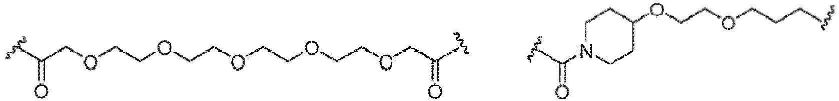

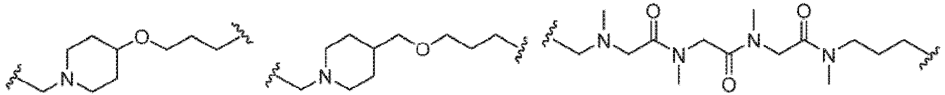

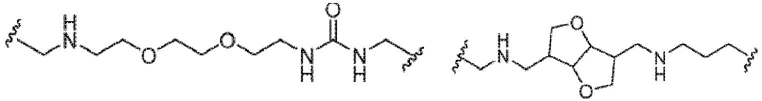

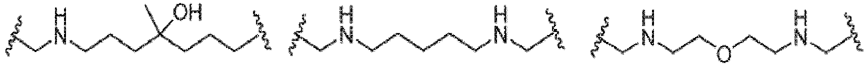

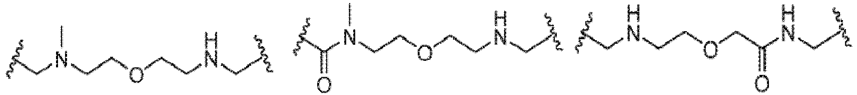

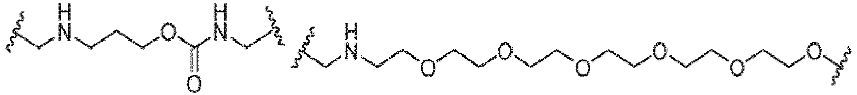

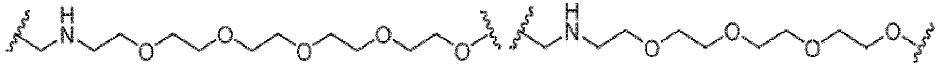

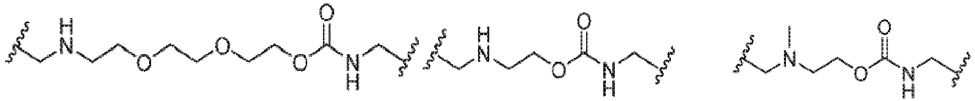

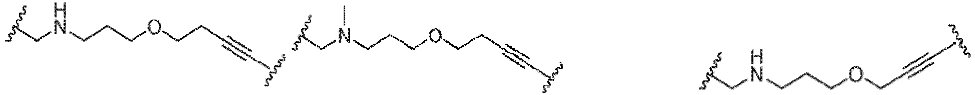

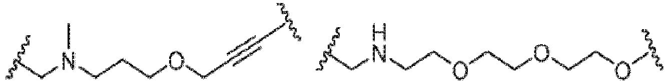

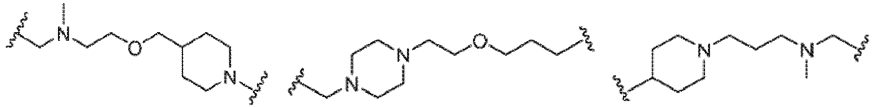

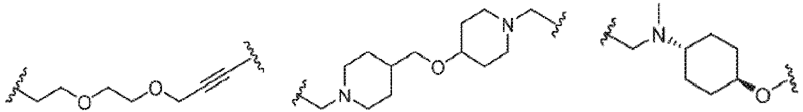

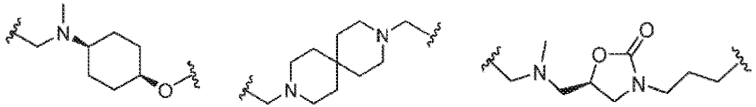

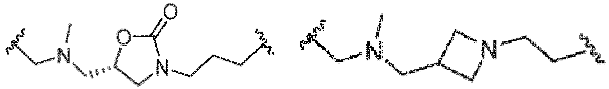

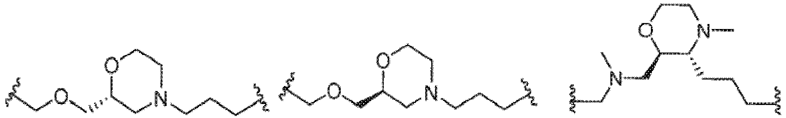

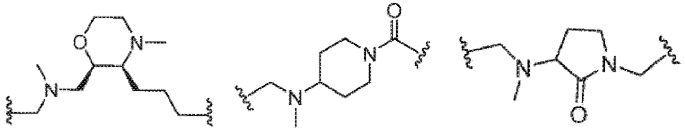

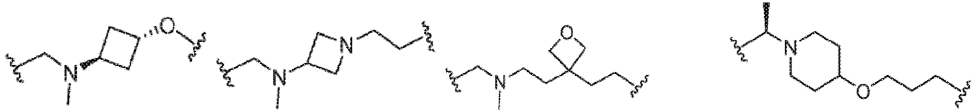

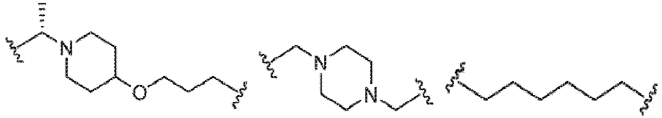

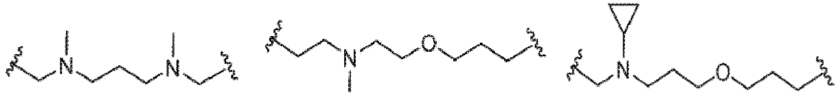

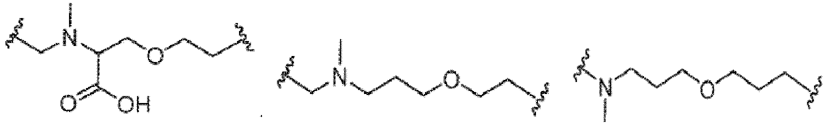

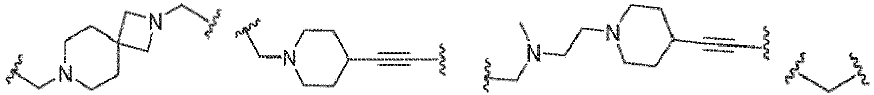

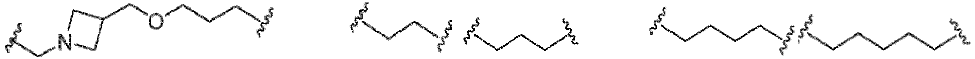

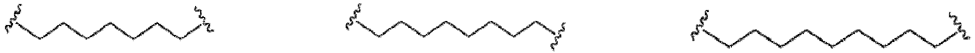

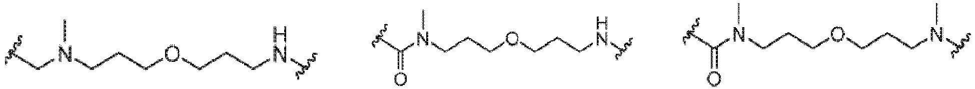

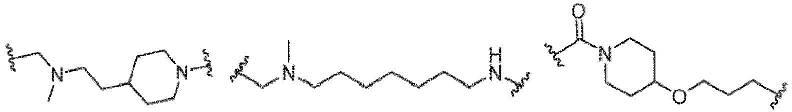

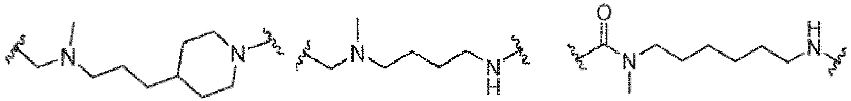

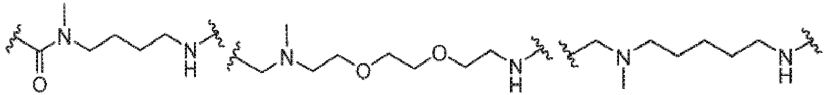

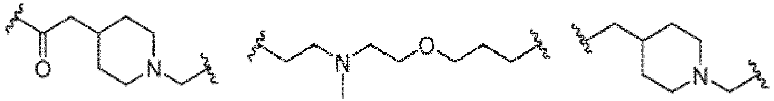

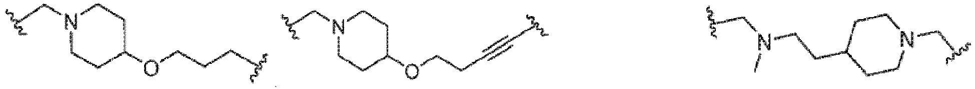

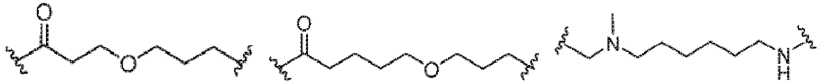

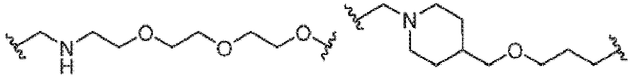

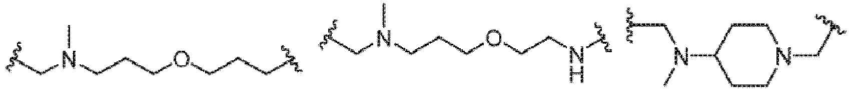

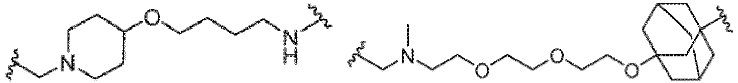

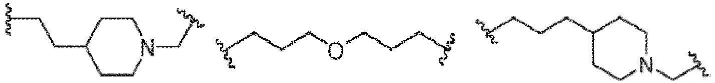

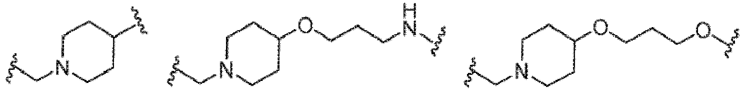

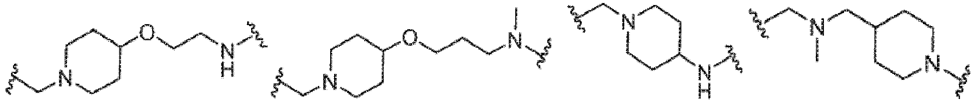

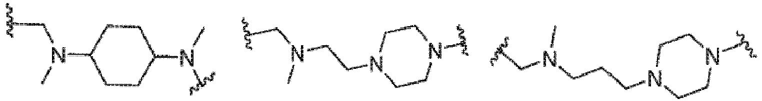

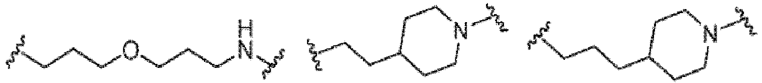

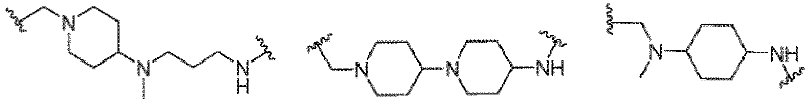

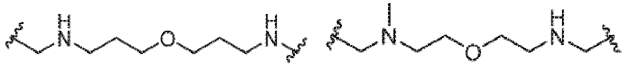

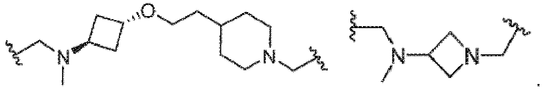

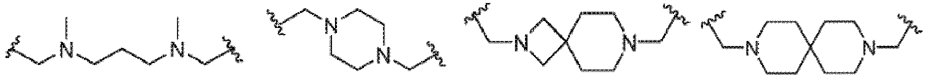

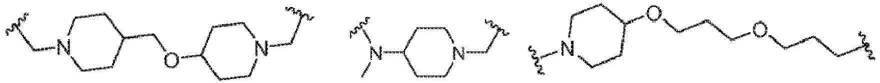

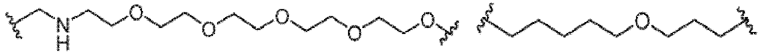

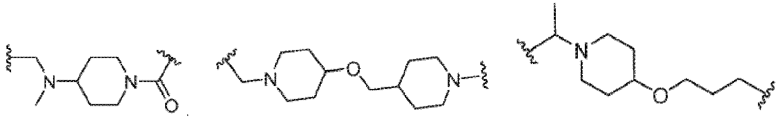

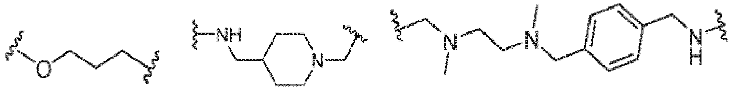

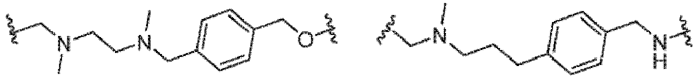

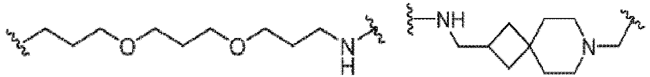

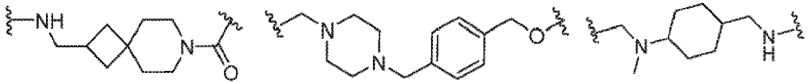

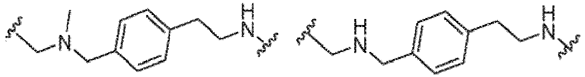

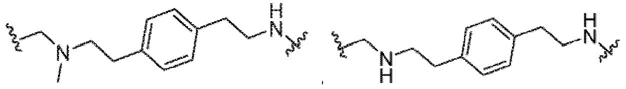

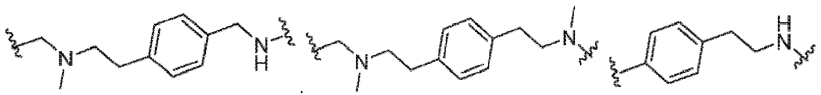

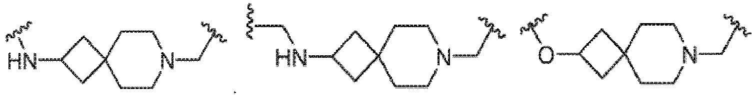

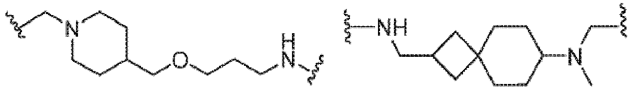

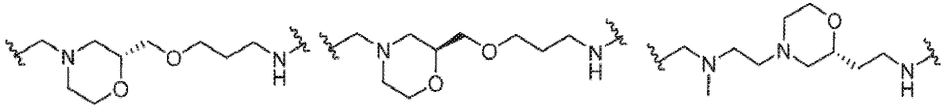

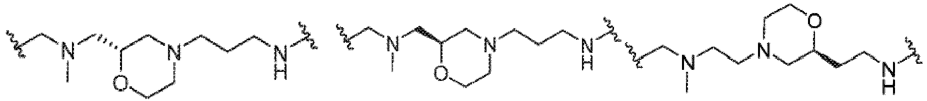

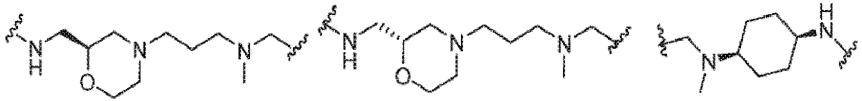

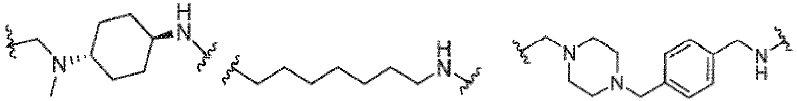

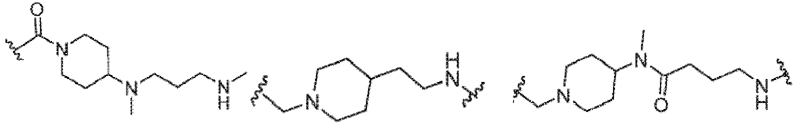

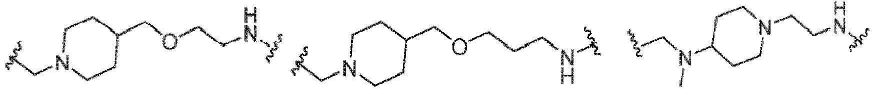

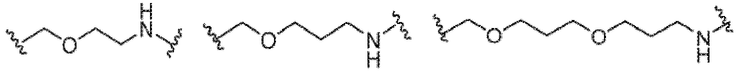

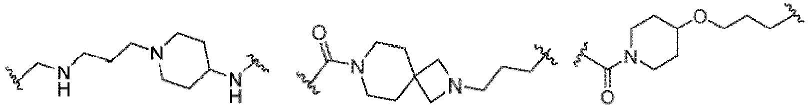

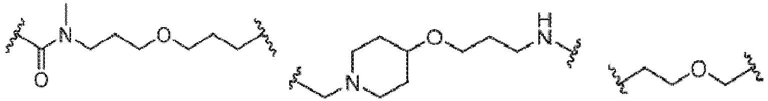

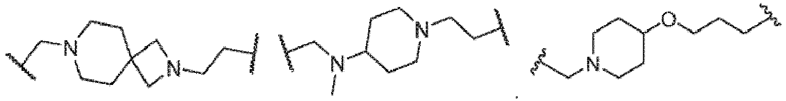

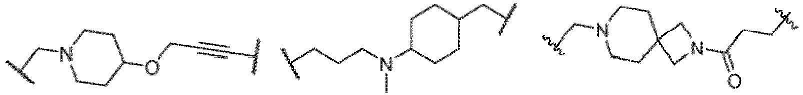

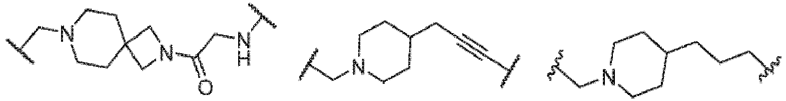

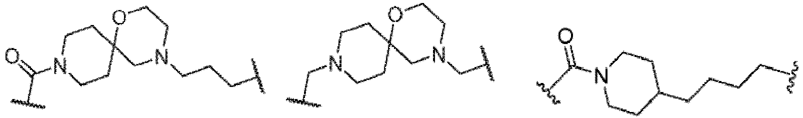

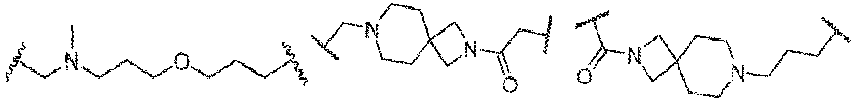

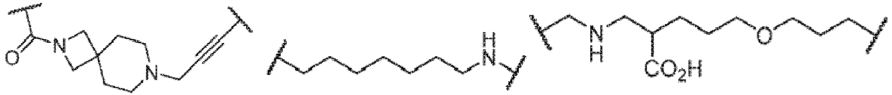

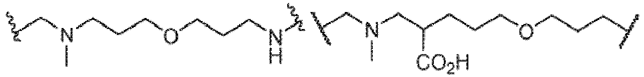

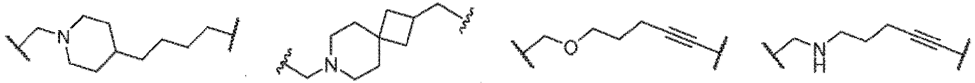

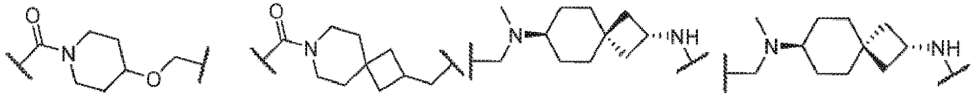

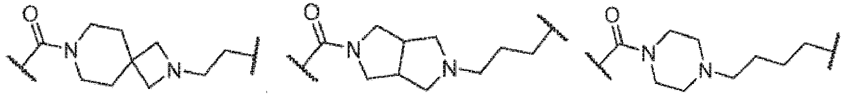

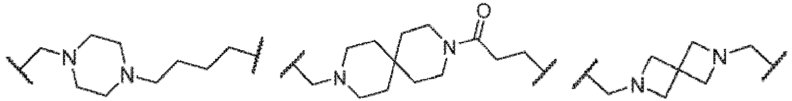

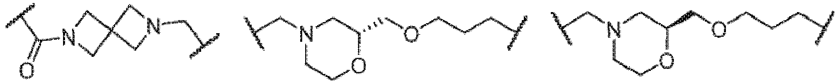

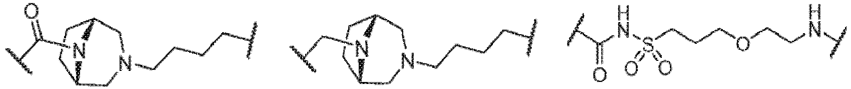

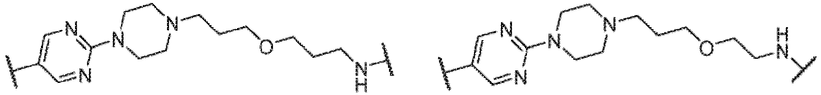

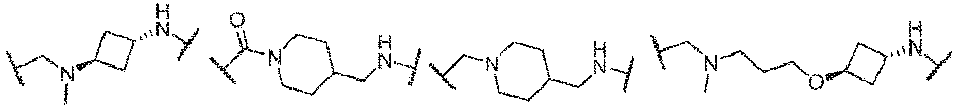

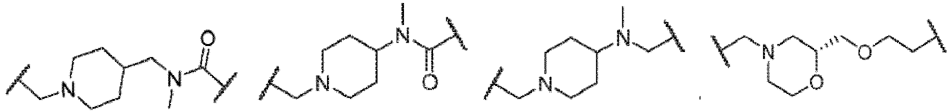

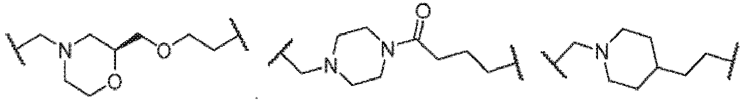

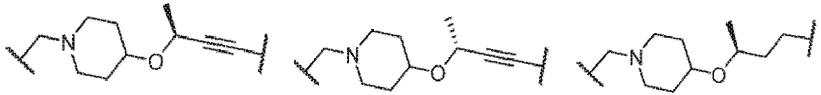

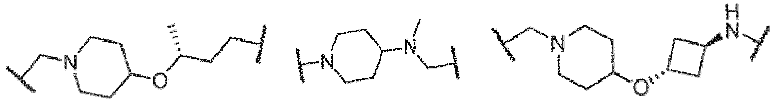

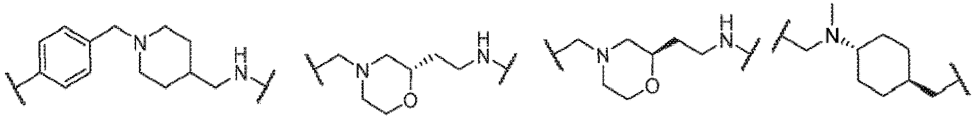

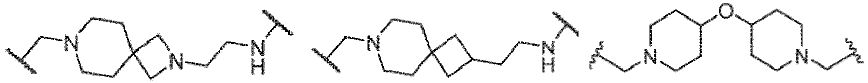

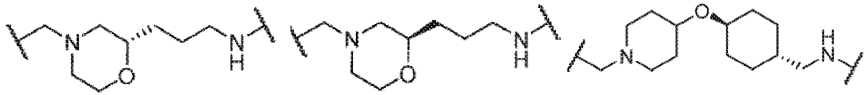

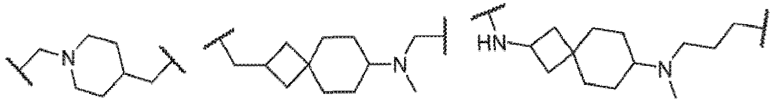

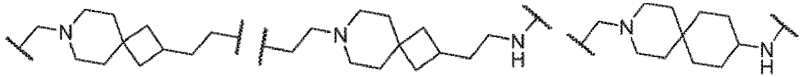

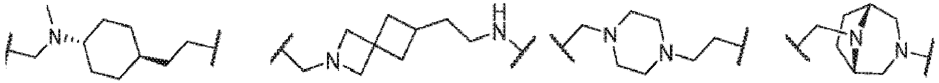

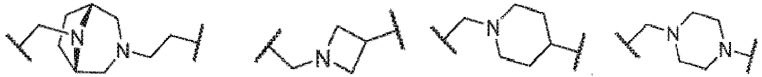

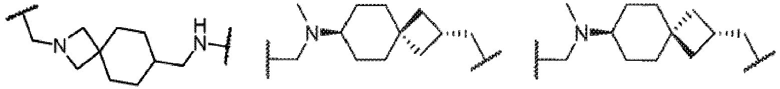

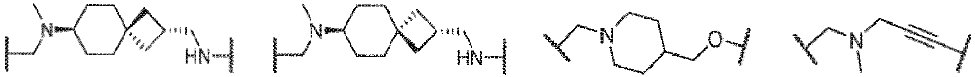

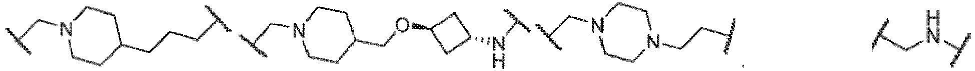

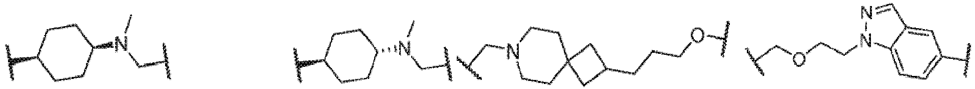

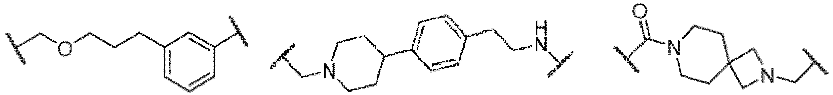

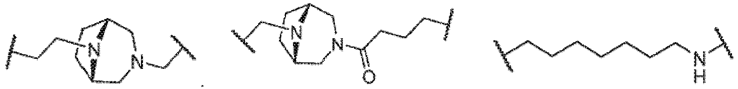

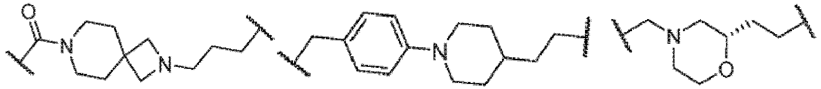

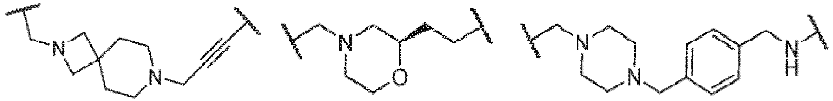

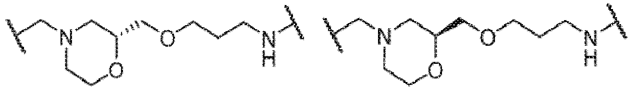

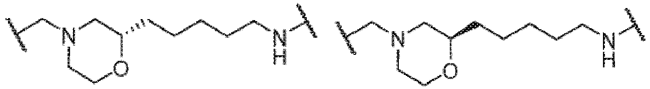

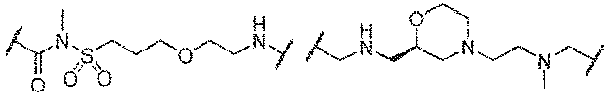

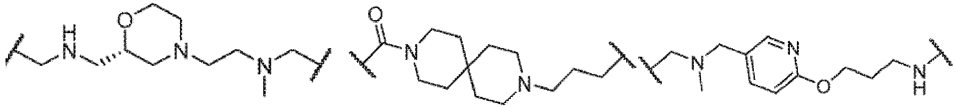

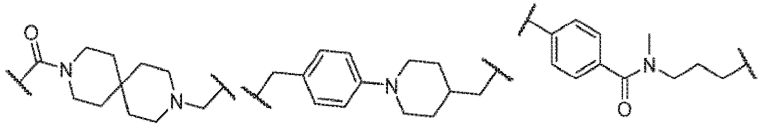

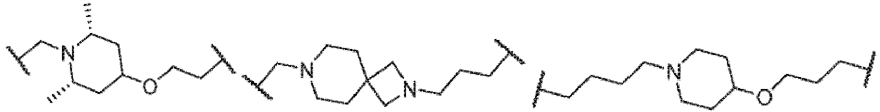

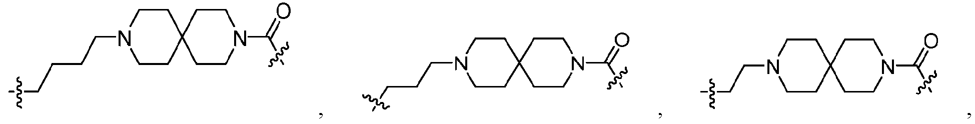

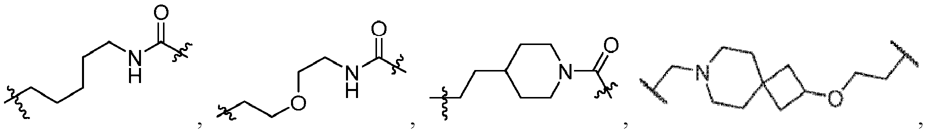

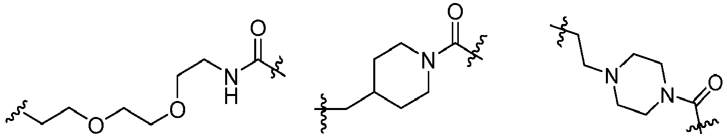

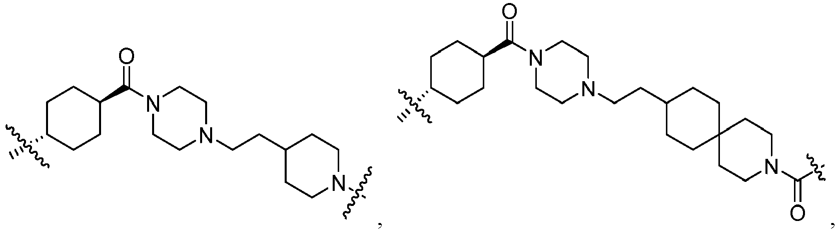

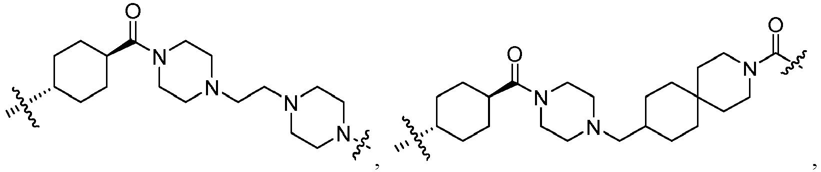

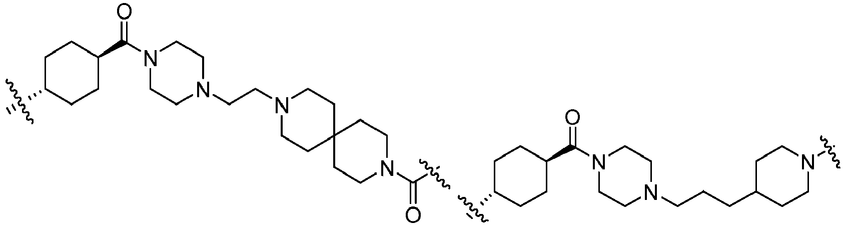

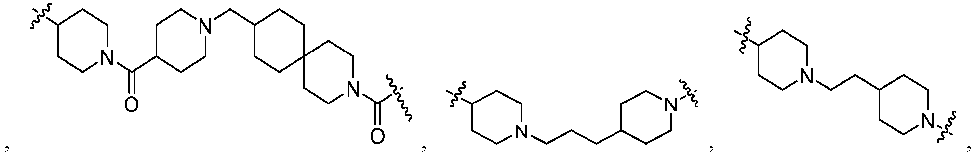

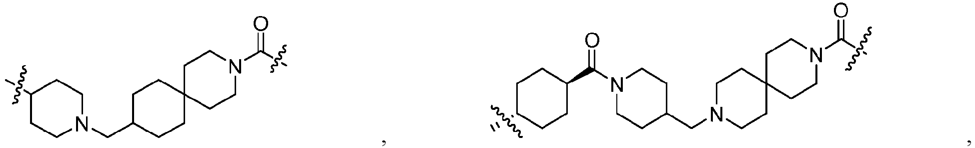

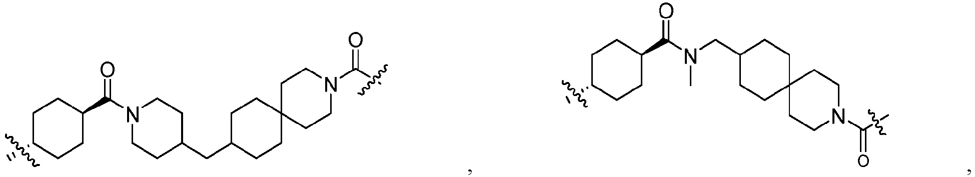

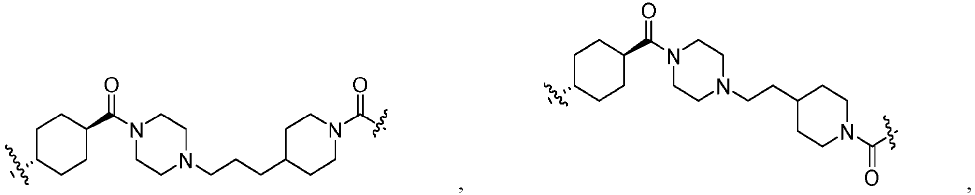

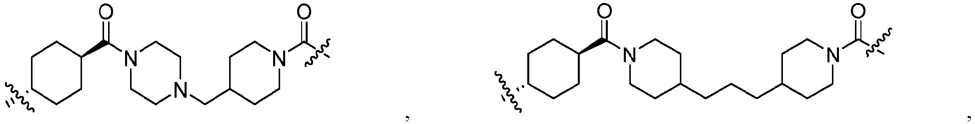

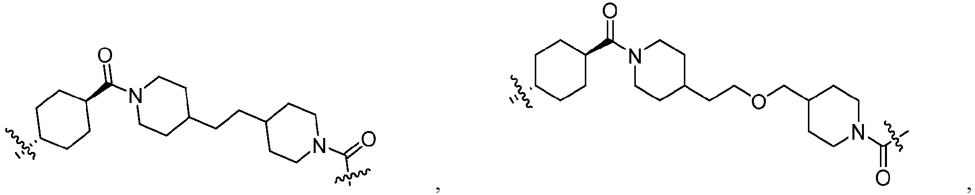

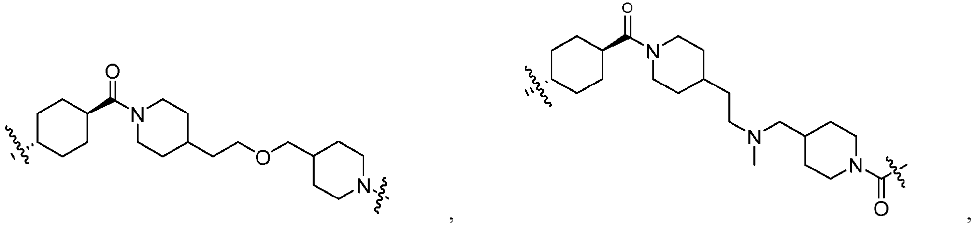

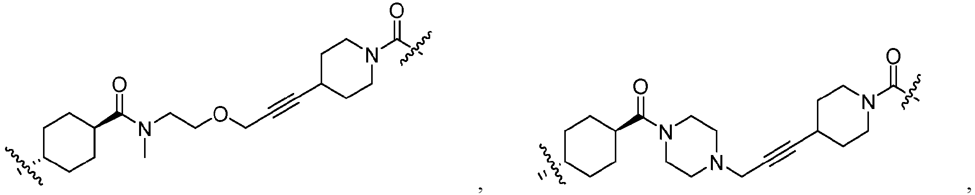

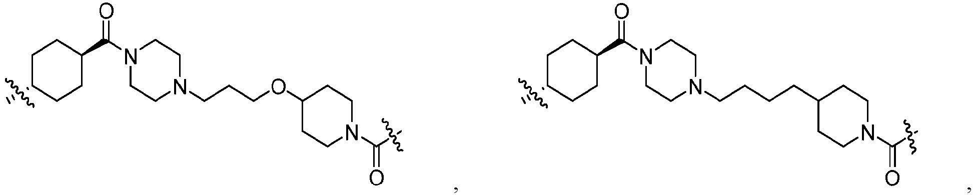

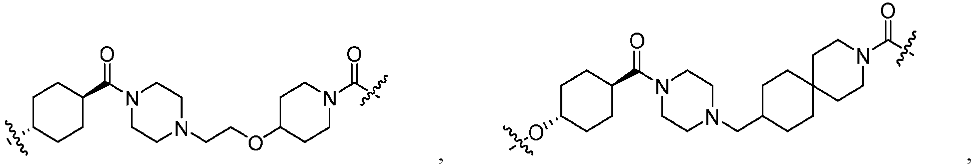

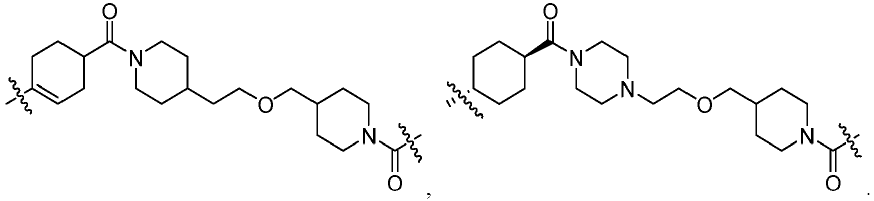

In certain embodiments of the present invention, L is any one of the following structures:

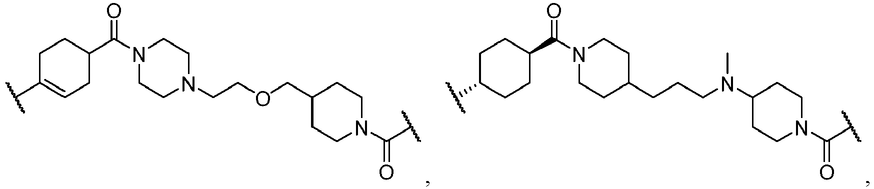

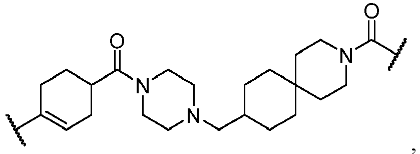

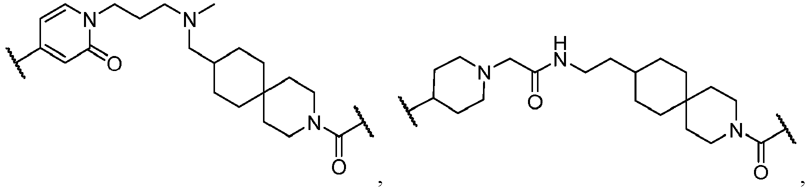

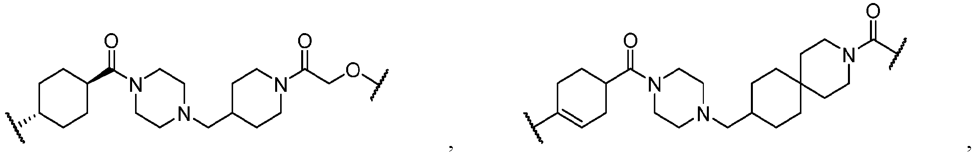

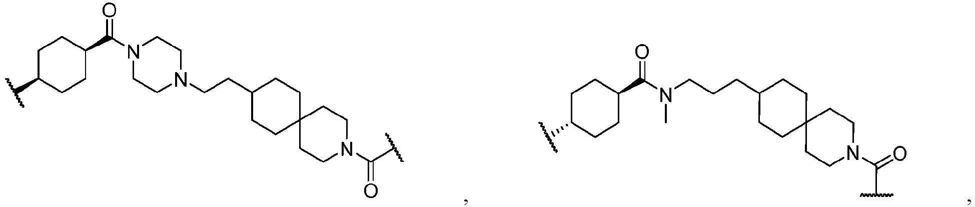

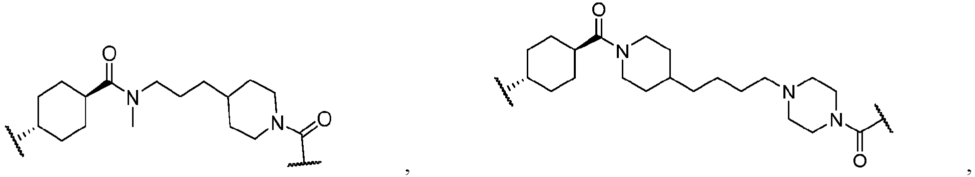

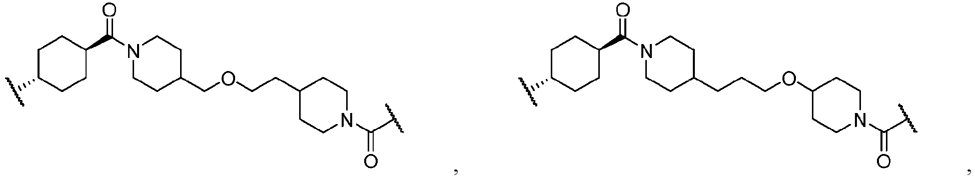

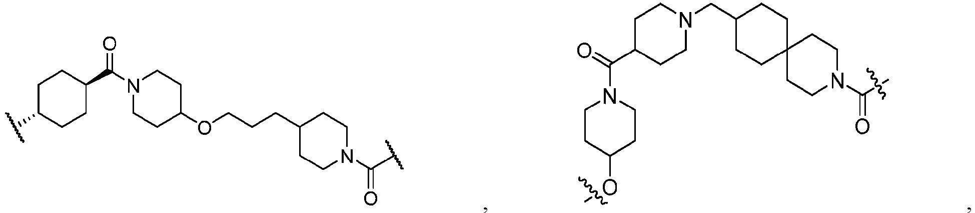

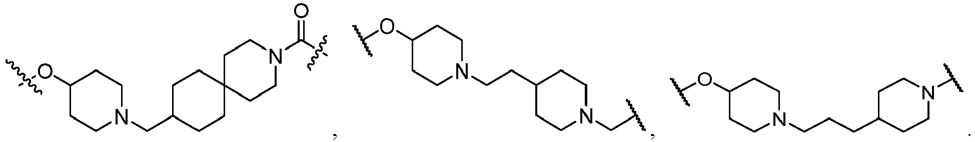

Preferably, in certain embodiments of the present invention, L is LA, wherein in LA, 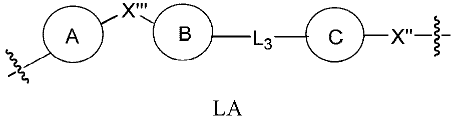
ring A is a bond, C₃-C₁₂ cycloalkylene (e.g., 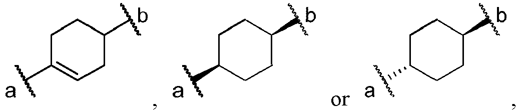 wherein end a is linked to S, and end b is linked to X‴) or 3- to 12-membered heterocycloalkylene containing 1-2 heteroatoms selected from N, O and S (e.g., 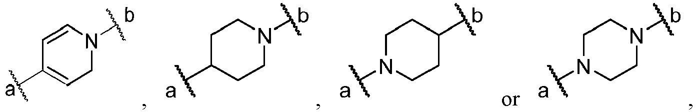
wherein end a is linked to S, and end b is linked to X‴), and the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl and -O-(C₁-C₆ alkyl);
ring B is a bond, C₃-C₁₂ cycloalkylene (e.g., 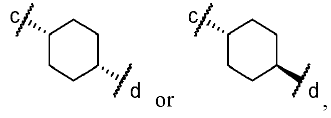 wherein end c is linked to X'", and end d is linked to L₃) or 3- to 12-membered heterocycloalkylene containing 1-2 heteroatoms selected from N, O and S (e.g., 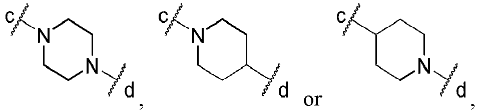 wherein end c is linked to X'", and end d is linked to L₃), and the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl and -O-(C₁-C₆ alkyl); ring C is C₃-C₁₂ cycloalkylene (e.g., 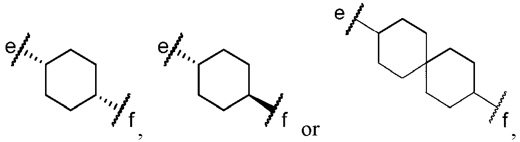 wherein end e is linked to L₃, and end f is linked to X") or 3- to 12-membered heterocycloalkylene containing 1-2 heteroatoms selected from N, O and S (e.g., 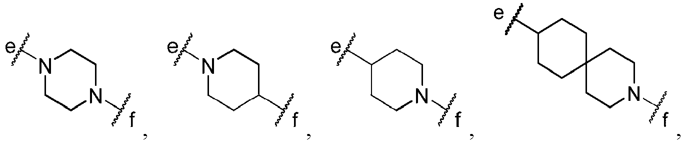 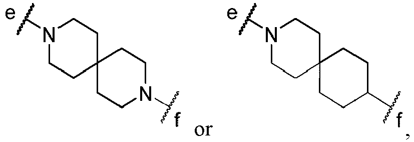 wherein end e is linked to L₃, and end f is linked to X"), and the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl and -O-(C₁-C₆ alkyl); X" is a bond, -NH-, -NCH₃-, -O-, -C(CH₃)₂-, -S-, -C=C-, -C≡C-, -CHF-, -CHCF₃-, -C(O)-, -S(O)-, -S(O)₂-, -C(O)O-, -OC(O)-, -C(O)NH-, -C(O)NCH₃-, -NHC(O)-, -NCH₃C(O)- or -C(O)CH₂O-;
L₃ is -(CH₂)ₖ, wherein one or two methylene groups in L₃ are optionally replaced by a group selected from -O-, - NH-, -C≡C-, -N(C₁-C₆ alkyl)-, -N(C₁-C₆ haloalkyl)-, -C(O)-, -N(C₁-C₆ hydroxyalkyl)- and -N(C₃-C₈ cycloalkyl)-, and k is 0, 1, 2, 3, 4, 5, 6 or 7;
X‴ is a bond, -NH-, -NCH₃-, -O-, -C(CH₃)₂-, -S-, -C=C-, -C≡C-, -CHF-, -CHCF₃-, -C(O)-, -S(O)-, -S(O)₂-, -C(O)O-, -OC(O)-, -C(O)NH-, -C(O)NCH₃-, -CH₂NCH₃-, -NHC(O)- or -NCH₃C(O)-.

In some embodiments, in LA:
ring A is a bond, C₃-C₁₂ cycloalkylene or 3- to 12-membered heterocycloalkylene containing 1-2 heteroatoms selected from N, O and S, and the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl and -O-(C₁-C₆ alkyl);
ring B is a bond, C₃-C₁₂ cycloalkylene or 3- to 12-membered heterocycloalkylene containing 1-2 heteroatoms selected from N, O and S, and the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl and -O-(C₁-C₆ alkyl);
ring C is C₃-C₁₂ cycloalkylene or 3- to 12-membered heterocycloalkylene containing 1-2 heteroatoms selected from N, O and S, and the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl and -O-(C₁-C₆ alkyl); X" is a bond, -NH-, -NCH₃-, -O-, -C(CH₃)₂-, -S-, -C=C-, -C≡C-, -CHF-, -CHCF₃-, -C(O)-, -S(O)-, -S(O)₂-, -C(O)O-, -OC(O)-, -C(O)NH-, -C(O)NCH₃-, -NHC(O)- or -NCH₃C(O)-;
L₃ is -(CH₂)ₖ, wherein one or two methylene groups in L₃ are optionally replaced by a group selected from -O-, - NH-, -N(C₁-C₆ alkyl)-, -N(C₁-C₆ haloalkyl)-, -N(C₁-C₆ hydroxyalkyl)- and -N(C₃-C₈ cycloalkyl)-, and k is 0, 1, 2, 3 or 4;
X‴ is a bond, -NH-, -NCH₃-, -O-, -C(CH₃)₂-, -S-, -C=C-, -C≡C-, -CHF-, -CHCF₃-, -C(O)-, -S(O)-, -S(O)₂-, -C(O)O-, -OC(O)-, -C(O)NH-, -C(O)NCH₃-, -NHC(O)- or -NCH₃C(O)-.

In certain embodiments of the present invention, in LA, L₃ is -(CH₂)ₖ, wherein one or two methylene groups in L₃ are optionally replaced by a group selected from -O-, -NH-, -C≡C-, -N(C₁-C₆ alkyl)-, -N(C₁-C₆ haloalkyl)-, -N(C₁-C₆ hydroxyalkyl)- and -N(C₃-C₈ cycloalkyl)-, and k is 0, 1, 2, 3, 4, 5, 6 or 7.

Preferably, in certain embodiments of the present invention, in LA, ring A is a bond.

Preferably, in certain embodiments of the present invention, in LA, ring A is 3- to 7-membered saturated or partially unsaturated cycloalkylene, 4- to 11-membered saturated or partially unsaturated spirocycloalkylene, 4- to 11-membered saturated or partially unsaturated fused cycloalkylene, 8- to 10-membered bicyclic saturated or partially unsaturated cycloalkylene, 4- to 7-membered saturated or partially unsaturated heterocycloalkylene having 1-2 heteroatoms independently selected from nitrogen, oxygen and sulfur, 4- to 11-membered saturated or partially unsaturated spiroheterocycloalkylene having 1-2 heteroatoms independently selected from nitrogen, oxygen and sulfur, 4- to 11-membered saturated or partially unsaturated fused heterocycloalkylene having 1-2 heteroatoms independently selected from nitrogen, oxygen and sulfur or 8- to 10-membered bicyclic saturated or partially unsaturated heterocycloalkylene having 1-2 heteroatoms independently selected from nitrogen, oxygen and sulfur. Preferably, in certain embodiments of the present invention, in LA, ring A is 3- to 6-membered saturated cycloalkylene or 4- to 7-membered saturated monocyclic heterocycloalkylene having 1 or 2 heteroatoms independently selected from nitrogen.

Preferably, in certain embodiments of the present invention, in LA, ring A is cyclohexylene (e.g., cyclohex-1,4-diyl, e.g., *trans* cyclohex-1,4-diyl), piperidinylidene (e.g., piperidine-1,4-diyl) or piperazinylidene (e.g., piperazine-1,4-diyl).

In certain embodiments of the present invention, in LA, ring A is 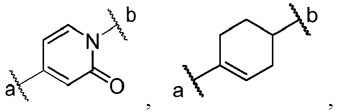 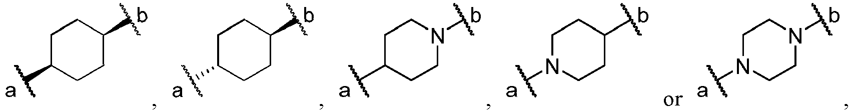 wherein end a is linked to S, and end b is linked to X‴.

In certain embodiments of the present invention, in LA, ring A is 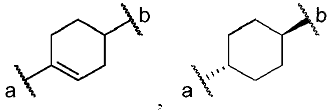 or 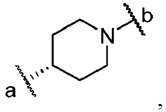 wherein end a is linked to S, and end b is linked to X'".

In certain embodiments of the present invention, in LA, ring A is 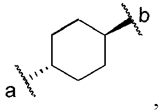 wherein end a is linked to S, and end b is linked to X'".

Preferably, in certain embodiments of the present invention, in LA, ring B is a bond.

Preferably, in certain embodiments of the present invention, in LA, ring B is 3- to 7-membered saturated or partially unsaturated cycloalkylene, 4- to 11-membered saturated or partially unsaturated spirocycloalkylene, 4- to 11-membered saturated or partially unsaturated fused cycloalkylene, 8- to 10-membered bicyclic saturated or partially unsaturated cycloalkylene, 4- to 7-membered saturated or partially unsaturated heterocycloalkylene having 1-2 heteroatoms independently selected from nitrogen, oxygen and sulfur, 4- to 11-membered saturated or partially unsaturated spiroheterocycloalkylene having 1-2 heteroatoms independently selected from nitrogen, oxygen and sulfur, 4- to 11-membered saturated or partially unsaturated fused heterocycloalkylene having 1-2 heteroatoms independently selected from nitrogen, oxygen and sulfur or 8- to 10-membered bicyclic saturated or partially unsaturated heterocycloalkylene having 1-2 heteroatoms independently selected from nitrogen, oxygen and sulfur. Preferably, in certain embodiments of the present invention, in LA, ring B is 4- to 7-membered saturated monocyclic heterocycloalkylene containing 1 or 2 nitrogen heteroatoms or 7- to 11-membered spiroheterocycloalkylene or fused heterocycloalkylene containing 1 or 2 nitrogen heteroatoms.

Preferably, in certain embodiments of the present invention, in LA, ring B is piperidinylidene (e.g., piperidine-1,4-diyl) or piperazinylidene (e.g., piperazine-1,4-diyl).

In certain embodiments of the present invention, in LA, ring B is 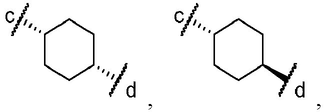 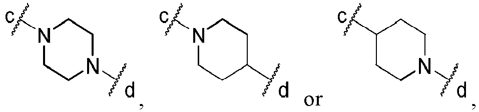 wherein end c is linked to X'", and end d is linked to L₃.

Preferably, in certain embodiments of the present invention, ring B is 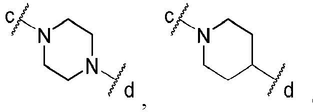 or 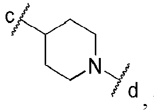 wherein end c is linked to X‴, and end d is linked to L₃.

Preferably, in certain embodiments of the present invention, in LA, ring C is 3- to 7-membered saturated or partially unsaturated cycloalkylene, 4- to 11-membered saturated or partially unsaturated spirocycloalkylene, 4- to 11-membered saturated or partially unsaturated fused cycloalkylene, 8- to 10-membered bicyclic saturated or partially unsaturated cycloalkylene, 4- to 7-membered saturated or partially unsaturated heterocycloalkylene having 1-2 heteroatoms independently selected from nitrogen, oxygen and sulfur, 4- to 11-membered saturated or partially unsaturated spiroheterocycloalkylene having 1-2 heteroatoms independently selected from nitrogen, oxygen and sulfur, 4- to 11-membered saturated or partially unsaturated fused heterocycloalkylene having 1-2 heteroatoms independently selected from nitrogen, oxygen and sulfur or 8- to 10-membered bicyclic saturated or partially unsaturated heterocycloalkylene having 1-2 heteroatoms independently selected from nitrogen, oxygen and sulfur. Preferably, in certain embodiments of the present invention, in LA, ring C is 4- to 7-membered saturated monocyclic heterocycloalkylene containing 1 or 2 nitrogen heteroatoms or 7- to 11-membered spiroheterocycloalkylene or fused heterocycloalkylene containing 1 or 2 nitrogen heteroatoms.

Preferably, in certain embodiments of the present invention, in LA, ring C is piperidinylidene (e.g., piperidine-1,4-diyl), piperazinylidene (e.g., piperazine-1,4-diyl),

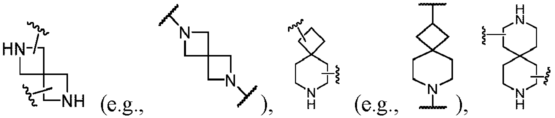

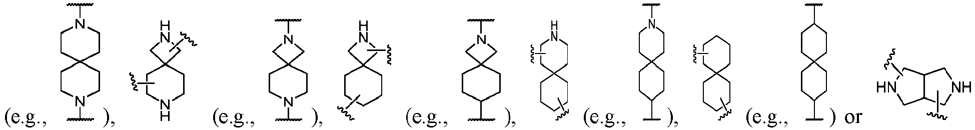

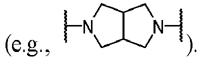

In certain embodiments of the present invention, in LA, ring C is 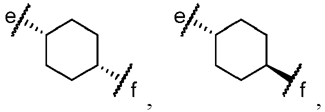 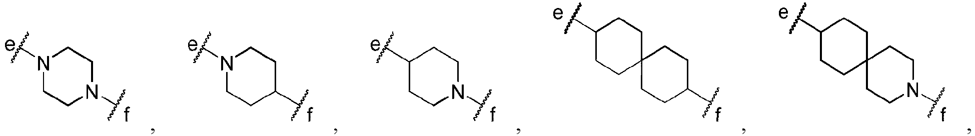 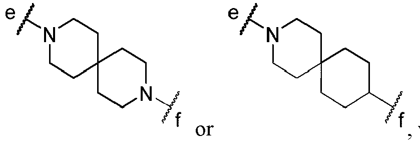 wherein end e is linked to L₃, and end f is linked to X".

In certain embodiments of the present invention, in LA, ring C is 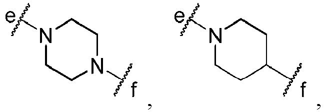 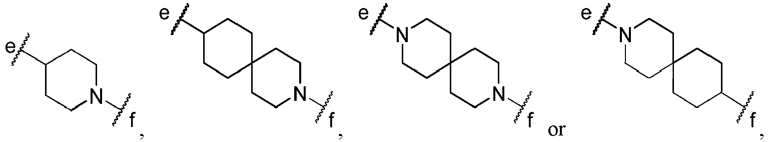 wherein end e is linked to L₃, and end f is linked to X".

Preferably, in certain embodiments of the present invention, in LA, ring A is 3- to 6-membered saturated cycloalkylene; ring B is 4- to 7-membered saturated monocyclic heterocycloalkylene containing 1 or 2 nitrogen heteroatoms; ring C is 4- to 7-membered saturated monocyclic heterocycloalkylene containing 1 or 2 nitrogen heteroatoms or 7- to 11-membered spiroheterocycloalkylene or fused heterocycloalkylene containing 1 or 2 nitrogen heteroatoms; X" is a bond or -C(O)-; L₃ is -(CH₂)ₖ, wherein k is 1, 2, 3, 4 or 5 (e.g., k is 1, 2, 3 or 4).

Preferably, in certain embodiments of the present invention, in LA, X" is a bond or -C(O)-.

Preferably, in certain embodiments of the present invention, in LA, X" is a bond.

Preferably, in certain embodiments of the present invention, in LA, X" is -C(O)-.

Preferably, in certain embodiments of the present invention, in LA, X" is -C(O)CH₂O-.

Preferably, in certain embodiments of the present invention, in LA, X‴ is a bond or -C(O)-.

Preferably, in certain embodiments of the present invention, in LA, X‴ is a bond.

Preferably, in certain embodiments of the present invention, in LA, X‴ is -C(O)-.

Preferably, in certain embodiments of the present invention, in LA, k is 1, 2, 3, 4 or 5.

Preferably, in certain embodiments of the present invention, in LA, L₃ is -(CH₂)ₖ, wherein k is 1, 2, 3, 4 or 5. Preferably, in certain embodiments of the present invention, in LA, L₃ is -(CH₂)ₖ, wherein one or two methylene groups in L₃ are optionally replaced by -O-, -NH-, -C≡C- or -N(C₁-C₆ alkyl)- (e.g., -N(CH₃)-), and k is 1, 2, 3, 4 or 5.

Preferably, in certain embodiments of the present invention, in LA, k is 1, 2, 3 or 4. Preferably, in certain embodiments of the present invention, in LA, L₃ is -(CH₂)ₖ-, wherein one or two of CH₂ contained in L₃ are each independently optionally replaced by -O-, -NH- or -N(C₁-C₆ alkyl)- (e.g., -N(CH₃)-), or one of -CH₂CH₂- contained in L₃ is optionally replaced by -C≡C-, k is 1, 2, 3 or 4.

Preferably, in certain embodiments of the present invention, in LA, L₃ is -(CH₂)ₖ-, wherein one methylene group in L₃ is optionally replaced by a group, which is -O-, -NH- or -N(C₁-C₆ alkyl)- (e.g., -N(CH₃)-); k is 1, 2, 3 or 4. Preferably, in certain embodiments of the present invention, LA is LA-1: 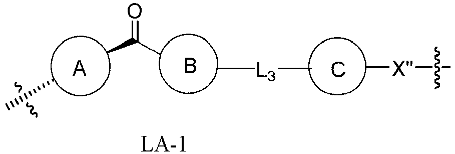 wherein ring A, ring B, ring C, L₃ and X" are as defined and described in LA.

In certain embodiments of the present invention, LA is LA-2: 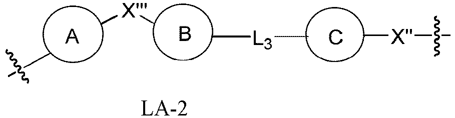 wherein ring A is 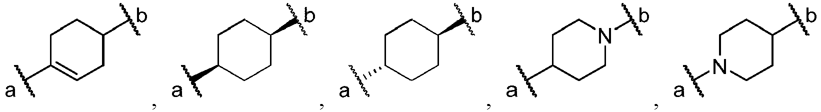 or 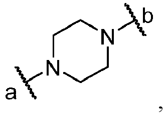 , wherein end a is linked to S, and end b is linked to X‴; 1, 2, 3 or 4 hydrogen atoms in ring A are optionally substituted with F;
X‴ is -C(O)-;
ring B is 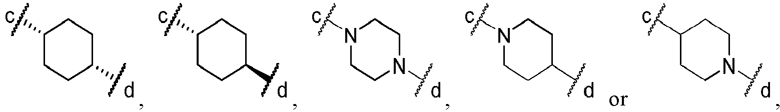 wherein end c is linked to X‴, and end d is linked to L₃; 1, 2, 3 or 4 hydrogen atoms in ring B are optionally substituted with F;
L₃ is -(CH₂)ₖ-, wherein one or two of CH₂ contained in L₃ are each independently optionally replaced by -O-, -NH- or -N(C₁-C₆ alkyl)- (e.g., -N(CH₃)-), or one of -CH₂CH₂- contained in L₃ is optionally replaced by -C≡C-, k is 1, 2, 3 or 4;
ring C is 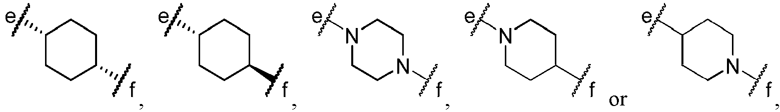 wherein end e is linked to L₃, and end f is linked to X"; 1, 2, 3 or 4 hydrogen atoms in ring C are optionally substituted with F; X" is -C(O)-.

Preferably, in certain embodiments of the present invention, in LA, LA-1 and LA-2, ring A is 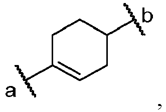 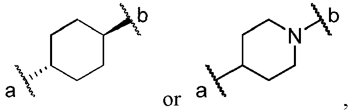 wherein end a is linked to S, and end b is linked to X‴.

Preferably, in certain embodiments of the present invention, ring B is 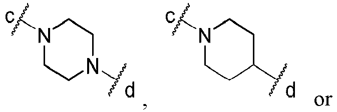 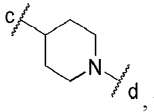 wherein end c is linked to X‴, and end d is linked to L₃.

In certain embodiments of the present invention, in LA, LA-1 and LA-2, L₃ is -(CH₂)ₖ-, wherein one or two of CH₂ contained in L₃ are each independently optionally replaced by -O-, -NH- or -N(CH₃)-; k is 1, 2, 3 or 4.

In certain embodiments of the present invention, in LA, LA-1 and LA-2, L₃ is -(CH₂)ₖ-, wherein one or two of CH₂ contained in L₃ are each independently optionally replaced by -O-; k is 1, 2, 3 or 4.

Preferably, in certain embodiments of the present invention, in LA, LA-1 and LA-2, k is 3 or 4, preferably 4. Preferably, in certain embodiments of the present invention, in LA, LA-1 and LA-2, L₃ is -(CH₂)ₖ-, wherein one of CH₂ contained in L₃ is optionally replaced by -O-; k is 4.

In certain embodiments of the present invention, in LA, LA-1 and LA-2, L₃ may be -CH₂-, -(CH₂)₂-, -(CH₂)₃-, - (CH₂)₄-, 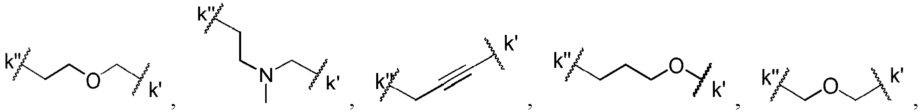 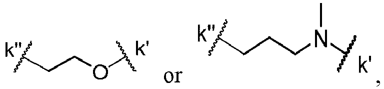 wherein end k" is linked to ring B, and end k' is linked to ring C.

Preferably, in certain embodiments of the present invention, in LA, LA-1 and LA-2, L₃ may be -(CH₂)₄-,

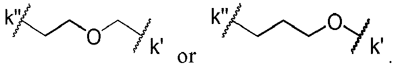

Preferably, in certain embodiments of the present invention, in LA, LA-1 and LA-2, ring C is 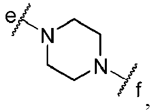 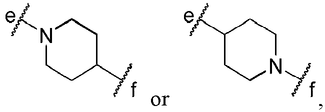 wherein end e is linked to L₃, and end f is linked to X".

In certain embodiments of the present invention, LA-2 is any one of the following structures:

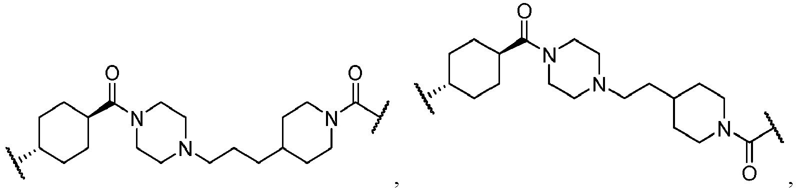

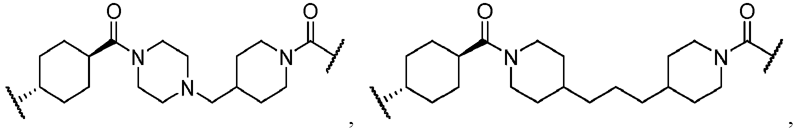

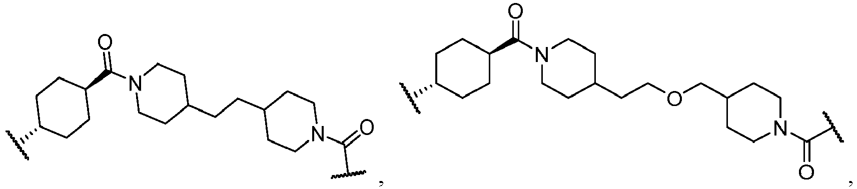

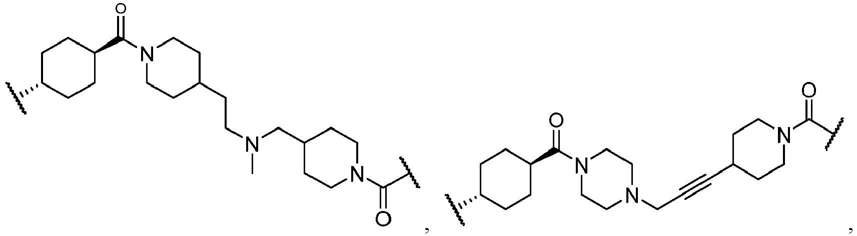

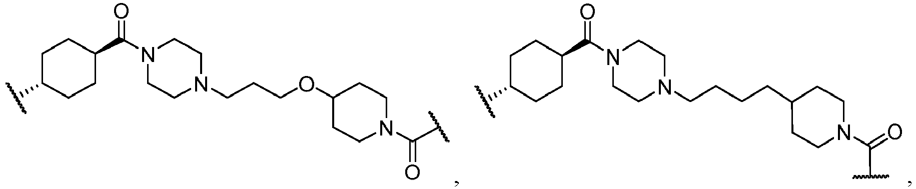

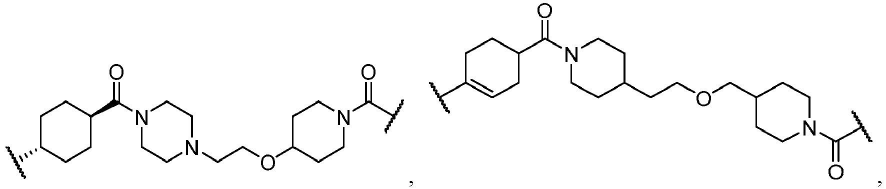

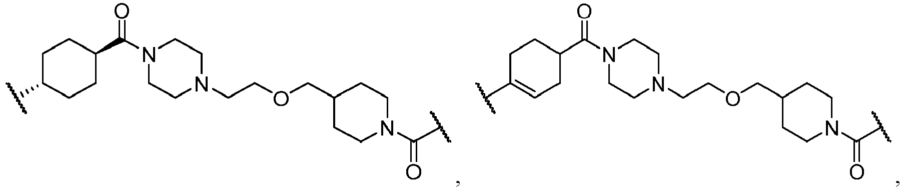

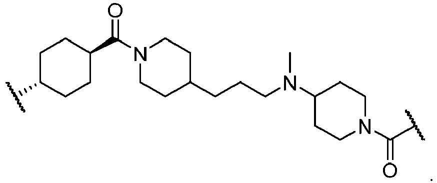

Preferably, in certain embodiments of the present invention, LA-2 is any one of the following structures:

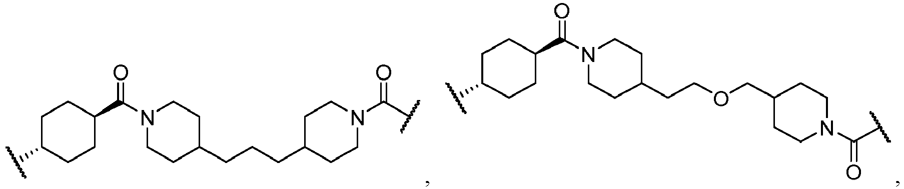

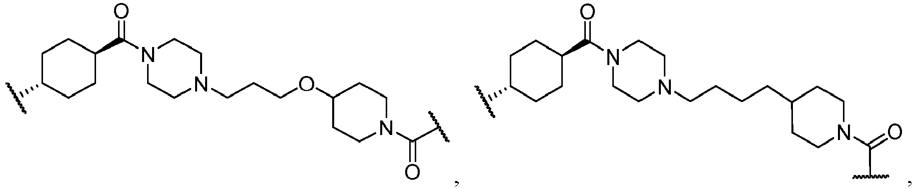

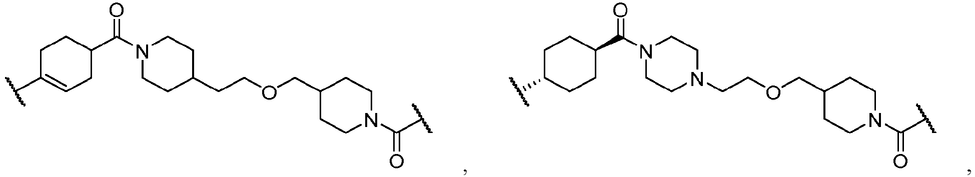

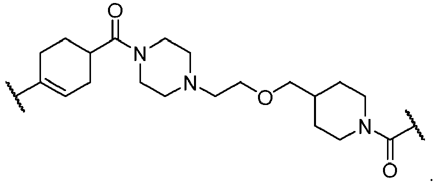

In certain embodiments of the present invention, LA is LA-3: 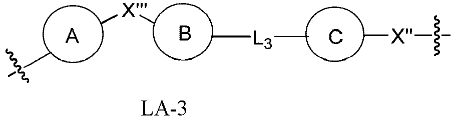 wherein ring A is 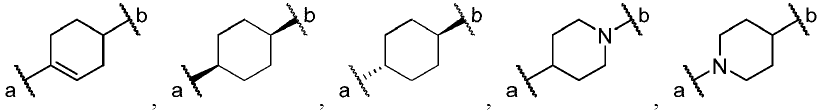 or 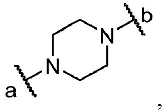 wherein end a is linked to S, and end b is linked to X‴; 1, 2, 3 or 4 hydrogen atoms in ring A are optionally substituted with F;
X‴ is -C(O)-;
ring B is 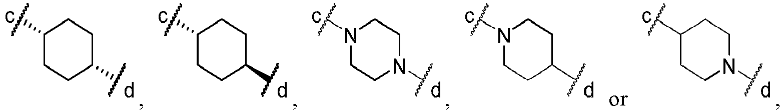 wherein end c is linked to X‴, and end d is linked to L₃; 1, 2, 3 or 4 hydrogen atoms in ring B are optionally substituted with F;
L₃ is -(CH₂)ₖ-, wherein one of CH₂ contained in L₃ is optionally replaced by -O-, -NH- or -N(C₁-C₆ alkyl)- (e.g., - N(CH₃)-); k is 1 or 2;
ring C is 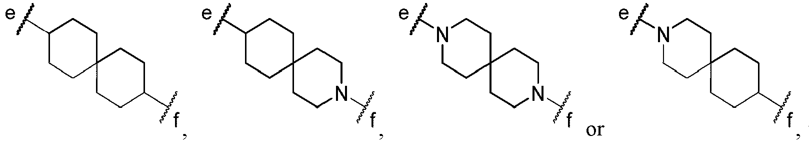 wherein end e is linked to L₃, and end f is linked to X"; 1, 2, 3 or 4 hydrogen atoms in ring C are optionally substituted with F; X" is -C(O)-.

Preferably, in certain embodiments of the present invention, in LA, LA-1 and LA-3, ring A is 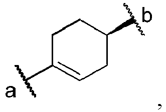 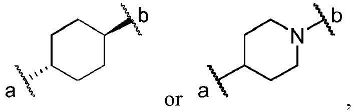 preferably 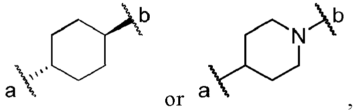 wherein end a is linked to S, and end b is linked to X‴.

Preferably, in certain embodiments of the present invention, in LA, LA-1 and LA-3, ring B is 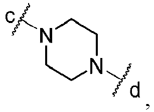 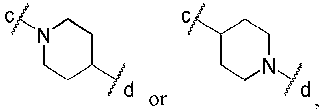 wherein end c is linked to X‴, and end d is linked to L₃.

Preferably, in certain embodiments of the present invention, in LA, LA-1 and LA-3, L₃ is -(CH₂)ₖ-, wherein k is 1 or 2.

Preferably, in certain embodiments of the present invention, in LA, LA-1 and LA-3, ring C is 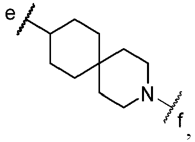 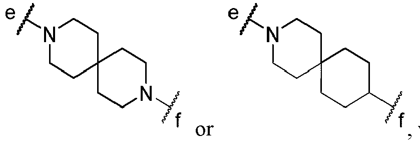 wherein end c is linked to X‴, and end d is linked to L₃.

In certain embodiments of the present invention, LA-3 is any one of the following structures:

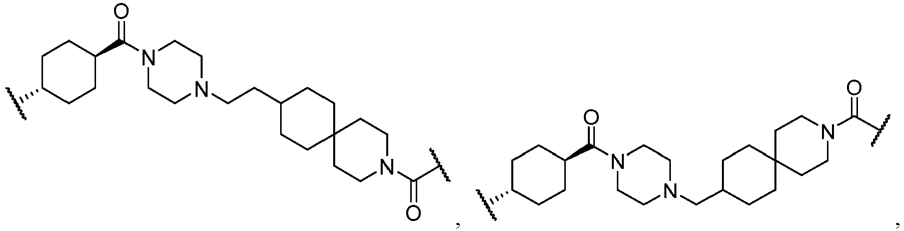

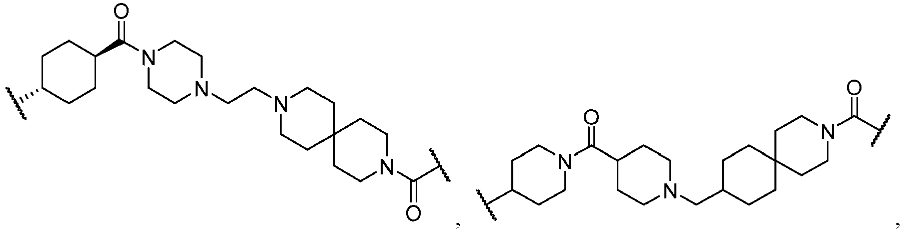

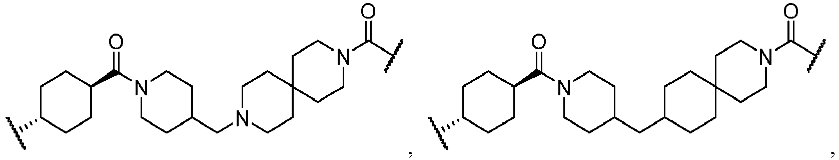

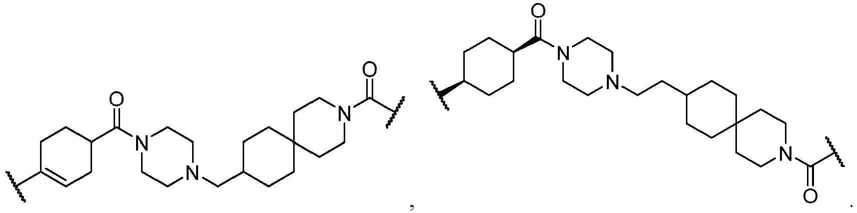

Preferably, in certain embodiments of the present invention, LA-3 is any one of the following structures:

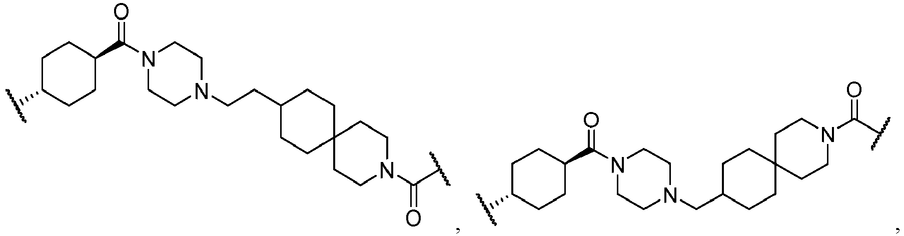

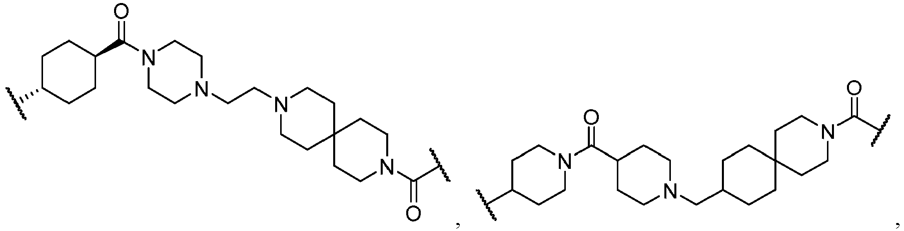

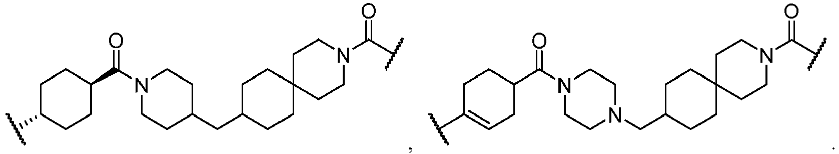

In certain embodiments of the present invention, LA is LA-4: 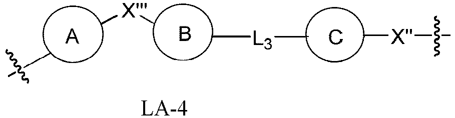 wherein ring A is 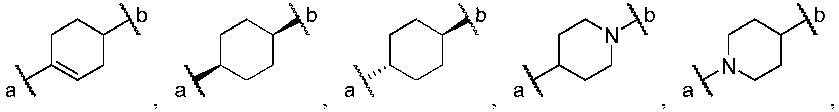 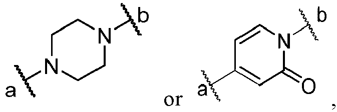 wherein end a is linked to S, and end b is linked to X‴; 1, 2, 3 or 4 hydrogen atoms in ring A are optionally substituted with F;
X‴ is a bond, -C(O)NH- or -C(O)NCH₃-;
ring B is a bond;
L₃ is -(CH₂)ₖ, wherein one or two methylene groups in L₃ are optionally replaced by -O-, -NH-, -C(O)-, -C≡C- or - N(C₁-C₆ alkyl)-, and k is 0, 1, 2, 3, 4, 5, 6 or 7;
ring C is 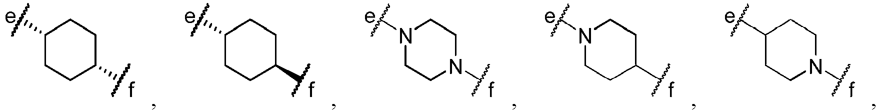 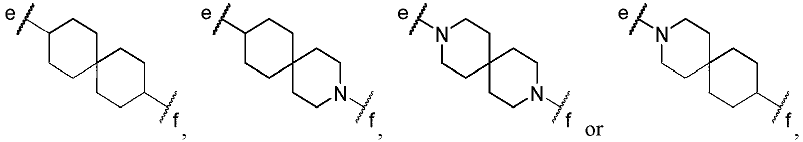 wherein end e is linked to L₃, and end f is linked to X"; 1, 2, 3 or 4 hydrogen atoms in ring C are optionally substituted with F; X" is -C(O)-.

In certain embodiments of the present invention, in LA-4, ring A is 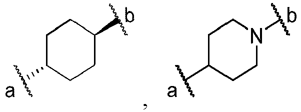 or 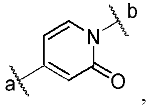 wherein end a is linked to S, and end b is linked to X‴.

In certain embodiments of the present invention, in LA-4, L₃ is -CH₂-, 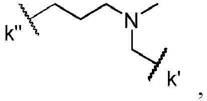 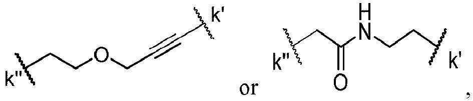 wherein end k" is linked to ring B, and end k' is linked to ring C.

In certain embodiments of the present invention, in LA-4, ring C is 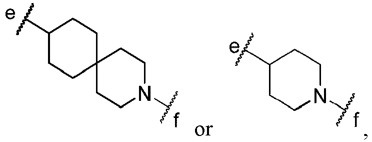 wherein end e is linked to L₃, and end f is linked to X".

In certain embodiments of the present invention, LA-4 is any one of the following structures:

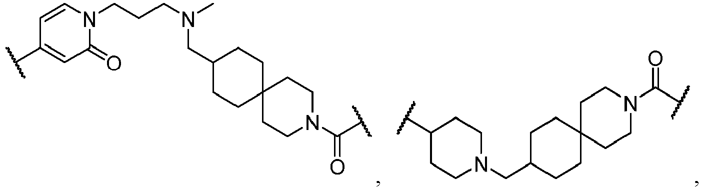

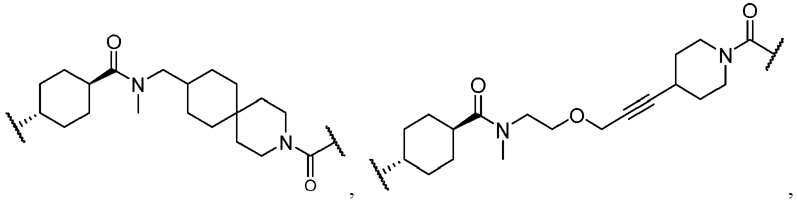

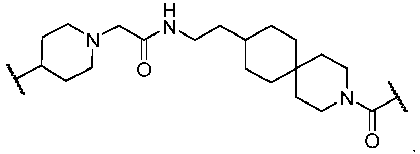

Preferably, in certain embodiments of the present invention, LA-4 is any one of the following structures:

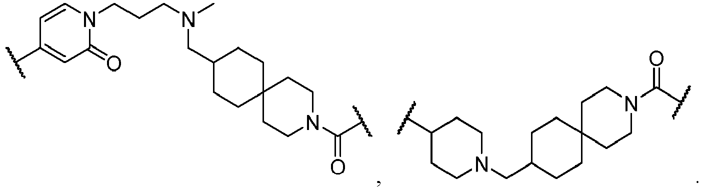

In certain embodiments of the present invention, LA is LA-5: 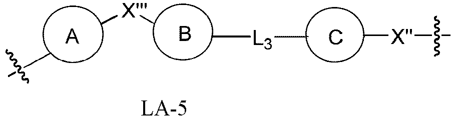 wherein ring A, X'", ring B, L₃ and ring C are as defined in LA-2 or LA-3;
X" is -C(O)CH₂O-, and -C(O)- in X" is linked to ring C.

In certain embodiments of the present invention, in LA-5, ring A is 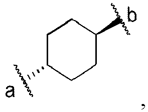 wherein end a is linked to S, and end b is linked to X‴.

In certain embodiments of the present invention, in LA-5, ring B is 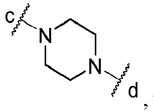 wherein end c is linked to X'", and end d is linked to L₃.

In certain embodiments of the present invention, in LA-5, L₃ is -(CH₂)ₖ, wherein k is 1 or 2, preferably 1. In certain embodiments of the present invention, in LA-5, ring C is 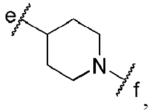 wherein end e is linked to L₃, and end f is linked to X".

Preferably, in certain embodiments of the present invention, LA-5 is

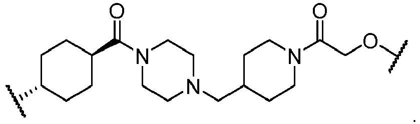

Preferably, in certain embodiments of the present invention, LA is

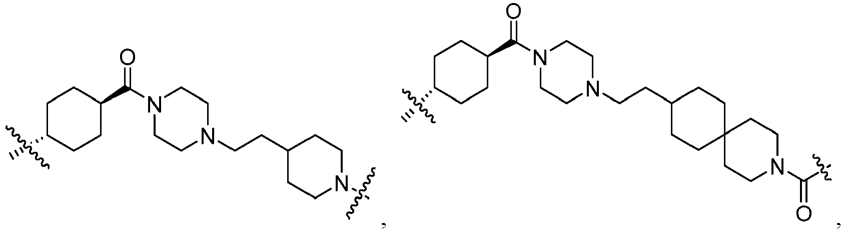

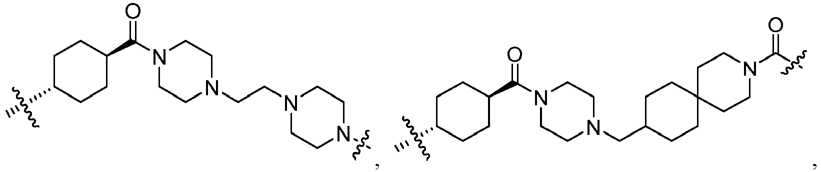

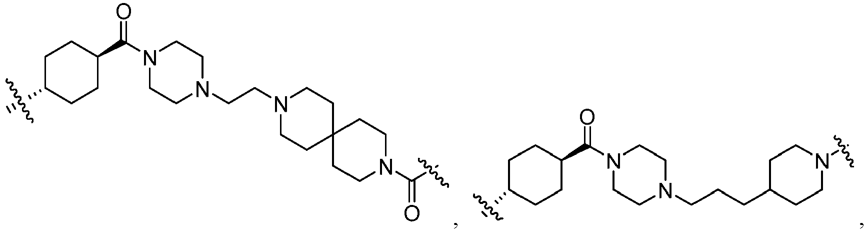

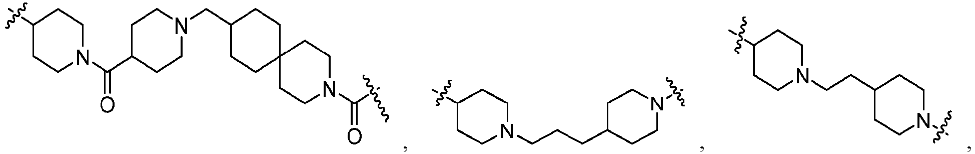

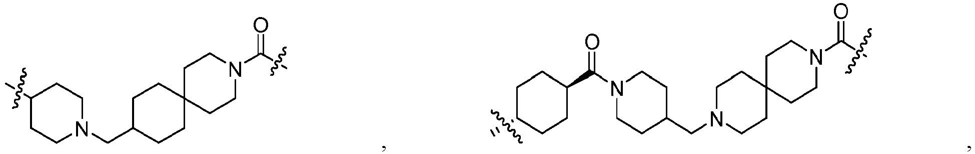

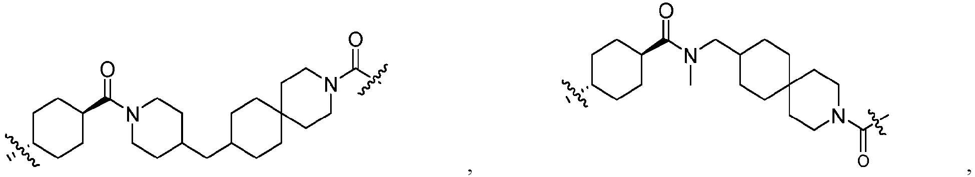

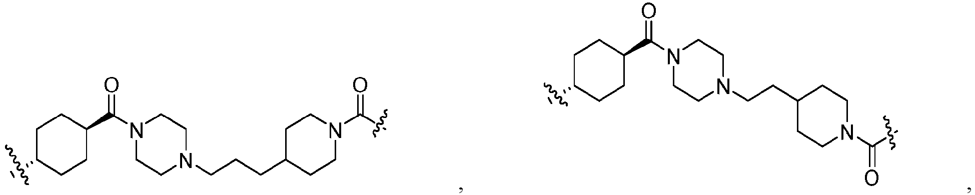

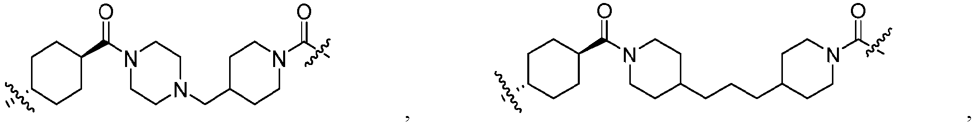

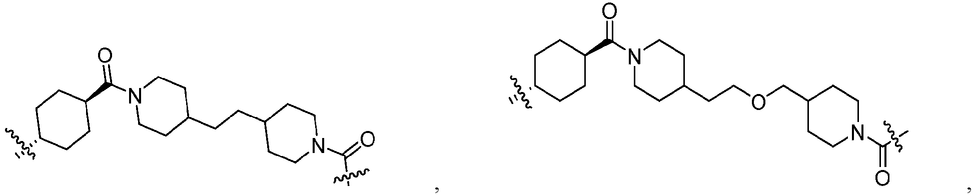

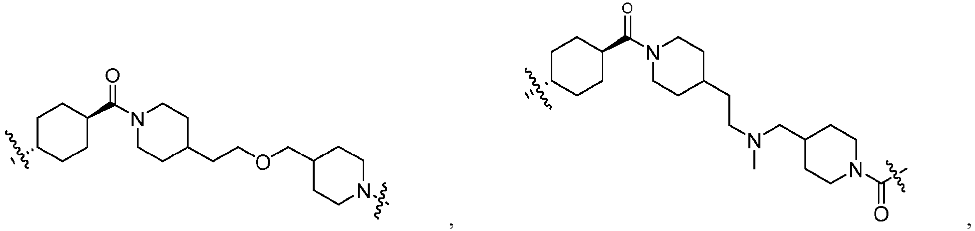

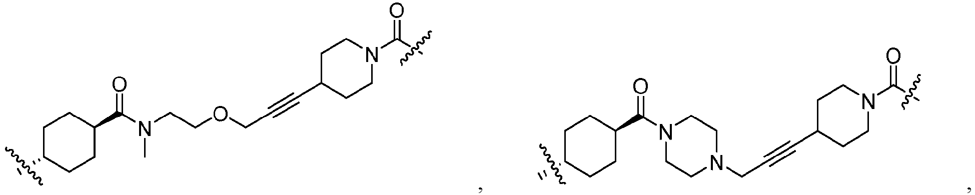

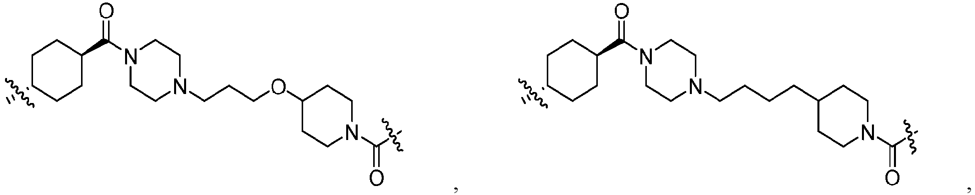

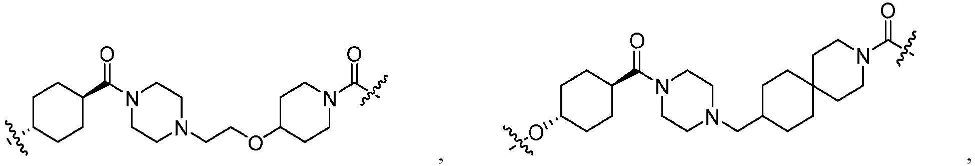

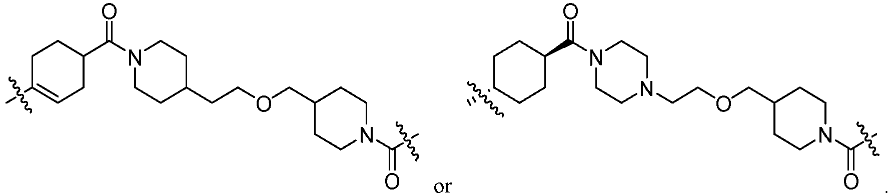

Preferably, in certain embodiments of the present invention, E is a small molecule ligand of CRBN, VHL, XAIP or MDM2 E3 ubiquitin ligases.

Preferably, in certain embodiments of the present invention, E is E1, E1a, E1b or E1c 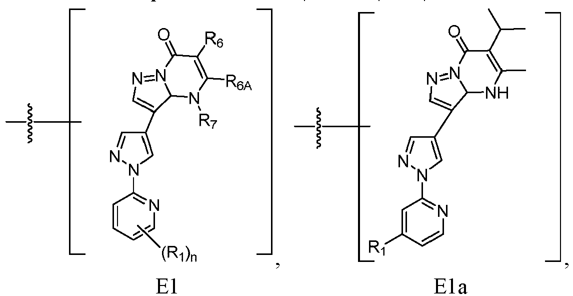 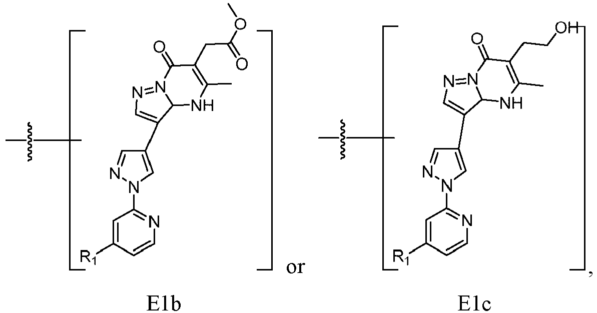 wherein E1, E1a, E1b or E1c:
each R¹ is independently halogen, optionally substituted acyl, optionally substituted alkyl, optionally substituted alkenyl, optionally substituted alkynyl, an optionally substituted carboncycle, an optionally substituted heterocycle,
optionally substituted aryl, optionally substituted heteroaryl, -CN or -O(CH₂)_{X}R^{1A};
and at least one R¹ is -O(CH₂)ₓR^{1A};
R⁶ is unsubstituted isopropyl, -(CH₂)C(O)OMe or -(CH₂)₂OH;
R^{6A} is substituted alkyl;
R⁷ is hydrogen, optionally substituted acyl, optionally substituted alkyl or a nitrogen protecting group;
R^{1A} is hydrogen, optionally substituted alkyl, optionally substituted alkenyl, optionally substituted alkynyl, an optionally substituted carboncycle, an optionally substituted heterocycle, optionally substituted aryl, optionally substituted heteroaryl, -OR^{A}, -N(R^{B})₂ or -SR^{A};
each R^{A} is independently hydrogen, optionally substituted acyl or optionally substituted alkyl, and is an oxygen protecting group when linked to O and a sulfur protecting group when linked to S;
each R^{B} is independently hydrogen, optionally substituted acyl, optionally substituted alkyl or a nitrogen protecting group;
n is 1, 2, 3 or 4; and
x is 0, 1, 2, 3, 4, 5 or 6, and when R⁶ is isopropyl, R^{6A} is methyl, R⁷ is hydrogen, and n is 1, R¹ is not methyl or hydroxymethyl.

Preferably, in certain embodiments of the present invention, E is E2 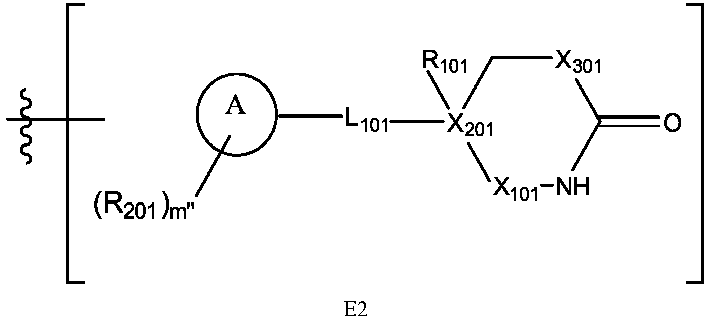 wherein in E2,
X₁₀₁ is abond, -CH₂-, -CHCF₃-, -S(O)₂-, -S(O)-, -P(O)R₁₀₀-, -P(O)OR₁₀₀-, -P(O)N(R₁₀₀)₂, -C(O)-, -C(S)- or 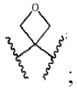
X₂₀₁ is C or Si;
X₃₀₁ is -CH₂-, -O-, -S-, -C(R₁₀₀)₂- or -Si(R₁₀₀)₂-;
R₁₀₁ is selected from hydrogen, deuterium, halogen, -CN, -OR₁₀₀, -SR₁₀₀, -S(O)R₁₀₀, -S(O)₂R₁₀₀, -N(R₁₀₀)₂, - P(O)(OR₁₀₀)₂, -P(O)[N(R₁₀₀)₂₁(OR₁₀₀), -P(O)[N(R₁₀₀)₂]₂, -Si(OH)₂(R₁₀₀)-, -Si(OH)(R₁₀₀)₂-, -Si(R₁₀₀)₃- and optionally substituted C₁-C₄ alkyl;
R₂₀₁ is selected from hydrogen, -R₆₀₁, halogen, -CN, -NO₂, -OR₁₀₀, -SR₁₀₀, -S(O)R₁₀₀, -S(O)₂R₁₀₀, -N(R₁₀₀)₂, - S(O)₂N(R₁₀₀)₂, -C(O)R₁₀₀, -C(O)OR₁₀₀, -C(O)N(R₁₀₀)₂, -OC(O)R₁₀₀, -OC(O)N(R₁₀₀)₂, -NR₁₀₀C(O)OR₁₀₀, - NR₁₀₀C(O)R₁₀₀, -NR₁₀₀C(O)N(R₁₀₀)₂, -OP(O)(R₁₀₀)₂, -OP(O)(OR₁₀₀)₂, -OP(O)(OR₁₀₀)[N(R₁₀₀)₂], - OP(O)[N(R₁₀₀)₂], -NP(O)(R₁₀₀)₂, -NR₁₀₀P(O)(OR₁₀₀)₂, -N(R₁₀₀)P(O)(OR₁₀₀)[N(R₁₀₀)₂], -N(R₁₀₀)P(O)[N(R₁₀₀)₂]₂ and -NR₁₀₀S(O)₂R₁₀₀;
L₁₀₁ is a bond or C₁-C₄ alkylene, and 1 or 2 methylene in the C₁-C₄ alkylene are optionally replaced by a group selected from -O-, -C(O)-, -C(S)-, -C(R₁₀₀)₂-, -CH(R₁₀₀)-, -C(F)₂-, -N(R₁₀₀)-, -S-, -S(O)₂- and -CH=CH; ring A is bicyclic or tricyclic, and is selected from 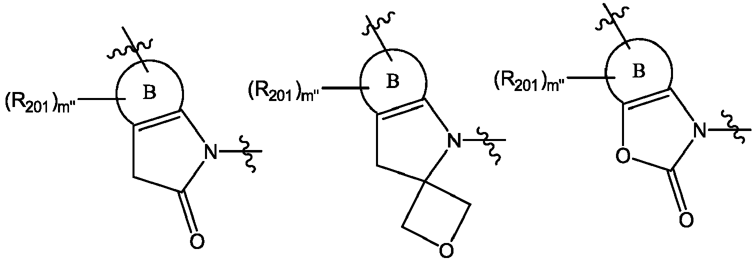 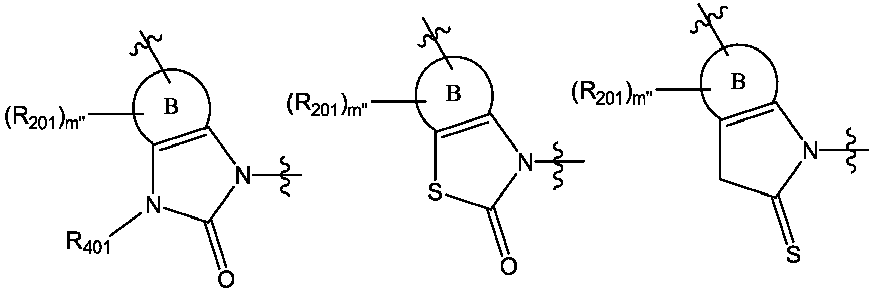 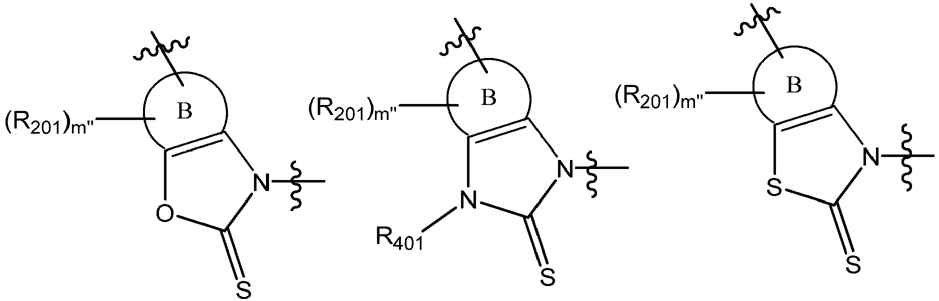 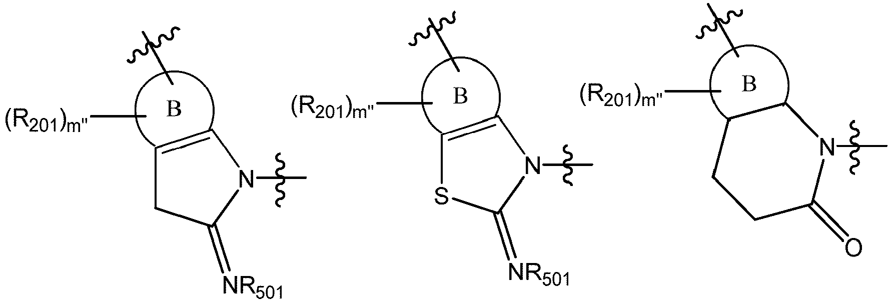 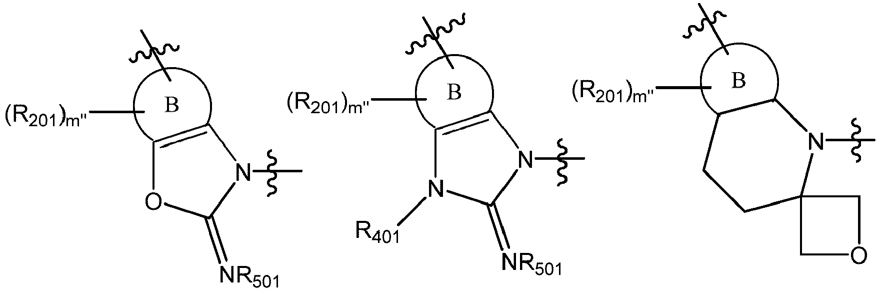 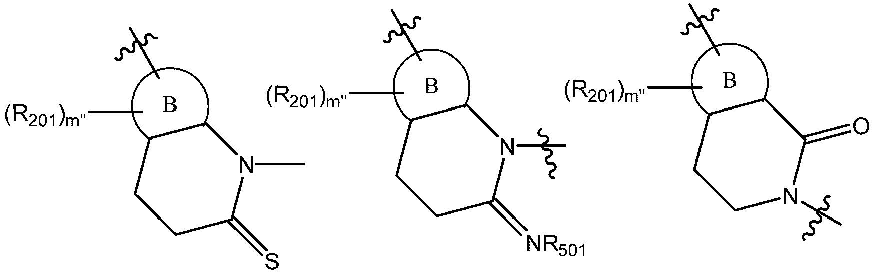 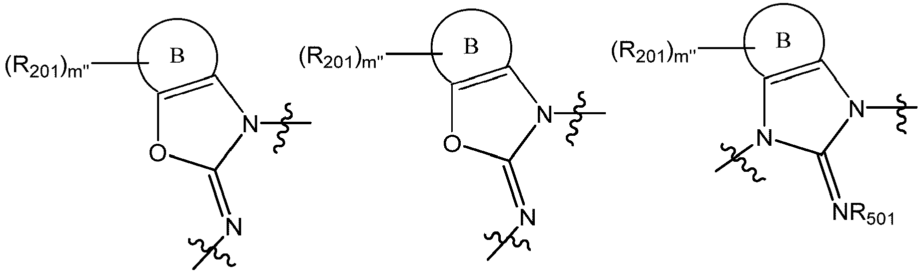 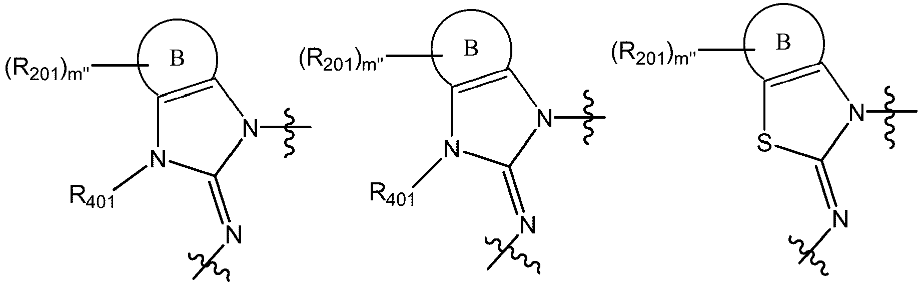 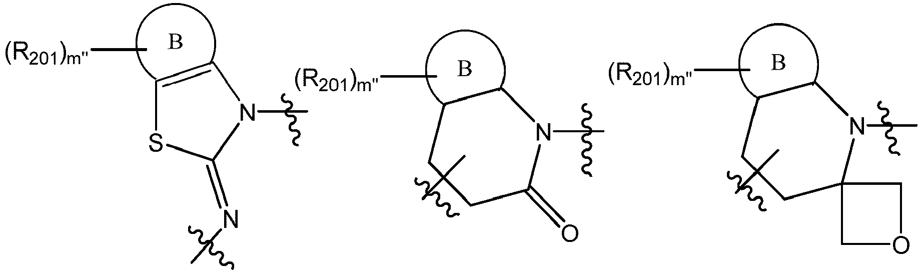 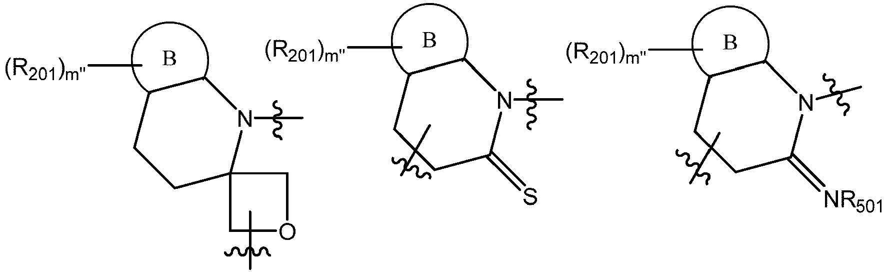 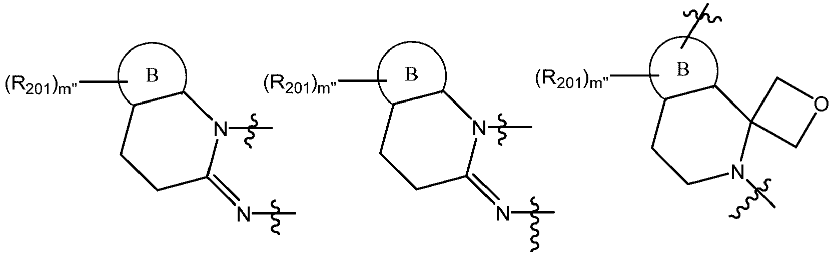 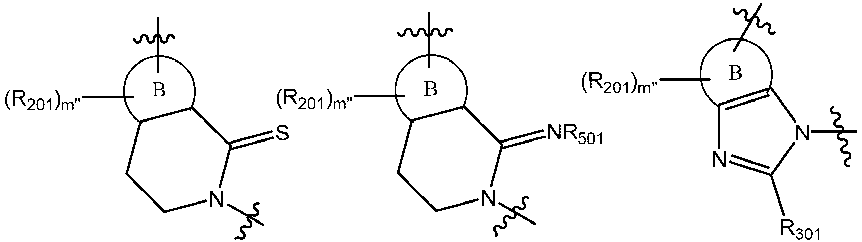 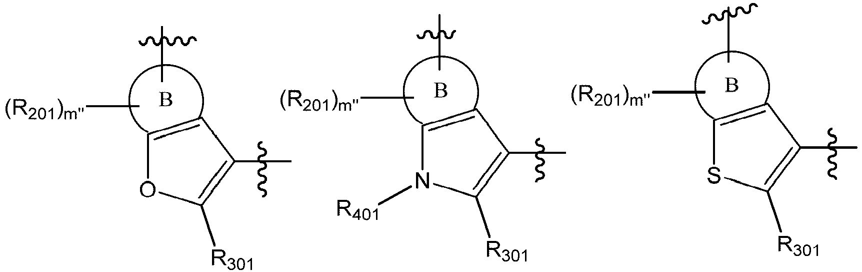 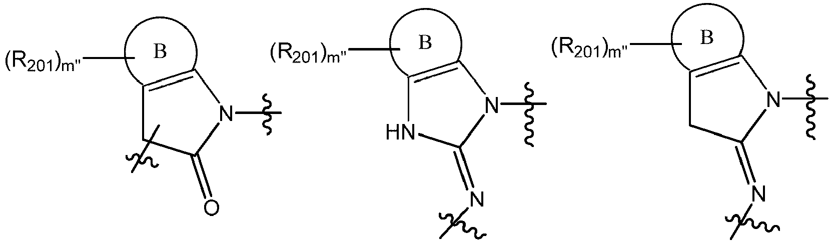 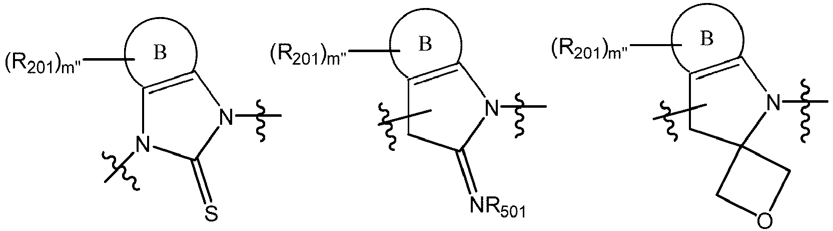 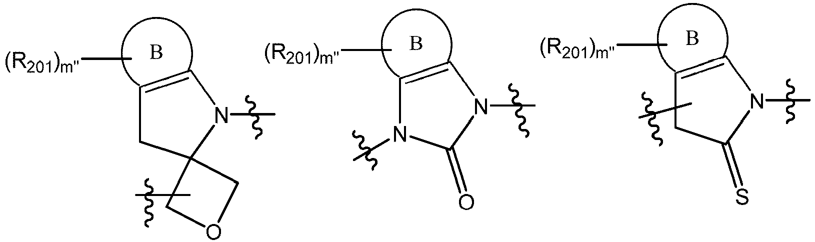 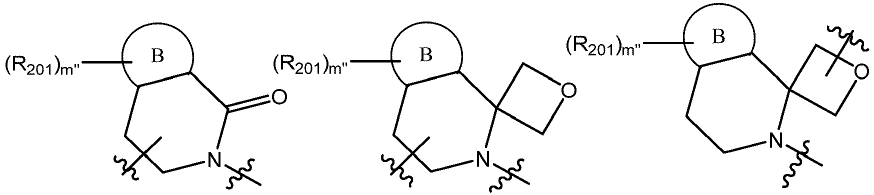 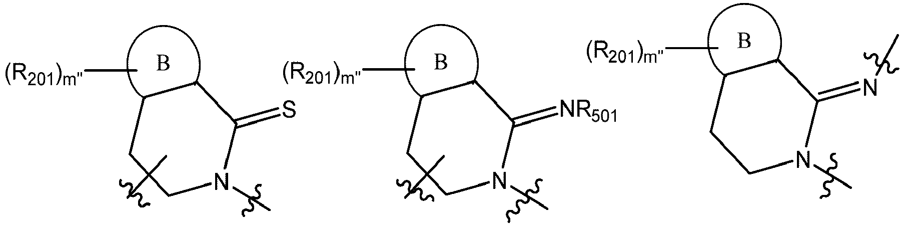 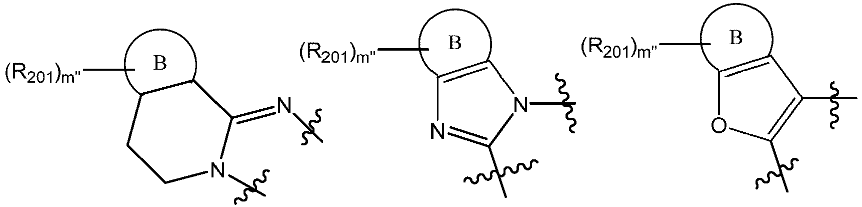 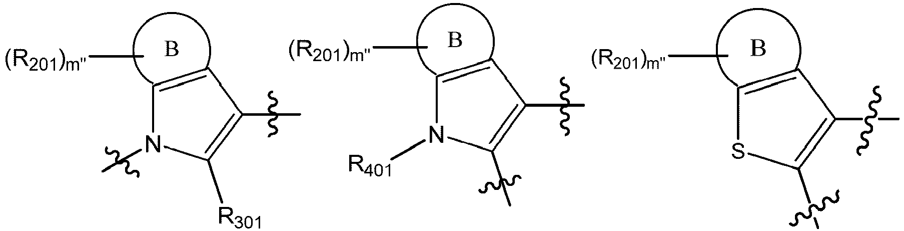 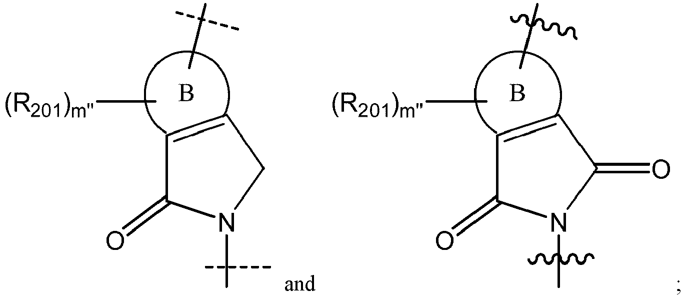 wherein:
   ring B is 6-membered heteroaryl containing 1-2 N atoms, phenyl, 5- to 7-membered saturated or partially unsaturated cycloalkyl, 5- to 7-membered saturated or partially unsaturated heterocycloalkyl containing 1-2 heteroatoms selected from N, O and S or 5-membered heteroaryl containing 1-3 heteroatoms selected from N, O and S;
   R₃₀₁ is selected from hydrogen, halogen, -OR₁₀₀, -SR₁₀₀ and -N(R₁₀₀)₂;
   R₄₀₁ is hydrogen, -R₆₀₁, -halogen, -CN, nitro, -OR₁₀₀, -SR₁₀₀, -N(R₁₀₀)₂, -S(O)R₁₀₀, -S(O)₂R₁₀₀, -N(R₁₀₀)₂, - S(O)₂N(R₁₀₀)₂, -C(O)R₁₀₀, -C(O)OR₁₀₀, -C(O)N(R₁₀₀)₂, -OC(O)R₁₀₀, -OC(O)N(R₁₀₀)₂, -NR₁₀₀C(O)OR₁₀₀, - NR₁₀₀C(O)R₁₀₀, -NR₁₀₀C(O)N(R₁₀₀)₂ or -NR₁₀₀S(O)₂R₁₀₀;
   R₅₀₁ is hydrogen, -CN or C₁-C₄ alkyl;
   R₆₀₁ is optionally substituted C₁-C₆ alkyl, phenyl, 4- to 7-membered saturated or partially unsaturated heterocycloalkyl containing 1-2 heteroatoms selected from N, O and S or 5- to 6-membered heteroaryl containing 1-4 heteroatoms selected from N, O and S;
   R₁₀₀ is selected from hydrogen, optionally substituted C₁-C₆ alkyl, phenyl, 4- to 7-membered saturated or partially unsaturated heterocycloalkyl containing 1-2 heteroatoms selected from N, O and S and 5- to 6-membered heteroaryl containing 1-4 heteroatoms selected from N, O and S, or two R₁₀₀, together with the N atom linked thereto, form saturated or partially unsaturated 4- to 7-membered heteroaryl containing 0-3 heteroatoms selected from N, O and S in addition to the linking N atom;
   m" is 0, 1, 2, 3 or 4.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E3: 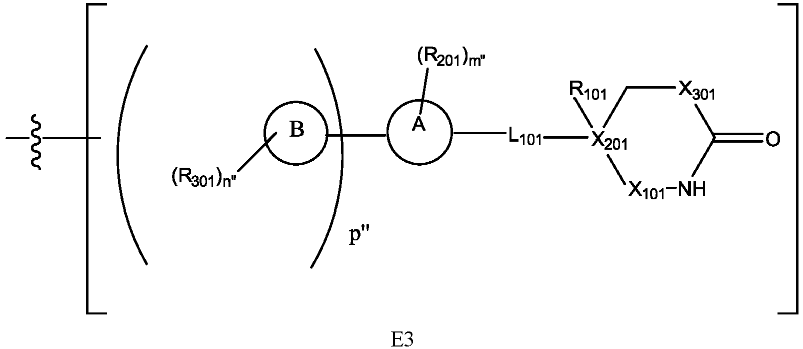 wherein
X₁₀₁ is abond, -CH₂-, -CHCF₃-, -S(O)₂-, -S(O)-, -P(O)R₁₀₀-, -P(O)OR₁₀₀-, -P(O)N(R₁₀₀)₂, -C(O)-, -C(S)- or 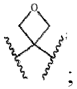
X₂₀₁ is C, N or Si;
X₃₀₁ is -CH₂-, -O-, -S-, -C(R₁₀₀)₂- or -Si(R₁₀₀)₂-;
R₁₀₁ is selected from hydrogen, deuterium, halogen, -CN, -OR₁₀₀, -SR₁₀₀, -S(O)R₁₀₀, -S(O)₂R₁₀₀, -N(R₁₀₀)₂, - P(O)(OR₁₀₀)₂, -P(O)[N(R₁₀₀)₂₁(OR₁₀₀), -P(O)[N(R₁₀₀)₂]₂, -Si(OH)₂(R₁₀₀)-, -Si(OH)(R₁₀₀)₂-, -Si(R₁₀₀)₃- and optionally substituted C₁-C₄ alkyl;
L₁₀₁ is a bond or C₁-C₄ alkylene, and 1 or 2 methylene in the C₁-C₄ alkylene are optionally replaced by a group selected from -O-, -C(O)-, -C(S)-, -C(R₁₀₀)₂-, -CH(R₁₀₀)-, -C(F)₂-, -N(R₁₀₀)-, -S-, -S(O)₂- and -CH=CH; ring A is absent or is monocyclic or bicyclic, and is selected from: 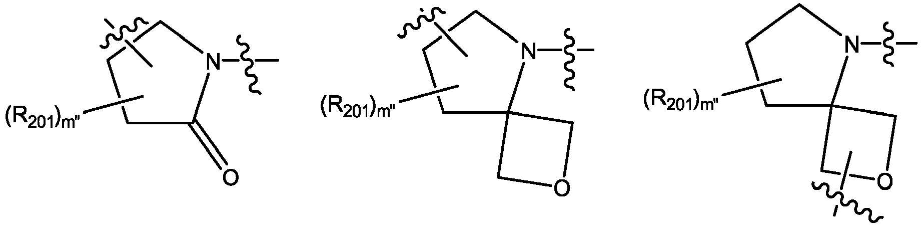 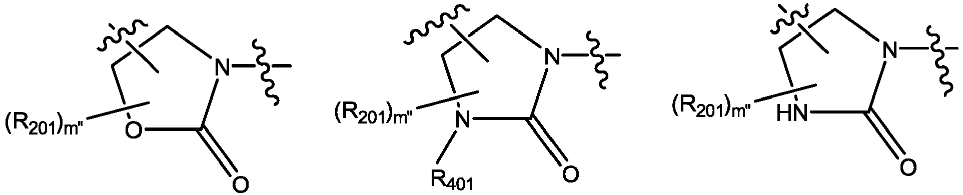 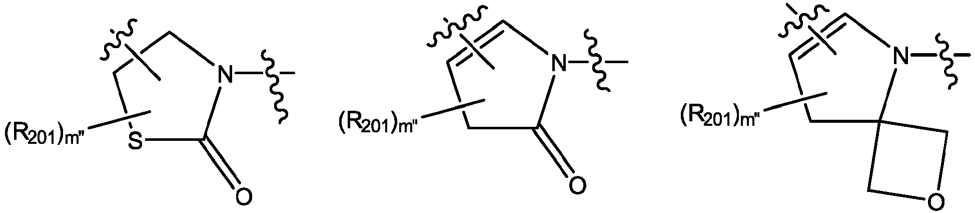 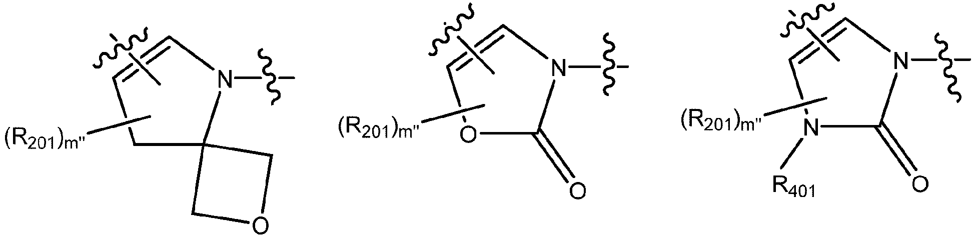 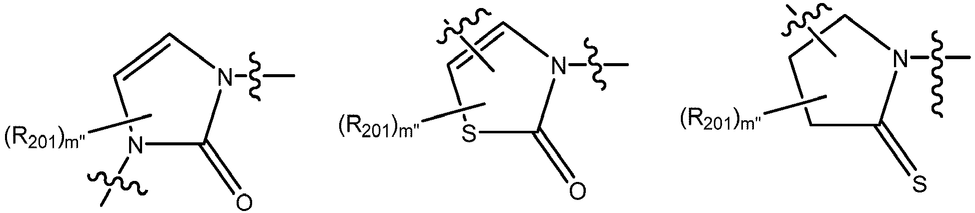 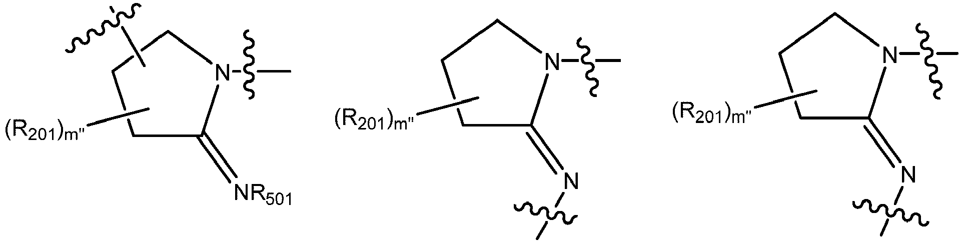 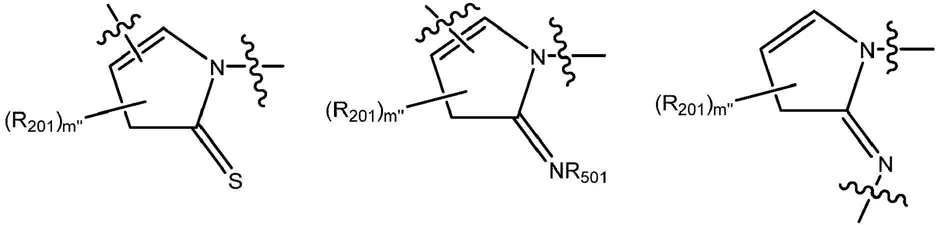 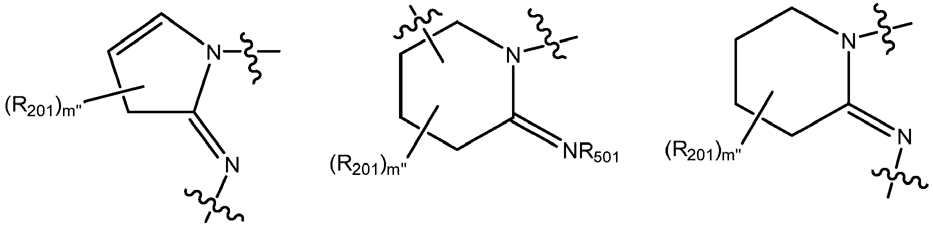 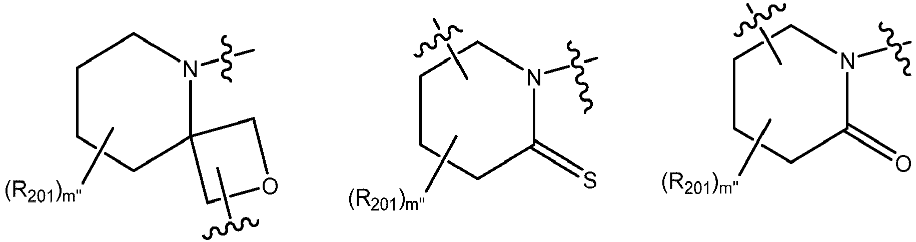 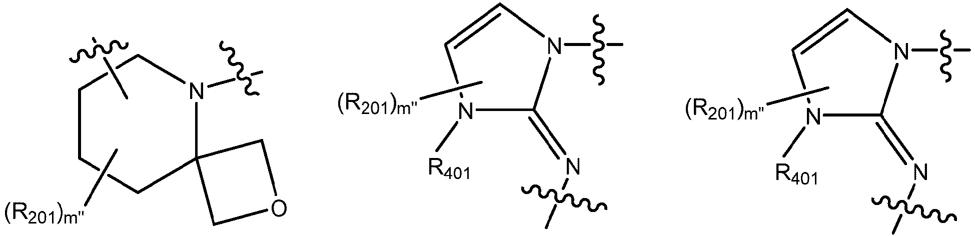 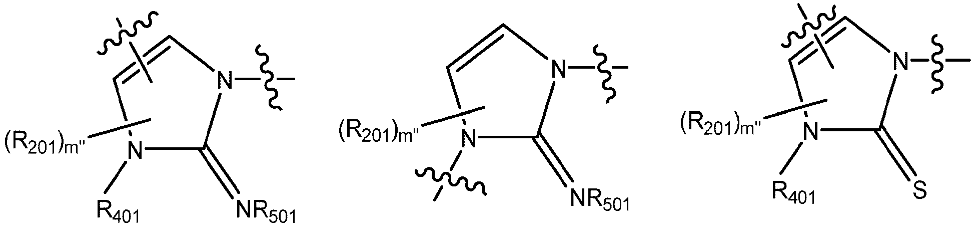 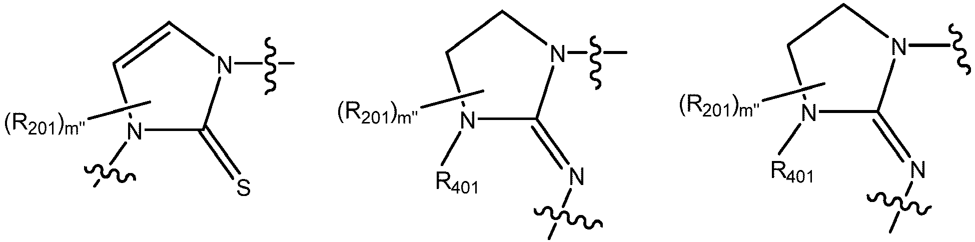 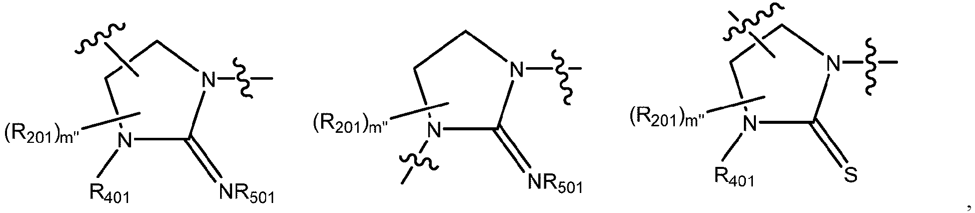 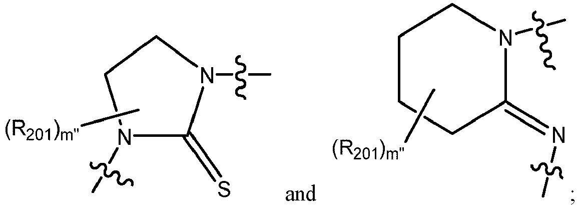
R₂₀₁ is selected from hydrogen, -R₆₀₁, halogen, -CN, -NO₂, -OR₁₀₀, -SR₁₀₀, -S(O)R₁₀₀, -S(O)₂R₁₀₀, -N(R₁₀₀)₂, - S(O)₂NR₁₀₀, -C(O)R₁₀₀, -C(O)OR₁₀₀, -C(O)N(R₁₀₀)₂, -OC(O)R₁₀₀, -OC(O)N(R₁₀₀)₂, -NR₁₀₀C(O)OR₁₀₀, - NR₁₀₀C(O)R₁₀₀, -NR₁₀₀C(O)N(R₁₀₀)₂, -OP(O)(R₁₀₀)₂, -OP(O)(OR₁₀₀)₂, -OP(O)(OR₁₀₀)[N(R₁₀₀)₂], - OP(O)[N(R₁₀₀)₂], -NP(O)(R₁₀₀)₂, -NR₁₀₀P(O)(OR₁₀₀)₂, -N(R₁₀₀)P(O)(OR₁₀₀)[N(R₁₀₀)₂], -N(R₁₀₀)P(O)[N(R₁₀₀)₂]₂ and -NR₁₀₀S(O)₂R₁₀₀;
ring B is 6-membered heteroaryl containing 1-2 N atoms, phenyl, 5- to 7-membered saturated or partially unsaturated cycloalkyl, 5- to 7-membered saturated or partially unsaturated heterocycloalkyl containing 1-2 heteroatoms selected from N, O and S or 5-membered heteroaryl containing 1-3 heteroatoms selected from N, O and S; R₃₀₁ and R₄₀₁ are each independently hydrogen, -R₆₀₁, halogen, -CN, -NO₂, -OR₁₀₀, -SR₁₀₀, -S(O)R₁₀₀, -S(O)₂R₁₀₀, -N(R₁₀₀)₂, -S(O)₂NR₁₀₀, -C(O)R₁₀₀, -C(O)OR₁₀₀, -C(O)N(R₁₀₀)₂, -OC(O)R₁₀₀, -OC(O)N(R₁₀₀)₂, -NR₁₀₀C(O)OR₁₀₀, -NR₁₀₀C(O)R₁₀₀, -NR₁₀₀C(O)N(R₁₀₀)₂ or -NR₁₀₀S(O)₂R₁₀₀;
R₅₀₁ is hydrogen, -CN or C₁-C₄ alkyl;
R₆₀₁ is optionally substituted C₁-C₆ alkyl, phenyl, 4- to 7-membered saturated or partially unsaturated heterocycloalkyl containing 1-2 heteroatoms selected from N, O and S or 5- to 6-membered heteroaryl containing 1-4 heteroatoms selected from N, O and S;
R₁₀₀ is selected from hydrogen, optionally substituted C₁-C₆ alkyl, phenyl, 4- to 7-membered saturated or partially unsaturated heterocycloalkyl containing 1-2 heteroatoms selected from N, O and S and 5- to 6-membered heteroaryl containing 1-4 heteroatoms selected from N, O and S, or two R₁₀₀, together with the N atom linked thereto, form saturated or partially unsaturated 4- to 7-membered heteroaryl containing 0-3 heteroatoms selected from N, O and S in addition to the linking N atom;
P" is 0 or 1;
m" is 0, 1 or 2;
n" is 0, 1, 2, 3 or 4.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E4: 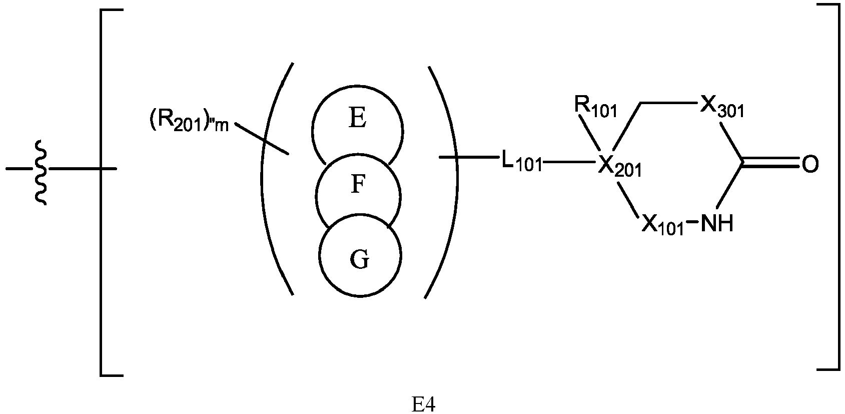 wherein in E4,
X₁₀₁ is abond, -CH₂-, -CHCF₃-, -S(O)₂-, -S(O)-, -P(O)R₁₀₀-, -P(O)OR₁₀₀-, -P(O)N(R₁₀₀)₂, -C(O)-, -C(S)- or 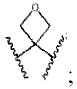
X₂₀₁ is C, N or Si;
X₃₀₁ is -CH₂-, -O-, -S-, -C(R₁₀₀)₂- or -Si(R₁₀₀)₂-;
R₁₀₁ is selected from hydrogen, deuterium, halogen, -CN, -OR₁₀₀, -SR₁₀₀, -S(O)R₁₀₀, -S(O)₂R₁₀₀, -N(R₁₀₀)₂, - P(O)(OR₁₀₀)₂, -P(O)[N(R₁₀₀)₂₁(OR₁₀₀), -P(O)[N(R₁₀₀)₂]₂, -Si(OH)₂(R₁₀₀)-, -Si(OH)(R₁₀₀)₂-, -Si(R₁₀₀)₃- and optionally substituted C₁-C₄ alkyl;
L₁₀₁ is a bond or C₁-C₄ alkylene, and 1 or 2 methylene in the C₁-C₄ alkylene are optionally replaced by a group selected from -O-, -C(O)-, -C(S)-, -C(R₁₀₀)₂-, -CH(R₁₀₀)-, -C(F)₂-, -N(R₁₀₀)-, -S-, -S(O)₂- and -CH=CH;
R₂₀₁ is selected from hydrogen, -R₆₀₁, halogen, -CN, -NO₂, -OR₁₀₀, -SR₁₀₀, -S(O)R₁₀₀, -S(O)₂R₁₀₀, -N(R₁₀₀)₂, - S(O)₂NR₁₀₀, -C(O)R₁₀₀, -C(O)OR₁₀₀, -C(O)N(R₁₀₀)₂, -OC(O)R₁₀₀, -OC(O)N(R₁₀₀)₂, -NR₁₀₀C(O)OR₁₀₀, - NR₁₀₀C(O)R₁₀₀, -NR₁₀₀C(O)N(R₁₀₀)₂, -OP(O)(R₁₀₀)₂, -OP(O)(OR₁₀₀)₂, -OP(O)(OR₁₀₀)[N(R₁₀₀)₂], - OP(O)[N(R₁₀₀)₂], -NP(O)(R₁₀₀)₂, -NR₁₀₀P(O)(OR₁₀₀)₂, -N(R₁₀₀)P(O)(OR₁₀₀)[N(R₁₀₀)₂], -N(R₁₀₀)P(O)[N(R₁₀₀)₂]₂ and -NR₁₀₀S(O)₂R₁₀₀;
R₅₀₁ is optionally substituted C₁-C₆ alkyl, phenyl, 4- to 7-membered saturated or partially unsaturated heterocycloalkyl containing 1-2 heteroatoms selected from N, O and S or 5- to 6-membered heteroaryl containing 1-4 heteroatoms selected from N, O and S;
R₁₀₀ is selected from hydrogen, optionally substituted C₁-C₆ alkyl, phenyl, 4- to 7-membered saturated or partially unsaturated heterocycloalkyl containing 1-2 heteroatoms selected from N, O and S and 5- to 6-membered heteroaryl containing 1-4 heteroatoms selected from N, O and S, or two R₁₀₀, together with the N atom linked thereto, form saturated or partially unsaturated 4- to 7-membered heteroaryl containing 0-3 heteroatoms selected from N, O and S in addition to the linking N atom;
ring E, ring F and ring G are each independently 6-membered heteroaryl containing 1-2 N atoms, phenyl, 5- to 7-membered saturated or partially unsaturated cycloalkyl, 5- to 7-membered saturated or partially unsaturated heterocycloalkyl containing 1-2 heteroatoms selected from N, O and S or 5-membered heteroaryl containing 1-3 heteroatoms selected from N, O and S;
m" is 0, 1, 2, 3, 4, 5, 6, 7, 8, 9, 10, 11, 12, 13, 14, 15 or 16.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E5: 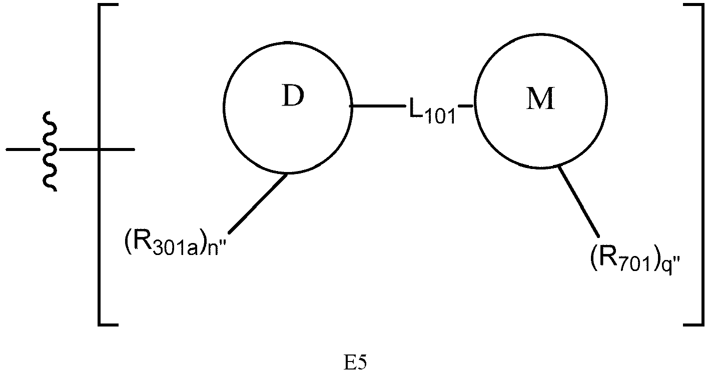
wherein in E5, ring M is selected from 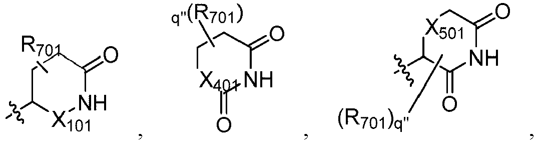 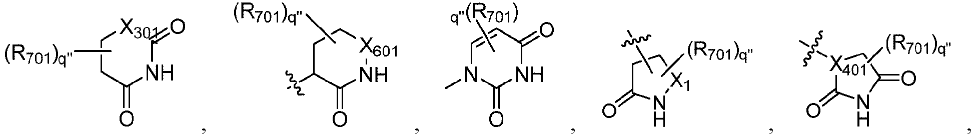 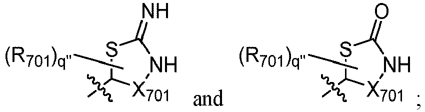
each X₁₀₁, X₆₀₁ and X₇₀₁ are each independently a bond, -CH₂-, -CHCF₃-, -S(O)₂-, -S(O)-, -P(O)R₁₀₀-, -P(O)OR₁₀₀-, -P(O)N(R₁₀₀)₂, -C(O)-, -C(S)- or 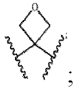
each X₃₀₁ and X₅₀₁ are each independently a bond, -C(R₁₀₀)₂-, -NR₁₀₀-, -O-, -S- or -Si(R₁₀₀)₂-;
X₄₀₁ is selected from 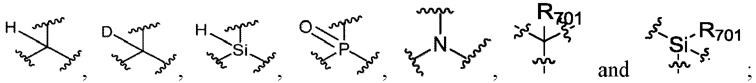
each R₁₀₀ is independently selected from hydrogen, optionally substituted C₁-C₆ alkyl, phenyl, 4- to 7-membered saturated or partially unsaturated heterocycloalkyl containing 1-2 heteroatoms selected from N, O and S and 5- to 6-membered heteroaryl containing 1-4 heteroatoms selected from N, O and S, or two R₁₀₀, together with the N atom linked thereto, form saturated or partially unsaturated 4- to 7-membered heteroaryl containing 0-3 heteroatoms selected from N, O and S in addition to the linking N atom;
each R₃₀₁ₐ is independently hydrogen, deuterium, -R₆₀₁, halogen, -CN, -NO₂, -OR₁₀₀, -SR₁₀₀, -N(R₁₀₀)₂, -Si(R₁₀₀)₃, -S(O)R₁₀₀, -S(O)₂R₁₀₀, -S(O)₂N(R₁₀₀)₂, -C(O)R₁₀₀, -C(O)OR₁₀₀, -C(O)N(R₁₀₀)₂, -C(O)N(R₁₀₀)OR₁₀₀, - C(R₁₀₀)₂N(R₁₀₀)C(O)R₁₀₀, -C(R₁₀₀)₂N(R₁₀₀)C(O)N(R₁₀₀)₂, -OC(O)R₁₀₀, -OC(O)N(R₁₀₀)₂, -NR₁₀₀C(O)OR₁₀₀, - NR₁₀₀C(O)R₁₀₀, -NR₁₀₀C(O)N(R₁₀₀)₂, -OP(O)(R₁₀₀)₂, -OP(O)(OR₁₀₀)₂, -OP(O)(OR₁₀₀)[N(R₁₀₀)₂], - OP(O)[N(R₁₀₀)₂], -NP(O)(R₁₀₀)₂, -NR₁₀₀P(O)(OR₁₀₀)₂, -N(R₁₀₀)P(O)(OR₁₀₀)[N(R₁₀₀)₂], -N(R₁₀₀)P(O)[N(R₁₀₀)₂]₂ or -NR₁₀₀S(O)₂R₁₀₀;
each R₆₀₁ is independently optionally substituted C₁-C₆ alkyl, phenyl, 4- to 7-membered saturated or partially unsaturated heterocycloalkyl containing 1-2 heteroatoms selected from N, O and S or 5- to 6-membered heteroaryl containing 1-4 heteroatoms selected from N, O and S;
each R₇₀₁ is independently hydrogen, deuterium, halogen, -CN, -OR₁₀₀, -SR₁₀₀, -S(O)R₁₀₀, -S(O)₂R₁₀₀, -N(R₁₀₀)₂, - P(O)(OR₁₀₀)₂, -P(O)[N(R₁₀₀)₂₁(OR₁₀₀), -P(O)[N(R₁₀₀)₂]₂, -Si(OH)₂(R₁₀₀)-, -Si(OH)(R₁₀₀)₂-, -Si(R₁₀₀)₃- or optionally substituted C1-C4 alkyl; or
R₇₀₁ and X₁₀₁ or X₃₀₁, together with the atom linked thereto, form a 5- to 7-membered saturated or partially unsaturated carboncycle or a heterocycle containing 1-3 heteroatoms selected from boron, N, O, Si and S;
or two R₇₀₁ on the same carbon, together with the atom linked thereto, form a 3- to 6-membered ring or a 4- to 7-membered heterocycle containing 1-2 heteroatoms selected from boron, N, O, Si and S;
or two R₇₀₁ on adjacent carbons, together with the atom linked thereto, form a 3- to 7-membered saturated or partially unsaturated carboncycle or a heterocycle containing 1-3 heteroatoms selected from boron, N, O, Si and S, or form a saturated or partially unsaturated 7- to 13-membered bridged heterocycle or spiro heterocycle containing 1-3 heteroatoms selected from boron, N, O, Si and S;
each ring D is independently 6-membered heteroaryl containing 1-4 heteroatoms selected from N, O and S, phenyl, 5- to 7-membered saturated or partially unsaturated cycloalkyl, 5- to 7-membered saturated or partially unsaturated heterocycloalkyl containing 1-3 heteroatoms selected from N, O and S or 5-membered heteroaryl containing 1-4 heteroatoms selected from N, O and S;
L₁₀₁ is a bond or C₁-C₄ alkylene, and 1 or 2 methylene in the C₁-C₄ alkylene are optionally replaced by a group selected from -O-, -C(O)-, -C(S)-, -C(R₁₀₀)₂-, -CH(R₁₀₀)-, -C(F)₂-, -N(R₁₀₀)-, -S-, -S(O)₂- and -CH=CH-;
n" is 0, 1, 2, 3 or 4;
q" is 0, 1, 2, 3 or 4.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E6, E6a, E6b, E6c, E6d, E6e, E6f, E6g, E6h or E6i: 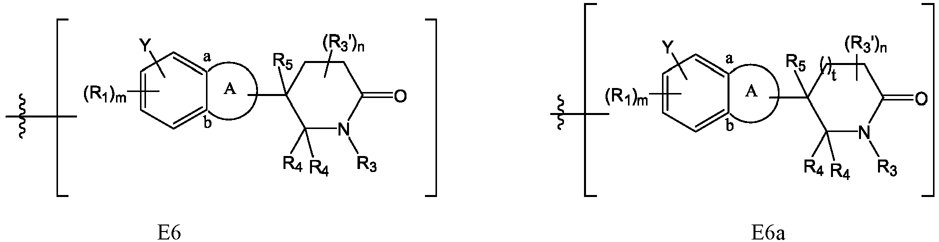 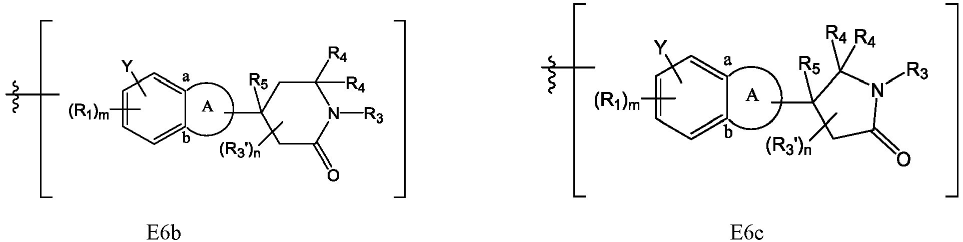 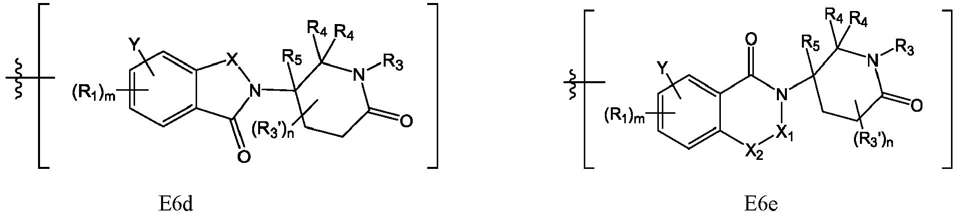 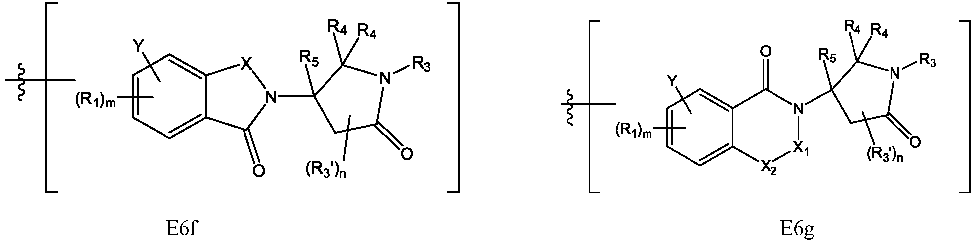 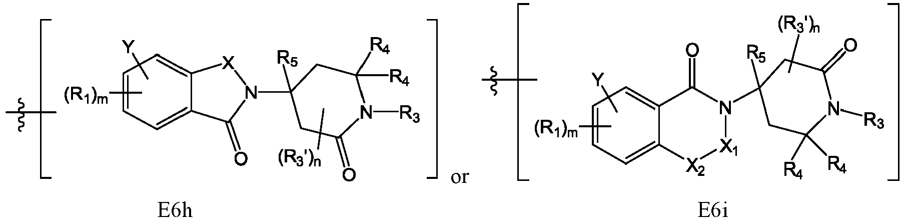 wherein in E6, E6a, E6b, E6c, E6d, E6e, E6f, E6g, E6h or E6i: 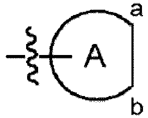 is 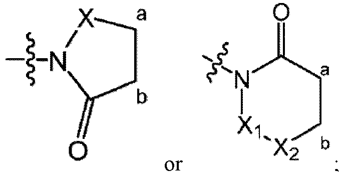
Y is a bond, Y₁, O, NH, NR₂, C(O)O, C(O)NR₂', Y₁-O, Y₁-NH, Y₁-NR₂, Y₁-C(O), Y1-C(O)O, Y₁-OC(O), Y₁-C(O)NR₂' or Y₁-NR₂'C(O), wherein Y₁ is C₁-C₆ alkylene, C₂-C₆ alkenyl or C₂-C₆ alkane alkynyl;
X is C(O) or C(R₃)₂;
X₁-X₂ is C(R₃)=N or C(R₃)₂-C(R₃)₂;
each R₁ is independently halogen, nitro, NH₂, OH, C(O)OH, C₁-C₆ alkyl or C₁-C₆ alkoxy;
R₂ is C₁-C₆ alkyl, C₂-C₆ alkenyl, C₃-C₈ cycloalkyl, 3- to 8-membered heterocycloalkyl or C(O)-3- to 8-membered heterocycloalkyl, and R₂ may be optionally substituted with one or more halogen, N(Rₐ)₂, NHC(O)Rₐ, NHC(O)ORₐ, OR_{b}, C₃-C₈ cycloalkyl, 3- to 8-membered heterocycloalkyl, C₆-C₁₀ aryl or 5- to 10-membered heteroaryl, wherein the C₃-C₈ cycloalkyl, the 3- to 8-membered heterocycloalkyl, the C₆-C₁₀ aryl or the 5- to 10-membered heteroaryl may be optionally substituted with one or more halogen, NH₂, CN, nitro, OH, C(O)OH, C₁-C₆ alkyl, C₁-C₆ haloalkyl, C₁-C₆ alkoxy or C₁-C₆ haloalkoxy;
R₂' is H, C₁-C₆ alkyl, C₂-C₆ alkenyl, C₃-C₈ cycloalkyl or 3- to 8-membered heterocycloalkyl, and when R₂' is not H, it may be optionally substituted with one or more halogen, N(Rₐ)₂, NHC(O)Rₐ, NHC(O)ORₐ, OR_{b}, C₃-C₈ cycloalkyl, 3- to 8-membered heterocycloalkyl, C₆-C₁₀ aryl or 5- to 10-membered heteroaryl, wherein the C₃-C₈ cycloalkyl, the 3- to 8-membered heterocycloalkyl, the C₆-C₁₀ aryl or the 5- to 10-membered heteroaryl may be optionally substituted with another one or more halogen, NH₂, CN, nitro, OH, C(O)OH, C₁-C₆ alkyl, C₁-C₆ haloalkyl, C₁-C₆ alkoxy or C₁-C₆ haloalkoxy;
each R₃ is independently H or C₁-C₃ alkyl, and the C₁-C₃ alkyl is optionally substituted with C₆-C₁₀ aryl or 5- to 10-membered heteroaryl;
each R₃' is independently C₁-C₃ alkyl;
each R₄ is independently H or C₁-C₃ alkyl; or two R₄, together with the carbon atom linked thereto, form C(O), C₃-C₆ cycloalkyl or 4-, 5- or 6-membered heterocycloalkyl containing 1 or 2 heteroatoms selected from N and O;
R₅ is H, C₁-C₃ alkyl, For Cl;
each Rₐ is independently H or C₁-C₆ alkyl;
R_{b} is H or tosyl;
t is 0 or 1;
m is 0, 1, 2 or 3;
n is 0, 1 or 2.
Ring A, X, X₁, X₂, Y₁₀₀, R₁, R₃, R_{3'}, R₄, R₅, t, m and n are as defined in WO2017/007612 and US2018/0134684, which are incorporated herein by reference in their entirety.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E7, E7b, E7c, E7d or E7e: 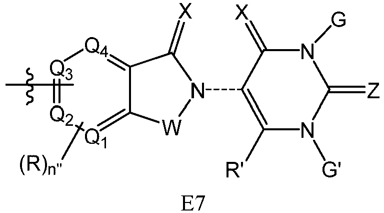 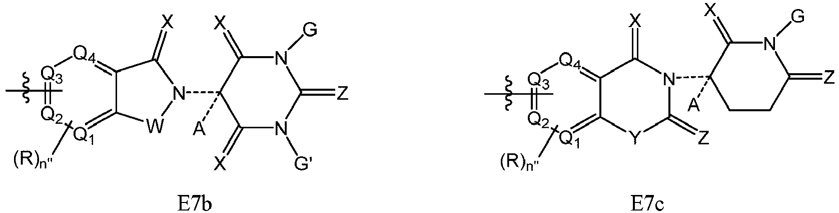 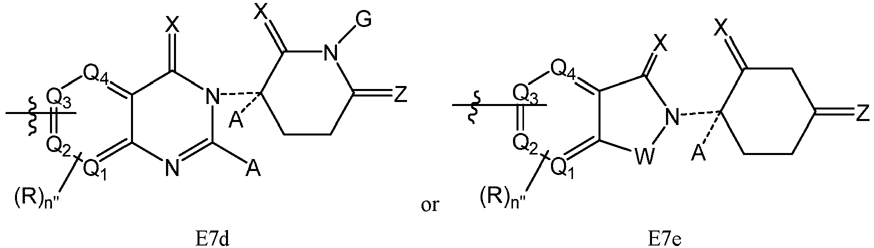 wherein in E7, E7b, E7c, E7d and E7e:
W is CH₂, CRₐR_{b}, C(S), C(O) or SO₂;
X is H₂, CH₂, O or S;
Y is NH, N-alkyl, N-aryl, N-heteroaryl, N-cycloalkyl, N-heterocycloalkyl, O or S;
Z is H₂, CH₂, O or S, and in E7c, Z cannot be H₂ at the same time;
G and G' are each independently selected from hydrogen, C₁-C₆ alkyl, OH, -CH₂-heterocycloalkyl optionally substituted with R' and -CH₂-phenyl optionally substituted with R';
Q₁, Q₂, Q₃ and Q₄ are each independently carbon, nitrogen or nitrogen oxide;
A is hydrogen, C₁-C₆ alkyl, cycloalkyl or halogen;
R, Rₐ and R_{b} are each independently selected from hydrogen, hydroxy, halogen, -NH₂, -N(C₁-C₆ alkyl)₁₋₂, C₁-C₆ alkyl, C₁-C₆ alkoxy, C₁-C₆ haloalkyl, -CONR_{'}R_{"}, -OR, -NR_{'}R_{"}, -SR_{'}, -SO₂R_{'}, -SO₂NR_{'}R_{"}, -CR_{'}R_{"}, -CR_{'}NR_{'}R_{"}, aryl, heteroaryl, C₃-C₈ cycloalkyl, 3-8 membered heterocycloalkyl, -P(O)(OR_{'})R_{"}, -P(O)R_{'}R_{"}, -OP(O)(OR_{'})R_{"}, -Cl, -F, - Br, -I, -CF₃, -CN, -NR_{'}SO₂NR_{'}R_{"}, -NR_{'}C(O)NR_{'}R_{"}, -C(O)NR_{'}C(O)R_{"}, -NR_{'}C(=N-CN)NR_{'}R_{"}, -C(=N-CN)NR_{'}R_{"}, - NR_{'}C(=N-CN)R_{"}, -NR_{'}C(=C-NO2)NR_{'}R_{"}, -SO₂NR_{'}COR_{"}, -NO₂, -COR_{'}, -C(C=N-OR_{'})R_{"}, -CR_{'}=CR_{'}R_{"}, -CCR_{'}, - S(C=O)(C=N-R_{'})R_{"}, -SF₅ and -OCF₃;
R_{'} and R_{"} are each independently selected from a bond, hydrogen, C₁-C₆ alkyl, cycloalkyl, aryl, heteroaryl and heterocycloalkyl;
n^{"} is an integer of 1-4;
----- is a bond, which may be an R stereoisomer, an S stereoisomer, or a non-stereoisomer.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is:

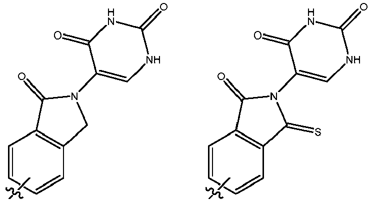

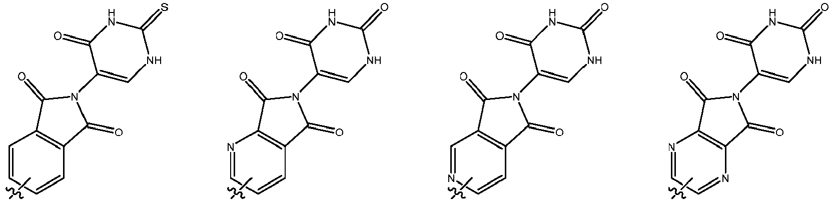

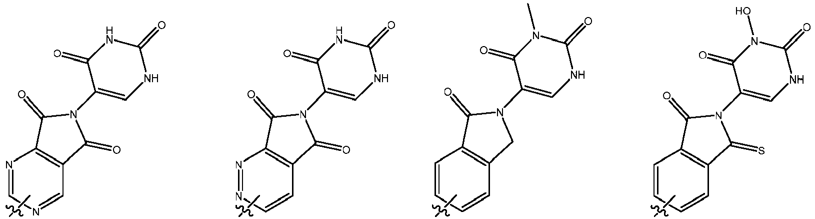

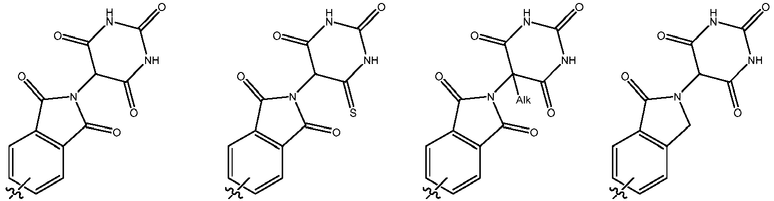

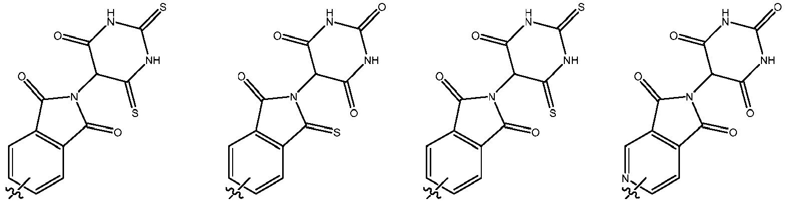

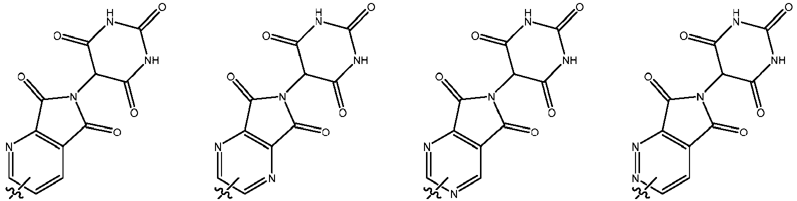

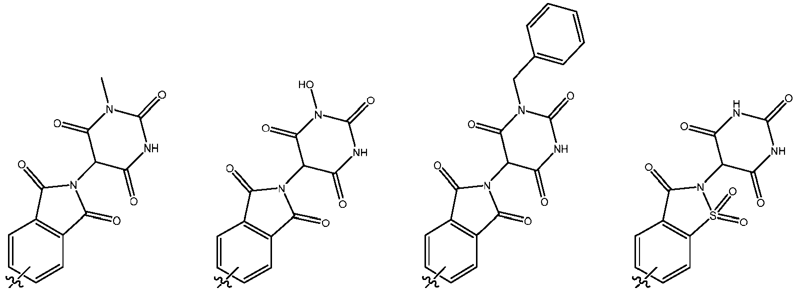

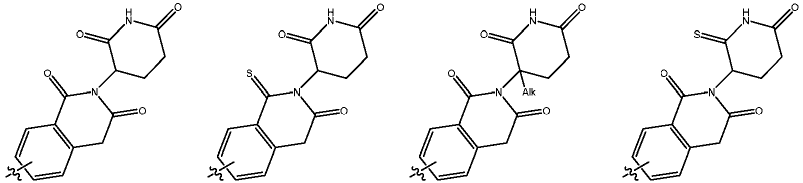

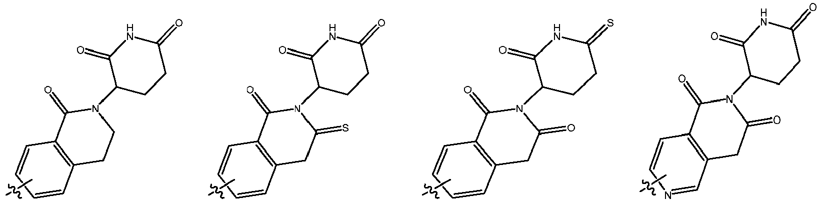

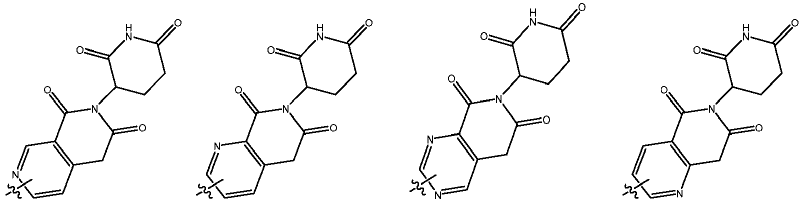

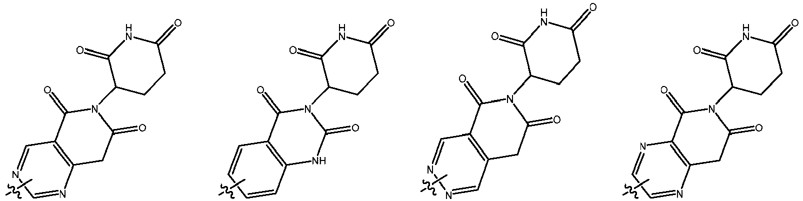

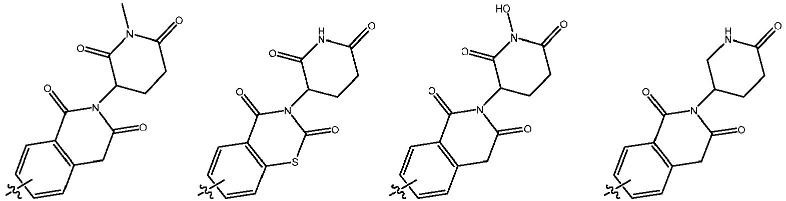

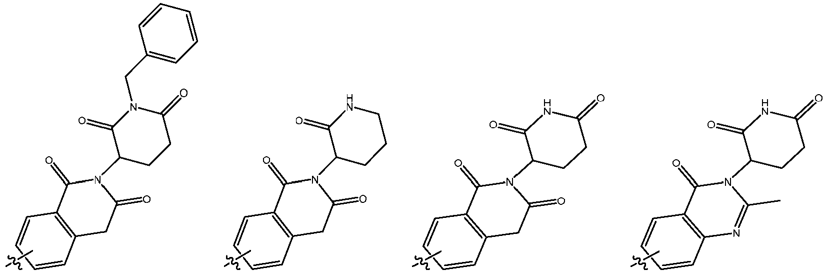

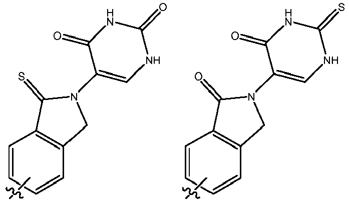

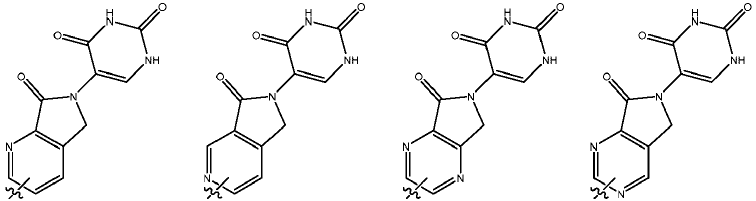

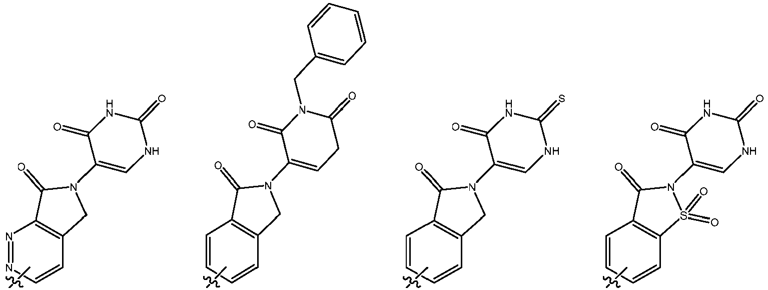

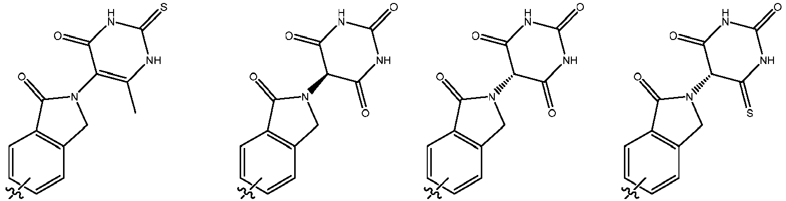

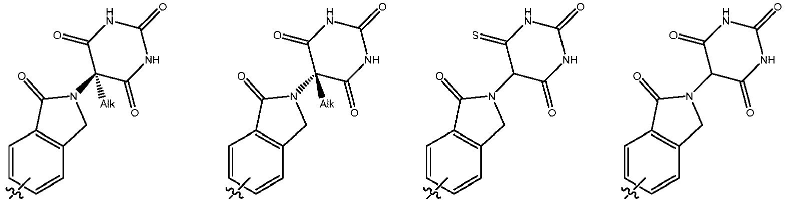

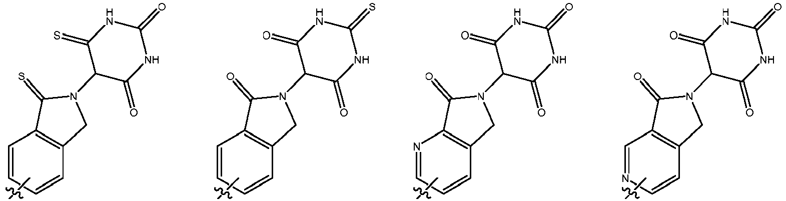

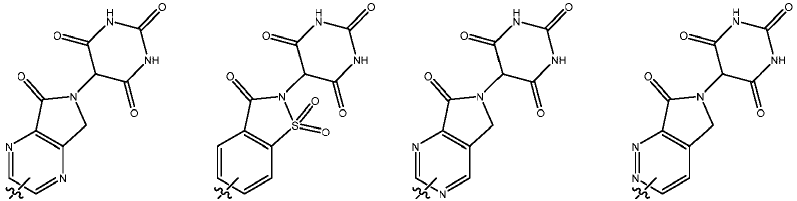

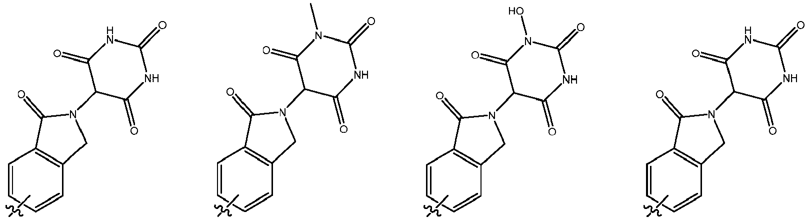

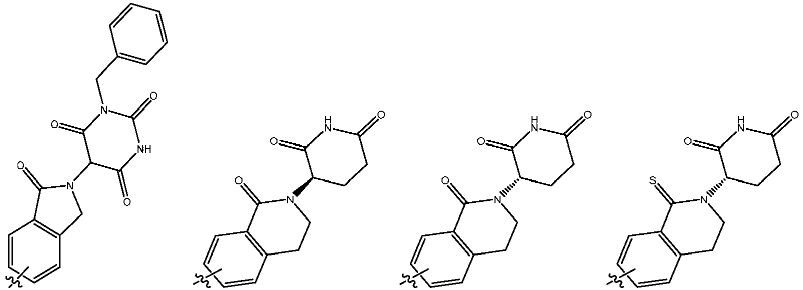

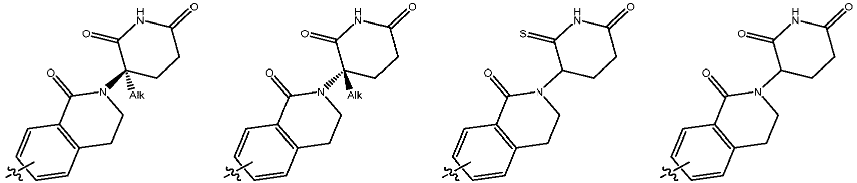

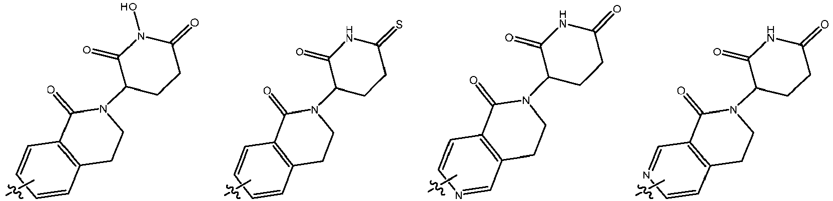

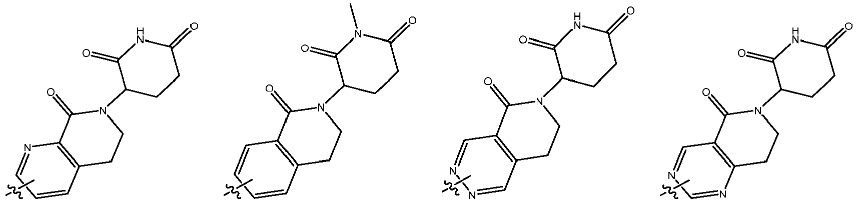

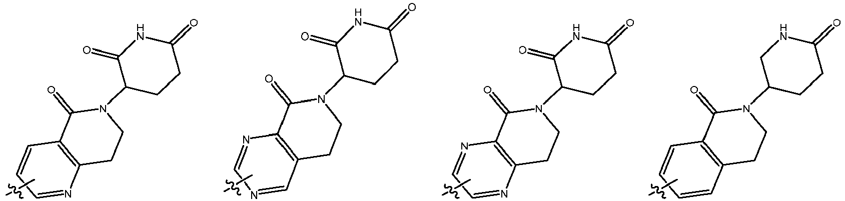

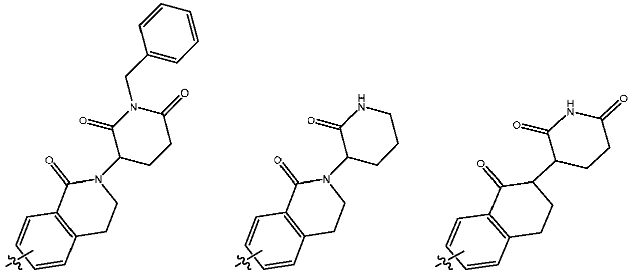

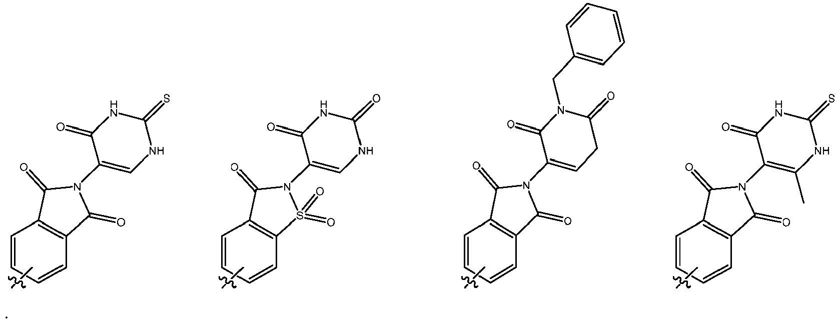

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E8, E8a, E8b, E8c, E8d, E8e, E8f, E8g, E8h, E8i, E8j, E8k, E8l, E8m, E8n, E8o, E8p, E8q, E8s, E8t, E8u, E8v, E8w, E8x, E8y, E8z, E8aa, E8bb or E8cc: 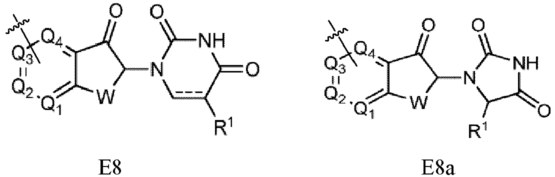 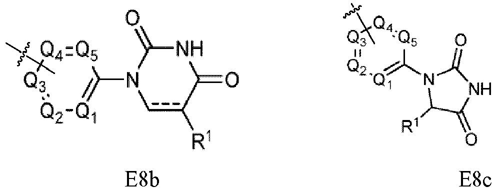 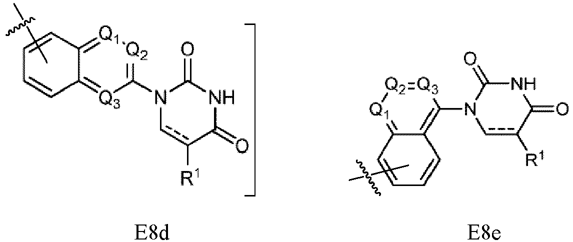 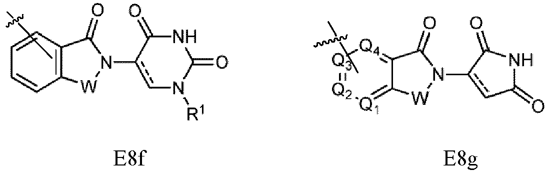 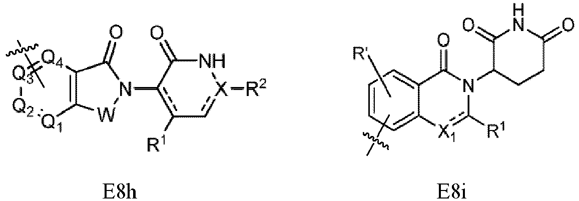 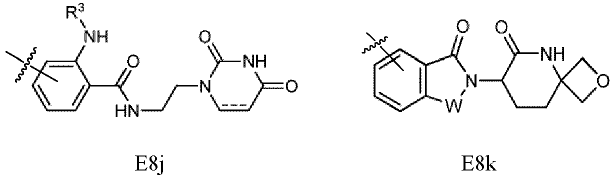 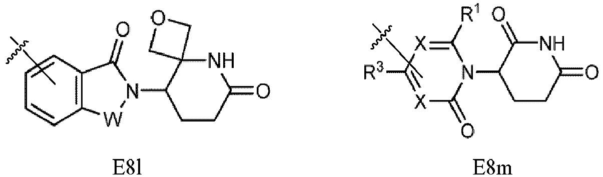 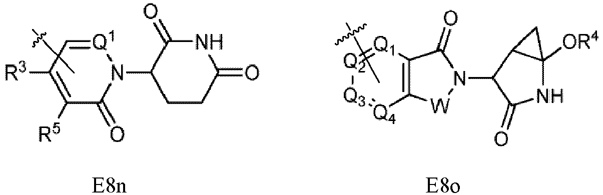 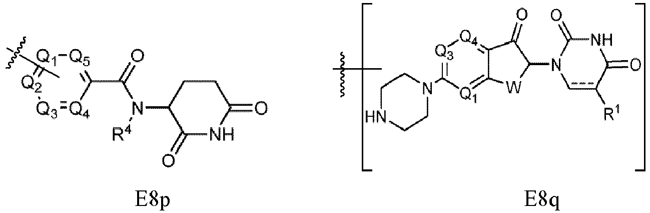 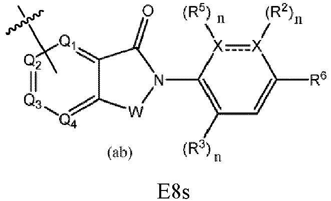 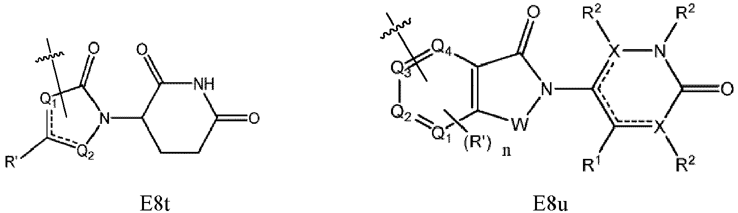 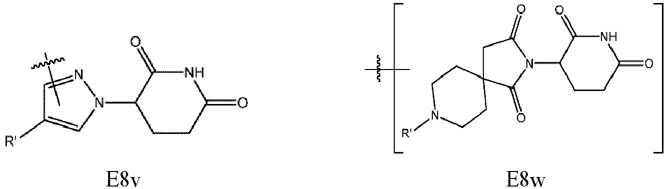 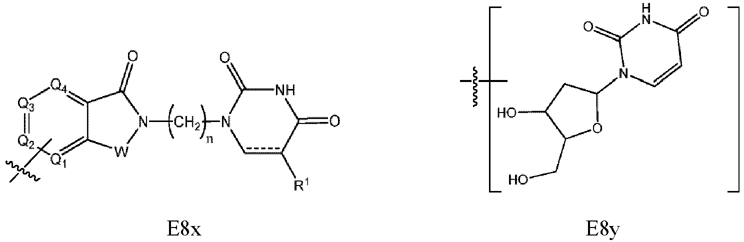 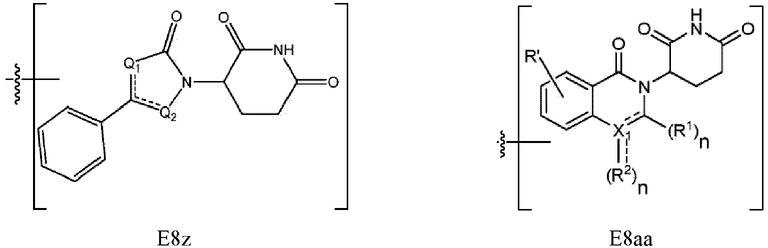 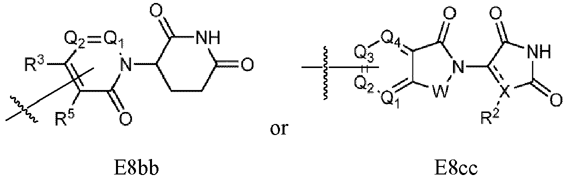 wherein in E8, E8a, E8b, E8c, E8d, E8e, E8f, E8g, E8h, E8i, E8j, E8k, E8l, E8m, E8n, E8o, E8p, E8q, E8s, E8t, E8u, E8v, E8w, E8x, E8y, E8z, E8aa, E8bb or E8cc:
each W is independently selected from CH₂, CHR, C(O) and SO₂;
Q₁, Q₂, Q₃, Q₄ and Q₅ are each independently carbon, N, or N substituted with R', or nitrogen oxide;
R¹ is absent, or is hydrogen, hydroxy, cyano, C₁-C₃ alkyl or C(O);
R² is absent or is hydrogen, hydroxy, cyano, C₁-C₃ alkyl, CHF₂, CF₃, CHO or C(O)NH₂;
R³ is selected from hydrogen, alkyl (e.g., C₁-C₆ alkyl or C₁-C₃ alkyl), optionally substituted alkyl (e.g., C₁-C₆ alkyl or C₁-C₃ alkyl), alkoxy (e.g., C₁-C₆ alkoxy or C₁-C₃ alkoxy) and optionally substituted alkoxy (e.g., C₁-C₆ alkoxy or C₁-C₃ alkoxy);
R⁴ is selected from hydrogen, alkyl and optionally substituted alkyl;
R⁵ or R⁶ are each independently selected from hydrogen, halogen, C(O)R', cyano, hydroxy and CF₃;
X is carbon, nitrogen or C(O);
X₁ is carbon, nitrogen or C(O);
R' is selected from hydrogen, halogen, amine, alkyl (e.g., C₁-C₃ alkyl), optionally substituted alkyl (e.g., C₁-C₃ alkyl), alkoxy (e.g., C₁-C₃ alkoxy), optionally substituted alkoxy (e.g., C₁-C₃ alkoxy), NR²R³, C(O)OR² and optionally substituted phenyl;
R is H, OH, lower alkyl, lower alkoxy, cyano, halogenated lower alkoxy or halogenated lower alkyl;
n is 0, 1, 2, 3 or 4;
----- is a single bond or a double bond.

In one preferred embodiment of the compound of formula I, and/or the stereoisomer, the hydrate, the solvate, the prodrug and/or the pharmaceutically acceptable salt thereof in the present invention, E is E9a, E9b, E9c, E9d, E9e, E9f, E9g, E9h, E9i, E9j, E9k, E9l, E9m, E9n, E9o, E9p, E9q, E9r or E9s: 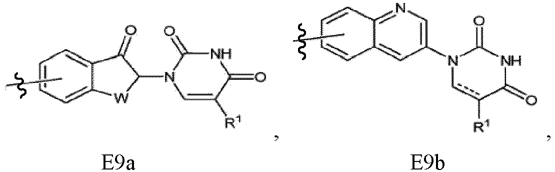 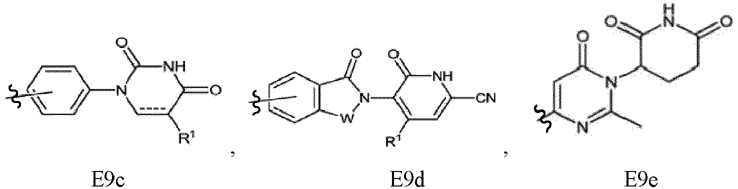 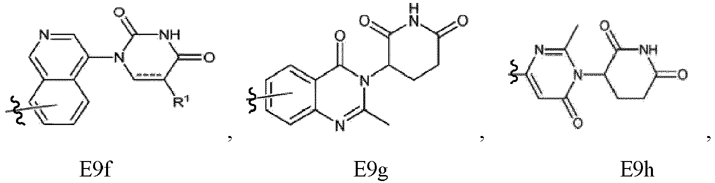 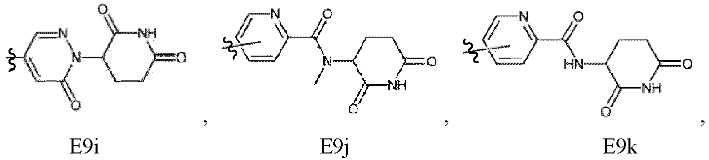 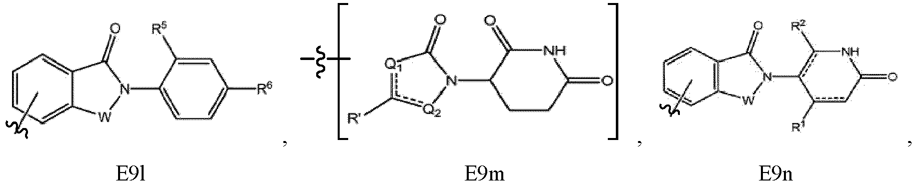 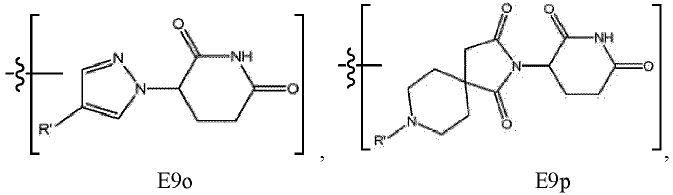 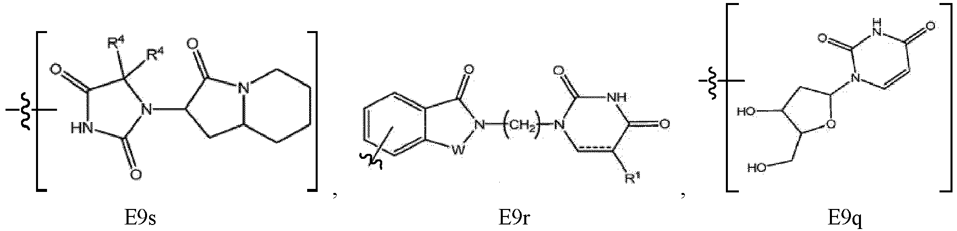 wherein in E9a, E9b, E9c, E9d, E9e, E9f, E9g, E9h, E9i, E9j, E9k, E9l, E9m, E9n, E9o, E9p, E9q, E9r or E9s:
W is independently selected from CH₂, CHR, C=O, SO₂, NH and N-alkyl;
R¹ is selected from H, CN and C₁-C₃ alkyl;
R² is H or C₁-C₃ alkyl;
R⁴ is methyl or ethyl;
R⁵ is H or halogen;
R⁶ is H or halogen;
R is H;
R' is H;
Q₁ and Q₂ are each independently C or N substituted with a group independently selected from Hand C₁-C₃ alkyl;
-̅ -̅ -̅ -̅ -̅ is a single or double bond.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E10, E10a or E10b: 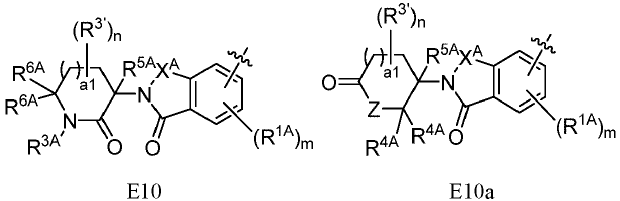 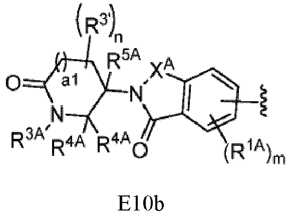 wherein in E10, E10a or E10b: X^{A} is C(O) or C(R^{3A})₂;
Z is NR^{3A} or CR^{3A};
each R^{1A} is independently halogen, hydroxy, -C₁-C₆ alkyl or C₁-C₆ alkoxy;
each R^{3A} is independently hydrogen or C₁-C₃ alkyl;
each R^{3'} is independently C₁-C₃ alkyl;
each R^{4A} is independently hydrogen or C₁-C₃ alkyl, or two R^{4A}, together with the carbon atom linked thereto, form C(O) or C3-C6 cycloalkyl, or together form 3-, 4-, 5- or 6-membered heterocycloalkyl containing 1 or 2 heteroatoms selected from N and O;
R^{5A} is selected from hydrogen, C₁-C₃ alkyl and halogen;
each R^{6A} is independently hydrogen or C₁-C₃ alkyl, or two R^{6A}, together with the carbon atom linked thereto, form C(O), C₃-C₆ cycloalkyl, or together form 3-, 4-, 5- or 6-membered heterocycloalkyl containing 1 or 2 heteroatoms selected from N and O;
m is 0, 1, 2 or 3;
n is 0, 1 or 2;
a1 is 0 or 1.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E11: 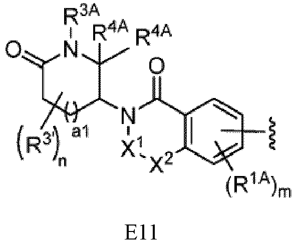
wherein in E11, -X₁-X₂- is -C(R^{3A})=N- or -C(R^{3A})₂-C(R^{3A})₂-;
each R^{1A} is independently halogen, hydroxy, C₁-C₆ alkyl or C₁-C₆ alkoxy;
R^{3A} is hydrogen or C₁-C₃ alkyl;
each R^{3'} is independently C₁-C₃ alkyl;
R^{4A} is hydrogen or C₁-C₃ alkyl; or two R^{4A}, together with the carbon atom linked thereto, form C(O), C₃-C₆ cycloalkyl, or together form 4-, 5- or 6-membered heterocycloalkyl containing 1 or 2 heteroatoms selected from N and O;
m is 0, 1, 2 or 3;
n is 0, 1 or 2;
a1 is 0 or 1.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E12: 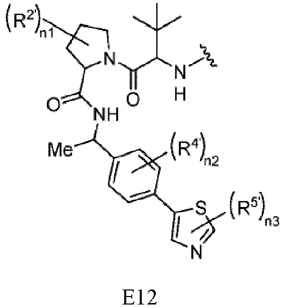 wherein in E12,
each R^{2'} is independently halogen, hydroxy, C₁-C₆ alkyl or C₁-C₆ alkoxy;
each R^{4'} is independently halogen, hydroxy, C₁-C₆ alkyl or C₁-C₆ alkoxy;
each R^{5'} is independently halogen, hydroxy, C₁-C₆ alkyl or C₁-C₆ alkoxy;
n1 is 0, 1, 2, 3, 4, 5 or 6;
n2 is 0, 1, 2, 3 or 4;
n3 is 0, 1 or 2.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E13: 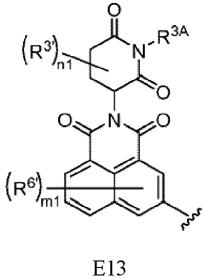 wherein in E13,
R^{3A} is hydrogen or C1-C3 alkyl;
each R^{3'} is C₁-C₃ alkyl;
each R^{6'} is independently halogen, hydroxy, C₁-C₆ alkyl or C₁-C₆ alkoxy;
n1 is 0, 1, 2, 3, 4 or 5;
m1 is 0, 1, 2, 3, 4 or 5.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E14, E14a, E14b, E14c, E14d or E14e: 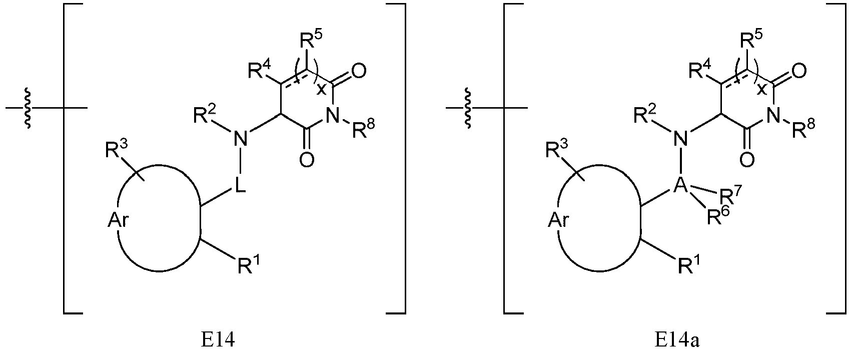 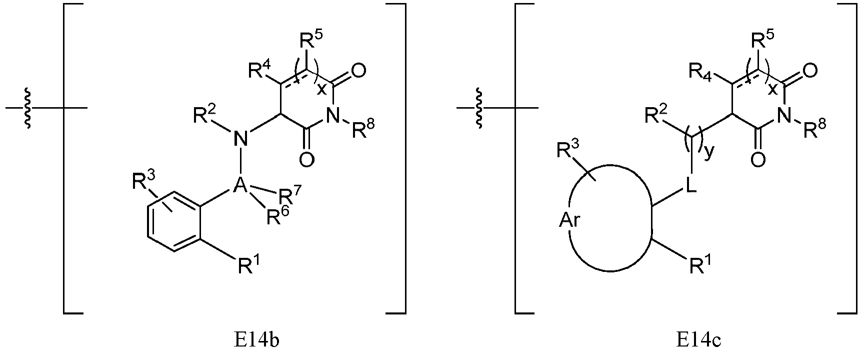 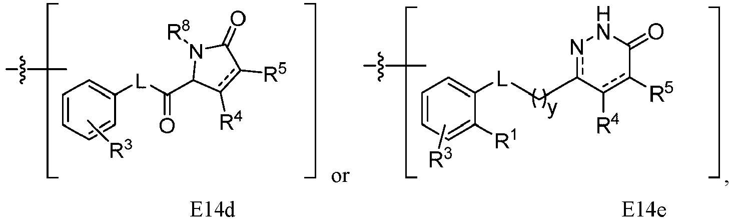 wherein in E14, E14a, E14b, E14c, E14d and E14e,
Ar is aryl, cycloalkyl, heterocycloalkyl or heteroaryl;
L is absent or is -SO₂, -SO₂R', SO₂R'R", -SO₂NR'R", -SO₂NR'R"C(=O), -NR'SO₂R", -R'SO₂NR'R‴, -C(=O), - C(=O)R', -OC(=O)R', -C(=O)NR'R", -NR'C(=O)R", -NR'C(=O), R"C(=O), -OR', -NR'R", -SR', -N₃-C(=O)OR', - O(CR'R")ᵣC(=O)R', -O(CR'R")ᵣNR"C(=O)R', -O(CR'R")ᵣNR"SO₂R', -OC(=O)NR'R', -NR'C(=O)OR" or optionally substituted C₁-C₆ alkyl;
R', R" and R‴ are each independently selected from H, substituted or unsubstituted alkyl, substituted or unsubstituted alkenyl, substituted or unsubstituted ether group, substituted or unsubstituted cycloalkyl, substituted or unsubstituted heterocycloalkyl, substituted or unsubstituted cycloalkenyl, substituted or unsubstituted aryl, substituted or unsubstituted heteroaryl, substituted or unsubstituted arylalkyl, substituted or unsubstituted heteroalkyl, substituted or unsubstituted alkylated acylaryl and substituted or unsubstituted amine; r is an integer of 1 to 6;
R¹, R² and R³ are each independently selected from H, halogen, hydroxy, azide, alkoxy, sulfhydryl, imino, amino, phosphonate, phosphate, carbonyl, carboxy, cyano, silyl, ether, alkylthio, sulfonyl, sulfonamido, ketone, aldehyde, substituted or unsubstituted amine, substituted or unsubstituted amide, nitro, ester, morpholinyl, dioxolane, substituted or unsubstituted alkyl, substituted or unsubstituted haloalkyl, substituted or unsubstituted cycloalkyl, substituted or unsubstituted heterocycloalkyl, substituted or unsubstituted cycloalkenyl, substituted or unsubstituted aryl, substituted or unsubstituted heteroaryl, substituted or unsubstituted arylalkyl, substituted or unsubstituted heteroarylalkyl, substituted or unsubstituted alkylheteroaryl, substituted or unsubstituted alkenyl, substituted or unsubstituted alkynyl, substituted or unsubstituted cycloalkyl, substituted or unsubstituted heterocycloalkyl and a combination thereof;
or R¹ and R² are combined to form a 5- to 7-membered heterocycle, and when R¹ and R² are combined to form a 5-to 7-membered heterocycle, Ar does not form a fused ring with the 5- to 7-membered heterocycle, but is a substituent of the 5- to 7-membered heterocycle; R⁴ and R⁵ are hydrogen, halogen, hydroxyl, azide, ether, alkoxy, sulfhydryl, alkylthio, sulfonyl, sulfonamido amide, ketone, aldehyde, ester, substituted or unsubstituted alkyl, substituted or unsubstituted haloalkyl, substituted or unsubstituted aralkyl, substituted or unsubstituted alkenone, unsubstituted alkenyl, substituted or unsubstituted alkynyl, substituted or unsubstituted cycloalkyl, heterocyclyl or a combination thereof;
R⁸ is hydrogen, substituted or unsubstituted alkyl, substituted or unsubstituted haloalkyl, substituted or unsubstituted aryl, substituted or unsubstituted alkenyl, substituted or unsubstituted alkynyl, substituted or unsubstituted cycloalkyl, heterocyclyl, substituted or unsubstituted aryl, substituted or unsubstituted alkylaryl or a combination thereof;
----- is a single or double bond;
x is 0, 1 or 2;
y is an integer of 1 to 6.

In some embodiments, in E14, E14a, E14b, E14c, E14d and E14e, Ar, R¹, R², R³, R⁴, R⁵, R⁶, R⁷, R⁸, A, L, x, y and ----- are as defined and described in WO2017/161119 (incorporated herein by reference in its entirety).

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E15, E15a, E15b, E15c, E15d, E15e, E15f, E15g or E15h: 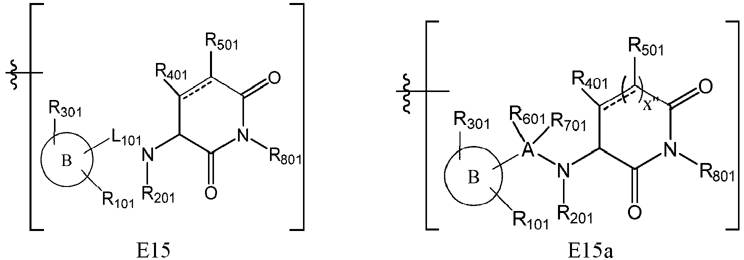 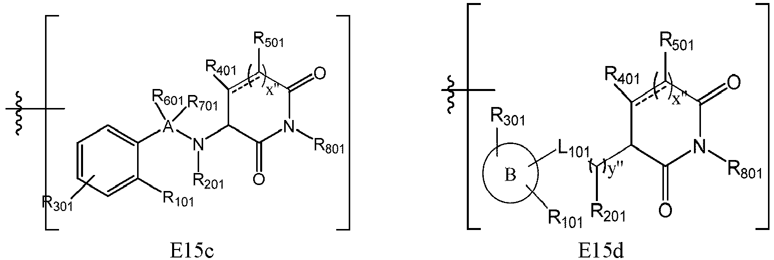 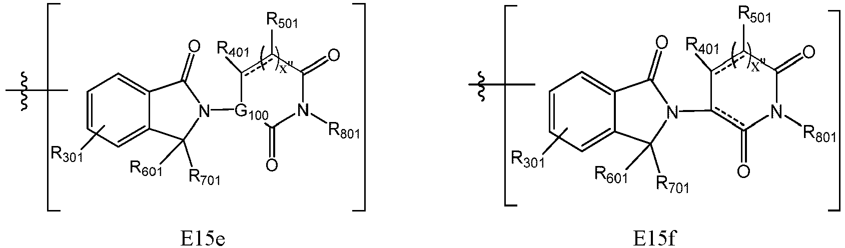 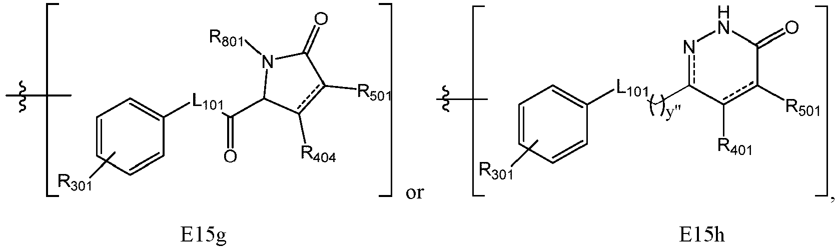 wherein in E15, E15a, E15b, E15c, E15d, E15e, E15f, E15g and E15h:
ring B is aryl, heteroaryl, cycloalkyl or heterocycloalkyl;
L₁₀₁ is a bond or C₁-C₆ alkyl;
A is C, S, substituted or unsubstituted C₁-C₈ alkyl or a combination thereof;
G₁₀₀ is C, S, N, substituted or unsubstituted C₁-C₈ alkyl or a combination thereof;
R₁₀₁, R₂₀₁ and R₃₀₁ are each selected from hydrogen, halogen, hydroxy, azide, alkoxy, sulfhydryl, imino, amido, phosphonate, phosphinate, carbonyl, carboxy, cyano, silyl, ether group, alkylthio, sulfonyl, sulfonamido, ketone, aldehyde, substituted or unsubstituted amine, substituted or unsubstituted amide, nitro, ester, morpholino, dioxolane, substituted or unsubstituted alkyl, substituted or unsubstituted alkyl halide, substituted or unsubstituted aralkyl, substituted or unsubstituted alkenyl, substituted or unsubstituted alkynyl, substituted or unsubstituted cycloalkyl, substituted or unsubstituted heterocycloalkyl, substituted or unsubstituted cycloalkenyl, substituted or unsubstituted aryl, substituted or unsubstituted heteroaryl, substituted or unsubstituted heteroalkyl, substituted or unsubstituted alkylheteroaryl and a combination thereof; or wherein R₁₀₁ and R₂₀₁ are combined to form 5- to 7-membered heterocycloalkyl; wherein when R₁₀₁ and R₂₀₁ are combined to form a 5- to 7-membered heterocycle, ring B is optionally not fused to the 5- to 7-membered heterocycle, but is a substituent of the 5- to 7-membered heterocycle; R₄₀₁ and R₅₀₁ are each selected from hydrogen, halogen, hydroxy, azide, ether, alkoxy, sulfhydryl, alkylthio, sulfonyl, sulfonamido, ketone, aldehyde, ester, substituted or unsubstituted alkyl, substituted or unsubstituted alkyl halide, substituted or unsubstituted aralkyl, substituted or unsubstituted alkenyl, substituted or unsubstituted alkynyl, substituted or unsubstituted cycloalkyl, heterocycloalkyl and a combination thereof;
R₆₀₁ and R₇₀₁ are each independently oxo, hydrogen or C₁-C₈ alkyl, or R₆₀₁ and R₇₀₁ are combined together to form oxo;
R₈₀₁ is selected from hydrogen, substituted or unsubstituted alkyl, substituted or unsubstituted alkyl halide, substituted or unsubstituted aralkyl, substituted or unsubstituted alkenyl, substituted or unsubstituted alkynyl, substituted or unsubstituted cycloalkyl, heterocycloalkyl, substituted or unsubstituted aryl, substituted or unsubstituted alkaryl and a combination thereof;
x" and y" are each independently 0, 1 or 2;
-̅ -̅ -̅ -̅ -̅ -̅ is a single or double bond;

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E16, E16a or E16b: 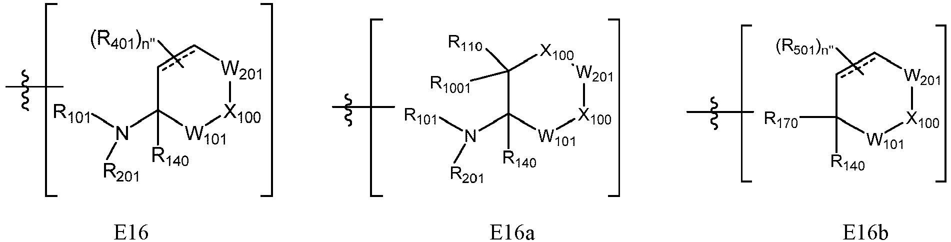 wherein in E16, E16a and E16b:
W₁₀₁ is CR₆₀₁R₇₀₁, C(O), C(S), C=CH₂, SO₂, S(O), P(O)O alkyl, P(O)NH alkyl, P(O)N (alkyl)₂, P(O) alkyl, P(O)OH or P(O)NH₂;
W₂₀₁ is CR₈₀₁R₉₀₁, C(O), C(S), C=CH₂, SO₂, S(O), P(O)O alkyl, P(O)NH alkyl, P(O)N (alkyl)₂, P(O) alkyl, P(O)OH or P(O)NH₂;
X₁₀₀ is independently selected from NH, NR₃₀₁, CH₂, CHR₃₀₁, C(R₃₀₁)₂, O and S;
n" is 0, 1, 2 or 3;
-̅ -̅ -̅ -̅ -̅ -̅ is a single or double bond; wherein when -̅ -̅ -̅ -̅ -̅ -̅ is a single bond, n" is 0, 1, 2 or 3; when -̅ -̅ -̅ -̅ -̅ -̅ is a double bond, n" is 0, 1 or 2;
R₁₀₁ is selected from: 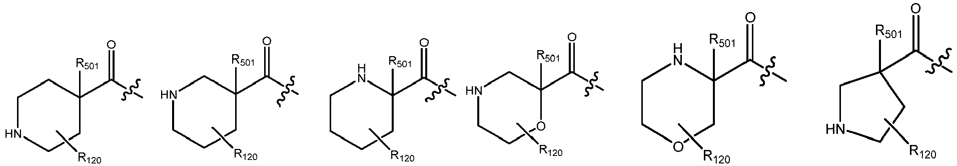 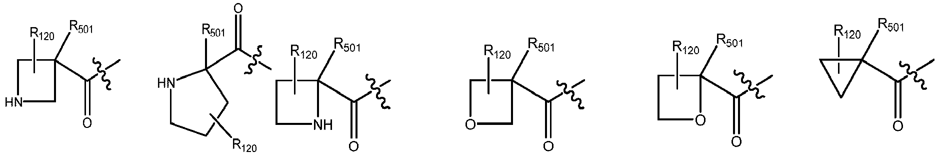 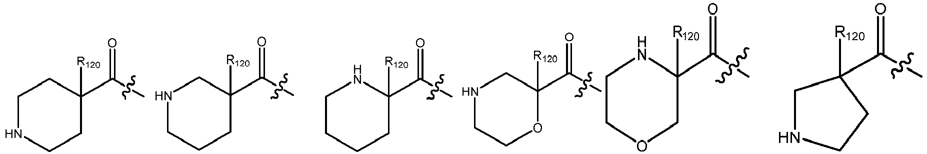 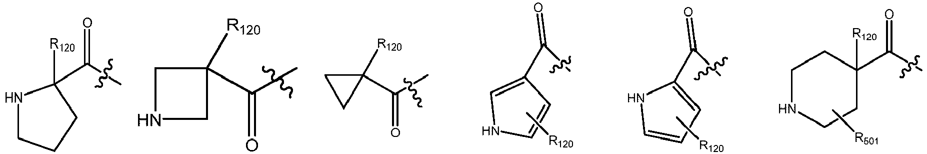 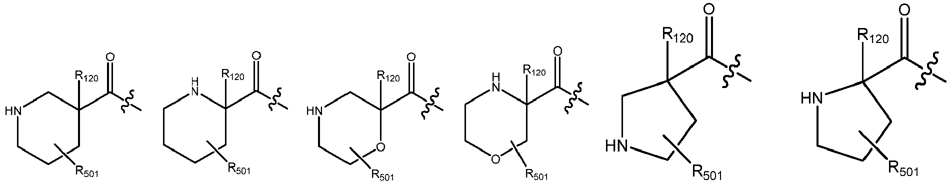 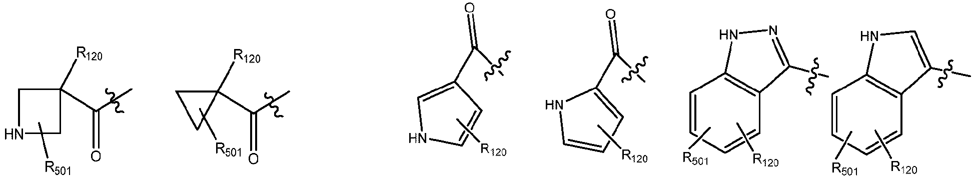 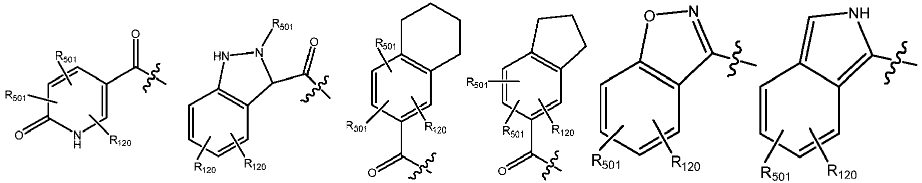 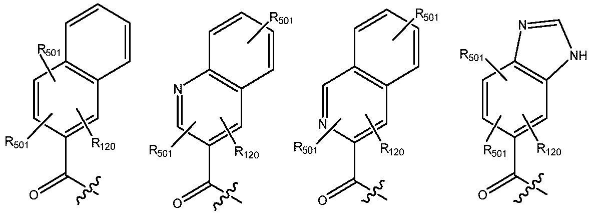 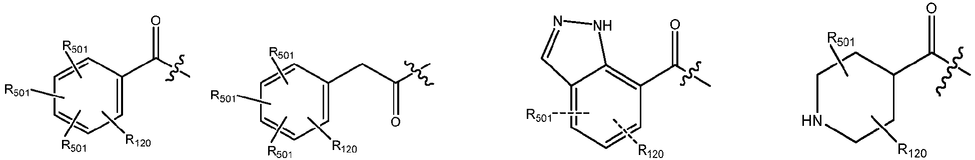 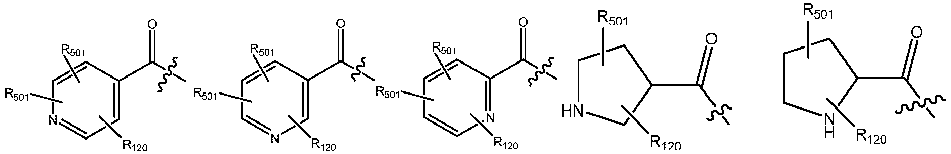 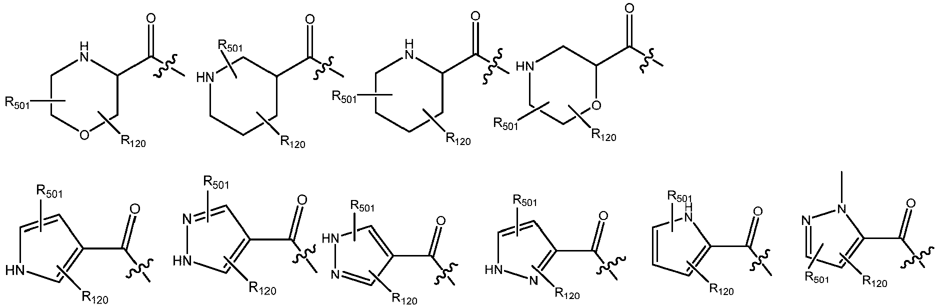
and R₁₀₁*;
R₂₀₁ is alkyl, hydrogen, heteroalkyl, aryl, heteroaryl or heterocycloalkyl;
or R₁₀₁ and R₂₀₁ are combined to form 4-, 5-, 6-, 7-, 8-, 9- or 10-membered heterocycloalkyl or heteroaryl, and the heterocycloalkyl or the heteroaryl is optionally substituted with one or more substituents selected from and R₁₂₀;
R₁₀₁* is selected from: 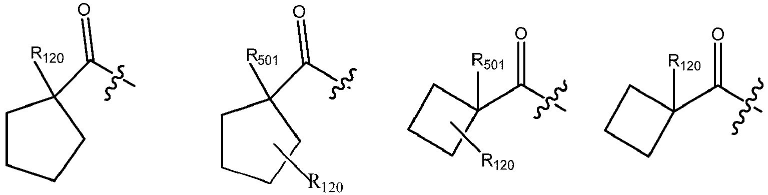 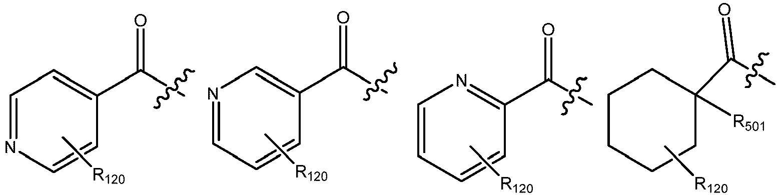 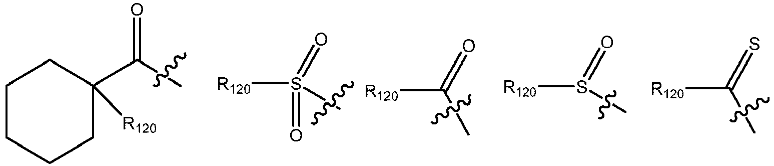
R₃₀₁ is selected from: alkyl, -C(O)H, C(O)OH, -C(O) alkyl, -C(O)O alkyl, alkenyl, alkynyl, aryl, heteroaryl and heteroalkyl;
R₄₀₁ is selected from alkyl, alkenyl, alkynyl, halogen, hydroxy, alkoxy, azido, amino, cyano, -NH(alkyl), -N(alkyl)₂, -NHS(O)₂(alkyl), -N(alkyl)SO₂alkyl, -NHSO₂(aryl, heteroaryl or heterocycloalkyl), -N(alkyl)SO₂(aryl, heteroaryl or heterocycloalkyl), -NHSO₂(alkenyl), -N(alkyl)SO₂(alkenyl), -NHSO₂(alkynyl), -N(alkyl)SO₂(alkynyl), haloalkyl, aryl, heteroaryl, heteroalkyl and cycloalkyl;
or two R₄₀₁, together with the carbon atom linked thereto, form a 3-, 4-, 5- or 6-membered ring;
and R₁₄₀ are each independently selected from hydrogen, alkyl, alkenyl, alkynyl, halogen, hydroxy, alkoxy, azido, amino, cyano, -NH(alkyl), -N(alkyl)₂, -NHS(O)₂(alkyl), -N(alkyl)SO₂(alkyl), -NHSO₂(aryl, heteroaryl or heterocycloalkyl), -N(alkyl)SO₂(aryl, heteroaryl or heterocycloalkyl), -NHSO₂(alkenyl), -N(alkyl)SO₂(alkenyl), - NHSO₂(alkynyl), -N(alkyl)SO₂(alkynyl), haloalkyl, aryl, heteroaryl, heteroalkyl and cycloalkyl;
or is independently selected from C(O)R₄₀₁, cyano, aryl, aryloxy, heterocycloalkyl, heteroaryl, arylalkyl, alkoxy, hydroxy, O-aralkyl and cycloalkyl;
R₆₀₁, R₇₀₁, R₈₀₁, R₉₀₁, R₁₀₀₁ or R₁₁₀ is independently selected from hydrogen, alkyl, heteroalkyl, hydroxy, alkoxy, amine, -NH(alkyl) and -N(alkyl)₂;
or R₆₀₁ and R₇₀₁, together with the carbon atom linked thereto, form a 3-, 4-, 5- or 6-membered spiro carbon ring, or form a 4-, 5- or 6-membered spiro heterocycle containing 1 or 2 heteroatoms selected from N and O;
or R₈₀₁ and R₉₀₁, together with the carbon atom linked thereto, form a 3-, 4-, 5- or 6-membered spiro carbon ring, or form a 4-, 5- or 6-membered spiro heterocycle containing 1 or 2 heteroatoms selected from N and O;
or R₁₀₀₁ and R₁₁₀, together with the carbon atom linked thereto, form a 3-, 4-, 5- or 6-membered spiro carbon ring, or form a 4-, 5- or 6-membered spiro heterocycle containing 1 or 2 heteroatoms selected from N and O;
or R₆₀₁ and R₈₀₁ together form 1 or 2 carbon bridged rings;
or R₆₀₁ and R₁₀₀₁ together form 1 or 2 carbon bridged rings;
or R₈₀₁ and R₁₀₀₁ together form 1 or 2 carbon bridged rings;
or R₆₀₁ and R₁₄₀ together form 3, 4, 5 or 6 carbon fused rings;
or R₁₄₀ and R₁₀₀₁ together form 3, 4, 5 or 6 carbon fused rings;
or R₈₀₁ and R₁₄₀ together form 1 or 2 carbon bridged rings;
or R₄₀₁ and R₁₄₀ together form 3, 4, 5 or 6 carbon fused rings, wherein is located on the α carbon of R₁₄₀, or together form 1, 2, 3 or 4 carbon bridged rings, wherein is not located on the α carbon of R₁₄₀;
R₁₂₀ is L₁₀₀;
R₁₇₀ is selected from: 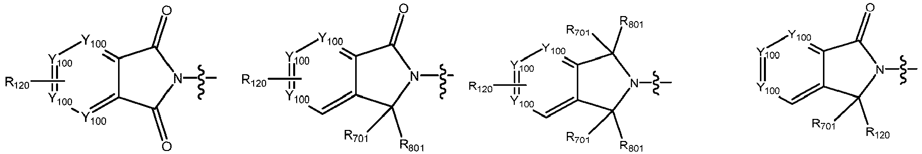 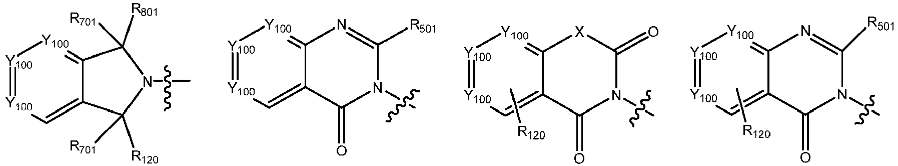 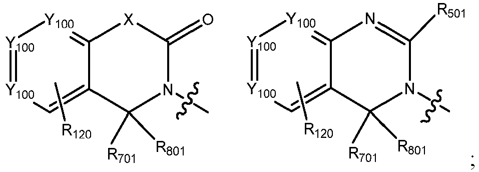
Y₁₀₀ is selected from N, CH and CR₁₀₁, wherein 0, 1, 2 or 3 Y₁₀₀ are N;
R₁₀₁ is independently selected from hydrogen, alkyl, alkenyl, alkynyl, haloalkyl, alkoxy, hydroxy, aryl, heteroaryl, heterocycloalkyl, aralkyl, heteroaralkyl, heterocycloalkyl, aryloxy, heteroaryloxy, CN, -C(O)O alkyl, -C(O)OH, NO₂, F, Cl, Br, I, CF₃, NH₂, NH(alkyl), N(alkyl)₂ and heteroalkyl.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E17, E17a, E17b or E17c, 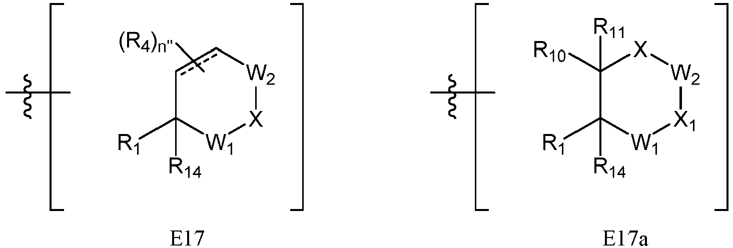 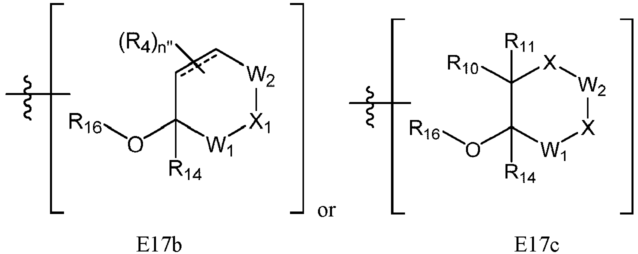 wherein in E17, E17a, E17b and E17c,
W₁ is CR₆R₇, C=O, C=S, C=CH₂, SO₂, S(O), P(O)O alkyl, P(O)NH alkyl, P(O)N (alkyl)₂, P(O) alkyl, P(O)OH or P(O)H₂;
W₂ is CR₈R₉, C=O, C=S, C=CH₂, SO₂, S(O), P(O)O alkyl, P(O)NH alkyl, P(O)N (alkyl)₂, P(O) alkyl, P(O)OH or P(O)H₂;
X and X₁ are each independently selected from NH, NR³, CH₂, CHR³, C(R³)₂, O and S;
n" is 0, 1, 2 or 3;
-̅ -̅ -̅ -̅ -̅ -̅ is a single or double bond; and when -̅ -̅ -̅ -̅ -̅ -̅ is a single bond, n" is 0, 1, 2 or 3; when -̅ -̅ -̅ -̅ -̅ -̅ is a double bond, n" is 0, 1 or 2;
R₁ is 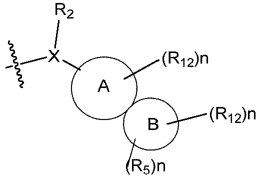 or R₁*, wherein X is O or N, and when X is O, R₂ is absent;
ring A is phenyl, 5- to 6-membered heteroaryl, 3- to 6-membered cycloalkyl or 4- to 6-membered heterocycloalkyl, and the phenyl, the 5- to 6-membered heteroaryl, the 3- to 6-membered cycloalkyl or the 4- to 6-membered heterocycloalkyl is optionally substituted with R¹²,
ring B is absent, or is phenyl or 5- to 6-membered heteroaryl, and the phenyl or the 5- to 6-membered heteroaryl is optionally substituted with R₅ or R₁₂;
R₂ is alkyl, H, an aliphatic group, a heteroaliphatic group, aryl, heteroaryl or heterocycloalkyl;
R₁* is optionally substituted aryl, heteroaryl, cycloalkyl, heterocycloalkyl, alkylamino, alkoxy, an ester group, sulfonyl or amido;
R₃ is alkyl, -C(O)H, -C(O)OH, -C(O) alkyl, -C(O)O alkyl, alkenyl or alkynyl;
R₄ is alkyl, alkenyl, alkynyl, halogen, hydroxy, alkoxy, azido, amino, -NH alkyl, -N(alkyl)₂, -NHSO₂ alkyl, - N(alkyl)SO₂ alkyl, -NHSO₂ aryl, -N(alkyl)SO₂ aryl, -NHSO₂ alkenyl, -N(alkyl)SO₂ alkenyl, -NHSO₂ alkynyl, - N(alkyl)SO₂ alkynyl or haloalkane;
or 2 R₄, together with the C atom linked thereto, form a 3-, 4-, 5- or 6-membered ring;
R₆, R₇, R₈, R₉, R₁₀ and R₁₁ are independently selected from hydrogen, alkyl, aliphatic series, heteroaliphatic series, hydroxy, alkoxy, amine, -NH(aliphatic series, including alkyl) and -N(aliphatic series, including alkyl)₂;
or R₆ and R₇, together with the carbon atom bonded thereto, form a 3-, 4-, 5- or 6-membered spiro carbon ring, or a 4-, 5- or 6-membered spiro heterocycle, the heteroatom being 1 or 2 N or O;
or R₈ and R₉, together with the carbon atom bonded thereto, form a 3-, 4-, 5- or 6-membered spiro carbon ring, or a 4-, 5- or 6-membered spiro heterocycle, the heteroatom being 1 or 2 N or O;
or R₁₀ and R₁₁, together with the carbon atom bonded thereto, form a 3-, 4-, 5- or 6-membered spiro carbon ring, or a 4-, 5- or 6-membered spiro heterocycle, the heteroatom being 1 or 2 N or O;
or R₆ and R₈ form 1 or 2 carbon bridged rings;
or R₆ and R₁₀ form 1 or 2 carbon bridged rings;
or R₈ and R₁₀ form 1 or 2 carbon bridged rings;
or R₁₄ and R₆ form 3, 4, 5 or 6 carbon fused rings;
or R₁₄ and R₁₀ form 3, 4, 5 or 6 carbon fused rings;
or R₁₄ and R₈ form 1 or 2 carbon bridged rings;
or R₁₄ and R₄ form 3, 4, 5 or 6 carbon fused rings, and R₄ is on the α carbon linked to R₁₄, or form 1, 2, 3 or 4 carbon bridged rings, and R₄ is not on the α carbon linked to R₁₄;
R₁₂ is a chain linking the target ligand;
R₅ and R₁₄ are each independently selected from hydrogen, alkyl, alkene, alkyne, halogen, hydroxy, alkoxy, azido, amino, cyano, -NH(aliphatic series, including alkyl), -N(aliphatic series, including alkyl)₂, -NHSO₂(aliphatic series, including alkyl), -N(aliphatic series, including alkyl)SO₂ alkyl, -NHSO₂(aryl, heteroaryl or heterocycle), C(O)R₄, - N(alkyl)SO₂(aryl, heteroaryl or heterocycle), -NHSO₂ alkenyl, -N(alkyl)SO₂ alkenyl, -NHSO₂ alkynyl, - N(alkyl)SO₂ alkynyl, haloalkyl, aliphatic series, heteroaliphatic series, aryl, heteroaryl, heteroalkyl and carboncycle;
wherein each R₅ may be optionally substituted with one or more groups selected from alkyl, alkene, alkyne, halogen, hydroxy, alkoxy, azido, amino, -NH alkyl, -N(alkyl)₂, aryl, heterocycloalkyl, heteroaryl, haloalkyl and cycloalkyl;
R₁₆ is

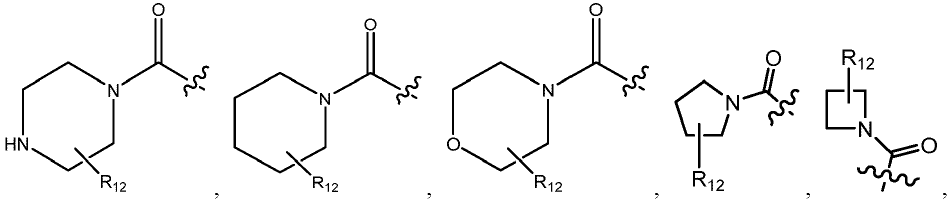

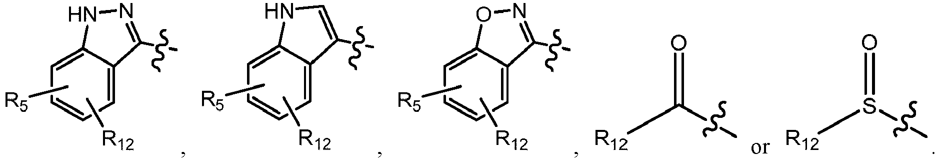

In some embodiments, in E17, E17a, E17b and E17c, R₁, R₄, R₁₀, R₁₁, R₁₄, R₁₆, W₁, W₂, X, -̅ -̅ -̅ -̅ -̅ -̅ and n" are as defined in WO2018/237026, which is incorporated herein by reference in its entirety.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E18, E18a, E18b, E18c, E18d, E18e, E18f, E18g, E18h, E18i, E18j E18k, E181 or E18m, 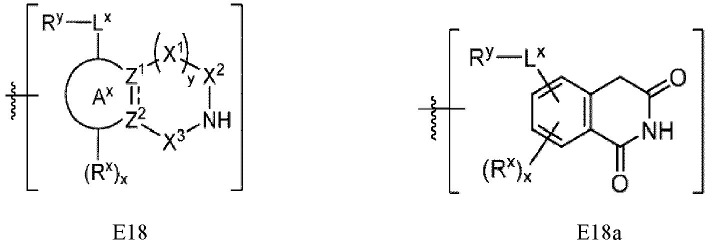 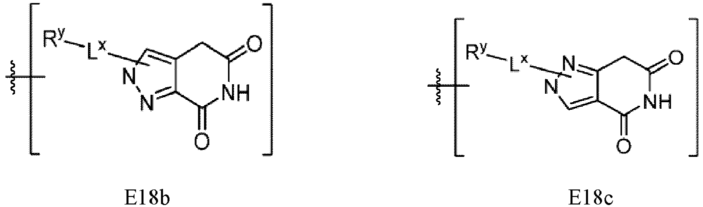 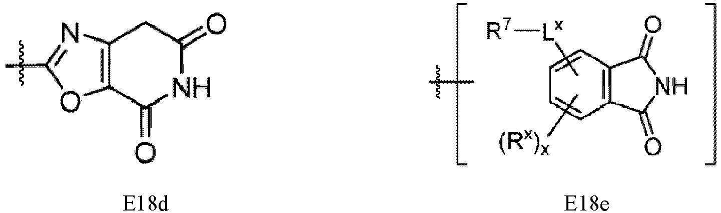 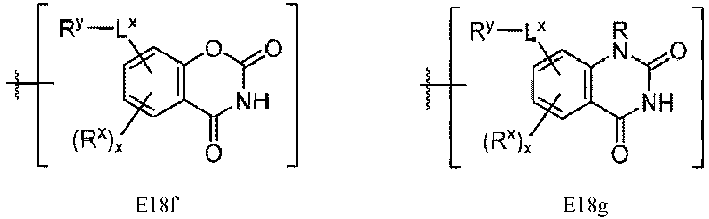 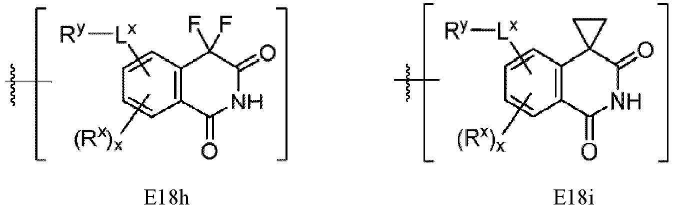 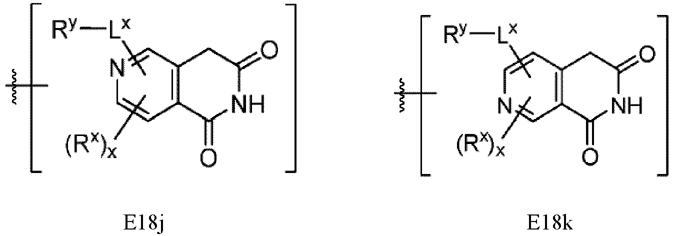 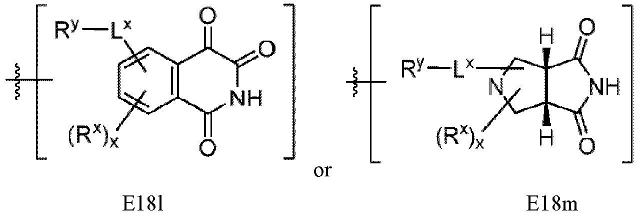 wherein in E18, E18a, E18b, E18c, E18d, E18e, E18f, E18g, E18h, E18i, E18j, E18k, E181 and E18m,
X¹ is -C(R)₂, -O-, -NR-, -CF₂-, 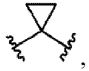 -C(O)-, -C(S)- or 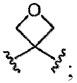
X² and X³ are each independently -CH₂-, -C(O)-, -C(S)-, -C(R)₂C(O)- or 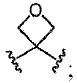
Z¹ and Z² are each independently a carbon atom or a nitrogen atom;
ring A^{x} is selected from phenyl, 4- to 6-membered saturated or partially unsaturated cycloalkyl or heterocycloalkyl containing 1-3 heteroatoms selected from N, O and S and 5- to 6-membered heteroaryl containing 1-4 heteroatoms selected from N, O and S;
L^{x} is a bond or C₁-C₃ alkylene, and 1-2 methylene groups in the alkylene are optionally replaced by a group selected from -O-, -S-, -C(O)-, -C(S)-, -C(R)₂-, -CRF, -CF₂-, -NR-, -S(O)₂-, -CH=CH- and alkynylene;
R^{x} is selected from hydrogen, deuterium, -R^{z}, halogen, -CN, -NO₂, -OR, -SR, -N(R)₂, -S(O)R, -S(O)₂R, - S(O)₂N(R)₂, -CF(R)₂-, -CF₂R-, -CF₃-, -C(R)₂(OR)-, -C(R)₂[N(R)₂]-, -C(O)R, -C(O)OR, -C(O)N(R)₂, - C(O)N(R)OR, -OC(O)R, -OC(O)N(R)₂, -C(S)NR₂, -NRC(O)OR, -NRC(O)R, -NRC(O)N(R)₂, -NRS(O)₂R,-C(R)₂N(R)C(O)R, -C(R)₂N(R)C(O)N(R)₂, -OP(O)(R)₂, -OP(O)(OR)₂, -OP(O)(OR)[N(R)₂], -OP(O)[N(R)₂], - NP(O)(R)₂, -NRP(O)(OR)₂, -N(R)P(O)(OR)[N(R)₂], -N(R)P(O)[N(R)₂]₂, -Si(OR)R₂ and -SiR₃; or
two R^{x}, together with the atom linked thereto, form optionally substituted 5- to 8-membered partially unsaturated or aryl-fused heterocycloalkyl containing 0-2 heteroatoms selected from N, O and S;
each R is independently hydrogen or a optionally substituted group selected from C₁-C₆ alkyl, phenyl, 4- to 7-membered saturated or partially unsaturated heterocycloalkyl containing 1-2 heteroatoms selected from N, O and S and a 5- to 6-membered heteroaryl ring containing 1-4 heteroatoms selected from N, O and S; or
two R on the same carbon atom or the same nitrogen atom, together with the atom linked thereto, form optionally substituted 4- to 7-membered saturated or partially unsaturated cycloalkyl or heterocycloalkyl, or form heteroaryl containing 1-4 heteroatoms selected from N, O and S;
R^{y} is selected from hydrogen and 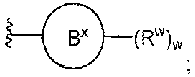
ring B^{x} is selected from phenyl, 4- to 10-membered saturated or partially unsaturated monocyclic or bicyclic cycloalkyl, heterocycloalkyl containing 1-3 heteroatoms selected from N, O and S and 5- to 6-membered heteroaryl containing 1-4 heteroatoms selected from N, O and S, and ring B^{x} is further optionally substituted with 1-2 oxo;
each R^{w} is independently selected from hydrogen, deuterium, R_{z}, halogen, -CN, -NO₂, -OR, -SR, -N(R)₂, -S(O)R, - S(O)₂R, -S(O)₂N(R)₂, -CF(R)₂-, -CF₂R-, -CF₃-, -C(R)₂(OR)-, -C(R)₂[N(R)₂]-, -C(O)R, -C(O)OR, -C(O)N(R)₂, - C(O)N(R)OR, -OC(O)R, -OC(O)N(R)₂, -C(S)NR₂, -NRC(O)OR, -NRC(O)R, -NRC(O)N(R)₂, -NRS(O)₂R, - C(R)₂N(R)C(O)R, -C(R)₂N(R)C(O)N(R)₂, -OP(O)(R)₂, -OP(O)(OR)₂, -OP(O)(OR)[N(R)₂], -OP(O)[N(R)₂], - NP(O)(R)₂, -NRP(O)(OR)₂, -N(R)P(O)(OR)[N(R)₂], -N(R)P(O)[N(R)₂]₂, -Si(OR)R₂ and -SiR₃;
each R_{z} is independently C₁-C₆ alkyl, phenyl, 4- to 7-membered saturated or partially unsaturated heterocycloalkyl containing 1-2 heteroatoms selected from N, O and S or a 5- to 6-membered heteroaryl ring containing 1-4 heteroatoms selected from N, O and S;
x is 0, 1, 2, 3 or 4;
y is 0, 1 or 2;
w is 0, 1, 2, 3 or 4.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E19, E19a, E19b, E19c, E19d, E19e, E19f, E19g, E19h or E19i, 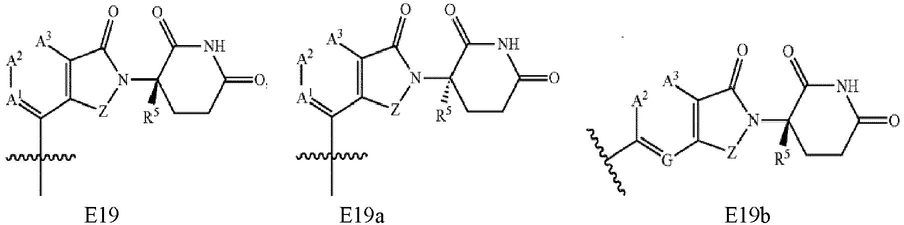 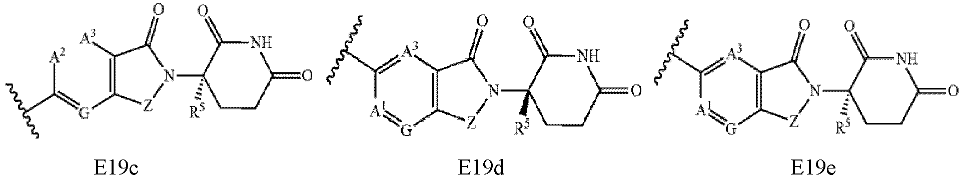 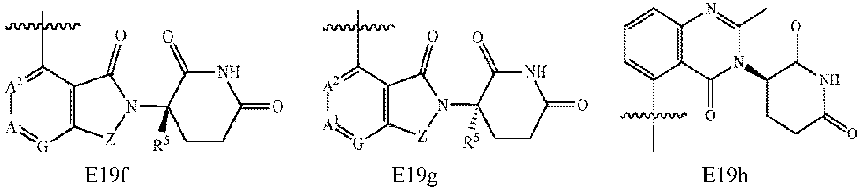 or 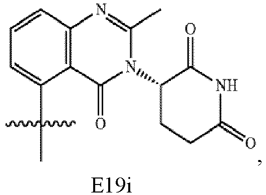 wherein in E19, E19a, E19b, E19c, E19d, E19e, E19f, E19g, E19h and E19i,
A¹ is -C(R^{16a})= or -N=;
A² is -C(R^{16b})= or -N=;
A³ is -C(R^{16c})= or -N=;
G is -C(R^{16d})= or -N=;
Z is -CH₂ or -C(=O)-;
R⁵ is H, methyl or F;
R^{16a}, R^{16b}, R^{16c} and R^{16d} are each independently selected from H, halogen and C₁-C₄ alkyl.

In some embodiments, in E19, E19a, E19b, E19c, E19d, E19e, E19f, E19g, E19h and E19i, A¹, A², A³, Z, G and R⁵ are as defined and described in WO2017/176958 (incorporated herein by reference in its entirety).

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E20, E20a, E20b, E20c, E20d, E20e or E20f, 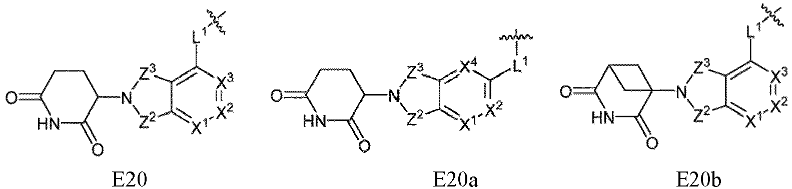 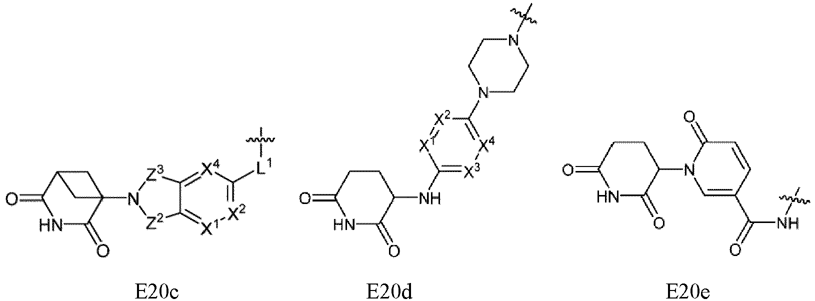 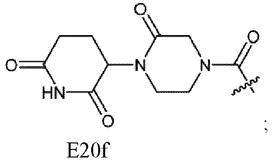 wherein in E20, E20a, E20b, E20c, E20d, E20e and E20f,
L¹ is selected from a bond, 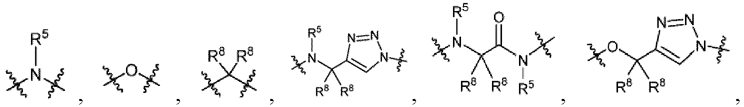 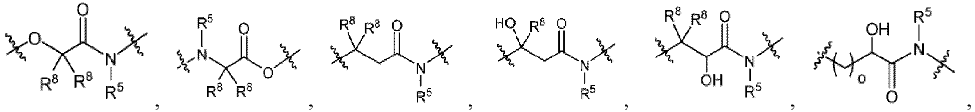 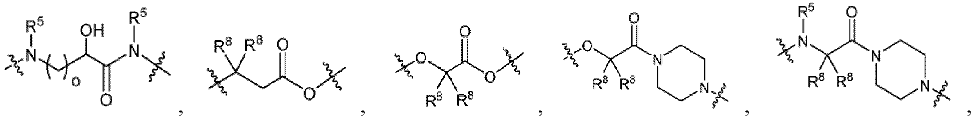 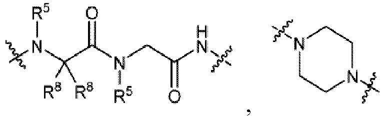 and 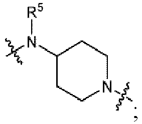
X¹, X², X³ and X⁴ are each independently selected from nitrogen and CR⁴, and at most two of X¹, X², X³ and X⁴ are both selected from nitrogen;
Z² and Z³ are selected from -CH₂- and -C(O)-, and at least one of Z¹ and Z² is selected from -C(O)-;
o is selected from 0, 1, 2, 3 or 4;
each R⁴ is independently selected from hydrogen, halogen, hydroxy, C₁-C₆ alkyl, C₁-C₆ alkoxy and C₁-C₆ haloalkyl;
each R⁵ is independently selected from hydrogen, C₁-C₆ alkyl and -C(O)-alkyl;
each R⁸ is independently selected from hydrogen, C₁-C₆ alkyl and C₁-C₆ haloalkyl; or two R⁸, together with the atom linked thereto, form cyclopropyl.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E21, 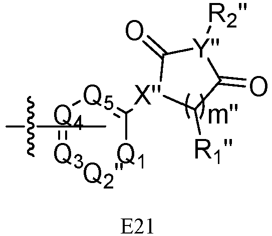 wherein in E21,
X" is C or N;
Y" is C, N, O or S;
Q₁, Q₂, Q₃, Q₄ and Q₅ are each independently CR₃" or N;
each R₃" is independently hydrogen, deuterium, hydroxy, amino, cyano, halogen, C₁-C₆ alkyl, C₃-C₈ cycloalkyl, 3-to 8-membered heterocycloalkyl (e.g., morpholinyl, e.g., 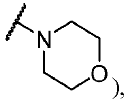 6- to 10-membered aryl, 5- to 10-membered heteroaryl, -O(C₁-C₆ alkyl), -O-(C₃-C₈ cycloalkyl), -O-(3- to 8-membered heterocycloalkyl), N(C₁-C₆ alkyl)₁₋₂, NH(C₃-C₈ cycloalkyl), NH(3- to 8-membered heterocycloalkyl), -O-(C₆-C₁₀ aryl) or -O-(5- to 10-membered heteroaryl), and the alkyl, the cycloalkyl, the heterocycloalkyl, the aryl and the heteroaryl are optionally substituted with 1-3 groups independently selected from hydroxy, halogen, cyano and amino;
m" is 1, 2 or 3;
each R₁" is independently hydrogen, deuterium, hydroxy, amino, cyano, halogen, C₁-C₆ alkyl, C₃-C₈ cycloalkyl, 3-to 8-membered heterocycloalkyl, 6- to 10-membered aryl, 5- to 10-membered heteroaryl or -O(C₁-C₆ alkyl), and the alkyl, the cycloalkyl, the heterocycloalkyl, the aryl and the heteroaryl are optionally substituted with 1-3 groups independently selected from hydroxy, halogen, cyano and amino;
R₂" is hydrogen, deuterium, C₁-C₆ alkyl or C₃-C₆ cycloalkyl, and the C₁-C₆ alkyl and the C₃-C₆ cycloalkyl are optionally substituted with 1-3 groups independently selected from hydroxy, halogen, cyano and amino.

In certain embodiments of the present invention, 1 or 2 of Q₁, Q₂, Q₃, Q₄ and Q₅ in E21 are N, and the rest are each independently CR₃".

In certain embodiments of the present invention, Q₁, Q₂, Q₃, Q₄ and Q₅ in E21 are each independently CR₃".

In certain embodiments of the present invention, X" in E21 is N.

In certain embodiments of the present invention, X" in E21 is C.

In certain embodiments of the present invention, Y" in E21 is N.

In certain embodiments of the present invention, each R₁" in E21 is independently hydrogen, deuterium, -F, -Cl or C₁-C₆ alkyl, and the alkyl is optionally substituted with 1-3 halogens; preferably, R₁" is hydrogen.

In certain embodiments of the present invention, R₂" in E21 is hydrogen or C₁-C₆ alkyl, and the alkyl is optionally substituted with 1-3 halogens; preferably, R₂" is hydrogen.

In certain embodiments of the present invention, each R₃" in E21 is independently hydrogen, deuterium, halogen, - O(C₁-C₆ alkyl) or C₁-C₆ alkyl, and the alkyl is optionally substituted with 1-3 halogens; preferably, each R₃" is independently hydrogen, deuterium, F, Cl, methyl, methoxy, ethoxy, trifluoromethoxy, 2-hydroxypropan-2-yl or trifluoromethyl.

In certain embodiments of the present invention, m in E21 is 2.

In certain embodiments of the present invention, E21 has a structure shown in the following formula, 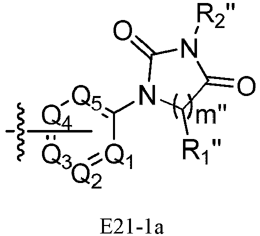 wherein Q₁, Q₂, Q₃, Q₄, Q₅, R₁", R₂" and m" are as defined in E21 described above.

In certain embodiments of the present invention, E21 has a structure shown in the following formula, 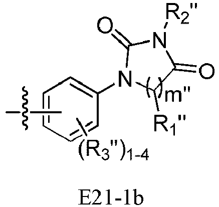 wherein R₁", R₂", R₃" and m" are as defined in E21 described above.

In certain embodiments of the present invention, E21 has a structure shown in the following formula, 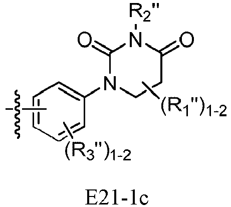 wherein R₁", R₂" and R₃" are as defined in E21 described above. In certain embodiments of the present invention, E21 has a structure shown in the following formula, 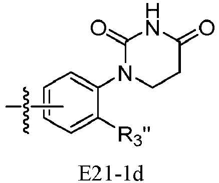 wherein R₃" is as defined in E21 described above. In certain embodiments of the present invention, E21 has a structure shown in the following formula, 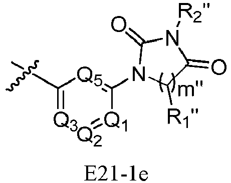 wherein Q₁, Q₂, Q₃, Q₅, R₁", R₂" and m" are as defined in E21 described above.

In certain embodiments of the present invention, E21 has a structure shown in the following formula, 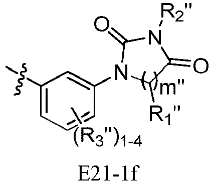 wherein R₁", R₂", R₃" and m" are as defined in E21 described above.

In certain embodiments of the present invention, E21 has a structure shown in the following formula, 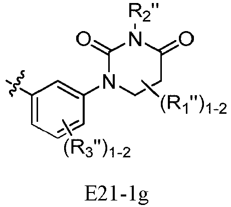 wherein R₁", R₂" and R₃" are as defined in E21-1 described above.

In certain embodiments of the present invention, E21 has a structure shown in the following formula, 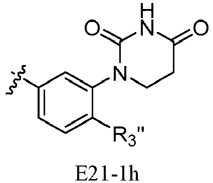 wherein R₃" is as defined in E21 described above.

In certain embodiments of the present invention, E21 has a structure shown in the following formula, 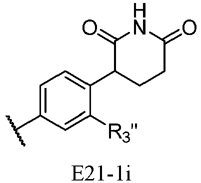 wherein R₃" is as defined in E21 described above.

In certain embodiments of the present invention, E21 has a structure shown in the following formula, 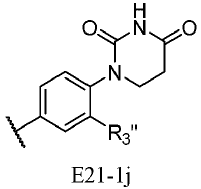 wherein R₃" is as defined in E21 described above.

In certain embodiments of the present invention, in E21 and E21-1a to 1j, R₃" is hydrogen, halogen (e.g., F or Cl), C₁-C₆ alkyl or -O(C₁-C₆ alkyl) (e.g., -OCH₃), and the C₁-C₆ alkyl and the -O(C₁-C₆ alkyl) are optionally substituted with 1-3 halogens (e.g., F).

In certain embodiments of the present invention, in E21 and E21-1a to 1j, R₃" is hydrogen, F, Cl, CF₃, -OCF₃ or - OCH₃.

In certain preferred embodiments of the present invention, in E21-1h, R₃" is halogen (e.g., F or Cl) or -O(C₁-C₆ alkyl) (e.g., -OCH₃).

In certain embodiments of the present invention, E21-1h is any one of the following structures:

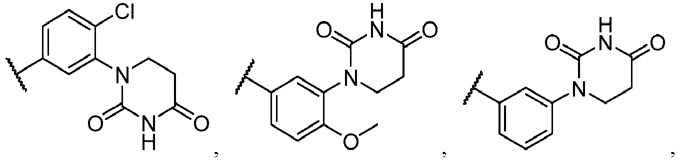

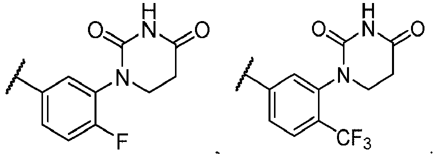

In certain preferred embodiments of the present invention, E21-1h is any one of the following structures:

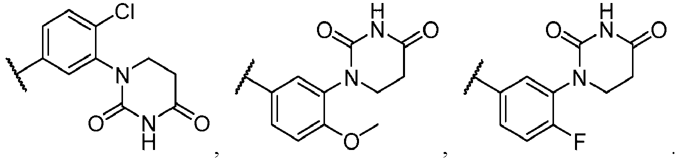

In certain embodiments of the present invention, E21-1i is the following structure:

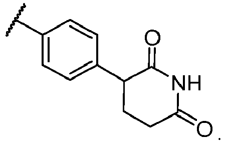

In certain embodiments of the present invention, E21-1j is any one of the following structures:

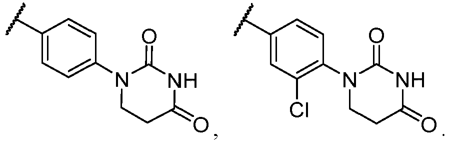

In certain embodiments of the present invention, E21 is selected from

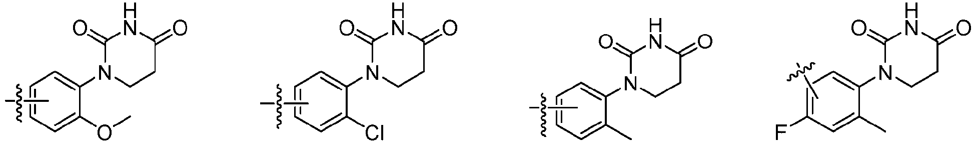

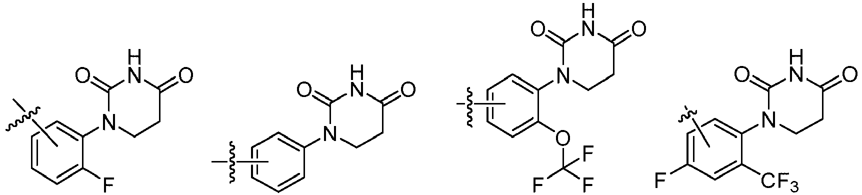

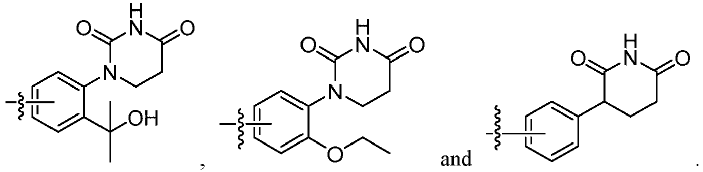

In certain embodiments of the present invention, E21 is selected from

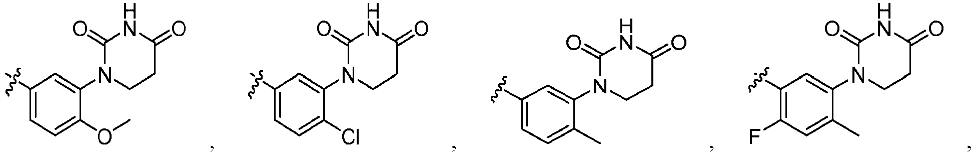

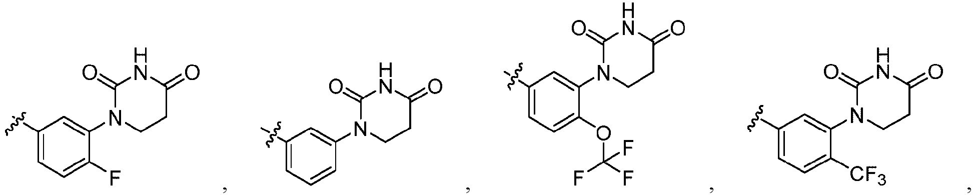

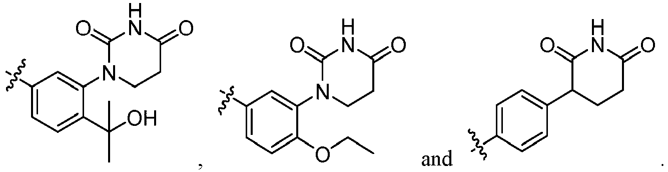

In certain preferred embodiments of the present invention, E21 has a structure shown in the following formula,

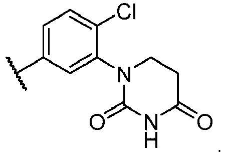

In certain preferred embodiments of the present invention, E21 has a structure shown in the following formula,

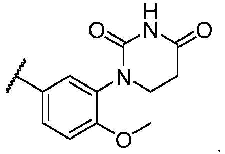

In certain preferred embodiments of the present invention, E21 has a structure shown in the following formula,

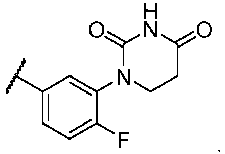

In certain preferred embodiments of the present invention, E21 has a structure shown in the following formula,

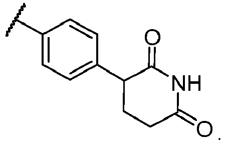

In certain preferred embodiments of the present invention, E21 has a structure shown in the following formula,

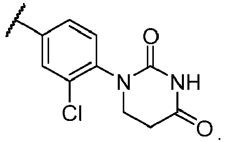

In certain embodiments of the present invention, E21 has a structure shown in the following formula, 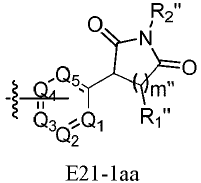 wherein Q₁, Q₂, Q₃, Q₄, Q₅, R₁", R₂" and m" are as defined in E21 described above.

In certain embodiments of the present invention, E21 has a structure shown in the following formula, 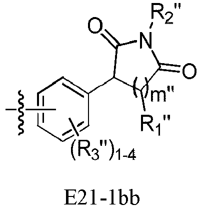 wherein R₁", R₂", R₃" and m" are as defined in E21 described above.

In certain embodiments of the present invention, E21 has a structure shown in the following formula, 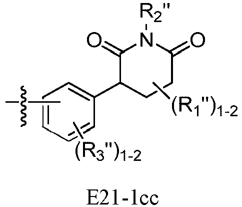 wherein R₁", R₂" and R₃" are as defined in E21 described above.

In certain embodiments of the present invention, E21 has a structure shown in the following formula, 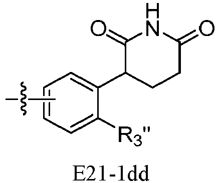 wherein R₃" is as defined in E21 described above.

In certain embodiments of the present invention, E21 has a structure shown in the following formula, 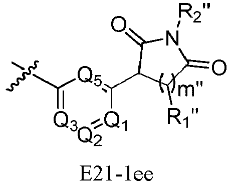 wherein Q₁, Q₂, Q₃, Q₅, R₁", R₂" and m" are as defined in E21 described above.

In certain embodiments of the present invention, E21 has a structure shown in the following formula, 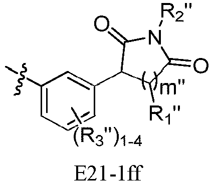 wherein R₁", R₂", R₃" and m" are as defined in E21-1 described above.

In certain embodiments of the present invention, E21 has a structure shown in the following formula, 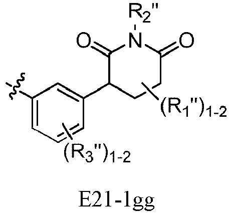 wherein R₁", R₂" and R₃" are as defined in E21 described above.

In certain embodiments of the present invention, E21 has a structure shown in the following formula, 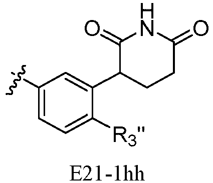 wherein R₃" is as defined in E21 described above.

In certain embodiments of the present invention, E21 is selected from

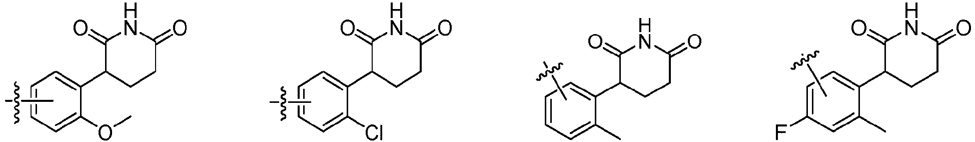

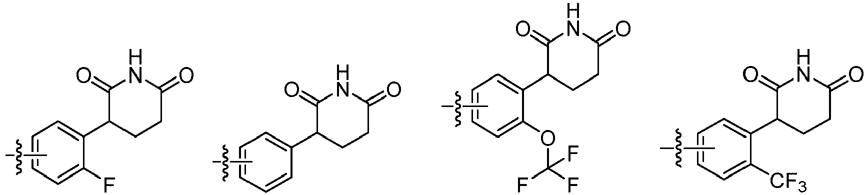

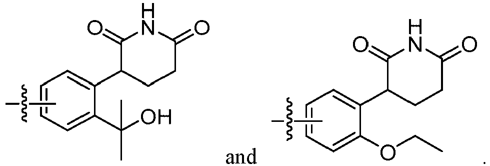

In certain embodiments of the present invention, E21 is selected from

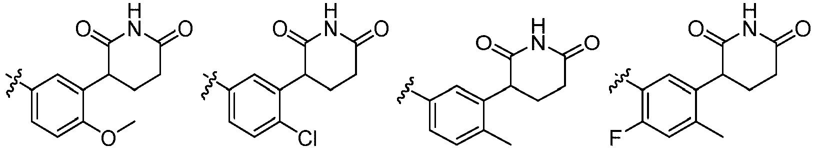

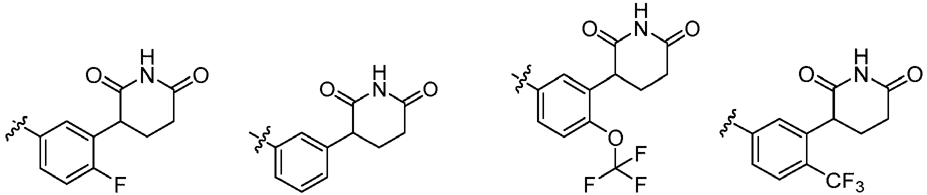

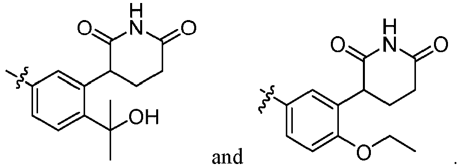

Preferably, in certain embodiments of the present invention, E21 is any one of the following structures:

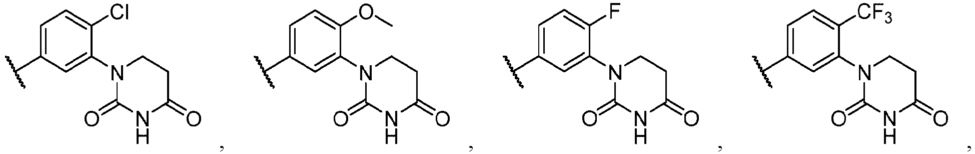

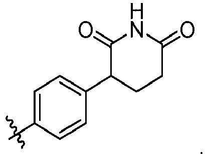

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E22, 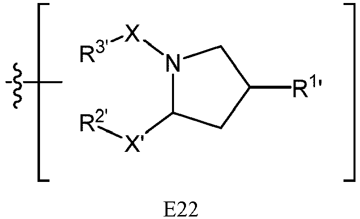
or a pharmaceutically acceptable salt thereof, wherein in E22,
R^{1'} is optionally substituted C₁-C₆ alkyl, optionally substituted -(CH₂)ₙOH, optionally substituted -(CH₂)ₙSH, optionally substituted -(CH₂)ₙO-(C₁-C₆ alkyl) or optionally substituted -(CH₂)ₙ-WC(O)CW-(C₀-C₆ alkyl), wherein each W is independently hydrogen or C₁-C₃ alkyl, optionally substituted -(CH₂)ₙC(O)OH, optionally substituted - (CH₂)ₙC(O)-(C₁-C₆ alkyl), optionally substituted -(CH₂)ₙNHC(O)-R₁, optionally substituted -(CH₂)ₙC(O)-NR₁R₂, optionally substituted -(CH₂)ₙOC(O)-NR₁R₂, -(CH₂O)ₙH, optionally substituted -(CH₂)ₙOC(O)-(C₁-C₆ alkyl), optionally substituted -(CH₂O)ₙC(O)OH, optionally substituted -(OCH₂)ₙO-(C₁-C₆ alkyl), optionally substituted - (CH₂O)ₙC(O)-(C₁-C₆ alkyl), optionally substituted -(CH₂O)ₙC(O)-NR₁R₂, -(CH₂CH₂O)ₙH, optionally substituted - (CH₂CH₂O)ₙCOOH, optionally substituted -(OCH₂CH₂)ₙO-(C₁-C₆ alkyl), optionally substituted - (OCH₂CH₂)ₙC(O)-NR₁R₂, optionally substituted -S(O)₂Rₛ, optionally substituted S(O)Rₛ, nitro, cyano or halogen (F, Cl, Br or I, preferably F or Cl);
R₁ and R₂ are each independently hydrogen or C₁-C₆ alkyl, and the alkyl is optionally substituted with 1 or 2 hydroxy or with 1, 2 or 3 halogens (preferably F);
Rₛ is C₁-C₆ alkyl, optionally substituted aryl, heteroaryl, heterocycloalkyl or -(CH₂)ₘNR₁R₂;
X and X' are each independently C(O), C(S), S(O) or S(O)₂ (preferably, X and X' are both C(O));
R^{2'} is optionally substituted -(CH₂)ₙ-C(O)ᵤ(NR₁)ᵥ(SO₂)_{w} alkyl, optionally substituted -(CH₂)ₙ-C(O)ᵤ(NR₁)ᵥ(SO₂)_{w}NR_{1N}R_{2N}, optionally substituted -(CH₂)ₙ-C(O)ᵤ(NR₁)ᵥ(SO₂)_{w}-aryl, optionally substituted - (CH₂)ₙ-C(O)ᵤ(NR₁)ᵥ(SO₂)_{w}-heteroaryl, optionally substituted -(CH₂)ₙ-C(O)ᵤ(NR₁)ᵥ(SO₂)_{w}-heterocycloalkyl, optionally substituted -NR¹-(CH₂)ₙ-C(O)ᵤ(NR₁)ᵥ(SO₂)_{w}-alkyl, optionally substituted -NR¹⁻(CH₂)ₙ-C(O)ᵤ(NR₁)ᵥ(SO₂)_{w}NR_{1N}R_{2N}, optionally substituted -NR¹-(CH₂)ₙ-C(O)ᵤ(NR₁)ᵥ(SO₂)_{w}NR₁C(O)R_{1N}, optionally substituted -NR¹-(CH₂)n-C(O)ᵤ(NR₁)ᵥ(SO₂)_{w}-aryl, optionally substituted -NR¹-(CH₂)ₙ-aryl-heteroaryl, optionally substituted -NR¹-(CH₂)ₙ-C(O)ᵤ(NR₁)ᵥ(SO₂)_{w}-heteroaryl, optionally substituted -NR¹-(CH₂)ₙ-C(O)ᵤ(NR₁)ᵥ(SO₂)_{w}-heterocycloalkyl, optionally substituted -X^{R2'}-alkyl, optionally substituted -X^{R2'}-aryl, optionally substituted -X^{R2'}-heteroaryl or optionally substituted -X^{R2'}-heterocycloalkyl; R^{3'} is optionally substituted alkyl, optionally substituted -(CH₂)ₙ(O)ᵤ(NR₁)ᵥ(SO₂)_{w}-alkyl, optionally substituted - (CH₂)ₙC(O)ᵤ(NR₁)ᵥ(SO₂)_{w}-NR_{1N}R_{2N}, optionally substituted -(CH₂)ₙC(O)ᵤ(NR₁)ᵥ(SO₂)_{w}-C(O)NR₁R₂, optionally substituted -(CH₂)ₙC(O)ᵤ(NR₁)ᵥ(SO₂)_{w}-aryl, optionally substituted -(CH₂)ₙC(O)ᵤ(NR₁)ᵥ(SO₂)_{w}-heteroaryl, optionally substituted -(CH₂)ₙC(O)ᵤ(NR₁)ᵥ(SO₂)_{w}-heterocycloaryl, optionally substituted -NR¹-(CH₂)ₙ-C(O)ᵤ(NR₁)ᵥ(SO₂)_{w}-alkyl, optionally substituted -NR¹-(CH₂)ₙ-C(O)ᵤ(NR₁)ᵥ(SO₂)_{w}-NR_{1N}R_{2N}, optionally substituted -NR¹-(CH₂)ₙ-C(O)ᵤ(NR₁)ᵥ(SO₂)_{w}-NR₁C(O)R_{1N},
optionally substituted -NR¹-(CH₂)ₙ-C(O)ᵤ(NR₁)ᵥ(SO₂)_{w}-aryl, optionally substituted -NR¹-(CH₂)ₙ-C(O)ᵤ(NR₁)ᵥ(SO₂)_{w}-heteroaryl, optionally substituted -NR¹-(CH₂)ₙ-C(O)ᵤ(NR₁)ᵥ(SO₂)_{w}-heterocycloalkyl, optionally substituted -O-(CH₂)ₙ-C(O)ᵤ(NR₁)ᵥ(SO₂)_{w}-alkyl, optionally substituted -O-(CH₂)ₙ-C(O)ᵤ(NR₁)ᵥ(SO₂)_{w}-NR_{1N}R_{2N}, optionally substituted -O-(CH₂)ₙ-C(O)ᵤ(NR₁)ᵥ(SO₂)_{w}-NR₁C(O)R_{1N}, optionally substituted -O-(CH₂)ₙ-C(O)ᵤ(NR₁)ᵥ(SO₂)_{w}-aryl, optionally substituted -O-(CH₂)ₙ-C(O)ᵤ(NR₁)ᵥ(SO₂)_{w}-heteroaryl, optionally substituted - O-(CH₂)ₙ-C(O)ᵤ(NR₁)ᵥ(SO₂)_{w}-heterocycloalkyl, -(CH₂)ₙ-(V)_{n'}-(CH₂)ₙ-(V)_{n'}-alkyl, optionally substituted -(CH₂)ₙ-(V)_{n'}-(CH₂)ₙ-(V)_{n'}-aryl, optionally substituted -(CH₂)ₙ-(V)_{n'}-(CH₂)ₙ-(V)_{n'}-heteroaryl, optionally substituted -(CH₂)ₙ-(V)_{n'}-(CH₂)ₙ-(V)_{n'}-heterocycloalkyl, optionally substituted -(CH₂)ₙ-N(R_{1'})[C(O)]_{m'}-(V)_{n'}-alkyl, optionally substituted -(CH₂)ₙ₋N(R_{1'})[C(O)]_{m'}-(V)_{n'}-aryl, optionally substituted -(CH₂)ₙ-N(R_{1'})[C(O)]_{m'}-(V)_{n'}-heteroaryl, optionally substituted -(CH₂)ₙ-N(R_{1'})[C(O)]_{m'}-(V)_{n'}-heterocycloalkyl, optionally substituted -X^{R3'}-alkyl, optionally substituted -X^{R3'}-aryl, optionally substituted -X^{R3'}-heteroaryl or optionally substituted -X^{R3'}-heterocycloalkyl;
R_{1N} and R_{2N} are each independently hydrogen, optionally substituted -(CH₂)ₙ-aryl, optionally substituted -(CH₂)ₙ-heteroaryl, optionally substituted -(CH₂)ₙ-heterocycloalkyl, C₁-C₆ alkyl, and the C₁-C₆ alkyl is optionally substituted with 1 or 2 hydroxy or optionally substituted with 1, 2 or 3 halogens;
V is O, S or NR₁;
R¹ and R^{1'} are each independently hydrogen or C₁-C₃ alkyl;
each X^{R2'} or X^{R3'} is independently optionally substituted -(CH₂)ₙ-, -(CH₂)ₙ-CH(Xᵥ)=CH(Xᵥ)- (*cis* or *trans*), -(CH₂)ₙ-C=CH-, -(CH₂CH₂O)- or C₃-C₆ cycloalkyl, and Xᵥ is hydrogen, halogen or optionally substituted C₁-C₃ alkyl; each m is independently 0, 1, 2, 3, 4, 5 or 6;
each m' is independently 1 or 2;
each n is independently 0, 1, 2, 3, 4, 5 or 6;
each n' is independently 0 or 1;
each u is independently 0 or 1;
each v is independently 1 or 2;
each w is independently 0 or 1.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E23, E23a, E23b, E23c, E23d or E23e, 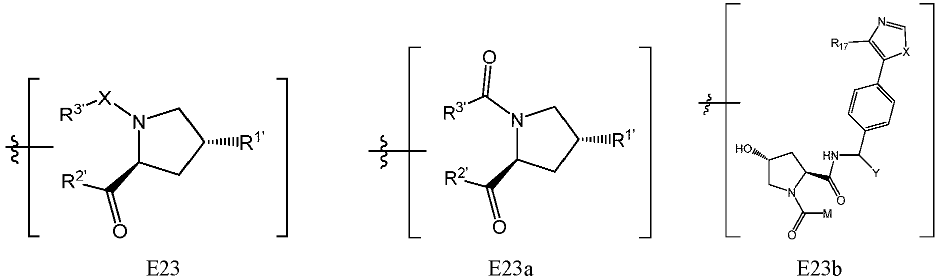 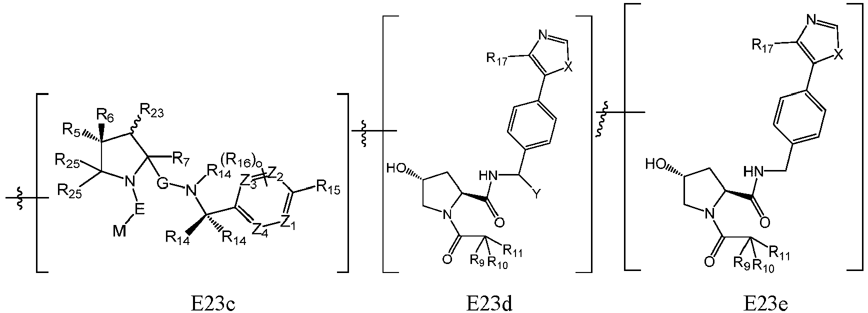 wherein in E23, E23a, E23b, E23c, E23d and E23e,
R^{1'}, R^{2'}, R^{3'} and X are as defined in E22;
R₅ and R₆ are independently selected from OH, SH and optionally substituted alkyl; or R₅ and R₆, together with the carbon atom linked thereto, form C=O;
R₇ is H or optionally substituted alkyl;
E is a bond, C=O or C=S;
G is a bond, optionally substituted alkyl, -COOH or C=J;
J is O or N-R₈;
R₈ is H, CN, optionally substituted alkyl or optionally substituted alkoxy;
M is aryl, heteroaryl, heterocyclyl or 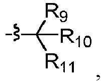 and the aryl, the heteroaryl and the heterocyclyl may be optionally substituted;
R₉ and R₁₀ are independently selected from H, alkyl, cycloalkyl, hydroxyalkyl, thioalkyl, a ULM-linked disulfide bond, heteroaryl and haloalkyl, and the alkyl, the cycloalkyl, the hydroxyalkyl, the thioalkyl, the ULM-linked disulfide bond, the heteroaryl or the haloalkyl may be optionally substituted; or R₉ and R₁₀, together with the carbon atom linked thereto, form optionally substituted cycloalkyl;
R₁₁ is heterocycloalkyl, alkoxy, heteroaryl, aryl or 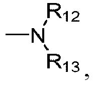 , and the heterocycloalkyl, the alkoxy, the heteroaryl and the aryl may be optionally substituted;
R₁₂ is H or optionally substituted alkyl;
R₁₃ is H, alkyl, alkyl-C=O, (cycloalkyl)alkyl-C=O, aralkyl-C=O, aryl-C=O, (heterocycloalkyl)-C=O, aralkyl or alkoxy-C=O, and the alkyl, the alkyl-C=O, the (cycloalkyl)alkyl-C=O, the aralkyl-C=O, the aryl-C=O, the (heterocycloalkyl)-C=O, the aralkyl or the alkoxy-C=O may be optionally substituted;
R₁₄ is H, haloalkyl, cycloalkyl, alkyl or heterocycloalkyl, and the cycloalkyl, the alkyl or the heterocycloalkyl may be optionally substituted;
R₁₅ is H, heteroaryl, haloalkyl, aryl, alkoxy or heterocycloalkyl, and the heteroaryl, the haloalkyl, the aryl, the alkoxy or the heterocycloalkyl may be optionally substituted;
R₁₆ is halogen, alkyl, haloalkyl, CN or haloalkoxy, and the alkyl, the haloalkyl or the haloalkoxy may be optionally substituted;
R₁₇ is H, halogen, cycloalkyl, alkyl, alkenyl or haloalkyl, and the cycloalkyl, the alkyl and the alkenyl may be optionally substituted;
R₂₃ is H or OH;
R₂₅ is H or substituted alkyl; or 2 R₂₅ form =O or substituted cycloalkyl;
Z₁, Z₂, Z₃ and Z₄ are each independently selected from C and N;
X is O or S;
Y is H, methyl or ethyl;
o is 0, 1, 2, 3 or 4.

In some embodiments, in E23, E23a, E23b, E23c, E23d and E23e, R^{1'}, R^{2'}, R^{3'}, R₅, R₆, R₇, R₉, R₁₀, R₁₁, R₁₄, R₁₅, R₁₆, R₁₇, R₂₃, R₂₅, E, M, o, X, Y, Z₁, Z₂, Z₃ and Z₄ are as defined and described in US 2016/0272639, which is incorporated herein by reference in its entirety.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E23a, 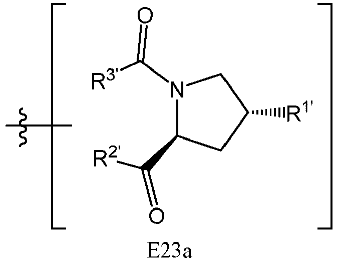 wherein in E23a,
R^{1'} is hydroxy;
R^{2'} is -NH-CH₂-aryl-HET;
R^{3'} is selected from optionally substituted alkyl, -(CH)R^{CR3'}-NH-C(O)-R^{3p1} and -(CH)R^{CR3'}-R^{3p2};
R^{CR3'} is optionally substituted C₁-C₄ alkyl;
R^{3p1} is optionally substituted C₁-C₆ alkyl, optionally substituted oxetanyl, -(CH₂)ₙOCH₃, phenyl optionally substituted with -O(CH₂CH₃) or morpholinyl linked to carbonyl at position 2 or position 3, and n is 1 or 2;
R^{3p2} is 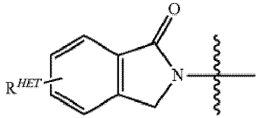 or optionally substituted phenyl;
HET is selected from optionally substituted thiazole, oxazole, isoxazole and isothiazole;
R^{HET} is selected from hydrogen, halogen, cyano, optionally substituted C₁-C₆ alkyl, optionally substituted C₁-C₆ alkoxy and optionally substituted aryl.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E23c, 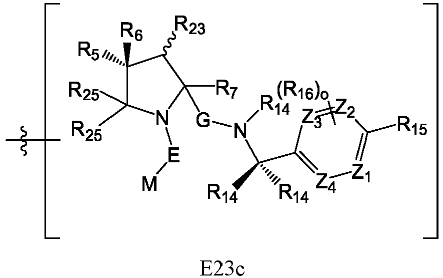
or a pharmaceutically acceptable salt thereof, wherein in E23c,
R₅ and R₆ are each independently -OH, -SH or optionally substituted alkyl, or R₅ and R₆, together with the carbon atom linked thereto, form carbonyl;
R₇ is H or optionally substituted alkyl;
E is a bond, C=O or C=S;
G is a bond, optionally substituted alkyl, -COOH or C=J;
J is O or N-R₈;
R₈ is H, CN, optionally substituted alkyl or optionally substituted alkoxy;
M is optionally substituted aryl, optionally substituted heteroaryl, optionally substituted heterocycloalkyl or 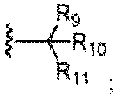
R₉ and R₁₀ are each independently H, optionally substituted alkyl, optionally substituted cycloalkyl, optionally substituted hydroxyalkyl, optionally substituted thioalkyl, disulfide-linked ULM, optionally substituted heteroaryl or haloalkyl; or R₉ and R₁₀, together with the carbon atom linked thereto, form optionally substituted cycloalkyl;
R₁₁ is optionally substituted heterocycloalkyl, optionally substituted alkoxy, optionally substituted heteroaryl, optionally substituted aryl or 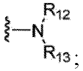
R₁₂ is H or optionally substituted alkyl;
R₁₃ is H, optionally substituted alkyl, optionally substituted alkylcarbonyl, optionally substituted (cycloalkyl)alkylcarbonyl, optionally substituted aralkylcarbonyl, optionally substituted arylcarbonyl, optionally substituted (heterocycloalkyl)carbonyl, optionally substituted aralkyl or optionally substituted (oxoalkyl)carbamate; each R₁₄ is independently H, haloalkyl, optionally substituted cycloalkyl, optionally substituted alkyl or optionally substituted heterocycloalkyl;
R₁₅ is H, optionally substituted heteroaryl, haloalkyl, optionally substituted aryl, optionally substituted alkoxy or optionally substituted heterocycloalkyl;
each R₁₆ is independently halogen, optionally substituted alkyl, optionally substituted haloalkyl, CN or optionally substituted haloalkoxy;
each R₂₅ is independently H or optionally substituted alkyl; or two R₂₅ groups may together form oxo or optionally substituted cycloalkyl;
R₂₃ is H or OH;
Z₁, Z₂, Z₃ and Z₄ are independently C or N; and
o is 0, 1, 2, 3 or 4.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E23d, 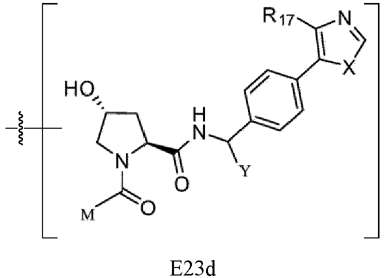 wherein in E23d,
X is O or S;
Y is H, methyl or ethyl;
R₁₇ is H, methyl, ethyl, hydroxymethyl or cyclopropyl;
M is optionally substituted heteroaryl or optionally substituted aryl.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E23d-1, 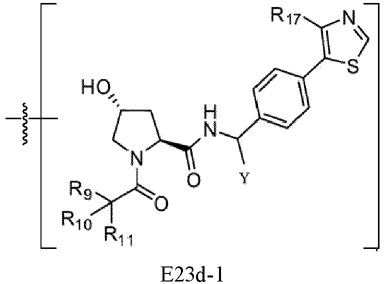 wherein in E23d-1,
Y is H, methyl or ethyl;
R₉ is H;
R₁₀ is isopropyl, tert-butyl, sec-butyl, cyclopentyl or cyclohexyl;
R₁₁ is optionally substituted amide, optionally substituted isoindolinone, optionally substituted isoxazole or an optionally substituted heterocycle;
R₁₇ is H, methyl, ethyl, hydroxymethyl or cyclopropyl.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is the specific compound according to Example 1 to Example 208 in CN108601764 (incorporated herein by reference in its entirety).

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E24, 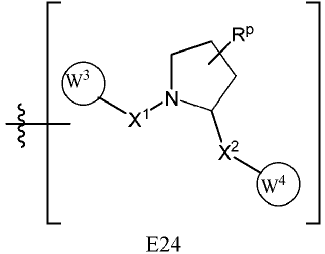
or a pharmaceutically acceptable salt thereof, wherein in E24,
X¹ and X² are each independently selected from a bond, O, NR^{Y3}, CR^{Y3}R^{Y4}, C=O, C=S, SO and SO₂;
R^{Y3} and R^{Y4} are each independently hydrogen, C₁-C₆ alkyl optionally substituted with 1 or more halogens or C₁-C₆ alkoxy optionally substituted with 0-3 R^{p};
R^{p} is 0, 1, 2 or 3 groups selected from hydrogen, halogen, hydroxy, C₁-C₃ alkyl and C(=O);
W³ is selected from optionally substituted T, optionally substituted -T-N(R^{1a}R^{1b})X³, optionally substituted -T-N(R^{1a}R^{1b}), optionally substituted -T-aryl, optionally substituted -T-heteroaryl, optionally substituted -T-bicyclic heteroaryl, optionally substituted -T-heterocycloalkyl, optionally substituted -T-bicyclic heterocycloalkyl, optionally substituted -NR¹-T-aryl, optionally substituted -NR¹-T-heteroaryl and optionally substituted -NR¹-T-heterocycloalkyl;
X³ is C(O), R¹, R^{1a} or R^{1b};
each R¹, R^{1a} or R^{1b} is independently selected from hydrogen, C₁-C₆ alkyl optionally substituted with 1 or more halogens or hydroxy, R^{Y3}C(O), R^{Y3}C(S), R^{Y3}S(O), R^{Y3}S(O)₂, N(R^{Y3}R^{Y4})C(O), N(R^{Y3}R^{Y4})C(S), N(R^{Y3}R^{Y4})S(O) and N(R^{Y3}R^{Y4})S(O)₂;
T is selected from optionally substituted alkyl and -(CH₂)ₙ-, and each of the methylene groups is optionally substituted with a substituent selected from halogen, hydroxy, an optionally substituted amino acid side chain, methyl, optionally substituted alkoxy, C₁-C₆ alkyl optionally substituted with one or more halogens, -C(O)NR¹R^{1a} and NR¹R^{1a};
or R¹ and R^{1a} may optionally form optionally substituted heterocycloalkyl;
n is 0, 1, 2, 3, 4, 5 or 6;
W⁴ is 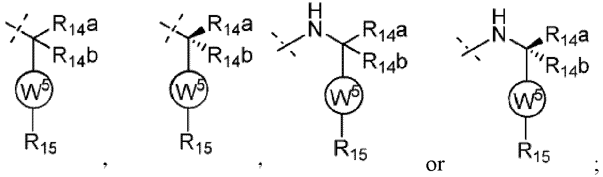
R₁₄ₐ and R_{14b} are each independently hydrogen, haloalkyl or optionally substituted alkyl;
W⁵ is optionally substituted phenyl or optionally substituted heteroaryl;
R₁₅ is optionally substituted hydrogen, cyano, halogen, hydroxy, nitro, NR₁₄ₐR_{14b}, OR₁₄ₐ, CONR₁₄ₐR_{14b}, NR₁₄ₐCONR_{14b}, SO₂NR₁₄ₐR_{14b}, NR₁₄ₐSO₂R_{14b}, optionally substituted alkyl, optionally substituted haloalkyl, optionally substituted haloalkoxy, optionally substituted aryl, optionally substituted heteroaryl, optionally substituted cycloalkyl or optionally substituted heterocycloalkyl.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E25 or E25a, 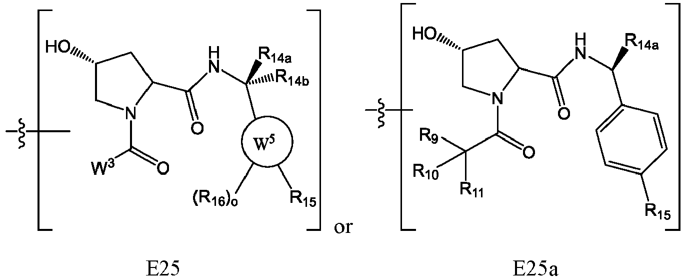 wherein in E25 and E25a,
W³ is optionally substituted aryl, optionally substituted heteroaryl or 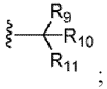
R₉ and R₁₀ are each independently hydrogen, optionally substituted alkyl, optionally substituted cycloalkyl, optionally substituted hydroxyalkyl, optionally substituted heteroaryl or haloalkyl, or R₉ and R₁₀, together with the carbon atom linked thereto, form optionally substituted cycloalkyl;
R₁₁ is selected from optionally substituted heterocycloalkyl, optionally substituted alkoxy, optionally substituted heteroaryl, optionally substituted aryl, 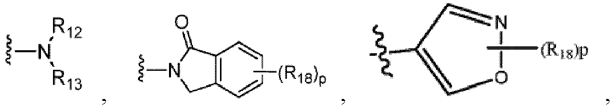 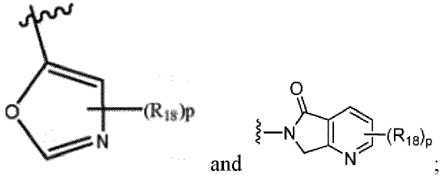
each R₁₂ is independently hydrogen or optionally substituted alkyl;
each R₁₃ is independently hydrogen, optionally substituted alkyl, optionally substituted alkylcarbonyl, optionally substituted (cycloalkyl)alkylcarbonyl, optionally substituted aralkylcarbonyl, optionally substituted arylcarbonyl, optionally substituted (heterocycloalkyl)carbonyl or optionally substituted aralkyl;
R₁₄ₐ and R_{14b} are each independently hydrogen, haloalkyl or optionally substituted alkyl;
W⁵ is selected from optionally substituted phenyl and optionally substituted 5- to 10-membered heteroaryl;
R₁₅ is optionally substituted hydrogen, cyano, halogen, hydroxy, nitro, NR₁₄ₐR_{14b}, OR₁₄ₐ, CONR₁₄ₐR_{14b}, NR₁₄ₐCONR_{14b}, SO₂NR₁₄ₐR_{14b}, NR₁₄ₐSO₂R_{14b}, optionally substituted alkyl, optionally substituted haloalkyl, optionally substituted haloalkoxy, optionally substituted aryl, optionally substituted heteroaryl, optionally substituted cycloalkyl or optionally substituted heterocycloalkyl;
each R₁₆ is independently halogen, cyano, optionally substituted alkyl, optionally substituted alkoxy, hydroxy or optionally substituted haloalkyl;
o is 0, 1, 2, 3 or 4;
each R₁₈ is independently selected from halogen, optionally substituted alkoxy, cyano, optionally substituted alkyl, haloalkyl and haloalkoxy;
p is 0, 1, 2, 3 or 4.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E26, E26a or E26b, 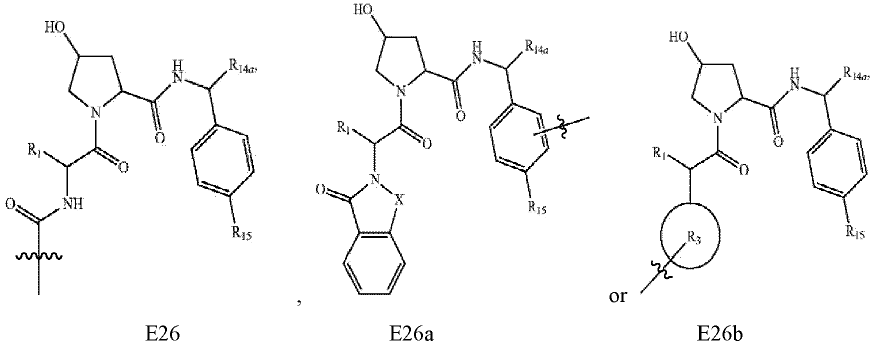 or a pharmaceutically acceptable salt thereof, wherein in E26, E26a and E26b,
R₁ is hydrogen, methyl, ethyl, isopropyl, tert-butyl, sec-butyl, cyclopropyl, cyclobutyl, cyclopentyl, cyclohexyl, optionally substituted alkyl, optionally substituted hydroxyalkyl, optionally substituted heteroaryl or haloalkyl; R₁₄ₐ is hydrogen, haloalkyl, optionally substituted alkyl, methyl, fluoromethyl, hydroxymethyl, ethyl, isopropyl or cyclopropyl;
X is CH₂ or C(O);
R₁₅ is selected from hydrogen, halogen, CN, hydroxy, nitro, optionally substituted heteroaryl, optionally substituted aryl, optionally substituted alkyl, optionally substituted haloalkyl, optionally substituted haloalkoxy, optionally substituted cycloalkyl and optionally substituted heterocycloalkyl;
R₃ is absent or is optionally substituted 5- to 6-membered heteroaryl.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E27, E27a, E27b, E27c, E27d, E27e, E27f or E27g, 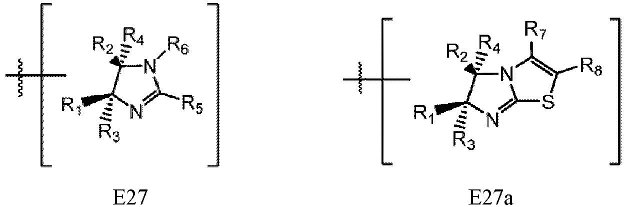 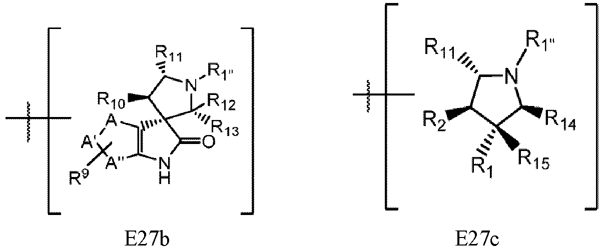 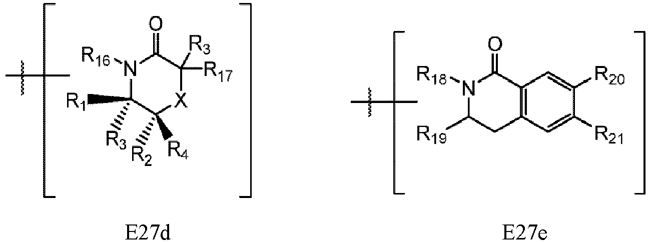 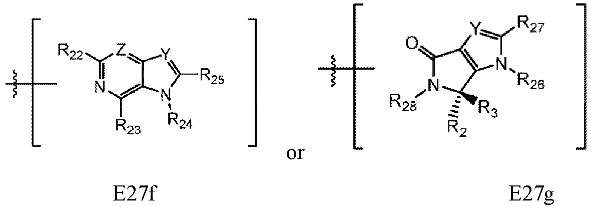 wherein in E27, E27a, E27b, E27c, E27d, E27e, E27f and E27g, X is selected from carbon, oxygen, sulfur, sulfoxide, sulfone and N-R^{a};
R^{a} is independently H or alkyl having a carbon number of 1 to 6;
Y and Z are independently carbon or nitrogen;
A, A' and A" are independently selected from C, N, O and S, and may also be one or two atoms forming a fused bicyclic ring or 6,5- and 5,5-fused aromatic bicyclic groups;
R₁ and R₂ are independently selected from aryl, heteroaryl and heteroaryl having one or two heteroatoms independently selected from sulfur and nitrogen, wherein the aryl or the heteroaryl may be monocyclic or bicyclic, or unsubstituted or substituted with one to three substituents independently selected from halogen, -CN, C₁-C₆ alkyl, C₃-C₆ cycloalkyl, -OH, alkoxy having 1 to 6 carbons, fluoro-substituted alkoxy having 1 to 6 carbons, sulfoxide having 1 to 6 carbons, sulfone having 1 to 6 carbons, ketone having 2 to 6 carbons, amide having 2 to 6 carbons and dialkylamine having 2 to 6 carbons;
R₃ and R₄ are independently selected from H, methyl and C₁-C₆ alkyl;
R₅ is selected from aryl, heteroaryl and heteroaryl having one or two heteroatoms independently selected from sulfur and nitrogen, wherein the aryl or the heteroaryl may be monocyclic or bicyclic, or unsubstituted or substituted with one to three substituents independently selected from halogen, -CN, C₁-C₆ alkyl, C₃-C₆ cycloalkyl, -OH, alkoxy having 1 to 6 carbons, fluoro-substituted alkoxy having 1 to 6 carbons, sulfoxide having 1 to 6 carbons, sulfone having 1 to 6 carbons, ketone having 2 to 6 carbons, amide having 2 to 6 carbons, dialkylamine having 2 to 6 carbons, alkyl ether (C₂-C₆), alkyl ketone (C₃-C₆), morpholinyl, alkyl ester (C₃-C₆) and alkyl cyanogen (C₃-C₆);
R₆ is H or -C(=O)R^{b}, wherein
R^{b} is selected from alkyl, cycloalkyl, mono-, di- or tri-substituted aryl or heteroaryl, 4-morpholinyl, 1-(3-oxopiperazinyl), 1-piperidinyl, 4-N-R^{c}-morpholinyl, 4-R^{C}-1-piperidinyl and 3-R^{c}-1-piperidinyl, wherein R^{c} is selected from alkyl, fluoro-substituted alkyl, cyanoalkyl, hydroxy-substituted alkyl, cycloalkyl, alkoxyalkyl, amidoalkyl, alkylsulfone, alkylsulfoxide, alkylamide, aryl, heteroaryl, mono-, di- or tri-substituted aryl or heteroaryl, CH₂CH₂R^{d} and CH₂CH₂CH₂R^{d}, wherein
R^{d} is selected from alkoxy, alkylsulfone, alkylsulfoxide, N-substituted formamide, -NHC(O)-alkyl, -NH-SO₂-alkyl, aryl, substituted aryl, heteroaryl and substituted heteroaryl;
R₇ is selected from H, C₁-C₆ alkyl, cycloalkyl, fluoro-substituted alkyl, cyano-substituted alkyl, 5- or 6-membered heteroaryl or aryl and substituted 5- or 6-membered heteroaryl or aryl;
R₈ is selected from -R^{e}-C(O)-R^{f}, -R^{e}-alkoxy, -R^{e}-aryl, -R^{e}-heteroaryl and -R^{e}-C(O)-R^{f}-C(O)-R^{g}, wherein
R^{e} is alkylene having 1 to 6 carbons or a bond;
R^{f} is a substituted 4- to 7-membered heterocycle;
R^{g} is selected from aryl, heteroaryl, substituted aryl or heteroaryl and a 4- to 7-membered heterocycle;
R₉ is selected from mono-, di- or tri-substituents on a fused bicyclic aromatic ring, wherein the substituent is independently selected from halogen, alkene, alkyne, alkyl, unsubstituted or substituted with Cl or F;
R₁₀ is selected from aryl and heteroaryl, wherein the heteroaryl may contain one or two heteroatoms such as sulfur or nitrogen, the aryl or the heteroaryl may be monocyclic or bicyclic, and the aryl or the heteroaryl may be unsubstituted or substituted with one to three substituents, including halogen, F, Cl, -CN, alkene, alkyne, C₁-C₆ alkyl, C₁-C₆ cycloalkyl, -OH, alkoxy having 1 to 6 carbons, fluoro-substituted alkoxy having 1 to 6 carbons, sulfoxide having 1 to 6 carbons, sulfone having 1 to 6 carbons and ketone having 2 to 6 carbons;
R₁₁ is -C(O)-N(R^{h})(Rⁱ), wherein R^{h} and Rⁱ are selected from the following: H, C₁-C₆ alkyl, alkoxy-substituted alkyl, sulfone-substituted alkyl, aryl, heteroaryl, mono-, di -or tri-substituted aryl or heteroaryl, alkyl carboxylic acid, heteroaryl carboxylic acid, fluoro-substituted alkyl carboxylic acid, aryl-substituted cycloalkyl and heteroaryl-substituted cycloalkyl, wherein R^{h} and Rⁱ are independently selected from H, linking to form a ring, 4-hydroxycyclohexane, monohydroxy and dihydroxy-substituted alkyl (C₃-C₆), 3-hydroxycyclobutane, phenyl-4-carboxylic acid and substituted phenyl-4-carboxylic acid;
R₁₂ and R₁₃ are independently selected from H, lower alkyl (C₁-C₆), lower alkenyl (C₂-C₆), lower alkynyl (C₂-C₆), cycloalkyl (4-, 5- and 6-membered rings), substituted cycloalkyl, cycloalkenyl, substituted cycloalkenyl, 5- and 6-membered aryl and heteroaryl, and R₁₂ and R₁₃ may be linked to form a 5- or 6-membered ring with or without substitution on the ring;
R₁₄ is selected from alkyl, substituted alkyl, alkenyl, substituted alkenyl, aryl, substituted aryl, heteroaryl, substituted heteroaryl, heterocycle, substituted heterocycle, cycloalkyl, substituted cycloalkyl, cycloalkenyl and substituted cycloalkenyl;
R₁₅ is CN;
R₁₆ is selected from C₁-C₆ alkyl where one or more hydrogens are replaced by fluorine, C₁-C₆ cycloalkyl, C₂-C₆ alkenyl, C₁-C₆ alkyl or C₃-C₆ cycloalkyl, alkyl or cycloalkyl where one CH₂ is replaced by S(=O), -S or -S(=O)₂, alkyl or cycloalkyl where terminal CH₃ is replaced by S(=O)₂N(alkyl)(alkyl), -C(=O)N(alkyl)(alkyl), - N(alkyl)S(=O)₂(alkyl), -C(=O)₂(alkyl) or -O(alkyl), C₁₋₆ alkyl or alkyl-cycloalkyl where hydrogen is replaced by hydroxy, 3- to 7-membered cycloalkyl or heterocycloalkyl optionally containing a -(C=O)- group and 5- to 6-membered aryl or heteroaryl, wherein the heterocycloalkyl or the heteroaryl may contain one to three heteroatoms independently selected from O, N and S, and the cycloalkyl, the heterocycloalkyl, the aryl or the heteroaryl may be unsubstituted or substituted with one to three substituents independently selected from halogen, C₁-C₆ alkyl, hydroxylated C₁-C₆ alkyl, thioether-containing C₁-C₆ alkyl, ether, sulfone, sulfoxide, fluoro-substituted ether and cyano;
R₁₇ is selected from (CH₂)ₙC(O)NR^{k}R^{l}, wherein R^{k} and R^{l} are independently selected from H, C₁-C₆ alkyl, hydroxylated C₁-C₆ alkyl, C₁-C₆ alkoxyalkyl, C₁-C₆ alkyl where one or more hydrogens are replaced by fluorine, C₁-C₆ alkyl where one carbon is replaced by S(O) or S(O)(O), C₁-C₆ alkoxyalkyl where one or more hydrogens are replaced by fluorine, C₁-C₆ alkyl where one or more hydrogens are replaced by cyano, 5- and 6-membered aryl or heteroaryl, alkylaryl where the alkyl contains 1-6 carbons and alkylheteroaryl where the alkyl contains 1-6 carbons, wherein the aryl or the heteroaryl may be further substituted, and n is an integer of 0-6;
R₁₈ is selected from substituted aryl, heteroaryl, alkyl and cycloalkyl, and the substitution is preferably -N(C₁-C₄ alkyl)(cycloalkyl), -N(C₁-C₄ alkyl)alkyl-cycloalkyl or -N(C₁-C₄ alkyl)[(alkyl)-(substituted heterocycle)-cycloalkyl; R₁₉ is selected from aryl, heteroaryl and bicyclic heteroaryl, these aryl or heteroaryl may be substituted with halogen, C₁₋₆ alkyl, C₁₋₆ cycloalkyl, CF₃, F, CN, alkyne or alkylsulfone, and the halogen substitution may be mono- or tri-substituted;
R₂₀ and R₂₁ are independently selected from C₁-C₆ alkyl, C₁-C₆ cycloalkyl, C₁-C₆ alkoxy, hydroxylated C₁-C₆ alkoxy and fluoro-substituted C₁-C₆ alkoxy, and R₂₀ and R₂₁ may also be linked to form a 5-, 6- or 7-membered ring or a heterocyclic ring, which may be further substituted;
R₂₂ is selected from H, C₁-C₆ alkyl, C₁-C₆ cycloalkyl, carboxylic acid, carboxylate, amide, reverse amide, sulfonamide, reverse sulfonamide, N-acyl urea and a nitrogen-containing 5-membered heterocycle, and the 5-membered heterocycle may be further substituted with C₁-C₆ alkyl, alkoxy, fluoro-substituted alkyl, CN or alkylsulfone;
R₂₃ is selected from aryl, heteroaryl, -O-aryl, -O-heteroaryl, -O-alkyl, -O-alkyl-cycloalkyl, -NH-alkyl, -NH-alkyl-cycloalkyl, -N(H)-aryl, -N(H)-heteroaryl, -N(alkyl)-aryl and -N(alkyl)-heteroaryl, and the aryl or the heteroaryl may be substituted with halogen, C₁-C₆ alkyl, hydroxylated C₁-C₆ alkyl, cycloalkyl, fluoro-substituted C₁-C₆ alkyl, CN, alkoxy, alkylsulfone, amide or sulfonamide;
R₂₄ is selected from -CH₂-(C₁-C₆ alkyl), -CH₂-cycloalkyl, -CH₂-aryl and CH₂-heteroaryl, wherein the alkyl, the cycloalkyl, the aryl or the heteroaryl may be substituted with halogen, alkoxy, hydroxylated alkyl, cyano-substituted alkyl, cycloalkyl or substituted cycloalkyl;
R₂₅ is selected from C₁-C₆ alkyl, C₁-C₆ alkyl-cycloalkyl, alkoxy-substituted alkyl, hydroxylated alkyl, aryl, heteroaryl, substituted aryl or heteroaryl, 5-, 6- and 7-membered nitrogen-containing saturated heterocycles and 5,6-fused and 6,6-fused nitrogen-containing saturated heterocycles, and these saturated heterocycles may be substituted with C₁₋₆ alkyl, fluoro-substituted C₁-C₆ alkyl, alkoxy, aryl or heteroaryl;
R₂₆ is selected from C₁-C₆ alkyl and C₃-C₆ cycloalkyl, and the alkyl or the cycloalkyl may be substituted with -OH, alkoxy, fluoro-substituted alkoxy, fluoro-substituted alkyl, -NH₂, -NH-alkyl, NH-C(O) alkyl, -NH-S(O)₂-alkyl or - S (O)₂-alkyl;
R₂₇ is selected from aryl, heteroaryl and bicyclic heteroaryl, wherein the aryl or the heteroaryl may be substituted with C₁-C₆ alkyl, alkoxy, NH₂, NH-alkyl, halogen or -CN, and the substitution may be independently mono-, di- or tri-substituted;
R₂₈ is selected from aryl, 5- and 6-membered heteroaryl, bicyclic heteroaryl, cycloalkyl and a saturated heterocycle such as piperidine, piperidone, tetrahydropyran and N-acyl-piperidine, wherein the cycloalkyl, the saturated heterocycle, the aryl or the heteroaryl may be further substituted with -OH, alkoxy or mono-, di- or tri-substituents including halogen, -CN, alkylsulfone and fluoro-substituted alkyl; and
R_{1"} is selected from alkyl, aryl-substituted alkyl, alkoxy-substituted alkyl, cycloalkyl, aryl-substituted cycloalkyl and alkoxy-substituted cycloalkyl.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E28, E28a, E28b, E28c, E28d, E28e, E28f, E28g, E28h or E28i, (MDM2) 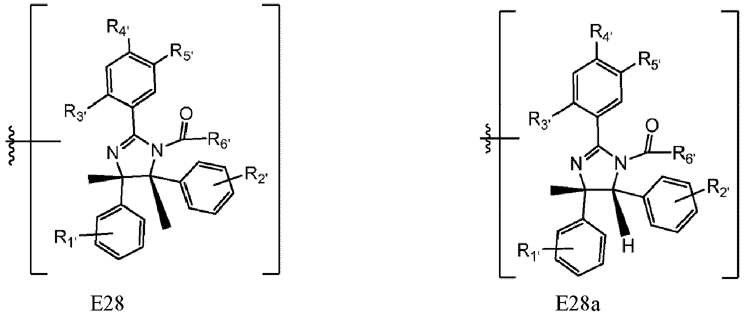 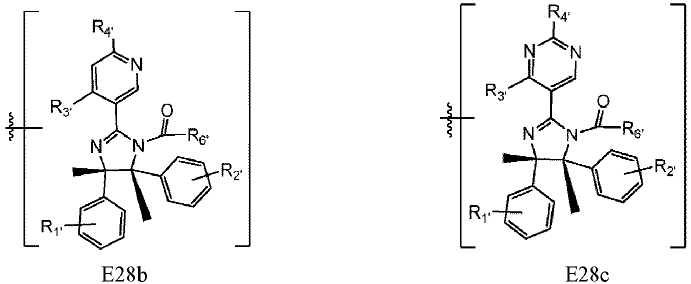 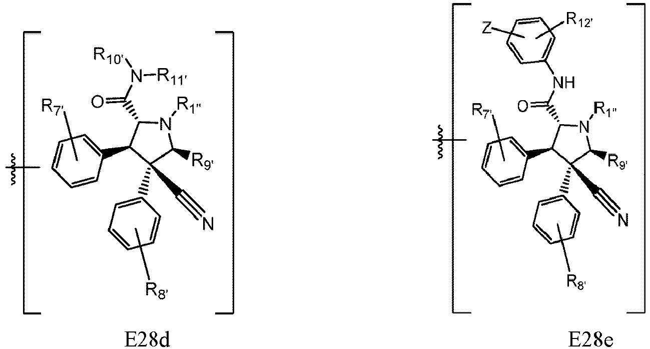 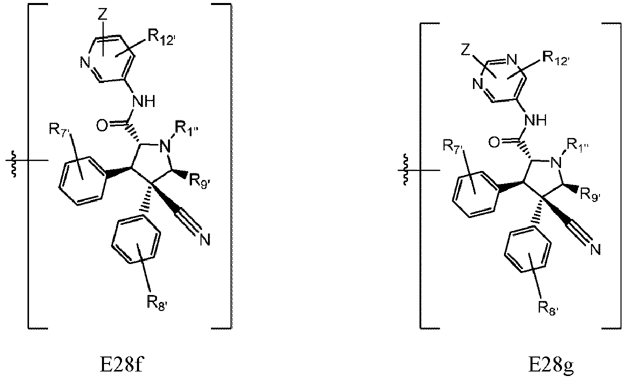 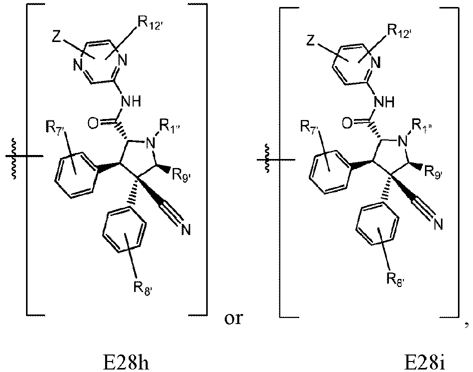
wherein in E28, E28a, E28b, E28c, E28d, E28e, E28f, E28g, E28h and E28i: R_{1'} and R_{2'} are each independently F, Cl, Br, I, ethynyl, cyano, nitro or CF₃;
R_{3'} is selected from -OCH₃, -OCH₂CH₃, -OCH₂CH₂F, -OCH₂CH₂OCH₃ and -OCH(CH₃)₂;
R_{4'} is selected from hydrogen, halogen, methyl, -CF₃, -OCH₃, -C(CH₃)₃, -CH(CH₃)₂, -cyclopropyl, cyano, - C(CH₃)₂OH, -C(CH₃)₂OCH₂CH₃, -C(CH₃)₂OCH₂OH, -C(CH₃)₂CH₂OCH₂CH₃, -C(CH₃)₂CH₂OCH₂CH₂OH, - C(CH₃)₂CN, -C(CH₃)₂C(O)CH₃, -C(CH₃)₂C(O)NHCH₃, -C(CH₃)₂C(O)N(CH₃)₂, -SCH₃, -SCH₂CH₃, -S(O)₂CH₃, - S(O)₂CH₂CH₃, -NHC(CH₃)₃, -NC(CH₃)₂, pyrrolidinyl and morpholinyl; R_{5'} is selected from halogen, cyclopropyl, -S(O)₂CH₃, -S(O)₂CH₂CH₃, pyrrolidinyl, -NH₂, -N(CH₃)₂ and - NHC(CH₃)₃;
R_{6'} is selected from hydrogen, 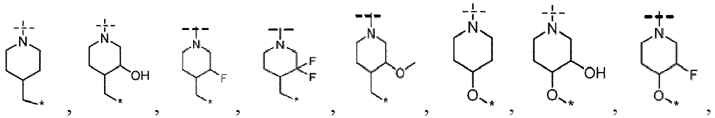 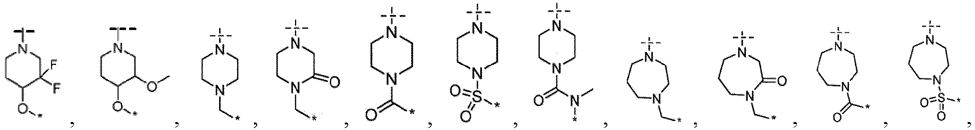 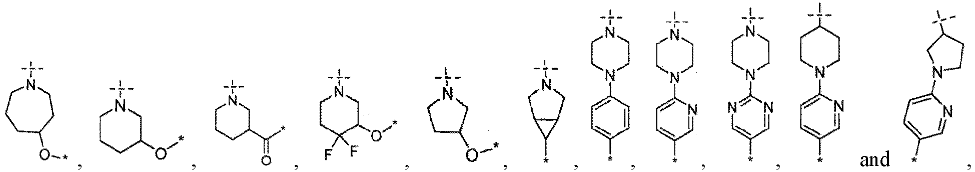
wherein the × in R_{6'} is a linking point linked to L, or R^{4'} may also be linked to L;
R_{7'} is 1 or more halogens;
R_{8'} is 1 or more groups selected from hydrogen, F, Cl, Br, I, cyano, nitro, ethynyl, cyclopropyl, methyl, ethyl, isopropyl, ethenyl, methoxy, ethoxy, isopropoxy, hydroxy, other C₁-C₆ alkyl, other C₁-C₆ alkenyl and other C₁-C₆ alkynyl;
R_{9'} is optionally substituted alkyl, optionally substituted alkenyl, optionally substituted alkynyl, optionally substituted aryl, optionally substituted heteroaryl or optionally substituted cycloalkyl;
Z is hydrogen, -OCH₃, -OCH₂CH₃ or halogen;
R_{10'} and R_{11'} are each independently hydrogen, (CH₂)ₙ-R', (CH₂)ₙ-NR'R", (CH₂)ₙ-NR'C(O)R", (CH₂)ₙ-NR'S(O)₂R", (CH₂)ₙ-C(O)OH, (CH₂)ₙ-C(O)OR', (CH₂)ₙ-C(O)NR'R", (CH₂)ₙ-OR', (CH₂)ₙ-SR', (CH₂)ₙ-SOR', (CH₂)ₙ-CH(OH)-R', (CH₂)ₙ-C(O)R', (CH₂)ₙ-S(O)₂R', (CH₂)ₙ-S(O)NR'R", (CH₂)ₙ-S(O)₂NR'R", (CH₂CH₂O)ₘ-(CH₂)ₙ-R', (CH₂CH₂O)ₘ-(CH₂)ₙ-OH,
(CH₂CH₂O)ₘ-(CH₂)ₙ-OR', (CH₂CH₂O)ₘ-(CH₂)ₙ-NR'R", (CH₂CH₂O)ₘ-(CH₂)ₙ-NR'C(O)R", (CH₂CH₂O)ₘ-(CH₂)ₙ-S(O)₂NR'R", (CH₂CH₂O)ₘ-(CH₂)ₙ-C(O)OH, (CH₂CH₂O)ₘ-(CH₂)ₙ-C(O)OR', (CH₂CH₂O)ₘ-(CH₂)ₙ-C(O)NR'R", (CH₂CH₂O)ₘ-(CH₂)ₙ-S(O)OR', (CH₂CH₂O)ₘ-(CH₂)ₙ-C(O)R', (CH₂CH₂O)ₘ-(CH₂)ₙ-S(O)NR'R", (CH₂CH₂O)ₘ-(CH₂)ₙ-S(O)₂NR'R", -(CH₂)ₚ-(CH₂CH₂O)ₘ-(CH₂)ₙR', -(CH₂)ₚ-(CH₂CH₂O)ₘ-(CH₂)ₙ-OH, -(CH₂)ₚ-(CH₂CH₂O)ₘ-(CH₂)ₙ-OR', -(CH₂)ₚ-(CH₂CH₂O)ₘ-(CH₂)ₙ-NR'R", -(CH₂)ₚ-(CH₂CH₂O)ₘ-(CH₂)ₙ-NR'C(O)R", -(CH₂)ₚ-(CH₂CH₂O)ₘ-(CH₂)ₙ-NR'S(O)₂R", -(CH₂)ₚ-(CH₂CH₂O)ₘ-(CH₂)ₙ-C(O)OH, -(CH₂)ₚ-(CH₂CH₂O)ₘ-(CH₂)ₙ-C(O)OR', -(CH₂)ₚ-(CH₂CH₂O)ₘ-(CH₂)ₙ-C(O)NR'R", -(CH₂)ₚ-(CH₂CH₂O)ₘ-(CH₂)ₙ-S(O)₂R', -(CH₂)ₚ-(CH₂CH₂O)ₘ-(CH₂)ₙ-C(O)R',
-(CH₂)ₚ-(CH₂CH₂O)ₘ-(CH₂)ₙ-S(O)NR'R", -(CH₂)ₚ-(CH₂CH₂O)ₘ-(CH₂)ₙ-S(O)₂NR'R", aryl-(CH₂)ₙ-C(O)OH or heteroaryl-alkyl-C(O)-alkyl-NR'R", and the alkyl is optionally substituted with OR'; or R^{10'} and R^{11'} are each independently heteroaryl-(CH₂)ₙ-heterocycloalkyl, and the heterocycloalkyl is optionally substituted with alkyl, hydroxy, C(O)OR' or C(O)R'; R' and R" are each independently selected from hydrogen, halogen, hydroxy, NH₂, NH(alkyl), NH(alkyl)₁₋₂, oxo, carboxyl, cycloalkyl and heteroaryl;
m, n and p are each independently 0, 1, 2, 3, 4, 5 or 6;
each R_{12'} is independently -O-alkyl, -O-alkyl-alkoxy, -C(O)-alkyl, -C(OH)-alkyl-alkoxy, -C(O)-NH-alkyl, -C(O)-N-(alkyl)₁₋₂, -S(O)-alkyl, S(O)₂-alkyl, -C(O)-cyclic amine, -O-aryl-alkyl or -O-aryl-alkoxy;
R^{1"} is selected from hydrogen, alkyl optionally substituted with aryl or alkoxy and cycloalkyl optionally substituted with aryl or alkoxy.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E29, 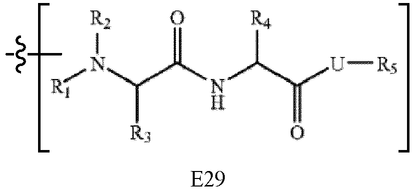
R₁ is independently selected from H, C₁-C₄-alkyl, C₁-C₄-alkenyl, C₁-C₄-alkynyl and C₃-C₁₀-cycloalkyl, which are unsubstituted or substituted;
R₂ is independently selected from H, C₁-C₄-alkyl, C₁-C₄-alkenyl, C₁-C₄-alkynyl and C₃-C₁₀-cycloalkyl, which are unsubstituted or substituted;
R₃ is independently selected from H, -CF₃, -C₂H₅, C₁-C₄-alkyl, C₁-C₄-alkenyl, C₁-C₄-alkynyl and -CH₂-Z, or any R₂and R₃ together form a heterocycle;
each Z is independently selected from H, -OH, F, Cl, -CH₃, -CF₃, -CH₂Cl, -CH₂F and -CH₂OH;
R₄ is independently selected from C₁-C₁₆ linear or branched alkyl, C₁-C₁₆-alkenyl, C₁-C₁₆-alkynyl, C₃-C₁₀-cycloalkyl, -(CH₂)₀₋₆-Z₁, -(CH₂)₀₋₆-aryl and -(CH₂)₀₋₆-het, wherein alkyl, cycloalkyl and phenyl are unsubstituted or substituted;
R₅ is independently selected from H, C₁₋₁₀-alkyl, aryl, phenyl, C₃₋₇-cycloalkyl, -(CH₂)₁₋₆-C₃₋₇-cycloalkyl, -C₁₋₁₀-alkyl-aryl, -(CH₂)₀₋₆-C₃₋₇-cycloalkyl-(CH₂)₀₋₆-phenyl, -(CH₂)₀₋₄-CH[(CH₂)₁₋₄-phenyl]₂, indanyl, -C(O)-C₁₋₁₀-alkyl, - C(O)-(CH₂)₁₋₆-C₃₋₇-cycloalkyl, -C(O)-(CH₂)₀₋₆-phenyl, -(CH₂)₀₋₆-C(O)-phenyl, -(CH₂)₀₋₆-het and -C(O)-(CH₂)₁₋₆-het, or R₅ is selected from amino acid residues, wherein alkyl, cycloalkyl, phenyl and aryl substituents are unsubstituted or substituted;
Z₁ is independently selected from -N(R₁₀)-C(O)-C₁₋₁₀-alkyl, -N(R₁₀)-C(O)-(CH₂)₀₋₆-C₃₋₇-cycloalkyl, -N(R₁₀)-C(O)-(CH₂)₀₋₆-phenyl, -N(R₁₀)-C(O)(CH₂)₁₋₆-het, -C(O)-N(R₁₁)(R₁₂), -C(O)-O-C₁₋₁₀-alkyl, -C(O)-O-(CH₂)₁₋₆-C₃₋₇-cycloalkyl, -C(O)-O-(CH₂)₀₋₆-phenyl, -C(O)-O-(CH₂)₁₋₆-het, -O-C(O)-C₁₋₁₀-alkyl, -O-C(O)-(CH₂)₁₋₆-C₃₋₇-cycloalkyl, -O-C(O)-(CH₂)₀₋₆-phenyl and -O-C(O)-(CH₂)₁₋₆-het, wherein alkyl, cycloalkyl and phenyl are unsubstituted or substituted;
het is independently selected from a 5- to 7-membered heterocyclic or 8- to 12-membered fused ring system containing 1-4 heteroatoms selected from N, O and S, comprising at least one 5- to 7-membered heterocycle containing 1, 2 or 3 heteroatoms selected from N, O and S, and the heterocyclic or fused ring system is unsubstituted or substituted on a carbon or nitrogen atom;
R₁₀ is selected from H, -CH₃, -CF₃, -CH₂OH and -CH₂Cl;
R₁₁ and R₁₂ are independently selected from H, C₁₋₄-alkyl, C₃₋₇-cycloalkyl, -(CH₂)₁₋₆-C₃₋₇-cycloalkyl and (CH₂)₀₋₆-phenyl, wherein alkyl, cycloalkyl and phenyl are unsubstituted or substituted; or R₁₁ and R₁₂, together with nitrogen, form het; and
U is independently 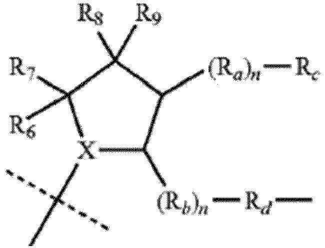 wherein:
   each n is independently selected from 0-5;
   X is selected from -CH and N;
   Rₐ and R_{b} are independently selected from an O, S and N atom and C₀₋₈-alkyl, wherein one or more carbon atoms in the alkyl chain are optionally replaced by a heteroatom selected from O, S and N, and each alkyl is independently unsubstituted or substituted;
   R_{d} is selected from Re-Q-(R_{f})ₚ(R_{g})_{q} and Ar₁-D-Ar₂;
   R_{c} is selected from H, or any R_{c} and R_{d} together form cycloalkyl or het, wherein if R_{c} and R_{d} form cycloalkyl or het, R₅ is linked to the ring formed at a C or N atom;
   p and q are independently selected from 0 and 1;
   Rₑ is selected from C₁₋₈-alkyl and alkylene, and each Rₑ is unsubstituted or substituted;
   Q is selected from N, O, S, S(O) and S(O)₂;
   Ar₁ and Ar₂ are independently selected from substituted or unsubstituted aryl and het;
   R_{f} and R_{g} are independently selected from H, -C₁₋₁₀-alkyl, C₁₋₁₀-alkylaryl, -OH, -O-C₁₋₁₀-alkyl, -(CH₂)₀₋₆-C₃₋₇-cycloalkyl, -O-(CH₂)₀₋₆-aryl, phenyl, aryl, phenyl-phenyl, -(CH₂)₁₋₆-het, -O-(CH₂)₁₋₆-het, -OR₁₃, -C(0)-R₁₃, -C(O)-N(R₁₃)(R₁₄), -N(R₁₃)(R₁₄), -S-R₁₃, -S(0)-R₁₃, -S(O)₂-R₁₃, -S(O)₂-NR₁₃R₁₄, -NR₁₃-S(O)₂-R₁₄, -S-Cₜ₋₁₀-alkyl, aryl-C₁₋₄-alkyl and het-C₁₋₄-alkyl, wherein alkyl, cycloalkyl, het and aryl are unsubstituted or substituted, and -SO₂-C₁₋₂-alkyl, -SO₂-C₁₋₂-alkylphenyl, -O-C₁₋₄-alkyl or any R_{g} and R_{f} together form a ring selected from het and aryl;
   D is selected from -CO-, -C(O)-C₁₋₇-alkylene or arylene, -CF₂-, -O-, and -S(O)ᵣ, wherein r is 0-2, 1,3-dioxolane or C₁₋₇-alkyl-OH, and alkyl and alkylene or arylene are unsubstituted or substituted with one or more halogens, OH, - O-C₁₋₆-alkyl, -S-C₁₋₆-alkyl or -CF₃; or each D is independently selected from N(Rₕ);
   Rₕ is selected from H, unsubstituted or substituted C₁₋₇-alkyl, aryl, unsubstituted or substituted -O-(C₁₋₇-cycloalkyl), -C(O)-C₁₋₁₀-alkyl, -C(O)-C₀₋₁₀-alkyl-aryl, -C-O-C₁₋₁₀-alkyl, -C-O-C₀₋₁₀-alkyl-aryl, -SO₂-C₁₋₁₀-alkyl and -SO₂-(C₀₋₁₀-alkylaryl);
   R₆, R₇, R₈ and R₉ are independently selected from H, -C₁₋₁₀-alkyl, -C₁₋₁₀-alkoxy, aryl-C₁₋₁₀-alkoxy, -OH, -O-C₁₋₁₀-alkyl, -(CH₂)₀₋₆-C₃₋₇-cycloalkyl, -O-(CH₂)₀₋₆-aryl, phenyl, -(CH₂)₁₋₆-het, -O-(CH₂)₁₋₆-het, -OR₁₃, -C(O)-R₁₃, -C(O)-N(R₁₃)(R₄), -N(R₁₃)(R₁₄), -S-R₁₃, -S(O)-R₁₃, -S(O)₂-R₁₃, -S(O)₂-NR₁₃R₁₄ and -NR₁₃-S(O)₂-R₁₄, wherein each alkyl, each cycloalkyl and each aryl are unsubstituted or substituted, and any R₆, R₇, R₈ and R₉ optionally together form a ring system;
   R₁₃ and R₁₄ are independently selected from H, C₁₋₁₀-alkyl, -(CH₂)₀₋₆-C₃₋₇-cycloalkyl, -(CH₂)₀₋₆-(CH)₀₋₁-(aryl)₁₋₂, - C(O)-C₁₋₁₀-alkyl, -C(O)-(CH₂)₁₋₆-C₃₋₇-cycloalkyl, -C(O)-O-(CH₂)₀₋₆-aryl, -C(O)-(CH₂)₀₋₆-O-fluorenyl, -C(O)-NH-(CH₂)₀₋₆-aryl, -C(O)-(CH₂)₀₋₆-aryl, -C(O)-(CH₂)₀₋₆-het, -C(S)-C₁₋₁₀-alkyl, -C(S)-(CH₂)₁₋₆-C₃₋₇-cycloalkyl, -C(S)-O-(CH₂)₀₋₆-aryl, -C(S)-(CH₂)₀₋₆-O-fluorenyl, -C(S)-NH-(CH₂)₀₋₆-aryl, -C(S)-(CH₂)₀₋₆-aryl and -C(S)-(CH₂)₁₋₆-het, wherein each alkyl, each cycloalkyl and each aryl are unsubstituted or substituted, or any R₁₃ and R₁₄, together with a nitrogen atom, form het;
   alkyl substituents of R₁₃ and R₁₄ are unsubstituted or substituted, and when substituted, the substituent is substituted with one or more substituents selected from C₁₋₁₀-alkyl, halogen, OH, -O-C₁₋₆-alkyl, -S-C₁₋₆-alkyl and -CF₃; and substituted phenyl or aryl of R₁₃ and R₁₄ is substituted with one or more substituents selected from halogen, hydroxy, C₁₋₄-alkyl, C₁₋₄-alkoxy, nitro, -CN, -O-C(O)-C₁₋₄-alkyl, -C(O)-O-C₁₋₄-aryl and a pharmaceutically acceptable salt or hydrate thereof.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E30, E30a, E30b, E30c or E30d, 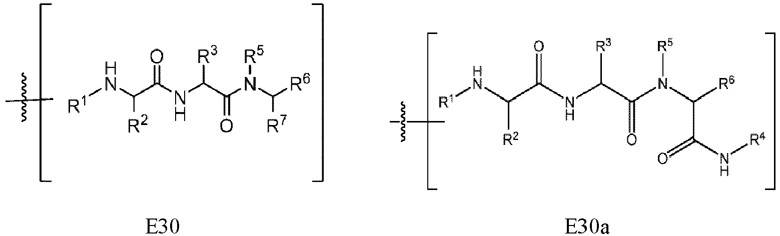 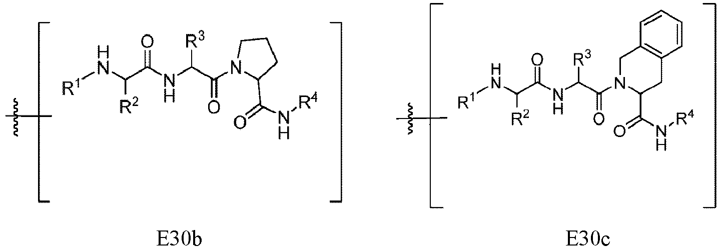 or 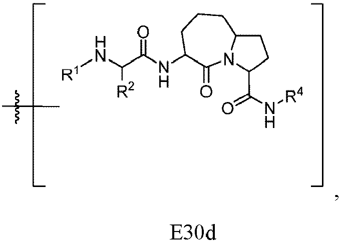 wherein in E30, E30a, E30b, E30c and E30d,
R¹ is selected from hydrogen and alkyl;
is selected from hydrogen and alkyl;
R³ is selected from hydrogen, alkyl, cycloalkyl and heterocycloalkyl;
R⁵ and R⁶ are each independently selected from hydrogen, alkyl, cycloalkyl and heterocycloalkyl; or R⁵ and R⁶, together with the atom linked thereto, form pyrrolidinyl or a piperidine ring and are optionally fused with 1-2 cycloalkyl, heterocycloalkyl, aryl or heteroaryl rings, and each of the rings is further optionally fused to another cycloalkyl, heterocycloalkyl, aryl or heteroaryl ring;
or R⁵ and R³, together with the atom linked thereto, form a 5- to 8-membered ring and are optionally fused with 1 or 2 cycloalkyl, heterocycloalkyl, aryl or heteroaryl rings;
is selected from alkyl, cycloalkyl, heterocycloalkyl, cycloalkylalkyl, heterocycloalkylalkyl, aryl or bicyclic aryl, arylalkyl, heteroaryl or bicyclic heteroaryl and heteroarylalkyl, and is further optionally substituted with 1-3 alkyl, cycloalkyl, heterocycloalkyl, cycloalkylalkyl, heterocycloalkylalkyl, aryl or bicyclic aryl, arylalkyl, heteroaryl or bicyclic heteroaryl or heteroarylalkyl substituents.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E31, 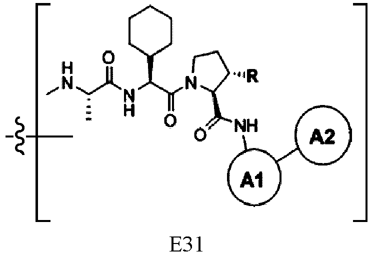
wherein in E31, A1 and A2 are each independently selected from optionally substituted monocyclic rings, fused rings, aryl and heteroaryl;
R is selected from H and Me.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E32, 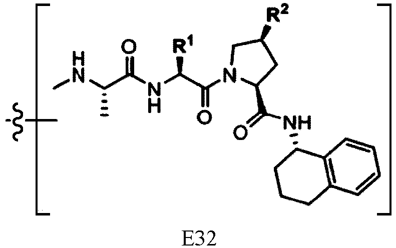 wherein in E32,
R¹ is selected from alkyl, cycloalkyl and heterocycloalkyl, and is most preferably selected from isopropyl, *tert-*butyl, cyclohexyl and tetrahydropyranyl; is selected from -OPh and H.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E33, 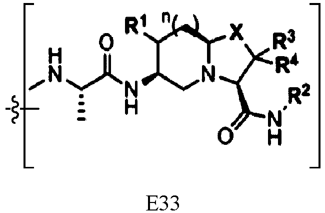 wherein in E33,
R¹ is selected from H, -CH₂OH, -CH₂CH₂OH, -CH₂NH₂ and -CH₂CH₂NH₂;
X is selected from S and CH₂;
n is 1, 2 or 3;
is selected from 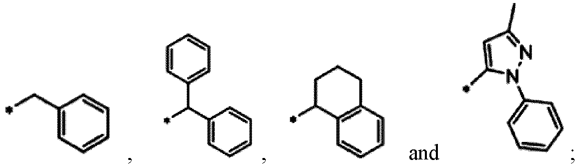
R³ and are independently selected from H and Me.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E34, 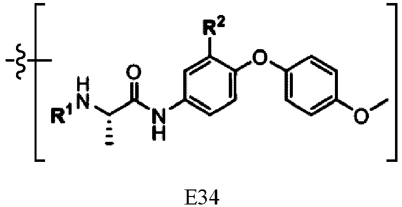 wherein in E34,
R¹ is selected from H and Me;
is selected from Hand

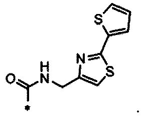

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E35, 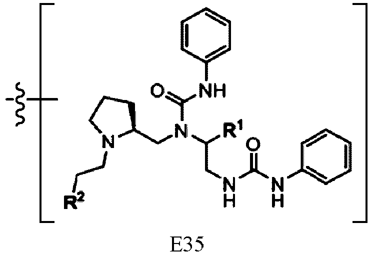 wherein in E35,
R¹ is selected from 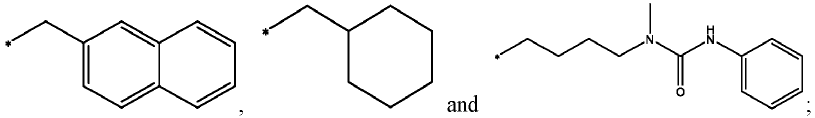
is selected from

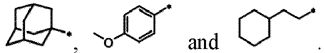

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E36, 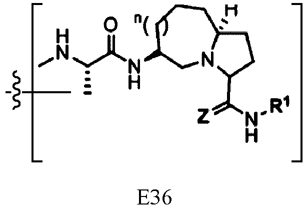 wherein in E36,
n is 0, 1 or 2, preferably 2;
Z is absent or is O;
R¹ is selected from: 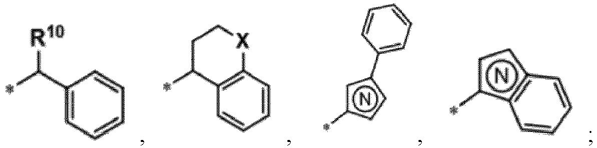
R¹⁰ in 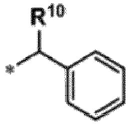 is selected from hydrogen, alkyl and aryl;
X is selected from CH₂ and O; and 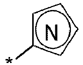 is nitrogen-containing heteroaryl.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E37, 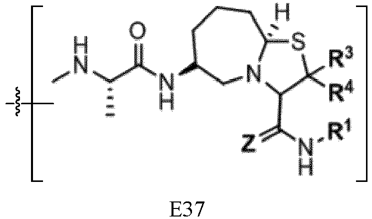 wherein in E37,
Z is absent or is O;
R³ and are independently selected from H and Me;
R¹ is selected from: 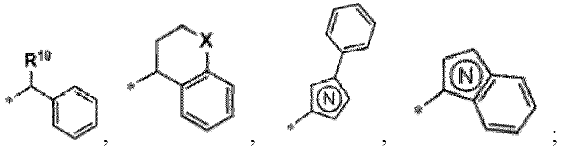
R¹⁰ in 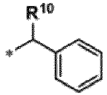 is selected from H, alkyl and aryl;
Xin 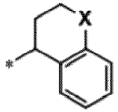 is selected from CH₂ and O; and 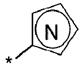 in 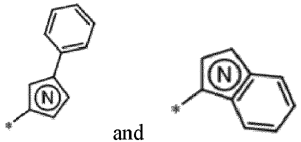 is nitrogen-containing heteroaryl.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E38, 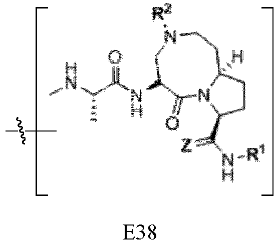 wherein in E38,
Z is absent or is O;
R¹ is selected from: 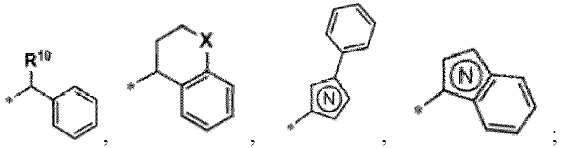
R¹⁰ in 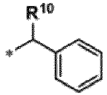 is selected from H, alkyl and aryl;
Xin 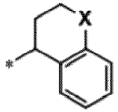 is selected from CH₂ and O; and 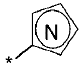 in 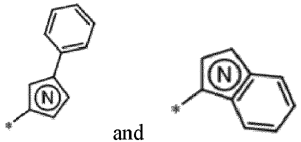 is nitrogen-containing heteroaryl; and
is selected from H, alkyl and acyl.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E39, 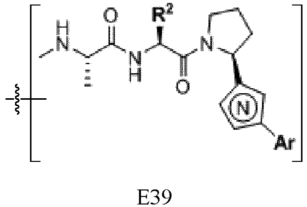 wherein in E39,
is selected from alkyl, cycloalkyl and heterocycloalkyl, more preferably selected from isopropyl, tert-butyl, cyclohexyl and tetrahydropyranyl, and most preferably selected from cyclohexyl; 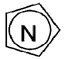
is 5- or 6-membered nitrogen-containing heteroaryl, more preferably 5-membered nitrogen-containing heteroaryl, and most preferably thiazole; and
Ar is aryl or heteroaryl.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E40, 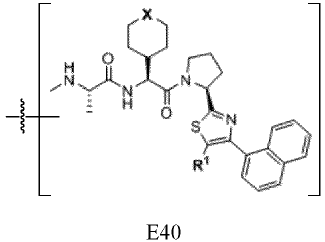 wherein in E40,
R¹ is selected from halogen (e.g., fluorine), cyano, 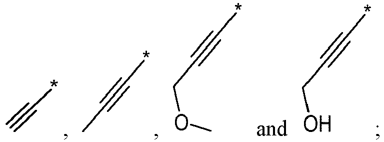
X is selected from O and CH₂.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E41, 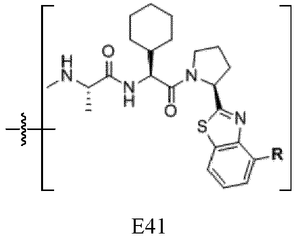 wherein in E41,
R is selected from alkyl, aryl, heteroaryl, arylalkyl, heteroarylalkyl and halogen (in variable substitution positions). In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E42, 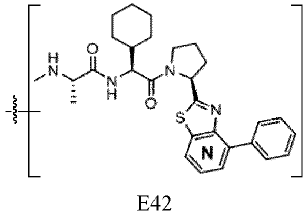 wherein in E42, 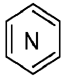 is 6-membered nitrogen-containing heteroaryl.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E43, 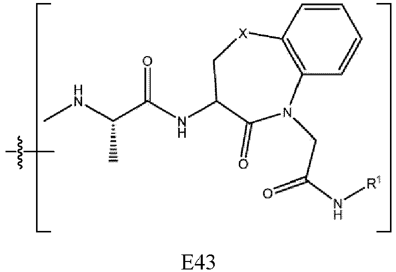 wherein in E43, X is selected from CH₂, O, NH and S.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E44, E44a or E44b, 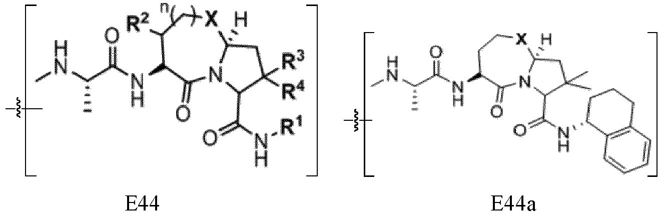 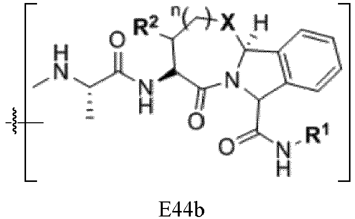 wherein in E44, E44a and E44b,
n is 1 or 2;
R², R³ and are independently selected from H and methyl;
X is independently selected from O and S;
   and
R¹ is selected from:

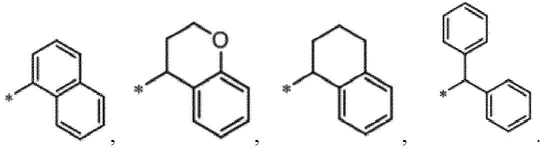

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E45, 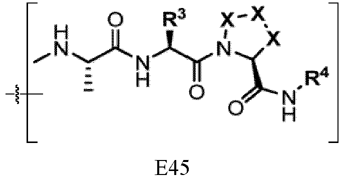 wherein in E45,
R³ and are each independently selected from hydrogen and methyl; 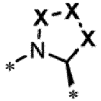 is a 5-membered heterocycle selected from the following:

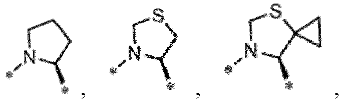

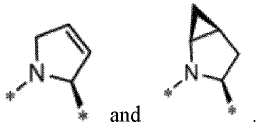

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is E46 or E46a, 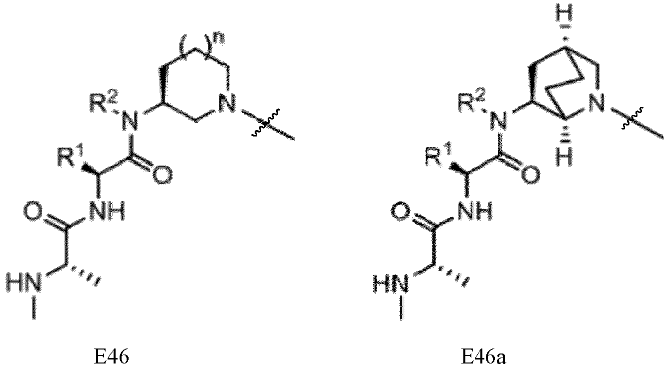 wherein:
R¹ is selected from 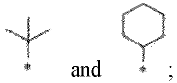
is selected from H and methyl;
n is selected from 0, 1 and 2.

In one preferred embodiment of the compound of formula I, and/or the stereoisomer, the enantiomer, the diastereomer, the deuteride, the hydrate, the solvate, the prodrug and/or the pharmaceutically acceptable salt thereof in the present invention, E is:

### CRBN:

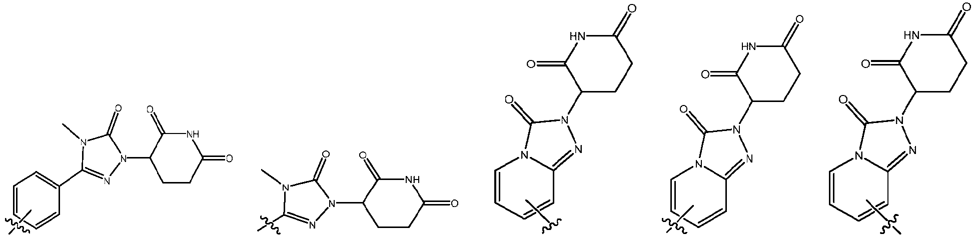

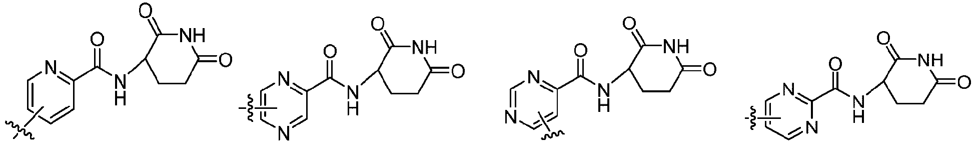

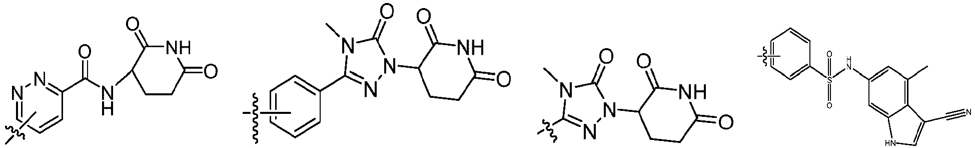

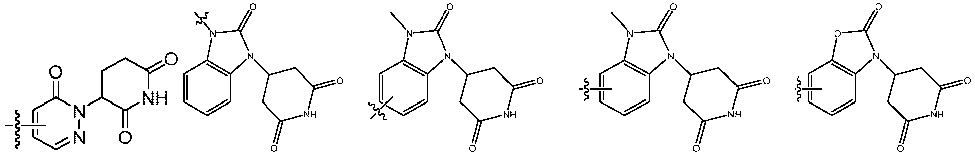

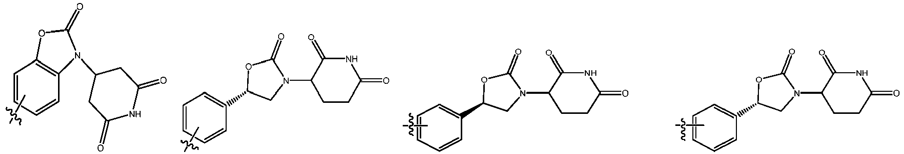

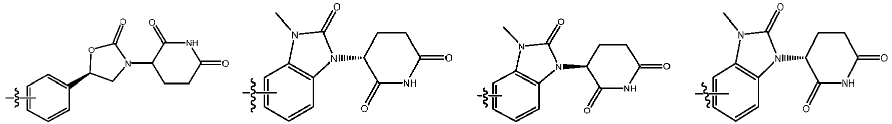

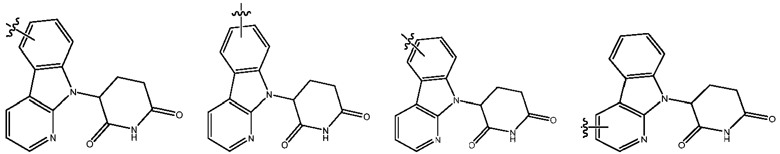

### VHL:

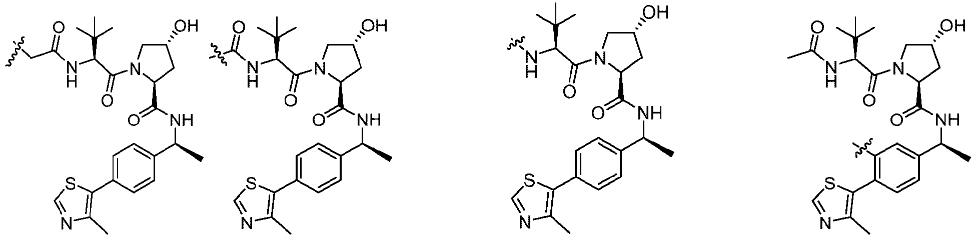

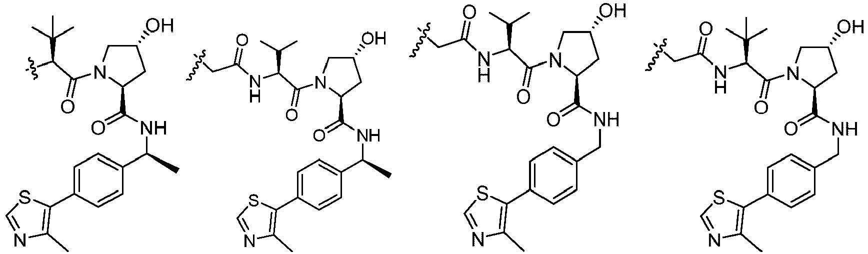

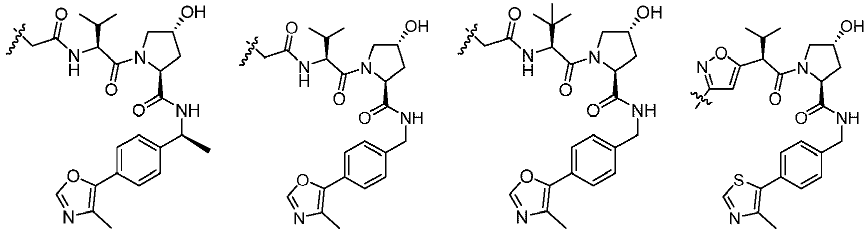

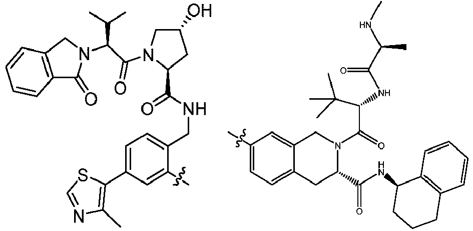

### MDMA:

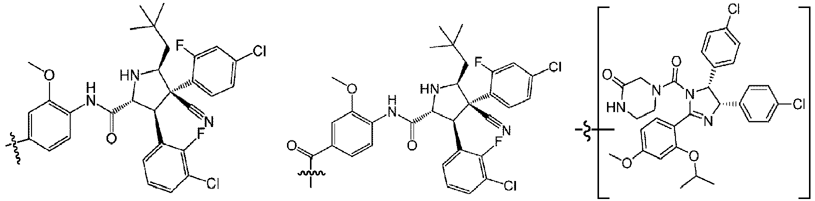

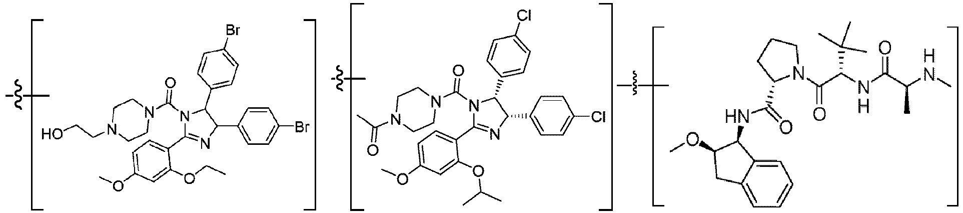

### IAP:

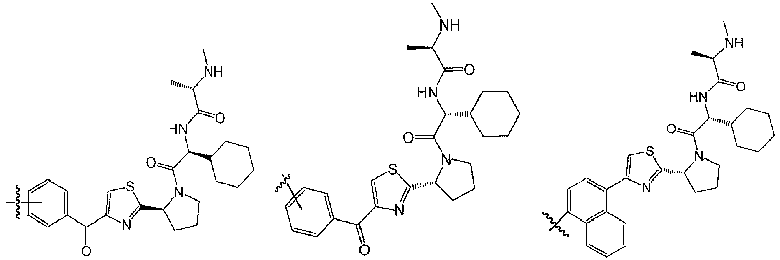

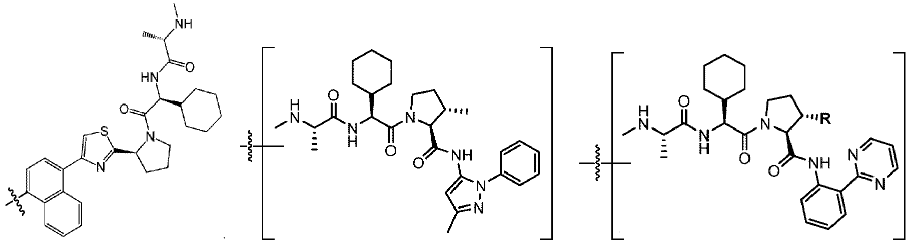

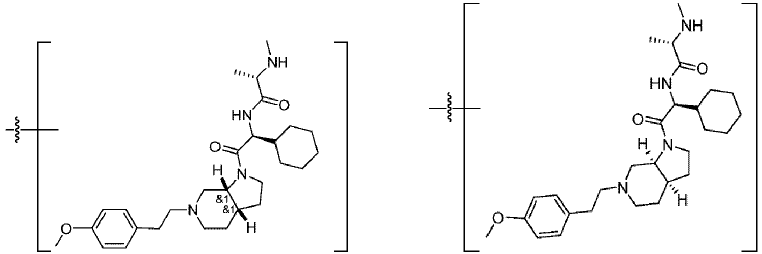

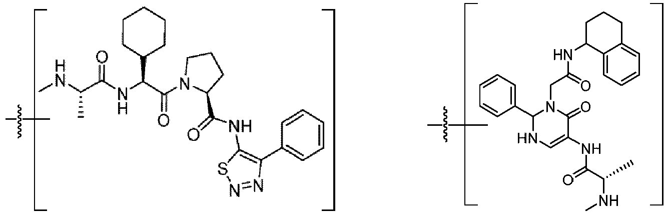

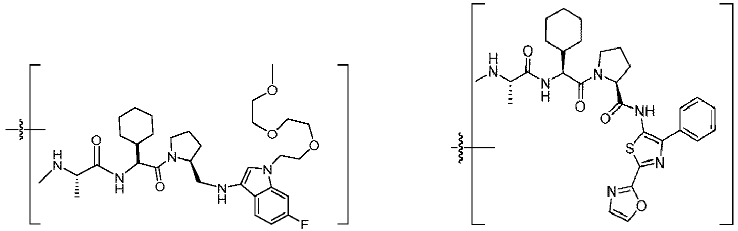

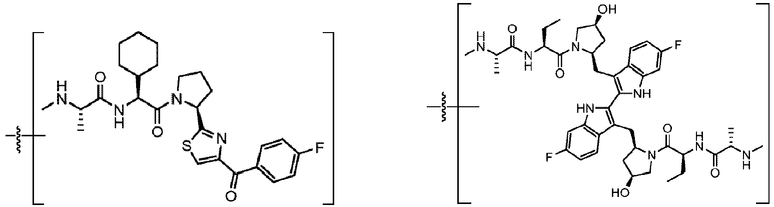

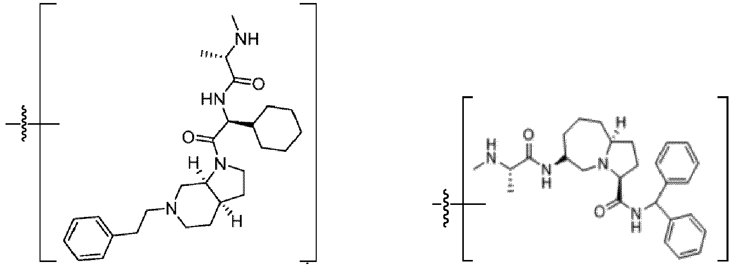

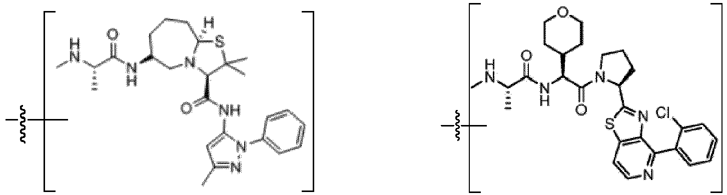

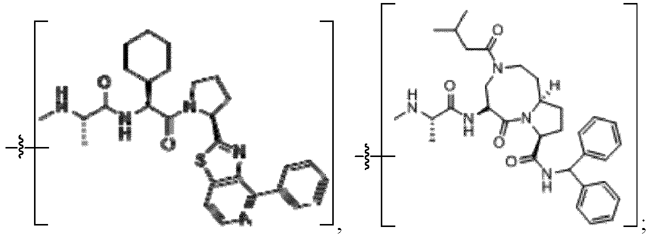

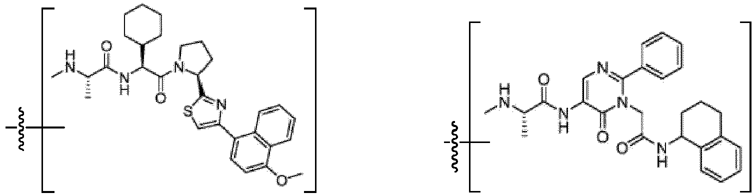

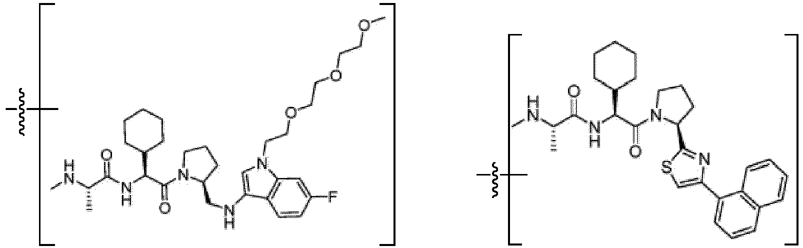

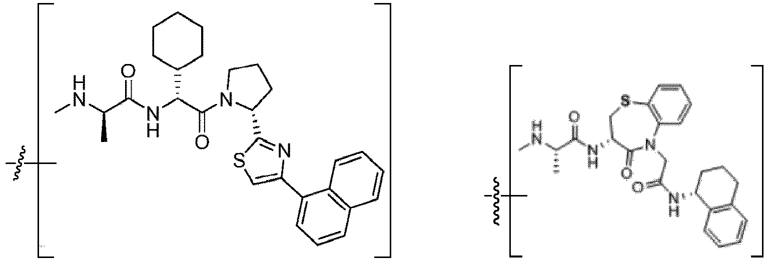

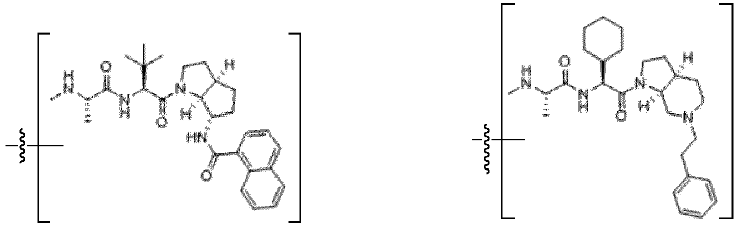

or

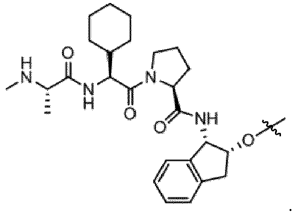

Preferably, in certain embodiments of the present invention, the compound of formula I is

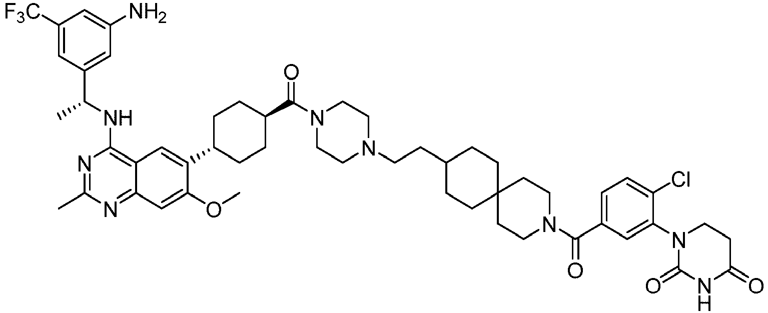

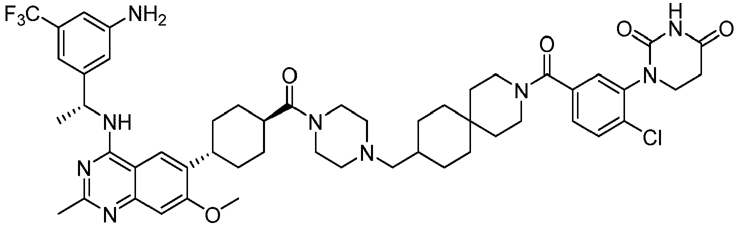

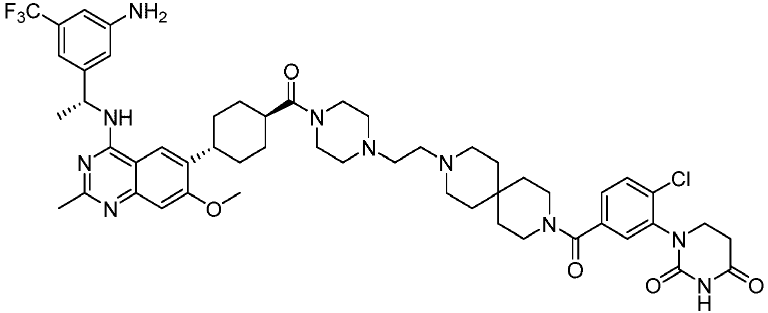

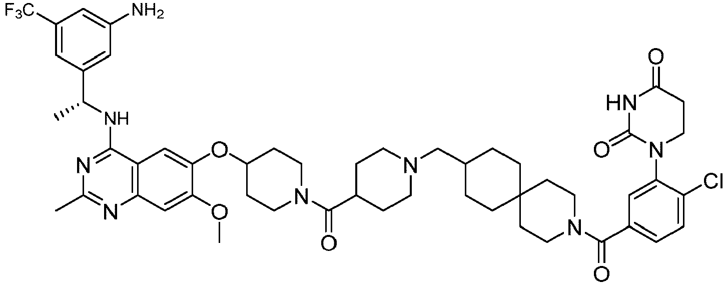

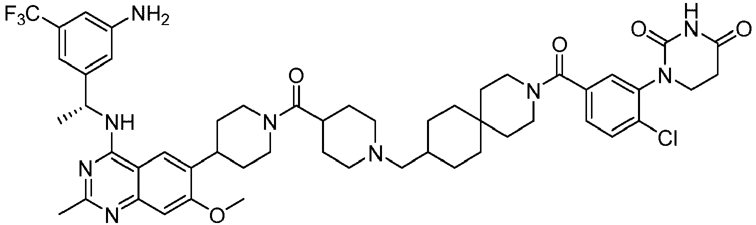

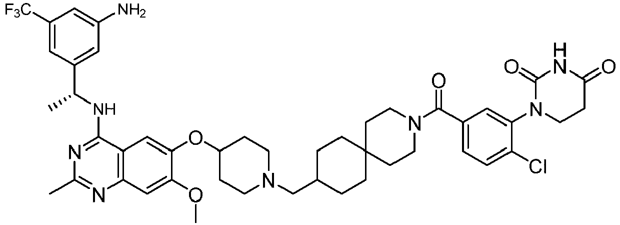

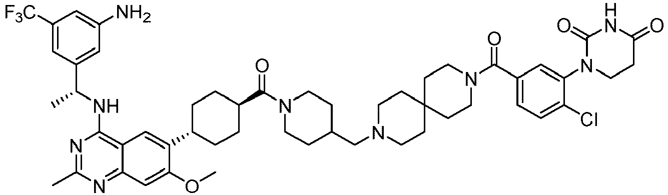

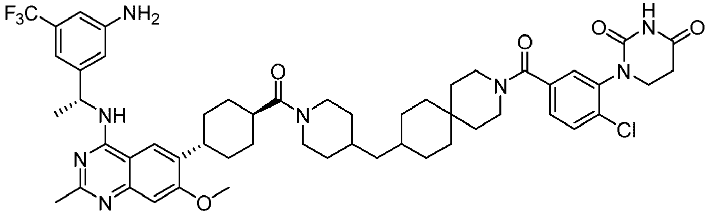

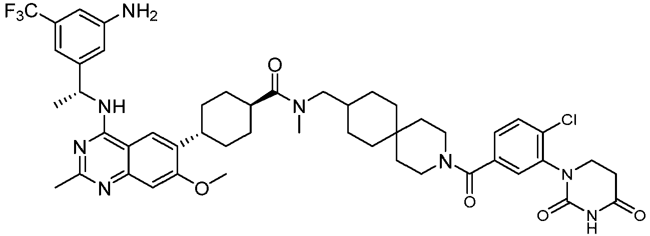

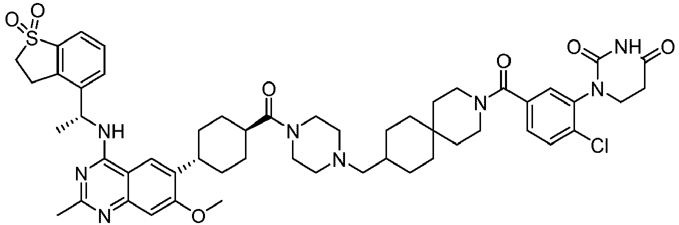

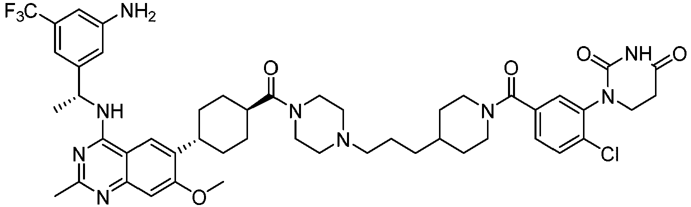

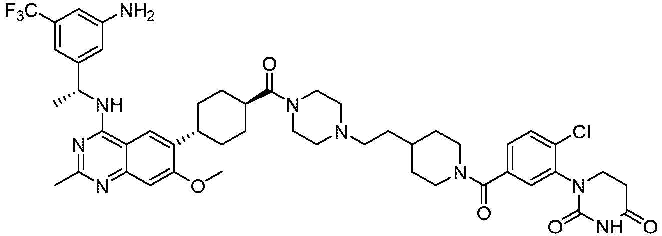

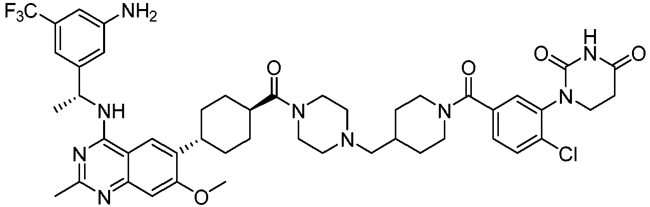

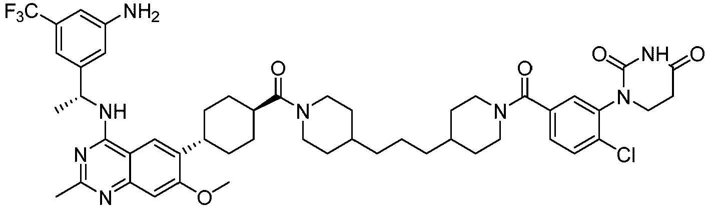

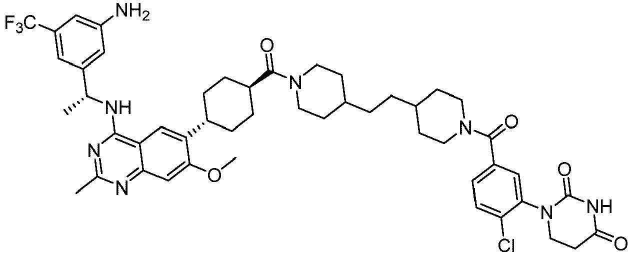

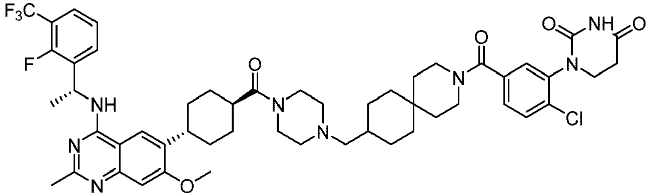

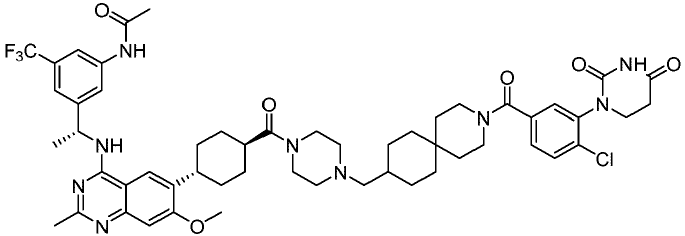

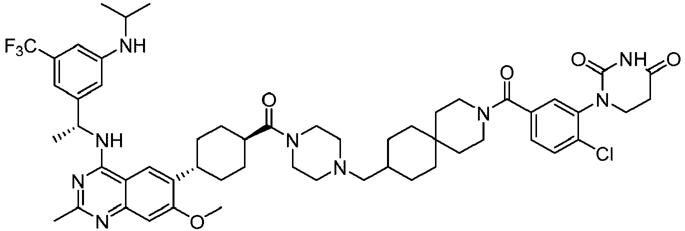

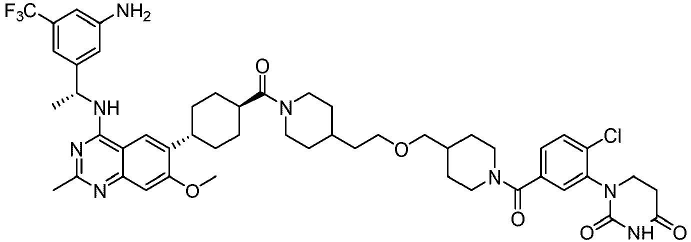

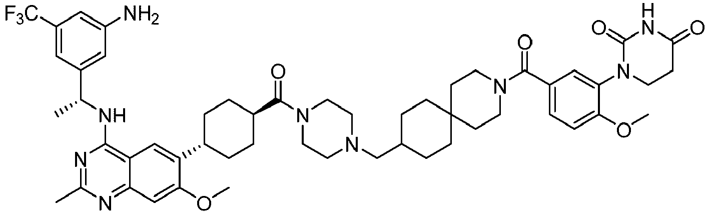

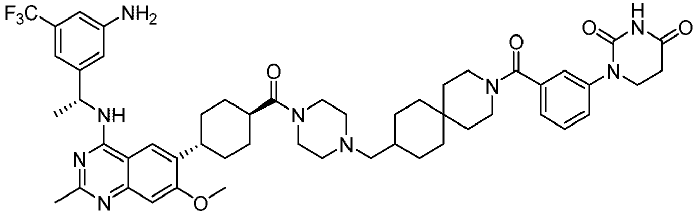

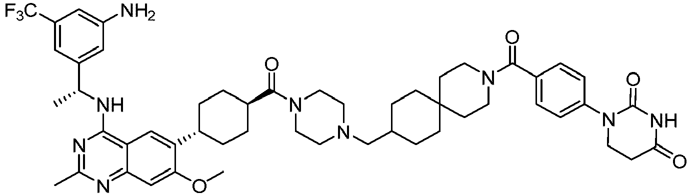

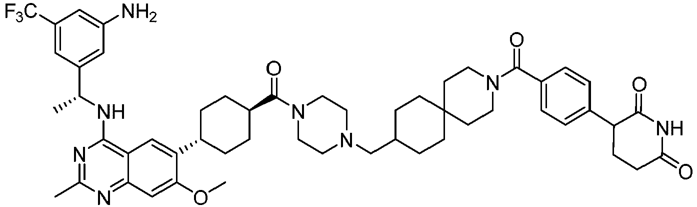

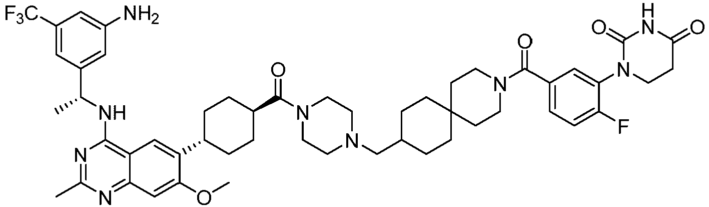

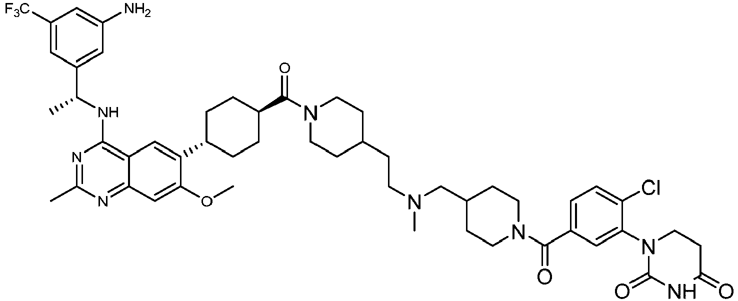

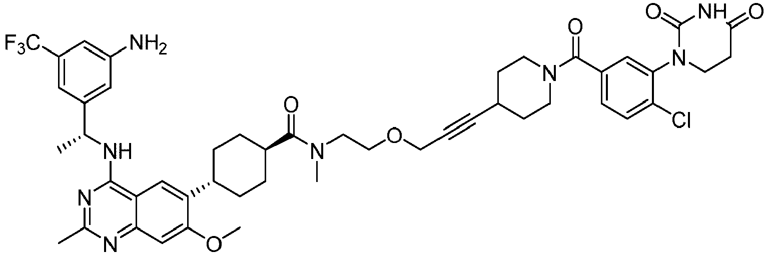

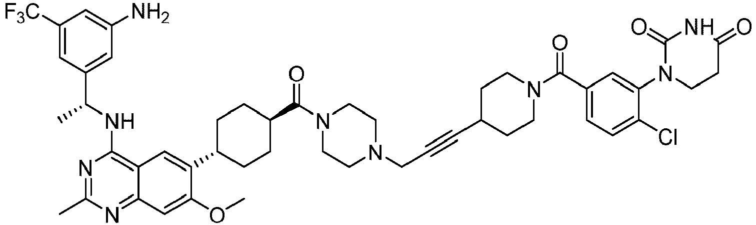

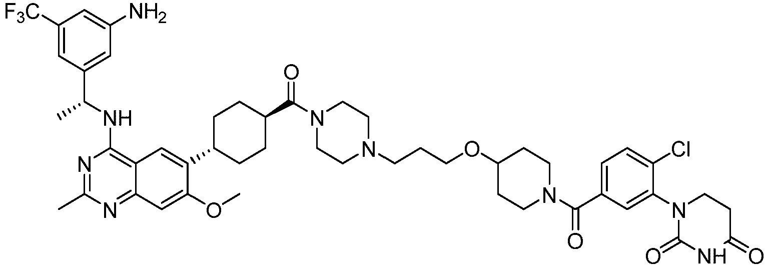

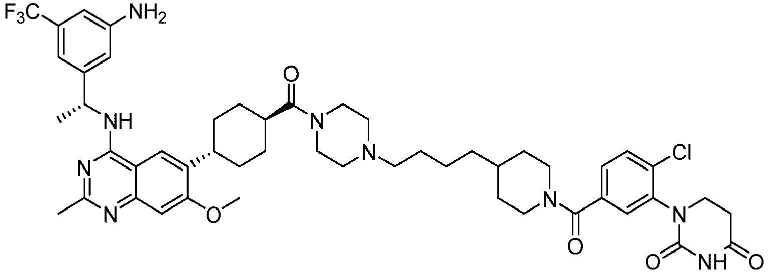

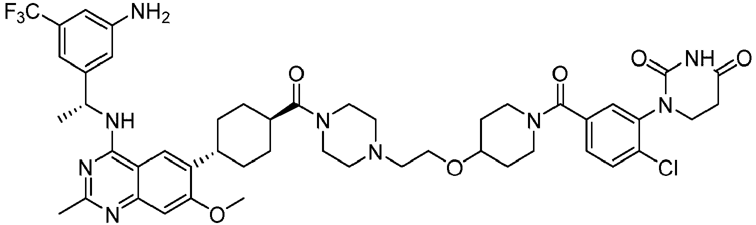

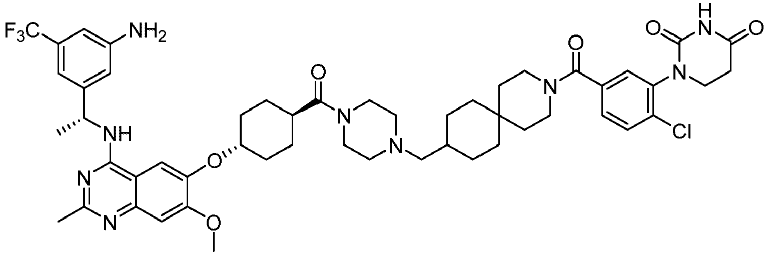

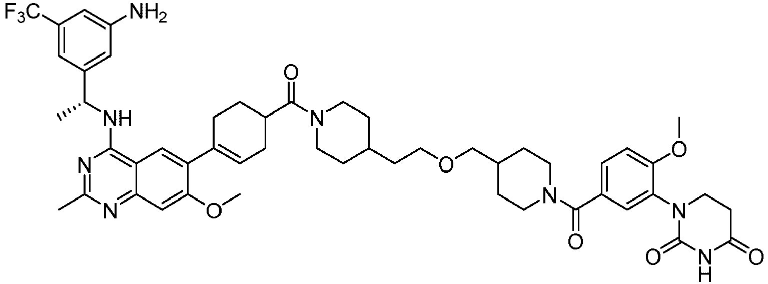

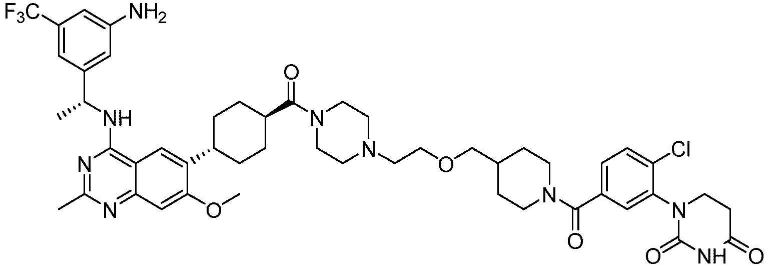

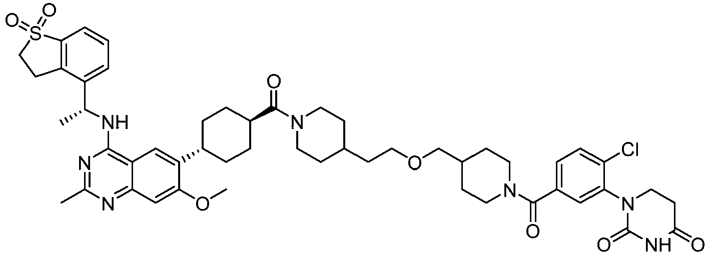

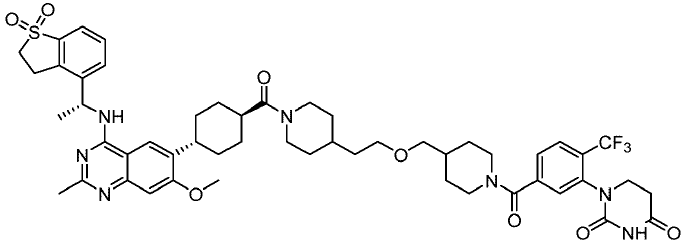

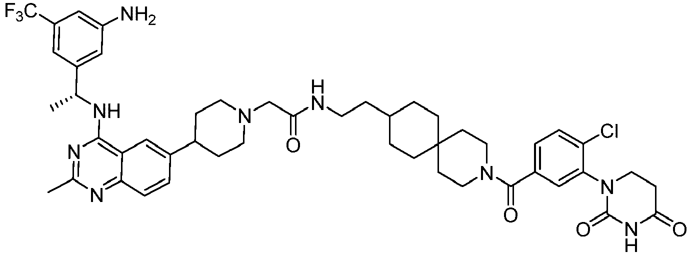

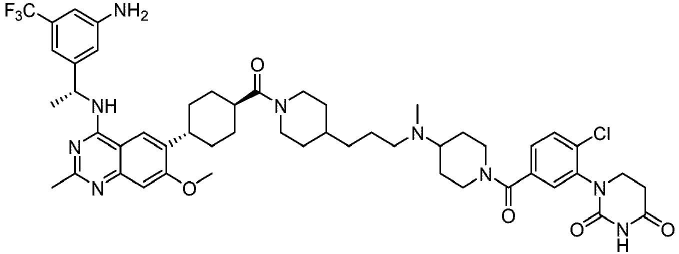

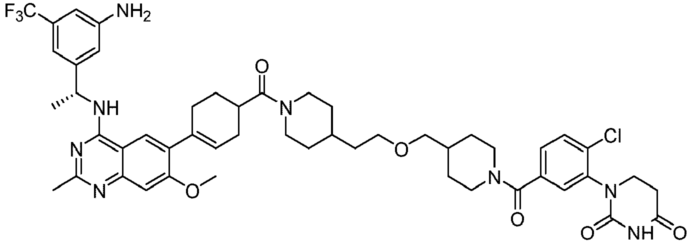

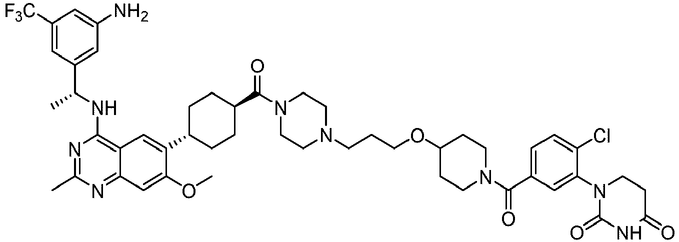

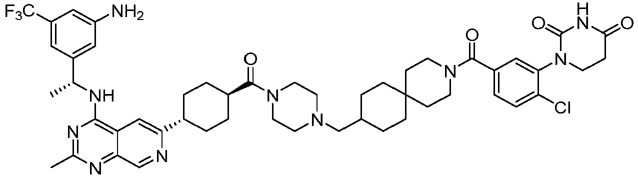

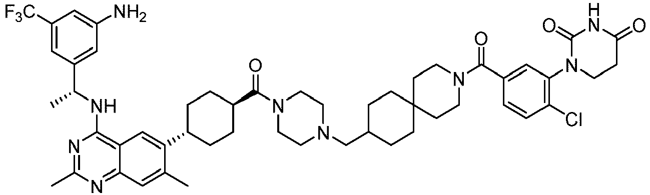

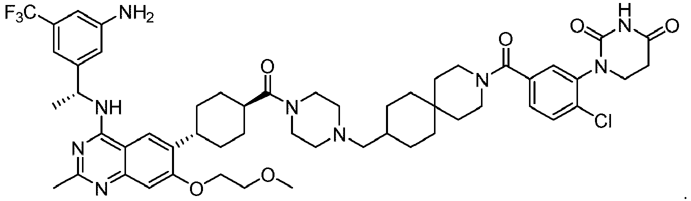

Preferably, in certain embodiments of the present invention, the compound of formula I is

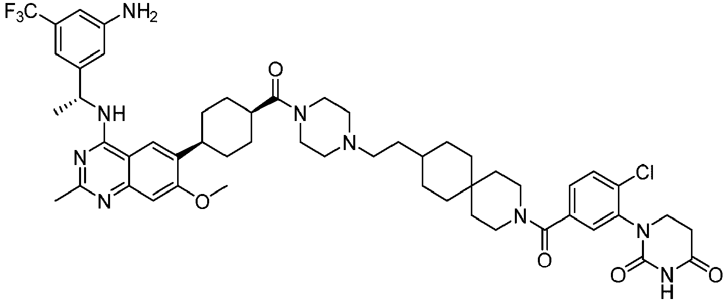

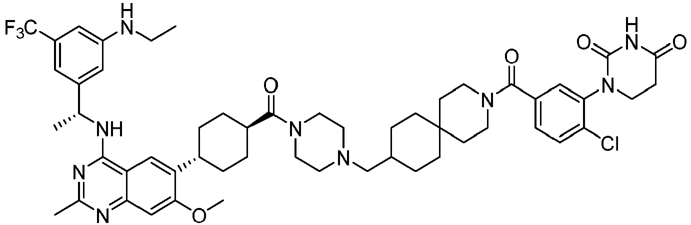

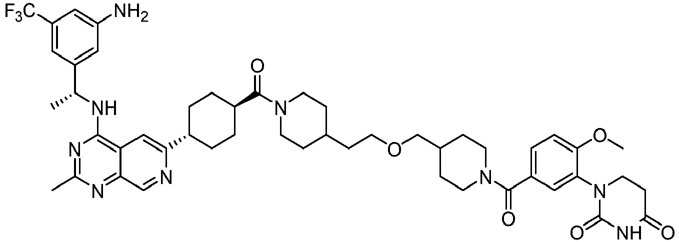

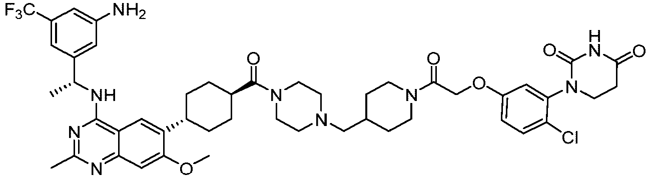

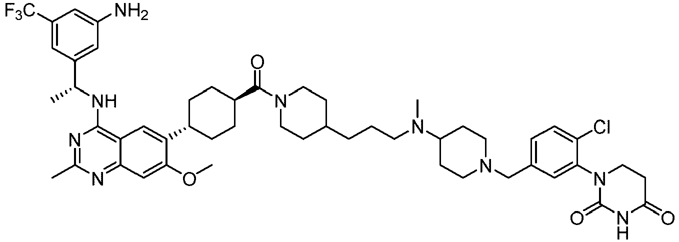

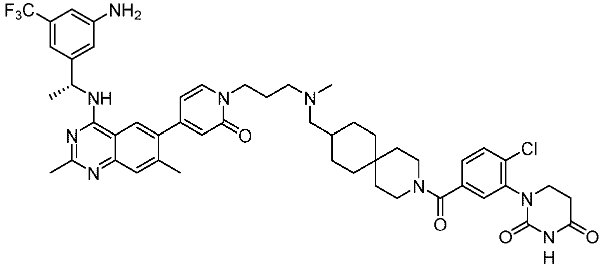

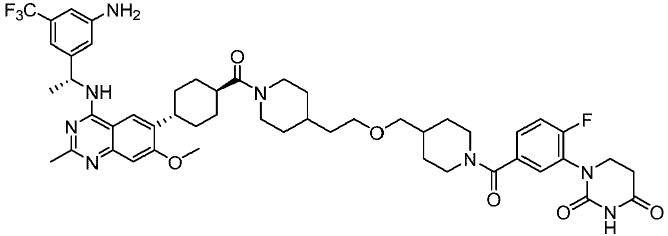

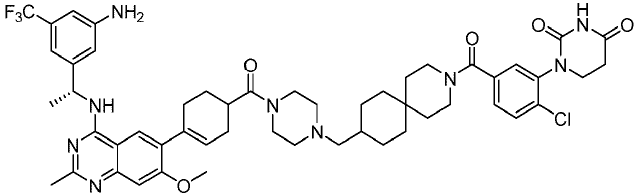

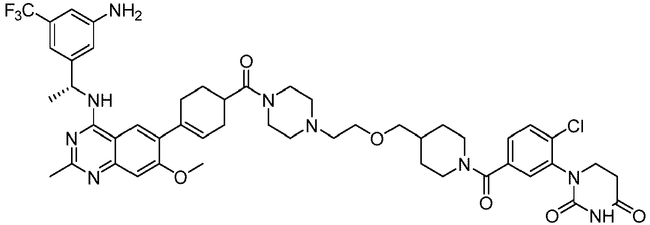

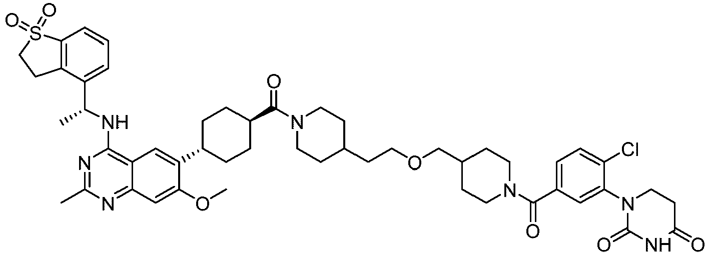

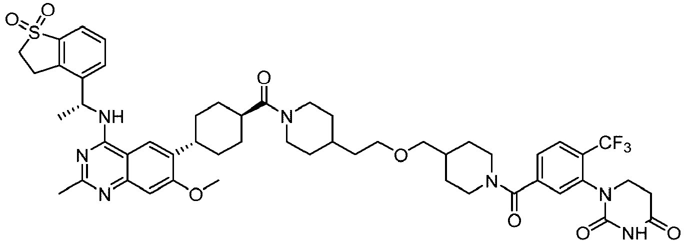

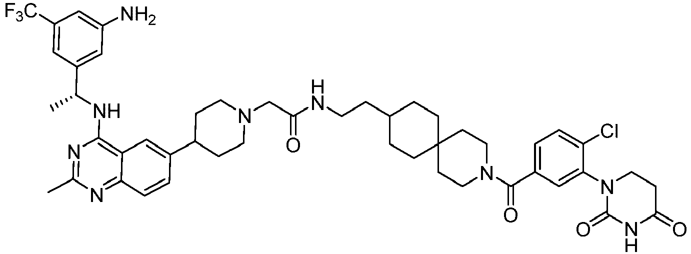

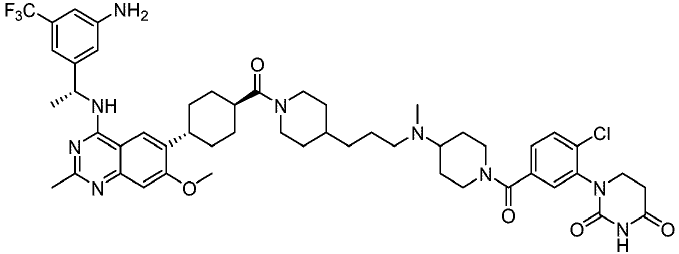

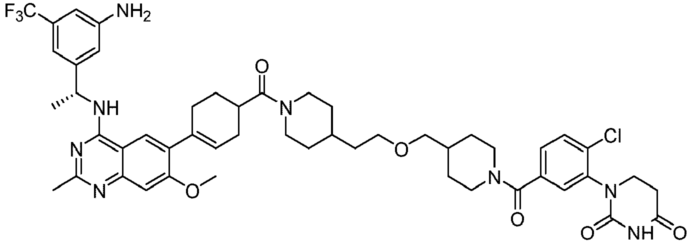

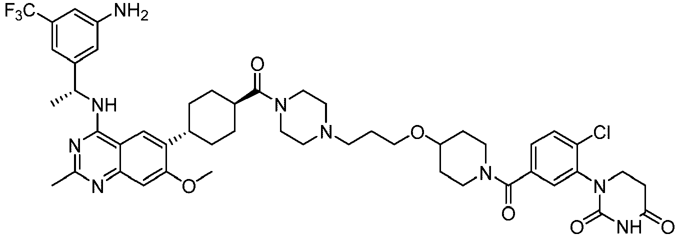

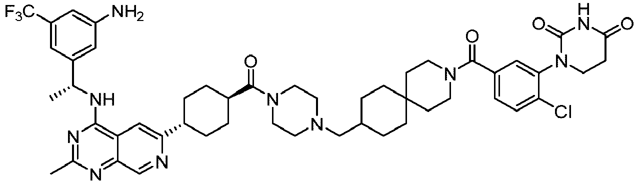

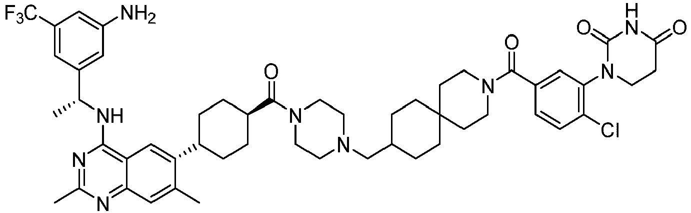

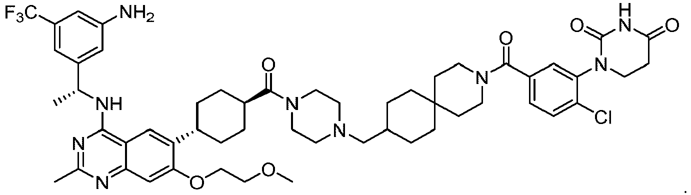

In certain embodiments, the deuteride has a structure formed by replacing 1, 2, 3, 4, 5, 6, 7, 8, 9 or 10 H with deuterium in a compound structure (for example, L, e.g., ring A, ring B, L₃ and ring C in LA, LA-1, LA-2, LA-3, LA-4 and LA-5). In certain embodiments, the deuterated LA structure is 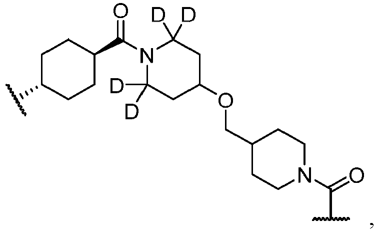 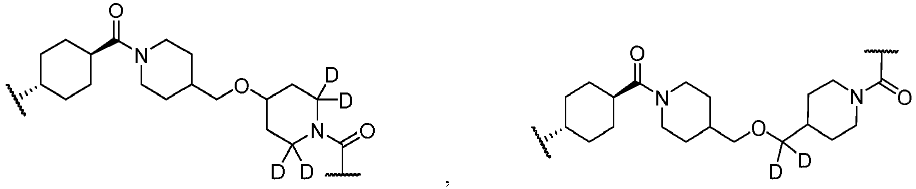 or

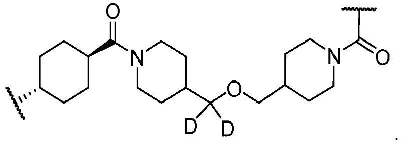

In certain embodiments, the deuteride has any one of the following structures:

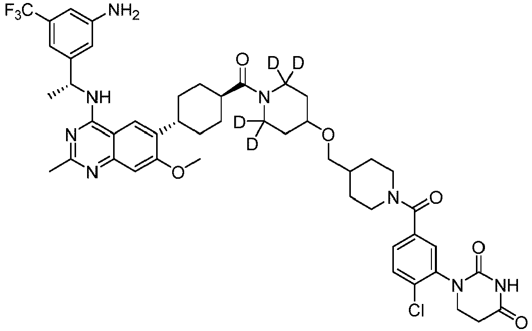

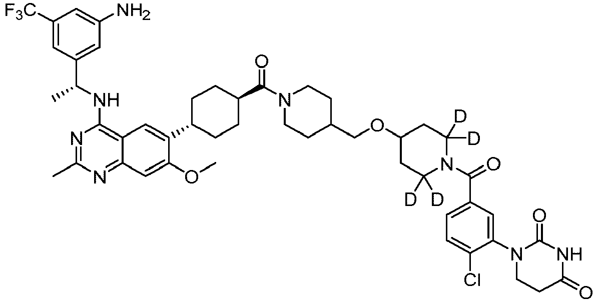

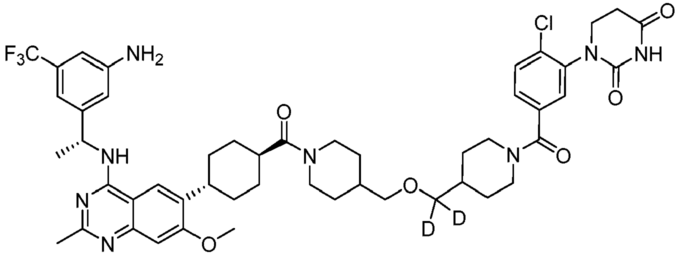

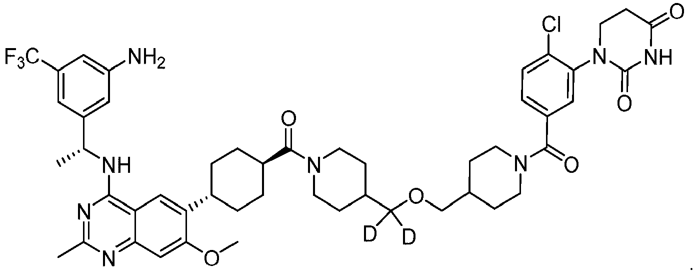

The present invention provides a method for preparing the compound of formula I, and/or the stereoisomer, the enantiomer, the diastereomer, the deuteride, the hydrate, the solvate, the metabolite, the prodrug and/or the pharmaceutically acceptable salt thereof.

The present invention provides a pharmaceutical composition comprising a therapeutically effective amount of the compound of formula I, and/or the stereoisomer, the enantiomer, the diastereomer, the deuteride, the hydrate, the solvate, the metabolite, the prodrug and/or the pharmaceutically acceptable salt thereof, and a pharmaceutically acceptable carrier, diluent, or excipient.

The present invention provides a method for degrading SOS1 protein, comprising contacting the compound of formula I, and/or the stereoisomer, the enantiomer, the diastereomer, the deuteride, the hydrate, the solvate, the metabolite, the prodrug and/or the pharmaceutically acceptable salt thereof or the pharmaceutical composition thereof with the SOS1 protein.

The present invention provides use of the compound of formula I, and/or the stereoisomer, the enantiomer, the diastereomer, the deuteride, the hydrate, the solvate, the metabolite, the prodrug and/or the pharmaceutically acceptable salt thereof or the pharmaceutical composition thereof as a medicament for the treatment or prevention of an SOS1-mediated disease or disorder.

The present invention provides use of the compound of formula I, and/or the stereoisomer, the enantiomer, the diastereomer, the deuteride, the hydrate, the solvate, the metabolite, the prodrug and/or the pharmaceutically acceptable salt thereof or the pharmaceutical composition thereof as a medicament for the treatment or prevention of a disease or disorder (cancer) caused by the interaction of SOS1 with Ras (e.g., KRAS) or SOS1 with Rac (e.g., KRAS).

The present invention provides use of the compound of formula I, and/or the stereoisomer, the enantiomer, the diastereomer, the deuteride, the hydrate, the solvate, the metabolite, the prodrug and/or the pharmaceutically acceptable salt thereof or the pharmaceutical composition thereof in preparing a medicament for the treatment or prevention of an SOS1-mediated disease or disorder (e.g., cancer).

The present invention provides use of the compound of formula I, and/or the stereoisomer, the enantiomer, the diastereomer, the deuteride, the hydrate, the solvate, the metabolite, the prodrug and/or the pharmaceutically acceptable salt thereof or the pharmaceutical composition thereof in preparing a medicament for the treatment or prevention of a disease or disorder (e.g., cancer) caused by the interaction of SOS1 with Ras (e.g., KRAS) or SOS1 with Rac (e.g., KRAS).

The present invention provides use of the compound of formula I, and/or the stereoisomer, the enantiomer, the diastereomer, the deuteride, the hydrate, the solvate, the metabolite, the prodrug and/or the pharmaceutically acceptable salt thereof or the pharmaceutical composition thereof in preparing a medicament for the treatment or prevention of cancer.

The present invention provides use of the compound of formula I, and/or the stereoisomer, the enantiomer, the diastereomer, the deuteride, the hydrate, the solvate, the metabolite, the prodrug and/or the pharmaceutically acceptable salt thereof or the pharmaceutical composition thereof in preparing a medicament for the treatment or prevention of pancreatic cancer, lung cancer, colorectal cancer, cholangiocarcinoma, multiple myeloma, melanoma, uterine cancer, endometrial cancer, thyroid cancer, acute myeloid leukemia, bladder cancer, urothelial cancer, gastric cancer, cervical cancer, head and neck squamous cell carcinoma, diffuse large B-cell lymphoma, esophageal cancer, chronic lymphocytic leukemia, hepatocellular carcinoma, breast cancer, ovarian cancer, prostate cancer, glioblastoma, renal cancer and sarcoma.

The present invention provides use of the compound of formula I, and/or the stereoisomer, the enantiomer, the diastereomer, the deuteride, the hydrate, the solvate, the metabolite, the prodrug and/or the pharmaceutically acceptable salt thereof or the pharmaceutical composition thereof in preparing a medicament for the treatment or prevention of neurofibromatosis type 1 (NF1), Noonan syndrome (NS), Noonan syndrome with multiple lentigines (NSML), capillary malformation-arteriovenous malformation syndrome (CM-AVM), Costello syndrome (CS), cardio-facio-cutaneous syndrome (CFC), Legius syndrome and hereditary gingival fibromatosis.

The present invention provides a method for treating or preventing an SOS1-mediated disease or disorder, comprising administering to a patient in need thereof a therapeutically effective amount of the compound of formula I, and/or the stereoisomer, the enantiomer, the diastereomer, the deuteride, the hydrate, the solvate, the metabolite, the prodrug and/or the pharmaceutically acceptable salt thereof or the pharmaceutical composition thereof.

The present invention provides a method for treating or preventing a disease or disorder (e.g., cancer) regulated by the interaction of SOS1 with Ras (e.g., KRAS) or SOS1 with Rac (e.g., KRAS), comprising administering to a patient in need thereof a therapeutically effective amount of the compound of formula I, and/or the stereoisomer, the enantiomer, the diastereomer, the deuteride, the hydrate, the solvate, the metabolite, the prodrug and/or the pharmaceutically acceptable salt thereof or the pharmaceutical composition thereof.

In certain embodiments of the present invention, the cancer may be selected from
heart: sarcoma (angiosarcoma, fibrosarcoma, rhabdomyosarcoma and liposarcoma), myxoma, rhabdomyoma, fibroma, lipoma and teratoma;
lung: bronchial cancer (squamous cell carcinoma, undifferentiated small cell carcinoma, undifferentiated large cell carcinoma and adenocarcinoma), bronchioloalveolar carcinoma (bronchiolar carcinoma), bronchial adenoma, sarcoma, lymphoma, chondroma, hamartoma and mesothelioma;
gastrointestinal tract and esophagus (squamous cell carcinoma, adenocarcinoma, leiomyosarcoma and lymphoma), stomach (cancer, lymphoma and leiomyosarcoma), pancreas (ductal adenocarcinoma, insulinoma, glucagonoma, gastrinoma, carcinoid tumor and vipoma), small intestine (adenocarcinoma, lymphoma, carcinoid tumor, Kaposi's sarcoma, leiomyoma, hemangioma, lipoma, neurofibroma and fibroma) and large intestine (adenocarcinoma, tubular adenoma, villous adenoma, hamartoma and leiomyoma);
genitourinary system: kidney (adenocarcinoma, Wilms' tumor, lymphoma and leukemia), bladder and urinary tract (squamous cell carcinoma, transitional cell carcinoma and adenocarcinoma), prostate (adenocarcinoma and sarcoma) and testis (seminoma, teratoma, embryonal carcinoma, teratoma, choriocarcinoma, sarcoma, interstitial cell carcinoma, fibroma, fibroadenoma, adenomatoid tumor and lipoma);
liver: liver cancer (hepatocellular carcinoma), cholangiocarcinoma, hepatoblastoma, angiosarcoma, hepatocellular adenoma and hemangioma;
biliary tract: gallbladder cancer, ampullary cancer and cholangiocarcinoma;
bone: osteosarcoma (osteosarcoma), fibrosarcoma, malignant fibrous histiocytoma, chondrosarcoma, Ewing's sarcoma, malignant lymphoma (reticulosarcoma), multiple myeloma, malignant giant cell tumor, chordoma, chondroma (osteochondral exostosis), benign chondroma, chondroblastoma, chondromyxoid fibroma, osteoid osteoma and giant cell tumor;
nervous system: skull (osteoma, hemangioma, granuloma, xanthoma and osteitis deformans), meninges (meningioma, meningosarcoma and glioma), brain (astrocytoma, medulloblastoma, glioma, ependymoma, germinal cell tumor (pinealoma), glioblastoma multiforme, oligodendroglioblastoma, neurilemmoma, retinoblastoma and congenital tumor), spinal neurofibroma, meningioma, glioma and sarcoma);
gynaecology: uterus (endometrial cancer (serous cystadenocarcinoma, mucinous cystadenocarcinoma and unclassified cancer), granulosa theca cell tumor, Sertoli-Leydig cell tumor, dysgerminoma and malignant teratoma), vulva (squamous cell carcinoma, intraepithelial carcinoma, adenocarcinoma, fibrosarcoma and melanoma), vagina (clear cell carcinoma, squamous cell carcinoma, botryoid sarcoma (embryonal rhabdomyosarcoma) and fallopian tube (cancer);
hematology: blood (myeloid leukemia (acute and chronic), acute lymphoblastic leukemia, chronic lymphocytic leukemia, myeloproliferative disease, multiple myeloma and myelodysplastic syndrome), Hodgkin's disease and non-Hodgkin's lymphoma (malignant lymphoma);
skin: malignant melanoma, basal cell carcinoma, squamous cell carcinoma, Kaposi's sarcoma, dysplastic nevus, lipoma, hemangioma, dermatofibroma, keloid and psoriasis; and
adrenal gland: neuroblastoma.

In certain embodiments of the present invention, the cancer may be selected from pancreatic cancer, lung cancer, colorectal cancer, cholangiocarcinoma, multiple myeloma, melanoma, uterine cancer, endometrial cancer, thyroid cancer, acute myeloid leukemia, bladder cancer, urothelial cancer, gastric cancer, cervical cancer, head and neck squamous cell carcinoma, diffuse large B-cell lymphoma, esophageal cancer, chronic lymphocytic leukemia, hepatocellular carcinoma, breast cancer, ovarian cancer, prostate cancer, glioblastoma, renal cancer and sarcoma. The present invention also provides use of the compound of formula I described above, and/or the stereoisomer, the enantiomer, the diastereomer, the deuteride, the hydrate, the solvate, the prodrug and/or the pharmaceutically acceptable salt thereof or the pharmaceutical composition described above in preparing a medicament for the treatment and/or prevention of a KRAS-mediated disease or disorder.

The present invention also provides a method for treating and/or preventing a KRAS-mediated disease or disorder, comprising administering to a patient in need thereof a therapeutically effective amount of the compound of formula I, and/or the stereoisomer, the enantiomer, the diastereomer, the deuteride, the hydrate, the solvate, the metabolite, the prodrug and/or the pharmaceutically acceptable salt thereof or the pharmaceutical composition thereof.

In certain embodiments of the present invention, the KRAS may be a KRAS mutant. The KRAS mutant is preferably one or more of KRAS G12C, KRAS G12D, KRAS G13D and KRAS G12V.

The present invention provides a method for preparing the compound of formula I, and/or the stereoisomer, the enantiomer, the diastereomer, the deuteride, the hydrate, the solvate, the metabolite, the prodrug and/or the pharmaceutically acceptable salt thereof:

when S is S6c 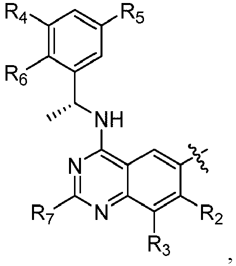 L is LA 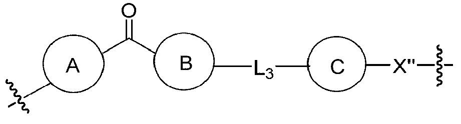 and ring B contains an NH group, the preparation method is 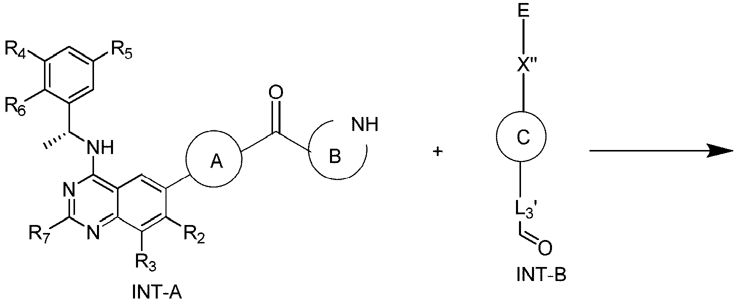 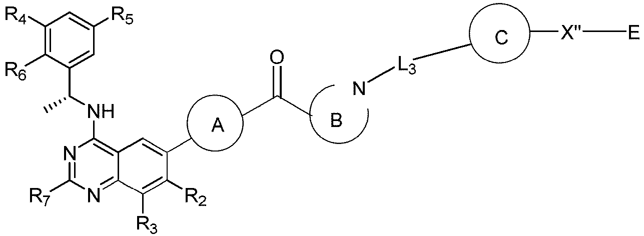 subjecting INT-A and INT-B to a reductive amination reaction to give the target compound, wherein the reductive amination reductant includes, but is not limited to, Pd/C, sodium borohydride, sodium cyanoborohydride, borane and sodium triacetoxyborohydride, wherein the 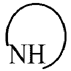 refers to ring B containing an NH group; R₂, R₃, R₄, R₅, R₆ and R₇ are as defined and described in S6b; 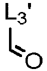 refers to L₃ containing an aldehyde group; ring A, ring B, ring C and X" are as defined in LA; E is as defined and described in the present invention, and E is preferably E7, E7a, E7b, E7c, E7d, E7e, E7a-1, E7a-2, E21, E21-1a, E21-1b, E21-1c, E21-1d, E21-1e, E21-1f, E21-1g, E21-1h, E21-1aa, E21-1bb, E21-1cc, E21-1dd, E21-1ee, E21-1ff, E21-1gg or E21-1hh.

When S is S6c 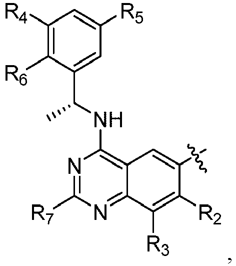 L is LA 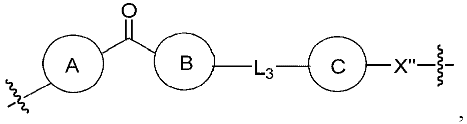 ring C contains an NH group, and X" is C(O), the preparation method is 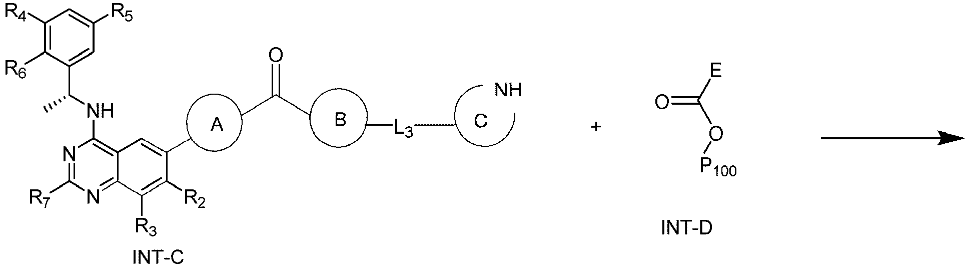 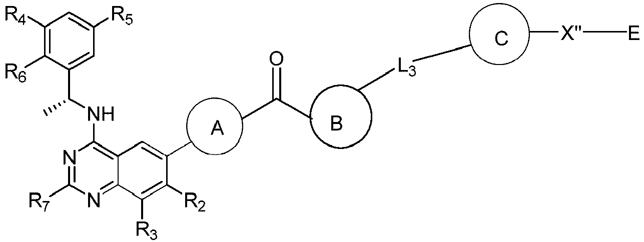 subjecting INT-C and INT-D to a substitution reaction under an alkaline condition to give the target compound, wherein the base includes, but is not limited to, triethylamine, *N*,*N*-diisopropylethylamine, potassium carbonate, sodium carbonate and sodium bicarbonate, wherein the 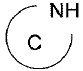 refers to ring C containing an NH group; P₁₀₀ is pentafluorophenyl or *p*-nitrophenyl; R₂, R₃, R₄, R₅, R₆ and R₇ are as defined and described in S6b; ring A, ring B, ring C and L₃ are as defined in LA; E is as defined and described in the present invention, and E is preferably E7, E7a, E7b, E7c, E7d, E7e, E7a-1, E7a-2, E21, E21-1a, E21-1b, E21-1c, E21-1d, E21-1e, E21-1f, E21-1g, E21-1h, E21-1aa, E21-1bb, E21-1cc, E21-1dd, E21-1ee, E21-1ff, E21-1gg or E21-1hh.

When S is S6d 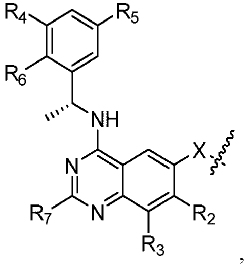 L is LA 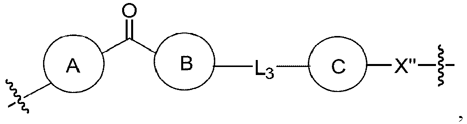 ring C contains an NH group, and X" is C(O), the preparation method is 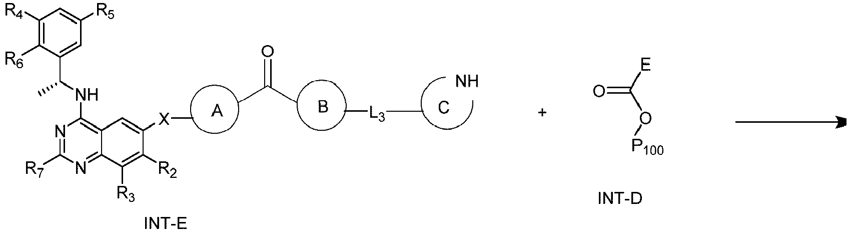 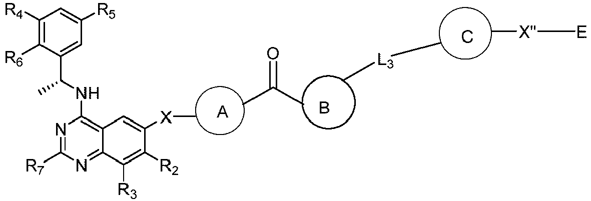 subjecting INT-E and INT-D to a substitution reaction under an alkaline condition to give the target compound, wherein the base includes, but is not limited to, triethylamine, *N*,*N*-diisopropylethylamine, potassium carbonate, sodium carbonate and sodium bicarbonate, wherein the 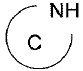 refers to ring C containing an NH group; P₁₀₀ is pentafluorophenyl or *p*-nitrophenyl; R₂, R₃, R₄, R₅, R₆ and R₇ are as defined and described in S6d; ring A, ring B, ring C and L₃ are as defined in LA; E is as defined and described in the present invention, and E is preferably E7, E7a, E7b, E7c, E7d, E7e, E7a-1, E7a-2, E21, E21-1a, E21-1b, E21-1c, E21-1d, E21-1e, E21-1f, E21-1g, E21-1h, E21-1aa, E21-1bb, E21-1cc, E21-1dd, E21-1ee, E21-1ff, E21-1gg or E21-1hh.

Detailed description: Unless otherwise stated, the following terms used in the specification and claims have the following meanings.

"Alkyl" refers to a saturated aliphatic hydrocarbon group including linear or branched alkyl; C₁-C₈ alkyl refers to alkyl containing 1-8 carbon atoms, e.g., methyl, ethyl, *n*-propyl, isopropyl, *n*-butyl, isobutyl, *tert*-butyl, sec-butyl, *n*-pentyl, 1,1-dimethylpropyl, 1,2-dimethylpropyl, 2,2-dimethylpropyl, 1-ethylpropyl, 2-methylbutyl, 3-methylbutyl, *n*-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, *n*-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, *n*-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl or various branched isomers thereof; preferably, C₁-C₆ alkyl; more preferably, C₁-C₄ alkyl. The alkyl may be substituted or unsubstituted. In some embodiments, the alkyl is C₁, C₂, C₃, C₄, C₅, C₆, C₇ or C₈ alkyl.

"Cycloalkyl" refers to a monocyclic or polycyclic hydrocarbon substituent that is saturated or partially unsaturated; "C₃-C₁₁ cycloalkyl" refers to cycloalkyl containing 3 to 11 carbon atoms; "C₃-C₈ cycloalkyl" refers to cycloalkyl containing 3 to 8 carbon atoms; "C₅-C₁₀ cycloalkyl" refers to cycloalkyl containing 5 to 10 carbon atoms.

Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl and the like, preferably cyclopropyl, cyclobutyl, cyclopentyl and cyclohexyl, preferably C₃-C₈ cycloalkyl, more preferably C₃-C₆ cycloalkyl.

Polycyclic cycloalkyl includes spiro cycloalkyl, fused cycloalkyl and bridged cycloalkyl. "Spiro cycloalkyl" refers to a polycyclic group in which a carbon atom (called spiro atom) is shared among monocyclic rings, wherein the rings may contain one or more double bonds, but none of the rings has a fully conjugated π-electron system. According to the number of the spiro atoms shared among the rings, the spiro cycloalkyl may be monospiro cycloalkyl, bispiro cycloalkyl or polyspiro cycloalkyl, preferably 7- to 12-membered bispiro cycloalkyl. Non-limiting examples of spiro cycloalkyl include:

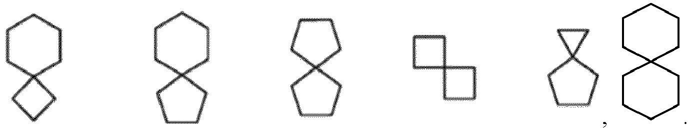

"Fused cycloalkyl" refers to an all-carbon polycyclic group in which each ring shares a pair of adjacent carbon atoms with the other rings in the system, wherein one or more of the rings may contain one or more double bonds, but none of the rings has a fully conjugated π-electron system. According to the number of the formed rings, the fused cycloalkyl may be bicyclic, tricyclic, tetracyclic or polycyclic, preferably bicyclic fused cycloalkyl. Non-limiting examples of fused cycloalkyl include:

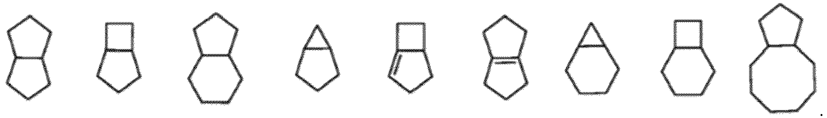

"Bridged cycloalkyl" refers to an all-carbon polycyclic group in which any two rings share two carbon atoms that are not directly connected to each other, wherein the rings may contain one or more double bonds, but none of the rings has a fully conjugated π-electron system. According to the number of the formed rings, the bridged cycloalkyl may be bicyclic, tricyclic, tetracyclic or polycyclic. Non-limiting examples of bridged cycloalkyl include:

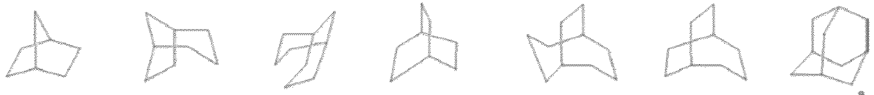

The cycloalkyl ring may be fused to an aryl, heteroaryl or heterocycloalkyl ring, wherein the ring connected to the parent structure is a cycloalkyl ring; non-limiting examples include indanyl, tetrahydronaphthyl, benzocycloheptyl, and the like. The cycloalkyl may be optionally substituted or unsubstituted.

In some embodiments, the cycloalkyl is C₃, C₄, C₅, C₆, C₇, C₈, C₉, C₁₀, C₁₁ or C₁₂ monocyclic or polycyclic (e.g., spiro, fused or bridged) cycloalkyl.

"Heterocycloalkyl" refers to a monocyclic or polycyclic hydrocarbon substituent that is saturated or partially unsaturated, wherein one or more (e.g., 2, 3, 4 or 5) of the ring atoms are selected from nitrogen, oxygen and S(O)ᵣ (wherein r is an integer of 0, 1 or 2), excluding ring portions of -O-O-, -O-S- or -S-S-, and the remaining ring atoms are carbon atoms. "3- to 11-membered heterocycloalkyl" refers to a cyclic group containing 3 to 11 ring atoms, "5-to 10-membered heterocycloalkyl" refers to a cyclic group containing 5 to 10 ring atoms, and "3- to 8-membered heterocycloalkyl" refers to a cyclic group containing 3 to 8 ring atoms, preferably "3- to 11-membered heterocycloalkyl" containing 1-2 heteroatoms selected from N, O and S, and more preferably "3- to 11-membered heterocycloalkyl" containing 1 or 2 N atoms.

Monocyclic heterocycloalkyl is preferably 3- to 8-membered monocyclic heterocycloalkyl containing 1-2 N heteroatoms; non-limiting examples of monocyclic heterocycloalkyl include pyrrolidinyl, piperidinyl, piperazinyl, morpholinyl, thiomorpholinyl, homopiperazinyl and the like, preferably piperidinyl and piperazinyl.

Polycyclic heterocycloalkyl includes spiro heterocyclyl, fused heterocyclyl and bridged heterocycloalkyl. "Spiro heterocycloalkyl" refers to a polycyclic heterocycloalkyl group in which an atom (called spiro atom) is shared among monocyclic rings, wherein one or more of the ring atoms are selected from nitrogen, oxygen and S(O)ᵣ (wherein r is an integer of 0, 1 or 2), and the remaining ring atoms are carbon atoms. The rings may contain one or more double bonds, but none of the rings has a fully conjugated π-electron system. According to the number of the spiro atoms shared among the rings, the spiro heterocycloalkyl may be monospiro heterocycloalkyl, bispiro heterocycloalkyl or polyspiro heterocycloalkyl, preferably saturated "3- to 11-membered bispiro heterocycloalkyl" containing 1-2 heteroatoms selected from N, O and S, and more preferably saturated "7- to 11-membered bispiro heterocycloalkyl" containing 1 or 2 N atoms. Non-limiting examples of spiro heterocycloalkyl include:

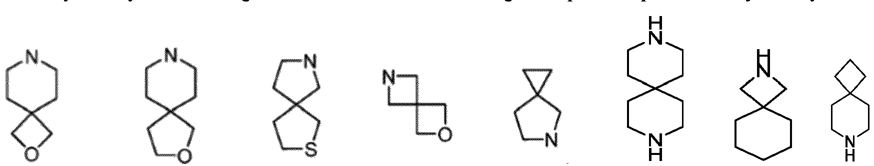

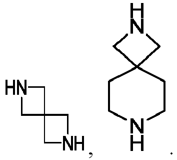

"Fused heterocycloalkyl" refers to a polycyclic heterocycloalkyl group in which each ring shares a pair of adjacent atoms with the other rings in the system, wherein one or more of the rings may contain one or more double bonds, but none of the rings has a fully conjugated π-electron system, wherein one or more of the ring atoms are selected from nitrogen, oxygen and S(O)ᵣ (wherein r is an integer of 0, 1 or 2), and the remaining ring atoms are carbon atoms. According to the number of the formed rings, the fused heterocycloalkyl may be bicyclic, tricyclic, tetracyclic or polycyclic, preferably "3- to 11-membered bicyclic fused heterocycloalkyl" containing 1-3 heteroatoms selected from N, O and S, and more preferably saturated "3- to 11-membered bicyclic fused heterocycloalkyl" containing 1 or 2 N atoms. Non-limiting examples of fused heterocycloalkyl include:

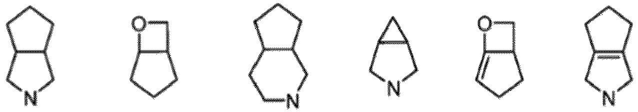

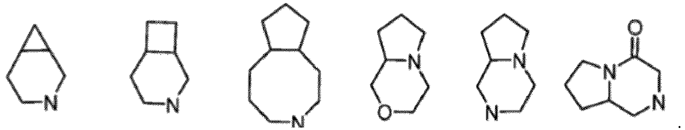

"Bridged heterocycloalkyl" refers to a polycyclic heterocycloalkyl group in which any two rings share two atoms that are not directly connected to each other, wherein the rings may contain one or more double bonds, but none of the rings has a fully conjugated π-electron system, wherein one or more of the ring atoms are selected from nitrogen, oxygen and S(O)ᵣ (wherein r is an integer of 0, 1 or 2), and the remaining ring atoms are carbon atoms. According to the number of the formed rings, the bridged heterocycloalkyl may be bicyclic, tricyclic, tetracyclic or polycyclic; non-limiting examples of bridged heterocycloalkyl include:

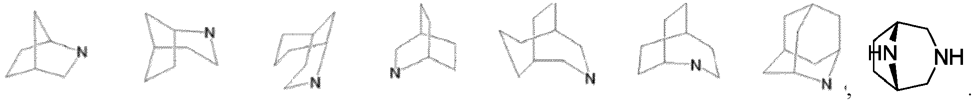

The heterocycloalkyl ring may be fused to an aryl, heteroaryl or cycloalkyl ring, wherein the ring connected to the parent structure is heterocycloalkyl ring; non-limiting examples include: 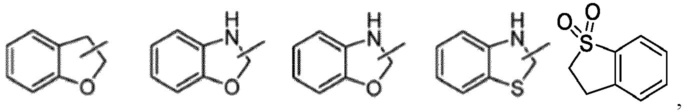 and the heterocycloalkyl may be optionally substituted or unsubstituted.

In some embodiments, the heterocycloalkyl is 3-, 4-, 5-, 6-, 7-, 8-, 9-, 10-, 11- or 12-membered monocyclic or polycyclic (e.g., spiro, fused or bridged) heterocycloalkyl, wherein the number of the heteroatoms may be 1, 2, 3, 4 or 5, each heteroatom being independently nitrogen, oxygen or S(O)ᵣ (wherein r is an integer of 0, 1 or 2).

"Aryl" refers to an all-carbon monocyclic or fused polycyclic group (i.e., rings that share a pair of adjacent carbon atoms) and a polycyclic group having a conjugated π-electron system, and "6- to 10-membered aryl" refers to all-carbon aryl containing 6-10 carbons, such as phenyl and naphthyl, preferably phenyl. The aryl ring may be fused to a heteroaryl, heterocycloalkyl or cycloalkyl ring, wherein the ring connected to the parent structure is the aryl ring; non-limiting examples include: 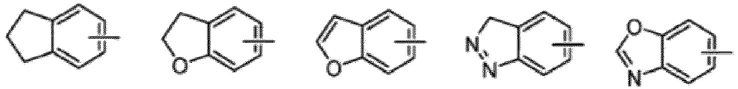 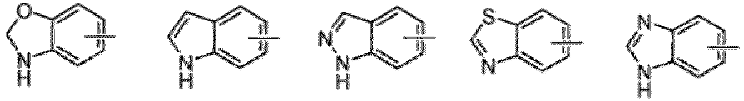 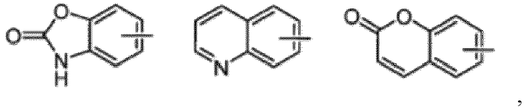 and the aryl may be optionally substituted or unsubstituted. In some embodiments, the aryl is 6- to 10-membered aryl.

"Heteroaryl" refers to a heteroaromatic system containing 1 to 4 heteroatoms, and the heteroatoms include nitrogen, oxygen or S(O)ᵣ (where r is an integer of 0, 1 or 2); 5- to 6-membered heteroaryl refers to a heteroaromatic system containing 5-6 ring atoms, and 5- to 10-membered heteroaryl refers to a heteroaromatic system containing 5-10 ring atoms, preferably 5- to 6-membered heteroaryl, and more preferably 5- to 6-membered heteroaryl containing 1 or 2 N atoms; non-limiting examples include furyl, thienyl, pyridyl, pyrrolyl, *N*-alkylpyrrolyl, pyrimidinyl, pyrazinyl, pyrazole, imidazolyl, triazolyl, tetrazolyl and the like, preferably pyridyl. The heteroaryl ring may be fused to an aryl, heterocycloalkyl or cycloalkyl ring, wherein the ring connected to the parent structure is the heteroaryl ring; non-limiting examples include: 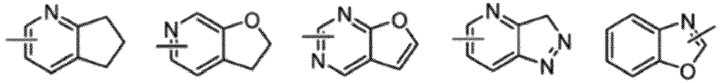 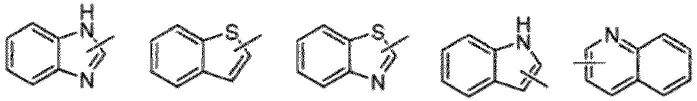 , and the heteroaryl may be optionally substituted or unsubstituted. In some embodiments, the heteroaryl is 5-, 6-, 7-, 8-, 9- or 10-membered heteroaryl, wherein the number of the heteroatoms may be 1, 2, 3, 4 or 5, each heteroatom being independently nitrogen, oxygen or S.

"Alkenyl" refers to alkyl as defined above consisting of at least two carbon atoms and at least one carbon-carbon double bond, and "C₂-₈ alkenyl" refers to linear or branched alkenyl containing 2-8 carbons, including but not limited to ethenyl, 1-propenyl, 2-propenyl, 1-, 2- or 3-butenyl and the like, preferably "C₂-₆ alkenyl", and more preferably "C₂-₄ alkenyl". The alkenyl may be substituted or unsubstituted. In some embodiments, the alkenyl is C₂, C₃, C₄, C₅, C₆, C₇ or C₈ alkenyl.

"Alkynyl" refers to alkyl as defined above consisting of at least two carbon atoms and at least one carbon-carbon triple bond, and "C₂-₈ alkynyl" refers to linear or branched alkynyl containing 2-8 carbons, including but not limited to ethynyl, 1-propynyl, 2-propynyl, 1-, 2- or 3-butynyl, preferably "C₂-₆ alkynyl", and more preferably "C₂-₄ alkynyl". The alkynyl may be substituted or unsubstituted. In some embodiments, the alkynyl is C₂, C₃, C₄, C₅, C₆, C₇ or C₈ alkynyl.

"-ylene" refers to a divalent group. For example, alkylene refers to divalent alkyl, alkenylene refers to divalent alkenyl, alkynylene refers to divalent alkynyl, cycloalkylene refers to divalent cycloalkyl, heterocycloalkylene refers to divalent heterocycloalkyl, arylene refers to divalent aryl, and heteroarylene refers to divalent heteroaryl, wherein the alkyl, the alkenyl, the alkynyl, the cycloalkyl, the heterocycloalkyl, the aryl and the heteroaryl are as defined above, and the "-ylene" may be optionally substituted or unsubstituted.

"Haloalkyl" refers to alkyl optionally substituted with one or more fluorine, chlorine, bromine or iodine atoms, wherein the alkyl is as defined above, and non-limiting examples include difluoromethyl, dichloromethyl, dibromomethyl, trifluoromethyl, trichloromethyl, tribromomethyl and the like.

"Hydroxyalkyl" refers to alkyl optionally substituted with one or more -OH, wherein the alkyl is as defined above, and non-limiting examples include hydroxymethyl, hydroxyethyl, hydroxypropyl and hydroxyisopropyl. "Alkoxy" refers to -O-alkyl, wherein the alkyl is as defined above, and non-limiting examples include methoxy, ethoxy, isopropoxy, *tert*-butoxy and the like.

"Cycloalkoxy" refers to -O-cycloalkyl, wherein the cycloalkyl is as defined above, and non-limiting examples include cyclopropaneoxy, cyclobutaneoxy, cyclopentanyloxy, cyclohexyloxy and the like.

"Heterocycloalkoxy" refers to -O-heterocycloalkyl, wherein the heterocycloalkyl is as defined above, and non-limiting examples include azetidinyloxy, azacyclopentyloxy, piperidinyloxy, piperazinyloxy, oxolanyloxy, oxanyloxy and the like.

"-C(O)C₁-C₃ alkyl" refers to -C(O)-CH₃, -C(O)-CH₂CH₃ and the like.

"Cyano" refers to -CN.

"Hydroxy" refers to -OH.

"Sulfonyl" refers to -S(O)₂-.

"Carboxyl" or "carboxylic acid" refers to -COOH.

"Oxo" refers to a =O group.

"Halogen" refers to fluorine, chlorine, bromine or iodine.

"Pd(PPh₃)₂Cl₂" refers to (bis(triphenylphosphine)palladium(II) chloride).

"TEA" refers to triethylamine.

"EA or EtOAc" refers to ethyl acetate.

"THF" refers to tetrahydrofuran.

"NaBH₄" refers to sodium borohydride.

"DCM" refers to dichloromethane.

"Tf₂O" refers to trifluoromethanesulfonic anhydride.

"(Bpin)₂" refers to bis(pinacolato)diboron.

"Pd(dppf)₂Cl₂" refers to [1,1'-bis(diphenylphosphino)ferrocene]palladium dichloride.

"KOAc" refers to potassium acetate.

"DMF" refers to *N*,*N*-dimethylformamide.

"NBS" refers to *N*-bromosuccinimide.

"EtOH" refers to ethanol.

"MeOH" refers to methanol.

"Pd/C" refers to palladium/carbon.

"Pd(OH)₂/C" refers to palladium hydroxide/carbon.

"DMSO" refers to dimethyl sulfoxide.

"HATU" refers to 2-(7-azabenzotriazol-1-yl)-*N*,*N*,*N*',*N*'-tetramethyluronium hexafluorophosphate.

"TFA" refers to trifluoroacetic acid.

"NMP" refers to *N*-methylpyrrolidone.

"IBX" refers to 2-iodoxybenzoic acid.

"DIEA" refers to *N*,*N*-diisopropylethylamine.

"STAB" refers to sodium triacetoxyborohydride.

"T₃P" refers to 2,4,6-tripropyl-1,3,5,2,4,6-trioxatriphosphorinane-2,4,6-trioxide.

"NaOH" refers to sodium hydroxide.

"DMAP" refers to 4-dimethylaminopyridine.

"TPSCl" refers to 2,4,6-triisopropylbenzenesulfonyl chloride.

"DPBS" refers to Dulbecco's phosphate-buffered saline.

"Dess-Martin" refers to a Dess-Martin reagent.

"PBS" refers to phosphate-buffered saline.

"SDS-PAGE" refers to sodium dodecyl sulfate-polyacrylamide gel electrophoresis.

"PVDF" refers to polyvinylidene fluoride.

"PE" refers to petroleum ether.

"NaHCO₃" refers to sodium bicarbonate.

"Na₂SO₄" refers to sodium sulfate.

"NH₄Cl" refers to ammonium chloride.

"AcOH" refers to acetic acid.

"HCl" refers to hydrochloric acid.

"DCC" refers to *N*,*N'*-dicyclohexylcarbodiimide.

"Ti(OEt)₄" refers to tetraethyl titanate.

"L-Selectride" refers to lithium triisobutylborohydride.

"BOP" refers to benzotriazol-1-yloxytris(dimethylamino)phosphonium hexafluorophosphate.

"DBU" refers to 1,8-diazabicyclo[5.4.0]undec-7-ene.

"LiOH.H₂O" refers to lithium hydroxide monohydrate.

"EDCI" refers to 1-(3-dimethylaminopropyl)-3-ethylcarbodiimide hydrochloride.

"NIS" refers to *N*-iodosuccinimide.

"Ac₂O" refers to acetic anhydride.

"HOAc" refers to acetic acid.

"Hantzsch-ester" refers to diethyl 1,4-dihydro-2,6-dimethyl-3,5-pyridinedicarboxylate.

"DMA" refers to dimethylacetamide.

"NiBr₂(dtbpy)" refers to (4,4-di-*tert*-butylbipyridine)nickel dibromide.

"K₂CO₃" refers to potassium carbonate.

"9-BBN" refers to 9-borabicyclo[3.3.1]nonane.

"MTBE" refers to methyl *tert*-butyl ether.

"LiOH" refers to lithium hydroxide.

"DCE" refers to dichloroethane.

"NaBH₃CN" refers to sodium cyanoborohydride.

"CBr₄" refers to carbon tetrabromide.

"PPh₃" refers to triphenylphosphine.

"*n*-BuLi" refers to *n*-butyllithium.

"NaH" refers to sodium hydride.

"i-PrOH" refers to isopropanol.

"ACN" refers to acetonitrile.

"NH₄HCO₃" refers to ammonium bicarbonate.

"*m*-CPBA" refers to *m*-chloroperoxybenzoic acid.

"KI" refers to potassium iodide.

"IPA" refers to isophthalic acid.

"N₂H₄-H₂O" refers to hydrazine hydrate.

"NMI" refers to *N*-methylimidazole.

"TCFH" refers to chloro-*N*,*N*,*N*',*N*'-tetramethylformamidinium hexafluorophosphate.

"Ruphos Pd G2" refers to chloro(2-dicyclohexylphosphino-2',6'-di-isopropoxy-1,1'-biphenyl)(2-amino-1,1'-biphenyl-2-yl)palladium (II).

"K₃PO₄" refers to potassium phosphate.

"(Boc)₂O" refers to di-*tert*-butyl dicarbonate.

"Prep-HPLC" refers to preparative high performance liquid chromatography.

"sat." refers to a saturated solution.

"aq" refers to an aqueous solution.

In the chemical structure herein, "-" as a connecting bond represents a single bond, and "=" represents a double bond (in the case of an undefined configuration, it may be *trans* or *cis*).

"Optional" or "optionally" means that the event or circumstance subsequently described may, but does not necessarily, occur, and that the description includes instances where the event or circumstance occurs or does not occur. For example, "a heterocycloalkyl group optionally substituted with alkyl" means that the alkyl may be, but not necessarily, present, and that the description includes cases where the heterocycloalkyl group is or is not substituted with the alkyl.

"Substituted" means that one or more, preferably up to 5, more preferably 1-3 hydrogen atoms in the group are independently substituted with a corresponding number of substituents. It goes without saying that a substituent is only in its possible chemical position, and those skilled in the art will be able to determine (experimentally or theoretically) possible or impossible substitution without undue effort. For example, it may be unstable when amino or hydroxy having a free hydrogen is bound to a carbon atom having an unsaturated (such as olefin) bond.

As used herein, unless otherwise indicated, the term "optionally substituted" may be unsubstituted or substituted; when it is substituted, the substituent may be one or more (e.g., 2, 3, 4, 5 or 6) groups independently selected from alkyl, alkenyl, alkynyl, hydroxy, hydroxyalkyl, haloalkyl, alkoxy, amino, aminoalkyl, cyano, halogen, oxo, cycloalkyl, heterocycloalkyl, aryl and heteroaryl, and the alkyl, the alkenyl, the alkynyl, the hydroxy, the hydroxyalkyl, the haloalkyl, the alkoxy, the amino and the aminoalkyl are optionally substituted with one or more (e.g., 2, 3, 4, 5 or 6) cycloalkyl, heterocycloalkyl, aryl or heteroaryl; the cycloalkyl, the heterocycloalkyl, the aryl and the heteroaryl are optionally substituted with one or more groups selected from alkyl, alkenyl, alkynyl, hydroxy, hydroxyalkyl, haloalkyl, alkoxy, amino, aminoalkyl, cyano, halogen and oxo.

"Pharmaceutical composition" refers to a mixture containing one or more of the compounds or the physiologically/pharmaceutically acceptable salts or pro-drugs thereof described herein, and other chemical components, for example, physiologically/pharmaceutically acceptable carriers and excipients. The pharmaceutical composition is intended to promote the administration to an organism, so as to facilitate the absorption of the active ingredient, thereby exerting biological activity.

The present invention also provides a pharmaceutically acceptable salt of the compound of formula (I). The term "pharmaceutically acceptable salt" refers to a relatively non-toxic acid addition salt or base addition salt of the compound of the present invention. The acid addition salt is a salt formed from the compound of formula (I) of the present invention and a suitable inorganic acid or organic acid. These salts may be prepared during the final isolation and purification of the compound or by reacting the purified compound of formula (I) in its free base form with a suitable organic acid or inorganic acid. The representative acid addition salt includes hydrobromide, sulfate, bisulfate, sulfite, acetate, oxalate, valerate, oleate, palmitate, stearate, laurate, borate, benzoate, lactate, phosphate, biphosphate, carbonate, bicarbonate, toluate, citrate, maleate, fumarate, succinate, methanesulfonate, p-toluenesulfonate, gluconate, lactobionate, lauryl sulfonate and the like. The base addition salt is a salt formed from the compound of formula (I) and a suitable inorganic base or organic base, including, for example, a salt formed with alkali metal, alkaline earth metal and quaternary ammonium cations, such as sodium salts, lithium salts, potassium salts, calcium salts, magnesium salts, tetramethyl quaternary ammonium salts, tetraethyl quaternary ammonium salts and the like; the amine salt includes salts formed with ammonia (NH₃) and primary, secondary or tertiary amines, such as methylamine salts, dimethylamine salts, trimethylamine salts, triethylamine salts, ethylamine salts and the like.

The compound of the present invention or the pharmaceutically acceptable salt thereof may be administered to mammals including humans, and may be administered orally, rectally, parenterally (intravenously, intramuscularly or subcutaneously), topically (powders, ointments or drops) or intratumorally.

The compound of the present invention may be administered at a dose of about 0.05-300 mg/kg body weight/day, preferably 10-300 mg/kg body weight/day, and more preferably 10-200 mg/kg body weight/day.

The compound of the present invention or the pharmaceutically acceptable salt thereof may be formulated into a solid dosage form for oral administration, including but not limited to capsules, tablets, pills, pulvis, granules and the like. In these solid dosage forms, the compound of formula (I) of the present invention, as an active ingredient, is mixed with at least one conventional inert excipient (or carrier), such as sodium citrate or dicalcium phosphate, or with the following ingredients: (1) fillers or extenders, such as starch, lactose, sucrose, glucose, mannitol, silicic acid and the like; (2) binders, such as hydroxymethyl cellulose, alginate, gelatin, polyvinylpyrrolidone, sucrose, acacia and the like; (3) humectants, such as glycerol and the like; (4) disintegrants, such as agar, calcium carbonate, potato or tapioca starch, alginic acid, certain complex silicates, sodium carbonate and the like; (5) solution retarders, such as paraffin and the like; (6) absorption accelerators, such as quaternary ammonium compounds and the like; (7) wetting agents, such as cetyl alcohol, glycerol monostearate and the like; (8) adsorbents, such as kaolin and the like; and (9) lubricants, such as talc, calcium stearate, magnesium stearate, solid polyethylene glycol, sodium lauryl sulfate and the like, or mixtures thereof. Capsules, tablets and pills may further comprise buffers.

The solid dosage forms such as tablets, dragees, capsules, pills and granules may be coated or microencapsulated using coatings and shells such as enteric coatings and other materials well known in the art. They may comprise opacifying agents, and the active ingredient in such a composition may be released in a certain part of the digestive tract in a delayed manner. Examples of embedding components that can be used are polymeric substances and waxbased substances. If necessary, the active ingredient can also be in microcapsule form with one or more of the above-mentioned excipients.

The compound of the present invention or the pharmaceutically acceptable salt thereof may be formulated into a liquid dosage form for oral administration, including but not limited to pharmaceutically acceptable emulsions, solutions, suspensions, syrups, tinctures and the like. In addition to the compound of formula (I) or the pharmaceutically acceptable salt thereof as an active ingredient, the liquid dosage form may comprise inert diluents commonly used in the art, such as water and other solvents, solubilizers and emulsifiers, for example, ethanol, isopropanol, ethyl carbonate, ethyl acetate, propylene glycol, 1,3-butanediol, dimethylformamide, and oils, especially cottonseed oil, peanut oil, corn germ oil, olive oil, castor oil, sesame oil and the like, or mixtures of these substances. In addition to such inert diluents, the liquid dosage form in the present invention may further comprise conventional adjuvants, such as wetting agents, emulsifiers, suspending agents, sweeteners, flavoring agents, perfuming agents and the like.

The suspending agents include, for example, ethoxylated isostearyl alcohols, polyoxyethylene sorbitol and sorbitan esters, microcrystalline cellulose, aluminum methylate, agar and the like, or mixtures of these substances.

The compound of the present invention or the pharmaceutically acceptable salt thereof may be formulated into a dosage form for parenteral injection, including but not limited to physiologically acceptable sterile aqueous or anhydrous solutions, dispersions, suspensions or emulsions, and sterile powders for redissolving into sterile injectable solutions or dispersions. Suitable carriers, diluents, solvents or excipients include water, ethanol, polyols and suitable mixtures thereof.

The compound of the present invention or the pharmaceutically acceptable salt thereof may also be formulated into a dosage form for topical administration, including, for example, ointments, pulvis, suppositories, drops, sprays, inhalants and the like. The compound of formula (I) or the pharmaceutically acceptable salt thereof in the present invention as an active ingredient is mixed under sterile conditions with a physiologically acceptable carrier and optional preservatives, buffers or propellants that may be required if necessary.

The present invention also provides a pharmaceutical composition comprising the compound of formula (I) or the pharmaceutically acceptable salt thereof as an active ingredient, and a pharmaceutically acceptable carrier, excipient or diluent. In the preparation of the pharmaceutical composition, the compound of formula (I) or the pharmaceutically acceptable salt thereof in the present invention is typically mixed with the pharmaceutically acceptable carrier, excipient or diluent.

The composition in the present invention may be formulated into a conventional pharmaceutical formulation according to a conventional preparation method. For example, tablets, pills, capsules, pulvis, granules, emulsions, suspensions, dispersions, solutions, syrups, elixirs, ointments, drops, suppositories, inhalants, sprays and the like. The compound or the pharmaceutically acceptable salt thereof described in the present invention may be administered alone or, if desired, in combination with other pharmaceutically acceptable therapeutic agents, such as other anti-tumor medicaments. The ingredients to be combined may be administered simultaneously or sequentially, and administered in a single formulation or in different formulations. The combination may include not only a combination of the compound in the present invention and one additional active agent, but also a combination of the compound in the present invention and two or more additional active agents.

In the present invention, other pharmaceutically acceptable therapeutic agents that may be used together with or in combination with the SOS1 degrading agent, the compound of formula (I), may be: EGFR and/or a mutant inhibitor thereof, ErbB2(Her2) and/or a mutant inhibitor thereof, ALK and/or a mutant inhibitor thereof, MEK and/or a mutant inhibitor thereof, Kras and/or a mutant inhibitor thereof, BCR-ABL and/or a mutant inhibitor thereof, FGFR1/FGFR2/FGFR3 and/or a mutant inhibitor thereof, ROS1 and/or a mutant inhibitor thereof, c-MET and/or a mutant inhibitor thereof, AXL and/or a mutant inhibitor thereof, NTRK1 and/or a mutant inhibitor thereof, RET and/or a mutant inhibitor thereof, taxane, a platinum-containing compound, an antimetabolite, a mitotic kinase inhibitor, an immunotherapeutic agent, an anti-angiogenic drug, a topoisomerase inhibitor, A-Raf/B-Raf/C-Raf and/or a mutant inhibitor thereof, ERK and/or a mutant inhibitor thereof, an apoptosis inhibitor, an mTOR inhibitor, an epigenetic modulator, an IGF1/2 and/or IGF1-R inhibitor, Ras GEF and/or a mutant inhibitor thereof or PI3K and/or a mutant inhibitor thereof.

In the present invention, other pharmaceutically acceptable therapeutic agents that may be used together with or in combination with the SOS1 degrading agent, the compound of formula (I), may be: afatinib, erlotinib, gefitinib, lapatinib, cetuximab, panitumumab, osimertinib, olmutinib, EGF-816, trastuzumab, pertuzumab, crizotinib, alectinib, entrectinib, brigatinib, trametinib, cobimetinib, binimetinib, selumetinib, refametinib, imatinib, dasatinib, nilotinib, nintedanib, crizotinib, lorlatinib, ceritinib, merestinib, paclitaxel, nab-paclitaxel, docetaxel, cisplatin, carboplatin, oxaliplatin, 5-fluorouracil, capecitabine, floxuridine, cytarabine, gemcitabine, a combination of trifluridine and tipiracil (=TAS102), palbociclib, ribociclib, abemaciclib, ipilimumab, nivolumab, pembrolizumab, atezolizumab, avelumab, durvalumab, pidilizumab, PDR-001 (=spartalizumab), bevacizumab, irinotecan, liposomal irinotecan, topotecan, ulixertinib, rapamycin, temsirolimus, everolimus, ridaforolimus, JQ-1, GSK 525762, OTX 015(=MK8628), CPI 0610, TEN-010 (=RO6870810), xentuzumab (antibody 60833 in WO2010/066868) or MEDI-573 (=dusigitumab).

According to the present invention, SOS1 kinase activity tests prove that the compound of formula I described in the present invention is able to effectively bind to the SOS1 target protein or have an inhibitory effect, and it is proved that the compound of formula I described herein is able to effectively and specifically degrade the SOS1 protein in NCI-H358 cells by means of Western-Blot. The compound of formula I, and/or the stereoisomer, the enantiomer, the diastereomer, the deuteride, the hydrate, the solvate, the metabolite, the prodrug and/or the pharmaceutically acceptable salt thereof in the present invention can effectively degrade the SOS1 protein, thereby achieving the effect of preventing or treating a disease or disorder related to SOS1 or caused by the interaction of SOS1 with Ras or SOS1 with Rac.

### DETAILED DESCRIPTION

The present invention will be further explained in detail below with reference to examples, which are not intended to limit the present invention, and the present invention is not merely limited to the contents of the examples. Starting materials in the examples of the present invention are known and commercially available, or may be synthesized by using or according to methods known in the art. Unless otherwise stated, experimental procedures without specific conditions indicated in the examples of the present invention are generally conducted according to conventional conditions, or according to conditions recommended by the manufacturers of the starting materials or commercial products.

### I. Compound preparation examples

### Intermediate 1: (R)-1-(3-nitro-5-(trifluoromethyl)phenyl)ethyl-1-amine hydrochloride

### Step 1: preparation of 1-(3-nitro-5-(trifluoromethyl)phenyl)ethyl-1-one

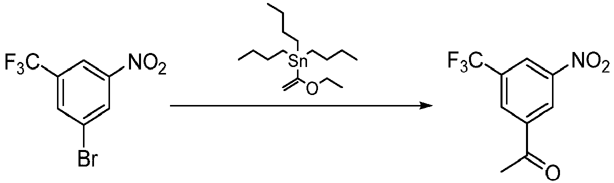

A reaction mixture of 1-bromo-3-nitro-5-(trifluoromethyl)benzene (18 g, 66.7 mmol), tributyl(1-ethoxyvinyl)stannane (31 g, 86.7 mmol), Pd(PPh₃)₂Cl₂ (4.7 g, 6.67 mmol), and TEA (13.5 g, 133 mmol) in a 1,4-dioxane (200 mL) solution was stirred at 80 °C under nitrogen atmosphere for reaction overnight. Potassium fluoride (saturated aqueous solution, 300 mL) was added to the reaction liquid to quench the reaction, and the resulting mixture was extracted with EA (200 mL × 3). The organic phase was collected and concentrated under reduced pressure. The concentrate was dissolved in THF (180 mL), hydrochloric acid (100 mL, 6 M) was added thereto, and the mixture was stirred at room temperature for 3 h. Water (300 mL) was added to the mixture to quench the reaction, and the resulting mixture was extracted with EA (200 mL × 3). The organic phase was collected, dried over anhydrous sodium sulfate, filtered, and concentrated under reduced pressure. The crude product
was purified by column chromatography to give the target product 1-(3-nitro-5-(trifluoromethyl)phenyl)ethyl-1-one.

¹H NMR (400 MHz, CDCl₃) δ 8.95 (t, *J* = 1.6 Hz, 1H), 8.69 (s, 1H), 8.54 (d, *J* = 0.5 Hz, 1H), 2.76 (s, 3H).

### Step 2: preparation of (R,E)-2-methyl-N-(1-(3-nitro-5-(trifluoromethyl)phenyl)ethylidene)propyl-2-sulfinamide

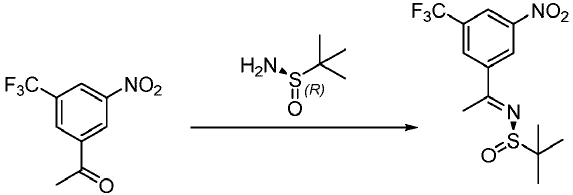

A reaction mixture of 1-(3-nitro-5-(trifluoromethyl)phenyl)ethyl-1-one (10.6 g, 45.5 mmol), (*R*)-2-methylpropyl-2-sulfinamide (8.3 g, 68.2 mmol), and tetraethyl titanate (25.9 g, 114 mmol) in a THF (200 mL) solution was stirred at 60 °C for reaction for 2 h. Water (300 mL) was added to the reaction mixture to quench the reaction, and the solution was extracted with EA (200 mL × 3). The organic phase was collected, dried over anhydrous sodium sulfate, filtered, and concentrated under reduced pressure. The concentrate was purified by column chromatography to give the target product (*R*,*E*)-2-methyl-*N*-(1-(3-nitro-5-(trifluoromethyl)phenyl)ethylidene)propyl-2-sulfinamide.

¹H NMR (400 MHz, DMSO-*d*₆) δ 8.87 (s, 1H), 8.61 (s, 1H), 8.43 (s, 1H), 2.90 (s, 3H), 1.37 (s, 9H).

### Step 3: preparation of (R)-2-methyl-N-((R)-1-(3-nitro-5-(trifluoromethyl)phenyl)ethyl)propyl-2-sulfinamide

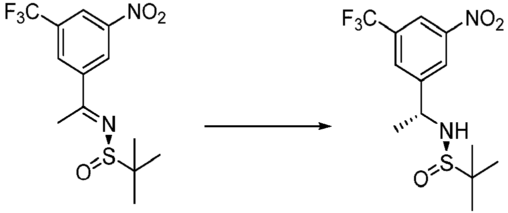

NaBH₄ (1.28 g, 33.7 mmol) was added to a mixture of (*R,E*)-2-methyl-*N*-(1-(3-nitro-S-(trifluoromethyl)phenyl)ethylidene)propyl-2-sulfinamide (10 g, 30.7 mmol) in a solution of THF (100 mL) and water (20 mL). After the reaction mixture was stirred at -10 °C for reaction for 1 min, water (300 mL) was added to the reaction mixture to quench the reaction, and the solution was extracted with EA (200 mL × 3). The organic phase was collected, dried over anhydrous sodium sulfate, filtered, and concentrated under reduced pressure. The concentrate was purified by column chromatography to give the target product (*R*)-2-methyl-*N*-((*R*)-1-(3-nitro-5-(trifluoromethyl)phenyl)ethyl)propyl-2-sulfinamide.

¹H NMR (400 MHz, CDCl₃) δ 8.45 - 8.42 (m, 2H), 7.96 (s, 1H), 4.76 - 4.68 (m, 1H), 3.56 (d, *J* = 4.2 Hz, 1H), 1.62 (d, *J* = 6.7 Hz, 3H), 1.26 (s, 9H).

### Step 4: preparation of (R)-1-(3-nitro-5-(trifluoromethyl)phenyl)ethyl-1-amine hydrochloride

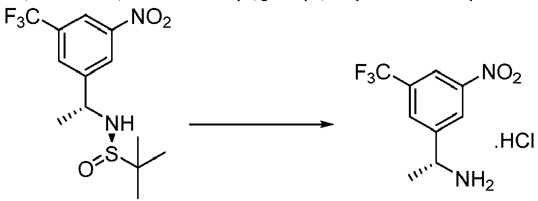

(*R*)-2-Methyl-*N*-((*R*)-1-(3-nitro-5-(trifluoromethyl)phenyl)ethyl)propyl-2-sulfinamide (5.5 g, 16.3 mmol) was placed in a solution of hydrochloric acid (80 mL, 4 M) in dioxane, and the mixture was stirred at 25 °C for reaction for 2 h. The crude product was filtered and washed with petroleum ether (100 mL) to give the target product (R)-1-(3-nitro-5-(trifluoromethyl)phenyl)ethyl-1-amine hydrochloride.

LC-MS: (ESI, *m*/*z):* [M+H]⁺= 235.2.

### Intermediate 2: ethyl 4-(4,4,5,5-tetramethyl-1,3,2-dioxaborolan-2-yl)cyclohex-3-ene-1-carboxylate

### Step 1: preparation of ethyl 4-(((trifluoromethyl)sulfonyl)oxy)cyclohex-3-ene-1-carboxylate

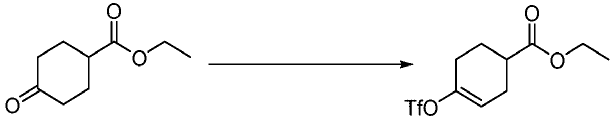

Tf₂O (9 g, 32.0 mmol) was added dropwise to a stirred mixture of ethyl 4-oxocyclohexane-1-formate (5 g, 32.0 mmol) and 2,6-dimethylpyridine (4.6 g, 42.9 mmol) in DCM (60 mL) at 0 °C. The reaction mixture was stirred at 0 °C for reaction for 1 h. The reaction mixture was then stirred at 25 °C for reaction for 15 min, and Tf₂O (7 g, 25.6 mmol) was added. Then the reaction mixture was stirred at 25 °C overnight and then concentrated. Water (300 mL) was added to quench the reaction, and the resulting mixture was extracted with EA (200 mL × 3). The organic phase was collected, dried over anhydrous sodium sulfate, filtered, and concentrated under reduced pressure. The concentrate was purified by column chromatography to give ethyl 4-(((trifluoromethyl)sulfonyl)oxy)cyclohex-3-ene-1 -carboxylate.

¹H NMR (400 MHz, DMSO-*d*₆) δ 5.90 (d, *J* = 3.9 Hz, 1H), 4.14 - 4.02 (m, 2H), 2.68 - 2.60 (m, 1H), 2.47 - 2.27 (m, 4H), 2.08 - 1.98 (m, 1H), 1.88 - 1.76 (m, 1H), 1.19 (t, *J* = 7.1 Hz, 3H).

### Step 2: preparation of ethyl 4-(4,4,5,5-tetramethyl-1,3,2-dioxaborolan-2-yl)cyclohex-3-ene-1-carboxylate

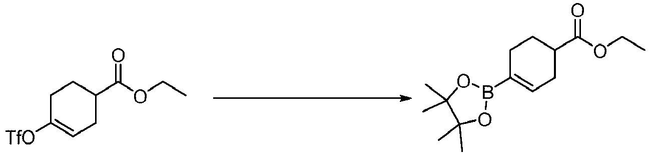

A mixture of ethyl 4-(((trifluoromethyl)sulfonyl)oxy)cyclohex-3-ene-1-carboxylate (4.7 g, 16.8 mmol), (Bpin)₂ (6.4 g, 25.2 mmol), Pd(dppf)₂Cl₂ (1.2 g, 1.68 mmol), and KOAc (4.9 g, 50.4 mmol) in a 1,4-dioxane (80 mL) solution was stirred at 90 °C for reaction overnight. Water (300 mL) was added to quench the reaction, and the resulting mixture was extracted with EA (200 mL × 3). The organic phase was collected, dried over anhydrous sodium sulfate, filtered, and concentrated under reduced pressure. The concentrate was purified by column chromatography to give ethyl 4-(4,4,5,5-tetramethyl-1,3,2-dioxaborolan-2-yl)cyclohex-3-ene-1-carboxylate.

¹H NMR (400 MHz, DMSO-*d*₆) δ 6.42 (d, *J* = 2.2 Hz, 1H), 4.11 - 4.02 (m, 2H), 2.33 - 1.95 (m, 4H), 1.93 - 1.83 (m, 1H), 1.53 - 1.38 (m, 1H), 1.21 - 1.14 (m, 16H).

### Intermediate 3: ((1R,4R)-4-(4-(((R)-1-(3-amino)-5-(trifluoromethyl)phenyl)ethyl)amino)-7-methoxy-2-methylquinazolin-6-yl)cyclohexyl)(piperazin-1 -yl)methanone

### Step 1: preparation of 2-amino-5-bromo-4-methoxybenzoic acid

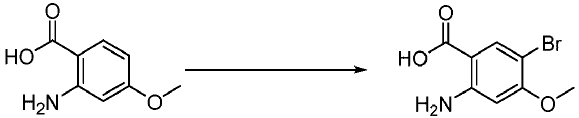

NBS (35 g, 197 mmol) was added to a stirred solution of 2-amino-4-methoxybenzoic acid (30 g, 179 mmol) in DMF (600 mL) at 0 °C, and the reaction mixture was stirred at 25 °C for reaction for 1 h. Sodium thiosulfate (saturated aqueous solution, 500 mL) was added to quench the reaction liquid, and the resulting mixture was extracted with EA (500 mL × 3). The organic phase was collected, washed with sodium chloride (saturated aqueous solution, 500 mL × 3), dried over anhydrous sodium sulfate, filtered and concentrated to give a crude product of 2-amino-5-bromo-4-methoxybenzoic acid, which was directly used in the next step without further purification.

LC-MS: (ESI, *m*/*z):* [M+H]⁺= 246.0.

### Step 2: preparation of 6-bromo-7-methoxy-2-methylquinazolin-4(3H)-one

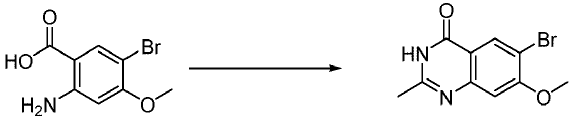

Ammonium acetate (53.2 g, 691 mmol) and trimethyl orthoacetate (83.0 g, 691 mmol) were added to a stired solution of 2-amino-5-bromo-4-methoxybenzoic acid (17.0 g, 69.1 mmol) in methanol (170 mL). The resulting reaction mixture was stirred in a closed container at 120 °C for reaction for 24 h. The reaction mixture was poured into water (1 L), and the solid product was filtered. The crude product was washed with EA (1 L) to give the target product 6-bromo-7-methoxy-2-methylquinazolin-4(3*H*)-one. The crude product was directly used in the next step without purification.

LC-MS: (ESI, *m*/*z*): [M+H]⁺= 268.9.

### Step 3: preparation of 6-bromo-4-chloro-7-methoxy-2-methylquinazoline

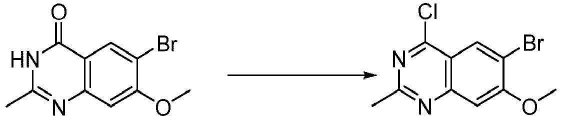

A mixture of 6-bromo-7-methoxy-2-methylquinazolin-4(3*H*)-one (10 g, 37.2 mmol) in a phosphorus oxychloride (200 mL) solution was stirred at 105 °C overnight. The reaction mixture was concentrated. Sodium bicarbonate (saturated aqueous solution, 500 mL) was added to quench the reaction, and the resulting mixture was extracted with EA (300 mL × 3). The organic phase was collected, dried over anhydrous sodium sulfate, filtered, and concentrated. The concentrate was purified by silica gel column chromatography to give the target product 6-bromo-4-chloro-7-methoxy-2-methylquinazoline.

LC-MS: (ESI, *m*/*z*): [M+H]⁺= 287.0.

### Step 4: preparation of (R)-6-bromo-7-methoxy-2-methyl-N-(1-(3-nitro-5-(trifluoromethyl)phenyl)ethyl)quinazolin-4-amine

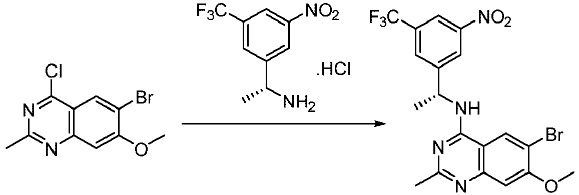

(*R*)-1-(3-Nitro-5-(trifluoromethyl))phenyl)ethyl-1-amine hydrochloride (1.7 g, 7.52 mmol) and DIEA (1.6 g, 12.5 mmol) were added to a mixture of 6-bromo-4-chloro-7-methoxy-2-methylquinazoline (1.8 g, 6.27 mmol) in an EtOH (12 mL) solution. The reaction mixture was stirred in microwave at 100 °C for reaction for 5 h. Water (300 mL) was added to quench the reaction, and the resulting mixture was extracted with EA (300 mL × 3). The organic phase was collected, dried over anhydrous sodium sulfate, filtered, and concentrated under reduced pressure. The concentrate was purified by column chromatography to give (*R*)-6-bromo-7-methoxy-2-methyl-*N*-(1-(3-nitro-5-(trifluoromethyl)phenyl)ethyl)quinazolin-4-amine as a yellow oil.

LC-MS: (ESI, *m*/*z*): [M+H]⁺= 484.9.

### Step 5: preparation of ethyl 4-(7-methoxy-2-methyl-4-(((R)-1-(3-nitro-5-(trifluoromethyl)phenyl)ethyl)amino)quinazolin-6-yl)cyclohex-3-ene-1-carboxylate

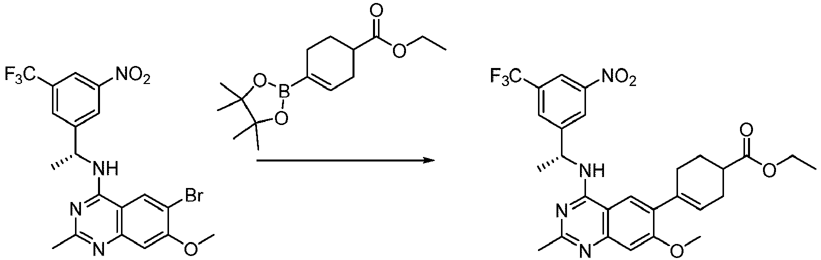

A reaction mixture of (*R*)-6-bromo-7 -methoxy-2-methyl-*N*-(1-(3-nitro-5-(trifluoromethyl)phenyl)ethyl)quinazolin-4-amine (2 g, 4.12 mmol), ethyl 4-(4,4,5,5-tetramethyl-1,3,2-dioxaborane-2-yl)cyclohex-3-ene-1-carboxylate (2.31 g, 8.24 mmol), sodium carbonate (873 mg, 8.24 mmol), and Pd(dppf)₂Cl₂ (300 mg, 0.41 mmol) in DMF (24 mL) and water (3 mL) was stirred at 110 °C for reaction overnight. Water (300 mL) was added to quench the reaction. The solution was extracted with EA (300 mL × 3) and washed with sodium chloride (saturated aqueous solution, 500 mL × 3), and the organic phases were combined, dried over anhydrous sodium sulfate, filtered, and concentrated under reduced pressure. The concentrate was purified by column chromatography to give ethyl 4-(7-methoxy-2-methyl-4-(((*R*)-1-(3-nitro-5-(trifluoromethyl)phenyl)ethyl)amino)quinazolin-6-yl)eyclohex-3-ene-1-carboxylate.

LC-MS: (ESI, *m*/*z):* [M+H]⁺= 559.7.

### Step 6: preparation of ethyl (R)-4-(4-((1-(3-amino-5-(trifluoromethyl)phenyl)ethyl)amino)-7-methoxy-2-methylquinazolin-6-yl)cyclohex-1 -carboxylate

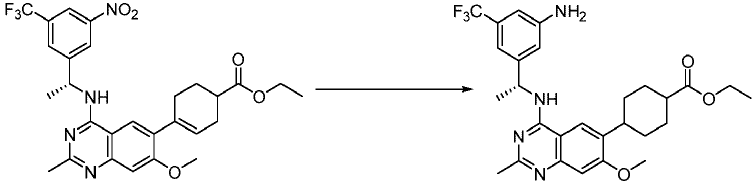

Pd/C (1.02 g, 0.6 w/w) was added to a mixture of ethyl 4-(7-methoxy-2-methyl-4-(((*R*)-1-(3-nitro-5-(trifluoromethyl)phenyl)ethyl)amino)quinazolin-6-yl)cyclohex-3-ene-1-carboxylate (1.7 g, 3.04 mmol) in an MeOH (60 mL) solution. The reaction mixture was stirred at 50 °C under hydrogen atmosphere for reaction for 4 days. The crude product was filtered to give ethyl (*R*)-4-(4-((1-(3-amino-5-(trifluoromethyl)phenyl)ethyl)amino)-7-methoxy-2-methylquinazolin-6-yl)cyclohex-1 -carboxylate.

LC-MS: (ESI, *m*/*z):* [M+H]⁺= 531.2.

### Step 7: preparation of (1R,4R)-4-(4-(((R)-1-(3-amino-5-(trifluoromethyl)phenyl)ethyl)amino)-7-methoxy-2-methylquinazolin-6-yl)cyclohexane-1 -formic acid

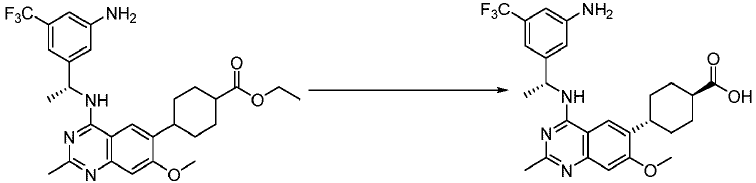

Lithium hydroxide (203 mg, 8.46 mmol) was added to a mixture of ethyl (*R*)-4-(4-((1-(3-amino-5-(trifluoromethyl)phenyl)ethyl)amino)-7-methoxy-2-methylquinazolin-6-yl)cyclohexane-1-carboxylate (1.5 g, 2.82 mmol) in methanol (8 mL), THF (8 mL) and water (16 mL). The reaction mixture was stirred at 60 °C for reaction overnight. The crude product was purified by preparative HPLC to give (1*R*,4*R*)-4-(4-(((*R*)-1-(3-amino-5-(trifluoromethyl)phenyl)ethyl)amino)-7-methoxy-2-methylquinazolin-6-yl)cyclohexane-1-formic acid.

LC-MS: (ESI, *m*/*z*): [M+H]⁺=503.2.

¹H NMR (400 MHz, DMSO-*d*₆) δ 8.13 (d, *J* = 8.0 Hz, 1H), 8.06 (s, 1H), 6.99 (s, 1H), 6.88 (s, 1H), 6.86 (s, 1H), 6.69 (s, 1H), 5.56 (dd, *J* = 13.6, 5.8 Hz, 3H), 3.88 (s, 3H), 2.92 (t, *J* = 11.7 Hz, 1H), 2.35 (s, 3H), 2.24 (t, *J* = 11.8 Hz, 1H), 2.05 (d, *J* = 10.8 Hz, 2H), 1.87 (d, *J* = 11.8 Hz, 2H), 1.66 - 1.40 (m, 7H).

### Step 8: tert-butyl 4-((1R,4R)-4-(4-(((R)-1-(3-amino-5-(trifluoromethyl)phenyl)ethyl)amino)-7-methoxy-2-methylquinazolin-6-yl)cyclohexane-1 -carbonyl)piperazine-1 -carboxylate

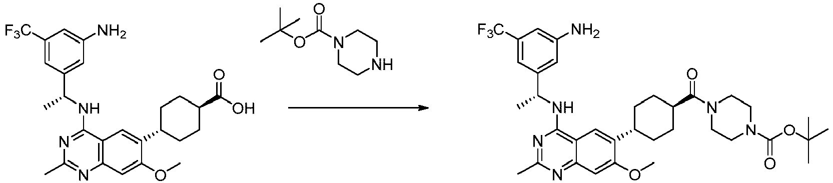

*tert*-Butyl piperazine-1-formate (163 mg, 0.88 mmol), DIEA (169 mg, 1.31 mmol), and T₃P (209 mg, 0.66 mmol) were added to a mixture of (1*R*,4*R*)-4-(4-(((*R*)-1-(3-amino-5-(trifluoromethyl)phenyl)ethyl)amino)-7-methoxy-2-methylquinazolin-6-yl)cyclohexane-1-formic acid (220 mg, 0.44 mmol) in an EA (12 mL) solution. The reaction mixture was stirred at 0 °C for 3 h. Water (100 mL) was added to quench the reaction, and the resulting mixture was extracted with EA (100 mL × 3). The organic phase was collected, dried over anhydrous sodium sulfate, filtered, and concentrated under reduced pressure. The concentrate was purified by column chromatography to give *tert-*butyl 4-((1*R*,4*R*)-4-(4-(((*R*)-1-(3-amino-5-(trifluoromethyl)phenyl)ethyl)amino)-7-methoxy-2-methylquinazolin-6-yl)cyclohexane-1 -carbonyl)piperazine-1 -carboxylate.

LC-MS: (ESI, *m*/*z*): [M+H]⁺=671.3.

¹H NMR (400 MHz, DMSO-*d*₆) δ 8.19 - 8.07 (m, 2H), 6.99 (s, 1H), 6.86 (d, *J* = 10.5 Hz, 2H), 6.70 (s, 1H), 5.56 (dd, *J* = 16.7, 9.3 Hz, 3H), 3.89 (s, 3H), 3.47 (m, 4H), 3.32 (m, 4H), 2.95 (s, 1H), 2.67 (s, 1H), 2.36 (s, 3H), 1.83 (d, *J=* 14.5 Hz, 4H), 1.59 (t, *J* = 22.3 Hz, 7H), 1.41 (s, 9H).

### Step 9: preparation of ((1R,4R)-4-(4-(((R)-1-(3-amino)-5-(trifluoromethyl)phenyl)ethyl)amino)-7-methoxy-2-methylquinazolin-6-yl)cyclohexyl)(piperazin-1-yl)methanone

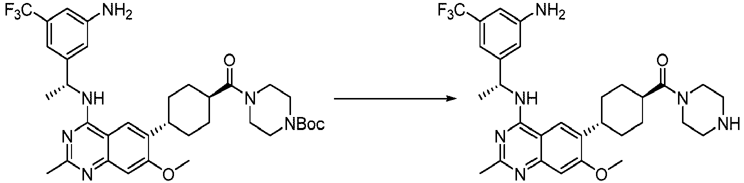

A mixture of *tert*-butyl 4-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperazine-1-carboxylate (220 mg, 0.33 mmol) in a solution of ethyl acetate in hydrochloric acid (3 M, 10 mL) was stirred at 25 °C for reaction for 2 h. The reactant was concentrated to give ((1*R*,4*R*)-4-(4-(((*R*)-1-(3-amino)-5-(trifluoromethyl)phenyl)ethyl)amino)-7-methoxy-2-methylquinazolin-6-yl)cyclohexyl)(piperazin-1-yl)methanone, and the crude product could be used in the next step without further purification.

LC-MS: (ESI, *m*/*z*): [M+H]⁺= 571.2.

### Intermediate 4: 2-(1-(2-(2,6-dioxopiperidin-3-yl)-1,3-dioxoisoindolin-5-yl)piperidin-4-yl)acetaldehyde

### Step 1: 2-(2,6-dioxopiperidin-3-yl)-5-fluoroisoindoline-1,3-dione

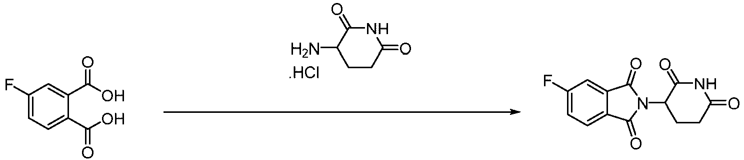

3-Aminopiperidine-2,6-dione hydrochloride (5.0 g, 0.03 mol) and potassium acetate (8.8 g, 0.09 mol) were added to a solution of 4-fluorophthalic acid (5.52 g, 0.03 mol) in acetic acid (50 mL). The reaction mixture was stirred at 120 °C for reaction overnight and concentrated under reduced pressure, and the concentrate was diluted with water (100 mL). The resulting mixture was stirred at room temperature for 30 min and filtered. The solid was washed with water (50 mL ×2) and dried under reduced pressure to give the product.

LC-MS: (ESI, *m*/*z*): [M+H]⁺= 277.1

### Step 2: preparation of 2-(2,6-dioxopiperidin-3-yl)-5-(4-(2-hydroxyethyl)piperidin-1-yl)isoindoline-1,3-dione

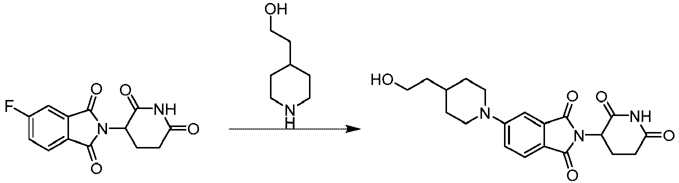

A mixture of 2-(2,6-dioxopiperidin-3-yl)-5-fluoroisoindoline-1,3-dione (500 mg, 1.81 mmol), 2-(piperidin-4-yl)ethyl-1-ol (280 mg, 2.17 mmol), and DIEA (701 mg, 5.43 mmol) in NMP (5 mL) was reacted in a microwave reactor at 140 °C for 5 h. The reaction mixture was diluted with water (50 mL), and the resulting mixture was extracted with ethyl acetate (50 mL × 2). The organic phase was collected, washed with water (100 mL × 2) and saturated brine (50 mL), dried over anhydrous sodium sulfate, filtered, and concentrated. The concentrate was purified by silica gel column chromatography to give the product.

LC-MS: (ESI, *m*/*z):* [M+H]⁺= 386.1.

### Step 3: preparation of 2-(1-(2-(2,6-dioxopiperidin-3-yl)-1,3-dioxoisoindolin-5-yl)piperidin-4-yl)acetaldehyde

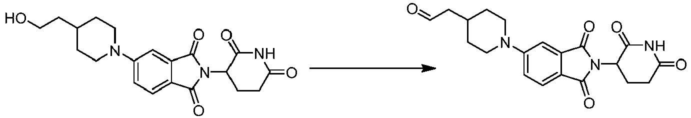

A mixture of 2-(2,6-dioxopiperidin-3-yl)-5-(4-(2-hydroxyethyl)piperidin-1-yl)isoindoline-1,3-dione (300 mg, 0.78 mmol) and IBX (436 mg, 1.56 mmol) in acetonitrile (6 mL) was stirred at 80 °C for reaction for 2 h. The reaction mixture was diluted with water (30 mL), and the resulting mixture was extracted with ethyl acetate (30 mL × 2). The organic phase was collected, washed with water (50 mL × 2) and saturated brine (30 mL), dried over anhydrous sodium sulfate, filtered, and concentrated. The concentrate was purified by silica gel column chromatography to give the product.

LC-MS: (ESI, *m*/*z):* [M+H]⁺= 384.1.

1H NMR (400 MHz, DMSO-*d*₆) δ 11.07 (s, 1H), 9.69 (t, *J* = 1.6 Hz, 1H), 7.65 (d, *J* = 8.6 Hz, 1H), 7.31 (d, *J* = 2.0 Hz, 1H), 7.23 (dd, *J* = 8.6, 2.2 Hz, 1H), 5.06 (dd, *J* = 12.9, 5.4 Hz, 1H), 4.03 (dd, *J* = 10.3, 2.9 Hz, 2H), 3.04 - 2.82 (m, 3H), 2.65 - 2.52 (m, 2H), 2.41 (dd, *J* = 6.7, 1.6 Hz, 2H), 2.17 - 1.97 (m, 2H), 1.73 (d, *J* = 11.1 Hz, 2H), 1.2-1.18 (m, 2H).

### Intermediate 5: 2-(3-(4-chloro-3-(2,4-dioxotetrahydropyrimidin-1(2H)-yl)benzoyl)-3-azaspiro[5.5]undecan-9-yl)acetaldehyde

### Step 1: preparation of 4-chloro-3-(2,4-dioxotetrahydropyrimidin-1(2H)-yl)benzoic acid

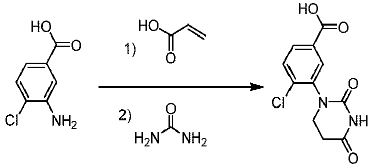

3-Amino-4-chlorobenzoic acid (5.0 g, 2.93 mmol) was suspended in acrylic acid (8.05 mL, 117 mmol), and the suspension was stirred at 100 °C for reaction for 3 h. Then the reaction liquid was cooled to room temperature while stirring. Acetic acid (33 mL) was added, and the stirred suspension was heated at 100 °C for 10 min. Urea (11.00 g, 183 mmol) was added, and the reaction liquid was stirred at 120 °C for reaction overnight. The reaction liquid was added to a mixture of ice water and concentrated hydrochloric acid (37%), and the mixture was stirred. The resulting suspension was stored in a refrigerator at 5 °C overnight and filtered, and the solid was washed with water and dried to give a solid. The solid was triturated in a hydrochloric acid solution (0.05 M), filtered, washed with methyl *tert-*butyl ether, and dried under reduced pressure at 40 °C to give the target product.

LC-MS: (ESI, *m*/*z*): [M+H]⁺=269.0

### Step 2: preparation of pentafluorophenyl 4-chloro-3-(2,4-dioxotetrahydropyrimidin-1(2H)-yl)benzoate

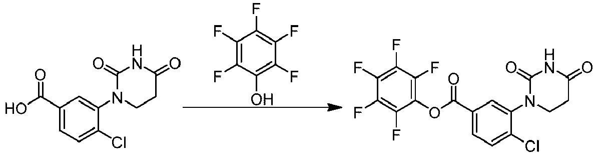

A mixture of 4-chloro-3-(2,4-dioxotetrahydropyrimidin-1(2*H*)-yl)benzoic acid (2.0 g, 7.58 mmol), 2,3,4,5,6-pentafluorophenol (1.67 g, 9.09 mmol), and *N*,*N'*-dicyclohexylcarbodiimide (1.87 g, 9.09 mmol) in an *N,N-*dimethylformamide (20 mL) solution was stirred at room temperature for reaction for 3 h. The reaction liquid was poured into water (200 mL), and the resulting solution was stirred for 0.5 h and extracted with ethyl acetate (200 mL × 3). The organic phase was collected, washed with water (500 mL × 2) and saturated brine (300 mL), dried over anhydrous sodium sulfate, filtered, and concentrated. The concentrate was purified by silica gel column chromatography to give the target product.

LC-MS: (ESI, *m*/*z*): [M+H]⁺=435.0

### Step 3: preparation of 2-(3-azaspiro[5.5]undecan-9-yl)ethan-1-ol

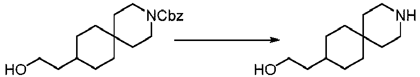

Pd(OH)₂/C (200 mg) was added to a mixture of benzyl 9-(2-hydroxyethyl)-3-azaspiro[5.5]undecane-3-carboxylate (1.0 g, 3 mmol) in an EA (10 mL) solution, and the reaction mixture was stirred at room temperature under hydrogen atmosphere for reaction for 16 h. The mixture was filtered, and the filtrate was concentrated to give the target product.

### Step 4: preparation of 1-(2-chloro-5-(9-(2-hydroxyethyl)-3-azaspiro[5.5]undecane-3-carbonyl)phenyl)dihydropyrimidine-2,4(1H,3H)-dione

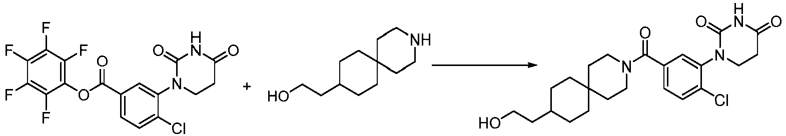

Pentafluorophenyl 4-chloro-3-(2,4-dioxotetrahydropyrimidin-1(2*H*)-yl)benzoate (1.0 g, 2.5 mmol) and DIEA (0.5 mL) were added to a mixture of 2-(3-azaspiro[5.5]undecan-9-yl)ethan-1-ol (500 mg, 2.5 mmol) in a DMSO (5 mL) solution. Water (30 mL) was added to quench the reaction, and the resulting mixture was extracted with EA (10 mL × 3). The organic phase was collected, washed with water (10 mL × 2) and saturated brine (10 mL), dried over anhydrous sodium sulfate, filtered, and concentrated. The concentrate was purified by column chromatography to give the target compound.

LC-MS: (ESI, *m*/*z):* [M+ H]⁺ = 448.1

### Step 5: preparation of 2-(3-(4-chloro-3-(2,4-dioxotetrahydropyrimidin-1(2H)-yl)benzoyl)-3-azaspiro[5.5]undecan-9-yl)acetaldehyde

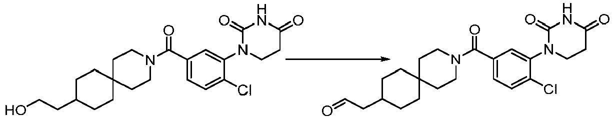

IBX (873 mg, 3.1 mmol) was added to a mixture of 1-(2-chloro-5-(9-(2-hydroxyethyl)-3-azaspiro[5.5]undecane-3-carbonyl)phenyl)dihydropyrimidine-2,4(1*H*,3*H*)-dione (700 mg, 1.56 mmol) in a THF (12 mL) solution. The reaction mixture was stirred at 80 °C for reaction for 2 h. Water (30 mL) was added to quench the reaction, and the solution was extracted with EA (10 mL × 3). The organic phase was collected, washed with water (10 mL × 2) and saturated brine (10 mL), dried over anhydrous sodium sulfate, filtered, and concentrated. The concentrate was purified by column chromatography to give the target compound.

LC-MS: (ESI, *m*/*z):* [M+ H]⁺ = 446.1

### Intermediate 6: 2-(4-(2-(2,6-dioxopiperidin-3-yl)-1,3-dioxoisoindolin-5-yl)piperazin-1-yl)acetaldehyde

### Step 1: preparation of tert-butyl 4-(2,2-dimethoxyethyl)piperazine-1-carboxylate

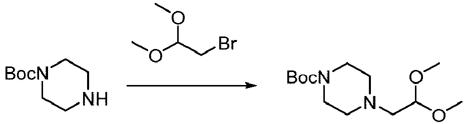

2-Bromo-1,1-dimethoxyethane (3.63 g, 21.51 mmol) was added to a mixture of *tert*-butyl piperazine-1-carboxylate (2.0 g, 10.75 mmol), potassium carbonate (4.45 g, 32.26 mmol), and potassium iodide (892 mg, 5.38 mmol) in acetone (20 mL), and the mixture was stirred at 80 °C for reaction overnight. The reaction mixture was concentrated under reduced pressure. The concentrate was dissolved in ethyl acetate (50 mL) and washed with water (50 mL × 2) and saturated brine (50 mL). The organic phase was collected, dried over anhydrous sodium sulfate, filtered, and concentrated. The concentrate was purified by silica gel column chromatography to give the desired product. LC-MS: (ESI, *m*/*z*): [M+H]⁺= 275.0.

### Step 2: preparation of 1-(2,2-dimethoxyethyl)piperazine

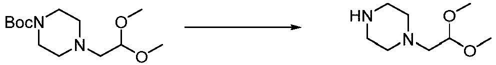

A mixture of *tert*-butyl 4-(2,2-dimethoxyethyl)piperazine-1-carboxylate (2.2 g, 8.03 mmol) in a hydrochloric acid/dioxane (4 M, 10 mL) solution was stirred at room temperature overnight. The mixture was concentrated under reduced pressure to give the target product (1.5 g, crude product). The crude product was directly used in the next step.

### Step 3: preparation of 5-(4-(2,2-dimethoxyethyl)piperazin-1-yl)-2-(2,6-dioxopiperidin-3-yl)isoindoline-1,3-dione

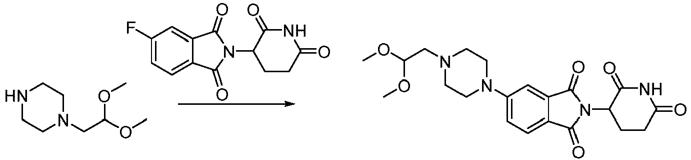

A mixture of 2-(2,6-dioxopiperidin-3-yl)-5-fluoroisoindoline-1,3-dione (1.48 g, 5.36 mmol), 1-(2,2-dimethoxyethyl)piperazine (1.4 g, 8.05 mmol), and DIEA (4.15 g, 36.16 mmol) in an NMP (10 mL) solution was reacted in a microwave reactor at 140 °C for 5 h. The mixture was diluted with water (50 mL), and the resulting mixture was extracted with ethyl acetate (50 mL × 2). The organic phase was collected, washed with water (100 mL × 2) and saturated brine (50 mL), dried over anhydrous sodium sulfate, filtered, and concentrated. The concentrate was purified by silica gel column chromatography to give the target product.

LC-MS: (ESI, *m*/*z*): [M+H]⁺= 431.1.

### Step 4: preparation of 2-(4-(2-(2,6-dioxopiperidin-3-yl)-1,3-dioxoisoindolin-5-yl)piperazin-1-yl)acetaldehyde

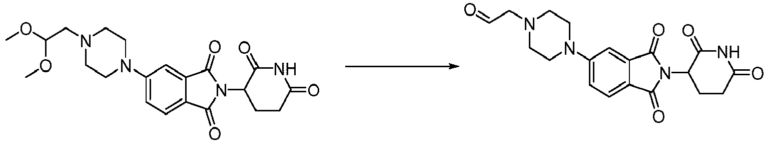

A mixture of 5-(4-(2,2-dimethoxyethyl)piperazin-1-yl)-2-(2,6-dioxopiperidin-3-yl)isoindoline-1,3-dione (1.8 g, 4.19 mmol) in a trifluoroacetic acid/dichloromethane (5 mL/5 mL) solution was stirred at room temperature for reaction for 60 h. The mixture was concentrated under reduced pressure, water was added, and the pH was adjusted to 8 with sodium bicarbonate (aqueous solution). The mixture was extracted with ethyl acetate (100 mL × 2). The organic phase was collected, washed with water (100 mL × 2) and saturated brine (100 mL), dried over anhydrous sodium sulfate, filtered, and concentrated. The concentrate was purified by silica gel column chromatography to give the target product.

LC-MS: (ESI, *m*/*z*): [M+H]⁺=385.0.

### Intermediate 7: 2-(9-(4-chloro-3-(2,4-dioxotetrahydropyrimidin-1(2H)-yl)benzoyl)-3,9-diazaspiro[5.5]undecan-3-yl)acetaldehyde

### Step 1: preparation of tert-butyl 9-(4-chloro-3-(2,4-dioxotetrahydropyrimidin-1(2H)-yl)benzoyl)-3,9-diazaspiro[5.5]undecane-3-carboxylate

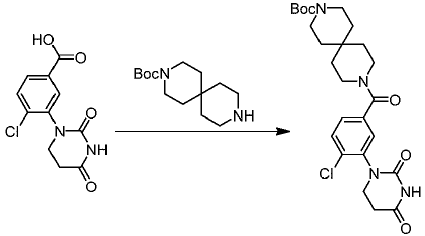

HATU (1.66 g, 4.37 mmol), *tert*-butyl 3,9-diazaspiro[5.5]undecyl-3-carboxylate (0.96 g, 3.78 mmol), and *N-*methylmorpholine (0.8 g, 7.92 mmol) were added to a solution of 3-(2,4-dioxotetrahydropyrimidin-1(2*H*)-yl)-4-methoxybenzoic acid (1.0 g, 3.78 mmol) in *N*,*N*-dimethylformamide (10 mL) at room temperature under nitrogen atmosphere. After the reaction mixture was stirred for reaction for 2 h, water (50 mL) was added to the reaction mixture to quench the reaction, and the resulting mixture was extracted with ethyl acetate (50 mL × 3). The organic phase was collected, washed with saturated brine (50 mL × 3), dried over anhydrous sodium sulfate, filtered, and concentrated. The concentrate was purified by silica gel column chromatography to give the target product.

LC-MS: (ESI, *m*/*z):* [M+H]⁺= 505.2

### Step 2: preparation of 1-(2-chloro-5-(3,9-diazaspiro[5.5]undecyl-3-carbonyl)phenyl)dihydropyrimidine-2,4(1H,3H)-dione

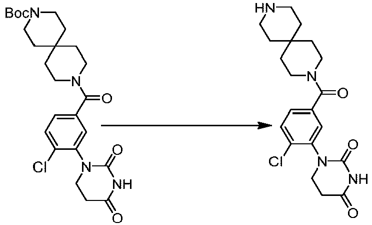

A reaction liquid of *tert-*butyl 9-(4-chloro-3-(2,4-dioxotetrahydropyrimidin-1(2*H*)-yl)benzoyl)-3,9-diazaspiro[5.5]undecane-3-carboxylate (1.56 g, 3.1 mmol) in a trifluoroacetic acid/dichloromethane (2.5 mL/5 mL) solution was stirred at room temperature for reaction for 4 h. The reaction liquid was concentrated under reduced pressure to give a crude product of the target compound. The crude product was directly used in the next step.

LC-MS: (ESI, *m*/*z):* [M+H]⁺= 405.1

### Step 3: preparation of 1-(2-chloro-5-(9-(2,2-dimethoxyethyl)-3,9-diazaspiro[5.5]undecane-3-carbonyl)phenyl)dihydropyrimidine-2,4(1H,3H)-dione

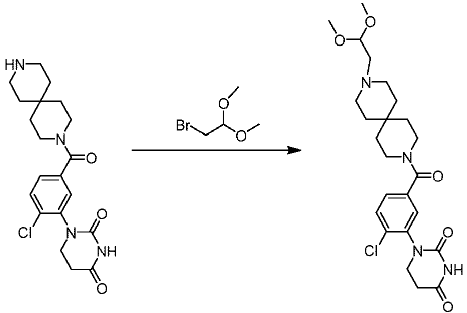

2-Bromo-1,1-dimethoxyethane (46 mg, 0.3 mmol) was added to a mixture of 1-(2-chloro-5-(3,9-diazaspiro[5.5]undecyl-3-carbonyl)phenyl)dihydropyrimidine-2,4(1*H*,3*H*)-dione (200 mg, 0.25 mmol), potassium carbonate (155 mg, 1.25 mmol), and potassium iodide (19 mg, 0.11 mmol) in an acetone (4 mL) solution. The reaction liquid was stirred at 80 °C for reaction for 3 h and then concentrated under reduced pressure. The concentrate was purified by silica gel column chromatography to give the target compound.

LC-MS: (ESI, *m*/*z):* [M+H]⁺= 493.3.

### Step 4: preparation of 2-(9-(4-chloro-3-(2,4-dioxotetrahydropyrimidin-1(2H)-yl)benzoyl)-3,9-diazaspiro[5.5]undecane-3-yl)acetaldehyde

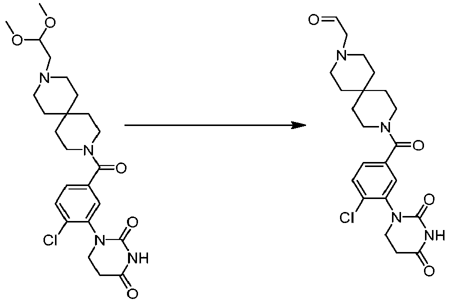

A mixture of 1-(2-chloro-5-(9-(2,2-dimethoxyethyl)-3,9-diazaspiro[5.5]undecane-3-carbonyl)phenyl)dihydropyrimidine-2,4(1*H*,3*H*)-dione (121 mg, 0.246 mmol) in a trifluoroacetic acid/dichloromethane (1 mL/2 mL) solution was stirred at room temperature for reaction for 4 h. The reaction liquid was concentrated under reduced pressure. The concentrate was purified by silica gel column chromatography to give the target compound.

LC-MS: (ESI, *m*/*z*): [M+H]⁺=447.1.

### Intermediate 8: 3-(1-(2-(2,6-dioxopiperidin-3-yl)-1,3-dioxoisoindolin-5-yl)piperidin-4-yl)propionaldehyde

### Step 1: preparation of 2-(2,6-dioxopiperidin-3-yl)-5-(4-(3-hydroxypropyl)piperidin-1-yl)isoindoline-1,3-dione

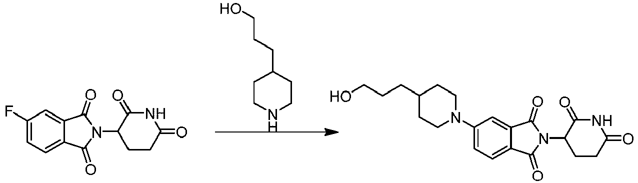

A mixture of 2-(2,6-dioxopiperidin-3-yl)-5-fluoroisoindoline-1,3-dione (500 mg, 1.81 mmol), 3-(piperidin-4-yl)propyl-1-ol (310 mg, 2.17 mmol) and DIEA (701 mg, 5.43 mmol) in NMP (5 mL) was reacted in a microwave reactor at 140 °C for 5 h. The reaction mixture was diluted with water (50 mL), and the resulting mixture was extracted with ethyl acetate (50 mL × 2). The organic phase was collected, washed with water (100 mL × 2) and saturated brine (50 mL), dried over anhydrous sodium sulfate, filtered, and concentrated. The concentrate was purified by silica gel column chromatography to give the target product.

LC-MS: (ESI, *m*/*z):* [M+H]⁺= 400.2.

### Step 2: preparation of 3-(1-(2-(2,6-dioxopiperidin-3-yl)-1,3-dioxoisoindolin-5-yl)piperidin-4-yl)propionaldehyde

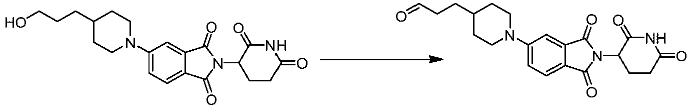

A mixture of 2-(2,6-dioxopiperidin-3-yl)-5-(4-(3-hydroxypropyl)piperidin-1-yl)isoindoline-1,3-dione (310 mg, 0.78 mmol) and IBX (436 mg, 1.56 mmol) in acetonitrile (6 mL) was stirred at 80 °C for reaction for 2 h. The reaction mixture was diluted with water (30 mL), and the resulting mixture was extracted with ethyl acetate (30 mL × 2). The organic phase was collected, washed with water (50 mL × 2) and saturated brine (30 mL), dried over anhydrous sodium sulfate, filtered, and concentrated. The concentrate was purified by silica gel column chromatography to give the product.

LC-MS: (ESI, *m*/*z*): [M+H]⁺= 398.2.

### Intermediate 9: (R)-N-(1-(3-amino-5-(trifluoromethyl)phenyl)ethyl)-7-methoxy-2-methyl-6-(piperidin-4-yl)quinazolin-4-amine

### Step 1: preparation of 2-amino-5-bromo-4-methoxybenzoic acid

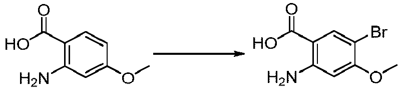

NBS (19.6 g, 110.0 mmol) was added to a stirred solution of 2-amino-4-methoxybenzoic acid (16.7 g, 100.0 mmol) in DMF (300 mL) at 0 °C. The reaction mixture was stirred at 25 °C for reaction for 1 h, and water (300 mL) was added to quench the reaction. The mixture was extracted with EA (300 mL × 3), and the organic phase was collected, washed with sodium chloride (a saturated aqueous solution, 300 mL × 3), dried over anhydrous ammonium sulfate, and concentrated to give the target crude product. The crude product was directly used in the next step without purification.

LC-MS: (ESI, *m*/*z):* [M+H]⁺= 247.4.

### Step 2: preparation of 6-bromo-7-methoxy-2-methylquinazolin-4-ol

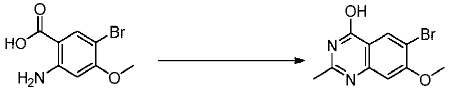

Ammonium acetate (61.0 g, 793 mmol) and trimethyl orthoacetate (95.1 g, 793 mmol) were added to a mixture of 2-amino-5-bromo-4-methoxybenzoic acid (19.5g, 79.3 mmol) in a MeOH (195 mL) solution. The reaction mixture was stirred in a closed container at 120 °C for reaction for 24 h. The reaction mixture was poured into water (500 mL) and the solid product was filtered. The crude product was washed with EA to give the target crude product, and the crude product was directly used in the next step without purification.

LC-MS: (ESI, *m*/*z*): [M+H]⁺=271.0.

### Step 3: preparation of tert-butyl 4-(4-hydroxy-7-methoxy-2-methylquinazolin-6-yl)-3,6-dihydropyridin-1(2H)-carboxylate

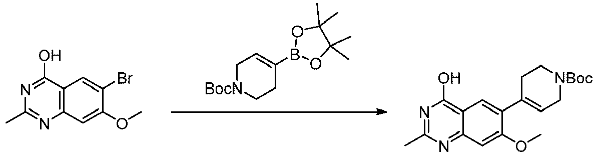

*tert*-Butyl 4-(4,4,5,5-tetrainethyl-1,3,2-dioxaborane-2-yl)-3,6-dihydropyridin-1(2*H*)-carboxylate (1.73 g, 6.18 mmol), sodium carbonate (982 mg, 9.27 mmol), and Pd(dppf)₂Cl₂ (226 mg, 0.319 mmol) were added to a mixture of 6-bromo-7-methoxy-2-methylquinazolin-4-ol (1.5 g, 3.09 mmol) in a mixed solution of DMF (30 mL) and water (4 mL). The reaction mixture was stirred at 110 °C for reaction overnight. The reaction was quenched with water (300 mL), and the mixture was extracted with EA (200 mL × 3). The organic phase was collected, dried over anhydrous sodium sulfate, and concentrated under reduced pressure. The concentrate was purified by column chromatography to give the target product.

LC-MS: (ESI, *m*/*z):* [M+H]⁺= 372.2.

### Step 4: preparation of tert-butyl 4-(4-hydroxy-7-methoxy-2-methylquinazolin-6-yl)piperidine-1-carboxylate

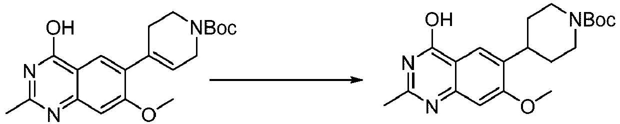

Pd/C (300 mg) was added to a solution of *tert*-butyl 4-(4-hydroxy-7-methoxyquinazolin-6-yl)-3,6-dihydropyridin-1(2*H*)-carboxylate (500 mg, 0.894 mmol) in MeOH (20 mL). The reaction mixture was stirred at 50 °C under H₂ atmosphere for reaction for 2 days. The reaction liquid was filtered through celite, and the filtrate was concentrated under reduced pressure to give the target product *tert*-butyl 4-(4-hydroxy-7-methoxy-2-methylquinazolin-6-yl)piperidine-1-carboxylate. The crude product was directly used in the next step.

LC-MS: (ESI, *m*/*z):* [M+H]⁺= 374.2.

### Step 5: preparation of tert-butyl 4-(7-methoxy-2-methyl-4-(((2,4,6-triisopropylphenyl)sulfonyl)oxy)quinazolin-6-yl)pieridine-1-carboxlate

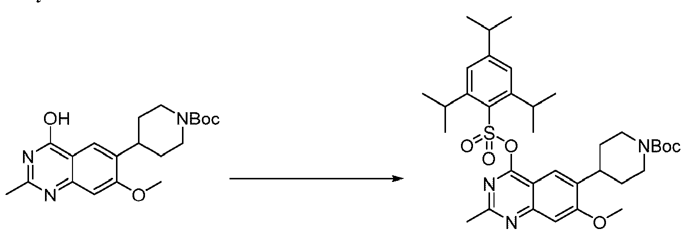

TEA (0.3 mL, 2.4 mmol) was added to a solution of *tert*-butyl 4-(4-hydroxy-7-methoxy-2-methylquinazolin-6-yl)piperidine-1-carboxylate (250.0 mg, 0.76 mmol), TPSCl (295.0 mg, 1.0 mmol), and DMAP (13.0 mg, 0.1 mmol) in DCM (5.0 mL). The reaction mixture was stirred at room temperature for reaction for 12 h. The reaction liquid was diluted with DCM and washed with a saturated NaHCO₃ solution. The organic phase was dried over Na₂SO₄, filtered, and concentrated. The resulting crude product was purified by column chromatography (the eluent system was EA/PE) to give the product *tert*-butyl 4-(7-methoxy-2-methyl-4-(((2,4,6-triisopropylphenyl)sulfonyl)oxy)quinazolin-6-yl)piperidine-1-carboxylate.

LC-MS: (ESI, *m*/*z):* [M+H]⁺= 640.0.

### Step 6: preparation of tert-butyl (R)-4-(7-methoxy-2-methyl-4-((1-(3-nitro-5-(trifluoromethyl)phenyl)ethyl)amino)quinazolin-6-yl)piperidine-1-carboxylate

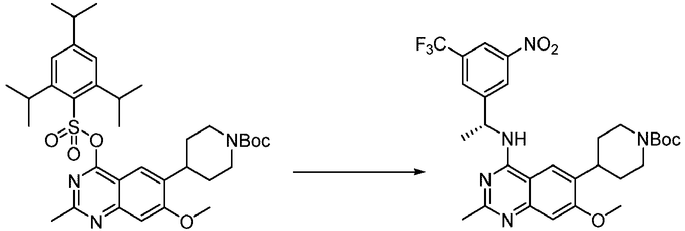

(*R*)-1-(3-Nitro-5-(trifluoromethyl)phenyl)ethan-1-amine hydrochloride (203 mg, 0.75 mmol) and TEA (0.8 mL) were added to a solution of *tert*-butyl 4-(7-methoxy-2-methyl-4-(((2,4,6-triisopropylphenyl)sulfonyl)oxy)quinazolin-6-yl)piperidine-1-carboxylate (400 mg, 0.03 mmol) in DMSO (8 mL). The reaction mixture was stirred at 90 °C for reaction overnight. Water was added to quench the reaction, and the resulting mixture was extracted with EA. The organic phase was dried over Na₂SO₄, filtered, and concentrated. The resulting crude product was purified by normal phase column chromatography to give the target product *tert*-butyl (*R*)-4-(7-methoxy-2-methyl-4-((1-(3-nitro-5-(trifluoromethyl)phenyl)ethyl)amino)quinazolin-6-yl)piperidine-1-carboxylate.

LC-MS: (ESI, *m*/*z):* [M+H]⁺= 590.3.

### Step 7: preparation of (R)-7-methoxy-2-methyl-N-(1-(3-nitro-5-(trifluoromethyl)phenyl)ethyl)-6-(piperidin-4-yl)quinazolin-4-amine

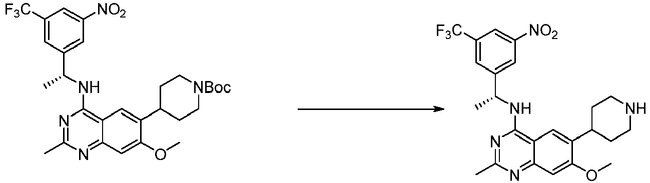

TFA was added to a solution of *tert*-butyl (*R*)-4-(7-methoxy-2-methyl-4-((1-(3-nitro-5-(trifluoromethyl)phenyl)ethyl)amino)quinazolin-6-yl)piperidine-1-carboxylate (220 mg, 0.328 mmol) in DCM. The reaction mixture was stirred at 25 °C for reaction for 2 h and then concentrated to give the target product (*R*)-7-methoxy-2-methyl-*N*-(1-(3-nitro-5-(trifluoromethyl)phenyl)ethyl)-6-(piperidin-4-yl)quinazolin-4-amine. The resulting crude product was directly used in the next step.

LC-MS: (ESI, *m*/*z):* [M+H]⁺= 490.2

### Step 8: preparation of (R)-N-(1-(3-amino-5-(trifluoromethyl)phenyl)ethyl)-7 -methoxy-2-methyl-6-(piperidin-4-yl)quinazolin-4-amine

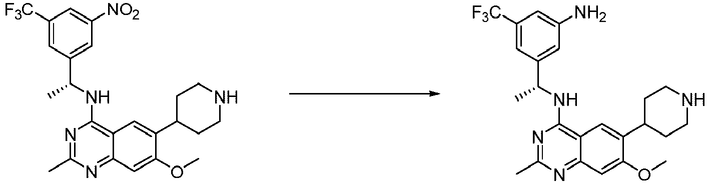

NH₄Cl (135.6 mg, 2.5 mmol) and Fe powder (141.5 mg, 2.5 mmol) were added to a solution of (*R*)-7-methoxy-2-methyl-*N*-(1-(3-nitro-5-(trifluoromethyl)phenyl)ethyl)-6-(piperidin-4-yl)quinazolin-4-amine (124 mg, 0.25 mmol) in EtOH/H₂O (3 mL/1 mL). The reaction mixture was stirred at 70 °C under N₂ atmosphere for reaction for 2 h. The reaction liquid was filtered and concentrated to give a crude product. The crude product was purified by preparative HPLC to give the target product (*R*)-*N*-(1-(3-amino-5-(trifluoromethyl)phenyl)ethyl)-7-methoxy-2-methyl-6-(piperidin-4-yl)quinazolin-4-amine.

LC-MS: (ESI, *m*/*z*): [M+H]⁺= 460.2.

¹H NMR (400 MHz, DMSO-*d*₆) δ 8.32 (s, 1H), 8.09 (s, 1H), 7.03 (s, 1H), 6.88 (d, *J* = 11.1 Hz, 1H), 6.70 (s, 1H), 5.57 (d, *J* = 6.9 Hz, 1H), 3.89 (s, 3H), 3.39 - 3.25 (m, 2H), 3.22 - 3.13 (m, 1H), 3.07 - 2.95 (m, 2H), 2.36 (s, 3H), 2.03 - 1.85 (m, 4H), 1.56 (d, *J* = 7.0 Hz, 3H).

### Intermediate 10: (R)-N-(1-(3-amino-5-(trifluoromethyl)phenyl)ethyl)-7-methoxy-2-methyl-6-(piperidin-4-oxy)quinazolin-4-amine

### Step 1: tert-butyl 4-((methylsulfonyl)oxy)piperidine-1-carboxylate

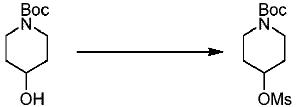

Methanesulfonyl chloride (7.4 g, 64.9 mmol) dissolved in a DCM (20.0 mL) solution was added dropwise to a mixture of *tert*-butyl 4-hydroxypiperidine-1-carboxylate (10.0 g, 49.7 mmol) and TEA (15.0 g, 149.1 mmol) in a DCM (100 mL) solution at 0 °C. The reaction mixture was stirred at room temperature for reaction overnight, then water (200 mL) was added, and the resulting mixture was extracted with DCM (200 mL × 2). The organic phase was collected, washed with saturated brine, dried over anhydrous sodium sulfate, and concentrated under reduced pressure. The concentrate was purified by column chromatography to give the target product.

LC-MS: (ESI, *m*/*z*): [M+H]⁺= 280.1.

### Step 2: preparation of 6,7-dimethoxy-2-methylquinazolin-4-ol

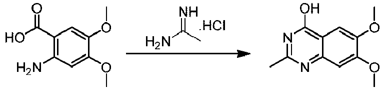

Acetamidine hydrochloride (19.0 g, 0.2 mol) and sodium acetate (16.4 g, 0.2 mol) were added to a mixture of 2-amino-4,5-dimethoxybenzoic acid (20.0 g, 0.1 mol) in a 2-methoxyethanol (120.0 mL) solution. The reaction mixture was stirred at 140 °C for reaction overnight. The reaction mixture was poured into water (250 mL), and the resulting mixture was stirred at room temperature for 10 min and then filtered. The filter cake was washed with water, and the solid was dried under vacuum to give the target product.

LC-MS: (ESI, *m*/*z*): [M+H]⁺= 221.2.

### Step 3: preparation of 7-methoxy-2-methylquinazolin-4,6-diol

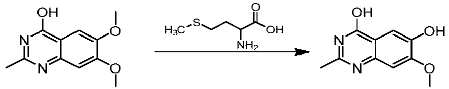

A reaction mixture of 6,7-dimethoxy-2-methylquinazolin-4-ol (9.1 g, 41.4 mmol) and DL-methionine (10.5 g, 70.4 mmol) in methanesulfonic acid (50 mL) was stirred at 80 °C for reaction overnight. Ice water was added to quench the reaction, and the resulting mixture was basified with a sodium hydroxide (2 N) solution. The precipitate was filtered off to give the target crude product.

LC-MS: (ESI, *m*/*z*): [M+H]⁺= 207.2.

### Step 4: preparation of tert-butyl 4-((4-hydroxy-7-methoxy-2-methylquinazolin-6-yl)oxy)piperidine-1-carboxylate

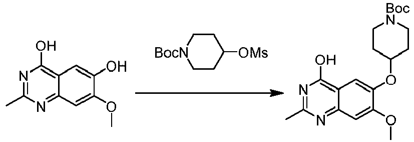

A reaction mixture of 7-methoxy-2-methylquinazolin-4,6-diol (1.4 g, 6.8 mmol), *tert*-butyl 4-((methylsulfonyl)oxy)piperidine-1-carboxylate (2.2 g, 8.2 mmol), and potassium carbonate (1.8 g, 13.6 mmol) in an NMP (10.0 mL) solution was stirred at 100 °C for reaction overnight. Then water (50.0 mL) was added, and the resulting mixture was extracted with EA (50 mL × 3). The organic phase was collected, washed with saturated brine, dried over anhydrous sodium sulfate, and concentrated under reduced pressure. The concentrate was purified by reversed-phase column chromatography (acetonitrile:water = 0-100%) to give the target product.

LC-MS: (ESI, *m*/*z):* [M+H]⁺= 390.2.

### Step 5: preparation of tert-butyl 4-((7-methoxy-2-methyl-4-(((2,4,6-triisopropylphenyl)sulfonyl)oxy)quinazolin-6-yl)oxy)piperidine-1-carboxylate

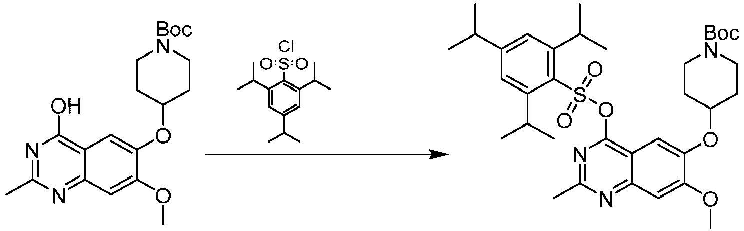

A reaction mixture of *tert*-butyl 4-((4-hydroxy-7-methoxy-2-methylquinazolin-6-yl)oxy)piperidine-1-carboxylate (800.0 mg, 2.0 mmol), 2,4,6-triisopropylbenzenesulfonyl chloride (748.0 mg, 2.4 mmol), DMAP (125.0 mg, 0.4 mmol), and TEA (623.0 mg, 6.0 mmol) in a DCM (10.0 mL) solution was stirred at room temperature for reaction overnight. The reaction mixture was concentrated, and the concentrate was purified by column chromatography to give the target product.

LC-MS: (ESI, *m*/*z*): [M+Na] ⁺ = 678.1.

### Step 6: preparation of tert-butyl (R)-4-((7-methoxy-2-methyl-4-((1-(3-nitro-5-(trifluoromethyl)phenyl)ethyl)amino)quinazolin-6-yl)oxy)piperidine-1-carboxylate

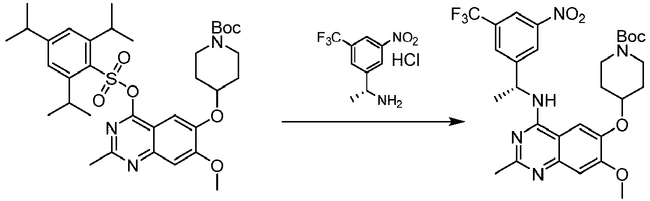

A reaction mixture of *tert*-butyl 4-((7-methoxy-2-methyl-4-(((2,4,6-triisopropylphenyl)sulfonyl)oxy)quinazolin-6-yl)oxy)piperidine-1-carboxylate (0.8 g, 1.2 mmol), (*R*)-1-(3-nitro-5-(trifluoromethyl)phenyl)ethan-1-amine hydrochloride (0.43 g, 1.8 mmol), and TEA (1.1 g, 10.8 mmol) in a DMSO (15.0 mL) solution was stirred at 90 °C for reaction overnight. Then water (50.0 mL) was added, and the resulting mixture was extracted with EA (50 mL × 3). The organic phase was collected, washed with saturated brine, dried over anhydrous sodium sulfate, and concentrated. The concentrate was purified by reversed-phase column chromatography (acetonitrile-water = 0-100%) to give the target product.

LC-MS: (ESI, *m*/*z*): [M+H]⁺= 606.0.

### Step 7: preparation of (R)-7-methoxy-2-methyl-N-(1-(3-nitro-5-(trifluoromethyl)phenyl)ethyl)-6-(piperidin-4-oxy)quinazolin-4-amine

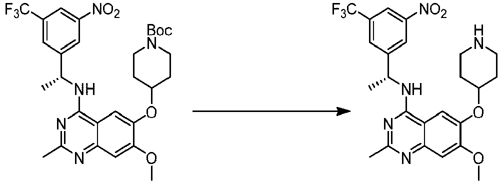

A mixture of *tert*-butyl (*R*)-4-((7-methoxy-2-methyl-4-((1-(3-nitro-5-(trifluoromethyl)phenyl)ethyl)amino)quinazolin-6-yl)oxy)piperidine-1-carboxylate (140 mg, 0.23 mmol) in a solution of DCM (5.0 mL) and TFA (1.0 mL) was stirred at room temperature for reaction for 2.0 h. The reactant was concentrated under reduced pressure to give (*R*)-7-methoxy-2-methyl-*N*-(1-(3-nitro-5-(trifluoromethyl)phenyl)ethyl)-6-(piperidin-4-oxy)quinazolin-4-amine.

LC-MS: (ESI, *m*/*z):* [M+H]⁺= 506.1.

### Step 8: preparation of (R)-N-(1-(3-amino-5-(trifluoromethyl)phenyl)ethyl)-7 -methoxy-2-methyl-6-(piperidin-4-oxy)quinazolin-4-amine

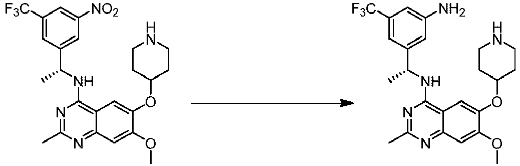

A reaction mixture of (*R*)-7 -methoxy-2-methyl-*N*-(1-(3-nitro-5-(trifluoromethyl)phenyl)ethyl)-6-(piperidin-4-oxy)quinazolin-4-amine (100.0 mg, 0.19 mmol) and Pd/C (20.0 mg) in a methanol (5.0 mL) solution was stirred at room temperature under hydrogen atmosphere for reaction overnight. The reaction liquid was filtered, and the filtrate was concentrated. The concentrate was purified by preparative HPLC to give the target product.

LC-MS: (ESI, *m*/*z*): [M+H]⁺=476.3.

¹H NMR (400 MHz, DMSO-*d*₆) δ 7.91 (d, *J* = 7.9 Hz, 1H), 7.78 (s, 1H), 7.04 (s, 1H), 6.88 (s, 1H), 6.85 (s, 1H), 6.69 (s, 1H), 5.66 - 5.46 (m, 3H), 4.54 - 5.48 (m, 1H), 3.87 (s, 3H), 3.01 - 2.93 (m, 2H), 2.64 - 2.53 (m, 2H), 2.35 (s, 3H), 1.93 - 1.89 (m, 2H), 1.58 - 1.44 (m, 5H).

### Intermediate 11: pentafluorophenyl 4-(2,4-dioxotetrahydropyrimidin-1(2H)-yl)benzoate

### Step 1: preparation of 4-((2-carboxyethyl)amino)benzoic acid

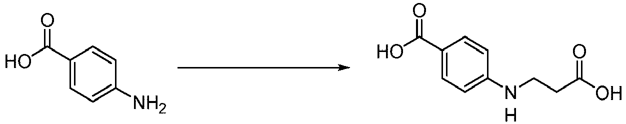

Acrylic acid (23.62 g, 328.1 mmol) was added to a solution of 4-aminobenzoic acid (15.0 g, 109.4 mmol) in AcOH/H₂O (40 mL/200 mL), and the resulting mixture was stirred at 110 °C for 12 h. The reaction liquid was cooled and filtered to give the target product 4-((2-carboxyethyl)amino)benzoic acid.

LC-MS: (ESI, *m*/*z*): [M+H]⁺= 210.0.

### Step 2: preparation of 4-(2,4-dioxotetrahydropyrimidin-1(2H)-yl)benzoic acid

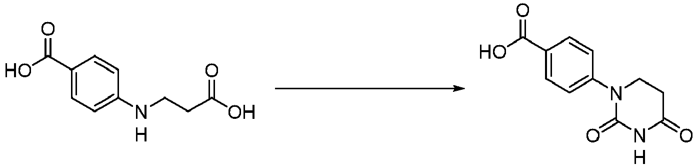

Urea (114.83 g, 1913.8 mmol) was added to a solution of 4-((2-carboxyethyl)amino)benzoic acid (20.0 g, 95.69 mmol) in AcOH (240 mL), and the resulting mixture was stirred at 110 °C for 24 h. After the reaction liquid was cooled, an HCl solution was added to adjust the pH to 1-2. A solid was precipitated, and the mixture was filtered to give the target product 4-(2,4-dioxotetrahydropyrimidin-1(2*H*)-yl)benzoic acid.

LC-MS: (ESI, *m*/*z):* [M+H]⁺= 233.1.

### Step 3: preparation of pentafluorophenyl 4-(2,4-dioxotetrahydropyrimidin-1(2H)-yl)benzoate

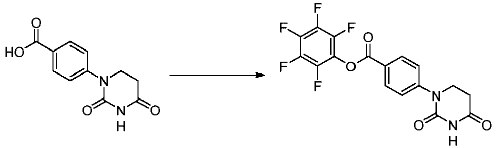

Pentafluorophenol (12.97 g, 70.51 mmol) and DCC (14.52 g, 70.51 mmol) were added to a solution of 4-(2,4-dioxotetrahydropyrimidin-1(2*H*)-yl)benzoic acid (15.0 g, 64.1 mmol) in DMF (200 mL), and the resulting mixture was stirred at room temperature overnight and extracted with ethyl acetate (100 mL × 3). The organic phase was washed with saturated brine (300 mL), dried over anhydrous sodium sulfate, filtered, and concentrated under reduced pressure. The crude product was purified by column chromatography (EA:PE = 3:7) to give the target product pentafluorophenyl 4-(2,4-dioxotetrahydropyrimidin-1(2*H*)-yl)benzoate.

¹H NMR (400 MHz, DMSO-*d*₆) δ 10.59 (s, 1H), 8.20 (d, *J* = 8.7 Hz, 2H), 7.64 (d, *J* = 8.7 Hz, 2H), 3.94 (t, *J* = 6.6 Hz, 2H), 2.76 (t, *J* = 6.6 Hz, 2H).

### Intermediate 12: tert-butyl (R)-(3-(1-aminoethyl)-5-(trifluoromethyl)phenyl)carbamate

### Step 1: preparation of 1-(3-nitro-5-(trifluoromethyl)phenyl)ethan-1-one

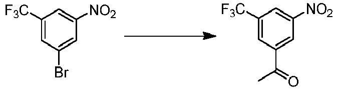

A reaction liquid of 1-bromo-3-nitro-5-(trifluoromethyl)benzene (50.0 g, 185.1 mmol), Pd(PPh₃)₂Cl₂ (12.9 g, 18.5 mmol), TEA (37.4 g, 370.2 mmol), and tributyl (1-ethoxyvinyl)stannane (86.6 g, 240.6 mmol) in dioxane (300 mL) was stirred at 80 °C for 16 h. Water (900 mL) was added to the reaction liquid to quench the reaction, and the resulting mixture was extracted with EA (500 mL × 3). The organic phases were combined, washed with saturated brine, dried over anhydrous Na₂SO₄, and concentrated under reduced pressure. The residue was stirred in a mixed solution of 6 N HCl (200 mL) and THF (200 mL) for 30 min, and then water (500 mL) was added. The resulting mixture was extracted with EA (500 mL × 2). The organic phases were combined, washed with saturated brine, dried over anhydrous sodium sulfate, filtered, and concentrated under reduced pressure. The crude product was purified by column chromatography (EA:PE = 0-1:9) to give 1-(3-nitro-5-(trifluoromethyl)phenyl)ethan-1-one.
¹H NMR (400 MHz, CDCl₃) δ 8.95 (s, 1H), 8.69 (s, 1H), 8.53 (s, 1H), 2.75 (s, 3H).

### Step 2: preparation of 1-(3-amino-5-(trifluoromethyl)phenyl)ethan-1-one

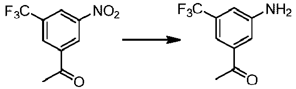

A mixture of 1-(3-nitro-5-(trifluoromethyl)phenyl)ethan-1-one (1.5 g, 6.4 mmol) and Pd/C (400 mg) in THF (30 mL) was stirred at 60 °C for 16 h. The reaction liquid was filtered, and the filtrate was concentrated under reduced pressure to give 1-(3-amino-5-(trifluoromethyl)phenyl)ethan-1-one. The resulting product was directly used in the next step.

LC-MS: (ESI, *m*/*z):* [M+H]⁺= 203.0.

### Step 3: preparation of tert-butyl (3-acetyl-5-(trifluoromethyl)phenyl)carbamate

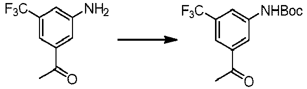

A mixture of 1-(3-amino-5-(trifluoromethyl)phenyl)ethan-1-one (1.1 g, 5.4 mmol) and (Boc)₂O (5.9 g, 27.0 mmol) in dioxane (20 mL) was stirred at 80 °C for 16 h. The reaction liquid was directly concentrated under reduced pressure, and the resulting crude product was purified by column chromatography (EA:PE = 0-3:17) to give *tert-*butyl (3-acetyl-5-(trifluoromethyl)phenyl)carbamate.

LC-MS: (ESI, *m*/*z*): [M+H]⁺=303.0.

¹H NMR (400 MHz, DMSO-*d*₆) δ 8.26 (s, 1H), 8.18 (d, *J* = 6.5 Hz, 1H), 8.05 (s, 1H), 2.67 (d, *J* = 11.4 Hz, 3H), 1.41 (d, *J* = 11.7 Hz, 9H).

### Step 4: preparation of tert-butyl (S,E)-(3-(1-((tert-butylsulfinyl)imino)ethyl)-5-(trifluoromethyl)phenyl)carbamate

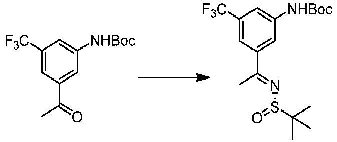

A mixture of *tert*-butyl (3-acetyl-5-(trifluoromethyl)phenyl)carbamate (1.0 g, 3.3 mmol), Ti(OEt)₄ (1.8 g, 6.6 mmol), diethylene glycol dimethyl ether (2.3 g, 17.5 mmol), and (*S*)-propane-2-sulfinamide (1.2 g, 9.9 mmol) in THF (10 mL) was stirred at 70 °C for 12 h. Water (50 mL) was added to the reaction liquid, and the resulting mixture was extracted with EA (30 mL × 3). The organic phases were combined, washed with saturated brine, dried over anhydrous sodium sulfate, filtered, and concentrated under reduced pressure. The crude product was purified by column chromatography (EA:PE = 0-1:4) to give *tert*-butyl (S,E)-(3-(1-((*tert*-butylsulfinyl)imino)ethyl)-5-(trifluoromethyl)phenyl)carbamate.

LC-MS: (ESI, *m*/*z*): [M+H]⁺= 407.0.

### Step 5: preparation of tert-butyl (3-((R)-1-(((S)-tert-butylsulfinyl)amino)ethyl)-5-(trifluoromethyl)phenyl)carbamate

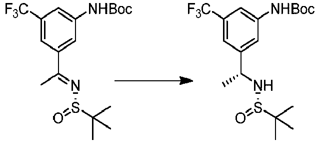

L-Selectride (1.0 mol/L/THF, 3.6 mL) was added to a solution of *tert*-butyl (*S,E*)-(3-(1-((*tert-*butylsulfinyl<sulfinyl>)imino)ethyl)-5-(trifluoromethyl)phenyl)carbamate (1.0 g, 2.4 mmol) in THF (20 mL) at - 78 °C, and the reaction liquid was stirred at -78 °C for 50 min. Water (50 mL) was added to the reaction liquid to quench the reaction, and then the resulting mixture was extracted with EA (50 mL × 2). The organic phases were combined, washed with saturated brine, dried over anhydrous sodium sulfate, filtered, and concentrated under reduced pressure. The crude product was purified by column chromatography (EA:PE = 0-1:1) to give *tert*-butyl (3-((*R*)-1-(((*S*)-*tert*-butylsulfinyl)amino)ethyl)-5-(trifluoromethyl)phenyl)carbamate.

LC-MS: (ESI, *m*/*z):* [M+H]⁺= 409.0.

¹H NMR (400 MHz, DMSO-*d*₆) δ 9.71 (s, 1H), 7.74 (d, *J* = 5.1 Hz, 2H), 7.39 (s, 1H), 4.15 (q, *J* = 6.7 Hz, 1H), 1.48 (s, 15H), 1.31 (d, *J* = 6.7 Hz, 3H), 1.11 (d, *J* = 2.1 Hz, 3H).

### Step 6: preparation of tert-butyl (R)-(3-(1-aminoethyl)-5-(trifluoromethyl)phenyl)carbamate

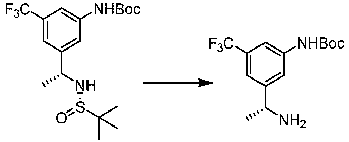

*tert*-Butyl (3-((*R*)-1-(((*S*)-*tert*-butylsulfinyl)amino)ethyl)-5-(trifluoromethyl)phenyl)carbamate (1.0 g, 2.4 mmol) was stirred in 1 N HCl/EtOAc (20 mL) at 0 °C for 3 h. The reaction liquid was rapidly filtered through a chromatographic column, washed with EtOAc, and then washed with a mixed solvent of DCM/MeOH (5/1). The filtrate was basified with 7 N NH₃/MeOH, and the organic phase was washed with water, dried over anhydrous Na₂SO₄, filtered, and concentrated to give *tert*-butyl (*R*)-(3-(1-aminoethyl)-5-(trifluoromethyl)phenyl)carbamate.

LC-MS: (ESI, *m*/*z*): [M+H]⁺=305.1.

¹H NMR (400 MHz, DMSO-*d*₆) δ 9.63 (s, 1H), 7.71 (s, 1H), 7.69 (s, 1H), 7.35 (s, 1H), 4.02 - 3.99 (m, 1H), 1.48 (s, 9H), 1.23 (d, *J* = 6.6 Hz, 3H).

### Intermediate 13: methyl (1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carboxylate

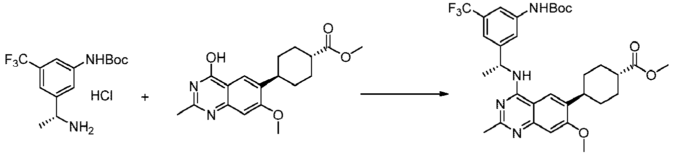

BOP (574 mg, 1.3 mmol) and DBU (380 mg, 2.5 mmol) were added to a solution of methyl (1*R*,4*R*)-4-(4-hydroxy-7-methoxy-2-methylquinazolin-6-yl)cyclohexane-1-carboxylate (330 mg, 1.0 mmol) in DMF (5 mL) while stirring. After stirring for 15 min, *tert*-butyl (*R*)-(3-(1-aminoethyl)-5-(trifluoromethyl)phenyl)carbamate hydrochloride (396 mg, 1.3 mmol) was added, and the reaction liquid was stirred at 70 °C for another 12 h. Water (10 mL) was added to the reaction liquid to quench the reaction, and then the resulting mixture was extracted with EA (30 mL × 3). The organic phase was dried over anhydrous Na₂SO₄ and then concentrated under reduced pressure. The resulting crude product was purified by column chromatography (EA:PE = 0-1) to give methyl (1*R*,4*R*)-4-(4-(((*R*)-1-(3-((*tert-*butoxycarbonyl)amino)-5-(trifluoromethyl)phenyl)ethyl)amino)-7-methoxy-2-methylquinazolin-6-yl)cyclohexane-1-carboxylate.

LC-MS: (ESI, *m*/*z):* [M+H]⁺= 617.4.

¹H NMR (400 MHz, DMSO-*d*₆) δ 9.69 (s, 1H), 8.12 (s, 1H), 7.77 (d, *J* = 25.2 Hz, 2H), 7.41 (s, 1H), 7.01 (s, 1H), 5.71 - 5.55 (m, 1H), 3.90 (s, 3H), 3.62 (s, 3H), 2.96 (d, *J* = 11.8 Hz, 1H), 2.39 (d, *J* = 10.4 Hz, 4H), 2.06 (d, *J* = 12.0 Hz, 2H), 1.89 (d, *J* = 14.0 Hz, 2H), 1.64 - 1.50 (m, 7H), 1.46 (s, 9H).

### Intermediate 14: (1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carboxylic acid

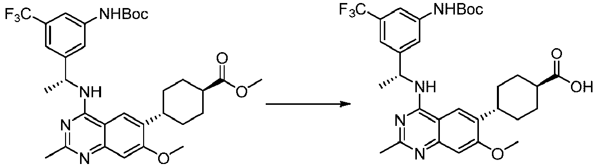

LiOH·H₂O (48 mg, 1.2 mmol) was added to a solution of methyl (1*R*,4*R*)-4-(4-(((*R*)-1-(3-((*tert-*butoxycarbonyl)amino)-5-(trifluoromethyl)phenyl)ethyl)amino)-7-methoxy-2-methylquinazolin-6-yl)cyclohexane-1-carboxylate (250 mg, 0.4 mmol) in THF/H₂O (10 mL/5 mL), and the reaction liquid was stirred at 40 °C overnight. The reaction liquid was adjusted to pH with 10% citric acid to be weakly acidic, and then the resulting mixture was extracted with EtOAc (30 mL × 3). The organic phases were combined, washed with saturated brine, and concentrated under reduced pressure to give (1*R*,4*R*)-4-(4-(((*R*)-1-(3-((*tert-*butoxycarbonyl)amino)-5-(trifluoromethyl)phenyl)ethyl)amino)-7-methoxy-2-methylquinazolin-6-yl)cyclohexane-1-carboxylic acid.

LC-MS: (ESI, *m*/*z*): [M+H]⁺= 603.1.

¹H NMR (400 MHz, DMSO-*d*₆) δ 9.67 (s, 1H), 8.20 (d, *J* = 7.4 Hz, 1H), 8.07 (s, 1H), 7.77 (d, *J* = 24.0 Hz, 2H), 7.41 (s, 1H), 7.00 (s, 1H), 5.68 - 5.52 (m, 1H), 3.89 (s, 3H), 2.95 (d, *J* = 11.3 Hz, 1H), 2.40 - 2.23 (m, 4H), 2.06 (d, *J* = 12.0 Hz, 2H), 1.88 (d, *J* = 12.0 Hz, 2H), 1.67 - 1.41 (m, 16H).

### Intermediate 15: (1R,4R)-4-(7-methoxy-2-methyl-4-(((R)-1-(3-nitro-5-(trifluoromethyl)phenyl)ethyl)amino)quinazolin-6-yl)cyclohexane-1 -carboxylic acid

### Step 1: preparation of methyl (1R,4R)-4-(7-methoxy-2-methyl-4-(((R)-1-(3-nitro-5-(trifluoromethyl)phenyl)ethyl)amino)quinazolin-6-yl)cyclohexane-1-carboxylate

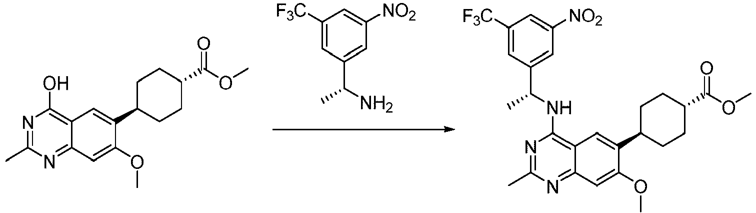

BOP (871 mg, 1.97 mmol) and DBU (577 mg, 3.80 mmol) were added to a solution of methyl (1*R*,4*R*)-4-(4-hydroxy-7-methoxy-2-methylquinazolin-6-yl)cyclohexane-1-carboxylate (500 mg, 1.52 mmol) in DMF (10 mL) while stirring. After the resulting mixture was stirred for another 15 min, (*R*)-1-(3-nitro-5-(trifluoromethyl)phenyl)ethan-1-amine (461 mg, 1.97 mmol) was added. The reaction liquid was then stirred at 70 °C overnight. Water (50 mL) was added to the reaction liquid, and the resulting mixture was extracted with EA (50 mL × 3). The organic phases were combined, washed with saturated brine (100 mL), dried over anhydrous Na₂SO₄, filtered, and concentrated under reduced pressure. The resulting crude product was purified by flash column chromatography (EA:PE = 0-1) to give methyl (1*R*,4*R*)-4-(7-methoxy-2-methyl-4-(((*R*)-1-(3-nitro-5-(trifluoromethyl)phenyl)ethyl)amino)quinazolin-6-yl)cyclohexane-1-carboxylate.

LC-MS: (ESI, *m*/*z*): [M+H]⁺=547.1.

¹H NMR (400 MHz, DMSO-*d*₆) δ 8.62 (s, 1H), 8.34 (d, *J* = 6.0 Hz, 3H), 8.04 (s, 1H), 7.01 (s, 1H), 5.68 (p, *J* = 6.9 Hz, 1H), 3.89 (s, 3H), 3.64 (s, 3H), 2.94 (t, *J* = 11.4 Hz, 1H), 2.48 - 2.37 (m, 1H), 2.34 (s, 3H), 2.08 (d, *J* = 12.0 Hz, 2H), 1.97 - 1.86 (m, 2H), 1.69 (d, *J* = 7.1 Hz, 3H), 1.65 - 1.47 (m, 4H).

### Step 2: preparation of (1R,4R)-4-(7-methoxy-2-methyl-4-(((R)-1-(3-nitro-5-(trifluoromethyl)phenyl)ethyl)amino)quinazolin-6-yl)cyclohexane-1 -carboxylic acid

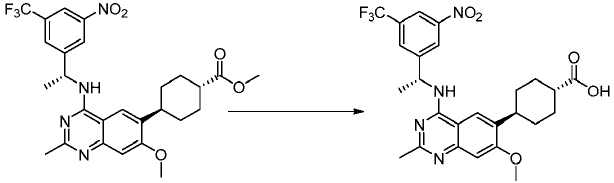

LiOH·H₂O (240 mg, 8.0 mmol) was added to a solution of methyl (1*R*,4*R*)-4-(7-methoxy-2-methyl-4-(((*R*)-1-(3-nitro-5-(trifluoromethyl)phenyl)ethyl)amino)quinazolin-6-yl)cyclohexane-1-carboxylate (300 mg, 0.55 mmol) in THF/H₂O (15 mL, 1:2). The reaction liquid was then stirred at 50 °C overnight. Water (50 mL) was added to the reaction liquid to quench the reaction, and then the resulting mixture was extracted with EA (50 mL × 3). The organic phases were combined, washed with saturated brine (100 mL), dried over anhydrous Na₂SO₄, filtered, and concentrated under reduced pressure to give (1*R*,4*R*)-4-(7-methoxy-2-methyl-4-(((*R*)-1-(3-nitro-5-(trifluoromethyl)phenyl)ethyl)amino)quinazolin-6-yl)cyclohexane-1 -carboxylic acid.

LC-MS: (ESI, *m*/*z*): [M+H]⁺=533.3.

¹H NMR (400 MHz, DMSO-*d*₆) δ 12.09 (s, 1H), 8.89 (s, 1H), 8.63 (s, 1H), 8.36 (d, *J* = 10.6 Hz, 2H), 8.14 (s, 1H), 7.02 (s, 1H), 5.89 - 5.64 (m, 1H), 3.92 (s, 3H), 2.95 (t, *J* = 11.6 Hz, 1H), 2.41 (s, 3H), 2.37 - 2.26 (m, 1H), 2.08 (d, *J* = 10.7 Hz, 2H), 1.96 - 1.85 (m, 2H), 1.72 (d, *J* = 7.1 Hz, 3H), 1.68 - 1.44 (m, 4H).

### Intermediate 16: (1R,4R)-4-(4-(((R)-1-(4-bromothiophen-2-yl)ethyl)amino)-7-methoxy-2-methylquinazolin-6-yl)cyclohexane-1-carboxylic acid

### Step 1: preparation of 1-(1,3-dicarbonylisoindolin-2-yl) 4-methyl (1R,4R)-cyclohexane-1,4-dicarboxylate

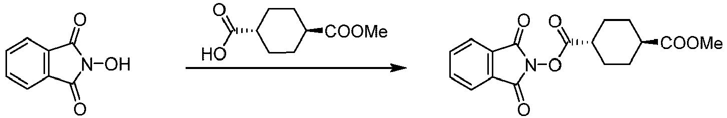

EDCI (34.7 g, 118.2 mmol) and DMAP (1.3 g, 10.7 mmol) were added to a solution of (1R,4R)-4-(methoxycarbonyl)cyclohexane-1-carboxylic acid (20 g, 107.4 mmol) and 2-hydroxyisoindoline-1,3-dione (20 g, 107.4 mmol) in DCM (300 mL). Then the reaction liquid was stirred at room temperature for 2 h. Brine and DCM were added to the reaction liquid. The organic phase was isolated, dried over anhydrous Na₂SO₄, filtered, and concentrated under reduced pressure. The crude product was purified by column chromatography (DCM) to give 1-(1,3-dicarbonylisoindolin-2-yl) 4-methyl (1*R*,4*R*)-cyclohexane-1,4-dicarboxylate.

LC-MS: (ESI, *m*/*z*): [M+Na]⁺=354.1.

### Step 2: preparation of 2-amino-5-iodo-4-methoxybenzonitrile

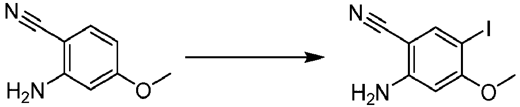

NIS (133 g, 613 mmol) was added to a solution of 2-amino-4-methoxybenzonitrile (86.3 g, 582 mmol) in DMF (1000 mL). Then the reaction liquid was stirred at room temperature for 2 h. Brine and DCM were added to the reaction liquid. The organic phase was isolated, dried over anhydrous Na₂SO₄, filtered, and concentrated under reduced pressure to give 2-amino-5-iodo-4-methoxybenzonitrile.

LC-MS: (ESI, *m*/*z*): [M+Na]⁺= 296.9.

### Step 3: preparation of N-(2-cyano-4-iodo-5-methoxyphenyl)acetamide

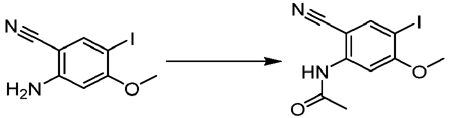

Ac₂O (71.3 g, 700 mmol) was added to a solution of 2-amino-5-iodo-4-methoxybenzonitrile (160 g, 580 mmol) in HOAc (1600 mL). Then the reaction liquid was stirred at room temperature overnight, filtered, and then concentrated to give *N*-(2-cyano-4-iodo-5-methoxyphenyl)acetamide.

LC-MS: (ESI, *m*/*z):* [M+H]⁺= 317.0.

### Step 4: preparation of methyl (1R,4R)-4-(4-acetylamino-5-cyano-2-methoxyphenyl)cyclohexane-1-carboxylate

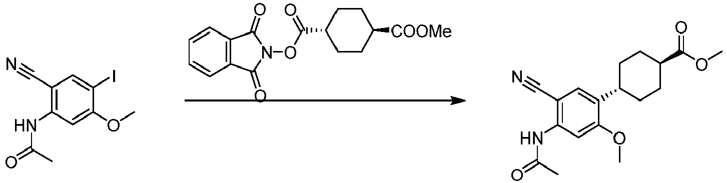

NiBr₂(dtbpy) (2.31 g, 4.75 mmol) was added to a solution of *N*-(2-cyano-4-iodo-5-methoxyphenyl)acetamide (15.0 g, 47.45 mmol), 1-(1,3-dicarbonylisoindolin-2-yl) 4-methyl (1*R*,4*R*)-cyclohexane-1,4-dicarboxylate (31.40 g, 94.90 mmol) and Hantzsch-ester (24.00 g, 94.90 mmol) in DMA (237 mL, 0.2 M). The reaction liquid was purged with N₂ for 5 min and irradiated with two Kessil PR160-purple LED lamps (30 W High Luminous DEX 2100 LED, *λ*max = 390 nm) for 16 h. The reaction temperature was controlled at 30-40 °C. After the reaction was completed, the reaction liquid was poured into water (1 L), and then the resulting mixture was extracted with EtOAc (150 mL × 3). The organic phases were combined, washed with saturated brine (400 mL × 3), dried over anhydrous Na₂SO₄, and concentrated under reduced pressure. The resulting crude product was purified by column chromatography (MeOH:DCM = 1:99). The eluate containing the product with a concentration value of 80 mL was collected and filtered. The filtrate was concentrated to give a mixture with a cis-to-trans ratio of 1:1, and the filter cake was a mixture with a cis-to-trans ratio of 15:1. The mixture with the cis-to-trans ratio of 15:1 was dissolved in DMA (85 mL), and a 0.5 M aqueous K₂CO₃ solution (200 mL) was added. The resulting mixture was stirred at room temperature for 2 h and filtered to give a mixture with a cis-to-trans ratio of 20:1. The mixture was recrystallized with acetonitrile (180 mL) to give the target product (with a cis-to-trans ratio of 97:2).

LC-MS: (ESI, *m*/*z):* [M+H]⁺= 331.1.

¹H NMR (400 MHz, CDCl₃) δ 8.04 (s, 1H), 7.55 (s, 1H), 7.29 (s, 1H), 3.89 (s, 3H), 3.69 (s, 3H), 2.90-2.84 (m, 1H), 2.35-2.26 (m, 1H), 2.25 (s, 3H), 2.11-2.08 (m, 2H), 1.92-1.89 (m, 2H), 1.63-1.56 (m, 2H), 1.39-1.33 (m, 2H). Step 5: preparation of methyl (1*R*,4*R*)-4-(7-methoxy-2-methyl-4-carbonyl-3,4-dihydroquinazolin-6-yl)cyclohexane-1 -carboxylate

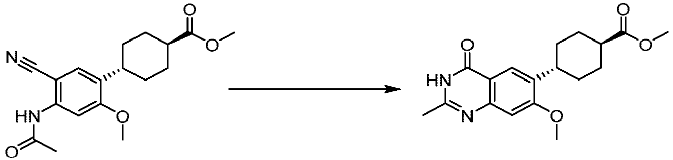

Methyl (1*R*,4*R*)-4-(4-acetylamino-5-cyano-2-methoxyphenyl)cyclohexane-1-carboxylate (3 g, 9.08 mmol) was added to *i*-PrOH (80 mL), and the resulting mixture was purged with HCl gas at room temperature for 1 h. The reaction liquid was filtered, and the solid was dried to give methyl (1*R*,4*R*)-4-(7-methoxy-2-methyl-4-carbonyl-3,4-dihydroquinazolin-6-yl)cyclohexane-1-carboxylate.

LC-MS: (ESI, *m*/*z*): [M+H]⁺= 331.1.

¹H NMR (400 MHz, DMSO-*d*₆) δ 7.87 (s, 1H), 7.23 (s, 1H), 3.95 (s, 3H), 3.61 (s, 3H), 2.98-2.87 (m, 1H), 2.58 (s, 3H), 2.47-2.39 (m, 1H), 2.06-1.98 (m, 2H), 1.88-1.80 (m, 2H), 1.59-1.42 (m, 4H).

### Step 6: preparation of (R,Z)-N-(1-(4-bromothiophen-2-yl)ethylidene)-2-methylpropane-2-sulfinamide

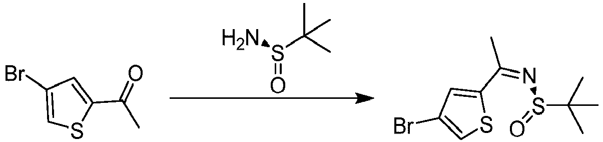

A reaction liquid of 1-(4-bromothiophen-2-yl)ethan-1-one (10.0 g, 48.80 mmol), (R)-2-methylpropane-2-sulfinamide (8.90 g, 73.20 mmol), and Ti(OEt)₄ (27.8 g, 122.00 mmol) in THF (70 mL) was stirred at 80 °C for 3 h. Water (200 mL) was added to the reaction liquid to quench the reaction, and the resulting mixture was extracted with EA (100 mL × 3). The organic phases were combined, dried over anhydrous Na₂SO₄, filtered, and concentrated under reduced pressure. The resulting crude product was purified by column chromatography (DCM:MeOH = 100:1) to give (*R*,*Z*)-*N*-(1-(4-bromothiophen-2-yl)ethylidene)-2-methylpropane-2-sulfinamide.

¹H NMR (400 MHz, DMSO-*d*₆) δ 7.96 (d, *J=* 1.2 Hz, 1H), 7.85 (d, *J=* 1.2 Hz, 1H), 2.68 (s, 3H), 1.19 (s, 9H). Step 7: preparation of (*R*)-*N*-((*R*)-1-(4-bromothiophen-2-yl)ethyl)-2-methylpropane-2-sulfmamide

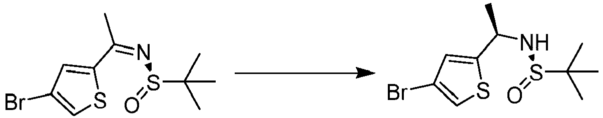

9-BBN/THF (0.5 M, 200 mL) was added to a solution of (*R*,*Z*)-*N*-(1-(4-bromothiophen-2-yl)ethylidene)-2-methylpropane-2-sulfinamide (7.0 g, 22.7 mmol) in THF (10 mL). The reaction liquid was stirred at 25 °C for 0.5 h. MeOH (10 mL) was added to the reaction liquid to quench the reaction, then water (100 mL) was added, and the resulting mixture was extracted with EA (100 mL × 3). The organic phases were combined, dried over anhydrous Na₂SO₄, filtered, and concentrated under reduced pressure. The resulting crude product was purified by column chromatography (DCM:MeOH = 100:1) to give (*R*)-*N*-((*R*)-1-(4-bromothiophen-2-yl)ethyl)-2-methylpropane-2-sulfinamide.

¹H NMR (400 MHz, DMSO-*d*₆) δ 7.54 (d, *J* = 1.2 Hz, 1H), 7.08 (s, 1H), 5.93 (d, *J* = 7.2 Hz, 1H), 4.64-4.58 (m, 1H), 1.48 (d, *J* = 6.8 Hz, 3H),1.12 (s, 9H).

### Step 8: preparation of (R)-1-(4-bromothiophen-2-yl)ethan-1-amine hydrochloride

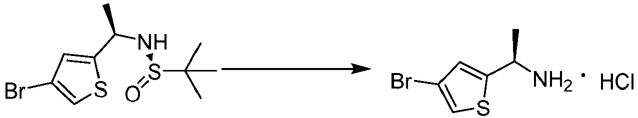

HCl (12 M, 454 mmol) was added to a solution of (*R*)-*N*-((*R*)-1-(4-bromothiophen-2-yl)ethyl)-2-methylpropane-2-sulfinamide (6.0 g, 19.34 mmol) in 1,4-dioxane (60 mL). The reaction liquid was then stirred at 25 °C for 2 h. The reaction liquid was directly concentrated. The resulting crude product was stirred in an MTBE (100 mL) solution, filtered, and dried to give (*R*)-1-(4-bromothiophen-2-yl)ethan-1-amine hydrochloride.

¹H NMR (400 MHz, DMSO-*d*₆) δ 8.62 (s, 3H), 7.71 (s, 1H), 7.32 (s, 1H), 4.71-4.67 (m, 1H), 1.57 (d, *J =* 6.8 Hz, 3H).

### Step 9: preparation of methyl (1R,4R)-4-(4-(((R)-1-(4-bromothiophen-2-yl)ethyl)amino)-7-methoxy-2-methylquinazolin-6-yl)cyclohexane-1 -carboxylate

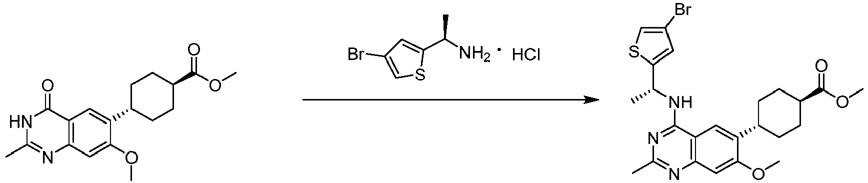

DBU (3.45 g, 22.67 mmol) was added to a solution of methyl (1*R*,4*R*)-4-(7-methoxy-2-methyl-4-carbonyl-3,4-dihydroquinazolin-6-yl)cyclohexane-1-carboxylate (3.00 g, 9.07 mmol) and BOP (5.21 g, 11.79 mmol) in DMF (35 mL) at room temperature. After stirring for 15 min, a solution of (*R*)-1-(4-bromothiophen-2-yl)ethan-1-amine hydrochloride (2.86 g, 11.79 mmol) in DMF (35 mL) was added dropwise. Then the reaction liquid was stirred at room temperature for 20 min, and then heated to 70 °C and stirred for another 8 h. The reaction liquid was purified by reversed-phase column chromatography to give methyl (1R,4R)-4-(4-(((R)-1-(4-bromothiophene-2-yl)ethyl)amino)-7-methoxy-2-methylquinazolin-6-yl)cyclohexane-1-carboxylate.

LC-MS: (ESI, *m*/*z):* [M+H]⁺= 518.0, 520.0.

### Step 10: preparation of (1R,4R)-4-(4-(((R)-1-(4-bromothiophen-2-yl)ethyl)amino)-7-methoxy-2-methylquinazolin-6-yl)cyclohexane-1-carboxylic acid

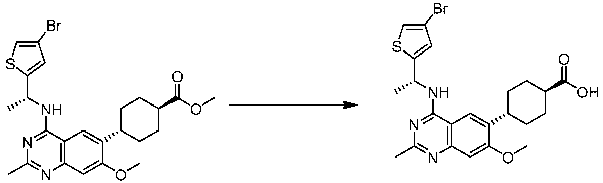

LiOH (0.81 g, 19.30 mmol) was added to a solution of methyl (1*R*,4*R*)-4-(4-(((*R*)-1-(4-bromothiophen-2-yl)ethyl)amino)-7-methoxy-2-methylquinazolin-6-yl)cyclohexane-1-carboxylate (2.00 g, 3.86 mmol) in THF/H₂O (20.0 mL/20.0 mL). The reaction liquid was stirred at 25 °C for 16 h and acidified with a 2 N HCl solution, and a precipitate was formed. The mixture was filtered, and the filter cake was dried under vacuum to give (1R,4R)-4-(4-(((*R*)-1-(4-bromothiophene-2-yl)ethyl)amino)-7-methoxy-2-methylquinazolin-6-yl)cyclohexane-1-carboxylic acid. LC-MS: (ESI, *m*/*z):* [M+H]⁺= 504.0, 506.0.

### Intermediate 17: pentafluorophenyl 4-(2,6-dioxopiperidin-3-yl)benzoate

### Step 1: preparation of methyl 4-(4-cyano-1-methoxy-1-oxybutan-2-yl)benzoate

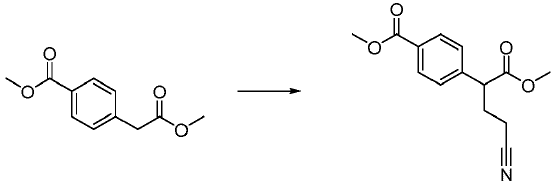

Benzyltrimethylammonium hydroxide (6.03 g, 36.057 mmol) and acrylonitrile (5.727 g, 79.32 mmol) were added to a solution of methyl 4-(2-methoxy-2-oxoethane)benzoate (15.0 g, 72.12 mmol) in toluene (200 mL). The resulting mixture was stirred at 30 °C for 16 h and extracted with ethyl acetate (100 mL × 5). The organic phase was washed with saturated brine (500 mL), dried over anhydrous sodium sulfate, filtered, and concentrated under reduced pressure. The crude product was purified by column chromatography (EA:PE = 3:7) to give the target product methyl 4-(4-cyano-1-methoxy-1-oxobutan-2-yl)benzoate.

¹H NMR (400 MHz, DMSO-*d*₆) δ 7.95 (d, J = 4.0 Hz, 2H), 7.46 (d, *J* = 6.0 Hz, 2H), 3.90-3.80 (m, 4H), 3.62 (s, 3H), 2.48-2.38 (m, 2H), 2.36-2.26 (m, 1H), 2.10-1.96 (m, 1H).

### Step 2: preparation of 4-(2,6-dioxopiperidin-3-yl)benzoic acid

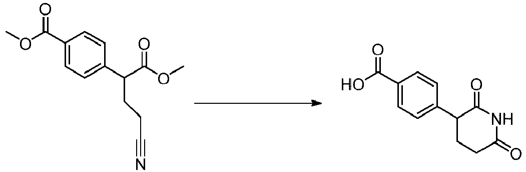

Methyl 4-(4-cyano-1-methoxy-1-oxobutan-2-yl)benzoate (8.5 g, 32.56 mmol) was dissolved in acetic acid (30 mL), and sulfuric acid (15 mL) was added. The resulting mixture was stirred at 110 °C for 12 h and extracted with dichloromethane/methanol (10/1, 150 mL × 3). The organic phase was washed with saturated brine (500 mL), dried over anhydrous sodium sulfate, filtered, and concentrated under reduced pressure. The crude product was purified by column chromatography (MeOH:DCM = 1:10) to give 4-(2,6-dioxopiperidin-3-yl)benzoate.

¹H NMR (400 MHz, DMSO-*d*₆) δ 12.89 (s, 1H), 10.89 (s, 1H), 7.91 (d, *J =* 4.0 Hz, 2H), 7.36 (d, *J* = 4.0 Hz, 2H), 4.02-3.92 (m, 1H), 2.72-2.63 (m, 1H), 2.56-2.45 (m, 1H), 2.30-2.20 (m, 1H), 2.10-2.00 (m, 1H).

### Step 3: preparation of pentafluorophenyl 4-(2,6-dioxopiperidin-3-yl)benzoate

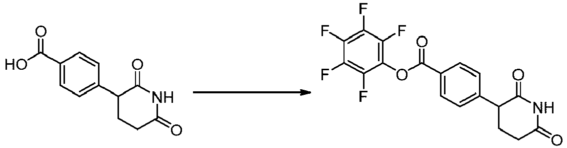

4-(2,6-Dioxopiperidin-3-yl)benzoic acid (1.3 g, 5.579 mmol) was dissolved in DMF (100 mL), and pentafluorophenol (1.129 g, 6.14 mmol) and DCC (1.265 g, 6.14 mmol) were added. The resulting mixture was stirred at room temperature overnight and extracted with ethyl acetate (100 mL × 3). The organic phase was washed with saturated brine (150 mL), dried over anhydrous sodium sulfate, filtered, and concentrated under reduced pressure. The crude product was purified by column chromatography (EA:PE = 3:7) to give pentafluorophenyl 4-(2,6-dioxopiperidin-3-yl)benzoate.

¹H NMR (400 MHz, DMSO-*d*₆) δ 10.95 (s, 1H), 8.17 (d, *J* = 4.0 Hz, 2H), 7.56 (d, *J* = 4.0 Hz, 2H), 4.14-4.08 (m, 1H), 2.79-2.69 (m, 1H), 2.66-2.55 (m, 1H), 2.30-2.23 (m, 1H), 2.15-2.05 (m, 1H).

### Intermediate 18: pentafluorophenyl 3-(2,4-dioxotetrahydropyrimidin-1(2H)-yl)-4-methoxybenzoate

### Step 1: preparation of 3-(2,4-dioxotetrahydropyrimidin-1(2H)-yl)-4-methoxybenzoic acid

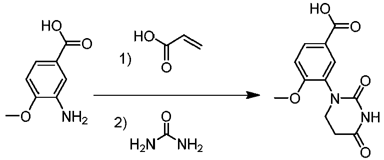

3-Amino-4-methoxybenzoic acid (5.0 g, 2.93 mmol) was suspended in acrylic acid (8.05 mL, 117 mmol), and the suspension was stirred at 100 °C for reaction for 3 h. Then the reaction liquid was cooled to room temperature while stirring. Acetic acid (33 mL) was added, and the stirred suspension was heated at 100 °C for 10 min. Urea (11.00 g, 183 mmol) was added, and the reaction liquid was stirred at 120 °C overnight. The reaction liquid was added to a mixture of ice water and concentrated hydrochloric acid (37%), and the mixture was stirred. The resulting suspension was stored in a refrigerator at 5 °C overnight and filtered, and the solid was washed with water and dried to give a solid. The solid was triturated in a hydrochloric acid solution (0.05 M), filtered, washed with methyl *tert-*butyl ether, and dried under reduced pressure at 40 °C to give the target product.

¹H NMR (400 MHz, DMSO-*d*₆) δ 12.70 (s, 1H), 10.34 (s, 1H), 7.92 (dd, *J =* 8.6, 2.2 Hz, 1H), 7.83 (d, *J* = 2.2 Hz, 1H), 7.21 (d, *J* = 8.8 Hz, 1H), 3.94 - 3.82 (m, 3H), 3.60 (t, *J* = 6.7 Hz, 2H), 2.69 (s, 2H).

### Step 2: preparation of pentafluorophenyl 3-(2,4-dioxotetrahydropyrimidin-1(2H)-yl)-4-methoxybenzoate

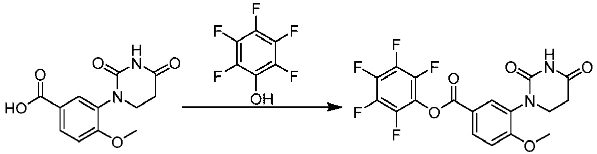

A mixture of 3-(2,4-dioxotetrahydropyrimidin-1(2*H*)-yl)-4-methoxybenzoic acid (2.0 g, 7.58 mmol), 2,3,4,5,6-pentafluorophenol (1.67 g, 9.09 mmol), and *N*,*N*'-dicyclohexylcarbodiimide (1.87 g, 9.09 mmol) in an *N,N-*dimethylformamide (20 mL) solution was stirred at room temperature for reaction for 3 h. The reaction liquid was poured into water (200 mL), and the resulting solution was stirred for 0.5 h and extracted with ethyl acetate (200 mL × 3). The organic phase was collected, washed with water (500 mL × 2) and saturated brine (300 mL), dried over anhydrous sodium sulfate, filtered, and concentrated. The concentrate was purified by column chromatography to give the target product pentafluorophenyl 3-(2,4-dioxotetrahydropyrimidine-1(2*H*)-yl)-4-methoxybenzoate.

LC-MS: (ESI, *m*/*z*):[M+H]⁺=431.1.

### Intermediate 19: pentafluorophenyl 3-(2,4-dioxotetrahydropyrimidin-1(2H)-yl)-4-fluorobenzoate

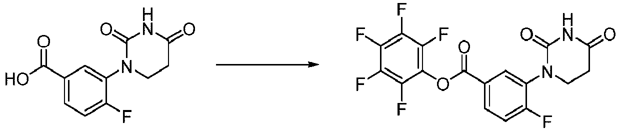

### Intermediate 19 was prepared by reference to the preparation method of intermediate 18. LC-MS: (ESI, m/z):[M+H]⁺=419.0.

### Intermediate 20: pentafluorophenyl 3-(2,4-dioxotetrahydropyrimidin-1(2H)-yl)benzoate

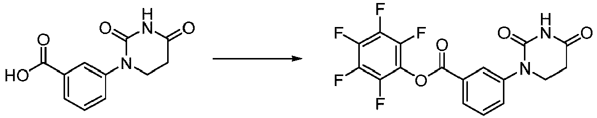

Intermediate 19 was prepared by reference to the preparation method of intermediate 18.

LC-MS: (ESI, *m*/*z*):[M+H]⁺=401.0.

### Intermediate 21: methyl (1R,4R)-4-(4-(((R)-1-(4-(2-((dimethylamino)methyl)phenyl)thiophen-2-yl)ethyl)amino)-7-methoxy-2-methylquinazolin-6-yl)cyclohexane-1-carboxylate

### Step 1: preparation of methyl (1R,4R)-4-(4-(((R)-1-(4-(2-formylphenyl)thiophen-2-yl)ethyl)amino)-7-methoxy-2-methylquinazolin-6-yl)cyclohexane-1 -carboxylate

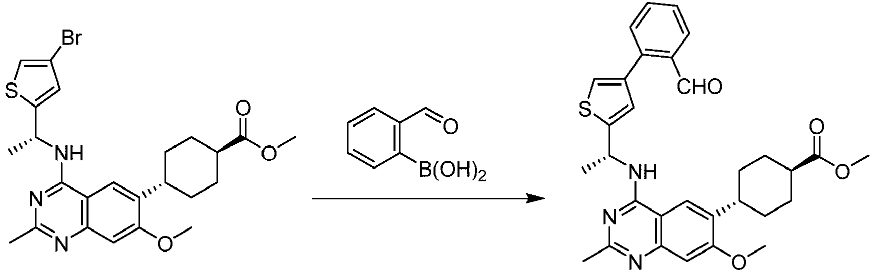

K₃PO₄ (255 mg, 1.2 mmol), (PPh₃)₂PdCl₂ (34 mg, 0.048 mmol), and (2-formylphenyl) boronic acid (87 mg, 0.58 mmol) were added to a solution of methyl (1*R*,4*R*)-4-(4-(((*R*)-1-(4-bromothiophen-2-yl)ethyl)amino)-7-methoxy-2-methylquinazolin-6-yl)cyclohexane-1-carboxylate (250 mg, 0.48 mmol) in 1,4-dioxane/H₂O (1.5 mL/0.5 mL). The reaction liquid was stirred at 100 °C overnight, concentrated, and then purified by column chromatography (PE/EA = 2/1 to 1/2) to give methyl (1*R*,4*R*)-4-(4-(((*R*)-1-(4-(2-formylphenyl)thiophen-2-yl)ethyl)amino)-7-methoxy-2-methylquinazolin-6-yl)cyclohexane-1-carboxylate.

LC-MS: (ESI, *m*/*z*): [M+H]⁺=544.1.

### Step 2: preparation of methyl (1R,4R)-4-(4-(((R)-1-(4-(2-((dimethylamino)methyl)phenyl)thiophen-2-yl)ethyl)amino)-7-methoxy-2-methylquinazolin-6-yl)cyclohexane-1-carboxylate

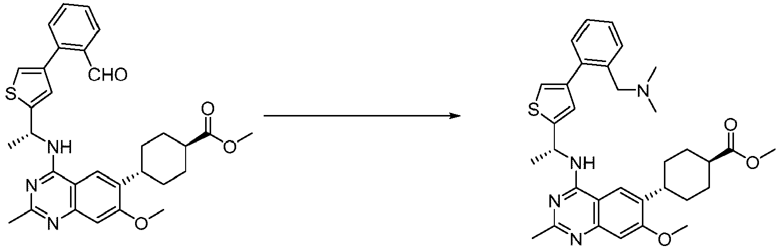

MeOH (3 mL), DCE (3 mL), and AcOH (5 drops) were added to a mixture of methyl (1*R*,4*R*)-4-(4-(((*R*)-1-(4-(2-formylphenyl)thiophen-2-yl)ethyl)amino)-7-methoxy-2-methylquinazolin-6-yl)cyclohexane-1-carboxylate (200 mg, 0.36 mmol) and dimethylamine hydrochloride (60 mg, 0.74 mmol). The above solution was stirred at room temperature for 1 h, and NaBH₃CN (46 mg, 0.74 mmol) was added. Then the reaction liquid was stirred at room temperature for another 3 h. DCM and water were added to the reaction liquid. The organic phase was isolated, dried over anhydrous Na₂SO₄, filtered, and concentrated under reduced pressure. The resulting crude product was purified by Prep-HPLC to give methyl (1*R*,4*R*)-4-(4-(((*R*)-1-(4-(2-((dimethylamino)methyl)phenyl)thiophen-2-yl)ethyl)amino)-7-methoxy-2-methylquinazolin-6-yl)cyclohexane-1-carboxylate.

LC-MS: (ESI, *m*/*z*): [M+H]⁺=573.3

¹H NMR (400 MHz, CDCl₃) δ 7.44-7.34 (m, 3H), 7.33-7.28 (m, 4H), 7.12 (s, 1H), 6.11-6.02 (m, 1H), 5.69-5.60 (m, 1H), 3.92 (s, 3H), 3.69 (s, 3H), 3.38 (s, 2H), 3.04-2.98 (m, 1H), 2.63 (s, 3H), 2.44-2.33 (m, 1H), 2.24-2.18 (m, 6H), 2.16-2.07 (m, 2H), 2.05-1.95 (m, 2H), 1.82-1.79 (d, *J=* 6.8 Hz, 3H), 1.67-1.60 (m, 4H).

### Intermediate 22: tert-butyl 4-(3-bromoprop-1-yn-1-yl)piperidine-1-carboxylate

### Step 1: preparation of tert-butyl 4-(2,2-dibromovinyl)piperidine-1-carboxylate

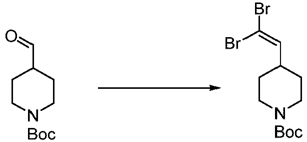

PPh₃ (22.13 g, 84.48 mmol) was added to a solution of CBr₄ (14.0 g, 42.24 mmol) in DCM (200 mL) while stirring at 0 °C, and the resulting mixture was stirred at 0 °C for another 30 min, followed by the addition of *tert*-butyl 4-formylpiperidine-1-carboxylate (6.0 g, 28.16 mmol). The reaction liquid was stirred at room temperature overnight, diluted with EA (200 mL), and filtered. The filtrate was concentrated under reduced pressure. The resulting crude product was purified by flash column chromatography (EA:PE = 0-3:7) to give *tert-butyl* 4-(2,2-dibromovinyl)piperidine-1 -carboxylate.

LC-MS: (ESI, m/z): [M-tBu]⁺=313.9.

### Step 2: preparation of tert-butyl 4-(3-hydroxyprop-1-yn-1-yl)piperidine-1-carboxylate

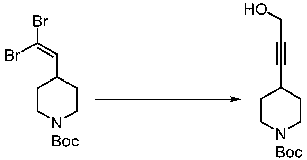

*n*-BuLi (13.6 mL, 1.6 M/*n*-hexane) was added to a solution of *tert*-butyl 4-(2,2-dibromovinyl)piperidine-1-carboxylate (4.0 g, 10.86 mmol) in THF (60 mL) at -78 °C. The resulting mixture was stirred at -78 °C for 1 h, and then paraformaldehyde (977 mg, 32.60 mmol) was added. The reaction liquid was stirred at room temperature overnight. NH₄Cl (100 mL) was added to the reaction liquid to quench the reaction, and the resulting mixture was extracted with EA (100 mL × 3). The organic phases were combined, washed with saturated brine (300 mL), dried over anhydrous Na₂SO₄, filtered, and concentrated. The resulting crude product was purified by flash column chromatography (EA:PE = 0-4:1) to give *tert*-butyl 4-(3-hydroxyprop-1-yn-1-yl)piperidine-1-carboxylate. LC-MS: (ESI, m/z): [M-Boc]⁺=140.2.

¹H NMR (400 MHz, DMSO-*d*₆) δ 5.04 (t, *J* = 5.9 Hz, 1H), 4.04 (dd, *J* = 5.8, 2.0 Hz, 2H), 3.65 - 3.55 (m, 2H), 3.04 (t, *J* = 10.1 Hz, 2H), 2.60 (ddd, *J* = 8.8, 7.0, 3.5 Hz, 1H), 1.77 -1.64 (m, 2H), 1.43 - 1.31 (m, 11H).

### Step 3: preparation of tert-butyl 4-(3-bromoprop-1-yn-1-yl)piperidine-1-carboxylate

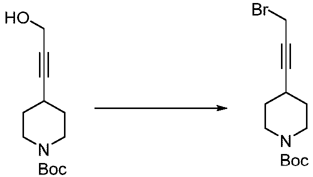

CBr₄ (1.94 g, 5.83 mmol) was added to a solution of *tert*-butyl 4-(3-hydroxyprop-1-yn-1-yl)piperidine-1-carboxylate (700 mg, 2.92 mmol) and PPh₃ (1.94 g, 5.83 mmol) in DCM (10 mL) while stirring at 0 °C. The reaction liquid was stirred at room temperature overnight. The reaction liquid was diluted with EA (10 mL) and then filtered. The filtrate was concentrated under reduced pressure. The resulting crude product was purified by flash column chromatography (EA:PE = 0-3:7) to give *tert*-butyl 4-(3-bromoprop-1-yn-1-yl)piperidine-1-carboxylate.

LC-MS: (ESI, m/z): [M-tBu]⁺=246.0.

¹H NMR (400 MHz, DMSO-*d*₆) δ 4.24 (d, *J* = 2.0 Hz, 2H), 3.64 - 3.54 (m, 2H), 3.13 - 3.00 (m, 2H), 2.75 - 2.65 (m, 1H), 1.78 - 1.68 (m, 2H), 1.44 - 1.33 (m, 11H).

### Intermediate 23: tert-butyl 4-(2-(piperidin-4-ylmethoxy)ethyl)piperidine-1-carboxylate

### Step 1: preparation of tert-butyl 4-(2-(pyridin-4-ylmethoxy)ethyl)piperidine-1-carboxylate

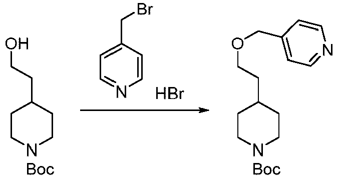

NaH (60%, 1.2 g, 30.58 mmol) was added to a solution of *tert*-butyl 4-(2-hydroxyethyl)piperidine-1-carboxylate (1.0 g, 4.36 mmol) in THF (20 mL) at 0 °C, and the resulting mixture was stirred at 0 °C for 20 min. After warming to room temperature, 4-(bromomethyl)pyridine hydrogen bromide (1.3 g, 5.13 mmol) was added, and the reaction mixture was stirred at room temperature for reaction overnight. Water (30 mL) was added to the reaction liquid, and the resulting mixture was extracted with EA (30 mL × 3). The organic phases were combined, washed with brine (30 mL), dried over Na₂SO₄, and concentrated under reduced pressure. The concentrate was purified by flash column chromatography (EA:PE = 0-4:1) to give the target product *tert*-butyl 4-(2-(pyridin-4-ylmethoxy)ethyl)piperidine-1-carboxylate.

LC-MS: (ESI, *m*/*z):* [M+H]⁺= 321.1.

¹H NMR (400 MHz, CDCl₃) δ 8.57 (d, *J=* 5.9 Hz, 2H), 7.24 (d, *J=* 5.7 Hz, 2H), 4.51 (s, 2H), 4.09 (d, *J=* 15.9 Hz, 2H), 3.55 (t, *J* = 6.1 Hz, 2H), 2.70 (t, *J* = 12.2 Hz, 2H), 1.66 (d, *J* = 13.9 Hz, 2H), 1.58 (m, 2H), 1.45 (s, 9H), 1.26 (m, 1H), 1.13 (d, *J* = 8.4 Hz, 2H).

### Step 2: preparation of tert-butyl 4-(2-(piperidin-4-ylmethoxy)ethyl)piperidine-1-carboxylate

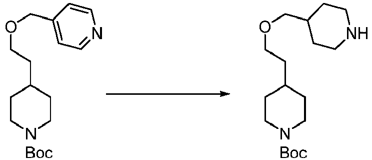

A mixture of *tert*-butyl 4-(2-(pyridin-4-ylmethoxy)ethyl)piperidine-1-carboxylate (1.9 g, 5.9 mmol) and Pd/C (570 mg) in i-PrOH/H₂O (30 mL/35 mL) was stirred at 75 °C overnight. The reaction liquid was filtered through celite, and the filtrate was concentrated to give the target product *tert*-butyl 4-(2-(piperidin-4-ylmethoxy)ethyl)piperidine-1-carboxylate. The resulting product was directly used in the next step without purification.

LC-MS: (ESI, *m*/*z):* [M+H]⁺= 327.2.

### Intermediate 24: 2-(1-(2-(2,6-dioxopiperidin-3-yl)-1,3-dioxoisoindolin-4-yl)piperidin-4-yl)acetaldehyde

### Step 1: preparation of 2-(2,6-dioxopiperidin-3-yl)-4-(4-(2-hydroxyethyl)piperidin-1-yl)isoindoline-1,3-dione

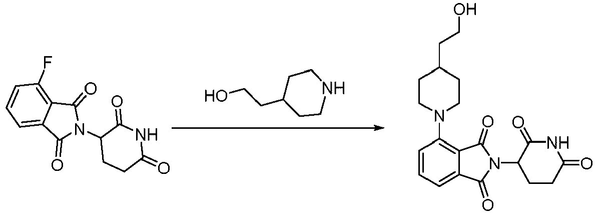

2-(Piperidin-4-yl)ethan-1-ol (467 mg, 3.6 mmol) and DIEA (1.4 g, 10.8 mmol) were added to a solution of 2-(2,6-dioxopiperidin-3-yl)-4-fluoroisoindoline-1,3-dione (1.0 g, 3.6 mmol) in DMF (10 mL). The reaction liquid was stirred at 100 °C overnight. Water (100 mL) was added to the reaction liquid to quench the reaction, and then the resulting mixture was extracted with EA (20 mL × 3). The organic phases were combined, washed with saturated brine (60 mL), dried over anhydrous Na₂SO₄, filtered, and concentrated under reduced pressure. The resulting crude product was purified by flash column chromatography (EA:PE = 0-1:1) to give 2-(2,6-dioxopiperidin-3-yl)-4-(4-(2-hydroxyethyl)piperidin-1 -yl)isoindoline-1,3-dione.

LC-MS: (ESI, *m*/*z*): [M+H]⁺=386.1.

### Step 2: preparation of 2-(1-(2-(2,6-dioxopiperidin-3-yl)-1,3-dioxoisoindolin-4-yl)piperidin-4-yl)acetaldehyde

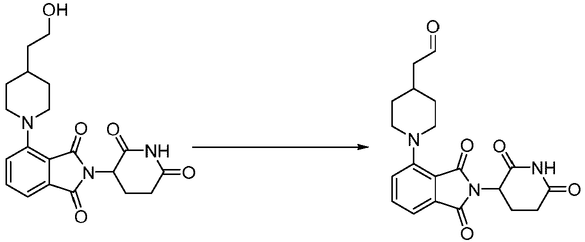

IBX (1.7 g, 6.0 mmol) was added to a solution of 2-(2,6-dioxopiperidin-3-yl)-4-(4-(2-hydroxyethyl)piperidin-1-yl)isoindoline-1,3-dione (1.1 g, 3.0 mmol) in ACN (20 mL) while stirring. The reaction liquid was stirred at 80 °C for 2 h. The reaction liquid was filtered, and the filtrate was concentrated. The resulting crude product was purified by flash column chromatography (EA:PE = 0-1:1) to give 2-(1-(2-(2,6-dioxopiperidin-3-yl)-1,3-dioxoisoindolin-4-yl)piperidin-4-yl)acetaldehyde.

LC-MS: (ESI, *m*/*z*): [M+H]⁺=384.0.

¹H NMR (400 MHz, DMSO-*d*₆) δ 11.08 (s, 1H), 9.72 (s, 1H), 7.77 - 7.58 (m, 1H), 7.34 (dd, *J* = 7.7, 4.9 Hz, 2H), 5.09 (dd, *J* = 12.9, 5.4 Hz, 1H), 3.68 (d, *J* = 11.8 Hz, 2H), 2.97-2.82 (m, 3H), 2.64 - 2.53 (m, 2H), 2.45 (d, J = 6.6 Hz, 2H), 2.13 - 1.99 (m, 3H), 1.82-1.72 (m, 2H), 1.50-1.30 (m, 2H).

### Intermediate 25: 3-(1-(2-(2,6-dioxopiperidin-3-yl)-1,3-dioxoisoindolin-4-yl)piperidin-4-yl)propionaldehyde

### Step 1: preparation of 3-(piperidin-4-yl)propan-1-ol

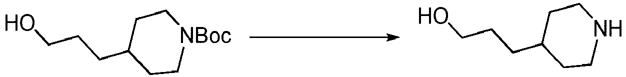

TFA (4.0 mL) was added to *tert*-butyl 4-(3-hydroxypropyl)piperidine-1-carboxylate (930.0 mg, 3.8 mmol) in DCM (8.00 mL). The reaction liquid was stirred at room temperature overnight. The reaction liquid was directly concentrated to give 3-(piperidin-4-yl)propan-1-ol. The resulting crude product was directly used in the next step. LC-MS: (ESI, *m*/*z*): [M+H]⁺=144.2.

### Step 2: preparation of 2-(2,6-dioxopiperidin-3-yl)-4-(4-(3-hydroxypropyl)piperidin-1-yl)isoindoline-1,3-dione

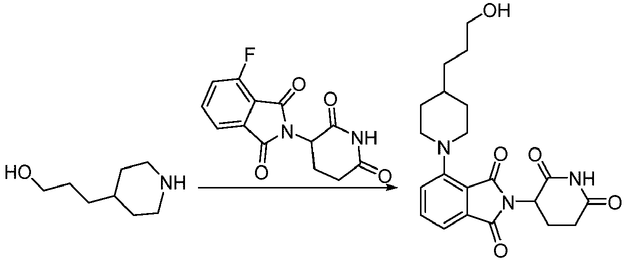

2-(2,6-Dioxopiperidin-3-yl)-4-fluoroisoindoline-1,3-dione (1.0 g, 3.8 mmol) and DIEA (1.5 g, 11.4 mmol) were added to a solution of 3-(piperidin-4-yl)propan-1-ol (730 mg, TFA salt, 3.8 mmol) in DMF (10 mL) while stirring. The reaction liquid was stirred at 100 °C overnight. Water (100 mL) was added to the reaction liquid to quench the reaction, and then the resulting mixture was extracted with EA (20 mL × 3). The organic phase was washed with saturated brine (60 mL), dried over anhydrous Na₂SO₄, filtered, and concentrated. The resulting crude product was purified by flash column chromatography (EA:PE = 0-1:1) to give 2-(2,6-dioxopiperidin-3-yl)-4-(4-(3-hydroxypropyl)piperidin-1-yl)isoindoline-1,3-dione.

LC-MS: (ESI, *m*/*z*): [M+H]⁺=400.0.

### Step 3: preparation of 3-(1-(2-(2,6-dioxopiperidin-3-yl)-1,3-dioxoisoindolin-4-yl)piperidin-4-yl)propionaldehyde

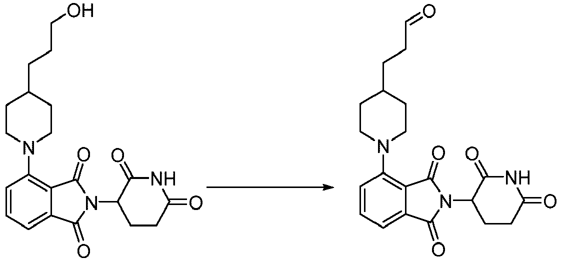

IBX (982 mg, 3.5 mmol) was added to a solution of 2-(2,6-dioxopiperidin-3-yl)-4-(4-(3-hydroxypropyl)piperidin-1-yl)isoindoline-1,3-dione (700 mg, 1.7 mmol) in ACN (20 mL) while stirring. The reaction liquid was stirred at 80 °C for 2 h. The reaction liquid was filtered, and the filtrate was concentrated. The resulting crude product was purified by flash column chromatography (EA:PE = 0-1:1) to give 3-(1-(2-(2,6-dioxopiperidin-3-yl)-1,3-dioxoisoindolin-4-yl)piperidin-4-yl)propionaldehyde.

LC-MS: (ESI, *m*/*z*): [M+H]⁺=398.0.

¹H NMR (400 MHz, DMSO-*d*₆) δ 11.08 (s, 1H), 9.71 (t, *J* = 1.4 Hz, 1H), 7.74 - 7.60 (m, 1H), 7.33 (dd, *J=* 7.7, 3.4 Hz, 2H), 5.09 (dd, *J* = 12.9, 5.4 Hz, 1H), 3.69 (d, *J=* 11.9 Hz, 2H), 2.93-2.79 (m, 3H), 2.64 - 2.46 (m, 4H), 2.07 - 2.00 (m, 1H), 1.80-1.72 (m, 2H), 1.54 (dd, J = 14.0, 7.1 Hz, 2H), 1.40-1.27 (m, 3H).

### Intermediate 26: benzyl 9-(piperazin-1-ylmethyl)-3-azaspiro[5.5]undecane-3-carboxylate

### Step 1: preparation of tert-butyl 9-((4-(((9H-fluoren-9-yl)methoxy)carbonyl)piperazin-1-yl)methyl)-3-azaspiro [5.5]undecane-3 -carboxylate

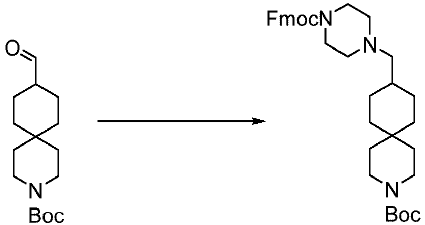

*tert*-Butyl 9-formyl-3-azaspiro[5.5]undecane-3-carboxylate (1.0 g, 3.56 mmol) and (9*H*-fluoren-9-yl)methylpiperazine-1-carboxylate (1.35 g, 3.91 mmol) were dissolved in THF (20 mL), and STAB (2.26 g, 10.68 mmol) was added. The resulting mixture was stirred at room temperature overnight, and water (100 mL) was added, followed by extraction with ethyl acetate (100 mL × 3). The organic phase was washed with saturated brine (150 mL), dried over anhydrous sodium sulfate, filtered, and concentrated under reduced pressure. The crude product was purified by column chromatography (EA/PE = 3:2) to give *tert*-butyl 9-((4-(((9*H*-fluoren-9-yl)methoxy)carbonyl)piperazin-1-yl)methyl)-3-azaspiro[5.5]undecane-3-carboxylate.

LC-MS: (ESI, *m*/*z):* [M+H]⁺=574.2.

### Step 2: preparation of (9H-fluoren-9-yl)methyl 4-((3-azaspiro[5.5]undecane-9-yl)methyl)piperazine-1-carboxylate

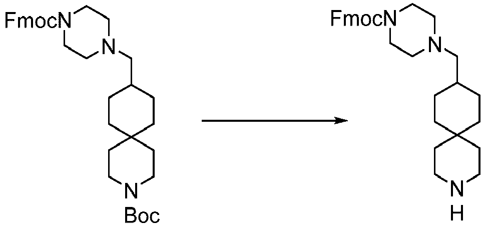

*tert*-Butyl 9-((4-(((9*H*-fluoren-9-yl)methoxy)carbonyl)piperazin-1-yl)methyl)-3-azaspiro[5.5]undecane-3-carboxylate (1.1 g, 1.92 mmol) was dissolved in DCM (10 mL), and TFA (2 mL) was added. The resulting mixture was stirred at room temperature for 1 h and concentrated under reduced pressure to give (9*H*-fluoren-9-yl)methyl 4-((3-azaspiro[5.5]undecane-9-yl)methyl)piperazine-1-carboxylate, which was directly used in the next step.

LC-MS: (ESI, *m*/*z*): [M+H]⁺=474.1.

### Step 3: preparation of benzyl 9-((4-(((9H-fluoren-9-yl)methoxy)carbonyl)piperazin-1-yl)methyl)-3-azaspiro [5.5]undecane-3 -carboxylate

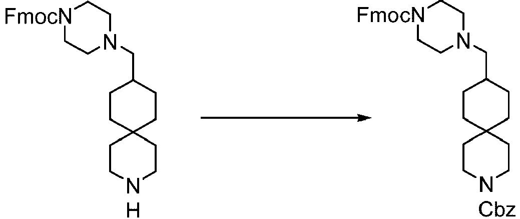

(9*H*-Fluoren-9-yl)methyl 4-((3-azaspiro[5.5]undecane-9-yl)methyl)piperazine-1-carboxylate (1.1 g, 2.33 mmol) was dissolved in ACN (20 mL), and a saturated NaHCO₃ solution (20 mL) was added, followed by the addition of CbzCl (0.60 g, 3.50 mmol) while stirring. The resulting mixture was reacted at room temperature overnight and then concentrated under reduced pressure to remove acetonitrile. Water (50 mL) was added, and the resulting mixture was extracted with ethyl acetate (100 mL × 3). The organic phase was washed with saturated brine (150 mL), dried over anhydrous sodium sulfate, filtered, and concentrated under reduced pressure. The crude product was purified by column chromatography (EA/PE =11:9) to give benzyl 9-((4-(((9*H*-fluoren-9-yl)methoxy)carbonyl)piperazin-1-yl)methyl)-3-azaspiro[5.5]undecane-3-carboxylate.

LC-MS: (ESI, *m*/*z*): [M+H]⁺=608.1.

### Step 4: preparation of benzyl 9-(piperazin-1-ylmethyl)-3-azaspiro[5.5]undecane-3-carboxylate

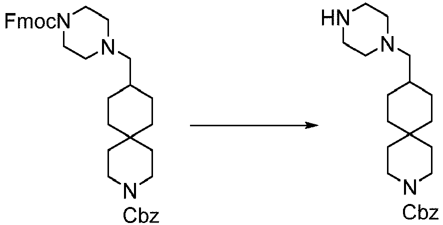

Benzyl 9-((4-(((9*H*-fluoren-9-yl)methoxy)carbonyl)piperazin-1-yl)methyl)-3 -azaspiro [5.5]undecane-3 -carboxylate (0.97 g, 1.60 mmol) was dissolved in CAN (10 mL), and piperidine (2 mL) was added. The resulting mixture was stirred at room temperature for 3 h and then concentrated under reduced pressure to remove the solvent. Water (50 mL) was added, and the resulting mixture was extracted with ethyl acetate (100 mL × 3). The organic phase was washed with saturated brine (150 mL), dried over anhydrous sodium sulfate, filtered, and concentrated under reduced pressure. The crude product was purified by column chromatography (MeOH/DCM = 3:17) to give benzyl 9-(piperazin-1 -ylmethyl)-3-azaspiro[5.5]undecane-3-carboxylate.

LC-MS: (ESI, *m*/*z):* [M+H]⁺=386.1.

### Intermediate 27: methyl 4-(4-hydroxy-7-methoxy-2-methylquinazolin-6-yl)cyclohex-3-ene-1-carboxylate

### Step 1: preparation of methyl 4-(((trifluoromethoxy)sulfonyl)oxy)cyclohex-3-ene-1-carboxylate

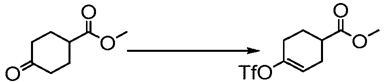

2,6-Dimethyl-1λ²-piperazine (51.4 g, 480.37 mmol) and Tf₂O (90.38 g, 320.50 mmol) were added to a solution of methyl 4-oxocyclohexane-1-carboxylate (50.0 g, 320.51 mmol) in dichloromethane (500 mL). The resulting mixture was stirred at 0 °C for 1 h, and then Tf₂O (72.3 g, 256.38 mmol) was added. The mixture was stirred at room temperature overnight, and the organic phase was washed with water, dried over anhydrous sodium sulfate, and concentrated under reduced pressure. The crude product was subjected to column chromatography (PE:EA = 5:1) to give methyl 4-(((trifluoromethoxy)sulfonyl)oxy)cyclohex-3-ene-1-carboxylate.

¹H NMR (400 MHz, DMSO-*d*₆) δ 5.90 (s, 1H), 3.63 (s, 3H), 2.70 - 2.60 (m, 1H), 2.47 - 2.28 (m, 4H), 2.11 - 1.98 (m, 1H), 1.81 (tt, *J* = 8.9, 6.0 Hz, 1H).

### Step 2: preparation of methyl 4-(4,4,5,5-tetramethyl-1,3,2-dioxaborolan-2-yl)cyclohex-3-ene-1-carboxylate

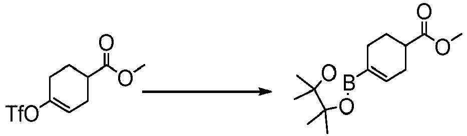

(Bpin)₂ (39.1 g, 153.94 mmol), Pd(dppf)₂Cl₂(7.5 g, 10.24 mmol), and potassium acetate (30.4 g, 310.2 mmol) were added to a solution of methyl 4-(((trifluoromethoxy)sulfonyl)oxy)cyclohex-3-ene-1-carboxylate (30.0 g, 104.17 mmol) in dioxane (500 mL), and the resulting mixture was stirred at 90 °C overnight. The reaction liquid was diluted with water (2 L), and the resulting mixture was extracted with ethyl acetate (500 mL × 3). The organic phase was washed with saturated brine (500 mL), dried over anhydrous sodium sulfate, and concentrated under reduced pressure. The crude product was purified by column chromatography (PE:EA = 0:100) to give methyl 4-(4,4,5,5-tetramethyl-1,3,2-dioxaborolan-2-yl)cyclohex-3 -ene-1 -carboxylate.

¹H NMR (400 MHz, CDCl₃) δ 6.53 (s, 1H), 3.71 - 3.64 (m, 3H), 2.61 - 2.46 (m, 1H), 2.42 - 1.95 (m, 6H), 1.28 - 1.19 (m, 12H).

### Step 3: preparation of methyl 4-(4-hydroxy-7-methoxy-2-methylquinazolin-6-yl)cyclohex-3-ene-1-carboxylate

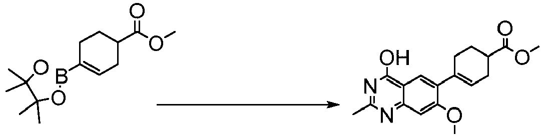

Methyl 4-(4,4,5,5-tetramethyl-1,3,2-dioxaborolan-2-yl)cyclohex-3-ene-1-carboxylate (25.0 g, 93.98 mmol), Pd(dppf)₂Cl₂ (5.3 g, 7.24 mmol), and Na₂CO₃ (15.3 g, 144.34 mmol) were added to a solution of 6-bromo-7-methoxy-2-methylquinazolin-4-ol (19.4 g, 72.12 mmol) in DMF/H₂O (300 mL/30 mL), and the resulting mixture was stirred at 110 °C overnight. The reaction liquid was diluted with water (1 L), and the resulting mixture was extracted with ethyl acetate (500 mL × 3). The organic phase was washed with saturated brine (500 mL), dried over anhydrous sodium sulfate, filtered, and concentrated under reduced pressure. The crude product was purified by column chromatography (PE:EA = 0:100) to give the product methyl 4-(4-hydroxy-7-methoxy-2-methylquinazolin-6-yl)cyclohex-3-ene-1-carboxylate.

LC-MS: (ESI, *m*/*z*): [M+H]⁺=329.0.

### Intermediate 28: methyl (1R,4R)-4-(7-methoxy-2-methyl-4-(((R)-1-(4-(2-((methylamino)methyl)phenyl)thiophen-2-yl)ethyl)amino)quinazolin-6-yl)cyclohexane-1 -carboxylate

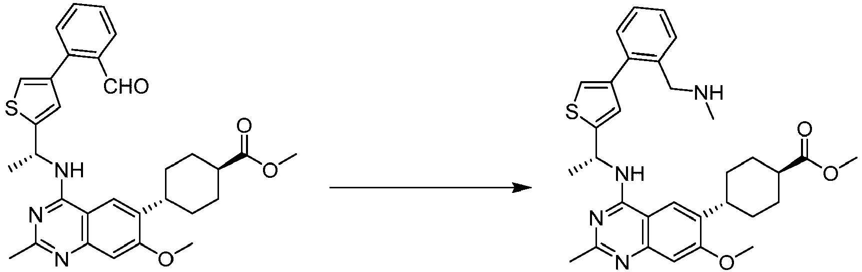

Methyl (1*R*,4*R*)-4-(4-(((*R*)-1-(4-(2-formylphenyl)thiophen-2-yl)ethyl)amino)-7-methoxy-2-methylquinazolin-6-yl)cyclohexane-1-carboxylate (180 mg, 0.33 mmol) and a methylamine solution (0.33 mL, 0.66 mmol, 2 M in THF) were added to MeOH/DCE (2 mL/2 mL), and then a few drops of AcOH (4 drops) were added dropwise. The mixed solution was stirred at room temperature for 1 h, and then NaBH₃CN (42 mg, 0.66 mmol) was added. The resulting mixture was stirred for reaction for another 3 h. DCM and water were added to the reaction liquid, and the organic phase was isolated, dried over anhydrous Na₂SO₄, filtered, and concentrated under reduced pressure. The crude product was purified by Prep-HPLC to give methyl (1R,4R)-4-(7-methoxy-2-methyl-4-(((*R*)-1-(4-(2-((methylamino)methyl)phenyl)thiophen-2-yl)ethyl)amino)quinazolin-6-yl)cyclohexane-1-carboxylate.

LC-MS: (ESI, *m*/*z):* [M+H] ⁺=559.4.

¹H NMR (400 MHz, CDCl₃) δ 7.42-7.39 (m, 1H), 7.35-7.26 (m, 5H), 7.24-7.20 (m, 1H), 7.10 (s, 1H), 6.08-6.01 (m, 1H), 5.65-5.60 (m, 1H), 3.92 (s, 3H), 3.73-3.69 (m, 5H), 3.04-2.98 (m, 1H), 2.63 (s, 3H), 2.44-2.36 (m, 4H), 2.14-1.99 (m, 4H), 1.80 (d, *J=* 6.8 Hz, 3H), 1.70-1.53 (m, 4H).

### Example 1: 1-(5-(9-(2-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)ethyl)-3-azaspiro[5.5]undecane-3-carbonyl)-2-chlorophenyl)dihydropyrimidine-2,4(1H,3H)-dione

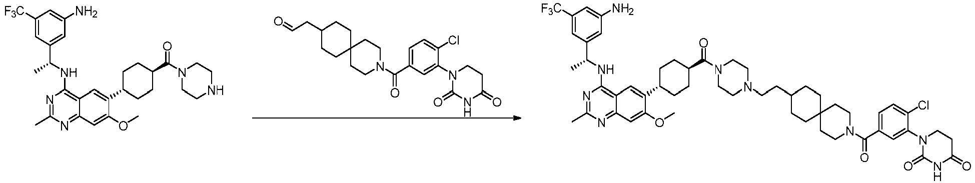

A mixture of ((1*R*,4*R*)-4-(4-(((*R*)-1-(3-amino-5-(trifluoromethyl)phenyl)ethyl)amino)-7-methoxy-2-methylquinazolin-6-yl)cyclohexyl)(piperazin-1-yl)methanone (50 mg, 0.09 mmol), 2-(3-(4-chloro-3-(2,4-dioxotetrahydropyrimidin-1(2*H*)-yl)benzoyl)-3-azaspiro[5.5]undecane-9-yl)acetaldehyde (39 mg, 0.09 mmol), and STAB (55 mg, 0.27 mmol) in a THF (2 mL) solution was stirred at 25 °C for reaction for 1 h. Water (50 mL) was added to quench the reaction, and the resulting mixture was extracted with EA (50 mL × 3). The organic phase was collected, dried over anhydrous sodium sulfate, filtered, and concentrated under reduced pressure. The concentrate was purified by preparative HPLC (acetonitrile/0.08% aqueous ammonium bicarbonate solution, 5% to 95%) to give 1-(5-(9-(2-(4-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperazin-1-yl)ethyl)-3-azaspiro [5.5]undecane-3 -carbonyl)-2-chlorophenyl)dihydropyrimidine-2,4(1*H*,3*H*)-dione.

LC-MS: (ESI, *m*/*z*): [M+H]⁺= 1000.1.

¹H NMR (400 MHz, DMSO-d₆) δ 10.51 (s, 1H), 8.14 - 8.03 (m, 2H), 7.64 (d, *J=* 8.2 Hz, 1H), 7.55 (d, *J =* 1.6 Hz, 1H), 7.39 (d, *J =* 8.2 Hz, 1H), 7.00 (s, 1H), 6.88 (s, 1H), 6.86 (s, 1H), 6.70 (s, 1H), 5.62 - 5.49 (m, 3H), 3.89 (s, 3H), 3.80 - 3.70 (m, 1H), 3.67 - 3.42 (m, 7H), 3.31 - 3.23 (m, 2H), 2.99 - 2.88 (m, 1H), 2.78 - 2.59 (m, 3H), 2.40 - 2.25 (m, 9H), 1.94 - 1.76 (m, 4H), 1.74 - 1.20 (m, 18H), 1.16 - 0.98 (m, 4H).

### Example 2: 1-(5-(9-((4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carbonyl)-2-chlorophenyl)dihydropyrimidine-2,4(1H,3H)-dione

### Step 1: preparation of tert-butyl 9-((4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carboxylate

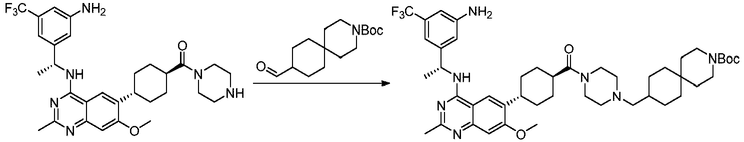

STAB (107 mg, 0.5 mmol) was slowly added to a mixture of ((1*R*,4*R*)-4-(4-(((*R*)-1-(3-amino-5-(trifluoromethyl)phenyl)ethyl)amino)-7-methoxy-2-methylquinazolin-6-yl)cyclohexyl)(piperazin-1-yl)methanone (140.0 mg, 0.2 mmol) and *tert*-butyl 9-formyl-3-azaspiro[5.5]undecane-3-carboxylate (100.0 mg, 0.36 mmol) in a THF (6.0 mL) solution. The reaction mixture was stirred at room temperature for reaction overnight. Water (20.0 mL) was added to quench the reaction, and the resulting mixture was extracted with EA (20.0 mL × 2). The organic phase was collected, dried over anhydrous sodium sulfate, and concentrated under reduced pressure to give the target product. The crude product could be directly used in the next step.

LC-MS: (ESI, *m*/*z*): [M+H]⁺=836.2.

### Step 2: (4-(((3-azaspiro[5.5]undecane-9-yl)methyl)piperazin-1-yl)((1R,4R)-4-(4-(((R)-1-(3-amino-5-(trifluoromethyl)phenyl)ethyl)amino)-7-methoxy-2-methylquinazolin-6-yl)cyclohexyl)methanone

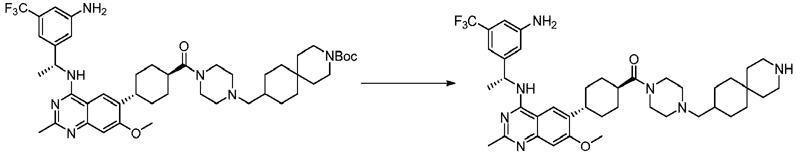

A mixture of *tert*-butyl 9-((4-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carboxylate (140 mg, 0.2 mmol) in a TFA/DCM (3.0 mL, 1:5) solution was stirred at room temperature for 2 h. The reactant was concentrated under reduced pressure to give the target product. The crude product was directly used in the next step.

LC-MS: (ESI, *m*/*z):* [M+H]⁺= 736.2.

### Step 3: preparation of 1-(5-(9-((4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carbonyl)-2-chlorophenyl)dihydropyrimidine-2,4(1H,3H)-dione

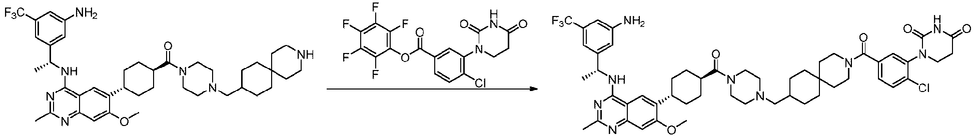

DIEA (54.2 mg, 0.42 mmol) and pentafluorophenyl 4-chloro-3-(2,4-dioxotetrahydropyrimidin-1(2*H*)-yl)benzoate (73.0 mg, 0.17 mmol) were added to a mixture of (4-(((3-azaspiro[5.5]undecane-9-yl)methyl)piperazin-1-yl)((1*R*,4*R*)-4-(4-(((*R*)-1-(3-amino-5-(trifluoromethyl)phenyl)ethyl)amino)-7-methoxy-2-methylquinazolin-6-yl)cyclohexyl)methanone (100 mg, 0.14 mmol) in a DMSO (5.0 mL) solution, and the reaction mixture was stirred at room temperature for reaction for 5.0 h. Water (15.0 mL) was added to quench the reaction, and the resulting mixture was extracted with EA (20 mL × 3). The organic phase was collected, washed with saturated brine (20.0 mL), dried over anhydrous sodium sulfate, and concentrated under reduced pressure. The concentrate was purified by preparative HPLC to give the target product.

LC-MS: (ESI, m/z): [M+H]⁺=986.4.

¹H NMR (400 MHz, DMSO-*d*₆) δ 10.51 (s, 1H), 8.16 - 8.02 (m, 2H), 7.63 (d, *J* = 8.2 Hz, 1H), 7.54 (s, 1H), 7.38 (d, *J=* 8.2 Hz, 1H), 6.99 (s, 1H), 6.87 (s, 1H), 6.85 (s, 1H), 6.70 (s, 1H), 5.65-5.47 (m, 3H), 3.88 (s, 3H), 3.79-3.71 (m, 1H), 3.67-3.54 (m, 3H), 3.51-3.42 (m, 4H), 2.97-2.90 (m, 1H), 2.78-2.72 (m, 2H), 2.67-2.61 (m, 1H), 2.39-2.23 (m, 7H), 2.15-2.05 (m, 2H), 1.87-1.78 (m, 4H), 1.73 - 1.21 (m, 17H), 1.17-0.98 (m, 4H).

### Example 3: 1-(5-(9-(2-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)ethyl)-3,9-diazaspiro[5.5]undecane-3-carbonyl)-2-chlorophenyl)dihydropyrimidine-2,4(1H,3H)-dione

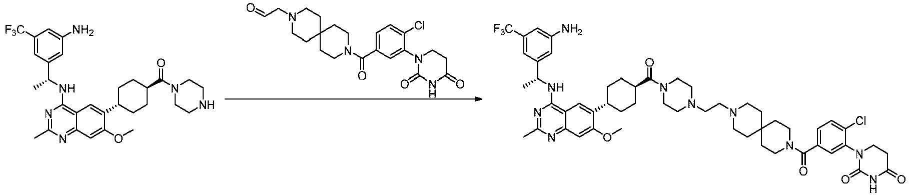

### Example 3 was prepared by reference to the preparation method of Example 2.

LC-MS: (ESI, *m*/*z*): [M+H]⁺= 1001.3.

¹H NMR (400 MHz, DMSO-d₆) δ 10.51 (s, 1H), 8.09-8.06 (m, 2H), 7.64 (d, *J* = 8.1 Hz, 1H), 7.55 (s, 1H), 7.39 (d, *J=* 7.6 Hz, 1H), 7.00 (s, 1H), 6.88 (s, 1H), 6.86 (s, 1H), 6.70 (s, 1H), 5.65 - 5.47 (m, 3H), 3.89 (s, 3H), 3.82 - 3.40 (m, 9H), 3.02 - 2.90 (m, 1H), 2.82 - 2.71 (m, 2H), 2.70 - 2.60 (m, 1H), 2.46 - 2.24 (m, 15H), 1.94 - 1.75 (m, 4H), 1.73 - 1.26 (m, 16H).

### Example 4: (R)-1-(5-(9-((4-(4-(4-((1-(3-amino-5-(trifluoromethyl)phenyl)ethyl)amino)amino)-7-methoxy-2-methylquinazolin-6-yl)piperidine-1-carbonyl)piperidin-1-yl)methyl)-3-azaspiro[5.5]undecane-3-carbonyl)-2-chlorophenyl)dihydropyrimidine-2,4(1H,3H)-dione

### Step 1: preparation of tert-butyl 4-(7-methoxy-2-methyl-4-(((2,4,6-triisopropylphenyl)sulfonyl)oxy)quinazolin-6-yl)-3,6-dihydropyridin-1(2H)-carboxylate

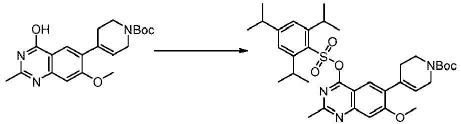

TPSCl (245 mg, 0.809 mmol), DMAP (35 mg, 0.269 mmol), and TEA (163 mg, 1.617 mmol) were added to a mixture of *tert*-butyl 4-(4-hydroxy-7-methoxy-2-methylquinazolin-6-yl)piperidine-1-carboxylate (200 mg, 0.539 mmol) in a DCM (10 mL) solution. The reaction mixture was stirred at 25 °C for reaction overnight. The reactant mixture was diluted with DCM and washed with sodium bicarbonate (a saturated aqueous solution). The organic phase was collected, dried over anhydrous sodium sulfate, filtered, and concentrated under reduced pressure. The concentrate was purified by column chromatography to give the target product.

LC-MS: (ESI, *m*/*z):* [M+H]⁺=638.2.

### Step 2: preparation of tert-butyl (R)-4-(7-methoxy-2-methyl-4-((1-(3-nitro-5-(trifluoromethyl)phenyl)ethyl)amino)quinazolin-6-yl)-3,6-dihydropyridin-1(2H)-carboxylate

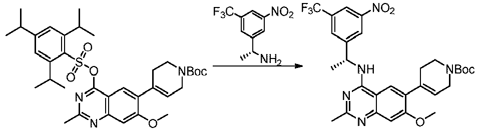

(*R*)-1-(3-Nitro-5-(trifluoromethyl)phenyl)ethan-1-amine (150 mg, 0.414 mmol) and TEA (105 mg, 1.035 mmol) were added to a mixture of *tert*-butyl 4-(7-methoxy-2-methyl-4-(((2,4,6-triisopropylphenyl)sulfonyl)oxy)quinazolin-6-yl)-3,6-dihydropyridin-1(2*H*)-carboxylate (220 mg, 0.345 mmol) in a DCM (5 mL) solution. The reaction mixture was stirred at 90 °C for reaction overnight. Water was added to the reaction mixture to quench the reaction, and the resulting mixture was extracted with EA. The organic phase was collected, dried over anhydrous sodium sulfate, and concentrated under reduced pressure. The concentrate was purified by column chromatography to give the target compound.

LC-MS: (ESI, *m*/*z*): [M+H]⁺=588.2.

### Step 3: preparation of (R)-7-methoxy-2-methyl-N-(1-(3-nitro-5-(trifluoromethyl)phenyl)ethyl)-6-(1,2,3,6-tetrahydropyridin-4-yl)quinazolin-4-amine

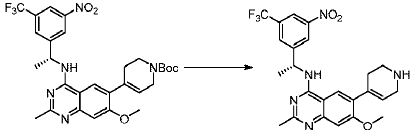

Trifluoroacetic acid (2 mL) was added to a mixture of tert-butyl (*R*)-4-(7-methoxy-2-methyl-4-((1-(3-nitro-5-(trifluoromethyl)phenyl)ethyl)amino)quinazolin-6-yl)-3,6-dihydropyridin-1(2*H*)-carboxylate (200 mg, 0.340 mmol) in a DCM (4 mL) solution. The reaction mixture was stirred at 25 °C for 1.0 h. An aqueous sodium hydroxide solution (40%) was added to the reaction mixture to quench the reaction, and the resulting mixture was extracted with DCM (50 mL × 3). The organic phase was collected, dried over anhydrous sodium sulfate, and concentrated under reduced pressure to give the target product.

LC-MS: (ESI, *m*/*z*): [M+H]⁺=488.2.

### Step 4: preparation of tert-butyl (R)-4-(4-(7-methoxy-2-methyl-4-((1-(3-nitro-5-(trifluoromethyl)phenyl)ethyl)amino)quinazolin-6-yl)-1,2,3,6-tetrahydropyridin-1 -carbonyl)piperidine-1 -formate

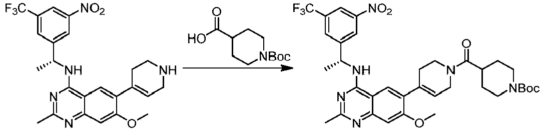

A reaction mixture of (*R*)-7-methoxy-2-methyl-*N*-(1-(3-nitro-5-(trifluoromethyl)phenyl)ethyl)-6-(1,2,3,6-tetrahydropyridin-4-yl)quinazolin-4-amine (10 mg, 0.017 mmol), 1-(*tert*-butoxycarbonyl)piperidine-4-carboxylic acid (9 mg, 0.034 mmol), HATU (10 mg, 0.025 mmol), and DIEA(7 mg, 0.051 mmol) in DMF (1.0 mL) was stirred at 25 °C for reaction overnight. The reaction liquid was concentrated under reduced pressure, and the concentrate was purified by column chromatography to give the target product. The crude product was used in the next step.

LC-MS: (ESI, *m*/*z):* [M+H]⁺= 699.4.

### Step 5: preparation of (R)-(4-(7-methoxy-2-methyl-4-((1-(3-nitro-5-(trifluoromethyl)phenyl)ethyl)amino)quinazolin-6-yl)-3,6-dihydropyridin-1(2H)-yl)(piperidin-4-yl)methanone

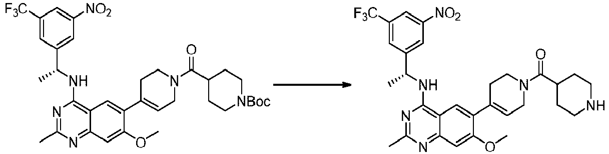

Trifluoroacetic acid (2 mL) was added to a mixture of *tert*-butyl (R)-4-(4-(7-methoxy-2-methyl-4-((1-(3-nitro-5-(trifluoromethyl)phenyl)ethyl)amino)quinazolin-6-yl)-1,2,3,6-tetrahydropyridin-1 -carbonyl)piperidine-1 -formate (200 mg, 0.340 mmol) in a DCM (4 mL) solution. After the reaction mixture was stirred at 25 °C for reaction for 1.0 h, an aqueous NaOH solution (40%) was added to the reaction mixture to quench the reaction, and the resulting mixture was extracted with DCM (50 mL × 3). The organic phase was collected, dried over anhydrous sodium sulfate, and concentrated under reduced pressure to give the target product.

LC-MS: (ESI, *m*/*z*): [M+H]⁺=599.3.

### Step 6: preparation of tert-butyl (R)-9-((4-(4-(7-methoxy-2-methyl-4-((1-(3-nitro-5-(trifluoromethyl)phenyl)ethyl)amino)quinazolin-6-yl)-1,2,3,6-tetrahydropyridin-1 -carbonyl)piperidin-1 - yl)methyl)-3 -azaspiro [5.5]undecane-3 -carboxylate

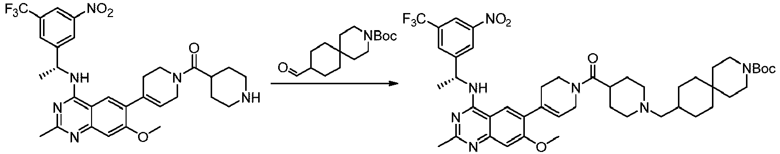

STAB (107 mg, 0.5 mmol) was slowly added to a mixture of (*R*)-(4-(7-methoxy-2-methyl-4-((1-(3-nitro-5-(trifluoromethyl)phenyl)ethyl)amino)quinazolin-6-yl)-3,6-dihydropyridin-1(2*H*)-yl)(piperidin-4-yl)methanone (140.0 mg, 0.2 mmol) and tert-butyl 9-formyl-3-azaspiro[5.5]undecane-3-carboxylate (100.0 mg, 0.36 mmol) in a THF (6.0 mL) solution. The reaction mixture was stirred at room temperature for reaction overnight. Water (20.0 mL) was added to quench the reaction, and the solution was extracted with EA (20.0 mL × 2). The organic phase was collected, dried over anhydrous sodium sulfate, and concentrated under reduced pressure to give the target crude product. The crude product was used in the next step.

LC-MS: (ESI, *m*/*z*): [M+H]⁺=864.5.

### Step 7: preparation of tert-butyl (R)-9-((4-(4-(4-((1-(3-amino-5-(trifluoromethyl)phenyl)ethyl)amino)amino)-7-methoxy-2-methylquinazolin-6-yl)piperidine-1-carbonyl)piperidin-1-yl)methyl)-3-azaspiro[5.5]undecane-3-carboxylate

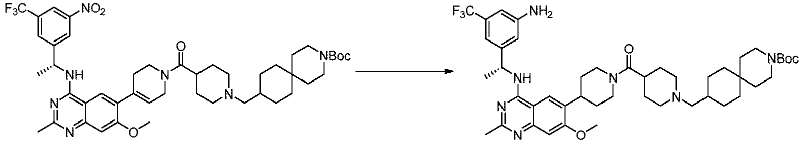

A mixture of *tert*-butyl (*R*)-9-((4-(4-(7-methoxy-2-methyl-4-((1-(3-nitro-5-(trifluoromethyl)phenyl)ethyl)amino)quinazolin-6-yl)-1,2,3,6-tetrahydropyridin-1 -carbonyl)piperidin-1 - yl)methyl)-3-azaspiro[5.5]undecane-3-carboxylate (100.0 mg, 0.19 mmol) and Pd/C (20.0 mg) in a MeOH (5.0 mL) solution was stirred at room temperature under hydrogen atmosphere for reaction overnight. The reaction liquid was filtered, and the filtrate was concentrated to give the target crude product. The crude product was directly used in the next step.

LC-MS: (ESI, *m*/*z):* [M+H]⁺= 836.7.

### Step 8: preparation of (R)-(1-((3-azaspiro[5.5]undecane-9-yl)methyl)piperidin-4-yl)(4-(4-((1-(3-amino-5-(trifluoromethyl)phenyl)ethyl)amino)-7-methoxy-2-methylquinazolin-6-yl)piperidin-1-yl)methanone

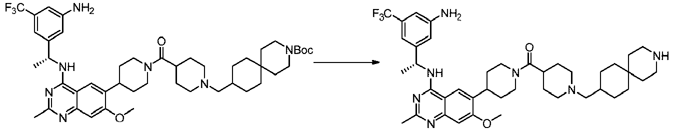

A reaction mixture of *tert*-butyl (*R*)-9-((4-(4-(4-((1-(3-amino-5-(trifluoromethyl)phenyl)ethyl)amino)amino)-7-methoxy-2-methylquinazolin-6-yl)piperidine-1-carbonyl)piperidin-1-yl)methyl)-3-azaspiro[5.5]undecane-3-carboxylate (140 mg, 0.2 mmol) in a mixed solution of TFA/DCM (3.0 mL, 1:5) was stirred at room temperature for reaction for 2.0 h. The reaction liquid was concentrated under reduced pressure to give the target crude product. The crude product was used in the next step.

LC-MS: (ESI, *m*/*z):* [M+H]⁺= 736.7.

### Step 9: preparation of (R)-1-(5-(9-((4-(4-(4-((1-(3-amino-5-(trifluoromethyl)phenyl)ethyl)amino)amino)-7-methoxy-2-methylquinazolin-6-yl)piperidine-1-carbonyl)piperidin-1-yl)methyl)-3-azaspiro[5.5]undecane-3-carbonyl)-2-chlorophenyl)dihydropyrimidine-2,4(1H,3H)-dione

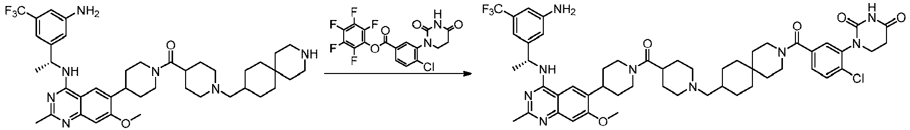

DIEA (54.2 mg, 0.42 mmol) and pentafluorophenyl 4-chloro-3-(2,4-dioxotetrahydropyrimidin-1(2*H*)-yl)benzoate (73.0 mg, 0.17 mmol) were added to a mixture of (*R*)-(1-((3-azaspiro[5.5]undecane-9-yl)methyl)piperidin-4-yl)(4-(4-((1-(3-amino-5-(trifluoromethyl)phenyl)ethyl)amino)-7-methoxy-2-methylquinazolin-6-yl)piperidin-1-yl)methanone (100.0 mg, 0.14 mmol) in a DMSO (5.0 mL) solution, and the reaction mixture was stirred at room temperature for reaction for 3.0 h. Water (15 mL) was added to quench the reaction, and the resulting mixture was extracted with EA (20 mL × 2). The organic phase was collected, washed with saturated brine (20 mL), and dried over anhydrous sodium sulfate. The concentrate was purified by preparative HPLC to give the target product.

LC-MS: (ESI, m/z): [M+H]⁺=986.4

¹H NMR (400 MHz, DMSO-*d*₆) δ 10.51 (s, 1H), 8.14 (d, *J =* 7.3 Hz, 1H), 8.05 (s, 1H), 7.63 (d, *J =* 8.2 Hz, 1H), 7.54 (s, 1H), 7.39 (d, *J* = 8.2 Hz, 1H), 7.02 (s, 1H), 6.87 (s, 1H), 6.84 (s, 1H), 6.69 (s, 1H), 5.62-5.44 (m, 3H), 4.69-4.59 (m, 1H), 4.12-4.01 (m, 1H), 3.90 (s, 3H), 3.82-3.71 (m, 1H), 3.66-3.52 (m, 3H), 3.27-3.10 (m, 3H), 2.86-2.65 (m, 4H), 2.71-2.51 (m, 2H), 2.36 (s, 3H), 2.19-2.04 (m, 2H), 1.93-1.83 (m, 2H), 1.85-7.72 (m, 2H), 1.69-1.42 (m, 16H), 1.41-1.21 (m, 2H), 1.17-0.97 (m, 4H).

### Example 5: (R)-1-(5-(9-((4-(4-((4-((1-(3-amino-5-(trifluoromethyl)phenyl)ethyl)amino)-7-methoxy-2-methylquinazolin-6-yl)oxy)piperidine-1-carbonyl)piperidin-1-yl)methyl)-3-azaspiro[5.5]undecane-3-carbonyl)-2-chlorophenyl)dihydropyrimidine-2,4(1H,3H)-dione

### Step 1: preparation of tert-butyl (R)-4-(4-((7-methoxy-2-methyl-4-((1-(3-nitro-5-(trifluoromethyl)phenyl)ethyl)amino)quinazolin-6-yl)oxy)piperidine-1-carbonyl)piperidine-1-carboxylate

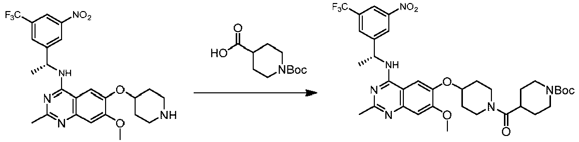

A mixture of (*R*)-1-(5-(9-((4-(4-(4-((1-(3-amino-5-(trifluoromethyl)phenyl)ethyl)amino)amino)-7-methoxy-2-methylquinazolin-6-yl)piperidine-1-carbonyl)piperidin-1-yl)methyl)-3-azaspiro[5.5]undecane-3-carbonyl)-2-chlorophenyl)dihydropyrimidine-2,4(1*H*,3*H*)-dione (140 mg, 0.277 mmol), 1-(*tert*-butoxycarbonyl)piperidine-4-formic acid (70 mg, 0.305 mmol), HATU (126 mg, 0.332 mmol), and DIEA (107 mg, 0.831 mmol) in a DMF (5 mL) solution was stirred at room temperature for reaction overnight. The reaction mixture was poured into water (50 mL), stirred, and filtered. The filter cake was dried to give the target product. The crude product was directly used in the next step.

LC-MS: (ESI, *m*/*z*): [M+H]⁺=717.5.

### Step 2: preparation of (R)-(4-((7-methoxy-2-methyl-4-((1-(3-nitro-5-(trifluoromethyl)phenyl)ethyl)amino)quinazolin-6-yl)oxy)piperidin-1-yl)(piperidin-4-yl)methanone

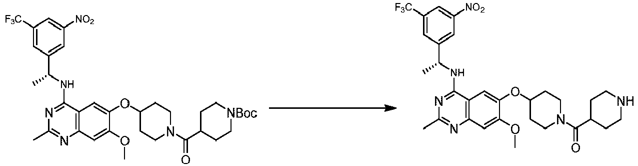

A mixture of *tert*-butyl (*R*)-4-(4-((7-methoxy-2-methyl-4-((1-(3-nitro-5-(trifluoromethyl)phenyl)ethyl)amino)quinazolin-6-yl)oxy)piperidine-1-carbonyl)piperidine-1-carboxylate (170 mg, crude product) in a TFA/DCM (4 mL, 1:3) solution was stirred at room temperature for reaction for 1 h. The reaction mixture was concentrated under reduced pressure to give the target product. The crude product was directly used in the next step.

LC-MS: (ESI, *m*/*z*): [M+H]⁺= 617.3.

### Step 3: Preparation of tert-butyl (R)-9-((4-(4-((7-methoxy-2-methyl-4-((1-(3-nitro-5-(trifluoromethyl)phenyl)ethyl)amino)quinazolin-6-yl)oxy)piperidine-1-carbonyl)piperidin-1-yl)methyl)-3 - azaspiro [5.5]undecane-3 -carboxylate

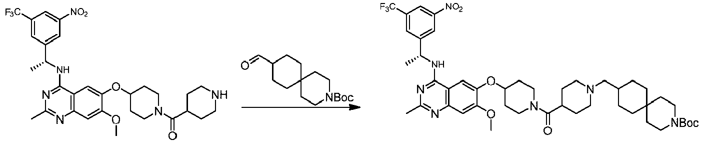

A reaction mixture of (*R*)-(4-((7-methoxy-2-methyl-4-((1-(3-nitro-5-(trifluoromethyl)phenyl)ethyl)amino)quinazolin-6-yl)oxy)piperidin-1 -yl)(piperidin-4-yl)methanone (180 mg, crude product), *tert*-butyl 9-formyl-3-azaspiro[5.5]undecane-3-carboxylate (70 mg, 0.249 mmol), and STAB (240 mg, 1.13 mmol) in a THF (5 mL) solution was stirred at room temperature for reaction overnight. Water (50 mL) was added to quench the reaction, and the resulting mixture was extracted with EA (50 mL × 3). The organic phase was collected, washed with water (100 mL × 2) and saturated brine (100 mL), dried over anhydrous sodium sulfate, filtered, and concentrated. The concentrate was purified by column chromatography to give the target product.

LC-MS: (ESI, *m*/*z):* [M+H]⁺= 882.5.

### Step 4: preparation of (R)-(1-((3-azaspiro[5.5]undecane-9-yl)methyl)piperidin-4-yl)(4-((7-methoxy-2-methyl-4-((1-(3-nitro-5-(trifluoromethyl)phenyl)ethyl)amino)quinazofin-6-yl)oxy)piperidin-1-yl)methanone

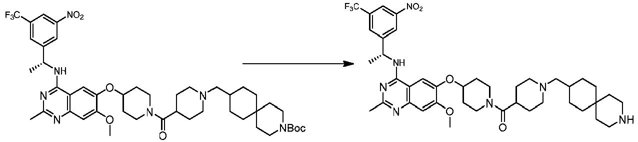

A reaction mixture of *tert*-butyl (*R*)-9-((4-(4-((7-methoxy-2-methyl-4-((1-(3-nitro-5-(trifluoromethyl)phenyl)ethyl)amino)quinazolin-6-yl)oxy)piperidine-1-carbonyl)piperidin-1-yl)methyl)-3-azaspiro[5.5]undecane-3-carboxylate (182 mg, 0.206 mmol) in a solution of DCM (4 mL) and TFA (1 mL) was stirred at 25 °C for reaction for 2 h. The reactant was concentrated under reduced pressure to give the target product. LC-MS: (ESI, *m*/*z*): [M+H]⁺=782.50.

### Step 5: preparation of (R)-1-(2-chloro-5-(9-((4-(4-((7-methoxy-2-methyl-4-((1-(3-nitro-5-(trifluoromethyl))phenyl)ethyl)amino)quinazolin-6-yl)oxy)piperidine-1-carbonyl)piperidin-1-yl)methyl)-3 - azaspiro[5.5]undecane-3-carbonyl)phenyl)dihydropyrimidine-2,4(1H,3H)-dione

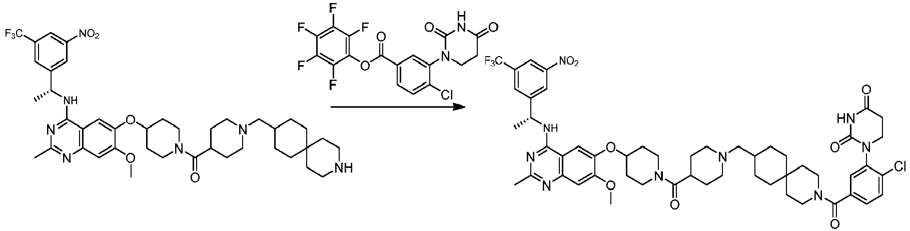

DIEA (0.1 mL) and pentafluorophenyl 4-chloro-3-(2,4-dioxotetrahydropyrimidin-1(2*H*)-yl)benzoate (9.0 mg, 0.23 mmol) were added to a mixture of (*R*)-(1-((3-azaspiro[5.5]undecane-9-yl)methyl)piperidin-4-yl)(4-((7-methoxy-2-methyl-4-((1-(3-nitro-5-(trifluoromethyl)phenyl)ethyl)amino)quinazolin-6-yl)oxy)piperidin-1-yl)methanone (145 mg, 0.19 mmol) in a DMSO (4.0 mL) solution. The reaction mixture was stirred at room temperature for reaction overnight. Water was added to quench the reaction, and the resulting mixture was extracted with EA. The organic phase was collected, dried over anhydrous sodium sulfate, and filtered. The filtrate was concentrated to give the target product.

LC-MS: (ESI, *m*/*z*): [M-18+H]⁺=1031.43.

### Step 6: preparation of (R)-1-(5-(9-((4-(4-((4-((1-(3-amino-5-(trifluoromethyl)phenyl)ethyl)amino)-7-methoxy-2-methylquinazolin-6-yl)oxy)piperidine-1-carbonyl)piperidin-1-yl)methyl)-3-azaspiro[5.5]undecane-3-carbonyl)-2-chlorophenyl)dihydropyrimidine-2,4(1H,3H)-dione

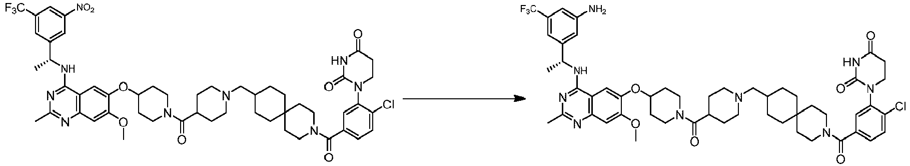

Ammonium chloride (83.0 mg, 1.55 mmol) and iron powder (86.6 mg, 1.55 mmol) were added to a mixture of (*R*)-1-(2-chloro-5-(9-((4-(4-((7-methoxy-2-methyl-4-((1-(3-nitro-5-(trifluoromethyl))phenyl)ethyl)amino)quinazolin-6-yl)oxy)piperidine-1-carbonyl)piperidin-1-yl)methyl)-3-azaspiro[5.5]undecane-3-carbonyl)phenyl)dihydropyrimidine-2,4(1*H*,3*H*)-dione (160 mg, 0.16 mmol) in an ethanol/water (3 mL/1 mL) solution. The reaction mixture was stirred at 70 °C under nitrogen atmosphere for reaction for 2 h. The reaction liquid was filtered through celite, and the filtrate was concentrated. The concentrate was purified by preparative HPLC to give the target product.

LC-MS: (ESI, *m*/*z*): [M+H]⁺=1002.5

¹H NMR (400 MHz, DMSO-d₆) δ 10.50 (s, 1H), 7.91 (d, *J* = 7.9 Hz, 1H), 7.85 (s, 1H), 7.63 (d, *J* = 8.2 Hz, 1H), 7.54 (d, *J=* 1.8 Hz, 1H), 7.38 (dd, *J* = 8.2, 1.8 Hz, 1H), 7.06 (s, 1H), 6.87 (s, 1H), 6.84 (s, 1H), 6.69 (s, 1H), 5.64 - 5.35 (m, 3H), 4.72 - 4.64 (m, 1H), 3.87 (s, 3H), 3.81 - 3.69 (m, 3H), 3.66 - 3.54 (m, 3H), 3.47 - 3.39 (m, 2H), 2.89 - 2.68 (m, 4H), 2.63 - 2.53 (m, 1H), 2.35 (s, 3H), 2.15 - 2.03 (m, 2H), 2.00 - 1.81 (m, 4H), 1.76 - 1.19 (m, 20H), 1.16 - 0.92 (m, 4H).

### Example 6: (R)-1-(5-(9-((4-((4-((1-(3-amino-5-(trifluoromethyl)phenyl)ethyl)amino)-7-methoxy-2-methylquinazolin-6-yl)oxy)piperidin-1-yl)methyl)-3-azaspiro[5.5]undecane-3-carbonyl)-2-chlorophenyl)dihydropyrimidine-2,4(1H,3H)-dione

### Step 1: preparation of tert-butyl (R)-9-((4-((7-methoxy-2-methyl-4-((1-(3-nitro-5-(trifluoromethyl)phenyl)ethyl)amino)quinazolin-6-yl)oxy)piperidin-1-yl)methyl)-3-azaspiro[5.5]undecane-3-carboxylate

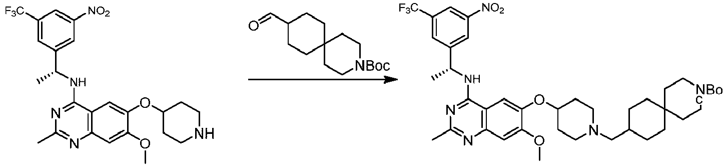

A mixture of (*R*)-7-methoxy-2-methyl-N (1-(3-nitro-5-(trifluoromethyl)phenyl)ethyl)-6-(piperidin-4-oxy)quinazolin-4-amine (100 mg, 0.198 mmol), tert-butyl 9-formyl-3-azaspiro[5.5]undecane-3-carboxylate (83 mg, 0.297 mmol), and STAB (210 mg, 0.99 mmol) in a THF (5 mL) solution was stirred at room temperature overnight. Water (50 mL) was added to quench the reaction, and the resulting mixture was extracted with EA (50 mL × 3). The organic phase was washed with water (100 mL × 2) and saturated brine (100 mL), dried over Na₂SO₄, filtered, and concentrated. The resulting crude product was purified by flash column chromatography to give the target product *tert*-butyl (*R*)-9-((4-((7-methoxy-2-methyl-4-((1-(3-nitro-5-(trifluoromethyl)phenyl)ethyl)amino)quinazolin-6-yl)oxy)piperidin-1-yl)methyl)-3-azaspiro[5.5]undecane-3-carboxylate.

LC-MS: (ESI, *m*/*z):* [M+H]⁺= 771.5.

### Step 2: preparation of (R)-6-((1-((3-azaspiro[5.5]undecane-9-yl)methyl)piperidin-4-yl)oxy)-7-methoxy-2-methyl-N-(1-(3-nitro-5-(trifluoromethyl)phenyl)ethyl)quinazolin-4-amine

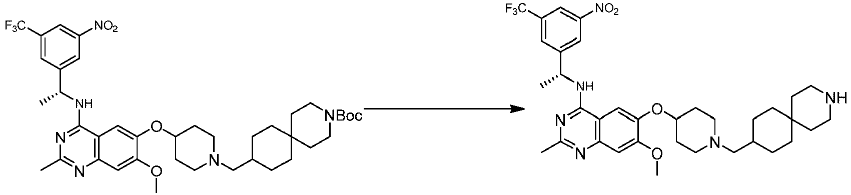

A reaction liquid of *tert*-butyl (*R*)-9-((4-((7-methoxy-2-methyl-4-((1-(3-nitro-5-(trifluoromethyl)phenyl)ethyl)amino)quinazolin-6-yl)oxy)piperidin-1-yl)methyl)-3-azaspiro[5.5]undecane-3-carboxylate (130 mg, 0.169 mmol) in TFA/DCM (4 mL, 1:3) was stirred at room temperature for 1 h. The reaction liquid was concentrated to give the target product (*R*)-6-((1-((3-azaspiro[5.5]undecane-9-yl)methyl)piperidin-4-yl)oxy)-7-methoxy-2-methyl-*N*-(1-(3-nitro-5-(trifluoromethyl)phenyl)ethyl)quinazolin-4-amine. The crude product was directly used in the next step.

LC-MS: (ESI, *m*/*z):* [M+H]⁺= 671.3.

### Step 3: preparation of (R)-1-(2-chloro-5-(9-((4-((7-methoxy-2-methyl-4-((1-(3-nitro-5-(trifluoromethyl)phenyl)ethyl)amino)quinazolin-6-yl)oxy)piperidin-1-yl)methyl)-3-azaspiro[5.5]undecane-3-carbonyl)phenyl)dihydropyrimidine-2,4(1H,3H)-dione

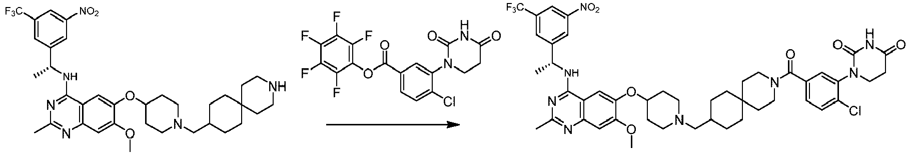

A reaction liquid of (*R*)-6-((1-((3-azaspiro[5.5]undecane-9-yl)methyl)piperidin-4-yl)oxy)-7-methoxy-2-methyl-*N-*(1-(3-nitro-5-(trifluoromethyl)phenyl)ethyl)quinazolin-4-amine (130 mg, crude product), pentafluorophenyl 4-chloro-3-(2,4-dioxotetrahydropyrimidin-1(2*H*)-yl)benzoate (75 mg, 0.170 mmol), and DIEA (110 mg, 0.85 mmol) in DMSO (5 mL) was stirred at room temperature overnight and then poured into water (50 mL), and the resulting mixture was stirred and filtered. The filter cake was dried under vacuum to give the target product (*R*)-1-(2-chloro-5-(9-((4-((7-methoxy-2-methyl-4-((1-(3-nitro-5-(trifluoromethyl)phenyl)ethyl)amino)quinazolin-6-yl)oxy)piperidin-1-yl)methyl)-3-azaspiro[5.5]undecane-3-carbonyl)phenyl)dihydropyrimidine-2,4(1*H*,3*H*)-dione. The crude product was directly used in the next step.

LC-MS: (ESI, *m*/*z*): [M+H]⁺= 921.4.

### Step 4: preparation of (R)-1-(5-(9-((4-((4-((1-(3-amino-5-(trifluoromethyl)phenyl)ethyl)amino)-7-methoxy-2-methylquinazolin-6-yl)oxy)piperidin-1-yl)methyl)-3-azaspiro[5.5]undecane-3-carbonyl)-2-chlorophenyl)dihydropyrimidine-2,4(1H,3H)-dione

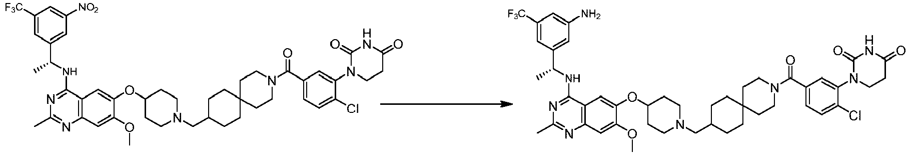

A reaction liquid of (*R*)-1-(2-chloro-5-(9-((4-((7-methoxy-2-methyl-4-((1-(3-nitro-5-(trifluoromethyl)phenyl)ethyl)amino)quinazolin-6-yl)oxy)piperidin-1-yl)methyl)-3-azaspiro[5.5]undecane-3-carbonyl)phenyl)dihydropyrimidine-2,4(1*H*,3*H*)-dione (120 mg, 0.130 mmol), Fe powder (73 mg, 1.30 mmol), and NH₄Cl (70 mg, 1.30 mmol) in EtOH/H₂O (8 mL, 3:1) was stirred at room temperature for reaction for 1 h, then filtered, and concentrated. The resulting crude product was purified by preparative HPLC to give the target product (*R*)-1-(5-(9-((4-((4-((1-(3-amino-5-(trifluoromethyl)phenyl)ethyl)amino)-7-methoxy-2-methylquinazolin-6-yl)oxy)piperidin-1-yl)methyl)-3-azaspiro[5.5]undecane-3-carbonyl)-2-chlorophenyl)dihydropyrimidine-2,4(1*H*,3*H*)-dione.

LC-MS: (ESI, *m*/*z):* [M+H]⁺= 891.4.

¹H NMR (400 MHz, DMSO-*d*₆) δ 10.51 (s, 1H), 7.91 (d, J = 7.8 Hz, 1H), 7.78 (s, 1H), 7.63 (d, *J=* 8.2 Hz, 1H), 7.55 (d, *J* = 1.8 Hz, 1H), 7.39 (dd, *J* = 8.3, 1.8 Hz, 1H), 7.04 (s, 1H), 6.87 (s, 1H), 6.83 (s, 1H), 6.69 (s, 1H), 5.61 - 5.50 (m, 3H), 4.450 - 4.42 (m, 1H), 3.86 (s, 3H), 3.80 - 3.70 (m, 1H), 3.67 - 3.52 (m, 3H), 2.78 - 2.61 (m, 4H), 2.35 (s, 3H), 2.30 - 2.10 (m, 4H), 1.99 - 1.90 (m, 2H), 1.75 - 1.65 (m, 4H), 1.60 - 0.94 (m, 16H).

### Example 7: 1-(5-(9-((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)methyl)-3,9-diazaspiro [5.5]undecane-3 -carbonyl)-2-chlorophenyl)dihydropyrimidine-2,4(1H,3H)-dione

### Step 1: preparation of tert-butyl 9-((1-((benzyloxy)carbonyl)piperidin-4-yl)methyl)-3,9-diazaspiro[5.5]undecane-3-carboxylate

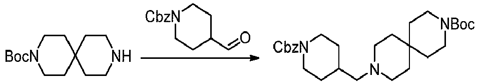

STAB (770.0 mg, 3.6 mmol) was slowly added to a solution of 3,9-diazaspiro[5.5]undecane-3-carboxylate (300.0 mg, 1.2 mmol) and *tert*-butyl phenyl 4-formylpiperidine-1-carboxylate (346.0 mg, 1.4 mmol) in THF (5 mL), and the reaction liquid was stirred at room temperature overnight. Water (20 mL) was added to quench the reaction, and the resulting mixture was extracted with EA (50 mL × 2). The organic phase was washed with a saturated NaHCO₃ solution, dried over Na₂SO₄, filtered, and concentrated. The resulting crude product was purified by silica gel column chromatography (EtOAc-PE, 0-30%) to give the target product *tert-butyl* 9-((1-((benzyloxy)carbonyl)piperidin-4-yl)methyl)-3,9-diazaspiro[5.5]undecane-3-carboxylate.

LC-MS: (ESI, *m*/*z):* [M+H]⁺= 486.4.

### Step 2: preparation of tert-butyl 9-(piperidin-4-ylmethyl)-3,9-diazaspiro[5.5]undecane-3-carboxylate

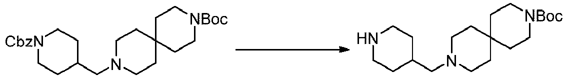

A reaction liquid of *tert*-butyl 9-((1-((benzyloxy)carbonyl)piperidin-4-yl)methyl)-3,9-diazaspiro[5.5]undecane-3-carboxylate (300.0 mg, 0.6 mmol) and 10% Pd/C (60.0 mg) in EtOAc (5 mL) was stirred at 70 °C under H₂ atmosphere (60 psi) overnight, then filtered through celite, and concentrated to give the target product *tert*-butyl 9-(piperidin-4-ylmethyl)-3,9-diazaspiro[5.5]undecane-3-carboxylate. The resulting crude product was directly used in the next step.

LC-MS: (ESI, *m*/*z):* [M+H]⁺= 352.4.

### Step 3: preparation of tert-butyl 9-((1-((1R,4R)-4-(7-methoxy-2-methyl-4-(((R)-1-(3-nitro-5-(trifluoromethyl)phenyl)ethyl)amino)quinazolin-6-yl)cyclohexane-1-carbonyl)piperidin-4-yl)methyl)-3,9-diazaspiro[5.5]undecane-3-carboxylate

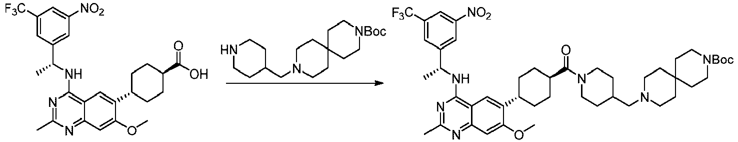

DIEA (78.0 mg, 0.6 mmol) was slowly added dropwise to a solution of (1*R*,4*R*)-4-(7-methoxy-2-methyl-4-(((*R*)-1-(3-nitro-5-(trifluoromethyl)phenyl)ethyl)amino)quinazolin-6-yl)cyclohexyl-1-carboxylic acid (140.0 mg, 0.2 mmol), *tert*-butyl 9-(piperidin-4-ylmethyl)-3,9-diazaspiro[5.5]undecane-3-carboxylate (84.0 mg, 0.24 mmol), and HATU (114 mg, 0.3 mmol) in DMF (2.0 mL). The reaction liquid was stirred at room temperature for reaction for 2 h. Ice water (20 mL) was added to quench the reaction, and the resulting mixture was filtered. The filter cake was dried under vacuum to give the target product *tert*-butyl 9-((1-((1*R*,4*R*)-4-(7-methoxy-2-methyl-4-(((*R*)-1-(3-nitro-5-(trifluoromethyl)phenyl)ethyl)amino)quinazolin-6-yl)cyclohexane-1-carbonyl)piperidin-4-yl)methyl)-3,9-diazaspiro[5.5]undecane-3-carboxylate. The resulting crude product was directly used in the next step.

LC-MS: (ESI, *m*/*z):* [M+H]⁺= 866.5.

### Step 4: preparation of tert-butyl 9-((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)methyl)-3,9-diazaspiro[5.5]undecane-3-carboxylate

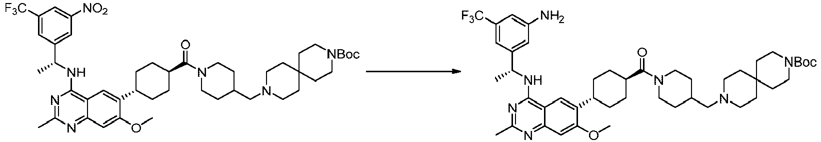

A reaction liquid of *tert*-butyl 9-((1-((1*R*,4*R*)-4-(7-methoxy-2-methyl-4-(((*R*)-1-(3-nitro-5-(trifluoromethyl)phenyl)ethyl)amino)quinazolin-6-yl)cyclohexane-1-carbonyl)piperidin-4-yl)methyl)-3,9-diazaspiro[5.5]undecane-3-carboxylate (130.0 mg, 0.15 mmol) and 10% Pd/C (30.0 mg) in MeOH (10.0 mL) was stirred at 70 °C under H₂ atmosphere (90 psi) overnight, then filtered through celite, and concentrated to give the target product *tert*-butyl 9-((1-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperidin-4-yl)methyl)-3,9-diazaspiro[5.5]undecane-3-carboxylate. The resulting crude product was directly used in the next step.

LC-MS: (ESI, *m*/*z):* [M+H]⁺= 836.5.

### Step 5: preparation of (4-((3,9-diazaspiro[5.5]undecane-3-yl)methyl)piperidin-1-yl)((1R,4R)-4-(4-(((R)-1-(3-amino-5-(trifluoromethyl)phenyl)ethyl)amino)-7-methoxy-2-methylquinazolin-6-yl)cyclohexyl)methanone

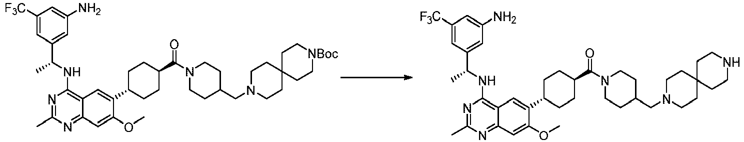

A reaction liquid of tert-butyl 9-((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)methyl)-3,9-diazaspiro[5.5]undecane-3-carboxylate (90.0 mg, 0.1 mmol) in TFA/DCM (1.0 mL/3.0 mL) was stirred at room temperature for reaction for 1 h and then directly concentrated to give the target product (4-((3,9-diazaspiro[5.5]undecane-3-yl)methyl)piperidin-1-yl)((1*R*,4*R*)-4-(4-(((*R*)-1-(3-amino-5-(trifluoromethyl)phenyl)ethyl)amino)-7-methoxy-2-methylquinazolin-6-yl)cyclohexyl)methanone. The resulting crude product was directly used in the next step.

LC-MS: (ESI, *m*/*z):* [M+H]⁺= 736.4.

### Step 6: 1-(5-(9-((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)methyl)-3,9-diazaspiro [5.5]undecane-3 -carbonyl)-2-chlorophenyl)dihydropyrimidine-2,4(1H,3H)-dione

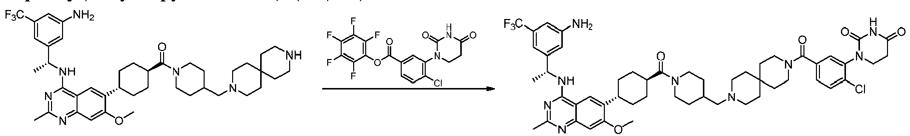

DIEA (54.20 mg, 0.42 mmol) and pentafluorophenyl 4-chloro-3-(2,4-dioxotetrahydropyrimidin-1(2*H*)-yl)benzoate (73.0 mg, 0.17 mmol) were added to a solution of (4-((3,9-diazaspiro[5.5]undecane-3-yl)methyl)piperidin-1-yl)((1*R*,4*R*)-4-(4-(((*R*)-1-(3-amino-5-(trifluoromethyl)phenyl)ethyl)amino)-7-methoxy-2-methylquinazolin-6-yl)cyclohexyl)methanone (100.0 mg, 0.12 mmol) in DMSO (5.0 mL), and the reaction liquid was stirred at room temperature for 2 h. Water (15.0 mL) was added to the reaction liquid, and the resulting mixture was extracted with EA (20 mL × 2). The organic phase was washed with saturated brine (20 mL), dried over anhydrous Na₂SO₄, and concentrated. The crude product was purified by preparative HPLC (acetonitrile/0.05% aqueous NH₄HCO₃ solution, 5% to 95%) to give the target product 1-(5-(9-((1-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperidin-4-yl)methyl)-3,9-diazaspiro[5.5]undecane-3-carbonyl)-2-chlorophenyl)dihydropyrimidine-2,4(1*H*,3*H*)-dione. LC-MS: (ESI, *m*/*z):* [M+H]⁺= 986.4.

¹H NMR (400 MHz, DMSO-*d*₆) δ 10.51 (s, 1H), 8.16-8.02 (m, 2H), 7.63 (d, *J* = 8.2 Hz, 1H), 7.54 (s, 1H), 7.38 (d, *J=* 8.2 Hz, 1H), 6.99 (s, 1H), 6.88 (s, 1H), 6.85 (s, 1H), 6.70 (s, 1H), 5.65-5.47 (m, 3H), 4.41-4.38 (m, 1H), 3.89-3.75 (m, 4H), 3.79-3.71 (m, 1H), 3.67-3.54 (m, 3H), 3.31-2.90 (m, 2H), 2.78-2.72 (m, 2H), 2.70-2.60 (m, 1H), 2.55-2.51 (m, 1H), 2.45-2.20 (m, 7H), 2.15-2.05 (m, 2H), 1.95-1.29 (m, 24H), 1.17-0.98 (m, 2H).

Example 8: 1-(5-(9-((1-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperidin-4-yl)methyl)-3-azaspiro[5.5]undecane-3-carbonyl)-2-chlorophenyl)dihydropyrimidine-2,4(1*H*,3*H*)-dione

### Step 1: preparation of tert-butyl 9-(hydroxy(pyridin-4-yl)methyl)-3-azaspiro[5.5]undecane-3-carboxylate

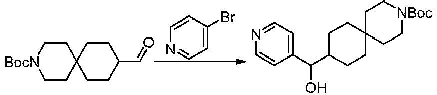

A solution of 4-bromopyridine (1.0 g, 6.3 mmol) in anhydrous THF (5.0 mL) was added dropwise to a solution of *n*-BuLi (2.3 mL, 2.5 M/*n*-hexane, 5.7 mmol) in anhydrous THF (15 mL) at -78 °C. The resulting mixture was stirred at -78 °C for 15 min, and then a solution of *tert*-butyl 9-formyl-3-azaspiro[5.5]undecane-3-carboxylate (1.5 g, 5.3 mmol) in THF was slowly added dropwise. The reaction liquid was stirred at -78 °C for another 45 min. Water (20 mL) was added at -70 °C to quench the reaction, and the resulting mixture was extracted with EA (20 mL × 2). The organic phase was washed with saturated brine, dried over Na₂SO₄, filtered, and concentrated. The resulting crude product was purified by silica gel column chromatography (EtOAc-PE, 0-30%) to give the target product *tert*-butyl 9-(hydroxy(pyridin-4-yl)methyl)-3 -azaspiro [5.5]undecane-3 -carboxylate.

LC-MS: (ESI, *m*/*z):* [M+H]⁺= 361.1.

### Step 2: preparation of tert-butyl 9-(acetoxy(pyridin-4-yl)methyl)-3-azaspiro[5.5]undecane-3-carboxylate

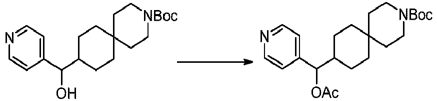

A reaction liquid of *tert*-butyl 9-(hydroxy(pyridin-4-yl)methyl)-3-azaspiro[5.5]undecane-3-carboxylate (1.0 g, 2.7 mmol), Ac₂O (0.4 g, 4.0 mol), and TEA (0.2 mL) in DCE (10.0 mL) was stirred at 70 °C for 5 h, concentrated, and then extracted with chloroform. The organic phase was concentrated. The resulting crude product was purified by silica gel column chromatography to give the target product *tert*-butyl 9-(acetoxy(pyridin-4-yl)methyl)-3-azaspiro [5.5]undecane-3 -carboxylate.

LC-MS: (ESI, *m*/*z):* [M+H]⁺= 403.2.

### Step 3: preparation of tert-butyl 9-(pyridin-4-ylmethyl)-3-azaspiro[5.5]undecane-3-carboxylate

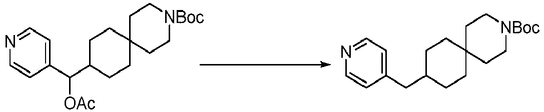

A reaction liquid of *tert*-butyl 9-(acetoxy(pyridin-4-yl)methyl)-3-azaspiro[5.5]undecane-3-carboxylate (600.0 mg, 1.5 mmol), 10% Pd/C (150.0 mg), and ammonium formate (472.0 mg, 7.5 mmol) in MeOH (10.0 mL) was stirred at 80 °C overnight, then filtered through celite, and concentrated to give the target product *tert*-butyl 9-(pyridin-4-ylmethyl)-3-azaspiro[5.5]undecane-3-carboxylate. The resulting crude product was directly used in the next step.

LC-MS: (ESI, *m*/*z):* [M+H]⁺= 345.2.

### Step 4: preparation of tert-butyl 9-(piperidin-4-ylmethyl)-3-azaspiro[5.5]undecane-3-carboxylate

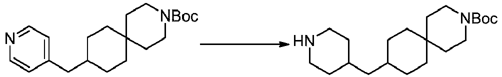

A reaction liquid of *tert*-butyl 9-(pyridin-4-ylmethyl)-3-azaspiro[5.5]undecane-3-carboxylate (450.0 mg, 1.3 mmol) and 10% Pd/C (80.0 mg) in *i*-PrOH (10.0 mL) and H₂O (5.0 mL) was stirred at 70 °C under H₂ atmosphere (90 psi) overnight, then filtered through celite, and concentrated to give the target product *tert*-butyl 9-(piperidin-4-ylmethyl)-3-azaspiro[5.5]undecane-3-carboxylate. The resulting crude product was directly used in the next step.

LC-MS: (ESI, *m*/*z):* [M+H]⁺= 351.1.

### Step 5: preparation of tert-butyl 9-((1-((1R,4R)-4-(7-methoxy-2-methyl-4-(((R)-1-(3-nitro-5-(trifluoromethyl)phenyl)ethyl)amino)quinazolin-6-yl)cyclohexane-1-carbonyl)piperidin-4-yl)methyl)-3-azaspiro [5.5]undecane-3 -carboxylate

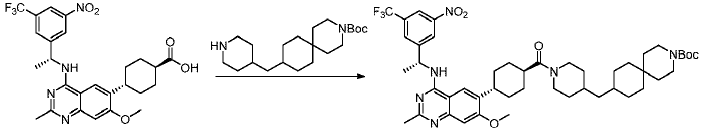

DIEA (78.0 mg, 0.6 mmol) was slowly added dropwise to a solution of (1*R*,4*R*)-4-(7-methoxy-2-methyl-4-(((*R*)-1-(3-nitro-5-(trifluoromethyl)phenyl)ethyl)amino)quinazolin-6-yl)cyclohexyl-1-carboxylic acid (140.0 mg, 0.2 mmol), tert-butyl 9-(piperidin-4-ylmethyl)-3-azaspiro[5.5]undecane-3-carboxylate (84.0 mg, 0.24 mmol), and HATU (114 mg, 0.3 mmol) in DMF (2.0 mL), and then the reaction liquid was stirred at room temperature for 2 h. Ice water (20 mL) was added to quench the reaction, and the resulting mixture was filtered. The filter cake was dried under vacuum to give the target product tert-butyl 9-((1-((1R,4R)-4-(7-methoxy-2-methyl-4-(((R)-1-(3-nitro-5-(trifluoromethyl)phenyl)ethyl)amino)quinazolin-6-yl)cyclohexane-1-carbonyl)piperidin-4-yl)methyl)-3-azaspiro[5.5]undecane-3-carboxylate. The resulting crude product was directly used in the next step.

LC-MS: (ESI, *m*/*z):* [M+H]⁺= 865.4.

### Step 6: preparation of tert-butyl 9-((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)methyl)-3-azaspiro[5.5]undecane-3-carboxylate

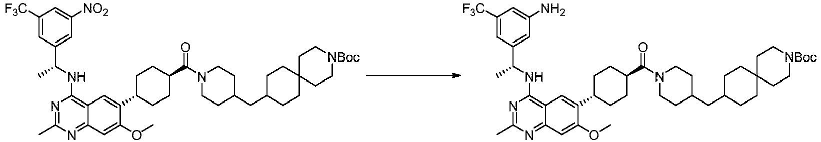

A reaction liquid of tert-butyl 9-((1-((1R,4R)-4-(7-methoxy-2-methyl-4-(((R)-1-(3-nitro-5-(trifluoromethyl)phenyl)ethyl)amino)quinazolin-6-yl)cyclohexane-1-carbonyl)piperidin-4-yl)methyl)-3-azaspiro[5.5]undecane-3-carboxylate (130.0 mg, 0.15 mmol) and 10% Pd/C (30.0 mg) in MeOH (10.0 mL) was stirred at 70 °C under H₂ atmosphere (90 psi) overnight, then filtered through celite, and concentrated to give the target product tert-butyl 9-((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)methyl)-3-azaspiro[5.5]undecane-3-carboxylate. The resulting crude product was directly used in the next step.

LC-MS: (ESI, *m*/*z):* [M+H]⁺= 835.5.

### Step 7: preparation of (4-((3-azaspiro[5.5]undecane-9-yl)methyl)piperidin-1-yl)((1R,4R)-4-(4-(((R)-1-(3-amino-5-(trifluoromethyl)phenyl)ethyl)amino)-7-methoxy-2-methylquinazolin-6-yl)cyclohexyl)methanone

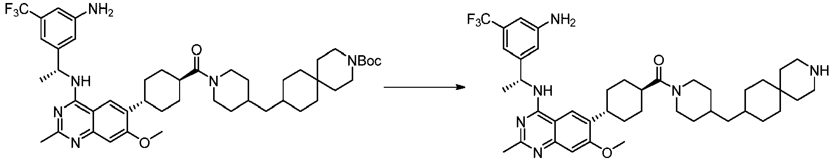

A reaction liquid of tert-butyl 9-((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)methyl)-3-azaspiro[5.5]undecane-3-carboxylate (130.0 mg, 0.15 mmol) in TFA/DCM (3.0 mL, 1:5) was stirred at room temperature for 1 h and then directly concentrated to give the product (4-((3-azaspiro[5.5]undecane-9-yl)methyl)piperidin-1-yl)((1R,4R)-4-(4-(((R)-1-(3-amino-5-(trifluoromethyl)phenyl)ethyl)amino)-7-methoxy-2-methylquinazolin-6-yl)cyclohexyl)methanone. The resulting crude product was directly used in the next step.

LC-MS: (ESI, *m*/*z):* [M+H]⁺= 736.4.

### Step 8: 1-(5-(9-((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)methyl)-3-azaspiro[5.5]undecane-3-carbonyl)-2-chlorophenyl)dihydropyrimidine-2,4(1H,3H)-dione

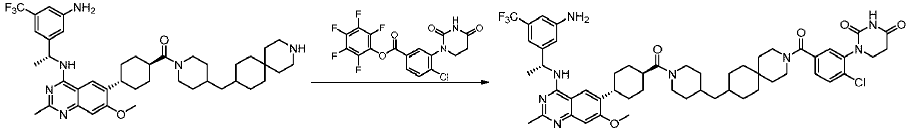

DIEA (54.20 mg, 0.42 mmol) and pentafluorophenyl 4-chloro-3-(2,4-dioxotetrahydropyrimidin-1(2*H*)-yl)benzoate (73.0 mg, 0.17 mmol) were added to a solution of (4-((3-azaspiro[5.5]undecane-9-yl)methyl)piperidin-1-yl)((1*R*,4*R*)-4-(4-(((*R*)-1-(3-amino-5-(trifluoromethyl)phenyl)ethyl)amino)-7-methoxy-2-methylquinazolin-6-yl)cyclohexyl)methanone (100.0 mg, 0.14 mmol) in DMSO (5.0 mL), and the reaction liquid was stirred at room temperature for 2 h. Water (15 mL) was added to quench the reaction, and the resulting mixture was extracted with EA (20 mL × 2). The organic phase was washed with saturated brine (20 mL), dried over anhydrous Na₂SO₄, and concentrated. The crude product was purified by preparative HPLC (acetonitrile/0.05% aqueous NH₄HCO₃ solution, 5% to 95%) to give the target product 1-(5-(9-((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)methyl)-3-azaspiro[5.5]undecane-3-carbonyl)-2-chlorophenyl)dihydropyrimidine-2,4(1*H*,3*H*)-dione.

LC-MS: (ESI, *m*/*z*): [M+H]⁺= 985.4.

¹H NMR (400 MHz, DMSO-*d*₆) δ 10.51 (s, 1H), 8.16-8.02 (m, 2H), 7.63 (d, *J* = 8.2 Hz, 1H), 7.54 (s, 1H), 7.38 (d, *J=* 8.2 Hz, 1H), 6.99 (s, 1H), 6.88 (s, 1H), 6.86 (s, 1H), 6.70 (s, 1H), 5.65-5.47 (m, 3H), 4.41-4.38 (m, 1H), 3.89-3.75 (m, 4H), 3.79-3.71 (m, 1H), 3.67-3.54 (m, 3H), 3.31-2.90 (m, 2H), 2.78-2.72 (m, 2H), 2.70-2.60 (m, 1H), 2.38-2.35 (s, 3H), 1.86-1.75 (m, 4H), 1.73-1.28 (m, 22H), 1.20-0.95 (m, 8H).

### Example 9: (1R,4R)-4-(4-(((R)-1-(3-amino-5-(trifluoromethyl)phenyl)ethyl)amino)-7-methoxy-2-methylquinazolin-6-yl)-N-((3-(4-chloro-3-(2,4-dioxotetrahydropyrimidin-1(2H)-yl)benzoyl)-3-azaspiro[5.5]undecane-9-yl)methyl)-N methylcyclohexane-1-carboxamide

### Step 1: preparation of tert-butyl 9-((benzyl(methyl)amino)methyl)-3-azaspiro[5.5]undecane-3-carboxylate

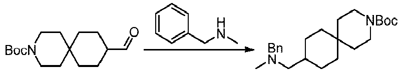

STAB (680.0 mg, 3.3 mmol) was added to a solution of tert-butyl 9-formyl-3-azaspiro[5.5]undecane-3-carboxylate (300.0 mg, 1.1 mmol) and *N*-methyl-1-benzylamine (130.0 mg, 1.1 mmol) in THF (10.0 mL), and the reaction liquid was stirred at room temperature overnight. The reaction liquid was poured into an aqueous NaHCO₃ solution (20 mL), and the resulting mixture was extracted with EA (20 mL × 2). The organic phase was washed with water and saturated brine, dried over Na₂SO₄, filtered, and concentrated. The resulting crude product was purified by silica gel column chromatography (EtOAc-PE, 0-30%) to give the target product *tert-butyl* 9-((benzyl(methyl)amino)methyl)-3-azaspiro[5.5]undecane-3-carboxylate.

LC-MS: (ESI, *m*/*z):* [M+H]⁺= 387.2.

### Step 2: preparation of tert-butyl 9-((methylamino)methyl)-3-azaspiro[5.5]undecane-3-carboxylate

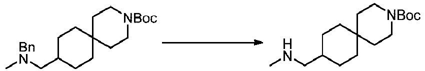

A reaction liquid of tert-butyl 9-((benzyl(methyl)amino)methyl)-3-azaspiro[5.5]undecane-3-carboxylate (240.0 mg, 0.6 mmol) and 10% Pd/C (50.0 mg) in MeOH (10.0 mL) was stirred at 50 °C under H₂ atmosphere (60 psi) overnight, then filtered through celite, and concentrated to give the target product tert-butyl 9-((methylamino)methyl)-3-azaspiro[5.5]undecane-3-carboxylate. The resulting crude product was directly used in the next step.

LC-MS: (ESI, *m*/*z*): [M+H]⁺= 297.2.

### Step 3: preparation of tert-butyl 9-(((1R,4R)-4-(7-methoxy-2-methyl-4-(((R)-1-(3-nitro-5-(trifluoromethyl)phenyl)ethyl)amino)quinazolin-6-yl)-N-methylcyclohexane-1-amido)methyl)-3-azaspiro [5.5]undecane-3 -carboxylate

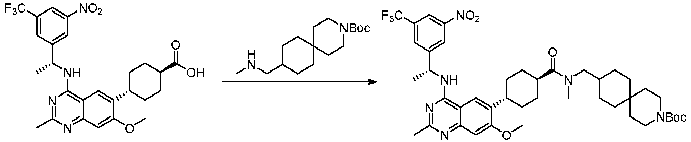

DIEA (78.0 mg, 0.6 mmol) was slowly added dropwise to a solution of (1*R*,4*R*)-4-(7-methoxy-2-methyl-4-(((R)-1-(3-nitro-5-(trifluoromethyl)phenyl)ethyl)amino)quinazolin-6-yl)cyclohexyl-1-carboxylic acid (120.0 mg, 0.2 mmol), *tert*-butyl 9-((methylamino)methyl)-3-azaspiro[5.5]undecane-3-carboxylate (65.0 mg, 0.22 mmol), and HATU (98.8 mg, 0.26 mmol) in DMF (2.0 mL). The reaction liquid was stirred at room temperature for 2 h. Ice water (20 mL) was added to quench the reaction, and the resulting mixture was stirred and filtered. The filter cake was dried under vacuum to give the target product *tert*-butyl 9-(((1*R*,4*R*)-4-(7-methoxy-2-methyl-4-(((*R*)-1-(3-nitro-5-(trifluoromethyl)phenyl)ethyl)amino)quinazolin-6-yl)-*N*-methylcyclohexane-1-amido)methyl)-3-azaspiro[5.5]undecane-3-carboxylate. The resulting crude product was directly used in the next step.

LC-MS: (ESI, *m*/*z):* [M+H]⁺= 811.5.

### Step 4: preparation of tert-butyl 9-(((1R,4R)-4-(4-(((R)-1-(3-amino-5-(trifluoromethyl)phenyl)ethyl)amino)-7-methoxy-2-methylquinazolin-6-yl)-N-methylcyclohexane-1-amido)methyl)-3-azaspiro[5.5]undecane-3-carboxylate

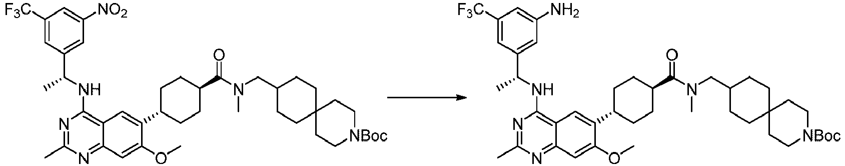

A reaction liquid of *tert*-butyl 9-(((1*R*,4*R*)-4-(7-methoxy-2-methyl-4-(((*R*)-1-(3-nitro-5-(trifluoromethyl)phenyl)ethyl)amino)quinazolin-6-yl)-*N*-methylcyclohexane-1-amido)methyl)-3-azaspiro[5.5]undecane-3-carboxylate (130.0 mg, 0.15 mmol) and 10% Pd/C (30.0 mg) in MeOH (10.0 mL) was stirred at 70 °C under H₂ atmosphere (90 psi) overnight and then filtered through celite. The filtrate was concentrated to give the target product *tert*-butyl 9-(((1*R*,4*R*)-4-(4-(((*R*)-1-(3-amino-5-(trifluoromethyl)phenyl)ethyl)amino)-7-methoxy-2-methylquinazolin-6-yl)-*N*-methylcyclohexane-1-amido)methyl)-3-azaspiro[5.5]undecane-3-carboxylate. The resulting crude product was directly used in the next step.

LC-MS: (ESI, *m*/*z):* [M+H]⁺= 781.5.

### Step 5: preparation of (1R,4R)-N-((3-azaspiro[5.5]undecane-9-yl)methyl)-4-(4-(((R)-1-(3-amino-5-(trifluoromethyl)phenyl)ethyl)amino)-7-methoxy-2-methylquinazolin-6-yl)-N-methylcyclohexane-1-carboxamide

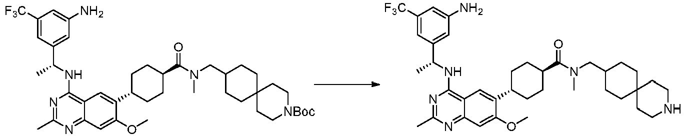

A reaction liquid of *tert*-butyl 9-(((1*R*,4*R*)-4-(4-(((*R*)-1-(3-amino-5-(trifluoromethyl)phenyl)ethyl)amino)-7-methoxy-2-methylquinazolin-6-yl)-*N*-methylcyclohexane-1-amido)methyl)-3-azaspiro[5.5]undecane-3-carboxylate (100.0 mg, 0.1 mmol) in TFA/DCM (1.0 mL/3.0 mL) was stirred at room temperature for 1 h and then directly concentrated to give the target product (1*R*,4*R*)-*N*-((3-azaspiro[5.5]undecane-9-yl)methyl)-4-(4-(((*R*)-1-(3-amino-5-(trifluoromethyl)phenyl)ethyl)amino)-7-methoxy-2-methylquinazolin-6-yl)-*N*-methylcyclohexane-1-carboxamide. The resulting crude product was directly used in the next step.

LC-MS: (ESI, *m*/*z):* [M+H]⁺= 681.3.

### Step 6: preparation of (1R,4R)-4-(4-(((R)-1-(3-amino-5-(trifluoromethyl)phenyl)ethyl)amino)-7-methoxy-2-methylquinazolin-6-yl)-N-((3-(4-chloro-3-(2,4-dioxotetrahydropyrimidin-1(2H)-yl)benzoyl)-3-azaspiro[5.5]undecane-9-yl)methyl)-N-methylcyclohexane-1-carboxamide

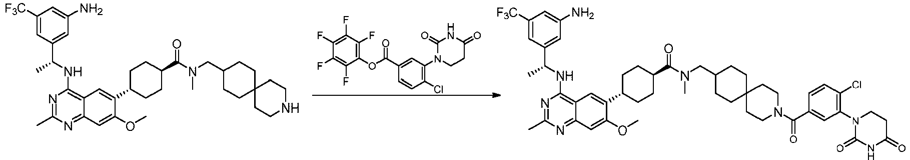

DIEA (54.2 mg, 0.42 mmol) and pentafluorophenyl 4-chloro-3-(2,4-dioxotetrahydropyrimidin-1(2*H*)-yl)benzoate (63.0 mg, 0.17 mmol) were added to a solution of (1*R*,4*R*)-N-((3-azaspiro[5.5]undecane-9-yl)methyl)-4-(4-(((R)-1-(3-amino-5-(trifluoromethyl)phenyl)ethyl)amino)-7-methoxy-2-methylquinazolin-6-yl)-*N*-methylcyclohexane-1-carboxamide (90.0 mg, 0.13 mmol) in DMSO (5.0 mL), and the reaction liquid was stirred at room temperature for reaction for 2 h. Water (15 mL) was added to quench the reaction, and the resulting mixture was extracted with EA (30 mL × 2). The organic phase was washed with saturated brine, dried over anhydrous Na₂SO₄, and concentrated. The crude product was purified by preparative HPLC (acetonitrile/0.05% aqueous NH₄HCO₃ solution, 5% to 95%) and then purified by preparative TLC (5.0% MeOH/DCM) to give the target product (1*R*,4*R*)-4-(4-(((*R*)-1-(3-amino-5-(trifluoromethyl)phenyl)ethyl)amino)-7-methoxy-2-methylquinazolin-6-yl)-*N*-((3-(4-chloro-3-(2,4-dioxotetrahydropyrimidin-1(2*H*)-yl)benzoyl)-3-azaspiro[5.5]undecane-9-yl)methyl)-*N*-methylcyclohexane-1-carboxamide.

LC-MS: (ESI, *m*/*z):* [M+H]⁺= 931.6.

¹H NMR (400 MHz, DMSO-*d*₆) δ 10.51 (s, 1H), 8.15 (d, *J* = 7.6 Hz, 1H), 8.05 (d, *J* = 11.2 Hz, 1H), 7.63 (d, *J* = 8.2 Hz, 1H), 7.55 (s, 1H), 7.38 (d, *J* = 8.3 Hz, 1H), 6.99 (s, 1H), 6.88 (s, 1H), 6.85 (s, 1H), 6.70 (s, 1H), 5.65 - 5.47 (m, 3H), 3.89 (s, 3H), 3.80 - 3.70 (m, 1H), 3.65 - 3.51 (m, 3H), 3.25 -3.15 (s, 2H), 3.02 (s, 2H), 2.94 (d, *J* = 10.9 Hz, 1H), 2.80 (s, 1H), 2.76 - 2.69 (m, 2H), 2.68 - 2.62 (m, 1H), 2.36 (s, 3H), 1.92 - 1.82 (m, 3H), 1.76 - 1.51 (m, 11H), 1.45 - 1.33 (m, 4H), 1.32 - 120 (m, 4H), 1.15 - 1.02 (s, 4H).

### Example 10: 1-(2-chloro-5-(9-((4-((1R,4R)-4-(4-(((R)-1-(1,1-dioxo-2,3-dihydrobenzo[b]thiophen-4-yl)ethyl)amino)-7-methoxy-2-methylquinazolin-6-yl)cyclohexane-1-carbonyl)piperazin-1-yl)methyl)-3-azaspiro[5.5]undecane-3-carbonyl)phenyl)dihydropyrimidine-2,4(1H,3H)-dione

### Step 1: preparation of 4-bromobenzo[b]thiophene 1,1-dioxide

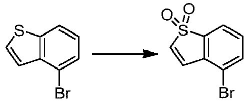

*m*-CPBA (4.8 g, 28.4 mmol) was added to a solution of 4-bromobenzo[b]thiophene (3.0 g, 14.2 mmol) in DCM (60.0 mL), and the reaction liquid was stirred at 25 °C for reaction overnight. Ice water (150 mL) was added to quench the reaction, and the resulting mixture was extracted with EA (80 mL × 3). The organic phases were combined, washed with brine (30 mL), dried over anhydrous Na₂SO₄, and concentrated. The crude product was purified by column chromatography (EA/PE = 60/40) to give the target product 4-bromobenzo[b]thiophene 1,1-dioxide.

LC-MS: (ESI, *m*/*z):* [M+H]⁺= 245.1.

### Step 2: preparation of 4-bromo-2,3-dihydrobenzo[b]thiophene 1,1-dioxide

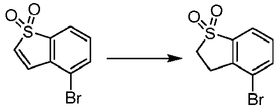

NaBH₄ (465.3 mg, 12.2 mmol) was added to a solution of 4-bromobenzo[b]thiophene 1,1-dioxide (4.0 g, 16.3 mmol) in MeOH (40.0 mL) at 0 °C, and the reaction liquid was stirred at 25 °C for reaction for 4.0 h. Water (100 mL) was added to quench the reaction, and the resulting mixture was extracted with EA (80 mL × 3). The organic phases were combined, washed with brine (30 mL), dried over anhydrous Na₂SO₄, and concentrated. The crude product was purified by column chromatography (EA/PE = 70/30) to give the target product 4-bromo-2,3-dihydrobenzo[b]thiophene 1,1-dioxide.

LC-MS: (ESI, *m*/*z):* [M+H]⁺= 247.1.

### Step 3: preparation of 1-(1,1-dioxo-2,3-dihydrobenzo[b]thiophen-4-yl)ethanone

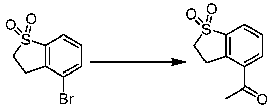

A reaction liquid of 4-bromo-2,3-dihydrobenzo[b]thiophene 1,1-dioxide (4.0 g, 16.3 mmol), Pd(PPh₃)₂Cl₂ (117.7 mg, 1.6 mmol), TEA (4.9 g, 48.9 mmol), and tributyl(1-ethoxyethylene)stannane (8.8 g, 24.4 mmol) in 1,4-dioxane (80.0 mL) was purged with N₂ 3 times and then stirred at 80 °C under N₂ atmosphere for reaction for 16.0 h. Ice water (200 mL) was added to quench the reaction, and the resulting mixture was extracted with EA (80 mL × 3). The organic phases were combined, washed with brine (50 mL), dried over anhydrous Na₂SO₄, and concentrated. The crude product was stirred in 6 N HCl (10 mL) at room temperature for 2 h, then concentrated, and purified by column chromatography (EA/PE = 30/70) to give the target product 1-(1,1-dioxo-2,3-dihydrobenzo[b]thiophen-4-yl)ethanone.

LC-MS: (ESI, *m*/*z*): [M+H]⁺= 210.1.

### Step 4: preparation of (E)-N-(1-(1,1-dioxo-2,3-dihydrobenzo[b]thiophen-4-yl)ethylidene)-2-methylpropane-2-sulfinamide

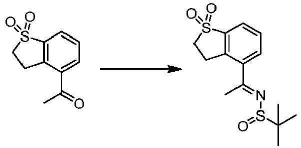

A reaction liquid of 1-(1,1-dioxo-2,3-dihydrobenzo[b]thiophen-4-yl)ethanone (2.0 g, 9.5 mmol), Ti(OEt)₄ (5.4 g, 1.6 mmol), and (*R*)-propane-2-sulfinamide (1.7 g, 14.2 mmol) in THF (40.0 mL) was stirred at 70 °C for reaction for 4.0 h. Ice water (100 mL) was added to quench the reaction, and the resulting mixture was extracted with EA (80 mL × 3). The organic phases were combined, washed with brine (30 mL), dried over anhydrous Na₂SO₄, and concentrated. The crude product was purified by column chromatography (EA/PE = 50/50) to give the target product (*E*)-*N*-(1-(1,1-dioxo-2,3-dihydrobenzo[b]thiophen-4-yl)ethylidene)-2-methylpropane-2-sulfinamide.

LC-MS: (ESI, *m*/*z):* [M+H]⁺= 314.2.

### Step 5: preparation of N-((R)-1-(1,1-dioxo-2,3-dihydrobenzo[b]thiophen-4-yl)ethyl)-2-methylpropane-2-sulfinamide

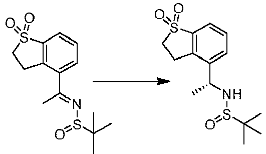

NaBH₄ (0.5 g, 12.6 mmol) was added to a solution of (*E*)-*N*-(1-(1,1-dioxo-2,3-dihydrobenzo[b]thiophen-4-yl)ethylidene)-2-methylpropane-2-sulfinamide (2.0 g, 6.3 mmol) in THF/H₂O (20.0 mL/4.0 mL) at 0 °C. The reaction liquid was stirred at 0 °C for reaction for 5.0 min. Ice water (100 mL) was added to quench the reaction, and the resulting mixture was extracted with EA (80 mL × 3). The organic phases were combined, washed with brine (40 mL), dried over anhydrous Na₂SO₄, and concentrated. The crude product was purified by column chromatography (EA/PE = 50/50) to give the target product *N*-((*R*)-1-(1,1-dioxo-2,3-dihydrobenzo[b]thiophen-4-yl)ethyl)-2-methylpropane-2-sulfinamide.

LC-MS: (ESI, *m*/*z):* [M+H]⁺= 316.3.

¹H NMR (400 MHz, CDCl₃) δ 7.71 (d, *J* = 7.7 Hz, 1H), 7.67 (d, *J* = 7.6 Hz, 1H), 7.53 (t, *J* = 7.6 Hz, 1H), 4.76 - 4.66 (m, 1H), 3.59 - 3.47 (m, 3H), 3.43 - 3.28 (m, 2H), 1.54 (d, *J* = 6.6 Hz, 3H), 1.24 (s, 9H).

### Step 6: preparation of (R)-4-(1-aminoethyl)-2,3-dihydrobenzo[b]thiophene 1,1-dioxohydrochloride

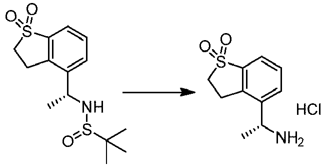

A solution of *N*-((*R*)-1-(1,1-dioxo-2,3-dihydrobenzo[b]thiophen-4-yl)ethyl)-2-methylpropane-2-sulfinamide in dioxane hydrochloride (4 N, 20.0 mL) was stirred at 25 °C for reaction for 2.0 h. The reaction liquid was concentrated. The residue was washed with PE (20 mL × 2), and the solid was dried to give the target product (*R*)-4-(1-aminoethyl)-2,3-dihydrobenzo[b]thiophene 1,1-dioxohydrochloride. The resulting crude product was directly used in the next step.

LC-MS: (ESI, *m*/*z*): [M+H]⁺= 211.2.

¹HNMR (400 MHz, DMSO-*d*₆) δ 8.56 (s, 2H), 7.94 (d, *J* = 7.6 Hz, 1H), 7.77 (d, *J* = 7.5 Hz, 1H), 7.67 (d, *J* = 7.7 Hz, 1H), 4.65 - 4.49 (m, 1H), 3.69 - 3.56 (m, 2H), 3.55-3.42 (m, 2H), 1.54 (d, *J* = 6.8 Hz, 3H).

### Step 7: preparation of methyl (1R,4R)-4-(4-(((R)-1-(1,1-dioxo-2,3-dihydrobenzo[b]thiophen-4-yl)ethyl)amino)-7-methoxy-2-methylquinazolin-6-yl)cyclohexane-1-carboxylate

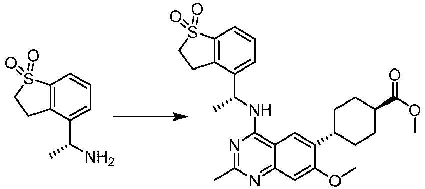

A reaction liquid of (*R*)-4-(1-aminoethyl)-2,3-dihydrobenzo[b]thiophene 1,1-dioxide (0.7 g, 3.3 mmol), DIEA (928.8 mg, 7.2 mmol), and methyl (1*R*,4*R*)-4-(4-chloro-7-methoxy-2-methylquinazolin-6-yl)cyclohexane-1-carboxylate (835.2 mg, 2.4 mmol) in dioxane (10.0 mL) was stirred at 110 °C for reaction for 20.0 h. Water (50 mL) was added to quench the reaction, and the resulting mixture was extracted with EA (30.0 mL × 3). The organic phases were combined, washed with brine (30 mL), dried over anhydrous Na₂SO₄, and concentrated. The crude product was purified by preparative HPLC (acetonitrile/0.05% aqueous HCOOH solution, 5% to 95%) to give the target product methyl (1*R*,4*R*)-4-(4-(((*R*)-1-(1,1-dioxo-2,3-dihydrobenzo[b]thiophen-4-yl)ethyl)amino)-7-methoxy-2-methylquinazolin-6-yl)cyclohexane-1-carboxylate.

LC-MS: (ESI, *m*/*z):* [M+H]⁺= 524.5.

### Step 8: preparation of (1R,4R)-4-(4-(((R)-1-(1,1-dioxo-2,3-dihydrobenzo[b]thiophen-4-yl)ethyl)amino)-7-methoxy-2-methylquinazolin-6-yl)cyclohexane-1-carboxylic acid

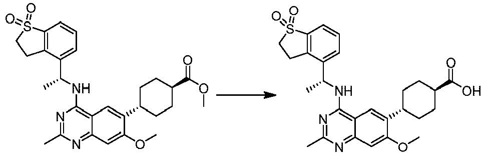

LiOH (24 mg, 1.0 mmol) was added to a solution of methyl (1*R*,4*R*)-4-(4-(((R)-1-(1,1-dioxo-2,3-dihydrobenzo[b]thiophen-4-yl)ethyl)amino)-7-methoxy-2-methylquinazolin-6-yl)cyclohexane-1-carboxylate (100.0 mg, 0.2 mmol) in THF/H₂O (2.0 mL/1.0 mL). The reaction liquid was stirred at 50 °C for reaction for 12.0 h, adjusted to pH 4-5 with 1 N HCl, and then concentrated to give the target product (1*R*,4*R*)-4-(4-(((*R*)-1-(1,1-dioxo-2,3-dihydrobenzo[b]thiophen-4-yl)ethyl)amino)-7-methoxy-2-methylquinazolin-6-yl)cyclohexane-1-carboxylic acid. The resulting crude product was directly used in the next step.

LC-MS: (ESI, *m*/*z):* [M+H]⁺= 510.1.

### Step 9: preparation of tert-butyl 4-((1R,4R)-4-(4-(((R)-1-(1,1-dioxo-2,3-dihydrobenzo[b]thiophen-4-yl)ethyl)amino)-7-methoxy-2-methylquinazolin-6-yl)cyclohexane-1-carbonyl)piperazine-1-carboxylate

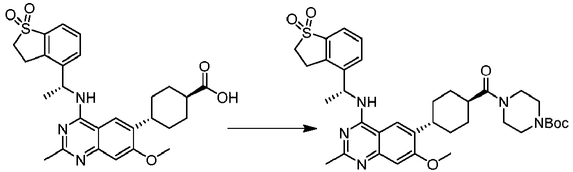

HATU (124.0 mg, 0.24 mmol) was added to a solution of (1*R*,4*R*)-4-(4-(((*R*)-1-(1,1-dioxo-2,3-dihydrobenzo[b]thiophen-4-yl)ethyl)amino)-7-methoxy-2-methylquinazolin-6-yl)cyclohexane-1-carboxylic acid (110.0 mg, 0.2 mmol), *tert*-butyl piperazine-1-carboxylate (45.0 mg, 0.22 mmol), and DIEA (85.0 mg, 0.6 mmol) in DMF (2.0 mL). The reaction liquid was stirred at room temperature for reaction for 2.0 h. Ice water (40 mL) was added to quench the reaction, and then the precipitate was filtered off. The filter cake was dried under vacuum to give the target product *tert*-butyl 4-((1*R*,4*R*)-4-(4-(((*R*)-1-(1,1-dioxo-2,3-dihydrobenzo[b]thiophen-4-yl)ethyl)amino)-7-methoxy-2-methylquinazofin-6-yl)cyclohexane-1-carbonyl)piperazine-1-carboxylate. The resulting crude product was directly used in the next step.

LC-MS: (ESI, *m*/*z*): [M+H]⁺= 678.1.

### Step 10: preparation of ((1R,4R)-4-(4-(((R)-1-(1,1-dioxo-2,3-dihydrobenzo[b]thiophen-4-yl)ethyl)amino)-7-methoxy-2-methylquinazolin-6-yl)cyclohexyl)(piperazin-1-yl)methanone

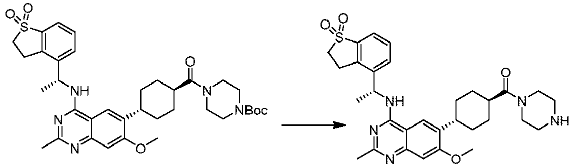

A reaction liquid of *tert*-butyl 4-((1*R*,4*R*)-4-(4-(((*R*)-1-(1,1-dioxo-2,3-dihydrobenzo[b]thiophen-4-yl)ethyl)amino)-7-methoxy-2-methylquinazolin-6-yl)cyclohexane-1-carbonyl)piperazine-1-carboxylate (110.0 mg, 0.1 mmol) in TFA/DCM (1.0 mL/3.0 mL) was stirred at room temperature for reaction for 1 h and then concentrated to give the target product ((1*R*,4*R*)-4-(4-(((*R*)-1-(1,1-dioxo-2,3-dihydrobenzo[b]thiophen-4-yl)ethyl)amino)-7-methoxy-2-methylquinazolin-6-yl)cyclohexyl)(piperazin-1-yl)methanone. The resulting crude product was directly used in the next step.

LC-MS: (ESI, *m*/*z):* [M+H]⁺= 578.2.

### Step 11: preparation of tert-butyl 9-((4-((1R,4R)-4-(4-(((R)-1-(1,1-dioxo-2,3-dihydrobenzo[b]thiophen-4-yl)ethyl)amino)-7-methoxy-2-methylquinazolin-6-yl)cyclohexane-1-carbonyl)piperazin-1-yl)methyl)-3-azaspiro 5.5 undecane-3 -carboxylate

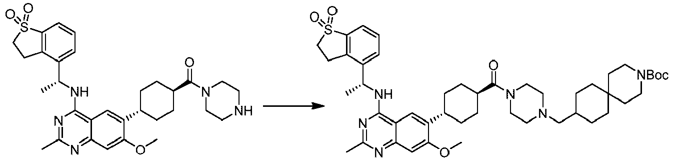

STAB (64.0 mg, 0.3 mmol) was added to a solution of ((1*R*,4*R*)-4-(4-(((*R*)-1-(1,1-dioxo-2,3-dihydrobenzo[b]thiophen-4-yl)ethyl)amino)-7-methoxy-2-methylquinazolin-6-yl)cyclohexyl)(piperazin-1-yl)methanone (60.0 mg, 0.1 mmol) and *tert*-butyl 9-formyl-3-azaspiro[5.5]undecane-3-carboxylate (35.0 mg, 0.12 mmol) in THF (5.0 mL). The reaction liquid was stirred at room temperature for reaction for 2.0 h. Ice water (10 mL) was added to quench the reaction, and the resulting mixture was extracted with EA (30.0 mL × 3). The combined organic phases were washed with brine (10 mL), dried over anhydrous Na₂SO₄, and concentrated. The crude product was purified by column chromatography (MeOH/DCM, 0-10%) to give the target product *tert*-butyl 9-((4-((1*R*,4*R*)-4-(4-(((*R*)-1-(1,1-dioxo-2,3-dihydrobenzo[b]thiophen-4-yl)ethyl)amino)-7-methoxy-2-methylquinazolin-6-yl)cyclohexane-1-carbonyl)piperazin-1-yl)methyl)-3-azaspiro[5.5]undecane-3-carboxylate.

LC-MS: (ESI, *m*/*z):* [M+H]⁺= 843.3.

### Step 12: preparation of (4-((3-azaspiro[5.5]undecane-9-yl)methyl)piperazin-1-yl)((1R,4R)-4-(4-(((R)-1-(1,1-dioxo-2,3-dihydrobenzo[b]thiophen-4-yl)ethyl)amino)-7-methoxy-2-methylquinazolin-6-yl)cyclohexyl)methanone

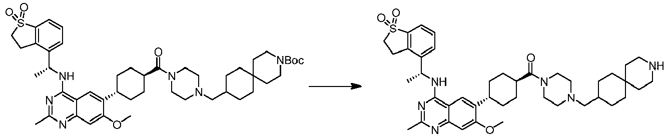

A reaction liquid of *tert*-butyl 9-((4-((1*R*,4*R*)-4-(4-(((*R*)-1-(1,1-dioxo-2,3-dihydrobenzo[b]thiophen-4-yl)ethyl)amino)-7-methoxy-2-methylquinazolin-6-yl)cyclohexane-1-carbonyl)piperazin-1-yl)methyl)-3-azaspiro[5.5]undecane-3-carboxylate (70.0 mg, 0.08 mmol) in TFA/DCM (1.0 mL/3.0 mL) was stirred at room temperature for reaction for 1.0 h and then concentrated to give the target product (4-((3-azaspiro[5.5]undecane-9-yl)methyl)piperazin-1-yl)((1*R*,4*R*)-4-(4-(((*R*)-1-(1,1-dioxo-2,3-dihydrobenzo[b]thiophen-4-yl)ethyl)amino)-7-methoxy-2-methylquinazolin-6-yl)cyclohexyl)methanone. The resulting crude product was directly used in the next step.

LC-MS: (ESI, *m*/*z):* [M+H]⁺= 743.3.

### Step 13: preparation of 1-(2-chloro-5-(9-((4-((1R,4R)-4-(4-(((R)-1-(1,1-dioxo-2,3-dihydrobenzo[b]thiophen-4-yl)ethyl)amino)-7-methoxy-2-methylquinazolin-6-yl)cyclohexane-1-carbonyl)piperazin-1-yl)methyl)-3-azaspiro[5.5]undecane-3-carbonyl)phenyl)dihydropyrimidine-2,4(1H,3H)-dione

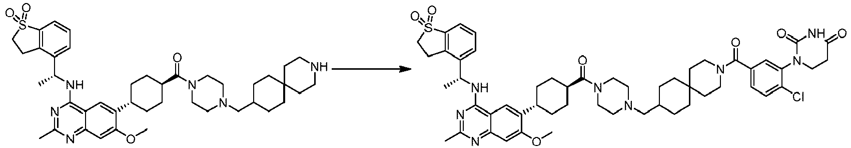

DIEA (31.20 mg, 0.24 mmol) and pentafluorophenyl 4-chloro-3-(2,4-dioxotetrahydropyrimidin-1(2*H*)-yl)benzoate (43.4 mg, 0.1 mmol) were added to a solution of (4-((3-azaspiro[5.5]undecane-9-yl)methyl)piperazin-1-yl)((1*R*,4*R*)-4-(4-(((*R*)-1-(1,1-dioxo-2,3-dihydrobenzo[b]thiophen-4-yl)ethyl)amino)-7-methoxy-2-methylquinazolin-6-yl)cyclohexyl)methanone (60.0 mg, 0.08 mmol) in DMSO (1.0 mL). The reaction liquid was stirred at room temperature for reaction for 2.0 h. Water was added to quench the reaction, and the resulting mixture was extracted with EA. The combined organic phases were washed with brine, dried over anhydrous Na₂SO₄, and concentrated. The crude product was purified by preparative HPLC (acetonitrile/0.05% aqueous NH₄HCO₃ solution, 5% to 95%) to give the target product 1-(2-chloro-5-(9-((4-((1*R*,4*R*)-4-(4-(((R)-1-(1,1-dioxo-2,3-dihydrobenzo[b]thiophen-4-yl)ethyl)amino)-7-methoxy-2-methylquinazolin-6-yl)cyclohexane-1-carbonyl)piperazin-1-yl)methyl)-3-azaspiro[5.5]undecane-3-carbonyl)phenyl)dihydropyrimidine-2,4(1*H*,3*H*)-dione.

LC-MS: (ESI, *m*/*z*): [M+H]⁺= 993.4.

¹H NMR (400 MHz, DMSO-*d*₆) δ 10.51 (s, 1H), 8.29 (d, *J =* 7.0 Hz, 1H), 8.06 (s, 1H), 7.80 (d, *J =* 7.5 Hz, 1H), 7.70-7.50 (m, 4H), 7.39 (dd, *J* = 8.2, 1.9 Hz, 1H), 6.98 (s, 1H), 5.65-5.55 (m, 1H), 3.87 (s, 3H), 3.81-3.70 (m, 2H), 3.68-3.52 (m, 4H), 3.54-3.42 (m, 5H), 2.98-2.91 (m, 1H), 2.7-2.71 (m, 2H), 2.70-2.62 (m, 1H), 2.39-2.22 (m, 7H), 2.18-2.10 (m, 2H), 1.91-1.77 (m, 4H), 1.75-1.18 (m, 19H), 1.13-1.00 (m, 4H).

### Example 11: 1-(5-(4-(3-(4-((1R,4R)-4-(4-(((R)-1-(3-amino-5-(trifluoromethyl)phenyl)ethyl)amino)-7-methoxy-2-methylquinazolin-6-yl)cyclohexane-1 -carbonyl)piperazin-1 -yl)propyl)piperidine-1 -carbonyl)-2-chlorophenyl)dihydropyrimidine-2,4(1H,3H)-dione

### Step 1: preparation of benzyl 4-(3-(1-(tert-butoxycarbonyl)piperidin-4-yl)propyl)piperazine-1-carboxylate

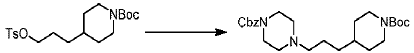

A reaction liquid of *tert*-butyl 4-(3-(toluenesulfonyloxy)propyl)piperidine-1-carboxylate (500 mg, 2.3 mmol), benzyl piperazine-1-carboxylate (1.0 g, 2.7 mmol), K₂CO₃ (1.5 g, 11.3 mmol), and KI (450.0 mg, 2.7 mmol) in DMF (10.0 mL) was stirred at 70 °C for reaction overnight. Ice water (100 mL) was added to quench the reaction, and the resulting mixture was extracted with EA (30 mL × 3). The combined organic phases were washed with brine (15 mL), dried over anhydrous Na₂SO₄, and concentrated. The crude product was purified by column chromatography (EA/PE = 0-30%) to give the target product benzyl 4-(3-(1-(*tert*-butoxycarbonyl)piperidin-4-yl)propyl)piperazine-1 -carboxylate.

LC-MS: (ESI, *m*/*z):* [M+H]⁺= 446.5.

### Step 2: preparation of tert-butyl 4-(3-(piperazin-1-yl)propyl)piperidine-1-carboxylate

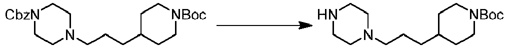

A reaction liquid of benzyl 4-(3-(1-(*tert*-butoxycarbonyl)piperidin-4-yl)propyl)piperazine-1-carboxylat\e (400.0 mg, 0.6 mmol) and Pd(OH)₂/C (10%, 100.0 mg) in EtOAc (20.0 mL) was stirred at 70 °C under H₂ (60 psi) for reaction for 12.0 h and then filtered through celite. The filter cake was washed with EA (10 mL × 2), and the filtrates were combined and concentrated to give the target product *tert*-butyl 4-(3-(piperazin-1-yl)propyl)piperidine-1-carboxylate. The resulting crude product was directly used in the next step.

LC-MS: (ESI, *m*/*z):* [M+H]⁺= 312.2.

### Step 3: preparation of tert-butyl 4-(3-(4-((1R,4R)-4-(7-methoxy-2-methyl-4-(((R)-1-(3-nitro-5-(trifluoromethyl)phenyl)ethyl)amino)quinazolin-6-yl)cyclohexane-1 -carbonyl)piperazin-1 -yl)propyl)piperidine-1 - carboxylate

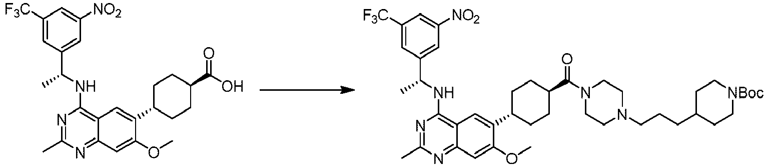

HATU (91.0 mg, 0.24 mmol) was added to a solution of (1*R*,4*R*)-4-(7-methoxy-2-methyl-4-(((*R*)-1-(3-nitro-5-(trifluoromethyl)phenyl)ethyl)amino)quinazolin-6-yl)cyclohexyl-1-carboxyfic acid (85.0 mg, 0.2 mmol), *tert*-butyl 4-(3-(piperazin-1-yl)propyl)piperidine-1-carboxylate (68.0 mg, 0.22 mmol), and DIEA (59.0 mg, 0.6 mmol) in DMF (2.0 mL). The reaction liquid was stirred at room temperature for reaction for 2.0 h. Ice water (20 mL) was added to quench the reaction, and the resulting mixture was filtered. The precipitate was collected, washed with water (10 mL × 2), and dried to give the target product *tert*-butyl 4-(3-(4-((1*R*,4*R*)-4-(7-methoxy-2-methyl-4-(((*R*)-1 -(3 -nitro-5 -(trifhioromethyl)phenyl)ethyl)amino)quinazolin-6-yl)cyclohexane-1 -carbonyl)piperazin-1 - yl)propyl)piperidine-1 -carboxylate.

LC-MS: (ESI, *m*/*z):* [M+H]⁺= 826.5.

### Step 4: preparation of tert-butyl 4-(3-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)propyl)piperidine-1-carboxylate

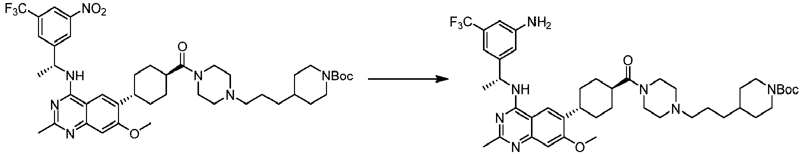

A reaction liquid of *tert*-butyl 4-(3-(4-((1*R*,4*R*)-4-(7-methoxy-2-methyl-4-(((*R*)-1-(3-nitro-5-(trifluoromethyl)phenyl)ethyl)amino)quinazolin-6-yl)cyclohexyl-1 -carbonyl)piperazin-1 -yl)propyl)piperidine-1 - carboxylate (130.0 mg, 0.18 mmol) and 10% Pd/C (30.0 mg) in MeOH (10.0 mL) was stirred at 60 °C under H₂ (90 psi) for reaction overnight and then filtered through celite. The filtrate was concentrated to give the target product *tert*-butyl 4-(3-(4-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperazin-1-yl)propyl)piperidine-1-carboxylate. The resulting crude product was directly used in the next step.

LC-MS: (ESI, m/z): [M+H]⁺= 796.5.

### Step 5: preparation of ((1R,4R)-4-(4-(((R)-1-(3-amino-5-(trifluoromethyl)phenyl)ethyl)amino)-7-methoxy-2-methylquinazolin-6-yl)cyclohexyl)(4-(3-(piperidin-4-yl)propyl)piperazin-1-yl)methanone

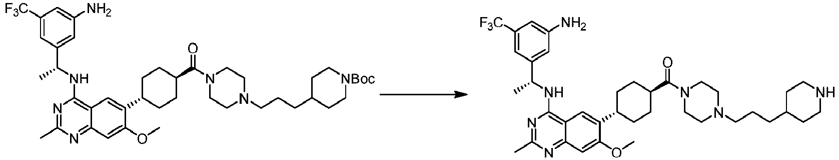

A reaction liquid of *tert*-butyl 4-(3-(4-((1*R*,4*R*)-4-(4-(((*R*)-1-(3-amino-5-(trifluoromethyl)phenyl)ethyl)amino)-7-methoxy-2-methylquinazolin-6-yl)cyclohexyl-1-carbonyl)piperazin-1-yl)propyl)piperidine-1-carboxylate (100.0 mg, 0.1 mmol) in TFA/DCM (1.0 mL/3.0 mL) was stirred at room temperature for reaction for 1 h and then concentrated to give the target product ((1*R*,4*R*)-4-(4-(((*R*)-1-(3-amino-5-(trifluoromethyl)phenyl)ethyl)amino)-7-methoxy-2-methylquinazolin-6-yl)cyclohexyl)(4-(3-(piperidin-4-yl)propyl)piperazin-1-yl)methanone.

LC-MS: (ESI, m/z): [M+H]⁺= 696.3.

### Step 6: preparation of 1-(5-(4-(3-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)propyl)piperidine-1-carbonyl)-2-chlorophenyl)dihydropyrimidine-2,4(1H,3H)-dione

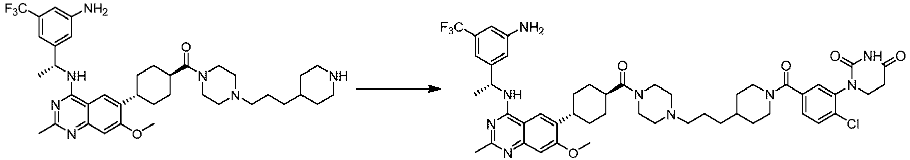

DIEA (103 mg, 0.8 mmol) and pentafluorophenyl 4-chloro-3-(2,4-dioxotetrahydropyrimidin-1(2*H*)-yl)benzoate (73 mg, 0.17 mmol) were added to a solution of ((1*R*,4*R*)-4-(4-(((*R*)-1-(3-amino-5-(trifluoromethyl)phenyl)ethyl)amino)-7-methoxy-2-methylquinazolin-6-yl)cyclohexyl)(4-(3-(piperidin-4-yl)propyl)piperazin-1-yl)methanone (100 mg, 0.14 mmol) in DMSO (5.0 mL), and the reaction liquid was stirred at room temperature for reaction for 2.0 h. Water (15 mL) was added to quench the reaction, and the resulting mixture was extracted with EA (30.0 mL × 3). The organic phases were combined, washed with brine (15 mL), and dried over anhydrous Na₂SO₄, and the reaction liquid was concentrated. The crude product was purified by preparative HPLC (acetonitrile/0.05% aqueous NH₄HCO₃ solution, 5% to 95%) to give the target product 1-(5-(4-(3-(4-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperazin-1-yl)propyl)piperidine-1-carbonyl)-2-chlorophenyl)dihydropyrimidine-2,4(1*H*,3*H*)-dione.

LC-MS: (ESI, m/z): [M+H]⁺= 946.4.

¹H NMR (400 MHz, DMSO-*d*₆) δ 10.51 (s, 1H), 8.13 (d, *J* = 5.5 Hz, 1H), 8.06 (s, 1H), 7.63 (d, *J* = 8.2 Hz, 1H), 7.54 (d, *J* = 1.6 Hz, 1H), 7.38 (d, *J =* 8.4 Hz, 1H), 6.99 (s, 1H), 6.88 (s, 1H), 6.86 (s, 1H), 6.70 (s, 1H), 5.63 - 5.50 (m, 3H), 4.44 (s, 1H), 3.89 (s, 3H), 3.79-3.74 (m, 1H), 3.64 - 3.59 (m, 1H), 3.48-3.41 (m, 4H), 3.18-2.90 (m, 2H), 2.78 - 2.62 (m, 4H), 2.38-2.21 (m, 9H), 1.90 - 1.43 (m, 17H), 1.24-1.19 (m, 2H), 1.10-1.08 (m, 2H).

### Example 12: 1-(5-(4-(2-(4-((1R,4R)-4-(4-(((R)-1-(3-amino-5-(trifluoromethyl)phenyl)ethyl)amino)-7-methoxy-2-methylquinazolin-6-yl)cyclohexane-1 -carbonyl)piperazin-1 -yl)ethyl)piperidine-1 -carbonyl)-2-chlorophenyl)dihydropyrimidine-2,4(1H,3H)-dione

### Step 1: preparation of benzyl 4-(2-(1-(tert-butoxycarbonyl)piperidin-4-yl)ethyl)piperazine-1-carboxylate

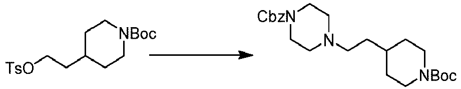

A reaction liquid of *tert*-butyl 4-(2-(toluenesulfonyloxy)ethyl)piperidine-1-carboxylate (500.0 mg, 2.3 mmol), benzyl piperazine-1-carboxylate (1.0 g, 2.7 mmol), K₂CO₃ (1.5 g, 11.3 mmol), and KI (450.0 mg, 2.7 mmol) in DMF (10.0 mL) was stirred at 70 °C for reaction overnight. Ice water (100 mL) was added to quench the reaction, and the resulting mixture was extracted with EA (30.0 mL × 3). The combined organic phases were washed with brine (20 mL), dried over anhydrous Na₂SO₄, and concentrated. The crude product was purified by column chromatography (EA/PE = 0-30%) to give the target product benzyl 4-(2-(1-(*tert*-butoxycarbonyl)piperidin-4-yl)ethyl)piperazine-1 -carboxylate.

LC-MS: (ESI, m/z): [M+H]⁺= 432.7.

### Step 2: preparation of tert-butyl 4-(2-(piperazin-1-yl)ethyl)piperidine-1-carboxylate

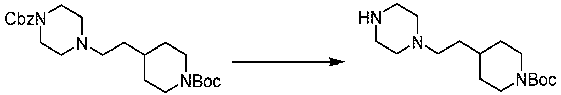

A reaction liquid of benzyl 4-(2-(1-(*tert*-butoxycarbonyl)piperidin-4-yl)ethyl)piperazine-1-carboxylate (400.0 mg, 0.6 mmol) and 10% Pd(OH)₂/C (100 mg) in EtOAc (20 mL) was stirred at 70 °C under H₂ (60 psi) for reaction for 12 h and then filtered through celite. The filter cake was washed with EA (10 mL × 2), and the filtrate was concentrated to give the target product *tert*-butyl 4-(2-(piperazin-1-yl)ethyl)piperidine-1-carboxylate.

LC-MS: (ESI, m/z): [M+H]⁺= 298.2.

### Step 3: preparation of tert-butyl 4-(2-(4-((1R,4R)-4-(7-methoxy-2-methyl-4-(((R)-1-(3-nitro-5-(trifluoromethyl)phenyl)ethyl)amino)quinazolin-6-yl)cyclohexane-1 -carbonyl)piperazin-1-yl)ethyl)piperidine-1 - carboxylate

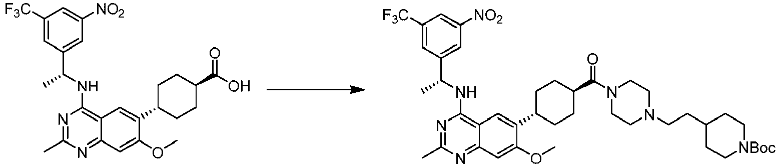

HATU (91.0 mg, 0.24 mmol) was added to a solution of (1*R*,4*R*)-4-(7-methoxy-2-methyl-4-(((*R*)-1-(3-nitro-5-(trifluoromethyl)phenyl)ethyl)amino)quinazolin-6-yl)cyclohexane-1-carboxylic acid (85.0 mg, 0.2 mmol), *tert-*butyl 4-(2-(piperazin-1-yl)ethyl)piperidine-1-carboxylate (68.0 mg, 0.22 mmol), and DIEA (59.0 mg, 0.6 mmol) in DMF (2.0 mL). The reaction liquid was stirred at room temperature for reaction for 2 h. Ice water (20.0 mL) was added to quench the reaction, and the resulting mixture was filtered. The precipitate was collected, washed with water (10 mL × 2), and dried under vacuum to give the target product *tert*-butyl 4-(2-(4-((1*R*,4*R*)-4-(7-methoxy-2-methyl-4-(((*R*)-1-(3-nitro-5-(trifluoromethyl)phenyl)ethyl)amino)quinazolin-6-yl)cyclohexane-1-carbonyl)piperazin-1-yl)ethyl)piperidine-1-carboxylate.

LC-MS: (ESI, m/z): [M+H]⁺= 812.5.

### Step 4: preparation of tert-butyl 4-(2-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)ethyl)piperidine-1-carboxylate

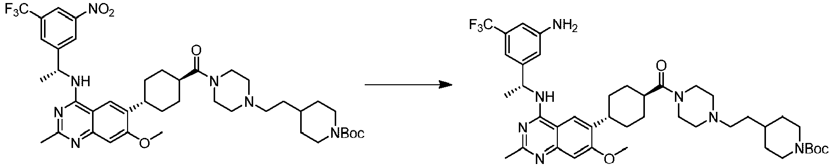

A reaction liquid of *tert*-butyl 4-(2-(4-((1*R*,4*R*)-4-(7-methoxy-2-methyl-4-(((*R*)-1-(3-nitro-5-(trifluoromethyl)phenyl)ethyl)amino)quinazolin-6-yl)cyclohexane-1 -carbonyl)piperazin-1 -yl)ethyl)piperidine-1 - carboxylate (130 mg, 0.18 mmol) and 10% Pd/C (30 mg) in MeOH (10 mL) was stirred at 60 °C under H₂ (90 psi) for reaction overnight and then filtered through celite. The filter cake was washed with methanol (5 mL × 2), and the filtrates were combined and concentrated to give the target product tert-butyl 4-(2-(4-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperazin-1 -yl)ethyl)piperidine-1 -carboxylate.

LC-MS: (ESI, m/z): [M+H]⁺= 782.5.

### Step 5: preparation of ((1R,4R)-4-(4-(((R)-1-(3-amino-5-(trifluoromethyl)phenyl)ethyl)amino)-7-methoxy-2-methylquinazolin-6-yl)cyclohexyl)(4-(2-(piperidin-4-yl)ethyl)piperazin-1-yl)methanone

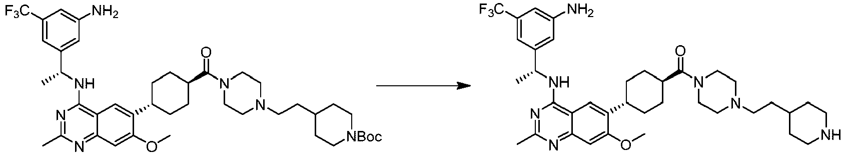

A reaction liquid of *tert*-butyl 4-(2-(4-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperazin-1-yl)ethyl)piperidine-1-carboxylate (100 mg, 0.1 mmol) in TFA/DCM (1 mL/3 mL) was stirred at room temperature for reaction for 1.0 h and then concentrated to give the target product ((1*R*,4*R*)-4-(4-(((*R*)-1-(3-amino-5-(trifluoromethyl)phenyl)ethyl)amino)-7-methoxy-2-methylquinazolin-6-yl)cyclohexyl)(4-(2-(piperidin-4-yl)ethyl)piperazin-1-yl)methanone.

LC-MS: (ESI, m/z): [M+H]⁺= 682.2.

### Step 6: preparation of 1-(5-(4-(2-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)ethyl)piperidine-1-carbonyl)-2-chlorophenyl)dihydropyrimidine-2,4(1H,3H)-dione

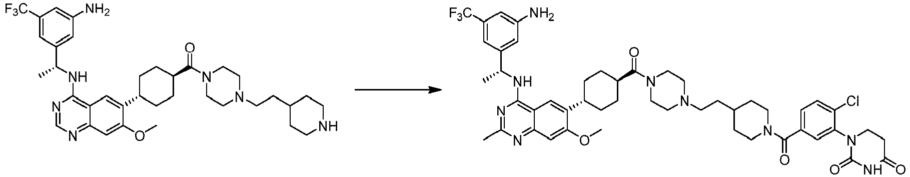

DIEA (103 mg, 0.8 mmol) and pentafluorophenyl 4-chloro-3-(2,4-dioxotetrahydropyrimidin-1(2*H*)-yl)benzoate (73 mg, 0.17 mmol) were added to a solution of ((1*R*,4*R*)-4-(4-(((*R*)-1-(3-amino-5-(trifluoromethyl)phenyl)ethyl)amino)-7-methoxy-2-methylquinazolin-6-yl)cyclohexyl)(4-(2-(piperidin-4-yl)ethyl)piperazin-1-yl)methanone (100 mg, 0.14 mmol) in DMSO (5.0 mL), and the reaction liquid was stirred at room temperature for reaction for 2.0 h. Water (15 mL) was added to quench the reaction, and the resulting mixture was extracted with EA (30 mL × 3). The combined organic phases were washed with brine (20 mL), dried over anhydrous Na₂SO₄, and concentrated. The crude product was purified by preparative HPLC (acetonitrile/0.05% aqueous NH₄HCO₃ solution, 5% to 95%) to give the target product 1-(5-(4-(2-(4-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperazin-1-yl)ethyl)piperidine-1-carbonyl)-2-chlorophenyl)dihydropyrimidine-2,4(1*H*,3*H*)-dione.

LC-MS: (ESI, m/z): [M+H]⁺= 932.4.

¹H NMR (400 MHz, DMSO-*d*₆) δ 10.51 (s, 1H), 8.12 (d, *J =* 7.6 Hz, 1H), 8.06 (s, 1H), 7.64 (d, *J =* 8.2 Hz, 1H), 7.54 (s, 1H), 7.38 (d, *J* = 8.3 Hz, 1H), 6.99 (s, 1H), 6.88 (s, 1H), 6.85 (s, 1H), 6.70 (s, 1H), 5.65-5.50 (m, 3H), 4.50-4.40 (m, 1H), 3.88 (s, 3H), 3.78-3.72 (m, 1H), 3.68-3.61 (m, 1H), 3.52-3.41 (m, 4H), 3.07-2.91 (m, 2H), 2.77-2.60 (m, 3H), 2.38-2.30 (m, 9H), 1.88-1.52 (m, 16H), 1.45-1.35 (m, 2H), 1.28-1.01 (m, 2H).

Example 13: 1-(5-(4-((4-((1*R*,4*R*)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)methyl)piperidine-1-carbonyl)-2-chlorophenyl)dihydropyrimidine-2,4(1*H*,3*H*)-dione

### Step 1: preparation of benzyl 4-((1-(tert-butoxycarbonyl)piperidin-4-yl)methyl)piperazine-1-carboxylate

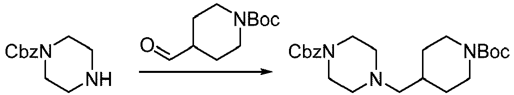

STAB (1.2 g, 5.5 mmol) was slowly added to a solution of benzyl piperazine-1-carboxylate (500.0 mg, 2.27 mmol) and *tert*-butyl 4-formylpiperidine-1-carboxylate (726.5.0 mg, 3.4 mmol) in THF (10.0 mL), and the reaction liquid was stirred at room temperature for reaction overnight. Water (20 mL) was added to quench the reaction, and the resulting mixture was extracted with EA (20 mL × 2). The organic phase was dried over anhydrous Na₂SO₄ and concentrated to give the crude product. The crude product was purified by column chromatography (EA/PE = 10/90) to give the target product benzyl 4-((1-(*tert*-butoxycarbonyl)piperidin-4-yl)methyl)piperazine-1-carboxylate.

LC-MS: (ESI, m/z): [M+H]⁺= 418.1.

### Step 2: preparation of tert-butyl 4-(piperazin-1-ylmethyl)piperidine-1-carboxylate

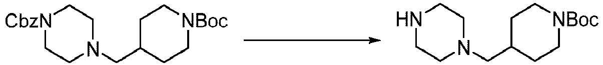

Pd(OH)₂/C (300.0 mg, 0.36 mmol) was added to a solution of benzyl 4-((1-(*tert*-butoxycarbonyl)piperidin-4-yl)methyl)piperazine-1-carboxylate in EtOAc (15.0 mL), and the reaction liquid was stirred at 80 °C under H₂ atmosphere for reaction overnight, filtered, and concentrated to give the target product *tert*-butyl 4-(piperazin-1-ylmethyl)piperidine-1-carboxylate. The resulting crude product was directly used in the next step.

LC-MS: (ESI, m/z): [M+H]⁺= 284.1.

### Step 3: preparation of (1R,4R)-4-(7-methoxy-2-methyl-4-(((R)-1-(3-nitro-5-(trifluoromethyl)phenyl)ethyl)amino)quinazolin-6-yl)cyclohexane-1 -carboxylic acid

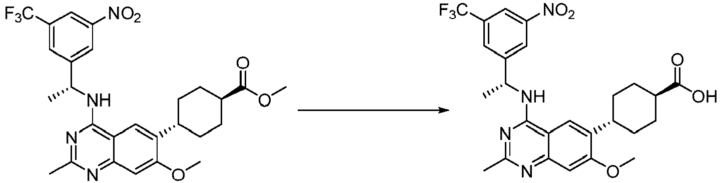

LiOH (9.60 mg, 0.4 mmol) was added to a solution of methyl (1*R*,4*R*)-4-(7-methoxy-2-methyl-4-(((*R*)-1-(3-nitro-5-(trifluoromethyl)phenyl)ethyl)amino)quinazolin-6-yl)cyclohexane-1-carboxylate (100.0 mg, 0.2 mmol) in THF/H₂O (1:1), and the reaction liquid was stirred at room temperature for reaction for 16 h, adjusted to pH 4-5 with 1 N HCl, and then extracted with a mixed solvent of MeOH/CH₂Cl₂ (1/10, 10.0 mL × 2). The organic phase was dried over Na₂SO₄ and concentrated to give the target product (1*R*,4*R*)-4-(7-methoxy-2-methyl-4-(((*R*)-1-(3-nitro-5-(trifluoromethyl)phenyl)ethyl)amino)quinazolin-6-yl)cyclohexane-1-carboxylic acid. The resulting crude product was directly used in the next step.

LC-MS: (ESI, m/z): [M+H]⁺= 533.1.

### Step 4: preparation of tert-butyl 4-((4-((1R,4R)-4-(7-methoxy-2-methyl-4-(((R)-1-(3-nitro-5-(trifluoromethyl)phenyl)ethyl)amino)quinazolin-6-yl)cyclohexane-1-carbonyl)piperazin-1-yl)methyl)piperidine-1-carboxylate

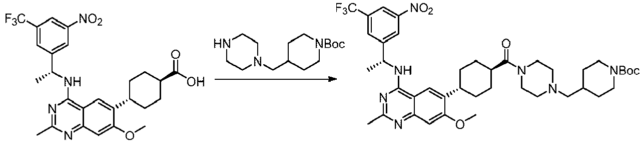

DIEA (65.20 mg, 0.52 mmol), HATU (197.6 mg, 0.52 mmol), and *tert*-butyl 4-(piperazin-1-ylmethyl)piperidine-1-carboxylate (57.6 mg, 0.2 mmol) were added to a solution of (1*R*,4*R*)-4-(7-methoxy-2-methyl-4-(((*R*)-1-(3-nitro-5-(trifluoromethyl)phenyl)ethyl)amino)quinazolin-6-yl)cyclohexane-1-carboxylic acid (90.00 mg, 0.17 mmol) in DMF (5.0 mL). The reaction liquid was stirred at room temperature for reaction for 5 h and then diluted with water (40 mL), and the resulting mixture was extracted with EA (20 mL × 3). The organic phase was dried over Na₂SO₄ and concentrated. The resulting crude product was purified by column chromatography (EA/PE = 50/50) to give the target product *tert*-butyl 4-((4-((1*R*,4*R*)-4-(7-methoxy-2-methyl-4-(((R)-1-(3-nitro-5-(trifluoromethyl)phenyl)ethyl)amino)quinazolin-6-yl)cyclohexane-1 -carbonyl)piperazin-1 -yl)methyl)piperidine-1 - carboxylate.

LC-MS: (ESI, m/z): [M+H]⁺= 798.2.

### Step 5: preparation of tert-butyl 4-((4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)methyl)piperidine-1-carboxylate

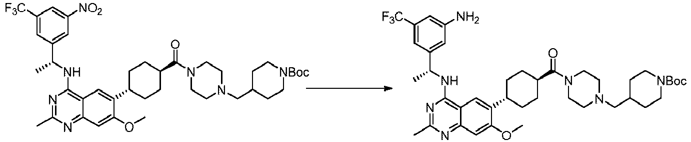

Pd/C (15.0 mg) was added to a solution of *tert*-butyl 4-((4-((1*R*,4*R*)-4-(7-methoxy-2-methyl-4-(((*R*)-1-(3-nitro-5-(trifluoromethyl)phenyl)ethyl)amino)quinazolin-6-yl)cyclohexane-1-carbonyl)piperazin-1-yl)methyl)piperidine-1-carboxylate (40 mg, 0.05 mmol) in EA (15.0 mL). The reaction liquid was stirred at 80 °C under H₂ atmosphere for reaction for 16 h and then filtered. The filter cake was washed with EA (10 mL × 2). The organic phase was concentrated to give the target product *tert*-butyl 4-((4-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperazin-1-yl)methyl)piperidine-1-carboxylate. The resulting crude product was directly used in the next step.

LC-MS: (ESI, m/z): [M+H]⁺= 768.3.

### Step 6: preparation of ((1R,4R)-4-(4-(((R)-1-(3-amino-5-(trifluoromethyl)phenyl)ethyl)amino)-7-methoxy-2-methylquinazolin-6-yl)cyclohexyl)(4-(piperidin-4-ylmethyl)piperazin-1-yl)methanone

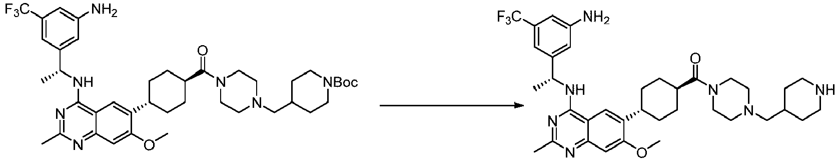

A solution of *tert*-butyl 4-((4-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperazin-1-yl)methyl)piperidine-1-carboxylate (30 mg, 0.04 mmol) in TFA/DCM (3.0 mL, 1:5) was stirred at room temperature for reaction for 2 h. The reaction liquid was concentrated to give the target product ((1*R*,4*R*)-4-(4-(((*R*)-1-(3-amino-5-(trifluoromethyl)phenyl)ethyl)amino)-7-methoxy-2-methyl quinazolin-6-yl)cyclohexyl)(4-(piperidin-4-ylmethyl)piperazin-1 -yl)methanone. The resulting crude product was directly used in the next step.

LC-MS: (ESI, m/z): [M+H]⁺= 668.3.

### Step 7: preparation of 1-(5-(4-((4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)methyl)piperidine-1-carbonyl)-2-chlorophenyl)dihydropyrimidine-2,4(1H,3H)-dione

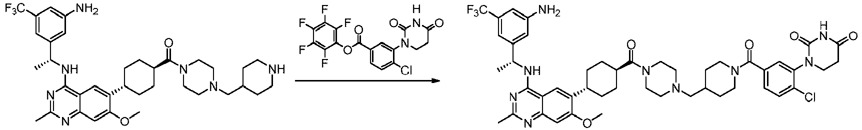

DIEA (14.50 mg, 0.112 mmol) and pentafluorophenyl 4-chloro-3-(2,4-dioxotetrahydropyrimidin-1(2*H*)-yl)benzoate (19.30 mg, 0.044 mmol) were added to a solution of ((1*R*,4*R*)-4-(4-(((*R*)-1-(3-amino-5-(trifluoromethyl)phenyl)ethyl)amino)-7-methoxy-2-methylquinazolin-6-yl)cyclohexyl)(4-(piperidin-4-ylmethyl)piperazin-1-yl)methanone (25.0 mg, 0.037 mmol) in DMSO (5.0 mL). The reaction liquid was stirred at room temperature for reaction for 5 h and then diluted with water (50 mL), and the resulting mixture was extracted with EtOAc (25 mL × 3). The organic phases were combined, washed with brine (10 mL), dried over Na₂SO₄, and concentrated. The crude product was subjected to preparative HPLC (acetonitrile/0.05% aqueous NH₄HCO₃ solution, 5% to 95%) to give the target product 1-(5-(4-((4-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperazin-1-yl)methyl)piperidine-1-carbonyl)-2-chlorophenyl)dihydropyrimidine-2,4(1*H*,3*H*)-dione.

LC-MS: (ESI, m/z): [M+H]⁺= 918.4.

¹H NMR (400 MHz, DMSO-*d*₆) δ 10.51 (s, 1H), 8.20-8.04 (m, 2H), 7.64 (s, 1H), 7.54 (s, 1H), 7.38 (s, 1H), 6.99 (s, 1H), 6.93-6.83 (m, 2H), 6.70 (s, 1H), 5.60-5.45 (m, 3H), 4.54-4.44 (m, 1H), 3.98-3.80 (m, 4H), 3.80-3.67 (m, 2H), 3.05-2.90 (m, 2H), 2.84-2.60 (m, 5H), 2.45-2.10 (m, 10H), 1.92-1.40 (m, 16H), 1.15-0.98 (m, 2H).

### Example 14: 1-(5-(4-(3-(1-((1R,4R)-4-(4-(((R)-1-(3-amino-5-(trifluoromethyl)phenyl)ethyl)amino)-7-methoxy-2-methylquinazolin-6-yl)cyclohexane-1 -carbonyl)piperidin-4-yl)propyl)piperidine-1 -carbonyl)-2-chlorophenyl)dihydropyrimidine-2,4(1H,3H)-dione

### Step 1: preparation of benzyl 4-(3-(piperidin-4-yl)propyl)piperidine-1-carboxylate

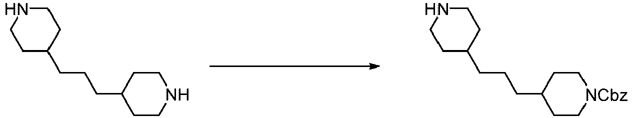

1,3-Di(piperidin-4-yl)propane (6.0 g, 28.6 mmol) was dissolved in acetonitrile/water (1:1, 720 mL), and NaHCO₃ (12.21 g, 71.5 mmol) and CbzCl (4.88 g, 28.6 mmol) were added. The resulting mixture was stirred at room temperature overnight, concentrated under reduced pressure, and extracted with MeOH/DCM (1/10, 200 mL × 5). The organic phase was dried over anhydrous sodium sulfate, filtered, and concentrated under reduced pressure. The crude product was purified by column chromatography (20% MeOH/DCM, 0.1% ammonia water) to give the target product benzyl 4-(3-(piperidin-4-yl)propyl)piperidine-1-carboxylate.

LC-MS: (ESI, m/z): [M+H]⁺= 345.2.

### Step 2: preparation of benzyl 4-(3-(1-((1R,4R)-4-(7-methoxy-2-methyl-4-(((R)-1-(3-nitro-5-(trifluoromethyl)phenyl)ethyl)amino)quinazolin-6-yl)cyclohexane-1 -carbonyl)piperidin-4-yl)propyl)piperidine-1 - carboxylate

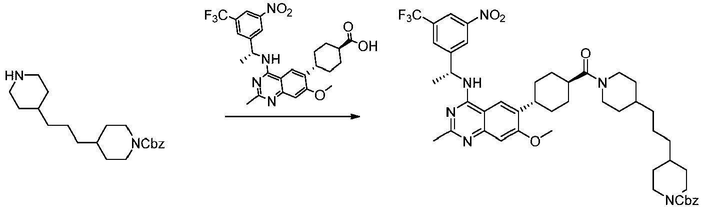

Benzyl 4-(3-(piperidin-4-yl)propyl)piperidine-1-carboxylate (116 mg, 0.337 mmol), HATU (110 mg, 0.292 mmol), and DIEA (87 mg, 0.675 mmol) were added to a solution of (1*R*,4*R*)-4-(7-methoxy-2-methyl-4-(((R)-1-(3-nitro-5-(trifluoromethyl)phenyl)ethyl)amino)quinazolin-6-yl)cyclohexane-1-carboxylic acid (120 mg, 0.225 mmol) in DMF (10 mL). The reaction liquid was stirred at room temperature for 2 h, and water (100 mL) was added. The resulting mixture was stirred for 10 min and extracted with ethyl acetate (100 mL × 3). The organic phase was washed with saturated brine (150 mL), dried over anhydrous sodium sulfate, filtered, and concentrated under reduced pressure. The crude product was purified by column chromatography (100% EA/PE) to give the target product benzyl 4-(3-(1-((1*R*,4*R*)-4-(7-methoxy-2-methyl-4-(((*R*)-1-(3-nitro-5-(trifluoromethyl)phenyl)ethyl)amino)quinazolin-6-yl)cyclohexane-1 -carbonyl)piperidin-4-yl)propyl)piperidine-1 - carboxylate.

LC-MS: (ESI, m/z): [M+H]⁺= 859.5.

### Step 3: preparation of benzyl 4-(3-(1-((1R,4R)-4-(4-(((R)-1-(3-amino-5-(trifluoromethyl)phenyl)ethyl)amino)-7-methoxy-2-methyl quinazolin-6-yl)cyclohexane-1 -carbonyl)piperidin-4-yl)propyl)piperidine-1 -carboxylate

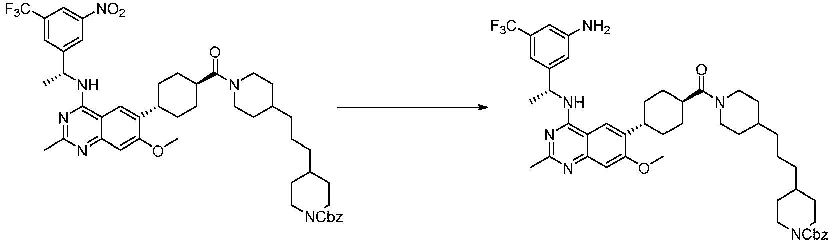

Raney-Ni (0.2 mL) and N₂H₄-H₂O (0.2 mL) were added to a solution of benzyl 4-(3-(1-((1*R*,4*R*)-4-(7-methoxy-2-methyl-4-(((*R*)-1-(3-nitro-5-(trifluoromethyl)phenyl)ethyl)amino)quinazolin-6-yl)cyclohexane-1-carbonyl)piperidin-4-yl)propyl)piperidine-1-carboxylate (110 mg, 0.13 mmol) in EtOH (10 mL). The resulting mixture was stirred at room temperature for 0.5 h, filtered, and concentrated under reduced pressure to give the product benzyl 4-(3-(1-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperidin-4-yl)propyl)piperidine-1-carboxylate.

LC-MS: (ESI, m/z): [M+H]⁺= 829.4.

### Step 4: preparation of ((1R,4R)-4-(4-(((R)-1-(3-amino-5-(trifluoromethyl)phenyl)ethyl)amino)-7-methoxy-2-methylquinazolin-6-yl)cyclohexyl)(4-(3-(piperidin-4-yl)propyl)piperidin-1-yl)methanone

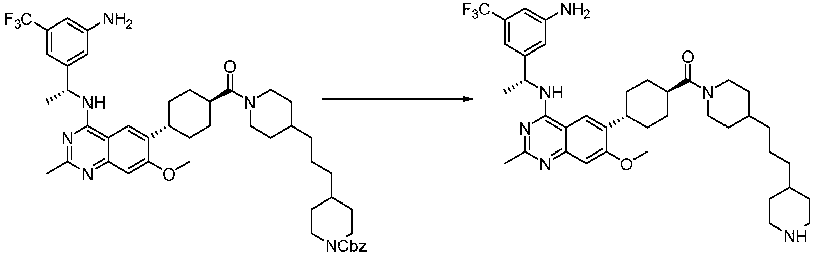

Pd(OH)₂/C (10 mg) was added to a solution of benzyl 4-(3-(1-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperidin-4-yl)propyl)piperidine-1-carboxylate (85 mg, 0.1 mmol) in ethyl acetate (10 mL). The resulting mixture was stirred at 75 °C under hydrogen atmosphere for 8 h, filtered, and concentrated under reduced pressure to give ((1*R*,4*R*)-4-(4-(((*R*)-1-(3-amino-5-(trifluoromethyl)phenyl)ethyl)amino)-7-methoxy-2-methylquinazolin-6-yl)cyclohexyl)(4-(3-(piperidin-4-yl)propyl)piperidin-1-yl)methanone.

LC-MS: (ESI, m/z): [M+H]⁺= 695.4.

### Step 5: preparation of 1-(5-(4-(3-(1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)propyl)piperidine-1-carbonyl)-2-chlorophenyl)dihydropyrimidine-2,4(1H,3H)-dione

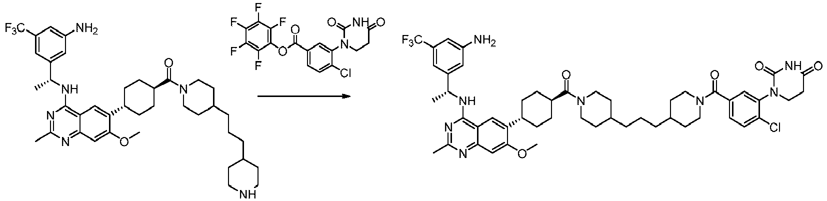

((1*R*,4*R*)-4-(4-(((R)-1-(3-Amino-5-(trifluoromethyl)phenyl)ethyl)amino)-7-methoxy-2-methylquinazolin-6-yl)cyclohexyl)(4-(3-(piperidin-4-yl)propyl)piperidin-1-yl)methanone (60 mg, 0.09 mmol) and pentafluorophenyl 4-chloro-3-(2,4-dioxotetrahydropyrimidin-1(2*H*)-yl)benzoate (41 mg, 0.095 mmol) were dissolved in DMSO (5 mL). DIEA (35 mg, 0.27 mmol) was added, and the reaction liquid was then stirred at room temperature for 2 h. Water (50 mL) was added to the reaction liquid, and the resulting mixture was stirred for 2 min and filtered. The solid was purified by column chromatography (10% MeOH/DCM) and then purified by p-TLC to give the target product 1-(5-(4-(3-(1-((1*R*,4*R*)-4-(4-(((*R*)-1-(3-amino-5-(trifluoromethyl)phenyl)ethyl)amino)-7-methoxy-2-methylquinazolin-6-yl)cyclohexane-1 -carbonyl)piperidin-4-yl)propyl)piperidine-1 -carbonyl)-2-chlorophenyl)dihydropyrimidine-2,4(1*H*,3*H*)-dione.

LC-MS: (ESI, m/z): [M+H]⁺= 945.2.

¹H NMR (400 MHz, DMSO-*d*₆) δ 10.50 (s, 1H), 8.20-8.04 (m, 2H), 7.63 (d, *J* = 8.2 Hz, 1H), 7.54 (d, *J* = 1.7 Hz, 1H), 7.38 (dd, *J=* 8.2, 1.8 Hz, 1H), 6.99 (s, 1H), 6.88 (s, 1H), 6.85 (s, 1H), 6.70 (s, 1H), 5.65 - 5.44 (m, 3H), 4.55-4.40 (m, 2H), 3.98-3.84 (m, 4H), 3.80-3.70 (m, 1H), 3.66-3.48 (m, 2H), 3.30-3.20 (m, 1H), 3.10-2.90 (m, 3H), 2.78-2.60 (m, 4H), 2.48-2.40 (m, 2H), 2.39-2.30 (m, 2H), 1.90-1.42 (m, 14H), 1.24-0.83 (m, 12H).

### Example 15: 1-(5-(4-(2-(1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)ethyl)piperidine-1-carbonyl)-2-chlorophenyl)dihydropyrimidine-2,4(1H,3H)-dione

### Step 1: preparation of 1,2-di(piperidin-4-yl)ethane

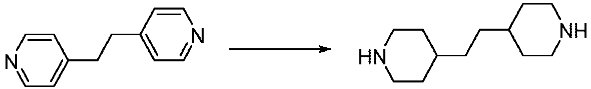

Pd/C (1.2 g, 11.32 mmol) was added to a solution of 1,2-di(pyridin-4-yl)ethane (4.0 g, 21.74 mmol) in IPA/H₂O (120 mL/140 mL). The resulting mixture was stirred in an autoclave charged with H₂ at 75 °C for 72 h, filtered, rinsed with dichloromethane (100 mL × 4), and concentrated under reduced pressure to give the target product 1,2-di(piperidin-4-yl)ethane.

LC-MS: (ESI, m/z): [M+H]⁺= 197.2.

### Step 2: preparation of benzyl 4-(2-(piperidin-4-yl)ethyl)piperidine-1-carboxylate

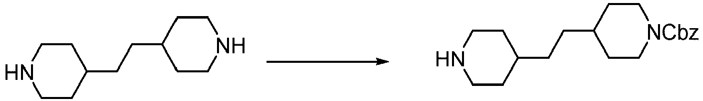

NaHCO₃ (1.0 g, 11.9 mmol) and CbzCl (0.872 g, 5.10 mmol) were added to a solution of 1,2-di(piperidin-4-yl)ethane (1.0 g, 5.10 mmol) in ACN/H₂O (60 mL/60 mL). The reaction liquid was stirred at room temperature overnight, and water (200 mL) was added. The resulting mixture was stirred for 10 min and extracted with ethyl acetate (100 mL × 3). The organic phase was washed with saturated brine (150 mL), dried over anhydrous sodium sulfate, filtered, and concentrated under reduced pressure. The crude product was purified by column chromatography (30% ACN/H₂O) to give the target product benzyl 4-(2-(piperidin-4-yl)ethyl)piperidine-1-carboxylate.

LC-MS: (ESI, m/z): [M+H]⁺= 331.4.

### Step 3: preparation of benzyl 4-(2-(1-((1R,4R)-4-(7-methoxy-2-methyl-4-(((R)-1-(3-nitro-5-(trifluoromethyl)phenyl)ethyl)amino)quinazolin-6-yl)cyclohexane-1-carbonyl)piperidin-4-yl)ethyl)piperidine-1-carboxylate

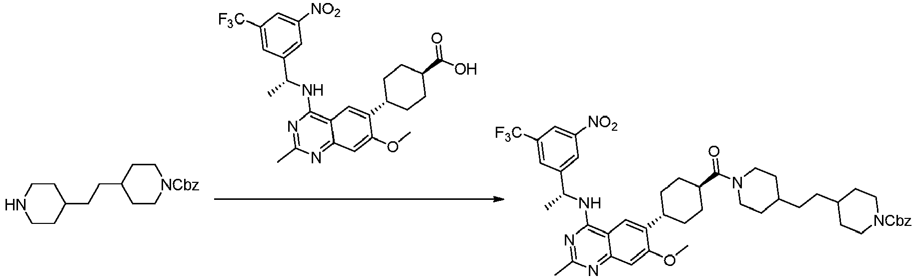

(1*R*,4*R*)-4-(7-Methoxy-2-methyl-4-(((R)-1-(3-nitro-5-(trifluoromethyl)phenyl)ethyl)amino)quinazolin-6-yl)cyclohexane-1-carboxylic acid (170 mg, 0.319 mmol), HATU (182.14 mg, 0.479 mmol), and DIEA(123.66 mg, 0.9586 mmol) were added to a solution of benzyl 4-(2-(piperidin-4-yl)ethyl)piperidine-1-carboxylate (160 mg, 0.477 mmol) in DMF (20 mL). The reaction liquid was stirred at room temperature overnight, and water (200 mL) was added. The resulting mixture was stirred for 10 min and extracted with ethyl acetate (100 mL × 3). The organic phase was washed with saturated brine (150 mL), dried over anhydrous sodium sulfate, filtered, and concentrated under reduced pressure. The crude product was purified by column chromatography (20% EA/PE) to give the target product benzyl 4-(2-(1-((1*R*,4*R*)-4-(7-methoxy-2-methyl-4-(((*R*)-1-(3-nitro-5-(trifluoromethyl)phenyl)ethyl)amino)quinazolin-6-yl)cyclohexane-1 -carbonyl)piperidin-4-yl)ethyl)piperidine-1 - carboxylate.

LC-MS: (ESI, m/z): [M+H]⁺= 845.7.

### Step 4: preparation of benzyl 4-(2-(1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)ethyl)piperidine-1-carboxylate

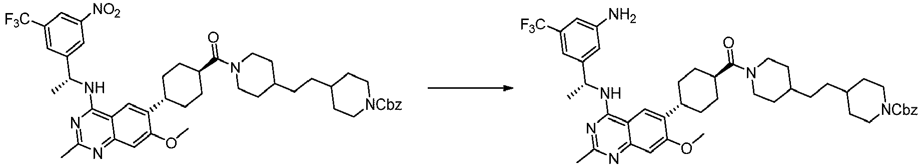

Benzyl 4-(2-(1-((1*R*,4*R*)-4-(7-methoxy-2-methyl-4-(((*R*)-1-(3-nitro-5-(trifluoromethyl)phenyl)ethyl)amino)quinazolin-6-yl)cyclohexane-1 -carbonyl)piperidin-4-yl)ethyl)piperidine-1 - carboxylate (200 mg, 0.236 mmol) was dissolved in ethanol (30 mL), and Raney-Ni (1 mL) and N₂H₄-H₂O (1 mL) were added. The resulting mixture was stirred at room temperature for 1 h, filtered, concentrated, and dried to give the target product benzyl 4-(2-(1-((1R,4R)-4-(4-(((R)-1-(3-amino-5-(trifluoromethyl)phenyl)ethyl)amino)-7-methoxy-2-methyl quinazolin-6-yl)cyclohexane-1-carbonyl)piperidin-4-yl)ethyl)piperidine-1 -carboxylate.

LC-MS: (ESI, m/z): [M+H]⁺= 815.4.

### Step 5: preparation of ((1R,4R)-4-(4-(((R)-1-(3-amino-5-(trifluoromethyl)phenyl)ethyl)amino)-7-methoxy-2-methylquinazolin-6-yl)cyclohexyl)(4-(2-(piperidin-4-yl)ethyl)piperidin-1-yl)methanone

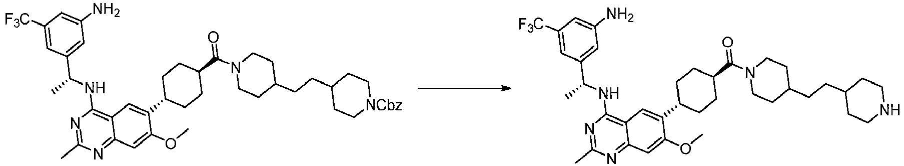

Benzyl 4-(2-(1-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperidin-4-yl)ethyl)piperidine-1-carboxylate (160 mg, 0.196 mmol) was dissolved in ethyl acetate (20 mL), and Pd(OH)₂ (32 mg, 20%) was added. The resulting mixture was stirred at 60 °C for 2 h, filtered, concentrated, and dried to give the target product ((1*R*,4*R*)-4-(4-(((*R*)-1-(3-amino-5-(trifluoromethyl)phenyl)ethyl)amino)-7-methoxy-2-methylquinazolin-6-yl)cyclohexyl)(4-(2-(piperidin-4-yl)ethyl)piperidin-1 -yl)methanone.

LC-MS: (ESI, m/z): [M+H]⁺= 681.2.

### Step 6: preparation of 1-(5-(4-(2-(1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)ethyl)piperidine-1-carbonyl)-2-chlorophenyl)dihydropyrimidine-2,4(1H,3H)-dione

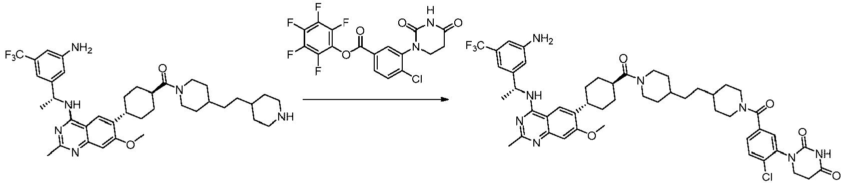

((1*R*,4R)-4-(4-(((R)-1-(3-Amino-5-(trifluoromethyl)phenyl)ethyl)amino)-7-methoxy-2-methylquinazolin-6-yl)cyclohexyl)(4-(2-(piperidin-4-yl)ethyl)piperidin-1-yl)methanone (120 mg, 0.176 mmol) and pentafluorophenyl 4-chloro-3-(2,4-dioxotetrahydropyrimidin-1(2H)-yl)benzoate (84.25 mg, 0.194 mmol) were dissolved in DMSO (5 mL), and DIEA (68.30 mg, 0.529 mmol) was added. The reaction liquid was stirred at room temperature for 2 h. Water (50 mL) was added to the reaction liquid, and the resulting mixture was stirred for 2 min and filtered The solid was purified by reversed-phase preparative chromatography to give the target product 1-(5-(4-(2-(1-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperidin-4-yl)ethyl)piperidine-1-carbonyl)-2-chlorophenyl)dihydropyrimidine-2,4(1*H*,3*H*)-dione. LC-MS: (ESI, m/z): [M+H]⁺= 931.3.

¹H NMR (400 MHz, DMSO-*d*₆) δ 10.50 (s, 1H), 8.20-8.03 (m, 2H), 7.63 (d, *J =* 8.2 Hz, 1H), 7.54 (d, *J* = 1.7 Hz, 1H), 7.41 - 7.34 (m, 1H), 6.99 (s, 1H), 6.88 (s, 1H), 6.85 (s, 1H), 6.70 (s, 1H), 5.65 - 5.49 (m, 3H), 4.54-4.34 (m, 2H), 3.98-3.85 (m, 4H), 3.80-3.70 (m, 1H), 3.67-3.53 (m, 2H), 3.10-2.86 (m, 3H), 2.80-2.60 (m, 4H), 2.36 (s, 3H), 1.90 - 1.36 (m, 17H), 1.35-1.18 (m, 5H), 1.16-0.86 (m, 4H).

### Example 16: 1-(2-chloro-5-(9-((4-((1R,4R)-4-(4-(((R)-1-(2-fluoro-3-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carbonyl)phenyl)dihydropyrimidine-2,4(1H,3H)-dione

### Step 1: preparation of 1-(2-fluoro-3-(trifluoromethyl)phenyl)ethan-1-one

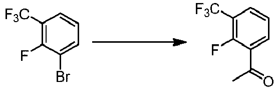

A reaction liquid of 1-bromo-2-fluoro-3-(trifluoromethyl)benzene (4.0 g, 16.4 mmol), Pd(PPh₃)₂Cl₂ (1.1 g, 1.6 mmol), TEA (4.9 g, 48.9 mmol), and tributyl(1-ethoxyvinyl)stannane (8.8 g, 24.4 mmol) in dioxane (80.0 mL) was stirred at 80 °C for reaction for 16.0 h. Ice water (200 mL) was added to quench the reaction, and the resulting mixture was extracted with EA (80 mL × 3). The organic phases were combined, washed with brine, dried over anhydrous sodium sulfate, filtered, and concentrated. The residue was stirred in 6 N HCl (20 mL), and the filtrate was concentrated under reduced pressure. The crude product was purified by column chromatography (EA/PE, 0-40%) to give the target product 1-(2-fluoro-3-(trifluoromethyl)phenyl)ethan-1-one.

### Step 2: preparation of (S,E)-N-(1-(2-fluoro-3-(trifluoromethyl)phenyl)ethylidene)-2-methylpropane-2-sulfinamide

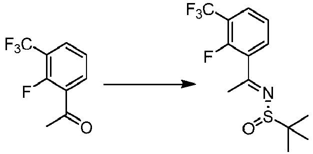

A reaction mixture of 1-(2-fluoro-3-(trifluoromethyl)phenyl)ethan-1-one (2.0 g, 9.7 mmol), Ti(OEt)₄ (5.5 g, 24.2 mmol), and (*S*)-propane-2-sulfinamide (1.7 g, 14.5 mmol) in a THF (40.0 mL) solution was stirred at 60 °C for reaction for 2 h. Water (100 mL) was added to quench the reaction, and the solution was extracted with EA (80 mL × 3). The organic phase was collected, dried over anhydrous sodium sulfate, filtered, and concentrated under reduced pressure. The concentrate was purified by column chromatography to give the target product (*S*,*E*)-*N*-(1-(2-fluoro-3-(trifluoromethyl)phenyl)ethylidene)-2-methylpropane-2-sulfinamide.

LC-MS: (ESI, m/z): [M+H]⁺= 310.2.

### Step 3: preparation of (S)-N-((R)-1-(2-fluoro-3-(trifluoromethyl)phenyl)ethyl)-2-methylpropane-2-sulfinamide

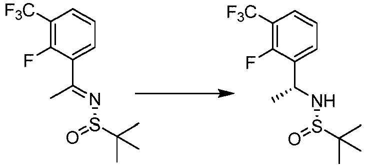

L-Selectride (1.0 mol/L/THF, 7.1 mL) was added to a solution of (*S*,*E*)-*N*-(1-(2-fluoro-3-(trifluoromethyl)phenyl)ethylidene)-2-methylpropane-2-sulfinamide (2.0 g, 6.4 mmol) in THF (20.0 mL) at -78 °C, and the reaction mixture was stirred at -78 °C for reaction for 50 min. Ice water (100 mL) was added to quench the reaction, and the solution was extracted with EA (80 mL × 3). The organic phase was collected, dried over anhydrous sodium sulfate, filtered, and concentrated under reduced pressure. The concentrate was purified by column chromatography (EA/PE, 0-60%) to give the target product (*S*)-*N*-((*R*)-1-(2-fluoro-3-(trifluoromethyl)phenyl)ethyl)-2-methylpropane-2-sulfinamide.

LC-MS: (ESI, m/z): [M+H]⁺= 312.2.

¹H NMR (400 MHz, CDCl₃) δ 7.65-7.59 (m, 1H), 7.57-7.51 (m, 1H), 7.27-7.22 (m, 1H), 4.91 - 4.81 (m, 1H), 3.55 (d, *J* = 5.2 Hz, 1H), 1.56 (d, *J* = 6.7 Hz, 3H), 1.23 (s, 9H).

### Step 4: preparation of (R)-1-(2-fluoro-3-(trifluoromethyl)phenyl)ethan-1-amine hydrochloride

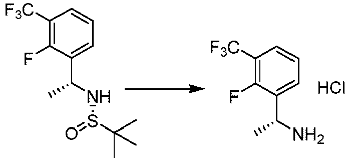

A reaction mixture of (*S*)-*N*-((*R*)-1-(2-fluoro-3-(trifluoromethyl)phenyl)ethyl)-2-methylpropane-2-sulfinamide (2.0 g, 3.2 mmol) in dioxane hydrochloride (4 N, 20.0 mL) was stirred at 25 °C for reaction for 1.0 h. The reaction liquid was concentrated under reduced pressure. The concentrate was washed with PE (20 mL × 3) to give the target product (*R*)-1-(2-fluoro-3-(trifluoromethyl)phenyl)ethan-1-amine hydrochloride.

LC-MS: (ESI, m/z): [M+H]⁺= 208.1.

### Step 5: preparation of methyl (1R,4R)-4-(4-(((R)-1-(2-fluoro-3-(trifluoromethyl)phenyl)ethyl)amino)-7-methoxy-2-methylquinazolin-6-yl)cyclohexane-1 -carboxylate

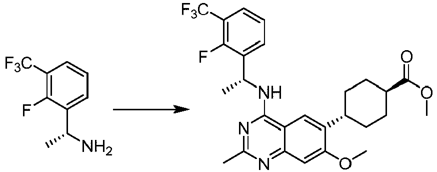

A reaction mixture of (*R*)-1-(2-fluoro-3-(trifluoromethyl)phenyl)ethan-1-amine (0.7 g, 3.3 mmol), DIEA (928.8 mg, 7.2 mmol), and methyl (1*R*,4*R*)-4-(4-chloro-7-methoxy-2-methylquinazolin-6-yl)cyclohexyl-1-carboxylate (835 mg, 2.4 mmol) in dioxane (10.0 mL) was stirred at 110 °C for reaction for 20.0 h. Water (50 mL) was added to quench the reaction, and the resulting mixture was extracted with EA (30.0 mL × 2). The organic phases were combined, washed with brine, dried over anhydrous sodium sulfate, and concentrated under reduced pressure. The concentrate was purified by preparative HPLC (acetonitrile/0.05% aqueous HCOOH solution, 5% to 95%) to give the target product methyl (1*R*,4*R*)-4-(4-(((*R*)-1-(2-fluoro-3-(trifluoromethyl)phenyl)ethyl)amino)-7-methoxy-2-methylquinazolin-6-yl)cyclohexane-1-carboxylate.

LC-MS: (ESI, m/z): [M+H]⁺= 520.3.

### Step 6: preparation of (1R,4R)-4-(4-(((R)-1-(2-fluoro-3-(trifluoromethyl)phenyl)ethyl)amino)-7-methoxy-2-methylquinazolin-6-yl)cyclohexane-1 -carboxylic acid

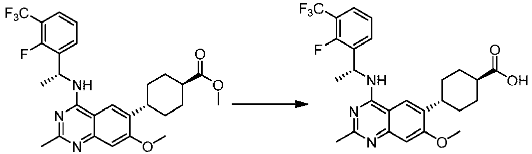

A reaction mixture of methyl (1*R*,4*R*)-4-(4-(((*R*)-1-(2-fluoro-3-(trifluoromethyl)phenyl)ethyl)amino)-7-methoxy-2-methylquinazolin-6-yl)cyclohexane-1-carboxylate (100 mg, 0.2 mmol) and LiOH (48 mg, 2.0 mmol) in THF/H₂O (2.0 mL/1.0 mL) was stirred at 50 °C for reaction for 12.0 h. The reaction liquid was adjusted to pH 4-5 with 1 N HCl and concentrated under reduced pressure to give the target product (1*R*,4*R*)-4-(4-(((*R*)-1-(2-fluoro-3-(trifluoromethyl)phenyl)ethyl)amino)-7-methoxy-2-methylquinazolin-6-yl)cyclohexane-1-carboxylic acid.

LC-MS: (ESI, m/z): [M+H]⁺= 506.4.

### Step 7: preparation of tert-butyl 4-((1R,4R)-4-(4-(((R)-1-(2-fluoro-3-(trifluoromethyl)phenyl)ethyl)amino)-7-methoxy-2-methylquinazolin-6-yl)cyclohexane-1-carbonyl)piperazine-1-carboxylate

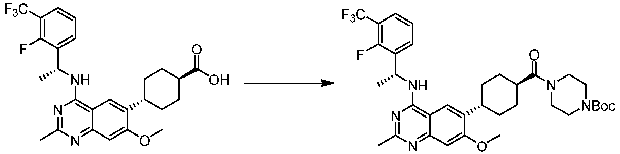

HATU (124 mg, 0.24 mmol) was added to a solution of (1*R*,4*R*)-4-(4-(((*R*)-1-(2-fluoro-3-(trifluoromethyl)phenyl)ethyl)amino)-7-methoxy-2-methylquinazolin-6-yl)cyclohexane-1 -carboxylic acid (110 mg, 0.2 mmol), tert-butyl piperazine-1-carboxylate (45 mg, 0.22 mmol), and DIEA (85 mg, 0.6 mmol) in DMF (2 mL), and the reaction mixture was stirred at room temperature for reaction for 2 h. Ice water (20 mL) was added to quench the reaction, and the resulting mixture was filtered. The precipitate was collected and then dried under vacuum to give the target product *tert*-butyl 4-((1*R*,4*R*)-4-(4-(((*R*)-1-(2-fluoro-3-(trifluoromethyl)phenyl)ethyl)amino)-7-methoxy-2-methylquinazolin-6-yl)cyclohexane-1-carbonyl)piperazine-1-carboxylate.

LC-MS: (ESI, m/z): [M+H]⁺= 674.5.

### Step 8: preparation of ((1R,4R)-4-(4-(((R)-1-(2-fluoro-3-(trifluoromethyl)phenyl)ethyl)amino)-7-methoxy-2-methylquinazolin-6-yl)cyclohexyl)(piperazin-1-yl)methanone

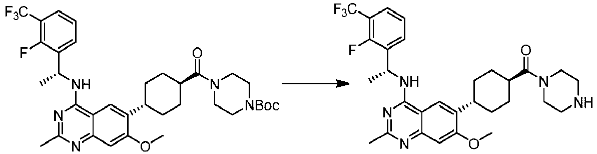

A reaction mixture of *tert*-butyl 4-((1*R*,4*R*)-4-(4-(((*R*)-1-(2-fluoro-3-(trifluoromethyl)phenyl)ethyl)amino)-7-methoxy-2-methylquinazolin-6-yl)cyclohexane-1-carbonyl)piperazine-1-carboxylate (110 mg, 0.1 mmol) in TFA/DCM (1 mL/3 mL) was stirred at room temperature for reaction for 1.0 h. The reaction liquid was concentrated under reduced pressure to give the target product ((1*R*,4*R*)-4-(4-(((*R*)-1-(2-fluoro-3-(trifluoromethyl)phenyl)ethyl)amino)-7-methoxy-2-methylquinazolin-6-yl)cyclohexyl)(piperazin-1-yl)methanone. LC-MS: (ESI, m/z): [M+H]⁺= 574.2.

### Step 9: preparation of tert-butyl 9-((4-((1R,4R)-4-(4-(((R)-1-(2-fluoro-3-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carboxylate

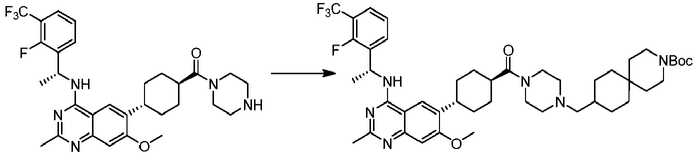

STAB (64 mg, 0.3 mmol) was added to a solution of ((1*R*,4*R*)-4-(4-(((R)-1-(2-fluoro-3-(trifluoromethyl)phenyl)ethyl)amino)-7-methoxy-2-methylquinazolin-6-yl)cyclohexyl)(piperazin-1-yl)methanone (60 mg, 0.1 mmol) and *tert*-butyl 9-formyl-3-azaspiro[5.5]undecane-3-carboxylate (35 mg, 0.12 mmol) in THF (5.0 mL), and then the reaction mixture was stirred at room temperature for reaction for 2.0 h. Ice water (10 mL) was added to quench the reaction, and the resulting mixture was extracted with EA (30.0 mL × 2). The organic phases were combined, washed with brine, dried over anhydrous sodium sulfate, and concentrated under reduced pressure. The concentrate was purified by column chromatography (MeOH/DCM, 0-10%) to give the target product *tert-*butyl 9-((4-((1*R*,4*R*)-4-(4-(((*R*)-1-(2-fluoro-3-(trifluoromethyl)phenyl)ethyl)amino)-7-methoxy-2-methylquinazolin-6-yl)cyclohexyl-1-carbonyl)piperazin-1-yl)methyl)-3-azaspiro[5.5]undecane-3-carboxylate.

LC-MS: (ESI, m/z): [M+H]⁺= 839.4.

### Step 10: preparation of (4-((3-azaspiro[5.5]undecane-9-yl)methyl)piperazin-1-yl)((1R,4R)-4-(4-(((R)-1-(2-fluoro-3-(trifluoromethyl)phenyl)ethyl)amino)-7-methoxy-2-methylquinazolin-6-yl)cyclohexyl)methanone

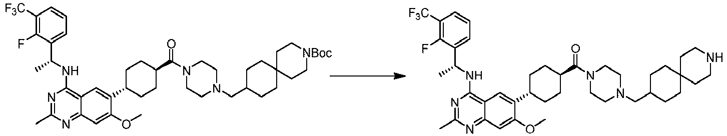

A reaction mixture of *tert*-butyl 9-((4-((1*R*,4*R*)-4-(4-(((*R*)-1-(2-fluoro-3-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carboxylate (70 mg, 0.08 mmol) in TFA/DCM (1 mL/3 mL) was stirred at room temperature for reaction for 1.0 h. The reaction liquid was directly concentrated to give the target product (4-((3-azaspiro[5.5]undecane-9-yl)methyl)piperazin-1-yl)((1*R*,4*R*)-4-(4-(((*R*)-1-(2-fluoro-3-(trifluoromethyl)phenyl)ethyl)amino)-7-methoxy-2-methylquinazolin-6-yl)cyclohexyl)methanone.

LC-MS: (ESI, m/z): [M+H]⁺= 739.5.

### Step 11: 1-(2-chloro-5-(9-((4-((1R,4R)-4-(4-(((R)-1-(2-fluoro-3-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carbonyl)phenyl)dihydropyrimidine-2,4(1H,3H)-dione

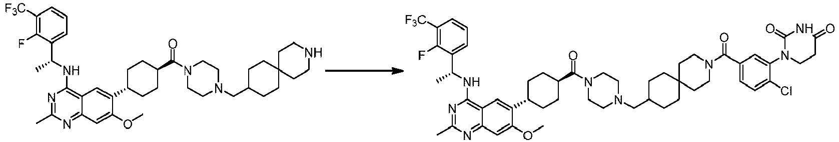

DIEA (31 mg, 0.24 mmol) and pentafluorophenyl 4-chloro-3-(2,4-dioxotetrahydropyrimidin-1(2*H*)-yl)benzoate (43 mg, 0.1 mmol) were added to a solution of (4-((3-azaspiro[5.5]undecane-9-yl)methyl)piperazin-1-yl)((1*R*,4*R*)-4-(4-(((*R*)-1-(2-fluoro-3-(trifluoromethyl)phenyl)ethyl)amino)-7-methoxy-2-methylquinazolin-6-yl)cyclohexyl)methanone (60 mg, 0.08 mmol) in DMSO (1.0 mL), and then the reaction mixture was stirred at room temperature for reaction for 2.0 h. Water (15 mL) was added to quench the reaction, and the resulting mixture was extracted with EA (30 mL × 2). The organic phases were combined, washed with brine, dried, and concentrated under reduced pressure. The concentrate was purified by preparative HPLC (acetonitrile/0.05% aqueous NH₄HCO₃ solution, 5% to 95%) to give the target product 1-(2-chloro-5-(9-((4-((1*R*,4*R*)-4-(4-(((*R*)-1-(2-fluoro-3-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M+H]⁺= 989.4.

¹H NMR (400 MHz, DMSO-*d*₆) δ 10.51 (s, 1H), 8.26 (s, 1H), 8.10 (s, 1H), 7.80 (s, 1H), 7.63 (d, *J* = 7.5 Hz, 2H), 7.55 (s, 1H), 7.42 - 7.34 (m, 2H), 6.99 (s, 1H), 5.77 (s, 1H), 3.88 (s, 3H), 3.82-3.72 (m, 1H), 3.68-3.42 (m, 7H), 3.02-2.92 (m, 1H), 2.78-2.70 (m, 2H), 2.70-2.63 (m, 1H), 2.38-2.25 (m, 7H), 2.18-2.10 (m, 2H), 1.90-1.78 (m, 4H), 1.71-1.22 (m, 18H), 1.98-1.00 (m, 4H).

### Example 17: N-(3-((R)-1-((6-((1R,4R)-4-(4-((3-(4-chloro-3-(2,4-dioxotetrahydropyrimidin-1(2H)-yl)benzoyl)-3-azaspiro[5.5]undecane-9-yl)methyl)piperazine-1-carbonyl)cyclohexyl)-7-methoxy-2-methylquinazolin-4-yl)amino)ethyl)-5-(trifluoromethyl)phenyl)acetamide

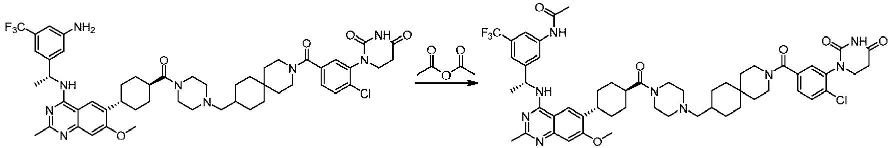

A reaction mixture of 1-(5-(9-((4-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carbonyl)-2-chlorophenyl)dihydropyrimidine-2,4(1*H*,3*H*)-dione (70 mg, 0.07 mmol) in acetic anhydride (3.0 mL) was stirred at room temperature for reaction for 12.0 h. The reaction liquid was directly concentrated under reduced pressure. The concentrate was purified by preparative HPLC (acetonitrile/0.05% aqueous NH₄HCO₃ solution, 5% to 95%) to give the target product *N*-(3-((*R*)-1-((6-((1*R*,4*R*)-4-(4-((3-(4-chloro-3-(2,4-dioxotetrahydropyrimidin-1(2*H*)-yl)benzoyl)-3-azaspiro[5.5]undecane-9-yl)methyl)piperazine-1-carbonyl)cyclohexyl)-7-methoxy-2-methylquinazolin-4-yl)amino)ethyl)-5-(trifluoromethyl)phenyl)acetamide.

LC-MS: (ESI, m/z): [M+H]⁺= 1028.3.

¹H NMR (400 MHz, DMSO-*d*₆) δ 10.50 (s, 1H), 10.22 (s, 1H), 8.19 (d, *J =* 7.3 Hz, 1H), 8.06 (s, 1H), 7.96 (s, 1H), 7.78 (s, 1H), 7.63 (d, *J=* 8.1 Hz, 1H), 7.54 (s, 1H), 7.47 (s, 1H), 7.38 (d, *J=* 7.9 Hz, 1H), 7.00 (s, 1H), 5.68 - 5.54 (m, 1H), 3.88 (s, 3H), 3.79-3.72 (m, 1H), 3.62-3.41 (m, 7H), 2.98-2.91 (m, 1H), 2.78-2.70 (m, 2H), 2.70-2.63 (m, 1H), 2.38-2.22 (m, 7H), 2.18-2.08 (m, 2H), 2.03 (s, 3H), 1.89-1.78 (m, 4H), 1.74 - 1.22 (m, 18H), 1.18-0.98 (m, 4H).

### Example 18: 1-(2-chloro-5-(9-((4-((1R,4R)-4-(4-(((R)-1-(3-(isopropylamino)-5-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carbonyl)phenyl)dihydropyrimidine-2,4(1H,3H)-dione

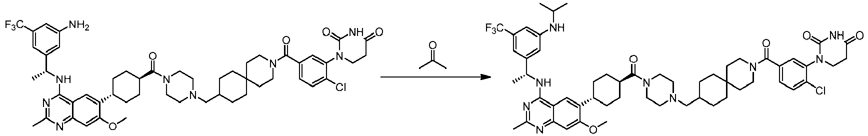

STAB (89 mg, 0.42 mmol) was added to a solution of 1-(5-(9-((4-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carbonyl)-2-chlorophenyl)dihydropyrimidine-2,4(1*H*,3*H*)-dione (70 mg, 0.07 mmol), acetone (40 mg, 0.7 mmol), and AcOH (0.2 mL) in MeOH/THF (1.0 mL/1.0 mL) at 0 °C. The reaction mixture was then stirred at room temperature for reaction for 30 h. Water was added to quench the reaction, and the resulting mixture was extracted with EA. The organic phases were combined, washed with brine, dried over anhydrous sodium sulfate, and concentrated under reduced pressure. The concentrate was purified by preparative HPLC (acetonitrile/0.05% aqueous NH₄HCO₃ solution, 5% to 95%) to give the target product 1-(2-chloro-5-(9-((4-((1*R*,4*R*)-4-(4-(((*R*)-1-(3-(isopropylamino)-5-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M+H]⁺= 1028.3.

¹H NMR (400 MHz, DMSO-*d*₆) δ 10.50 (s, 1H), 10.22 (s, 1H), 8.19 (d, *J =* 7.3 Hz, 1H), 8.06 (s, 1H), 7.96 (s, 1H), 7.78 (s, 1H), 7.63 (d, *J=* 8.1 Hz, 1H), 7.54 (s, 1H), 7.47 (s, 1H), 7.38 (d, *J=* 7.9 Hz, 1H), 7.00 (s, 1H), 5.68 - 5.54 (m, 1H), 3.88 (s, 3H), 3.82-3.56 (m, 8H), 2.98-2.91 (m, 1H), 2.78-2.70 (m, 2H), 2.70-2.63 (m, 2H), 2.38-2.22 (m, 7H), 2.18-2.08 (m, 2H), 1.95-1.75 (m, 4H), 1.89-1.48 (m, 15H), 1.42 - 1.25 (m, 6H), 1.18-0.98 (m, 7H).

### Example 19: 1-(5-(4-((2-(1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)ethoxy)methyl)piperidine-1-carbonyl)-2-chlorophenyl)dihydropyrimidine-2,4(1H,3H)-dione

### Step 1: preparation of benzyl 4-((2-(1-(tert-butoxycarbonyl)piperidin-4-yl)ethoxy)methyl)piperidine-1-carboxylate

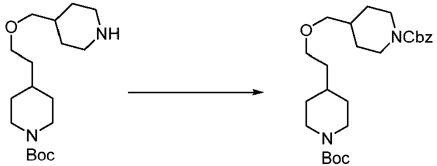

A reaction liquid of *tert*-butyl 4-(2-(piperidin-4-ylmethoxy)ethyl)piperidine-1-carboxylate (1.9 g, 5.81 mmol) and benzyl chloroformate (1.19 mg, 6.97 mmol) in ACN/sat. NaHCO₃ (30 mL/30 mL) was stirred at room temperature overnight. Water (50 mL) was added to the reaction liquid to quench the reaction, and then the resulting mixture was extracted with EA (50 mL × 3). The organic phase was washed with saturated brine, dried over Na₂SO₄, and concentrated under reduced pressure. The crude product was purified by flash column chromatography (EA:PE = 0-7:3) to give the target product benzyl 4-((2-(1-(*tert*-butoxycarbonyl)piperidin-4-yl)ethoxy)methyl)piperidine-1-carboxylate.

LC-MS: (ESI, *m*/*z):* [M+H]⁺= 461.3.

### Step 2: preparation of benzyl 4-((2-(piperidin-4-yl)ethoxy)methyl)piperidine-1-carboxylate

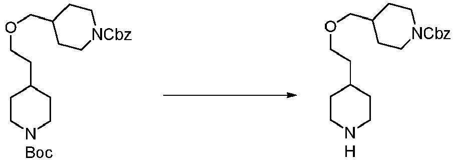

TFA (2 mL) was added to a solution of benzyl 4-((2-(1-(*tert*-butoxycarbonyl)piperidin-4-yl)ethoxy)methyl)piperidine-1-carboxylate (600 mg, 1.3 mmol) in DCM (5 mL). The reaction liquid was stirred at room temperature for 2 h and then directly concentrated under reduced pressure to give the target product benzyl 4-((2-(piperidin-4-yl)ethoxy)methyl)piperidine-1-carboxylate. The resulting crude product was directly used in the next step without purification.

LC-MS: (ESI, m/z): [M+H]⁺= 361.1.

### Step 3: preparation of (1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carboxylic acid

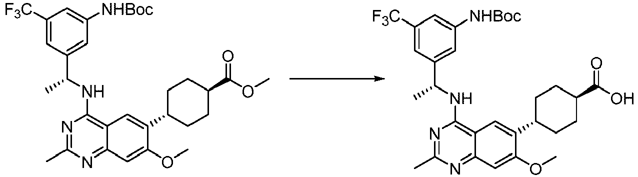

A reaction liquid of methyl (1*R*,4*R*)-4-(4-(((*R*)-1-(3-((*tert*-butoxycarbonyl)amino)-5-(trifluoromethyl)phenyl)ethyl)amino)-7-methoxy-2-methylquinazolin-6-yl)cyclohexane-1-carboxylate (21 mg, 0.34 mmol) and LiOH (71 mg, 1.70 mmol) in THF/H₂O (8 mL/4 mL) was stirred at 50 °C overnight. A 10% aqueous citric acid solution (5 mL) was added to the reaction liquid, and the resulting mixture was extracted with EA (20 mL ×3). The organic phase was washed with saturated brine (100 mL), dried over Na₂SO₄, filtered, and concentrated under reduced pressure to give the target product (1*R*,4*R*)-4-(4-(((*R*)-1-(3-((tert-butoxycarbonyl)amino)-5-(trifluoromethyl)phenyl)ethyl)amino)-7-methoxy-2-methylquinazolin-6-yl)cyclohexane-1-carboxylic acid.

LC-MS: (ESI, m/z): [M+H]⁺= 603.1.

### Step 4: preparation of benzyl 4-((2-(1-((1R,4R)-4-(4-(((R)-1-(3-(tert-butoxycarbonyl)amino)-5-(trifluoromethyl)phenyl)ethyl)amino)-7-methoxy-2-methylquinazolin-6-yl)cyclohexane-1-carbonyl)piperidin-4-yl)ethoxy)methyl)piperidine-1-carboxylate

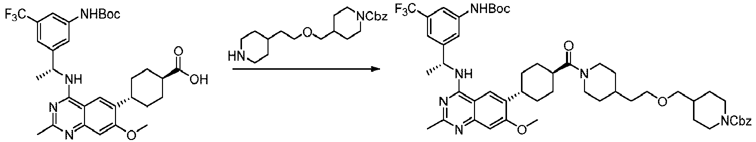

Benzyl 4-(((2-piperidin-4-yl)ethoxy)methyl)piperidine-1-carboxylate (155 mg, 0.43 mmol), HATU (190 mg, 0.50 mmol), and DIEA (128 mg, 0.99 mmol) were added to a solution of (1*R*,4*R*)-4-(4-(((*R*)-1-(3-((*tert-*butoxycarbonyl)amino)-5-(trifluoromethyl)phenyl)ethyl)amino)-7-methoxy-2-methylquinazolin-6-yl)cyclohexane-1-carboxylic acid (200 mg, 0.33 mmol) in DMF (10 mL). The reaction mixture was stirred at room temperature for 2 h. Water (100 mL) was added to the reaction liquid, and the resulting mixture was extracted with EA (30 mL × 3). The organic phase was washed with saturated brine (100 mL), dried over Na₂SO₄, filtered, and concentrated under reduced pressure. The resulting crude product was purified by flash column chromatography (EA:PE = 0-4:1) to give the target product benzyl 4-((2-(1*R*,4*R*)-4-(4-(((*R*)-1-(3-(tert-butoxycarbonyl)amino)-5-(trifluoromethyl)phenyl)ethyl)amino)-7-methoxy-2-methylquinazolin-6-yl)cyclohexane-1-carbonyl)piperidin-4-yl)ethoxy)methyl)piperidine-1-carboxylate.

LC-MS: (ESI, *m*/*z*): [M+H]⁺= 945.3.

### Step 5: preparation of tert-butyl (3-((R)-1-((7-methoxy-2-methyl-6-((1R,4R)-4-(4-(2-(piperidin-4-ylmethoxy)ethyl)piperidine-1-carbonyl)cyclohexyl)quinazolin-4-yl)amino)ethyl)-5-(trifluoromethyl)phenyl)carbamate

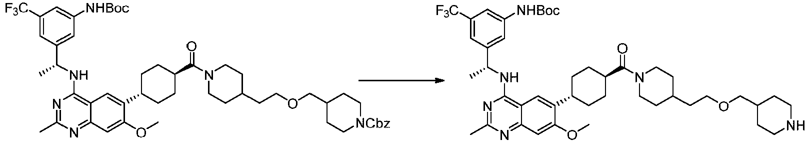

Pd(OH)₂/C (100 mg) was added to a solution of benzyl 4-((2-(1*R*,4*R*)-4-(4-(((*R*)-1-(3-(tert-butoxycarbonyl)amino)-5-(trifluoromethyl)phenyl)ethyl)amino)-7-methoxy-2-methylquinazolin-6-yl)cyclohexane-1-carbonyl)piperidin-4-yl)ethoxy)methyl)piperidine-1-carboxylate (230 mg, 0.24 mmol) in EA (20 mL). The reaction mixture was stirred at 70 °C overnight. The reaction liquid was filtered through celite, and the filtrate was concentrated under reduced pressure to give the target product *tert*-butyl (3-((R)-1-((7-methoxy-2-methyl-6-((1*R*,4*R*)-4-(4-(2-(piperidin-4-ylmethoxy)ethyl)piperidine-1-carbonyl)cyclohexyl)quinazolin-4-yl)amino)ethyl)-5-(trifluoromethyl)phenyl)carbamate. The resulting product was directly used in the next step without purification. LC-MS: (ESI, *m*/*z):* [M+H]⁺= 811.3.

### Step 6: preparation of tert-butyl (3-((R)-1-((6-((1R,4R)-4-(4-(2-((1-(4-chloro-3-(2,4-dioxotetrahydropyrimidin-1(2H)-yl)benzoyl)piperidin-4-yl)methoxy)ethyl)piperidine-1-carbonyl)cyclohexyl)-7-methoxy-2-methylquinazolin-4-yl)amino)ethyl)-5-(trifluoromethyl)phenyl)carbamate

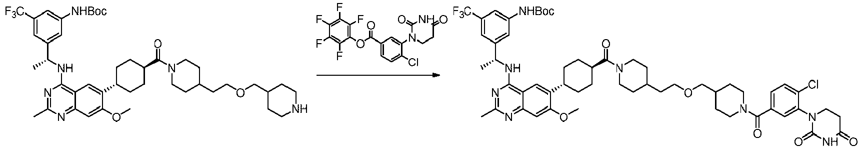

A solution of *tert*-butyl (3-((*R*)-1-((7-methoxy-2-methyl-6-((1*R*,4*R*)-4-(4-(2-(piperidin-4-ylmethoxy)ethyl)piperidine-1-carbonyl)cyclohexyl)quinazolin-4-yl)amino)ethyl)-5 - (trifluoromethyl)phenyl)carbamate (200 mg, 0.24 mmol), pentafluorophenyl 4-chloro-3-(2,4-dioxotetrahydropyrimidin-1(2*H*)-yl)benzoate (126 mg, 0.29 mmol), and DIEA (93 mg, 0.72 mmol) in DMSO (5 mL) was stirred at room temperature overnight. The reaction liquid was diluted with water (50 mL), and then the resulting mixture was extracted with DCM (20 mL × 2). The organic phases were combined, washed with saturated brine, dried, filtered, and concentrated under reduced pressure. The resulting crude product was purified by flash column chromatography (MeOH:DCM = 0-1:9) to give the target product *tert*-butyl (3-((*R*)-1-((6-((1*R*,4*R*)-4-(4-(2-((1-(4-chloro-3-(2,4-dioxotetrahydropyrimidin-1(2*H*)-yl)benzoyl)piperidin-4-yl)methoxy)ethyl)piperidine-1-carbonyl)cyclohexyl)-7-methoxy-2-methylquinazolin-4-yl)amino)ethyl)-5-(trifluoromethyl)phenyl)carbamate. LC-MS: (ESI, *m*/*z*): [M+H]⁺= 1061.3.

### Step 7: preparation of 1-(5-(4-((2-(1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)ethoxy)methyl)piperidine-1-carbonyl)-2-chlorophenyl)dihydropyrimidine-2,4(1H,3H)-dione

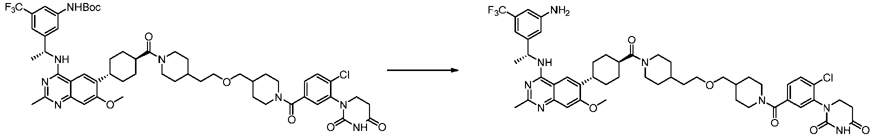

TFA (1.0 mL) was added dropwise to a solution of *tert*-butyl (3-((*R*)-1-((6-((1*R*,4*R*)-4-(4-(2-((1-(4-chloro-3-(2,4-dioxotetrahydropyrimidin-1(2*H*)-yl)benzoyl)piperidin-4-yl)methoxy)ethyl)piperidine-1-carbonyl)cyclohexyl)-7-methoxy-2-methylquinazolin-4-yl)amino)ethyl)-5-(trifluoromethyl)phenyl)carbamate in DCM (3 mL). The reaction liquid was stirred at room temperature for 2 h. DIEA (5 mL) was added to the reaction liquid, and then the resulting mixture was diluted with water (50 mL) and extracted with DCM (30 mL × 3). The organic phase was washed with saturated brine (100 mL), dried over Na₂SO₄, filtered, and concentrated under reduced pressure. The crude product was purified by Pre-HPLC (CH₃CN/0.08% aqueous NH₄HCO₃ solution, 5% to 95%) to give the target product 1-(5-(4-((2-(1-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperidin-4-yl)ethoxy)methyl)piperidine-1-carbonyl)-2-chlorophenyl)dihydropyrimidine-2,4(1*H*,3*H*)-dione.

LC-MS: (ESI, *m*/*z*): [M+H]⁺= 961.1.

¹H NMR (400 MHz, DMSO-*d*₆) δ 10.50 (s, 1H), 9.32 (s, 1H), 8.25 (s, 1H), 7.63 (d, *J* = 8.2 Hz, 1H), 7.54 (d, *J* = 1.8 Hz, 1H), 7.37 (dd, *J* = 8.2, 1.8 Hz, 1H), 7.04 (s, 1H), 6.86 (d, *J* = 7.3 Hz, 2H), 6.74 (s, 1H), 5.75-5.55 (m, 3H), 4.50-4.30 (m, 2H), 3.98-3.87 (m, 4H), 3.80-3.70 (m, 1H), 3.68-3.54 (m, 2H), 3.41 (t, *J* = 6.2 Hz, 2H), 3.23 (d, J = 6.0 Hz, 2H), 3.12-2.90 (m, 3H), 2.82-2.60 (m, 4H), 1.92-1.40 (m, 22H), 1.12-0.92 (m, 5H).

### Example 20: 1-(5-(9-((4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carbonyl)-2-methoxyphenyl)dihydropyrimidine-2,4(1H,3H)-dione

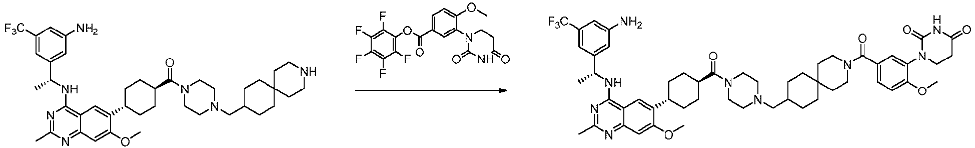

A reaction liquid of (4-((3-azaspiro[5.5]undecane-9-yl)methyl)piperazin-1-yl)((1*R*,4*R*)-4-(4-(((*R*)-1-(3-amino-5-(trifluoromethyl)phenyl)ethyl)amino)-7-methoxy-2-methylquinazolin-6-yl)cyclohexyl)methanone (200 mg, 0.12 mmol), pentafluorophenyl 3-(2,4-dioxotetrahydropyrimidin-1(2*H*)-yl)-4-methoxybenzoate (62 mg, 0.15 mmol), and DIEA (77 mg, 0.60 mmol) in DMSO (5 mL) was stirred at room temperature overnight and then diluted with water (50 mL). The resulting mixture was then extracted with DCM (20 mL × 3). The organic phase was washed with saturated brine (60 mL), dried over Na₂SO₄, filtered, and concentrated under reduced pressure. The resulting crude product was purified by thin layer preparative plate (DCM:MeOH = 10:1) to give the target product 1-(5-(9-((4-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carbonyl)-2-methoxyphenyl)dihydropyrimidine-2,4(1H,3H)-dione.

LC-MS: (ESI, *m*/*z*): [M+H]⁺= 982.2.

¹H NMR (400 MHz, DMSO-*d*₆) δ 10.33 (s, 1H), 8.14 - 8.02 (m, 2H), 7.36 (dd, *J =* 8.4, 2.1 Hz, 1H), 7.32 (d, *J* = 2.1 Hz, 1H), 7.15 (d, *J* = 8.7 Hz, 1H), 6.99 (s, 1H), 6.87 (s, 1H), 6.85 (s, 1H), 6.70 (s, 1H), 5.62 - 5.49 (m, 3H), 3.88 (s, 3H), 3.84 (s, 3H), 3.59 (t, *J* = 6.7 Hz, 2H), 3.52-3.42 (m, 6H), 3.02-2.92 (m, 1H), 2.70-2.68 (m, 3H), 2.40-2.24 (m, 7H), 2.16-2.06 (m, 2H), 1.90-1.75 (m, 4H), 1.78-1.40 (m, 14H), 1.32-1.22 (m, 4H), 1.12-0.92 (m, 4H).

### Example 21: 1-(3-(9-((4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)methyl)-3 -azaspiro [5.5]undecane-3-carbonyl)phenyl)dihydropyrimidine-2,4(1H,3H)-dione

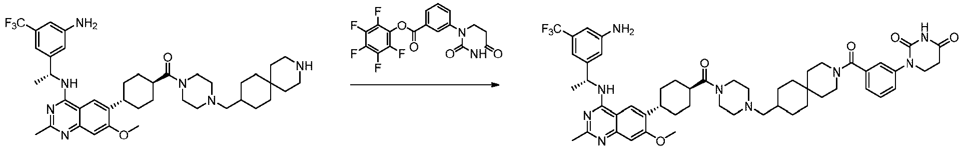

A reaction liquid of (4-((3-azaspiro[5.5]undecane-9-yl)methyl)piperazin-1-yl)((1*R*,4*R*)-4-(4-(((*R*)-1-(3-amino-5-(trifluoromethyl)phenyl)ethyl)amino)-7-methoxy-2-methylquinazolin-6-yl)cyclohexyl)methanone (200 mg, 0.12 mmol), pentafluorophenyl 3-(2,4-dioxotetrahydropyrimidin-1(2*H*)-yl)benzoate (60 mg, 0.15 mmol), and DIEA (77 mg, 0.60 mmol) in DMSO (5 mL) was stirred at room temperature overnight and then diluted with water (50 mL). The resulting mixture was then extracted with DCM (20 mL × 3). The organic phase was washed with saturated brine (60 mL), dried over Na₂SO₄, filtered, and concentrated under reduced pressure. The resulting crude product was purified by column chromatography (MeOH/DCM = 0-1:9) and then purified by thin layer preparative plate (DCM:MeOH = 10:1) to give the target product 1-(3-(9-((4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carbonyl)phenyl)dihydropyrimidine-2,4(1*H*,3*H*)-dione.

LC-MS: (ESI, *m*/*z):* [M+H]⁺= 952.5.

¹H NMR (400 MHz, DMSO-*d*₆) δ 10.41 (s, 1H), 8.20-8.04 (m, 2H), 7.47-7.34 (m, 3H), 7.22 (d, *J* = 7.4 Hz, 1H), 6.99 (s, 1H), 6.88 (s, 1H), 6.85 (s, 1H), 6.70 (s, 1H), 5.63 - 5.48 (m, 3H), 3.88 (s, 3H), 3.82 (t, *J* = 6.7 Hz, 2H), 3.62-3.50 (m, 2H), 3.50-3.40 (m, 4H), 3.02-2.90 (m, 1H), 2.71 (t, *J* = 6.6 Hz, 2H), 2.68-2.60 (m, 1H), 2.40-2.30 (m, 5H), 2.20-2.10 (m, 2H), 1.90-1.80 (m, 4H), 1.68-1.40 (m, 18H), 1.11-0.92 (m, 4H).

### Example 22: 1-(4-(9-((4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)methyl)-3 -azaspiro [5.5]undecane-3-carbonyl)phenyl)dihydropyrimidine-2,4(1H,3H)-dione

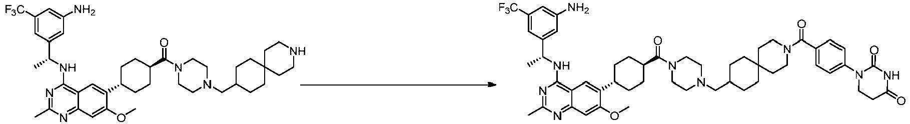

(4-((3-Azaspiro[5.5]undecane-9-yl)methyl)piperazin-1-yl)((1*R*,4*R*)-4-(4-(((*R*)-1-(3-amino-5-(trifluoromethyl)phenyl)ethyl)amino)-7-methoxy-2-methylquinazolin-6-yl)cyclohexyl)methanone (80 mg, 0.109 mmol) and pentafluorophenyl 4-(2,4-dioxotetrahydropyrimidin-1(2*H*)-yl)benzoate (44 mg, 0.109 mmol) were dissolved in DMSO (5 mL). DIEA (42 mg, 0.327 mmol) was added, and the reaction liquid was then stirred at room temperature for 2 h. Water (50 mL) was added to the reaction liquid, and the resulting mixture was stirred for 2 min and filtered. The solid was purified by column chromatography (10% MeOH/DCM) and then purified by p-TLC (MeOH/DCM = 1/10) to give 1-(4-(9-((4-((1R,4R)-4-(4-(((R)-1-(3-amino-5-(trifluoromethyl)phenyl)ethyl)amino)-7-methoxy-2-methylquinazolin-6-yl)cyclohexane-1 -carbonyl)piperazin-1 -yl)methyl)-3 -azaspiro [5.5]undecane-3 - carbonyl)phenyl)dihydropyrimidine-2,4(1*H*,3*H*)-dione.

LC-MS: (ESI, *m*/*z):* [M+H]⁺=952.6.

¹H NMR (400 MHz, DMSO-*d*₆) δ 10.43 (s, 1H), 8.09 (d, *J* = 22.5 Hz, 2H), 7.39 (s, 4H), 6.99 (s, 1H), 6.86 (d, *J* = 10.9 Hz, 2H), 6.70 (s, 1H), 5.64 - 5.48 (m, 3H), 3.88 (s, 3H), 3.83 (t, *J* = 6.5 Hz, 2H), 3.60 - 3.53 (m, 2H), 3.50 - 3.40 (m, 5H), 2.94 (s, 1H), 2.77 - 2.59 (m, 4H), 2.31 (d, *J* = 37.6 Hz, 8H), 2.12 (s, 2H), 1.91 - 1.75 (m, 4H), 1.75 - 1.60 (s, 4H), 1.60 - 1.40 (m, 11H), 1.15 - 0.95 (m, 4H).

### Example 23: 3-(4-(9-((4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)methyl)-3 -azaspiro [5.5]undecane-3-carbonyl)phenyl)piperidine-2,6-dione

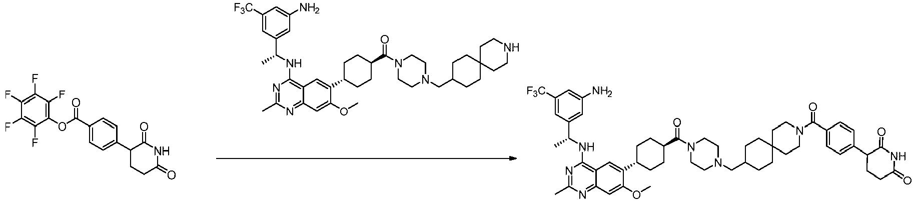

Pentafluorophenyl 4-(2,6-dioxopiperidin-3-yl)benzoate (143.12 mg, 0.358 mmol) and (4-((3-azaspiro[5.5]undecane-9-yl)methyl)piperazin-1-yl)((1*R*,4*R*)-4-(4-(((*R*)-1-(3-amino-5-(trifluoromethyl)phenyl)ethyl)amino)-7-methoxy-2-methylquinazolin-6-yl)cyclohexyl)methanone (240 mg, 0.326 mmol) were dissolved in DMSO (5 mL). DIEA (126.2 mg, 0.978 mmol) was added, and the reaction liquid was then stirred at room temperature for 2 h. Water (50 mL) was added to the reaction liquid, and the resulting mixture was stirred for 2 min and filtered. The solid was purified by reversed-phase preparative chromatography to give 3-(4-(9-((4-((1*R*,4*R*)-4-(4-(((*R*)-1-(3-amino-5-(trifluoromethyl)phenyl)ethyl)amino)-7-methoxy-2-methylquinazolin-6-yl)cyclohexyl-1-carbonyl)piperazin-1-yl)methyl)-3-azaspiro[5.5]undecane-3-carbonyl)phenyl)piperidine-2,6-dione.

LC-MS: (ESI, m/z): [M+H]⁺=951.1.

¹H NMR (400 MHz, DMSO-*d*₆) δ 10.86 (s, 1H), 8.20-8.10 (m, 1H), 8.06 (s, 1H), 7.33 (d, *J* = 8.1 Hz, 2H), 7.28 (d, *J* = 8.1 Hz, 2H), 6.99 (s, 1H), 6.88 (s, 1H), 6.85 (s, 1H), 6.70 (s, 1H), 5.67-5.47 (m, 3H), 3.94 - 3.88 (m, 4H), 3.65-3.42 (m, 8H), 3.00-2.90 (m, 1H), 2.71 - 2.62 (m, 4H), 2.42-2.22 (m, 10H), 2.16 - 2.02 (m, 4H), 1.90-1.76 (m, 6H), 1.74-1.36 (m, 10H), 1.20-0.92 (m, 4H).

### Example 24: 1-(5-(9-((4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carbonyl)-2-fluorophenyl)dihydropyrimidine-2,4(1H,3H)-dione

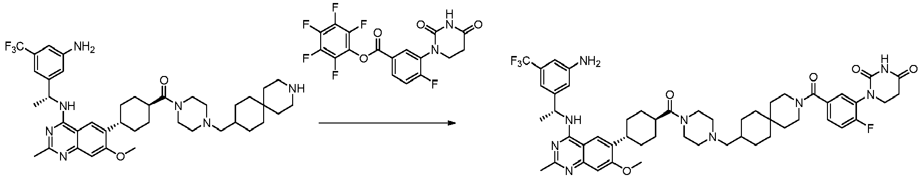

(4-((3-Azaspiro[5.5]undecane-9-yl)methyl)piperazin-1-yl)((1*R*,4*R*)-4-(4-(((*R*)-1-(3-amino-5-(trifluoromethyl)phenyl)ethyl)amino)-7-methoxy-2-methylquinazolin-6-yl)cyclohexyl)methanone (100 mg, 0.136 mmol) and pentafluorophenyl 3-(2,4-dioxotetrahydropyrimidin-1(2*H*)-yl)-4-fluorobenzoate (57 mg, 0.136 mmol) were dissolved in DMSO (5 mL). DIEA (53 mg, 0.41 mmol) was added, and the reaction liquid was stirred at room temperature for 2 h and diluted with water (50 mL). The resulting mixture was stirred for 2 min and filtered. The solid was purified by column chromatography (MeOH:DCM = 1:9) and then purified twice by p-TLC (MeOH:DCM = 1:10) to give 1-(5-(9-((4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carbonyl)-2-fluorophenyl)dihydropyrimidine-2,4(1*H*,3*H*)-dione.

LC-MS: (ESI, *m*/*z):* [M+H]⁺=970.7.

¹H NMR (400 MHz, DMSO-*d*₆) δ 10.52 (s, 1H), 8.14-8.08 (m, 1H), 8.06 (s, 1H), 7.50 (d, *J* = 7.9 Hz, 1H), 7.37 (d, *J* = 8.6 Hz, 2H), 6.99 (s, 1H), 6.89 (s, 1H), 6.86 (s, 1H), 6.70 (s, 1H), 5.61 - 5.48 (m, 3H), 3.88 (s, 3H), 3.75 (t, *J* = 6.6 Hz, 2H), 3.64 - 3.53 (m, 2H), 3.52-3.40 (m, 5H), 3.02-2.90 (m, 1H), 2.77 - 2.56 (m, 4H), 2.38 - 2.21 (m, 8H), 2.18-2.07 (m, 2H), 1.90 - 1.77 (m, 4H), 1.73-1.40 (m, 15H), 1.13-0.96 (m, 4H).

### Example 25: 1-(5-(4-(((2-(1-((1R,4R)-4-(4-(((R)-1-(3-amino-5-(trifluoromethyl)phenyl)ethyl)amino)-7-methoxy-2-methylquinazolin-6-yl)cyclohexane-1 -carbonyl)piperidin-4-yl)ethyl)(methyl)amino)methyl)piperidine-1 - carbonyl)-2-chlorophenyl)dihydropyrimidine-2,4(1H,3H)-dione

### Step 1: preparation of benzyl 4-(((2-(1-(tert-butoxycarbonyl)piperidin-4-yl)ethyl)(methyl)amino)methyl)piperidine-1-carboxylate

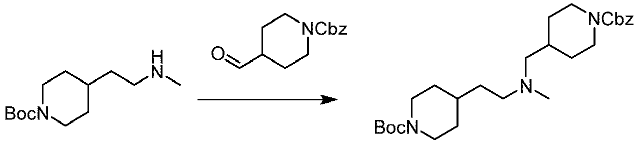

STAB (814 mg, 3.84 mmol) was added to a solution of tert-butyl 4-(2-(methylamino)ethyl)piperidine-1-carboxylate (310 mg, 1.28 mmol) and benzyl 4-formylpiperidine-1-carboxylate (316 mg, 1.28 mmol) in THF (10 mL). The reaction liquid was stirred at room temperature for 2 h. The reaction liquid was diluted with water (50 mL), and the resulting mixture was extracted with EA (50 mL × 3). The organic phases were combined, washed with saturated brine (150 mL), dried over anhydrous Na₂SO₄, filtered, and concentrated. The resulting crude product was subjected to flash column chromatography (MeOH/DCM = 0-1:4) to give benzyl 4-(((2-(1-(*tert*-butoxycarbonyl)piperidin-4-yl)ethyl)(methyl)amino)methyl)piperidine-1-carboxylate.

LC-MS: (ESI, m/z): [M+H]⁺=474.2.

### Step 2: preparation of benzyl 4-((methyl(2-(piperidin-4-yl)ethyl)amino)methyl)piperidine-1-carboxylate

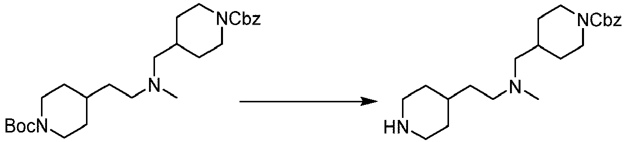

A solution of benzyl 4-(((2-(1-(tert-butoxycarbonyl)piperidin-4-yl)ethyl)(methyl)amino)methyl)piperidine-1-carboxylate (400 mg, 0.85 mmol) in TFA/DCM (10 mL, 1:4) was stirred at room temperature for 1 h. The reaction liquid was directly concentrated to give benzyl 4-((methyl(2-(piperidin-4-yl)ethyl)amino)methyl)piperidine-1-carboxylate. The crude product was directly used in the next step.

LC-MS: (ESI, m/z): [M+H]⁺=374.4.

### Step 3: preparation of benzyl 4-(((2-(1-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carbonyl)piperidin-4-yl)ethyl)(methyl)amino)methyl)piperidine-1-carboxylate

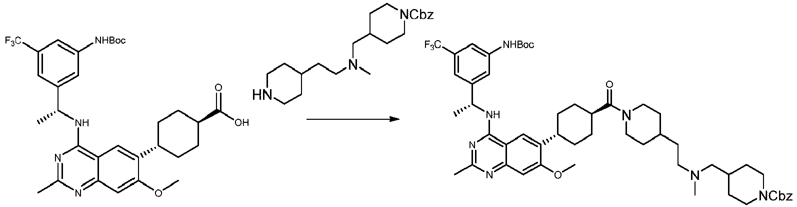

Benzyl 4-((methyl(2-(piperidin-4-yl)ethyl)amino)methyl)piperidine-1-carboxylate (139 mg,0.37 mmol), HATU (142 mg,0.37 mmol), and DIEA (161 mg, 1.25 mmol) were added to a solution of (1*R*,4*R*)-4-(4-(((*R*)-1-(3-((*tert-*butoxycarbonyljam ino)-5-(trifhioromethyl)phenyl)ethyl)amino)-7-methoxy-2-methylquinazolin-6-yl)cyclohexane-1-carboxylic acid (150 mg, 0.25 mmol) in DMF (10 mL). The reaction liquid was stirred at room temperature for 2 h. Water (50 mL) was added to quench the reaction, and the resulting mixture was extracted with EA (50 mL × 3). The organic phases were combined, washed with saturated brine (150 mL), dried over anhydrous Na₂SO₄, filtered, and concentrated. The resulting crude product was purified by column chromatography (MeOH:DCM = 0-1:9) to give benzyl 4-(((2-(1-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carbonyl)piperidin-4-yl)ethyl)(methyl)amino)methyl)piperidine-1-carboxylate.

LC-MS: (ESI, m/z): [M+H]⁺=958.3.

### Step 4: preparation of tert-butyl (3-((R)-1-((7-methoxy-2-methyl-6-((1R,4R)-4-(4-(2-(methyl(piperidin-4-ylmethyl)amino)ethyl)piperidine-1 -carbonyl)cyclohexyl)quinazolin-4-yl)amino)ethyl)-5 - (trifluoromethyl)phenyl)carboxylate

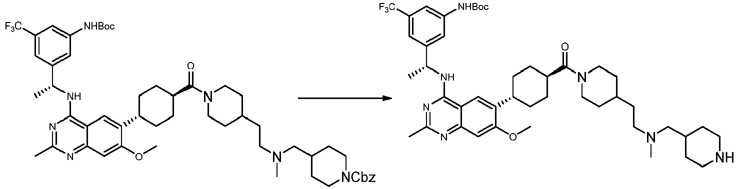

Pd(OH)₂/C (60 mg, 30%) was added to a solution of benzyl 4-(((2-(1-((1*R*,4*R*)-4-(4-(((*R*)-1-(3-((*tert-*butoxycarbonyl)amino)-5-(trifhioromethyl)phenyl)ethyl)amino)-7-methoxy-2-methylquinazolin-6-yl)cyclohexane-1-carbonyl)piperidin-4-yl)ethyl)(methyl)amino)methyl)piperidine-1-carboxylate (200 mg, 0.21 mmol) in EtOAc (10 mL). The reaction liquid was stirred at 70 °C for 4 h under H₂ atmosphere. The reaction liquid was filtered and concentrated to give tert-butyl (3-((R)-1-((7-methoxy-2-methyl-6-((1R,4R)-4-(4-(2-(methyl(piperidin-4-ylmethyl)amino)ethyl)piperidine-1-carbonyl)cyclohexyl)quinazolin-4-yl)amino)ethyl)-5-(trifluoromethyl)phenyl)carboxylate. The resulting product was directly used in the next step.

LC-MS: (ESI, m/z): [M+H]⁺=824.4.

### Step 5: preparation of tert-butyl (3-((R)-1-((6-((1R,4R)-4-(4-(2-(((1-(4-chloro-3-(2,4-dioxotetrahydropyrimidin-1(2H)-yl)benzoyl)piperidin-4-yl)methyl)(methyl)amino)ethyl)piperidine-1-carbonyl)cyclohexyl)-7-methoxy-2-methylquinazolin-4-yl)amino)ethyl)-5-(trifluoromethyl)phenyl)carboxylate

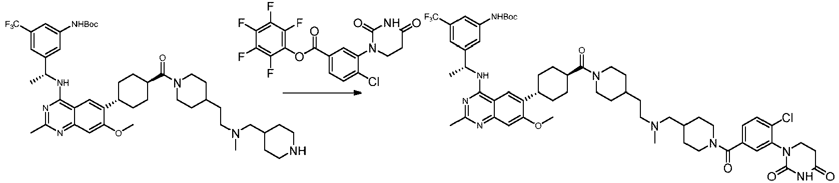

A mixture of tert-butyl (3-((*R*)-1-((7-methoxy-2-methyl-6-((1*R*,4*R*)-4-(4-(2-(methyl(piperidin-4-ylmethyl)amino)ethyl)piperidine-1-carbonyl)cyclohexyl)quinazolin-4-yl)amino)ethyl)-5 - (trifluoromethyl)phenyl)carboxylate (135 mg, 0.16 mmol), pentafluorophenyl 3-(2,4-dioxotetrahydropyrimidin-1(2H)-yl)-4-methoxybenzoate (69 mg, 0.16 mmol), and DIEA (62 mg, 0.48 mmol) in DMSO (5 mL) was stirred at room temperature overnight. The reaction liquid was poured into water (50 mL), and the resulting mixture was stirred for 10 min and filtered. The filter cake was purified by flash column chromatography (MeOH:DCM = 0-1:4) to give tert-butyl (3-((*R*)-1-((6-((1*R*,4*R*)-4-(4-(2-(((1-(4-chloro-3-(2,4-dioxotetrahydropyrimidin-1(2*H*)-yl)benzoyl)piperidin-4-yl)methyl)(methyl)amino)ethyl)piperidine-1-carbonyl)cyclohexyl)-7-methoxy-2-methylquinazolin-4-yl)amino)ethyl)-5-(trifluoromethyl)phenyl)carboxylate.

LC-MS: (ESI, m/z): [M+H]⁺=1074.2.

### Step 6: preparation of 1-(5-(4-(((2-(1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)ethyl)(methyl)amino)methyl)piperidine-1-carbonyl)-2-chlorophenyl)dihydropyrimidine-2,4(1H,3H)-dione

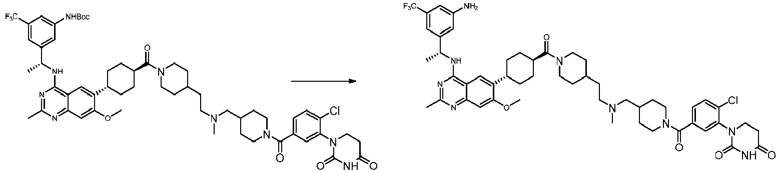

A mixture of tert-butyl (3-((*R*)-1-((6-((1*R*,4*R*)-4-(4-(2-(((1-(4-chloro-3-(2,4-dioxotetrahydropyrimidin-1(2*H*)-yl)benzoyl)piperidin-4-yl)methyl)(methyl)amino)ethyl)piperidine-1-carbonyl)cyclohexyl)-7-methoxy-2-methylquinazolin-4-yl)amino)ethyl)-5-(trifluoromethyl)phenyl)carboxylate(120mg,0.11mmol)andTFA/DCM(5 mL, 1:4) was stirred at room temperature for 1 h. The reaction liquid was directly concentrated, and then the residue was dissolved with EA (50 mL). The reaction liquid was washed with NaHCO₃ (aq, 30 mL × 2). The organic phase was washed with saturated brine (50 mL), dried over anhydrous Na₂SO₄, filtered, and concentrated. The crude product was purified by Prep-HPLC to give 1-(5-(4-(((2-(1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)ethyl)(methyl)amino)methyl)piperidine-1-carbonyl)-2-chlorophenyl)dihydropyrimidine-2,4(1*H*,3*H*)-dione. LC-MS: (ESI, m/z): [M+H]⁺=974.6.

¹H NMR (400 MHz, DMSO-*d*₆) δ 10.49 (s, 1H), 8.18 - 7.99 (m, 2H), 7.63 (d, *J* = 8.2 Hz, 1H), 7.54 (d, *J* = 1.8 Hz, 1H), 7.38 (d, *J* = 8.2 Hz, 1H), 6.99 (s, 1H), 6.88 (s, 1H), 6.86 (s, 1H), 6.70 (s, 1H), 5.63 - 5.47 (m, 3H), 4.49 - 4.38 (m, 1H), 3.95 - 3.85 (m, 4H), 3.79 - 3.69 (m, 1H), 3.67 - 3.50 (m, 2H), 3.09 - 2.87 (m, 3H), 2.78-2.63 (m, 4H), 2.35 (s, 3H), 2.33 - 2.26 (m, 2H), 2.15 - 2.08 (m, 4H), 1.92-1.48 (m, 20H), 1.40-1.30 (m, 2H), 1.15-0.98 (m, 4H).

### Example 26: (1R,4R)-4-(4-(((R)-1-(3-amino-5-(trifluoromethyl)phenyl)ethyl)amino)-7-methoxy-2-methylquinazolin-6-yl)-N-(2-((3-(1-(4-chloro-3-(2,4-dioxotetrahydropyrimidin-1(2H)-yl)benzoyl)piperidin-4-yl)prop-2-yn-1-yl)oxo)ethyl)-N-methylcyclohexane-1-carboxamide

### Step 1: preparation of tert-butyl (3-((R)-1-((6-((1R,4R)-4-((2-hydroxyethyl)(methyl)carbonyl)cyclohexyl)-7-methoxy-2-methylquinazolin-4-yl)amino)ethyl)-5-(trifluoromethyl)phenyl)carboxylate

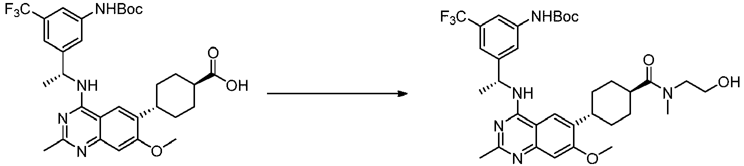

2-(Methylamino)ethan-1-ol (90 mg, 1.2 mmol), HATU (137 mg, 0.36 mmol), and DIEA (93 mg, 0.72 mmol) were added to a solution of (1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carboxylic acid (150 mg, 0.24 mmol) in DMF (5 mL). The reaction liquid was stirred at room temperature for 2 h. Water (50 mL) was added to quench the reaction, and the resulting mixture was extracted with EA (20 mL × 3). The organic phase was washed with saturated brine (60 mL), dried over anhydrous Na₂SO₄, filtered, and concentrated. The crude product was purified by flash column chromatography (MeOH:DCM = 0-1:9) to give tert-butyl (3-((*R*)-1-((6-((1*R*,4*R*)-4-((2-hydroxyethyl)(methyl)carbonyl)cyclohexyl)-7-methoxy-2-methylquinazolin-4-yl)amino)ethyl)-5-(trifluoromethyl)phenyl)carboxylate.

LC-MS: (ESI, m/z): [M+H]⁺=660.2.

### Step 2: preparation of tert-butyl 4-(3-(2-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)-N methylcyclohexane-1-amido)ethoxy)prop-1-yn-1-yl)piperidine-1-carboxylate

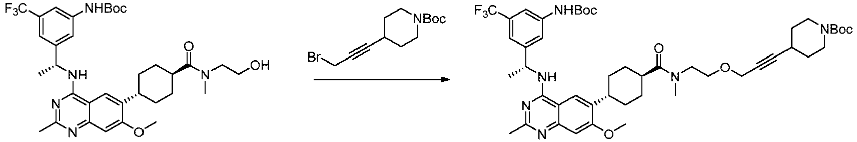

A mixture of tert-butyl (3-((R)-1-((6-((1R,4R)-4-((2-hydroxyethyl)(methyl)carbonyl)cyclohexyl)-7-methoxy-2-methylquinazolin-4-yl)amino)ethyl)-5-(trifluoromethyl)phenyl)carboxylate (150 mg, 0.22 mmol), tert-butyl 4-(3-bromoprop-1-yn-1-yl)piperidine-1-carboxylate (133 mg, 0.44 mmol), and K₂CO₃ (151 mg, 1.1 mmol) in acetonitrile (20 mL) was stirred at 70 °C overnight. The reaction liquid was diluted with water (50 mL) and extracted with EA (20 mL × 3). The organic phase was washed with saturated brine (60 mL), dried over anhydrous Na₂SO₄, filtered, and concentrated under reduced pressure. The crude product was purified by flash column chromatography (MeOH:DCM = 0-1:9) to give tert-butyl 4-(3-(2-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)-N methylcyclohexane-1-amido)ethoxy)prop-1 -yn-1 -yl)piperidine-1 -carboxylate.

LC-MS: (ESI, m/z): [M+H]⁺=881.3.

### Step 3: preparation of (1R,4R)-4-(4-(((R)-1-(3-amino-5-(trifluoromethyl)phenyl)ethyl)amino)-7-methoxy-2-methylquinazolin-6-yl)-N-methyl-N-(2-((3-(piperidin-4-yl)prop-2-yn-1-yl)oxy)ethyl)cyclohexane-1-carboxamide

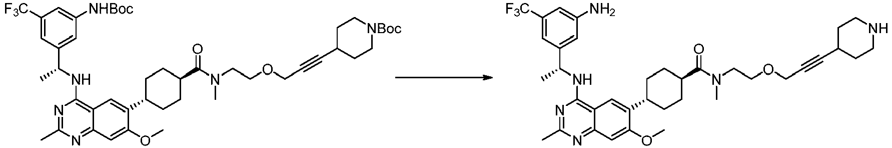

TFA (1.0 mL) was added to a solution of tert-butyl 4-(3-(2-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)-N methylcyclohexane-1-amido)ethoxy)prop-1-yn-1-yl)piperidine-1-carboxylate (100 mg, 0.11 mmol) in DCM (3 mL). The reaction liquid was stirred at room temperature for 2 h. The reaction liquid was directly concentrated to give (1*R*,4*R*)-4-(4-(((*R*)-1-(3-amino-5-(trifluoromethyl)phenyl)ethyl)amino)-7-methoxy-2-methylquinazoline-6-yl)-A-methyl-A-(2-((3-(piperidine-4-yl)prop-2-yn-1-yl)oxy)ethyl)cyclohexane-1-carboxamide. The resulting crude product was directly used in the next step.

LC-MS: (ESI, m/z): [M+H]⁺=681.3.

### Step 4: preparation of (1R,4R)-4-(4-(((R)-1-(3-amino-5-(trifluoromethyl)phenyl)ethyl)amino)-7-methoxy-2-methylquinazolin-6-yl)-N-(2-((3-(1-(4-chloro-3-(2,4-dioxotetrahydropyrimidin-1(2H)-yl)benzoyl)piperidin-4-yl)prop-2-yn-1-yl)oxo)ethyl)-N-methylcyclohexane-1-carboxamide

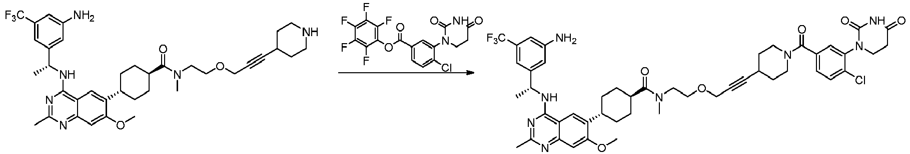

A reaction liquid of (1*R*,4*R*)-4-(4-(((*R*)-1-(3-amino-5-(trifluoromethyl)phenyl)ethyl)amino)-7-methoxy-2-methylquinazolin-6-yl)-*N*-methyl-*N*-(2-((3-(piperidin-4-yl)prop-2-yn-1-yl)oxy)ethyl)cyclohexane-1-carboxamide (100 mg, TFA salt), pentafluorophenyl 4-chloro-3-(2,4-dioxotetrahydropyrimidin-1(2*H*)-yl)benzoate (60 mg, 0.14 mmol), and DIEA (71 mg, 0.55 mmol) in DMSO (5 mL) was stirred at 25 °C overnight. The reaction liquid was diluted with water (50 mL) and filtered. The filtrate was concentrated. The resulting crude product was purified by Prep-HPLC (CH₃CN/0.08% aqueous NH₄HCO₃ solution, 5% to 95%) to give (1R,4R)-4-(4-(((R)-1-(3-amino-5-(trifluoromethyl)phenyl)ethyl)amino)-7-methoxy-2-methylquinazolin-6-yl)-*N*-(2-((3-(1-(4-chloro-3-(2,4-dioxotetrahydropyrimidin-1(2*H*)-yl)benzoyl)piperidin-4-yl)prop-2-yn-1-yl)oxo)ethyl)-*N*-methylcyclohexane-1-carboxamide.

LC-MS: (ESI, m/z): [M+H]⁺=931.4.

¹H NMR (400 MHz, DMSO-*d*₆) δ 10.50 (s, 1H), 8.15 (d, *J* = 7.7 Hz, 1H), 8.07 (s, 1H), 7.62 (d, *J* = 8.2 Hz, 1H), 7.52 (d, J = 1.8 Hz, 1H), 7.34 (dd, *J* = 8.2, 1.9 Hz, 1H), 6.99 (d, *J* = 5.6 Hz, 3H), 6.76 (s, 1H), 6.45 (t, *J* = 5.9 Hz, 1H), 5.61 (d, *J* = 7.1 Hz, 1H), 4.84-4.61 (m, 1H), 3.95-3.83 (m, 5H), 3.78-3.70 (m, 1H), 3.61-3.51 (m, 2H), 3.50-3.41 (m, 3H), 3.40-3.33 (m, 2H), 3.08 (s, 1H), 3.00-2.90 (m, 1H), 2.83 (s, 2H), 2.78-2.60 (m, 2H), 2.67 - 2.54 (m, 3H), 2.34 (s, 3H), 1.94-1.78 (m, 4H), 1.74-1.49 (m, 10H), 1.45-1.29 (m, 2H).

### Example 27: 1-(5-(4-(3-(4-((1R,4R)-4-(4-(((R)-1-(3-amino-5-(trifluoromethyl)phenyl)ethyl)amino)-7-methoxy-2-methylquinazolin-6-yl)cyclohexane-1 -carbonyl)piperazin-1 -yl)prop-1 -yn-1 -yl)piperidine-1 -carbonyl)-2-chlorophenyl)dihydropyrimidine-2,4(1H,3H)-dione

### Step 1: preparation of tert-butyl 4-(3-(4-((1R,4R)-4-(7-methoxy-2-methyl-4-(((R)-1-(3-nitro-5-(trifluoromethyl)phenyl)ethyl)amino)quinazolin-6-yl)cyclohexane-1 -carbonyl)piperazin-1 -yl)prop-1 -yn-1 - yl)piperidine-1-carboxylate

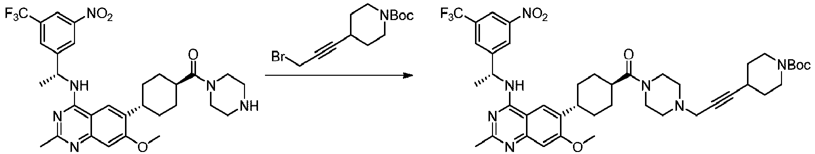

A reaction liquid of ((1R,4R)-4-(7-methoxy-2-methyl-4-(((R)-1-(3-nitro-5-(trifluoromethyl)phenyl)ethyl)amino)quinazolin-6-yl)cyclohexyl)(piperazin-1-yl)methanone (300 mg, 0.50 mmol), tert-butyl 4-(3-bromoprop-1-yn-1-yl)piperidine-1-carboxylate (150 mg, 0.50 mmol), and K₂CO₃ (138 mg, 1.0 mmol) in THF (30 mL) was stirred at 80 °C for 2 h. Water (50 mL) was added to the reaction liquid, and the resulting mixture was extracted with EA (30 mL × 3). The organic phases were combined, washed with saturated brine (50 mL), dried over anhydrous Na₂SO₄, filtered, and concentrated under reduced pressure. The resulting crude product was purified by flash column chromatography (MeOH:DCM = 0-1:9) to give tert-butyl 4-(3-(4-((1R,4R)-4-(7-methoxy-2-methyl-4-(((*R*)-1-(3-nitro-5-(trifluoromethyl)phenyl)ethyl)amino)quinazolin-6-yl)cyclohexane-1-carbonyl)piperazin-1-yl)prop-1-yn-1-yl)piperidine-1-carboxylate.

LC-MS: (ESI, m/z): [M+H]⁺=822.3.

### Step 2: preparation of tert-butyl 4-(3-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)prop-1-yn-1-yl)piperidine-1-carboxylate

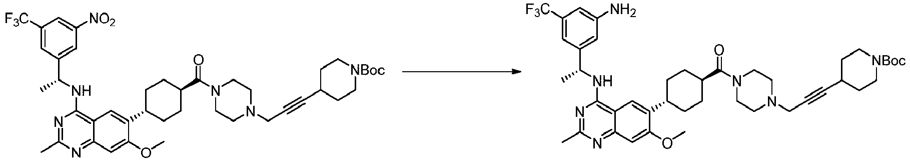

Fe (115 mg, 2.0 mmol) and NH₄Cl (106 mg, 2.0 mmol) were added to a solution of tert-butyl 4-(3-(4-((1R,4R)-4-(7-methoxy-2-methyl-4-(((R)-1 -(3-nitro-5-(trifluoromethyl)phenyl)ethyl)amino)quinazolin-6-yl)cyclohexane-1-carbonyl)piperazin-1-yl)prop-1-yn-1-yl)piperidine-1-carboxylate (170 mg, 0.2 mmol) in EtOH (25 mL). The reaction liquid was stirred at 70 °C for 5 h. The reaction liquid was filtered through celite, and the filtrate was concentrated. The resulting crude product was purified by flash column chromatography (MeOH:DCM = 0-1:9) to give tert-butyl 4-(3-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)prop-1-yn-1-yl)piperidine-1-carboxylate.

LC-MS: (ESI, m/z): [M+H]⁺=792.2.

### Step 3: preparation of ((1R,4R)-4-(4-(((R)-1-(3-amino-5-(trifluoromethyl)phenyl)ethyl)amino)-7-methoxy-2-methylquinazolin-6-yl)cyclohexyl)(4-(3-(piperidin-4-yl)prop-2-yn-1-yl)piperazin-1-yl)methanone

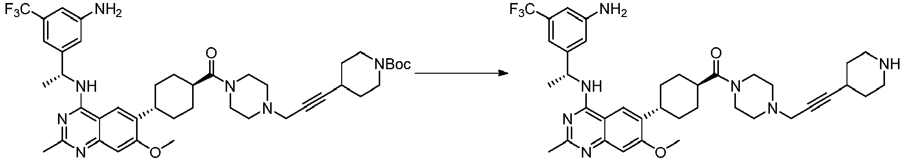

TFA (1.0 mL) was added to a solution of tert-butyl 4-(3-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)prop-1-yn-1-yl)piperidine-1-carboxylate (150 mg, 0.19 mmol) in DCM (3 mL). The reaction liquid was stirred at room temperature for 2 h. The reaction liquid was directly concentrated to give ((1R,4R)-4-(4-(((R)-1-(3-amino-5-(trifluoromethyl)phenyl)ethyl)amino)-7-methoxy-2-methylquinazolin-6-yl)cyclohexyl)(4-(3-(piperidin-4-yl)prop-2-yn-1-yl)piperazin-1-yl)methanone. The resulting crude product was directly used in the next step.

LC-MS: (ESI, m/z): [M+H]⁺=692.2.

### Step 4: preparation of 1-(5-(4-(3-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)prop-1-yn-1-yl)piperidine-1-carbonyl)-2-chlorophenyl)dihydropyrimidine-2,4(1H,3H)-dione

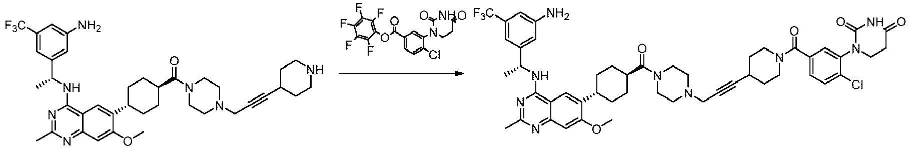

A reaction liquid of ((1R,4R)-4-(4-(((R)-1-(3-amino-5-(trifluoromethyl)phenyl)ethyl)amino)-7-methoxy-2-methylquinazolin-6-yl)cyclohexyl)(4-(3-(piperidin-4-yl)prop-2-yn-1-yl)piperazin-1-yl)methanone (200 mg, 0.19 mmol), pentafluorophenyl 4-chloro-3-(2,4-diyloxytetrahydropyrimidin-1(2*H*)-yl)benzoate (100 mg, 0.23 mmol), and DIEA (73 mg, 0.57 mmol) in DMSO (5 mL) was stirred at room temperature overnight. The reaction liquid was diluted with water (50 mL), extracted with DCM (20 mL × 3), dried over anhydrous Na₂SO₄, filtered, and concentrated under reduced pressure. The resulting crude product was purified by flash column chromatography (MeOH:DCM = 0-1:9) and then purified by Prep-TLC (DCM:MeOH = 10:1) to give 1-(5-(4-(3-(4-((1R,4R)-4-(4-(((*R*)-1-(3-amino-5-(trifluoromethyl)phenyl)ethyl)amino)-7-methoxy-2-methylquinazofin-6-yl)cyclohexane-1-carbonyl)piperazin-1-yl)prop-1-yn-1-yl)piperidine-1-carbonyl)-2-chlorophenyl)dihydropyrimidine-2,4(1*H*,3*H*)-dione.

LC-MS: (ESI, m/z): [M+H]⁺=942.2.

¹H NMR (400 MHz, DMSO-*d*₆) δ10.50 (s, 1H), 8.23-8.10 (m, 1H), 8.06 (s, 1H), 7.61 (d, *J* = 8.2 Hz, 1H), 7.56 (d, *J* = 1.8 Hz, 1H), 7.39 (dd, *J* = 8.2, 1.9 Hz, 1H), 6.99 (s, 1H), 6.88 (s, 1H), 6.85 (s, 1H), 6.70 (s, 1H), 5.69 - 5.39 (m, 3H), 3.88 (s, 3H), 3.78-3.71 (m, 1H), 3.63-3.43 (m, 6H), 3.33-3.20 (m, 4H), 2.94 (t, *J* = 11.2 Hz, 1H), 2.81 - 2.61 (m, 4H), 2.50-2.40 (m, 2H), 2.40-2.31 (m, 5H), 1.91-1.42 (m, 16H).

### Example 28: 1-(5-(4-(3-(4-((1R,4R)-4-(4-(((R)-1-(3-amino-5-(trifluoromethyl)phenyl)ethyl)amino)-7-methoxy-2-methylquinazolin-6-yl)cyclohexane-1 -carbonyl)piperazin-1 -yl)propoxy)piperidine-1 -carbonyl)-2-chlorophenyl)dihydropyrimidine-2,4(1H,3H)-dione

### Step 1: preparation of benzyl 4-(3-(piperazin-1-yl)propoxy)piperidine-1-carboxylate

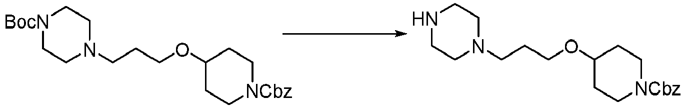

TFA (1 mL) was added to a solution of tert-butyl 4-(3-((1-((benzyloxy)carbonyl)piperidin-4-yl)oxo)propyl)piperazine-1-carboxylate (200 mg, 0.43 mmol) in DCM (3 mL). The reaction liquid was stirred at room temperature for 2 h. The reaction liquid was directly concentrated to give benzyl 4-(3-(piperazine-1-yl)propoxy)piperidine-1-carboxylate. The resulting crude product was directly used in the next step.

LC-MS: (ESI, m/z): [M+H]⁺=362.0.

### Step 2: preparation of benzyl 4-(3-(4-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carbonyl)piperazin-1-yl)propoxy)piperidine-1 -carboxylate

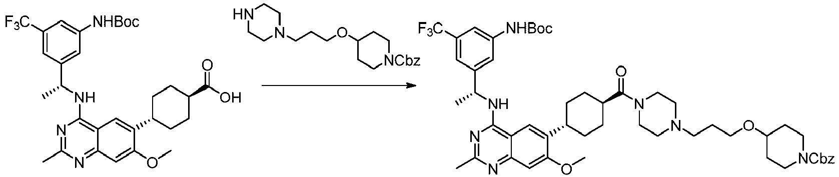

(1*R*,4*R*)-4-(4-(((*R*)-1-(3-((*tert*-Butoxycarbonyl)amino)-5-(trifluoromethyl)phenyl)ethyl)amino)-7-methoxy-2-methylquinazolin-6-yl)cyclohexane-1-carboxylic acid (150 mg, 0.25 mmol), HATU (142 mg, 0.37 mmol), and DIEA (96 mg, 0.74 mmol) were added to a solution of benzyl 4-(3-(piperazin-1-yl)propoxy)piperidine-1-carboxylate (200 mg, TFA salt) in DMF (6 mL) while stirring. The reaction liquid was stirred at room temperature for 2 h. Water (60 mL) was added to the reaction liquid to quench the reaction, and the resulting mixture was extracted with EA (20 mL × 3). The organic phases were combined, washed with saturated brine (60 mL), dried over anhydrous Na₂SO₄, filtered, and concentrated under reduced pressure. The resulting crude product was purified by flash column chromatography (EA:PE = 0-4:1) to give benzyl 4-(3-(4-((1*R*,4*R*)-4-(4-(((*R*)-1-(3-((*tert-*butoxycarbonyl)amino)-5-(trifluoromethyl)phenyl)ethyl)amino)-7-methoxy-2-methylquinazoline-6-yl)cyclohexane-1-carbonyl)piperazine-1-yl)propoxy)piperidine-1-carboxylate.

LC-MS: (ESI, m/z): [M+H]⁺=946.1.

### Step 3: preparation of tert-butyl (3-((R)-1-((7-methoxy-2-methyl-6-((1R,4R)-4-(4-(3-(piperidin-4-yloxy)propyl)piperazine-1 -carbonyl)cyclohexyl)quinazolin-4-yl)amino)ethyl)-5 - (trifluoromethyl)phenyl)carbamate

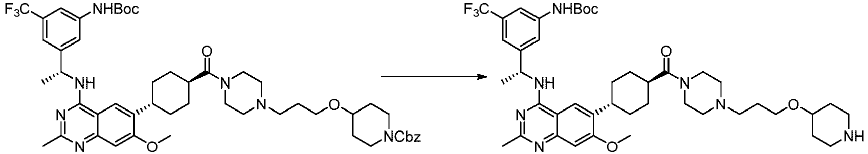

Pd(OH)₂/C (80 mg) was added to a solution of benzyl 4-(3-(4-((1*R*,4*R*)-4-(4-(((*R*)-1-(3-((*tert-*butoxycarbonyl)amino)-5-(trifluoromethyl)phenyl)ethyl)amino)-7-methoxy-2-methylquinazolin-6-yl)cyclohexane-1-carbonyl)piperazin-1-yl)propoxy)piperidine-1-carboxylate (160 mg, 0.17 mmol) in EA (20 mL) while stirring. The reaction liquid was stirred at 70 °C overnight under H₂ atmosphere. The reaction liquid was filtered through celite, and the filtrate was concentrated to give tert-butyl (3-((*R*)-1-((7-methoxy-2-methyl-6-((1*R*,4*R*)-4-(4-(3-(piperidin-4-yloxy)propyl)piperazine-1-carbonyl)cyclohexyl)quinazolin-4-yl)amino)ethyl)-5-(trifluoromethyl)phenyl)carbamate. The resulting crude product was directly used in the next step.

LC-MS: (ESI, m/z): [M+H]⁺=812.2.

### Step 4: preparation of tert-butyl (3-((R)-1-((6-((1R,4R)-4-(4-(3-((1-(4-chloro-3-(2,4-dioxotetrahydropyrimidin-1(2H)-yl)benzoyl)piperidin-4-yl)oxo)propyl)piperazine-1-carbonyl)cyclohexyl)-7-methoxy-2-methylquinazolin-4-yl)amino)ethyl)-5-(trifluoromethyl)phenyl)carbamate

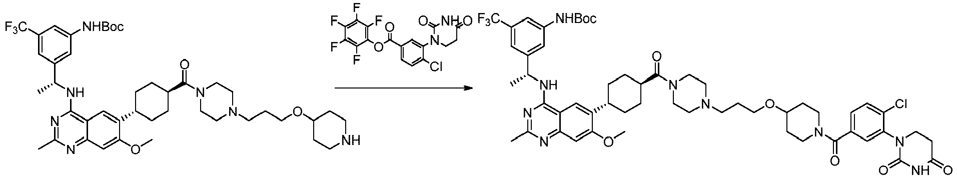

A reaction liquid of tert-butyl (3-((R)-1-((7-methoxy-2-methyl-6-((1R,4R)-4-(4-(3-(piperidin-4-yloxy)propyl)piperazine-1 -carbonyl)cyclohexyl)quinazolin-4-yl)amino)ethyl)-5 - (trifluoromethyl)phenyl)carbamate (110 mg, 0.13 mmol), pentafluorophenyl 4-chloro-3-(2,4-dioxo-1(2*H*)-yl)benzoate (71 mg, 0.16 mmol), and DIEA (52 mg, 0.40 mmol) in DMSO (3 mL) was stirred at room temperature overnight. The reaction liquid was diluted with water (50 mL) and extracted with DCM (20 mL × 3). The organic phases were combined, washed with saturated brine (60 mL), dried over anhydrous Na₂SO₄, filtered, and concentrated under reduced pressure. The resulting crude product was purified by flash column chromatography (MeOH:DCM = 0-1:9) to give tert-butyl (3-((R)-1-((6-((1R,4R)-4-(4-(3-((1-(4-chloro-3-(2,4-dioxotetrahydropyrimidin-1(2*H*)-yl)benzoyl)piperidin-4-yl)oxo)propyl)piperazine-1-carbonyl)cyclohexyl)-7-methoxy-2-methylquinazolin-4-yl)amino)ethyl)-5-(trifluoromethyl)phenyl)carbamate.

LC-MS: (ESI, m/z): [M+H]⁺=1062.5.

### Step 5: preparation of 1-(5-(4-(3-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)propoxy)piperidine-1-carbonyl)-2-chlorophenyl)dihydropyrimidine-2,4(1H,3H)-dione

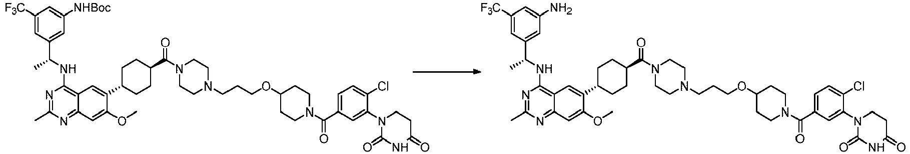

TFA (1.0 mL) was added to a solution of tert-butyl (3-((R)-1-((6-((1R,4R)-4-(4-(3-((1-(4-chloro-3-(2,4-dioxotetrahydropyrimidin-1(2*H*)-yl)benzoyl)piperidin-4-yl)oxo)propyl)piperazine-1-carbonyl)cyclohexyl)-7-methoxy-2-methylquinazolin-4-yl)amino)ethyl)-5-(trifluoromethyl)phenyl)carbamate (80 mg, 0.07 mmol) in DCM (3 mL). The reaction liquid was stirred at room temperature for 2 h. The reaction liquid was directly concentrated. The resulting crude product was dissolved in a mixed solution of DCM (20 mL) and saturated NaHCO₃ (20 mL). The resulting mixture was stirred for 30 min. The organic phase was isolated. The aqueous phase was extracted with DCM (20 mL × 2). The organic phases were combined, washed with saturated brine (60 mL), dried over anhydrous Na₂SO₄, filtered, and concentrated under reduced pressure. The resulting crude product was purified by flash column chromatography (MeOH:DCM = 0-1:9) to give 1-(5-(4-(3-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)propoxy)piperidine-1-carbonyl)-2-chlorophenyl)dihydropyrimidine-2,4(1H,3H)-dione.

LC-MS: (ESI, m/z): [M+H]⁺=962.4.

¹H NMR (400 MHz, DMSO-*d*₆) δ 10.50 (s, 1H), 8.39-8.17 (m, 1H), 8.09 (s, 1H), 7.63 (d, *J* = 8.2 Hz, 1H), 7.57 (d, *J* = 1.8 Hz, 1H), 7.40 (dd, *J* = 8.2, 2.0 Hz, 1H), 7.00 (s, 1H), 6.88 (s, 1H), 6.86 (s, 1H), 6.70 (s, 1H), 5.63 - 5.49 (m, 3H), 3.95-3.83 (m, 4H), 3.80-3.70 (m, 1H), 3.68-3.57 (m, 1H), 3.57-3.39 (m, 8H), 3.30-3.18 (m, 2H), 3.00-2.90 (m, 1H), 2.79 - 2.60 (m, 3H), 2.42-2.24 (m, 9H), 1.90-1.77 (m, 6H), 1.72-1.37 (m, 11H).

### Example 29: 1-(5-(4-(4-(4-((1R,4R)-4-(4-(((R)-1-(3-amino-5-(trifluoromethyl)phenyl)ethyl)amino)-7-methoxy-2-methylquinazolin-6-yl)cyclohexane-1 -carbonyl)piperazin-1 -yl)butyl)piperidine-1 -carbonyl)-2-chlorophenyl)dihydropyrimidine-2,4(1H,3H)-dione

### Step 1: preparation of tert-butyl 4-(4-carbonylbutyl)piperidine-1-carboxylate

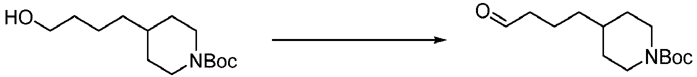

Dess-martin (3.96 g, 9.34 mmol) was added to a solution of tert-butyl 4-(4-hydroxybutyl)piperidine-1-carboxylate (1.2 g, 4.67 mmol) in DCM (20 mL) at 0 °C. The reaction liquid was stirred at room temperature overnight. Water (50 mL) was added to the reaction liquid, and the resulting mixture was extracted with DCM (50 mL × 3). The organic phases were combined, washed with saturated brine (100 mL), dried over anhydrous Na₂SO₄, filtered, and concentrated under reduced pressure. The resulting crude product was purified by flash column chromatography (EA:PE = 0-1:4) to give tert-butyl 4-(4-carbonylbutyl)piperidine-1-carboxylate.

¹H NMR (400 MHz, DMSO-*d*₆) δ 9.66 (t, *J* = 1.3 Hz, 1H), 3.91 (d, *J* = 12.0 Hz, 2H), 2.66 (s, 2H), 2.45 - 2.36 (m, 2H), 1.67 - 1.48 (m, 4H), 1.43 - 1.34 (m, 10H), 1.24 - 1.13 (m, 2H), 0.94 (2H).

### Step 2: preparation of benzyl 4-(4-(1-(tert-butoxycarbonyl)piperidin-4-yl)butyl)piperazine-1-carboxylate

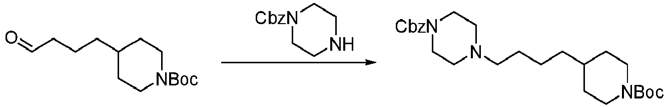

STAB (973 mg, 4.59 mmol) was added to a solution of tert-butyl 4-(4-carbonylbutyl)piperidine-1-carboxylate (390 mg, 1.53 mmol) and benzyl piperidine-1-carboxylate (390 mg, 1.53 mmol) in THF (10 mL). The reaction liquid was stirred at room temperature for 2 h. Water (50 mL) was added to the reaction liquid, and the resulting mixture was extracted with EA (50 mL × 3). The organic phases were combined, washed with saturated brine (150 mL), dried over anhydrous Na₂SO₄, filtered, and concentrated under reduced pressure. The resulting crude product was purified by flash column chromatography (EA:PE = 0-3:2) to give benzyl 4-(4-(1-(*tert*-butoxycarbonyl)piperidin-4-yl)butyl)piperazine-1 -carboxylate.

LC-MS: (ESI, m/z): [M+H]⁺=460.3.

### Step 3: preparation of tert-butyl 4-(4-(piperazin-1-yl)butyl)piperidine-1-carboxylate

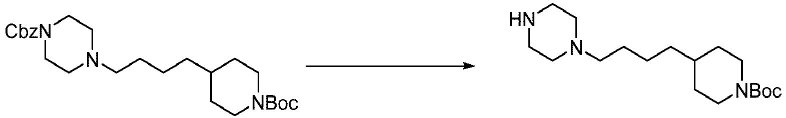

Pd(OH)₂/C (210 mg, 30%) was added to a solution of benzyl 4-(4-(1-(tert-butoxycarbonyl)piperidin-4-yl)butyl)piperazine-1-carboxylate (700 mg, 1.53 mmol) in EtOAc (10 mL) while stirring. The reaction liquid was stirred at 70 °C for 4 h under H₂ atmosphere. The reaction liquid was filtered, and the filtrate was concentrated to give tert-butyl 4-(4-(piperazin-1-yl)butyl)piperidine-1-carboxylate.

LC-MS: (ESI, m/z): [M+H]⁺=326.2.

### Step 4: preparation of tert-butyl 4-(4-(4-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carbonyl)piperazin-1-yl)butyl)piperidine-1-carboxylate

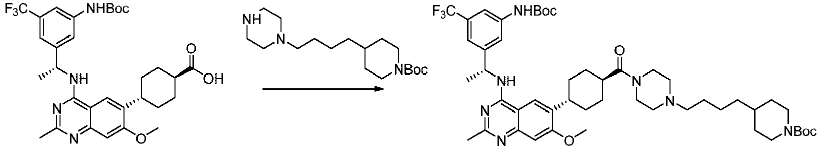

*tert*-Butyl 4-(4-(piperazin-1-yl)butyl)piperidine-1-carboxylate (120 mg, 0.37 mmol), HATU (140 mg, 0.37 mmol), and DIEA (108 mg, 0.84 mmol) were added to a solution of (1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carboxylic acid (170 mg, 0.28 mmol) in DMF (10 mL) while stirring. The reaction liquid was stirred at room temperature for 2 h. Water (50 mL) was added to the reaction liquid, and the resulting mixture was extracted with EA (50 mL × 3). The organic phases were combined, washed with saturated brine (150 mL), dried over anhydrous Na₂SO₄, filtered, and concentrated under reduced pressure. The resulting crude product was purified by flash column chromatography (MeOH:DCM = 0-1:9) to give tert-butyl 4-(4-(4-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carbonyl)piperazin-1-yl)butyl)piperidine-1-carboxylate.

LC-MS: (ESI, m/z): [M+H]⁺=910.3.

### Step 5: preparation of ((1R,4R)-4-(4-(((R)-1-(3-amino-5-(trifluoromethyl)phenyl)ethyl)amino)-7-methoxy-2-methylquinazolin-6-yl)cyclohexyl)(4-(4-(piperidin-4-yl)butyl)piperazin-1-yl)methanone

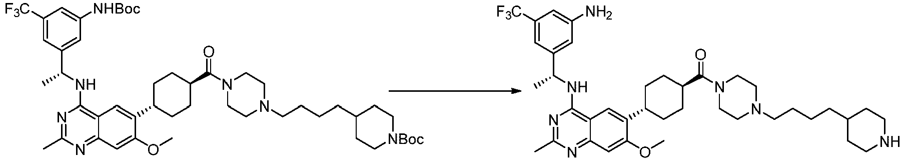

A solution of tert-butyl 4-(4-(4-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carbonyl)piperazin-1-yl)butyl)piperidine-1-carboxylate (180 mg, 0.20 mmol) in TFA/DCM (5 mL, 1:4) was stirred at room temperature for 1 h. The reaction liquid was directly concentrated to give ((1R,4R)-4-(4-(((R)-1-(3-amino-5-(trifluoromethyl)phenyl)ethyl)amino)-7-methoxy-2-methylquinazolin-6-yl)cyclohexyl)(4-(4-(piperidin-4-yl)butyl)piperazin-1-yl)methanone.

LC-MS: (ESI, m/z): [M+H]⁺=710.3.

### Step 6: preparation of 1-(5-(4-(4-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)butyl)piperidine-1-carbonyl)-2-chlorophenyl)dihydropyrimidine-2,4(1H,3H)-dione

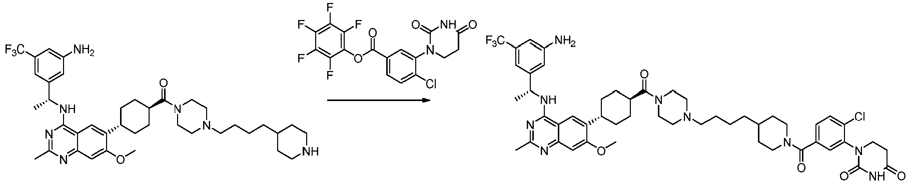

A reaction liquid of ((1R,4R)-4-(4-(((R)-1-(3-amino-5-(trifluoromethyl)phenyl)ethyl)amino)-7-methoxy-2-methylquinazolin-6-yl)cyclohexyl)(4-(4-(piperidin-4-yl)butyl)piperazin-1-yl)methanone (160 mg, 0.23 mmol)), pentafluorophenyl 4-chloro-3-(2,4-dioxotetrahydropyrimidin-1(2H)-yl)benzoate (98 mg, 0.23 mmol), and DIEA (148 mg, 1.15 mmol) in DMSO (5 mL) was stirred at room temperature overnight. The reaction liquid was poured into water (50 mL), and the resulting mixture was stirred for 10 min and filtered. The filter cake was purified by Prep-HPLC to give 1-(5-(4-(4-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)butyl)piperidine-1-carbonyl)-2-chlorophenyl)dihydropyrimidine-2,4(1*H*,3*H*)-dione.

LC-MS: (ESI, m/z): [M+H]⁺=960.4.

¹H NMR (400 MHz, DMSO-*d*₆) δ 10.50 (s, 1H), 8.15 - 7.98 (m, 2H), 7.63 (d, *J* = 8.2 Hz, 1H), 7.54 (d, *J* = 1.9 Hz, 1H), 7.37 (dd, *J* = 8.2, 1.9 Hz, 1H), 6.99 (s, 1H), 6.88 (s, 1H), 6.86 (s, 1H), 6.70 (s, 1H), 5.64 - 5.48 (m, 3H), 4.50 - 4.35 (m, 1H), 3.88 (s, 3H), 3.80 - 3.70 (m, 1H), 3.65 - 3.55 (m, 1H), 3.51 - 3.41 (m, 4H), 3.00-2.90 (m, 2H), 2.78-2.61 (m, 4H), 2.38 - 2.23 (m, 9H), 1.91-1.76 (m, 4H), 1.76 - 1.37 (m, 13H), 1.35 -1.20 (m, 4H), 1.13 - 1.00 (m, 2H).

### Example 30: 1-(5-(4-(2-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)ethoxy)piperidine-1-carbonyl)-2-chlorophenyl)dihydropyrimidine-2,4(1H,3H)-dione

### Step 1: preparation of benzyl 4-(2-ethoxy-2-carbonylethoxy)piperidine-1-carboxylate

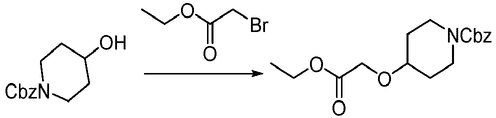

Methyl 2-bromoacetate (3.9 g, 25.4 mmol) was added to a solution of benzyl 4-hydroxypiperidine-1-carboxylate (3.0 g, 12.7 mmol) and NaH (335.0 mg, 13.9 mmol) in THF (30 mL), and the reaction liquid was stirred at 25 °C overnight. Ice water was added to quench the reaction, and the resulting mixture was extracted with EtOAc (30 mL × 2). The organic phases were combined, washed with saturated brine, dried over anhydrous Na₂SO₄, filtered, and concentrated under reduced pressure. The resulting crude product was purified by flash column chromatography (PE:EA = 4:1) to give benzyl 4-(2-ethoxy-2-carbonylethoxy)piperidine-1-carboxylate.

LC-MS: (ESI, m/z): [M+H]⁺=308.1.

### Step 2: preparation of benzyl 4-(2-hydroxyethoxy)piperidine-1-carboxylate

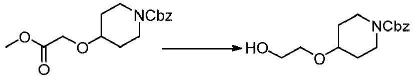

A reaction liquid of benzyl 4-(2-ethoxy-2-carbonylethoxy)piperidine-1-carboxylate (1.0 g, 3.2 mmol) and LiBH₄ (0.3 g, 12.8 mmol) in THF (15.0 mL) was stirred at 60 °C for 12 h. Ice water was added to quench the reaction, and the resulting mixture was extracted with EtOAc (30 mL × 2). The organic phases were combined, washed with saturated brine, dried over anhydrous Na₂SO₄, filtered, and concentrated under reduced pressure to give benzyl 4-(2-hydroxyethoxy)piperidine-1 -carboxylate.

LC-MS: (ESI, m/z): [M+H]⁺=280.1.

### Step 3: preparation of benzyl 4-(2-(tosyloxy)ethoxy)piperidine-1-carboxylate

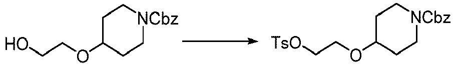

A reaction liquid of benzyl 4-(2-hydroxyethoxy)piperidine-1-carboxylate (700 mg, 2.5 mmol), TsCl (570 mg, 3.0 mmol), TEA (765 mg, 7.5 mmol), and DMAP (24 mg, 0.2 mmol) in DCM (20 mL) was stirred at room temperature for 15 h. Ice water was added to quench the reaction, and the resulting mixture was extracted with EtOAc (30 mL × 2). The organic phases were combined, washed with saturated brine, dried over anhydrous Na₂SO₄, filtered, and concentrated under reduced pressure. The resulting crude product was purified by flash column chromatography (PE:EA = 3:1) to give benzyl 4-(2-(tosyloxy)ethoxy)piperidine-1-carboxylate.

LC-MS: (ESI, m/z): [M+H]⁺= 434.3.

### Step 4: preparation of tert-butyl 4-(2-((1-((benzyloxy)carbonyl)piperidin-4-yl)oxo)ethyl)piperazine-1-carboxylate

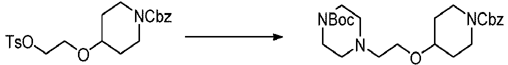

A reaction liquid of benzyl 4-(2-(tosyloxy)ethoxy)piperidine-1-carboxylate (700 mg, 1.6 mmol), tert-butyl piperazine-1-carboxylate (241 mg, 1.3 mmol), K₂CO₃ (662 mg, 4.8 mmol), and KI (318 mg, 1.9 mmol) in DMF (10 mL) was stirred at 70 °C overnight. Ice water was added to quench the reaction, and the resulting mixture was extracted with EtOAc (30 mL × 2). The organic phases were combined, washed with saturated brine, dried over anhydrous Na₂SO₄, filtered, and concentrated under reduced pressure. The resulting crude product was purified by column chromatography (EtOAc:PE = 0-3:7) to give tert-butyl 4-(2-((1-((benzyloxy)carbonyl)piperidin-4-yl)oxo)ethyl)piperazine-1-carboxylate.

LC-MS: (ESI, m/z): [M+H]⁺=448.3.

### Step 5: preparation of benzyl 4-(2-(piperazin-1-yl)ethoxy)piperidine-1-carboxylate

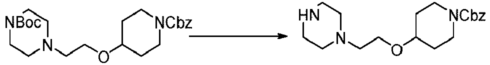

A reaction liquid of tert-butyl 4-(2-((1-((benzyloxy)carbonyl)piperidin-4-yl)oxo)ethyl)piperazine-1-carboxylate (390 mg, 0.87 mmol) in TFA/DCM (1 mL/3 mL) was stirred at room temperature for 1.0 h. The reaction liquid was directly concentrated to give benzyl 4-(2-(piperazine-1-yl)ethoxy)piperidine-1-carboxylate.

LC-MS: (ESI, m/z): [M+H]⁺=348.2.

### Step 6: preparation of benzyl 4-(2-(4-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carbonyl)piperazin-1-yl)ethoxy)piperidine-1-carboxylate

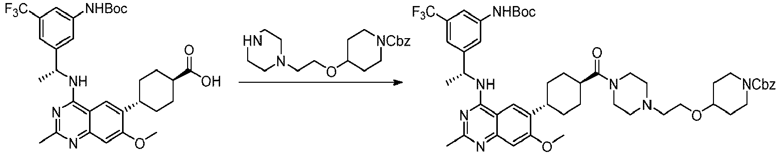

Benzyl 4-(2-(piperazin-1-yl)ethoxy)piperidine-1-carboxylate (76.0 mg, 0.22 mmol), HATU (91.0 mg, 0.24 mmol), and DIEA (59.0 mg, 0.6 mmol) were added to a solution of (1*R*,4*R*)-4-(4-(((*R*)-1-(3-((*tert*-butoxycarbonyl)amino)-5-(trifluoromethyl)phenyl)ethyl)amino)-7-methoxy-2-methylquinazolin-6-yl)cyclohexane-1-carboxylic acid (85.0 mg, 0.2 mmol) in DMF (2 mL). The reaction liquid was stirred at room temperature for 2 h. Ice water (20 mL) was added to quench the reaction, and the resulting mixture was filtered. The resulting filter cake was dried under vacuum to give benzyl 4-(2-(4-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carbonyl)piperazin-1-yl)ethoxy)piperidine-1-carboxylate.

LC-MS: (ESI, m/z): [M+H]⁺=932.2.

### Step 7: preparation of tert-butyl (3-((R)-1-((7-methoxy-2-methyl-6-((1R,4R)-4-(4-(2-(piperidin-4-oxy)ethyl)piperazine-1-carbonyl)cyclohexyl)quinazolin-4-yl)amino)ethyl)-5-(trifluoromethyl)phenyl)carbamate

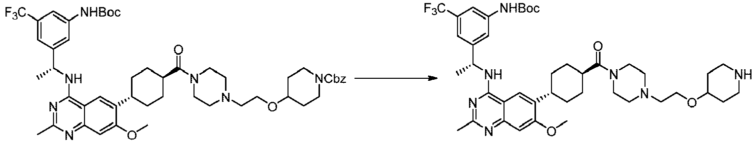

A solution of benzyl 4-(2-(4-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carbonyl)piperazin-1-yl)ethoxy)piperidine-1-carboxylate (130.0 mg, 0.14 mmol) and 20% Pd(OH)₂/C (60.0 mg) in THF (10 mL) was stirred at 60 °C overnight under H₂ (90 psi) atmosphere. The reaction liquid was filtered through celite, and the filtrate was concentrated under reduced pressure to give tert-butyl (3-((R)-1-((7-methoxy-2-methyl-6-((1R,4R)-4-(4-(2-(piperidine-4-oxy)ethyl)piperazine-1-carbonyl)cyclohexyl)quinazolin-4-yl)amino)ethyl)-5-(trifluoromethyl)phenyl)carbamate.

LC-MS: (ESI, m/z): [M+H]⁺=798.5.

### Step 8: preparation of tert-butyl (3-((R)-1-((6-((1R,4R)-4-(4-(2-((1-(4-chloro-3-(2,4-dioxotetrahydropyrimidin-1(2H)-yl)benzoyl)piperidin-4-yl)oxo)ethyl)piperazine-1-carbonyl)cyclohexyl)-7-methoxy-2-methylquinazolin-4-yl)amino)ethyl)-5-(trifluoromethyl)phenyl)carbamate

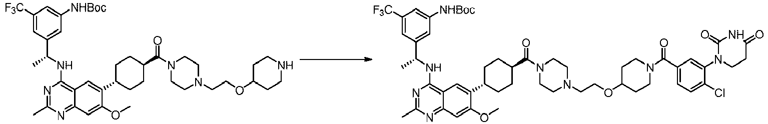

DIEA (103.2 mg, 0.8 mmol) and pentafluorophenyl 4-chloro-3-(2,4-dioxotetrahydropyrimidin-1(2*H*)-yl)benzoate (73.0 mg, 0.17 mmol) were added to a solution of tert-butyl (3-((R)-1-((7-methoxy-2-methyl-6-((1R,4R)-4-(4-(2-(piperidin-4-oxy)ethyl)piperazine-1-carbonyl)cyclohexyl)quinazolin-4-yl)amino)ethyl)-5-(trifluoromethyl)phenyl)carbamate (90.0 mg, 0.14 mmol) in DMSO (5 mL). The reaction liquid was stirred at room temperature for 2.0 h. Water (15 mL) was added to quench the reaction, and the resulting mixture was extracted with EtOAc (30 mL × 2). The organic phases were combined, washed with saturated brine, dried over anhydrous Na₂SO₄, filtered, and concentrated under reduced pressure. The resulting crude product was purified by Prep-HPLC (acetonitrile/0.05% aqueous NH₄HCO₃ solution, 5%-95%) to give tert-butyl (3-((R)-1-((6-((1R,4R)-4-(4-(2-((1-(4-chloro-3-(2,4-dioxotetrahydropyrimidin-1(2*H*)-yl)benzoyl)piperidin-4-yl)oxo)ethyl)piperazine-1-carbonyl)cyclohexyl)-7-methoxy-2-methylquinazolin-4-yl)amino)ethyl)-5-(trifluoromethyl)phenyl)carbamate. LC-MS: (ESI, m/z): [M+H]⁺=1048.7.

### Step 9: preparation of 1-(5-(4-(2-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)ethoxy)piperidine-1-carbonyl)-2-chlorophenyl)dihydropyrimidine-2,4(1H,3H)-dione

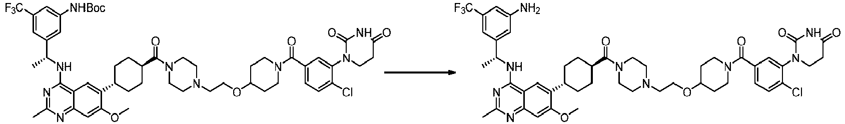

A solution of tert-butyl (3-((*R*)-1-((6-((1*R*,4*R*)-4-(4-(2-((1-(4-chloro-3-(2,4-dioxotetrahydropyrimidin-1(2*H*)-yl)benzoyl)piperidin-4-yl)oxo)ethyl)piperazine-1-carbonyl)cyclohexyl)-7-methoxy-2-methylquinazolin-4-yl)amino)ethyl)-5-(trifluoromethyl)phenyl)carbamate (75.0 mg, 0.1 mmol) in TFA/DCM (1.0 mL/3.0 mL) was stirred at room temperature for 1.0 h. The reaction liquid was directly concentrated under reduced pressure, and the residue was basified with 8% aqueous NaHCO₃ solution and extracted with DCM. The organic phase was isolated, washed with saturated brine, dried over anhydrous Na₂SO₄, and concentrated under reduced pressure to give 1-(5-(4-(2-(4-((1R,4R)-4-(4-(((R)-1-(3-amino-5-(trifluoromethyl)phenyl)ethyl)amino)-7-methoxy-2-methylquinazolin-6-yl)cyclohexane-1 -carbonyl)piperazin-1 -yl)ethoxy)piperidine-1 -carbonyl)-2-chlorophenyl)dihydropyrimidine-2,4(1*H*,3*H*)-dione.

LC-MS: (ESI, m/z): [M+H]⁺=948.6.

¹H NMR (400 MHz, DMSO-*d*₆) δ 10.51 (s, 1H), 8.13 (d, *J* = 7.5 Hz, 1H), 8.06 (s, 1H), 7.63 (d, *J* = 8.2 Hz, 2H), 7.57 (d, *J* = 1.9 Hz, 1H), 7.40 (dd, *J* = 8.2, 2.0 Hz, 1H), 6.99 (s, 1H), 6.86 (d, *J* = 10.7 Hz, 2H), 6.70 (s, 1H), 5.64 - 5.46 (m, 3H), 3.95-3.85 (m, 4H), 3.81 - 3.70 (m, 1H), 3.64 - 3.53 (m, 4H), 3.53-3.42 (m, 4H), 3.32-3.12 (m, 2H), 2.94 (t, *J* = 11.5 Hz, 1H), 2.80-2.60 (m, 3H), 2.48-2.30 (m, 7H), 1.94-1.74 (m, 6H), 1.72-1.39 (m, 10H), 1.28-1.11 (m, 2H).

### Example 31: 1-(5-(9-((4-((1R,4R)-4-((4-(((R)-1-(3-amino-5-(trifluoromethyl)phenyl)ethyl)amino)-7-methoxy-2-methylquinazolin-6-yl)oxo)cyclohexane-1-carbonyl)piperazin-1-yl)methyl)-3 -azaspiro [5.5]undecane-3 -carbonyl)-2-chlorophenyl)dihydropyrimidine-2,4(1H,3H)-dione

### Step 1: preparation of 6,7-dimethoxy-2-methylquinazolin-4-ol

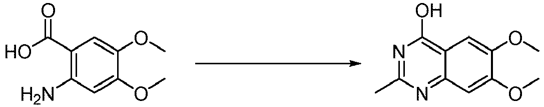

2-Amino-4,5-dimethoxybenzoic acid (20.0 g, 101.5 mmol) was added to acetamidine hydrochloride (19.1 g, 203.19 mmol) in methoxyethanol (120 mL). The reaction liquid was stirred at 140 °C for 12 h, and H₂O (250 mL) was added. The resulting mixture was stirred for 10 min, filtered, and concentrated under reduced pressure to give 6,7-dimethoxy-2-methylquinazolin-4-ol.

LC-MS: (ESI, m/z): [M+H]⁺=221.0.

### Step 2: preparation of 7-methoxy-2-methylquinazolin-4,6-diol

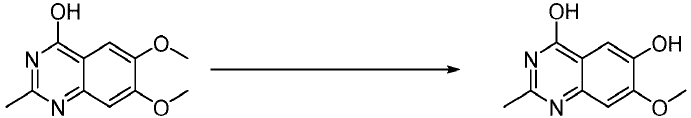

Methionine (13.16 g, 92.91 mmol) was added to 6,7-dimethoxy-2-methylquinazolin-4-ol (10.0 g, 44.4 mmol) in methanesulfonic acid (60 mL). The reaction liquid was stirred at 80 °C overnight. Ice water (20 mL) was added, and the resulting mixture was adjusted to be neutral with a saturated NaOH solution, stirred in an ice-water bath, and filtered. The filter cake was stirred in a mixed solution of acetonitrile/water (10:1) and filtered to give 7-methoxy-2-methylquinazolin-4,6-diol.

LC-MS: (ESI, m/z): [M+H]⁺=207.1.

### Step 3: preparation of methyl (1R,4R)-4-((4-hydroxy-7-methoxy-2-methylquinazolin-6-yl)oxo)cyclohexane-1-carboxylate

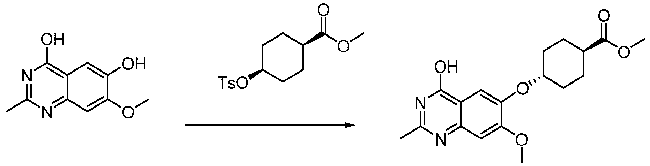

Methyl (1S,4S)-4-(tosyloxo)cyclohexane-1-carboxylate (5.08 g, 16.1 mmol) and K₂CO₃ (3.69 g, 26.8 mmol) were added to a solution of 7-methoxy-2-methylquinazolin-4,6-diol (2.75 g, 13.4 mmol) in NMP (100 mL). The reaction liquid was stirred at 30 °C overnight and extracted with ethyl acetate (100 mL × 3). The organic phase was washed with saturated brine (150 mL), dried over anhydrous sodium sulfate, filtered, and concentrated under reduced pressure. The crude product was purified by reversed-phase chromatography (H₂O:ACN = 3:1) and subjected to chiral resolution to give methyl (1*R*,4*R*)-4-((4-hydroxy-7-methoxy-2-methylquinazolin-6-yl)oxo)cyclohexane-1-carboxylate.

¹H NMR (400 MHz, DMSO-*d*₆) δ 12.03 (s, 1H), 7.44 (s, 1H), 7.06 (s, 1H), 4.43 - 4.33 (m, 1H), 3.87 (s, 3H), 3.61 (s, 3H), 2.44 - 2.36 (m, 1H), 2.31 (s, 3H), 2.13 - 2.03 (m, 2H), 1.97 (d, *J* = 13.3 Hz, 2H), 1.57 - 1.42 (m, 4H). Step 4: preparation of methyl (1R,4R)-4-((4-(((R)-1-(3-((tert-butoxycarbonyl)amino)-5-(trifluoromethyl)phenyl)ethyl)amino)-7-methoxy-2-methylquinazolin-6-yl)oxo)cyclohexane-1-carboxylate

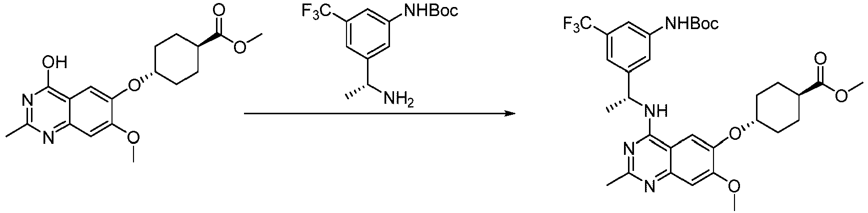

BOP (275.9 mg, 624.2 mmol), DBU (131.8 mg, 867.1 mmol), and tert-butyl (R)-(3-(1-aminoethyl)-5-(trifluoromethyl)phenyl)carbamate (137 mg, 0.45 mmol) were added to a solution of methyl (1*R*,4*R*)-4-((4-hydroxy-7-methoxy-2-methylquinazolin-6-yl)oxo)cyclohexane-1-carboxylate (120 mg, 346.8 mmol) in DMF (100 mL). The reaction liquid was stirred at 70 °C overnight and extracted with ethyl acetate (100 mL × 3). The organic phase was washed with saturated brine (150 mL), dried over anhydrous sodium sulfate, filtered, and concentrated under reduced pressure. The crude product was purified by reversed-phase chromatography (H₂O:ACN = 5:1) to give methyl (1R,4R)-4-((4-(((R)-1-(3-((tert-butoxycarbonyl)amino)-5-(trifluoromethyl)phenyl)ethyl)amino)-7-methoxy-2-methylquinazolin-6-yl)oxo)cyclohexane-1 -carboxylate.

LC-MS: (ESI, m/z): [M+H]⁺=633.1.

### Step 5: preparation of (1R,4R)-4-((4-(((R)-1-(3-((tert-butoxycarbonyl)amino)-5-(trifluoromethyl)phenyl)ethyl)amino)-7-methoxy-2-methylquinazolin-6-yl)oxo)cyclohexane-1-carboxylic acid

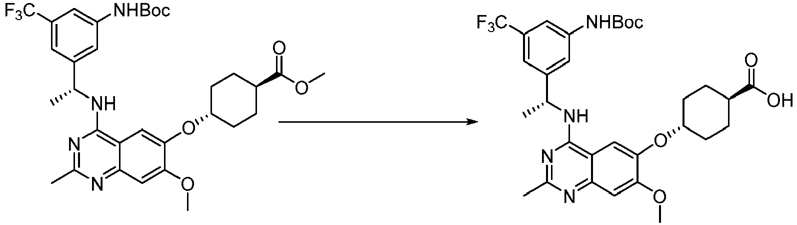

LiOH (320 mg, 7619.05 mmol) was added to methyl (1R,4R)-4-((4-(((R)-1-(3-((tert-butoxycarbonyl)amino)-5-(trifluoromethyl)phenyl)ethyl)amino)-7-methoxy-2-methylquinazolin-6-yl)oxo)cyclohexane-1-carboxylate (300 mg, 474.7 mmol) in THF/H₂O (50 mL/25 mL). The reaction liquid was stirred at 50 °C overnight. A citric acid solution (10%) was added to adjust the pH to neutrality under an ice bath, and the resulting mixture was extracted with ethyl acetate (200 mL). The organic phase was washed with saturated brine (150 mL), dried over anhydrous sodium sulfate, filtered, and concentrated under reduced pressure to give (1*R*,4*R*)-4-((4-(((*R*)-1-(3-((*tert-*butyloxycarbonyl)amino)-5-(trifluoromethyl)phenyl)ethyl)amino)-7-methoxy-2-methylquinazolin-6-yl)oxo)cyclohexane-1-carboxylic acid.

LC-MS: (ESI, m/z): [M+H]⁺=619.0.

### Step 6: preparation of benzyl 9-((4-((1R,4R)-4-((4-(((R)-1-(3-((tert-butoxycarbonyl)amino)-5-(trifluoromethyl)phenyl)ethyl)amino)-7-methoxy-2-methylquinazolin-6-yl)oxo)cyclohexane-1-carbonyl)piperazin-1 -yl)methyl)-3-azaspiro [5.5]undecane-3 -carboxylate

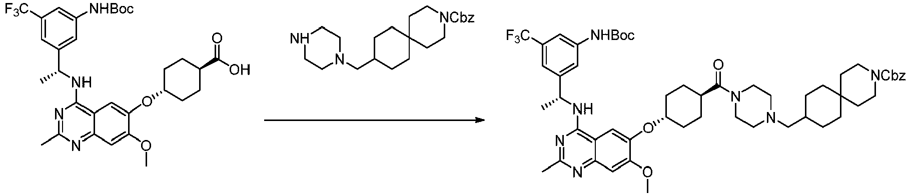

Benzyl 9-(piperazin-1-ylmethyl)-3-azaspiro[5.5]undecane-3-carboxylate (280.3 mg, 728.051 mmol), HATU (276.69 mg, 728.13 mmol), and DIEA (187.8 mg, 1455.8 mmol) were added to a solution of (1R,4R)-4-((4-(((R)-1-(3-((*tert*-butoxycarbonyl)amino)-5-(trifluoromethyl)phenyl)ethyl)amino)-7-methoxy-2-methylquinazolin-6-yl)oxo)cyclohexane-1-carboxylic acid (280 mg, 445.86 mmol) in DMF (20 mL). The reaction liquid was stirred at room temperature overnight, and water (200 mL) was added. The resulting mixture was stirred for 10 min and extracted with ethyl acetate (100 mL × 3). The organic phase was washed with saturated brine (150 mL), dried over anhydrous sodium sulfate, filtered, and concentrated under reduced pressure, and the crude product was purified by column chromatography (EA:PE = 1:4) to give benzyl 9-((4-((1*R*,4*R*)-4-((4-(((*R*)-1-(3-((*tert-*butoxycarbonyl)amino)-5-(trifluoromethyl)phenyl)ethyl)amino)-7-methoxy-2-methylquinazolin-6-yl)oxo)cyclohexane-1-carbonyl)piperazin-1-yl)methyl)-3-azaspiro[5.5]undecane-3-carboxylate.

LC-MS: (ESI, m/z): [M+H]⁺=986.1.

### Step 7: preparation of tert-butyl (3-((R)-1-((6-(((1R,4R)-4-(4-((3-azaspiro[5.5]undecan-9-yl)methyl)piperazine-1-carbonyl)cyclohexyl)oxo)-7-methoxy-2-methylquinazolin-4-yl)amino)ethyl)-5-(trifluoromethyl)phenyl)carbamate

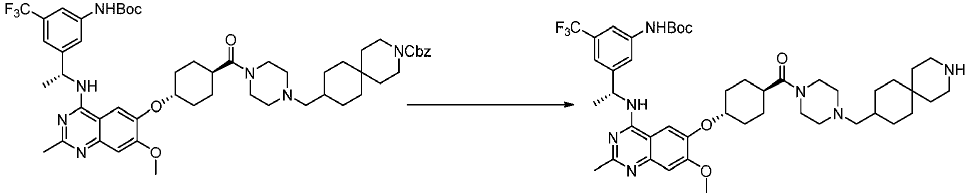

Benzyl 9-((4-((1R,4R)-4-((4-(((R)-1-(3-((tert-butoxycarbonyl)amino)-5-(trifluoromethyl)phenyl)ethyl)amino)-7-methoxy-2-methylquinazolin-6-yl)oxo)cyclohexane-1-carbonyl)piperazin-1-yl)methyl)-3 -azaspiro [5.5]undecane-3-carboxylate (360 mg, 0.582 mmol) was dissolved in ethyl acetate (20 mL), and Pd(OH)₂ (72 mg, 20%) was added. The reaction liquid was stirred at 50 °C for 2 h, filtered, and concentrated to dryness to give tert-butyl (3-((R)-1-((6-(((1*R*,4*R*)-4-(4-((3-azaspiro[5.5]undecan-9-yl)methyl)piperazine-1-carbonyl)cyclohexyl)oxo)-7-methoxy-2-methylquinazolin-4-yl)amino)ethyl)-5-(trifluoromethyl)phenyl)carbamate.

LC-MS-MC21-1867-57: [M+H]⁺=852.2.

### Step 8: preparation of tert-butyl (3-((R)-1-((-6-(((1R,4R)-4-(4-((3-(4-chloro-3-(2,4-dicarbonyltetrahydropyrimidin-1(2H)-yl)benzoyl)-3-azaspiro [5.5]undecan-9-yl)methyl)piperazine-1-carbonyl)cyclohexyl)oxo)-7-methoxy-2-methylquinazolin-4-yl)amino)ethyl)-5-(trifluoromethyl) phenyl)carbamate

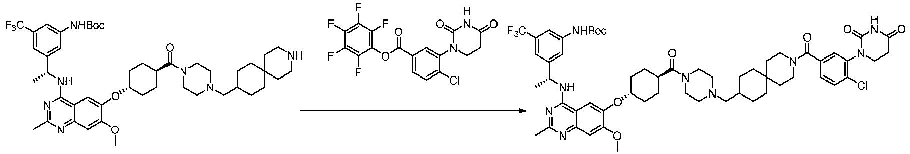

tert-Butyl (3-((*R*)-1-((6-(((1*R*,4*R*)-4-(4-((3-azaspiro[5.5]undecan-9-yl)methyl)piperazine-1-carbonyl)cyclohexyl)oxo)-7-methoxy-2-methylquinazolin-4-yl)amino)ethyl)-5-(trifluoromethyl)phenyl)carbamate (230 mg, 0.272 mmol) and pentafluorophenyl 4-chloro-3-(2,4-dioxotetrahydropyrimidin-1(2*H*)-yl)benzoate (140.7 mg, 0.324 mmol) were dissolved with DMSO (5 mL), and DIEA (100 mg, 0.775 mmol) was added. The reaction liquid was stirred at room temperature for 2 h, and water (50 mL) was added to the reaction liquid. The resulting mixture was stirred for 2 min and filtered to give the crude product tert-butyl (3-((R)-1-((6-(((1R,4R)-4-(4-((3-(4-chloro-3-(2,4-dicarbonyltetrahydropyrimidin-1(2*H*)-yl)benzoyl)-3-azaspiro[5.5]undecan-9-yl)methyl)piperazine-1-carbonyl)cyclohexyl)oxo)-7-methoxy-2-methylquinazolin-4-yl)amino)ethyl)-5-(trifluoromethyl)phenyl)carbamate.

LC-MS: (ESI, m/z): [M+H]⁺=1102.0.

### Step 9: preparation of 1-(5-(9-((4-((1R,4R)-4-((4-(((R)-1-(3-amino-5-(trifluoromethyl)phenyl)ethyl)amino)-7-methoxy-2-methylquinazolin-6-yl)oxo)cyclohexane-1-carbonyl)piperazin-1-yl)methyl)-3 -azaspiro [5.5]undecane-3-carbonyl)-2-chlorophenyl)dihydropyrimidine-2,4(1H,3H)-dione

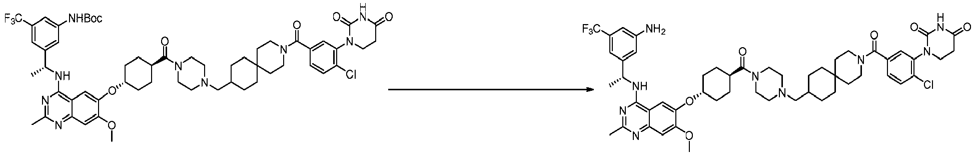

tert-Butyl (3-((*R*)-1-((6-(((1*R*,4*R*)-4-(4-((3-(4-chloro-3-(2,4-dicarbonyltetrahydropyrimidin-1(2*H*)-yl)benzoyl)-3-azaspiro[5.5]undecan-9-yl)methyl)piperazine-1-carbonyl)cyclohexyl)oxo)-7-methoxy-2-methylquinazolin-4-yl)amino)ethyl)-5-(trifluoromethyl)phenyl)carbamate (250 mg, 0.227 mmol) was dissolved with dichloromethane (20 mL), and trifluoroacetic acid (5 mL) was added. The reaction liquid was stirred at room temperature for 2 h. A saturated aqueous sodium bicarbonate solution was added to adjust the pH to neutrality, and the resulting mixture was extracted with ethyl acetate. The organic phase was washed with saturated brine (100 mL), dried over anhydrous sodium sulfate, filtered, and concentrated under reduced pressure. The resulting crude product was purified by preparative reversed-phase chromatography to give 1-(5-(9-((4-((1R,4R)-4-((4-(((R)-1-(3-amino-5-(trifluoromethyl)phenyl)ethyl)amino)-7-methoxy-2-methylquinazolin-6-yl)oxo)cyclohexane-1-carbonyl)piperazin-1-yl)methyl)-3-azaspiro[5.5]undecane-3-carbonyl)-2-chlorophenyl)dihydropyrimidine-2,4(1*H*,3*H*)-dione.

LC-MS: (ESI, m/z): [M+H]⁺=1002.1.

¹H NMR (400 MHz, MeOD) δ 8.45 (s, 1H), 7.88 (s, 1H), 7.66 (d, *J* = 8.2 Hz, 1H), 7.56 (s, 1H), 7.44 (d, *J* = 8.1 Hz, 1H), 7.07 (s, 1H), 6.98 (s, 2H), 6.84 (s, 1H), 5.75 (q, *J* = 6.8 Hz, 1H), 4.57-4.47(m, 1H), 4.00 (s, 3H), 3.84-3.61 (m, 8H), 3.51-3.49 (m, 2H), 2.93-2.72 (m, 3H), 2.61-2.42 (m, 7H), 2.34-2.21 (m, 4H), 1.95-1.33 (m, 18H), 1.30-1.03 (m, 4H).

### Example 32: 1-(5-(4-((2-(1-(4-(4-(((R)-1-(3-amino-5-(trifluoromethyl)phenyl)ethyl)amino)-7-methoxy-2-methylquinazolin-6-yl)cyclohex-3-ene-1-carbonyl)piperidin-4-yl)ethoxy)methyl)piperidine-1-carbonyl)-2-methoxyphenyl)dihydropyrimidine-2,4(1H,3H)-dione

### Step 1: preparation of tert-butyl 4-(2-(piperidin-4-ylmethoxy)ethyl)piperidine-1-carboxylate

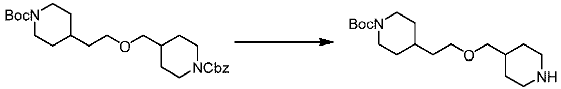

A mixture of benzyl 4-((2-(1-(tert-butoxycarbonyl)piperidin-4-yl)ethoxy)methyl)piperidine-1-carboxylate (300.0 mg, 0.6 mmol) and 10% Pd(OH)₂/C (150.0 mg) in THF (20 mL) was stirred at 70 °C for 12 h under H₂ atmosphere (60 psi). The reaction liquid was filtered, and the filtrate was concentrated under reduced pressure to give *tert*-butyl 4-(2-(piperidin-4-ylmethoxy)ethyl)piperidine-1-carboxylate.

LC-MS: (ESI, m/z): [M+H]⁺=325.3.

### Step 2: preparation of tert-butyl 4-(2-((1-(3-(2,4-dicarbonyltetrahydropyrimidin-1(2H)-yl)-4-methoxybenzoyl)piperidin-4-yl)methoxy)ethyl)piperidine-1-carboxylate

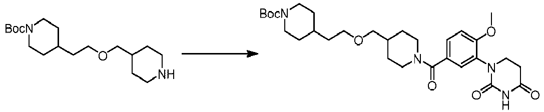

DIEA (103.2 mg, 0.8 mmol) and pentafluorophenyl 3-(2,4-dicarbonyltetrahydropyrimidin-1(2*H*)-yl)-4-methoxybenzoate (247 mg, 0.57 mmol) were added to a solution of tert-butyl 4-(2-(piperidin-4-ylmethoxy)ethyl)piperidine-1-carboxylate (170 mg, 0.52 mmol) in DMSO (5 mL). The reaction liquid was stirred at room temperature for 2 h. Water was added to quench the reaction, and the resulting mixture was extracted with EtOAc (30 mL × 2). The organic phases were combined, washed with saturated brine, dried over anhydrous Na₂SO₄, and concentrated. The resulting crude product was purified by flash column chromatography (PE:EA = 3:1) to give tert-butyl 4-(2-((1-(3-(2,4-dicarbonyltetrahydropyrimidin-1(2*H*)-yl)-4-methoxybenzoyl)piperidin-4-yl)methoxy)ethyl)piperidine-1 -carboxylate.

LC-MS: (ESI, m/z): [M+H]⁺=573.2.

### Step 3: preparation of 1-(2-methoxy-5-(4-((2-(piperidin-4-yl)ethoxy)methyl)piperidine-1-carbonyl)phenyl)dihydropyrimidine-2,4(1H,3H)-dione

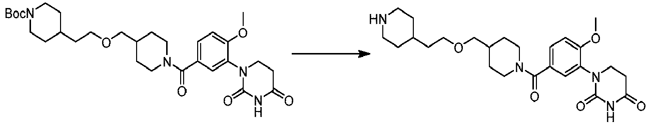

A solution of tert-butyl 4-(2-((1-(3-(2,4-dicarbonyltetrahydropyrimidin-1(2*H*)-yl)-4-methoxybenzoyl)piperidin-4-yl)methoxy)ethyl)piperidine-1-carboxylate (200 mg, 0.42 mmol) in TFA/DCM (1 mL/3 mL) was stirred at room temperature for 1 h. The reaction liquid was directly concentrated to give 1-(2-methoxy-5-(4-((2-(piperidine-4-yl)ethoxy)methyl)piperidine-1-carbonyl)phenyl)dihydropyrimidine-2,4(1*H*,3*H*)-dione. The resulting crude product was directly used in the next step.

LC-MS: (ESI, m/z): [M+H]⁺= 473.1.

### Step 4: preparation of methyl 4-(4-(((R)-1-(3-((tert-butoxycarbonyl)amino)-5-(trifluoromethyl)phenyl)ethyl)amino)-7-methoxy-2-methylquinazolin-6-yl)cyclohex-3-ene-1-carboxylate

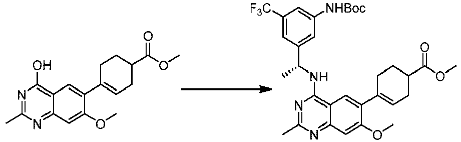

tert-Butyl (R)-(3-(1-aminoethyl)-5-(trifluoromethyl)phenyl)carbamate (273 mg, 0.91 mmol) was added to a mixture of methyl 4-(4-hydroxy-7-methoxy-2-methylquinazolin-6-yl)cyclohex-3-ene-1-carboxylate (200 mg, 0.61 mmol), DBU (228 mg, 1.5 mmol), and BOP (404 mg, 0.91 mmol) in DMF (5 mL). The reaction liquid was stirred at 70 °C for 14 h. Water was added to the reaction liquid, and the resulting mixture was extracted with EtOAc (30 mL × 2). The organic phases were combined, washed with saturated brine, and dried over anhydrous Na₂SO₄. The organic phase was concentrated under reduced pressure and the resulting crude product was purified by flash column chromatography (PE:EA = 1:1) to give methyl 4-(4-(((*R*)-1-(3-((*tert*-butoxycarbonyl)amino)-5-(trifluoromethyl)phenyl)ethyl)amino)-7-methoxy-2-methylquinazolin-6-yl)cyclohex-3-ene-1-carboxylate.

LC-MS: (ESI, m/z): [M+H]⁺= 615.3.

### Step 5: preparation of 4-(4-(((R)-1-(3-((tert-butoxycarbonyl)amino)-5-(trifluoromethyl)phenyl)ethyl)amino)-7-methoxy-2-methylquinazolin-6-yl)cyclohex-3-ene-1-carboxylic acid

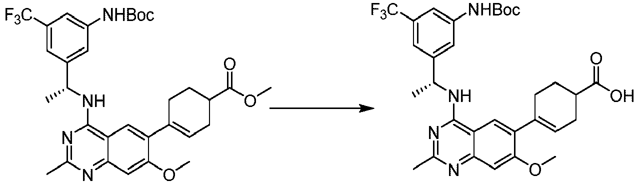

A mixture of methyl 4-(4-(((R)-1-(3-((tert-butoxycarbonyl)amino)-5-(trifluoromethyl)phenyl)ethyl)amino)-7-methoxy-2-methylquinazolin-6-yl)cyclohex-3-ene-1-carboxylate (150 mg, 0.24 mmol) and LiOH (110 mg, 4.8 mmol) in THF/H₂O (4 mL/2 mL) was stirred at 50 °C for 15 h. The reaction liquid was concentrated under reduced pressure to give 4-(4-(((*R*)-1-(3-((*tert*-butoxycarbonyl)amino)-5-(trifluoromethyl)phenyl)ethyl)amino)-7-methoxy-2-methylquinazolin-6-yl)cyclohex-3-ene-1-carboxylic acid.

LC-MS: (ESI, m/z): [M+H]⁺= 601.2.

### Step 6: preparation of tert-butyl (3-((1R)-1-((6-(4-(4-(2-((1-(3-(2,4-dioxotetrahydropyrimidin-1(2H)-yl)-4-methoxybenzoyl)piperidin-4-yl)methoxy)ethyl)piperidine-1-carbonyl)cyclohex-1-en-1-yl)-7-methoxy-2-methylquinazolin-4-yl)amino)ethyl)-5-(trifluoromethyl)phenyl)carbamate

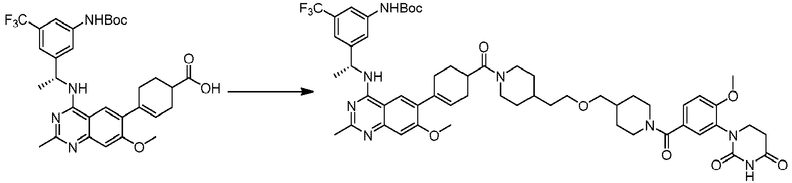

A mixture of 4-(4-(((*R*)-1-(3-((*tert*-butoxycarbonyl)amino)-5-(trifluoromethyl)phenyl)ethyl)amino)-7-methoxy-2-methylquinazolin-6-yl)cyclohex-3-ene-1-carboxylic acid (120.0 mg, 0.2 mmol), 1-(2-methoxy-5-(4-((2-(piperidin-4-yl)ethoxy)methyl)piperidine-1-carbonyl)phenyl)dihydropyrimidine-2,4(1*H*,3*H*)-dione (104 mg, 0.22 mmol), HATU (91.0 mg, 0.24 mmol), and DIEA (59.0 mg, 0.6 mmol) in DMF (3 mL) was stirred at room temperature for 2 h. Water was added to the reaction liquid, and the resulting mixture was filtered. The filter cake was purified by Prep-HPLC (acetonitrile/0.05% aqueous NH₄HCO₃ solution, 5%-95%) to give tert-butyl (3-((1R)-1-((6-(4-(4-(2-((1-(3-(2,4-dioxotetrahydropyrimidin-1(2*H*)-yl)-4-methoxybenzoyl)piperidin-4-yl)methoxy)ethyl)piperidine-1-carbonyl)cyclohex-1-en-1-yl)-7-methoxy-2-methylquinazolin-4-yl)amino)ethyl)-5 - (trifluoromethyl)phenyl)carbamate.

LC-MS: (ESI, m/z): [M+H]⁺=1056.6.

### Step 7: preparation of 1-(5-(4-((2-(1-(4-(4-(((R)-1-(3-amino-5-(trifluoromethyl)phenyl)ethyl)amino)-7-methoxy-2-methylquinazolin-6-yl)cyclohex-3-ene-1-carbonyl)piperidin-4-yl)ethoxy)methyl)piperidine-1-carbonyl)-2-methoxyphenyl)dihydropyrimidine-2,4(1H,3H)-dione

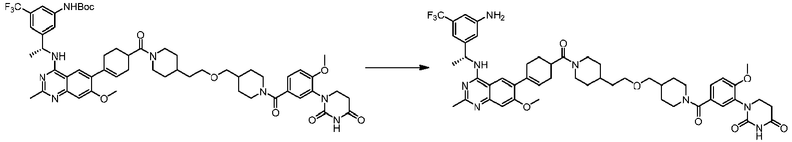

A reaction liquid of tert-butyl (3-((1*R*)-1-((6-(4-(4-(2-((1-(3-(2,4-dioxotetrahydropyrimidin-1(2*H*)-yl)-4-methoxybenzoyl)piperidin-4-yl)methoxy)ethyl)piperidine-1-carbonyl)cyclohex-1-en-1-yl)-7-methoxy-2-methylquinazolin-4-yl)amino)ethyl)-5-(trifluoromethyl)phenyl)carbamate (100.0 mg, 0.1 mmol) in TFA/DCM (1 mL/3 mL) was stirred at room temperature for 1 h. The reaction liquid was concentrated, and the residue was basified with an 8% aqueous NaHCO₃ solution and then extracted with DCM (20 mL × 3). The organic phase was washed with saturated brine, dried over anhydrous sodium sulfate, and concentrated under reduced pressure to give 1-(5-(4-((2-(1-(4-(4-(((R)-1-(3-amino-5-(trifluoromethyl)phenyl)ethyl)amino)-7-methoxy-2-methylquinazolin-6-yl)cyclohex-3-ene-1-carbonyl)piperidin-4-yl)ethoxy)methyl)piperidin-1-carbonyl)-2-methoxyphenyl)dihydropyrimidine-2,4(1*H*,3*H*)-dione.

LC-MS: (ESI, m/z): [M+H]⁺= 955.2.

¹H NMR (400 MHz, DMSO-*d*₆) δ 10.33 (s, 1H), 8.29 (s, 1H), 8.08 (s, 1H), 7.38 (dd, *J* = 8.5, 2.1 Hz, 1H), 7.32 (d, *J* = 2.0 Hz, 1H), 7.15 (d, *J* = 8.6 Hz, 1H), 6.99 (s, 1H), 6.89 (s, 1H), 6.85 (s, 1H), 6.69 (s, 1H), 5.83 (s, 1H), 5.60-5.50 (m, 3H), 4.47-4.33 (m, 1H), 4.00-3.90 (m, 1H), 3.86 (s, 3H), 3.84 (s, 3H), 3.61-3.58 (t, *J* = 6.7 Hz, 2H), 3.45-3.38 (t, *J* = 6.3 Hz, 2H), 3.33-3.28 (m, 2H), 3.25-3.21 (d, *J* = 6.3 Hz, 2H), 3.08-2.97 (m, 1H), 2.95-2.82 (m, 2H), 2.70-2.65 (t, *J* = 6.4 Hz, 2H), 2.58-2.52 (m, 2H), 2.40-2.32 (m, 5H), 2.25-2.15 (m, 1H), 1.90-1.38 (m, 13H), 1.28-0.92 (m, 5H).

### Example 33: 1-(5-(4-((2-(4-((1R,4R)-4-(4-(((R)-1-(3-amino-5-(trifluoromethyl)phenyl)ethyl)amino)-7-methoxy-2-methylquinazolin-6-yl)cyclohexane-1 -carbonyl)piperazin-1 -yl)ethoxy)methyl)piperidine-1 -carbonyl)-2-chlorophenyl)dihydropyrimidine-2,4(1H,3H)-dione

### Step 1: preparation of tert-butyl 4-(2-hydroxyethyl)piperazine-1-carboxylate

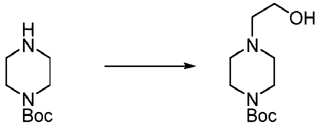

A reaction liquid of tert-butyl piperazine-1-carboxylate (1.0 g, 5.38 mmol), 2-bromoethane-1-ol (0.67 g, 5.38 mmol), and K₂CO₃ (1.48 g, 10.76 mmol) in DMF (20 mL) was stirred at room temperature overnight. Water (50 mL) was added to the reaction liquid, and the resulting mixture was extracted with EA (50 mL × 3). The organic phase was washed with saturated brine (100 mL), dried over anhydrous Na₂SO₄, filtered, and concentrated under reduced pressure. The resulting crude product was purified by flash column chromatography (MeOH:DCM = 0-1:9) to give tert-butyl 4-(2-hydroxyethyl)piperazine-1-carboxylate.

¹H NMR (400 MHz, MeOD) δ 3.67 (t, *J* = 5.9 Hz, 2H), 3.47 - 3.39 (m, 4H), 2.53 (t, *J* = 5.9 Hz, 2H), 2.50 - 2.42 (m, 4H), 1.45 (s, 9H).

### Step 2: preparation of tert-butyl 4-(2-(pyridin-4-ylmethoxy)ethyl)piperazine-1-carboxylate

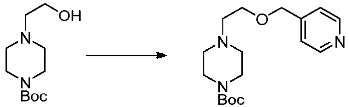

NaH (140 mg, 60%, 3.48 mmol) was added to a solution of tert-butyl 4-(2-hydroxyethyl)piperazine-1-carboxylate (400 mg, 1.74 mmol) in anhydrous THF (10 mL) at 0 °C. The reaction liquid was stirred for 2 h. 4-(Bromomethyl)pyridine hydrobromide (528 mg, 2.09 mmol) was then added, and the reaction liquid was stirred at room temperature overnight. Water (50 mL) was added to the reaction liquid, and the resulting mixture was extracted with EA (50 mL × 3). The organic phase was washed with saturated brine (150 mL), dried over anhydrous Na₂SO₄, filtered, and concentrated under reduced pressure. The resulting crude product was purified by column chromatography (MeOH:DCM = 0-1:9) to give tert-butyl 4-(2-(pyridin-4-ylmethoxy)ethyl)piperazine-1-carboxylate.

LC-MS: (ESI, m/z): [M+H]⁺=322.6.

### Step 3: preparation of tert-butyl 4-(2-(piperidin-4-ylmethoxy)ethyl)piperazine-1-carboxylate

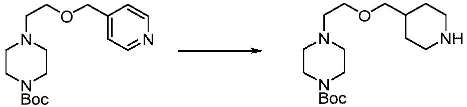

Pd/C (180 mg) was added to a solution of tert-butyl 4-(2-(pyridin-4-ylmethoxy)ethyl)piperazine-1-carboxylate (600 mg, 2.60 mmol) in *i*-PrOH/H₂O (13 mL, 6:7) while stirring. Then the reaction liquid was then stirred at 75 °C overnight under H₂ atmosphere. The reaction liquid was filtered and concentrated under reduced pressure to give *tert*-butyl 4-(2-(piperidin-4-ylmethoxy)ethyl)piperazine-1-carboxylate. The resulting crude product was directly used in the next step.

LC-MS: (ESI, m/z): [M+H]⁺=328.2.

### Step 4: preparation of tert-butyl 4-(2-((1-(4-chloro-3-(2,4-dicarbonyltetrahydropyrimidin-1(2H)-yl)benzoyl)piperidin-4-yl)methoxy)ethyl)piperazine-1-carboxylate

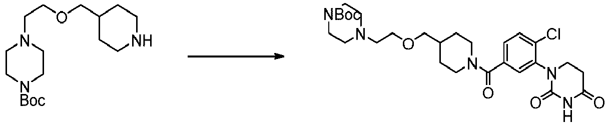

DIEA (213 mg, 1.65 mmol) and pentafluorophenyl 4-chloro-3-(2,4-dioxotetrahydropyrimidin-1(2*H*)-yl)benzoate (238 mg, 0.55 mmol) were added to a solution of tert-butyl 4-(2-(piperidin-4-ylmethoxy)ethyl)piperazine-1-carboxylate (180 mg, 0.55 mmol) in DMSO (5 mL). The reaction liquid was stirred at room temperature for 2 h. Water (50 mL) was added to the reaction liquid, and the resulting mixture was extracted with EA (50 mL × 3). The organic phase was washed with saturated brine (150 mL), dried over anhydrous Na₂SO₄, filtered, and concentrated under reduced pressure. The resulting crude product was purified by flash column chromatography (MeOH:DCM = 0-1:9) to give tert-butyl 4-(2-((1-(4-chloro-3-(2,4-dicarbonyltetrahydropyrimidin-1(2*H*)-yl)benzoyl)piperidin-4-yl)methoxy)ethyl)piperazine-1-carboxylate.

LC-MS: (ESI, m/z): [M+H]⁺=578.2.

### Step 5: preparation of 1-(2-chloro-5-(4-((2-(piperazin-1-yl)ethoxy)methyl)piperidine-1-carbonyl)phenyl)dihydropyrimidine-2,4(1H,3H)-dione

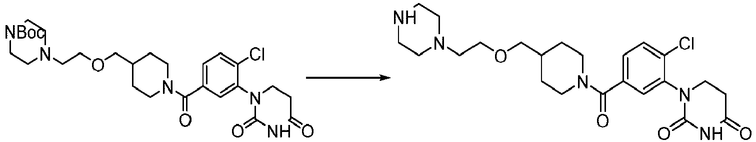

A solution of tert-butyl 4-(2-((1-(4-chloro-3-(2,4-dioxotetrahydropyrimidin-1(2*H*)-yl)benzoyl)piperidin-4-yl)methoxy)ethyl)piperazine-1-carboxylate (160 mg, 0.28 mmol) in TFA/DCM (5 mL, 1:4) was stirred at room temperature for 1 h. The reaction liquid was directly concentrated to give 1-(2-chloro-5-(4-((2-(piperazine-1-yl)ethoxy)methyl)piperidine-1-carbonyl)phenyl)dihydropyrimidine-2,4(1*H*,3*H*)-dione. The resulting crude product was directly used in the next step.

LC-MS: (ESI, m/z): [M+H]⁺=478.3.

### Step 6: preparation of tert-butyl (3-((R)-1-((6-((1R,4R)-4-(4-(2-((1-(4-chloro-3-(2,4-dioxotetrahydropyrimidin-1(2H)-yl)benzoyl)piperidin-4-yl)methoxy)ethyl)piperazine-1-carbonyl)cyclohexyl)-7-methoxy-2-methylquinazolin-4-yl)amino)ethyl)-5-(trifluoromethyl)phenyl)carbamate

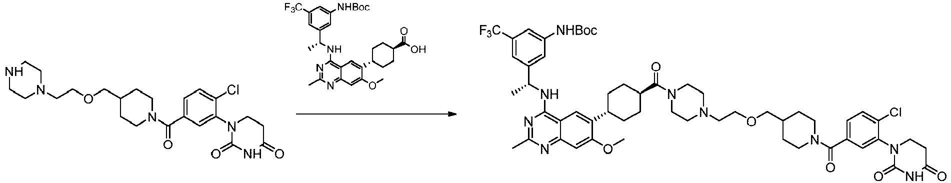

A mixture of 1-(2-chloro-5-(4-((2-(piperazin-1-yl)ethoxy)methyl)piperidine-1-carbonyl)phenyl)dihydropyrimidine-2,4(1*H*,3*H*)-dione (160 mg), (1*R*,4*R*)-4-(4-(((*R*)-1-(3-((*tert-*butoxycarbonyl)amino)-5-(trifhioromethyl)phenyl)ethyl)amino)-7-methoxy-2-methylquinazolin-6-yl)cyclohexane-1-carboxylic acid (150 mg, 0.25 mmol), HATU (143 mg, 0.38 mmol), and DIEA (160 mg, 1.25 mmol) in DMF (5 mL) was stirred at room temperature overnight. Water (50 mL) was added to the reaction liquid, and the resulting mixture was extracted with EA (50 mL × 3). The organic phase was washed with saturated brine (150 mL), dried over anhydrous Na₂SO₄, filtered, and concentrated under reduced pressure. The resulting crude product was purified by flash column chromatography (MeOH:DCM = 0-1:9) to give tert-butyl (3-((R)-1-((6-((1*R*,4*R*)-4-(4-(2-((1-(4-chloro-3-(2,4-dioxotetrahydropyrimidin-1(2*H*)-yl)benzoyl)piperidin-4-yl)methoxy)ethyl)piperazine-1-carbonyl)cyclohexyl)-7-methoxy-2-methylquinazolin-4-yl)amino)ethyl)-5-(trifluoromethyl)phenyl)carbamate.

LC-MS: (ESI, m/z): [M+H]⁺=1062.1.

### Step 7: preparation of 1-(5-(4-((2-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)ethoxy)methyl)piperidine-1-carbonyl)-2-chlorophenyl)dihydropyrimidine-2,4(1H,3H)-dione

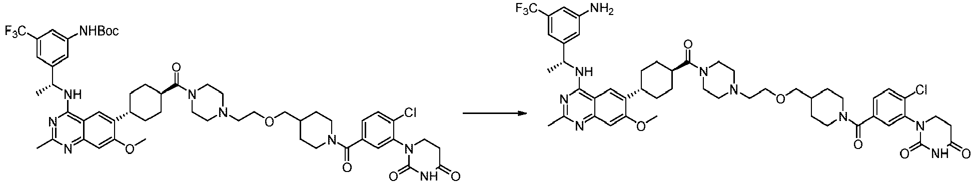

A solution of tert-butyl (3-((*R*)-1-((6-((1*R*,4*R*)-4-(4-(2-((1-(4-chloro-3-(2,4-dioxotetrahydropyrimidin-1(2*H*)-yl)benzoyl)piperidin-4-yl)methoxy)ethyl)piperazine-1-carbonyl)cyclohexyl)-7-methoxy-2-methylquinazolin-4-yl)amino)ethyl)-5-(trifluoromethyl)phenyl)carbamate (100 mg, 0.09 mmol) in TFA/DCM (5 mL, 1:4) was stirred at room temperature for 1 h. The reaction liquid was directly concentrated, and the crude product was purified by Prep-HPLC to give 1-(5-(4-((2-(4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)ethoxy)methyl)piperidine-1-carbonyl)-2-chlorophenyl)dihydropyrimidine-2,4(1*H*,3*H*)-dione.

LC-MS: (ESI, m/z): [M+H]⁺=962.4.

¹H NMR (400 MHz, DMSO-*d*₆) δ 10.49 (s, 1H), 8.20-8.10 (m, 1H), 8.05 (s, 1H), 7.62 (d, *J* = 8.2 Hz, 1H), 7.54 (s, 1H), 7.37 (d, *J* = 8.1 Hz, 1H), 6.99 (s, 1H), 6.86 (d, *J* = 10.0 Hz, 2H), 6.70 (s, 1H), 5.63 - 5.44 (m, 3H), 4.51- 4.38 (m, 1H), 3.89 (s, 3H), 3.80 - 3.70 (m, 1H), 3.68 -3.52 (m, 2H), 3.53-3.40 (m, 6H), 3.27-3.24 (m, 2H), 3.04-2.94 (m, 2H), 2.81-2.61 (m, 5H), 2.45-2.30 (m, 7H), 1.92-1.45 (m, 15H), 1.20-1.01 (m, 2H).

### Example 34: 1-(5-(9-(2-(4-((1S,4S)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)ethyl)-3-azaspiro [5.5]undecane-3 -carbonyl)-2-chlorophenyl)dihydropyrimidine-2,4(1H,3H)-dione

### Step 1: preparation of tert-butyl (R)-4-(4-(4-((1-(3-amino-5-(trifluoromethyl)phenyl)ethyl)amino)-7-methoxy-2-methylquinazolin-6-yl)cyclohexane-1 -carbonyl)piperazine-1 -carboxylate

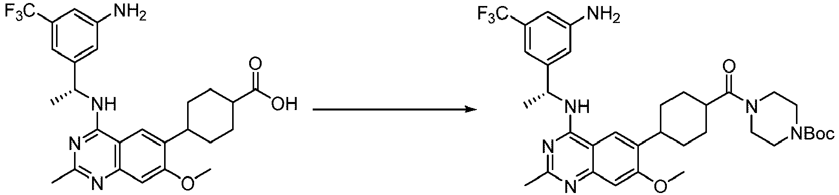

(*R*)-4-(4-((1-(3-Amino-5-(trifluoromethyl)phenyl)ethyl)amino)-7-methoxy-2-methylquinazolin-6-yl)cyclohexane-1-carboxylic acid (220 mg, 0.44 mmol) was dissolved with ethyl acetate (12 mL) and cooled to 0 °C, and N-Boc piperazine (163 mg, 0.88 mmol), DIEA (169 mg, 1.31 mmol), and T₃P (209 mg, 0.66 mmol) were separately added. The reaction liquid was stirred at 0 °C for 3 h. Water (30 mL) was added to quench the reaction, and the resulting mixture was extracted with ethyl acetate (30 mL × 3). The organic phase was washed once with saturated brine (20 mL), dried over anhydrous sodium sulfate, filtered, and concentrated under reduced pressure, and the crude product was purified by column chromatography (PE:EA = 10:1) to give tert-butyl (R)-4-(4-(4-((1-(3-amino-5-(trifluoromethyl)phenyl)ethyl)amino)-7-methoxy-2-methylquinazolin-6-yl)cyclohexane-1-carbonyl)piperazine-1-carboxylate.

LC-MS: (ESI, *m*/*z):* [M+H]⁺= 671.3.

### Step 2: preparation of (R)-(4-(4-((1-(3-amino-5-(trifluoromethyl)phenyl)ethyl)amino)-7-methoxy-2-methylquinazolin-6-yl)cyclohexyl)(piperazin-1-yl)methanone

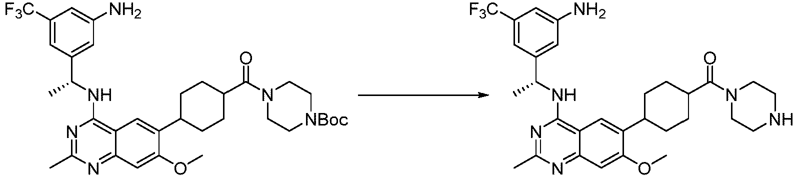

tert-Butyl (R)-4-(4-(4-((1-(3-amino-5-(trifluoromethyl)phenyl)ethyl)amino)-7-methoxy-2-methylquinazolin-6-yl)cyclohexane-1-carbonyl)piperazine-1-carboxylate (220 mg, 0.33 mmol) was added to a solution of hydrogen chloride in ethyl acetate (3 M/EA, 10 mL). The reaction liquid was stirred at room temperature for 2 h and directly concentrated under reduced pressure to give (R)-(4-(4-((1-(3-amino-5-(trifluoromethyl)phenyl)ethyl)amino)-7-methoxy-2-methylquinazolin-6-yl)cyclohexyl)(piperazin-1-yl)methanone, which was directly used in the next step without purification.

### Step 3: preparation of 1-(5-(9-(2-(4-((1S,4S)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)ethyl)-3-azaspiro[5.5]undecane-3-carbonyl)-2-chlorophenyl)dihydropyrimidine-2,4(1H,3H)-dione

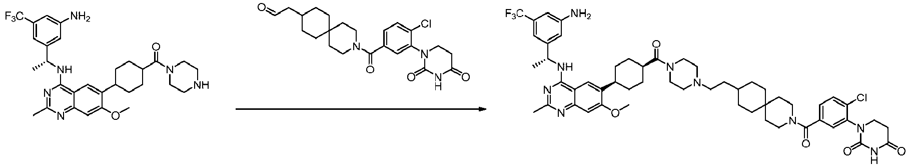

(R)-(4-(4-((1-(3-Amino-5-(trifluoromethyl)phenyl)ethyl)amino)-7-methoxy-2-methylquinazolin-6-yl)cyclohexyl)(piperazin-1-yl)methanone (115 mg, 0.165 mmol) was dissolved with tetrahydrofuran (5 mL), and 2-(3-(4-chloro-3-(2,4-oxotetrahydropyrimidin-1(2H)-yl)benzoyl)-3-azaspiro[5.5]undecan-9-yl)acetaldehyde (39 mg, 0.09 mmol) and sodium triacetoxyborohydride (55 mg, 0.27 mmol) were sequentially added. The reaction liquid was stirred at room temperature for 2 h. Water (10 mL) was added to quench the reaction, and the resulting mixture was extracted with dichloromethane (30 mL × 3), dried over anhydrous sodium sulfate, filtered, and concentrated under reduced pressure. The crude product was purified by preparative reversed-phase chromatography (first with a formic acid system followed by an ammonium bicarbonate system) to give 1-(5-(9-(2-(4-((1*S*,4*S*)-4-(4-(((*R*)-1-(3-amino-5-(trifluoromethyl)phenyl)ethyl)amino)-7-methoxy-2-methylquinazolin-6-yl)cyclohexane-1-carbonyl)piperazin-1 -yl)ethyl)-3 -azaspiro [5.5]undecane-3 -carbonyl)-2-chlorophenyl)dihydropyrimidine-2,4(1*H*,3*H*)-dione.

LC-MS: (ESI, *m*/*z):* [M+H]⁺= 1000.1.

¹H NMR (400 MHz, DMSO-*d*₆) δ 10.50 (s, 1H), 8.31 (d, *J* = 7.9 Hz, 1H), 7.95 (s, 1H), 7.63 (d, *J* = 8.2 Hz, 1H), 7.54 (d, *J* = 1.9 Hz, 1H), 7.38 (dd, *J=* 8.2, 1.8 Hz, 1H), 6.98 (s, 1H), 6.88 (s, 1H), 6.84 (s, 1H), 6.69 (s, 1H), 5.66 - 5.42 (m, 3H), 3.87 (s, 3H), 3.75-3.50 (m, 5H), 3.50-3.40 (m, 5H), 3.00-2.70 (m, 5H), 2.39-2.23 (m, 7H), 2.00-1.84 (m, 4H), 1.75-1.63 (m, 6H), 1.59-1.18 (m, 13H), 1.13-0.96 (m, 4H).

### Example 35: 1-(2-chloro-5-(9-((4-((1R,4R)-4-(4-(((R)-1-(3-(ethylamino)-5-(trifluoromethyl)phenyl)ethyl)amino)-7-methoxy-2-methylquinazolin-6-yl)cyclohexane-1 -carbonyl)piperazin-1 -yl)methyl)-3 -azaspiro [5.5]undecane-3 - carbonyl)phenyl)dihydropyrimidine-2,4(1H,3H)-dione

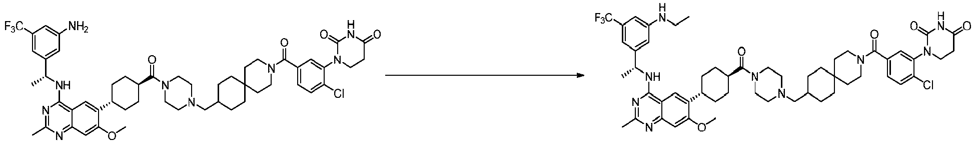

Acetaldehyde (40% aqueous solution, 16 mg, 0.142 mmol) was added to a solution of 1-(5-(9-((4-((1R,4R)-4-(4-(((*R*)-1-(3-amino-5-(trifluoromethyl)phenyl)ethyl)amino)-7-methoxy-2-methylquinazolin-6-yl)cyclohexane-1-carbonyl)piperazin-1-yl)methyl)-3 -azaspiro [5.5]undecane-3 -carbonyl)-2-chlorophenyl)dihydropyrimidine-2,4(1*H*,3*H*)-dione (70 mg, 0.071 mmol) in THF (3 ml). The mixture was stirred at room temperature for 30 min. Sodium triacetoxyborohydride (75 mg, 0.355 mmol) was added, and the resulting mixture was stirred at room temperature overnight. The reaction liquid was diluted with water (15 mL), extracted with DCM (20 mL × 3), dried over anhydrous Na₂SO₄, filtered, and concentrated under reduced pressure, and the resulting crude product was purified by prep-HPLC to give 1-(2-chloro-5-(9-((4-((1R,4R)-4-(4-(((R)-1-(3-(ethylamino)-5-(trifluoromethyl)phenyl)ethyl)amino)-7-methoxy-2-methylquinazolin-6-yl)cyclohexane-1-carbonyl)piperazin-1-yl)methyl)-3-azaspiro[5.5]undecane-3-carbonyl)phenyl)dihydropyrimidine-2,4(1*H*,3*H*)-dione.

LC-MS: (ESI, *m*/*z):* [M+H]⁺=1014.3.

¹H NMR (400 MHz, DMSO-*d*₆) δ 10.52 (s, 1H), 8.13 (s, 1H), 8.07 (s, 1H), 7.64 (d, *J* = 8.3 Hz, 1H), 7.55 (s, 1H), 7.39 (d, *J* = 8.0 Hz, 1H), 7.00 (s, 1H), 6.90 (d, *J* = 6.5 Hz, 2H), 6.64 (s, 1H), 6.05 (s, 1H), 5.60 (t, *J* = 6.4 Hz, 1H), 3.89 (s, 3H), 3.75-3.40 (m, 8H), 3.11 - 3.00 (m, 2H), 3.00-2.90 (m, 1H), 2.87-2.70 (m, 2H), 2.70-2.60 (m, 1H), 2.40-2.25 (m, 7H), 2.18-2.07 (m, 2H), 1.90-1.75 (m, 4H), 1.75-1.20 (m, 18H), 1.18-0.96 (m, 7H).

### Example 36: 1-(5-(4-((2-(1-((1R,4R)-4-(4-(((R)-1-(3-amino-5-(trifluoromethyl)phenyl)ethyl)amino)-2-methylpyrido[3,4-d]pyrimidin-6-yl)cyclohexane-1-carbonyl)piperidin-4-yl)ethoxy)methyl)piperidine-1-carbonyl)-2-methoxyphenyl)dihydropyrimidine-2,4(1H,3H)-dione

### Step 1: preparation of 5-amino-2-chloropyridine-4-carboxamide

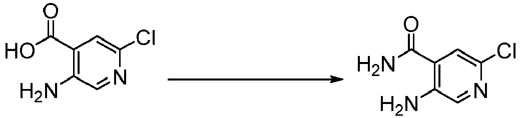

5-Amino-2-chloropyridine-4-carboxylic acid (14.0 g, 81.13 mmol) was dissolved with DCM (200 mL), and HOBt (13.2 g, 97.67 mmol) and EDCI (18.7 g, 97.67 mmol) were added. The reaction liquid was stirred at room temperature for 1 h. NH₃·H₂O (200 mL) was added, and the resulting mixture was stirred for 2 h, filtered, and concentrated under reduced pressure to give the crude product 5-amino-2-chloropyridine-4-carboxamide.

LC-MS: (ESI, *m*/*z):* [M+H]⁺=172.0.

### Step 2: preparation of methyl 4-(5-amino-4-carbamoylpyridin-2-yl)cyclohex-3-ene-1-carboxylate

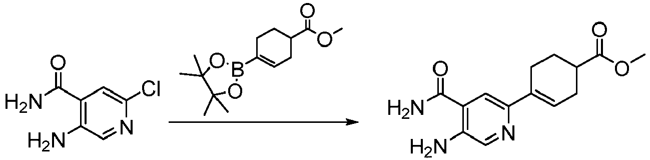

5-Amino-2-chloropyridine-4-carboxamide (5.0 g, 29.24 mmol) was dissolved with dioxane/H₂O (200 mL/20 mL), and Cs₂CO₃ (47.51 g, 146.18 mmol), Pd(dppf)Cl₂ (2.67 g, 3.65 mmol), and methyl 4-(4,4,5,5-tetramethyl-1,3,2-dioxaborolan-2-yl)cyclohex-3-ene-1-carboxylate (10.1 g, 38.1 mmol) were separately added. The reaction liquid was heated to 100 °C under nitrogen atmosphere, stirred overnight, diluted with water (500 mL), extracted with ethyl acetate (600 mL), dried over anhydrous sodium sulfate, and concentrated under reduced pressure, and the crude product was purified by column chromatography (PE:EA = 1:3) to give methyl 4-(5-amino-4-carbamoylpyridin-2-yl)cyclohex-3-ene-1-carboxylate.

LC-MS: (ESI, *m*/*z):* [M+H]⁺=276.0.

### Step 3: preparation of methyl 4-(4-hydroxy-2-methylpyrido[3,4-d]pyrimidin-6-yl)cyclohex-3-ene-1-carboxylate

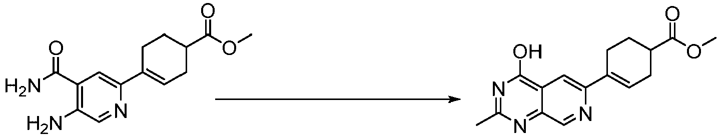

Methyl 4-(5-amino-4-carbamoylpyridin-2-yl)cyclohex-3-ene-1-carboxylate (4.0 g, 14.55 mmol) was added to a solution of trimethyl orthoacetate (17.46 g, 145.5 mmol) in methanol (100 ml). The reaction liquid was stirred at 120 °C overnight in a sealed container, cooled, and concentrated under reduced pressure to give the crude product methyl 4-(4-hydroxy-2-methylpyrido [3,4-*d*]pyrimidin-6-yl)cyclohex-3-ene-1-carboxylate.

LC-MS: (ESI, *m*/*z):* [M+H]⁺=300.0.

### Step 4: preparation of methyl (1R,4R)-4-(4-hydroxy-2-methylpyrido[3,4-d]pyrimidin-6-yl)cyclohexane-1-carboxylate

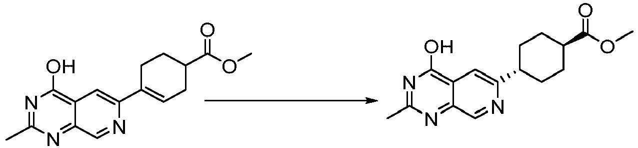

Methyl 4-(4-hydroxy-2-methylpyrido[3,4-*d*]pyrimidin-6-yl)cyclohex-3-ene-1-carboxylate (800 mg, 3.33 mmol) and Pd(OH)₂ (320 mg) were added to a solution of methanol (50 mL). The reaction liquid was purged 3 times with hydrogen, heated to 70 °C, stirred overnight, filtered, and concentrated under reduced pressure. The crude product was dissolved with a solution of methanol (40 mL). Sodium methoxide (1 mL, 5.4 mmol/L) was added, and the mixture was stirred at 70 °C overnight, adjusted to pH 3-4 with H₂SO₄, stirred at 50 °C overnight, concentrated under reduced pressure, and purified by preparative reversed-phase chromatography to give methyl (1*R*,4*R*)-4-(4-hydroxy-2-methylpyrido[3,4-*d*]pyrimidin-6-yl)cyclohexane-1-carboxylate.

LC-MS: (ESI, *m*/*z):* [M+H]⁺=302.1.

### Step 5: preparation of methyl (1R,4R)-4-(2-methyl-4-(((2,4,6-triisopropylphenyl)sulfonyl)oxy)pyrido[3,4-d]pyrimidin-6-yl)cyclohexane-1-carboxylate

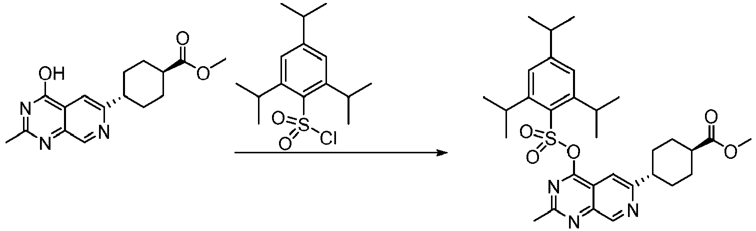

2,4,6-Triisopropylphenylsulfonyl chloride (1.0 g, 3.3 mmol), DMAP (20.3 mg, 0.165 mmol), and TEA (503 mg, 4.98 mmol) were added to a solution of methyl (1*R*,4*R*)-4-(4-hydroxy-2-methylpyrido[3,4-*d*]pyrimidin-6-yl)cyclohexane-1-carboxylate (500 mg, 1.66 mmol) in dichloromethane (30 mL). The reaction liquid was stirred at room temperature overnight. The organic phase was washed with water (50 mL) and saturated brine (40 mL), dried over anhydrous sodium sulfate, filtered, concentrated under reduced pressure, and purified by column chromatography (PE:EA = 1:3) to give methyl (1R,4R)-4-(2-methyl-4-(((2,4,6-triisopropylphenyl)sulfonyl)oxy)pyrido[3,4-*d*]pyrimidin-6-yl)cyclohexane-1-carboxylate.

LC-MS: (ESI, *m*/*z):* [M+H]⁺=568.2.

### Step 6: preparation of methyl (1R,4R)-4-(2-methyl-4-((1-(3-nitro-5-(trifluoromethyl)phenyl)ethyl)amino)pyrido[3,4-d]pyrimidin-6-yl)cyclohexane-1-carboxylate

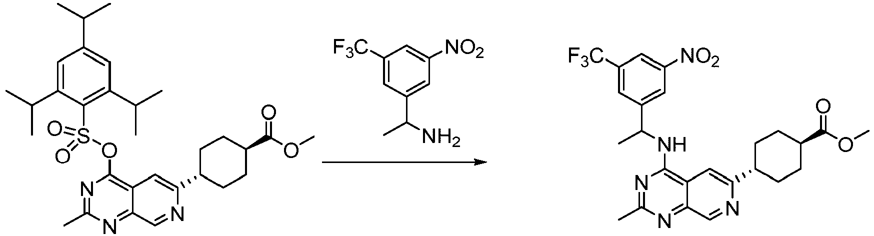

Methyl (1*R*,4*R*)-4-(2-methyl-4-(((2,4,6-triisopropylphenyl)sulfonyl)oxy)pyrido[3,4-*d*]pyrimidin-6-yl)cyclohexane-1-carboxylate (800 mg, 1.41 mmol) and TEA (546.1 mg, 4.23 mmol) were added to a solution of 1-(3-nitro-5-(trifluoromethyl)phenyl)ethyl-1-amine (462 mg, 1.97 mmol) in DMSO (20 mL). The reaction liquid was stirred at room temperature overnight. The crude product was purified by reversed-phase column chromatography (ACN/H₂O = 2/1) to give methyl (1R,4R)-4-(2-methyl-4-((1-(3-nitro-5-(trifluoromethyl)phenyl)ethyl)amino)pyrido[3,4-*d*]pyrimidin-6-yl)cyclohexane-1-carboxylate.

LC-MS: (ESI, m/z): [M+H]⁺=518.4.

### Step 7: preparation of (1R,4R)-4-(2-methyl-4-((1-(3-nitro-5-(trifluoromethyl)phenyl)ethyl)amino)pyrido[3,4-d]pyrimidin-6-yl)cyclohexane-1-carboxylic acid

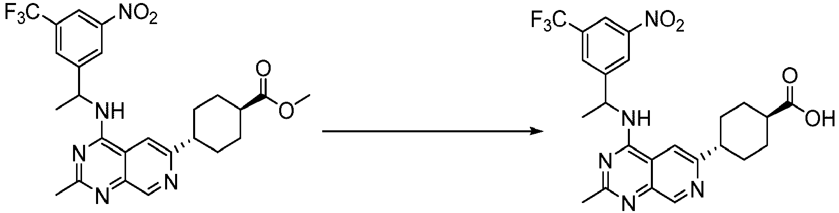

Methyl (1*R*,4*R*)-4-(2-methyl-4-((1-(3 -nitro-5-(trifluoromethyl)phenyl)ethyl)amino)pyrido [3,4-*d*]pyrimidin-6-yl)cyclohexane-1-carboxylate (400 mg, 0.774 mmol) was dissolved in a solution of THF/H₂O (40 mL/10 mL), and LiOH (93 mg, 3.87 mmol) was added. The reaction liquid was heated to 50 °C and stirred overnight. HCl (1 M) was added to adjust the pH to neutrality, and the resulting mixture was dissolved with water (50 mL), extracted with ethyl acetate (100 mL), and concentrated under reduced pressure to give the crude product (1*R*,4*R*)-4-(2-methyl-4-((1-(3-nitro-5-(trifluoromethyl)phenyl)ethyl)amino)pyrido[3,4-*d*]pyrimidin-6-yl)cyclohexane-1-carboxylic acid.

LC-MS: (ESI, *m*/*z*): [M+H]⁺=504.2.

### Step 8: preparation of 1-(2-methoxy-5-(4-((2-(1-((1R,4R)-4-(2-methyl-4-(((R)-1-(3-nitro-5-(trifluoromethyl)phenyl)ethyl)amino)pyrido[3,4-d]pyrimidin-6-yl)cyclohexane-1-carbonyl)piperidin-4-yl)ethoxy)methyl)piperidine-1-carbonyl)phenyl)dihydropyrimidine-2,4(1H,3H)-dione

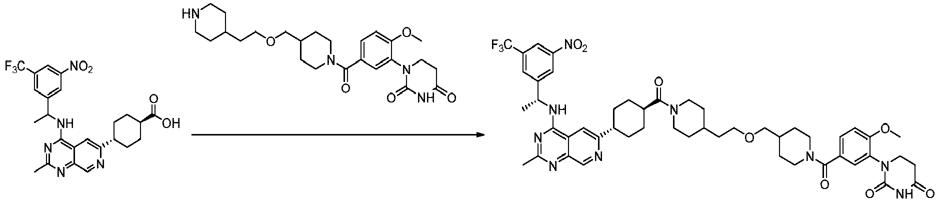

(1*R*,4*R*)-4-(2-Methyl-4-((1-(3-nitro-5-(trifluoromethyl)phenyl)ethyl)amino)pyrido[3,4-*d*]pyrimidin-6-yl)cyclohexane-1-carboxylic acid (180 mg, 0.357 mmol), HATU (203.5 mg, 0.536 mmol), and DIEA (138.2 mg, 1.07 mmol) were added to a solution of 1-(2-methoxy-5-(4-((2-(piperidin-4-yl)ethoxy)methyl)piperidine-1-carbonyl)phenyl)dihydropyrimidine-2,4(1*H*,3*H*)-dione in DMF (6 mL). The reaction liquid was stirred at room temperature overnight, dissolved with water (50 mL), extracted with dichloromethane (30 mL × 3), and concentrated under reduced pressure. The crude product was purified by reversed-phase column chromatography (ACN/H₂O = 1/2) to give 1-(2-methoxy-5-(4-((2-(1-((1R,4R)-4-(2-methyl-4-(((R)-1-(3-nitro-5-(trifluoromethyl)phenyl)ethyl)amino)pyrido [3,4-*d*]pyrimidin-6-yl)cyclohexane-1-carbonyl)piperidin-4-yl)ethoxy)methyl)piperidine-1-carbonyl)phenyl)dihydropyrimidine-2,4(1H,3H)-dione.

LC-MS: (ESI, m/z): [M+H]⁺= 958.5.

### Step 9: preparation of 1-(5-(4-((2-(1-((1R,4R)-4-(4-(((R)-1-(3-amino-5-(trifluoromethyl)phenyl)ethyl)amino)-2-methylpyrido[3,4-d]pyrimidin-6-yl)cyclohexane-1-carbonyl)piperidin-4-yl)ethoxy)methyl)piperidine-1-carbonyl)-2-methoxyphenyl)dihydropyrimidine-2,4(1H,3H)-dione

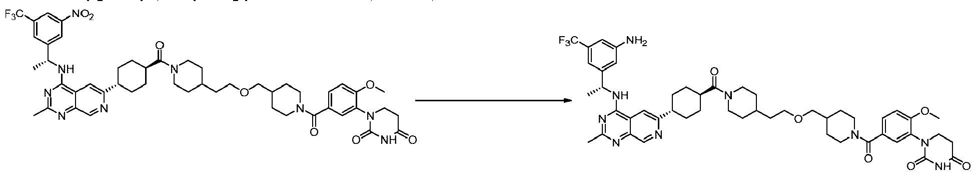

1-(2-Methoxy-5-(4-((2-(1-((1R,4R)-4-(2-methyl-4-(((R)-1-(3-nitro-5-(trifluoromethyl)phenyl)ethyl)amino)pyrido [3,4-*d*]pyrimidin-6-yl)cyclohexane-1-carbonyl)piperidin-4-yl)ethoxy)methyl)piperidine-1-carbonyl)phenyl)dihydropyrimidine-2,4(1*H*,3*H*)-dione (300 mg, 0.313 mmol) was dissolved in ethanol (20 mL), and Raney-Ni (aq., 1 mL) and hydrazine hydrate (0.5 mL) were added. The reaction liquid was stirred at room temperature for 1 h, filtered, and concentrated under reduced pressure. The crude product was purified by preparative reversed-phase chromatography to give 1-(5-(4-((2-(1-((1R,4R)-4-(4-(((R)-1-(3-amino-5 -(trifluoromethyl)phenyl)ethyl)amino)-2-methylpyrido [3,4-d*]*pyrimidin-6-yl)cyclohexane-1-carbonyl)piperidin-4-yl)ethoxy)methyl)piperidine-1-carbonyl)-2-methoxyphenyl)dihydropyrimidine-2,4(1*H*,3*H*)-dione.

LC-MS: (ESI, m/z): [M+H]⁺=928.5.

¹H NMR (400 MHz, DMSO-*d*₆) δ 10.33 (s, 1H), 8.91 (s, 1H), 8.58 (d, *J* = 7.9 Hz, 1H), 8.10 (s, 1H), 7.36 (dd, *J* = 8.4, 1.9 Hz, 1H), 7.32 (d, *J* = 1.9 Hz, 1H), 7.15 (d, *J* = 8.5 Hz, 1H), 6.89 (s, 1H), 6.85 (s, 1H), 6.71 (s, 1H), 5.57 - 5.51 (m, 3H), 4.45-4.33 (m, 1H), 4.00-3.90 (m, 1H), 3.84 (s, 3H), 3.59 (t, *J* = 6.6 Hz, 2H), 3.41 (t, *J* = 6.2 Hz, 2H), 3.24 (d, *J* = 6.2 Hz, 2H), 3.05-2.95 (m, 2H), 2.83-2.70 (m, 2H), 2.70-2.65 (m, 3H), 2.43 (s, 3H), 2.05-1.96 (m, 2H), 1.88-1.40 (m, 20H), 1.23-0.89 (m, 4H).

### Example 37: 1-(5-(2-(4-((4-((1R,4R)-4-(4-(((R)-1-(3-amino-5-(trifluoromethyl)phenyl)ethyl)amino)-7-methoxy-2-methylquinazolin-6-yl)cyclohexane-1 -carbonyl)piperazin-1 -yl)methyl)piperidin-1 -yl)-2-oxoethoxy)-2-chlorophenyl)dihydropyrimidine-2,4(1H,3H)-dione

### Step 1: preparation of tert-butyl 4-(piperidin-4-ylmethyl)piperazine-1-carboxylate

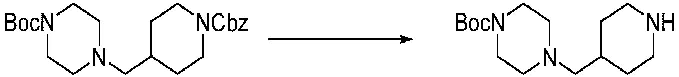

A mixture of tert-butyl 4-((1-((benzyloxy)carbonyl)piperidin-4-yl)methyl)piperazine-1-carboxylate (500 mg, 1.20 mmol) and Pd/C (100 mg) in MeOH (10 mL) was stirred at room temperature for 2.0 h under H₂ atmosphere. The reaction liquid was filtered and concentrated under reduced pressure to give tert-butyl 4-(piperidin-4-ylmethyl)piperazine-1-carboxylate.

LC-MS: (ESI, *m*/*z*): [M+H]⁺=284.1.

### Step 2: preparation of tert-butyl 4-((1-(2-(4-chloro-3-(2,4-dioxotetrahydropyrimidin-1(2H)-yl)phenoxy)acetyl)piperidin-4-yl)methyl)piperazine-1-carboxylate

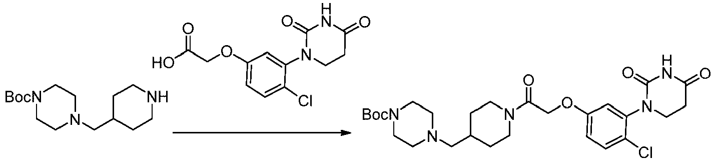

A mixture of 2-(4-chloro-3-(2,4-dioxotetrahydropyrimidin-1(2*H*)-yl)phenoxy)acetic acid (240 mg, 0.805 mmol), tert-butyl 4-(piperidin-4-ylmethyl)piperazine-1-carboxylate (251 mg, 0.886 mmol), HATU (612 mg, 1.61 mmol), and DIEA (312 mg, 2.42 mmol) in DMF (5 mL) was stirred at room temperature for 3 h. Water (20 mL) was added to quench the reaction, and the resulting mixture was extracted with DCM (20 mL × 2). The organic phase was isolated, dried over anhydrous Na₂SO₄, filtered, and concentrated under reduced pressure. The crude product was purified by column chromatography (MeOH:DCM = 0-1:24) to give tert-butyl 4-((1-(2-(4-chloro-3-(2,4-dioxotetrahydropyrimidin-1(2*H*)-yl)phenoxy)acetyl)piperidin-4-yl)methyl)piperazine-1-carboxylate.

LC-MS: (ESI, *m*/*z):* [M+H]⁺= 564.2.

### Step 3: preparation of 1-(2-chloro-5-(2-oxo-2-(4-(piperazin-1-ylmethyl)piperidin-1-yl)ethoxy)phenyl)dihydropyrimidine-2,4(1H,3H)-dione

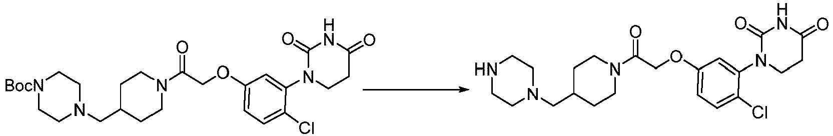

A mixture of tert-butyl 4-((1-(2-(4-chloro-3-(2,4-dioxotetrahydropyrimidin-1(2*H*)-yl)phenoxy)acetyl)piperidin-4-yl)methyl)piperazine-1-carboxylate (350 mg, 0.622 mmol) in TFA/DCM (10 mL/3 mL) was stirred at room temperature for 2 h. The reaction liquid was directly concentrated to give 1-(2-chloro-5-(2-oxo-2-(4-(piperazine-1-ylmethyl)piperidine-1-yl)ethoxy)phenyl)dihydropyrimidine-2,4(1*H*,3*H*)-dione.

LC-MS: (ESI, *m*/*z):* [M+H]⁺= 464.2.

### Step 4: preparation of tert-butyl (3-((R)-1-((6-((1R,4R)-4-(4-((1-(2-(4-chloro-3-(2,4-dioxotetrahydropyrimidin-1(2H)-yl)phenoxy)acetyl)piperidin-4-yl)methyl)piperazine-1-carbonyl)cyclohexyl)-7-methoxy-2-methylquinazolin-4-yl)amino)ethyl)-5-(trifluoromethyl)phenyl)carboxylate

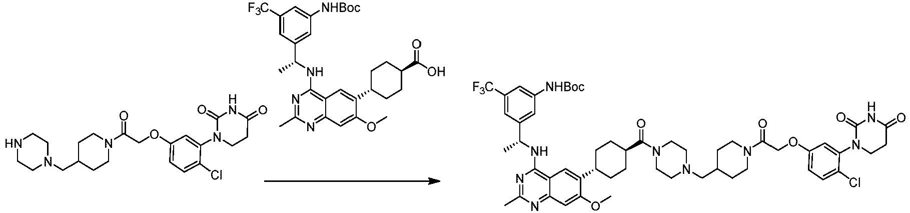

(1*R*,4*R*)-4-(4-(((*R*)-1-(3-((*tert*-Butoxycarbonyl)amino)-5-(trifluoromethyl)phenyl)ethyl)amino)-7-methoxy-2-methylquinazolin-6-yl)cyclohexane-1-carboxylic acid (405.6 mg, 0.674 mmol) was added to a solution of 1-(2-chloro-5-(2-oxo-2-(4-(piperazin-1-ylmethyl)piperidin-1-yl)ethoxy)phenyl)dihydropyrimidine-2,4(1*H*,3*H*-dione (260 mg, 0.562 mmol), HATU (426 mg, 1.12 mmol), and DIEA (217 mg, 3.36 mmol) in DMF (3 mL). Then the reaction liquid was stirred at room temperature for 1 h. Water (20 mL) was added to the reaction liquid to quench the reaction, and the resulting mixture was extracted with DCM (20 mL × 2). The organic phase was dried over anhydrous Na₂SO₄, filtered, and concentrated under reduced pressure. The crude product was purified by column chromatography (MeOH:DCM = 0-2:23) to give tert-butyl (3-((R)-1-((6-((1R,4R)-4-(4-((1-(2-(4-chloro-3-(2,4-dioxotetrahydropyrimidin-1(2*H*)-yl)phenoxy)acetyl)piperidin-4-yl)methyl)piperazine-1-carbonyl)cyclohexyl)-7-methoxy-2-methylquinazolin-4-yl)amino)ethyl)-5-(trifluoromethyl)phenyl)carboxylate.

LC-MS: (ESI, *m*/*z*): [M+H]⁺=1048.6.

### Step 5: preparation of 1-(5-(2-(4-((4-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperazin-1-yl)methyl)piperidin-1-yl)-2-oxoethoxy)-2-chlorophenyl)dihydropyrimidine-2,4(1H,3H)-dione

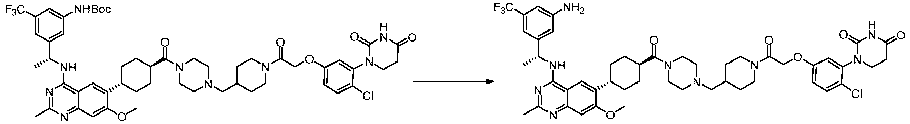

A solution of tert-butyl (3-((*R*)-1-((6-((1*R*,4*R*)-4-(4-((1-(2-(4-chloro-3-(2,4-dioxotetrahydropyrimidin-1(2*H*)-yl)phenoxy)acetyl)piperidin-4-yl)methyl)piperazine-1-carbonyl)cyclohexyl)-7-methoxy-2-methylquinazolin-4-yl)amino)ethyl)-5-(trifluoromethyl)phenyl)carboxylate (140 mg, 0.13 mmol) in DCM/TFA (3 mL/1 mL) was stirred at room temperature for 2 h. The reaction liquid was directly concentrated under reduced pressure. The resulting crude product was purified by Prep-HPLC (acetonitrile/0.05% aqueous NH₄HCO₃ solution, 5%-95%) to give 1-(5-(2-(4-((4-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperazin-1-yl)methyl)piperidin-1-yl)-2-oxoethoxy)-2-chlorophenyl)dihydropyrimidine-2,4(1*H*,3*H*)-dione.

LC-MS: (ESI, *m*/*z*): [M+H]⁺=948.4.

¹H NMR (400 MHz, DMSO-*d*₆) δ 10.47 (s, 1H), 8.24-7.87 (m, 2H), 7.45 (d, *J* = 8.9 Hz, 1H), 7.11 (d, *J* = 2.9 Hz, 1H), 6.99 (s, 1H), 6.93 (dd, *J* = 9.0, 3.0 Hz, 1H), 6.86 (d, *J* = 10.9 Hz, 2H), 6.70 (s, 1H), 5.67-5.40 (m, 3H), 4.93 - 4.63 (m, 2H), 4.40-4.24 (m, 1H), 3.89 (s, 3H), 3.82-3.65 (m, 2H), 3.63-3.42 (m, 4H), 3.09-2.89 (m, 2H), 2.77-2.55 (m, 5H), 2.41-2.25 (m, 7H), 2.12 (d, *J* = 6.5 Hz, 2H), 1.9-1.48 (m, 14H), 1.15-0.88 (m, 2H).

### Example 38: 1-(5-((4-((3-(1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)propyl)(methyl)amino)piperidin-1-yl)methyl)-2-chlorophenyl)dihydropyrimidine-2,4(1H,3H)-dione

### Step 1: preparation of 1-(2-chloro-5-(hydroxymethyl)phenyl)dihydropyrimidine-2,4(1H,3H)-dione

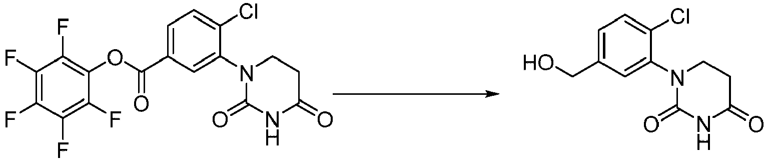

Pentafluorophenyl 4-chloro-3-(2,4-dioxotetrahydropyrimidin-1(2*H*)-yl)benzoate (2.0 g, 4.61 mmol) was dissolved in anhydrous tetrahydrofuran (40 mL) and cooled to -60 °C under nitrogen atmosphere, and a solution of lithium aluminum hydride (9.22 mL, 1.0 mmol/L) in tetrahydrofuran was added dropwise to the above solution while stirring. After the addition, the reaction liquid was stirred at -60 °C for 1 h. A saturated aqueous ammonium chloride solution (20 mL) was added dropwise to the reaction liquid, and the resulting mixture was filtered. The mother liquor was extracted with ethyl acetate (30 mL × 3). The organic phase was washed with saturated brine (100 mL), dried over anhydrous sodium sulfate, filtered, and concentrated under reduced pressure. The crude product was purified by column chromatography (methanol:dichloromethane = 1:9) to give 1-(2-chloro-5-(hydroxymethyl)phenyl)dihydropyrimidine-2,4(*1*H,3*H*)-dione.

LC-MS: (ESI, *m*/*z*): [M+H]⁺=255.0

### Step 2: preparation of 4-chloro-3-(2,4-dioxotetrahydropyrimidin-1(2H)-yl)benzaldehyde

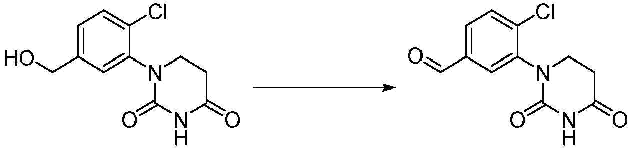

1-(2-Chloro-5-(hydroxymethyl)phenyl)dihydropyrimidine-2,4(1*H*,3*H*)-dione (450 mg, 1.76 mmol) was dissolved with acetonitrile (20 mL), and IBX (988 mg, 3.53 mmol) was added. The reaction liquid was heated to 80 °C and stirred for 1 h. The reaction liquid was filtered, the filter cake was washed with methanol (20 ml × 3), and the filtrate was concentrated under reduced pressure. The crude product was purified by column chromatography (methanol:dichloromethane = 1:9) to give 4-chloro-3-(2,4-dioxotetrahydropyrimidin-1(2*H*)-yl)benzaldehyde. LC-MS: (ESI, *m*/*z*): [M+H]⁺=253.0.

¹H NMR (400 MHz, DMSO-*d*₆) δ 10.57 (s, 1H), 10.01 (s, 1H), 8.06 (d, *J* = 1.8 Hz, 1H), 7.93 (dd, *J* = 8.2, 1.9 Hz, 1H), 7.84 (d, *J* = 8.2 Hz, 1H), 3.80 (dd, J= 16.6, 9.3 Hz, 1H), 3.64 (dt, *J* = 12.1, 6.1 Hz, 1H), 2.77 (dd, *J* = 12.5, 6.4 Hz, 2H).

### Step 3: preparation of tert-butyl 4-(3-oxopropyl)piperidine-1-carboxylate

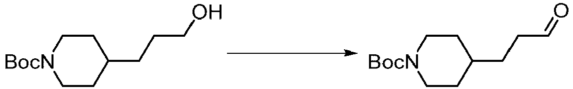

*tert*-Butyl 4-(3-hydroxypropyl)piperidine-1-carboxylate (500 mg, 2.05 mmol) was dissolved in acetonitrile (20 mL), and IBX (1.15 g, 4.11 mmol) was added. The reaction liquid was heated to 70 °C and stirred for 1 h. The reaction liquid was filtered, the filter cake was washed with methanol (20 mL × 3), and the filtrate was concentrated under reduced pressure. The crude product was purified by column chromatography (ethyl acetate:petroleum ether = 1:1) to give tert-butyl 4-(3-oxopropyl)piperidine-1-carboxylate.

¹H NMR (400 MHz, DMSO-*d*₆) δ 9.68 (t, *J* = 1.5 Hz, 1H), 3.91 (d, *J* = 12.0 Hz, 2H), 2.65 (m, 2H), 2.46 (td, *J* = 7.5, 1.5 Hz, 2H), 1.61 (d, *J* = 13.0 Hz, 2H), 1.46 (2H), 1.42 - 1.33 (m, 10H), 0.94 (2H).

### Step 4: preparation of benzyl 4-((3-(1-(tert-butoxycarbonyl)piperidin-4-yl)propyl)(methyl)amino)piperidine-1-carboxylate

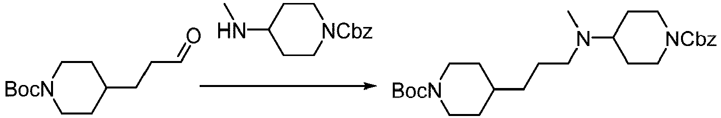

tert-Butyl 4-(3-oxopropyl)piperidine-1-carboxylate (300 mg, 1.2 mmol) and benzyl 4-(methylamino)piperidine-1-carboxylate (308 mg, 1.2 mmol) were dissolved with tetrahydrofuran (20 mL), and sodium triacetoxyborohydride (1.2 g, 6.0 mmol) was added while stirring. The reaction liquid was stirred at room temperature for 2 h. Water (50 mL) was added, and the resulting mixture was extracted with dichloromethane (30 mL × 3). The organic phase was washed with saturated brine (100 mL), dried over anhydrous sodium sulfate, filtered, and concentrated under reduced pressure. The crude product was purified by column chromatography (methanol:dichloromethane = 1:9) to give benzyl 4-((3-(1-(*tert*-butoxycarbonyl)piperidin-4-yl)propyl)(methyl)amino)piperidine-1-carboxylate.

LC-MS: (ESI, *m*/*z):* [M+H]⁺=474.3.

### Step 5: preparation of tert-butyl 4-(3-(methyl(piperidin-4-yl)amino)propyl)piperidine-1-carboxylate

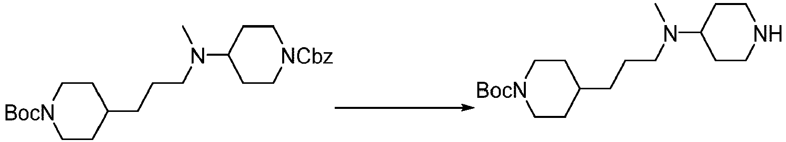

Benzyl 4-((3-(1-(*tert*-butoxycarbonyl)piperidin-4-yl)propyl)(methyl)amino)piperidine-1-carboxylate (400 mg, 0.84 mmol) was dissolved in EA (20 mL), and Pd(OH)₂/C (200 mg, 0.28 mmol) was added. The reaction liquid was heated to 70 °C under hydrogen atmosphere and stirred overnight. The reaction liquid was cooled to room temperature and filtered, and the filtrate was concentrated under reduced pressure to give tert-butyl 4-(3-(methyl(piperidin-4-yl)amino)propyl)piperidine-1-carboxylate. The crude product was directly used in the next step without purification.

LC-MS: (ESI, m/z): [M+H]⁺=340.3.

### Step 6: preparation of tert-butyl 4-(3-((1-(4-chloro-3-(2,4-dioxotetrahydropyrimidin-1(2H)-yl)phenyl)piperidin-4-yl)(methyl)amino)propyl)piperidine-1-carboxylate

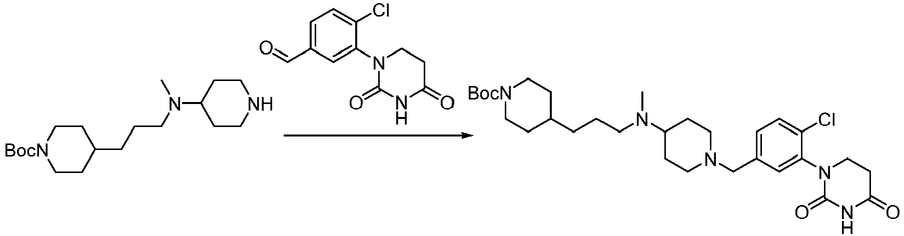

tert-Butyl 4-(3-(methyl(piperidin-4-yl)amino)propyl)piperidine-1-carboxylate (280 mg, 0.82 mmol) and 4-chloro-3-(2,4-dioxotetrahydropyrimidin-1(2*H*)-yl)benzaldehyde (311 mg, 1.23 mmol) were dissolved with tetrahydrofuran (20 mL), and sodium triacetoxyborohydride (869 mg, 4.1 mmol) was added. The reaction liquid was heated to 50 °C and stirred for 2 h, diluted with water (50 mL), and extracted with dichloromethane (30 mL × 3). The organic phase was washed with saturated brine (100 mL), dried over anhydrous sodium sulfate, filtered, and concentrated under reduced pressure. The crude product was purified by column chromatography (methanol:dichloromethane = 1:9) to give tert-butyl 4-(3-((1-(4-chloro-3-(2,4-dioxotetrahydropyrimidin-1(2*H*)-yl)phenyl)piperidin-4-yl)(methyl)amino)propyl)piperidine-1-carboxylate.

LC-MS: (ESI, *m*/*z*): [M+H]⁺=576.2.

### Step 7: preparation of 1-(2-chloro-5-((4-(methyl(3-(piperidin-4-yl)propyl)amino)-1-yl)methyl)phenyl)dihydropyrimidine-2,4(1H,3H)-dione

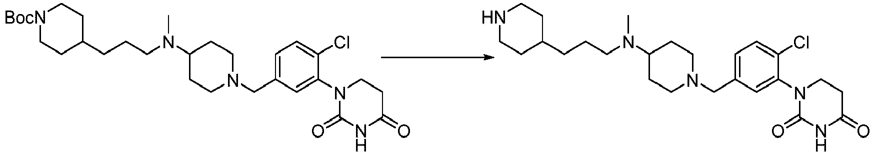

tert-Butyl 4-(3-((1-(4-chloro-3 -(2,4-dioxotetrahydropyrimidin-1(2*H*)-yl)phenyl)piperidin-4-yl)(methyl)amino)propyl)piperidine-1-carboxylate (180 mg, 0.31 mmol) was dissolved with dichloromethane (5 mL), and trifluoroacetic acid (2 mL) was added. The reaction liquid was stirred at room temperature for 2 h and directly concentrated under reduced pressure to give 1-(2-chloro-5-((4-(methyl(3-(piperidin-4-yl)propyl)amino)-1-yl)methyl)phenyl)dihydropyrimidine-2,4(1*H*,3*H*)-dione, which was directly used in the next step without purification.

LC-MS: (ESI, *m*/*z):* [M+H]⁺=476.3.

### Step 8: preparation of tert-butyl (3-((R)-1-((6-((1R,4R)-4-(4-(3-((1-(4-chloro-3-(2,4-dioxotetrahydropyrimidin-1(2H)-yl)phenyl)piperidin-4-yl)(methyl)amino)propyl)piperidine-1-carbonyl)cyclohexyl)-7-methoxy-2-methylquinazolin-4-yl)amino)ethyl)-5-(trifluoromethyl)phenyl)carboxylate

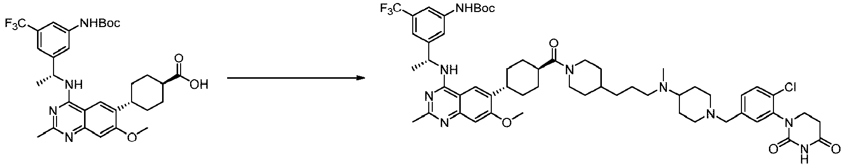

1-(2-Chloro-5-((4-(methyl(3-(piperidin-4-yl)propyl)amino)-1-yl)methyl)phenyl)dihydropyrimidine-2,4(1*H*,3*H*)-dione (180 mg, 0.31 mmol) was dissolved in DMF (10 mL), and (1*R*,4*R*)-4-(4-(((*R*)-1-(3-(*tert-*butoxycarbonyl)amino)-7-methoxy-2-methylquinazolin-6-yl)cyclohexane-1-carboxylic acid (150 mg, 0.24 mmol), HATU (118 mg, 0.31 mmol), and *N*,*N*-diisopropylethylamine (93 mg, 0.72 mmol) were added. The reaction liquid was stirred at room temperature for 2 h. Water (100 mL) was added, and the resulting mixture was extracted with ethyl acetate (30 mL × 3). The organic phase was washed with saturated brine (100 mL), dried over anhydrous sodium sulfate, filtered, and concentrated under reduced pressure. The crude product was purified by column chromatography (methanol:dichloromethane = 1:9) to give tert-butyl (3-((R)-1-((6-((1R,4R)-4-(4-(3-((1-(4-chloro-3-(2,4-dioxotetrahydropyrimidin-1(2*H*)-yl)phenyl)piperidin-4-yl)(methyl)amino)propyl)piperidine-1-carbonyl)cyclohexyl)-7-methoxy-2-methylquinazolin-4-yl)amino)ethyl)-5-(trifluoromethyl)phenyl)carboxylate. LC-MS: (ESI, m/z): [M+H]⁺=1060.5.

### Step 9: preparation of 1-(5-((4-((3-(1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)propyl)(methyl)amino)piperidin-1-yl)methyl)-2-chlorophenyl)dihydropyrimidine-2,4(1H,3H)-dione

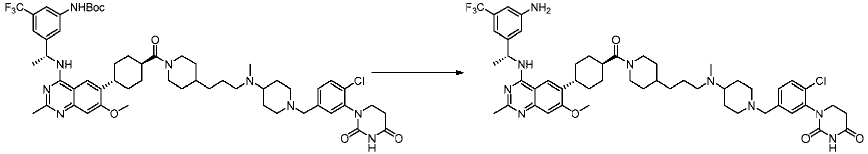

*tert*-Butyl (3-((*R*)-1-((6-((1*R*,4*R*)-4-(4-(3-((1-(4-chloro-3-(2,4-dioxotetrahydropyrimidin-1(2*H*)-yl)phenyl)piperidin-4-yl)(methyl)amino)propyl)piperidine-1-carbonyl)cyclohexyl)-7-methoxy-2-methylquinazolin-4-yl)amino)ethyl)-5-(trifluoromethyl)phenyl)carboxylate (200 mg, 0.19 mmol) was dissolved with dichloromethane (5 mL), and trifluoroacetic acid (2 mL) was added. The reaction liquid was stirred at room temperature for 2 h and concentrated under reduced pressure. The resulting oil was dissolved with a mixed solution (20 mL) of 10% methanol/dichloromethane. 5% aqueous sodium bicarbonate solution (20 mL) was added, and the resulting mixture was stirred for 1 h. The resulting organic phase was isolated, dried over anhydrous sodium sulfate, filtered, and concentrated under reduced pressure to give the crude product, which was purified by Pre-HPLC (CH₃CN/0.08% aqueous NH₄HCO₃ solution, 5%-95%) to give 1-(5-((4-((3-(1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)propyl)(methyl)amino)piperidin-1-yl)methyl)-2-chlorophenyl)dihydropyrimidine-2,4(1*H*,3*H*)-dione.

LC-MS: (ESI, m/z): [M+H]⁺=960.5.

¹H NMR (400 MHz, DMSO-*d*₆) δ 10.45 (s, 1H), 8.16 - 8.02 (m, 2H), 7.50 (d, J = 8.2 Hz, 1H), 7.38 (s, 1H), 7.29 (d, J = 8.3 Hz, 1H), 6.99 (s, 1H), 6.88 (s, 1H), 6.85 (s, 1H), 6.70 (s, 1H), 5.64 - 5.47 (m, 3H), 4.45-4.35 (m, 1H), 3.96-3.85 (m, 4H), 3.76 - 3.54 (m, 2H), 3.47-3.40 (m, 2H), 3.08-2.90 (m, 2H), 2.89-2.77 (m, 2H), 2.77-2.60 (m, 3H), 2.35 (s, 6H), 2.15 (s, 3H), 1.98-1.40 (m, 23H), 1.24-1.18 (m, 2H), 1.08-0.82 (m, 2H).

### Example 39: 5-(4-((2-(1-((1S,4S-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)ethoxy)methyl)piperidine-1 -carbonyl)-2-(2,6-dioxopiperidin-3 -yl)isoindoline-1,3 -dione

### Step 1: preparation of 2-(2,6-dioxopiperidin-3-yl)-1,3-dioxoisoindoline-5-carboxylic acid

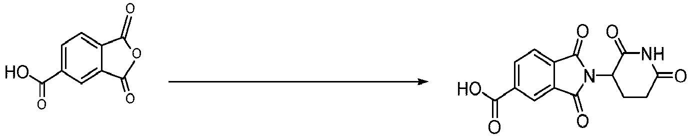

1,3-Dioxo-1,3-dihydroisobenzofuran-5-carboxylic acid (1.0 g, 5.21 mmol) was dissolved with AcOH (10 mL), and potassium acetate (945 mg, 5.73 mmol) and 3-aminopiperidine-2,6-dione hydrochloride (945 mg, 5.73 mmol) were added. The reaction liquid was heated to 90 °C and stirred overnight, and concentrated under reduced pressure. Water (20 mL) was added to the crude product, and the resulting mixture was stirred for 10 min and filtered. The filter cake was washed with water (10 mL × 3) and dried under vacuum to give the crude product 2-(2,6-dioxopiperidin-3 -yl)-1,3 -dioxoisoindoline-5 -carboxylic acid.

LC-MS: (ESI, *m*/*z):* [M+H] ⁺=303.0.

### Step 2: preparation of pentafluorophenyl 2-(2,6-dioxopiperidin-3-yl)-1,3-dioxoisoindoline-5-carboxylate

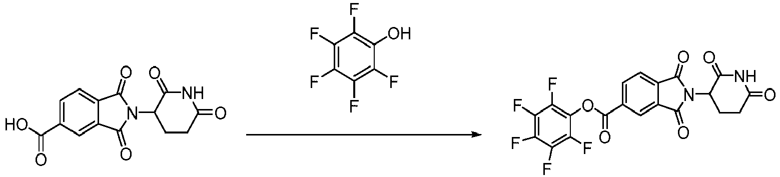

2-(2,6-Dioxopiperidin-3-yl)-1,3-dioxoisoindoline-5-carboxylic acid (1.4 g, 4.64 mmol) was dissolved with DMF (20 mL), and DCC (1.43 g, 6.96 mmol) and 2,3,4,5,6-pentafluorophenol (1.30 g, 6.96 mmol) were added. The reaction liquid was stirred at room temperature overnight, diluted with water (150 mL), extracted with ethyl acetate (100 mL × 3), dried over anhydrous sodium sulfate, and concentrated under reduced pressure. The crude product was purified by column chromatography (PE:EA = 1:1) to give pentafluorophenyl 2-(2,6-dioxopiperidin-3-yl)-1,3-dioxoisoindoline-5 -carboxylate.

¹H NMR (400 MHz, DMSO-*d*₆) δ 11.16 (s, 1H), 8.41 (dd, *J* = 7.8, 1.3 Hz, 1H), 8.28 (s, 1H), 8.06 (d, *J* = 7.8 Hz, 1H), 5.20 (dd, *J* = 12.8, 5.4 Hz, 1H), 2.66 - 2.47 (m, 5H).

### Step 3: preparation of tert-butyl 4-(2-((1-(2-(2,6-dioxopiperidin-3-yl)-1,3-dioxoisoindoline-5-carbonyl)piperidin-4-yl)methoxy)ethyl)piperidine-1 -carboxylate

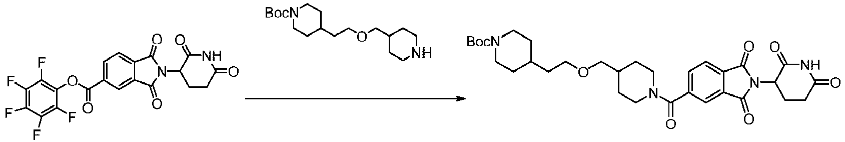

Pentafluorophenyl 2-(2,6-dioxopiperidin-3-yl)-1,3-dioxoisoindoline-5-carboxylate (480 mg, 1.02 mmol) was added to a solution of tert-butyl 4-(2-(piperidin-4-ylmethoxy)ethyl)piperidine-1-carboxylate (400 mg, 1.22 mmol) in DMSO (10 mL). The reaction liquid was stirred at room temperature overnight, diluted with water (50 mL), extracted with ethyl acetate (30 mL × 3), dried over anhydrous sodium sulfate, and concentrated under reduced pressure. The crude product was purified by column chromatography (PE:EA = 1:10) to give tert-butyl 4-(2-((1-(2-(2,6-dioxopiperidin-3 -yl)-1,3-dioxoisoindoline-5-carbonyl)piperidin-4-yl)methoxy)ethyl)piperidine-1-carboxylate.

LC-MS: (ESI, *m*/*z*): [M+H]⁺=611.3.

### Step 4: preparation of 2-(2,6-dioxopiperidin-3-yl)-5-(4-((2-(piperidin-4-yl)ethoxy)methyl)piperidine-1-carbonyl)isoindoline-1,3-dione

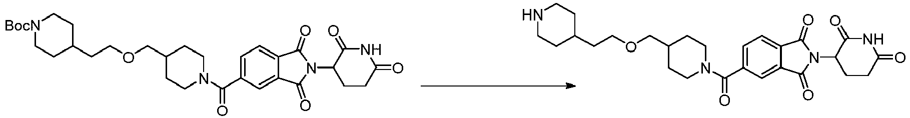

tert-Butyl 4-(2-((1-(2-(2,6-dioxopiperidin-3-yl)-1,3-dioxoisoindoline-5-carbonyl)piperidin-4-yl)methoxy)ethyl)piperidine-1-carboxylate (250 mg, 0.41 mmol) was dissolved with DCM (5 mL), and trifluoroacetic acid (1 mL) was added. The reaction liquid was stirred at room temperature overnight and concentrated under reduced pressure to give the crude product 2-(2,6-dioxopiperidin-3-yl)-5-(4-((2-(piperidin-4-yl)ethoxy)methyl)piperidine-1 -carbonyl)isoindoline-1,3-dione.

LC-MS: (ESI, *m*/*z*): [M+H]⁺=511.3.

### Step 5: preparation of tert-butyl (3-((1R)-1-((6-((1S,4S)-4-(4-(2-((1-(2-(2,6-dioxopiperidin-3-yl)-1,3-dioxoisoindolin-5-carbonyl)piperidin-4-yl)methoxy)ethyl)piperidine-1-carbonyl)cyclohexyl)-7-methoxy-2-methylquinazolin-4-yl)amino)ethyl)-5-(trifluoromethyl)phenyl)carboxylate

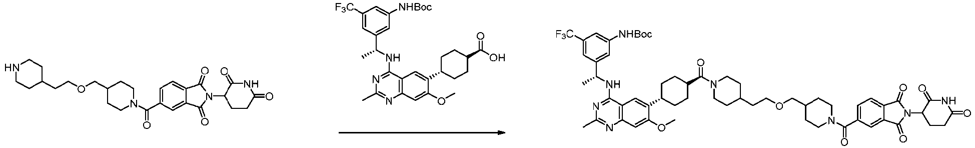

2-(2,6-Dioxopiperidin-3-yl)-5-(4-((2-(piperidin-4-yl)ethoxy)methyl)piperidine-1-carbonyl)isoindoline-1,3-dione (200 mg, 0.40 mmol), HATU (130 mg, 0.35 mmol), and DIEA (89 mg, 0.69 mmol) were added to a solution of (1*S*,4*S*)-4-(4-(((*R*)-1-(3-((*tert*-butoxycarbonyl)amino)-5-(trifluoromethyl)phenyl)ethyl)amino)-7-methoxy-2-methylquinazolin-6-yl)cyclohexane-1-carboxylic acid (140 mg, 0.23 mmol) in DMF (10 mL). The reaction liquid was stirred at room temperature for 2 h, diluted with water (50 mL), stirred for 10 min, and filtered. The solid was washed with water (5 mL × 2) to give the crude product tert-butyl (3-((1*R*)-1-((6-((1*S*,4*S*)-4-(4-(2-((1-(2-(2,6-dioxopiperidin-3-yl)-1,3-dioxoisoindoline-5-carbonyl)piperidin-4-yl)methoxy)ethyl)piperidine-1-carbonyl)cyclohexyl)-7-methoxy-2-methylquinazolin-4-yl)amino)ethyl)-5-(trifluoromethyl)phenyl)carboxylate. LC-MS: (ESI, *m*/*z*): [M+H]⁺=1095.5.

### Step 6: preparation of 5-(4-((2-(1-((1S,4S)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)ethoxy)methyl)piperidine-1-carbonyl)-2-(2,6-dioxopiperidin-3 -yl)isoindoline-1,3 -dione

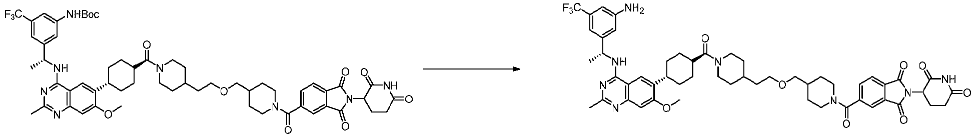

tert-Butyl (3-((1*R*)-1-((6-((1*S*,4*S*)-4-(4-(2-((1-(2-(2,6-dioxopiperidin-3-yl)-1,3-dioxoisoindoline-5-carbonyl)piperidin-4-yl)methoxy)ethyl)piperidine-1-carbonyl)cyclohexyl)-7-methoxy-2-methylquinazolin-4-yl)amino)ethyl)-5-(trifluoromethyl)phenyl)carboxylate (250 mg, 0.23 mmol) was dissolved with DCM (5 mL), and trifluoroacetic acid (1 mL) was added. The reaction liquid was stirred at room temperature overnight and concentrated under reduced pressure. The crude product was purified by preparative reversed-phase chromatography to give 5-(4-((2-(1-((1*S*,4*S*)-4-(4-(((*R*)-1-(3-amino-5-(trifluoromethyl)phenyl)ethyl)amino)-7-methoxy-2-methylquinazolin-6-yl)cyclohexane-1-carbonyl)piperidin-4-yl)ethoxy)methyl)piperidine-1-carbonyl)-2-(2,6-dioxopiperidin-3 -yl)isoindoline-1,3 -dione.

LC-MS: (ESI, *m*/*z*): [M+H]⁺=995.4.

¹H NMR (400 MHz, DMSO-*d*₆) δ 11.13 (s, 1H), 8.15 (s, 1H), 8.11 (d, *J* = 8.1 Hz, 1H), 8.06 (s, 1H), 7.98 (d, *J* = 7.6 Hz, 1H), 7.88 - 7.82 (m, 2H), 6.99 (s, 1H), 6.88 (s, 1H), 6.85 (s, 1H), 6.70 (s, 1H), 5.59 - 5.50 (m, 3H), 5.18 (dd, *J* = 12.8, 5.4 Hz, 1H), 4.47-4.39 (m, 2H), 3.95-3.85 (m, 4H), 3.41 (t, *J* = 6.4 Hz, 2H), 3.24 (d, *J* = 5.3 Hz, 2H), 3.12-2.77 (m, 5H), 2.70-2.55 (m, 4H), 2.36 (s, 3H), 2.09-1.40 (m, 21H), 1.22-0.90 (m, 4H).

### Example 40: 1-(5-(4-(((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl-2,2,6,6-d₄)oxy)methyl)piperidine-1-carbonyl)-2-chlorophenyl)dihydropyrimidine-2,4(1H,3H)-dione

### Step 1: preparation of 1-phenylpiperidin-2,2,6,6-d₄-4-ol

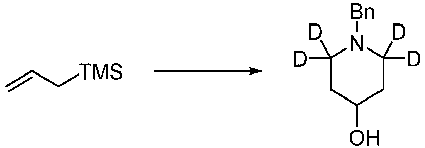

Trifluoroacetic acid (2.34 g, 20.5 mmol) was added dropwise to benzylamine (2.19 g, 20.5 mmol), and a solution of CD₂O in D₂O (20%, 1.5 g of CD₂O was dissolved in 6 mL of D₂O) was added. The reaction liquid was sonicated for 10 min and stirred at 20 °C for 1 h until the solution became clear. Allyltrimethylsilane (2.56 g, 22.5 mmol) was added, and the resulting mixture was heated to 40 °C and stirred overnight, diluted with water (10 mL), and adjusted to above pH 9 with solid potassium carbonate. Water (50 mL) was added, and the resulting mixture was extracted with ethyl acetate (50 mL × 3). The organic phase was washed with saturated brine (100 mL), dried over anhydrous sodium sulfate, filtered, and concentrated under reduced pressure. The crude product was purified by column chromatography (MeOH:DCM = 1:9, 1% ammonia water was added to the eluent) to give 1-phenylpiperidin-2,2,6,6-*d*₄-4-ol.

LC-MS: (ESI, *m*/*z*): [M+H]⁺=196.3.

### Step 2: preparation of piperidine-2,2,6,6-d₄-4-ol

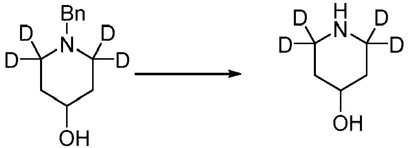

1-Phenylpiperidine-2,2,6,6-*d*₄-4-ol (470 mg, 2.4 mmol) was dissolved in methanol (30 mL), and Pd/C (100 mg) and Pd(OH)₂/C (100 mg) were added. The reaction liquid was stirred at 70 °C overnight under hydrogen atmosphere, filtered, and concentrated under reduced pressure to give piperidine-2,2,6,6-*d*₄-4-ol.

LC-MS: (ESI, *m*/*z*): [M+H]⁺=106.4.

### Step 3: preparation of tert-butyl 4-hydroxypiperidine-1-carboxylate-2,2,6,6-d₄

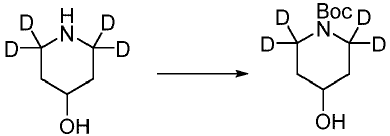

Piperidine-2,2,6,6-*d*₄-4-ol (215 mg, 2.05 mmol) was dissolved with DCM (10 mL), and (Boc)₂O (491 mg, 2.26 mmol), TEA (620 mg, 6.15 mmol), and DMAP (25 mg, 0.21 mmol) were added. The reaction liquid was stirred at room temperature overnight. The reaction liquid was diluted with water (50 mL) and extracted with dichloromethane (50 mL × 3). The organic phase was washed with saturated brine (100 mL), dried over anhydrous sodium sulfate, filtered, and concentrated under reduced pressure. The crude product was purified by column chromatography (EA:PE = 1:1) to give tert-butyl 4-hydroxypiperidine-1-carboxylate-2,2,6,6-*d*₄.

LC-MS: (ESI, *m*/*z):* [M+Na]⁺=228.

### Step 4: preparation of tert-butyl 4-(pyridin-4-ylmethoxy)piperidine-1-carboxylate-2,2,6,6-d₄

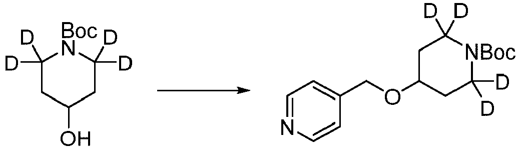

tert-Butyl 4-hydroxypiperidine-1-carboxylate-2,2,6,6-*d*₄ (200 mg, 0.98 mmol) was dissolved with THF (20 mL), and NaH (60%, 235 mg, 5.88 mmol) was added at 0 °C. The reaction liquid was stirred for 2 h. Bromopicoline hydrobromide (296 mg, 1.18 mmol) was added, and the reaction liquid was heated to 70 °C and stirred overnight. The reaction liquid was diluted with water (50 mL) and extracted with ethyl acetate (50 mL × 3). The organic phase was washed with saturated brine (100 mL), dried over anhydrous sodium sulfate, filtered, and concentrated under reduced pressure. The crude product was purified by column chromatography (MeOH:DCM = 1:19) to give *tert-*butyl 4-(pyridin-4-ylmethoxy)piperidine-1-carboxylate-2,2,6,6-*d*₄.

LC-MS: (ESI, *m*/*z*): [M+H]⁺=297.2.

### Step 5: preparation of tert-butyl 4-(piperidin-4-ylmethoxy)piperidine-1-carboxylate-2,2,6,6-d₄

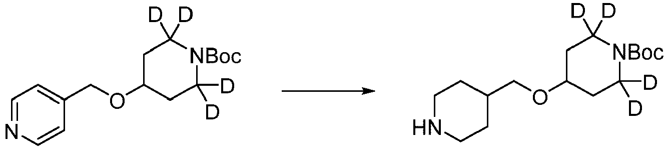

tert-Butyl 4-(pyridin-4-ylmethoxy)piperidine-1-carboxylate-2,2,6,6-*d*₄ (150 mg, 0.51 mmol) was dissolved with a mixed solution of isopropanol (6 mL) and water (7 mL), and Pd(OH)₂/C (45 mg) was added. The reaction liquid was stirred at 70 °C overnight under hydrogen atmosphere. The reaction liquid was filtered and concentrated under reduced pressure to give tert-butyl 4-(piperidine-4-ylmethoxy)piperidine-1-carboxylate-2,2,6,6-*d*₄.

LC-MS: (ESI, *m*/*z*): [M+H]⁺=303.2.

### Step 6: preparation of tert-butyl 4-((1-(4-chloro-3-(2,4-dioxotetrahydropyrimidin-1(2H)-yl)benzoyl)piperidin-4-yl)methoxy)piperidine-1-carboxylate-2,2,6,6-d₄

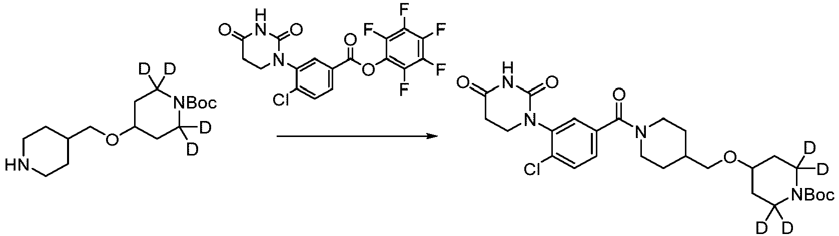

tert-Butyl 4-(piperidin-4-ylmethoxy)piperidine-1-carboxylate-2,2,6,6-*d*₄ (130 mg, 0.43 mmol) was dissolved with DMSO (5 mL), and DIEA (133 mg, 1.04 mmol) and pentafluorophenyl 4-chloro-3-(2,4-dioxotetrahydropyrimidin-1(2*H*)-yl)benzoate (150 mg, 0.35 mmol) were added. The reaction liquid was stirred at room temperature for 2 h. Water (30 mL) was added, and the resulting mixture was stirred for 5 min and extracted with ethyl acetate (30 mL × 3). The organic phase was washed with saturated brine (50 mL), dried over anhydrous sodium sulfate, filtered, and concentrated under reduced pressure. The crude product was purified by column chromatography (MeOH:DCM = 1:9) to give tert-butyl 4-((1-(4-chloro-3-(2,4-dioxotetrahydropyrimidin-1(2*H*)-yl)benzoyl)piperidin-4-yl)methoxy)piperidine-1-carboxylate-2,2,6,6-*d*₄.

LC-MS: (ESI, *m*/*z*): [M+Na]⁺=575.3.

### Step 7: preparation of 1-(2-chloro-5-(4-(((piperidin-4-yl-2,2,6,6-d₄)oxy)methyl)piperidine-1-carbonyl)phenyl)dihydropyrimidine-2,4(1H,3H)-dione

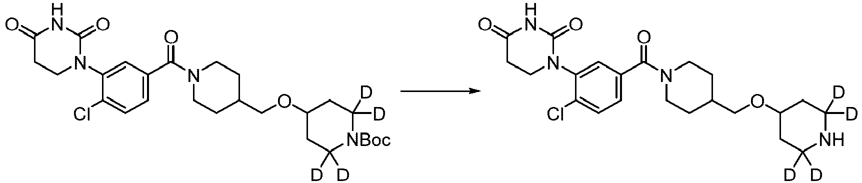

tert-Butyl 4-((1-(4-chloro-3-(2,4-dioxotetrahydropyrimidin-1(2*H*)-yl)benzoyl)piperidin-4-yl)methoxy)piperidine-1-carboxylate-2,2,6,6-*d*₄ (130 mg, 0.24 mmol) was dissolved with DCM (5 mL), and trifluoroacetic acid (1 mL) was added. The reaction liquid was stirred at room temperature for 2 h and concentrated under reduced pressure to give 1-(2-chloro-5-(4-(((piperidin-4-yl-2,2,6,6-*d*₄)oxy)methyl)piperidine-1-carbonyl)phenyl)dihydropyrimidine-2,4(1*H*,3*H*)-dione, which was directly used in the next step.

LC-MS: (ESI, *m*/*z*): [M+H]⁺=453.2.

### Step 8: preparation of tert-butyl (3-((R)-1-((6-((1R,4R)-4-(4-((1-(4-chloro-3-(2,4-dioxotetrahydropyrimidin-1(2H)-yl)benzoyl)piperidin-4-yl)methoxy)piperidine-1-carbonyl-2,2,6,6-d₄)cyclohexyl)-7-methoxy-2-methylquinazolin-4-yl)amino)ethyl)-5-(trifluoromethyl)phenyl)carboxylate

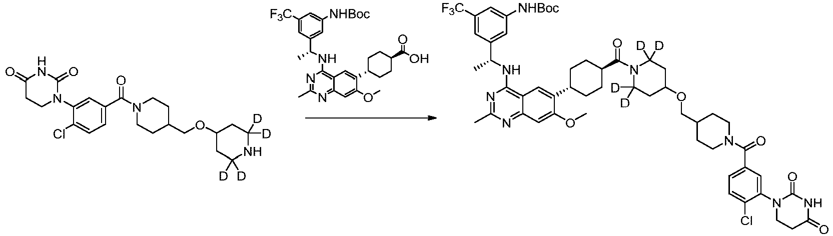

1-(2-Chloro-5-(4-(((piperidin-4-yl-2,2,6,6-*d*₄)oxy)methyl)piperidine-1-carbonyl)phenyl)dihydropyrimidine-2,4(1*H*,3*H*)-dione (130 mg, 0.29 mmol) was dissolved with DMSO (5 mL), and (1*R*,4*R*)-4-(4-(((*R*)-1-(3-((*tert-*butoxycarbonyl)amino)-5-(trifluoromethyl)phenyl)ethyl)amino)-7-methoxy-2-methylquinazolin-6-yl)cyclohexane-1-carboxylic acid (130 mg, 0.22 mmol), HATU (110 mg, 0.29 mmol), and DIEA (142 mg, 1.10 mmol) were added. The reaction liquid was stirred at room temperature for 2 h. Water (30 mL) was added, and the resulting mixture was stirred for 5 min and extracted with ethyl acetate (30 mL × 3). The organic phase was washed with saturated brine (50 mL), dried over anhydrous sodium sulfate, filtered, and concentrated under reduced pressure. The crude product was purified by column chromatography (MeOH:DCM = 1:9) to give tert-butyl (3-((*R*)-1-((6-((1*R*,4*R*)-4-(4-((1-(4-chloro-3-(2,4-dioxotetrahydropyrimidin-1(2*H*)-yl)benzoyl)piperidin-4-yl)methoxy)piperidine-1-carbonyl-2,2,6,6-*d*₄)cyclohexyl)-7-methoxy-2-methylquinazolin-4-yl)amino)ethyl)-5-(trifluoromethyl)phenyl)carboxylate.

LC-MS: (ESI, *m*/*z*): [M+H]⁺=1037.5.

### Step 9: preparation of 1-(5-(4-(((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl-2,2,6,6-d₄)oxy)methyl)piperidine-1-carbonyl)-2-chlorophenyl)dihydropyrimidine-2,4(1H,3H)-dione

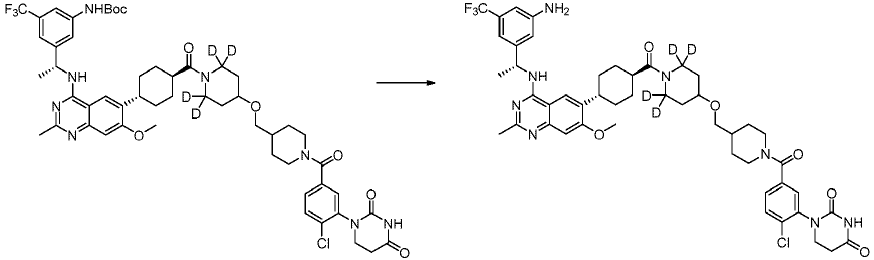

tert-Butyl (3-((*R*)-1-((6-((1*R*,4*R*)-4-(4-((1-(4-chloro-3-(2,4-dioxotetrahydropyrimidin-1(2*H*)-yl)benzoyl)piperidin-4-yl)methoxy)piperidine-1-carbonyl-2,2,6,6-*d*₄)cyclohexyl)-7-methoxy-2-methylquinazolin-4-yl)amino)ethyl)-5-(trifluoromethyl)phenyl)carboxylate (130 mg, 0.13 mmol) was dissolved in DCM (5 mL), and TFA (1 mL) was added. The reaction liquid was stirred at room temperature for 2 h and concentrated under reduced pressure. The crude product was dissolved with DCM (20 mL), and a saturated aqueous sodium bicarbonate solution (20 mL) was added to adjust the pH to neutrality. The resulting mixture was stirred for 10 min, extracted with MeOH/DCM (v:v = 1:9, 20 mL × 3), dried over anhydrous sodium sulfate, filtered, and concentrated under reduced pressure. The crude was purified by preparative reversed-phase chromatography to give 1-(5-(4-(((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl-2,2,6,6-*d*₄)oxy)methyl)piperidine-1-carbonyl)-2-chlorophenyl)dihydropyrimidine-2,4(1*H*,3*H*)-dione.

LC-MS: (ESI, *m*/*z):* [M+H]⁺=937.4.

¹H NMR (400 MHz, DMSO-*d*₆) δ 10.50 (s, 1H), 8.14-8.02 (m, 2H), 7.64 (d, *J* = 8.2 Hz, 1H), 7.54 (d, *J* = 1.9 Hz, 1H), 7.38 (dd, *J=* 8.2, 1.9 Hz, 1H), 6.99 (s, 1H), 6.86 (d, *J*= 10.5 Hz, 2H), 6.70 (s, 1H), 5.64-5.46 (m, 3H), 4.51-4.39 (m, 1H), 3.88 (s, 3H), 3.75- 3.42 (m, 4H), 3.12 - 3.01 (m, 1H), 2.95 (t, *J* = 11.2 Hz, 1H), 2.83 - 2.57 (m, 5H), 2.35 (s, 3H), 1.95 -1.50 (m, 17H), 1.45-1.27 (m, 2H), 1.25-1.07 (m, 2H).

### Example 41: 5-(4-((2-(1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)ethoxy)methyl)piperidin-1-yl)-2-(2,4-dioxocyclohexyl)isoindoline-1,3 -dione

### Step 1: preparation of tert-butyl 4-(2-((1-(2-(2,4-dioxocyclohexyl)-1,3-dioxoisoindolin-5-yl)piperidin-4-yl)methoxy)ethyl)piperidine-1 -carboxylate

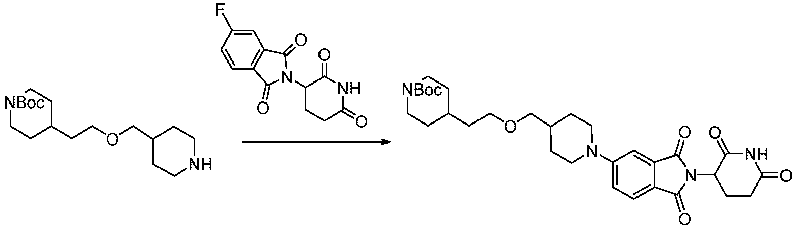

DIEA (675 mg, 3.9 mmol) was added to a solution of tert-butyl 4-(2-(piperidin-4-ylmethoxy)ethyl)piperidine-1-carboxylate (430 mg, 1.3 mmol) and 2-(2,6-dioxopiperidin-3-yl)-5-fluoroisoindoline-1,3-dione (718 mg, 2.6 mmol) in DMF (3 mL). The reaction mixture was heated to 110 °C and stirred overnight. The reaction liquid was poured into water, and the resulting mixture was extracted with EA (30 mL × 2). The organic phase was dried over anhydrous Na₂SO₄ and concentrated under reduced pressure. The resulting crude product was purified by automatic column chromatography (EA/PE) to give tert-butyl 4-(2-((1-(2-(2,4-dioxocyclohexyl)-1,3-dioxoisoindolin-5-yl)piperidin-4-yl)methoxy)ethyl)piperidine-1 -carboxylate.

LC-MS: (ESI, *m*/*z*): [M+H]⁺=583.4.

### Step 2: preparation of 2-(2,6-dioxopiperidin-3-yl)-5-(4-((2-(piperidin-4-yl)ethoxy)methyl)piperidin-1-yl)isoindoline-1,3-dione

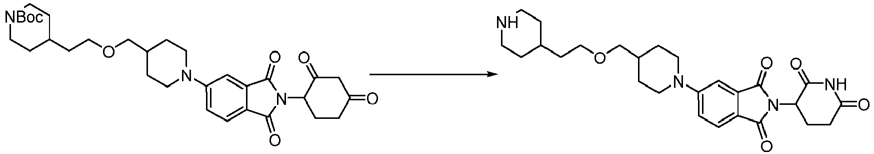

A solution of tert-butyl 4-(2-((1-(2-(2,4-dioxocyclohexyl)-1,3-dioxoisoindolin-5-yl)piperidin-4-yl)methoxy)ethyl)piperidine-1-carboxylate (210 mg, 0.36 mmol) in HCl/1,4-dioxane (4 M, 5 mL) was stirred at room temperature for 1 h. The reaction liquid was directly concentrated to give 2-(2,6-dioxopiperidine-3-yl)-5-(4-((2-(piperidine-4-yl)ethoxy)methyl)piperidine-1-yl)isoindoline-1,3 -dione.

LC-MS: (ESI, *m*/*z*): [M+H]⁺=483.3.

### Step 3: preparation of 5-(4-((2-(1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)ethoxy)methyl)piperidin-1-yl)-2-(2,4-dioxocyclohexyl)isoindoline-1,3 -dione

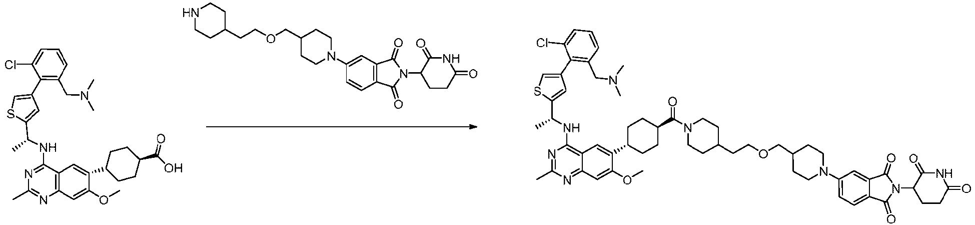

HATU (77 mg, 0.20 mmol) and DIEA (65 mg, 0.51 mmol) were added to a solution of (1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carboxylic acid (100 mg, 0.17 mmol) and 2-(2,6-dioxopiperidin-3-yl)-5-(4-((2-(piperidin-4-yl)ethoxy)methyl)piperidin-1-yl)isoindoline-1,3-dione (87 mg, 0.17 mmol) in DMF (5 mL). The reaction liquid was stirred at room temperature for 2 h. The reaction liquid was poured into water, and the resulting mixture was extracted with EA (20 mL × 3), dried over anhydrous Na₂SO₄, and concentrated under reduced pressure. The resulting crude product was purified by prep-HPLC to give 5-(4-((2-(1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)ethoxy)methyl)piperidin-1-yl)-2-(2,4-dioxocyclohexyl)isoindoline-1,3-dione.

LC-MS: (ESI, m/z): [M-H]⁻=1057.4.

¹H NMR (400 MHz, DMSO-*d*₆) δ 11.07 (s, 1H), 8.31 (d, *J* = 8.1 Hz, 1H), 8.05 (s, 1H), 7.64 (d, *J* = 8.5 Hz, 1H), 7.49 - 7.38 (m, 2H), 7.36 - 7.29 (m, 2H), 7.23 (dd, *J* = 9.8, 1.7 Hz, 2H), 7.02 (s, 1H), 6.96 (s, 1H), 6.03 - 5.87 (m, 1H), 5.10-5.00 (m, 1H), 4.42-4.31 (m, 1H), 4.10-4.00 (m, 2H), 3.97-3.85 (m, 4H), 3.41 (t, *J* = 6.3 Hz, 2H), 3.23 (d, J = 6.2 Hz, 2H), 3.18 - 3.07 (m, 2H), 3.05 - 2.81 (m, 5H), 2.71 - 2.52 (m, 3H), 2.42 (s, 3H), 2.01 (s, 6H), 1.89-1.44 (m, 20H), 1.26-0.86 (m, 4H).

### Example 42: (R)-1-(5-(9-(((3-(4-(4-((1-(3-amino-5-(trifluoromethyl)phenyl)ethyl)amino)-2,7-dimethylquinazolin-6-yl)-2-oxopyridin-1(2H)-yl)propyl)(methyl)amino)methyl)-3-azaspiro[5.5]undecane-3-carbonyl)-2-chlorophenyl)dihydropyrimidine-2,4(1H,3H)-dione

### Step 1: preparation of benzyl (3-hydroxypropyl)(methyl)carboxylate

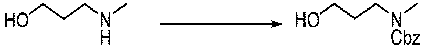

3-(Methylamino)propan-1-ol (5.0 g, 56.17 mmol) and benzyl chloroformate (10.5 g, 61.79 mmol) were dissolved in 1,4-dioxane (40 mL), and a sodium hydroxide solution (2 mol/L, 48 mL) was added. The reaction liquid was stirred at 50 °C for 0.5 h and directly concentrated under reduced pressure to give the crude product benzyl (3-hydroxypropyl)(methyl)carboxylate.

LC-MS: (ESI, *m*/*z):* [M+H] ⁺=224.3.

### Step 2: preparation of benzyl (3-bromopropyl)(methyl)carboxylate

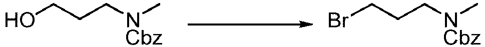

Benzyl (3-hydroxypropyl)(methyl)carboxylate (4.6 g, 20 mmol) was dissolved in tetrahydrofuran (40 mL), and triphenylphosphine (7.8 g, 30 mmol) and carbon tetrabromide (10 g, 30 mmol) were separately added. The reaction liquid was stirred at room temperature for 2 h, diluted with water (200 mL), extracted with ethyl acetate (200 mL × 3), dried over anhydrous sodium sulfate, and concentrated under reduced pressure. The crude product was purified by column chromatography (PE:EA = 1:1) to give benzyl (3-bromopropyl)(methyl)carboxylate.

LC-MS: (ESI, *m*/*z):* [M+H] ⁺=288.1.

### Step 3: preparation of benzyl (3-(4-bromo-2-oxopyridin-1(2H)-yl)propyl)(methyl)carboxylate

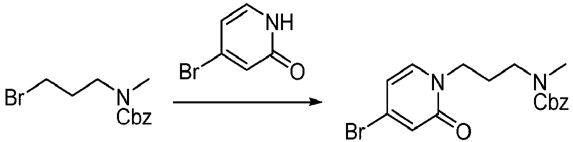

4-Bromopyridin-2(1*H*)-one (790 mg, 4.5 mmol) was dissolved with anhydrous DMF (5 mL) and cooled to 0 °C, and NaH (218 mg, 5.5 mmol) was added. The reaction liquid was stirred for 0.5 h. Then Benzyl (3-bromopropyl)(methyl)carboxylate (1.3 g, 4.5 mmol) was added at room temperature, and the resulting mixture was stirred for 12 h, diluted with water (100 mL), and extracted with ethyl acetate (150 mL × 4). The organic phase was washed with saturated brine (100 mL), dried over anhydrous sodium sulfate, filtered, and concentrated under reduced pressure. The crude product was purified by column chromatography (PE:EA = 1:2) to give benzyl (3-(4-bromo-2-oxopyridin-1(2*H*)-yl)propyl)(methyl)carboxylate.

LC-MS: (ESI, *m*/*z):* (ESI, *m*/*z):* [M+H] ⁺=379.0.

### Step 4: preparation of benzyl methyl(3-(2-oxo-4-(4,4,5,5-tetramethyl-1,3,2-dioxaborolan-2-yl)pyridin-1(2H)-yl)propyl)carboxylate

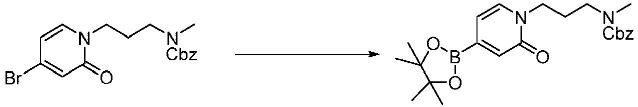

Benzyl (3-(4-bromo-2-oxopyridin-1(2*H*)-yl)propyl)(methyl)carboxylate (1.2 g, 3.2 mmol) and 4,4,4',4',5,5,5',5'-octamethyl-2,2'-bis(1,3,2-dioxaborolan) (979 mg, 3.8 mmol) were dissolved with anhydrous 1,4-dioxane, and Pd(dppf)Cl₂ (120 mg) and potassium carbonate (880 mg, 6.47 mmol) were added. The reaction liquid was stirred at 100 °C overnight under nitrogen atmosphere, diluted with water (100 mL), and extracted with ethyl acetate (100 mL × 3). The organic phase was washed with saturated brine (100 mL), dried over anhydrous sodium sulfate, filtered, and concentrated under reduced pressure. The crude product was purified by column chromatography (ethyl acetate:petroleum ether = 1:1) to give the product benzyl methyl(3-(2-oxo-4-(4,4,5,5-tetramethyl-1,3,2-dioxaborolan-2-yl)pyridin-1(2*H*)-yl)propyl)carboxylate.

LC-MS: (ESI, *m*/*z):* [M+H] ⁺=427.4.

### Step 5: preparation of benzyl (3-(4-(4-hydroxy-2,7-dimethylquinazolin-6-yl)-2-oxopyridin-1(2H)-yl)propyl)(methyl)carboxylate

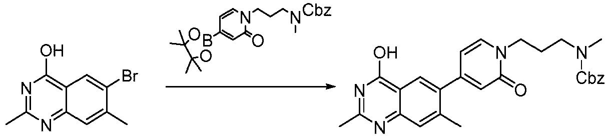

6-Bromo-2,7-dimethylquinazolin-4-ol (450 mg, 1.77 mmol) and benzyl methyl(3-(2-oxo-4-(4,4,5,5-tetramethyl-1,3,2-dioxaborolan-2-yl)pyridin-1(2*H*)-yl)propyl)carboxylate (980 mg, 2.3 mmol) were dissolved in DMF/water solution (10/1, 4 mL), and potassium carbonate (480 mg, 3.5 mmol) and Pd(dppf)Cl₂ (90 mg) were added. The reaction liquid was purged with nitrogen, heated to 100 °C under nitrogen atmosphere, stirred for 2 h, diluted with water (50 mL), and extracted with ethyl acetate (50 mL × 3). The organic phase was washed with saturated brine (100 mL), dried over anhydrous sodium sulfate, filtered, and concentrated under reduced pressure. The crude product was purified by column chromatography (DCM:MEOH = 10:1) to give the product benzyl (3-(4-(4-hydroxy-2,7-dimethylquinazolin-6-yl)-2-oxopyridin-1(2*H*)-yl)propyl)(methyl)carboxylate.

LC-MS: (ESI, *m*/*z):* (ESI, *m*/*z):* [M+H] ⁺=473.3.

### Step 6: preparation of benzyl (R)-(3-(4-(4-((1-(3-((tert-butoxycarbonyl)amino)-5-(trifluoromethyl)phenyl)ethyl)amino)-2,7-dimethylquinazolin-6-yl)-2-oxopyridin-1(2H)-yl)propyl)(methyl)carboxylate

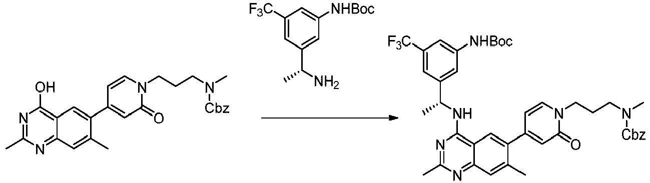

Benzyl (3-(4-(4-hydroxy-2,7-dimethylquinazolin-6-yl)-2-oxopyridin-1(2*H*)-yl)propyl)(methyl)carboxylate (480 mg, 1.02 mmol) was dissolved with DMF (4 mL), and DBU (388 mg, 2.55 mmol) and BOP (583 mg,1.32 mmol) were added. The reaction liquid was stirred at room temperature for 0.5 h. tert-Butyl (R)-(3-(1-aminoethyl)-5-(trifluoromethyl)phenyl)carboxylate (403 mg, 1.32 mmol) was added, and the reaction liquid was heated to 70 °C and stirred overnight, diluted with water (50 mL), and extracted with ethyl acetate (50 mL × 3). The organic phase was washed with saturated brine (100 mL), dried over anhydrous sodium sulfate, filtered, and concentrated under reduced pressure. The crude product was purified by column chromatography (ethyl acetate:petroleum ether = 1:1) to give the product benzyl (R)-(3-(4-(4-((1-(3-((tert-butoxycarbonyl)amino)-5-(trifluoromethyl)phenyl)ethyl)amino)-2,7-dimethylquinazolin-6-yl)-2-oxopyridin-1(2*H*)-yl)propyl)(methyl)carboxylate.

LC-MS: (ESI, *m*/*z):* [M+H] ⁺=759.4.

### Step 7: preparation of tert-butyl (R)-(3-(1-((2,7-dimethyl-6-(1-(3-(methylamino)propyl)-2-oxo-1,2-dihydropyridin-4-yl)quinazolin-4-yl)amino)ethyl)-5-(trifluoromethyl)phenyl)carboxylate

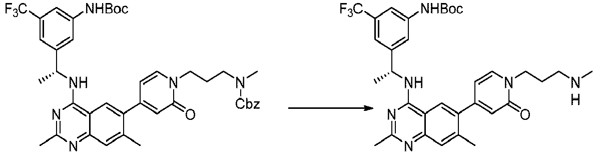

Benzyl (R)-(3-(4-(4-((1-(3-((tert-butoxycarbonyl)amino)-5-(trifluoromethyl)phenyl)ethyl)amino)-2,7-dimethylquinazolin-6-yl)-2-oxopyridin-1(2*H*)-yl)propyl)(methyl)carboxylate (300 mg, 0.4 mmol) was dissolved with ethyl acetate (4 mL), and Pd/C (60 mg) was added. The reaction liquid was purged with hydrogen, stirred at room temperature for 2 h under hydrogen atmosphere, filtered, and concentrated to give the crude product tert-butyl (R)-(3-(1-((2,7-dimethyl-6-(1-(3-(methylamino)propyl)-2-oxo-1,2-dihydropyridin-4-yl)quinazolin-4-yl)amino)ethyl)-5-(trifluoromethyl)phenyl)carboxylate.

LC-MS: (ESI, *m*/*z):* [M+H] ⁺=625.5.

### Step 8: preparation of tert-butyl (R)-9-(((3-(4-(4-((1-(3-((tert-butoxycarbonyl)amino)-5-(trifluoromethyl)phenyl)ethyl)amino)-2,7-dimethylquinazolin-6-yl)-2-oxopyridin-1(2H)-yl)propyl)methyl)amino)methyl)-3 -azaspiro [5.5]undecane-3 -carboxylate

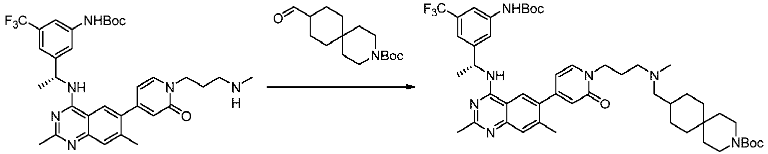

tert-Butyl (*R*)-(3-(1-((2,7-dimethyl-6-(1-(3-(methylamino)propyl)-2-oxo-1,2-dihydropyridin-4-yl)quinazolin-4-yl)amino)ethyl)-5-(trifluoromethyl)phenyl)carboxylate (130 mg, 0.21 mmol) and tert-butyl 9-formyl-3-azaspiro[5.5]undecane-3-carboxylate (59 mg, 0.21 mmol) were dissolved with THF (4 mL), and sodium triacetoxyborohydride (131 mg, 0.63 mmol) was added. The reaction liquid was stirred at room temperature for 2 h, diluted with water (50 mL), and extracted with ethyl acetate (30 mL × 3). The organic phase was washed with saturated brine (50 mL), dried over anhydrous sodium sulfate, filtered, and concentrated under reduced pressure. The crude product was purified by column chromatography (dichloromethane:methanol = 10:1) to give the product tert-butyl (R)-9-(((3-(4-(4-((1-(3-((tert-butoxycarbonyl)amino)-5-(trifluoromethyl)phenyl)ethyl)amino)-2,7-dimethylquinazolin-6-yl)-2-oxopyridin-1(2*H*)-yl)propyl)methyl)amino)methyl)--azaspiro[5.5]undecane-3-carboxylate.

LC-MS: (ESI, *m*/*z):* [M+H] ⁺=890.5.

### Step 9: preparation of (R)-1-(3-(((3-azaspiro[5.5]undecan-9-yl)methyl)(methyl)amino)propyl)-4-(4-((1-(3-amino-5-(trifluoromethyl)phenyl)ethyl)amino)-2,7-dimethylquinazolin-6-yl)pyridin-2(1H)-one

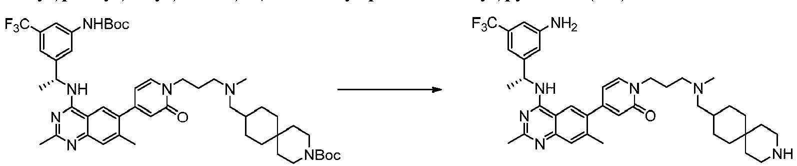

tert-Butyl (R)-9-(((3-(4-(4-((1-(3-((tert-butoxycarbonyl)amino)-5-(trifluoromethyl)phenyl)ethyl)amino)-2,7-dimethylquinazolin-6-yl)-2-oxopyridin-1(2*H*)-yl)propyl)methyl)amino)methyl)-3-azaspiro[5.5]undecane-3-carboxylate (100 mg, 0.11 mmol) was added to a solution of hydrogen chloride in 1,4-dioxane (4 M, 3 mL). The reaction liquid was reacted at room temperature for 2 h and directly concentrated under reduced pressure to give the crude product (R)-1-(3-(((3-azaspiro[5.5]undecan-9-yl)methyl)(methyl)amino)propyl)-4-(4-((1-(3-amino-5-(trifluoromethyl)phenyl)ethyl)amino)-2,7-dimethylquinazolin-6-yl)pyridin-2(1*H*)-one.

LC-MS: (ESI, *m*/*z):* [M+H] ⁺=690.5.

### Step 10: preparation of (R)-1-(5-(9-(((3-(4-(4-((1-(3-amino-5-(trifluoromethyl)phenyl)ethyl)amino)-2,7-dimethylquinazolin-6-yl)-2-oxopyridin-1(2H)-yl)propyl)(methyl)amino)methyl)-3-azaspiro[5.5]undecane-3-carbonyl)-2-chlorophenyl)dihydropyrimidine-2,4(1H,3H)-dione

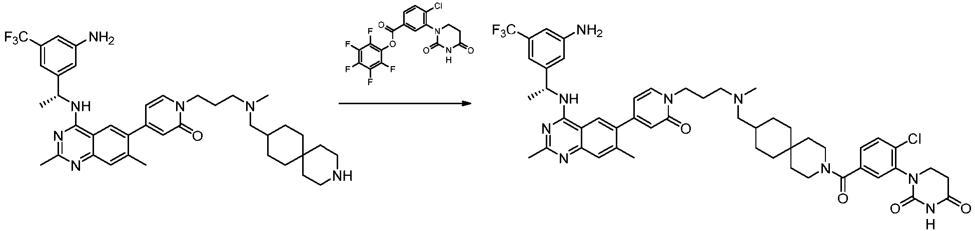

(*R*)-1-(3-(((3-Azaspiro[5.5]undecan-9-yl)methyl)(methyl)amino)propyl)-4-(4-((1-(3-amino-5-(trifluoromethyl)phenyl)ethyl)amino)-2,7-dimethylquinazolin-6-yl)pyridin-2(1*H*)-one (80 mg, 0.12 mmol) and pentafluorophenyl 4-chloro-3-(2,4-dioxotetrahydropyrimidin-1(2*H*)-yl)benzoate (51 mg, 0.12 mmol) were dissolved in DMSO (3 mL), and DIEA (60 mg, 0.48 mmol) was added. The reaction liquid was stirred at room temperature for 2 h, diluted with water (30 mL), extracted with ethyl acetate (30 mL × 3), washed with saturated brine, dried over anhydrous sodium sulfate, filtered, and concentrated under reduced pressure. The crude product was purified by Prep-HPLC to give the product (R)-1-(5-(9-(((3-(4-(4-((1-(3-amino-5-(trifluoromethyl)phenyl)ethyl)amino)-2,7-dimethylquinazolin-6-yl)-2-oxopyridin-1(2*H*)-yl)propyl)(methyl)amino)methyl)-3-azaspiro[5.5]undecane-3-carbonyl)-2-chlorophenyl)dihydropyrimidine-2,4(1*H*,3*H*)-dione.

LC-MS: (ESI, *m*/*z):* [M-H] ⁻=940.5.

¹H NMR (400 MHz, DMSO-*d*₆) δ 10.51 (s, 1H), 8.36 (d, *J* = 8.2 Hz, 1H), 8.29 (s, 1H), 7.73 (d, *J* = 6.6 Hz, 1H), 7.63 (d, *J* = 8.2 Hz, 1H), 7.54 (s, 1H), 7.50 (s, 1H), 7.38 (d, *J* = 8.3 Hz, 1H), 6.88 (s, 1H), 6.84 (s, 1H), 6.69 (s, 1H), 6.42 (s, 1H), 6.36 (d, *J* = 7.0 Hz, 1H), 5.60-5.48 (m, 3H), 4.00-3.90 (m, 2H), 3.80-3.50 (m, 5H), 3.29-3.20 (m, 3H), 2.77-2.70 (m, 2H), 2.44-2.26 (m, 7H), 2.20-2.06 (m, 4H), 1.87-1.78 (m, 2H), 1.74-1.65 (m, 2H), 1.62-1.20 (m, 10H), 1.16-0.92 (m, 4H).

### Example 43: 1-(5-(9-((4-((1R,4R)-4-(4-(((R)-1-(3-amino-5-(trifluoromethyl)phenyl)ethyl)amino)-7-(2-methoxy ethoxy)-2-methyl quin azolin-6-yl)cyclohexane-1 -carbonyl)piperazin-1 -yl)methyl)-3 - azaspiro[5.5]undecane-3-carbonyl)-2-chlorophenyl)dihydropyrimidine-2,4(1H,3H)-dione Step 1: preparation of methyl 4-(2-methoxyethoxy)-2-nitrobenzoate

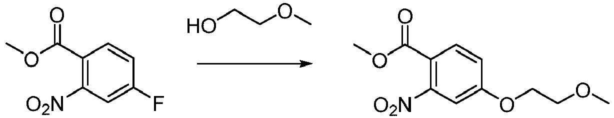

2-Methoxyethane-1-ol (365 mg, 4.8 mmol) was added to a three-necked flask and dissolved with THF (10 mL). The reaction liquid was purged three times with nitrogen. t-BuOK (1 M/THF, 4.2 mL, 4.2 mmol) was added at 0 °C, and the resulting mixture was reacted at 0 °C for 1 h. A solution of methyl 4-fluoro-2-nitrobenzoate (600 mg, 3.0 mmol) in THF (10 mL) was added dropwise, and the resulting mixture was successively reacted at 0 °C for 2 h. Water (50 mL) was added, and the resulting mixture was extracted with ethyl acetate (50 mL × 3). The organic phase was washed with saturated brine (100 mL), dried over anhydrous sodium sulfate, filtered, and concentrated under reduced pressure. The crude product was purified by column chromatography (EA:PE = 2:3) to give methyl 4-(2-methoxyethoxy)-2-nitrobenzoate.

¹H NMR (400 MHz, DMSO-*d*₆) δ 7.87 (d, *J* = 8.7 Hz, 1H), 7.57 (d, *J* = 2.5 Hz, 1H), 7.34 (dd, *J* = 8.7, 2.5 Hz, 1H), 4.29 - 4.24 (m, 2H), 3.81 (s, 3H), 3.70 - 3.66 (m, 2H), 3.31 (s, 3H).

### Step 2: preparation of methyl 2-amino-4-(2-methoxyethoxy)benzoate

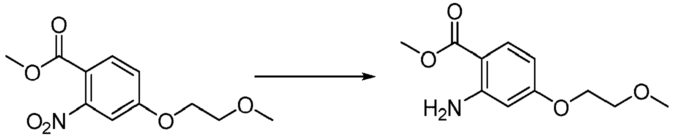

Methyl 4-(2-methoxyethoxy)-2-nitrobenzoate (850 mg, 3.3 mmol) was dissolved in EtOH (20 mL), and Raney-Ni (1 mL) was added. The reaction liquid was stirred at room temperature for 2 h under H₂ atmosphere, filtered, and concentrated under reduced pressure to give methyl 2-amino-4-(2-methoxyethoxy)benzoate.

LC-MS: (ESI, m/z): [M+H] ⁺=226.1.

### Step 3: preparation of methyl 2-amino-5-bromo-4-(2-methoxyethoxy)benzoate

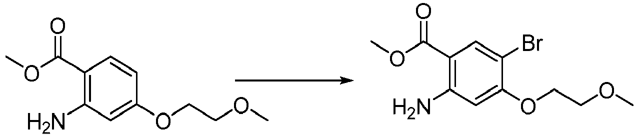

Methyl 2-amino-4-(2-methoxyethoxy)benzoate (680 mg, 3.0 mmol) was dissolved with DMF (10 mL), and NBS (484 mg, 2.7 mmol) was added at 0 °C. The reaction liquid was reacted at 0 °C for 1 h. Water (100 mL) was added, and the resulting mixture was stirred for 5 min and extracted with ethyl acetate (100 mL × 3). The organic phase was washed with saturated brine (200 mL), dried over anhydrous sodium sulfate, filtered, and concentrated under reduced pressure. The crude product was purified by column chromatography (EA:PE = 2:3) to give methyl 2-amino-5-bromo-4-(2-methoxyethoxy)benzoate.

LC-MS: (ESI, m/z): [M+H] ⁺=304.0.

### Step 4: preparation of 2-amino-5-bromo-4-(2-methoxyethoxy)benzoic acid

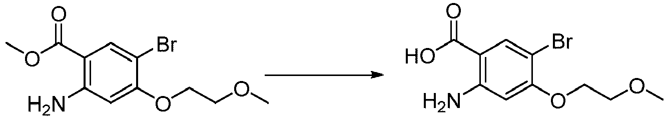

Methyl 2-amino-5-bromo-4-(2-methoxyethoxy)benzoate (710 mg, 2.3 mmol) was dissolved in THF/H₂O (15 mL, 2: 1), and LiOH (570 mg, 23.0 mmol) was added. The reaction liquid was stirred at 50 °C overnight. Water (20 mL) was added, and the resulting mixture was adjusted to pH 6 with 1 M hydrochloric acid, extracted with a mixed solution of MeOH/DCM (1/10, 30 mL × 3), dried over anhydrous sodium sulfate, filtered, and concentrated under reduced pressure to give 2-amino-5-bromo-4-(2-methoxyethoxy)benzoic acid.

LC-MS: (ESI, m/z): [M+3] ⁺=292.0.

### Step 5: preparation of 6-bromo-7-(2-methoxyethoxy)-2-methylquinazolin-4-ol

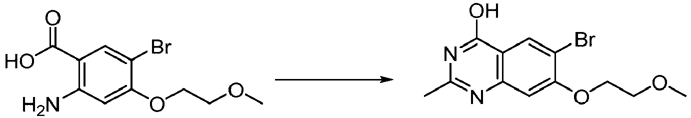

2-Amino-5-bromo-4-(2-methoxyethoxy)benzoic acid (665 mg, 2.3 mmol) was added to a sealed container and dissolved with MeOH (20 mL), and ammonium acetate (2.66 g, 34.5 mmol) and trimethyl orthoacetate (4.14 g, 34.5 mmol) were added. The reaction liquid was reacted at 120 °C overnight and concentrated under reduced pressure to remove methanol. Water (50 mL) was added, and the resulting mixture was extracted with ethyl acetate (50 mL × 3). The organic phase was washed with saturated brine (100 mL), dried over anhydrous sodium sulfate, filtered, and concentrated under reduced pressure. The crude product was purified by column chromatography (MeOH:DCM = 1:4) to give 6-bromo-7-(2-methoxyethoxy)-2-methylquinazolin-4-ol.

LC-MS: (ESI, m/z): [M+H] ⁺=312.9.

### Step 6: preparation of methyl 4-(4-hydroxy-7-(2-methoxyethoxy)-2-methylquinazolin-6-yl)cyclohex-3-ene-1-carboxylate

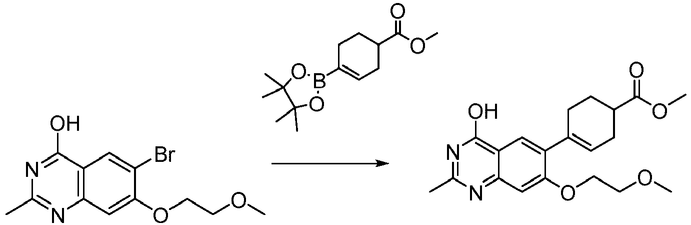

6-Bromo-7-(2-methoxyethoxy)-2-methylquinazolin-4-ol (210 mg, 0.67 mmol) was dissolved with a mixed solution of DMF/H₂O (11 mL, 10:1), and methyl 4-(4,4,5,5-tetramethyl-1,3,2-dioxaborolan-2-yl)cyclohex-3-ene-1-carboxylate (233 mg, 0.88 mmol), Na₂CO₃ (143 mg, 1.34 mmol), and Pd(dppf)Cl₂ (25 mg, 0.03 mmol) were added. The reaction liquid was reacted at 110 °C overnight under nitrogen atmosphere. Water (100 mL) was added, and the resulting mixture was stirred for 5 min and extracted with ethyl acetate (50 mL × 3). The organic phase was washed with saturated brine (100 mL), dried over anhydrous sodium sulfate, filtered, and concentrated under reduced pressure. The crude product was purified by column chromatography (MeOH:DCM = 1:9) to give methyl 4-(4-hydroxy-7-(2-methoxyethoxy)-2-methylquinazolin-6-yl)cyclohex-3-ene-1-carboxylate.

LC-MS: (ESI, m/z): [M+H] ⁺=373.1.

### Step 7: preparation of methyl (1R,4R)-4-(4-hydroxy-7-(2-methoxyethoxy)-2-methylquinazolin-6-yl)cyclohexane-1-carboxylate

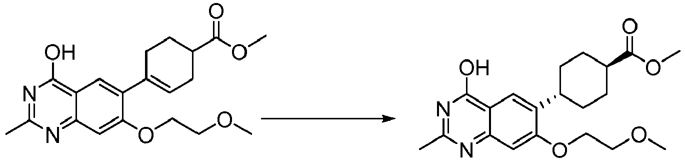

Methyl 4-(4-hydroxy-7-(2-methoxyethoxy)-2-methylquinazolin-6-yl)cyclohex-3-ene-1-carboxylate (500 mg, 1.3 mmol) was added to an autoclave and dissolved with MeOH (30 mL), and Pd(OH)₂/C (150 mg, 20%) was added. The reaction liquid was reacted at 70 °C for 48 h under hydrogen atmosphere (2 MPa), filtered, and concentrated under reduced pressure. The crude product was isolated by preparative reversed-phase chromatography to give methyl (1R,4R)-4-(4-hydroxy-7-(2-methoxyethoxy)-2-methylquinazolin-6-yl)cyclohexane-1-carboxylate.

LC-MS: (ESI, m/z): [M+H] ⁺=375.2.

### Step 8: preparation of methyl (1R,4R)-4-(4-(((R)-1-(3-((tert-butoxycarbonyl)amino)-5-(trifluoromethyl)phenyl)ethyl)amino)-7-(2-methoxyethoxy)-2-methylquinazolin-6-yl)cyclohexane-1-carboxylate

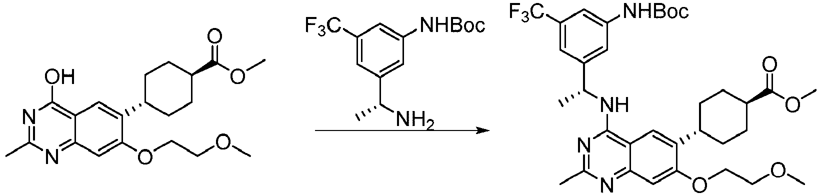

Methyl (1*R*,4*R*)-4-(4-hydroxy-7-(2-methoxyethoxy)-2-methylquinazolin-6-yl)cyclohexane-1-carboxylate (80 mg, 0.21 mmol) was dissolved in DMF (5 mL), and BOP (123 mg, 0.28 mmol) and DBU (81 mg, 0.53 mmol) were added. The reaction liquid was stirred at room temperature for 0.5 h. tert-Butyl (R)-(3-(1-aminoethyl)-5-(trifluoromethyl)phenyl)carboxylate (70 mg, 0.23 mmol) was added, and the resulting mixture was reacted at 70 °C overnight in an oil bath. Water (50 mL) was added, and the resulting mixture was stirred for 5 min and extracted with ethyl acetate (30 mL × 3). The organic phase was washed with saturated brine (50 mL), dried over anhydrous sodium sulfate, filtered, and concentrated under reduced pressure. The crude product was purified by reversed-phase chromatography to give methyl (1R,4R)-4-(4-(((R)-1-(3-((tert-butoxycarbonyl)amino)-5-(trifluoromethyl)phenyl)ethyl)amino)-7-(2-methoxyethoxy)-2-methylquinazolin-6-yl)cyclohexane-1-carboxylate. LC-MS: (ESI, m/z): [M+H] ⁺=661.2.

### Step 9: preparation of (1R,4R)-4-(4-(((R)-1-(3-((tert-butoxycarbonyl)amino)-5-(trifluoromethyl)phenyl)ethyl)amino)-7-(2-methoxyethoxy)-2-methylquinazolin-6-yl)cyclohexane-1-carboxylic acid

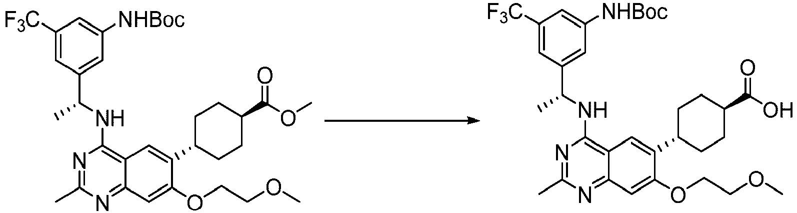

Methyl (1*R*,4*R*)-4-(4-(((*R*)-1-(3-((*tert*-butoxycarbonyl)amino)-5-(trifluoromethyl)phenyl)ethyl)amino)-7-(2-methoxyethoxy)-2-methylquinazolin-6-yl)cyclohexane-1-carboxylate (66 mg, 0.10 mmol) was dissolved in a mixed solution of THF/H₂O (15 mL, 2:1), and LiOH (48 mg, 2.0 mmol) was added. The reaction liquid was stirred at 50 °C overnight. Water (20 mL) was added, and the resulting mixture was adjusted to pH 6 with concentrated hydrochloric acid, extracted with MeOH/DCM (10%, 30 mL × 3), dried over anhydrous sodium sulfate, filtered, and concentrated under reduced pressure. The crude product was purified by reversed-phase chromatography to give (1*R*,4*R*)-4-(4-(((*R*)-1-(3-((*tert*-butoxycarbonyl)amino)-5-(trifluoromethyl)phenyl)ethyl)amino)-7-(2-methoxyethoxy)-2-methylquinazolin-6-yl)cyclohexane-1 -carboxylic acid.

LC-MS: (ESI, m/z): [M+H] ⁺=647.4.

### Step 10: preparation of tert-butyl (3-((R)-1-((6-((1R,4R)-4-(4-((3-(4-chloro-3-(2,4-dioxotetrahydropyrimidin-1(2H)-yl)benzoyl)-3-azaspiro[5.5]undecan-9-yl)methyl)piperazine-1-carbonyl)cyclohexyl)-7-(2-methoxyethoxy)-2-methylquinazolin-4-yl)amino)ethyl)-5-(trifluoromethyl)phenyl)carboxylate

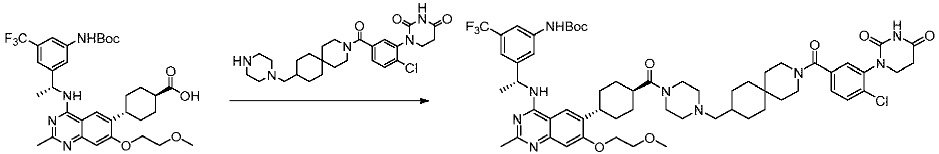

(1R,4R)-4-(4-(((R)-1-(3-((tert-Butoxycarbonyl)amino)-5-(trifluoromethyl)phenyl)ethyl)amino)-7-(2-methoxyethoxy)-2-methylquinazolin-6-yl)cyclohexane-1-carboxylic acid (50 mg, 0.08 mmol) was dissolved in DMF (5 mL), and 1-(2-chloro-5-(9-(piperazin-1-ylmethyl)-3-azaspiro[5.5]undecane-3-carbonyl)phenyl)dihydropyrimidine-2,4(1*H*,3*H*)-dione (48 mg, 0.10 mmol), DIEA (52 mg, 0.40 mmol), and pyBOP (50 mg, 0.10 mmol) were added. The reaction liquid was reacted at room temperature for 1 h. Water (50 mL) was added, and the resulting mixture was stirred for 5 min and extracted with ethyl acetate (30 mL × 3). The organic phase was washed with saturated brine (50 mL), dried over anhydrous sodium sulfate, filtered, and concentrated under reduced pressure. The crude product was purified by preparative reversed-phase chromatography to give *tert-*butyl (3-((*R*)-1-((6-((1*R*,4*R*)-4-(4-((3-(4-chloro-3-(2,4-dioxotetrahydropyrimidin-1(2*H*)-yl)benzoyl)-3-azaspiro[5.5]undecan-9-yl)methyl)piperazine-1-carbonyl)cyclohexyl)-7-(2-methoxyethoxy)-2-methylquinazolin-4-yl)amino)ethyl)-5-(trifluoromethyl)phenyl)carboxylate.

LC-MS: (ESI, m/z): [M+H] ⁺=1130.4.

### Step 11: preparation of 1-(5-(9-((4-((1R,4R)-4-(4-(((R)-1-(3-amino-5-(trifluoromethyl)phenyl)ethyl)amino)-7-(2-methoxyethoxy)-2-methyl quin azolin-6-yl)cyclohexane-1 -carbonyl)piperazin-1 -yl)methyl)-3 - azaspiro[5.5]undecane-3-carbonyl)-2-chlorophenyl)dihydropyrimidine-2,4(1H,3H)-dione

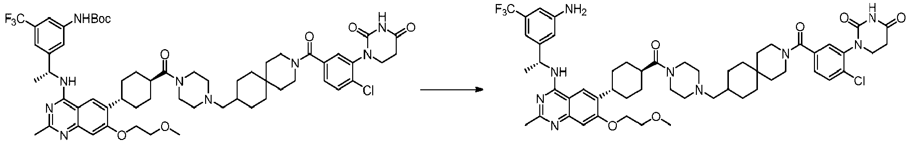

tert-Butyl (3-((*R*)-1-((6-((1*R*,4*R*)-4-(4-((3-(4-chloro-3-(2,4-dioxotetrahydropyrimidin-1(2*H*)-yl)benzoyl)-3-azaspiro[5.5]undecan-9-yl)methyl)piperazine-1-carbonyl)cyclohexyl)-7-(2-methoxyethoxy)-2-methylquinazolin-4-yl)amino)ethyl)-5-(trifluoromethyl)phenyl)carboxylate (80 mg, 0.07 mmol) was dissolved in DCM (5 mL), and TFA (1 mL) was added. The reaction liquid was stirred at room temperature for 1 h and concentrated under reduced pressure. The residue was dissolved with MeOH/DCM (1:9, 20 mL), and a saturated aqueous sodium bicarbonate solution (20 mL) was added to adjust the pH to neutrality. The resulting mixture was stirred for 10 min, extracted with MeOH/DCM (1:9, 20 mL × 3), dried over anhydrous sodium sulfate, filtered, and concentrated under reduced pressure. The crude product was purified by preparative reversed-phase chromatography to give 1-(5-(9-((4-((1*R*,4*R*)-4-(4-(((*R*)-1-(3-amino-5-(trifluoromethyl)phenyl)ethyl)amino)-7-(2-methoxyethoxy)-2-methylquinazolin-6-yl)cyclohexane-1-carbonyl)piperazin-1-yl)methyl)-3-azaspiro[5.5]undecane-3-carbonyl)-2-chlorophenyl)dihydropyrimidine-2,4(1/7,3//)-dione.

LC-MS: (ESI, m/z): [M+H] ⁺=1030.5.

¹H NMR (400 MHz, DMSO-*d*₆) δ 10.51 (s, 1H), 8.12 (d, *J* = 7.7 Hz, 1H), 8.05 (s, 1H), 7.63 (d, *J* = 8.2 Hz, 1H), 7.55 (d, *J* = 1.8 Hz, 1H), 7.39 (d, *J* = 8.2 Hz, 1H), 6.98 (s, 1H), 6.87 (s, 1H), 6.85 (s, 1H), 6.70 (s, 1H), 5.61 - 5.48 (m, 3H), 4.22-4.17 (m, 2H), 3.75 (d, *J* = 4.6 Hz, 3H), 3.65 - 3.52 (m, 3H), 3.51-3.41 (m, 4H), 3.36 (s, 3H), 3.02-2.88 (m, 1H), 2.79-2.71 (m, 2H), 2.68-2.60 (m, 1H), 2.29-2.38 (m, 5H), 2.28-2.21 (m, 2H), 2.16-2.06 (m, 2H), 1.95-1.85 (m, 2H), 1.84-1.80 (m, 2H), 1.76-1.19 (m, 18H), 1.17-0.95 (m, 4H).

### Example 44: 1-(5-(4-((2-(1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)ethoxy)methyl)piperidine-1-carbonyl)-2-fluorophenyl)dihydropyrimidine-2,4(1H,3H)-dione

### Step 1: preparation of benzyl 4-((2-(1-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carbonyl)piperidin-4-yl)ethoxy)methyl)piperidine-1 -carboxylate

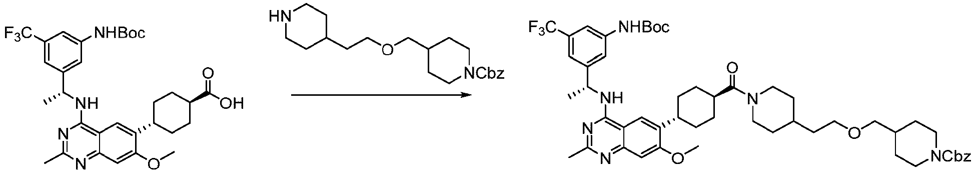

(1*R*,4*R*)-4-(4-(((*R*)-1-(3-((*tert*-Butoxycarbonyl)amino)-5-(trifluoromethyl)phenyl)ethyl)amino)-7-methoxy-2-methylquinazolin-6-yl)cyclohexane-1-carboxylic acid (100 mg, 0.17 mmol) was dissolved in DMF (8 mL), and benzyl 4-((2-(piperidin-4-yl)ethoxy)methyl)piperidine-1-carboxylate (72 mg, 0.2 mmol), HATU (90 mg, 0.23 mmol), and DIEA (64 mg, 0.5 mmol) were separately added. The reaction liquid was stirred at room temperature for 5 h. Water (50 mL) was added to the reaction liquid, and the resulting mixture was extracted with ethyl acetate (30 mL × 3). The organic phase was washed with saturated brine (50 mL), dried over anhydrous sodium sulfate, filtered, and concentrated under reduced pressure. The crude product was purified by column chromatography (methanol:dichloromethane = 1:9) to give benzyl 4-((2-(1-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carbonyl)piperidin-4-yl)ethoxy)methyl)piperidine-1 -carboxylate.

LC-MS: (ESI, *m*/*z):* [M+H] ⁺=945.5.

### Step 2: preparation of tert-butyl (3-((R)-1-((7-methoxy-2-methyl-6-((1R,4R)-4-(4-(2-(piperidin-4-ylmethoxy)ethyl)piperidine-1-carbonyl)cyclohexyl)quinolinozol-4-yl)amino)ethyl)-5 - (trifluoromethyl)phenyl)carboxylate

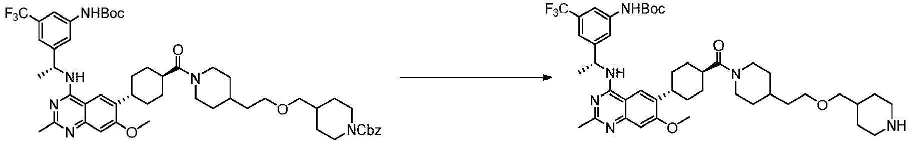

Benzyl 4-((2-(1-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carbonyl)piperidin-4-yl)ethoxy)methyl)piperidine-1-carboxylate (100 mg, 0.11 mmol) was dissolved with ethyl acetate (8 mL), and Pd(OH)₂/C (50 mg, 0.14 mmol) was added. The reaction liquid was heated to 70 °C under hydrogen atmosphere and stirred overnight. The reaction liquid was cooled to room temperature and filtered, and the filtrate was concentrated under reduced pressure to give *tert-butyl* (3-((*R*)-1-((7-methoxy-2-methyl-6-((1R,4R)-4-(4-(2-(piperidin-4-ylmethoxy)ethyl)piperidine-1-carbonyl)cyclohexyl)quinolinazol-4-yl)amino)ethyl)-5-(trifluoromethyl)phenyl)carboxylate.

LC-MS: (ESI, *m*/*z):* [M+H] ⁺=811.5.

### Step 3: preparation of tert-butyl (3-((R)-1-((6-((1R,4R)-4-(4-(2-((1-(3-(2,4-dioxotetrahydropyrimidin-1(2H)-yl)-4-fluorobenzoyl)piperidin-4-yl)methoxy)ethyl)piperidine-1-carbonyl)cyclohexyl)-7-methoxy-2-methylquinazolin-4-yl)amino)ethyl)-5-(trifluoromethyl)phenyl)carboxylate

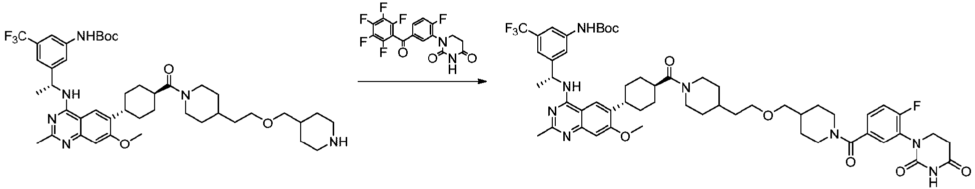

tert-Butyl (3-((R)-1-((7-methoxy-2-methyl-6-((1R,4R)-4-(4-(2-(piperidin-4-ylmethoxy)ethyl)piperidine-1-carbonyl)cyclohexyl)quinolinozol-4-yl)amino)ethyl)-5-(trifluoromethyl)phenyl)carboxylate (80 mg, 0.098 mmol) was dissolved with DMSO (8 mL), and 1-(2-fluoro-5-(pentafluorobenzoyl)phenyl)dihydropyrimidine-2,4(1*H*,3*H*)-dione (40 mg, 0.098 mmol) and DIEA (40 mg, 0.28 mmol) were added. The reaction liquid was stirred at room temperature for 2 h. Water (100 mL) was added to the reaction liquid, and the resulting mixture was extracted with ethyl acetate (30 mL × 3). The organic phase was washed with saturated brine (50 mL), dried over anhydrous sodium sulfate, filtered, and concentrated under reduced pressure. The crude product was purified by column chromatography (methanol:dichloromethane = 1:9) to give tert-butyl (3-((R)-1-((6-((1R,4R)-4-(4-(2-((1-(3-(2,4-dioxotetrahydropyrimidin-1(2*H*)-yl)-4-fluorobenzoyl)piperidin-4-yl)methoxy)ethyl)piperidine-1-carbonyl)cyclohexyl)-7-methoxy-2-methylquinazolin-4-yl)amino)ethyl)-5-(trifluoromethyl)phenyl)carboxylate. LC-MS: (ESI, *m*/*z):* [M+H] ⁺=1045.3.

### Step 4: preparation of 1-(5-(4-((2-(1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)ethoxy)methyl)piperidine-1-carbonyl)-2-fluorophenyl)dihydropyrimidine-2,4(1H,3H)-dione

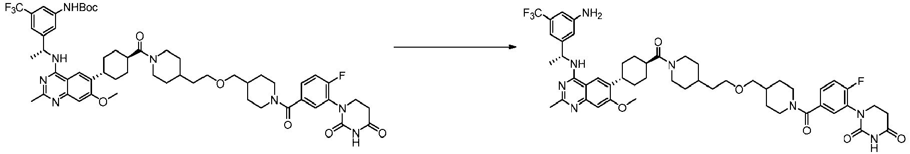

tert-Butyl (3-((*R*)-1-((6-((1*R*,4*R*)-4-(4-(2-((1-(3-(2,4-dioxotetrahydropyrimidin-1(2*H*)-yl)-4-fluorobenzoyl)piperidin-4-yl)methoxy)ethyl)piperidine-1-carbonyl)cyclohexyl)-7-methoxy-2-methylquinazolin-4-yl)amino)ethyl)-5-(trifluoromethyl)phenyl)carboxylate (50 mg, 0.047 mmol) was dissolved with dichloromethane (5 mL), and trifluoroacetic acid (2 mL) was added. The reaction liquid was stirred at room temperature for 2 h. The reaction liquid was directly concentrated under reduced pressure. The crude product was purified by Prep-HPLC to give 1-(5-(4-((2-(1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)ethoxy)methyl)piperidine-1-carbonyl)-2-fluorophenyl)dihydropyrimidine-2,4(1*H*,3*H*)-dione.

LC-MS: (ESI, *m*/*z):* [M+H] ⁺=945.4.

¹H NMR (400 MHz, MeOD) δ 10.52 (s, 1H), 8.19 - 7.99 (m, 2H), 7.49 (d, *J* = 7.9 Hz, 1H), 7.37 (d, *J* = 8.3 Hz, 2H), 6.99 (s, 1H), 6.87 (s, 1H), 6.85 (s, 1H), 6.70 (s, 1H), 5.65 - 5.47 (m, 3H), 4.50-4.33 (m, 2H), 3.96-3.85 (m, 4H), 3.74 (t, *J=* 6.7 Hz, 2H), 3.41 (t, *J* = 6.4 Hz, 2H), 3.23 (d, *J* = 6.2 Hz, 2H), 3.08 - 2.89 (m, 3H), 2.72 (t, *J* = 6.7 Hz, 2H), 2.69-2.60 (m, 1H), 2.40-2.30 (m, 4H), 1.99-1.37 (m, 22H), 1.16-0.94 (m, 4H).

### Example 45: 1-(5-(4-((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)methoxy)piperidine-1-carbonyl-2,2,6,6-d₄)-2-chlorophenyl)dihydropyrimidine-2,4(1H,3H)-dione

### Step 1: preparation of tert-butyl 4-((1-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carbonyl)piperidin-4-yl)methoxy)piperidine-1-carboxylate-2,2,6,6-d₄

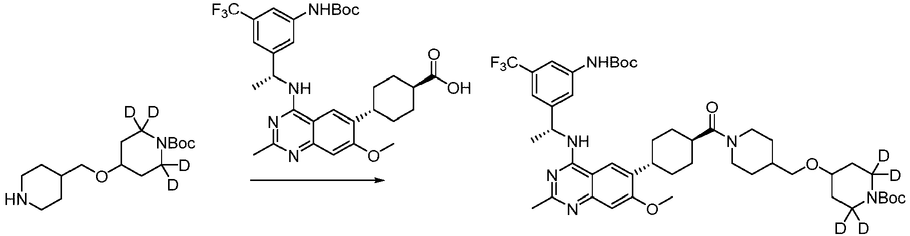

tert-Butyl 4-(piperidin-4-ylmethoxy)piperidine-1-carboxylate-2,2,6,6-*d*₄ (150 mg, 0.50 mmol) was dissolved with DMF (5 mL), and (1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carboxylic acid (150 mg, 0.25 mmol), HATU (123 mg, 0.32 mmol), and DIEA (161 mg, 1.25 mmol) were added. The reaction liquid was stirred at room temperature for 2 h. Water (50 mL) was added, and the resulting mixture was stirred for 5 min and extracted with ethyl acetate (30 mL × 3). The organic phase was washed with saturated brine (50 mL), dried over anhydrous sodium sulfate, filtered, and concentrated under reduced pressure. The crude product was purified by column chromatography (MeOH:DCM = 1:9) to give tert-butyl 4-((1-((1*R*,4*R*)-4-(4-(((*R*)-1-(3-((*tert*-butoxycarbonyl)amino)-5-(trifluoromethyl)phenyl)ethyl)amino)-7-methoxy-2-methylquinazolin-6-yl)cyclohexane-1-carbonyl)piperidin-4-yl)methoxy)piperidine-1-carboxylate-2,2,6,6-*d*₄.

LC-MS: (ESI, *m*/*z):* [M+H] ⁺=887.4.

### Step 2: preparation of ((1R,4R)-4-(4-(((R)-1-(3-amino-5-(trifluoromethyl)phenyl)ethyl)amino)-7-methoxy-2-methylquinazolin-6-yl)cyclohexyl)(4-(((piperidin-4-yl-2,2,6,6-d₄)oxy)methyl)piperidin-1-yl)methanone

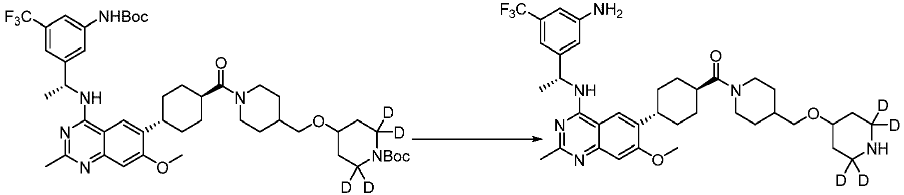

tert-Butyl 4-((1-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carbonyl)piperidin-4-yl)methoxy)piperidine-1-carboxylate-2,2,6,6-*d*₄ (200 mg, 0.23 mmol) was dissolved in DCM (5 mL), and TFA (1 mL) was added. The reaction liquid was stirred at room temperature for 2 h and concentrated under reduced pressure to give ((1R,4R)-4-(4-(((R)-1-(3-amino-5-(trifhioromethyl)phenyl)ethyl)amino)-7-methoxy-2-methylquinazolin-6-yl)cyclohexyl)(4-(((piperidin-4-yl-2,2,6,6-*d*₄)oxy)methyl)piperidin-1-yl)methanone, which was directly used in the next step.

LC-MS: (ESI, *m*/*z):* [M+H] ⁺=687.4.

### Step 3: preparation of 1-(5-(4-((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)methoxy)piperidine-1-carbonyl-2,2,6,6-d₄)-2-chlorophenyl)dihydropyrimidine-2,4(1H,3H)-dione

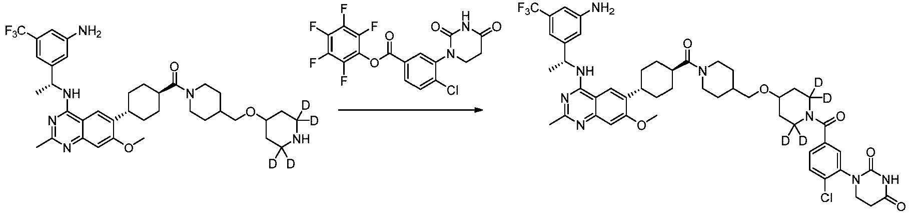

((1R,4R)-4-(4-(((R)-1-(3-Amino-5-(trifluoromethyl)phenyl)ethyl)amino)-7-methoxy-2-methylquinazolin-6-yl)cyclohexyl)(4-(((piperidin-4-yl-2,2,6,6-*d*₄)oxy)methyl)piperidin-1-yl)methanone (200 mg, 0.29 mmol) was dissolved in DMSO (5 mL), and DIEA (149 mg, 1.15 mmol) and pentafluorophenyl 4-chloro-3-(2,4-dioxotetrahydropyrimidin-1(2*H*)-yl)benzoate (100 mg, 0.23 mmol) were added. The reaction liquid was stirred at room temperature for 2 h. Water (50 mL) was added, and the resulting mixture was stirred for 5 min and extracted with ethyl acetate (30 mL × 3). The organic phase was washed with saturated brine (50 mL), dried over anhydrous sodium sulfate, filtered, and concentrated under reduced pressure. The crude product was purified by preparative reversed-phase chromatography to give 1-(5-(4-((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)methoxy)piperidine-1-carbonyl-2,2,6,6-*d*₄)-2-chlorophenyl)dihydropyrimidine-2,4(1*H*,3*H*)-dione.

LC-MS: (ESI, *m*/*z):* (ESI, *m*/*z):* [M+H] ⁺=937.5.

¹H NMR (400 MHz, DMSO-*d*₆) δ 10.50 (s, 1H), 8.13 (s, 1H), 8.09 (s, 1H), 7.63 (d, *J* = 8.2 Hz, 1H), 7.57 (d, *J* = 1.9 Hz, 1H), 7.40 (dd, *J* = 8.2, 2.0 Hz, 1H), 6.99 (s, 1H), 6.88 (s, 1H), 6.85 (s, 1H), 6.70 (s, 1H), 5.62 - 5.50 (m, 3H), 4.42 (d, *J* = 11.5 Hz, 1H), 4.0 - 3.92 (m, 4H), 3.74 (s, 1H), 3.67 - 3.47 (m, 2H), 3.29 (d, *J* = 4.5 Hz, 2H), 3.09 - 2.87 (m, 2H), 2.76-2.73 (m, 2H), 2.67-2.63 (m, 1H), 2.36 (s, 3H), 1.94 - 1.50 (m, 17H), 1.50 - 1.38 (m, 2H), 1.16 - 0.91 (m, 2H).

### Example 46: 3-(2-chloro-5-(4-((2-(1-(4-(4-(((R)-1-(4-(2-chloro-6-((dimethylamino)methyl)phenyl)thien-2-yl)ethyl)amino)-7-methoxy-2-methylquinazolin-6-yl)cyclohex-3-ene-1-carbonyl)piperidin-4-yl)ethoxy)methyl)piperidine-1-carbonyl)phenyl)piperidine-2,6-dione

### Step 1: preparation of methyl 4-(4-(((R)-1-(4-bromothien-2-yl)ethyl)amino)-7-methoxy-2-methylquinazolin-6-yl)cyclohex-3-ene-1-carboxylate

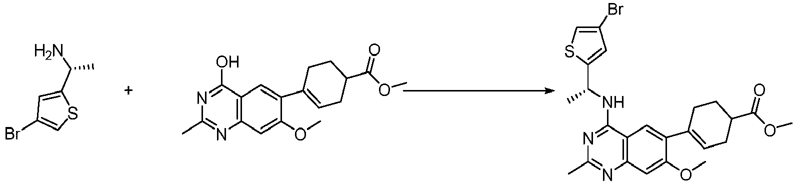

(R)-1-(4-Bromo-2-yl)ethan-1-amine (607 mg, 3 mmol) was dissolved in DMF (5 mL), and BOP (574.6 mg, 1.3 mmol) and DBU (456.0 mg, 3.0 mmol) were added. The reaction liquid was stirred at room temperature for 0.5 h. Methyl 4-(4-hydroxy-7-methoxy-2-methylquinazolin-6-yl)cyclohex-3-ene-1-carboxylate (328 mg, 1 mmol) was added to the reaction liquid, and the resulting mixture was heated to 70 °C and reacted for 16 h. The reaction liquid was diluted with water (50 mL) and extracted with ethyl acetate (30 mL × 3). The upper organic phases were combined, washed with saturated brine, dried over anhydrous Na₂SO₄, filtered, and concentrated under reduced pressure. The crude product was purified by column chromatography (MeOH:DCM = 1:9) to give methyl 4-(4-(((*R*)-1-(4-bromothien-2-yl)ethyl)amino)-7-methoxy-2-methylquinazolin-6-yl)cyclohex-3-ene-1-carboxylate.

LC-MS: (ESI, *m*/*z):* [M+H] ⁺= 516.1.

### Step 2: preparation of methyl 4-(4-(((R)-1-(4-(2-chloro-6-benzaldehyde)thien-2-yl)ethyl)amino)-7-methoxy-2-methylquinazolin-6-yl)cyclohex-3 -ene-1 -carboxylate

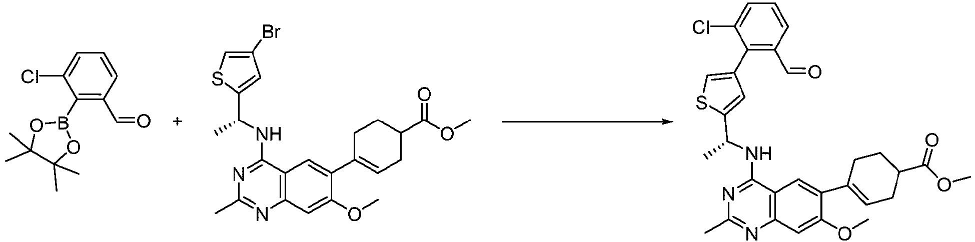

3-Chloro-2-(4,4,5,5-tetramethyl-1,3,2-dioxaborolan-2-yl)benzaldehyde (206 mg, 0.77 mmol) and methyl 4-(4-(((*R*)-1-(4-bromothien-2-yl)ethyl)amino)-7-methoxy-2-methylquinazolin-6-yl)cyclohex-3-ene-1-carboxylate (400 mg, 0.77 mmol) were dissolved in DMF/H₂O (3 mL/0.3 mL), and Pd(dppf)Cl₂ (100 mg) and K₂CO₃ (214 mg, 1.55 mmol) were added. The reaction liquid was purged with nitrogen and stirred at 100 °C for 2 h under nitrogen atmosphere. After the reaction was completed, the reaction liquid was diluted with water (50 mL) and extracted with ethyl acetate (30 mL × 3). The organic phases were combined and washed with saturated brine, dried over anhydrous Na₂SO₄, filtered, and concentrated under reduced pressure. The crude product was purified by column chromatography (MeOH:DCM = 1:9) to give methyl 4-(4-(((R)-1-(4-(2-chloro-6-benzaldehyde)thien-2-yl)ethyl)amino)-7-methoxy-2-methylquinazolin-6-yl)cyclohex-3-ene-1-carboxylate.

LC-MS: (ESI, *m*/*z):* [M+H] ⁺= 576.3.

### Step 3: preparation of methyl 4-(4-(((R)-1-(4-(2-chloro-6-((dimethylamino)methyl)phenyl)thien-2-yl)ethyl)amino)-7-methoxy-2-methylquinazolin-6-yl)cyclohex-3-ene-1-carboxylate

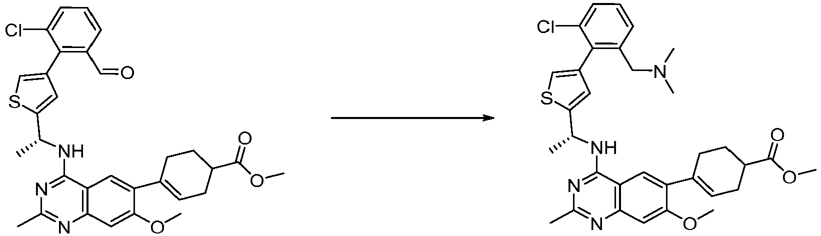

Methyl 4-(4-(((R)-1-(4-(2-chloro-6-benzaldehyde)thien-2-yl)ethyl)amino)-7-methoxy-2-methylquinazolin-6-yl)cyclohex-3-ene-1-carboxylate (130 mg, 0.22 mmol) was dissolved in 1,2-dichloroethane (5 mL), and sodium triacetoxyborohydride (143 mg, 0.68 mmol) was added. The reaction liquid was stirred at 80 °C for 1 h. The reaction liquid was diluted with water (50 mL) and extracted with dichloromethane (30 mL × 3). The organic phases were combined, dried over anhydrous Na₂SO₄, and concentrated under reduced pressure. The crude product was purified by column chromatography (MeOH:DCM = 1:19) to give methyl 4-(4-(((*R*)-1-(4-(2-chloro-6-((dimethylamino)methyl)phenyl)thien-2-yl)ethyl)amino)-7-methoxy-2-methylquinazolin-6-yl)cyclohex-3-ene-1-carboxylate.

LC-MS: (ESI, *m*/*z):* [M+H] ⁺= 605.3.

### Step 4: preparation of 4-(4-(((R)-1-(4-(2-chloro-6-((dimethylamino)methyl)phenyl)thien-2-yl)ethyl)amino)-7-methoxy-2-methylquinazolin-6-yl)cyclohex-3-ene-1-carboxylic acid

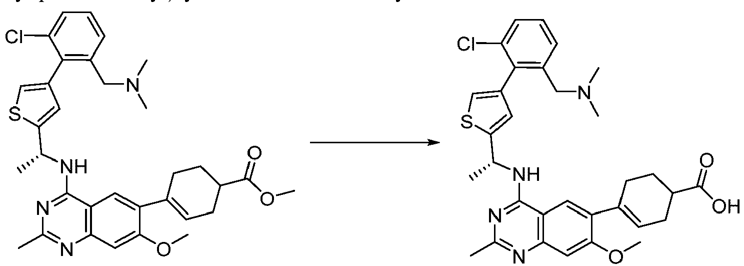

Methyl 4-(4-(((*R*)-1-(4-(2-chloro-6-((dimethylamino)methyl)phenyl)thien-2-yl)ethyl)amino)-7-methoxy-2-methylquinazolin-6-yl)cyclohex-3-ene-1-carboxylate (170 mg, 0.28 mmol) was dissolved with MeOH/H₂O (2 mL/0.2 mL), and LiOH (52 mg, 1.3 mmol) was added. The reaction liquid was heated to 60 °C and stirred for 1 h. The reaction liquid was cooled, adjusted to pH 3-4 with dilute hydrochloric acid (1.0 mol/L), adjusted to pH 7-8 with a saturated NaHCO₃ solution, and extracted with ethyl acetate (30 mL × 3). The organic phases were combined, dried over anhydrous Na₂SO₄, filtered, and concentrated under reduced pressure to give (*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohex-3-ene-1-carboxylic acid.

LC-MS: (ESI, *m*/*z):* [M+H] ⁺= 591.3.

### Step 5: preparation of 3-(2-chloro-5-(4-((2-(1-(4-(4-(((R)-1-(4-(2-chloro-6-((dimethylamino)methyl)phenyl)thien-2-yl)ethyl)amino)-7-methoxy-2-methylquinazolin-6-yl)cyclohex-3-ene-1-carbonyl)piperidin-4-yl)ethoxy)methyl)piperidine-1-carbonyl)phenyl)piperidine-2,6-dione

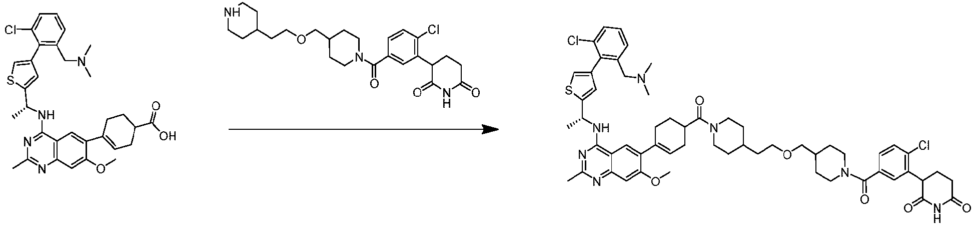

4-(4-(((*R*)-1-(4-(2-Chloro-6-((dimethylamino)methyl)phenyl)thien-2-yl)ethyl)amino)-7-methoxy-2-methylquinazolin-6-yl)cyclohex-3-ene-1-carboxylic acid (125 mg, 0.22 mmol) and 3-(2-chloro-5-(4-((2-(piperidin-4-yl)ethoxy)methyl)piperidine-1-carbonyl)phenyl)piperidine-2,6-dione (108 mg, 0.22 mmol) were dissolved with DMF (3 mL), and HATU (96 mg, 0.25 mmol) and DIEA (82 mg, 0.64 mmol) were added. The reaction liquid was stirred at room temperature for 1 h. The reaction liquid was diluted with water (50 mL) and extracted with ethyl acetate (30 mL × 3). The organic phase was washed with saturated brine, dried over anhydrous Na₂SO₄, filtered, and concentrated under reduced pressure. The crude product was purified by Prep-HPLC to give the product 3-(2-chloro-5-(4-((2-(1-(4-(4-(((*R*)-1-(4-(2-chloro-6-((dimethylamino)methyl)phenyl)thien-2-yl)ethyl)amino)-7-methoxy-2-methylquinazolin-6-yl)cyclohex-3-ene-1-carbonyl)piperidin-4-yl)ethoxy)methyl)piperidin-1-carbonyl)phenyl)piperidin-2,6-dione.

LC-MS: (ESI, *m*/*z*): [M+H]⁺=1049.3.

¹H NMR (400 MHz, DMSO-*d*₆) δ 10.50 (s, 1H), 8.41 (s, 1H), 8.05 (s, 1H), 7.63 (d, *J* = 8.2 Hz, 1H), 7.54 (s, 1H), 7.48 - 7.30 (m, 4H), 7.24 (s, 1H), 7.02 (s, 1H), 6.96 (s, 1H), 6.01 - 5.89 (m, 1H), 5.83 (s, 1H), 4.47-4.37 (m, 1H), 3.99-3.91 (m, 1H), 3.86 (s, 3H), 3.74 (s, 1H), 3.68-3.52 (m, 2H), 3.44-3.37 (m, 2H), 3.26-3.18 (m, 3H), 3.18-2.96 (m, 3H), 2.90-2.82 (m, 1H), 2.81-2.70 (m, 2H), 2.43 (s, 3H), 2.39-2.29 (m, 2H), 2.25-2.16 (m, 1H), 2.03 (s, 6H), 1.89-1.55 (m, 10H), 1.49-1.40 (m, 2H), 1.30-1.20 (m, 2H), 1.20 - 0.95 (m, 4H).

### Example 47: (1R,4R)-N-(3-(1-(4-chloro-3-(2,4-dioxotetrahydropyrimidin-1(2H)-yl)benzoyl)piperidin-4-yl)propyl)-4-(7-methoxy-2-methyl-4-(((R)-1-(4-(2-((methylamino)methyl)phenyl)thien-2-yl)ethyl)amino)quinazolin-6-yl)-N-methylcyclohexane-1-carboxamide

### Step 1: preparation of benzyl 4-(3-hydroxypropyl)piperidine-1-carboxylate

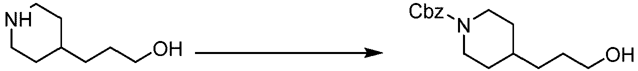

CbzCl (5.40 mL, 38.40 mmol) was added to a solution of 3-(piperidin-4-yl)propan-1-ol (5.00 g, 34.91 mmol) in THF (20 mL) at 0 °C. The reaction liquid was reacted for 1 h. After the reaction was completed, a saturated aqueous NaHCO₃ solution (50 mL) was added to the reaction liquid to quench the reaction, and the resulting mixture was extracted with ethyl acetate (100 mL × 3). The organic phases were combined, washed with brine (100 mL), dried over sodium sulfate, and filtered, and the filtrate was concentrated. The crude product was purified by silica gel column chromatography (MeOH:DCM = 1:100-1:10) to give benzyl 4-(3-hydroxypropyl)piperidine-1-carboxylate as a pale yellow oil.

LC-MS: (ESI, *m*/*z*): [M+H]⁺=278.2.

### Step 2: preparation of benzyl 4-(3-oxopropyl)piperidine-1-carboxylate

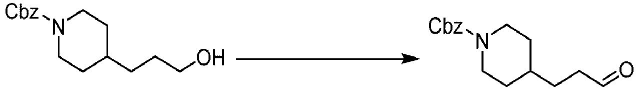

Dess-Martin (5.50 g, 13.0 mmol) was added to a solution of benzyl 4-(3-hydroxypropyl)piperidine-1-carboxylate (3.00 g, 10.8 mmol) in DCM (60 mL) at 0 °C for reaction for 1 h. After the reaction was completed, saturated Na₂S₂O₃ (50 mL) and saturated NaHCO₃ (50 mL) were added to the reaction liquid to quench the reaction, and the resulting mixture was extracted with DCM (80 mL × 3). The organic phases were combined, washed with brine (100 mL), dried over sodium sulfate, filtered, and concentrated by rotary evaporation. The crude product was purified by silica gel chromatography (EA:PE = 3:17-3:7) to give benzyl 4-(3-oxopropyl)piperidine-1-carboxylate. ¹H NMR (400 MHz, CDCl₃) δ 9.78 (s, 1H), 7.38-7.28 (m, 5H), 5.12 (s, 2H), 4.28-4.02 (m, 2H), 2.90-2.65 (m, 2H), 2.51-2.42 (m, 2H), 1.74-1.54 (m, 4H), 1.51-1.37 (m, 1H), 1.21-1.03 (m, 2H).

### Step 3: preparation of benzyl 4-(3-(methylamino)propyl)piperidine-1-carboxylate

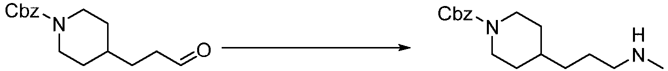

Methylamine (846 mg, 27.24 mmol) and AcOH (0.1 mL) were added to a solution of benzyl 4-(3-oxopropyl)piperidine-1-carboxylate (1.50 g, 5.45 mmol) in THF (10 mL). The reaction liquid was reacted at 30 °C for 4 h. Then NaBH₃CN (684 mg, 10.90 mmol) was added, and the resulting mixture was successively reacted for 16 h. After the reaction was completed, saturated NaHCO₃ (50 mL) was added to the reaction liquid to quench the reaction, and the resulting mixture was extracted with ethyl acetate (100 mL × 3). The organic phases were combined, washed with brine (100 mL), dried over sodium sulfate, filtered, and concentrated by rotary evaporation. The crude product was purified by silica gel chromatography (NH₃/MeOH:DCM = 1:9-2:8) to give benzyl 4-(3-(methylamino)propyl)piperidine-1 -carboxylate.

LC-MS: (ESI, *m*/*z*): [M+H] ⁺=291.7.

### Step 4: preparation of benzyl 4-(3-((tert-butoxycarbonyl)(methyl)amino)propyl)piperidine-1-carboxylate

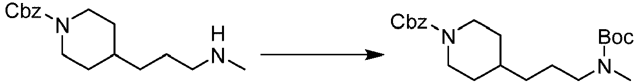

(Boc)₂O (1.2 mL, 5.10 mmol) was added to a solution of benzyl 4-(3-(methylamino)propyl)piperidine-1-carboxylate (295 mg, 1.02 mol) in DCM (3 mL). The reaction liquid was reacted at room temperature for 16 h. After the reaction was completed, water (50 mL) was added to the reaction liquid to quench the reaction, and the resulting mixture was extracted with ethyl acetate (50 mL × 3). The organic phases were combined, washed with brine (100 mL), dried over sodium sulfate, filtered, and concentrated by rotary evaporation. The crude product was purified by silica gel chromatography (NH₃/MeOH:DCM = 1:19-1:9) to give benzyl 4-(3-((*tert-*butoxycarbonyl)(methyl)amino)propyl)piperidine-1-carboxylate.

LC-MS: (ESI, *m*/*z*): [M-100+H] ⁺=291.2.

### Step 5: preparation of tert-butyl methyl(3-piperidin-4-yl)propyl)carboxylate

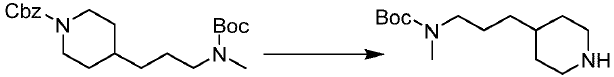

Pd/C (80 mg) was added to a solution of benzyl 4-(3-((*tert*-butoxycarbonyl)(methyl)amino)propyl)piperidine-1-carboxylate (291 mg, 0.75 mmol) in MeOH (5 mL). The reaction liquid was purged three times with hydrogen and reacted at room temperature for 16 h. The reaction liquid was concentrated by rotary evaporation to give the crude product *tert*-butyl methyl(3-piperidin-4-yl)propyl)carboxylate, which was used in the next step without further purification.

LC-MS: (ESI, *m*/*z*): [M+H] ⁺=257.2.

### Step 6: preparation of tert-butyl (3-(1-(4-chloro-3-(2,4-dioxotetrahydropyrimidin-1(2H)-yl)benzoyl)piperidin-4-yl)propyl)(methyl)carboxylate

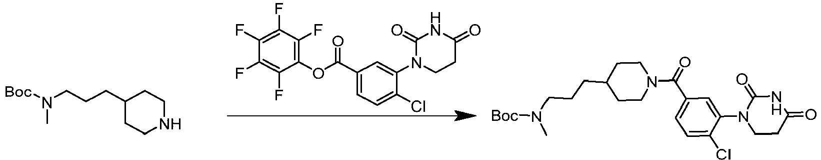

DIEA (232 mg, 1.8 mmol) and pentafluorophenyl 4-chloro-3-(2,4-dioxotetrahydropyrimidin-1(2*H*)-yl)benzoate (381 mg, 0.88 mmol) were added to a solution of *tert*-butyl methyl(3-(piperidin-4-yl)propyl)carbamate (150 mg, 0.59 mmol) in DMSO (2 mL). The reaction liquid was reacted at room temperature for 16 h. After the reaction was completed, water (50 mL) was added to the reaction liquid to quench the reaction, and the resulting mixture was extracted with ethyl acetate (50 mL × 3). The organic phases were combined, washed with brine (100 mL), dried over sodium sulfate, filtered, and concentrated by rotary evaporation. The crude product was purified by silica gel chromatography (NH₃/MeOH:DCM = 1:19-1:9) to give the target compound *tert*-butyl (3-(1-(4-chloro-3-(2,4-dioxotetrahydropyrimidin-1(2*H*)-yl)benzoyl)piperidin-4-yl)propyl)(methyl)carboxylate.

LC-MS: (ESI, *m*/*z*): [M+Na] ⁺=529.1.

### Step 7: preparation of 1-(2-chloro-5-(4-(3-(methylamino)propyl)piperidine-1-carbonyl)phenyl)dihydropyrimidine-2,4(1H,3H)-dione

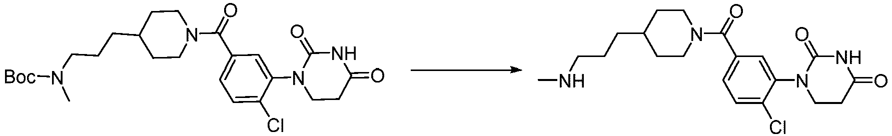

The compound *tert*-butyl (3-(1-(4-chloro-3-(2,4-dioxotetrahydropyrimidin-1(2*H*)-yl)benzoyl)piperidin-4-yl)propyl)(methyl)carboxylate (256 mg, 0.50 mmol) was dissolved in DCM (2 mL), and TFA (1 mL) was added at room temperature. The reaction liquid was reacted for 0.5 h. The reaction liquid was concentrated to give the crude product 1-(2-chloro-5-(4-(3-(methylamino)propyl)piperidine-1-carbonyl)phenyl)dihydropyrimidine-2,4(1*H*,3*H*)-dione, which was used in the next step without further purification.

LC-MS: (ESI, *m*/*z*): [M+H] ⁺=407.0.

### Step 8: preparation of tert-butyl (2-(5-((R)-1-((6-((1R,4R)-4-((3-(1-(4-chloro-3-(2,4-dioxotetrahydropyrimidin-1(2H)-yl)benzoyl)piperidin-4-yl)propyl)(methyl)carbonyl)cyclohexyl)-7-methoxy-2-methylquinazolin-4-yl)amino)ethyl)thien-3-yl)phenyl)(methyl)carboxylate

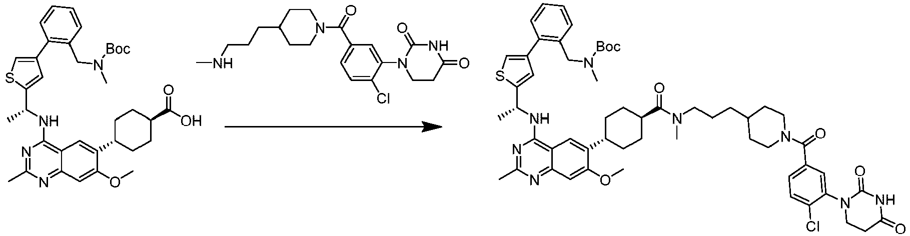

1-(2-Chloro-5-(4-(3-(methylamino)propyl)piperidine-1-carbonyl)phenyl)dihydropyrimidine-2,4(1*H*,3*H*)-dione (216 mg, 0.53 mmol), TCFH (249 mg, 0.89 mmol), and NMI (146 mg, 1.78 mmol) were added to a solution of the compound (1*R*,4*R*)-4-(4-(((*R*)-1-(4-(2-(((*tert*-butoxycarbonyl)(methyl)amino)methyl)phenyl)thien-2-yl)ethyl)amino)-7-methoxy-2-methylquinazolin-6-yl)cyclohexane-1-carboxylic acid (229 mg, 0.36 mmol) in DMF (3 mL). The reaction liquid was reacted at room temperature for 16 h. After the reaction was completed, water (20 mL) was added to the reaction liquid to quench the reaction, and the resulting mixture was extracted with ethyl acetate (20 mL × 3). The organic phases were combined, washed with brine (50 mL), dried over sodium sulfate, filtered, and concentrated under vacuum. The crude product was purified by silica gel chromatography (NH₃/MeOH:DCM = 1:19-1:9) to give the target compound *tert*-butyl (2-(5-((*R*)-1-((6-((1*R*,4*R*)-4-((3-(1-(4-chloro-3-(2,4-dioxotetrahydropyrimidin-1(2*H*)-yl)benzoyl)piperidin-4-yl)propyl)(methyl)carbonyl)cyclohexyl)-7-methoxy-2-methylquinazolin-4-yl)amino)ethyl)thien-3-yl)phenyl)(methyl)carboxylate.

LC-MS: (ESI, *m*/*z*): [M+H]⁺=1033.2.

### Step 9: preparation of (1R,4R)-N-(3-(1-(4-chloro-3-(2,4-dioxotetrahydropyrimidin-1(2H)-yl)benzoyl)piperidin-4-yl)propyl)-4-(7-methoxy-2-methyl-4-(((R)-1-(4-(2-((methylamino)methyl)phenyl)thien-2-yl)ethyl)amino)quinazolin-6-yl)-N-methylcyclohexane-1-carboxamide

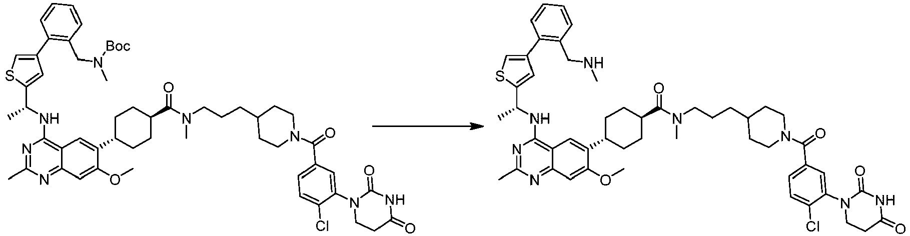

TFA (1 mL) was added to a solution of the compound *tert*-butyl (2-(5-((*R*)-1-((6-((1*R*,4*R*)-4-((3-(1-(4-chloro-3-(2,4-dioxotetrahydropyrimidin-1(2*H*)-yl)benzoyl)piperidin-4-yl)propyl)(methyl)carbonyl)cyclohexyl)-7-methoxy-2-methylquinazolin-4-yl)amino)ethyl)thien-3-yl)phenyl)(methyl)carboxylate (107 mg, 0.10 mmol) in DCM (2 mL) at room temperature. The reaction liquid was reacted for 0.5 h. The reaction liquid was concentrated to give the crude product, which was purified by high performance liquid chromatography to give the target compound (1*R*,4*R*)-*N-*(3-(1-(4-chloro-3-(2,4-dioxotetrahydropyrimidine-1(2*H*)-yl)benzoyl)piperidin-4-yl)propyl)-4-(7-methoxy-2-methyl-4-(((R)-1-(4-(2-((methylamino)methyl)phenyl)thien-2-yl)ethyl)amino)quinazolin-6-yl)-N-methylcyclohexane-1-carboxamide.

LC-MS: (ESI, *m*/*z*): [M+H]⁺=933.4.

¹H NMR (400 MHz, CD₃OD) δ 7.99 (s, 1H), 7.66-7.58 (m, 1H), 7.54-7.48 (m, 1H), 7.43-7.36 (m, 2H), 7.33-7.26 (m, 3H), 7.18 (d, *J* = 1.2 Hz, 1H), 7.15-7.11 (m, 1H), 7.01 (m, 1H), 6.08-6.01 (m, 1H), 4.65-4.53 (m, 1H), 3.95 (s, 3H), 3.81-3.69 (m, 5H), 3.48-3.34 (m, 2H), 3.16-3.04 (m, 4H), 2.95-2.68 (m, 5H), 2.52 (s, 3H), 2.22 (s, 3H), 2.00-1.57 (m, 16H), 1.35-1.11 (m, 4H).

### Example 48: 1-(2-chloro-5-(4-((2-(1-((1R,4R)-4-(4-(((R)-1-(4-(2-((dimethylamino)methyl))))phenyl)thien-2-yl)ethyl)amino)-7-methoxy-2-methylquinazolin-6-yl)cyclohexane-1-carbonyl)piperidin-4-yl)ethoxy)methyl)piperidine-1-carbonyl)phenyl)dihydropyrimidine-2,4(1H,3H)-dione

### Step 1: preparation of benzyl 4-(2-((1-(tert-butoxycarbonyl)piperidin-4-yl)methoxy)ethyl)piperidine-1-carboxylate

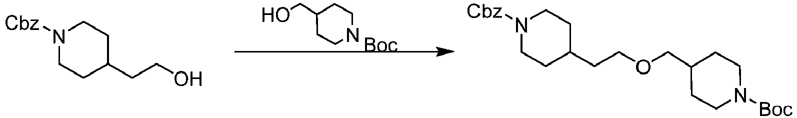

The compound benzyl 4-(2-hydroxyethyl)piperidine-1-carboxylate (400 mg, 1.52 mmol) was dissolved in THF (5 mL), and triethylamine (230 mg, 2.28 mmol) was added. The reaction liquid was cooled to 0 °C, and methanesulfonyl chloride (208 mg, 1.82 mmol) was added dropwise. After the addition, the reaction liquid was heated to room temperature and reacted for 2 h. An aqueous ammonium chloride solution was added to quench the reaction, and the resulting mixture was extracted with ethyl acetate (20 mL × 3). The organic phases were combined and washed with saturated brine, dried over anhydrous sodium sulfate, filtered, and concentrated to give an intermediate. This intermediate was dissolved in tetrahydrofuran, and the resulting solution was added to a solution of potassium *tert*-butoxide (340 mg, 3.04 mmol) and the compound *tert*-butyl 4-(hydroxymethyl)piperidine-1-carboxylate (652 mg, 3.04 mmol) in THF (6 mL). The reaction liquid was reacted at 30 °C for 16 h. The reaction liquid was diluted with water and extracted with ethyl acetate (20 ml × 3). The organic phases were combined, washed with saturated brine, dried over anhydrous sodium sulfate, filtered, concentrated, and purified by column chromatography (PE:EA = 5:1) to give the target product benzyl 4-(2-((1-(*tert*-butoxycarbonyl)piperidin-4-yl)methoxy)ethyl)piperidine-1 -carboxylate.

LC-MS: (ESI, *m*/*z*): [M+Na] ⁺=483.2.

### Step 2: preparation of tert-butyl 4-((2-(piperidin-4-yl)ethoxy)methyl)piperidine-1-carboxylate

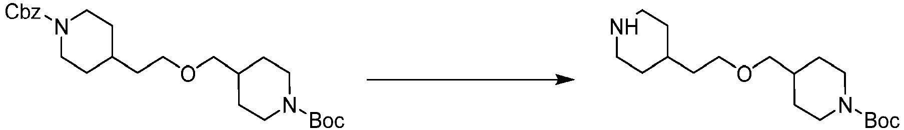

The compound benzyl 4-(2-((1-(*tert*-butoxycarbonyl)piperidin-4-yl)methoxy)ethyl)piperidine-1-carboxylate was dissolved in methanol (180 mg, 0.39 mmol), and palladium on carbon (70 mg) was added. The reaction liquid was purged three times under hydrogen (1 atmosphere pressure) atmosphere, reacted at room temperature for 16 h, and filtered through celite. The filtrate was concentrated to give the crude product *tert*-butyl 4-((2-(piperidin-4-yl)ethoxy)methyl)piperidine-1-carboxylate, which was directly used in the next step.

### Step 3: preparation of tert-butyl 4-((2-(1-((1R,4R)-4-(4-(((R)-1-(4-(2-((dimethylamino)methyl)phenyl)thien-2-yl)ethyl)amino)-7-methoxy-2-methylquinazolin-6-yl)cyclohexane-1-carbonyl)piperidin-4-yl)ethoxy)methyl)piperidine-1 -carboxylate

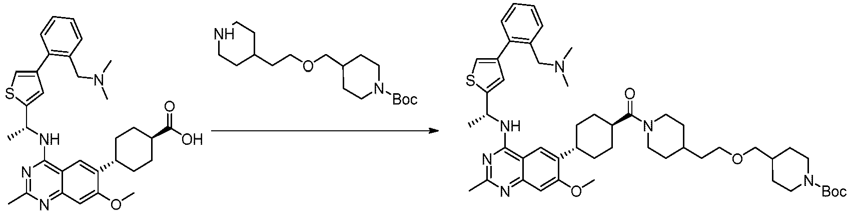

The compound tert-butyl 4-((2-(piperidin-4-yl)ethoxy)methyl)piperidine-1-carboxylate (114 mg, 0.35 mmol), TcFH (162 mg, 0.58 mmol), and NMI (94 mg, 1.15 mmol) were added to a solution of the compound (1*R*,4*R*)-4-(4-(((*R*)-1-(4-(2-((dimethylamino)methyl)phenyl)thien-2-yl)ethyl)amino)-7-methoxy-2-methylquinazolin-6-yl)cyclohexane-1-carboxylic acid (130 mg, 0.23 mmol) in DMF (2 mL). The reaction liquid was reacted at room temperature for 16 h. After the reaction was completed, water (20 mL) was added to the reaction liquid to quench the reaction, and the resulting mixture was extracted with ethyl acetate (20 mL × 3). The organic phases were combined, washed with brine (50 mL), dried over sodium sulfate, filtered, and concentrated under vacuum. The crude product was purified by column chromatography (NH₃/MeOH:DCM = 1:19-1:9) to give the target product *tert*-butyl 4-((2-(1-((1*R*,4*R*)-4-(4-(((*R*)-1-(4-(2-((dimethylamino)methyl)phenyl)thien-2-yl)ethyl)amino)-7-methoxy-2-methylquinazolin-6-yl)cyclohexane-1-carbonyl)piperidin-4-yl)ethoxy)methyl)piperidine-1-carboxylate.

LC-MS: (ESI, *m*/*z*): [M+H] ⁺=867.3.

### Step 4: preparation of ((1R,4R)-4-(4-(((R)-1-(4-(2-((dimethylamino)methyl)phenyl)thien-2-yl)ethyl)amino)-7-methoxy-2-methylquinazolin-6-yl)cyclohexyl)(4-(2-(piperidin-4-ylmethoxy)ethyl)piperidin-1-yl)methanone

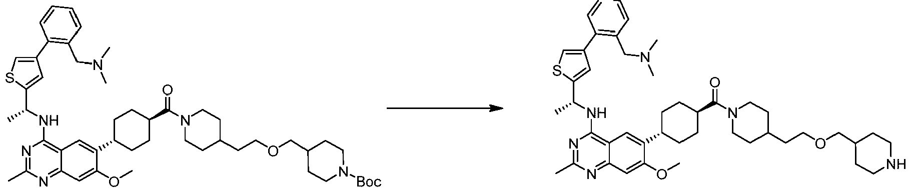

The compound *tert*-butyl 4-((2-(1-((1*R*,4*R*)-4-(4-(((*R*)-1-(4-(2-((dimethylamino)methyl)phenyl)thien-2-yl)ethyl)amino)-7-methoxy-2-methylquinazolin-6-yl)cyclohexane-1-carbonyl)piperidin-4-yl)ethoxy)methyl)piperidine-1-carboxylate (40 mg, 0.05 mmol) was dissolved in DCM (2 mL) at room temperature, and TFA (1 mL) was added. The reaction liquid was reacted at room temperature for 0.5 h. The reaction liquid was concentrated to give the crude product ((1*R*,4*R*)-4-(4-(((*R*)-1-(4-(2-((dimethylamino)methyl)phenyl)thien-2-yl)ethyl)amino)-7-methoxy-2-methylquinazolin-6-yl)cyclohexyl)(4-(2-(piperidin-4-ylmethoxy)ethyl)piperidin-1-yl)methanone, which was directly used in the next step without further purification.

LC-MS: (ESI, *m*/*z*): [M+H] ⁺=767.3.

### Step 5: preparation of 1-(2-chloro-5-(4-((2-(1-((1R,4R)-4-(4-(((R)-1-(4-(2-((dimethylamino)methyl))))phenyl)thien-2-yl)ethyl)amino)-7-methoxy-2-methylquinazolin-6-yl)cyclohexane-1-carbonyl)piperidin-4-yl)ethoxy)methyl)piperidine-1-carbonyl)phenyl)dihydropyrimidine-2,4(1H,3H)-dione

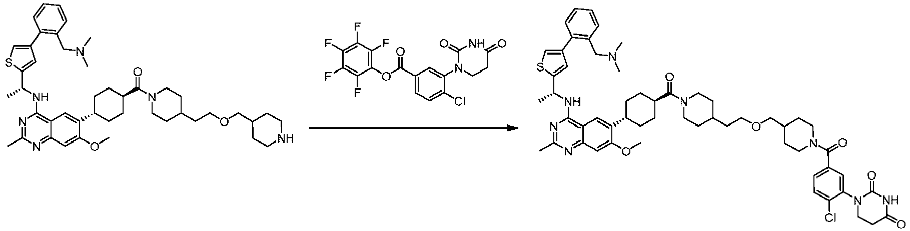

DIEA (103 mg, 0.80 mmol) and pentafluorophenyl 4-chloro-3-(2,4-dioxotetrahydropyrimidin-1(2*H*)-yl)benzoate (42 mg, 0.10 mmol) were added to a solution of ((1R,4R)-4-(4-(((R)-1-(4-(2-((dimethylamino)methyl)phenyl)thien-2-yl)ethyl)amino)-7-methoxy-2-methylquinazolin-6-yl)cyclohexyl)(4-(2-(piperidin-4-ylmethoxy)ethyl)piperidin-1-yl)methanone (50 mg, 0.07 mmol) in DMSO (2 mL). The reaction liquid was reacted at room temperature for 16 h. After the reaction was completed, water (10 mL) was added to the reaction liquid to quench the reaction, and the resulting mixture was extracted with ethyl acetate (10 mL × 3). The organic phases were combined, washed with brine (10 mL), dried over sodium sulfate, filtered, and concentrated by rotary evaporation. The crude product was purified by preparative chromatography to give 1-(2-chloro-5-(4-((2-(1-((1*R*,4*R*)-4-(4-(((*R*)-1-(4-(2-((dimethylamino)methyl))))phenyl)thien-2-yl)ethyl)amino)-7-methoxy-2-methylquinazolin-6-yl)cyclohexane-1-carbonyl)piperidin-4-yl)ethoxy)methyl)piperidine-1-carbonyl)phenyl)dihydropyrimidine-2,4(1*H*,3*H*)-dione. LC-MS: (ESI, *m*/*z*): [M+H] ⁺=1017.3.

¹H NMR (400 MHz, CD₃OD) δ 7.99 (s, 1H), 7.65-7.59 (m, 1H), 7.52 (s, 1H), 7.44-7.26 (m, 5H), 7.24-7.16 (m, 2H), 7.01 (s, 1H), 6.11-6.01 (m, 1H), 4.66-4.46 (m, 2H), 4.09-3.98 (m, 1H), 3.95 (s, 3H), 3.81-3.67 (m, 3H), 3.53-3.40 (m, 4H), 3.16-3.00 (m, 3H), 2.93-2.57 (m, 5H), 2.52 (s, 3H), 2.23-2.05 (m, 7H), 1.95-1.57 (m, 15H), 1.56-1.51 (m, 2H), 1.34-1.00 (m, 7H).

### Example 49: (1R,4R)-N-(3-(3-(4-chloro-3-(2,4-dioxotetrahydropyrimidin-1(2H)-yl)benzoyl)-3-azaspiro[5.5]undecan-9-yl)propyl)-4-(7-methoxy-2-methyl-4-(((R)-1-(4-(2-((methylamino)methyl)phenyl)thien-2-yl)ethyl)amino)quinazolin-6-yl)-N methylcyclohexane-1-carboxamide

### Step 1: preparation of tert-butyl (E)-9-(3-ethoxy-3-oxoprop-1-en-1-yl)-3-azaspiro[5.5]undecane-3-carboxylate

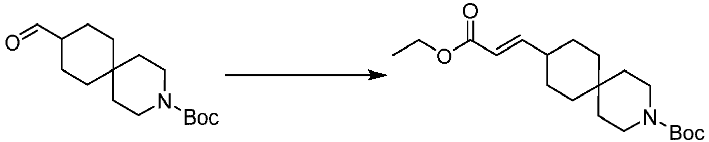

NaH (214 mg, 5.33 mmol, 60%) was added to DMF (15 mL) at 0 °C, and then ethyl 2-(diethoxyphosphoryl)acetate (1.20 g, 5.33 mmol) was added. The reaction liquid was reacted at 0 °C for 30 min. *tert*-Butyl 9-formyl-3-azaspiro[5.5]undecane-3-carboxylate (1.00 g, 3.55 mmol) was added, and the resulting mixture was stirred at room temperature for 3 h. After the reaction was completed, the reaction liquid was cooled to 0 °C, diluted with an aqueous NH₄Cl solution (100 mL), and extracted with ethyl acetate (100 mL × 3). The combined organic phase was washed with brine (100 mL), dried over sodium sulfate, filtered, concentrated under reduced pressure, and purified by column chromatography (PE:EA = 0-1:4) to give *tert*-butyl (*E*)-9-(3-ethoxy-3-oxoprop-1-en-1-yl)-3-azaspiro [5.5]undecane-3 -carboxylate.

LC-MS: (ESI, *m*/*z*): [M+H-56] ⁺=296.2.

### Step 2: preparation of tert-butyl 9-(3-ethoxy-3-oxopropyl)-3-azaspiro[5.5]undecane-3-carboxylate

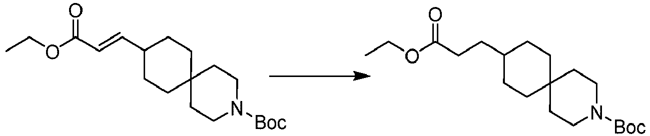

A solution of *tert*-butyl (*E*)-9-(3-ethoxy-3-oxoprop-1-en-1-yl)-3-azaspiro[5.5]undecane-3-carboxylate (1.1 g, 3.13 mmol) in EtOH (20 mL) was added to Pd/C (150 mg) at room temperature under H₂ atmosphere. The reaction liquid was stirred overnight. The mixture was filtered and concentrated to give *tert*-butyl 9-(3-ethoxy-3-oxopropyl)-3-azaspiro [5.5]undecane-3 -carboxylate.

LC-MS: (ESI, *m*/*z*): [M+H-56] ⁺=298.2.

¹H NMR (400 MHz, CDCl₃) δ 4.12 (q, *J* = 7.2 Hz, 2H), 3.40-3.30 (m, 4H), 2.30 (t, *J* = 7.6 Hz, 2H), 1.71-1.62 (m, 2H), 1.58-1.52 (m, 5H), 1.47-1.42 (m, 10H), 1.31-1.22 (m, 6H), 1.13-1.02 (m, 4H).

### Step 3: preparation of tert-butyl 9-(3-hydroxypropyl)-3-azaspiro[5.5]undecane-3-carboxylate

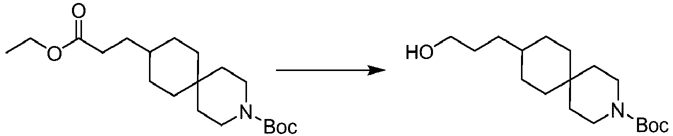

*tert*-Butyl 9-(3-ethoxy-3-oxopropyl)-3-azaspiro[5.5]undecane-3-carboxylate (1 g, 2.83 mmol) was dissolved in THF (25 mL), and LAH (3.4 mmol, 3.4 mL, 1 M/THF) was added dropwise at 0 °C. The reaction liquid was reacted at 0 °C for 1 h. Then Na₂SO₄·10H₂O was added to quench the reaction, and the resulting mixture was stirred for 20 min or longer. The mixture was filtered, and the filtrate was concentrated to give *tert*-butyl 9-(3-hydroxypropyl)-3-azaspiro[5.5]undecane-3-carboxylate, which was directly used in the next step.

¹H NMR (400 MHz, CDCl₃) δ 3.63 (t, *J* = 7.2 Hz, 2H), 3.42-3.29 (m, 4H), 1.69-1.53 (m, 6H), 1.46-1.42 (m, 10H), 1.32-1.22 (m, 6H), 1.12-1.03 (m, 4H).

### Step 4: preparation of tert-butyl 9-(3-oxopropyl)-3-azaspiro[5.5]undecane-3-carboxylate

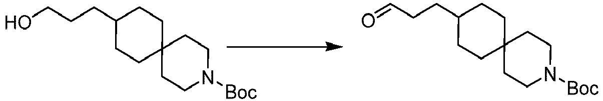

Dess-Martin oxidant (1.37 g, 3.3 mmol) was added to a solution of *tert*-butyl 9-(3-hydroxypropyl)-3-azaspiro[5.5]undecane-3-carboxylate (850 mg, 2.7 mmol) in DCM (25 mL) in batches. The mixture was stirred at room temperature for 1.5 h. An aqueous Na₂SO₃ solution (50 mL) and an aqueous NaHCO₃ solution (50 mL) were added to quench the reaction, and the resulting mixture was extracted with DCM (100 mL L × 3). The combined organic phase was dried over anhydrous sodium sulfate, and filtered, and the filtrate was concentrated and purified by silica gel column chromatography (PE:EtOAc = 20:1-2:1) to give *tert*-butyl 9-(3-oxopropyl)-3-azaspiro [5.5]undecane-3 -carboxylate.

LC-MS: (ESI, *m*/*z*): [M+H-100] ⁺=210.2.

### Step 5: preparation of tert-butyl 9-(3-((4-methoxybenzyl)(methyl)amino)propyl)-3-azaspiro[5.5]undecane-3-carboxylate

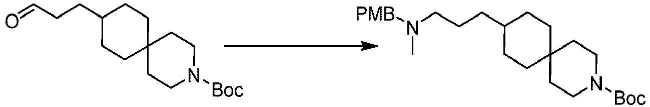

*tert*-Butyl 9-(3-oxopropyl)-3-azaspiro[5.5]undecane-3-carboxylate (400 mg, 1.29 mmol) and 1-(4-methoxyphenyl)-*N*-methylmethanamine (0.39 g, 2.58 mmol) were dissolved in DCM/AcOH (25 mL/0.5 mL). The reaction liquid was stirred at room temperature for 1 h. Then NaBH₃CN (162 mg, 2.58 mmol) was added, and the resulting mixture was reacted at room temperature for 16 h. The reaction liquid was diluted with an aqueous NH₄Cl solution (50 mL) and extracted with DCM (50 mL × 3). The combined organic phase was dried over sodium sulfate, and filtered, and the filtrate was concentrated and purified by column chromatography (DCM:MeOH = 200:1-5:1) to give *tert-butyl* 9-(3-((4-methoxybenzyl)(methyl)amino)propyl)-3-azaspiro[5.5]undecane-3-carboxylate.

LC-MS: (ESI, *m*/*z*): [M+H] ⁺=445.4.

### Step 6: preparation of N-(4-methoxybenzyl)-N-methyl-3-(3-azaspiro[5.5]undecan-9-yl)propan-1-amine

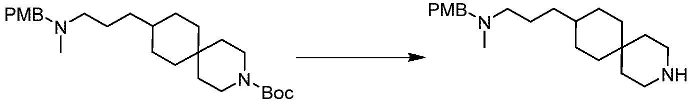

*tert*-Butyl 9-(3-((4-methoxybenzyl)(methyl)amino)propyl)-3-azaspiro[5.5]undecane-3-carboxylate (400 mg, 0.89 mmol) was dissolved in DCM (4.5 mL), and TFA (1.5 mL) was added. The reaction liquid was stirred at room temperature for 2 h and concentrated to give *N*-(4-methoxybenzyl)-*N*-methyl-3-(3-azaspiro[5.5]undecan-9-yl)propan-1-amine, which was directly used in the next step.

LC-MS: (ESI, *m*/*z*): [M+H] ⁺=345.3.

### Step 7: preparation of 1-(2-chloro-5-(9-(3-((4-methoxybenzyl)(methyl)amino)propyl)-3-azaspiro[5.5]undecane-3-carbonyl)phenyl)dihydropyrimidine-2,4(1H,3H)-dione

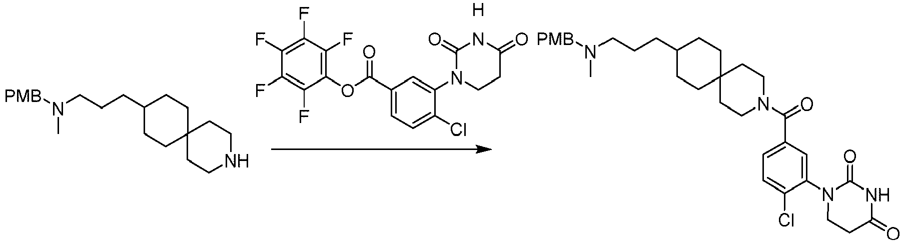

Pentafluorophenyl 4-chloro-3-(2,4-dioxotetrahydropyrimidin-1(2*H*)-yl)benzoate (470 mg, 1.08 mmol) and DIEA (350 mg, 2.7 mmol) were added to a solution of *N*-(4-methoxybenzyl)-*N*-methyl-3-(3-azaspiro[5.5]undecan-9-yl)propan-1-amine (400 mg, 0.9 mmol) in DMSO (2 mL). The reaction liquid was stirred at room temperature for 2 h. Water (10 mL) was added, and the resulting mixture was extracted with EtOAc (20 mL × 2). The organic phases were combined, washed with saturated brine (50 mL × 3), dried over anhydrous sodium sulfate, and filtered, and the filtrate was concentrated and purified by column chromatography (DCM:MeOH = 200:1-5:1) to give 1-(2-chloro-5-(9-(3-((4-methoxybenzyl)(methyl)amino)propyl)-3-azaspiro[5.5]undecane-3-carbonyl)phenyl)dihydropyrimidine-2,4(1*H*,3*H*)-dione.

LC-MS: (ESI, *m*/*z*): [M+H] ⁺= 595.2.

### Step 8: preparation of 1-(2-chloro-5-(9-(3-(methylamino)propyl)-3-)azaspiro[5.5]undecane-3-carbonyl)phenyl)dihydropyrimidine-2,4(1H,3H)-dione

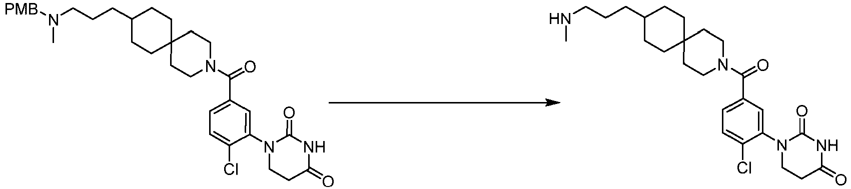

1-(2-Chloro-5-(9-(3-((4-methoxybenzyl)(methyl)amino)propyl)-3-azaspiro[5.5]undecane-3-carbonyl)phenyl)dihydropyrimidine-2,4(1*H*,3*H*)-dione (130 mg, 0.218 mmol) and DIEA (14 mg, 0.11 mmol) were dissolved in DCM (3 mL), and 2-chloroethyl chloroformate (47 mg, 0.33 mmol) was added dropwise at 0 °C. The mixture was stirred at room temperature for 2 h. Then MeOH (3 mL) was added, and the resulting mixture was reacted at 65 °C for 1 h. The mixture was concentrated and purified by column chromatography (DCM:MeOH = 200:1-5:1) to give 1-(2-chloro-5-(9-(3-(methylamino)propyl)-3-)azaspiro[5.5]undecane-3-carbonyl)phenyl)dihydropyrimidine-2,4(1*H*,3*H*)-dione.

LC-MS: (ESI, *m*/*z*): [M+H] ⁺=475.2.

### Step 9: preparation of tert-butyl (2-(5-((R)-1-((6-((1R,4R)-4-((3-(3-(4-chloro-3-(2,4-dioxotetrahydropyrimidine))-1(2H)-yl)benzoyl)-3-azaspiro[5.5]undecan-9-yl)propyl)(methyl)carbamoyl)cyclohexyl)-7-methoxy-2-methylquinazolin-4-yl)amino)ethyl)thien-3-yl)benzyl)(methyl)aminocarboxylate

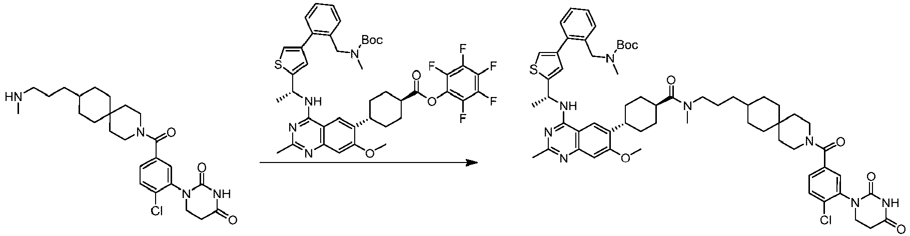

1 -(2-Chloro-5 -(9-(3 -(methylamino)propyl)-3 -azaspiro [5.5]undecane-3 -carbonyl)phenyl)dihydropyrimidine-2,4(1*H*,3*H*)-dione (100 mg, 0.21 mmol) was dissolved in DMSO (2 mL), and pentafluorophenyl (1*R*,4*R*)-4-(4-(((*R*)-1-(4-(2-(((*tert*-butoxycarbonyl)(methyl)amino)methyl)phenyl)thien-2-yl)ethyl)amino)-7-methoxy-2-methylquinazolin-6-yl)cyclohexane-1-carboxylate (187 mg, 0.23 mmol) and DIEA (82 mg, 0.63 mmol) were added. The reaction liquid was reacted at room temperature for 2 h. After the reaction was completed, water (2 mL) was added, and the resulting mixture was extracted with ethyl acetate and water. The combined organic phase was dried over sodium sulfate, filtered, concentrated, and purified by column chromatography (DCM:MeOH = 100:1-5:1) to give the target compound *tert*-butyl (2-(5-((*R*)-1-((6-((1*R*,4*R*)-4-((3-(3-(4-chloro-3-(2,4-dioxotetrahydropyrimidine))-1(2*H*)-yl)benzoyl)-3 -azaspiro [5.5]undecan-9-yl)propyl)(methyl)carbamoyl)cyclohexyl)-7-methoxy-2-methylquinazolin-4-yl)amino)ethyl)thien-3-yl)benzyl)(methyl)aminocarboxylate.

LC-MS: (ESI, *m*/*z*): [1/2(M-100)+H] ⁺=501.2.

### Step 10: preparation of (1R,4R)-N-(3-(3-(4-chloro-3-(2,4-dioxotetrahydropyrimidin-1(2H)-yl)benzoyl)-3-azaspiro[5.5]undecan-9-yl)propyl)-4-(7-methoxy-2-methyl-4-(((R)-1-(4-(2-((methylamino)methyl)phenyl)thien-2-yl)ethyl)amino)quinazolin-6-yl)-N methylcyclohexane-1-carboxamide

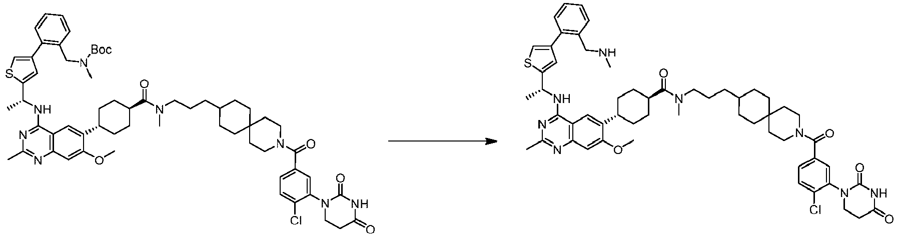

The compound tert-butyl (2-(5-((*R*)-1-((6-((1*R*,4*R*)-4-((3-(3-(4-chloro-3-(2,4-dioxotetrahydropyrimidine))-1(2*H*)-yl)benzoyl)-3-azaspiro[5.5]undecan-9-yl)propyl)(methyl)carbamoyl)cyclohexyl)-7-methoxy-2-methylquinazolin-4-yl)amino)ethyl)thien-3-yl)benzyl)(methyl)aminocarboxylate (170 mg, crude product) was dissolved in DCM (4.5 mL), and TFA (1.5 mL) was added. The mixture was stirred at room temperature for 2 h, concentrated, and purified by high performance liquid chromatography to give (1*R*,4*R*)-*N*-(3-(3-(4-chloro-3-(2,4-dioxotetrahydropyrimidin-1(2*H*)-yl)benzoyl)-3-azaspiro[5.5]undecan-9-yl)propyl)-4-(7-methoxy-2-methyl-4-(((*R*)-1-(4-(2-((methylamino)methyl)phenyl)thien-2-yl)ethyl)amino)quinazolin-6-yl)-N-methylcyclohexane-1-carboxamide. LC-MS: (ESI, *m*/*z*): [M+H] ⁺=1001.4.

¹H NMR (400 MHz, CD₃OD) δ 8.02-7.96 (m, 1H), 7.65-7.58 (m, 1H), 7.54-7.48 (m, 1H), 7.42-7.36 (m, 2H), 7.32-7.26 (m, 3H), 7.20-7.10 (m, 2H), 7.01 (s, 1H), 6.06 (q, *J* = 6.8 Hz, 1H), 3.95 (s, 3H), 3.80-3.63 (m, 6H), 3.48-3.34 (m, 4H), 3.16-2.99 (m, 3H), 2.93-2.82 (m, 3H), 2.76- 2.62 (m, 1H), 2.52 (s, 3H), 2.22 (s, 3H), 2.03-1.85 (m, 4H), 1.80-1.42 (m, 16H), 1.37-1.15 (m, 8H).

### Example 50: 1-(2-chloro-5-(9-(2-(4-((1R,4R)-4-(7-methoxy-2-methyl-4-(((R)-1-))4-(2-((methylamino)methyl)phenyl)thien-2-yl)ethyl)amino)quinazolin-6-yl)cyclohexane-1-carbonyl)piperazin-1-yl)ethyl)-3-azaspiro[5.5]undecane-3-carbonyl)phenyl)dihydropyrimidine-2,4(1H,3H)-dione

### Step 1: preparation of tert-butyl 9-(2-ethoxy-2-oxoethylidene)-3-azaspiro[5.5]undecane-3-carboxylate

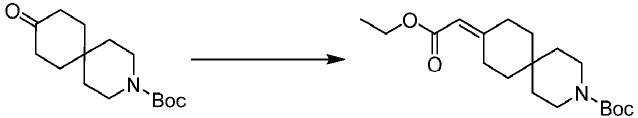

Ethyl 2-(diethoxyphosphoryl)acetate (1.26 g, 5.61 mmol) was added to a solution of NaH (230 mg, 5.61 mmol, 60%) in DMF (15 mL) while stirring. The mixture was stirred at 0 °C for 30 min. Then *tert*-butyl 9-oxo-3-azaspiro[5.5]undecane-3-carboxylate (1 g, 3.74 mmol) was added, and the resulting mixture was stirred at room temperature for 3 h. The mixture was diluted with an aqueous NH₄Cl solution (100 mL) and extracted with ethyl acetate (100 mL × 3). The combined organic phase was washed with brine (100 mL), dried over sodium sulfate, filtered, concentrated under reduced pressure, and purified by column chromatography (EA:PE = 0-1:4) to give *tert-*butyl 9-(2-ethoxy-2-oxoethylidene)-3-azaspiro[5.5]undecane-3-carboxylate.

LC-MS: (ESI, *m*/*z*): [M+H]⁺=338.3.

### Step 2: preparation of tert-butyl 9-(2-ethoxy-2-oxoethyl)-3-azaspiro[5.5]undecane-3-carboxylate

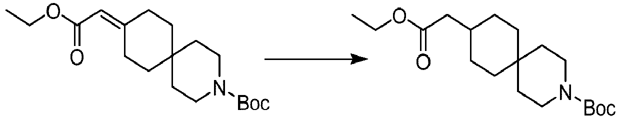

*tert*-Butyl 9-(2-ethoxy-2-oxoethylidene)-3-azaspiro[5.5]undecane-3-carboxylate (1.1 g, 3.26 mmol) was dissolved in EtOH (20 mL), and the reaction liquid was stirred overnight under H₂ atmosphere. The mixture was filtered and concentrated to give *tert*-butyl 9-(2-ethoxy-2-oxoethyl)-3-azaspiro[5.5]undecane-3-carboxylate.

LC-MS: (ESI, *m*/*z*): [M+H-56]⁺=284.2.

### Step 3: preparation of tert-butyl 9-(2-hydroxyethyl)-3-azaspiro[5.5]undecane-3-carboxylate

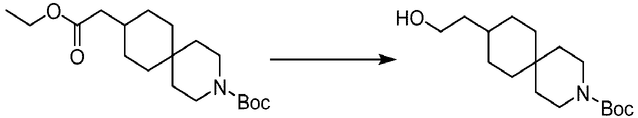

LAH (3.6 mmol, 3.6 mL, 1 M/THF) was added to a solution of *tert*-butyl 9-(2-ethoxy-2-oxoethyl)-3-azaspiro[5.5]undecane-3-carboxylate (1 g, 3 mmol) in THF (25 mL) while stirring. The reaction liquid was reacted at 0 °C to room temperature for 1 h. Na₂SO₄·10H₂O was added to the mixture at room temperature, and the resulting mixture was stirred for 1 h or longer. The mixture was filtered and concentrated to give the target compound *tert-*butyl 9-(2-hydroxyethyl)-3-azaspiro[5.5]undecane-3-carboxylate.

LC-MS: (ESI, *m*/*z*): [M+H-56]⁺=242.2.

### Step 4: preparation of tert-butyl 9-(2-oxoethyl)-3-azaspiro[5.5]undecane-3-carboxylate

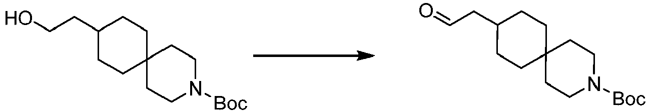

Dess-Martin oxidant (1.42 g, 3.6 mmol) was added to a solution of *tert*-butyl 9-(2-hydroxyethyl)-3-azaspiro[5.5]undecane-3-carboxylate (850 mg, 2.5 mmol) in DCM (25 mL) while stirring. The mixture was stirred at room temperature for 1.5 h. The mixture was diluted with an aqueous Na₂SO₃ solution (50 mL) and an aqueous NaHCO₃ solution (50 mL) and extracted with DCM (100 mL × 3). The combined organic phase was dried over sodium sulfate, filtered, concentrated under reduced pressure, and purified by column chromatography (EA:PE = 0-3:2) to give *tert*-butyl 9-(2-oxoethyl)-3-azaspiro[5.5]undecane-3-carboxylate.

LC-MS: (ESI, *m*/*z*): [M+H-56]⁺=240.2.

### Step 5: preparation of tert-butyl 9-(2-(4-(4-methoxybenzyl)piperazin-1-yl)ethyl)-3-azaspiro[5.5]undecane-3-carboxylate

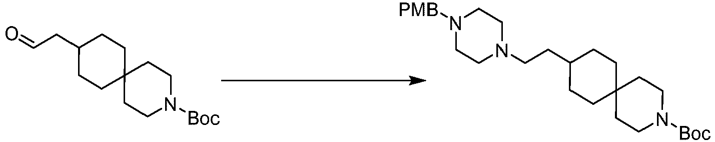

1-(4-Methoxybenzyl)piperazine (0.79 g, 3.81 mmol) was added to a solution of *tert*-butyl 9-(2-oxoethyl)-3-azaspiro[5.5]undecane-3-carboxylate (750 mg, 2.54 mmol) in DCM/HAc (25 mL/0.5 mL) while stirring. The mixture was stirred at room temperature for 1 h. Then NaBH₃CN (320 mg, 5.08 mmol) was added. The mixture was diluted with an aqueous NH₄Cl solution (50 mL) and extracted with DCM (50 mL × 3). The combined organic phase was dried over sodium sulfate, filtered, concentrated under reduced pressure, and purified by column chromatography (DCM:MeOH = 0-1:4) to give *tert*-butyl 9-(2-(4-(4-methoxybenzyl)piperazin-1-yl)ethyl)-3-azaspiro [5.5]undecane-3 -carboxylate.

LC-MS: (ESI, *m*/*z*): [M+H]⁺=486.4.

### Step 6: preparation of 9-(2-(4-(4-methoxybenzyl)piperazin-1-yl)ethyl)-3-azaspiro[5.5]undecane

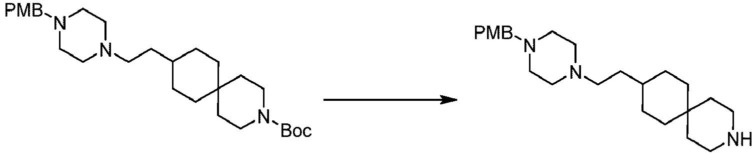

TFA (0.5 mL) was added dropwise to a solution of *tert*-butyl 9-(2-(4-(4-methoxybenzyl)piperazin-1-yl)ethyl)-3-azaspiro[5.5]undecane-3-carboxylate (100 mg, 0.21 mmol) in DCM (1.5 mL) while stirring. The mixture was stirred at room temperature for 2 h, and the reaction liquid was concentrated under reduced pressure to give 9-(2-(4-(4-methoxybenzyl)piperazin-1-yl)ethyl)-3-azaspiro[5.5]undecane.

LC-MS: (ESI, *m*/*z*): [M+H]⁺=386.4.

### Step 7: 1-(2-chloro-5-(9-(2-(4-(4-methoxybenzyl)piperazin-1-yl)ethyl)-3-azaspiro[5.5]undecane-3-carbonyl)phenyl)dihydropyrimidine-2,4(1H,3H)-dione

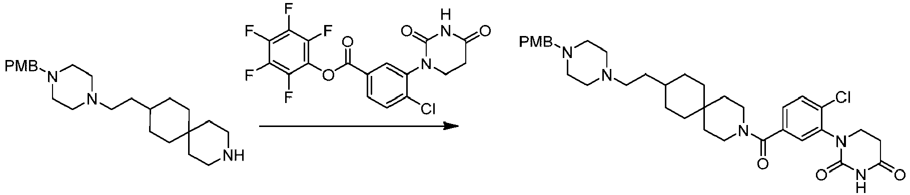

Pentafluorophenyl 4-chloro-3-(2,4-dioxotetrahydropyrimidin-1(2*H*)-yl)benzoate (99 mg, 0.23 mmol) and DIEA (80 mg, 0.62 mmol) were added to a solution of 9-(2-(4-(4-methoxybenzyl)piperazin-1-yl)ethyl)-3-azaspiro[5.5]undecane (100 mg, crude product) in DMSO (2 mL) while stirring. The mixture was stirred at room temperature for 2 h and extracted with EA and water. The combined organic phase was dried over sodium sulfate, filtered, concentrated under reduced pressure, and purified by column chromatography (DCM:MeOH = 0-1:4) to give 1-(2-chloro-5-(9-(2-(4-(4-methoxybenzyl)piperazin-1-yl)ethyl)-3-azaspiro[5.5]undecane-3-carbonyl)phenyl)dihydropyrimidine-2,4(1*H*,3*H*)-dione.

LC-MS: (ESI, *m*/*z*): [M+H]⁺=636.4.

### Step 8: preparation of 1-(2-chloro-5-(9-(2-(piperazin-1-yl)ethyl)))-3-azaspiro[5.5]undecane-3-carbonyl)phenyl)dihydropyrimidine-2,4(1H,3H)-dione

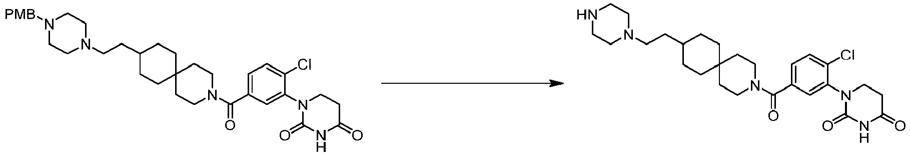

1-(2-Chloro-5-(9-(2-(4-(4-methoxybenzyl)piperazin-1-yl)ethyl)-3-azaspiro[5.5]undecane-3-carbonyl)phenyl)dihydropyrimidine-2,4(1*H*,3*H*)-dione (90 mg, 0.14 mmol) was dissolved in DCM (2 mL), and 2-chloroethyl chloroformate (30 mg, 0.22 mmol) and DIEA (9 mg, 0.071 mmol) were added. The mixture was stirred at 0 °C to room temperature for 2 h. Then MeOH (3 mL) was added at 65 °C and the mixture was successively stirred for 1 h. The mixture was concentrated and purified by column chromatography (DCM:MeOH = 0-1:4) to give 1-(2-chloro-5-(9-(2-(piperazin-1-yl)ethyl)))-3-azaspiro[5.5]undecane-3-carbonyl)phenyl)dihydropyrimidine-2,4(1*H*,3*H*)-dione.

LC-MS: (ESI, *m*/*z*): [M+H]⁺=516.3.

### Step 9: preparation of tert-butyl (2-(5-((R)-1-((6-((1R,4R)-4-(4-(2-(3-(4-chloro-3-(2,4-dioxotetrahydropyrimidin-1(2H)-yl)benzoyl)-3-azaspiro[5.5]undecan-9-yl)ethyl)piperazine-1-carbonyl)cyclohexyl)-7-methoxy-2-methylquinazolin-4-yl)amino)ethyl)thien-3-yl)benzyl)(methyl)aminocarboxylate

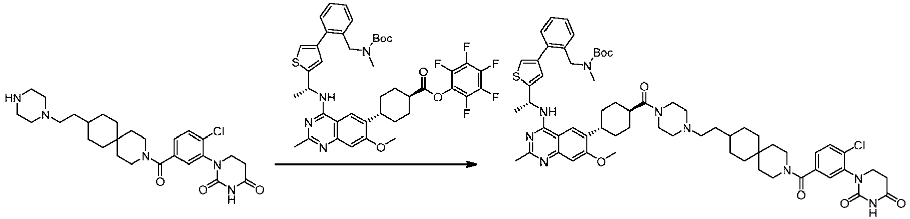

1-(2-Chloro-5-(9-(2-(piperazin-1-yl)ethyl)-3-azaspiro[5.5]undecane-3-carbonyl)phenyl)dihydropyrimidine-2,4(1*H*,3*H*)-dione (63 mg, 0.12 mmol) was dissolved in DMSO (2 mL), and pentafluorophenyl (1*R*,4*R*)-4-(4-(((*R*)-1-(4-(2-(((*tert*-butoxycarbonyl)(methyl)amino)methyl)phenyl)thien-2-yl)ethyl)amino)-7-methoxy-2-methylquinazolin-6-yl)cyclohexane-1-carboxylate (109 mg, 0.13 mmol) and DIEA (48 mg, 0.36 mmol) were added. The mixture was stirred at room temperature for 2 h and extracted with EA and water. The combined organic phase was dried over sodium sulfate, filtered, and concentrated to give tert-butyl (2-(5-((*R*)-1-((6-((1*R*,4*R*)-4-(4-(2-(3-(4-chloro-3-(2,4-dioxotetrahydropyrimidin-1(2*H*)-yl)benzoyl)-3-azaspiro[5.5]undecan-9-yl)ethyl)piperazine-1-carbonyl)cyclohexyl)-7-methoxy-2-methylquinazolin-4-yl)amino)ethyl)thien-3-yl)benzyl)(methyl)aminocarboxylate.

LC-MS: (ESI, *m*/*z):* [(M-56)/2+H]+=544.3.

### Step 10: preparation of 1-(2-chloro-5-(9-(2-(4-((1R,4R)-4-(7-methoxy-2-methyl-4-(((R)-1-))-4-(2-((methylamino)methyl)phenyl)thien-2-yl)ethyl)amino)quinazolin-6-yl)cyclohexane-1-carbonyl)piperazin-1-yl)ethyl)-3-azaspiro[5.5]undecane-3-carbonyl)phenyl)dihydropyrimidine-2,4(1H,3H)-dione

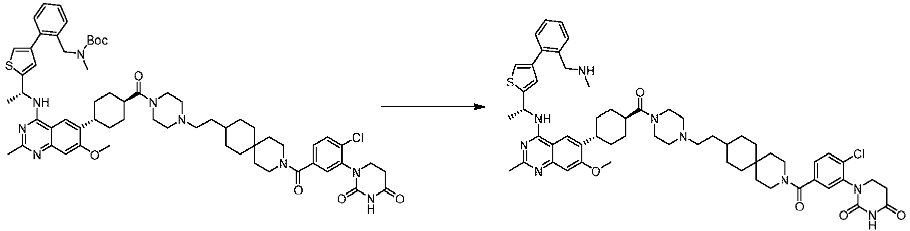

*tert*-Butyl (2-(5-((*R*)-1-((6-((1*R*,4*R*)-4-(4-(2-(3-(4-chloro-3-(2,4-dioxotetrahydropyrimidin-1(2*H*)-yl)benzoyl)-3-azaspiro[5.5]undecan-9-yl)ethyl)piperazine-1-carbonyl)cyclohexyl)-7-methoxy-2-methylquinazolin-4-yl)amino)ethyl)thien-3-yl)benzyl)(methyl)carbamate (160 mg, crude product) was dissolved in DCM (4.5 mL), and TFA (1.5 mL) was added. The mixture was stirred at room temperature for 2 h, concentrated, and purified by preparative chromatography to give 1-(2-chloro-5-(9-(2-(4-((1*R*,4*R*)-4-(7-methoxy-2-methyl-4-(((*R*)-1-))-4-(2-((methylamino)methyl)phenyl)thien-2-yl)ethyl)amino)quinazolin-6-yl)cyclohexane-1-carbonyl)piperazin-1-yl)ethyl)-3-azaspiro[5.5]undecane-3-carbonyl)phenyl)dihydropyrimidine-2,4(1*H*,3*H*)-dione.

LC-MS: (ESI, *m*/*z*): [M+H] ⁺=1042.4.

¹H NMR (400 MHz, CD₃OD) δ 7.98 (s, 1H), 7.68-7.56 (m, 1H), 7.57-7.48 (m, 1H), 7.45-7.35 (m, 2H), 7.34-7.24 (m, 3H), 7.22-7.10 (m, 2H), 7.01 (s, 1H), 6.06 (q, *J* = 6.9 Hz, 1H), 3.95 (s, 3H), 3.83-3.73 (m, 2H), 3.74-3.68 (m, 3H), 3.66-3.55 (m, 4H), 3.45-3.34 (m, 2H), 3.12-3.01 (m, 1H), 2.93-2.76 (m, 2H), 2.76-2.66 (m, 1H), 2.55-2.39 (m, 8H), 2.21 (s, 3H), 2.03-1.86 (m, 4H), 1.81-1.75 (m, 4H), 1.70-1.57 (m, 6H), 1.45-1.16 (m, 14H).

### Example 51: 1-(5-(9-((4-(4-(4-(((R)-1-(3-amino-5-(trifluoromethyl)phenyl)ethyl)amino)-7-methoxy-2-methylquinazolin-6-yl)cyclohex-3-ene-1-carbonyl)piperazin-1-yl)methyl)-3-azaspiro[5.5]undecane-3-carbonyl)-2-chlorophenyl)dihydropyrimidine-2,4(1H,3H)-dione

### Step 1: preparation of benzyl 9-((4-(tert-butoxycarbonyl)piperazin-1-yl)methyl)-3-azaspiro[5.5]undecane-3-carboxylate

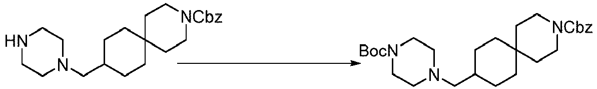

Benzyl 9-(piperazin-1-ylmethyl)-3-azaspiro[5.5]undecane-3-carboxylate (400 mg, 1.04 mmol) was dissolved with DCM (10 mL), and (Boc)₂O (272 mg, 1.25 mmol), DMAP (13 mg, 0.1 mmol), and TEA (315 mg, 3.12 mmol) were separately added. The reaction liquid was stirred at room temperature overnight and directly concentrated under reduced pressure. The crude product was purified by column chromatography (PE:EA = 1:1) to give benzyl 9-((4-(*tert*-butoxycarbonyl)piperazin-1-yl)methyl)-3-azaspiro[5.5]undecane-3-carboxylate.

LC-MS: (ESI, *m*/*z*): [M+H] ⁺=486.3.

### Step 2: preparation of tert-butyl 4-((3-azaspiro[5.5]undecan-9-yl)methyl)piperazine-1-carboxylate

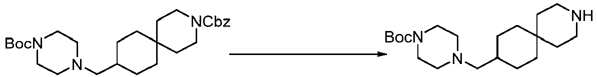

Benzyl 9-((4-(tert-butoxycarbonyl)piperazin-1-yl)methyl)-3-azaspiro[5.5]undecane-3-carboxylate (300 mg, 0.62 mmol) was dissolved with ethyl acetate (10 mL), and Pd(OH)₂/C (100 mg) was added. The reaction liquid was stirred at 70 °C for 3 h, cooled, and filtered. The solid was washed three times with ethyl acetate (10 mL × 3) and concentrated under reduced pressure to give the crude product *tert*-butyl 4-((3-azaspiro[5.5]undecane-9-yl)methyl)piperazine-1 -carboxylate.

LC-MS: (ESI, *m*/*z*): [M+H] ⁺=352.2.

### Step 3: preparation of tert-butyl 4-((3-(4-chloro-3-(2,4-dioxotetrahydropyrimidin-1(2H)-yl)benzoyl)-3-azaspiro[5.5]undecan-9-yl)methyl)piperazine-1-carboxylate

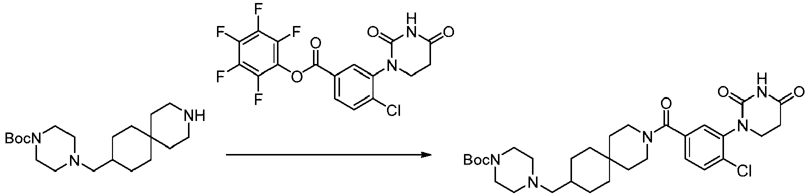

*tert*-Butyl 4-((3-azaspiro[5.5]undecan-9-yl)methyl)piperazine-1-carboxylate (200 mg, 0.57 mmol) was dissolved with DMSO (10 mL), and pentafluorophenyl 4-chloro-3-(2,4-dioxotetrahydropyrimidin-1(2*H*)-yl)benzoate (247 mg, 0.57 mmol) and DIEA (220 mg, 1.71 mmol) were added. The reaction liquid was stirred at room temperature for 2 h. Water (50 mL) was added, and the resulting mixture was extracted with ethyl acetate (25 mL × 4), washed with saturated brine, dried over anhydrous sodium sulfate, and concentrated under reduced pressure. The crude product was purified by column chromatography (PE:EA = 1:10) to give *tert*-butyl 4-((3-(4-chloro-3-(2,4-dioxotetrahydropyrimidin-1(2*H*)-yl)benzoyl)-3-azaspiro[5.5]undecan-9-yl)methyl)piperazine-1-carboxylate. LC-MS: (ESI, *m*/*z*): [M+H] ⁺=602.2.

### Step 4: preparation of 1-(2-chloro-5-(9-(piperazin-1-ylmethyl)-3-azaspiro[5.5]undecane-3-carbonyl)phenyl)dihydropyrimidine-2,4(1H,3H)-dione

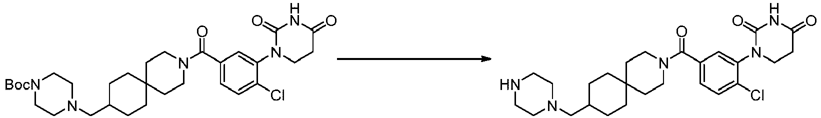

*tert*-Butyl 4-((3-(4-chloro-3-(2,4-dioxotetrahydropyrimidin-1(2*H*)-yl)benzoyl)-3-azaspiro[5.5]undecan-9-yl)methyl)piperazine-1-carboxylate (350 mg, 0.58 mmol) was dissolved with DCM (5 mL), and TFA (1 mL) was added. The reaction liquid was stirred at room temperature for 2 h and concentrated under reduced pressure. The residue was diluted with water (20 mL), and a saturated sodium bicarbonate solution was slowly added dropwise to adjust the reaction liquid to neutrality. The reaction liquid was extracted with ethyl acetate (25 mL × 4), washed with saturated brine, dried over anhydrous sodium sulfate, and concentrated under reduced pressure to give the crude product 1-(2-chloro-5-(9-(piperazin-1-ylmethyl)-3-azaspiro[5.5]undecan-3-carbonyl)phenyl)dihydropyrimidine-2,4(1*H*,3*H*)-dione.

LC-MS: (ESI, *m*/*z*): [M+H] ⁺=502.3.

### Step 5: preparation of tert-butyl (3-((1R)-1-((6-(4-(4-((3-(4-chloro-3-(2,4-dioxotetrahydropyrimidin-1(2H)-yl)benzoyl)-3-azaspiro[5.5]undecan-9-yl)methyl)piperazine-1-carbonyl)cyclohexan-1-yl)-7-methoxy-2-methylquinazolin-4-yl)amino)ethyl)-5-(trifluoromethyl)phenyl)carboxylate

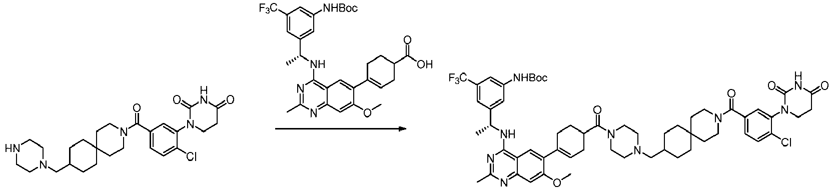

1-(2-Chloro-5-(9-(piperazin-1-ylmethyl)-3-azaspiro[5.5]undecane-3-carbonyl)phenyl)dihydropyrimidine-2,4(1*H*,3*H*)-dione (125 mg, 0.25 mmol) was dissolved with DMF (10 mL), and 4-(4-(((*R*)-1-(3-((tert-butoxycarbonyl)amino)-5-(trifluoromethyl)phenyl)ethyl)amino)-7-methoxy-2-methylquinazolin-6-yl)cyclohex-3-ene-1-carboxylic acid (140 mg, 0.23 mmol), HATU (114 mg, 0.30 mmol), and DIEA (129 mg, 1.00 mmol) were separately added. The reaction liquid was stirred at room temperature for 2 h. Water (100 mL) was added, and the solid was precipitated, collected by filtration, washed 3 times with water, dissolved with dichloromethane, dried over anhydrous sodium sulfate, and concentrated under reduced pressure. The crude product was purified by column chromatography (MeOH:DCM = 1:10) to give *tert*-butyl (3-((1*R*)-1-((6-(4-(4-((3-(4-chloro-3-(2,4-dioxotetrahydropyrimidin-1(2*H*)-yl)benzoyl)-3-azaspiro[5.5]undecan-9-yl)methyl)piperazine-1-carbonyl)cyclohexan-1-yl)-7-methoxy-2-methylquinazolin-4-yl)amino)ethyl)-5-(trifluoromethyl)phenyl)carboxylate.

LC-MS: (ESI, *m*/*z*): [M+H] ⁺=1095.5.

### Step 6: preparation of 1-(5-(9-((4-(4-(4-(((R)-1-(3-amino-5-(trifluoromethyl)phenyl)ethyl)amino)-7-methoxy-2-methylquinazolin-6-yl)cyclohex-3-ene-1-carbonyl)piperazin-1-yl)methyl)-3-azaspiro[5.5]undecane-3-carbonyl)-2-chlorophenyl)dihydropyrimidine-2,4(1H,3H)-dione

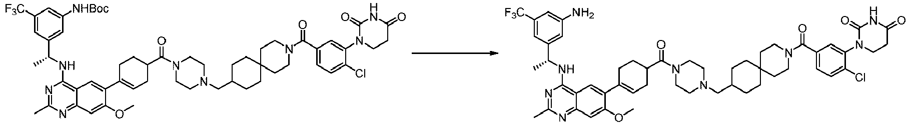

tert-Butyl (3-((1*R*)-1-((6-(4-(4-((3-(4-chloro-3-(2,4-dioxotetrahydropyrimidin-1(2*H*)-yl)benzoyl)-3-azaspiro[5.5]undecan-9-yl)methyl)piperazine-1-carbonyl)cyclohexan-1-yl)-7-methoxy-2-methylquinazolin-4-yl)amino)ethyl)-5-(trifluoromethyl)phenyl)carboxylate (100 mg, 0.09 mmol) was dissolved with DCM (5 mL), and TFA (1 mL) was added. Then the reaction liquid was stirred at room temperature overnight, concentrated under reduced pressure, and diluted with water (20 mL). A sodium bicarbonate solution was slowly added dropwise to adjust the reaction liquid to neutrality. The reaction liquid was extracted with dichloromethane (25 mL × 4), dried over anhydrous sodium sulfate, and concentrated under reduced pressure. The crude product was purified by preparative reversed-phase chromatography to give 1-(5-(9-((4-(4-(4-(((*R*)-1-(3-amino-5-(trifluoromethyl)phenyl)ethyl)amino)-7-methoxy-2-methylquinazolin-6-yl)cyclohex-3-ene-1-carbonyl)piperazin-1-yl)methyl)-3-azaspiro[5.5]undecane-3-carbonyl)-2-chlorophenyl)dihydropyrimidine-2,4(1*H*,3*H*)-dione. LC-MS: (ESI, *m*/*z*): [M+H] ⁺=984.3.

1H NMR (400 MHz, DMSO-*d*₆) δ 10.50 (s, 1H), 8.18 (s, 1H), 8.06 (s, 1H), 7.63 (d, *J* = 8.2 Hz, 1H), 7.55 (d, *J* = 1.7 Hz, 1H), 7.39 (d, *J* = 8.3 Hz, 1H), 6.99 (s, 1H), 6.89 (s, 1H), 6.85 (s, 1H), 6.69 (s, 1H), 5.83 (s, 1H), 5.59-5.47 (m, 3H), 3.85 (s, 3H), 3.80-3.70 (m, 1H), 3.67-3.45 (m, 7H), 2.94-2.82 (m, 1H), 2.77-2.71 (m, 2H), , 2.42-2.10 (m, 13H), 1.89-1.81 (m, 1H), 1.73-1.25 (m, 15H), 1.18-0.94 (m, 4H).

### Example 52: 3-(4-(9-((4-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)methyl)-3-azaspiro[5.5]undecane-3-carbonyl)phenyl)piperidine-2,6-dione

### Step 1: preparation of tert-butyl 9-((4-(((9H-fluoren-9-yl)methoxy)carbonyl)piperazin-1-yl)methyl)-3-azaspiro [5.5]undecane-3 -carboxylate

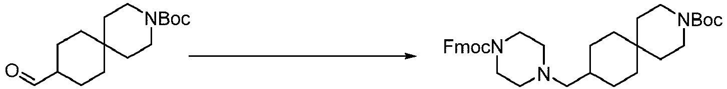

*tert*-Butyl 9-aldehyde-3-azaspiro[5.5]undecane-3-carboxylate (200 mg, 0.71 mmol) was dissolved in a tetrahydrofuran solution (4 mL), and (9*H*-fluoren-9-yl)methyl piperazine-1-carboxylate (219 mg, 0.71 mmol) and sodium triacetoxyborohydride (449 mg, 2.13 mmol) were added. The reaction liquid was stirred at room temperature overnight, diluted with water (40 mL), extracted with ethyl acetate (80 mL × 2), dried over anhydrous sodium sulfate, filtered, and concentrated under reduced pressure. The crude product was purified by column chromatography (ethyl acetate:petroleum ether = 1:1) to give *tert*-butyl 9-((4-(((9*H-*fluoren-9-yl)methoxy)carbonyl)piperazin-1-yl)methyl)-3-azaspiro[5.5]undecane-3-carboxylate.

LC-MS: (ESI, *m*/*z*): [M+H] ⁺=574.4.

### Step 2: preparation of (9H-fluoren-9-yl)methyl 4-((3-azaspiro[5.5]undecan-9-yl)methyl)piperazine-1-carboxylate

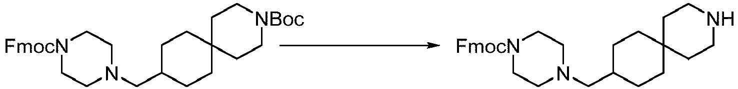

*tert*-Butyl 9-((4-(((9*H*-fluoren-9-yl)methoxy)carbonyl)piperazin-1-yl)methyl)-3 -azaspiro [5.5]undecane-3 - carboxylate (400 mg, 0.7 mmol) was dissolved in a solution of hydrogen chloride in 1,4-dioxane (4 mmol/L, 5 mL). The reaction liquid was stirred at room temperature for 1 h and directly concentrated under reduced pressure to give (9*H*-fluoren-9-yl)methyl 4-((3-azaspiro[5.5]undecane-9-yl)methyl)piperazine-1-carboxylate.

LC-MS: (ESI, *m*/*z*): [M+H] ⁺=474.5.

### Step 3: preparation of (9H-fluoren-9-yl)methyl 4-((3-(4-(2,6-dioxopiperidin-3-yl)benzoyl)-3-azaspiro[5.5]undecan-9-yl)methyl)piperazine-1-carboxylate

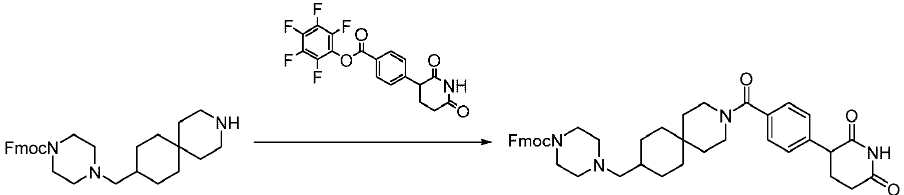

(9*H*-Fluoren-9-yl)methyl 4-((3-azaspiro[5.5]undecan-9-yl)methyl)piperazine-1-carboxylate (340 mg, 0.72 mmol) was dissolved with DMSO (4 mL), and pentafluorophenyl 4-(2,6-dioxopiperidin-3-yl)benzoate (287 mg, 0.72 mmol) and DIEA (371 mg, 2.87 mmol) were added. The reaction liquid was stirred at room temperature overnight, diluted with water (40 mL), and extracted with ethyl acetate (40 mL × 3). The organic phase was washed with saturated brine (40 mL), dried over anhydrous sodium sulfate, and concentrated under reduced pressure. The crude product was purified by column chromatography (PE:EA = 1:2) to give (9*H*-fluoren-9-yl)methyl 4-((3-(4-(2,6-dioxopiperidin-3-yl)benzoyl)-3-azaspiro[5.5]undecane-9-yl)methyl)piperazine-1-carboxylate.

LC-MS: (ESI, *m*/*z*): [M+H] ⁺=689.5.

### Step 4: preparation of 3-(4-(9-(piperazin-1-ylmethyl)-3-azaspiro[5.5]undecane-3-carbonyl)phenyl)piperidine-2,6-dione

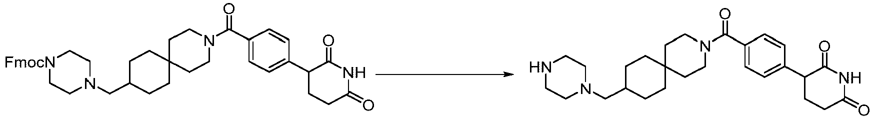

(9*H*-Fluoren-9-yl)methyl 4-((3-(4-(2,6-dioxopiperidin-3-yl)benzoyl)-3-azaspiro[5.5]undecan-9-yl)methyl)piperazine-1-carboxylate (250 mg, 0.36 mmol) was dissolved with dichloromethane (4 mL), and piperidine (0.25 mL) was added. The reaction liquid was stirred at room temperature for 3 h and directly concentrated under reduced pressure to give 3-(4-(9-(piperazin-1-ylmethyl)-3-azaspiro[5.5]undecan-3-carbonyl)phenyl)piperidine-2,6-dione.

LC-MS: (ESI, *m*/*z*): [M+H] ⁺=467.4.

### Step 5: preparation of 3-(4-(9-((4-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)methyl)-3-azaspiro[5.5]undecane-3-carbonyl)phenyl)piperidine-2,6-dione

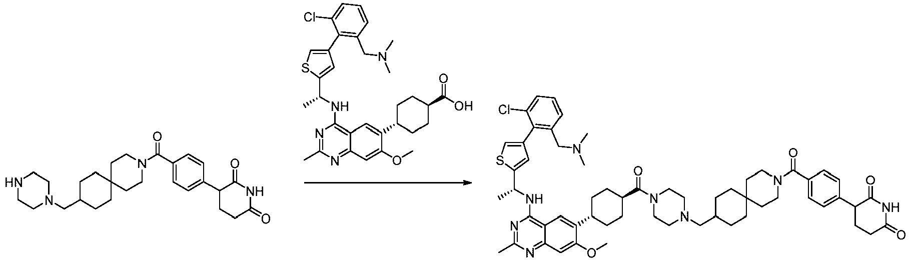

3-(4-(9-(Piperazin-1-ylmethyl)-3-azaspiro[5.5]undecane-3-carbonyl)phenyl)piperidine-2,6-dione (200 mg, 0.43 mmol) was dissolved with DMF (3 mL), and (1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carboxylic acid (254 mg, 0.43 mmol), HATU (196 mg, 0.52 mmol), and DIEA (221 mg, 1.7 mmol) were separately added. The reaction liquid was stirred at room temperature for 3 h, diluted with water (40 mL), and extracted with ethyl acetate (40 mL × 3). The organic phase was washed with saturated brine (40 mL), dried over anhydrous sodium sulfate, and concentrated under reduced pressure. The crude product was purified by Prep-HPLC to give 3-(4-(9-((4-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)methyl)-3-azaspiro[5.5]undecane-3-carbonyl)phenyl)piperidine-2,6-dione.

LC-MS: (ESI, *m*/*z):* [M+H] ⁺=1041.6.

1H NMR (400 MHz, DMSO-*d*₆) δ 10.86 (s, 1H), 8.31 (d, *J* = 8.5 Hz, 1H), 8.05 (s, 1H), 7.45 - 7.40 (s, 2H), 7.36 - 7.32 (s, 3H), 7.28 (d, *J* = 8.1 Hz, 2H), 7.24 (d, *J* = 1.2 Hz, 1H), 7.01 (s, 1H), 6.96 (s, 1H), 5.99 - 5.94 (m, 1H), 3.94 - 3.87 (m, 4H), 3.57 (s, 2H), 3.46 (s, 5H), 3.12 (s, 2H), 2.94 (s, 1H), 2.74 - 2.59 (m, 4H), 2.42 (s, 3H), 2.23 (d, *J* = 13.2 Hz, 5H), 2.17 - 2.04 (m, 4H), 2.01 (s, 7H), 1.88 - 1.76 (m, 5H), 1.71 (d, *J* = 6.9 Hz, 4H), 1.68 (s, 1H), 1.64 - 1.48 (m, 8H), 1.23 (s, 2H), 1.07 (s, 3H).

### Example 53: 1-(5-(4-((2-(4-(4-(4-(((R)-1-(3-amino-5-(trifluoromethyl)phenyl)ethyl)amino)-7-methoxy-2-methylquinazolin-6-yl)cyclohex-3-ene-1-carbonyl)piperazin-1-yl)ethoxy)methyl)piperidine-1-carbonyl)-2-chlorophenyl)dihydropyrimidine-2,4(1H,3H)-dione

### Step 1: preparation of tert-butyl 4-(2-((1-(4-chloro-3-(2,4-dioxotetrahydropyrimidin-1(2H)-yl)benzoyl)piperidin-4-yl)methoxy)ethyl)piperazine-1 -carboxylate

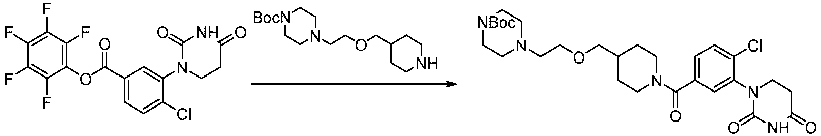

Pentafluorophenyl 4-chloro-3-(2,4-dioxotetrahydropyrimidin-1(2*H*)-yl)benzoate (140 mg, 0.32 mmol) was dissolved with DMSO (5 mL), and *tert*-butyl 4-(2-(piperidin-4-ylmethoxy)ethyl)piperazine-1-carboxylate (116 mg, 0.35 mmol) and DIEA (124 mg, 0.96 mmol) were added. The reaction liquid was stirred at room temperature for 2 h. Water (50 mL) was added, and the resulting mixture was stirred for 5 min and extracted with ethyl acetate (30 mL × 3). The organic phase was washed with saturated brine (50 mL), dried over anhydrous sodium sulfate, filtered, and concentrated under reduced pressure. The crude product was purified by column chromatography (MeOH:DCM = 1:9) to give *tert*-butyl 4-(2-((1-(4-chloro-3-(2,4-dioxotetrahydropyrimidin-1(2*H*)-yl)benzoyl)piperidin-4-yl)methoxy)ethyl)piperazine-1-carboxylate.

LC-MS: (ESI, *m*/*z*): [M+H] ⁺=578.2.

### Step 2: preparation of 1-(2-chloro-5-(4-((2-(piperazin-1-yl)ethoxy)methyl)piperidine-1-carbonyl)phenyl)dihydropyrimidine-2,4(1H,3H)-dione

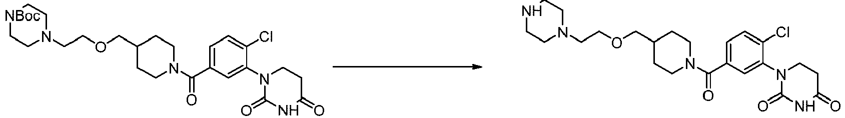

*tert*-Butyl 4-(2-((1-(4-chloro-3-(2,4-dioxotetrahydropyrimidin-1(2*H*)-yl)benzoyl)piperidin-4-yl)methoxy)ethyl)piperazine-1-carboxylate (170 mg, 0.29 mmol) was dissolved in DCM (5 mL), and TFA (1 mL) was added. The reaction liquid was stirred at room temperature for 2 h and concentrated under reduced pressure to give 1-(2-chloro-5-(4-((2-(piperazin-1-yl)ethoxy)methyl)piperidine-1-carbonyl)phenyl)dihydropyrimidine-2,4(1*H*,3*H*)-dione, which was directly used in the next step.

LC-MS: (ESI, *m*/*z*): [M+H] ⁺=478.2.

### Step 3: preparation of tert-butyl (3-((1R)-1-((6-(4-(4-(2-((1-(4-chloro-3-(2,4-dioxotetrahydropyrimidin-1(2H)-yl)benzoyl)piperidin-4-yl)methoxy)ethyl)piperazine-1-carbonyl)cyclohex-1-en-1-yl)-7-methoxy-2-methylquinazolin-4-yl)amino)ethyl)-5-(trifluoromethyl)phenyl)carboxylate

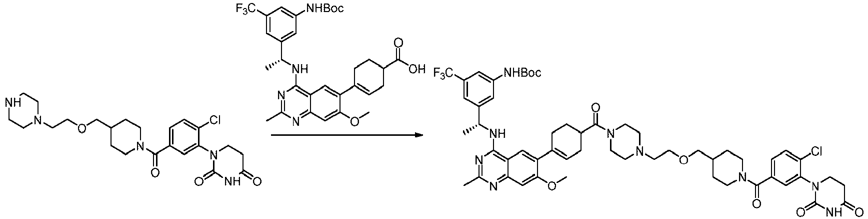

1-(2-Chloro-5-(4-((2-(piperazin-1-yl)ethoxy)methyl)piperidine-1-carbonyl)phenyl)dihydropyrimidine-2,4(1*H*,3*H*)-dione (170 mg, 0.36 mmol) was dissolved in DMF (5 mL), and 4-(4-(((*R*)-1-(3-((*tert-*butoxycarbonyl)amino)-5-(trifluoromethyl)phenyl)ethyl)amino)-7-methoxy-2-methylquinazolin-6-yl)cyclohex-3-ene-1-carboxylic acid (150 mg, 0.25 mmol), HATU (124 mg, 0.32 mmol), and DIEA (161 mg, 1.25 mmol) were added. The reaction liquid was stirred at room temperature for 2 h. Water (50 mL) was added, and the resulting mixture was stirred for 5 min and extracted with ethyl acetate (30 mL × 3). The organic phase was washed with saturated brine (50 mL), dried over anhydrous sodium sulfate, filtered, and concentrated under reduced pressure. The crude product was purified by column chromatography (MeOH:DCM = 1:9) to give *tert*-butyl (3-((1*R*)-1-((6-(4-(4-(2-((1-(4-chloro-3-(2,4-dioxotetrahydropyrimidin-1(2*H*)-yl)benzoyl)piperidin-4-yl)methoxy)ethyl)piperazine-1-carbonyl)cyclohex-1-en-1-yl)-7-methoxy-2-methylquinazolin-4-yl)amino)ethyl)-5-(trifluoromethyl)phenyl)carboxylate.

LC-MS: (ESI, *m*/*z*): [M+H] ⁺=1060.4.

### Step 4: preparation of 1-(5-(4-((2-(4-(4-(4-(((R)-1-(3-amino-5-(trifluoromethyl)phenyl)ethyl)amino)-7-methoxy-2-methylquinazolin-6-yl)cyclohex-3-ene-1-carbonyl)piperazin-1-yl)ethoxy)methyl)piperidine-1-carbonyl)-2-chlorophenyl)dihydropyrimidine-2,4(1H,3H)-dione

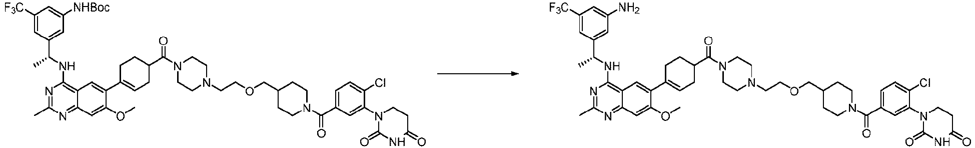

*tert*-Butyl (3-((1*R*)-1-((6-(4-(4-(2-((1-(4-chloro-3-(2,4-dioxotetrahydropyrimidin-1(2*H*)-yl)benzoyl)piperidin-4-yl)methoxy)ethyl)piperazine-1-carbonyl)cyclohex-1-en-1-yl)-7-methoxy-2-methylquinazolin-4-yl)amino)ethyl)-5-(trifluoromethyl)phenyl)carboxylate (140 mg, 0.13 mmol) was dissolved in DCM (5 mL), and TFA (1 mL) was added. The reaction liquid was stirred at room temperature for 2 h and concentrated under reduced pressure. The residue was dissolved with MeOH/DCM (1/9, 20 mL), and a saturated aqueous sodium bicarbonate solution (20 mL) was added to adjust the pH to neutrality. The resulting mixture was stirred for 10 min, extracted with MeOH/DCM (1/9, 20 mL × 3), dried over anhydrous sodium sulfate, filtered, and concentrated under reduced pressure. The crude product was purified by preparative reversed-phase chromatography to give 1-(5-(4-((2-(4-(4-(4-(((R)-1-(3-amino-5-(trifluoromethyl)phenyl)ethyl)amino)-7-methoxy-2-methylquinazolin-6-yl)cyclohex-3-en-1-carbonyl)piperazin-1-yl)ethoxy)methyl)piperidine-1-carbonyl)-2-chlorophenyl)dihydropyrimidine-2,4(1*H*,3*H*)-dione.

LC-MS: (ESI, *m*/*z*): [M+H] ⁺=960.4.

¹H NMR (400 MHz, DMSO-*d*₆) δ 10.50 (s, 1H), 8.18 (d, *J* = 7.8 Hz, 1H), 8.06 (s, 1H), 7.63 (d, *J =* 8.2 Hz, 1H), 7.54 (d, *J* = 2.0 Hz, 1H), 7.38 (dd, *J* = 8.2, 2.0 Hz, 1H), 6.99 (s, 1H), 6.88 (s, 1H), 6.85 (s, 1H), 6.69 (s, 1H), 5.83 (s, 1H), 5.55 - 5.45 (m, 3H), 4.50 - 4.36 (m, 1H), 3.85 (s, 3H), 3.75 (d, *J* = 7.2 Hz, 1H), 3.67 - 3.37 (m, 9H), 3.06 (s, 1H), 2.87 (s, 1H), 2.81 - 2.69 (m, 3H), 2.47 - 2.16 (m, 13H), 1.89 -1.41 (m, 9H), 1.20 - 1.13 (m, 2H).

### Example 54: 1-(2-chloro-5-(4-(3-(1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)propoxy)piperidine-1-carbonyl)phenyl)dihydropyrimidine-2,4(1H,3H)-dione

### Step 1: preparation of tert-butyl 4-(3-(p-toluenesulfonyl)propyl)piperidine-1-carboxylate

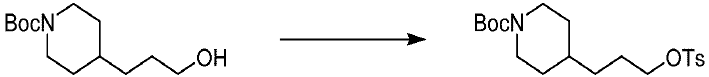

*tert*-Butyl 4-(3-hydroxypropyl)piperidine-1-carboxylate (3.6 g, 14.81 mmol) was dissolved with dichloromethane (20 mL), and TsCl (3.4 g, 17.80 mmol), DMAP (180 mg, 1.48 mmol), and TEA (4.5 g, 44.43 mmol) were separately added. The reaction liquid was stirred at room temperature overnight and directly concentrated under reduced pressure. The crude product was purified by column chromatography (PE:EA = 5:1) to give *tert*-butyl 4-(3-(*p-*toluenesulfonyl)propyl)piperidine-1 -carboxylate.

LC-MS: (ESI, *m*/*z*): [M+H] ⁺=398.1.

### Step 2: preparation of benzyl 4-(3-(1-(tert-butoxycarbonyl)piperidin-4-yl)propoxy)piperidine-1-carboxylate

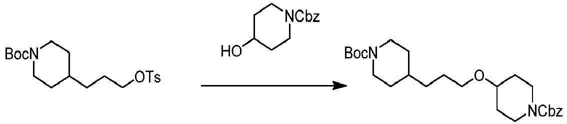

Benzyl 4-hydroxypiperidine-1-carboxylate (4.4 g, 18.51 mmol) was dissolved with anhydrous DMF (10 mL) and cooled to 0 °C, and NaH (0.6 g, 24.68 mmol) was added. The reaction liquid was stirred for 0.5 h, and then stirred at room temperature for 1 h. *tert*-Butyl 4-(3-(*p*-toluenesulfonyl)propyl)piperidine-1-carboxylate (4.9 g, 12.34 mmol) was added, and the reaction liquid was stirred at room temperature overnight. Water (100 mL) was added, and the resulting mixture was extracted with ethyl acetate (100 mL × 4), washed with saturated brine, dried over anhydrous sodium sulfate, and concentrated under reduced pressure. The crude product was purified by column chromatography (PE:EA = 5:1) to give benzyl 4-(3-(1-(*tert*-butoxycarbonyl)piperidin-4-yl)propoxy)piperidine-1-carboxylate.

LC-MS: (ESI, *m*/*z*): [M+H] ⁺=461.2.

### Step 3: preparation of tert-butyl 4-(3-(piperidine-4-oxy)propyl)piperidine-1-carboxylate

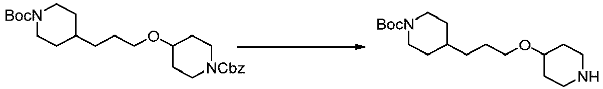

Benzyl 4-(3-(1-(*tert*-butoxycarbonyl)piperidin-4-yl)propoxy)piperidine-1-carboxylate (540 mg, 1.17 mmol) was dissolved with ethyl acetate (10 mL), and Pd(OH)₂/C (20%, 170 mg) was added. The reaction liquid was heated to 70 °C, stirred for 3 h, cooled, and filtered. The solid was washed three times with ethyl acetate (20 mL × 3) and concentrated under reduced pressure to give the crude product tert-butyl 4-(3-(piperidine-4-oxy)propyl)piperidine-1-carboxylate.

LC-MS: (ESI, *m*/*z*): [M+H] ⁺=327.3.

### Step 4: preparation of tert-butyl 4-(3-((1-(4-chloro-3-(2,4-dioxotetrahydropyrimidin-1(2H)-yl)benzoyl)piperidin-4-yl)oxy)propyl)piperidine-1-carboxylate

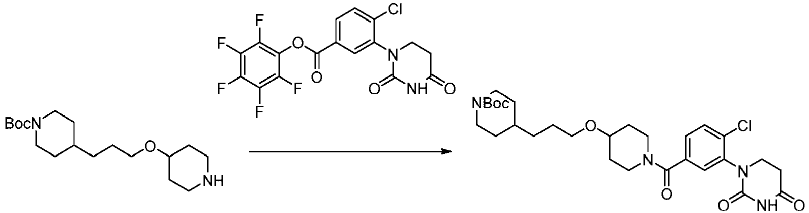

tert-Butyl 4-(3-(piperidine-4-oxy)propyl)piperidine-1-carboxylate (150 mg, 0.46 mmol) was dissolved with DMSO (10 mL), and pentafluorophenyl 4-chloro-3-(2,4-dioxotetrahydropyrimidin-1(2*H*)-yl)benzoate (200 mg, 0.46 mmol) and DIEA (178 mg, 1.38 mmol) were separately added. The reaction liquid was stirred at room temperature for 2 h. Water (50 mL) was added, and the resulting mixture was extracted with ethyl acetate (25 mL × 4), washed with saturated brine, dried over anhydrous sodium sulfate, and concentrated under reduced pressure. The crude product was purified by column chromatography (PE:EA = 1:10) to give *tert*-butyl 4-(3-((1-(4-chloro-3-(2,4-dioxotetrahydropyrimidin-1(2*H*)-yl)benzoyl)piperidin-4-yl)oxy)propyl)piperidine-1-carboxylate.

LC-MS: (ESI, *m*/*z*): [M+H] ⁺=577.3.

### Step 5: preparation of 1-(2-chloro-5-(4-(3-(piperidin-4-yl)propoxy)piperidine-1-carbonyl)phenyl)dihydropyrimidine-2,4(1H,3H)-dione

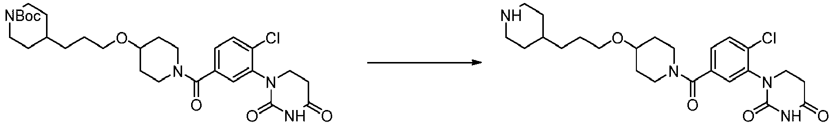

tert-Butyl 4-(3-((1-(4-chloro-3-(2,4-dioxotetrahydropyrimidin-1(2*H*)-yl)benzoyl)piperidin-4-yl)oxy)propyl)piperidine-1-carboxylate (220 mg, 0.58 mmol) was dissolved with DCM (5 mL), and TFA (1 mL) was added. The reaction liquid was stirred at room temperature overnight and concentrated under reduced pressure. The residue was diluted with water (10 mL), and a saturated sodium bicarbonate solution was slowly added dropwise to adjust the reaction liquid to neutrality. The resulting mixture was extracted with ethyl acetate (25 mL × 4), washed with saturated brine, dried over anhydrous sodium sulfate, and concentrated under reduced pressure to give the crude product 1-(2-chloro-5-(4-(3-(piperidin-4-yl)propoxy)piperidin-1-carbonyl)phenyl)dihydropyrimidine-2,4(1*H*,3*H*)-dione.

LC-MS: (ESI, *m*/*z*): [M+H] ⁺=477.2.

### Step 6: preparation of 1-(2-chloro-5-(4-(3-(1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)propoxy)piperidine-1-carbonyl)phenyl)dihydropyrimidine-2,4(1H,3H)-dione

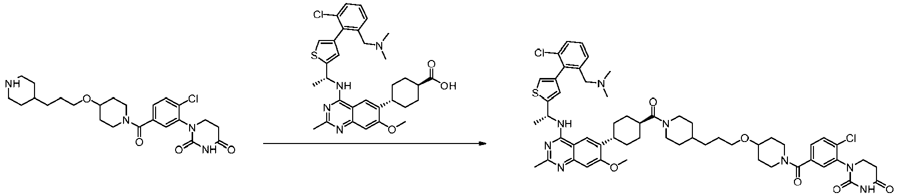

1-(2-Chloro-5-(4-(3-(piperidin-4-yl)propoxy)piperidine-1-carbonyl)phenyl)dihydropyrimidine-2,4(1*H*,3*H*)-dione (71 mg, 0.14 mmol), HATU (63 mg, 0.17 mmol), and DIEA (54 mg, 0.42 mmol) were added to a solution of (1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carboxylic acid (80 mg, 0.14 mmol) in DMF (10 mL). The reaction liquid was stirred at room temperature for 2 h. Water (100 mL) was added, and the resulting mixture was extracted with ethyl acetate (25 mL × 4), washed with saturated brine, dried over anhydrous sodium sulfate, and concentrated under reduced pressure. The crude product was purified by preparative reversed-phase chromatography to give 1-(2-chloro-5-(4-(3-(1-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1 -carbonyl)piperidin-4-yl)propoxy)piperidine-1 - carbonyl)phenyl)dihydropyrimidine-2,4(1*H*,3*H*)-dione.

LC-MS: (ESI, *m*/*z*): [M+H] ⁺=1051.4.

¹H NMR (400 MHz, DMSO-*d*₆) δ 10.50 (s, 1H), 8.31 (d, *J* = 8.4 Hz, 1H), 8.06 (s, 1H), 7.64 (d, *J* = 8.2 Hz, 1H), 7.57 (s, 1H), 7.42 (d, *J* = 8.5 Hz, 2H), 7.36 (d, *J* = 7.8 Hz, 1H), 7.24 (s, 1H), 7.02 (s, 1H), 6.97 (s, 1H), 5.97 (s, 1H), 4.45-4.35 (m, 1H), 3.98-3.84 (m, 5H), 3.82-3.71 (m, 1H), 3.67-3.57 (m, 1H), 3.61-3.50 (m, 2H), 3.41 (t, *J* = 6.3 Hz, 3H), 3.18-2.90 (m, 6H), 2.78-2.70 (m, 1H), 2.70-2.60 (m, 1H), 2.43 (s, 3H), 2.02 (s, 6H), 1.90-1.40 (m, 20H), 1.30-1.21 (m, 2H), 1.08-0.87 (m, 2H).

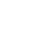

### Example 55: 1-(2-chloro-5-(4-((2-(1-((1R,4R)-4-(4-(((R)-1-(1,1-dioxido-2,3-dihydrobenzo[b]thiophen-4-yl)ethyl)amino)-7-methoxy-2-methylquinazolin-6-yl)cyclohexane-1-carbonyl)piperidin-4-yl)ethoxy)methyl)piperidine-1-carbonyl)phenyl)dihydropyrimidine-2,4(1H,3H)-dione

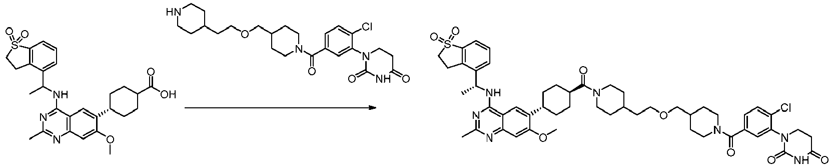

4-(4-((1-(1,1-Dioxido-2,3-dihydrobenzo[*b*]thiophen-4-yl)ethyl)amino)-7-methoxy-2-methylquinazolin-6-yl)cyclohexane-1-carboxylic acid (70 mg, 0.14 mmol) was dissolved with DMF (3 mL), and 1-(2-chloro-5-(4-((2-(piperidin-4-yl)ethoxy)methyl)piperidine-1-carbonyl)phenyl)dihydropyrimidine-2,4(1*H*,3*H*)-dione (73.3 mg, 0.15 mmol), HATU (79.8 mg, 0.21 mmol), and DIEA (91 mg, 0.7 mmol) were separately added. The reaction liquid was stirred at room temperature for 1 h, diluted with water (40 mL), and extracted with ethyl acetate (40 mL × 3). The organic phase was washed with saturated brine (40 mL), dried over anhydrous sodium sulfate, and concentrated under reduced pressure. The crude product was purified by prep-HPLC to give 1-(2-chloro-5-(4-((2-(1-((1*R*,4*R*)-4-(4-(((*R*)-1-(1,1-dioxide-2,3-dihydrobenzo[*b*]thiophen-4-yl)ethyl)amino)-7-methoxy-2-methylquinazolin-6-yl)cyclohexane-1 -carbonyl)piperidin-4-yl)ethoxy)methyl)piperidine-1 -carbonyl)phenyl)dihydropyrimidine-2,4(1*H*,3*H*)-dione.

LC-MS: (ESI, *m*/*z*): [M+H] ⁺=968.4.

¹H NMR (400 MHz, DMSO-*d*₆) δ 10.50 (s, 1H), 8.27 (d, *J* = 7.0 Hz, 1H), 8.06 (s, 1H), 7.80 (d, *J* = 7.6 Hz, 1H), 7.67-7.57 (m, 2H), 7.56 - 7.48 (m, 2H), 7.38 (d, *J* = 8.4 Hz, 1H), 6.98 (s, 1H), 5.59 (t, *J* = 7.4 Hz, 1H), 4.39 (d, *J* = 11.8 Hz, 2H), 3.91 (s, 1H), 3.87 (s, 3H), 3.81-3.70 (m, 2H), 3.67 - 3.59 (m, 3H), 3.54-3.46 (m, 1H), 3.42 (t, *J* = 6.4 Hz, 2H), 3.29 (s, 1H), 3.24 (d, *J* = 6.4 Hz, 2H), 3.11 - 2.90 (m, 3H), 2.82-2.62 (m, 4H), 2.32 (s, 3H), 2.12 - 1.23 (m, 20H), 1.20-0.90 (m, 4H).

### Example 56: 1-(2-chloro-5-(4-(4-(1-((1R,4R)-4-(4-(((R)-1-(4-(2-chloro-6-((dimethylamino)methyl)phenyl)thien-2-yl)ethyl)amino)-7-methoxy-2-methylquinazofin-6-yl)cyclohexane-1-carbonyl)piperidin-4-yl)butyl)piperazine-1-carbonyl)phenyl)dihydropyrimidine-2,4(1H,3H)-dione

### Step 1: preparation of tert-butyl 4-(4-(4-(4-chloro-3-(2,4-dioxotetrahydropyrimidin-1(2H)-yl)benzoyl)piperazin-1-yl)butyl)piperidine-1 -carboxylate

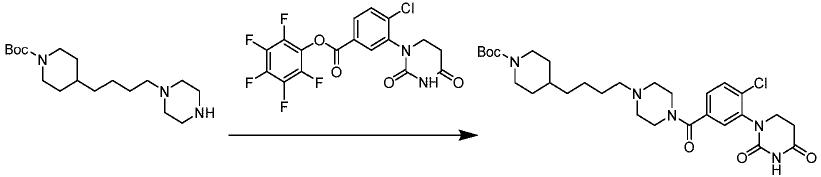

tert-Butyl 4-(4-(piperazin-1-yl)butyl)piperidine-1-carboxylate (100 mg, 0.31 mmol) and pentafluorophenyl 4-chloro-3-(2,4-dioxotetrahydropyrimidin-1(2*H*)-yl)benzoate (133 mg, 0.31 mmol) were dissolved in DMSO (3 mL), and DIEA (198 mg, 1.54 mmol) was added while stirring. The reaction liquid was stirred at room temperature for 1 h. After the reaction was completed, the reaction liquid was poured into water (30 mL), and the resulting mixture was extracted with EA (30 mL × 3). The organic phases were combined, washed with saturated brine, dried over anhydrous sodium sulfate, filtered, and concentrated. The crude product was purified by column chromatography (DCM:MeOH = 10:1) to give *tert*-butyl 4-(4-(4-(4-chloro-3-(2,4-dioxotetrahydropyrimidin-1(2*H*)-yl)benzoyl)piperazin-1 -yl)butyl)piperidine-1 -carboxylate.

LC-MS: (ESI, *m*/*z*): [M+H] ⁺=576.3.

### Step 2: preparation of 1-(2-chloro-5-(4-(4-(piperidin-4-yl)butyl)piperazine-1-carbonyl)phenyl)dihydropyrimidine-2,4(1H,3H)-dione

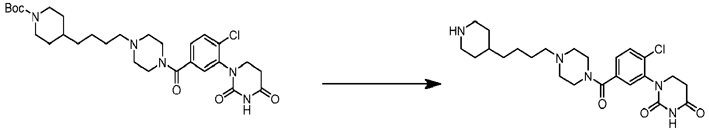

*tert*-Butyl 4-(4-(4-(4-chloro-3-(2,4-dioxotetrahydropyrimidin-1(2*H*)-yl)benzoyl)piperazin-1-yl)butyl)piperidine-1-carboxylate (112 mg, 0.19 mmol) was dissolved in DCM (2 mL), and 0.2 mL of TFA was added. The reaction liquid was reacted at room temperature for 1 h and directly concentrated under reduced pressure to give 1-(2-chloro-5-(4-(4-(piperidin-4-yl)butyl)piperazine-1-carbonyl)phenyl)dihydropyrimidine-2,4(1*H*,3*H*)-dione.

LC-MS: (ESI, *m*/*z*): [M+H] ⁺= 476.3

### Step 3: preparation of 1-(2-chloro-5-(4-(4-(1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)butyl)piperazine-1-carbonyl)phenyl)dihydropyrimidine-2,4(1H,3H)-dione

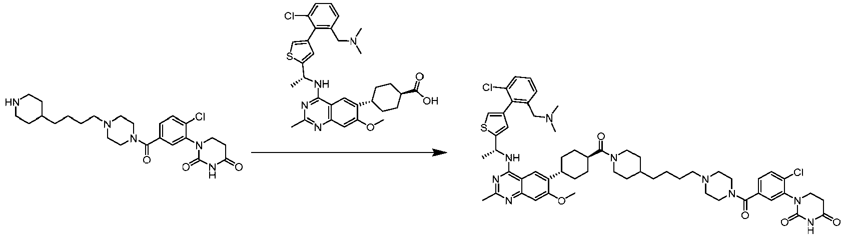

1-(2-Chloro-5-(4-(4-(piperidin-4-yl)butyl)piperazine-1-carbonyl)phenyl)dihydropyrimidine-2,4(1*H*,3*H*)-dione (165 mg, 0.195 mmol) and (1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carboxylic acid (115 mg, 0.195 mmol) were dissolved in DMF (3 mL), and HATU (86 mg, 0.227 mmol) and DIEA (75 mg, 0.584 mmol) were added while stirring. The reaction liquid was reacted at room temperature for 1 h. After the reaction was completed, the reaction liquid was poured into water (50 mL), and the resulting mixture was extracted with EA (30 mL × 3). The organic phases were combined, washed with saturated brine, dried over anhydrous sodium sulfate, filtered, and concentrated. The crude product was purified by prep-HPLC to give 1-(2-chloro-5-(4-(4-(1-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)butyl)piperazine-1-carbonyl)phenyl)dihydropyrimidine-2,4(1*H*,3*H*)-dione.

LC-MS: (ESI, *m*/*z*): [M+H] ⁺= 1050.7.

¹H NMR (400 MHz, DMSO-*d*₆) δ 10.50 (s, 1H), 8.30 (d, *J* = 8.3 Hz, 1H), 8.05 (s, 1H), 7.64 (d, *J* = 8.2 Hz, 1H), 7.56 (d, *J* = 2.0 Hz, 1H), 7.46 - 7.30 (m, 4H), 7.24 (d, *J* = 1.4 Hz, 1H), 7.02 (s, 1H), 6.96 (s, 1H), 5.96 (dd, *J* = 14.4, 7.2 Hz, 1H), 4.40 (d, *J* = 11.5 Hz, 1H), 3.89 (s, 4H), 3.78 - 3.71 (m, 1H), 3.62 (d, *J* = 6.0 Hz, 3H), 3.11 (d, *J* = 15.8 Hz, 2H), 3.01-2.92 (m, 2H), 2.76-2.63 (m, 2H), 2.68 - 2.58 (m, 2H), 2.42 (s, 6H), 2.34 - 2.18 (m, 4H), 2.01 (s, 6H), 1.85 (d, *J* = 9.6 Hz, 2H), 1.79 (d, *J* = 11.5 Hz, 2H), 1.72 (d, *J* = 7.0 Hz, 4H), 1.65 (d, *J* = 12.4 Hz, 2H), 1.60 - 1.50 (m, 3H), 1.42 (d, *J* = 7.4 Hz, 3H), 1.34 - 1.14 (m, 5H), 1.02 - 0.82 (m, 2H).

### Example 57: 1-(5-(4-((2-(1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)ethoxy)methyl)piperidine-1-carbonyl)-2-(trifluoromethyl)phenyl)dihydropyrimidine-2,4(1H,3H)-dione

### Step 1: preparation of tert-butyl 4 (2-((1-(3-(2,4-dioxotetrahydropyrimidin-1(2H)-yl)-4-(trifluoromethyl)benzoyl)piperidin-4-yl)methoxy)ethyl)piperidine-1-carboxylate

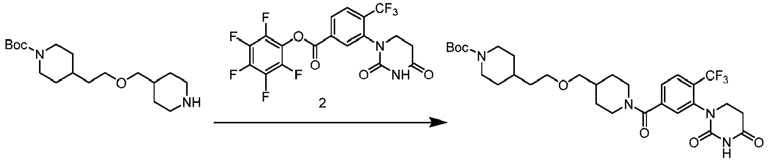

tert-Butyl 4-(2-(piperidin-4-ylmethoxy)ethyl)piperidine-1-carboxylate (100 mg, 0.31 mmol) and pentafluorophenyl 3-(2,4-dioxotetrahydropyrimidin-1(2*H*)-yl)-4-(trifluoromethyl)benzoate (133 mg, 0.31 mmol) were dissolved in DMSO (3 mL), and DIEA (198 mg, 1.54 mmol) was added while stirring. The reaction liquid was reacted at room temperature for 1 h. After the reaction was completed, the reaction liquid was diluted with water (30 mL) and extracted with ethyl acetate (30 mL × 3). The organic phases were combined, washed with saturated brine, dried over anhydrous sodium sulfate, filtered, and concentrated under reduced pressure. The crude product was isolated by column chromatography to give *tert*-butyl 4-(2-((1-(3-(2,4-dioxotetrahydropyrimidine-1(2*H*)-yl)-4-(trifluoromethyl)benzoyl)piperidin-4-yl)methoxy)ethyl)piperidine-1-carboxylate.

LC-MS: (ESI, *m*/*z*): [M+Na] ⁺=633.2.

### Step 2: preparation of 1-(5-(4-((2-(piperidin-4-yl)ethoxy)methyl)piperidine-1-carbonyl)-2-(trifluoromethyl)phenyl)dihydropyrimidine-2,4(1H,3H)-dione

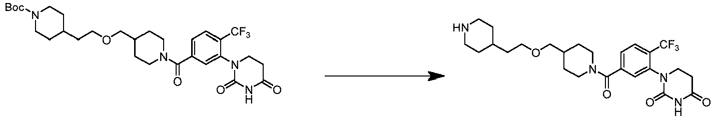

tert-Butyl 4-(2-((1-(3-(2,4-dioxotetrahydropyrimidin-1(2H)-yl)-4-(trifluoromethyl)benzoyl)piperidin-4-yl)methoxy)ethyl)piperidine-1-carboxylate (112 mg, 0.19 mmol) was dissolved in dichloromethane (2 mL), and trifluoroacetic acid (0.8 mL) was added. The reaction liquid was reacted at room temperature for 1 h. After the reaction was completed, the reaction liquid was concentrated under reduced pressure to give 1-(5-(4-((2-(piperidine-4-yl)ethoxy)methyl)piperidine-1-carbonyl)-2-(trifluoromethyl)phenyl)dihydropyrimidine-2,4(1*H*,3*H*)-dione, which was directly used in the next step without purification.

### Step 3: preparation of 1-(5-(4-((2-(1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)ethoxy)methyl)piperidine-1-carbonyl)-2-(trifluoromethyl)phenyl)dihydropyrimidine-2,4(1H,3H)-dione

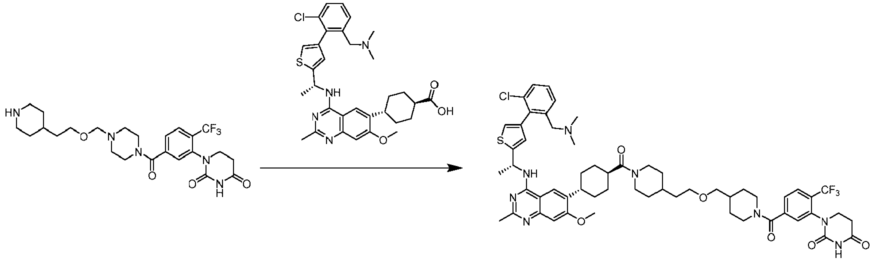

1-(5-(4-((2-(Piperidin-4-yl)ethoxy)methyl)piperidine-1-carbonyl)-2-(trifluoromethyl)phenyl)dihydropyrimidine-2,4(1*H*,3*H*)-dione (TFA salt, 165 mg, 0.195 mmol) and (1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carboxylic acid (115.3 mg, 0.195 mmol) were dissolved in DMF (3 mL), and HATU (86.3 mg, 0.227 mmol) and DIEA (75.4 mg, 0.584 mmol) were added while stirring. The reaction liquid was reacted at room temperature for 1 h. After the reaction was completed, the reaction liquid was diluted with water (30 mL) and extracted with ethyl acetate (30 mL × 3). The organic phases were combined, washed with saturated brine, dried over anhydrous sodium sulfate, filtered, and concentrated under reduced pressure. The crude product was purified by prep-HPLC to give 1-(5-(4-((2-(1-((1*R*,4*R*)-4-(4-(((*R*)-1-(4-(2-chloro-6-((dimethylamino)methyl)phenyl)thien-2-yl)ethyl)amino)-7-methoxy-2-methyl quinazolin-6-yl)cyclohexane-1 -carbonyl)piperidin-4-yl)ethoxy)methyl)piperidine-1 -carbonyl)-2-(trifluoromethyl)phenyl)dihydropyrimidine-2,4(1*H*,3*H*)-dione.

LC-MS: (ESI, *m*/*z*): [M+H] ⁺=1085.5.

¹H NMR (400 MHz, DMSO-*d*₆) δ 10.53 (s, 1H), 8.31 (d, *J* = 8.1 Hz, 1H), 8.05 (s, 1H), 7.87 (d, *J* = 8.1 Hz, 1H), 7.70 (s, 1H), 7.58 (d, *J* = 8.0 Hz, 1H), 7.43 (t, *J* = 8.0 Hz, 2H), 7.37 - 7.31 (m, 1H), 7.24 (d, *J* = 1.3 Hz, 1H), 7.02 (s, 1H), 6.96 (s, 1H), 6.00-5.92 (m, 1H), 4.53-4.31 (m, 2H), 3.94 - 3.82 (m, SH), 3.52-3.37 (m, 4H), 3.23 (d, *J* = 6.1 Hz, 2H), 3.12 (d, *J* = 2.1 Hz, 2H), 3.07 - 2.90 (m, 3H), 2.85-2.74 (m, 1H), 2.72-2.58 (m, 3H), 2.42 (s, 3H), 2.01 (s, 6H), 1.89-1.50 (m, 18H), 1.48-1.40 (m, 2H), 1.21-0.91 (m, 4H).

### Example 58: 1-(5-(4-((2-(1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)ethoxy)methyl)piperidine-1-carbonyl)-2-(trifluoromethoxy)phenyl)dihydropyrimidine-2,4(1H,3H)-dione Step 1: preparation of tert-butyl 4-(2-((1-(3-(2,4-dioxotetrahydropyrimidin-1(2H)-yl)-4-(trifluoromethoxy)benzoyl)piperidin-4-yl)methoxy)ethyl)piperidine-1-carboxylate

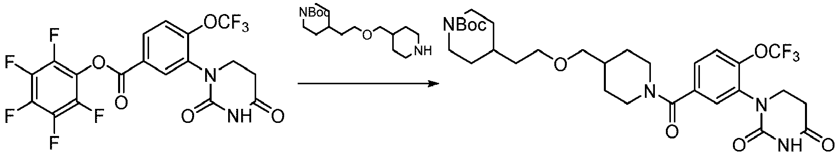

Pentafluorophenyl 3-(2,4-dioxotetrahydropyrimidin-1(2*H*)-yl)-4-(trifluoromethoxy)benzoate (150 mg, 0.31 mmol) and tert-butyl 4-(2-(piperidin-4-ylmethoxy)ethyl)piperidine-1-carboxylate (100 mg, 0.31 mmol) were dissolved in DMSO (3 mL), and DIEA (198 mg, 1.54 mmol) was added while stirring. The reaction liquid was stirred at room temperature for 1 h. After the reaction was completed, the reaction liquid was poured into water (30 mL), and the resulting mixture was extracted with EA (30 mL × 3). The organic phases were combined, washed with saturated brine, dried over anhydrous sodium sulfate, filtered, and concentrated. The crude product was purified by column chromatography (DCM:MeOH = 10:1) to give *tert*-butyl 4-(2-((1-(3-(2,4-dioxotetrahydropyrimidin-1(2*H*)-yl)-4-(trifluoromethoxy)benzoyl)piperidin-4-yl)methoxy)ethyl)piperidine-1-carboxylate.

LC-MS: (ESI, *m*/*z*): [M+Na] ⁺= 649.3.

### Step 2: preparation of 1-(5-(4-((2-(piperidin-4-yl)ethoxy)methyl)piperidine-1-carbonyl)-2-(trifluoromethoxy)phenyl)dihydropyrimidine-2,4(1H,3H)-dione

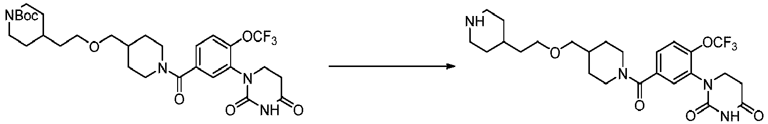

*tert*-Butyl 4-(2-((1-(3-(2,4-dioxotetrahydropyrimidin-1(2*H*)-yl)-4-(trifluoromethoxy)benzoyl)piperidin-4-yl)methoxy)ethyl)piperidine-1-carboxylate (110 mg, 0.17 mmol) was dissolved in DCM (2 mL), and TFA(0.2 mL) was added. The reaction liquid was reacted at room temperature for 1 h and directly concentrated under reduced pressure to give 1-(5-(4-((2-(piperidin-4-yl)ethoxy)methyl)piperidine-1-carbonyl)-2-(trifluoromethoxy)phenyl)dihydropyrimidine-2,4(1*H*,3*H*)-dione.

LC-MS: (ESI, *m*/*z*): [M+H] ⁺= 527.3

### Step 3: preparation of 1-(5-(4-((2-(1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)ethoxy)methyl)piperidine-1-carbonyl)-2-(trifluoromethoxy)phenyl)dihydropyrimidine-2,4(1H,3H)-dione

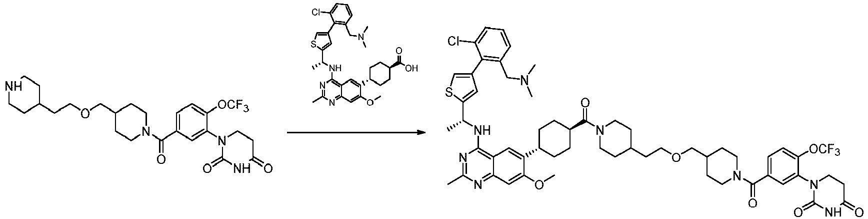

1-(5-(4-((2-(Piperidin-4-yl)ethoxy)methyl)piperidine-1-carbonyl)-2-(trifluoromethoxy)phenyl)dihydropyrimidine-2,4(1*H*,3*H*)-dione (90 mg, 0.17 mmol) and (1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carboxylic acid (100 mg, 0.17 mmol) were dissolved in DMF (3 mL), and HATU (84 mg, 0.22 mmol) and DIEA (66 mg, 0.51 mmol) were added while stirring. The reaction liquid was reacted at room temperature for 1 h. After the reaction was completed, the reaction liquid was poured into water (50 mL), and the resulting mixture was extracted with EA (30 mL × 3). The organic phases were combined, washed with saturated brine, dried over anhydrous sodium sulfate, filtered, and concentrated. The crude product was purified by prep-HPLC to give 1-(5-(4-((2-(1-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)ethoxy)methyl)piperidine-1-carbonyl)-2-(trifluoromethoxy)phenyl)dihydropyrimidine-2,4(1*H*,3*H*)-dione.

LC-MS: (ESI, *m*/*z*)*:* [M+H] ⁺= 1101.5.

¹H NMR (400 MHz, DMSO-*d*₆) δ 10.54 (s, 1H), 8.31 (d, *J* = 8.2 Hz, 1H), 8.05 (s, 1H), 7.59 (d, *J =* 2.0 Hz, 1H), 7.52 (dd, *J* = 8.4, 1.4 Hz, 1H), 7.49-7.30 (m, 4H), 7.24 (d, *J* = 1.3 Hz, 1H), 7.02 (s, 1H), 6.96 (s, 1H), 6.02 - 5.91 (m, 1H), 4.52 - 4.33 (m, 2H), 3.98-3.90 (m, 4H), 3.80-3.65 (m, 2H), 3.61-3.51 (m, 1H), 3.41 (t, *J* = 6.3 Hz, 2H), 3.23 (d, *J* = 6.2 Hz, 2H), 3.15 - 2.89 (m, 5H), 2.82-2.60 (m, 4H), 2.42 (s, 3H), 2.01 (s, 6H), 1.89 - 1.40 (m, 20H), 1.20-0.90 (m, 4H).

### Example 59: 1-(5-(9-((4-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)methyl)-3-azaspiro[5.5]undecane-3-carbonyl)-2-methoxyphenyl)dihydropyrimidine-2,4(1H,3H)-dione Step 1: preparation of tert-butyl 9-((4-((benzyloxy)carbonyl)piperazin-1-yl)methyl)-3-azaspiro[5.5]undecane-3-carboxylate

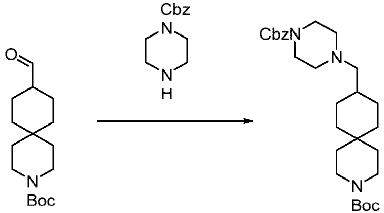

*tert*-Butyl 9-formyl-3-azaspiro[5.5]undecane-3-carboxylate (300 mg, 1.06 mmol) and benzyl piperazine-1-carboxylate (235 mg, 1.06 mmol) were dissolved with tetrahydrofuran (10 mL), and sodium triacetoxyborohydride (674 mg, 3.18 mmol) was added while stirring. The reaction liquid was stirred at room temperature for 2 h. Water (50 mL) was added, and the resulting mixture was extracted with dichloromethane (30 mL × 3). The organic phase was washed with saturated brine (100 mL), dried over anhydrous sodium sulfate, filtered, and concentrated under reduced pressure. The crude product was purified by column chromatography (methanol:dichloromethane = 1:9) to give tert-butyl 9-((4-((benzyloxy)carbonyl)piperazin-1-yl)methyl)-3-azaspiro[5.5]undecane-3-carboxylate.

LC-MS: (ESI, *m*/*z*): [M+H] ⁺=486.2.

### Step 2: preparation of tert-butyl 9-(piperazin-1-ylmethyl)-3-azaspiro[5.5]undecane-3-carboxylate

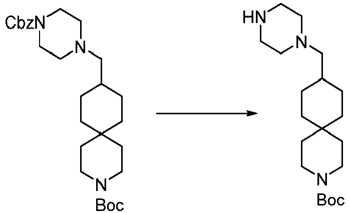

*tert*-Butyl 9-((4-((benzyloxy)carbonyl)piperazin-1-yl)methyl)-3-azaspiro[5.5]undecane-3-carboxylate (500 mg, 1.02 mmol) was dissolved in ethyl acetate (15 mL), and Pd(OH)₂/C (250 mg, 0.35 mmol) was added. The reaction liquid was heated to 70 °C under hydrogen atmosphere and stirred overnight. The reaction liquid was cooled to room temperature and filtered, and the filtrate was concentrated under reduced pressure to give tert-butyl 9-(piperazine-1-ylmethyl)-3-azaspiro[5.5]undecane-3-carboxylate. The crude product was directly used in the next step without purification.

LC-MS: (ESI, *m*/*z*): [M+H] ⁺=352.2.

### Step 3: preparation of tert-butyl 9-((4-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)methyl)-3-azaspiro 5.5 undecane-3 -carboxylate

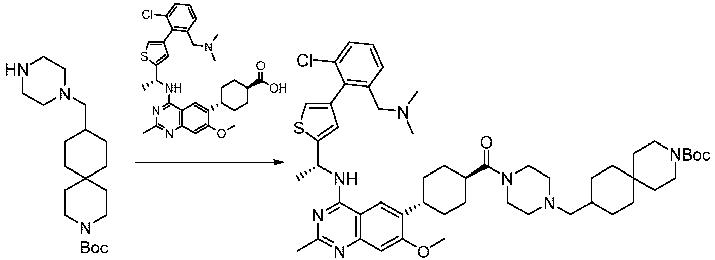

*tert*-Butyl 9-(piperazin-1-ylmethyl)-3-azaspiro[5.5]undecane-3-carboxylate (130 mg, 0.36 mmol) was dissolved in DMF (10 mL), and (1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carboxylic acid (200 mg, 0.33 mmol), HATU (188 mg, 0.49 mmol), and DIEA (127 mg, 0.99 mmol) were added. The mixture was stirred at room temperature for 1 h. Water (100 mL) was added to the reaction liquid, and the resulting mixture was extracted with ethyl acetate (3 × 30 mL). The organic phase was washed with saturated brine (100 mL), dried over anhydrous sodium sulfate, filtered, and concentrated under reduced pressure. The crude product was purified by column chromatography (methanol:dichloromethane = 1:9) to give *tert*-butyl 9-((4-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1 -yl)methyl)-3-azaspiro [5.5]undecane-3 -carboxylate.

LC-MS: (ESI, *m*/*z*): [M+H] ⁺=926.4.

### Step 4: preparation of (4-((3-azaspiro[5.5]undecan-9-yl)methyl)piperazin-1-yl)((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexyl)methanone

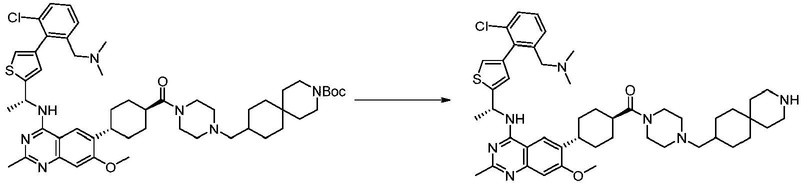

*tert*-Butyl 9-((4-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)methyl)-3-azaspiro [5.5]undecane-3 - carboxylate (230 mg, 0.25 mmol) was dissolved in DCM (3 mL), and TFA (1 mL) was added. The reaction liquid was stirred at room temperature for 2 h. The reaction liquid was directly concentrated under reduced pressure to give (4-((3-azaspiro[5.5]undecane-9-yl)methyl)piperazine-1-yl)((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexyl)methanone.

LC-MS: (ESI, *m*/*z*): [M+H] ⁺=826.5.

### Step 5: preparation of 1-(5-(9-((4-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)methyl)-3-azaspiro[5.5]undecane-3-carbonyl)-2-methoxyphenyl)dihydropyrimidine-2,4(1H,3H)-dione

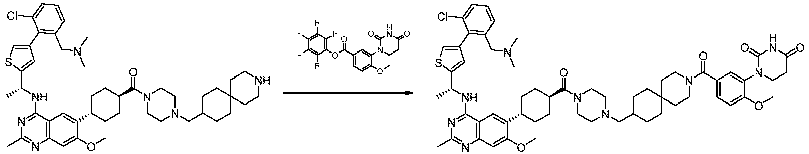

(4-((3-Azaspiro[5.5]undecan-9-yl)methyl)piperazin-1-yl) ((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexyl)methanone (110 mg, 0.12 mmol) was dissolved in DMSO (10 mL), and pentafluorophenyl 3-(2,4-dioxotetrahydropyrimidin-1(2*H*)-yl)-4-methoxybenzoate (57 mg, 0.13 mmol) and DIEA (46 mg, 0.36 mmol) were added. The reaction liquid was stirred at room temperature for 2 h. Water (100 mL) was added to the reaction liquid, and the resulting mixture was extracted with dichloromethane (30 mL × 3). The organic phase was washed with a saturated aqueous sodium chloride solution (100 mL), dried over anhydrous sodium sulfate, filtered, and concentrated under reduced pressure to give the crude product. The crude product was purified by pre-HPLC (CH₃CN/0.08% aqueous NH₄HCO₃ solution, 5%-95%) to give 1-(5-(9-((4-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)methyl)-3-azaspiro[5.5]undecane-3-carbonyl)-2-methoxyphenyl)dihydropyrimidine-2,4(1*H*,3*H*)-dione.

LC-MS: (ESI, *m*/*z*): [M+H] ⁺=1072.5.

¹H NMR (400 MHz, DMSO-*d*₆) δ 10.32 (s, 1H), 8.32 (d, *J* = 8.1 Hz, 1H), 8.05 (s, 1H), 7.43 (t, *J* = 8.1 Hz, 2H), 7.38 - 7.33 (m, 2H), 7.32 (d, *J* = 2.0 Hz, 1H), 7.24 (s, 1H), 7.15 (d, *J* = 8.6 Hz, 1H), 7.02 (s, 1H), 6.96 (s, 1H), 6.00 - 5.92 (m, 1H), 3.89 (s, 3H), 3.84 (s, 3H), 3.59 (t, *J* = 6.6 Hz, 2H), 3.49-3.37 (m, 4H), 3.17 - 3.09 (m, 2H), 2.94 (t, *J* = 12.0 Hz, 1H), 2.68 (t, *J* = 6.6 Hz, 2H), 2.65-2.43 (m, 2H), 2.42 (s, 3H), 2.33-2.26 (m, 4H), 2.16-2.08 (m, 2H), 2.02 (s, 6H), 1.83-1.46 (m, 21H), 1.35-1.24 (m, 2H), 1.16-0.90 (m, 4H).

### Example 60: 1-(5-(9-((4-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)methyl)-3-azaspiro[5.5]undecane-3-carbonyl)-2-fluorophenyl)dihydropyrimidine-2,4(1H,3H)-dione

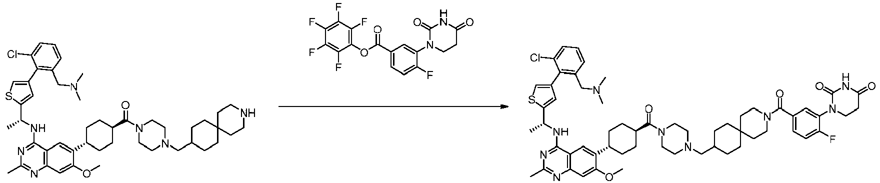

(4-((3-Azaspiro[5.5]undecan-9-yl)methyl)piperazin-1-yl)((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexyl)methanone (110 mg, 0.12 mmol) was dissolved in DMSO (5 mL), and pentafluorophenyl 3-(2,4-dioxotetrahydropyrimidin-1(2*H*)-yl)-4-fluorobenzoate (54 mg, 0.13 mmol) and DIEA (46 mg, 0.36 mmol) were added. The reaction liquid was stirred at room temperature for 2 h. Water (50 mL) was added to the reaction liquid, and the resulting mixture was extracted with dichloromethane (30 mL × 3). The organic phase was washed with a saturated aqueous sodium chloride solution (50 mL), dried over anhydrous sodium sulfate, filtered, and concentrated under reduced pressure to give the crude product. The crude product was purified by pre-HPLC (CH₃CN/0.08% aqueous NH₄HCO₃ solution, 5%-95%) to give 1-(5-(9-((4-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)methyl)-3-azaspiro[5.5]undecane-3-carbonyl)-2-fluorophenyl)dihydropyrimidine-2,4(1*H*,3*H*)-dione.

LC-MS: (ESI, *m*/*z*): [M+H] ⁺=1060.5.

¹H NMR (400 MHz, DMSO-*d*₆) δ 10.52 (s, 1H), 8.32 (d, *J* = 8.3 Hz, 1H), 8.05 (s, 1H), 7.50 (d, *J* = 7.9 Hz, 1H), 7.38 (m, 5H), 7.24 (s, 1H), 7.02 (s, 1H), 6.96 (s, 1H), 6.04 - 5.88 (m, 1H), 3.89 (s, 3H), 3.75 (t, *J* = 6.6 Hz, 2H), 3.57 (s, 2H), 3.46 (s, 4H), 3.30 - 3.26 (m, 1H), 3.11 (d, *J* = 15.1 Hz, 2H), 2.94 (m, 1H), 2.73 (t, *J* = 6.6 Hz, 2H), 2.63 (m, 1H), 2.42 (s, 3H), 2.32 (s, 2H), 2.29 (s, 2H), 2.11 (s, 2H), 2.01 (s, 6H), 1.90 - 1.39 (m, 19H), 1.34 (m, 2H), 1.07 (m, 4H).

### Example 61: 1-(2-chloro-5-(4-(3-((1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)oxy)propyl)piperidine-1-carbonyl)phenyl)dihydropyrimidine-2,4(1H,3H)-dione

### Step 1: preparation of tert-butyl 4-(3-((1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)oxy)propyl)piperidine-1-carboxylate

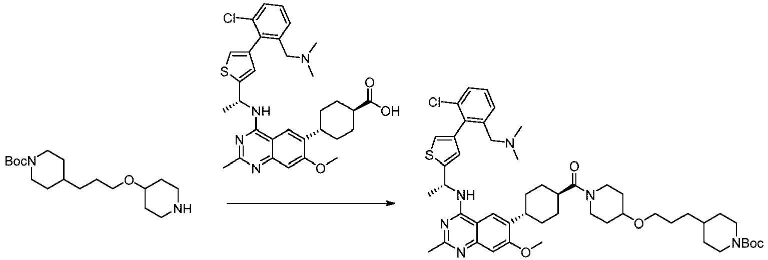

*tert*-Butyl 4-(3-(piperidine-4-oxy)propyl)piperidine-1-carboxylate (105 mg, 0.32 mmol), HATU (145 mg, 0.38 mmol), and DIEA (123 mg, 0.96 mmol) were added to a solution of (1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carboxylic acid (190 mg, 0.32 mmol) in DMF (5 mL). The reaction liquid was stirred at room temperature for 2 h, diluted with water (50 mL), extracted with ethyl acetate (25 mL × 4), washed with saturated brine, dried over anhydrous sodium sulfate, and concentrated under reduced pressure to give the crude product *tert*-butyl 4-(3-((1-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)oxy)propyl)piperidine-1-carboxylate.

LC-MS: (ESI, *m*/*z*): [M+H] ⁺=901.4.

### Step 2: preparation of ((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexyl)(4-(3-(piperidin-4-yl)propoxy)piperidin-1-yl)methanone

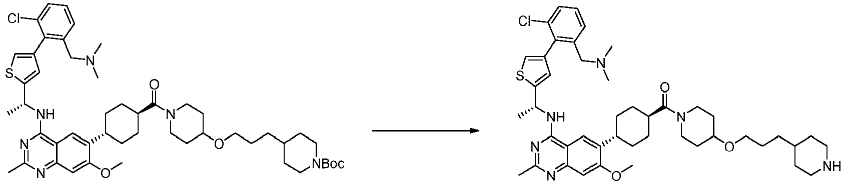

*tert*-Butyl 4-(3-((1-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)oxy)propyl)piperidine-1-carboxylate (170 mg, 0.19 mmol) was dissolved with DCM (5 mL), and TFA (1 mL) was added. The reaction liquid was stirred at room temperature for 2 h and directly concentrated under reduced pressure to give the crude product ((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexyl)(4-(3-(piperidin-4-yl)propoxy)piperidin-1-yl)methanone.

LC-MS: (ESI, *m*/*z*): [M+H] ⁺=801.4.

### Step 3: preparation of 1-(2-chloro-5-(4-(3-((1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)oxy)propyl)piperidine-1-carbonyl)phenyl)dihydropyrimidine-2,4(1H,3H)-dione

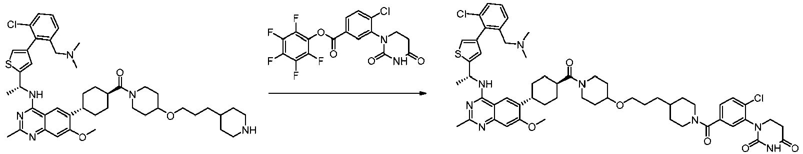

((1*R*,4*R*)-4-(4-(((*R*)-1-(4-(2-Chloro-6-((dimethylamino)methyl)phenyl)thien-2-yl)ethyl)amino)-7-methoxy-2-methylquinazolin-6-yl)cyclohexyl)(4-(3-(piperidin-4-yl)propoxy)piperidin-1-yl)methanone (150 mg, 0.19 mmol) was dissolved with DMSO (10 mL), and pentafluorophenyl 4-chloro-3-(2,4-dioxotetrahydropyrimidin-1(2*H*)-yl)benzoate (97 mg, 0.23 mmol) and DIEA (72 mg, 0.56 mmol) were separately added. The reaction liquid was stirred at room temperature for 2 h, diluted with water (50 mL), extracted with ethyl acetate (25 mL × 4), washed with saturated brine, drying over anhydrous sodium sulfate, and concentrated under reduced pressure. The crude product was purified by preparative reversed-phase chromatography to give 1-(2-chloro-5-(4-(3-((1-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1 -carbonyl)piperidin-4-yl)oxy)propyl)piperidine-1 -carbonyl)phenyl)dihydropyrimidine-2,4(1*H*,3*H*)-dione.

LC-MS: (ESI, *m*/*z*): [M+H] ⁺=1051.4.

¹H NMR (400 MHz, DMSO-*d*₆) δ 10.49 (s, 1H), 8.30 (d, *J* = 8.2 Hz, 1H), 8.05 (s, 1H), 7.63 (d, *J* = 8.2 Hz, 1H), 7.54 (d, *J* = 1.7 Hz, 1H), 7.48 - 7.30 (m, 4H), 7.24 (s, 1H), 7.02 (s, 1H), 6.96 (s, 1H), 6.03-5.87 (m, 1H), 4.53-4.33 (m, 1H), 3.93-3.81 (m, 4H), 3.79-3.45 (m, 5H), 3.41 (t, *J* = 6.2 Hz, 2H), 3.27 - 3.20 (m, 1H), 3.18 - 2.99 (m, 4H), 2.99-2.89 (m, 1H), 2.80-2.60 (m, 4H), 2.42 (s, 3H), 2.01 (s, 6H), 1.88 - 1.23 (m, 22H), 1.10-1.00 (m, 2H).

### Example 62: 1-(2-chloro-5-(9-(2-(4-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)ethyl)-3-azaspiro[5.5]undecane-3-carbonyl)phenyl)dihydropyrimidine-2,4(1H,3H)-dione

### Step 1: preparation of (9H-fluoren-9-yl)methyl 4-(2-(3-azaspiro[5.5]undecan-9-yl)ethyl)piperidine-1-carboxylate

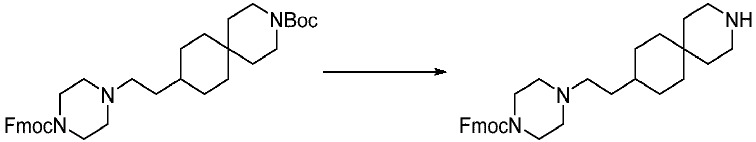

The compound *tert*-butyl 9-(2-(4-(((9*H*-fluoren-9-yl)methoxy)carbonyl)piperazin-1-yl)ethyl)-3-azaspiro[5.5]undecane-3-carboxylate (70 mg, 0.12 mmol) was dissolved in a solution of hydrogen chloride in 1,4-dioxane (4 M, 7 mL). The reaction liquid was stirred at room temperature for 1 h and directly concentrated under reduced pressure to give (9*H*-fluoren-9-yl)methyl 4-(2-(3-azaspiro[5.5]undecane-9-yl)ethyl)piperidine-1-carboxylate.

LC-MS: (ESI, *m*/*z*): [M+H] ⁺= 488.5.

### Step 2: preparation of (9H-fluoren-9-yl)methyl 4-(2-(3-(4-chloro-3-(2,4-dioxotetrahydropyrimidin-1(2H)-yl)benzoyl)-3-azaspiro[5.5]undecan-9-yl)ethyl)piperazine-1-carboxylate

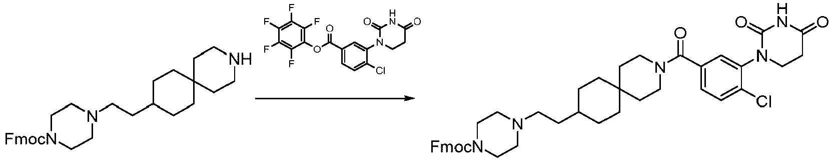

(9*H*-Fluoren-9-yl)methyl 4-(2-(3-azaspiro[5.5]undecan-9-yl)ethyl)piperidine-1-carboxylate (70 mg, 0.12 mmol) and pentafluorophenyl 4-chloro-3-(2,4-dioxotetrahydropyrimidin-1(2*H*)-yl)benzoate (52 mg, 0.12 mmol) were dissolved with DMSO (4 mL), and DIPEA (46 mg, 0.36 mmol) was added. The reaction liquid was stirred at room temperature overnight. The reaction liquid was diluted with water (40 mL), extracted with ethyl acetate (30 mL × 2), dried over anhydrous sodium sulfate, filtered, and concentrated. The crude product was purified by column chromatography (EA in PE = 0-4:1) to give (9*H*-fluoren-9-yl)methyl 4-(2-(3-(4-chloro-3-(2,4-dioxotetrahydropyrimidin-1(2*H*)-yl)benzoyl)-3-azaspiro[5.5]undecan-9-yl)ethyl)piperazine-1-carboxylate.

LC-MS: (ESI, *m*/*z*): [M+H] ⁺= 738.5.

### Step 3: preparation of 1-(2-chloro-5-(9-(2-(piperazin-1-yl)ethyl)-3-azaspiro[5.5]undecane-3-carbonyl)phenyl)dihydropyrimidine-2,4(1H,3H)-dione

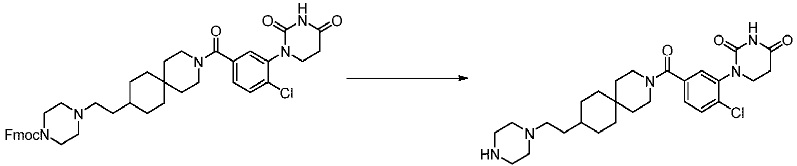

(9*H*-Fluoren-9-yl)methyl 4-(2-(3-(4-chloro-3-(2,4-dioxotetrahydropyrimidin-1(2*H*)-yl)benzoyl)-3-azaspiro[5.5]undecan-9-yl)ethyl)piperazine-1-carboxylate (70 mg, 0.09 mmol) was dissolved with DCM (2 mL), and piperidine (0.3 mL) was added. The reaction liquid was stirred at room temperature for 3 h, diluted with water (20 mL), extracted with dichloromethane (30 mL × 2), dried over anhydrous sodium sulfate, filtered, and concentrated. The crude product was purified by column chromatography (DCM:MeOH = 95:5) to give 1-(2-chloro-5-(9-(2-(piperazin-1-yl)ethyl)-3-azaspiro[5.5]undecane-3-carbonyl)phenyl)dihydropyrimidine-2,4(1H,3*H*)-dione. LC-MS: (ESI, *m*/*z*): [M+Na] ⁺= 516.3.

### Step 4: preparation of 1-(2-chloro-5-(9-(2-(4-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)ethyl)-3-azaspiro[5.5]undecane-3-carbonyl)phenyl)dihydropyrimidine-2,4(1H,3H)-dione

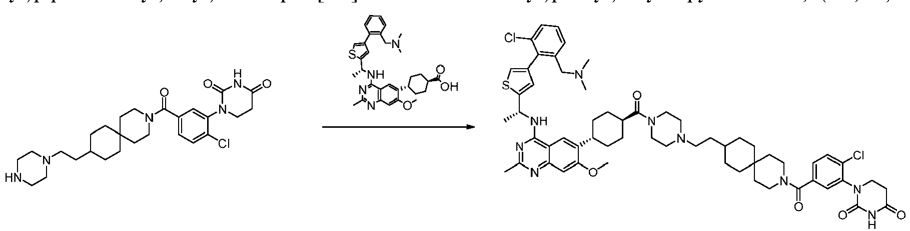

1-(2-Chloro-5-(9-(2-(piperazin-1-yl)ethyl)-3-azaspiro[5.5]undecane-3-carbonyl)phenyl)dihydropyrimidine-2,4(1*H*,3*H*)-dione (30 mg, 0.06 mmol) and (1*R*,4*R*)-4-(4-(((*R*)-1-(4-(2-((dimethylamino)methyl)phenyl)thien-2-yl)ethyl)amino)-7-methoxy-2-methylquinazolin-6-yl)cyclohexane-1-carboxylic acid (34 mg, 0.06 mmol) were dissolved with DMF (3 mL), and HATU (27 mg, 0.07 mmol) and DIEA (23 mg, 0.18 mmol) were added. The reaction liquid was stirred at room temperature overnight. The reaction liquid was diluted with water (30 mL), extracted with ethyl acetate (30 mL × 3), dried over anhydrous sodium sulfate, and concentrated under reduced pressure. The crude product was purified by prep-HPLC to give 1-(2-chloro-5-(9-(2-(4-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)ethyl)-3-azaspiro[5.5]undecane-3-carbonyl)phenyl)dihydropyrimidine-2,4(1*H*,3*H*)-dione.

LC-MS: (ESI, *m*/*z*): [M+H] ⁺= 1090.5.

¹H NMR (400 MHz, DMSO-*d*₆) δ 10.50 (s, 1H), 8.30 (d, *J* = 8.1 Hz, 1H), 8.05 (s, 1H), 7.63 (d, *J* = 8.2 Hz, 1H), 7.54 (s, 1H), 7.47 - 7.28 (m, 4H), 7.24 (s, 1H), 7.02 (s, 1H), 6.96 (s, 1H), 6.00-5.92 (m, 1H), 3.89 (s, 3H), 3.80-3.71 (m, 1H), 3.66-3.42 (m, 8H), 3.12 (s, 2H), 3.00-2.90 (m, 1H), 2.78-2.71 (m, 2H), 2.65-2.57 (m, 2H), 2.42 (s, 3H), 2.36-2.23 (m, 6H), 2.01 (s, 6H), 1.89-1.75 (m, 4H), 1.72-1.29 (m, 18H), 1.16-1.00 (m, 4H).

### Example 63: 1-(5-(4-((3-(1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)propyl)(methyl)amino)piperidine-1-carbonyl)-2-chlorophenyl)dihydropyrimidine-2,4(1H,3H)-dione

### Step 1: preparation of tert-butyl 4-(3-hydroxypropyl)piperidine-1-carboxylate

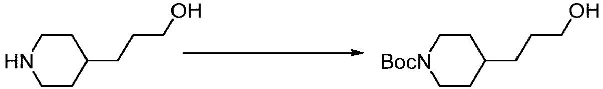

3-(Piperidin-4-yl)propan-1-ol (700 mg, 4.9 mmol) was dissolved in 1,4-dioxane (7 mL), and Boc anhydride (2.1 g, 9.8 mmol) and DMAP (30 mg, 0.24 mmol) were added. The reaction liquid was heated to 80 °C overnight. After the reaction was completed, the reaction liquid was poured into water (60 mL), and the resulting mixture was extracted with ethyl acetate (30 mL × 3) and dried over anhydrous sodium sulfate. The organic phase was concentrated by rotary evaporation. The crude product was purified by column chromatography (EA:PE = 0-3:7) to give tert-butyl 4-(3-hydroxypropyl)piperidine-1-carboxylate.

¹H NMR (400 MHz, DMSO-*d*₆) δ 4.34 (t, *J* = 5.1 Hz, 1H), 3.91 (d, *J* = 12.3 Hz, 2H), 3.37 (dd, *J* = 11.9, 6.3 Hz, 2H), 2.66 (s, 2H), 1.61 (d, *J* = 12.3 Hz, 2H), 1.50 - 1.28 (m, 12H), 1.20 (dd, *J =* 15.2, 6.9 Hz, 2H), 0.93 (m, 2H).

### Step 2: preparation of tert-butyl 4-(3-propionaldehyde)piperidine-1-carboxylate

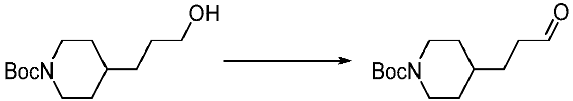

tert-Butyl 4-(3-hydroxypropyl)piperidine-1-carboxylate (300 mg, 1.2 mmol) was dissolved in acetonitrile (5 mL), and IBX (567 mg, 2.4 mmol) was added. The reaction liquid was heated to 70 °C, reacted for 2 h, and filtered, and the filter cake was washed with ethyl acetate (10 mL × 2). The organic phase was concentrated by rotary evaporation. The crude product was purified by column chromatography (EA:PE = 0-1:4) to give *tert-*butyl 4-(3-propionaldehyde)piperidine-1-carboxylate.

¹H NMR (400 MHz, DMSO-*d*₆) δ 9.68 (t, *J* = 1.5 Hz, 1H), 3.91 (d, *J* = 12.0 Hz, 2H), 2.65 (m, 2H), 2.46 (td, *J* = 7.5, 1.5 Hz, 2H), 1.61 (d, *J* = 13.0 Hz, 2H), 1.46 (dd, *J* = 14.5, 7.3 Hz, 2H), 1.42 - 1.33 (m, 10H), 0.94 (m, 2H). Step 3: preparation of benzyl 4-((3-(1-(*tert*-butoxycarbonyl)piperidin-4-yl)propyl)(methyl)amino)piperidine-1-carboxylate

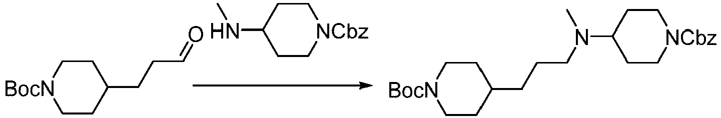

*tert*-Butyl 4-(3-propionaldehyde)piperidine-1-carboxylate (200 mg, 0.82 mmol) and benzyl 4-(methylamino)piperidine-1-carboxylate (205 mg, 0.82 mmol) were dissolved in tetrahydrofuran (4 mL), and sodium triacetoxyborohydride (525 mg, 2.48 mmol) was added. The reaction liquid was stirred at room temperature overnight. The reaction liquid was diluted with water (40 mL), extracted with ethyl acetate (30 mL × 3), dried over anhydrous sodium sulfate, filtered, and concentrated. The crude product was purified by column chromatography (EA:PE = 0-3:7) to give benzyl 4-((3-(1-(*tert*-butoxycarbonyl)piperidin-4-yl)propyl)(methyl)amino)piperidine-1-carboxylate as a white oil.

LC-MS: (ESI, *m*/*z*): [M+H] ⁺=474.3.

### Step 4: preparation of benzyl 4-(methyl(3-(piperidin-4-yl)propyl)amino)piperidine-1-carboxylate

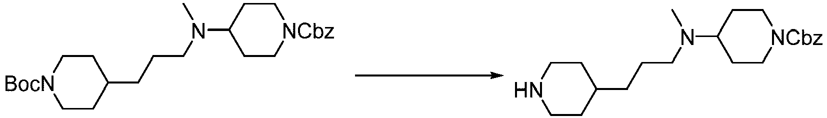

Benzyl 4-((3-(1-(*tert*-butoxycarbonyl)piperidin-4-yl)propyl)(methyl)amino)piperidine-1-carboxylate (90 mg, 0.19 mmol) was dissolved in DCM (5 mL), and trifluoroacetic acid (0.5 mL) was added. The reaction liquid was stirred at room temperature for 1 h and directly concentrated under reduced pressure to give benzyl 4-(methyl(3-(piperidin-4-yl)propyl)amino)piperidine-1-carboxylate as an oil.

LC-MS: (ESI, *m*/*z*): [M+H] ⁺= 374.1.

### Step 5: preparation of benzyl 4-((3-(1-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carbonyl)piperidin-4-yl)propyl)(methyl)amino)piperidine-1 -carboxylate

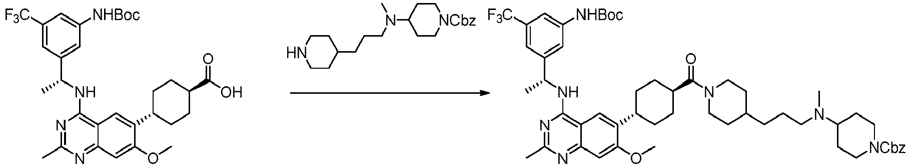

(1*R*,4*R*)-4-(4-(((*R*)-1-(3-((*tert*-Butoxycarbonyl)amino)-5-(trifluoromethyl)phenyl)ethyl)amino)-7-methoxy-2-methylquinazolin-6-yl)cyclohexane-1-carboxylic acid (110 mg, 0.18 mmol) and benzyl 4-(methyl(3-(piperidin-4-yl)propyl)amino)piperidine-1-carboxylate (trifluoroacetate salt, 90 mg, 0.18 mmol) were dissolved in DMF (3 mL), and HATU (83 mg, 0.22 mmol) and DIEA (70 mg, 0.54 mmol) were added. The reaction liquid was stirred at room temperature overnight, diluted with water (30 mL), extracted with ethyl acetate (30 mL × 3), dried over anhydrous sodium sulfate, filtered, and concentrated. The crude product was purified by column chromatography (MeOH:DCM = 0-1:9) to give benzyl 4-((3-(1-((1*R*,4*R*)-4-(4-(((*R*)-1-(3-((*tert*-butoxycarbonyl)amino)-5-(trifluoromethyl)phenyl)ethyl)amino)-7-methoxy-2-methylquinazolin-6-yl)cyclohexane-1-carbonyl)piperidin-4-yl)propyl)(methyl)amino)piperidine-1-carboxylate.

LC-MS: (ESI, *m*/*z*): [M+H] ⁺=958.2.

### Step 6: preparation of tert-butyl (3-((R)-1-((7-methoxy-2-methyl-6-((1R,4R)-4-(4-(3-(methyl(piperidin-4-yl)amino)propyl)piperidine-1-carbonyl)cyclohexyl)quinazolin-4-yl)amino)ethyl)-5 - (trifluoromethyl)phenyl)carboxylate

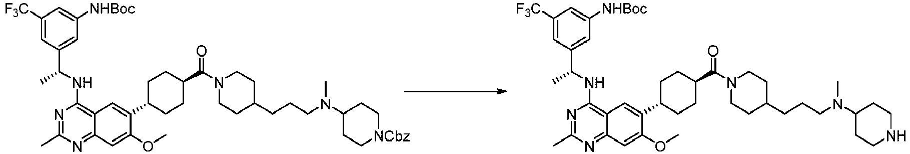

Benzyl 4-((3-(1-((1*R*,4*R*)-4-(4-(((*R*)-1-(3-((*tert*-butoxycarbonyl)amino)-5-(trifluoromethyl)phenyl)ethyl)amino)-7-methoxy-2-methylquinazolin-6-yl)cyclohexane-1-carbonyl)piperidin-4-yl)propyl)(methyl)amino)piperidine-1-carboxylate (100 mg, 0.1 mmol) was dissolved in MeOH (5 mL), and Pd/C (20 mg) was added. The reaction liquid was purged 3 times with hydrogen and stirred at room temperature for 1 h under hydrogen atmosphere. The reaction liquid was filtered, the filter cake was washed with methanol (5 mL × 3), and the filtrate was concentrated under reduced pressure to give *tert*-butyl (3-((*R*)-1-((7-methoxy-2-methyl-6-((1*R*,4*R*)-4-(4-(3-(methyl(piperidin-4-yl)amino)propyl)piperidine-1-carbonyl)cyclohexyl)quinazolin-4-yl)amino)ethyl)-5-(trifluoromethyl)phenyl)carboxylate.

LC-MS: (ESI, *m*/*z*): [M+H] ⁺=824.1.

### Step 7: preparation of tert-butyl (3-((R)-1-((6-((1R,4R)-4-(4-(3-((1-(4-chloro-3-(2,4-dioxotetrahydropyrimidin-1(2H)-yl)benzoyl)piperidin-4-yl)(methyl)amino)propyl)piperidine-1-carbonyl)cyclohexyl)-7-methoxy-2-methylquinazolin-4-yl)amino)ethyl)-5-(trifluoromethyl)phenyl)carboxylate

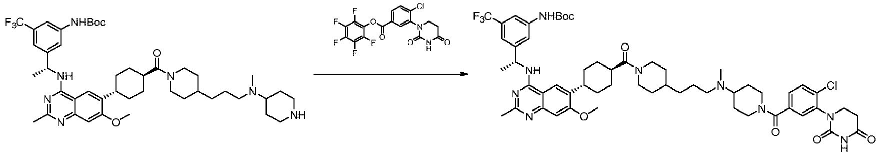

The compound *tert*-butyl (3-((*R*)-1-((7-methoxy-2-methyl-6-((1*R*,4*R*)-4-(4-(3-(methyl(piperidin-4-yl)amino)propyl)piperidine-1-carbonyl)cyclohexyl)quinazolin-4-yl)amino)ethyl)-5-(trifluoromethyl)phenyl)carboxylate (75 mg, 0.09 mmol) and pentafluorophenyl 4-chloro-3-(2,4-dioxotetrahydropyrimidin-1(2*H*)-yl)benzoate (39 mg, 0.09 mmol) were dissolved in DMSO (2 mL), and DIEA (35 mg, 0.27 mmol) was added. The reaction liquid was stirred at room temperature overnight. The reaction liquid was diluted with water (20 mL), extracted with ethyl acetate (30 mL × 2), dried over anhydrous sodium sulfate, filtered, and concentrated. The crude product was purified by column chromatography (MeOH:DCM = 0-5:95) to give *tert-*butyl (3-((*R*)-1-((6-((1*R*,4*R*)-4-(4-(3-((1-(4-chloro-3-(2,4-dioxotetrahydropyrimidin-1(2*H*)-yl)benzoyl)piperidin-4-yl)(methyl)amino)propyl)piperidine-1-carbonyl)cyclohexyl)-7-methoxy-2-methylquinazolin-4-yl)amino)ethyl)-5-(trifluoromethyl)phenyl)carboxylate.

LC-MS: (ESI, *m*/*z*): [M+H] ⁺= 1074.3.

### Step 8: preparation of 1-(5-(4-((3-(1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)propyl)(methyl)amino)piperidine-1-carbonyl)-2-chlorophenyl)dihydropyrimidine-2,4(1H,3H)-dione

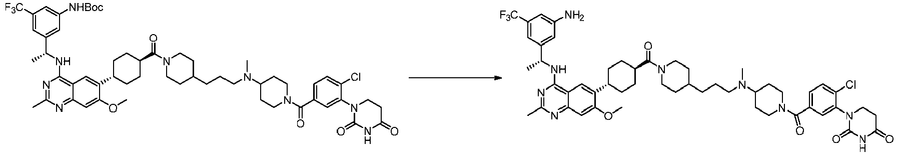

*tert*-Butyl (3-((*R*)-1-((6-((1*R*,4*R*)-4-(4-(3-((1-(4-chloro-3-(2,4-dioxotetrahydropyrimidin-1(2*H*)-yl)benzoyl)piperidin-4-yl)(methyl)amino) propyl)piperidine-1-carbonyl)cyclohexyl)-7-methoxy-2-methylquinazolin-4-yl)amino)ethyl)-5-(trifluoromethyl)phenyl)carboxylate (40 mg, 0.04 mmol) was dissolved in dichloromethane (3 mL), and trifluoroacetic acid (0.3 mL) was added. The reaction liquid was stirred at room temperature for 1 h, directly concentrated under reduced pressure, adjusted to pH 8-9 with a saturated aqueous sodium bicarbonate solution, extracted with dichloromethane (20 mL× 3), dried over anhydrous sodium sulfate, filtered, and concentrated. The crude product was purified by prep-HPLC to give 1-(5-(4-((3-(1-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperidin-4-yl)propyl)(methyl)amino)piperidine-1-carbonyl)-2-chlorophenyl)dihydropyrimidine-2,4(1*H*,3*H*)-dione.

LC-MS: (ESI, *m*/*z*): [M+H] ⁺=974.3.

¹H NMR (400 MHz, DMSO-*d*₆) δ 10.50 (s, 1H), 8.15 - 8.02 (m, 2H), 7.63 (d, *J* = 8.2 Hz, 1H), 7.56 (d, *J* = 1.9 Hz, 1H), 7.40 (d, *J* = 8.2 Hz, 1H), 6.99 (s, 1H), 6.86 (d, *J* = 10.8 Hz, 2H), 6.70 (s, 1H), 5.64 - 5.47 (m, 3H), 4.54-4.48 (m, 2H), 3.96-3.88 (m, 4H), 3.88-3.80 (m, 1H), 3.80-3.74 (m, 2H), 3.05-2.95 (m, 3H), 2.91-2.85 (m, 2H), 2.40-2.30 (m, 6H), 2.15 (s, 3H), 1.91-1.42 (m, 23H), 1.25-1.15 (m, 2H), 1.06 - 0.84 (m, 2H).

### Example 64: 1-(5-(4-((2-(1-(4-(4-(((R)-1-(3-amino-5-(trifluoromethyl)phenyl)ethyl)amino)-7-methoxy-2-methylquinazolin-6-yl)cyclohex-3-ene-1-carbonyl)piperidin-4-yl)ethoxy)methyl)piperidine-1-carbonyl)-2-chlorophenyl)dihydropyrimidine-2,4(1H,3H)-dione

### Step 1: preparation of methyl 4-(4-hydroxy-7-methoxy-2-methylquinazolin-6-yl)cyclohex-3-ene-1-carboxylate

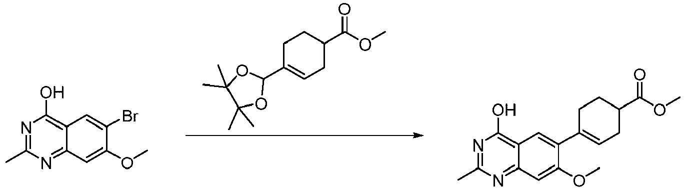

6-Bromo-7-methoxy-2-methylquinazolin-4-ol (6.2 g, 23.22 mmol) was added to a mixed solution of Pd(dppf)Cl₂ (850 mg, 1.16 mmol), sodium carbonate (4.9 g, 46.44 mmol), and *tert*-butyl 4-(4,4,5,5-tetramethyl-1,3-dioxolan-2-yl)cyclohex-3-ene-1-carboxylate (8.1 g, 30.19 mmol) in DMF/H₂O (80 mL/8 mL). The reaction liquid was stirred at room temperature overnight. After the reaction was completed, the reaction liquid was diluted with water (800 mL), extracted with ethyl acetate (200 mL × 5), washed with saturated brine, dried over anhydrous sodium sulfate, and concentrated under reduced pressure. The crude product was purified by column chromatography (PE:EA = 1:100) to give methyl 4-(4-hydroxy-7-methoxy-2-methylquinazolin-6-yl)cyclohex-3-ene-1-carboxylate.

LC-MS: (ESI, *m*/*z*): [M+H] ⁺=329.0.

### Step 2: preparation of methyl 4-(4-(((R)-1-(3-((tert-butoxycarbonyl)amino)-5-(trifluoromethyl)phenyl)ethyl)amino)-7-methoxy-2-methylquinazolin-6-yl)cyclohex-3-ene-1-carboxylate

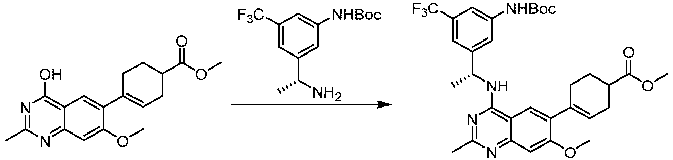

Methyl 4-(4-hydroxy-7-methoxy-2-methylquinazolin-6-yl)cyclohex-3-ene-1-carboxylate (600 mg, 1.82 mmol) was added to a solution of DBU (820 mg, 5.37 mmol) and BOP (1.19 g, 2.69 mmol) in DMF (10 mL). The reaction liquid was stirred at room temperature for 30 min. *tert*-Butyl (*R*)-(3-(1-aminoethyl)-5-(trifluoromethyl)phenyl)carboxylate (600 mg, 1.97 mmol) was added, and the reaction liquid was stirred at 70 °C overnight. The reaction liquid was diluted with water (100 mL), and the solid was precipitated, collected by filtration, washed three times with water, dissolved with dichloromethane (100 mL), dried over anhydrous sodium sulfate, and concentrated under reduced pressure. The crude product was purified by column chromatography (PE:EA = 1:1) to give methyl 4-(4-(((*R*)-1-(3-((*tert*-butoxycarbonyl)amino)-5-(trifluoromethyl)phenyl)ethyl)amino)-7-methoxy-2-methylquinazolin-6-yl)cyclohex-3-ene-1-carboxylate.

LC-MS: (ESI, *m*/*z*): [M+H] ⁺=615.2.

### Step 3: preparation of 4-(4-(((R)-1-(3-((tert-butoxycarbonyl)amino)-5-(trifluoromethyl)phenyl)ethyl)amino)-7-methoxy-2-methylquinazolin-6-yl)cyclohex-3-ene-1-carboxylic acid

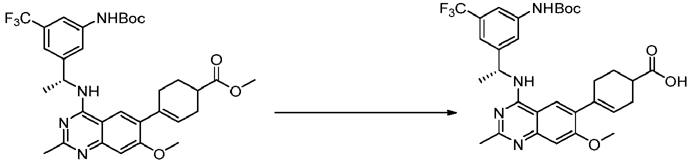

Methyl 4-(4-(((*R*)-1-(3-((*tert*-butoxycarbonyl)amino)-5-(trifluoromethyl)phenyl)ethyl)amino)-7-methoxy-2-methylquinazolin-6-yl)cyclohex-3-ene-1-carboxylate (1.1 g, 1.79 mmol) was added to a solution of lithium hydroxide (430 mg, 17.9 mmol) in THF/H₂O (20 mL/10 mL). The reaction liquid was stirred at 50 °C overnight under nitrogen atmosphere. 10% citric acid was slowly added dropwise in an ice bath, and the solution was adjusted to weak acidity or neutrality, extracted with ethyl acetate (100 mL × 4), washed with saturated brine, dried over anhydrous sodium sulfate, and concentrated under reduced pressure to give the crude product 4-(4-(((*R*)-1-(3-((*tert-*butoxycarbonyl)amino)-5-(trifluoromethyl)phenyl)ethyl)amino)-7-methoxy-2-methylquinazolin-6-yl)cyclohex-3-ene-1-carboxylic acid.

LC-MS: (ESI, *m*/*z*): [M+H] ⁺=601.2.

### Step 4: preparation of tert-butyl (3-((1R)-1-((6-(4-(4-(2-((1-(4-chloro-3-(2,4-dioxotetrahydropyrimidin-1(2H)-yl)benzoyl)piperidin-4-yl)methoxy)ethyl)piperidine-1-carbonyl)cyclohex-1-en-1-yl)-7-methoxy-2-methylquinazolin-4-yl)amino)ethyl)-5-(trifluoromethyl)phenyl)carboxylate

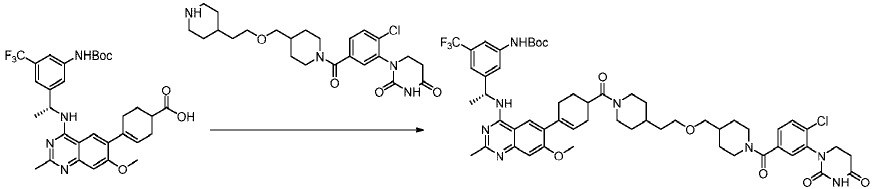

4-(4-(((*R*)-1-(3-((*tert*-Butoxycarbonyl)amino)-5-(trifluoromethyl)phenyl)ethyl)amino)-7-methoxy-2-methylquinazolin-6-yl)cyclohex-3-ene-1-carboxylic acid (130 mg, 0.22 mmol), HATU (100 mg, 0.26 mmol), and DIEA (85 mg, 0.66 mmol) were added to a solution of 1-(2-chloro-5-(4-((2-(piperidin-4-yl)ethoxy)methyl)piperidine-1-carbonyl)phenyl)dihydropyrimidine-2,4(1*H*,3*H*)-dione (140 mg, 0.23 mmol) in DMF (10 mL). The reaction liquid was stirred at room temperature for 2 h. Water (100 mL) was added, and the solid was precipitated, collected by filtration, washed 3 times with water, dissolved with dichloromethane, dried over anhydrous sodium sulfate, and concentrated under reduced pressure to give the crude product *tert*-butyl (3-((1*R*)-1-((6-(4-(4-(2-((1-(4-chloro-3-(2,4-dioxotetrahydropyrimidin-1(2*H*)-yl)benzoyl)piperidin-4-yl)methoxy)ethyl)piperidine-1-carbonyl)cyclohex-1-en-1-yl)-7-methoxy-2-methylquinazolin-4-yl)amino)ethyl)-5-(trifluoromethyl)phenyl)carboxylate.

LC-MS: (ESI, *m*/*z*): [M+H] ⁺=1059.5.

### Step 5: preparation of 1-(5-(4-((2-(1-(4-(4-(((R)-1-(3-amino-5-(trifluoromethyl)phenyl)ethyl)amino)-7-methoxy-2-methylquinazolin-6-yl)cyclohex-3-ene-1-carbonyl)piperidin-4-yl)ethoxy)methyl)piperidine-1-carbonyl)-2-chlorophenyl)dihydropyrimidine-2,4(1H,3H)-dione

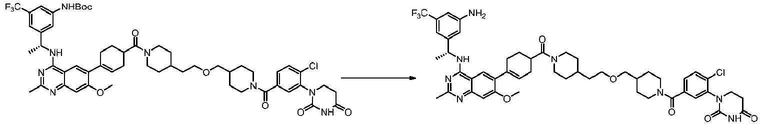

*tert*-Butyl (3-((1*R*)-1-((6-(4-(4-(2-((1-(4-chloro-3-(2,4-dioxotetrahydropyrimidin-1(2*H*)-yl)benzoyl)piperidin-4-yl)methoxy)ethyl)piperidine-1-carbonyl)cyclohex-1-en-1-yl)-7-methoxy-2-methylquinazolin-4-yl)amino)ethyl)-5-(trifluoromethyl)phenyl)carboxylate (190 mg, 0.18 mmol) was dissolved with dichloromethane (5 mL), and trifluoroacetic acid (1 mL) was added. The reaction liquid was stirred at room temperature for 2 h and concentrated under reduced pressure. The crude product was purified by preparative reversed-phase chromatography to give 1-(5-(4-((2-(1-(4-(4-(((*R*)-1-(3-amino-5-(trifluoromethyl)phenyl)ethyl)amino)-7-methoxy-2-methylquinazofin-6-yl)cyclohex-3-ene-1-carbonyl)piperidin-4-yl)ethoxy)methyl)piperidine-1-carbonyl)-2-chlorophenyl)dihydropyrimidine-2,4(1*H*,3*H*)-dione.

LC-MS: (ESI, *m*/*z*): [M+H] ⁺=959.2.

¹H NMR (400 MHz, DMSO-*d*₆) δ 10.50 (s, 1H), 8.19 (s, 1H), 8.07 (s, 1H), 7.63 (d, *J* = 8.3 Hz, 1H), 7.54 (d, *J* = 1.8 Hz, 1H), 7.38 (dd, *J* = 8.2, 1.8 Hz, 1H), 6.99 (s, 1H), 6.89 (s, 1H), 6.85 (s, 1H), 6.69 (s, 1H), 5.83 (s, 1H), 5.61-5.43 (m, 3H), 4.51-4.37 (m, 2H), 4.00-3.90 (m, 1H), 3.85 (s, 3H), 3.80-3.70 (m, 1H), 3.67-3.52 (m, 2H), 3.41 (t, *J* = 6.3 Hz, 2H), 3.24 (d, *J* = 6.4 Hz, 2H), 3.12-2.97 (m, 2H), 2.92-2.70 (m, 4H), 2.42-2.16 (m, 7H), 1.90-1.56 (m, 8H), 1.56-1.41 (m, 5H), 1.21-0.90 (m, 4H).

### Example 65: 1-(2-chloro-5-(4-(((1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)methoxy)methyl)piperidine-1-carbonyl)phenyl)dihydropyrimidine-2,4(1H,3H)-dione

### Step 1: preparation of tert-butyl 4-((pyridin-4-ylmethoxy)methyl)piperidine-1-carboxylate

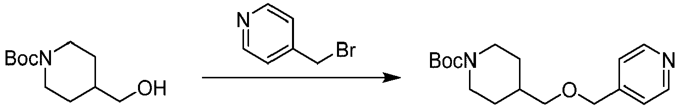

*tert*-Butyl 4-(hydroxymethyl)piperidine-1-carboxylate (1.0 g, 4.65 mmol) was dissolved in a THF (40 mL) solution, and NaH (744 mg, 18.6 mmol, 60%) was added at 0 °C. The reaction liquid was stirred for 2 h. Then 4-(bromomethyl)pyridine (795 mg, 4.65 mmol) was added, and the reaction liquid was stirred at room temperature for 2 h, diluted with water (100 mL), extracted with ethyl acetate (100 mL × 2), dried over anhydrous sodium sulfate, filtered, and concentrated under reduced pressure. The crude product was purified by column chromatography (ethyl acetate:petroleum ether = 1:0) to give *tert*-butyl 4-((pyridin-4-ylmethoxy)methyl)piperidine-1-carboxylate.

LC-MS: (ESI, m/z): [M+Na] ⁺=329.1.

### Step 2: preparation of tert-butyl 4-((piperidin-4-ylmethoxy)methyl)piperidine-1-carboxylate

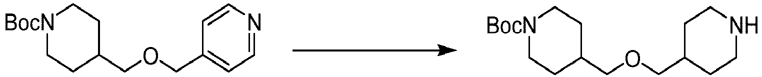

*tert*-Butyl 4-((pyridin-4-ylmethoxy)methyl)piperidine-1-carboxylate (300 mg, 0.98 mmol) was dissolved in a solution of *i*-PrOH/H₂O (6:7, 26 mL), and Pd(OH)/C (100 mg, 20%) was added under hydrogen atmosphere. The reaction liquid was stirred at 75 °C overnight and filtered through celite, and the filtrate was directly concentrated under reduced pressure to give *tert*-butyl 4-((piperidin-4-ylmethoxy)methyl)piperidine-1-carboxylate.

LC-MS: (ESI, m/z): [M+H] ⁺=313.2.

### Step 3: preparation of tert-butyl 4-(((1-(4-chloro-3-(2,4-dioxotetrahydropyrimidin-1(2H)-yl)benzoyl)piperidin-4-yl)methoxy)methyl)piperidine-1-carboxylate

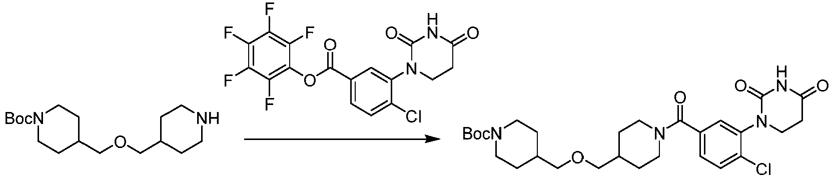

*tert*-Butyl 4-((piperidin-4-ylmethoxy)methyl)piperidine-1-carboxylate (120 mg, 0.38 mmol) and DIEA (147 mg, 1.14 mmol) were added to DMSO (10 mL), and pentafluorophenyl 4-chloro-3-(2,4-dioxotetrahydropyrimidin-1(2*H*)-yl)benzoate (165 mg, 0.38 mmol) was added. The reaction liquid was stirred at room temperature for 30 min, diluted with water (40 mL), and extracted with ethyl acetate (40 mL × 3). The organic phase was washed with saturated brine (40 mL), dried over anhydrous sodium sulfate, and concentrated under reduced pressure. The crude product was purified by column chromatography (PE:EA = 10:1) to give *tert*-butyl 4-(((1-(4-chloro-3-(2,4-dioxotetrahydropyrimidin-1(2*H*)-yl)benzoyl)piperidin-4-yl)methoxy)methyl)piperidine-1-carboxylate.

LC-MS: (ESI, m/z): [M+NH₄] ⁺=580.2.

### Step 4: preparation of 1-(2-chloro-5-(4-((piperidin-4-ylmethoxy)methyl)piperidine-1-carbonyl)phenyl)dihydropyrimidine-2,4(1H,3H)-dione

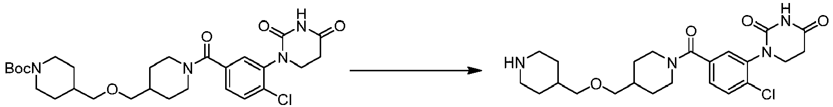

*tert*-Butyl 4-(((1-(4-chloro-3-(2,4-dioxotetrahydropyrimidin-1(2*H*)-yl)benzoyl)piperidin-4-yl)methoxy)methyl)piperidine-1-carboxylate (80 mg, 0.14 mmol) was dissolved with dichloromethane (5 mL), and a solution of hydrogen chloride in dioxane (4 M, 2 mL) was added. The reaction liquid was stirred at room temperature for 3 h and directly concentrated under reduced pressure to give 1-(2-chloro-5-(4-((piperidin-4-ylmethoxy)methyl)piperidine-1-carbonyl)phenyl)dihydropyrimidine-2,4(1*H*,3*H*)-dione.

LC-MS: (ESI, m/z): [M+H] ⁺=463.1.

### Step 5: preparation of 1-(2-chloro-5-(4-(((1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)methoxy)methyl)piperidine-1-carbonyl)phenyl)dihydropyrimidine-2,4(1H,3H)-dione

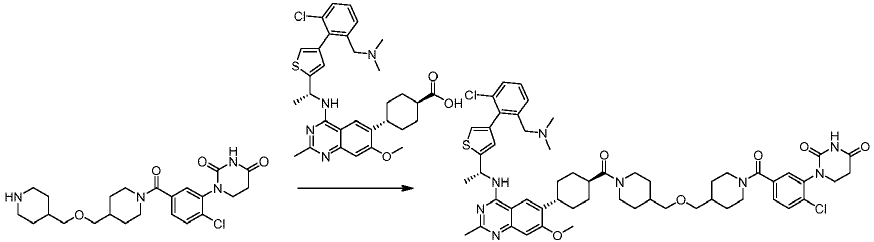

(1*R*,4*R*)-4-(4-(((*R*)-1-(4-(2-Chloro-6-((dimethylamino)methyl)phenyl)thien-2-yl)ethyl)amino)-7-methoxy-2-methylquinazolin-6-yl)cyclohexane-1-carboxylic acid (65 mg, 0.11 mmol) was dissolved with DMF (5 mL), and HATU (50 mg, 0.13 mmol), DIEA (50 mg, 0.39 mmol), and 1-(2-chloro-5-(4-((piperidin-4-ylmethoxy)methyl)piperidine-1-carbonyl)phenyl)dihydropyrimidine-2,4(1*H*,3*H*)-dione (60 mg, 0.13 mmol) were separately added. The reaction liquid was stirred at room temperature for 30 min, diluted with water (20 mL), and extracted with ethyl acetate (20 mL × 3). The organic phase was washed with saturated brine (20 mL), dried over anhydrous sodium sulfate, and concentrated under reduced pressure. The crude product was purified by prep-HPLC to give 1-(2-chloro-5-(4-(((1-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)methoxy)methyl)piperidine-1-carbonyl)phenyl)dihydropyrimidine-2,4(1*H*,3*H*)-dione.

LC-MS: (ESI, m/z): [M+H] ⁺=1037.4.

¹H NMR (400 MHz, DMSO-*d*₆) δ 10.51 (s, 1H), 8.31 (d, *J* = 8.1 Hz, 1H), 8.05 (s, 1H), 7.63 (d, *J* = 8.2 Hz, 1H), 7.54 (d*, J* = 1.8 Hz, 1H), 7.47-7.29 (m, 4H), 7.24 (s, 1H), 7.02 (s, 1H), 6.96 (s, 1H), 6.02 - 5.90 (m, 1H), 4.52 - 4.32 (m, 2H), 3.98-3.91(m, 1H), 3.89 (s, 3H), 3.80-3.69 (m, 1H), 3.66-3.54 (m, 2H), 3.28-3.17 (m, 4H), 3.15-3.09 (m, 2H), 3.09 - 2.89 (m, 3H), 2.84 - 2.69 (m, 3H), 2.69-2.60 (m, 2H), 2.42 (s, 3H), 2.01 (s, 6H), 1.91 - 1.46 (m, 17H), 1.20-0.89 (m, 4H).

### Example 66: 1-(5-(4-((2-(1-((1R,4R)-4-(4-(((R)-1-(1,1-dioxido-2,3-dihydrobenzo[b]thiophen-4-yl)ethyl)amino)-7-methoxy-2-methylquinazolin-6-yl)cyclohexane-1-carbonyl)piperidin-4-yl)ethoxy)methyl)piperidine-1-carbonyl)-2-(trifluoromethyl)phenyl)dihydropyrimidine-2,4(1H,3H)-dione

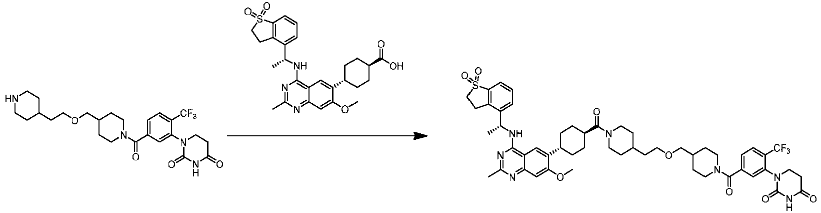

1-(5 -(4-((2-(Piperidin-4-yl)ethoxy)methyl)piperidine-1-carbonyl)-2-(trifluoromethyl)phenyl)dihydropyrimidine-2,4(1*H*,3*H*)-dione (120 mg, 0.24 mmol) and (1*R*,4*R*)-4-(4-(((*R*)-1-(1,1-dioxido-2,3-dihydrobenzo[*b*]thiophen-4-yl)ethyl)amino)-7-methoxy-2-methylquinazolin-6-yl)cyclohexane-1-carboxylic acid (120 mg, 0.24 mmol) were dissolved with DMF (3 mL), and HATU (108 mg, 0.28 mmol) and DIEA (90 mg, 0.71 mmol) were added. The reaction liquid was stirred at room temperature for 1 h. The reaction liquid was diluted with water (30 mL) and extracted with ethyl acetate (30 mL × 3). The organic phases were combined, washed with saturated brine, dried over anhydrous sodium sulfate, filtered, and concentrated. The crude product was purified by prep-HPLC to give 1-(5-(4-((2-(1-((1*R*,4*R*)-4-(4-(((*R*)-1-(1,1-dioxyde-2,3-dihydrobenzo[*b*]thiophen-4-yl)ethyl)amino)-7-methoxy-2-methylquinazolin-6-yl)cyclohexane-1-carbonyl)piperidin-4-yl)ethoxy)methyl)piperidine-1-carbonyl)-2-(trifluoromethyl)phenyl)dihydropyrimidine-2,4(1*H*,3*H*)-dione.

LC-MS: (ESI, m/z): [M+H] ⁺=1002.4.

¹H NMR (400 MHz, DMSO-*d*₆) δ 10.53 (s, 1H), 8.27 (d, *J* = 7.2 Hz, 1H), 8.06 (s, 1H), 7.87 (d, *J* = 8.2 Hz, 1H), 7.80 (d, *J* = 7.1 Hz, 1H), 7.70 (s, 1H), 7.59 (t, *J* = 6.6 Hz, 2H), 7.53 (t, *J* = 7.6 Hz, 1H), 6.98 (s, 1H), 5.58 (t, *J* = 7.0 Hz, 1H), 4.57 - 4.33 (m, 2H), 3.99 - 3.84 (m, 5H), 3.81 - 3.70 (m, 1H), 3.68 - 3.60 (m, 2H), 3.52-3.40 (m, 5H), 3.24 (d, *J* = 6.2 Hz, 2H), 3.14 - 2.89 (m, 3H), 2.85-2.65 (m, 4H), 2.32 (s, 3H), 1.92 - 1.40 (m, 20H), 1.23 - 0.89 (m, 4H).

### Example 67: 1-(2-chloro-5-(4-((2-(4-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)ethoxy)methyl)piperidine-1-carbonyl)phenyl)dihydropyrimidine-2,4(1H,3H)-dione

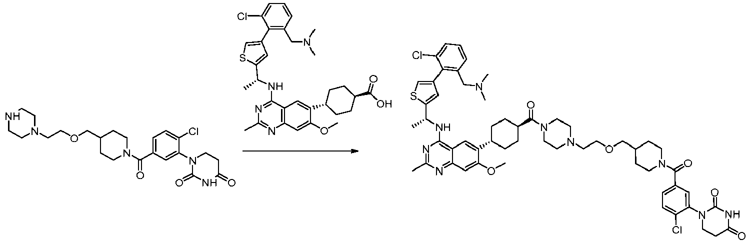

1-(2-Chloro-5-(4-((2-(piperazin-1-yl)ethoxy)methyl)piperidine-1-carbonyl)phenyl)dihydropyrimidine-2,4(1*H*,3*H*)-dione (150 mg, crude product) was dissolved in DMF (5 mL), and (1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carboxylic acid (94 mg, 0.22 mmol), HATU (107 mg, 0.28 mmol), and DIEA (145 mg, 1.1 mmol) were separately added. The reaction liquid was stirred at room temperature for 2 h. Water (50 mL) was added, and the resulting mixture was stirred for 5 min and extracted with ethyl acetate (30 mL × 3). The organic phase was washed with saturated brine (50 mL), dried over anhydrous sodium sulfate, filtered, and concentrated under reduced pressure. The crude product was purified by preparative reversed-phase chromatography to give 1-(2-chloro-5-(4-((2-(4-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)ethoxy)methyl)piperidine-1-carbonyl)phenyl)dihydropyrimidine-2,4(1*H*,3*H*)-dione.

LC-MS: (ESI, m/z): [M+H] ⁺=1052.4.

¹H NMR (400 MHz, DMSO-*d*₆) δ 10.48 (s, 1H), 8.29 (d, *J* = 8.3 Hz, 1H), 8.04 (s, 1H), 7.62 (d, *J* = 8.1 Hz, 1H), 7.53 (d, *J* = 1.6 Hz, 1H), 7.45 - 7.31 (m, 4H), 7.23 (s, 1H), 7.02 (s, 1H), 6.96 (s, 1H), 5.96 (t, *J* = 7.1 Hz, 1H), 4.52 - 4.28 (m, 1H), 3.89 (s, 3H), 3.80 - 3.40 (m, 10H), 3.26 (d, *J* = 6.3 Hz, 2H), 3.12 (s, 2H), 2.97 - 2.88 (m, 1H), 2.79 - 2.59 (m, 4H), 2.45 - 2.39 (m, 5H), 2.38 - 2.33 (m, 2H), 2.01 (s, 6H), 1.94 - 1.46 (m, 16H), 1.13 (d, *J* = 10.9 Hz, 2H).

### Example 68: 1-(2-chloro-5-(4-(3-(4-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)propoxy)piperidine-1-carbonyl)phenyl)dihydropyrimidine-2,4(1H,3H)-dione

### Step 1: preparation of tert-butyl 4-(3-(piperidin-4-yloxy)propyl)piperazine-1-carboxylate

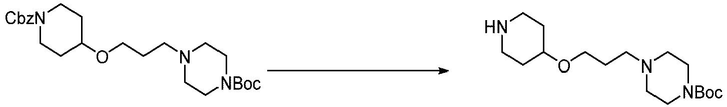

*tert*-Butyl 4-(3-((1-((benzyloxy)carbonyl)piperidin-4-yl)oxy)propyl)piperazine-1-carboxylate (400 mg, 0.87 mmol) and Pd/C (10%, 100 mg) were dissolved with ethyl acetate (10 mL). The reaction liquid was heated to 70 °C, stirred for 3 h, and filtered, and the solid was washed three times with ethyl acetate (10 mL × 3) and concentrated under reduced pressure to give *tert*-butyl 4-(3-(piperidin-4-yloxy)propyl)piperazine-1-carboxylate.

LC-MS: (ESI, m/z): [M+H] ⁺=328.3.

### Step 2: preparation of tert-butyl 4-(3-((1-(4-chloro-3-(2,4-dioxotetrahydropyrimidin-1(2H)-yl)benzoyl)piperidin-4-yl)oxy)propyl)piperazine-1 -carboxylate

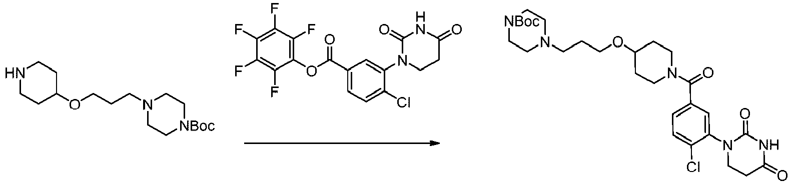

*tert*-Butyl 4-(3-(piperidin-4-yloxy)propyl)piperazine-1-carboxylate (230 mg, 0.70 mmol) was dissolved with DMSO (10 mL), and pentafluorophenyl 4-chloro-3-(2,4-dioxotetrahydropyrimidin-1(2*H*)-yl)benzoate (360 mg, 0.82 mmol) and DIEA (271 mg, 2.10 mmol) were separately added. The reaction liquid was stirred at room temperature overnight, diluted with water (50 mL), extracted with ethyl acetate (25 mL × 4), washed with saturated brine, dried over anhydrous sodium sulfate, and concentrated under reduced pressure. The crude product was purified by column chromatography (PE:EA = 1:10) to give *tert*-butyl 4-(3-((1-(4-chloro-3-(2,4-dioxotetrahydropyrimidin-1(2*H*)-yl)benzoyl)piperidin-4-yl)oxy)propyl)piperazine-1-carboxylate.

LC-MS: (ESI, m/z): [M+H] ⁺=578.3.

### Step 3: preparation of 1-(2-chloro-5-(4-(3-(piperazin-1-yl)propoxy)piperidine-1-carbonyl)phenyl)dihydropyrimidine-2,4(1H,3H)-dione

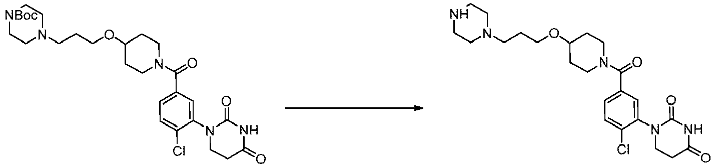

*tert*-Butyl 4-(3-((1-(4-chloro-3-(2,4-dioxotetrahydropyrimidin-1(2*H*)-yl)benzoyl)piperidin-4-yl)oxy)propyl)piperazine-1-carboxylate (220 mg, 0.47 mmol) was dissolved with dichloromethane (5 mL), and trifluoroacetic acid (1 mL) was added. The reaction liquid was stirred at room temperature overnight. A saturated aqueous sodium bicarbonate solution was slowly added dropwise to the reaction liquid to adjust the pH to 7-8. The resulting mixture was extracted with a mixed solution (25 mL × 4) of dichloromethane and isopropanol, dried over anhydrous sodium sulfate, filtered, and concentrated under reduced pressure to give 1-(2-chloro-5-(4-(3-(piperazin-1-yl)propoxy)piperidine-1-carbonyl)phenyl)dihydropyrimidine-2,4(1*H*,3*H*)-dione.

LC-MS: (ESI, m/z): [M+H] ⁺=478.2.

### Step 4: preparation of 1-(2-chloro-5-(4-(3-(4-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)propoxy)piperidine-1-carbonyl)phenyl)dihydropyrimidine-2,4(1H,3H)-dione

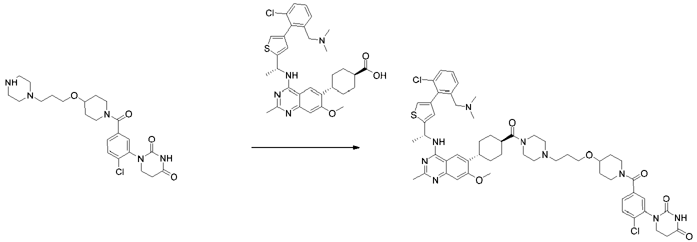

1-(2-Chloro-5-(4-(3-(piperazin-1-yl)propoxy)piperidine-1-carbonyl)phenyl)dihydropyrimidine-2,4(1*H*,3*H*)-dione (90 mg, 0.19 mmol), HATU (87 mg, 0.23 mmol), and DIEA (74 mg, 0.57 mmol) were separately added to a solution of (1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carboxylic acid (106 mg, 0.19 mmol) in DMF (10 mL). The reaction liquid was stirred at room temperature for 2 h and diluted with water (100 mL), and the solid was precipitated, collected by filtration, washed 3 times with water, dissolved with dichloromethane, dried over anhydrous sodium sulfate, filtered, and concentrated under reduced pressure. The crude product was purified by preparative reversed-phase chromatography to give 1-(2-chloro-5-(4-(3-(4-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)propoxy)piperidine-1-carbonyl)phenyl)dihydropyrimidine-2,4(1*H*,3*H*)-dione.

LC-MS: (ESI, m/z): [M+H] ⁺=1052.4.

¹H NMR (400 MHz, DMSO-*d*₆) δ 10.51 (s, 1H), 8.32 (d, *J* = 7.7 Hz, 1H), 8.05 (s, 1H), 7.63 (d, *J* = 8.3 Hz, 1H), 7.57 (s, 1H), 7.47 - 7.30 (m, 4H), 7.24 (s, 1H), 7.02 (s, 1H), 6.97 (s, 1H), 5.97 (t, *J* = 7.2 Hz, 1H), 4.00-3.84 (s, 4H), 3.80-3.71 (m, 1H), 3.66-3.57 (m, 1H), 3.49-3.35 (m, 8H), 3.20-3.07 (m, 3H), 3.00-2.84 (m,1H), 2.78-2.58 (m, 3H), 2.42 (s, 3H), 2.40-2.26 (m, 6H), 2.01 (s, 6H), 1.92-1.75 (m, 6H), 1.75 - 1.38 (m, 12H).

### Example 69: 1-(2-chloro-5-(4-((2-(1-((1R,4R)-4-(4-(((R)-1-(4-(2-chloro-6-((methylamino)methyl)phenyl)thien-2-yl)ethyl)amino)-7-methoxy-2-methylquinazolin-6-yl)cyclohexane-1-carbonyl)piperidin-4-yl)ethoxy)methyl)piperidine-1-carbonyl)phenyl)dihydropyrimidine-2,4(1H,3H)-dione

### Step 1: preparation of tert-butyl (1R,4R)-4-(4-(((R)-1-(4-(2-chloro-6-((methylamino)methyl)phenyl)thien-2-yl)ethyl)amino)-7-methoxy-2-methylquinazofin-6-yl)cyclohexane-1-carboxylate

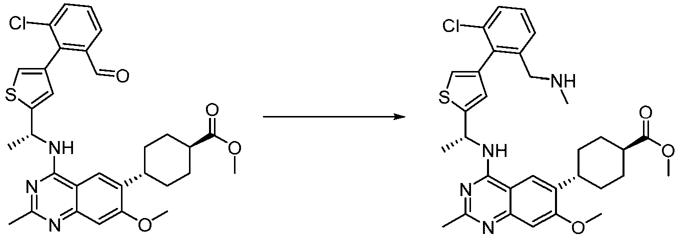

The compound methyl (1*R*,4*R*)-4-(4-(((*R*)-1-(4-(2-chloro-6-formylphenyl)thien-2-yl)ethyl)amino)-7-methoxy-2-methylquinazolin-6-yl)cyclohexane-1-carboxylate (450 mg, 0.78 mmol) and methylamine (2 M in THF, 7.8 mmol) were dissolved in dichloroethane (10 mL), and sodium triacetoxyborohydride (496 mg, 2.34 mmol) was added. The reaction liquid was heated to 80 °C and stirred for 1 h. The reaction liquid was directly concentrated under reduced pressure. The crude product was purified by column chromatography (MeOH:DCM = 1:10, flow rate: 40 mL/min) to give *tert*-butyl (1*R*,4*R*)-4-(4-(((*R*)-1-(4-(2-chloro-6-((methylamino)methyl)phenyl)thien-2-yl)ethyl)amino)-7-methoxy-2-methylquinazolin-6-yl)cyclohexane-1-carboxylate.

LC-MS: (ESI, m/z): [M+H] ⁺=593.3.

### Step 2: preparation of (1R,4R)-4-(4-(((R)-1-(4-(2-chloro-6-((methylamino)methyl)phenyl)thien-2-yl)ethyl)amino)-7-methoxy-2-methylquinazolin-6-yl)cyclohexane-1-carboxylic acid

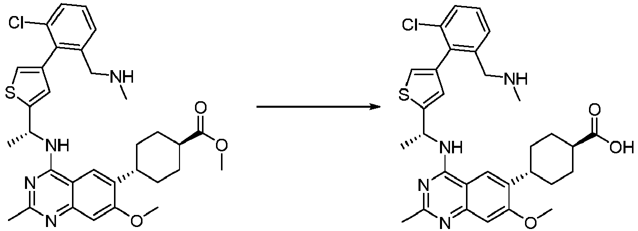

*tert*-Butyl (1*R*,4*R*)-4-(4-(((*R*)-1-(4-(2-chloro-6-((methylamino)methyl)phenyl)thien-2-yl)ethyl)amino)-7-methoxy-2-methylquinazolin-6-yl)cyclohexane-1-carboxylate (300 mg, 0.5 mmol) and lithium hydroxide (60 mg, 2.5 mmol) were dissolved with a mixed solution (MeOH/H₂O = 10/1, 5 mL). The reaction liquid was heated to 70 °C and stirred for 2 h. The reaction liquid was cooled, adjusted to pH 3-4 with 1 M HCl, adjusted to pH 7-8 with a saturated aqueous sodium bicarbonate solution, and extracted with ethyl acetate (100 mL × 3). The organic phases were combined, washed with saturated brine, dried over anhydrous sodium sulfate, filtered, and concentrated under reduced pressure. The crude product was purified by reversed-phase chromatography (ACN:H₂O = 1:1) to give (1*R*,4*R*)-4-(4-(((*R*)-1-(4-(2-chloro-6-((methylamino)methyl)phenyl)thien-2-yl)ethyl)amino)-7-methoxy-2-methylquinazolin-6-yl)cyclohexane-1 -carboxylic acid.

LC-MS: (ESI, m/z): [M+H] ⁺=579.2.

### Step 3: preparation of 1-(2-chloro-5-(4-((2-(1-((1R,4R)-4-(4-(((R)-1-(4-(2-chloro-6-((methylamino)methyl)phenyl)thien-2-yl)ethyl)amino)-7-methoxy-2-methylquinazolin-6-yl)cyclohexane-1-carbonyl)piperidin-4-yl)ethoxy)methyl)piperidine-1-carbonyl)phenyl)dihydropyrimidine-2,4(1H,3H)-dione

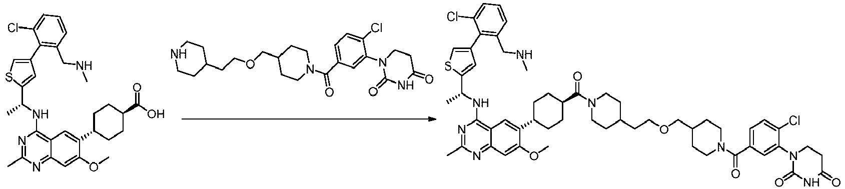

(1*R*,4*R*)-4-(4-(((*R*)-1-(4-(2-Chloro-6-((methylamino)methyl)phenyl)thien-2-yl)ethyl)amino)-7-methoxy-2-methylquinazolin-6-yl)cyclohexane-1-carboxylic acid (80 mg, 0.14 mmol) was dissolved with DMF (5 mL), and HATU (65 mg, 0.17 mmol), 1-(2-chloro-5-(4-((2-(piperidin-4-yl)ethoxy)methyl)piperidine-1-carbonyl)phenyl)dihydropyrimidine-2,4(1*H*,3*H*)-dione (80 mg, 0.17 mmol), and DIEA (54 mg, 0.42 mmol) were added. The reaction liquid was stirred at room temperature for 1 h, diluted with water (20 mL), and extracted with ethyl acetate (20 mL × 3). The organic phase was washed with saturated brine (40 mL), dried over anhydrous sodium sulfate, filtered, and concentrated under reduced pressure. The crude product was purified by prep-HPLC to give 1-(2-chloro-5-(4-((2-(1-((1*R*,4*R*)-4-(4-(((*R*)-1-(4-(2-chloro-6-((methylamino)methyl)phenyl)thien-2-yl)ethyl)amino)-7-methoxy-2-methylquinazolin-6-yl)cyclohexane-1-carbonyl)piperidin-4-yl)ethoxy)methyl)piperidine-1-carbonyl)phenyl)dihydropyrimidine-2,4(1*H*,3*H*)-dione.

LC-MS: (ESI, m/z): [M+H] ⁺=1037.4.

¹H NMR (400 MHz, DMSO-*d*₆) δ 10.50 (s, 1H), 8.32 (d, *J* = 8.3 Hz, 1H), 8.05 (s, 1H), 7.63 (d, *J* = 8.2 Hz, 1H), 7.54 (d, *J* = 1.7 Hz, 1H), 7.45 (d, *J* = 7.5 Hz, 1H), 7.42 - 7.30 (m, 3H), 7.28 (s, 1H), 7.02 (s, 1H), 6.98 (s, 1H), 6.07 - 5.86 (m, 1H), 4.38 (d, *J* = 12.6 Hz, 2H), 3.89 (s, 4H), 3.73 (d, *J* = 7.1 Hz, 1H), 3.66 - 3.51 (m, 2H), 3.44 - 3.36 (m, 4H), 3.23 (d, *J* = 6.2 Hz, 2H), 3.11 - 2.88 (m, 3H), 2.78 - 2.59 (m, 4H), 2.42 (s, 3H), 2.12 (s, 3H), 1.92 - 1.39 (m, 21H), 1.18 - 0.87 (m, 4H).

### Example 70: 1-(2-chloro-5-(4-(2-((1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)methoxy)ethyl)piperidine-1-carbonyl)phenyl)dihydropyrimidine-2,4(1H,3H)-dione

### Step 1: preparation of tert-butyl 4-(2-((1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)methoxy)ethyl)piperidine-1 -carboxylate

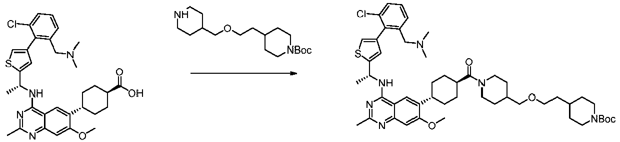

(1*R*,4*R*)-4-(4-(((*R*)-1-(4-(2-Chloro-6-((dimethylamino)methyl)phenyl)thien-2-yl)ethyl)amino)-7-methoxy-2-methylquinazolin-6-yl)cyclohexane-1-carboxylic acid (80 mg, 0.13 mmol), HATU (62 mg, 0.16 mmol), and DIEA (50 mg, 0.39 mmol) were added to a solution of *tert*-butyl 4-(2-(piperidin-4-ylmethoxy)ethyl)piperidine-1-carboxylate (43 mg, 0.13 mmol) in DMF (5 mL). The reaction liquid was stirred at room temperature for 1 h and diluted with water (50 mL), and the solid was precipitated, collected by filtration, washed 3 times with water, dissolved with dichloromethane, dried over anhydrous sodium sulfate, and concentrated under reduced pressure. The crude product was purified by preparative reversed-phase chromatography to give *tert*-butyl 4-(2-((1-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)methoxy)ethyl)piperidine-1-carboxylate.

LC-MS: (ESI, m/z): [M+H] ⁺=901.4.

### Step 2: preparation of ((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexyl)(4-((2-(piperidin-4-yl)ethoxy)methyl)piperidin-1-yl)methanone

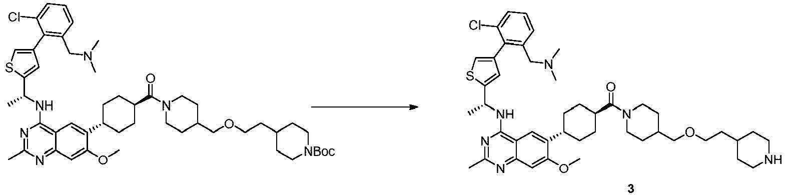

*tert*-Butyl 4-(2-((1-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)methoxy)ethyl)piperidine-1-carboxylate (80 mg, 0.09 mmol) was added to a solution of hydrogen chloride in dioxane (4 M, 2 mL). The reaction liquid was stirred at room temperature for 1 h and directly concentrated under reduced pressure to give the crude product ((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexyl)(4-((2-(piperidin-4-yl)ethoxy)methyl)piperidin-1-yl)methanone.

LC-MS: (ESI, m/z): [M+H] ⁺=801.3.

### Step 3: preparation of 1-(2-chloro-5-(4-(2-((1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)methoxy)ethyl)piperidine-1-carbonyl)phenyl)dihydropyrimidine-2,4(1H,3H)-dione

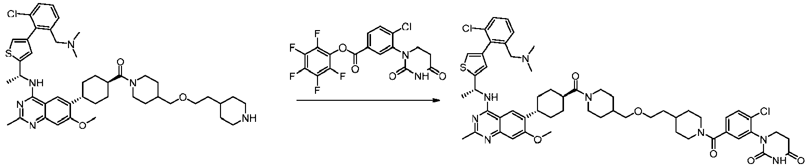

((1*R*,4*R*)-4-(4-(((*R*)-1-(4-(2-Chloro-6-((dimethylamino)methyl)phenyl)thien-2-yl)ethyl)amino)-7-methoxy-2-methylquinazolin-6-yl)cyclohexyl)(4-((2-(piperidin-4-yl)ethoxy)methyl)piperidin-1-yl)methanone (70 mg, 0.09 mmol) was added to a solution of pentafluorophenyl 4-chloro-3-(2,4-dioxotetrahydropyrimidin-1(2*H*)-yl)benzoate (39 mg, 0.08 mmol) and DIEA (35 mg, 0.27 mmol) in DMSO (5 mL). The reaction liquid was stirred at room temperature overnight, diluted with water (50 mL), extracted with ethyl acetate (25 mL × 4), washed with saturated brine, dried over anhydrous sodium sulfate, and concentrated under reduced pressure. The crude product was purified by preparative reversed-phase chromatography to give 1-(2-chloro-5-(4-(2-((1-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1 -carbonyl)piperidin-4-yl)methoxy)ethyl)piperidine-1 -carbonyl)phenyl)dihydropyrimidine-2,4(1*H*,3*H*)-dione.

LC-MS: (ESI, m/z): [M+H] ⁺=1051.5.

¹H NMR (400 MHz, DMSO-*d*₆) δ 10.49 (s, 1H), 8.30 (d, *J* = 8.1 Hz, 1H), 8.05 (s, 1H), 7.62 (d, *J* = 8.2 Hz, 1H), 7.53 (d, *J* = 2.0 Hz, 1H), 7.45-7.31 (m, 4H), 7.24 (d, *J* = 1.3 Hz, 1H), 7.02 (s, 1H), 6.96 (s, 1H), 6.03 - 5.89 (m, 1H), 4.48-4.32 (m, 2H), 3.97-3.85 (m, 4H), 3.79-3.68 (m, 1H), 3.66-3.52 (m, 2H), 3.40 (t, *J* = 6.4 Hz, 2H), 3.21 (d, *J* = 6.1 Hz, 2H), 3.16 - 2.90 (m, 5H), 2.82 - 2.52 (m, 7H), 2.42 (s, 3H), 2.01 (s, 6H), 1.88 - 1.43 (m, 20H), 1.19 - 0.90 (m, 4H).

### Example 71: 1-(2-chloro-5-(4-(4-(4-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)butyl)piperidine-1-carbonyl)phenyl)dihydropyrimidine-2,4(1H,3H)-dione

### Step 1: preparation of tert-butyl 4-(4-(4-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1 -yl)butyl)piperidine-1 -carboxylate

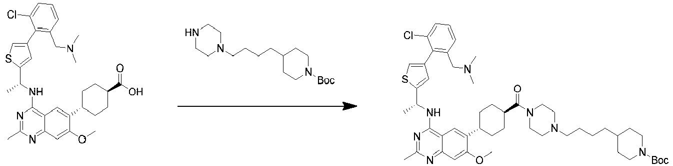

(1*R*,4*R*)-4-(4-(((*R*)-1-(4-(2-Chloro-6-((dimethylamino)methyl)phenyl)thien-2-yl)ethyl)amino)-7-methoxy-2-methylquinazolin-6-yl)cyclohexane-1-carboxylic acid (120 mg, 0.20 mmol), tert-butyl 4-(4-(piperazin-1-yl)butyl)piperidine-1-carboxylate (66 mg, 0.20 mmol), and HATU (91 mg, 0.24 mmol) were dissolved in DMF (3 mL), and DIEA (78 mg, 0.61 mmol) was added. The reaction liquid was stirred at room temperature for 1 h, diluted with water (30 mL), and extracted with ethyl acetate (30 mL × 3). The organic phases were combined, washed with saturated brine, dried over anhydrous sodium sulfate, filtered, and concentrated. The crude product was purified by column chromatography (DCM:MeOH = 10:1) to give *tert*-butyl 4-(4-(4-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)butyl)piperidine-1-carboxylate.

LC-MS: (ESI, m/z): [M+H] ⁺=900.6.

### Step 2: preparation of ((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexyl)(4-(4-(piperidin-4-yl)butyl)piperazin-1-yl)methanone

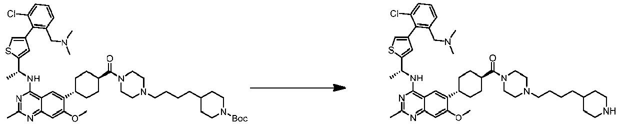

*tert*-Butyl 4-(4-(4-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)butyl)piperidine-1-carboxylate (149 mg, 0.17 mmol) was dissolved with a solution of hydrogen chloride in 1,4-dioxane (4 M, 5 mL). The reaction liquid was stirred at room temperature for 1 h and directly concentrated under reduced pressure to give ((1*R*,4*R*)-4-(4-*(((R)-1-(4-(2-chloro-6-((dimethylamino)methyl)phenyl)thien-2-yl)ethyl)amino)-7-methoxy-2-methylquinazolin-6-*yl)cyclohexyl)(4-(4-(piperidin-4-yl)butyl)piperazin-1-yl)methanone.

LC-MS: (ESI, m/z): [M+H] ⁺=800.4.

### Step 3: preparation of 1-(2-chloro-5-(4-(4-(4-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)butyl)piperidine-1-carbonyl)phenyl)dihydropyrimidine-2,4(1H,3H)-dione

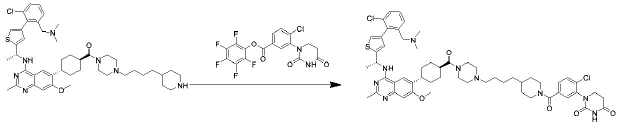

((1*R*,4*R*)-4-(4-(((*R*)-1-(4-(2-Chloro-6-((dimethylamino)methyl)phenyl)thien-2-yl)ethyl)amino)-7-methoxy-2-methylquinazolin-6-yl)cyclohexyl)(4-(4-(piperidin-4-yl)butyl)piperazin-1-yl)methanone (158 mg, 0.11 mmol) and pentafluorophenyl 4-chloro-3-(2,4-dioxotetrahydropyrimidin-1(2*H*)-yl)benzoate (47 mg, 0.11 mmol) were dissolved in DMSO (2 mL), and DIEA (42 mg, 0.32 mmol) was added while stirring. The reaction liquid was stirred at room temperature for 1 h. The reaction liquid was diluted with water (30 mL) and extracted with ethyl acetate (30 mL × 3). The upper organic phases were combined, washed with saturated brine, dried over anhydrous sodium sulfate, filtered, and concentrated under reduced pressure. The crude product was purified by prep-HPLC to give 1-(2-chloro-5-(4-(4-(4-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperazin-1-yl)butyl)piperidine-1-carbonyl)phenyl)dihydropyrimidine-2,4(1*H*,3*H*)-dione.

LC-MS: (ESI, m/z): [M+H] ⁺=1050.5.

¹H NMR (400 MHz, DMSO-*d*₆) δ 10.49 (s, 1H), 8.30 (d, *J* = 7.7 Hz, 1H), 8.05 (s, 1H), 7.63 (d, *J* = 8.3 Hz, 1H), 7.54 (s, 1H), 7.47 - 7.31 (m, 4H), 7.24 (s, 1H), 7.02 (s, 1H), 6.96 (s, 1H), 6.01 - 5.90 (m, 1H), 4.50-4.42 (m, 1H), 3.89 (s, 3H), 3.80-3.68 (m, 1H), 3.66-3.52 (m, 2H), 3.50-3.40 (m, 4H), 3.18-3.10 (m, 2H), 3.02-2.90 (m, 2H), 2.80-2.65 (m, 4H), 2.42 (s, 3H), 2.38-2.20 (m, 6H), 2.01 (s, 6H), 1.82-1.42 (m, 18H), 1.30-1.22 (m, 4H), 1.13-0.96 (m, 2H).

### Example 72: 1-(5-(9-((4-((1R,4R)-4-(4-(((R)-1-(3-amino-5-(trifluoromethyl)phenyl)ethyl)amino)-2,7-dimethylquinazolin-6-yl)cyclohexane-1-carbonyl)piperazin-1-yl)methyl)-3-azaspiro[5.5]undecane-3-carbonyl)-2-chlorophenyl)dihydropyrimidine-2,4(1H,3H)-dione

### Step 1: preparation of methyl 4-(4-hydroxy-2,7-methylquinazolin-6-yl)cyclohex-3-ene-1-carboxylate

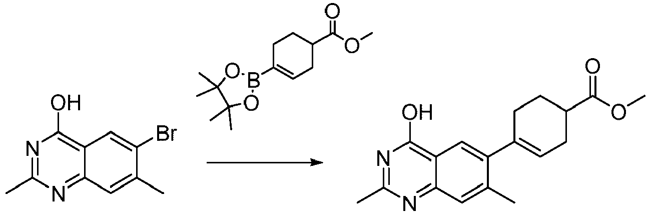

6-Bromo-2,7-dimethylquinazolin-4-ol (900 mg, 3.56 mmol) was dissolved with DMF/H₂O (22 mL, 10/1), and methyl 4-(4,4,5,5-tetramethyl-1,3,2-dioxopentan-2-yl)cyclohex-3-ene-1-carboxylate (1135 mg, 4.27 mmol), sodium carbonate (755 mg, 7.12 mmol), and Pd(dppf)Cl₂ (264 mg, 0.36 mmol) were added. The reaction liquid was stirred at 110 °C overnight under nitrogen atmosphere. Water (100 mL) was added, and the resulting mixture was stirred for 5 min and extracted with ethyl acetate (100 mL × 3). The organic phase was washed with saturated brine (200 mL), dried over anhydrous sodium sulfate, filtered, and concentrated under reduced pressure. The crude product was purified by column chromatography (MeOH:DCM = 1:9) to give methyl 4-(4-hydroxy-2,7-methylquinazolin-6-yl)cyclohex-3-ene-1-carboxylate.

LC-MS: (ESI, m/z): [M+H] ⁺=313.1.

### Step 2: preparation of methyl (1R,4R)-4-(4-hydroxy-2,7-dimethylquinazolin-6-yl)cyclohexane-1-carboxylate

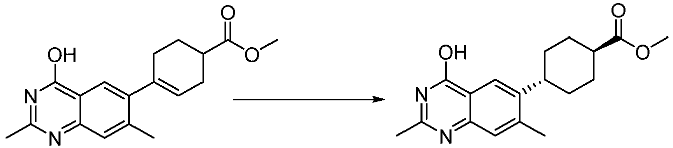

Methyl 4-(4-hydroxy-2,7-methylquinazolin-6-yl)cyclohex-3-ene-1-carboxylate (380 mg, 1.22 mmol) was added to an autoclave (100 mL) and dissolved with MeOH (30 mL), and Pd(OH)₂/C (115 mg, 30%) was added. The reaction liquid was reacted at 70 °C for 48 h under hydrogen atmosphere (2 MPa), filtered, and concentrated under reduced pressure. The crude product was purified by preparative reversed-phase chromatography to give methyl (1*R*,4*R*)-4-(4-hydroxy-2,7-dimethylquinazolin-6-yl)cyclohexane-1-carboxylate.

LC-MS: (ESI, m/z): [M+H] ⁺=315.2.

¹H NMR (400 MHz, DMSO) δ 12.09 (s, 1H), 8.15 (s, 1H), 7.56 (s, 1H), 3.92 (s, 3H), 2.95 (t, *J* = 11.6 Hz, 1H), 2.43 (s, 3H), 2.36 (s, 3H), 2.35 - 2.24 (m, 1H), 2.06 (d, *J* = 10.7 Hz, 2H), 1.90 - 1.84 (m, 2H), 1.67 - 1.40 (m, 4H). Step 3: preparation of methyl (1*R*,4*R*)-4-(2,7-dimethyl-4-(((*R*)-1-(3-nitro-5-(trifluoromethyl)phenyl)ethyl)amino)quinazolin-6-yl)cyclohexane-1-carboxylate

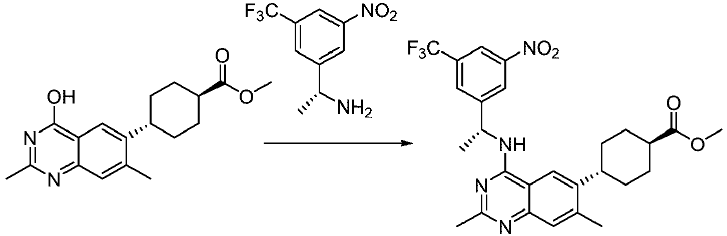

Methyl (1*R*,4*R*)-4-(4-hydroxy-2,7-dimethylquinazolin-6-yl)cyclohexane-1-carboxylate (250 mg, 0.80 mmol) was dissolved in DMF (5 mL), and BOP (506 mg, 1.19 mmol) and DBU (303 mg, 1.99 mmol) were added. The reaction liquid was stirred at room temperature for 0.5 h. (*R*)-1-(3-Nitro-5-(trifluoromethyl)phenyl)ethan-1-amine (224 mg, 0.96 mmol) was added, and the resulting mixture was heated to 70 °C and stirred overnight. Water (50 mL) was added, and the resulting mixture was stirred for 5 min and extracted with ethyl acetate (30 mL × 3). The organic phase was washed with saturated brine (50 mL), dried over anhydrous sodium sulfate, filtered, and concentrated under reduced pressure. The crude product was purified by reversed-phase chromatography to give methyl (1*R*,4*R*)-4-(2,7-dimethyl-4-(((*R*)-1-(3-nitro-5-(trifluoromethyl)phenyl)ethyl)amino)quinazolin-6-yl)cyclohexane-1-carboxylate.

LC-MS: (ESI, m/z): [M+H] ⁺=531.3.

### Step 4: preparation of (1R,4R)-4-(2,7-dimethyl-4-(((R)-1-(3-nitro-5-(trifluoromethyl)phenyl)ethyl)amino)quinazolin-6-yl)cyclohexane-1-carboxylic acid

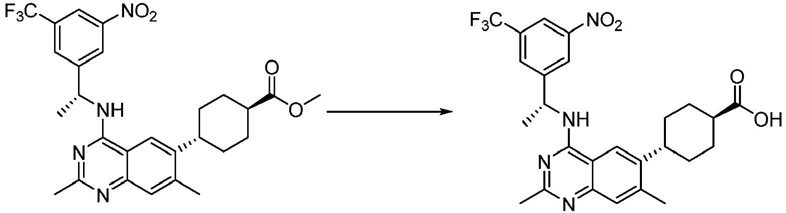

Methyl (1R,4R)-4-(2,7-dimethyl-4-(((*R*)-1-(3 -nitro-5 -(trifluoromethyl)phenyl)ethyl)amino)quinazolin-6-yl)cyclohexane-1-carboxylate (210 mg, 0.40 mmol) was dissolved in THF/H₂O (15 mL, 2:1), and lithium hydroxide (190 mg, 8.00 mmol) was added. The reaction liquid was stirred at 50 °C overnight. Water (20 mL) was added, and 1 M hydrochloric acid was added to adjust the pH to 6. The resulting mixture was extracted with MeOH/DCM (1/9, 30 mL × 3), dried over anhydrous sodium sulfate, filtered, and concentrated under reduced pressure to give (1*R*,4*R*)-4-(2,7-dimethyl-4-(((*R*)-1-(3-nitro-5-(trifluoromethyl)phenyl)ethyl)amino)quinazolin-6-yl)cyclohexane-1-carboxylic acid, which was directly used in the next step.

LC-MS: (ESI, m/z): [M+H] ⁺=517.4.

### Step 5: preparation of (1R,4R)-4-(4-(((R)-1-(3-amino-5-(trifluoromethyl)phenyl)ethyl)amino)-2,7-dimethylquinazolin-6-yl)cyclohexane-1 -carboxylic acid

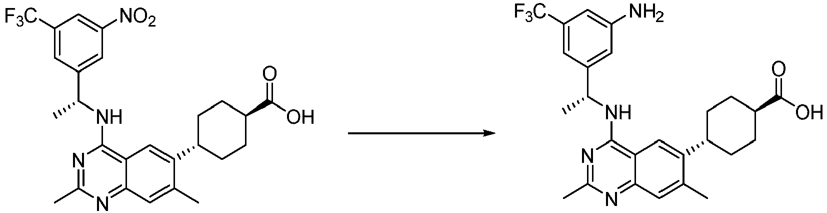

(1*R*,4*R*)-4-(2,7-Dimethyl-4-(((*R*)-1-(3-nitro-5-(trifluoromethyl)phenyl)ethyl)amino)quinazolin-6-yl)cyclohexane-1-carboxylic acid (150 mg, 0.29 mmol) was dissolved in ethanol (10 mL), and Raney-Ni (0.5 mL) and N₂H₄-H₂O (85%, 0.5 mL) were added. The reaction liquid was reacted at room temperature for 0.5 h, filtered, and concentrated under reduced pressure to give (1*R*,4*R*)-4-(4-(((*R*)-1-(3-amino-5-(trifluoromethyl)phenyl)ethyl)amino)-2,7-dimethylquinazolin-6-yl)cyclohexane-1-carboxylic acid.

LC-MS: (ESI, m/z): [M+H] ⁺=487.2.

### Step 6: preparation of 1-(5-(9-((4-((1R,4R)-4-(4-(((R)-1-(3-amino-5-(trifluoromethyl)phenyl)ethyl)amino)-2,7-dimethylquinazolin-6-yl)cyclohexane-1-carbonyl)piperazin-1-yl)methyl)-3-azaspiro[5.5]undecane-3-carbonyl)-2-chlorophenyl)dihydropyrimidine-2,4(1H,3H)-dione

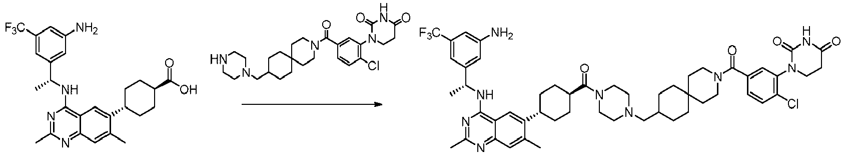

(1*R*,4*R*)-4-(4-(((*R*)-1-(3-Amino-5-(trifluoromethyl)phenyl)ethyl)amino)-2,7-dimethylquinazolin-6-yl)cyclohexane-1-carboxylic acid (70 mg, 0.14 mmol) was dissolved in DMF (5 mL), and 1-(2-chloro-5-(9-(piperazin-1-ylmethyl)-3-azaspiro[5.5]undecane-3-carbonyl)phenyl)dihydropyrimidine-2,4(1*H*,3*H*)-dione (84mg, 0.17 mmol), DIEA (55 mg, 0.42 mmol), and pyBOP (88 mg, 0.17 mmol) were added. The reaction liquid was reacted at room temperature for 1 h. Water (50 mL) was added, and the resulting mixture was stirred for 5 min and extracted with ethyl acetate (30 mL × 3). The organic phase was washed with saturated brine (50 mL), dried over anhydrous sodium sulfate, filtered, and concentrated under reduced pressure. The crude product was purified by preparative reversed-phase chromatography to give 1-(5-(9-((4-((1*R*,4*R*)-4-(4-(((*R*)-1-(3-amino-5-(trifluoromethyl)phenyl)ethyl)amino)-2,7-dimethylquinazofin-6-yl)cyclohexane-1-carbonyl)piperazin-1-yl)methyl)-3-azaspiro[5.5]undecane-3-carbonyl)-2-chlorophenyl)dihydropyrimidine-2,4(1*H*,3*H*)-dione.

LC-MS: (ESI, m/z): [M+H] ⁺=970.4.

¹H NMR (400 MHz, DMSO-*d*₆) δ 10.51 (s, 1H), 8.19 (d, *J* = 8.0 Hz, 1H), 8.08 (s, 1H), 7.64 (d, *J* = 8.2 Hz, 1H), 7.55 (d, *J* = 1.9 Hz, 1H), 7.45 - 7.31 (m, 2H), 6.87 (d, *J* = 11.3 Hz, 2H), 6.70 (s, 1H), 5.67 - 5.45 (m, 3H), 3.82-3.70 (m, 1H), 3.67 - 3.42 (m, 8H), 2.87 - 2.64 (m, 5H), 2.43 (s, 3H), 2.39 -2.22 (m, 7H), 2.18-2.09 (m, 2H), 1.90-1.78 (m, 4H), 1.73-1.24 (m, 14H), 1.18-0.94 (m, 4H).

### Example 73: 1-(2-methoxy-5-(9-((4-((1R,4R)-4-(7-methoxy-2-methyl-4-(((R)-1-(4-(2-((methylamino)methyl)phenyl)thien-2-yl)ethyl)amino)quinazolin-6-yl)cyclohexane-1-carbonyl)piperazin-1-yl)methyl)-3-azaspiro[5.5]undecane-3-carbonyl)phenyl)dihydropyrimidine-2,4(1H,3H)-dione

### Step 1: preparation of tert-butyl (2-(5-((R)-1-((6-((1R,4R)-4-(4-((3-(3-(2,4-dioxotetrahydropyrimidin-1(2H)-yl)-4-methoxybenzoyl)-3-azaspiro[5.5]undecan-9-yl)methyl)piperazine-1-carbonyl)cyclohexyl)-7-methoxy-2-methylquinazolin-4-yl)amino)ethyl)thien-3-yl)benzyl)(methyl)carboxylate

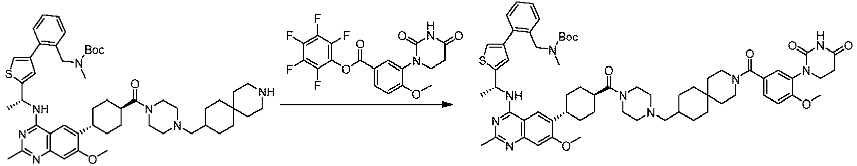

Pentafluorophenyl 3-(2,4-dioxotetrahydropyrimidin-1(2*H*)-yl)-4-methoxybenzoate (118 mg, 0.27 mmol) and (2-(5-((*R*)-1-((6-((1*R*,4*R*)-4-(4-((3-azaspiro[5.5]undecan-9-yl)methyl)piperazine-1-carbonyl)cyclohexyl)-7-methoxy-2-methylquinazolin-4-yl)amino)ethyl)thien-3-yl)benzyl)(methyl)carboxylic acid (300 mg, 0.34 mmol) were dissolved in DMSO (6 mL), and DIEA (132 mg, 1.02 mmol) was added. The reaction liquid was stirred at room temperature for 2 h. The reaction liquid was diluted with water (50 mL) and extracted with EA (50 mL × 3). The organic phases were combined, washed with brine (50 mL), dried over anhydrous sodium sulfate, filtered, and concentrated under reduced pressure. The crude product was purified by column chromatography (MeOH(NH₃)/DCM, 0%-20%) to give *tert*-butyl(2-(5-((*R*)-1-((6-((1*R*,4*R*)-4-(4-((3-(3-(2,4-dioxotetrahydropyrimidin-1(2*H*)-yl)-4-methoxybenzoyl)-3-azaspiro[5.5]undecan-9-yl)methyl)piperazine-1-carbonyl)cyclohexyl)-7-methoxy-2-methylquinazolin-4-yl)amino)ethyl)thien-3-yl)benzyl)(methyl)carboxylate.

LC-MS: (ESI, m/z): [M/2+H] ⁺ = 563.5.

### Step 2: preparation of 1-(2-methoxy-5-(9-((4-((1R,4R)-4-(7-methoxy-2-methyl-4-(((R)-1-(4-(2-((methylamino)methyl)phenyl)thien-2-yl)ethyl)amino)quinazolin-6-yl)cyclohexane-1-carbonyl)piperazin-1-yl)methyl)-3-azaspiro[5.5]undecane-3-carbonyl)phenyl)dihydropyrimidine-2,4(1H,3H)-dione

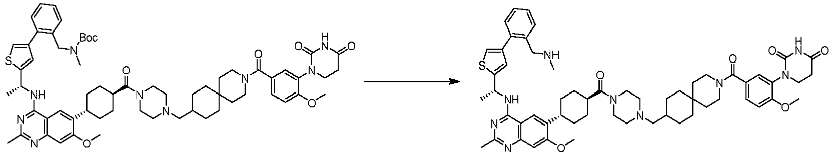

TFA (1 mL) was added to a solution of tert-butyl (2-(5-((*R*)-1-((6-((1*R*,4*R*)-4-(4-((3-(3-(2,4-dioxotetrahydropyrimidin-1(2*H*)-yl)-4-methoxybenzoyl)-3-azaspiro[5.5]undecan-9-yl)methyl)piperazine-1-carbonyl)cyclohexyl)-7-methoxy-2-methylquinazolin-4-yl)amino)ethyl)thien-3-yl)benzyl)(methyl)carboxylate (72 mg, 0.064 mmol) in DCM (3 mL) at room temperature while stirring. The reaction liquid was stirred at room temperature for 2 h. The reaction liquid was directly concentrated under reduced pressure. The crude product was purified by prep-HPLC to give 1-(2-methoxy-5-(9-((4-((1*R*,4*R*)-4-(7-methoxy-2-methyl-4-(((*R*)-1-(4-(2-((methylamino)methyl)phenyl)thien-2-yl)ethyl)amino)quinazolin-6-yl)cyclohexane-1-carbonyl)piperazin-1-yl)methyl)-3-azaspiro[5.5]undecane-3-carbonyl)phenyl)dihydropyrimidine-2,4(1*H*,3*H*)-dione.

LC-MS: (ESI, m/z): [M+H] ⁺=1024.4.

¹H NMR (400 MHz, CD₃OD) δ 7.99 (s, 1H), 7.46 - 7.36 (m, 3H), 7.34 - 7.27 (m, 3H), 7.22-7.12 (m, 3H), 7.01 (s, 1H), 6.10-6.02 (m, 1H), 3.95-3.91 (m, 6H), 3.75-3.45 (m, 12H), 3.12-3.01 (m, 1H), 2.80 (t, *J=* 6.7 Hz, 2H), 2.75-2.66 (m, 1H), 2.52 (s, 3H), 2.44 (m, 2H), 2.38 (m, 2H), 2.25 - 2.16 (m, 5H), 2.00 - 1.84 (m, 4H), 1.79 (d, *J* = 7.0 Hz, 4H), 1.71-1.54 (m, 9H), 1.37-1.12 (m, 7H).

### Example 74: 1-(2-fluoro-5-(9-((4-((1R,4R)-4-(7-methoxy-2-methyl-4-(((R)-1-(4-(2-((methylamino)methyl)phenyl)thien-2-yl)ethyl)amino)quinazolin-6-yl)cyclohexane-1-carbonyl)piperazin-1-yl)methyl)-3-azaspiro[5.5]undecane-3-carbonyl)phenyl)dihydropyrimidine-2,4(1H,3H)-dione

### Step 1: preparation of tert-butyl (2-(5-((R)-1-((6-((1R,4R)-4-(4-((3-(3-(2,4-dioxotetrahydropyrimidin-1(2H)-yl)-4-fluorobenzoyl)-3-azaspiro[5.5]undecan-9-yl)methyl)piperazine-1-carbonyl)cyclohexyl)-7-methoxy-2-methylquinazolin-4-yl)amino)ethyl)thien-3-yl)benzyl)(methyl)carboxylate

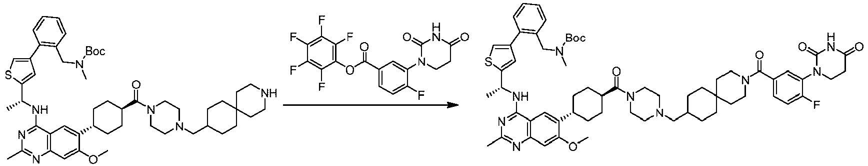

Pentafluorophenyl 3-(2,4-dioxotetrahydropyrimidin-1(2*H*)-yl)-4-fluorobenzoate (133 mg, 0.32 mmol) and *tert-*butyl (2-(5-((*R*)-1-((6-((1*R*,4*R*)-4-(4-((3-azaspiro[5.5]undecan-9-yl)methyl)piperazine-1-carbonyl)cyclohexyl)-7-methoxy-2-methylquinazolin-4-yl)amino)ethyl)thien-3-yl)benzyl)(methyl)carboxylate (350 mg, 0.39 mmol) were dissolved in DMSO (6 mL), and DIEA (132 mg, 1.02 mmol) was added. The reaction liquid was stirred at room temperature for 2 h. After the reaction was completed, the reaction liquid was diluted with water (50 mL) and extracted with EA (50 mL × 3). The organic phases were combined, washed with saturated brine (50 mL), dried over anhydrous sodium sulfate, filtered, and concentrated under reduced pressure. The crude product was purified by column chromatography (MeOH(NH₃)/DCM, 0%-20%) to give tert-butyl (2-(5-((*R*)-1-((6-((1*R*,4*R*)-4-(4-((3-(3-(2,4-dioxotetrahydropyrimidin-1(2*H*)-yl)-4-fluorobenzoyl)-3-azaspiro[5.5]undecan-9-yl)methyl)piperazine-1-carbonyl)cyclohexyl)-7-methoxy-2-methylquinazolin-4-yl)amino)ethyl)thien-3-yl)benzyl)(methyl)carboxylate. LC-MS: (ESI, m/z): [M/2+H] ⁺ = 556.9.

### Step 2: preparation of 1-(2-fluoro-5-(9-((4-((1R,4R)-4-(7-methoxy-2-methyl-4-(((R)-1-(4-(2-((methylamino)methyl)phenyl)thien-2-yl)ethyl)amino)quinazolin-6-yl)cyclohexane-1-carbonyl)piperazin-1-yl)methyl)-3-azaspiro[5.5]undecane-3-carbonyl)phenyl)dihydropyrimidine-2,4(1H,3H)-dione

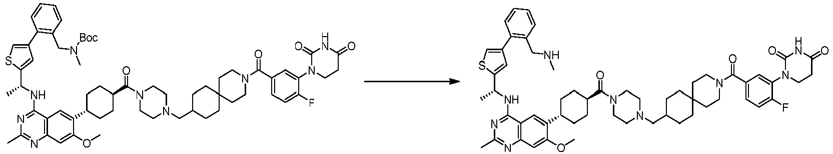

TFA (1 mL) was added to a solution of *tert*-butyl (2-(5-((*R*)-1-((6-((1*R*,4*R*)-4-(4-((3-(3-(2,4-dioxotetrahydropyrimidin-1(2*H*)-yl)-4-fluorobenzoyl)-3-azaspiro[5.5]undecan-9-yl)methyl)piperazine-1-carbonyl)cyclohexyl)-7-methoxy-2-methylquinazolin-4-yl)amino)ethyl)thien-3-yl)benzyl)(methyl)carboxylate (100 mg, 0.09 mmol) in DCM (3 mL) at room temperature. The reaction liquid was stirred at room temperature for 2 h. The reaction liquid was directly concentrated. The crude product was purified by prep-HPLC to give 1-(2-fluoro-5-(9-((4-((1*R*,4*R*)-4-(7-methoxy-2-methyl-4-(((*R*)-1-(4-(2-((methylamino)methyl)phenyl)thien-2-yl)ethyl)amino)quinazolin-6-yl)cyclohexane-1 -carbonyl)piperazin-1 -yl)methyl)-3 -azaspiro [5.5]undecane-3 - carbonyl)phenyl)dihydropyrimidine-2,4(1*H*,3*H*)-dione.

LC-MS: (ESI, m/z): [M+H] ⁺=1012.3.

¹H NMR (400 MHz, CD₃OD) δ 7.99 (s, 1H), 7.52 (dd, *J* = 7.1, 1.9 Hz, 1H), 7.46-7.39 (m, 2H), 7.36-7.27 (m, 4H), 7.21-7.17 (m, 1H), 7.15-7.11 (m, 1H), 7.01 (s, 1H), 6.08-6.03 (m, 1H), 3.95 (s, 3H), 3.83 (t, *J* = 6.7 Hz, 2H), 3.77-3.55 (m, 8H), 3.50-3.37 (m, 2H), 3.10-3.00 (m, 1H), 2.83 (t, *J* = 6.7 Hz, 2H), 2.76-2.65 (m, 1H), 2.52 (s, 3H), 2.44 (b, 2H), 2.38 (b, 2H), 2.25-2.16 (m, 5H), 2.00-1.83 (m, 4H), 1.82-1.75 (m, 4H), 1.69-1.46 (m, 9H), 1.36-1.06 (m, 7H).

### Example 75: 1-(5-(4-(((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)methoxy)methyl-d₂)piperidine-1-carbonyl)-2-chlorophenyl)dihydropyrimidine-2,4(1H,3H)-dione

### Step 1: preparation of tert-butyl 4-(hydroxymethyl-d₂)piperidine-1-carboxylate

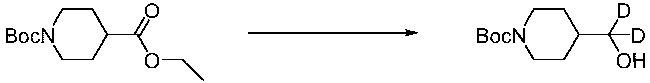

1-(*tert*-Butyl)-4-ethylpiperidine-1,4-dicarboxylate (500 mg, 1.93 mmol) was dissolved in ethanol (20 mL), and NaBD₄ (325 mg, 7.75 mmol) was added at 0 °C. The reaction liquid was stirred for 2 h. Water (30 mL) was added to the reaction liquid, and the resulting mixture was extracted with ethyl acetate (30 mL × 3). The organic phase was washed with saturated brine (100 mL), dried over anhydrous sodium sulfate, filtered, and concentrated under reduced pressure. The crude product was purified by column chromatography (ethyl acetate:petroleum ether =1:1) to give *tert*-butyl 4-(hydroxymethyl-*d*₂)piperidine-1-carboxylate.

¹H NMR (400 MHz, DMSO-*d*₆) δ 4.42 (s, 1H), 3.93 (d, *J* = 11.9 Hz, 2H), 2.67 (s, 2H), 1.62 (d, *J* = 12.9 Hz, 2H), 1.55 - 1.44 (m, 1H), 1.39 (s, 9H), 0.96 (m, 2H).

### Step 2: preparation of tert-butyl 4-((pyridin-4-ylmethoxy)methyl-d₂)piperidine-1-carboxylate

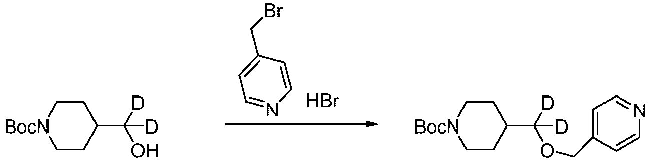

*tert*-Butyl 4-(hydroxymethyl-*d*₂)piperidine-1-carboxylate (250 mg, 1.14 mmol) was dissolved in anhydrous tetrahydrofuran (10 mL). The reaction liquid was cooled to 0 °C under nitrogen atmosphere. NaH (364 mg, 9.12 mmol) was added, and the resulting mixture was stirred for 3 h. 4-(Bromomethyl)pyridine hydrobromide (576 mg, 2.28 mmol) was added, and the resulting mixture was stirred at room temperature overnight. Water (20 mL) was added to the reaction liquid, and the resulting mixture was extracted with ethyl acetate (20 mL × 3). The organic phase was washed with saturated brine (60 mL), dried over anhydrous sodium sulfate, filtered, and concentrated under reduced pressure. The crude product was purified by column chromatography (methanol:dichloromethane = 1:9) to give *tert*-butyl 4-((pyridin-4-ylmethoxy)methyl-*d*₂)piperidine-1-carboxylate.

LC-MS: (ESI, m/z): [M+H] ⁺=309.1

¹H NMR (400 MHz, DMSO-*d*₆) δ 8.53 (dd, *J* = 4.5, 1.5 Hz, 2H), 7.31 (d, *J* = 5.8 Hz, 2H), 4.51 (s, 2H), 3.94 (d, *J* = 12.0 Hz, 2H), 2.70 (s, 2H), 1.76 (m, 1H), 1.67 (d, *J* = 12.9 Hz, 2H), 1.39 (s, 9H), 1.06 (m, 2H).

### Step 3: preparation of tert-butyl 4-((piperidin-4-ylmethoxy)methyl-d₂)piperidine-1-carboxylate

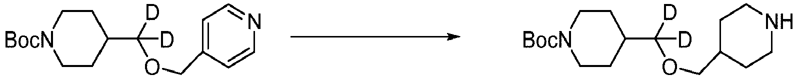

*tert*-Butyl 4-((pyridin-4-ylmethoxy)methyl-*d*₂)piperidine-1-carboxylate (180 mg, 0.58 mmol) was dissolved in isopropanol (10 mL) and water (10 mL), and Pd/C (60 mg, 0.058 mmol) was added. The reaction liquid was heated to 70 °C under hydrogen atmosphere and stirred overnight. The reaction liquid was cooled to room temperature and filtered, and the filtrate was concentrated under reduced pressure to give *tert*-butyl 4-((piperidine-4-ylmethoxyl)methyl-*d*₂)piperidine-1-carboxylate. The crude product was directly used in the next step without purification.

LC-MS: (ESI, m/z): [M+H] ⁺=315.2.

### Step 4: preparation of tert-butyl 4-(((1-((1R,4R)-4-(4-(((R)-1-(3-((tert-butoxycarbonyl)amino)-5-(trifluoromethyl)phenyl)ethyl)amino)-7-methoxy-2-methylquinazolin-6-yl)cyclohexane-1-carbonyl)piperidin-4-yl)methoxy)methyl-d₂)piperidine-1-carboxylate

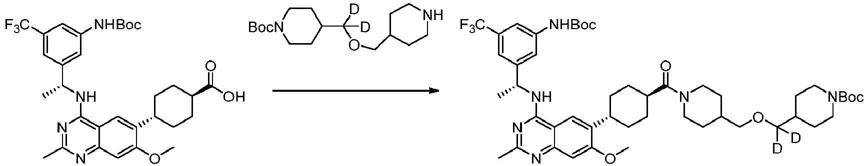

*tert*-Butyl 4-((piperidin-4-ylmethoxy)methyl-*d*₂)piperidine-1-carboxylate (130 mg, 0.41 mmol) was dissolved in DMF (10 mL), and (1*R*,4*R*)-4-(4-(((*R*)-1-(3-((*tert*-butoxycarbonyl)amino)-5-(trifluoromethyl)phenyl)ethyl)amino)-7-methoxy-2-methylquinazolin-6-yl)cyclohexane-1-carboxylic acid (150 mg, 0.25 mmol), HATU (123 mg, 0.32 mmol), and DIEA (96 mg, 0.74 mmol) were added. The reaction liquid was stirred at room temperature for 1 h. The reaction liquid was diluted with water (100 mL) and extracted with ethyl acetate (30 mL × 3). The organic phase was washed with saturated brine (50 mL), dried over anhydrous sodium sulfate, filtered, and concentrated under reduced pressure. The crude product was purified by column chromatography (methanol:dichloromethane = 1:9) to give *tert*-butyl 4-(((1-((1*R*,4*R*)-4-(4-(((*R*)-1-(3-((*tert*-butoxycarbonyl)amino)-5-(trifluoromethyl)phenyl)ethyl)amino)-7-methoxy-2-methylquinazolin-6-yl)cyclohexane-1-carbonyl)piperidin-4-yl)methoxy)methyl-*d*₂)piperidine-1-carboxylate.

LC-MS: (ESI, m/z): [M+H] ⁺=899.5.

### Step 5: preparation of ((1R,4R)-4-(4-(((R)-1-(3-amino-5-(trifluoromethyl)phenyl)ethyl)amino)-7-methoxy-2-methylquinazolin-6-yl)cyclohexyl)(4-((piperidin-4-ylmethoxy-d₂)methyl)piperidin-1-yl)methanone

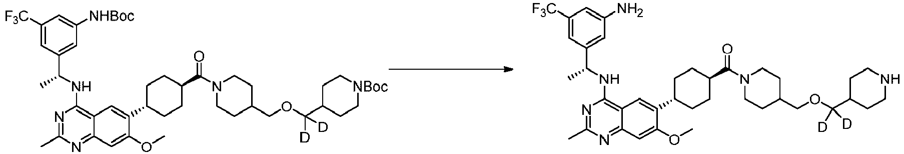

*tert*-Butyl 4-(((1-((1*R*,4*R*)-4-(4-(((*R*)-1-(3-((*tert*-butoxycarbonyl)amino)-5-(trifluoromethyl)phenyl)ethyl)amino)-7-methoxy-2-methylquinazolin-6-yl)cyclohexane-1-carbonyl)piperidin-4-yl)methoxy)methyl-*d*₂)piperidine-1-carboxylate (200 mg, 0.22 mmol) was dissolved in DCM (5 mL), and TFA (2 mL) was added. The reaction liquid was stirred at room temperature for 2 h. The reaction liquid was directly concentrated under reduced pressure to give ((1*R*,4*R*)-4-(4-(((*R*)-1-(3-amino-5-(trifluoromethyl)phenyl)ethyl)amino)-7-methoxy-2-methylquinazolin-6-yl)cyclohexyl)(4-((piperidin-4-ylmethoxy-*d*₂)methyl)piperidin-1-yl)methanone.

LC-MS: (ESI, m/z): [M+H] ⁺=699.4

### Step 6: preparation of 1-(5-(4-(((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)methoxy)methyl-d₂)piperidine-1-carbonyl)-2-chlorophenyl)dihydropyrimidine-2,4(1H,3H)-dione

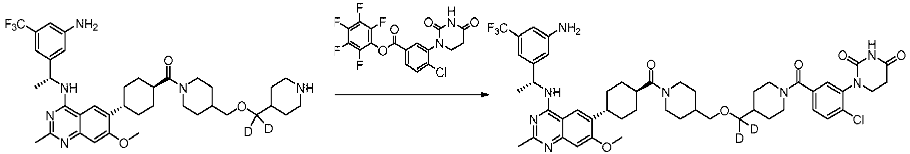

((1*R*,4*R*)-4-(4-(((*R*)-1-(3-Amino-5-(trifluoromethyl)phenyl)ethyl)amino)-7-methoxy-2-methylquinazolin-6-yl)cyclohexyl)(4-((piperidin-4-ylmethoxy-*d*₂)methyl)piperidin-1-yl)methanone (crude product, 200 mg, 0.22 mmol) was dissolved in DMSO (10 mL), and pentafluorophenyl 4-chloro-3-(2,4-dioxotetrahydropyrimidin-1(2*H*)-yl)benzoate (95 mg, 0.22 mmol) and DIEA (86 mg, 0.66 mmol) were added. The reaction liquid was stirred at room temperature for 2 h. Water (100 mL) was added to the reaction liquid, and the resulting mixture was extracted with dichloromethane (30 mL × 3). The organic phase was concentrated under reduced pressure to give the crude product. The crude product was purified by pre-HPLC (CH₃CN/0.08% aqueous NH₄HCO₃ solution, 5%-95%) to give 1-(5-(4-(((1-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperidin-4-yl)methoxy)methyl-*d*₂)piperidine-1-carbonyl)-2-chlorophenyl)dihydropyrimidine-2,4(1*H*,3*H*)-dione.

LC-MS: (ESI, m/z): [M+H] ⁺=949.4.

¹H NMR (400 MHz, DMSO-*d*₆) δ 10.50 (s, 1H), 8.15 - 8.02 (m, 2H), 7.63 (d, *J* = 8.2 Hz, 1H), 7.54 (d, *J* = 1.8 Hz, 1H), 7.38 (dd, *J* = 8.2, 1.9 Hz, 1H), 6.99 (s, 1H), 6.87 (s, 1H), 6.85 (s, 1H), 6.70 (s, 1H), 5.63 - 5.48 (m, 3H), 4.51-4.36 (m, 2H), 3.99 - 3.86 (m, 4H), 3.78-3.71 (m, 1H), 3.65-3.53 (m, 2H), 3.23 (d, *J =* 6.1 Hz, 2H), 3.09-2.90 (m, 3H), 2.81-2.62 (m, 4H), 2.35 (s, 3H), 1.91 - 1.52 (m, 18H), 1.18-0.92 (m, 4H).

### Example 76: 1-(5-(4-(((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)methoxy-d₂)methyl)piperidine-1-carbonyl)-2-chlorophenyl)dihydropyrimidine-2,4(1H,3H)-dione

### Step 1: preparation of tert-butyl 4-(((1-(4-chloro-3-(2,4-dioxotetrahydropyrimidin-1(2H)-yl)benzoyl)piperidin-4-yl)methoxy)methyl-d₂)piperidine-1-carboxylate

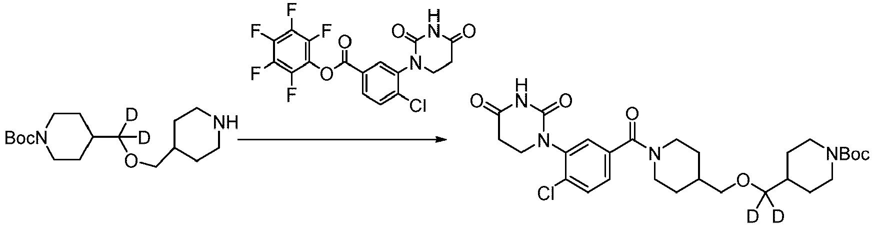

*tert*-Butyl 4-((piperidin-4-ylmethoxy)methyl-*d*₂)piperidine-1-carboxylate (180 mg, 0.57 mmol) was dissolved with dimethylsulfoxide (5 mL), and pentafluorophenyl 4-chloro-3-(2,4-dioxotetrahydropyrimidin-1(2*H*)-yl)benzoate (247 mg, 0.57 mmol) and *N,N*-diisopropylethylamine (220 mg, 1.71 mmol) were added. The reaction liquid was stirred at room temperature for 2 h, diluted with water (50 mL). The organic phase was extracted with dichloromethane (30 mL × 3), washed with saturated brine (100 mL), dried over anhydrous sodium sulfate, filtered, concentrated under reduced pressure, and the crude product was purified by column chromatography (methanol:dichloromethane = 1:9) to give *tert*-butyl 4-(((1-(4-chloro-3-(2,4-dioxotetrahydropyrimidin-1(2*H*)-yl)benzoyl)piperidin-4-yl)methoxy)methyl-*d*₂)piperidine-1-carboxylate.

LC-MS: (ESI, m/z): [M+H] ⁺=565.2.

### Step 2: preparation of 1-(2-chloro-5-(4-((piperidin-4-ylmethoxy-d₂)methyl)piperidine-1-carbonyl)phenyl)dihydropyrimidine-2,4(1H,3H-dione

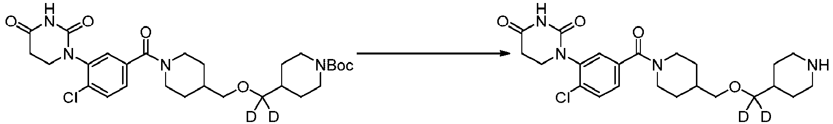

tert-Butyl 4-(((1-(4-chloro-3-(2,4-dioxotetrahydropyrimidin-1(2*H*)-yl)benzoyl)piperidin-4-yl)methoxy)methyl-*d*₂)piperidine-1-carboxylate (220 mg, 0.39 mmol) was dissolved with dichloromethane (5 mL), and trifluoroacetic acid (2 mL) was added. The reaction liquid was stirred at room temperature for 2 h and directly concentrated under reduced pressure to give 1-(2-chloro-5-(4-((piperidin-4-ylmethoxy-*d*₂)methyl)piperidine-1-carbonyl)phenyl)dihydropyrimidine-2,4(1*H*,3*H*)-dione, which was directly used in the next step without purification.

LC-MS: (ESI, m/z): [M+H] ⁺=465.3.

### Step 3: preparation of tert-butyl (3-((R)-1-((6-((1R,4R)-4-(4-(((1-(4-chloro-3-(2,4-dioxotetrahydropyrimidin-1(2H)-yl)benzoyl)piperidin-4-yl)methoxy)methyl-d₂)piperidine-1-carbonyl)cyclohexyl)-7-methoxy-2-methylquinazolin-4-yl)amino)ethyl)-5-(trifluoromethyl)phenyl)carboxylate

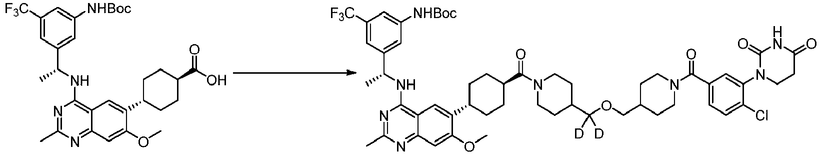

1-(2-Chloro-5 -(4-((piperidin-4-ylmethoxy-*d*₂)methyl)piperidine-1-carbonyl)phenyl)dihydropyrimidine-2,4(1*H*,3*H*)-dione (220 mg, 0.39 mmol) was dissolved with DMF (10 mL), and (1*R*,4*R*)-4-(4-(((*R*)*-*1*-*(3-((*tert-*butoxycarbonyl)amino)-5-(trifluoromethyl)phenyl)ethyl)amino)-7-methoxy-2-methylquinazolin-6-yl)cyclohexane-1-carboxylic acid (150 mg, 0.24 mmol), HATU (118 mg, 0.31 mmol), and *N,N-*diisopropylethylamine (93 mg, 0.72 mmol) were separately added. The reaction liquid was stirred at room temperature for 2 h. Water (100 mL) was added, and the resulting mixture was extracted with ethyl acetate (30 mL × 3). The organic phase was washed with saturated brine (100 mL), dried over anhydrous sodium sulfate, filtered, and concentrated under reduced pressure. The crude product was purified by column chromatography (methanol:dichloromethane = 1:9) to give *tert*-butyl (3-((*R*)-1-((6-((1*R*,4*R*)-4-(4-(((1-(4-chloro-3-(2,4-dioxotetrahydropyrimidin-1(2*H*)-yl)benzoyl)piperidin-4-yl)methoxy)methyl-*d*₂)piperidine-1-carbonyl)cyclohexyl)-7-methoxy-2-methylquinazolin-4-yl)amino)ethyl)-5-(trifluoromethyl)phenyl)carboxylate. LC-MS: (ESI, m/z): [M+H] ⁺=1049.3.

### Step 4: preparation of 1-(5-(4-(((1-((1R,4R)-4-(4-(((R)-1-(3-amino-5-(trifluoromethyl)phenyl)ethyl)amino)-7-methoxy-2-methylquinazolin-6-yl)cyclohexane-1-carbonyl)piperidin-4-yl)methoxy-d₂)methyl)piperidine-1-carbonyl)-2-chlorophenyl)dihydropyrimidine-2,4(1H,3H)-dione

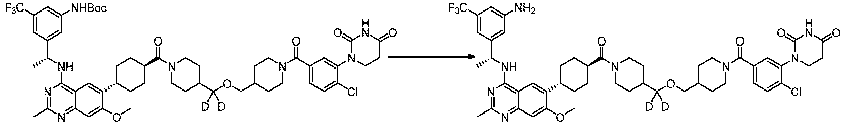

*tert*-Butyl (3-((*R*)-1-((6-((1*R*,4*R*)-4-(4-(((1-(4-chloro-3-(2,4-dioxotetrahydropyrimidin-1(2*H*)-yl)benzoyl)piperidin-4-yl)methoxy)methyl-*d*₂)piperidine-1-carbonyl)cyclohexyl)-7-methoxy-2-methylquinazolin-4-yl)amino)ethyl)-5-(trifluoromethyl)phenyl)carboxylate (200 mg, 0.19 mmol) was dissolved with dichloromethane (5 mL), and trifluoroacetic acid (2 mL) was added. The reaction liquid was stirred at room temperature for 2 h and concentrated under reduced pressure. The resulting crude product was dissolved with a mixed solution (20 mL) of 10% methanol/dichloromethane. Then 5% aqueous sodium bicarbonate solution (20 mL) was added, and the resulting mixture was stirred for 1 h and left to stand for separation. The organic phase was dried over anhydrous sodium sulfate, filtered, and concentrated under reduced pressure to give the crude product, which was purified by pre-HPLC (CH₃CN/0.08% aqueous NH₄HCO₃ solution, 5%-95%) to give 1-(5-(4-(((1-((1*R*,4*R*)-4-(4-(((*R*)-1-(3-amino-5-(trifluoromethyl)phenyl)ethyl)amino)-7-methoxy-2-methylquinazolin-6-yl)cyclohexane-1-carbonyl)piperidin-4-yl)methoxy-*d*₂)methyl)piperidine-1-carbonyl)-2-chlorophenyl)dihydropyrimidine-2,4(1*H*,3*H*)-dione.

LC-MS: (ESI, m/z): [M+H] ⁺=949.2.

¹H NMR (400 MHz, DMSO-*d*₆) δ 10.49 (s, 1H), 8.13 (d, *J* = 7.7 Hz, 1H), 8.06 (s, 1H), 7.63 (d, *J* = 8.2 Hz, 1H), 7.54 (d, *J* = 2.0 Hz, 1H), 7.37 (dd, *J =* 8.2, 2.0 Hz, 1H), 6.99 (s, 1H), 6.86 (d, *J =* 10.1 Hz, 2H), 6.70 (s, 1H), 5.57 (dd, *J* = 17.7, 10.2 Hz, 3H), 4.42 (d, *J* = 12.0 Hz, 2H), 3.92 (m, 4H), 3.81 - 3.51 (m, 3H), 3.24 (d, *J* = 6.2 Hz, 2H), 3.09 - 2.88 (m, 3H), 2.78 - 2.60 (m, 4H), 2.36 (s, 3H), 1.90 - 1.52 (m, 18H), 1.19 - 0.92 (m, 4H).

### Example 77: (R)-2-(4-(4-((1-(3-amino-5-(trifluoromethyl)phenyl)ethyl)amino)-2-methylquinazolin-6-yl)piperidin-1-yl)-N-(2-(3-(4-chloro-3-(2,4-dioxotetrahydropyrimidin-1(2H)-yl)benzoyl)-3-azaspiro[5.5]undecan-9-yl)ethyl)acetamide

### Step 1: preparation of 6-bromo-2-methylquinazolin-4-ol

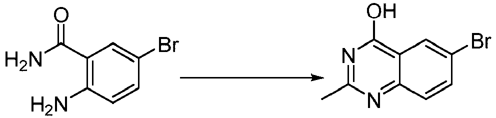

2-Amino-5-bromobenzamide (4.0 g, 14.55 mmol) was added to a solution of trimethyl orthoacetate (17.46 g, 145.5 mmol) in methanol (100 mL). The reaction liquid was stirred at 120 °C overnight in a sealed container, cooled, and concentrated under reduced pressure to give the crude product 6-bromo-2-methylquinazolin-4-ol, which was directly used in the next step without purification.

LC-MS: (ESI, m/z): [M+H] ⁺=238.9.

### Step 2: preparation of tert-butyl 4-(4-hydroxy-2-methylquinazolin-6-yl)-3,6-dihydropyridine-1(2H)-carboxylate

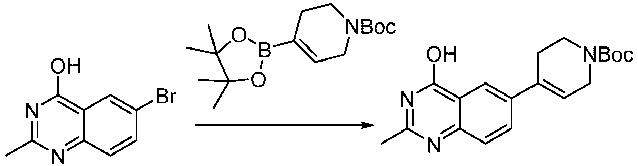

6-Bromo-2-methylquinazolin-4-ol (5.00 g, 16.67 mmol) was dissolved with DMF/H₂O (200 mL/20 mL), and potassium carbonate (5.80 g, 42.03 mmol), Pd(dppf)Cl₂ (1.54 g, 2.10 mmol), and tert-butyl 4-(4,4,5,5-tetramethyl-1,3,2-dioxaborolan-2-yl)-3,6-dihydropyridine-1(2*H*)-carboxylate (7.79 g, 25.21 mmol) were separately added. The reaction liquid was stirred at 110 °C overnight under nitrogen atmosphere, diluted with water (500 mL), and extracted with ethyl acetate (200 mL × 3). The organic phase was washed with saturated brine (200 mL), dried over anhydrous sodium sulfate, filtered, and concentrated under reduced pressure. The crude product was purified by column chromatography (PE:EA = 1:2) to give tert-butyl 4-(4-hydroxy-2-methylquinazolin-6-yl)-3,6-dihydropyridine-1(2*H*)-carboxylate.

LC-MS: (ESI, m/z): [M+H] ⁺=342.1.

### Step 3: preparation of tert-butyl 4-(4-hydroxy-2-methylquinazolin-6-yl)piperidine-1-carboxylate

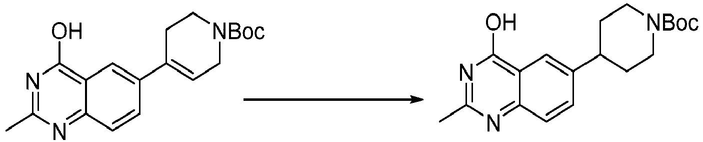

tert-Butyl 4-(4-hydroxy-2-methylquinazolin-6-yl)-3,6-dihydropyridine-1(2*H*)-carboxylate (2.5 g, 14.55 mmol) was added to a solution of Pd(OH)₂ (800 mg, 20%) in methanol (100 mL). The reaction liquid was stirred at 70 °C overnight in an autoclave under H₂ atmosphere (10 atm), cooled, filtered, and concentrated under reduced pressure to give the crude product tert-butyl 4-(4-hydroxy-2-methylquinazolin-6-yl)piperidine-1-carboxylate.

LC-MS: (ESI, m/z): [M+H] ⁺=344.1.

### Step 4: preparation of tert-butyl (R)-4-(2-methyl-4-((1-(3-nitro-5-(trifluoromethyl)phenyl)ethyl)amino)quinazolin-6-yl)piperidine-1 -carboxylate

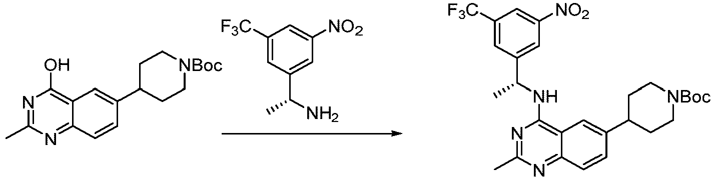

BOP (3.30 g, 7.47 mmol), DBU (1.90 g, 12.5 mmol), and (*R*)-1-(3-nitro-5-(trifluoromethyl)phenyl)ethan-1-amine (1.53 g, 6.54 mmol) were separately added to a solution of *tert*-butyl 4-(4-hydroxy-2-methylquinazolin-6-yl)piperidine-1-carboxylate (1.73 g, 5.03 mmol) in DMF (100 mL). The reaction liquid was stirred at 70 °C overnight, diluted with water (300 mL), and extracted with ethyl acetate (100 mL). The organic phase was washed with saturated brine (150 mL), dried over anhydrous sodium sulfate, filtered, and concentrated under reduced pressure. The crude product was purified by reversed-phase column chromatography (H₂O:ACN = 1:2) to give *tert-*butyl (*R*)-4-(2-methyl-4-((1-(3-nitro-5-(trifluoromethyl)phenyl)ethyl)amino)quinazolin-6-yl)piperidine-1-carboxylate.

LC-MS: (ESI, m/z): [M+H] ⁺=560.4.

### Step 5: preparation of (R)-2-methyl-N-(1-(3-nitro-5-(trifluoromethyl)phenyl)ethyl)-6-(piperidin-4-yl)quinazolin-4-amine

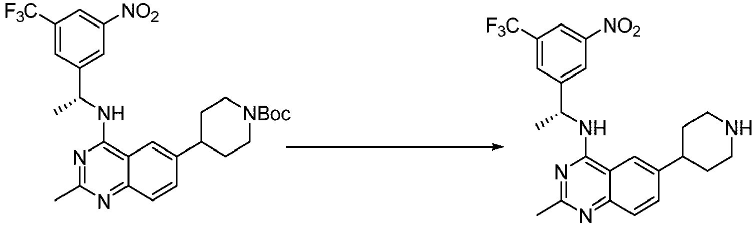

*tert*-Butyl (*R*)-4-(2-methyl-4-((1-(3-nitro-5-(trifluoromethyl)phenyl)ethyl)amino)quinazolin-6-yl)piperidine-1-carboxylate (300 mg, 0.537 mmol) was added to TFA (2 mL) in dichloromethane (20 mL). The reaction liquid was stirred at room temperature for 1 h and concentrated under reduced pressure to give the crude product (*R*)-2-methyl-*N*-(1-(3-nitro-5-(trifluoromethyl)phenyl)ethyl)-6-(piperidin-4-yl)quinazolin-4-amine.

LC-MS: (ESI, m/z): [M+H] ⁺=460.4.

### Step 6: preparation of tert-butyl (R)-2-(4-(2-methyl-4-((1-(3-nitro-5-(trifluoromethyl)phenyl)ethyl)amino)quinazolin-6-yl)piperidin-1-yl)acetate

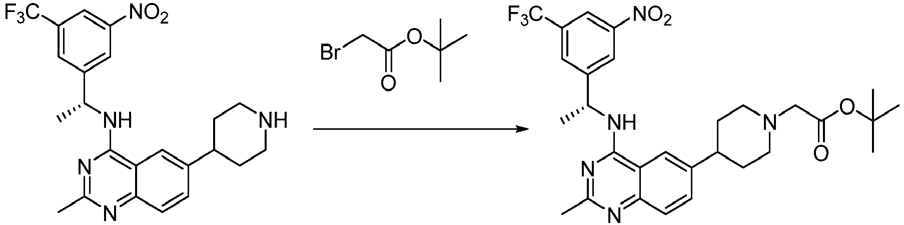

(*R*)-2-Methyl-*N*-(1-(3-nitro-5-(trifluoromethyl)phenyl)ethyl)-6-(piperidin-4-yl)quinazolin-4-amine (250 mg, 0.543 mmol) and potassium carbonate (300 mg, 2.17 mmol) were added to a solution of *tert*-butyl 2-bromoacetate (138 mg, 0.708 mmol) in THF (20 mL). The reaction liquid was stirred at 70 °C overnight, diluted with water (50 mL), extracted with ethyl acetate (50 mL × 3), dried over anhydrous sodium sulfate, filtered, and concentrated under reduced pressure. The crude product was purified by column chromatography (PE:EA = 5:1) to give *tert-*butyl (*R*)-2-(4-(2-methyl-4-((1-(3-nitro-5-(trifluoromethyl)phenyl)ethyl)amino)quinazolin-6-yl)piperidin-1-yl)acetate.

LC-MS: (ESI, m/z): [M+H] ⁺=574.3.

### Step 7: preparation of (R)-2-(4-(2-methyl-4-((1-(3-nitro-5-(trifluoromethyl)phenyl)ethyl)amino)quinazolin-6-yl)piperidin-1-yl)acetic acid

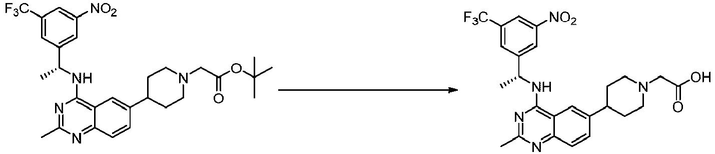

*tert*-Butyl (*R*)-2-(4-(2-methyl-4-((1-(3-nitro-5-(trifluoromethyl)phenyl)ethyl)amino)quinazofin-6-yl)piperidin-1-yl)acetate (300 mg, 0.524 mmol) was added to HCl/1,4-dioxane (4 M, 20 mL). The reaction liquid was stirred at room temperature overnight and directly concentrated under reduced pressure to give the crude product (*R*)-2-(4-(2-methyl-4-((1-(3-nitro-5-(trifluoromethyl)phenyl)ethyl)amino)quinazolin-6-yl)piperidin-1-yl)acetic acid.

LC-MS: (ESI, m/z): [M+H] ⁺=518.1.

### Step 8: preparation of tert-butyl (R)-9-(2-(2-(4-(2-methyl-4-((1-(3-nitro-5-(trifluoromethyl)phenyl)ethyl)amino)quinazolin-6-yl)piperidin-1-yl)acetamido)ethyl)-3-azaspiro[5.5]undecane-3-carboxylate

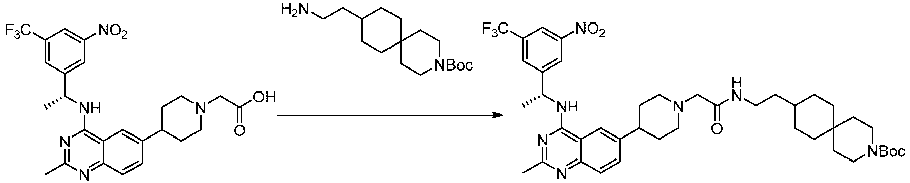

(*R*)-2-(4-(2-Methyl-4-((1-(3-nitro-5-(trifluoromethyl)phenyl)ethyl)amino)quinazolin-6-yl)piperidin-1-yl)acetic acid (167 mg, 0.323 mmol), HATU (154 mg, 0.405 mmol), and DIEA (105 mg, 0.814 mmol) were added to a solution of *tert*-butyl 9-(2-aminoethyl)-3-azaspiro[5.5]undecane-3-carboxylate (80 mg, 0.27 mmol) in DMF (20 mL). The reaction liquid was stirred at room temperature overnight, diluted with water (50 mL), and extracted with ethyl acetate (50 mL × 3). The crude product was purified by reversed-phase column chromatography (ACN:H₂O = 1:3) to give *tert*-butyl (*R*)-9-(2-(2-(4-(2-methyl-4-((1-(3-nitro-5-(trifluoromethyl)phenyl)ethyl)amino)quinazolin-6-yl)piperidin-1-yl)acetamido)ethyl)-3-azaspiro[5.5]undecane-3-carboxylate.

LC-MS: (ESI, m/z): [M+H] ⁺=796.4.

### Step 9: preparation of tert-butyl (R)-9-(2-(2-(4-(4-((1-(3-amino-5-(trifluoromethyl)phenyl)ethyl)amino)-2-methylquinazolin-6-yl)piperidin-1-yl)acetamido)ethyl)-3-azaspiro[5.5]undecane-3-carboxylate

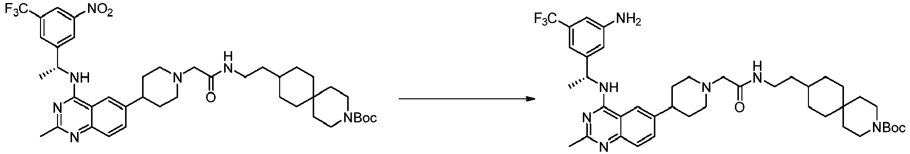

*tert*-Butyl (*R*)-9-(2-(2-(4-(2-methyl-4-((1-(3-nitro-5-(trifluoromethyl)phenyl)ethyl)amino)quinazolin-6-yl)piperidin-1-yl)acetamido)ethyl)-3-azaspiro[5.5]undecane-3-carboxylate (70 mg, 0.088 mmol) was dissolved with ethanol (10 mL), and Raney-Ni (0.3 mL, water suspension) and hydrazine hydrate monohydrate (0.2 mL) were separately added. The reaction liquid was stirred at room temperature for 1 h, filtered, and concentrated under reduced pressure to give the crude product *tert*-butyl (*R*)-9-(2-(2-(4-(4-((1-(3-amino-5-(trifluoromethyl)phenyl)ethyl)amino)-2-methylquinazolin-6-yl)piperidin-1-yl)acetamido)ethyl)-3-azaspiro [5.5]undecane-3 -carboxylate.

LC-MS: (ESI, m/z): [M+H] ⁺=766.6.

### Step 10: preparation of (R)-N-(2-(3-azaspiro[5.5]undecan-9-yl)ethyl)-2-(4-(4-((1-(3-amino-5-(trifluoromethyl)phenyl)ethyl)amino)-2-methylquinazolin-6-yl)piperidin-1-yl)acetamide

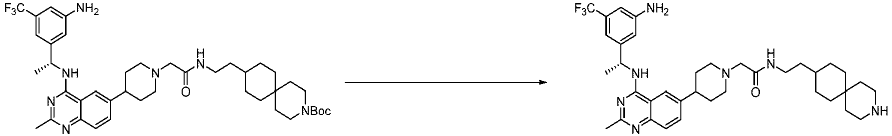

*tert*-Butyl (*R*)-9-(2-(2-(4-(4-((1-(3-amino-5-(trifluoromethyl)phenyl)ethyl)amino)-2-methylquinazolin-6-yl)piperidin-1-yl)acetamido)ethyl)-3-azaspiro[5.5]undecane-3-carboxylate (65 mg, 0.085 mmol) was dissolved with DCM (10 mL), and TFA (1 mL) was added. The reaction liquid was stirred at room temperature for 1 h and directly concentrated under reduced pressure to give the crude product (*R*)-*N*-(2-(3-azaspiro[5.5]undecane-9-yl)ethyl)-2-(4-(4-((1-(3-amino-5-(trifluoromethyl)phenyl)ethyl)amino)-2-methylquinazolin-6-yl)piperidin-1-yl)acetamide.

LC-MS: (ESI, m/z): [M+H] ⁺=666.4.

### Step 11: preparation of (R)-2-(4-(4-((1-(3-amino-5-(trifluoromethyl)phenyl)ethyl)amino)-2-methylquinazolin-6-yl)piperidin-1-yl)-N-(2-(3-(4-chloro-3-(2,4-dioxotetrahydropyrimidin-1(2H)-yl)benzoyl)-3-azaspiro[5.5]undecan-9-yl)ethyl)acetamide

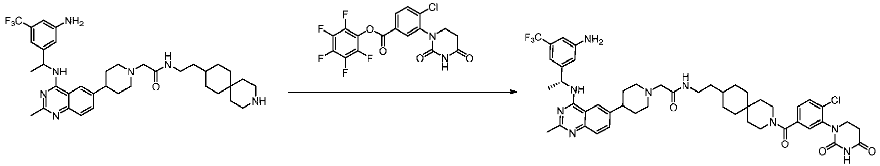

(*R*)-*N*-(2-(3-Azaspiro[5.5]undecan-9-yl)ethyl)-2-(4-(4-((1-(3-amino-5-(trifluoromethyl)phenyl)ethyl)amino)-2-methylquinazolin-6-yl)piperidin-1-yl)acetamide (50 mg, 0.075 mmol) was dissolved with DMSO (10 mL), and pentafluorophenyl 4-chloro-3-(2,4-dioxotetrahydropyrimidin-1(2*H*)-yl)benzoate (40 mg, 0.092 mmol) and DIEA (30 mg, 0.232 mmol) were separately added. The reaction liquid was stirred at room temperature overnight, diluted with water (20 mL), and extracted with dichloromethane (30 mL × 3). The organic phase was concentrated under reduced pressure. The crude product was purified by preparative reversed-phase chromatography to give (*R*)-2-(4-(4-((1-(3-amino-5-(trifluoromethyl)phenyl)ethyl)amino)-2-methylquinazolin-6-yl)piperidin-1-yl)-*N*-(2-(3-(4-chloro-3-(2,4-dioxotetrahydropyrimidin-1(2*H*)-yl)benzoyl)-3-azaspiro[5.5]undecan-9-yl)ethyl)acetamide. LC-MS: (ESI, m/z): [M+H] ⁺=916.5.

¹H NMR (400 MHz, DMSO-*d*₆) δ 10.51 (s, 1H), 8.27 (d, *J* = 7.9 Hz, 1H), 8.21 (s, 1H), 7.70-7.58 (m, 3H), 7.52 (d, *J* = 8.7 Hz, 2H), 7.37 (dd, *J* = 8.2, 1.8 Hz, 1H), 6.90 (s, 1H), 6.86 (s, 1H), 6.70 (s, 1H), 5.64 - 5.43 (m, 3H), 3.79 - 3.69 (m, 1H), 3.59-3.45 (m, 3H), 3.28 - 3.20 (m, 2H), 3.17-3.07 (m, 2H), 2.98-2.86 (m, 4H), 2.77 - 2.70 (m, 2H), 2.69 - 2.56 (m, 1H), 2.38 (s, 3H), 2.26-2.14 (m, 2H), 1.92 - 1.77 (m, 4H), 1.74-1.63 (m, 2H), 1.56 - 1.20 (m, 12H), 1.14-0.94 (m, 4H).

Example 78: 1-(5-(9-((4-((1*R*,4*R*)-4-(4-(((*R*)-1-(3-amino-5-(trifluoromethyl)phenyl)ethyl)amino)-2-methylpyrido[3,4-*d*]pyrimidin-6-yl)cyclohexane-1-carbonyl)piperazin-1-yl)methyl)-3-azaspiro[5.5]undecane-3-carbonyl)-2-chlorophenyl)dihydropyrimidine-2,4(1*H*,3*H*)-dione

### Step 1: preparation of benzyl 9-((4-((1R,4R)-4-(2-methyl-4-(((R)-1-(3-nitro-5-(trifluoromethyl)phenyl)ethyl)amino)pyrido[3,4-d]pyrimidin-6-yl)cyclohexane-1-carbonyl)piperazin-1-yl)methyl)-3 -azaspiro [5.5]undecane-3 -carboxylate

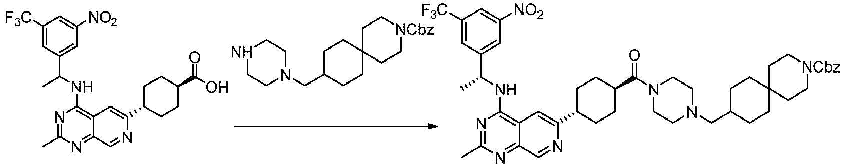

(1*R*,4*R*)-4-(2-Methyl-4-((1-(3-nitro-5-(trifluoromethyl)phenyl)ethyl)amino)pyrido[3,4-*d*]pyrimidin-6-yl)cyclohexane-1-carboxyfic acid (170 mg, 0.337 mmol), HATU (192 mg, 0.505 mmol), and DIEA (130 mg, 1.01 mmol) were added to a solution of benzyl 9-(piperazin-1-ylmethyl)-3-azaspiro[5.5]undecane-3-carboxylate in DMF (30 mL). The reaction liquid was stirred at room temperature overnight, diluted with water (50 mL), and extracted with dichloromethane (50 mL × 3). The crude product was purified by reversed-phase column chromatography (ACN:H₂O = 1:2) to give benzyl 9-((4-((1*R*,4*R*)-4-(2-methyl-4-(((*R*)-1-(3-nitro-5-(trifluoromethyl)phenyl)ethyl)amino)pyrido[3,4-*d*]pyrimidin-6-yl)cyclohexane-1-carbonyl)piperazin-1-yl)methyl)-3 -azaspiro [5.5]undecane-3 -carboxylate.

LC-MS: (ESI, m/z): [M+H] ⁺=871.6.

### Step 2: preparation of benzyl 9-((4-((1R,4R)-4-(4-(((R)-1-(3-amino-5-(trifluoromethyl)phenyl)ethyl)amino)-2-methylpyrido[3,4-d]pyrimidin-6-yl)cyclohexane-1-carbonyl)piperazin-1-yl)methyl)-3-azaspiro[5.5]undecane-3-carboxylate

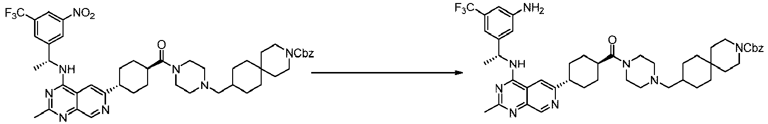

Benzyl 9-((4-((1*R*,4*R*)-4-(2-methyl-4-(((*R*)-1-(3 -nitro-5 -(trifluoromethyl)phenyl)ethyl)amino)pyrido [3,4-*d*]pyrimidin-6-yl)cyclohexane-1-carbonyl)piperazin-1-yl)methyl)-3-azaspiro[5.5]undecane-3-carboxylate (240 mg, 0.276 mmol) was dissolved in ethanol (20 mL), and Raney-Ni (water suspension, 0.3 mL) and hydrazine hydrate monohydrate (85%, 0.5 mL) were added. The reaction liquid was stirred at room temperature for 1 h, filtered, and concentrated under reduced pressure to give the crude product benzyl 9-((4-((1*R*,4*R*)-4-(4-(((*R*)-1-(3-amino-5-(trifluoromethyl)phenyl)ethyl)amino)-2-methylpyrido[3,4-*d*]pyrimidin-6-yl)cyclohexane-1-carbonyl)piperazin-1-yl)methyl)-3 -azaspiro [5.5]undecane-3 -carboxylate.

LC-MS: (ESI, m/z): [M+H] ⁺=841.6.

### Step 3: preparation of (4-((3-azaspiro[5.5]undecan-9-yl)methyl)piperazin-1-yl)((1R,4R)-4-(4-(((R)-1-(3-amino-5-(trifluoromethyl)phenyl)ethyl)amino)-2-methylpyrido[3,4-d]pyrimidin-6-yl)cyclohexyl)methanone

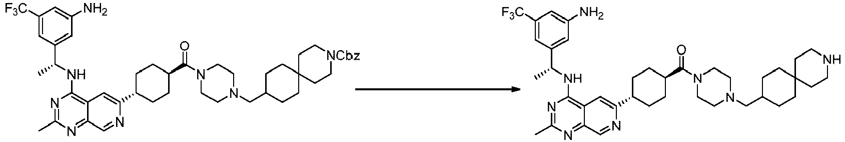

Benzyl 9-((4-((1*R*,4*R*)-4-(4-(((*R*)-1-(3-amino-5-(trifluoromethyl)phenyl)ethyl)amino)-2-methylpyrido[3,4-*d*]pyrimidin-6-yl)cyclohexane-1-carbonyl)piperazin-1-yl)methyl)-3-azaspiro[5.5]undecane-3-carboxylate (220 mg, 0.262 mmol) was dissolved in ethyl acetate (20 mL), and Pd(OH)₂ (44 mg, 20%) was added. The reaction liquid was stirred at 70 °C overnight under H₂ atmosphere, filtered, and concentrated under reduced pressure to give the crude product (4-((3-azaspiro[5.5]undecan-9-yl)methyl)piperazin-1-yl)((1R,4R)-4-(4-(((R)-1-(3-amino-5-(trifluoromethyl)phenyl)ethyl)amino)-2-methylpyrido[3,4-d]pyrimidin-6-yl)cyclohexyl)methanone.

LC-MS: (ESI, m/z): [M+H] ⁺=707.4.

### Step 4: preparation of 1-(5-(9-((4-((1R,4R)-4-(4-(((R)-1-(3-amino-5-(trifluoromethyl)phenyl)ethyl)amino)-2-methylpyrido[3,4-d]pyrimidin-6-yl)cyclohexane-1-carbonyl)piperazin-1-yl)methyl)-3-azaspiro[5.5]undecane-3-carbonyl)-2-chlorophenyl)dihydropyrimidine-2,4(1H,3H)-dione

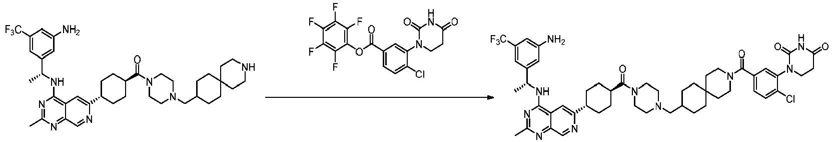

(4-((3-Azaspiro[5.5]undecan-9-yl)methyl)piperazin-1-yl)((1*R*,4*R*)-4-(4-(((*R*)-1-(3-amino-5-(trifluoromethyl)phenyl)ethyl)amino)-2-methylpyrido[3,4-*d*]pyrimidin-6-yl)cyclohexyl)methanone (200 mg, 0.283 mmol) was dissolved with DMSO (20 mL), and perfluorophenyl 4-chloro-3-(2,4-dioxotetrahydropyrimidin-1(2*H*)-yl)benzoate (147 mg, 0.338 mmol), and DIEA (110 mg, 0.853 mmol) were separately added. The reaction liquid was stirred at room temperature overnight, diluted with water (50 mL), and extracted with dichloromethane (50 mL × 3). The organic phase was concentrated under reduced pressure. The crude product was purified by preparative reversed-phase chromatography to give 1-(5-(9-((4-((1*R*,4*R*)-4-(4-(((*R*)-1-(3-amino-5-(trifluoromethyl)phenyl)ethyl)amino)-2-methylpyrido[3,4-*d*]pyrimidin-6-yl)cyclohexane-1-carbonyl)piperazin-1-yl)methyl)-3-azaspiro[5.5]undecane-3-carbonyl)-2-chlorophenyl)dihydropyrimidine-2,4(1*H*,3*H*)-dione.

LC-MS: (ESI, m/z): [M+H] ⁺=957.4.

¹H NMR (400 MHz, DMSO-*d*₆) δ10.51 (s, 1H), 8.91 (s, 1H), 8.58 (d, *J* = 7.9 Hz, 1H), 8.19 (s, 1H), 8.10 (s, 1H), 7.63 (d, *J* = 8.2 Hz, 1H), 7.55 (d, *J* = 1.9 Hz, 1H), 7.39 (dd, *J* = 8.2, 1.9 Hz, 1H), 6.89 (s, 1H), 6.85 (s, 1H), 6.71 (s, 1H), 5.61 - 5.47 (m, 3H), 3.78 - 3.72 (m, 1H), 3.64 - 3.56 (m, 3H), 3.54-3.41 (m, 6H), 3.30-3.24 (m, 2H), 2.82-2.71 (m, 3H), 2.70-2.61 (m, 1H), 2.43 (s, 3H), 2.36-2.21 (m, 4H), 2.17-2.07 (m, 2H), 2.04 - 1.98 (m, 2H), 1.85-1.71 (m, 2H), 1.77-1.31 (m, 18H), 1.16-0.90 (m, 4H).

### Example 79: 1-(2-chloro-5-(4-(3-(1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)propyl)piperidine-1-carbonyl)phenyl)dihydropyrimidine-2,4(1H,3H)-dione

### Step 1: preparation of 1-(2-chloro-5-(4-(3-(piperidin-4-yl)propyl)piperidine-1-carbonyl)phenyl)dihydropyrimidine-2,4(1H,3H)-dione

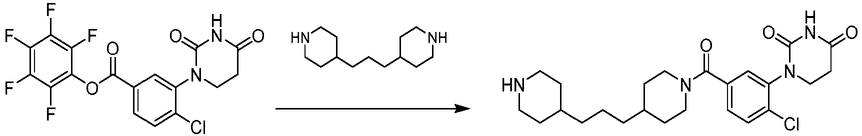

1,3-Di(piperidin-4-yl)propane (193.5 mg, 0.92 mmol) and DIEA (178 mg, 1.38 mmol) were dissolved in DMSO (10 mL), and pentafluorophenyl 4-chloro-3-(2,4-dioxotetrahydropyrimidin-1(2*H*)-yl)benzoate (200 mg, 0.46 mmol) was added. The reaction liquid was reacted at room temperature for 30 min. The reaction liquid was diluted with water (30 mL) and extracted with ethyl acetate (30 mL × 3). The organic phase was washed with saturated brine (40 mL), dried over anhydrous sodium sulfate, and concentrated under reduced pressure. The crude product was purified by column chromatography (MeOH:DCM = 1:9, flow rate: 40 mL/min) to give 1-(2-chloro-5-(4-(3-(piperidin-4-yl)propyl)piperidine-1-carbonyl)phenyl)dihydropyrimidine-2,4(1*H*,3*H*)-dione.

LC-MS: (ESI, m/z): [M+H] ⁺=461.2.

### Step 2: preparation of 1-(2-chloro-5-(4-(3-(1-((1R,4R)-4-(4-(((R)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)propyl)piperidine-1-carbonyl)phenyl)dihydropyrimidine-2,4(1H,3H)-dione

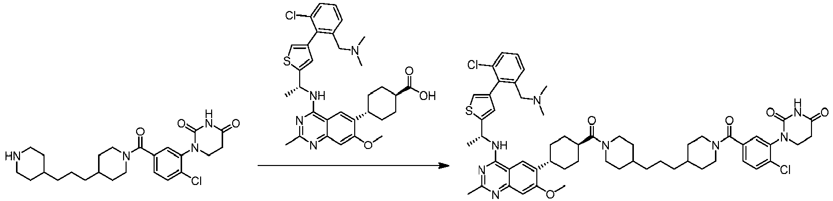

(1*R*,4*R*)-4-(4-(((*R*)-1-(4-(2-Chloro-6-((dimethylamino)methyl)phenyl)thien-2-yl)ethyl)amino)-7-methoxy-2-methylquinazolin-6-yl)cyclohexane-1-carboxylic acid (100 mg, 0.17 mmol), 1-(2-chloro-5-(4-(3-(piperidin-4-yl)propyl)piperidine-1-carbonyl)phenyl)dihydropyrimidine-2,4(1*H*,3*H*)-dione (80 mg, 0.17 mmol), and DIEA (65.8 mg, 0.51 mmol) were dissolved with DMF (5 mL), and HATU (77.5 mg, 0.20 mmol) was finally added. The reaction liquid was stirred at room temperature for 30 min, diluted with water (20 mL), and extracted with ethyl acetate (20 mL × 3). The organic phase was washed with saturated brine (40 mL), dried over anhydrous sodium sulfate, and concentrated under reduced pressure. The crude product was purified by prep-HPLC to give 1-(2-chloro-5-(4-(3-(1-((1*R*,4*R*)-4-(4-(((*R*)-1-(4-(2-chloro-6-((dimethylamino)methyl)phenyl)thien-2-yl)ethyl)amino)-7-methoxy-2-methylquinazolin-6-yl)cyclohexane-1-carbonyl)piperidin-4-yl)propyl)piperidine-1-carbonyl)phenyl)dihydropyrimidine-2,4(1*H*,3*H*)-dione.

LC-MS: (ESI, m/z): [M+H] ⁺=1035.5.

¹H NMR (400 MHz, DMSO-*d*₆) δ 10.51 (s, 1H), 8.31 (d, *J* = 8.1 Hz, 1H), 8.05 (s, 1H), 7.63 (d, *J* = 8.2 Hz, 1H), 7.54 (d, *J* = 1.6 Hz, 1H), 7.43-7.35 (m, 4H), 7.24 (s, 1H), 7.02 (s, 1H), 6.96 (s, 1H), 6.05-5.83 (m, 1H), 4.52-4.35 (m, 2H), 3.95-3.80 (m, 4H), 3.80-3.69 (m, 1H), 3.67-3.50 (m, 2H), 3.20-3.08 (m, 2H), 3.06-2.87 (m, 3H), 2.83 - 2.70 (m, 3H), 2.68-2.57 (m, 1H), 2.42 (s, 3H), 2.01 (s, 6H), 1.92-1.39 (m, 18H), 1.36-1.15 (m, 6H), 1.13-0.82 (m, 4H).

### II. Biological Activity Test Example

### Test Example 1: KRAS-G12C/SOS1 Binding Assay

The following method was used to determine the extent of inhibition of KRAS-G12C/SOS1 binding by the preferred compounds of the present invention under *in vitro* conditions. In this method, the competitive inhibition of KRAS-G12C/SOS1 binding by the preferred compounds was assayed by the homogeneous time-resolved fluorescence (HTRF) technology using the KRAS-G12C/SOS1 binding assay kit (Cisbio, 63ADK000CB16PEG).

The experimental procedures were as follows (refer to the kit instructions for details): the compounds of the present invention were each firstly dissolved in DMSO at a concentration of 20 mM, and then diluted in an equal gradient with a buffer in the kit, so that the final concentrations of the test compounds in the reaction system were in a range of 10000 nM-0.04 nM, and the final concentration of DMSO was 0.5%. 2 µL of the compounds were each incubated with 4 µL of 1× Tag1-SOS1, 4 µL of 1× Tag2-KRAS-G12C (containing 10 µM guanosine triphosphate, GTP) at 25 °C for 15 min. Then 5 µL of 1× Anti-Tag1-Tb3+ and 5 µL of 1× Anti-Tag2-XL665 were added to the reaction system, and the mixture was incubated on ice for 3 h. After the incubation, the fluorescence intensity of each well was measured at the excitation wavelength of 337 nm, the fluorescence intensity of each well was read at the emission wavelengths of 620 nm and 665 nm on a microplate reader EnVision (PerkinElmer, 2105) in an HTRF mode, and the Ratio value was calculated by the formula Ratio = (665 nm/620 nm) × 104. The inhibition rates of the compounds at each concentration were calculated by comparing the fluorescence intensity ratio with that of the control group, and then nonlinear curve fitting was performed by GraphPad Prism 8 in the logarithmic concentration-inhibition rate to obtain the IC₅₀ values of the compounds, as shown in Table 1.

### Test Example 2: SOS1 Degradation Assay

The degradation of SOS1 by the compounds was investigated on KRAS G12C mutant human non-small cell lung cancer cells NCI-H358 (ATCC or Stem Cell Bank, Chinese Academy of Sciences), KRAS G12D mutant human lung cancer cells A-427 (ATCC), KRAS G12V mutant human colorectal adenocarcinoma cells SW-620 (ATCC), and KRAS G13D mutant human colorectal adenocarcinoma epithelial cells DLD-1 (ATCC). The specific procedures were as follows:
0.95 mL of cells were seeded into each well of a 24-well cell culture plate to achieve a cell density of 5×10⁵ cells/well; the cell plate was incubated in an incubator at 37 °C with 5% CO₂ overnight. Then 50 µL of the compound solution after dilution was added to each of the wells with the corresponding cells, so that the final concentration of the compound was in a range of 0.03-3000 nM. The final concentration of DMSO was 0.25%. After dosing, the cell plate was incubated in an incubator at 37 °C with 5% CO₂ for 24 h. The cell culture medium in the 24-well plate was removed, and the plate was washed twice with 1× PBS. 180 µL of RIPA (strong) lysis buffer (Beyotime, P0013B) supplemented with 1 mM phenylmethylsulfonyl fluoride, a protease inhibitor mixture (Beyotime, P1008), and a protease phosphatase inhibitor mixture (Beyotime, P1045) was added to lyse adherent NCI-H358 cells at the bottom of the cell culture plate. After the plate was left to stand on ice for 30 min, the protein lysate in each well was transferred to a 1.5 mL centrifuge tube and centrifuged at 15000 g for 20 min at 4 °C. The cell lysate supernatant after centrifugation was cryopreserved at -80 °C for testing and was used for determining the degradation level of the SOS1 protein by a WB method. The concentration of total protein in the cell lysate supernatant was determined using the BCA Protein Quantification Kit (Tiangen, PA115-02).

### Test Example 3: Western Blot Assay on Human SOS1 Protein

The concentration of total protein in the cell lysate was determined by BCA. The total protein was adjusted to 0.5 µg/µL with PBS and 5× SDS-PAGE protein loading buffer (Beyotime, P0015L), incubated in a water bath at 100 °C for 15 min, incubated in an ice bath on ice for 5 min, centrifuged at 14000 g for 1 min at 4 °C, and mixed well as a loading sample for WB. Protein electrophoresis was performed on a precast gel (KeyGEN, KGMG010W15) with a loading volume of 10 µL (total protein: 5 µg) using a Tris-MOPS-SDS running solution (Adamas, P1598253) at a constant voltage of 120 V for 55 min. After the electrophoresis, the protein on the gel strip was transferred to a PVDF membrane at a constant current of 250 mA for 65 min. After the membrane transfer, the membrane was incubated in 1× QuickBlock blocking buffer (Beyotime, P0235) for 30 min at room temperature. After the blocking, the PVDF membrane was incubated with an SOS1 primary antibody (Abcam, ab140621) at 4 °C overnight, washed with a TBST buffer (2.4 g of Tris, 8.8 g of NaCl, 1.5 mL of Tween 20, with pH adjusted to 7.4, with the volume brought to 1 L) for 30 min (10 min/time), incubated with a secondary antibody (Abcam, ab205718) at room temperature for 2 h, washed with a TBST buffer for 30 min (10 min/time), and finally incubated with Clarity Western ECL Substrate (BIO-RAD, 170-5061) for 5 min for luminescence and color development, and a chemiluminescence imaging system (Clinx, ChemiScope 6200 Touch) was used for color development and protein mapping. The protein map was subjected to gray value analysis by the chemiluminescence imaging system from Clinx. The gray correction value for each sample was calculated by the formula: gray correction value = (gray value of target protein/gray value of corresponding internal reference) × 103. Then the degradation rate was calculated by comparing the gray correction value with the gray correction value of the control group. Then nonlinear curve fitting was performed by GraphPad Prism 8 in the logarithmic concentration-inhibition rate to obtain the DC₅₀ and Dₘₐₓ values of the compounds.

### Test Example 4: 3D Cell Proliferation Inhibition Assay

Inhibition of 3D cell proliferation by the compounds was investigated on KRAS G12C mutant human non-small cell lung cancer cells NCI-H358 (ATCC or Stem Cell Bank, Chinese Academy of Sciences), KRAS G12D mutant human metastatic pancreatic adenocarcinoma cells AsPC-1 (ATCC), KRAS G12V mutant human lung adenocarcinoma cells NCI-H441 (ATCC), and KRAS G13D mutant human colorectal adenocarcinoma epithelial cells DLD-1 (ATCC). The specific procedures were as follows:
200 nL of compound solution diluted in a gradient was added to corresponding microwells of a 384-well low-adsorption microwell plate (Corning, 3657) using an Echo sonic pipetting system (Labcyte, Echo 550), so that the final concentration of the test compound in the reaction system was in a range of 20000 nM-0.008 nM. Then 40 µL of cells at a certain density were added to the corresponding microplate. In addition to test wells for the test compounds, both a DMSO control well and a medium control well were set, with the DMSO control well containing DMSO and cells and the medium well containing only medium. After sample loading, the plate cover was applied, and the 384-well low-adsorption microplate was incubated in an incubator at 37 °C with 5% carbon dioxide for 7 days. After 7 days, the microplate was taken out, and 40 µL of CTG reagent (Promega, G9683) was added to each well. The plate was incubated at room temperature for 30 min and read on a microplate reader EnVision using the chemiluminescence program. The inhibition rates of the compounds at each concentration were calculated by the formula for the percentage inhibition of cell proliferation: inhibition rate % = (mean value of DMSO control group - single concentration reading value of compound)/(mean value of DMSO control group - mean value of medium control group) × 100, and then nonlinear curve fitting was performed by GraphPad Prism 8 in the logarithmic concentration-inhibition rate to obtain the IC₅₀ values of the compounds.

**Table 1**

| Example | KRAS G12C:SOS1 | NCI-H358 (G12C) | | A427 (G12D) | | SW620 (G12V) | | DLD-1 (G13D) | |
|---|---|---|---|---|---|---|---|---|---|
| | IC₅₀ (nM) | DC₅₀ (nM) | Dₘₐₓ (%) | DC₅₀ (nM) | Dₘₐₓ (%) | DC₅₀ (nM) | Dₘₐₓ (%) | DC₅₀ (nM) | Dₘₐₓ (%) |
| Example 1 | B | A | A | A | A | A | A | A | A |
| Example 2 | A | A | A | A | A | A | A | A | A |
| Example 3 | A | A | A | - | - | - | - | - | - |
| Example 4 | - | B | C | - | - | - | - | - | - |
| Example 5 | - | B | A | - | - | - | - | - | - |
| Example 6 | - | E | C | - | - | - | - | - | - |
| Example 7 | - | E | C | - | - | - | - | - | - |
| Example 8 | - | B | A | - | - | - | - | - | - |
| Example 9 | - | E | D | - | - | - | - | - | - |
| Example 10 | - | A | B | A | A | A | A | A | A |
| Example 11 | - | E | C | - | - | - | - | - | - |
| Example 12 | - | E | C | - | - | - | - | - | - |
| Example 13 | - | E | C | - | - | - | - | - | - |
| Example 14 | - | A | B | - | - | - | - | - | - |
| Example 15 | - | E | D | - | - | - | - | - | - |
| Example 16 | - | D | B | - | - | - | - | - | - |
| Example 17 | - | E | C | - | - | - | - | - | - |
| Example 18 | - | E | D | - | - | - | - | - | - |
| Example 19 | - | C | B | A | B | A | A | B | A |
| Example 20 | - | A | A | A | A | A | A | B | A |
| Example 21 | - | E | C | - | - | - | - | - | - |
| Example 22 | - | E | C | - | - | - | - | - | - |
| Example 23 | - | A | B | - | - | - | - | - | - |
| Example 24 | - | A | A | - | - | - | - | - | - |
| Example 25 | - | E | D | - | - | - | - | - | - |
| Example 26 | - | E | D | - | - | - | - | - | - |
| Example 27 | - | E | D | - | - | - | - | - | - |
| Example 28 | A | A | B | - | - | - | - | - | - |
| Example 29 | - | A | A | - | - | - | - | - | - |
| Example 30 | - | E | D | - | - | - | - | - | - |
| Example 31 | - | E | D | - | - | - | - | - | - |
| Example 32 | - | A | A | | | | | | |
| Example 33 | - | A | A | A | A | A | A | C | A |
| Example 34 | - | E | C | - | - | - | - | - | - |
| Example 35 | - | E | C | - | - | - | - | - | - |
| Example 36 | - | C | B | - | - | - | - | - | - |
| Example 37 | - | E | D | - | - | - | - | - | - |
| Example 38 | - | E | D | - | - | - | - | - | - |
| Example 39 | - | A | A | - | - | - | - | - | - |
| Example 40 | - | E | D | - | - | - | - | - | - |
| Example 41 | - | C | A | - | - | - | - | - | - |
| Example 42 | - | E | C | - | - | - | - | - | - |
| Example 43 | A | A | B | - | - | - | - | - | - |
| Example 44 | A | A | - | - | - | - | - | - | - |
| Example 45 | A | C | B | - | - | - | - | - | - |
| Example 46 | A | A | B | - | - | - | - | - | - |
| Example 47 | A | E | D | - | - | - | - | - | - |
| Example 48 | - | A | A | - | - | - | - | - | - |
| Example 49 | A | C | B | - | - | - | - | - | - |
| Example 50 | A | A | A | - | - | - | - | - | - |
| Example 51 | A | A | A | - | - | - | - | - | - |
| Example 52 | A | C | B | - | - | - | - | - | - |
| Example 53 | A | A | B | - | - | - | - | - | - |
| Example 54 | A | A | A | - | - | - | - | - | - |
| Example 55 | A | - | - | - | - | - | - | - | - |
| Example 56 | A | A | A | - | - | - | - | - | - |
| Example 57 | A | A | - | - | - | - | - | - | - |
| Example 58 | A | - | - | - | - | - | - | - | - |
| Example 59 | A | A | A | - | - | - | - | - | - |
| Example 60 | A | A | B | - | - | - | - | - | - |
| Example 61 | A | C | B | - | - | - | - | - | - |
| Example 62 | A | A | A | - | - | - | - | - | - |
| Example 63 | - | - | - | - | - | - | - | - | - |
| Example 64 | A | A | B | - | - | - | - | - | - |
| Example 65 | A | E | D | - | - | - | - | - | - |
| Example 66 | A | - | - | - | - | - | - | - | - |
| Example 67 | A | A | A | - | - | - | - | - | - |
| Example 68 | A | A | B | - | - | - | - | - | - |
| Example 69 | A | A | B | - | - | - | - | - | - |
| Example 70 | A | A | A | - | - | - | - | - | - |
| Example 71 | A | A | A | - | - | - | - | - | - |
| Example 72 | A | A | B | - | - | - | - | - | - |
| Example 73 | - | A | A | A | A | A | A | B | A |
| Example 74 | - | A | A | - | - | - | - | - | - |
| Example 75 | A | A | B | - | - | - | - | - | - |
| Example 76 | - | A | B | - | - | - | - | - | - |
| Example 77 | - | D | A | - | - | - | - | - | - |
| Example 78 | - | C | B | - | - | - | - | - | - |
| Example 79 | A | B | B | - | - | - | - | - | - |

**Table 2**

| Example | IC₅₀ (nM) | | | |
|---|---|---|---|---|
| | NCI-H358 (G12C) | NCI-H441 (G12V) | DLD-1 (G13D) | AsPC-1 (G12D) |
| Example 1 | A | C | C | B |
| Example 2 | A | E | B | E |
| Example 10 | A | A | B | C |
| Example 19 | A | D | E | E |
| Example 20 | A | A | B | D |
| Example 33 | A | E | D | E |
| Example 73 | A | E | D | E |

In Tables 1 and 2:
"-" represents not detected;
when DC₅₀ ≤ 50 nM, the level was A; when 50 nM < DC₅₀ ≤ 100 nM, the level was B; when 100 nM < DC₅₀ < 500 nM, the level was C; when 500 nM < DC₅₀ ≤ 1000 nM, the level was D; when 1000 nM < DC₅₀ ≤ 10 µM, the level was E;
when 80 ≤ Dₘₐₓ (%) ≤ 100, the level was A; when 50 ≤ Dₘₐₓ (%) < 80, the level was B; when 30 ≤ Dₘₐₓ (%) < 50, the level was C; when Dₘₐₓ (%) < 30, the level was D;
when IC₅₀ ≤ 15 nM, the level was A; when 15 nM < IC₅₀ ≤ 30 nM, the level was B; when 30 nM < IC₅₀ ≤ 50 nM, the level was C; when 50 nM < IC₅₀ ≤ 100 nM, the level was D; when 100 nM < IC₅₀ ≤ 1000 nM, the level was E. Experimental conclusion: the compounds of the present invention were able to effectively bind to the SOS1 target protein or have an inhibitory effect, and the compounds described herein were able to effectively and specifically degrade the SOS1 protein.

## Claims

1. A compound of formula I, and/or a stereoisomer, an enantiomer, a diastereomer, a deuteride, a hydrate, a solvate, a prodrug and/or a pharmaceutically acceptable salt thereof:
S-L-E I
wherein:
S is a small molecule compound capable of inhibiting the activity of SOS1 protein or binding to SOS1 protein;
L is a linking chain, which links S and E by means of covalent bonds;
E is a small molecule ligand of an E3 ubiquitin ligase complex.

2. The compound of formula I, and/or the stereoisomer, the enantiomer, the diastereomer, the deuteride, the hydrate, the solvate, the prodrug and/or the pharmaceutically acceptable salt thereof according to claim 1, wherein S is S1, S1', -X-S1', S1", -X-S1", S2, S2', -X-S2', S2", -X-S2", S3, S4, S5, S5' or -X-S2"; X is O, NH or S, preferably O; 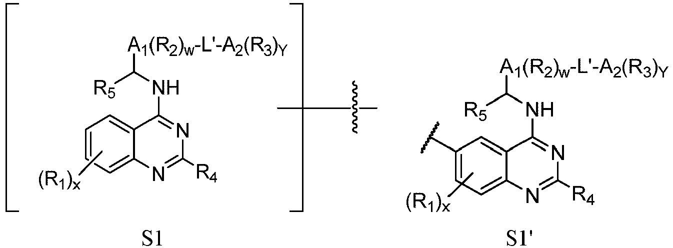 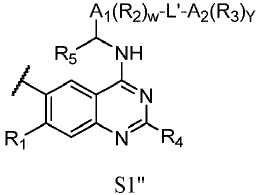 wherein in S1, S1' or S1":
R₁ is selected from hydrogen, halogen, -OH, -CN, -NO₂, C₁-C₆ alkyl sulfhydryl and -NRₐR_{b}, wherein Rₐ and R_{b} are each independently hydrogen, C₁-C₆ alkyl, C₃-C₈ heterocycloalkyl or C₃-C₈ cycloalkyl;
R₁ is selected from C₁-C₆ alkyl, C₁-C₆ alkoxy, C₃-C₆ cycloalkoxy, C₃-C₆ heterocycloalkoxy, C₂-C₆ alkenyl, C₂-C₆ alkynyl, C₃-C₈ cycloalkyl, C₄-C₈ cycloalkenyl, 4- to 7-membered heterocycloalkyl, 5- to 10-membered heterocycloalkenyl, spiroheterocycloalkyl, fused heterocycloalkyl, bridged heterocycloalkyl, phenyl, heteroaryl,
C₁-C₆ haloalkyl, -COOH and -COOR_{c}; R_{c} is -C₁-C₆ alkyl, C₃-C₆ alkenyl, C₃-C₆ alkynyl, C₃-C₈ cycloalkyl or -C₄-C₈ cycloalkenyl;
R₁ is -NS(O)(R_{d})(Rₑ), wherein C₁-C₆ and Rₑ are each independently C₁-C₆ alkyl, C₂-C₆ alkenyl, C₂-C₆ alkynyl, C₃-C₈ cycloalkyl, C₄-C₈ cycloalkenyl, 6- to 10-membered aryl or 5- to 10-membered heteroaryl;
R₁ is -NHC(O)-(C₁-C₆ alkyl) or -NHC(O)-NRₐR_{b}, wherein Rₐ and R_{b} are each independently hydrogen, -C₁-C₆ alkyl, C₃-C₈ heterocycloalkyl or C₃-C₈ cycloalkyl;
R₁ is -NH-(CH₂)ₖ-NH-C(O)-Rₐₐ, wherein Rₐₐ is C₁-C₆ alkyl, C₃-C₈ heterocycloalkyl or C₃-C₈ cycloalkyl, wherein k is 1 or 2;
R₁ is -NH-(CH₂)ᵢ-R_{f}, wherein i is 0, 1 or 2 and R_{f} is 4- to 7-membered heterocycloalkyl, heteroaryl or C₁-C₆ alkylsulfonyl;
furthermore, C₁-C₆ alkyl, C₁-C₆ alkoxy, 4- to 7-membered heterocycloalkyl and heteroaryl described above are optionally substituted with 1, 2 or 3 groups selected from halogen, -OH, oxo, -CN, -NO₂, C₁-C₆ alkyl, C₂-C₆ alkenyl, C₂-C₆ alkynyl, C₃-C₈ cycloalkyl, 4- to 7-membered heterocycloalkyl, C₁-C₆ alkoxy, C₁-C₆ haloalkyl, C₁-C₆ haloalkoxy, C₁-C₆ alkylsulfonyl, phenyl, benzyl, heteroaryl, -(CH₂)-heteroaryl, -NHC(O)(C₁-C₆ alkyl), C₃-C₈ cycloalkoxy, phenoxy, heteroaryloxy and -NRₐR_{b}; Rₐ and R_{b} are each independently selected from hydrogen, C₁-C₆ alkyl, C₃-C₈ heterocycloalkyl and C₃-C₈ cycloalkyl;
R₁ is -O-(CH₂)_{z}-phenyl, -O-(CH₂)_{z}-(4- to 7-membered heterocycloalkyl) or -O-(CH₂)_{z}-heteroaryl, wherein z is 0, 1 or 2, and the phenyl, the heterocycloalkyl and the heteroaryl are optionally substituted with a group selected from - OH, heterocycloalkyl and heterocycloalkenyl and can be substituted with methyl or oxo;
or R₁ is 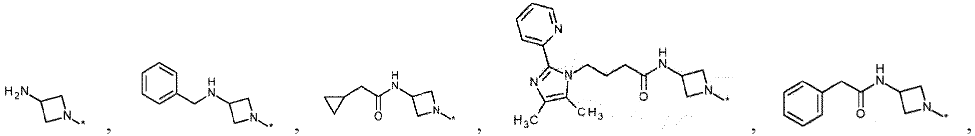 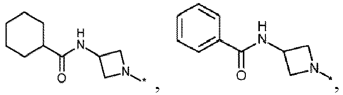 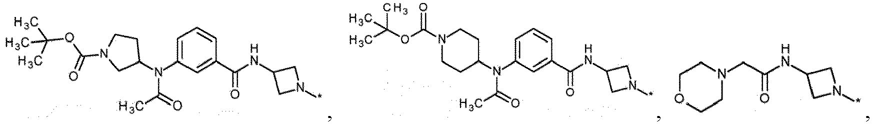 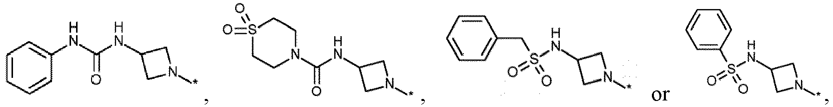
or 2 adjacent R₁, together with the carbon atom linked thereto, form 5- to 7-membered cycloalkyl or 5- to 7-membered heterocycloalkyl;
and x is 1, 2 or 3;
A₁ is C₄-C₁₂ cycloalkyl, heterocycloalkyl, aryl or heteroaryl;
R₂ is hydrogen, -OH, oxo, halogen, -CN, C₁-C₆ alkyl, C₁-C₆ alkoxy, C₂-C₆ alkenyl, -C₂-C₆ alkynyl, -C₃-C₈ cycloalkyl, -C₃-C₈ cycloalkoxy, -C₃-C₈ halocycloalkoxy, -C₄-C₈ cycloalkenyl, 4- to 7-membered heterocycloalkyl, -O-CH₂-4- to 7-membered heterocycloalkyl, 5- to 10-membered heterocycloalkenyl, spiroheterocycloalkyl, fused heterocycloalkyl, bridged heterocycloalkyl, phenyl, heteroaryl, -C₁-C₆ haloalkyl, -C₁-C₆ haloalkoxy or -C₁-C₆ alkylsulfonyl;
R₂ is -NRₐR_{b}, wherein Rₐ and R_{b} are each independently hydrogen, C₁-C₆ alkyl or -C(O)C₁-C₃ alkyl;
R₂ is -C(O)NRₐR_{b}, wherein Rₐ and R_{b} are each independently hydrogen, C₁-C₆ alkyl, C₃-C₈ heterocycloalkyl or C₃-C₈ cycloalkyl;
R₂ is -C(O)OR_{g}, wherein R_{g} is hydrogen or C₁-C₆ alkyl;
R₂ is -ORₕ; Rₕ is C₁-C₆ alkyl;
R₂ is -(CH₂)NRₐR_{b}, wherein Rₐ and R_{b} are each independently hydrogen, C₁-C₆ alkyl, C₃-C₈ heterocycloalkyl or C₃-C₈ cycloalkyl;
and w is 1, 2, 3 or 4;
A₂(R₃)_{Y} is hydrogen;
or A₂ is C₄-C₁₂ cycloalkyl, heterocycloalkyl, aryl or heteroaryl, and
R₃ is hydrogen, halogen, -OH, oxo, -CN, -NO₂, C₁-C₆ alkyl, C₂-C₆ alkenyl, C₂-C₆ alkynyl, C₃-C₈ cycloalkyl, C₄-C₈ cycloalkenyl, C₇-C₈ cycloalkynyl, 4- to 7-membered heterocycloalkyl, 5- to 10-membered heterocycloalkenyl, phenyl, heteroaryl or C₁-C₆ haloalkyl;
the alkyl, the alkenyl, the alkynyl, the cycloalkyl, the heterocycloalkyl, the aryl, the heteroaryl, the cycloalkenyl, the cycloalkynyl, and the heterocycloalkenyl are optionally substituted with 1, 2 or 3 substituents selected from halogen, -OH, oxo, -CN, -C₁-C₆ alkyl, -C₁-C₆ alkoxy, -C₁-C₆ haloalkyl, phenyl, heteroaryl and -C(O)NRᵢRⱼ, wherein
Rᵢ and Rⱼ are each independently hydrogen or C₁-C₆ alkyl;
or the alkyl, the alkenyl, the alkynyl, the cycloalkyl, the heterocycloalkyl, the aryl, the heteroaryl, the cycloalkenyl, the cycloalkynyl and the heterocycloalkenyl are optionally substituted with -NRₖRₗ, Rₖ and Rₗ are each independently hydrogen, C₁-C₆ alkyl, C₂-C₆ alkenyl, C₂-C₆ alkynyl, C₃-C₈ cycloalkyl, C₁-C₆ alkylsulfonyl, phenyl, heteroaryl, -CH₂-C(O)-Rₘ, -C(O)Rₚ or 4- to 7-membered heterocycloalkyl, and the alkyl, the alkynyl, the alkenyl, the cycloalkyl, the phenyl, the heteroaryl and the heterocycloalkyl are each independently optionally substituted with 1, 2 or 3 substituents selected from C₁-C₆ haloalkyl, -OH, oxo, phenyl, -CN, C₁-C₆ alkoxy and heteroaryl, and the heteroaryl is optionally substituted with methyl; and Rₘ is bicyclic heteroaryl, C₁-C₆ alkoxy or -NRₙRₒ, Rₙ and Rₒ are each independently hydrogen, C₁-C₆ alkyl or phenyl, the alkyl is optionally substituted with C₁-C₆ alkoxy or phenyl, or -NRₙRₒ is a 4- to 7-membered azacycloalkyl, and the azacycloalkyl is linked to the rest of the molecule through an N atom and further contains 1 or more heteroatoms selected from N and O; Rₚ is selected from C₁-C₆ alkoxy, C₁-C₆ alkyl optionally substituted with 1, 2 or 3 groups selected from -OH and C₁-C₆ alkoxy, monocyclic or bicyclic heteroaryl and 4- to 7-membered heterocycloalkyl, or Rₚ is -CH₂-NR_{q}Rᵣ, and R_{q} and Rᵣ are each independently selected from hydrogen, phenyl and C₁-C₆ alkyl optionally substituted with F;
or the alkyl, the alkenyl, the alkynyl, the cycloalkyl, the heterocycloalkyl, the aryl, the heteroaryl, the cycloalkenyl, the cycloalkynyl and the heterocycloalkenyl are optionally substituted with -NRₛRₜ, wherein -NRₛRₜ is 4- to 7-membered azacycloalkyl linked to the rest of the molecule through a nitrogen atom or 6- to 10-membered spiroazacycloalkyl linked to the rest of the molecule through a nitrogen atom and further contains up to two heteroatoms selected from N and O and is optionally substituted with 1, 2 or 3 substituents selected from -OH, oxo, C₁-C₆ alkyl, C₁-C₆ hydroxyalkyl and -C(O)OR_{z}, R_{z} is C₁-C₆ alkyl, halogen, -N(C₁-C₆ alkyl)₂, -CH₂N(C₁-C₆ alkyl)₂ or -C(O)NRₐR_{b}, and Rₐ and R_{b} are each independently hydrogen, C₁-C₆ alkyl, C₃-C₈ heterocycloalkyl or C₃-C₈ cycloalkyl;
or R₃ is C(O)Rᵥ, -C(O)NH₂, -C(O)NHRᵥ, -C(O)NRᵥR_{w}, or -C(O)ORᵥ, wherein Rᵥ and R_{w} are each independently hydrogen, C₁-C₄ alkyl, C₁-C₄ haloalkyl, phenyl or -(CH₂)₂NRₓR_{y}, wherein Rₓ and R_{y} are each independently hydrogen, C₁-C₄ alkyl or -(CH₂)₂N(CH₃)₂;
or R₃ is -NH₂, -NHR_{z}, -NR_{z}R_{za}, -NHC(O)R_{z}, -NHC(O)OR_{z}, -NHS(O)₂R_{z}, 4- to 7-membered heterocycloalkyl, heteroaryl, spiroheterocycloalkyl, fused heterocycloalkyl or bridged heterocycloalkyl, and R_{z} and R_{za} are each independently C₁-C₄ alkyl, C₁-C₄ haloalkyl or phenyl;
or R₃ is C₁-C₆ alkoxy, C₁-C₆ haloalkoxy, -O(CH₂)ₛ-C₃-C₈ cycloalkyl, -O(CH₂)ₛ-phenyl, -O(CH₂)ₛ-heterocycloalkyl, or -O(CH₂)ₛ-heteroaryl, wherein s is 0, 1, 2 or 3;
or R₃ is -S(O)₂R_{z}, -S(O)₂NH₂, -S(O)₂NHR_{z}, or -S(O)₂NR_{z}R_{za}, wherein R_{z} and R_{za} are each independently C₁-C₄ alkyl, C₁-C₄ haloalkyl or phenyl, and y is 0, 1, 2 or 3;
Yis 0, 1, 2, 3, 4 or 5;
L' is a bond, -(CH₂)ₖ-, -O(CH₂)ₖ-, -(CH₂)ₖ-O- or -O-(CH₂)ₖ-O-, wherein k is 0, 1, 2 or 3, or L' is -CH=CH-(CH₂)ₙ-, wherein n is 0, 1 or 2;
R₄ and R₅ are each independently C₁-C₆ alkyl, C₁-C₆ alkoxy, C₃-C₆ cycloalkyl or C₃-C₆ cycloalkoxy, wherein the alkyl, the alkoxy, the cycloalkyl and the cycloalkoxy are optionally substituted with cyano, hydroxy, C₁-C₆ alkoxy, C₃-C₆ cycloalkyl, C₃-C₆ cycloalkoxy or halogen; 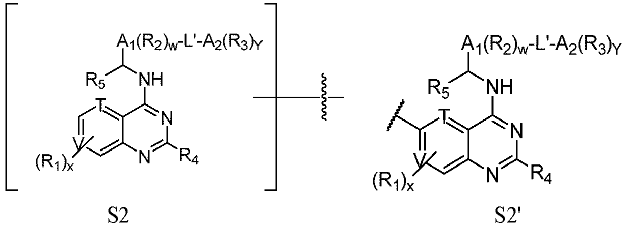 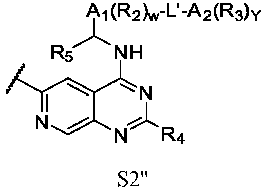 wherein in S2, S2' or S2":
R₁ is hydrogen, halogen, -OH, -CN, -NO₂, C₁-C₆ alkyl sulfhydryl or -NRₐR_{b}, wherein Rₐ and R_{b} are each independently hydrogen, C₁-C₆ alkyl, C₃-C₈ heterocycloalkyl or C₃-C₈ cycloalkyl;
R₁ is selected from C₁-C₆ alkyl, C₁-C₆ alkoxy, C₃-C₆ cycloalkoxy, C₃-C₆ heterocycloalkoxy, C₂-C₆ alkenyl, C₂-C₆ alkynyl, C₃-C₈ cycloalkyl, C₄-C₈ cycloalkenyl, 4- to 7-membered heterocycloalkyl, 5- to 10-membered heterocycloalkenyl, spiroheterocycloalkyl, fused heterocycloalkyl, bridged heterocycloalkyl, phenyl, heteroaryl, C₁-C₆ haloalkyl, -COOH and -COOR_{c}; R_{c} is -C₁-C₆ alkyl, C₃-C₆ alkenyl, C₃-C₆ alkynyl, C₃-C₈ cycloalkyl or -C₄-C₈ cycloalkenyl;
R₁ is -NS(O)(R_{d})(Rₑ), wherein R_{d} and Rₑ are each independently C₁-C₆ alkyl, C₂-C₆ alkenyl, C₂-C₆ alkynyl, C₃-C₈ cycloalkyl or C₄-C₈ cycloalkenyl;
R₁ is -NHC(O)-(C₁-C₆ alkyl) or -NHC(O)-NRₐR_{b}, wherein Rₐ and R_{b} are each independently hydrogen, -C₁-C₆ alkyl, C₃-C₈ heterocycloalkyl or C₃-C₈ cycloalkyl;
R₁ is -NH-(CH₂)ₖ-NH-C(O)-(C₁-C₆ alkyl), wherein k is 1 or 2;
R₁ is -NH-(CH₂)ᵢ-R_{f}, wherein i is 0, 1 or 2 and R_{f} is 4- to 7-membered heterocycloalkyl, heteroaryl or C₁-C₆ alkylsulfonyl;
furthermore, C₁-C₆ alkyl, C₁-C₆ alkoxy, 4- to 7-membered heterocycloalkyl and heteroaryl described above are optionally substituted with 1, 2 or 3 groups selected from halogen, -OH, oxo, -CN, -NO₂, C₁-C₆ alkyl, C₂-C₆ alkenyl, C₂-C₆ alkynyl, C₃-C₈ cycloalkyl, 4- to 7-membered heterocycloalkyl, C₁-C₆ alkoxy, C₁-C₆ haloalkyl, C₁-C₆ haloalkoxy, C₁-C₆ alkylsulfonyl, phenyl, benzyl, heteroaryl, -(CH₂)-heteroaryl, -NHC(O)(C₁-C₆ alkyl), C₃-C₈ cycloalkoxy, phenoxy, heteroaryloxy and -NRₐR_{b}; Rₐ and R_{b} are each independently selected from hydrogen, C₁-C₆ alkyl, C₃-C₈ heterocycloalkyl and C₃-C₈ cycloalkyl;
R₁ is -O-(CH₂)_{z}-phenyl, -O-(CH₂)_{z}-(4- to 7-membered heterocycloalkyl) or -O-(CH₂)_{z}-heteroaryl, wherein z is 0, 1 or 2, and the phenyl, the heterocycloalkyl and the heteroaryl are optionally substituted with a group selected from - OH, heterocycloalkyl and heterocycloalkenyl and can be substituted with methyl or oxo;
or R₁ is 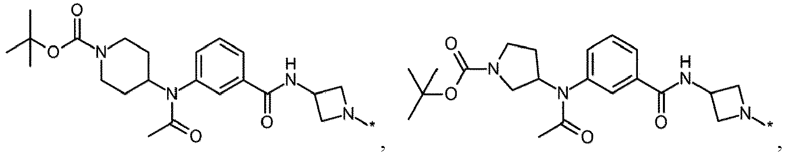 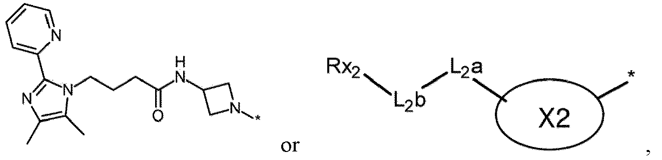 , L₂a is C(O), L₂b is a bond or C₁-C₆ alkylene, X₂ is 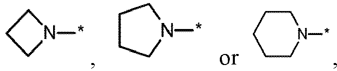 Rx₂ is 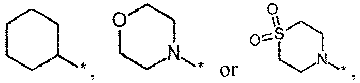
or R₁ is 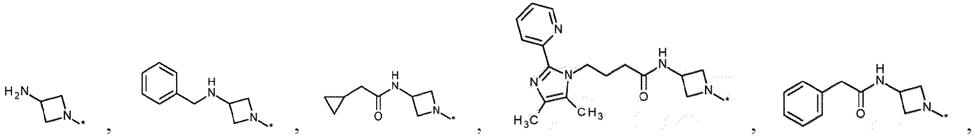 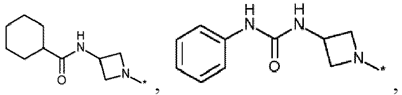 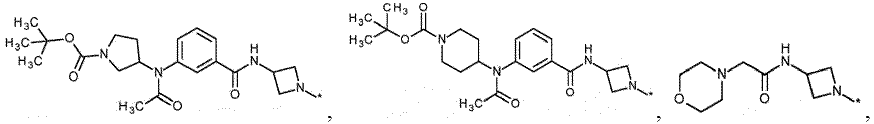 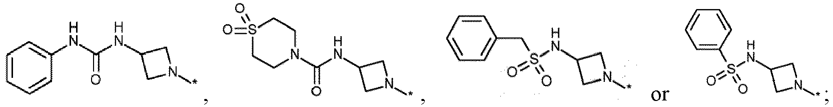
or 2 adjacent R₁, together with the carbon atom linked thereto, form 5- to 7-membered cycloalkyl or 5- to 7-membered heterocycloalkyl;
and x is 1, 2, 3 or 4;
A₁ is C₄-C₁₂ cycloalkyl, heterocycloalkyl, aryl or heteroaryl;
R₂ is hydrogen, -OH, oxo, halogen, -CN, C₁-C₆ alkyl, C₁-C₆ alkoxy, C₂-C₆ alkenyl, -C₂-C₆ alkynyl, -C₃-C₈ cycloalkyl, -C₃-C₈ cycloalkoxy, -C₃-C₈ halocycloalkoxy, -C₄-C₈ cycloalkenyl, 4- to 7-membered heterocycloalkyl, -O-CH₂-4- to 7-membered heterocycloalkyl, 5- to 10-membered heterocycloalkenyl, spiroheterocycloalkyl, fused heterocycloalkyl, bridged heterocycloalkyl, phenyl, heteroaryl, -C₁-C₆ haloalkyl, -C₁-C₆ haloalkoxy or -C₁-C₆ alkylsulfonyl;
R₂ is -NRₐR_{b}, wherein Rₐ and R_{b} are each independently hydrogen, C₁-C₆ alkyl, C₃-C₈ heterocycloalkyl or C₃-C₈ cycloalkyl;
R₂ is -C(O)NRₐR_{b}, wherein Rₐ and R_{b} are each independently hydrogen, C₁-C₆ alkyl, C₃-C₈ heterocycloalkyl or C₃-C₈ cycloalkyl;
R₂ is -C(O)OR_{g}, wherein R_{g} is hydrogen or C₁-C₆ alkyl;
R₂ is -ORₕ; Rₕ is C₁-C₆ alkyl;
R₂ is -(CH₂)NRₐR_{b}, wherein Rₐ and R_{b} are each independently hydrogen, C₁-C₆ alkyl, C₃-C₈ heterocycloalkyl or C₃-C₈ cycloalkyl;
and w is 1, 2, 3 or 4;
A₂(R₃)_{Y} is hydrogen;
or A₂ is C₄-C₁₂ cycloalkyl, heterocycloalkyl, aryl or heteroaryl, and
R₃ is hydrogen, halogen, -OH, oxo, -CN, -NO₂, C₁-C₆ alkyl, C₂-C₆ alkenyl, C₂-C₆ alkynyl, C₃-C₈ cycloalkyl, C₄-C₈ cycloalkenyl, C₂-C₈ cycloalkynyl, 4- to 7-membered heterocycloalkyl, 5- to 10-membered heterocycloalkenyl, phenyl, heteroaryl or C₁-C₆ haloalkyl;
the alkyl, the alkenyl, the alkynyl, the cycloalkyl, the heterocycloalkyl, the aryl, the heteroaryl, the cycloalkenyl, the cycloalkynyl, and the heterocycloalkenyl are optionally substituted with 1, 2 or 3 substituents selected from halogen, -OH, oxo, -CN, -C₁-C₆ alkyl, -C₁-C₆ alkoxy, -C₁-C₆ haloalkyl, phenyl, heteroaryl and -C(O)NRᵢRⱼ, wherein Rᵢ and Rⱼ are each independently hydrogen or C₁-C₆ alkyl;
or the alkyl, the alkenyl, the alkynyl, the cycloalkyl, the heterocycloalkyl, the aryl, the heteroaryl, the cycloalkenyl, the cycloalkynyl and the heterocycloalkenyl are optionally substituted with -NRₖRₗ, Rₖ and Rₗ are each independently hydrogen, C₁-C₆ alkyl, C₂-C₆ alkenyl, C₂-C₆ alkynyl, C₃-C₈ cycloalkyl, C₁-C₆ alkylsulfonyl, phenyl, heteroaryl, -CH₂-C(O)-Rₘ, -C(O)Rₚ or 4- to 7-membered heterocycloalkyl, and the alkyl, the alkynyl, the alkenyl, the cycloalkyl, the phenyl, the heteroaryl and the heterocycloalkyl are each independently optionally substituted with 1, 2 or 3 substituents selected from C₁-C₆ haloalkyl, -OH, oxo, phenyl, -CN, C₁-C₆ alkoxy and heteroaryl, and the heteroaryl is optionally substituted with methyl; and Rₘ is bicyclic heteroaryl, C₁-C₆ alkoxy or -NRₙRₒ, Rₙ and Rₒ are each independently hydrogen, C₁-C₆ alkyl or phenyl, the alkyl is optionally substituted with C₁-C₆ alkoxy or phenyl, or -NRₙRₒ is a 4- to 7-membered azacycloalkyl, and the azacycloalkyl is linked to the rest of the molecule through an N atom and further contains 1 or more heteroatoms selected from N and O; Rₚ is selected from C₁-C₆ alkoxy, C₁-C₆ alkyl optionally substituted with 1, 2 or 3 groups selected from -OH and C₁-C₆ alkoxy, monocyclic or bicyclic heteroaryl and 4- to 7-membered heterocycloalkyl, or Rₚ is -CH₂-NR_{q}Rᵣ, and R_{q} and Rᵣ are each independently selected from hydrogen, phenyl and C₁-C₆ alkyl optionally substituted with F;
or the alkyl, the alkenyl, the alkynyl, the cycloalkyl, the heterocycloalkyl, the aryl, the heteroaryl, the cycloalkenyl, the cycloalkynyl and the heterocycloalkenyl are optionally substituted with -NRₛRₜ, wherein -NRₛRₜ is 4- to 7-membered azacycloalkyl linked to the rest of the molecule through a nitrogen atom or 6- to 10-membered spiroazacycloalkyl linked to the rest of the molecule through a nitrogen atom and further contains up to two heteroatoms selected from N and O and is optionally substituted with 1, 2 or 3 substituents selected from -OH, oxo, C₁-C₆ alkyl, C₁-C₆ hydroxyalkyl and -C(O)OR_{z}, R_{z} is C₁-C₆ alkyl, halogen, -N(C₁-C₆ alkyl)₂, -CH₂N(C₁-C₆ alkyl)₂ or -C(O)NRₐR_{b}, and Rₐ and R_{b} are each independently hydrogen, C₁-C₆ alkyl, C₃-C₈ heterocycloalkyl or C₃-C₈ cycloalkyl;
or R₃ is C(O)Rᵥ, -C(O)NH₂, -C(O)NHRᵥ, -C(O)NRᵥR_{w}, or -C(O)ORᵥ, wherein Rᵥ and R_{w} are each independently hydrogen, C₁-C₄ alkyl, C₁-C₄ haloalkyl, phenyl or -(CH₂)₂NRₓR_{y}, wherein Rₓ and R_{y} are each independently hydrogen, C₁-C₄ alkyl or -(CH₂)₂NH(CH₃)₂;
or R₃ is -NH₂, -NHR_{z}, -NR_{z}R_{za}, -NHC(O)R_{z}, -NHC(O)OR_{z}, -NHS(O)₂R_{z}, 4- to 7-membered heterocycloalkyl, heteroaryl, spiroheterocycloalkyl, fused heterocycloalkyl or bridged heterocycloalkyl, and R_{z} and R_{za} are each independently C₁-C₄ alkyl, C₁-C₄ haloalkyl or phenyl;
or R₃ is C₁-C₆ alkoxy, C₁-C₆ haloalkoxy, -O(CH₂)ₛ-C₃-C₈ cycloalkyl, -O(CH₂)ₛ-phenyl, -O(CH₂)ₛ-heterocycloalkyl, or -O(CH₂)ₛ-heteroaryl, wherein s is 0, 1, 2 or 3;
or R₃ is -S(O)₂R_{z}, -S(O)₂NH₂, -S(O)₂NHR_{z}, or -S(O)₂NR_{z}R_{za}, wherein R_{z} and R_{za} are each independently C₁-C₄ alkyl, C₁-C₄ haloalkyl or phenyl;
Yis 0, 1, 2, 3, 4 or 5;
L' is a bond, -(CH₂)ₖ-, -O(CH₂)ₖ-, -(CH₂)ₖ-O- or -O-(CH₂)ₖ-O-, wherein k is 0, 1, 2 or 3, or L' is -CH=CH-(CH₂)ₙ-, wherein n is 0, 1 or 2;
R₄ and R₅ are each independently C₁-C₆ alkyl, C₁-C₆ alkoxy, C₃-C₆ cycloalkyl or C₃-C₆ cycloalkoxy, wherein the alkyl, the alkoxy, the cycloalkyl and the cycloalkoxy are optionally substituted with cyano, hydroxy, C₁-C₆ alkoxy, C₃-C₆ cycloalkyl, C₃-C₆ cycloalkoxy or halogen;
T and V are both N, or T is C and V is N, or T is N and V is C; 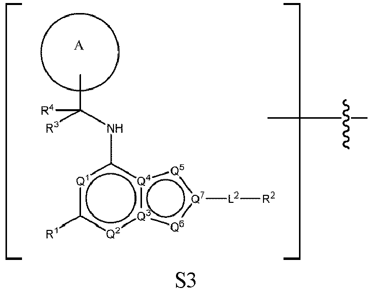
wherein in S3,
Q¹ and Q² are each independently CH or N;
Q³, Q⁴ and Q⁷ are each independently C or N, at least one of Q³ and Q⁴ is C, and not all of Q³, Q⁴ and Q⁷ are N; Q⁵ is CH, N, NH, O or S;
Q⁶ is CH, N, NH, N(C₁-C₆ alkyl), N(C₁-C₆ heteroalkyl), N(3- to 7-membered cycloalkyl), N(3- to 7-membered heterocycle), O or S;
at least one of Q¹, Q², Q³, Q⁴, Q⁵, Q⁶ and Q⁷ is N, NH, O or S;
R¹ is selected from hydrogen, C₁-C₆ alkyl, C₁-C₆ alkoxy, halogen, -NHR₁ₐ, -OR₁ₐ, C₃-C₆ cycloalkyl, C₃-C₆ cycloalkoxy and -CN; the C₁-C₆ alkyl, the C₁-C₆ alkoxy, the C₃-C₆ cycloalkyl and the C₃-C₆ cycloalkoxy are optionally substituted with a substituent selected from halogen, -NHR^{1a} and -OR^{1a}; R^{1a} is hydrogen, C₁-C₆ alkyl, 3-to 6-membered heterocycle or C₁-C₆ haloalkyl;
L² is selected from a bond, -C(O)-, -C(O)O-, -C(O)NH(CH₂)ₒ-, -S(O)₂-, -S(O)-, -S(=O)(=NH)-, -S(=O)[=N(C₁-C₆ alkyl)]-, -N(C₁-C₆ alkyl)-, -C(O)(CH₂)ₚ-, -(CH₂)ₚ- and O; o is 0, 1 or 2; p is an integer of 1 to 6;
R² is selected from hydrogen, halogen, -CN, -NO₂, C₁-C₆ alkyl sulfhydryl, C₂-C₆ alkynyl, C₁-C₆ alkyl, C₂-C₆ alkenyl, -NR^{2b}R^{2c}, -OR^{2a}, 3- to 14-membered cycloalkyl, 3- to 14-membered cycloalkenyl, 3- to 14-membered heterocycloalkyl, 6- to 10-membered aryl and 5- to 10-membered heteroaryl; the C₁-C₆ alkyl, the C₂-C₆ alkenyl, the 3- to 14-membered cycloalkyl, the 3- to 14-membered cycloalkenyl, the 3- to 14-membered heterocycloalkyl, the 6- to 10-membered aryl, and the 5- to 10-membered heteroaryl are each independently optionally substituted with a substituent selected from C₁-C₆ alkyl, C₁-C₆ haloalkyl, -OH, -OR^{2a}, oxo, halogen, -C(O)R^{2a}, -C(O)OR^{2a}, - C(O)NR^{2b}R^{2c}, -CN, -NR^{2b}R^{2c}, 3- to 6-membered cycloalkyl, 3- to 7-membered heterocycloalkyl, 6- to 10-membered aryl and 5- to 10-membered heteroaryl; R^{2a} is hydrogen, C₁-C₆ alkyl, C₁-C₆ haloalkyl, 3- to 7-membered heterocycloalkyl, or -(CH₂)ᵣOCH₃, and r is 1, 2 or 3;
R^{2b} is hydrogen or C₁-C₆ alkyl;
R^{2c} is hydrogen or C₁-C₆ alkyl;
R² is -NHC(O)-(C₁-C₆ alkyl) or -NHC(O)-NRₐR_{b}, wherein Rₐ and R_{b} are each independently hydrogen, C₁-C₆ alkyl, C₃-C₈ heterocycloalkyl or C₃-C₈ cycloalkyl;
R² is -NH-(CH₂)ₖ-NH-C(0)-(C₁-C₆ alkyl), wherein k is 1 or 2;
R² is -NH-(CH₂)ᵢ-R_{f}, wherein i is 0, 1 or 2 and R_{f} is 4- to 7-membered heterocycloalkyl, heteroaryl or C₁-C₆ alkylsulfonyl;
R³ and R⁴ are each independently hydrogen, C₁-C₆ alkyl, C₁-C₆ alkoxy or C₃-C₆ cycloalkoxy; at least one of R³ and R⁴ is not hydrogen, or R³ and R⁴, together with the atom linked thereto, form 3- to 6-membered cycloalkyl; the alkyl, the alkoxy, the cycloalkyl and the cycloalkoxy are optionally substituted with cyano, hydroxy, C₁-C₆ alkoxy, C₃-C₆ cycloalkyl, C₃-C₆ cycloalkoxy or halogen;
A is optionally substituted 6-membered aryl or optionally substituted 5- to 6-membered heteroaryl; 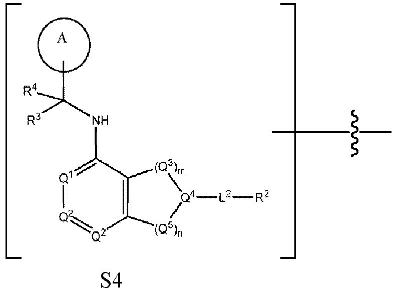
wherein in S4,
Q¹ is CH or N;
Q⁴ is CH, C or N;
each Q² is independently C-R¹ or N, and one of Q² is N and the other Q² is C-R¹;
each Q³ and Q⁵ molecule is independently C(R^{QC})₂, NR^{QN}, C(O), O, S or SO₂, R^{QC} is each independently hydrogen, F, Cl, Br or 6- to 10-membered aryl, and R^{QN} is each independently hydrogen, C₁-C₆ alkyl or 6- to 10-membered aryl;
at least one of Q¹, Q², Q³, Q⁴ and Q⁵ is N, NR^{QN}, O or SO₂;
m is 0, 1, 2 or 3;
n is 0, 1, 2 or 3;
and when m is 0, n is not 0;
R¹ is selected from hydrogen, C₁-C₆ alkyl, halogen, -C(O)NHR^{1a}, -NHR^{1a}, -OR^{1a}, cyclopropyl, azetidine and -CN; the C₁-C₆ alkyl and the azetidine are optionally substituted with a substituent selected from halogen, R^{1a}, -NHR^{1a} and -OR^{1a}; R^{1a} is hydrogen, C₁-C₆ alkyl, cyclopropyl, 3- to 6-membered heterocycle or C₁-C₆ haloalkyl;
L² is selected from a bond, -C(O)-, -C(O)O-, -C(O)NH(CH₂)ₒ-, -S(O)₂-, -S(O)-, -S(=O)(=NH)-, -S(=O)[=N(C₁-C₆ alkyl)]-, -N(C₁-C₆ alkyl)-, -C(O)(CH₂)ₚ-, -(CH₂)ₚ- and O; o is 0, 1 or 2; p is an integer of 1 to 6;
R² is selected from hydrogen, C₁-C₆ alkyl, -NR^{2b}R^{2c}, -OR^{2a}, 3- to 14-membered cycloalkyl, 3- to 14-membered cycloalkenyl, 3- to 14-membered heterocycloalkyl, 6- to 10-membered aryl and 5- to 10-membered heteroaryl; the C₁-C₆ alkyl, the 3- to 14-membered cycloalkyl, the 3- to 14-membered cycloalkenyl, the 3- to 14-membered heterocycloalkyl, the 6- to 10-membered aryl and the 5- to 10-membered heteroaryl are each independently optionally substituted with a substituent selected from C₁-C₆ alkyl, C₁-C₆ haloalkyl, C₁-C₆ hydroxyalkyl, C₁-C₆ methoxyalkyl, -OH, -OR^{2a}, oxo, halogen, =N, -C(O)R^{2a}, -C(O)OR^{2a}, -C(O)NR^{2b}R^{2c}, -S(O)₂R^{2a}, -CN, -NR^{2b}R^{2c}, 3-to 6-membered cycloalkyl, 3- to 7-membered heterocycloalkyl, 6- to 10-membered aryl and 5- to 10-membered heteroaryl; R^{2a} is hydrogen, C₁-C₆ alkyl, C₁-C₆ haloalkyl, 3- to 7-membered heterocycloalkyl, or -(CH₂)ᵣOCH₃, and R is 1, 2 or 3;
R^{2b} is hydrogen or C₁-C₆ alkyl;
R^{2c} is hydrogen or C₁-C₆ alkyl;
R³ and R⁴ are each independently hydrogen or C₁-C₆ alkyl optionally substituted with halogen or -OH, and at least one of R³ and R⁴ is hydrogen, or R³ and R⁴, together with the atom linked thereto, form 3- to 6-membered cycloalkyl; A is optionally substituted 6-membered aryl or optionally substituted 5- to 6-membered heteroaryl; provided that when 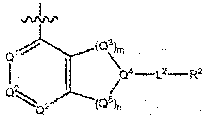 is 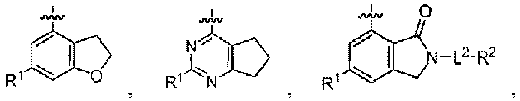 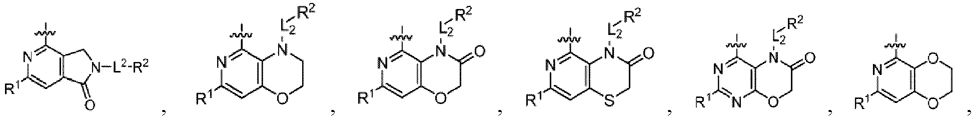 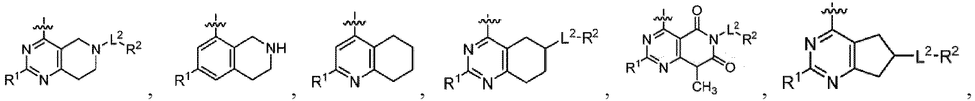 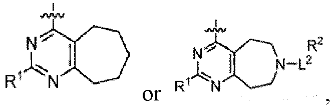 R¹ is not hydrogen; 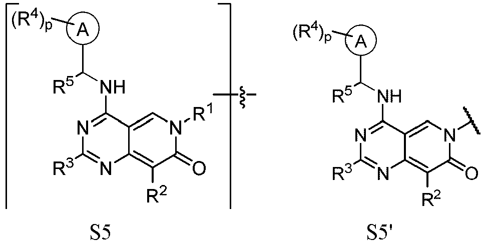
wherein in S5 or S5':
R¹ is hydrogen or R^{a1};
R^{a1} is selected from C₁-C₆ alkyl, C₁-C₆ haloalkyl, C₂-C₆ alkenyl, C₁-C₆ alkynyl, C₃-C₁₀ cycloalkyl, C₄-C₁₀ cycloalkenyl, 3- to 10-membered heterocycloalkyl, C₆-C₁₀ aryl and 5- to 10-membered heteroaryl, and the C₁-C₆ alkyl, the C₁-C₆ haloalkyl, the C₂-C₆ alkenyl, the C₁-C₆ alkynyl, the C₃-C₁₀ cycloalkyl, the C₄-C₁₀ cycloalkenyl, the 3- to 10-membered heterocycloalkyl, the C₆-C₁₀ aryl and the 5- to 10-membered heteroaryl are optionally substituted with 1 or more identical or different R^{b1} and/or R^{c1};
each R^{b1} is independently -OR^{c1}, -NR^{c1}R^{c1}, halogen, -CN, -C(O)R^{c1}, -C(O)OR^{c1}, -C(O)NR^{c1}R^{c1}, -S(O)₂R^{c1}, - S(O)₂NR^{c1}R^{c1}, -NHC(O)R^{c1}, -N(C₁-C₄ alkyl)C(O)R^{c1}, -NHC(O)OR^{c1} or -N(C₁-C₄ alkyl)C(O)OR^{c1};
each R^{c1} is independently C₁-C₆ alkyl, C₁-C₆ haloalkyl, C₂-C₆ alkenyl, C₂-C₆ alkynyl, C₃-C₁₀ cycloalkyl, C₄-C₁₀ cycloalkenyl, 3- to 10-membered heterocycloalkyl, C₆-C₁₀ aryl and 5- to 10-membered heteroaryl, and the C₁-C₆ alkyl, the C₁-C₆ haloalkyl, the C₂-C₆ alkenyl, the C₂-C₆ alkynyl, the C₃-C₁₀ cycloalkyl, the C₄-C₁₀ cycloalkenyl, the 3- to 10-membered heterocycloalkyl, the C₆-C₁₀ aryl and the 5- to 10-membered heteroaryl are each independently optionally substituted with 1 or more identical or different R^{d1} and/or R^{e1};
each R^{d1} is independently -OR^{e1}, -NR^{e1}R^{e1}, halogen, -CN, -C(O)R^{e1}, -C(O)OR^{e1}, -C(O)NR^{e1}R^{e1}, -S(O)₂R^{e1}, - S(O)_{Z}NR^{e1}R^{e1}, -NHC(O)R^{e1}, -N(C₁-C₄ alkyl)C(O)R^{e1}, -NHC(O)OR^{e1} or -N(C₁-C₄ alkyl)C(O)OR^{e1};
each R^{e1} is independently hydrogen, C₁-C₆ alkyl, C₁-C₆ haloalkyl, C₂-C₆ alkenyl, C₂-C₆ alkynyl, C₃-C₁₀ cycloalkyl, C₄-C₁₀ cycloalkenyl, 3- to 10-membered heterocycloalkyl, C₆-C₁₀ aryl or 5- to 10-membered heteroaryl;
R² is selected from hydrogen, C₁-C₄ alkyl, C₃-C₆ cycloalkyl, 3- to 6-membered heterocycloalkyl and halogen;
ring A is selected from C₆-C₁₀ aryl, 5- to 10-membered heteroaryl and 9- to 10-membered biheterocycloalkyl;
P is 1, 2 or 3;
each R⁴ is independently hydrogen, hydroxy, oxo, halogen, cyano, C₁-C₄ alkyl, -NH₂, C₁-C₄ haloalkyl, C₂-C₄ alkenyl, C₂-C₄ alkynyl, C₁-C₄ hydroxyalkyl, hydroxy-C₁-C₄ haloalkyl, C₃-C₆ cycloalkyl, C₃-C₆ cycloalkoxy, 3- to 6-membered heterocycloalkyl, C₃-C₆ hydroxycycloalkyl, C₁-C₄ haloalkyl substituted with 3- to 6-membered heterocycloalkyl or 3- to 6-membered heterocycloalkyl substituted with hydroxy, halogen, -NH₂, -S(O)₂-(C₁-C₄ alkyl) or oxo, and the substitution with the oxo is performed only on a non-aromatic ring;
R⁴ is -NRₐR_{b}, wherein Rₐ and R_{b} are each independently hydrogen, C₁-C₆ alkyl, C₃-C₈ heterocycloalkyl or C₃-C₈ cycloalkyl;
R⁴ is -C(O)NRₐR_{b}, wherein Rₐ and R_{b} are each independently hydrogen, C₁-C₆ alkyl, C₃-C₈ heterocycloalkyl or C₃-C₈ cycloalkyl;
R⁴ is -C(O)OR_{g}, wherein R_{g} is hydrogen or C₁-C₆ alkyl;
R⁴ is -ORₕ; Rₕ is C₁-C₆ alkyl;
or R⁴ is -(CH₂)NRₐR_{b}, wherein Rₐ and R_{b} are each independently hydrogen, C₁-C₆ alkyl, C₃-C₈ heterocycloalkyl or C₃-C₈ cycloalkyl;
R³ and Rare each independently hydrogen, C₁-C₆ alkyl, C₁-C₆ alkoxy, C₃-C₆ cycloalkyl or C₃-C₆ cycloalkoxy, wherein the alkyl, the alkoxy, the cycloalkyl and the cycloalkoxy are optionally substituted with cyano, hydroxy, C₁-C₆ alkoxy, C₃-C₆ cycloalkyl, C₃-C₆ cycloalkoxy or halogen.

3. The compound of formula I, and/or the stereoisomer, the enantiomer, the diastereomer, the deuteride, the hydrate, the solvate, the prodrug and/or the pharmaceutically acceptable salt thereof according to claim 1 or 2, wherein S is S1a, S1a', -X-S1a', S1a", -X-S1a", S2a, S2a', -X-S2a', S2a", -X-S2a", S3a, S4a, S5a, S5a' or -X-S5a'; X is O, NH or S, preferably O; 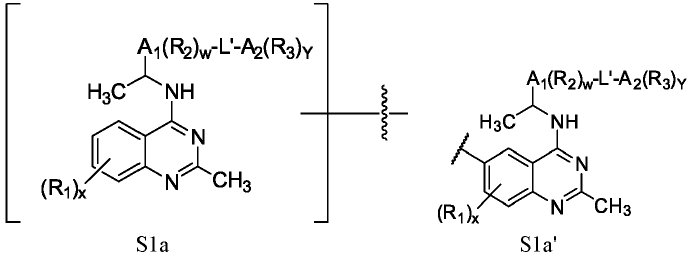 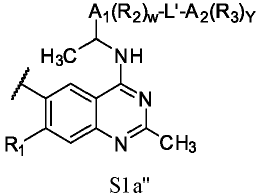 wherein in S1a, S1a' or S1a":
R₁ is selected from hydrogen, -OCH₃, -OCH₂CH₃, 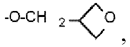 -CH₂OH, -C(O)OH, -C(O)OCH₃, -Br, - OCH(CH₃)₂, -O(CH₂)₂CH(CH₃)₂, -O(CH₂)₃CH₃, -O(CH₂)₂OCH₃, 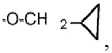 -O(CH₂)-phenyl, -N=S(O)(CH₃)₂, - CH₃, cyclopropyl, -N(CH₃)₂, -NHCH₃, -NH₂, 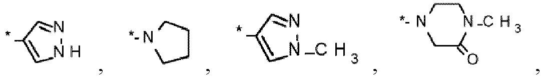 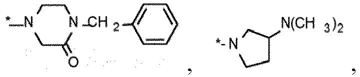 -C(CH₃)₂-OH, 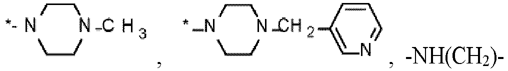 NH-C(O)CH₃, -NH(CH₂)-morpholine, -NH-C(O)CH₃, -NH-C(O)NHCH₃, -NH-C(O)-N(CH₃)₂, nitro, -NH-S(O)₂CH₃, -N=S(O)(CH₃)₂, hydroxy, -O-(CH₂)₂-S(O)₂CH₃, -F, 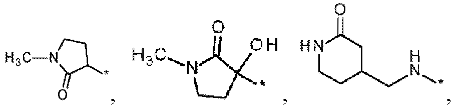 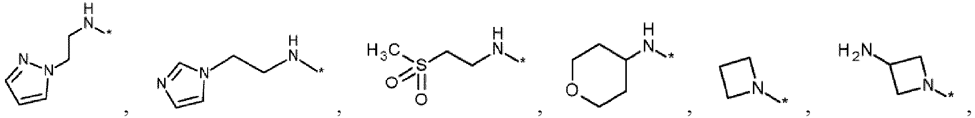 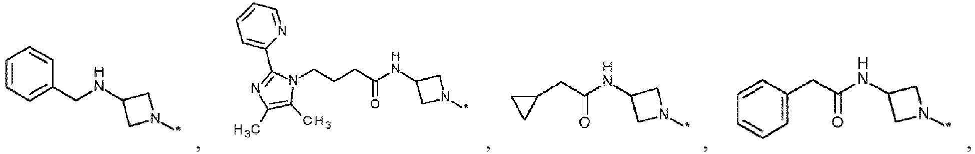 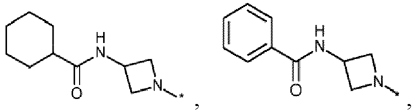 cyclopropyloxy, cyclobutyloxy, cyclopentyloxy, cyclohexyloxy, azetidinyl, azacyclopentyl, piperidinyl, piperazinyl, oxetanyl, oxolanyl, oxanyl, thietanyl, thiolanyl, thianyl, azetidinyloxy, azacyclopentyloxy, piperidinyloxy, piperazinyloxy, oxetanyloxy, oxolanyloxy, oxanyloxy, thietanyloxy, thiolanyloxy, thianyloxy, 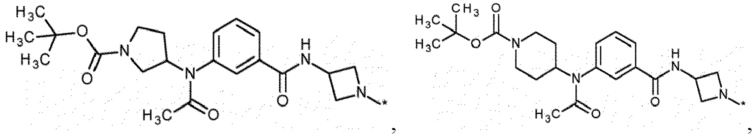 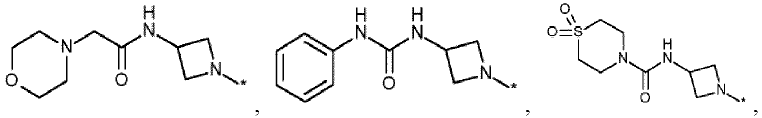 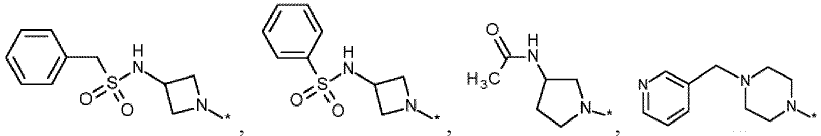 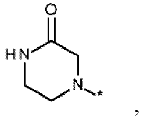 -OCH₂CH₂CH₃, -OCH₂CH₂CH₂N(CH₃)₂, -OCH₂CH₂CH₂OH, 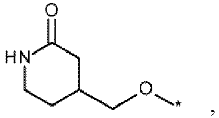 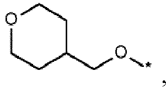 -OCH₂CH₂NC(O)CH₃, -OCH₂CH₂N(CH₃)₂, -OCH₂CH₂OH, 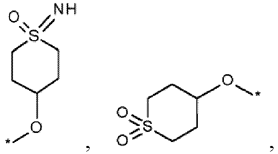 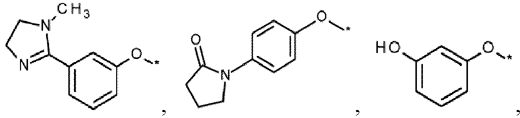 -SCH₃, -N(CH₃)₂, 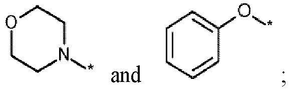
x is 1 or 2;
A₁ is selected from 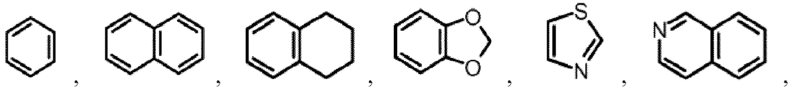 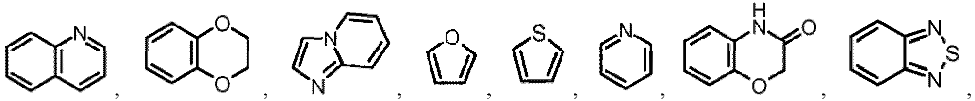 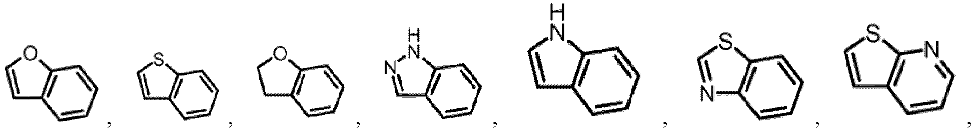 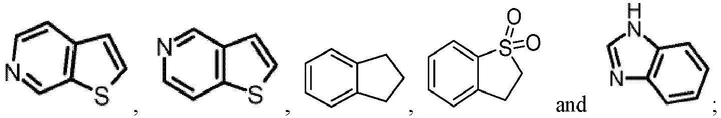
R₂ is selected from hydrogen, hydroxy, oxo, cyano, cyclopropyl, 1,1-dimethylcyclopropyl, -C(=CH₂)CH₃, - C(CH₃)(=CH₂)CH₃, -CH=CH(CH₂)₂CH₃, -CH=CHCH₃, -CH=CH-cyclopropyl, -C(O)NH₂, -C(O)OCH₃, - S(O)₂CH₃, -OCH₃, -CH₂NH₂, trifluoromethyl, methyl, trifluoromethoxy, halogen (F, Cl or Br), -NH₂, -NHC(O)CH₃, -NHCH₂CH₃ and -NHCH(CH₃)₂;
w is 1, 2 or 3;
A₂ is selected from 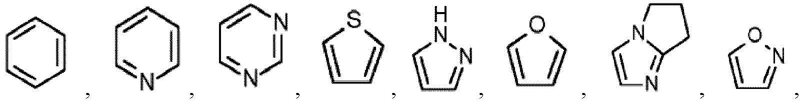 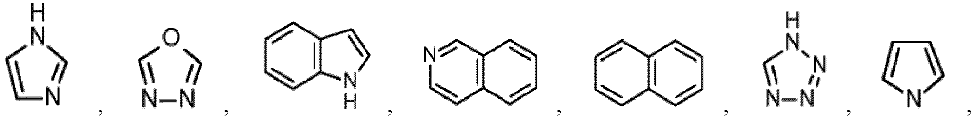 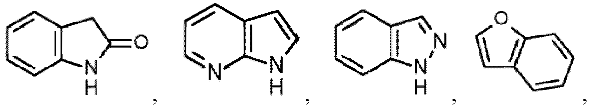 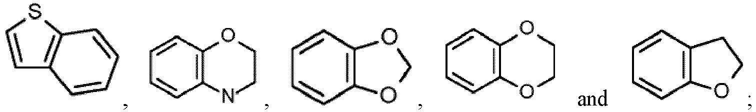
R₃ is selected from -C(O)NH(CH₂)₂CH₃, -C(O)N(CH₃)₂, -C(O)NH₂, -C(O)NH(CH₂)₂N(CH₃)₂, -CH₂C(O)NH₂, hydrogen, -F, -Cl, -Br, cyano, -CF₃, -CH₃, -CH₂CH₃, -CH=CH₂, -CH₂CN, -CH(CH₃)-NH₂, -CH=CH-CN, -C(O)-OH, -C(O)OCH₃, -C(O)CH₃, -C(CH₃)₂-C(O)-OCH₃, -C(CH₃)₂-CN, oxo, hydroxy, cyclopropyl, cyclobutyl, cyclopentyl, -NH₂, -NH-C(O)CH₃, -NH-S(O)₂CH₃, -NH-C(O)OC(CH₃)₃, 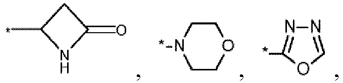 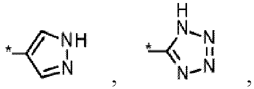 -S(O)₂CH₃, -S(O)₂NCH₃, -S(O)₂NH₂, -OCH₂CH₃, -O(CH₂)₂CH₃, -OCF₃, 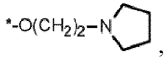 -OCH₂cyclopropyl, -OCH₃, -O(CH₂)₃CH₃, -OCH₂phenyl, -O-phenyl, -(CH₂)-OH, -(CH₂)₂-OH, -(CH₂)-OCH₃, -(CH₂)-OCH₂CH₃, -CH(OH)-CH₂-phenyl, -CH(OH)-CH₂CH₃, -CH(OH)-CH₂CH₂CH₃, -CH(OH)-CH₂CH₂CH₂CH₃, -CH(OH)-CH(CH₃)₂, -CH(OH)-phenyl, -CH(OH)-CN, -CH(OH)-CH₂-OH, -CH(OH)-CF₃, - CH(OH)-(CH₂)₂-phenyl, *-CH(OH)-C≡CH, -CH(NH₂)-CH₂-C(O)OH, -CH₂-NH-S(O)₂-CH₃, -CH₂-NH-(CH₂)₃CH₃, - CH₂-NH-CH₃, -CH₂-N(CH₃)₂, -CH₂-NH-CH₂CH₃, -CH(CH₃)-NH₂, -CH₂-NH₂, -CH₂CH₂-NH₂, -CH₂NH-CH₂-phenyl, -CH₂N(CH₂CH₃)₂, -CH₂NH-cyclopropyl, -CH₂NH-cyclobutyl, -CH₂NH-cyclopentyl, -CH₂NH-pyridyl, - CH₂NH-phenyl, -CH₂NH-(CH₂)₂-OH, -CH₂N(CH₃)-(CH₂)₂-OH, -CH₂NH-CH₂-CN, -CH₂N(CH₃)-CH₂-CN, - CH₂N(CH₃)-CH₂-CF₃, -CH₂N(CH₃)-CH₂-CF₂H, -CH₂NH-CH₂-CF₂H, -CH₂NH-(CH₂)₂-OCH₃, 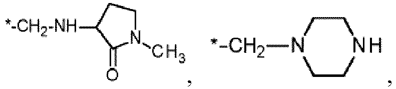 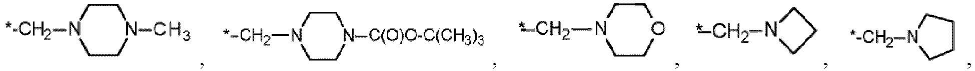 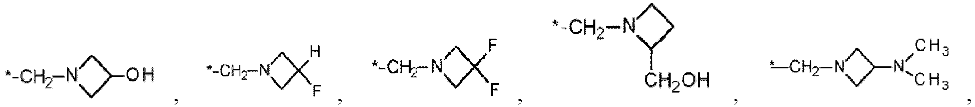 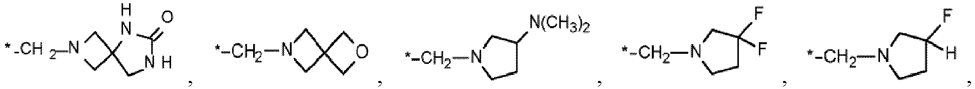 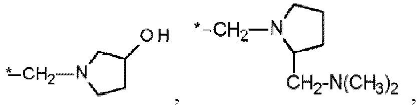 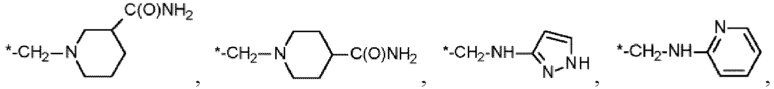 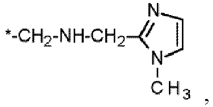 -CH₂NH-C(O)-OC(CH₃)₃, -CH₂CH₂NH-C(O)-OC(CH₃)₃, -CH₂NH-C(O)-CH₂NOH, - CH₂NH-C(O)-CH₂OCH₃, -CH(CH₃)NH-C(O)-OC(CH₃)₃, -CH₂NH-C(O)-CH₃, 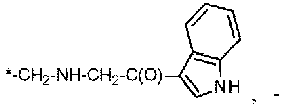 CH₂NHCH₂-C(O)-NH₂, -CH₂NHCH₂-C(O)-(CH₃)₂, -CH₂NHCH₂-C(O)-OCH₃, -CH₂NHCH₂-C(O)-NHCH₃, - CH₂NHCH₂-C(O)-NH(CH₂)₂-OCH₃, -CH₂NHCH₂-C(O)-NHCH₂-phenyl, 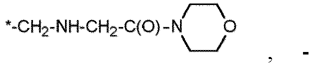 CH₂NHCH₂-C(O)-NH-phenyl, 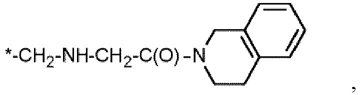 -CH₂NH-C(O)-CH₂NH-phenyl, 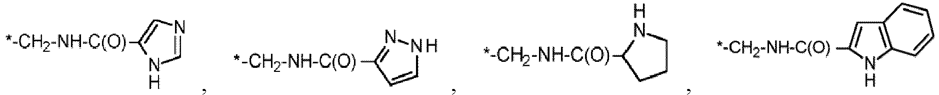 and - CH₂NH-C(O)-CH₂NH-CH₂CF₃;
y is 1 or 2;
L' is a bond, -(CH₂)ₖ- or -O(CH₂)ₖ-, wherein k is 1 or 2, or L' is -CH=CH-(CH₂)ₙ-, wherein n is 0, 1 or 2; 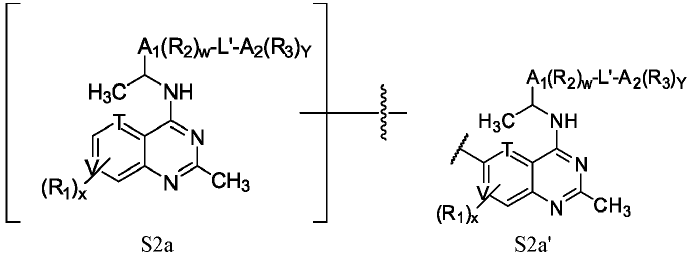 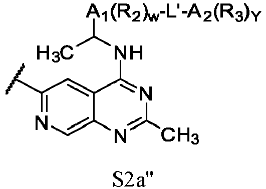
wherein in S2a, S2a' or S2a":
T and V are both N, or T is C and V is N, or T is N and V is C;
R₁ is selected from hydrogen, -OCH₃, -OCH₂CH₃, 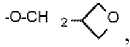 -CH₂OH, -C(O)OH, -C(O)OCH₃, -Br, - OCH(CH₃)₂, -O(CH₂)₂CH(CH₃)₂, -O(CH₂)₃CH₃, -O(CH₂)₂OCH₃, 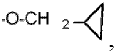 -O(CH₂)_{z}-phenyl, -N=S(O)(CH₃)₂, -CH₃, cyclopropyl, -N(CH₃)₂, -NHCH₃, -NH₂, 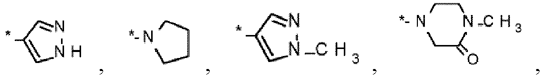 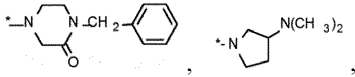 -C(CH₃)₂-OH, 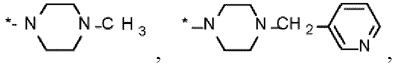 -NH(CH₂)-NH-C(O)CH₃, -NH(CH₂)-morpholine, -NH-C(O)CH₃, -NH-C(O)NHCH₃, -NH-C(O)-N(CH₃)₂, nitro, -NH-S(O)₂CH₃, -N=S(O)(CH₃)₂, hydroxy, -O-(CH₂)₂-S(O)₂CH₃, -F, 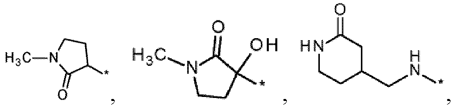 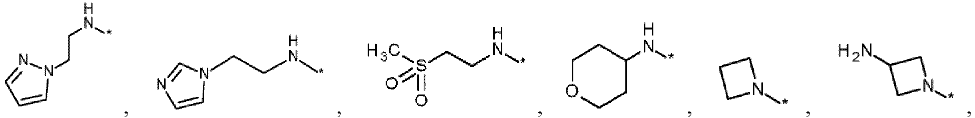 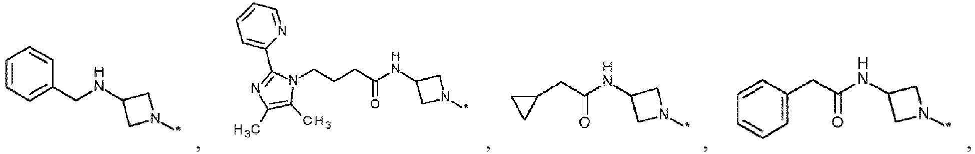 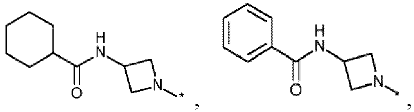 cyclopropyloxy, cyclobutyloxy, cyclopentyloxy, cyclohexyloxy, azetidinyl, azacyclopentyl, piperidinyl, piperazinyl, oxetanyl, oxolanyl, oxanyl, thietanyl, thiolanyl, thianyl, azetidinyloxy, azacyclopentyloxy, piperidinyloxy, piperazinyloxy, oxetanyloxy, oxolanyloxy, oxanyloxy, thietanyloxy, thiolanyloxy, thianyloxy, 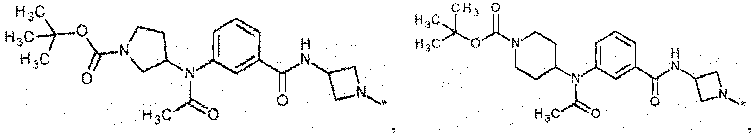 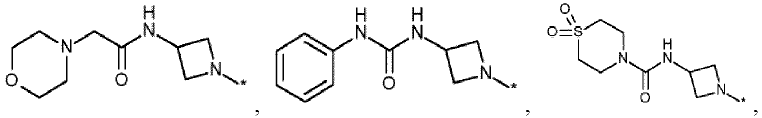 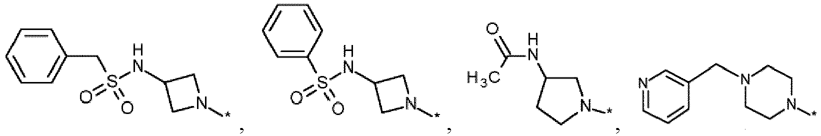 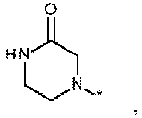 -OCH₂CH₂CH₃, -OCH₂CH₂CH₂N(CH₃)₂, -OCH₂CH₂CH₂OH, 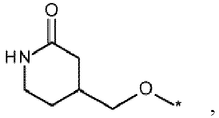 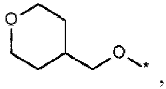 -OCH₂CH₂NC(O)CH₃, -OCH₂CH₂N(CH₃)₂, -OCH₂CH₂OH, 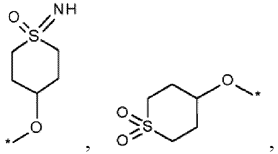 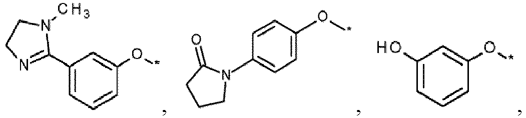 -SCH₃, -N(CH₃)₂, 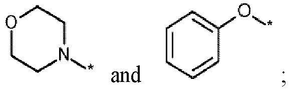
z is 1 or 2;
x is 1 or 2;
A₁ is selected from 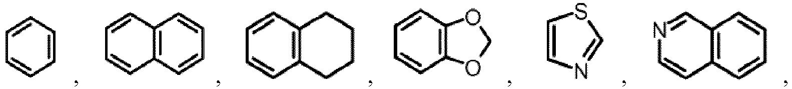 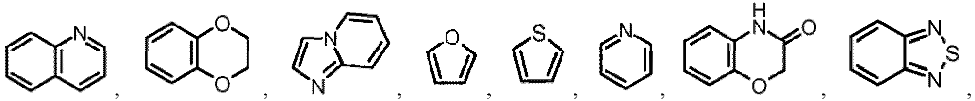 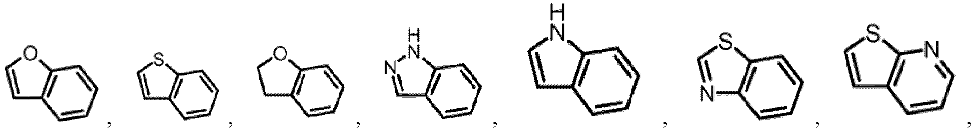 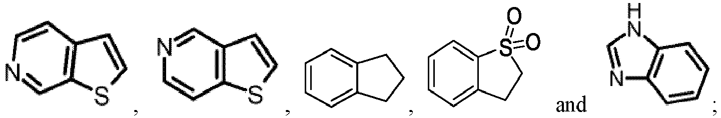
R₂ is selected from hydrogen, hydroxy, oxo, cyano, cyclopropyl, 1,1-dimethylcyclopropyl, -C(=CH₂)CH₃, - C(CH₃)(=CH₂)CH₃, -CH=CH(CH₂)₂CH₃, -CH=CHCH₃, -CH=CH-cyclopropyl, -C(O)NH₂, trifluoromethyl, methyl, trifluoromethoxy, -C(O)OCH₃, -S(O)₂CH₃, -OCH₃, -CH₂NH₂ and halogen (F, Cl or Br);
w is 1, 2 or 3;
A₂ is selected from 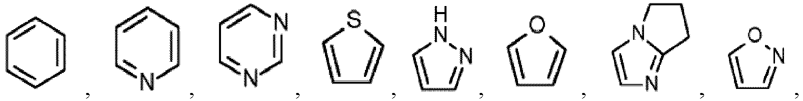 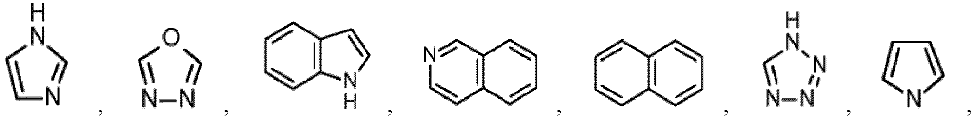 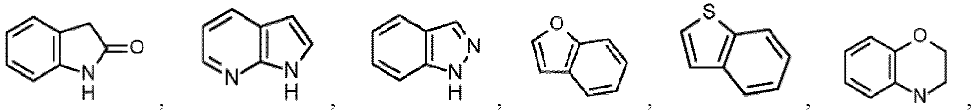 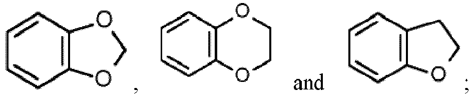
R³ is selected from -C(O)NH(CH₂)₂CH₃, -C(O)N(CH₃)₂, -C(O)NH₂, -C(O)NH(CH₂)₂N(CH₃)₂, -CH₂C(O)NH₂, hydrogen, -F, -Cl, -Br, cyano, -CF₃, -CH₃, -CH₂CH₃, -CH=CH₂, -CH₂CN, -CH(CH₃)-NH₂, -CH=CH-CN, -C(O)-OH, -C(O)OCH₃, -C(O)CH₃, -C(CH₃)₂-C(O)-OCH₃, -C(CH₃)₂-CN, oxo, hydroxy, cyclopropyl, cyclobutyl, cyclopentyl, -NH₂, -NH-C(O)CH₃, -NH-S(O)₂CH₃, -NH-C(O)OC(CH₃)₃, 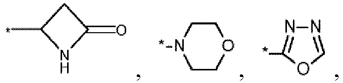 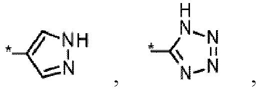 -S(O)₂CH₃, -S(O)₂NCH₃, -S(O)₂NH₂, -OCH₂CH₃, -O(CH₂)₂CH₃, -OCF₃, 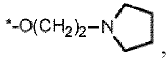 -OCH₂cyclopropyl, -OCH₃, -O(CH₂)₃CH₃, -OCH₂phenyl, -O-phenyl, -(CH₂)-OH, -(CH₂)₂-OH, -(CH₂)-OCH₃, -(CH₂)-OCH₂CH₃, -CH(OH)-CH₂-phenyl, -CH(OH)-CH₂CH₃, -CH(OH)-CH₂CH₂CH₃, -CH(OH)-CH₂CH₂CH₂CH₃, -CH(OH)-CH(CH₃)₂, -CH(OH)-phenyl, -CH(OH)-CN, -CH(OH)-CH₂-OH, -CH(OH)-CF₃, - CH(OH)-(CH₂)₂-phenyl, *-CH(OH)-C≡CH, -CH(NH₂)-CH₂-C(O)OH, -CH₂-NH-S(O)₂-CH₃, -CH₂-NH-(CH₂)₃CH₃, - CH₂-NH-CH₃, -CH₂-N(CH₃)₂, -CH₂-NH-CH₂CH₃, -CH(CH₃)-NH₂, -CH₂-NH₂, -CH₂CH₂-NH₂, -CH₂NH-CH₂-phenyl, -CH₂N(CH₂CH₃)₂, -CH₂NH-cyclopropyl, -CH₂NH-cyclobutyl, -CH₂NH-cyclopentyl, -CH₂NH-pyridyl,-CH₂NH-phenyl, -CH₂NH-(CH₂)₂-OH, -CH₂N(CH₃)-(CH₂)₂-OH, -CH₂NH-CH₂-CN, -CH₂N(CH₃)-CH₂-CN, - CH₂N(CH₃)-CH₂-CF₃, -CH₂N(CH₃)-CH₂-CF₂H, -CH₂NH-CH₂-CF₂H, -CH₂NH-(CH₂)₂-OCH₃, 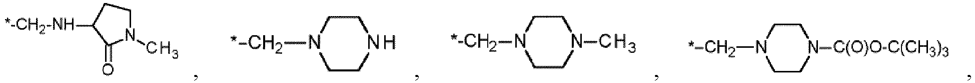 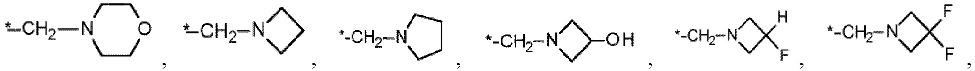 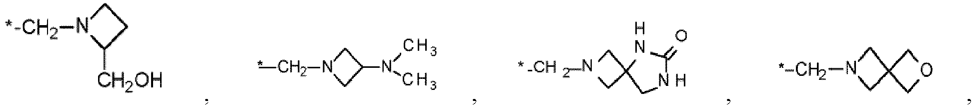 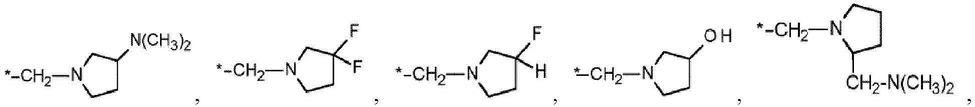 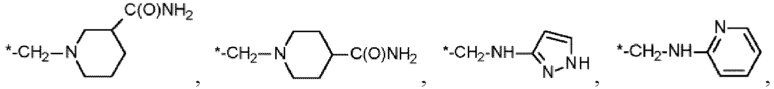 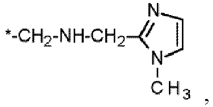 -CH₂NH-C(O)-OC(CH₃)₃, -CH₂CH₂NH-C(O)-OC(CH₃)₃, -CH₂NH-C(O)-CH₂NOH, - CH₂NH-C(O)-CH₂OCH₃, -CH(CH₃)NH-C(O)-OC(CH₃)₃, -CH₂NH-C(O)-CH₃, 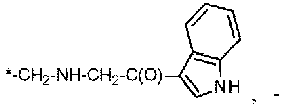 CH₂NHCH₂-C(O)-NH₂, -CH₂NHCH₂-C(O)-(CH₃)₂, -CH₂NHCH₂-C(O)-OCH₃, -CH₂NHCH₂-C(O)-NHCH₃, - CH₂NHCH₂-C(O)-NH(CH₂)₂-OCH₃, -CH₂NHCH₂-C(O)-NHCH₂-phenyl, 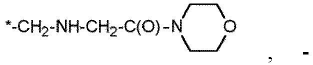 CH₂NHCH₂-C(O)-NH-phenyl, 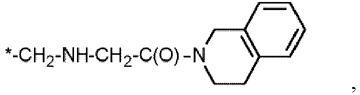 -CH₂NH-C(O)-CH₂NH-phenyl, 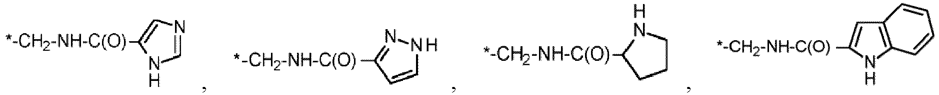 and - CH₂NH-C(O)-CH₂NH-CH₂CF₃;
Y is 1 or 2;
L' is a bond, -(CH₂)k- or -O(CH₂)ₖ-, wherein k is 1 or 2, or L' is -CH=CH-(CH₂)ₙ, wherein n is 0 or 1; 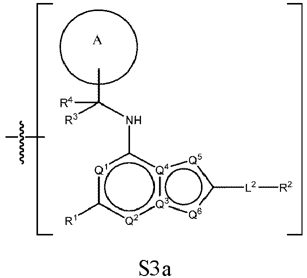
wherein in S3a,
Q¹ and Q² are each independently CH or N;
Q³ and Q⁴ are each independently C or N, and at least one of Q³ and Q⁴ is C;
Q⁶ is CH, N, NH, O or S;
Q⁵ is CH, N, NH, O or S;
at least one of Q¹, Q², Q³, Q⁴, Q⁵ and Q⁶ is N, NH, O or S;
R¹ is selected from hydrogen, C₁-C₆ alkyl, halogen, -OR^{1a}, cyclopropyl and -CN; R^{1a} is hydrogen or C₁-C₆ alkyl;
L² is selected from a bond, -C(O)-, -C(O)O-, -C(O)NH(CH₂)ₒ-, -S(O)₂-, -C(O)(CH₂)ₚ-, -(CH₂)ₚ- and O; o is 0, 1 or 2; p is an integer of 1 to 6;
R² is selected from hydrogen, -(CH₂)_{q}CH₃, 3- to 14-membered cycloalkyl, 3- to 14-membered cycloalkenyl, 3- to 14-membered heterocycloalkyl, 6- to 10-membered aryl and 5- to 10-membered heteroaryl; q is an integer of 1 to 5; the 3- to 14-membered cycloalkyl, the 3- to 14-membered cycloalkenyl, the 3- to 14-membered heterocycloalkyl, the 6- to 10-membered aryl and the 5- to 10-membered heteroaryl are each independently optionally substituted with a substituent selected from C₁-C₆ alkyl, -OH, halogen, -C(O)R^{2a} and -C(O)NR^{2b}R^{2c}; R^{2a} is C₁-C₆ alkyl or - (CH₂)ᵣOCH₃, and r is 1, 2 or 3; R^{2b} is hydrogen or C₁-C₆ alkyl; R^{2c} is hydrogen or C₁-C₆ alkyl;
R³ and R⁴ are each independently hydrogen or C₁-C₆ alkyl, and at least one of R³ and R⁴ is not hydrogen, or R³ and R⁴, together with the atom linked thereto, form 3- to 6-membered cycloalkyl;
A is optionally substituted phenyl or optionally substituted 5- to 6-membered heteroaryl; 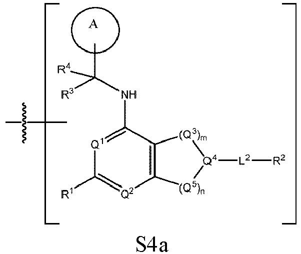
wherein in S4a,
Q¹ is CH or N;
Q⁴ is CH, C or N;
each Q² is independently CR¹ or N;
each Q³ and Q⁵ molecule is independently C(R^{QC})₂, NR^{QN}, C(O), O, S or SO₂, R^{QC} is each independently hydrogen, F, Cl, Br or 6- to 10-membered aryl, and R^{QN} is each independently hydrogen, C₁-C₆ alkyl or 6- to 10-membered aryl;
at least one of Q¹, Q², Q³, Q⁴ and Q⁵ is N, NR^{QN}, O or SO₂;
R¹ is selected from hydrogen, C₁-C₆ alkyl, halogen, cyclopropyl, cyano and -OR^{1A}, and R^{1a} is hydrogen or C₁-C₆ alkyl;
L² is selected from a bond, -C(O)-, -C(O)O-, -C(O)NH(CH₂)ₒ-, -S(O)₂-, -C(O)(CH₂)ₚ-, -(CH₂)ₚ- and O; o is 0, 1 or 2; p is an integer of 1 to 6;
R² is selected from hydrogen, -(CH₂)_{q}CH₃, 3- to 14-membered cycloalkyl, 3- to 14-membered cycloalkenyl, 3- to 14-membered heterocycloalkyl, 6- to 10-membered aryl and 5- to 10-membered heteroaryl; q is a multiple of 1 to 5; the 3- to 14-membered cycloalkyl, the 3- to 14-membered cycloalkenyl, the 3- to 14-membered heterocycloalkyl, the 6- to 10-membered aryl and the 5- to 10-membered heteroaryl are each independently optionally substituted with a group selected from C₁-C₆ alkyl, hydroxy, halogen, -C(O)R^{2a} and -C(O)NR^{2b}R^{2c}; R^{2a} is selected from C₁-C₆ alkyl and -(CH₂)ᵣOCH₃, and r is 1, 2 or 3; R^{2b} and R^{2c} are each independently hydrogen or C₁-C₆ alkyl;
R³ and R⁴ are each independently hydrogen or C₁-C₆ alkyl, and at least one of R³ and R⁴ is hydrogen, or R³ and
R⁴, together with the atom linked thereto, form 3- to 6-membered cycloalkyl;
A is optionally substituted 6-membered aryl or optionally substituted 5- to 6-membered heteroaryl; 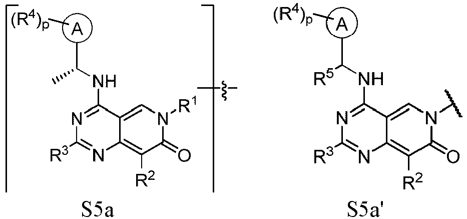
wherein, in S5a or S5a', R¹, R², R³, R⁴, ring A and p are as defined and described in S5 in claim 2.

4. The compound of formula I, and/or the stereoisomer, the enantiomer, the diastereomer, the deuteride, the hydrate, the solvate, the prodrug and/or the pharmaceutically acceptable salt thereof according to claim 1, wherein S is S6, S6', -X-S6', S6", S6‴, -X-S6‴, S6a, S6a", S6c, S6c", -X-S6c or -X-S6c", preferably S6c; X is O, NH or S, preferably O; 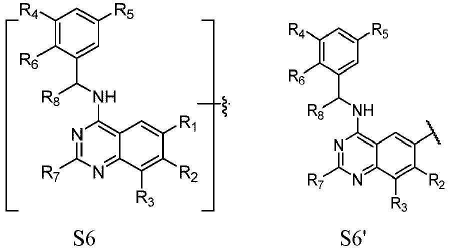 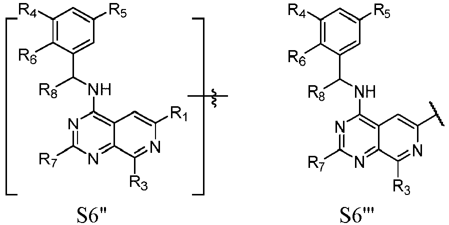 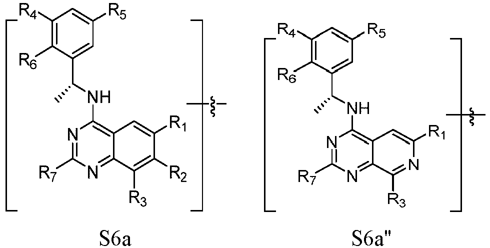 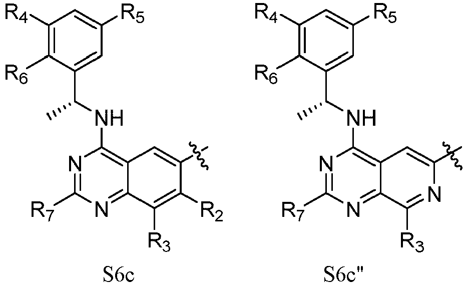 wherein in S6, S6', S6", S6‴, S6a, S6a", S6c or S6c":
R₂ is selected from hydrogen, halogen, -OH, -CN, -NO₂, C₁-C₆ alkyl sulfhydryl and -NRₐR_{b}, wherein Rₐ and R_{b} are each independently hydrogen, C₁-C₆ alkyl, C₃-C₈ heterocycloalkyl or C₃-C₈ cycloalkyl;
R₂ is selected from C₁-C₆ alkyl, C₁-C₆ alkoxy, C₃-C₆ cycloalkoxy, C₃-C₆ heterocycloalkoxy, C₂-C₆ alkenyl, C₂-C₆ alkynyl, C₃-C₈ cycloalkyl, C₄-C₈ cycloalkenyl, 4- to 7-membered heterocycloalkyl, 5- to 10-membered heterocycloalkenyl, spiroheterocycloalkyl, fused heterocycloalkyl, bridged heterocycloalkyl, phenyl, heteroaryl,
C₁-C₆ haloalkyl, -COOH and -COOR_{c}; R_{c} is -C₁-C₆ alkyl, C₃-C₆ alkenyl, C₃-C₆ alkynyl, C₃-C₈ cycloalkyl or -C₄-C₈ cycloalkenyl;
R₂ is -NS(O)(R_{d})(Rₑ), wherein R_{d} and Rₑ are each independently C₁-C₆ alkyl, C₂-C₆ alkenyl, C₂-C₆ alkynyl, C₃-C₈ cycloalkyl, C₄-C₈ cycloalkenyl, 6- to 10-membered aryl or 5- to 10-membered heteroaryl;
R₂ is -NHC(O)-(C₁-C₆ alkyl) or -NHC(O)-NRₐR_{b}, wherein Rₐ and R_{b} are each independently hydrogen, -C₁-C₆ alkyl, C₃-C₈ heterocycloalkyl or C₃-C₈ cycloalkyl;
R₂ is -NH-(CH₂)ₖ-NH-C(O)-Rₐₐ, wherein Rₐₐ is C₁-C₆ alkyl, C₃-C₈ heterocycloalkyl or C₃-C₈ cycloalkyl, wherein k is 1 or 2;
R₂ is -NH-(CH₂)ᵢ-R_{f}, wherein i is 0, 1 or 2 and R_{f} is 4- to 7-membered heterocycloalkyl, heteroaryl or C₁-C₆ alkylsulfonyl;
furthermore, C₁-C₆ alkyl, C₁-C₆ alkoxy, 4- to 7-membered heterocycloalkyl and heteroaryl described above are optionally substituted with 1, 2 or 3 groups selected from halogen, -OH, oxo, -CN, -NO₂, C₁-C₆ alkyl, C₂-C₆ alkenyl,
C₂-C₆ alkynyl, C₃-C₈ cycloalkyl, 4- to 7-membered heterocycloalkyl, C₁-C₆ alkoxy, C₁-C₆ haloalkyl, C₁-C₆ haloalkoxy, C₁-C₆ alkylsulfonyl, phenyl, benzyl, heteroaryl, -(CH₂)-heteroaryl, -NHC(O)(C₁-C₆ alkyl), C₃-C₈ cycloalkoxy, phenoxy, heteroaryloxy and -NRₐR_{b}; Rₐ and R_{b} are each independently selected from hydrogen, C₁-C₆ alkyl, C₃-C₈ heterocycloalkyl and C₃-C₈ cycloalkyl;
R₂ is -O-(CH₂)_{z}-phenyl, -O-(CH₂)_{z}-(4- to 7-membered heterocycloalkyl) or -O-(CH₂)_{z}-heteroaryl, wherein z is 0, 1 or 2, and the phenyl, the heterocycloalkyl and the heteroaryl are optionally substituted with a group selected from - OH, heterocycloalkyl and heterocycloalkenyl and can be substituted with methyl or oxo;
or R₂ is 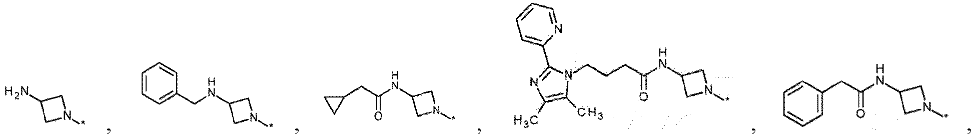 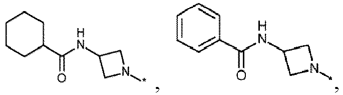 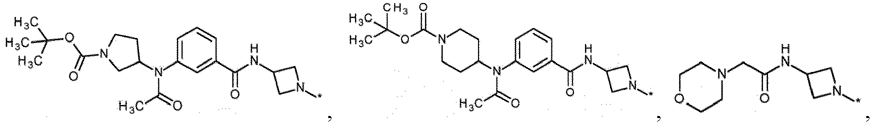 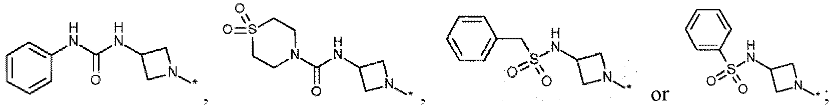
R₁ is hydrogen or -OR^{A};
R^{A} is hydrogen, C₃-C₁₀ cycloalkyl or 3- to 10-membered heterocycloalkyl, and the C₃-C₁₀ cycloalkyl and the 3- to 10-membered heterocycloalkyl are optionally substituted with 1 or more identical or different R^{a1} and/or R^{c1};
each R^{a1} is independently C₁-C₆ alkyl, C₂-C₆ alkenyl, C₂-C₆ alkynyl, C₃-C₁₀ cycloalkyl, C₆-C₁₀ aryl, 3- to 10-membered heterocycloalkyl or 5- to 10-membered heteroaryl, and the C₁-C₆ alkyl, the C₂-C₆ alkenyl, the C₂-C₆ alkynyl, the C₃-C₁₀ cycloalkyl, the C₆-C₁₀ aryl, the 3- to 10-membered heterocycloalkyl or the 5- to 10-membered heteroaryl is each independently optionally substituted with 1 or more identical or different R^{b1} and/or R^{c1};
each R^{b1} is independently substituted with -OR^{c1}, -NR^{c1}R^{c1}, halogen, -CN, -C(O)R^{c1}, -C(O)OR^{c1}, -C(O)NR^{c1}R^{c1}, - S(O)₂R^{c1}, -S(O)₂NR^{c1}R^{c1}, -NHC(O)R^{c1}, -N(C₁-C₄ alkyl)C(O)R^{c1} or oxo, and the substitution with the oxo is performed only on a non-aromatic ring;
each R^{c1} is independently hydrogen, C₁-C₆ alkyl, C₂-C₆ alkenyl, C₂-C₆ alkynyl, C₃-C₁₀ cycloalkyl, C₆-C₁₀ aryl, 3-to 10-membered heterocycloalkyl or 5- to 10-membered heteroaryl;
or R₁ is selected from C₃-C₁₀ cycloalkyl, C₃-C₁₀ cycloalkenyl, C₆-C₁₀ aryl, 3- to 10-membered heterocycloalkyl and 5- to 10-membered heteroaryl, and the C₃-C₁₀ cycloalkyl, the C₃-C₁₀ cycloalkenyl, the C₆-C₁₀ aryl, the 3- to 10-membered heterocycloalkyl or the 5- to 10-membered heteroaryl is optionally substituted with 1 or more R^{a2} and/or R^{b2};
each R^{a2} is independently selected from C₁-C₆ alkyl, C₂-C₆ alkenyl, C₂-C₆ alkynyl, C₃-C₁₀ cycloalkyl, C₆-C₁₀ aryl, 3- to 10-membered heterocycloalkyl and 5- to 10-membered heteroaryl, and the C₁-C₆ alkyl, the C₂-C₆ alkenyl, the C₂-C₆ alkynyl, the C₃-C₁₀ cycloalkyl, the C₆-C₁₀ aryl, the 3- to 10-membered heterocycloalkyl or the 5- to 10-membered heteroaryl is optionally substituted with 1 or more R^{c2} and/or R^{b2};
each R^{b2} is independently selected from -OR^{c2}, -NR^{c2}R^{c2}, halogen, -CN, -C(O)R^{c2}, -C(O)OR^{c2}, -C(O)NR^{c2}R^{c2}, - OC(O)R^{c2}, -S(O)₂R^{c2}, -S(O)₂NR^{c2}R^{c2}, -NHC(O)R^{c2}, -N(C₁-C₄ alkyl)C(O)R^{c2}, -NHC(O)OR^{c2}, oxo and =NH, and the substitution with the oxo or the =NH is performed only on a non-aromatic ring;
each R^{c2} is independently selected from hydrogen, C₁-C₆ alkyl, C₂-C₆ alkenyl, C₂-C₆ alkynyl, C₃-C₁₀ cycloalkyl, C₆-C₁₀ aryl, 3- to 10-membered heterocycloalkyl and 5- to 10-membered heteroaryl, and the C₁-C₆ alkyl, the C₂-C₆ alkenyl, the C₂-C₆ alkynyl, the C₃-C₁₀ cycloalkyl, the C₆-C₁₀ aryl, the 3- to 10-membered heterocycloalkyl or the 5-to 10-membered heteroaryl is optionally substituted with 1 or more R^{d2} and/or R^{e2};
each R^{d2} is independently -OR^{e2}, -NR^{e2}R^{e2}, halogen, -CN, -C(O)R^{e2}, -C(O)OR^{e2}, -C(O)NR^{e2}R^{e2}, -S(O)₂R^{e2}, - S(O)₂NR^{e2}R^{e2}, -NHC(O)R^{e2}, -N(C₁-C₄ alkyl)C(O)R^{e2} or oxo, and the substitution with the oxo is performed only on a non-aromatic ring;
each R^{e2} is independently selected from hydrogen, C₁-C₆ alkyl, C₂-C₆ alkenyl, C₂-C₆ alkynyl, C₃-C₁₀ cycloalkyl, C₆-C₁₀ aryl, 3- to 10-membered heterocycloalkyl and 5- to 10-membered heteroaryl, and the C₁-C₆ alkyl, the C₂-C₆ alkenyl, the C₂-C₆ alkynyl, the C₃-C₁₀ cycloalkyl, the C₆-C₁₀ aryl, the 3- to 10-membered heterocycloalkyl or the 5-to 10-membered heteroaryl is optionally substituted with 1 or more R^{f2} and/or R^{g2};
each R^{f2} is independently selected from -OR^{g2}, -NR^{g2}R^{g2}, halogen, -CN, -C(O)R^{g2}, -C(O)OR^{g2}, -C(O)NR^{g2}R^{g2}, - S(O)₂R^{g2}, -S(O)₂NR^{g2}R^{g2}, -NHC(O)R^{g2}, -N(C₁-C₄ alkyl)C(O)R^{g2} and oxo, and the substitution with the oxo is performed only on a non-aromatic ring;
each R^{g2} is independently selected from hydrogen, C₁-C₆ alkyl, C₂-C₆ alkenyl, C₂-C₆ alkynyl, C₃-C₁₀ cycloalkyl, C₆-C₁₀ aryl, 3- to 10-membered heterocycloalkyl and 5- to 10-membered heteroaryl;
or R₁ is selected from C₂-C₄ alkyl and C₂-C₄ alkenyl, and the C₂-C₄ alkyl and the C₂-C₄ alkenyl are optionally substituted with R^{b3};
R^{b3} is selected from -C(O)R^{c3}, -C(O)OR^{c3}, -C(O)NR^{c3}R^{c3}, -C(O)NHOR^{c3} and -C(O)N(C₁-C₄ alkyl)OR^{c3};
each R^{c3} is independently selected from hydrogen, C₁-C₆ alkyl, C₂-C₆ alkenyl, C₂-C₆ alkynyl, C₃-C₁₀ cycloalkyl, C₆-C₁₀ aryl, 3- to 10-membered heterocycloalkyl and 5- to 10-membered heteroaryl, and the C₁-C₆ alkyl, the C₂-C₆ alkenyl, the C₂-C₆ alkynyl, the C₃-C₁₀ cycloalkyl, the C₆-C₁₀ aryl, the 3- to 10-membered heterocycloalkyl or the 5-to 10-membered heteroaryl is optionally substituted with 1 or more R^{d3} and/or R^{e3};
each R^{d3} is independently selected from -OR^{e3}, -NR^{e3}R^{e3}, halogen, -CN, -C(O)R^{e3}, -C(O)OR^{e3}, -C(O)NR^{e3}R^{e3}, - S(O)₂R^{e3}, -S(O)₂NR^{e3}R^{e3}, -NHC(O)R^{e3}, -N(C₁-C₄ alkyl)C(O)R^{e3} and oxo, and the substitution with the oxo is performed only on a non-aromatic ring;
each R^{e3} is independently selected from hydrogen, C₁-C₆ alkyl, C₂-C₆ alkenyl, C₂-C₆ alkynyl, C₃-C₁₀ cycloalkyl, C₆-C₁₀ aryl, 3- to 10-membered heterocycloalkyl and 5- to 10-membered heteroaryl;
R₃ is selected from hydrogen, C₁-C₄ alkyl, -O(C₁-C₄ alkyl), -NH₂, -NH(C₁-C₄ alkyl), -N(C₁-C₄ alkyl)₂ and halogen; R₅ is selected from hydrogen, hydroxy and -NHR';
R' is selected from hydrogen, C₁-C₃ alkyl and -C(O)C₁-C₃ alkyl;
R₄ is selected from C₁-C₄ alkyl, hydroxy, oxo, cyano, C₁-C₄ alkoxy, C₁-C₄ haloalkyl, C₁-C₄ hydroxyalkyl, hydroxy-C₁-C₄ haloalkyl, C₂-C₆ alkenyl, -C₂-C₆ alkynyl, C₃-C₆ cycloalkoxy, C₃-C₆ cycloalkyl, 3- to 6-membered heterocycloalkyl, 3- to 6-membered hydroxyheterocycloalkyl, halogen and -SO₂-C₁-C₄ alkyl;
R₆ is selected from hydrogen, C₁-C₄ alkyl and halogen;
or R₄ and R₆, together with the carbon atom linked thereto, form optionally substituted C₅-C₆ cycloalkyl and 5- to 6-membered heterocycloalkyl, wherein the heteroatom in the 5- to 6-membered heterocycloalkyl is selected from a heteroatom and a heteroatom group containing 1, 2, 3 or 4 heteroatoms or heteroatom groups independently selected from -NH, -O-, -S- and N;
R₇ and R₈ are each independently C₁-C₆ alkyl, C₁-C₆ alkoxy, C₃-C₆ cycloalkyl or C₃-C₆ cycloalkoxy, wherein the alkyl, the alkoxy, the cycloalkyl and the cycloalkoxy are optionally substituted with cyano, hydroxy, C₁-C₆ alkoxy, C₃-C₆ cycloalkyl, C₃-C₆ cycloalkoxy or halogen.

5. The compound of formula I, and/or the stereoisomer, the enantiomer, the diastereomer, the deuteride, the hydrate, the solvate, the prodrug and/or the pharmaceutically acceptable salt thereof according to claim 4, wherein R₁ is hydrogen or -OR^{A};
R^{A} is hydrogen;
R₂ is hydrogen, C₁-C₄ alkyl or -O(C₁-C₄ alkyl), wherein the -O(C₁-C₄ alkyl) is optionally substituted with -O(C₁-C₄ alkyl);
R₃ is hydrogen;
R₅ is hydrogen or -NHR';
R' is selected from hydrogen, C₁-C₃ alkyl and -C(O)C₁-C₃ alkyl;
R₄ is C₁-C₄ haloalkyl;
R₆ is hydrogen or halogen;
or R₄ and R₆, together with the carbon atom linked thereto, form a ring such that the fragment 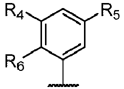 in S6 is 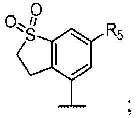
R₇ is C₁-C₆ alkyl;
R₈ is C₁-C₆ alkyl.

6. The compound of formula I, and/or the stereoisomer, the enantiomer, the diastereomer, the deuteride, the hydrate, the solvate, the prodrug and/or the pharmaceutically acceptable salt thereof according to claim 4 or 5, wherein the 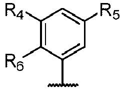 fragment in each S structure is 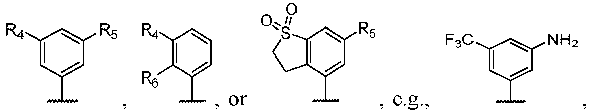 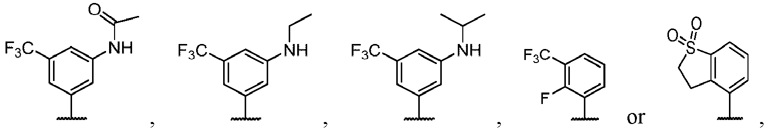 preferably 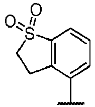 or 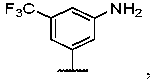 , and further preferably 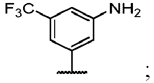 and/or, R₃ is hydrogen; and/or, R₇ is methyl.

7. The compound of formula I, and/or the stereoisomer, the enantiomer, the diastereomer, the deuteride, the hydrate, the solvate, the prodrug and/or the pharmaceutically acceptable salt thereof according to claim 4, wherein S6, S6', S6a or S6c is of any one of the following structures: 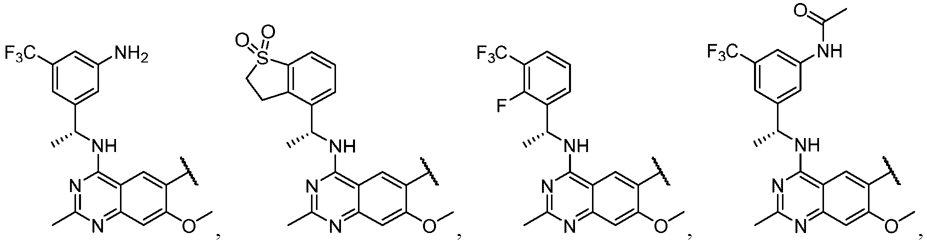 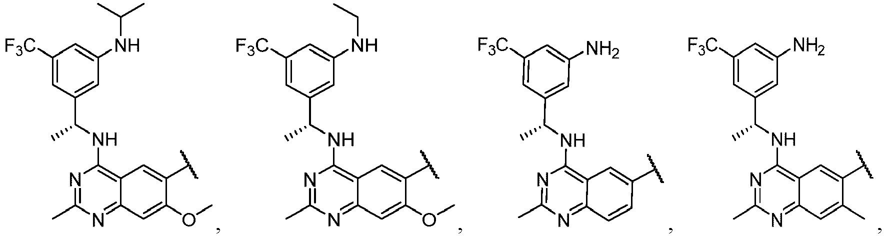 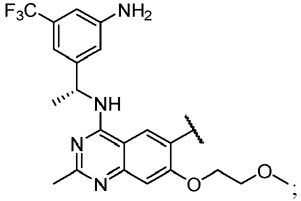 and/or, S6", S6‴, S6a" or S6c" is of the following structure: 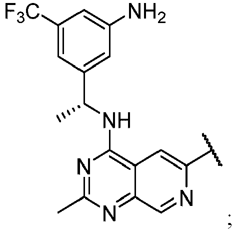 preferably, S is of the following structure: 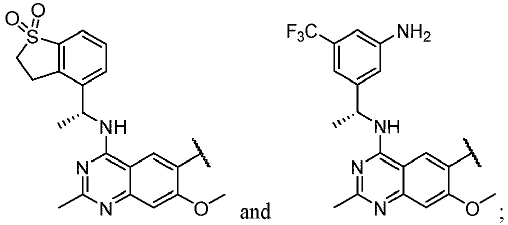 further preferably, S is of the following structure:

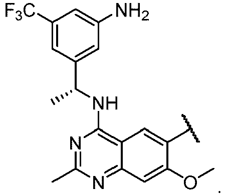

8. The compound of formula I, and/or the stereoisomer, the enantiomer, the diastereomer, the deuteride, the hydrate, the solvate, the prodrug and/or the pharmaceutically acceptable salt thereof according to any one of claims 1-7, wherein L is -(CH₂)ⱼ-, 1 or more methylene groups in the -(CH₂)ⱼ- are optionally replaced by a group selected from -NR^{3'}-, -O-, -CR^{1'}R^{2'}-, -C(O)-, -S(O)-, -S(O)₂-, -C(O)O-, -OC(O)-, -C(O)NR^{3'}-, -NR^{3'}C(O)-, -S(O)₂NR^{3'}-, - NR^{3'}S(O)₂-, ethenylene, ethynylene, phenyl, 8- to 10-membered bicyclic arylene, 3- to 7-membered saturated or partially unsaturated cycloalkylene, 5- to 11-membered saturated or partially unsaturated spirocycloalkylene, 5- to 11-membered saturated or partially unsaturated fused cycloalkylene, 8- to 10-membered bicyclic saturated or partially unsaturated cycloalkylene, 4- to 7-membered saturated or partially unsaturated heterocycloalkylene having 1-2 heteroatoms independently selected from nitrogen, oxygen and sulfur, 5- to 11-membered saturated or partially unsaturated spiroheterocycloalkylene having 1-2 heteroatoms independently selected from nitrogen, oxygen and sulfur, 5- to 11-membered saturated or partially unsaturated fused heterocycloalkylene having 1-2 heteroatoms independently selected from nitrogen, oxygen and sulfur, 8- to 10-membered bicyclic saturated or partially unsaturated heterocycloalkylene having 1-2 heteroatoms independently selected from nitrogen, oxygen and sulfur, 5- to 6-membered heteroarylene having 1-4 heteroatoms independently selected from nitrogen, oxygen and sulfur, and 8- to 10-membered bicyclic heteroaryl having 1-5 heteroatoms selected from nitrogen, oxygen and sulfur, wherein the ethenylene, the ethynylene, the cycloalkylene, the heterocycloalkylene, the phenyl, the spiroheterocycloalkylene, the fused heterocycloalkylene, the spirocycloalkylene, the fused cycloalkylene and the heteroarylene are each independently optionally substituted with 1 or more substituents selected from halogen, oxo, -NR^{3'}R^{4'}, -OR^{3'}, nitro, -CN, C₁-C₆ alkyl, C₃-C₁₀ cycloalkyl and C₃-C₁₀ heterocycloalkyl, wherein the alkyl, the cycloalkyl and the heterocycloalkyl are optionally substituted with 1 or more substituents selected from halogen, - OH, -NH₂, -CN, C₁-C₄ alkyl and C₃-C₆ cycloalkyl; R^{1'} and R^{2'} are each independently halogen, -OH, -NH₂, C₁-C₄ alkyl, C₁-C₄ chloroalkyl, C₁-C₄ hydroxyalkyl, -O(C₁-C₄ alkyl), -NH(C₁-C₄ alkyl), C₃-C₆ cycloalkyl, -O(C₃-C₆ cycloalkyl), -NH(C₃-C₆ cycloalkyl), C₃-C₆ heterocycloalkyl, -O(C₃-C₆ heterocycloalkyl) or -NH(C₃-C₆ cycloalkyl); R^{3'} and R^{4'} are each independently hydrogen, deuterium, C₁-C₄ alkyl, C₃-C₆ cycloalkyl, or C₃-C₆ heterocycloalkyl, and j is 2, 3, 4, 5, 6, 7, 8, 9, 10, 11 or 12;
preferably, L is -(CH₂)ⱼ-, 1, 2, 3, 4 or 5 methylene groups in the -(CH₂)ⱼ- are optionally replaced by a group selected from -NH-, -NCH₃-, -O-, -C(CH₃)₂-, -CHF-, -CHCF₃-, -C(O)-, -C(O)O-, -OC(O)-, -C(O)NH-, -C(O)NCH₃-, - NHC(O)-, -NCH₃C(O)-, ethenylene, ethynylene, cyclopropylene, cyclobutylene, cyclopentylene, cyclohexylene, oxiranylidene, oxetanylidene, oxolanylidene, oxanylidene, aziridinylidene, azetidinylidene, azolidinylidene, piperidinylidene, piperazinylidene, morpholinylidene, homomorpholinylidene, phenylene, pyrrolylidene, thienylidene, furylidene, imidazolylidene, pyrazolylidene, triazolylidene, tetrazolylidene, oxazolylidene, isoxazolylidene, thiazolylidene, isothiazolylidene, pyridinylidene, pyrimidinylidene, pyridazinylidene, pyrazinylidene, 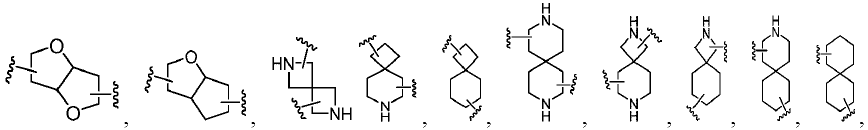 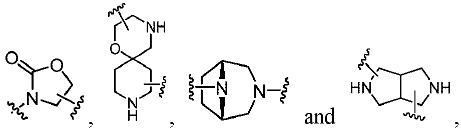 wherein the group for the replacement is optionally substituted with 1 or more substituents selected from halogen, oxo, -NR^{3'}R^{4'}, -OR^{3'} and C₁-C₄ alkyl, wherein the alkyl is optionally substituted with 1 or more substituents selected from halogen, -OH and -NH₂, R^{3'} and R^{4'} are each independently hydrogen, deuterium or C₁-C₄ alkyl, and j is 2, 3, 4, 5, 6, 7, 8, 9 or 10; preferably, L is -(CH₂)ⱼ-, wherein 1, 2 or 3, or 4 or 5 methylene groups in the -(CH₂)ⱼ- are optionally replaced by a group selected from -NH-, -NCH₃-, -O-, -C(O)-, -C(O)NH-, -NHC(O)-, -NCH₃C(O)-, -C(O)NCH₃-, cyclopropylene, cyclobutylene, cyclopentylene, cyclohexylene, aziridinylidene, azetidinylidene, azolidinylidene, piperidinylidene, piperazinylidene, 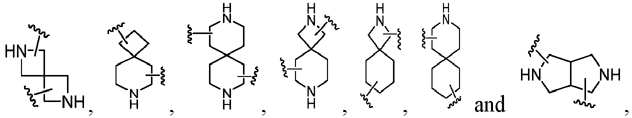 and j is 5, 6, 7, 8, 9 or 10.

9. The compound of formula I, and/or the stereoisomer, the enantiomer, the diastereomer, the deuteride, the hydrate, the solvate, the prodrug and/or the pharmaceutically acceptable salt thereof according to claim 8, wherein L is:

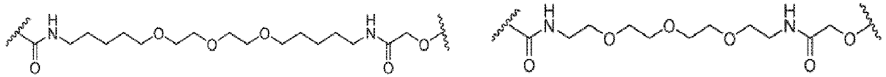

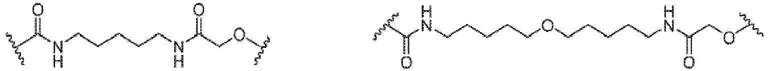

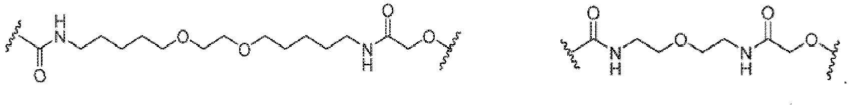

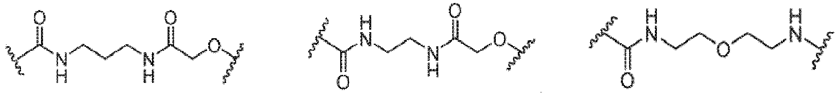

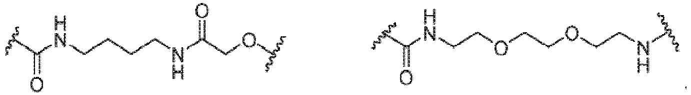

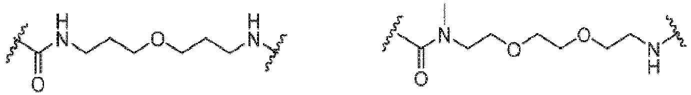

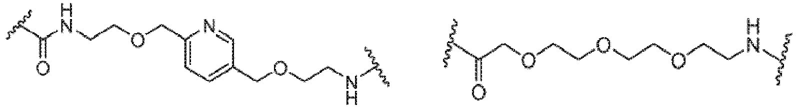

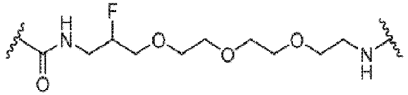

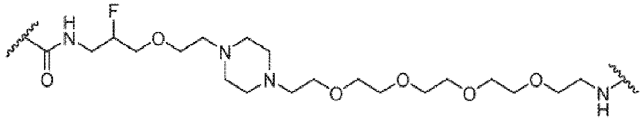

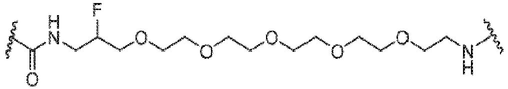

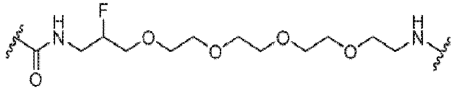

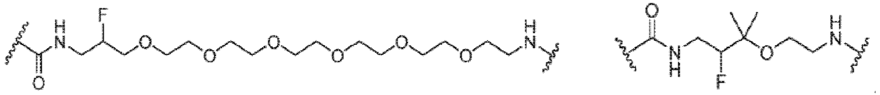

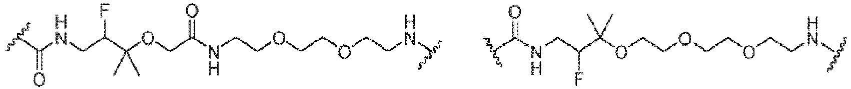

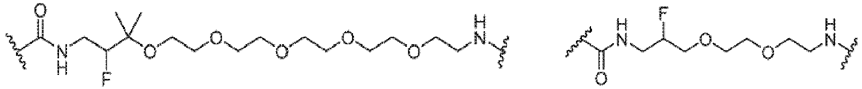

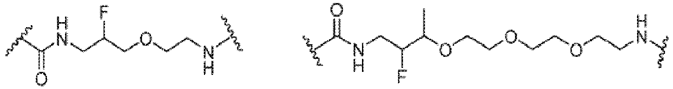

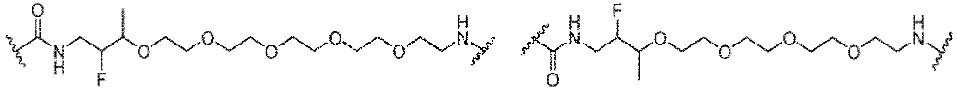

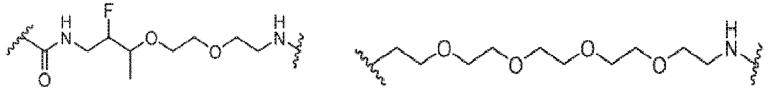

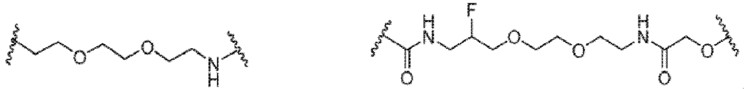

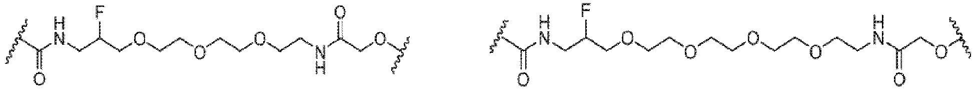

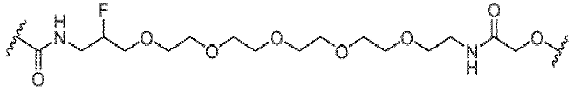

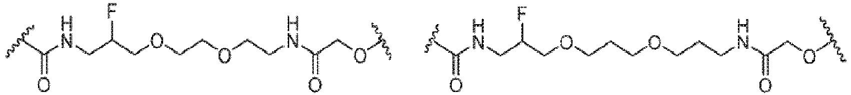

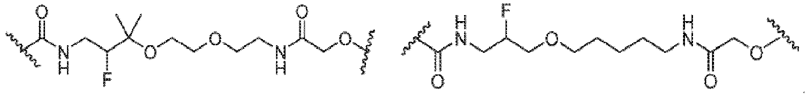

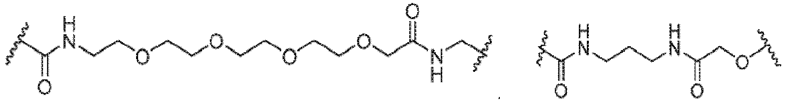

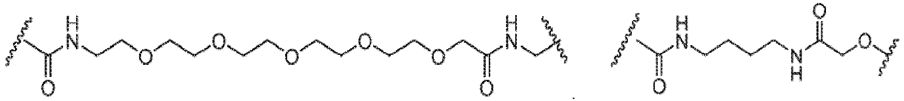

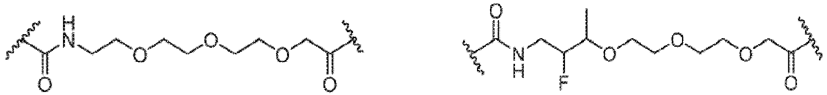

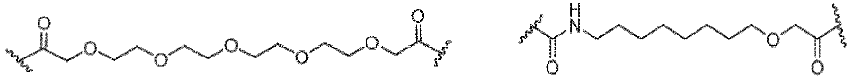

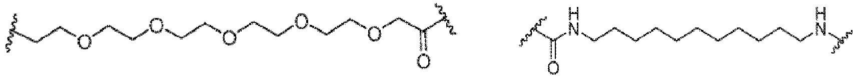

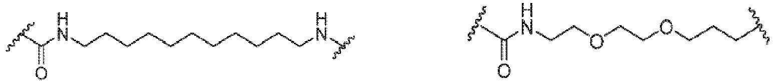

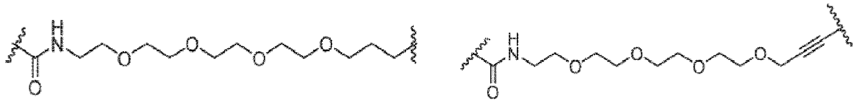

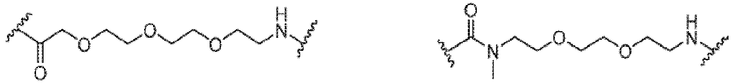

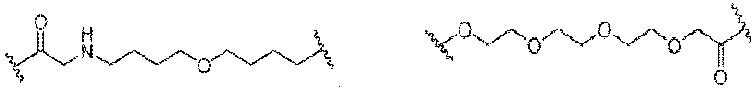

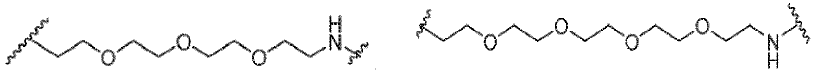

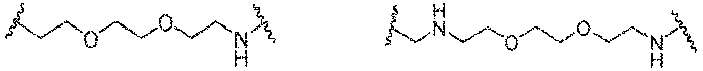

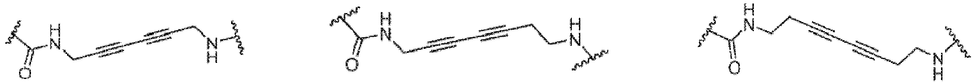

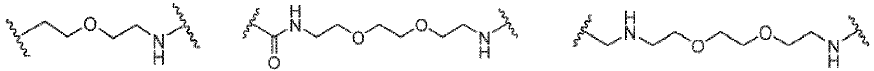

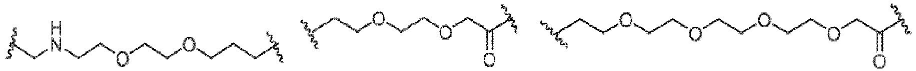

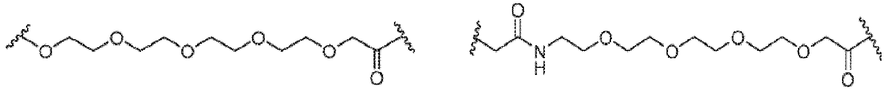

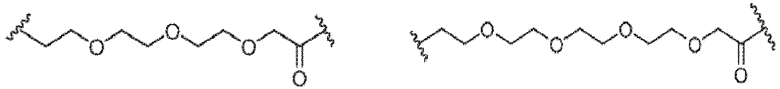

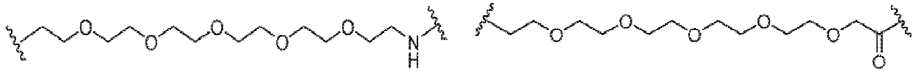

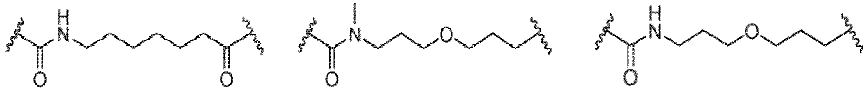

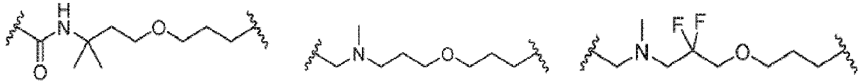

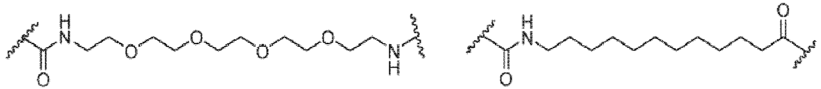

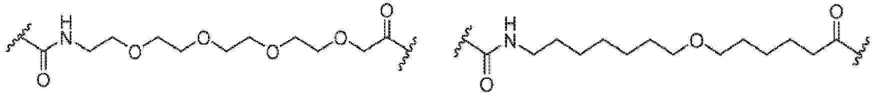

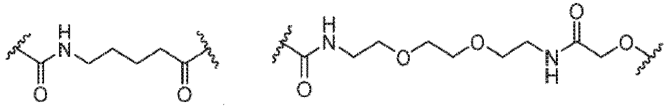

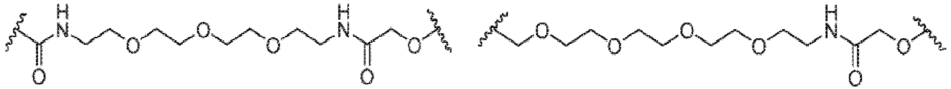

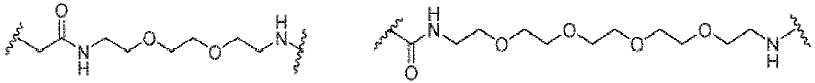

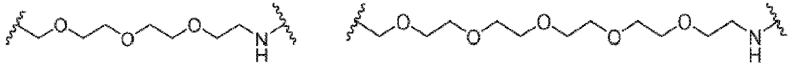

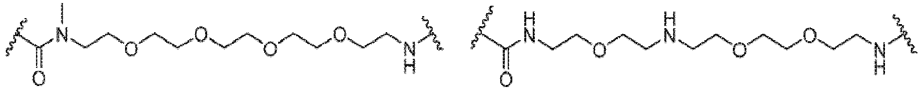

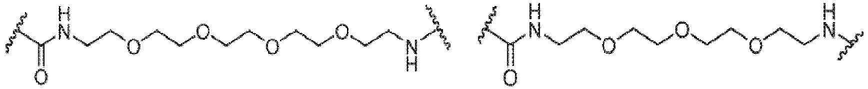

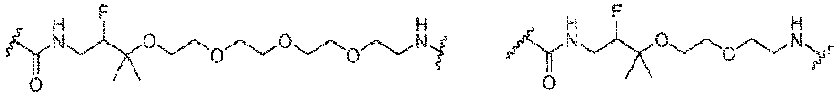

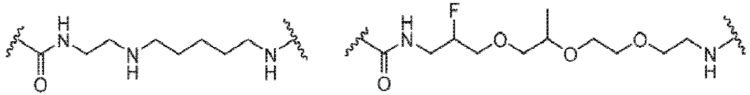

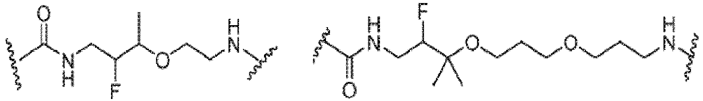

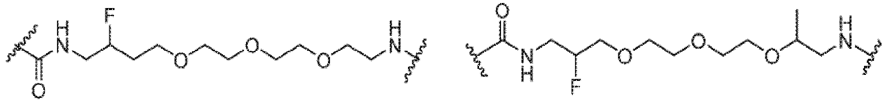

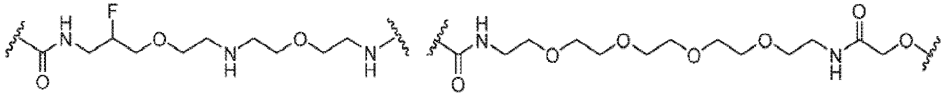

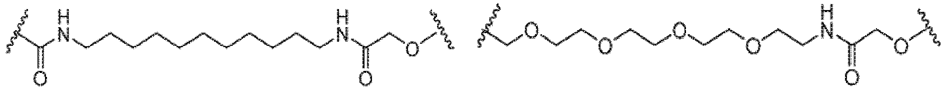

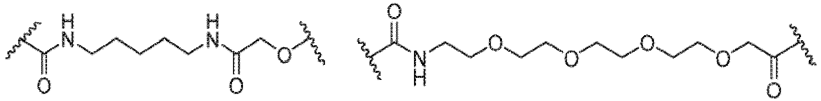

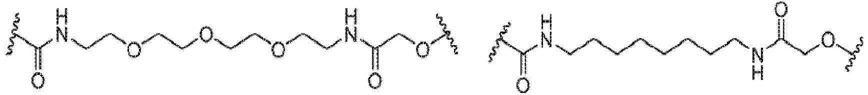

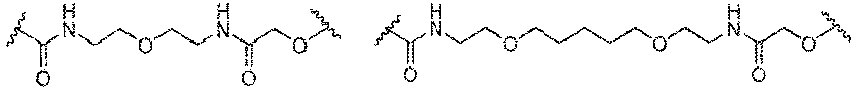

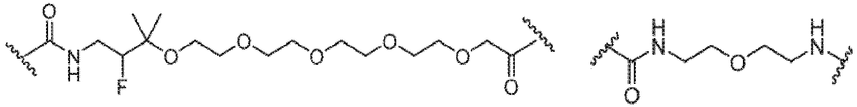

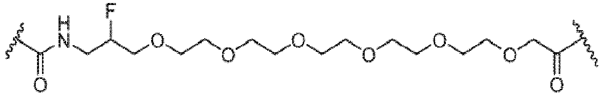

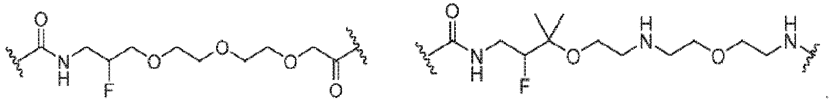

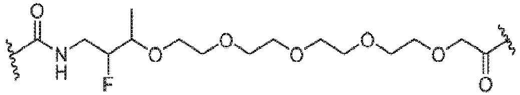

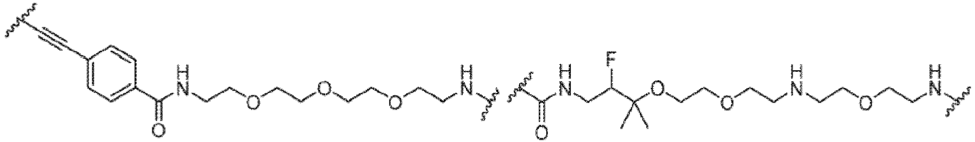

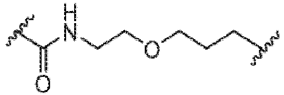

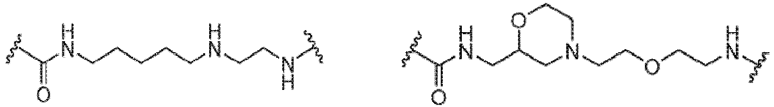

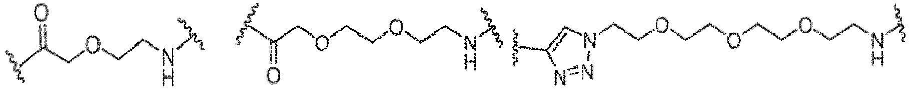

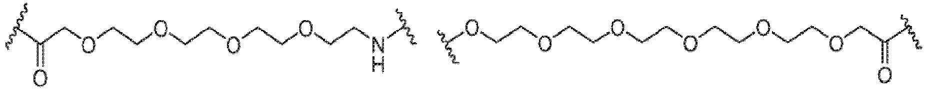

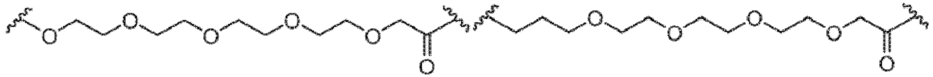

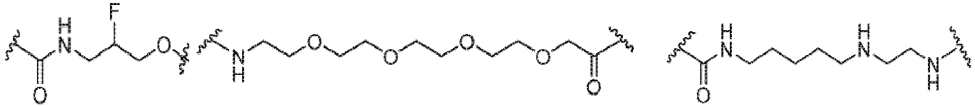

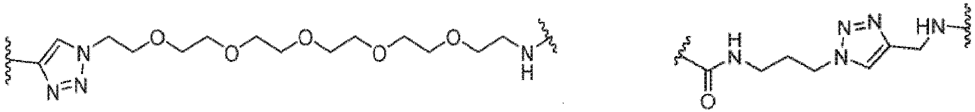

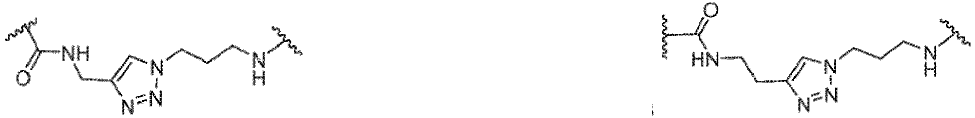

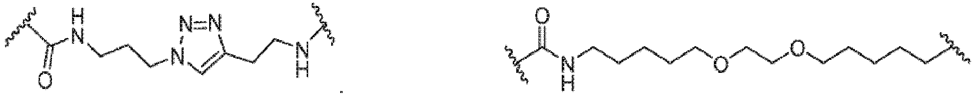

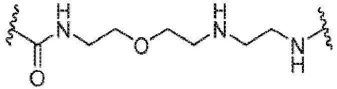

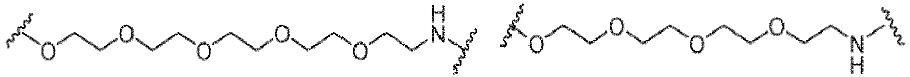

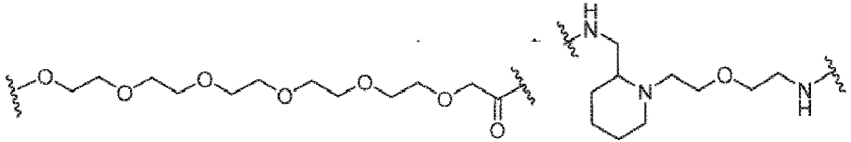

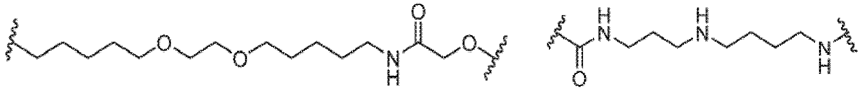

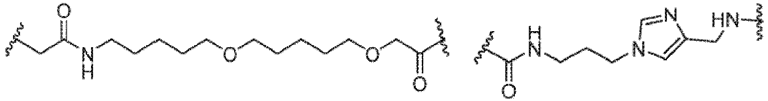

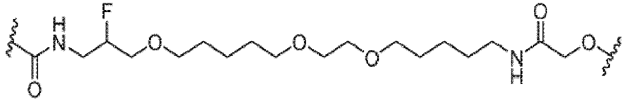

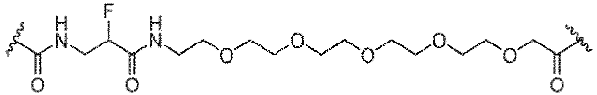

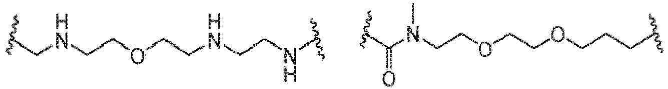

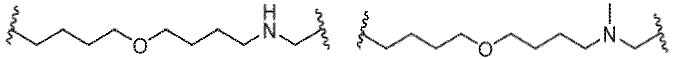

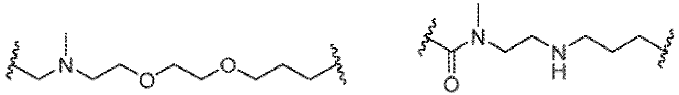

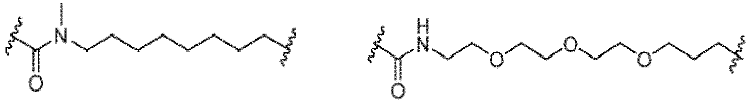

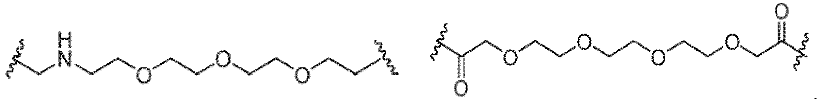

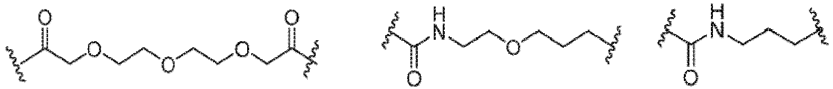

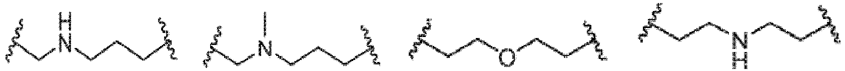

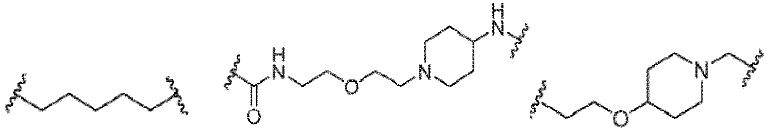

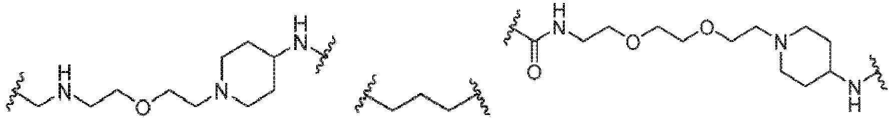

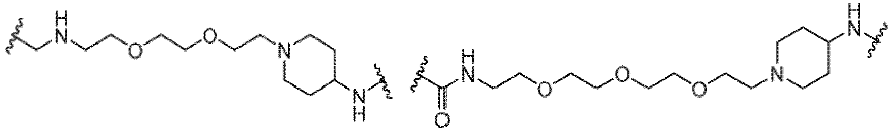

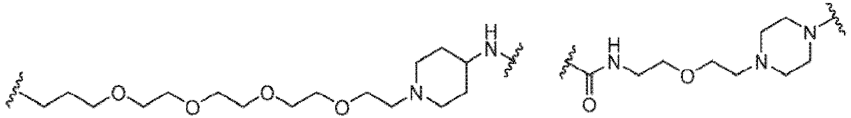

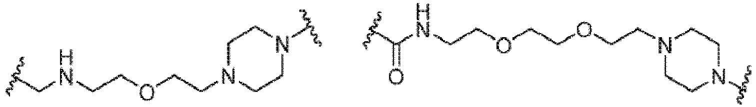

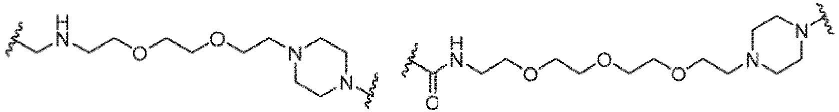

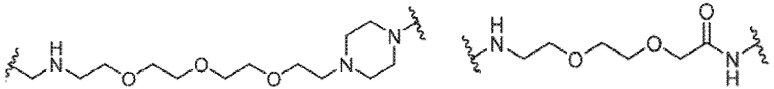

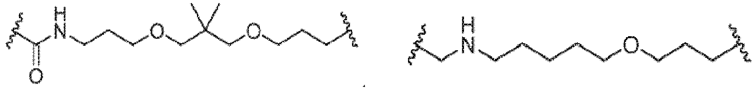

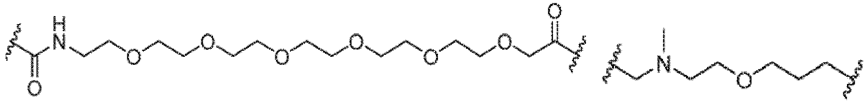

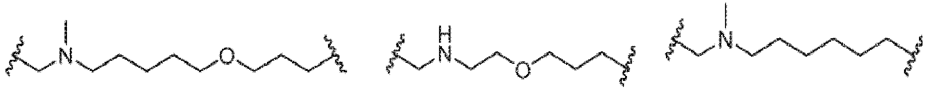

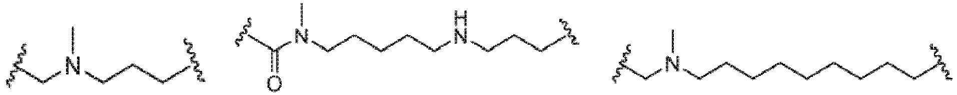

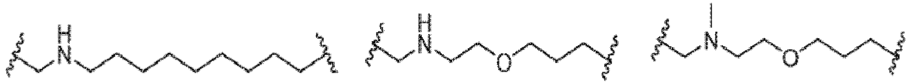

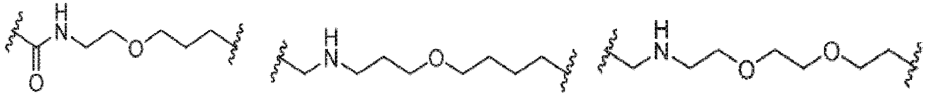

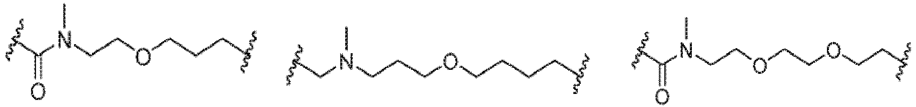

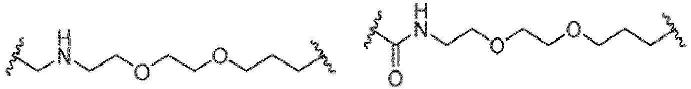

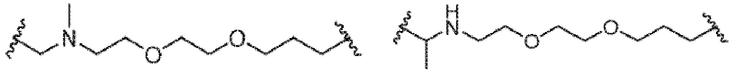

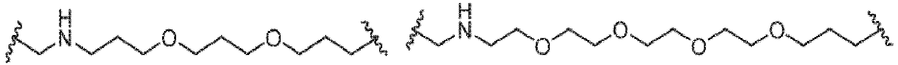

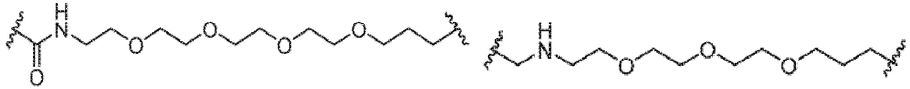

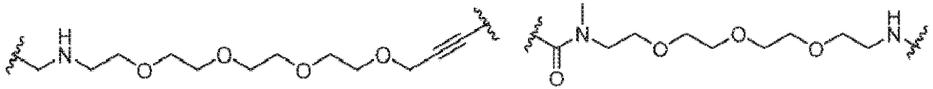

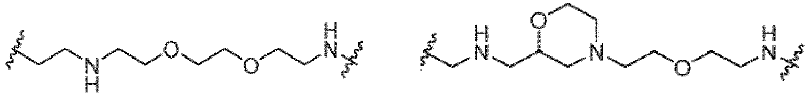

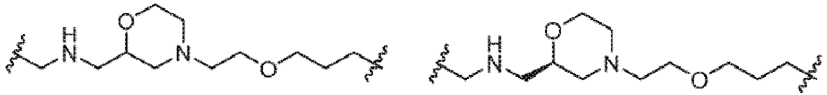

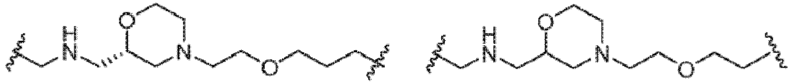

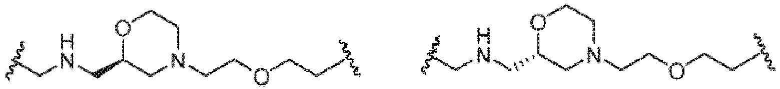

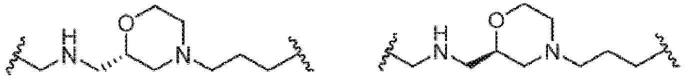

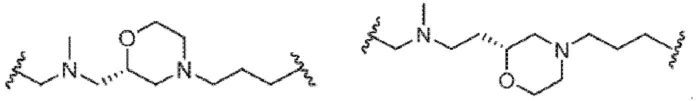

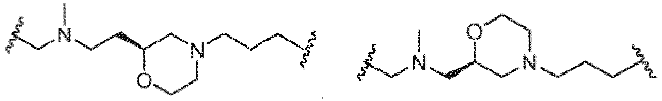

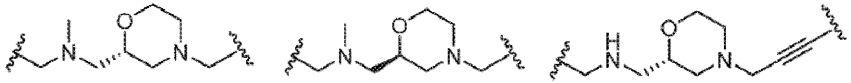

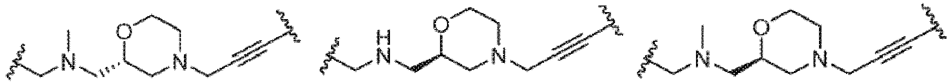

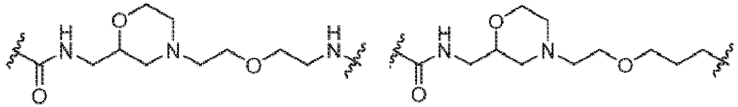

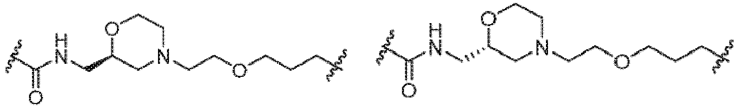

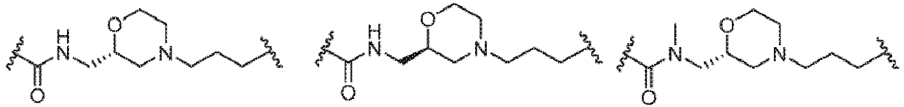

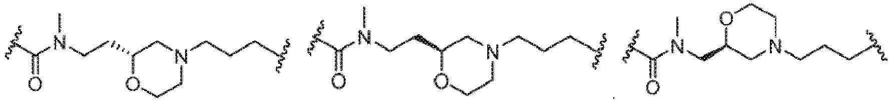

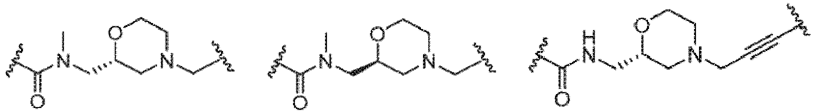

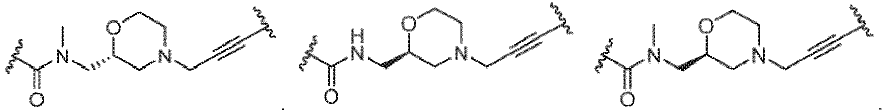

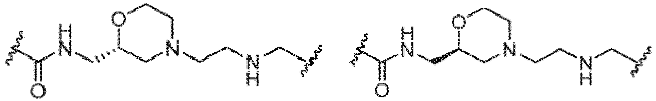

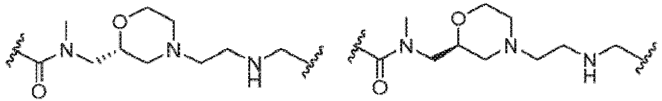

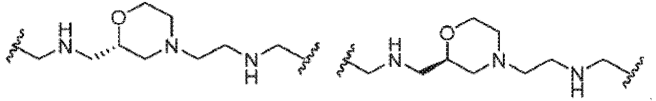

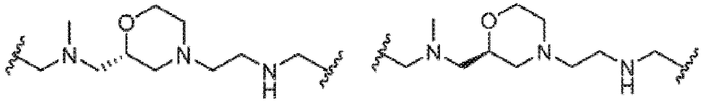

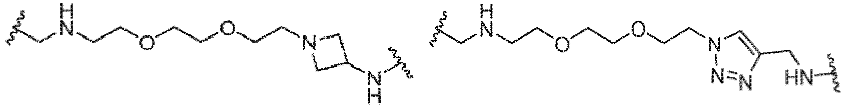

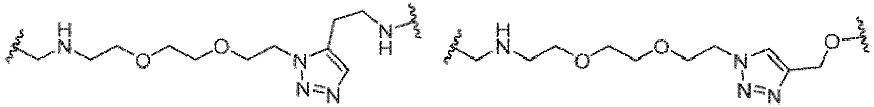

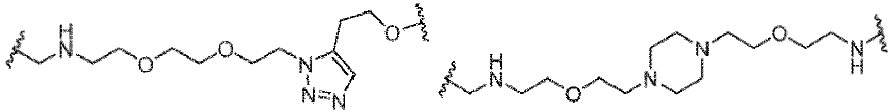

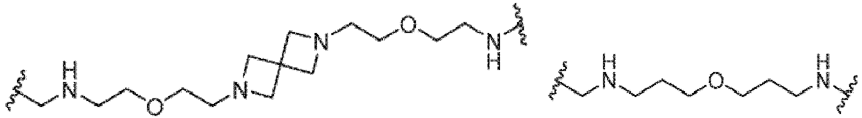

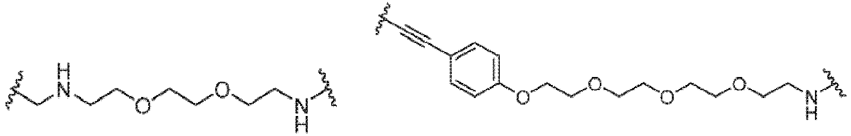

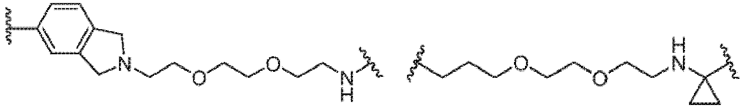

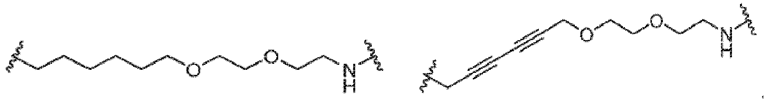

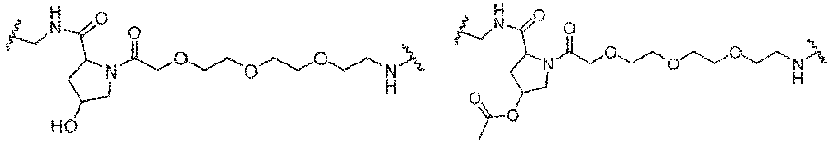

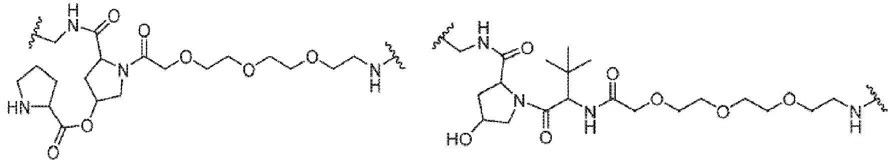

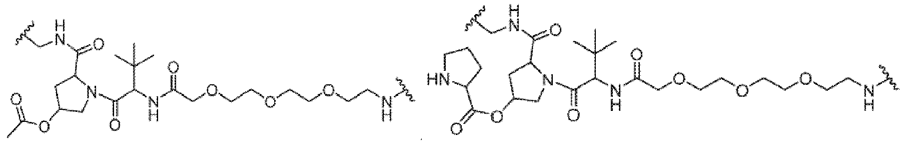

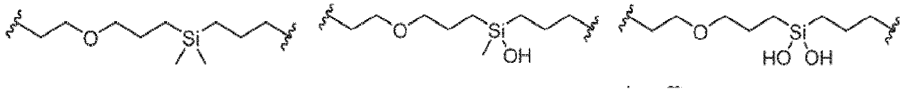

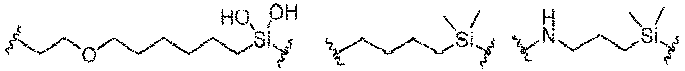

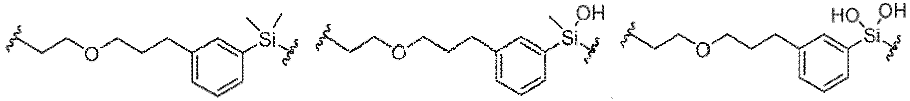

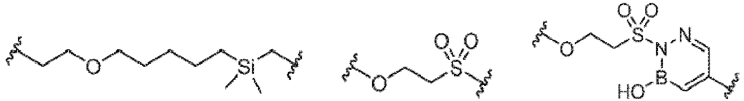

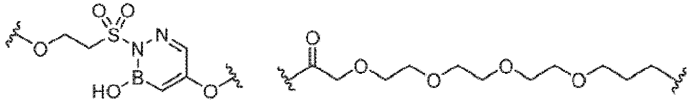

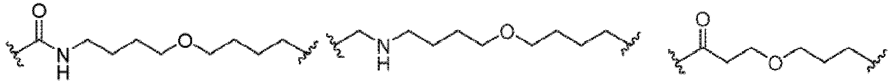

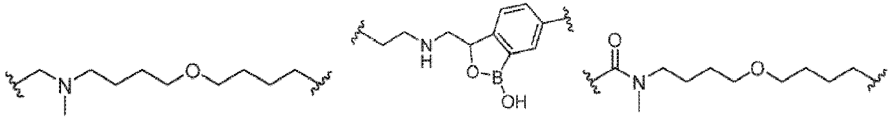

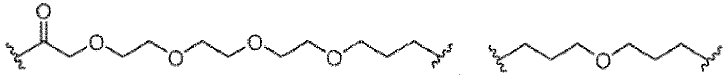

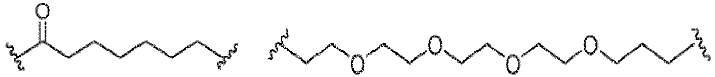

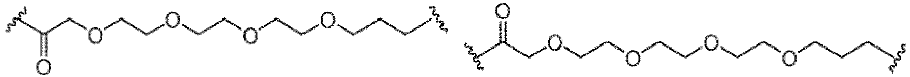

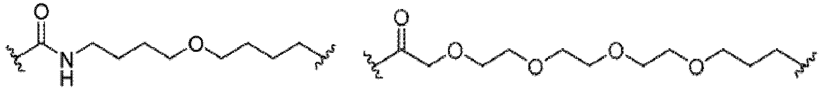

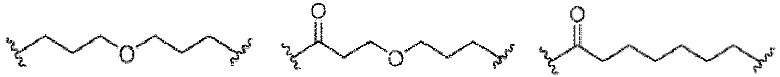

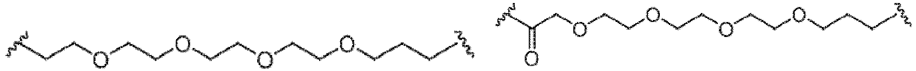

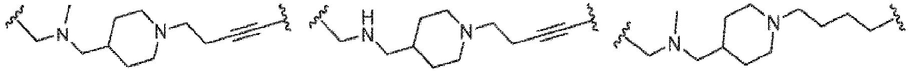

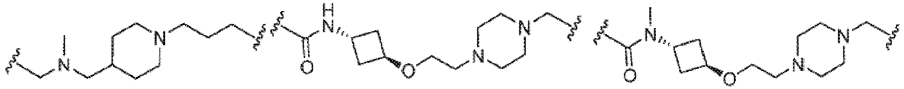

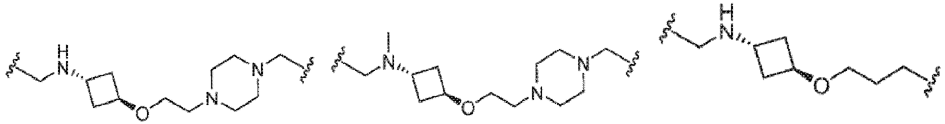

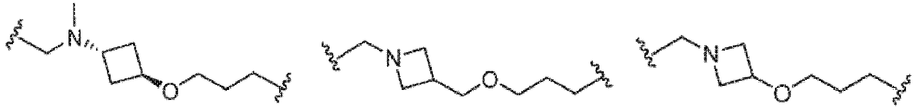

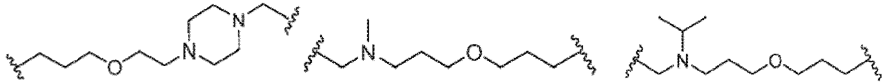

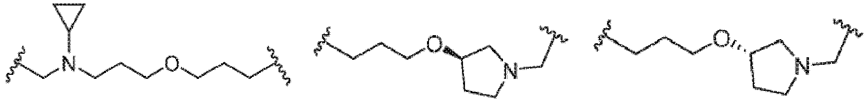

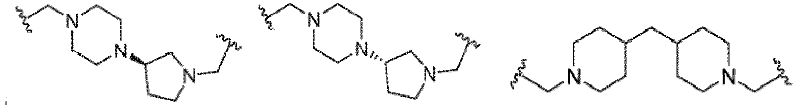

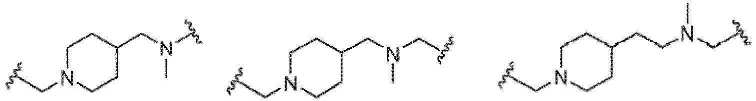

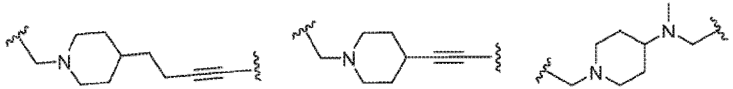

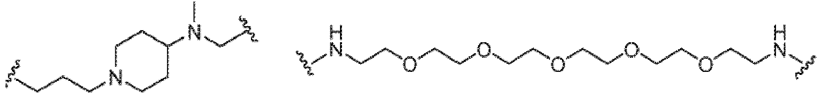

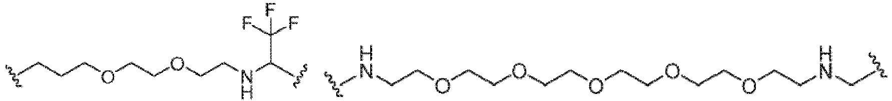

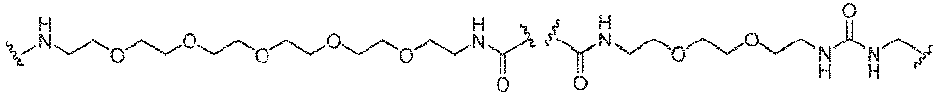

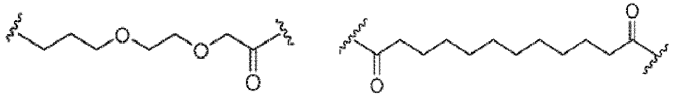

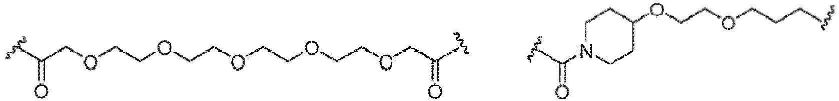

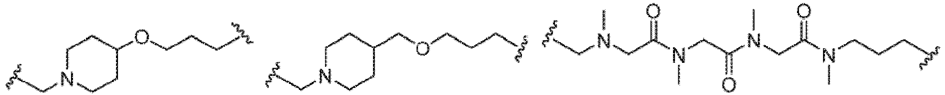

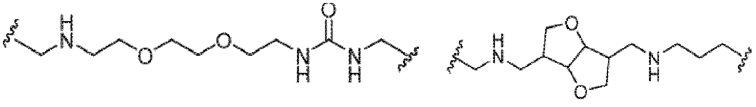

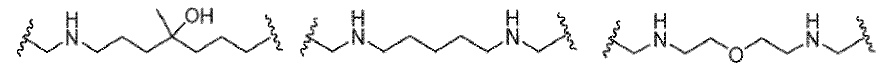

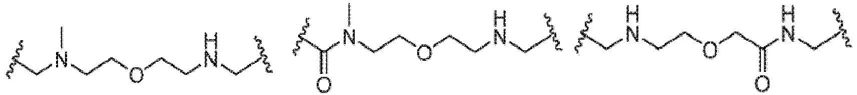

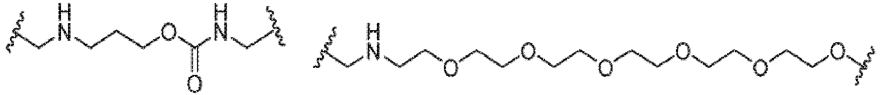

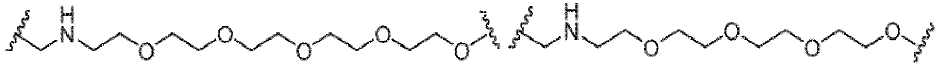

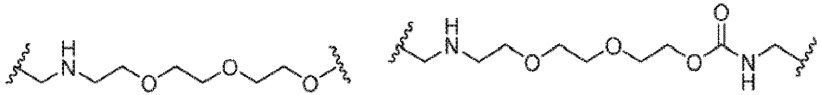

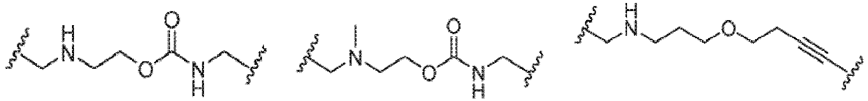

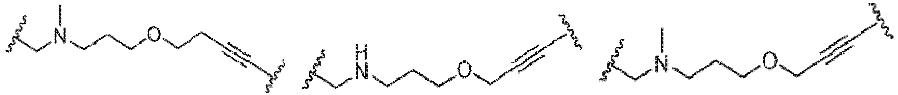

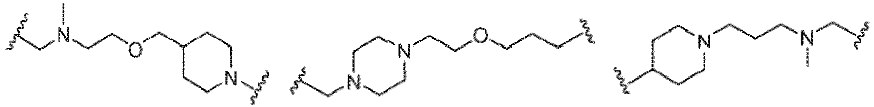

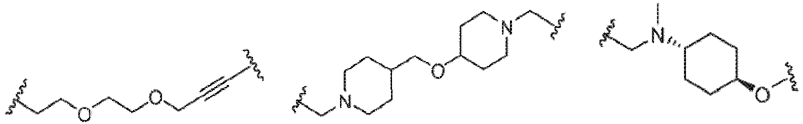

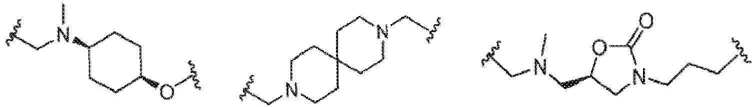

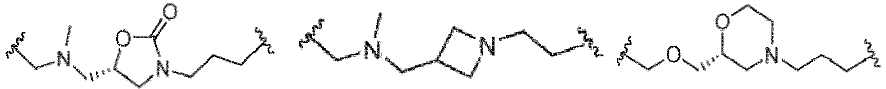

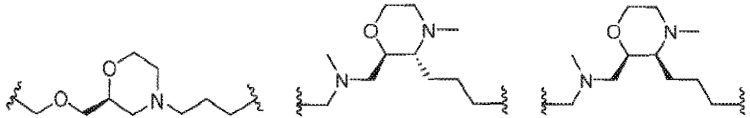

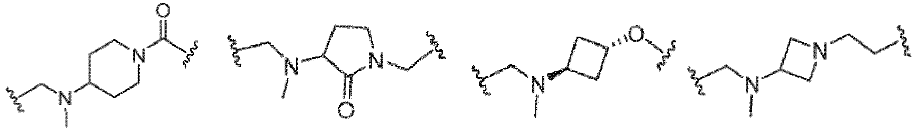

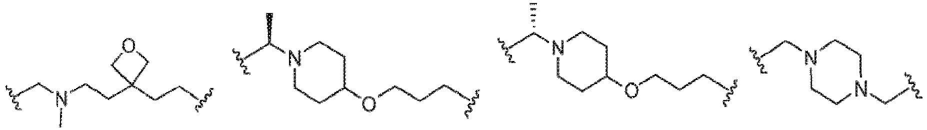

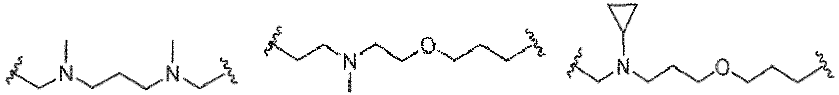

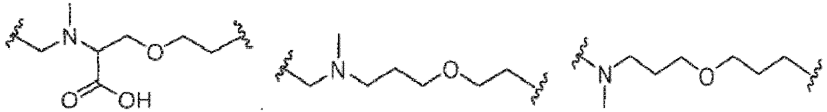

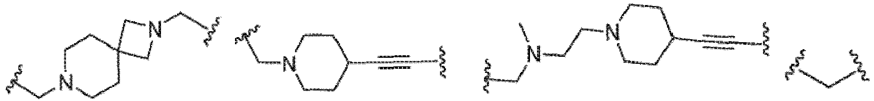

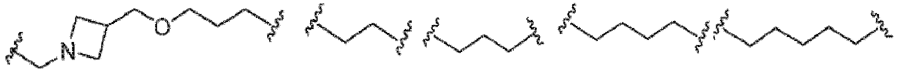

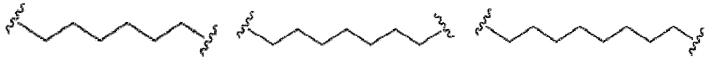

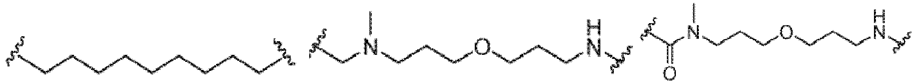

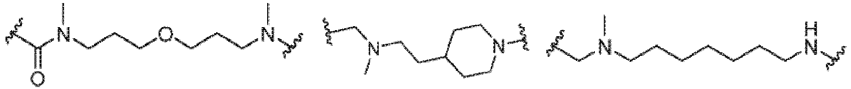

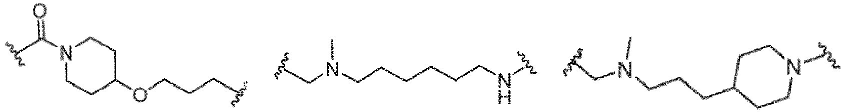

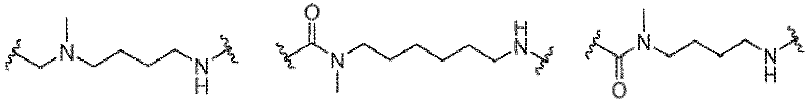

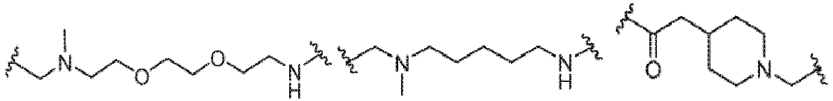

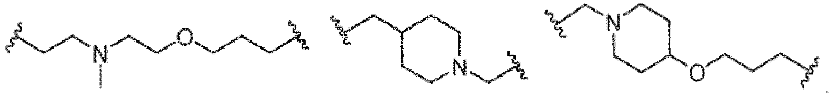

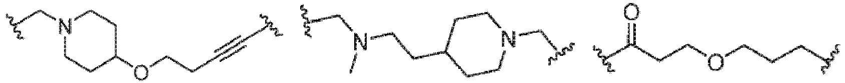

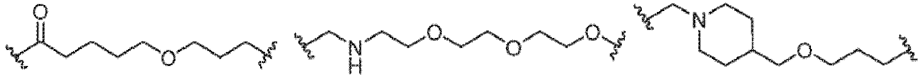

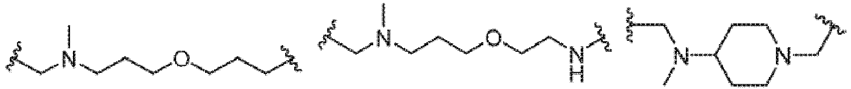

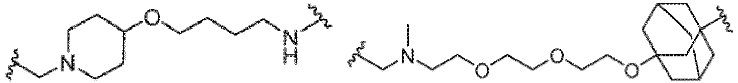

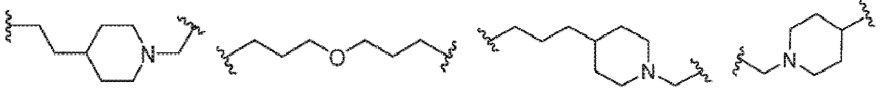

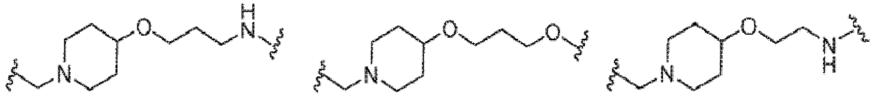

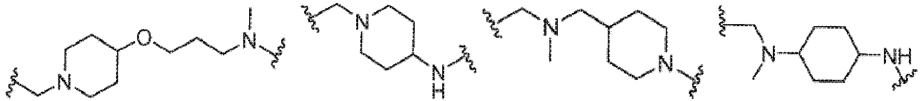

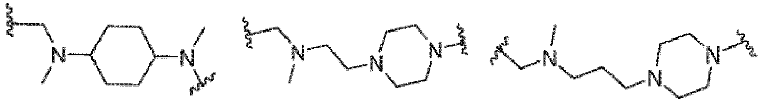

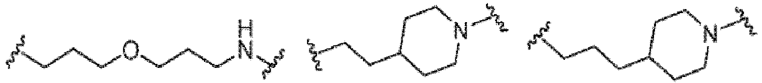

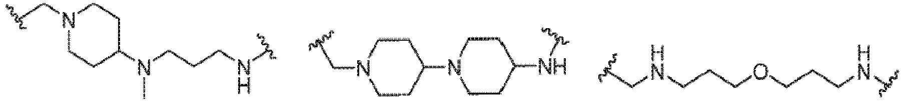

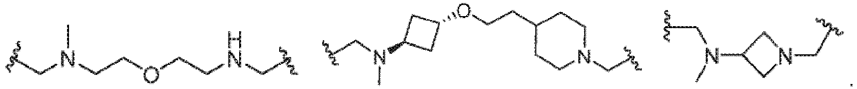

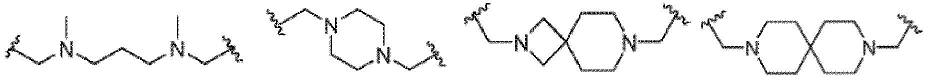

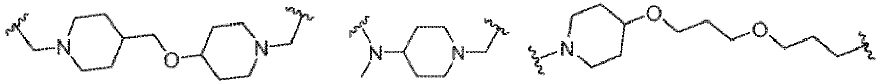

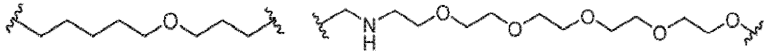

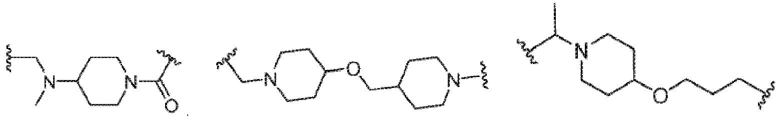

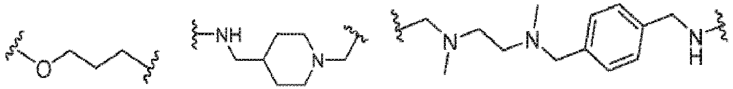

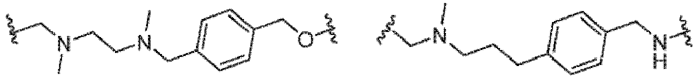

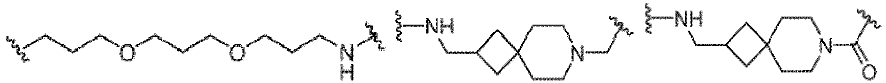

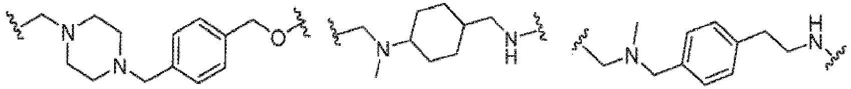

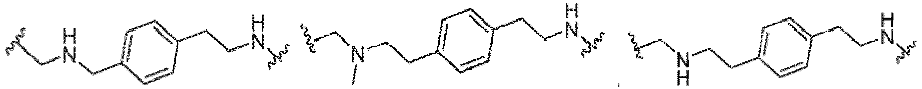

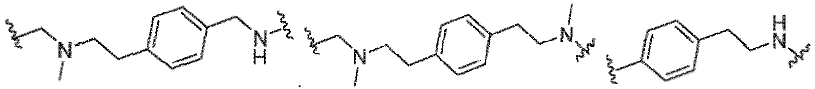

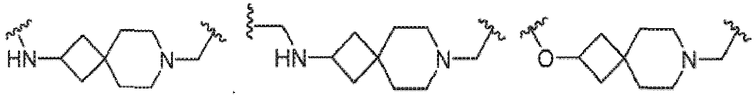

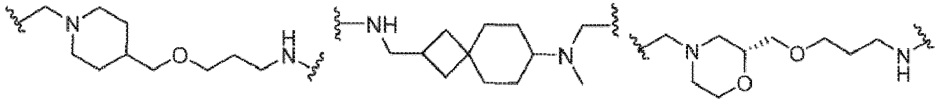

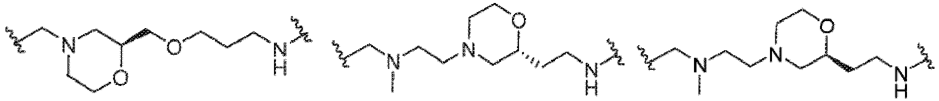

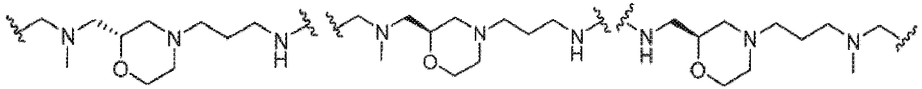

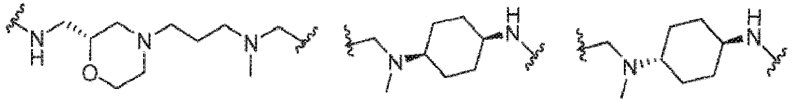

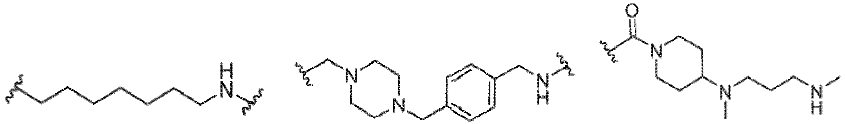

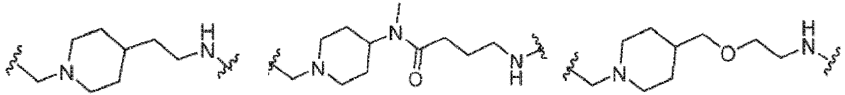

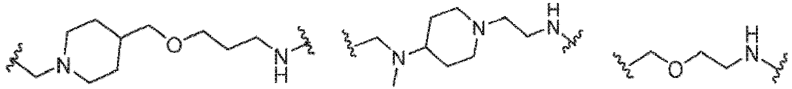

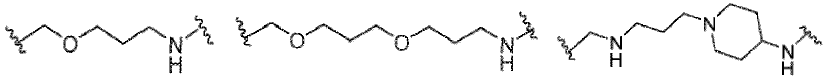

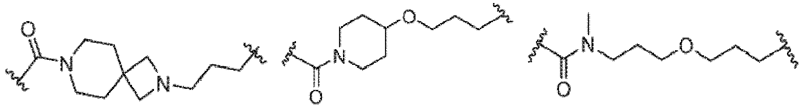

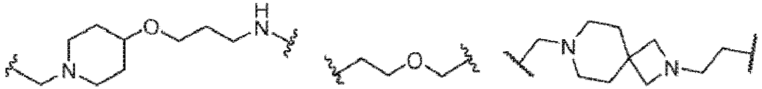

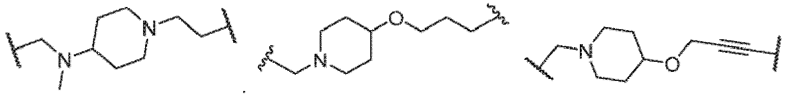

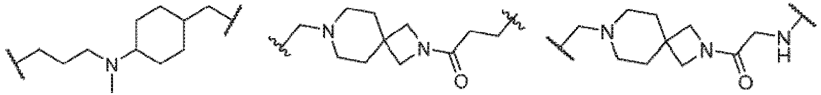

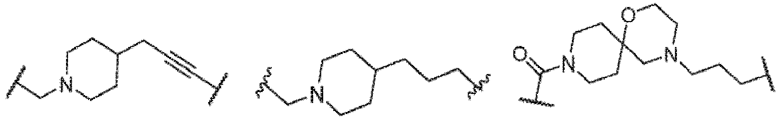

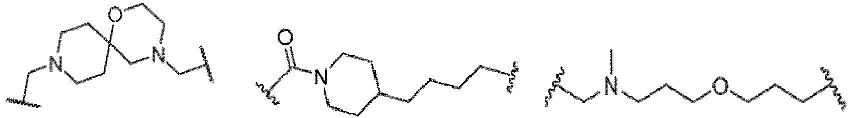

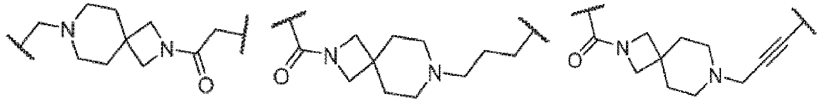

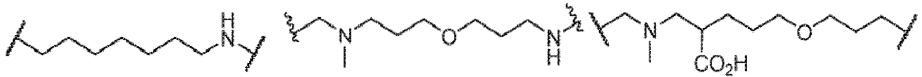

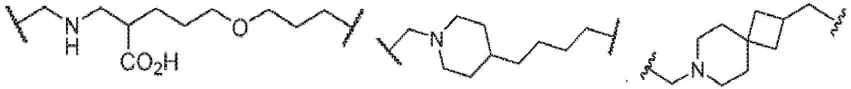

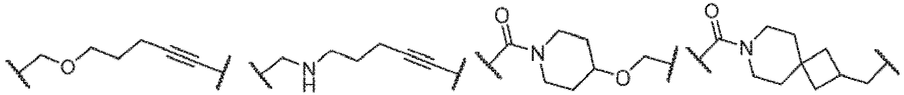

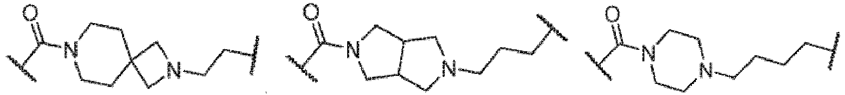

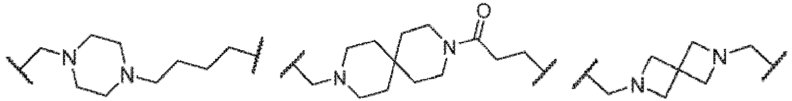

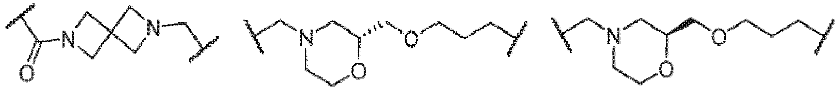

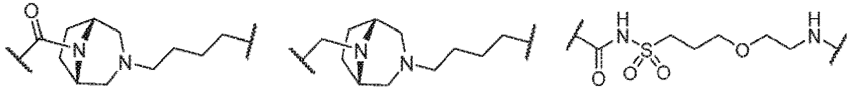

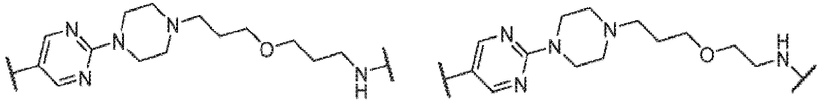

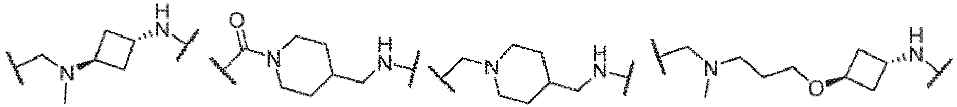

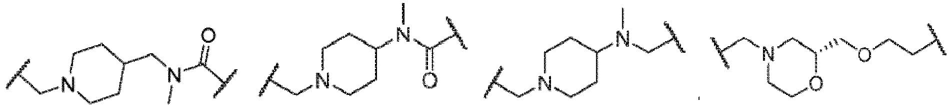

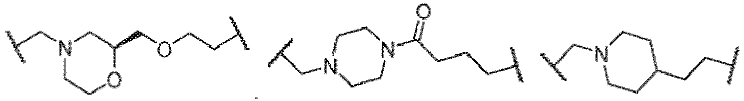

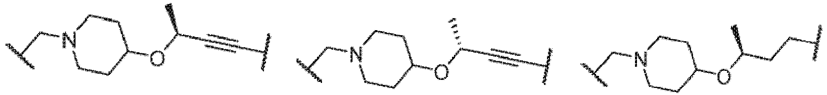

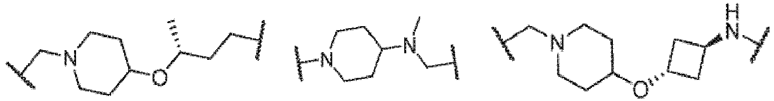

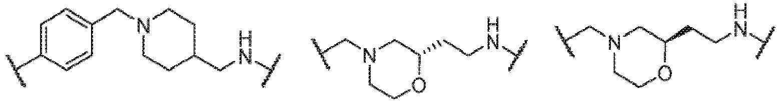

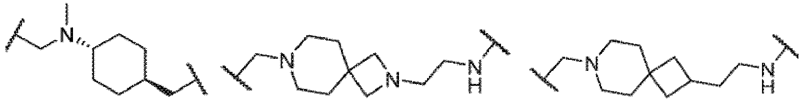

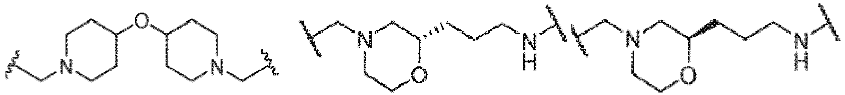

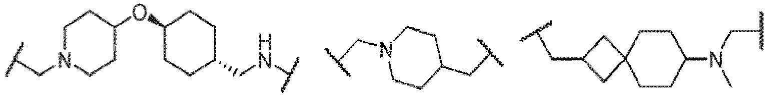

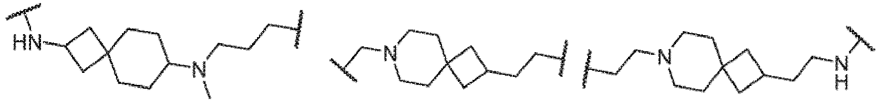

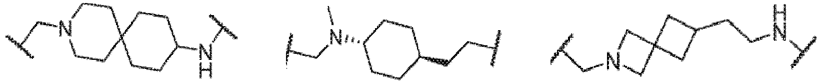

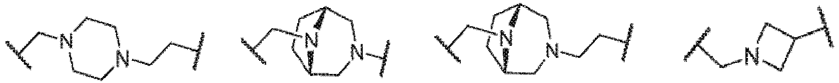

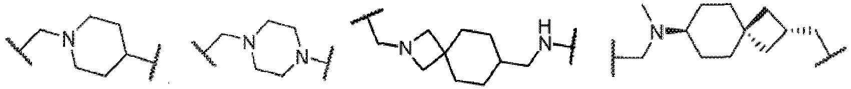

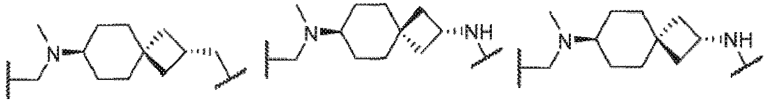

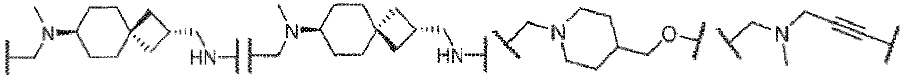

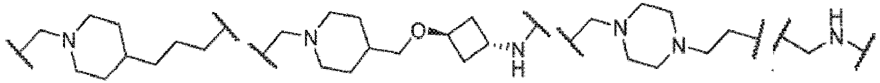

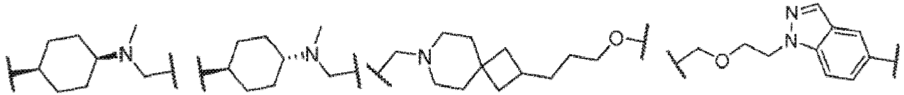

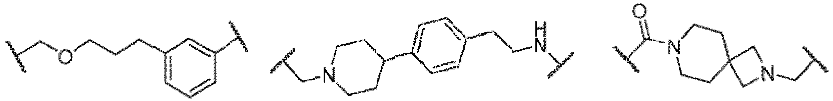

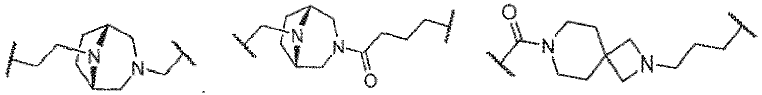

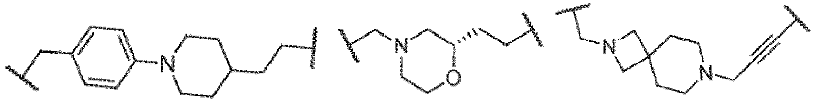

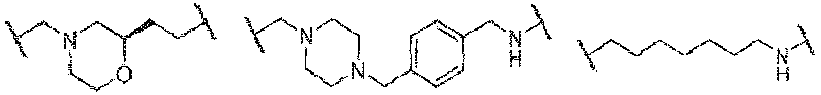

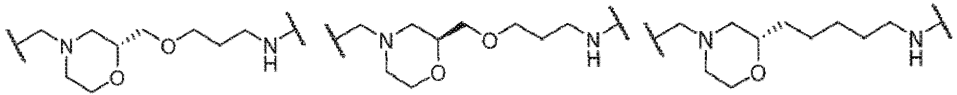

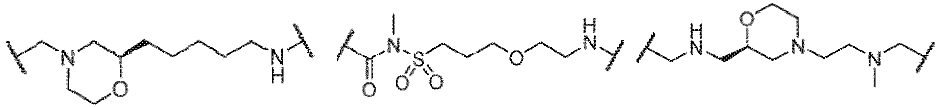

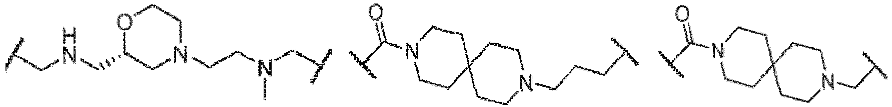

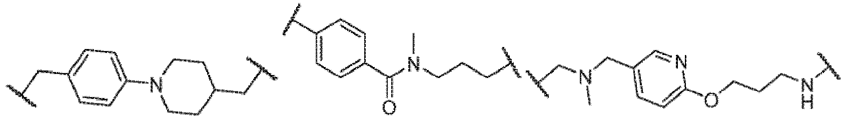

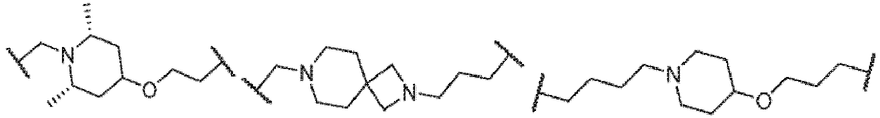

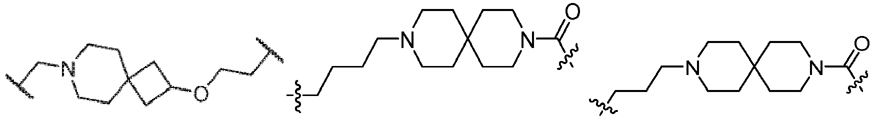

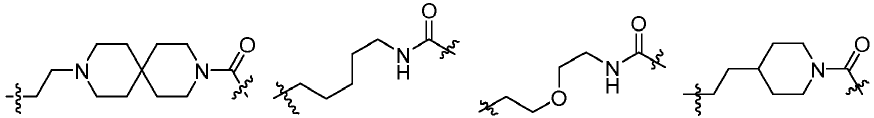

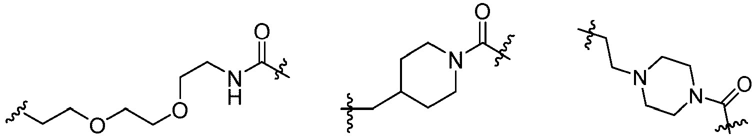

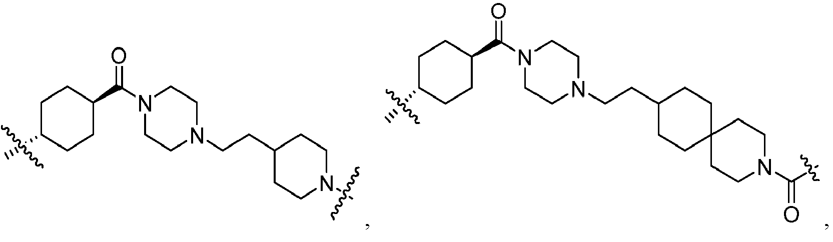

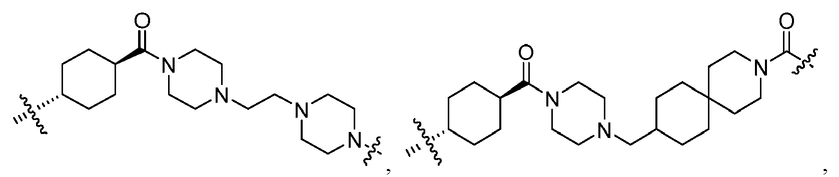

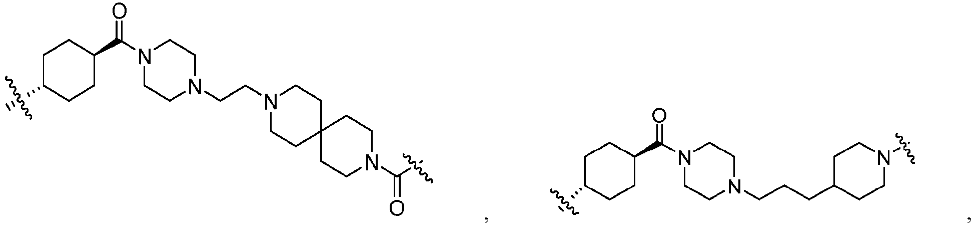

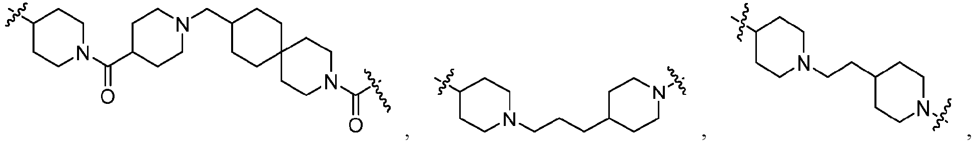

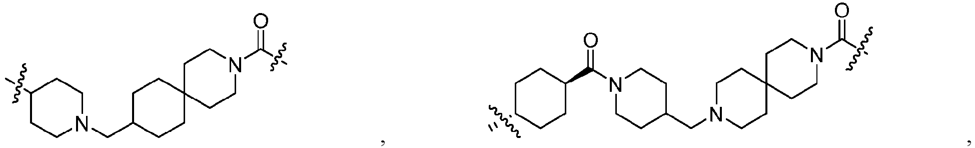

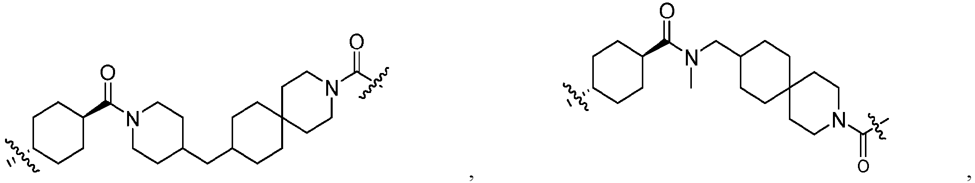

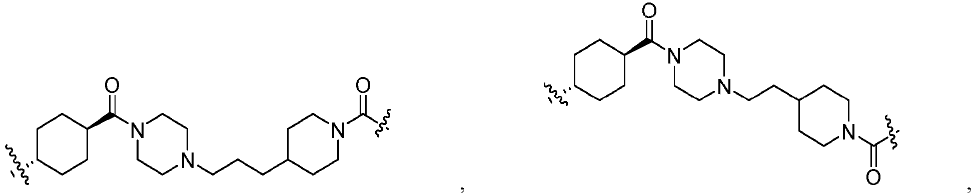

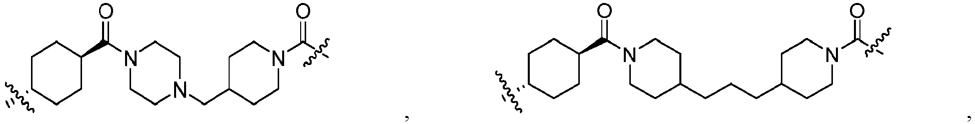

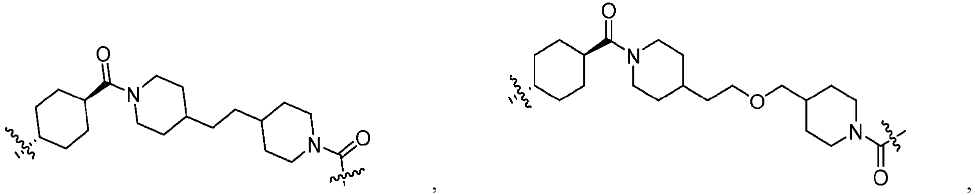

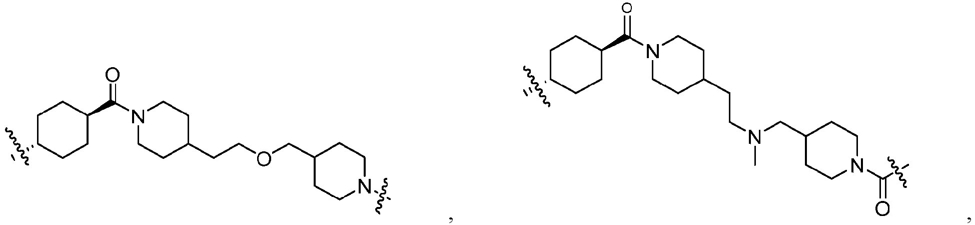

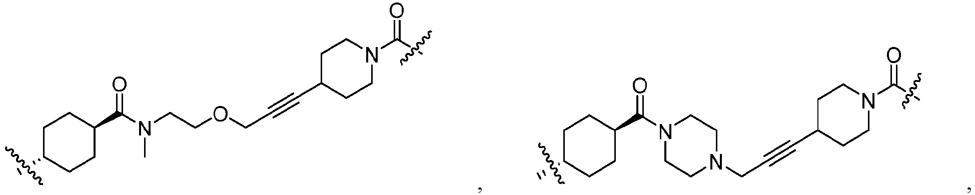

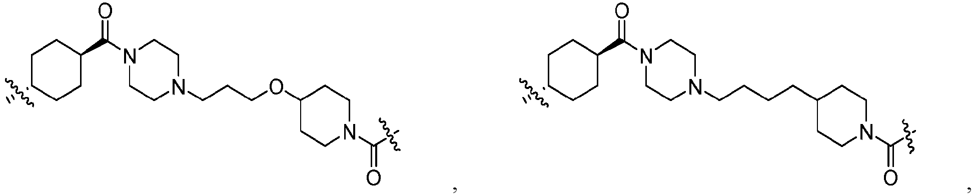

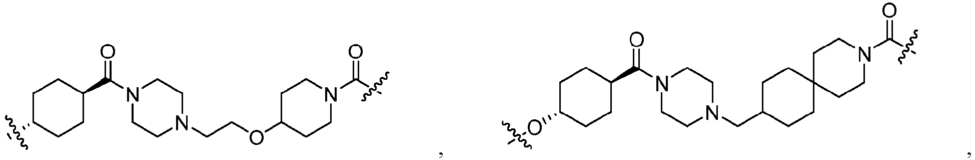

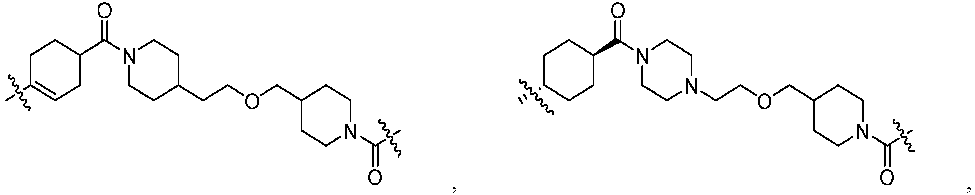

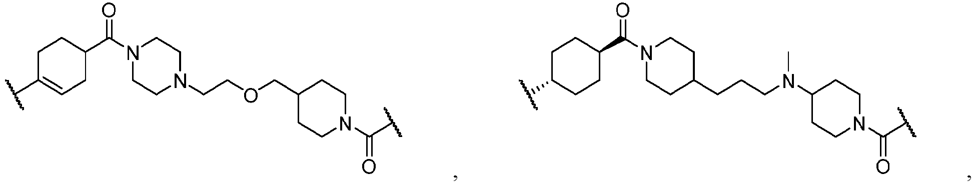

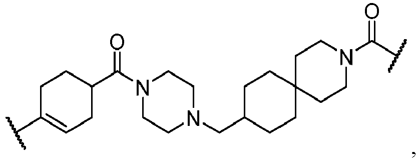

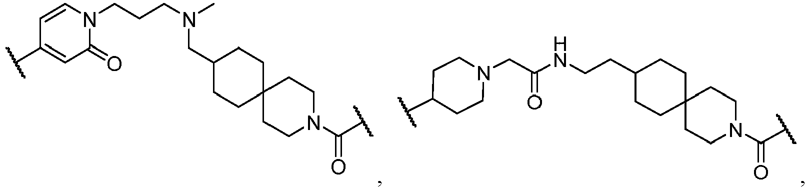

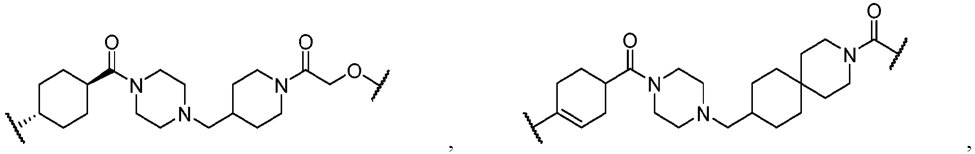

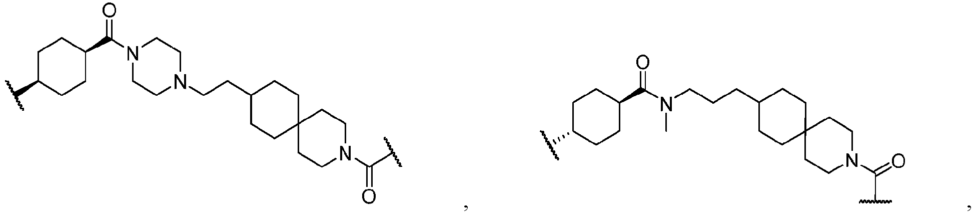

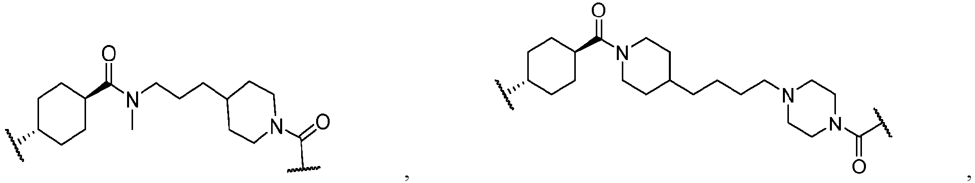

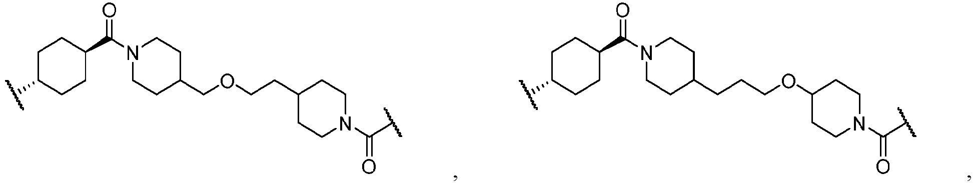

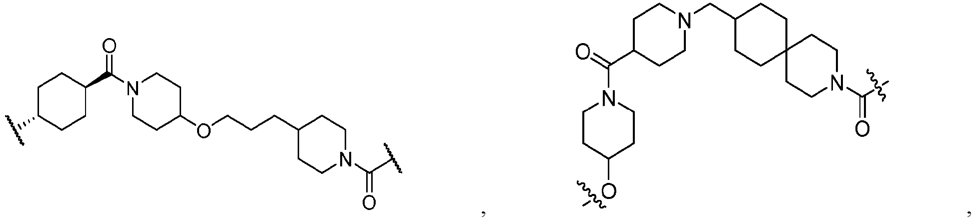

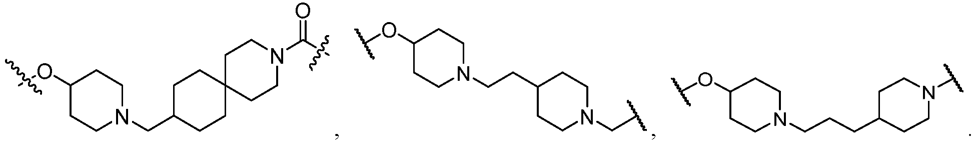

10. The compound of formula I, and/or the stereoisomer, the enantiomer, the diastereomer, the deuteride, the hydrate, the solvate, the prodrug and/or the pharmaceutically acceptable salt thereof according to any one of claims 1-7, wherein L is LA: 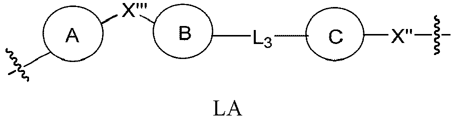 wherein in LA,
ring A is a bond, C₃-C₁₂ cycloalkylene or 3- to 12-membered heterocycloalkylene containing 1-2 heteroatoms selected from N, O and S, wherein the cycloalkylene and the heterocycloalkylene are optionally substituted with substituents selected from halogen, oxo, cyano, amino, hydroxy, C₁-C₆ alkyl, C₁-C₆ haloalkyl, C₁-C₆ hydroxyalkyl and -O-(C₁-C₆ alkyl);
ring B is a bond, C₃-C₁₂ cycloalkylene or 3- to 12-membered heterocycloalkylene containing 1-2 heteroatoms selected from N, O and S, wherein the cycloalkylene and the heterocycloalkylene are optionally substituted with substituents selected from halogen, oxo, cyano, amino, hydroxy, C₁-C₆ alkyl, C₁-C₆ haloalkyl, C₁-C₆ hydroxyalkyl and -O-(C₁-C₆ alkyl);
ring C is C₃-C₁₂ cycloalkylene or 3- to 12-membered heterocycloalkylene containing 1-2 heteroatoms selected from N, O and S, and the cycloalkylene and heterocycloalkylene are optionally substituted with substituents selected from halogen, oxo, cyano, amino, hydroxy, C₁-C₆ alkyl, C₁-C₆ haloalkyl, C₁-C₆ hydroxyalkyl and -O-(C₁-C₆ alkyl); X" is a bond, -NH-, -NCH₃-, -O-, -C(CH₃)₂-, -S-, -C=C-, -C≡C-, -CHF-, -CHCF₃-, -C(O)-, -S(O)-, -S(O)₂-, -C(O)O-, -OC(O)-, -C(O)NH-, -C(O)NCH₃-, -NHC(O)-, -NCH₃C(O)- or -C(O)CH₂O-;
L₃ is -(CH₂)ₖ, wherein one or two methylene groups in L₃ are optionally replaced by a group selected from -O-, - NH-, -C≡C-, -N(C₁-C₆ alkyl)-, -N(C₁-C₆ haloalkyl)-, -C(O)-, -N(C₁-C₆ hydroxyalkyl)- and -N(C₃-C₈ cycloalkyl)-, and k is 0, 1, 2, 3, 4, 5, 6 or 7;
X‴ is a bond, -NH-, -NCH₃-, -O-, -C(CH₃)₂-, -S-, -C=C-, -C≡C-, -CHF-, -CHCF₃-, -C(O)-, -S(O)-, -S(O)₂-, -C(O)O-, -OC(O)-, -C(O)NH-, -C(O)NCH₃-, -CH₂NCH₃-, -NHC(O)- or -NCH₃C(O)-.

11. The compound of formula I, and/or the stereoisomer, the enantiomer, the diastereomer, the deuteride, the hydrate, the solvate, the prodrug and/or the pharmaceutically acceptable salt thereof according to claim 10, wherein in LA, ring A is 3- to 6-membered saturated cycloalkylene; ring B is 4- to 7-membered saturated monocyclic heterocycloalkylene containing 1 or 2 nitrogen heteroatoms; ring C is 4- to 7-membered saturated monocyclic heterocycloalkylene containing 1 or 2 nitrogen heteroatoms or 7- to 11-membered spiroheterocycloalkylene or fused heterocycloalkylene containing 1 or 2 nitrogen heteroatoms; X" is a bond or -C(O)-; L₃ is -(CH₂)ₖ, wherein k is 1, 2, 3, 4 or 5.

12. The compound of formula I, and/or the stereoisomer, the enantiomer, the diastereomer, the deuteride, the hydrate, the solvate, the prodrug and/or the pharmaceutically acceptable salt thereof according to claim 10, wherein LA is LA-1: 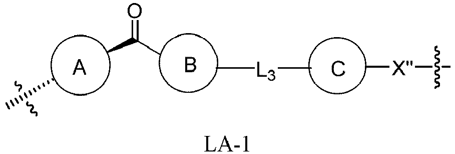 wherein in LA-1, ring A, ring B, ring C, L₃ and X" are as defined and described in claim 10.

13. The compound of formula I, and/or the stereoisomer, the enantiomer, the diastereomer, the deuteride, the hydrate, the solvate, the prodrug and/or the pharmaceutically acceptable salt thereof according to claim 10, wherein LA is LA-2, LA-3, LA-4 or LA-5: 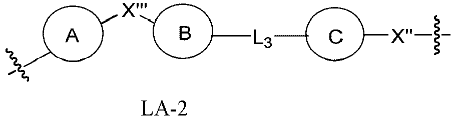
wherein in LA-2, ring A is 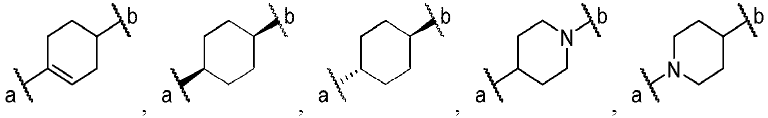
or 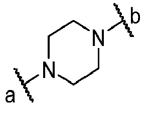 wherein end a is linked to S, and end b is linked to X‴; 1, 2, 3 or 4 hydrogen atoms in ring A are optionally substituted with F;
X‴ is -C(O)-;
ring B is 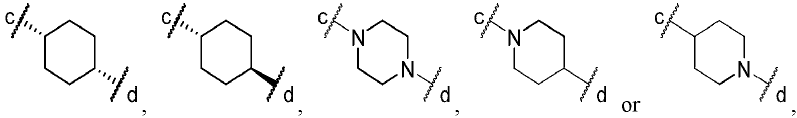 wherein end c is linked to X‴, and end d is linked to L₃; 1, 2, 3 or 4 hydrogen atoms in ring B are optionally substituted with F;
L₃ is -(CH₂)ₖ-, wherein one or two CH₂ groups contained in L₃ are each independently optionally replaced by -O-, -NH- or -N(C₁-C₆ alkyl)-, or one -CH₂CH₂- group contained in L₃ is optionally replaced by -C≡C-; k is 1, 2, 3 or 4;
ring C is 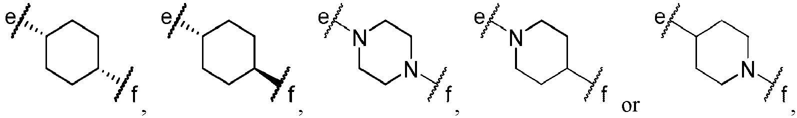 wherein end e is linked to L₃, and end f is linked to X"; 1, 2, 3 or 4 hydrogen atoms in ring C are optionally substituted with F;
X' is -C(O)-; 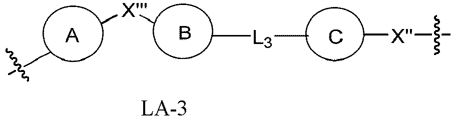
wherein in LA-3, ring A is 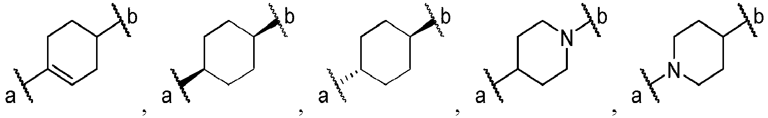 or 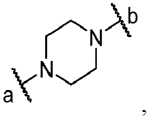 wherein end a is linked to S, and end b is linked to X‴; 1, 2, 3 or 4 hydrogen atoms in ring A are optionally substituted with F;
X‴ is -C(O)-;
ring B is 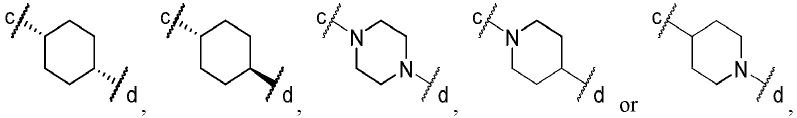 wherein end c is linked to X‴, and end d is linked to L₃; 1, 2, 3 or 4 hydrogen atoms in ring B are optionally substituted with F; L₃ is -(CH₂)ₖ-, wherein one CH₂ group contained in L₃ is optionally replaced by -O-, -NH- or -N(C₁-C₆ alkyl)-; k is 1 or 2;
ring C is 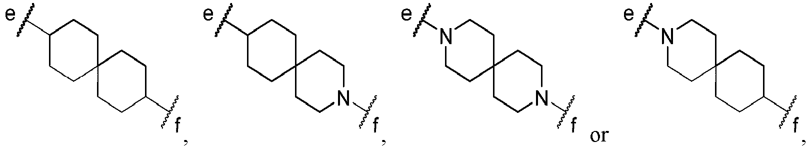 wherein end e is linked to L₃, and end f is linked to X"; 1, 2, 3 or 4 hydrogen atoms in ring C are optionally substituted with F;
X' is -C(O)-; 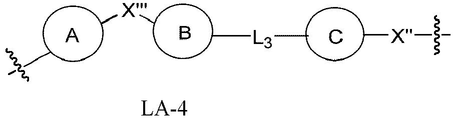
wherein in LA-4,
ring A is 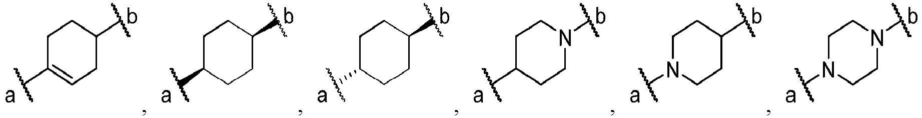
or 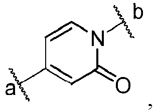 wherein end a is linked to S, and end b is linked to X‴; 1, 2, 3 or 4 hydrogen atoms in ring A are optionally substituted with F;
X‴ is a bond, -C(O)NH- or -C(O)NCH₃-;
ring B is a bond;
L₃ is -(CH₂)ₖ, wherein one or two methylene groups in L₃ are optionally replaced by -O-, -NH-, -C(O)-, -C≡C- or - N(C₁-C₆ alkyl)-, and k is 0, 1, 2, 3, 4, 5, 6 or 7;
ring C is 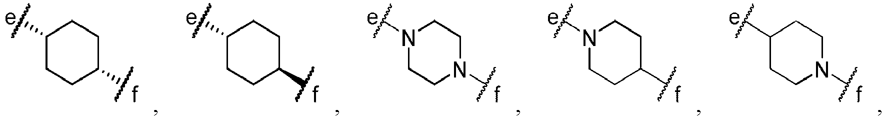 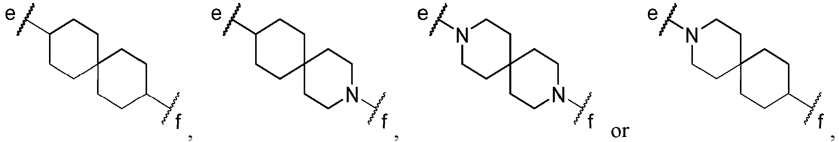 wherein end e is linked to L₃, and end f is linked to X"; 1, 2, 3 or 4 hydrogen atoms in ring C are optionally substituted with F;
X' is -C(O)-; 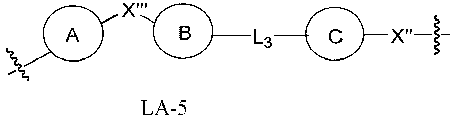
wherein ring A, X‴, ring B, L₃ and ring C are as defined in LA-2 or LA-3;
X" is -C(O)CH₂O-, and -C(O)- in X" is linked to ring C.

14. The compound of formula I, and/or the stereoisomer, the enantiomer, the diastereomer, the deuteride, the hydrate, the solvate, the prodrug and/or the pharmaceutically acceptable salt thereof according to claim 13, wherein in LA-2, ring A is 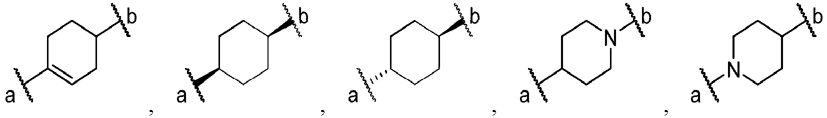 or 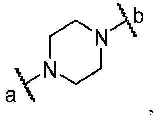 wherein end a is linked to S, and end b is linked to X‴; and/or, in LA-2, ring B is 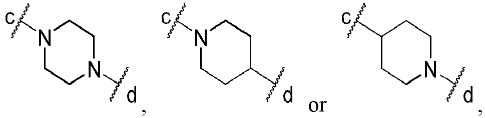 wherein end c is linked to X‴, and end d is linked to L₃; and/or, in LA-2, L₃ is -CH₂-, -(CH₂)₂-, -(CH₂)₃-, -(CH₂)₄-, 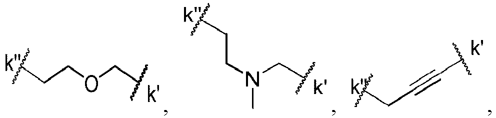 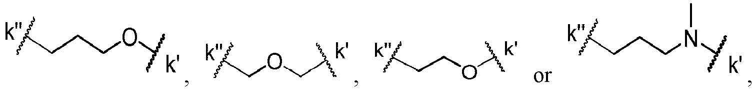 wherein end k" is linked to ring B, and end k' is linked to ring C; and/or, in LA-2, ring C is 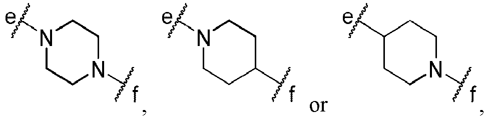 wherein end e is linked to L₃, and end f is linked to X"; and/or, in LA-3, ring A is 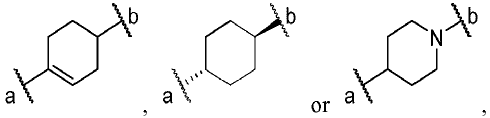 wherein end a is linked to S, and end b is linked to X‴; and/or, in LA-3, ring B is 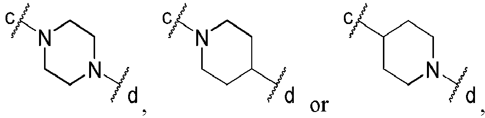 wherein end c is linked to X‴, and end d is linked to L₃; and/or, in LA-3, L₃ is -(CH₂)ₖ-, wherein k is 1 or 2; and/or, in LA-3, ring C is 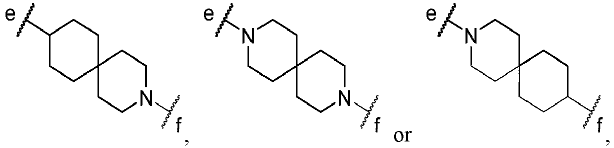 wherein end e is linked to L₃, and end f is linked to X"; and/or, in LA-4, ring A is 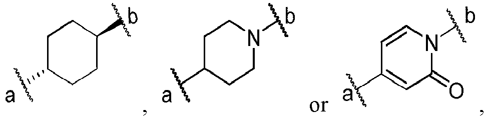 wherein end a is linked to S, and end b is linked to X‴; and/or, in LA-4, L₃ is -CH₂-, -(CH₂)₂-, 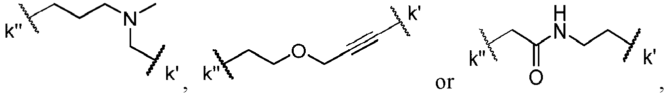 wherein end k" is linked to ring B, and end k' is linked to ring C; and/or, in LA-4, ring C is 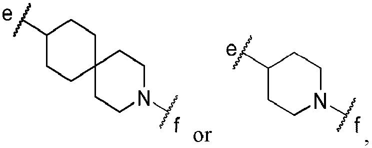 wherein end e is linked to L₃, and end f is linked to X"; and/or, in LA-5, ring A is 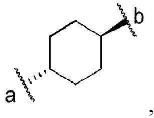 wherein end a is linked to S, and end b is linked to X‴; and/or, in LA-5, ring B is 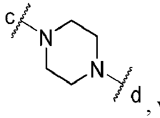 wherein end c is linked to X‴, and end d is linked to L₃; and/or, in LA-5, L₃ is -(CH₂)-; and/or, in LA-5, ring C is 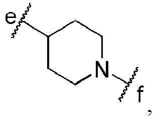 wherein end e is linked to L₃, and end f is linked to X".

15. The compound of formula I, and/or the stereoisomer, the enantiomer, the diastereomer, the deuteride, the hydrate, the solvate, the prodrug and/or the pharmaceutically acceptable salt thereof according to claim 10 or 13, wherein LA is of any one of the following structures: 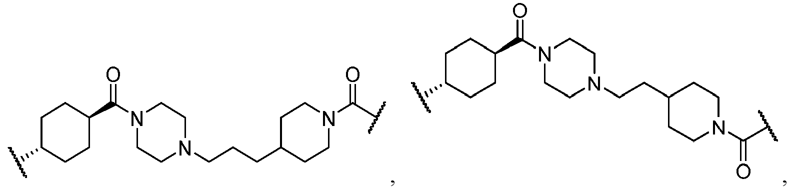 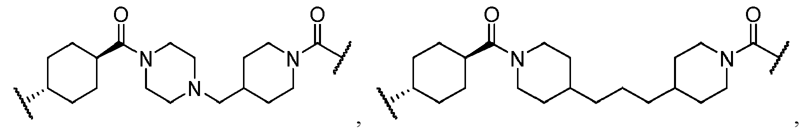 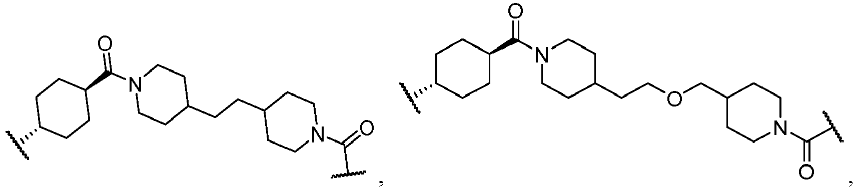 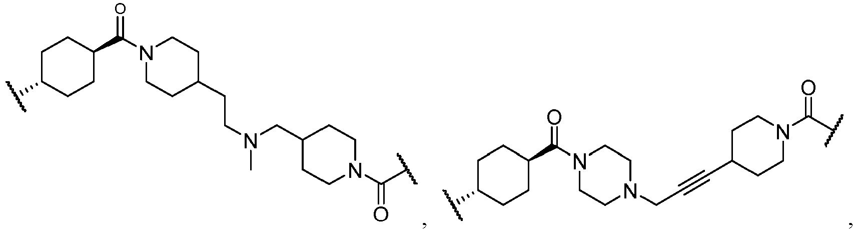 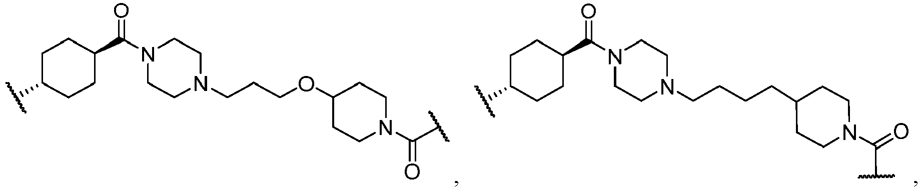 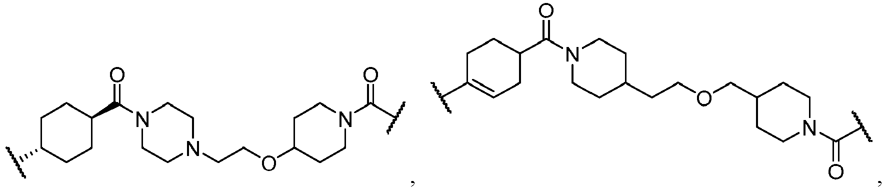 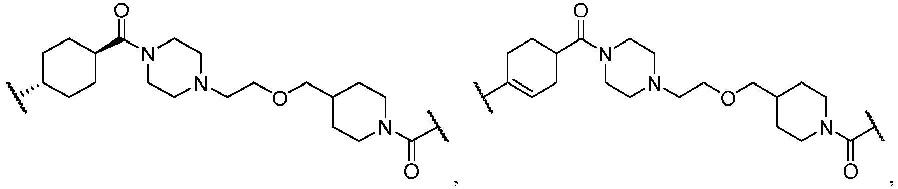 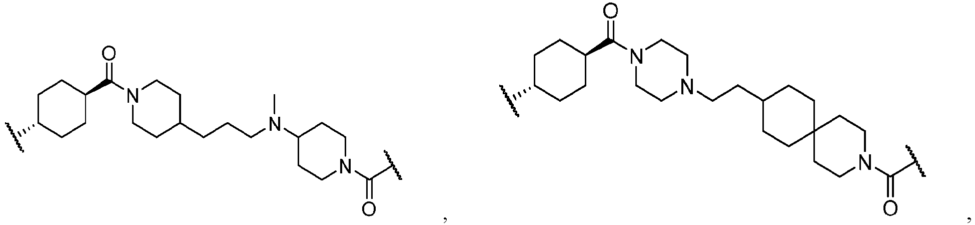 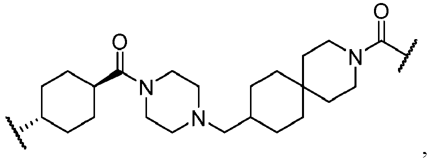 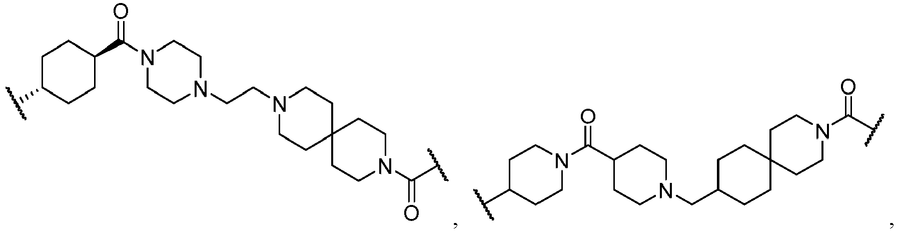 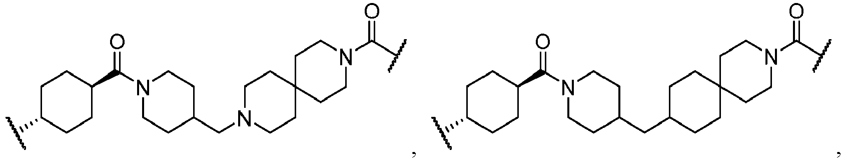 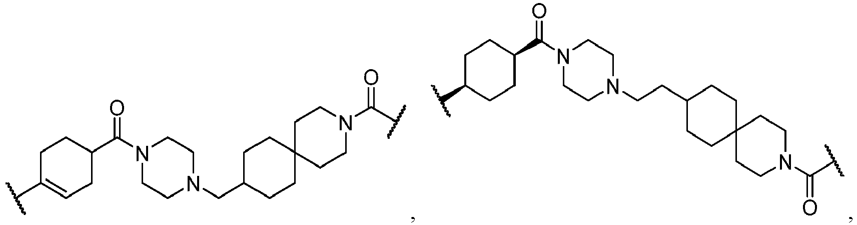 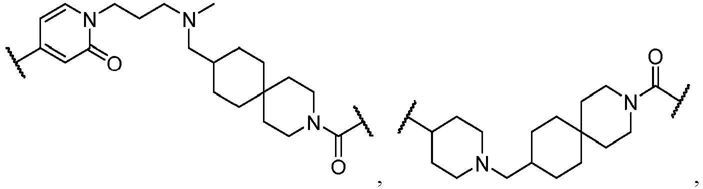 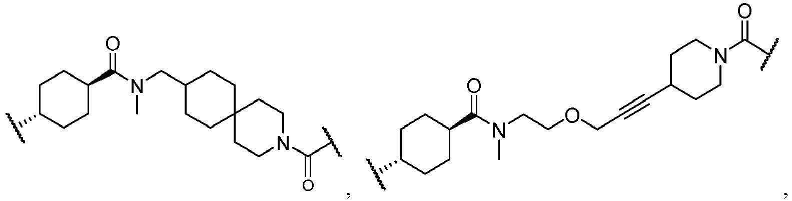 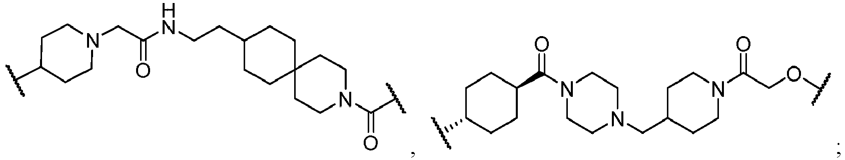 preferably, LA is of any one of the following structures:

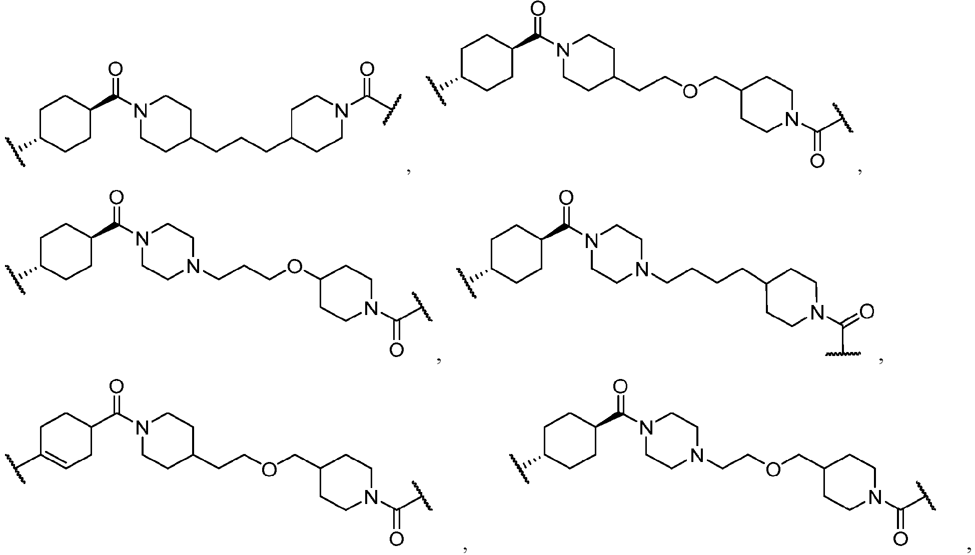

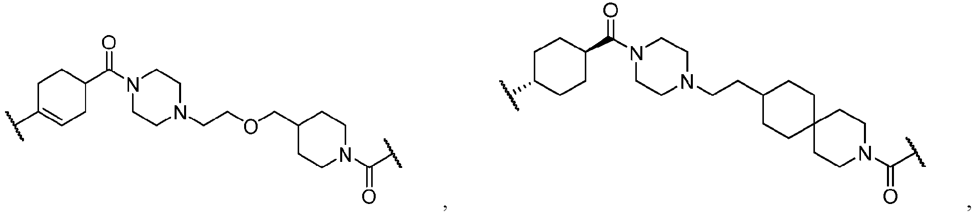

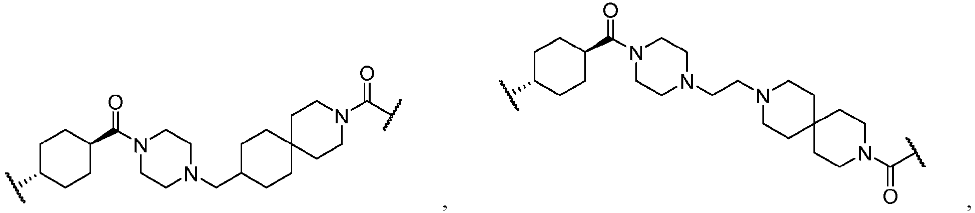

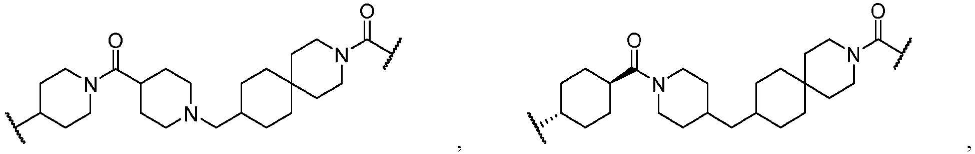

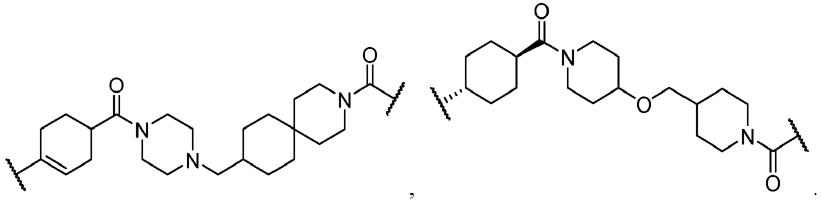

16. The compound of formula I, and/or the stereoisomer, the enantiomer, the diastereomer, the deuteride, the hydrate, the solvate, the prodrug and/or the pharmaceutically acceptable salt thereof according to any one of claims 1-15, wherein E is E21: 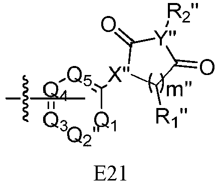 wherein in E21,
X" is C or N;
Y" is C, N, O or S;
Q₁, Q₂, Q₃, Q₄ and Q₅ are each independently CR₃" or N;
R₃" is each independently hydrogen, deuterium, hydroxy, amino, cyano, halogen, C₁-C₆ alkyl, C₃-C₈ cycloalkyl, 3-to 8-membered heterocycloalkyl, 6- to 10-membered aryl, 5- to 10-membered heteroaryl, -O(C₁-C₆ alkyl), -O-(C₃-C₈ cycloalkyl), -O-(3- to 8-membered heterocycloalkyl), N(C₁-C₆ alkyl)₁₋₂, NH(C₃-C₈ cycloalkyl), NH(3- to 8-membered heterocycloalkyl), -O-(C₆-C₁₀ aryl) or -O-(5- to 10-membered heteroaryl), wherein the alkyl, the cycloalkyl, the heterocycloalkyl, the aryl and the heteroaryl are optionally substituted with 1-3 groups independently selected from hydroxy, halogen, cyano and amino;
m" is 1, 2 or 3;
each R₁" is independently hydrogen, deuterium, hydroxy, amino, cyano, halogen, C₁-C₆ alkyl, C₃-C₈ cycloalkyl, 3-to 8-membered heterocycloalkyl, 6- to 10-membered aryl, 5- to 10-membered heteroaryl or -O(C₁-C₆ alkyl), and the alkyl, the cycloalkyl, the heterocycloalkyl, the aryl and the heteroaryl are optionally substituted with 1-3 groups independently selected from hydroxy, halogen, cyano and amino;
R₂" is hydrogen, deuterium, C₁-C₆ alkyl or C₃-C₆ cycloalkyl, and the C₁-C₆ alkyl and the C₃-C₆ cycloalkyl are optionally substituted with 1-3 groups independently selected from hydroxy, halogen, cyano and amino.

17. The compound of formula I, and/or the stereoisomer, the enantiomer, the diastereomer, the deuteride, the hydrate, the solvate, the prodrug and/or the pharmaceutically acceptable salt thereof according to claim 16, wherein E21 has the structure of formula E21-1a, E21-1b, E21-1c, E21-1d, E21-1e, E21-1f, E21-1g, E21-1aa, E21-1bb, E21-1cc, E21-1dd, E21-1ee, E21-1ff or E21-1gg: 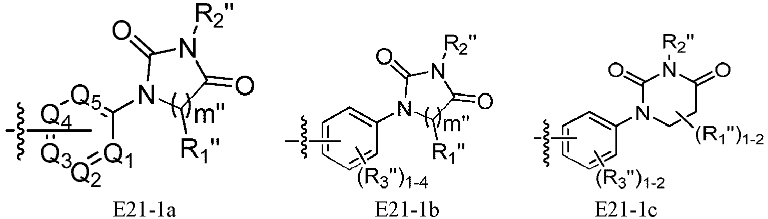 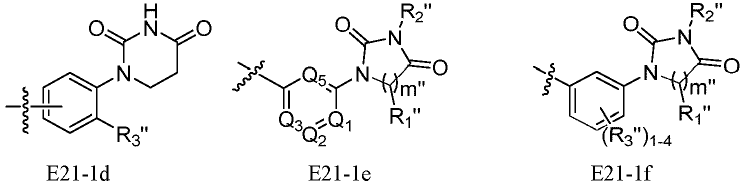 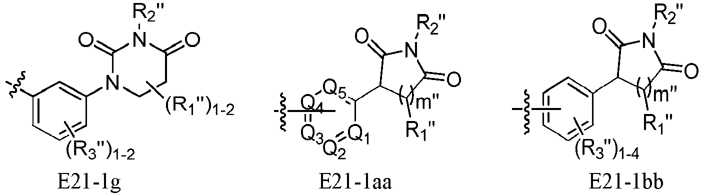 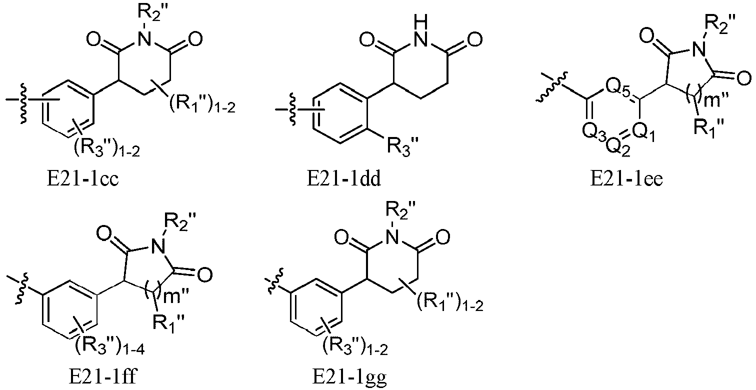 wherein Q₁, Q₂, Q₃, Q₄, Q₅, R₁", R₂", R₃" and m" are as defined and described in claim 16.

18. The compound of formula I, and/or the stereoisomer, the enantiomer, the diastereomer, the deuteride, the hydrate, the solvate, the prodrug and/or the pharmaceutically acceptable salt thereof according to claim 17, wherein E21 has the structure of formula E21-1h, E21-1i, E21-1j or E21-1hh, preferably E21-1h: 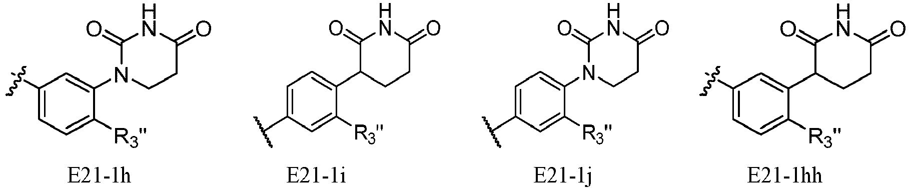 wherein R₃" is as defined in claim 17.

19. The compound of formula I, and/or the stereoisomer, the enantiomer, the diastereomer, the deuteride, the hydrate, the solvate, the prodrug and/or the pharmaceutically acceptable salt thereof according to any one of claims 16-18, wherein R₃" is hydrogen, halogen, C₁-C₆ alkyl or -O(C₁-C₆ alkyl), wherein the C₁-C₆ alkyl and the -O(C₁-C₆ alkyl) are optionally substituted with 1-3 halogens; for example, R₃" is hydrogen, F, Cl, CF₃, -OCF₃ or -OCH₃.

20. The compound of formula I, and/or the stereoisomer, the enantiomer, the diastereomer, the deuteride, the hydrate, the solvate, the prodrug and/or the pharmaceutically acceptable salt thereof according to claim 16, wherein E21 is: 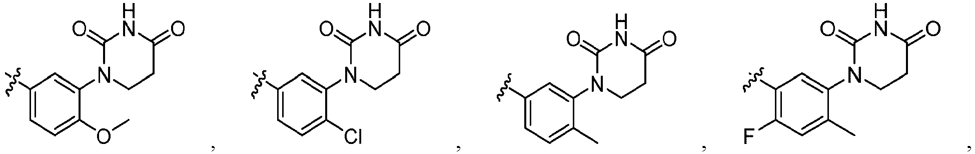 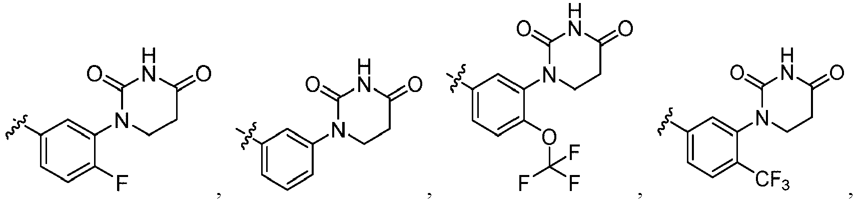 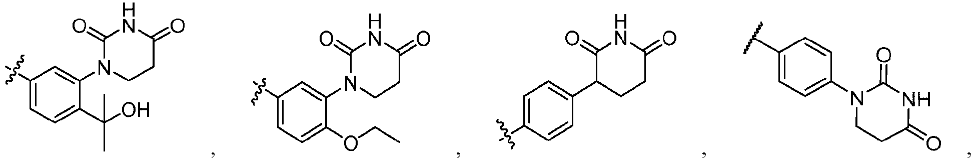 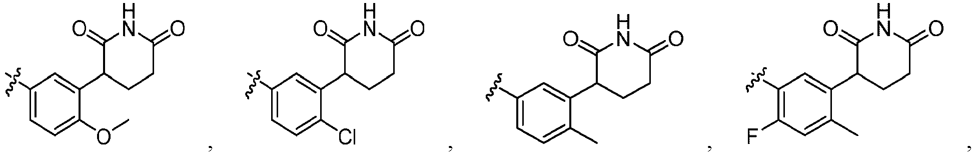 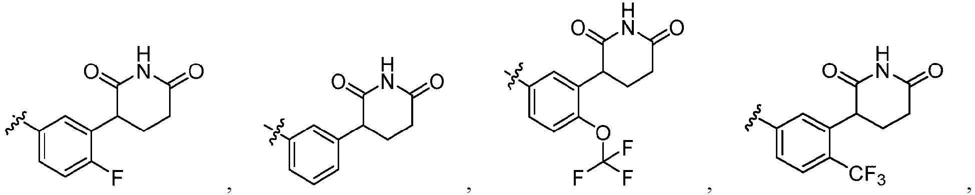 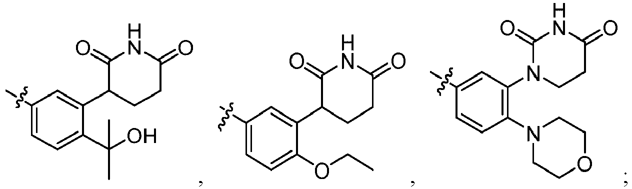 preferably, E21 is of any one of the following structures:

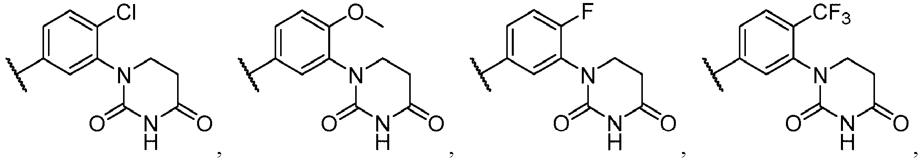

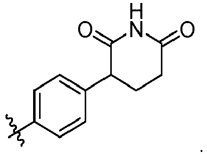

21. The compound of formula I, and/or the stereoisomer, the enantiomer, the diastereomer, the deuteride, the hydrate, the solvate, the prodrug and/or the pharmaceutically acceptable salt thereof according to claim 1, wherein the compound of formula I is any one of the following compounds:

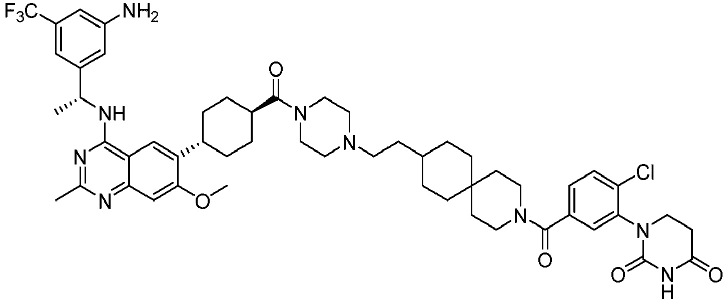

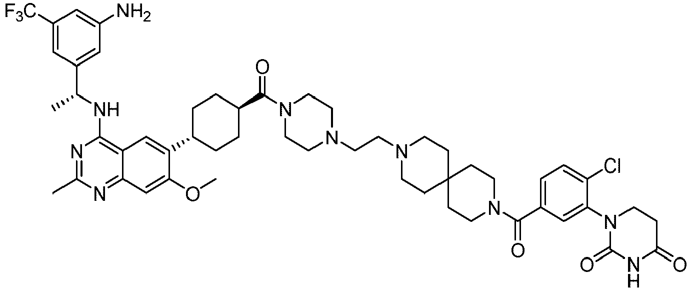

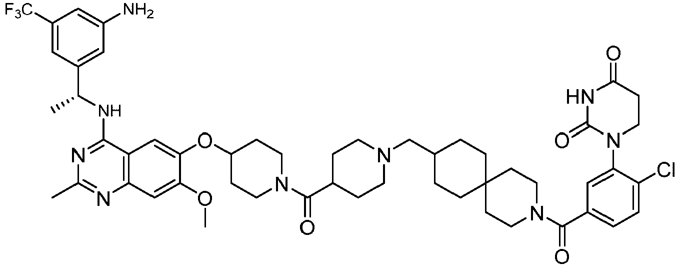

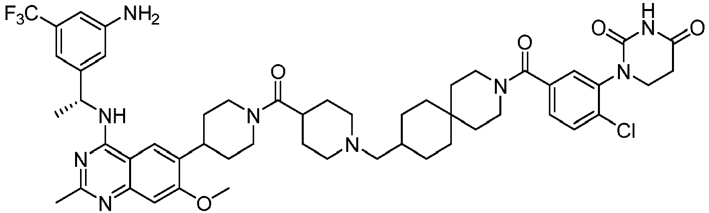

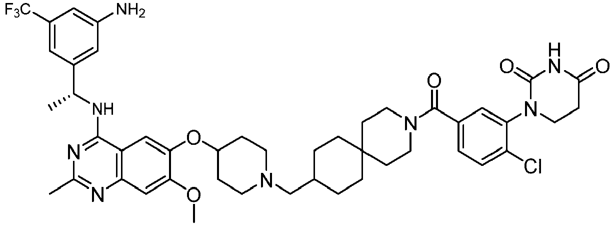

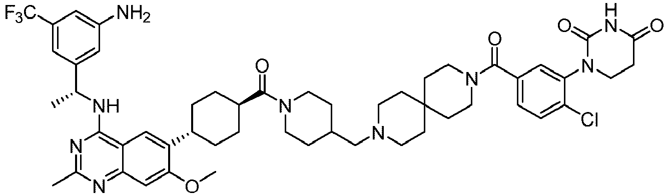

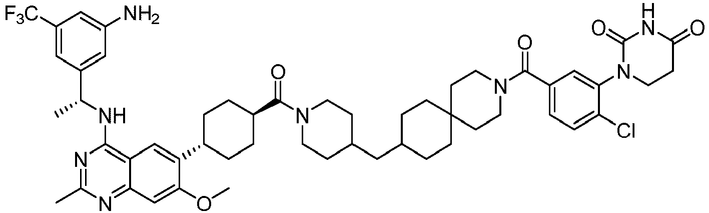

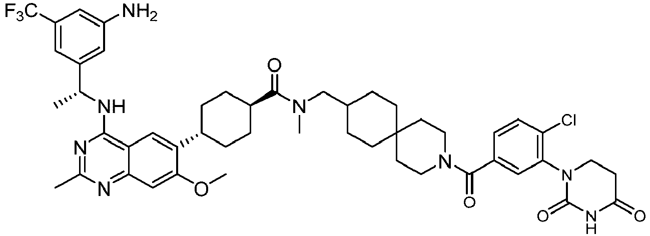

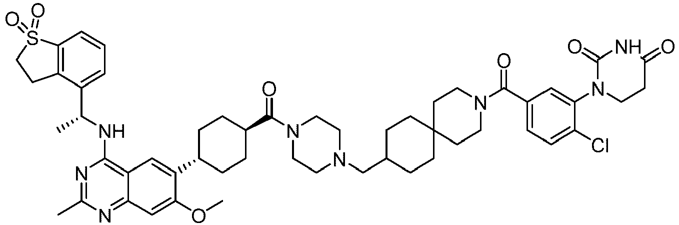

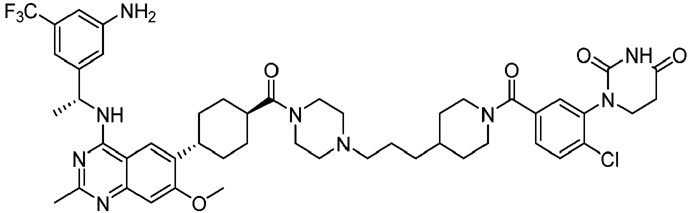

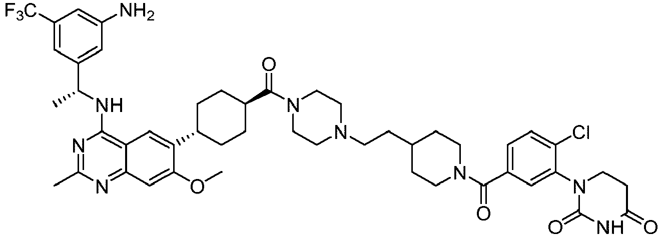

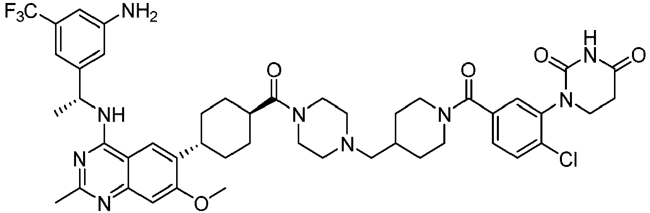

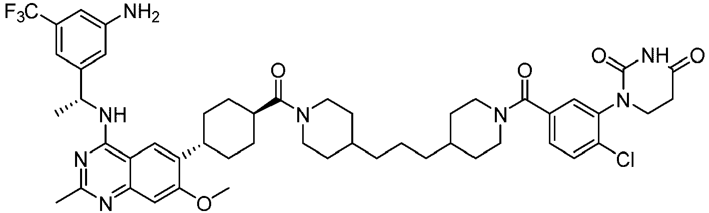

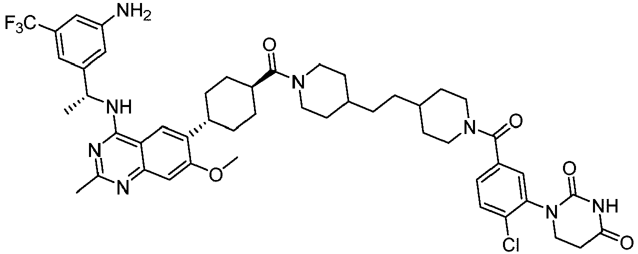

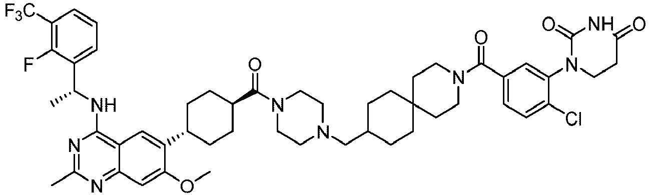

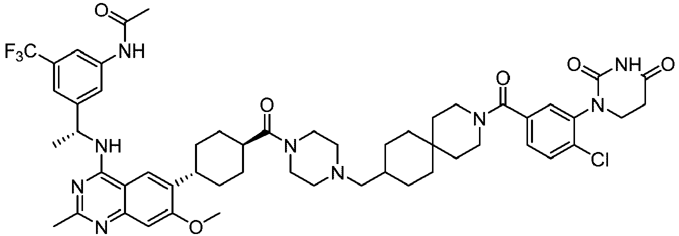

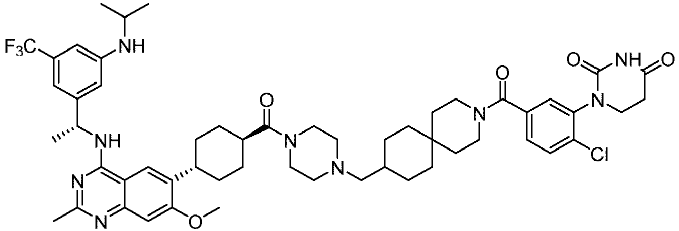

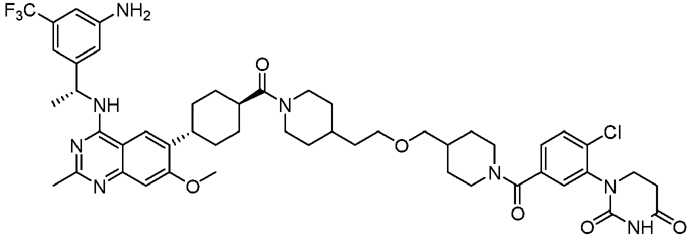

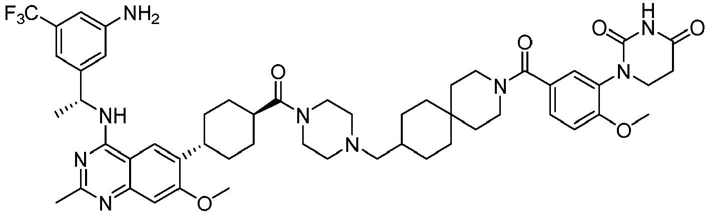

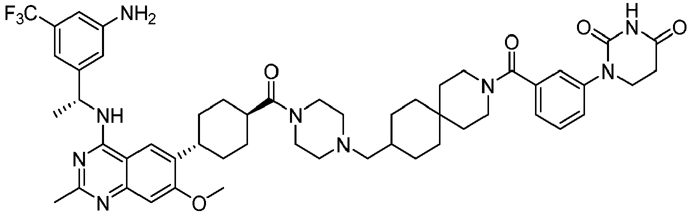

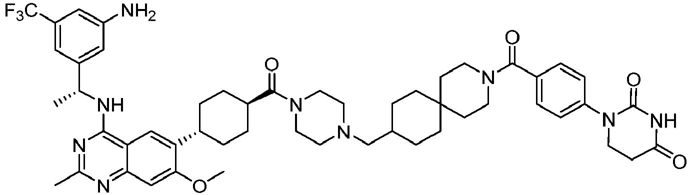

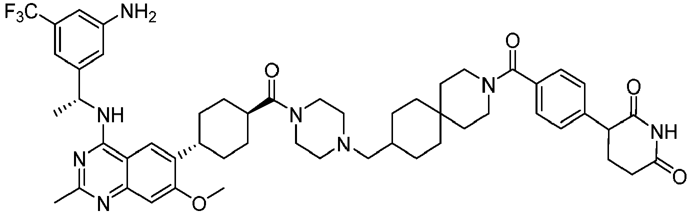

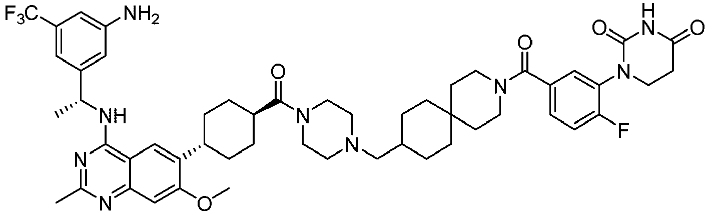

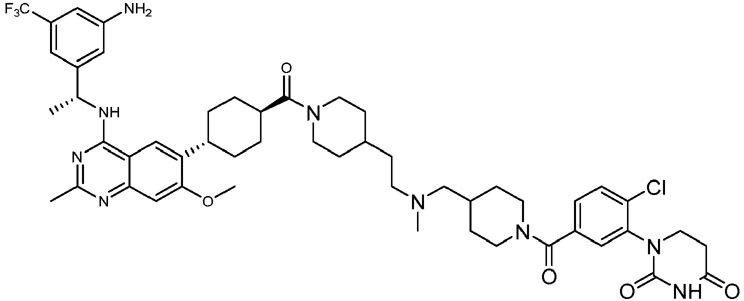

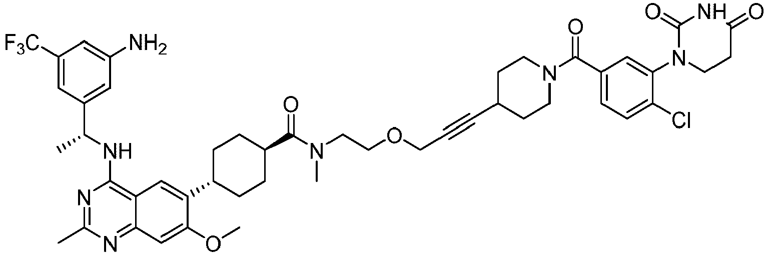

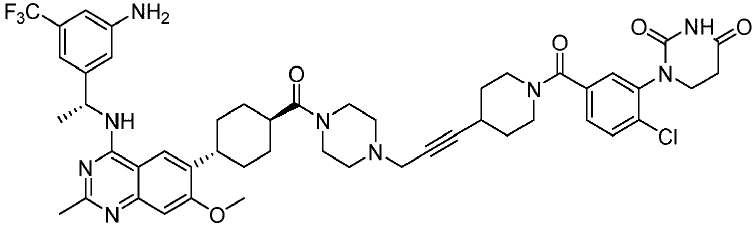

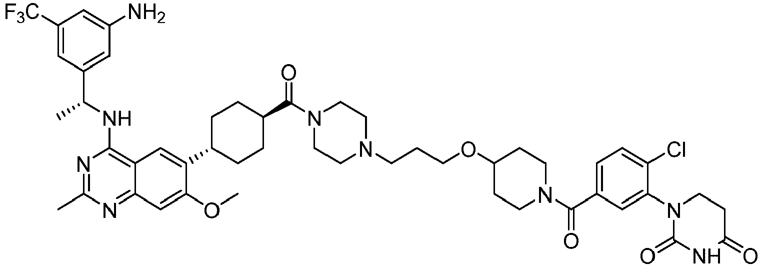

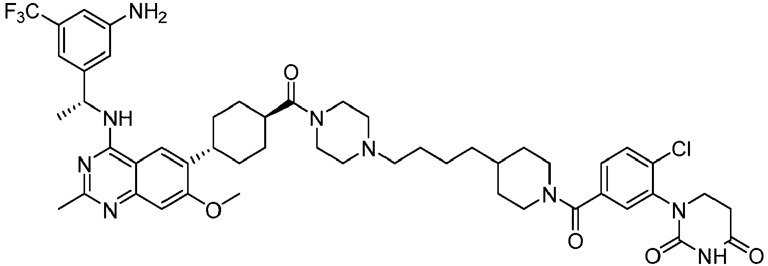

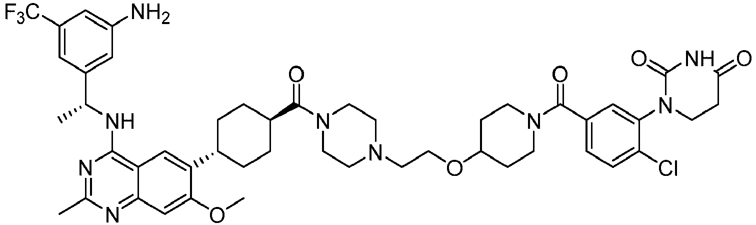

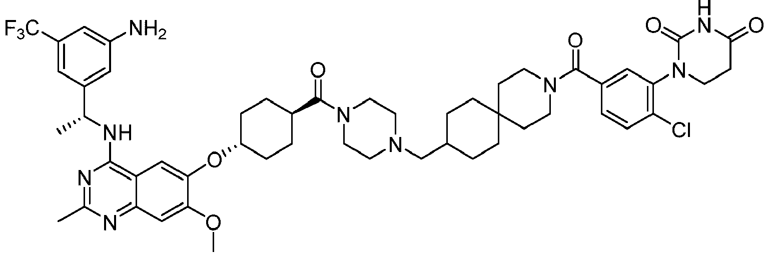

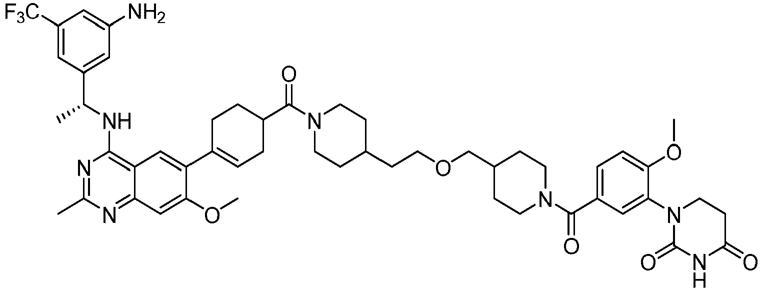

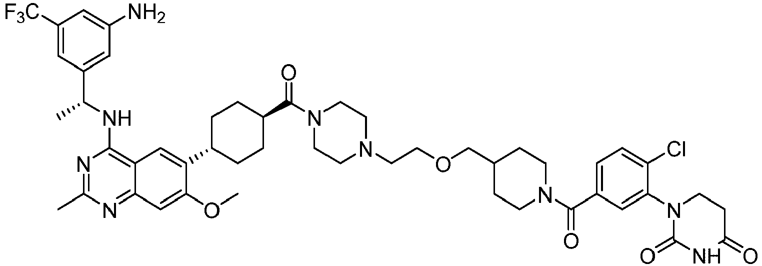

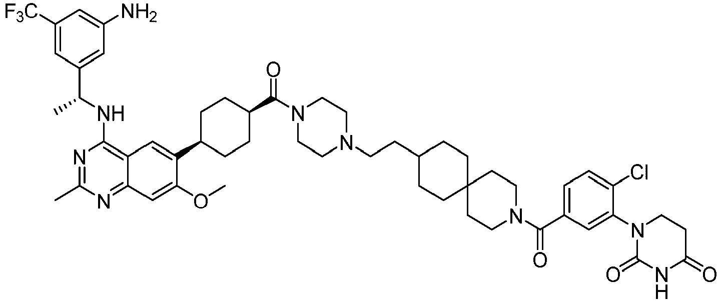

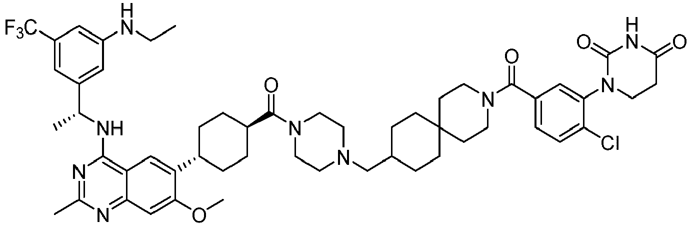

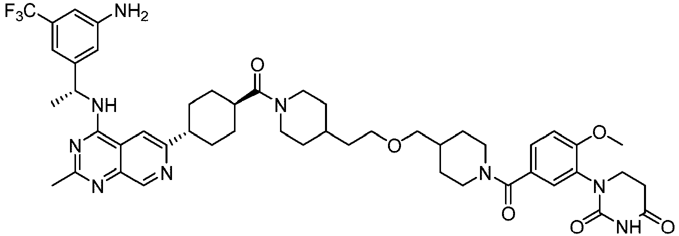

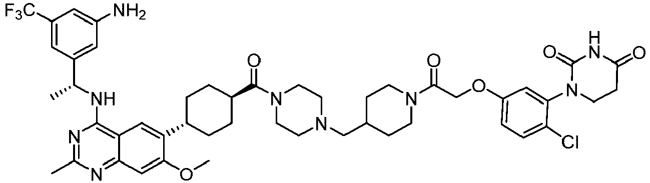

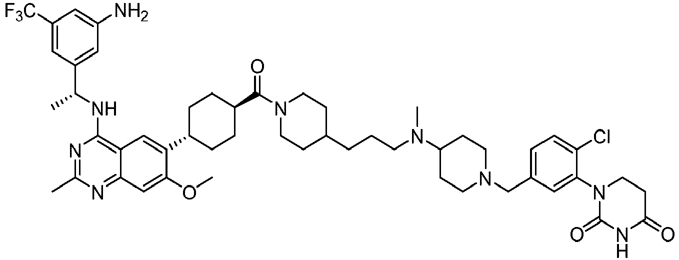

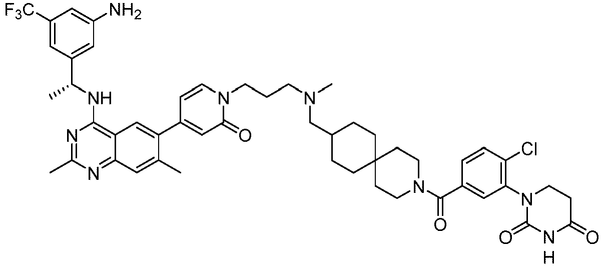

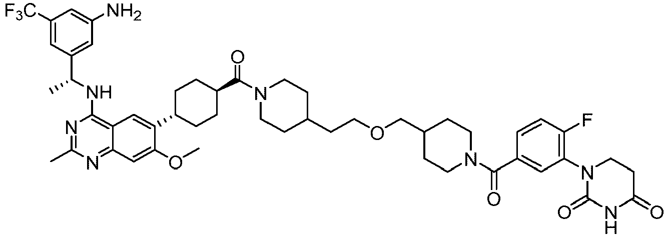

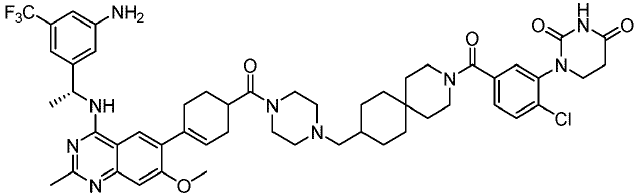

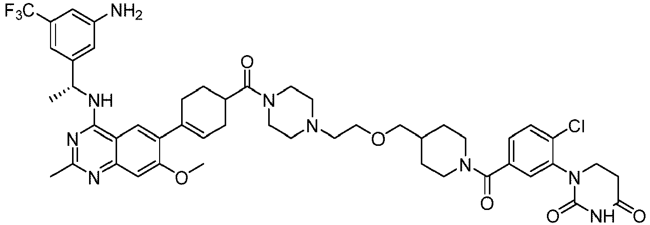

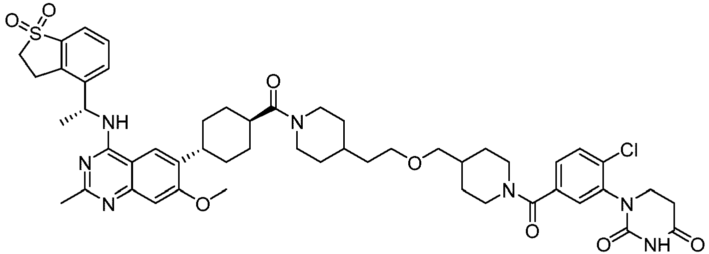

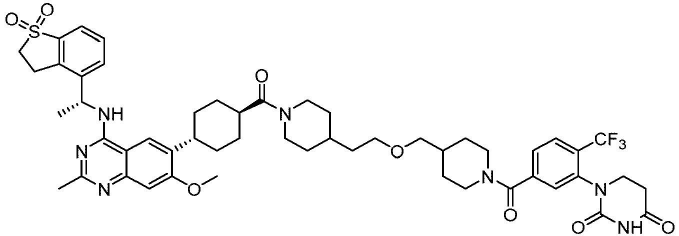

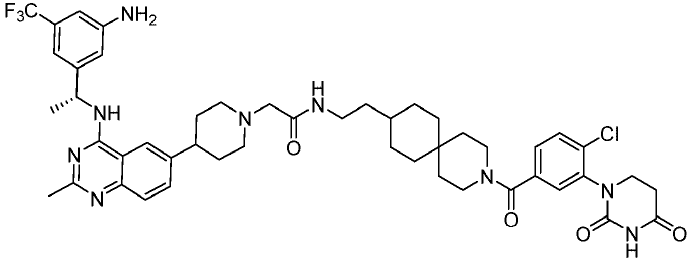

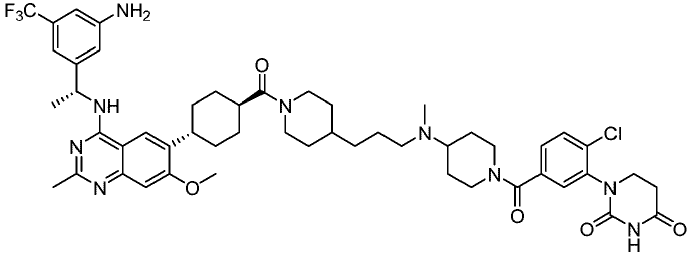

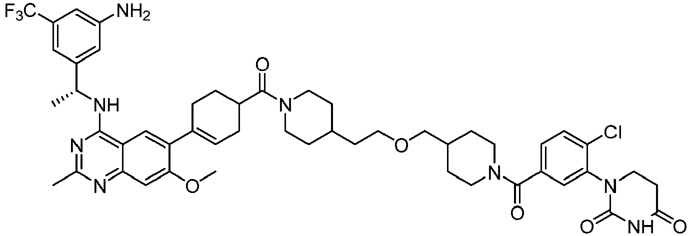

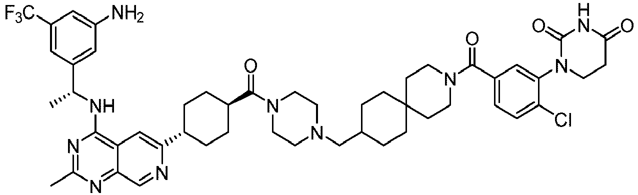

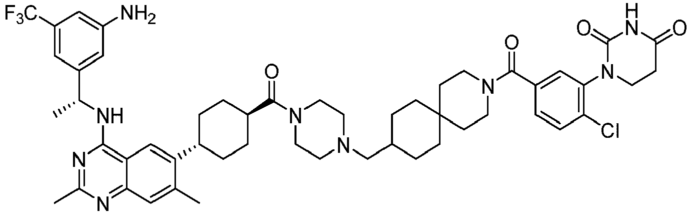

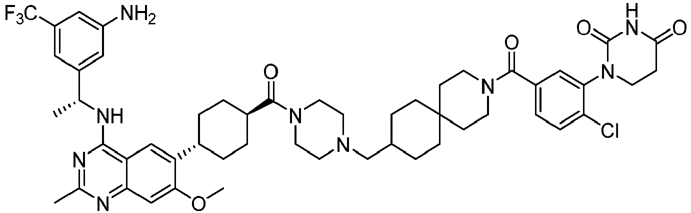

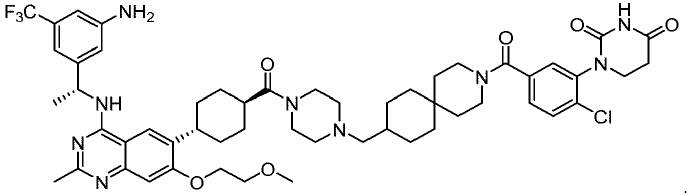

22. The compound of formula I, and/or the stereoisomer, the enantiomer, the diastereomer, the deuteride, the hydrate, the solvate, the prodrug and/or the pharmaceutically acceptable salt thereof according to claim 1, wherein the deuteride has any one of the following structures:

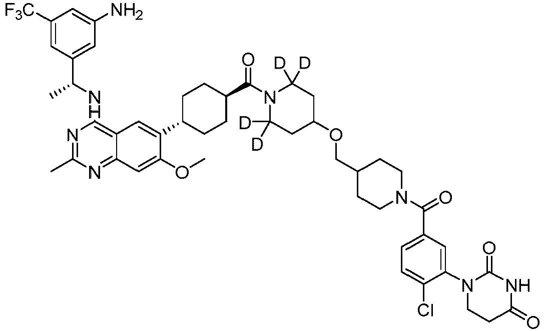

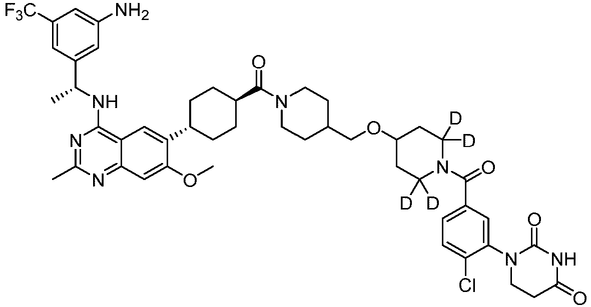

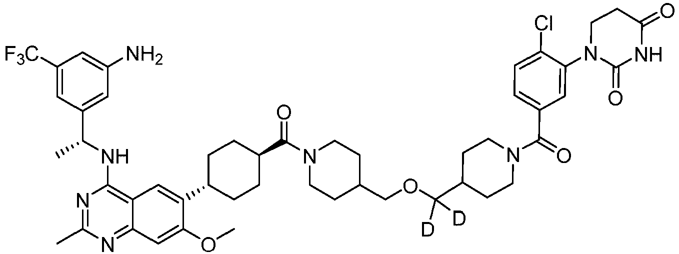

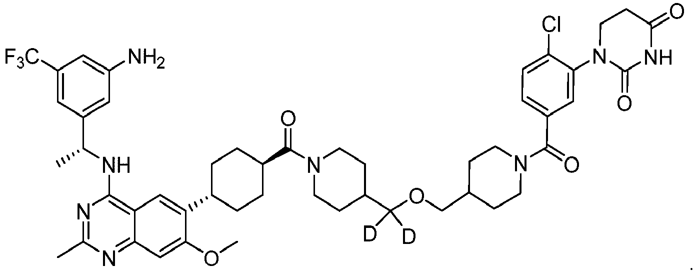

23. A pharmaceutical composition comprising the compound, and/or the stereoisomer, the enantiomer, the diastereomer, the deuteride, the hydrate, the solvate, the prodrug and/or the pharmaceutically acceptable salt thereof according to any one of claims 1-22, and a pharmaceutically acceptable carrier, diluent, or excipient.

24. Use of the compound, and/or the stereoisomer, the enantiomer, the diastereomer, the deuteride, the hydrate, the solvate, the prodrug and/or the pharmaceutically acceptable salt thereof according to any one of claims 1-22, or the pharmaceutical composition according to claim 23 in preparing a medicament for the treatment and/or prevention of an SOS1-mediated disease or disorder, or a disease or disorder caused by the interaction of SOS1 with Ras or SOS1 with Rac, or a cancer.

25. The use according to claim 24, wherein the cancer is selected from:
heart: sarcoma (angiosarcoma, fibrosarcoma, rhabdomyosarcoma and liposarcoma), myxoma, rhabdomyoma, fibroma, lipoma and teratoma;
lung: bronchial cancer (squamous cell carcinoma, undifferentiated small cell carcinoma, undifferentiated large cell carcinoma and adenocarcinoma), bronchioloalveolar carcinoma (bronchiolar carcinoma), bronchial adenoma, sarcoma, lymphoma, chondroma, hamartoma and mesothelioma;
gastrointestinal tract and esophagus (squamous cell carcinoma, adenocarcinoma, leiomyosarcoma and lymphoma), stomach (cancer, lymphoma and leiomyosarcoma), pancreas (ductal adenocarcinoma, insulinoma, glucagonoma, gastrinoma, carcinoid tumor and vipoma), small intestine (adenocarcinoma, lymphoma, carcinoid tumor, Kaposi's sarcoma, leiomyoma, hemangioma, lipoma, neurofibroma and fibroma) and large intestine (adenocarcinoma, tubular adenoma, villous adenoma, hamartoma and leiomyoma);
genitourinary system: kidney (adenocarcinoma, Wilms' tumor, lymphoma and leukemia), bladder and urinary tract (squamous cell carcinoma, transitional cell carcinoma and adenocarcinoma), prostate (adenocarcinoma and sarcoma) and testis (seminoma, teratoma, embryonal carcinoma, teratoma, choriocarcinoma, sarcoma, interstitial cell carcinoma, fibroma, fibroadenoma, adenomatoid tumor and lipoma);
fiver: liver cancer (hepatocellular carcinoma), cholangiocarcinoma, hepatoblastoma, angiosarcoma, hepatocellular adenoma and hemangioma;
biliary tract: gallbladder cancer, ampullary cancer and cholangiocarcinoma;
bone: osteosarcoma (osteosarcoma), fibrosarcoma, malignant fibrous histiocytoma, chondrosarcoma, Ewing's sarcoma, malignant lymphoma (reticulosarcoma), multiple myeloma, malignant giant cell tumor, chordoma, chondroma (osteochondral exostosis), benign chondroma, chondroblastoma, chondromyxoid fibroma, osteoid osteoma and giant cell tumor;
nervous system: skull (osteoma, hemangioma, granuloma, xanthoma and osteitis deformans), meninges (meningioma, meningosarcoma and glioma), brain (astrocytoma, medulloblastoma, glioma, ependymoma, germinal cell tumor (pinealoma), glioblastoma multiforme, oligodendroglioblastoma, neurilemmoma, retinoblastoma and congenital tumor), spinal neurofibroma, meningioma, glioma and sarcoma);
gynaecology: uterus (endometrial cancer (serous cystadenocarcinoma, mucinous cystadenocarcinoma and unclassified cancer), granulosa theca cell tumor, Sertoli-Leydig cell tumor, dysgerminoma and malignant teratoma), vulva (squamous cell carcinoma, intraepithelial carcinoma, adenocarcinoma, fibrosarcoma and melanoma), vagina (clear cell carcinoma, squamous cell carcinoma, botryoid sarcoma (embryonal rhabdomyosarcoma) and fallopian tube (cancer);
hematology: blood (myeloid leukemia (acute and chronic), acute lymphoblastic leukemia, chronic lymphocytic leukemia, myeloproliferative disease, multiple myeloma and myelodysplastic syndrome), Hodgkin's disease and non-Hodgkin's lymphoma (malignant lymphoma);
skin: malignant melanoma, basal cell carcinoma, squamous cell carcinoma, Kaposi's sarcoma, dysplastic nevus, lipoma, hemangioma, dermatofibroma, keloid and psoriasis; and
adrenal gland: neuroblastoma.

26. The use according to claim 24, wherein the cancer is pancreatic cancer, lung cancer, colorectal cancer, cholangiocarcinoma, multiple myeloma, melanoma, uterine cancer, endometrial cancer, thyroid cancer, acute myeloid leukemia, bladder cancer, urothelial cancer, gastric cancer, cervical cancer, head and neck squamous cell carcinoma, diffuse large B-cell lymphoma, esophageal cancer, chronic lymphocytic leukemia, hepatocellular carcinoma, breast cancer, ovarian cancer, prostate cancer, glioblastoma, renal cancer or sarcoma.

27. Use of the compound, and/or the stereoisomer, the enantiomer, the diastereomer, the deuteride, the hydrate, the solvate, the prodrug and/or the pharmaceutically acceptable salt thereof according to any one of claims 1-22, or the pharmaceutical composition according to claim 23 in preparing a medicament for the treatment and/or prevention of neurofibromatosis type 1 (NF1), Noonan syndrome (NS), Noonan syndrome with multiple lentigines (NSML), capillary malformation-arteriovenous malformation syndrome (CM-AVM), Costello syndrome (CS), cardio-facio-cutaneous syndrome (CFC), Legius syndrome and hereditary gingival fibromatosis.

28. Use of the compound, and/or the stereoisomer, the enantiomer, the diastereomer, the deuteride, the hydrate, the solvate, the prodrug and/or the pharmaceutically acceptable salt thereof according to any one of claims 1-22, or the pharmaceutical composition according to claim 23 in preparing a medicament for the treatment and/or prevention of a KRAS-mediated disease or disorder, wherein the KRAS is preferably a KRAS mutant, and the KRAS mutant is preferably one or more of KRAS G12C, KRAS G 12D, KRAS G13D and KRAS G12V.
